# EUROPEAN PATENT SPECIFICATION

(11) **EP 4 446 327 B1**
(45) Date of publication and mention of the grant of the patent: **24.09.2025**
(21) Application number: 23835637.2
(22) Date of filing: 07.03.2023
(51) Int. Cl.: C07D 493/04, C07D 495/04, H10K 85/60, H10K 50/11, C09K 11/06, C07D 405/14

(54) **COMPOUND AND ORGANIC LIGHT-EMITTING ELEMENT COMPRISING SAME**
VERBINDUNG UND ORGANISCHES LICHTEMITTIERENDES ELEMENT DAMIT
COMPOSÉ ET ÉLÉMENT ÉLECTROLUMINESCENT ORGANIQUE LE COMPRENANT

(30) Priority: 07.07.2022 KR 20220083828; 25.11.2022 KR 20220160689
(43) Date of publication of application: 16.10.2024
(73) Proprietor: LG Chem, Ltd., Yeongdeungpo-gu Seoul 07336 (KR)
(72) Inventor: LEE, Da Jung, Daejeon 34122 (KR); KIM, Seonwoo, Daejeon 34122 (KR); CHO, Woo Jin, Daejeon 34122 (KR); CHA, Yongbum, Daejeon 34122 (KR); HA, Jae Seung, Daejeon 34122 (KR); GEUM, Sujeong, Daejeon 34122 (KR); LEE, Woochul, Daejeon 34122 (KR); HWANG, Sunghyun, Daejeon 34122 (KR)
(74) Representative: Hoffmann Eitle
(86) International application number: PCT/KR2023/003100
(87) International publication number: WO 2024/010167

(56) References cited:
- CN-A- 111 377 942
- JP-A- 2007 077 094
- KR-A- 20100 093 064
- KR-A- 20150 009 259
- KR-A- 20220 042 160
- US-A1- 2022 162 222

## Description

### [Technical Field]

The present specification relates to a compound and an organic light emitting device including the same.

### <Cross-Reference to Related Applications>

This application claims priority to and the benefit of Korean Patent Application Nos. 10-2022-0083828 and 10-2022-0160689 filed in the Korean Intellectual Property Office on July 7, 2022 and November 25, 2022

### [Background Art]

In general, an organic light emitting phenomenon refers to a phenomenon in which electric energy is converted into light energy by using an organic material. An organic light emitting device using the organic light emitting phenomenon usually has a structure including an anode, a cathode, and an organic material layer interposed therebetween. Here, the organic material layer may have a multi-layered structure composed of different materials in order to improve the efficiency and stability of an organic light emitting device in many cases, and for example, may be composed of a hole injection layer, a hole transport layer, a light emitting layer, an electron transport layer, an electron injection layer, and the like. In the structure of the organic light emitting device, if a voltage is applied between two electrodes, holes are injected from an anode into the organic material layer and electrons are injected from a cathode into the organic material layer, and when the injected holes and electrons meet each other, an exciton is formed, and light is emitted when the exciton falls down again to a ground state. Such organic light emitting devices are known from KR 2022 0042160 A, for instance. There is a continuous need for developing a new material for the aforementioned organic light emitting device.

### [Detailed Description of the Invention]

### [Technical Problem]

The present specification has been made in an effort to provide a compound and an organic light emitting device including the same.

### [Technical Solution]

An exemplary embodiment of the present specification provides a compound of the following Chemical Formula 1. 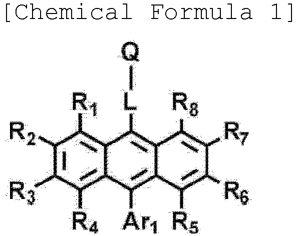 In Chemical Formula 1,
R₁ to R₈ are the same as or different from each other, and are each independently hydrogen, deuterium, a halogen group, a substituted or unsubstituted alkyl group, a substituted or unsubstituted cycloalkyl group, a substituted or unsubstituted aryl group, or a substituted or unsubstituted heteroaryl group,
Ar₁ is a substituted or unsubstituted aryl group, or a substituted or unsubstituted heteroaryl group, L is a direct bond or a substituted or unsubstituted arylene group,
Q is any one of the substituents of the following Chemical Formulae 2-1 to 2-6: 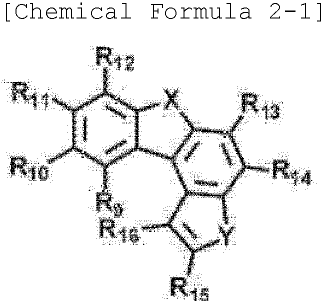 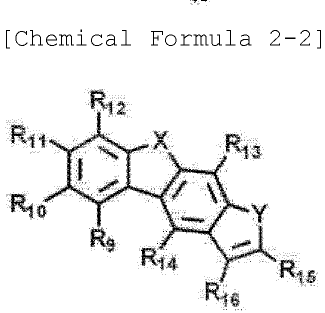 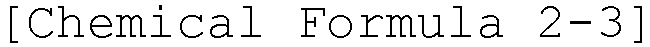 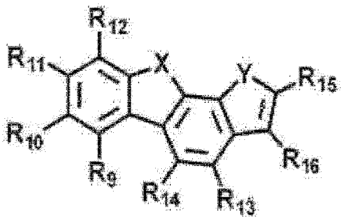 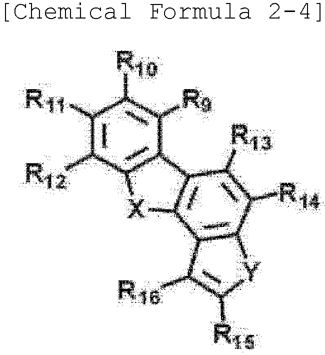 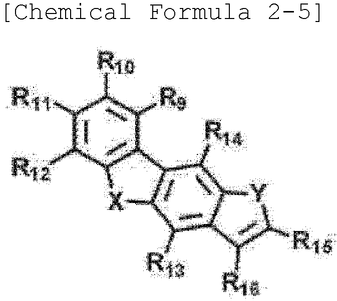 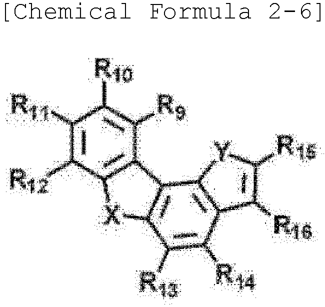
wherein in Chemical Formulae 2-1 to 2-6:
X and Y are the same as or different from each other, and are each independently O or S;
R₉ to R₁₆ are the same as or different from each other, and any one of R₉ to R₁₆ is linked to L of Chemical Formula 1;
the other groups which are not linked to L of Chemical Formula 1 among R₉ to R₁₆ are the same as or different from each other, and are each independently hydrogen, deuterium, a halogen, a cyano group, a silyl group, a substituted or unsubstituted alkyl group, a substituted or unsubstituted cycloalkyl group, a substituted or unsubstituted aryl group, or a substituted or unsubstituted heteroaryl group,
provided that in Chemical Formula 1, when L is a direct bond, Q is Chemical Formula 2-2, both X and Y are O, and any one of R₉ to R₁₄ and R₁₆ is linked to L of Chemical Formula 1, if any one of Ar₁ and R₁₅ is an unsubstituted phenyl group, the other is a substituted or unsubstituted aryl group having 10 to 60 carbon atoms, or a substituted or unsubstituted heteroaryl group having 2 to 60 carbon atoms.

Further, an exemplary embodiment of the present specification provides an organic light emitting device including: a first electrode; a second electrode provided to face the first electrode; and an organic material layer having one or more layers provided between the first electrode and the second electrode, in which one or more layers of the organic material layer include the above-described compound.

### [Advantageous Effects]

The compound described in the present specification can be used as a material for an organic material layer of an organic light emitting device. When an organic light emitting device is manufactured by including a compound of at least one exemplary embodiment of the exemplary embodiments in the present specification, an organic light emitting device having low voltage, high efficiency, and/or long service life characteristics can be obtained.

When the compound of the present invention is used in at least one layer of a hole transport layer, a hole injection layer, an electron transport layer, an electron injection layer and a light emitting layer, it is possible to obtain an effect in which the driving voltage of the device is lowered, or the efficiency of the device is increased, and/or the service life of the device is increased.

In particular, when the compound of the present invention is used as a blue host in the light emitting layer, it is possible to obtain an effect in which the driving voltage of the device is lowered, or the efficiency of the device is increased, and/or the service life of the device is increased.

### Brief Description of Drawings

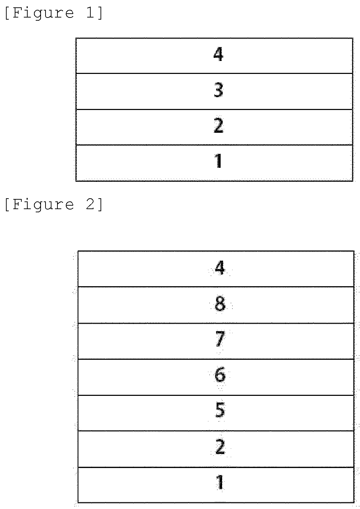

FIGS. 1 to 2 illustrate an example of the organic light emitting device according to the present invention.

### [Explanation of Reference Numerals and Symbols]

1: Substrate
2: Anode
3: Organic material layer
4: Cathode
5: Hole injection layer
6: Hole transport layer
7: Light emitting layer
8: Electron transport layer

### [Best Mode]

Hereinafter, the present specification will be described in more detail.

When one part "includes" one constituent element in the present specification, unless otherwise specifically described, this does not mean that another constituent element is excluded, but means that another constituent element may be further included.

When one member is disposed "on" another member in the present specification, this includes not only a case where the one member is brought into contact with another member, but also a case where still another member is present between the two members.

In the present specification, a first electrode may be an anode, and a second electrode may be a cathode. Alternatively, a first electrode may be a cathode, and a second electrode may be an anode.

Examples of the substituents in the present specification will be described below, but are not limited thereto.

The term "substitution" means that a hydrogen atom bonded to a carbon atom of a compound is changed into another substituent, and a position to be substituted is not limited as long as the position is a position at which the hydrogen atom is substituted, that is, a position at which the substituent may be substituted, and when two or more are substituted, the two or more substituents may be the same as or different from each other.

In the present specification, unless otherwise defined, the term "substituted or unsubstituted" means being substituted with one or two or more substituents selected from the group consisting of deuterium (-D), a halogen group, a cyano group (-CN), a silyl group, a boron group, an amine group, an alkyl group; an alkenyl group, an alkynyl group, an alkoxy group, a cycloalkyl group, an aryl group, and a heterocyclic group, being substituted with a substituent to which two or more substituents among the exemplified substituents are linked together, or having no substituent. For example, "the substituent to which two or more substituents are linked together" may be a biphenyl group. That is, the biphenyl group may also be interpreted as an aryl group, and may be interpreted as a substituent to which two phenyl groups are linked together.

Hereinafter, examples of the substituents of the present specification will be described, but are not limited thereto.

In the present specification, examples of a halogen (-X) include fluorine (-F), chlorine (-Cl), bromine (-Br) or iodine (-I).

In the present specification, a silyl group may be represented by a chemical formula of -SiYₐY_{b}Y_{c}, and the Yₐ, Y_{b}, and Y_{c} may be each unsubstituted or substituted with hydrogen, deuterium, a substituted or unsubstituted alkyl group, or a substituted or unsubstituted aryl group, and the like. Specific examples of the silyl group include a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group, and the like, but are not limited thereto.

In the present specification, a boron group may be unsubstituted or substituted with deuterium, a substituted or unsubstituted alkyl group, or a substituted or unsubstituted aryl group. Specific examples of the boron group include a trimethylboron group, a triethylboron group, a t-butyldimethylboron group, a triphenylboron group, a phenylboron group, and the like, but are not limited thereto.

In the present specification, an amine group may be selected from the group consisting of -NH₂, an alkylamine group, an N-alkylarylamine group, an arylamine group; an N-arylheteroarylamine group, an N-alkylheteroarylamine group, and a heteroarylamine group, and the number of carbon atoms thereof is not particularly limited, but is preferably 1 to 30. Specific examples of the amine group include a methylamine group, a dimethylamine group, an ethylamine group, a diethylamine group, a phenylamine group, a naphthylamine group, a biphenylamine group, an anthracenylamine group, a 9-methylanthracenylamine group, a diphenylamine group, a ditolylamine group, an N-phenyltolylamine group, a triphenylamine group, an N-phenylbiphenylamine group, an N-phenylnaphthylamine group, an N-biphenylnaphthylamine group, an N-naphthylfluorenylamine group, an N-phenylphenanthrenylamine group, an N-biphenylphenanthrenylamine group, an N-phenylfluorenylamine group, an N-phenylterphenylamine group; an N-phenanthrenylfluorenylamine group, an N-biphenylfluorenylamine group, and the like, but are not limited thereto.

In the present specification, an N-alkylarylamine group means an amine group in which an alkyl group and an aryl group are substituted with N of the amine group.

In the present specification, an N-arylheteroarylamine group means an amine group in which an aryl group and a heteroaryl group are substituted with N of the amine group.

In the present specification, an N-alkylheteroarylamine group means an amine group in which an alkyl group and a heteroaryl group are substituted with N of the amine group.

In the present specification, the alkyl group in the alkylamine group, the N-arylalkylamine group, the alkylthioxy group, the alkylsulfoxy group, and the N-alkylheteroarylamine group is the same as the above-described examples of the alkyl group. Specifically, examples of the alkylthioxy group include a methylthioxy group, an ethylthioxy group, a tert-butylthioxy group, a hexylthioxy group, an octylthioxy group, and the like, and examples of the alkylsulfoxy group include mesyl, an ethylsulfoxy group, a propylsulfoxy group, a butylsulfoxy group, and the like, but the examples are not limited thereto.

In the present specification, the alkyl group may be straight-chained or branched, and the number of carbon atoms thereof is not particularly limited, but is preferably 1 to 60. According to an exemplary embodiment, the number of carbon atoms of the alkyl group is 1 to 30. According to another exemplary embodiment, the number of carbon atoms of the alkyl group is 1 to 20. According to still another exemplary embodiment, the number of carbon atoms of the alkyl group is 1 to 10. Specific examples of the alkyl group include a methyl group, an ethyl group, a propyl group, an isopropyl group, a butyl group, an isobutyl group, a tert-butyl group, a pentyl group, a hexyl group, a heptyl group, an octyl group, and the like, but are not limited thereto.

In the present specification, the alkenyl group may be straight-chained or branched, and the number of carbon atoms thereof is not particularly limited, but is preferably 2 to 40. According to an exemplary embodiment, the number of carbon atoms of the alkenyl group is 2 to 20. According to another exemplary embodiment, the number of carbon atoms of the alkenyl group is 2 to 10. According to still another exemplary embodiment, the number of carbon atoms of the alkenyl group is 2 to 6. Specific examples thereof include vinyl, 1-propenyl, isopropenyl, 1-butenyl, 2-butenyl, 3-butenyl, 1-pentenyl, 2-pentenyl, 3-pentenyl, 3-methyl-1-butenyl, 1,3-butadienyl, allyl, 1-phenylvinyl-1-yl, 2-phenylvinyl-1-yl, 2,2-diphenylvinyl-1-yl, 2-phenyl-2-(naphthyl-1-yl)vinyl-1-yl, 2,2-bis(diphenyl-1-yl)vinyl-1-yl, a stilbenyl group, a styrenyl group, and the like, but are not limited thereto.

In the present specification, the alkynyl group may be straight or branched, and the number of carbon atoms thereof is not particularly limited, but is preferably 2 to 30. Specific examples thereof include an alkynyl group such as ethynyl, propynyl, 2-methyl-2-propynyl, 2-butynyl, and 2-pentynyl, and the like, but are not limited thereto.

In the present specification, the alkoxy group may be straight-chained, branched, or cyclic. The number of carbon atoms of the alkoxy group is not particularly limited, but is preferably 1 to 40. Specific examples thereof include methoxy, ethoxy, n-propoxy, isopropoxy, i-propyloxy, n-butoxy, isobutoxy, tert-butoxy, sec-butoxy, n-pentyloxy, neopentyloxy, isopentyloxy, n-hexyloxy, 3,3-dimethylbutyloxy, 2-ethylbutyloxy, n-octyloxy, n-nonyloxy, n-decyloxy, benzyloxy, p-methylbenzyloxy, and the like, but are not limited thereto.

Substituents including an alkyl group, an alkoxy group, and other alkyl group moieties described in the present specification include both a straight-chained form and a branched form.

In the present specification, a cycloalkyl group is not particularly limited, but has preferably 3 to 60 carbon atoms, and according to an exemplary embodiment, the number of carbon atoms of the cycloalkyl group is 3 to 30. According to another exemplary embodiment, the number of carbon atoms of the cycloalkyl group is 3 to 20. According to still another exemplary embodiment, the number of carbon atoms of the cycloalkyl group is 3 to 6. Specific examples thereof include a cyclopropyl group, a cyclobutyl group, a cyclopentyl group, a cyclohexyl group, a cycloheptyl group, a cyclooctyl group, and the like, but are not limited thereto.

In the present specification, an aryl group is not particularly limited, but has preferably 6 to 60 carbon atoms, and may be a monocyclic aryl group or a polycyclic aryl group. According to an exemplary embodiment, the number of carbon atoms of the aryl group is 6 to 30. According to an exemplary embodiment, the number of carbon atoms of the aryl group is 6 to 20. Examples of the monocyclic aryl group include a phenyl group, a biphenyl group, a terphenyl group, and the like, but are not limited thereto. Examples of the multicyclic aryl group include a naphthyl group, an anthracenyl group, a phenanthrenyl group, a pyrenyl group, a perylenyl group, a triphenylene group, a chrysenyl group, a fluorenyl group, and the like, but are not limited thereto.

In the present specification, the fluorenyl group may be substituted, and two substituents may be bonded to each other to form a spiro structure.

When the fluorenyl group is substituted, a substituted fluorenyl group may be formed such as 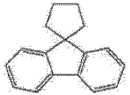 (a spirofluorenyl group), 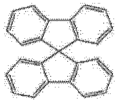 (a spirobifluorenyl group), 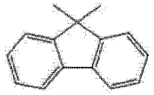 (a 9,9-dimethylfluorenyl group) and 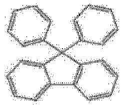 (a 9,9-diphenylfluorenyl group), but is not limited thereto.

The aryl group may be substituted with an alkyl group to act as an arylalkyl group. The alkyl group may be selected from the above-described examples.

In the present specification, a heterocyclic group is a ring including one or more atoms other than carbon, that is, one or more heteroatoms, and specifically, the heteroatom may include one or more atoms selected from the group consisting of O, N, S, and the like. The heterocyclic group may be monocyclic or polycyclic, may be an aromatic ring, an aliphatic ring, or a fused ring of the aromatic ring and the aliphatic ring, and may be selected from the examples of the heteroaryl group.

In the present specification, a heteroaryl group is an aryl group including one or more of N, O, P, S, Si, and Se as a heteroatom, and the number of carbon atoms thereof is not particularly limited, but is preferably 2 to 60. According to an exemplary embodiment, the number of carbon atoms of the heteroaryl group is 2 to 30. Examples of the heteroaryl group include a pyridine group, a pyrrole group, a pyrimidine group, a pyridazinyl group, a furan group, a thiophene group, an imidazole group, a pyrazole group, a dibenzofuran group, a dibenzothiophene group, a carbazole group, and the like, but are not limited thereto.

In the present specification, an alkylene group is the same as that defined in the alkyl group, except for being a divalent group.

In the present specification, an alkenylene group is the same as that defined in the alkenyl group, except for being a divalent group.

In the present specification, a cycloalkylene group is the same as that defined in the cycloalkyl group, except for being a divalent group.

In the present specification, a cycloalkenylene group is the same as that defined in the cycloalkenyl group, except for being a divalent group.

In the present specification, an arylene group is the same as that defined in the aryl group, except for being a divalent group.

In the present specification, a heteroarylene group is the same as that defined in the heteroaryl group, except for being a divalent group.

An exemplary embodiment of the present specification provides a compound of the following Chemical Formula 1. 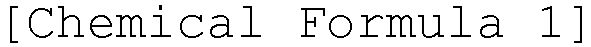 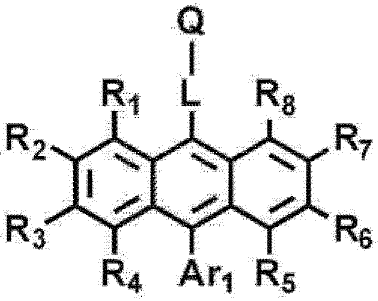 In Chemical Formula 1,
R₁ to R₈ are the same as or different from each other, and are each independently hydrogen, deuterium, a halogen group, a substituted or unsubstituted alkyl group, a substituted or unsubstituted cycloalkyl group, a substituted or unsubstituted aryl group, or a substituted or unsubstituted heteroaryl group,
Ar₁ is a substituted or unsubstituted aryl group, or a substituted or unsubstituted heteroaryl group,
L is a direct bond or a substituted or unsubstituted arylene group,
Q is any one of the substituents of the following Chemical Formulae 2-1 to 2-6, 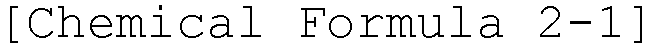 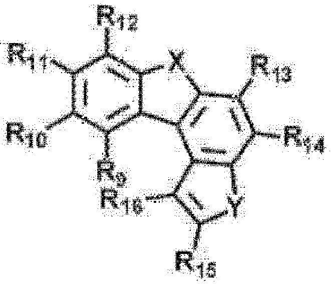 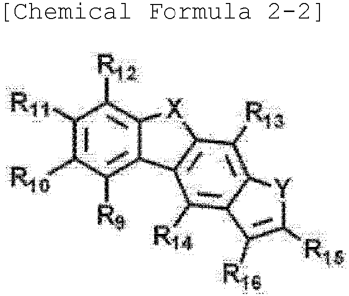 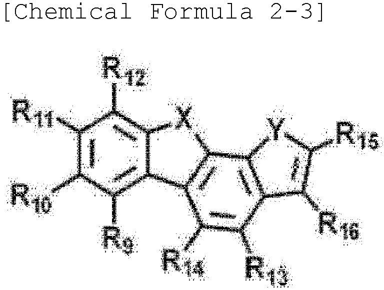 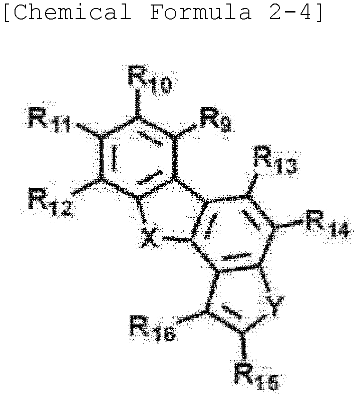 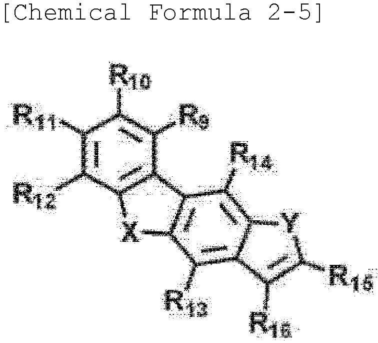 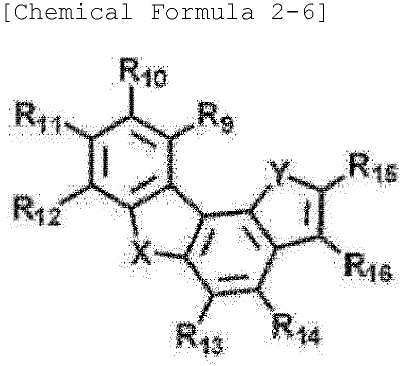
wherein in Chemical Formulae 2-1 to 2-6,
X and Y are the same as or different from each other, and are each independently O or S,
any one of R₉ to R₁₆ is linked to L of Chemical Formula 1,
the other groups which are not linked to L of Chemical Formula 1 among R₉ to R₁₆ are the same as or different from each other, and are each independently hydrogen, deuterium, a halogen, a cyano group, a silyl group, a substituted or unsubstituted alkyl group, a substituted or unsubstituted cycloalkyl group, a substituted or unsubstituted aryl group, or a substituted or unsubstituted heteroaryl group,
provided that in Chemical Formula 1, when L is a direct bond, Q is Chemical Formula 2-2, both X and Y are O, and any one of R₉ to R₁₄ and R₁₆ is linked to L of Chemical Formula 1, if any one of Ar₁ and R₁₅ is an unsubstituted phenyl group, the other is a substituted or unsubstituted aryl group having 10 to 60 carbon atoms, or a substituted or unsubstituted heteroaryl group having 2 to 60 carbon atoms. In other words, in Chemical Formula 1, when L is a direct bond, Q is Chemical Formula 2-2, both X and Y are O, and any one of R₉ to R₁₄ and R₁₆ is linked to L of Chemical Formula 1, if any one of Ar₁ and R₁₅ is an unsubstituted phenyl group, the other cannot be an unsubstituted phenyl group.

Compounds according to the above-described exemplary embodiments have stable forms in both oxidized and reduced states, and have high light emitting efficiency in converting excitons into light when the excitons are formed.

When the compound of the present invention is used in at least one layer of a hole transport layer, a hole injection layer, an electron transport layer, an electron injection layer and a light emitting layer, it is possible to obtain an effect in which the driving voltage of the device is lowered, or the efficiency of the device is increased, and/or the service life of the device is increased.

In particular, when the compound of the present invention is used as a blue host in the light emitting layer, it is possible to obtain an effect in which the driving voltage of the device is lowered, or the efficiency of the device is increased, and/or the service life of the device is increased.

According to an exemplary embodiment of the present specification, provided is a compound in which R₁ to R₈ are the same as or different from each other, and are each independently hydrogen, deuterium, a substituted or unsubstituted alkyl group having 1 to 60 carbon atoms, a substituted or unsubstituted cycloalkyl group having 3 to 60 carbon atoms, a substituted or unsubstituted aryl group having 6 to 60 carbon atoms, or a substituted or unsubstituted heteroaryl group having 2 to 60 carbon atoms.

According to an exemplary embodiment of the present specification, provided is a compound in which R₁ to R₈ are the same as or different from each other, and are each independently hydrogen, deuterium, a substituted or unsubstituted alkyl group having 1 to 40 carbon atoms, a substituted or unsubstituted cycloalkyl group having 3 to 40 carbon atoms, a substituted or unsubstituted aryl group having 6 to 40 carbon atoms, or a substituted or unsubstituted heteroaryl group having 2 to 40 carbon atoms.

According to an exemplary embodiment of the present specification, provided is a compound in which R₁ to R₈ are the same as or different from each other, and are each independently hydrogen, deuterium, a substituted or unsubstituted alkyl group having 1 to 20 carbon atoms, a substituted or unsubstituted cycloalkyl group having 3 to 20 carbon atoms, a substituted or unsubstituted aryl group having 6 to 20 carbon atoms, or a substituted or unsubstituted heteroaryl group having 2 to 20 carbon atoms.

According to an exemplary embodiment of the present specification, provided is a compound in which R₁ to R₈ are the same as or different from each other, and are each independently hydrogen, deuterium, or a substituted or unsubstituted alkyl group having 1 to 20 carbon atoms.

According to an exemplary embodiment of the present specification, provided is a compound in which R₁ to R₈ are the same as or different from each other, and are each independently hydrogen or deuterium.

According to an exemplary embodiment of the present specification, provided is a compound in which the Ar₁ isa substituted or unsubstituted aryl group having 6 to 60 carbon atoms, or a substituted or unsubstituted heteroaryl group having 2 to 60 carbon atoms.

According to an exemplary embodiment of the present specification, provided is a compound in which the Ar₁ isa substituted or unsubstituted aryl group having 6 to 40 carbon atoms, or a substituted or unsubstituted heteroaryl group having 2 to 40 carbon atoms.

According to an exemplary embodiment of the present specification, provided is a compound in which the Ar₁ is a substituted or unsubstituted aryl group having 6 to 30 carbon atoms, or a substituted or unsubstituted heteroaryl group having 2 to 30 carbon atoms.

According to an exemplary embodiment of the present specification, provided is a compound in which the Ar₁ is a substituted or unsubstituted phenyl group, a substituted or unsubstituted biphenyl group, a substituted or unsubstituted naphthyl group, a substituted or unsubstituted phenanthrene group, a substituted or unsubstituted dibenzofuran group, a substituted or unsubstituted dibenzothiophene group, or a substituted or unsubstituted carbazole group, and the term 'substituted or unsubstituted' means being unsubstituted or substituted with one or more substituents selected from the group consisting of deuterium, a halogen group, an alkyl group having 1 to 60 carbon atoms, an aryl group having 6 to 60 carbon atoms, and a heteroaryl group having 2 to 60 carbon atoms, or the one or more adjacent substituents are bonded to each other to form a ring.

According to an exemplary embodiment of the present specification, provided is a compound in which the Ar₁ is a substituted or unsubstituted phenyl group, a substituted or unsubstituted biphenyl group, a substituted or unsubstituted naphthyl group, a substituted or unsubstituted phenanthrene group, a substituted or unsubstituted dibenzofuran group, a substituted or unsubstituted dibenzothiophene group, or a substituted or unsubstituted carbazole group, and the term 'substituted or unsubstituted' means being unsubstituted or substituted with one or more substituents selected from the group consisting of deuterium, a halogen group, an alkyl group having 1 to 40 carbon atoms, an aryl group having 6 to 40 carbon atoms, and a heteroaryl group having 2 to 40 carbon atoms, or the one or more adjacent substituents are bonded to each other to form an aromatic hydrocarbon ring having 6 to 40 carbon atoms or an aromatic hetero ring having 2 to 40 carbon atoms.

According to an exemplary embodiment of the present specification, provided is a compound in which the Ar₁ is a substituted or unsubstituted phenyl group, a substituted or unsubstituted biphenyl group, a substituted or unsubstituted naphthyl group, a substituted or unsubstituted phenanthrene group, a substituted or unsubstituted dibenzofuran group, a substituted or unsubstituted dibenzothiophene group, or a substituted or unsubstituted carbazole group, and the term 'substituted or unsubstituted' means being unsubstituted or substituted with one or more substituents selected from the group consisting of deuterium, a halogen group, an alkyl group having 1 to 30 carbon atoms, an aryl group having 6 to 30 carbon atoms, and a heteroaryl group having 2 to 30 carbon atoms, or the one or more adjacent substituents are bonded to each other to form an aromatic hydrocarbon ring having 6 to 30 carbon atoms or an aromatic hetero ring having 2 to 30 carbon atoms.

According to an exemplary embodiment of the present specification, provided is a compound in which the Ar₁ is a substituted or unsubstituted phenyl group, a substituted or unsubstituted biphenyl group, a substituted or unsubstituted naphthyl group, a substituted or unsubstituted phenanthrene group, a substituted or unsubstituted dibenzofuran group, a substituted or unsubstituted dibenzothiophene group, or a substituted or unsubstituted carbazole group, and the term 'substituted or unsubstituted' means being unsubstituted or substituted with one or more substituents selected from the group consisting of deuterium, an alkyl group having 1 to 10 carbon atoms, an aryl group having 6 to 20 carbon atoms, and a heteroaryl group having 2 to 20 carbon atoms, or the one or more adjacent substituents are bonded to each other to form a phenyl ring, a naphthyl ring, a benzofuran ring, or a benzothiophene ring.

According to an exemplary embodiment of the present specification, provided is a compound in which L is a direct bond or a substituted or unsubstituted arylene group having 6 to 30 carbon atoms.

According to an exemplary embodiment of the present specification, provided is a compound in which L is a direct bond or a substituted or unsubstituted arylene group having 6 to 20 carbon atoms.

According to an exemplary embodiment of the present specification, provided is a compound in which L is a direct bond, a substituted or unsubstituted phenylene group, or a substituted or unsubstituted naphthylene group.

According to an exemplary embodiment of the present specification, provided is a compound in which L is a direct bond; a phenylene group which is unsubstituted or substituted with deuterium; or a naphthylene group which is unsubstituted or substituted with deuterium.

According to an exemplary embodiment of the present specification, provided is a compound in which L is a direct bond or an unsubstituted phenylene group.

According to an exemplary embodiment of the present specification, provided is a compound in which Chemical Formula 2-1 is any one of the following Chemical Formulae 2-1-A to 2-1-D.

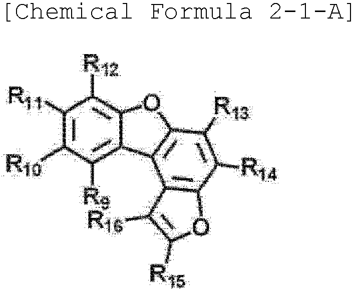

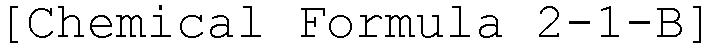

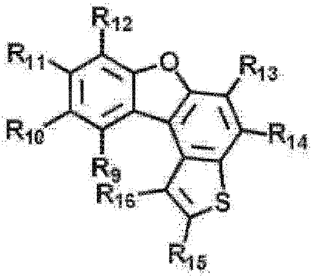

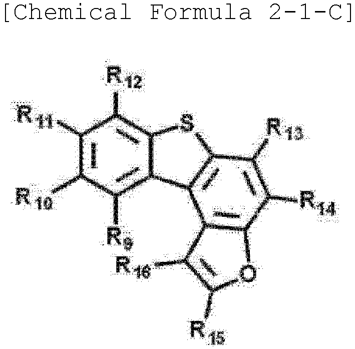

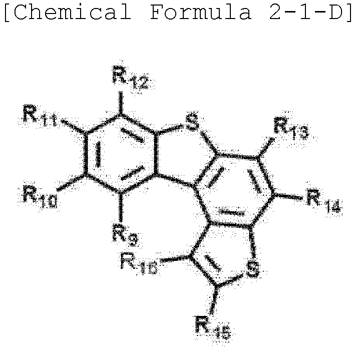

In Chemical Formulae 2-1-A to 2-1-D,
R₉ to R₁₆ are the same as those defined in Chemical Formulae 2-1 to 2-6.

According to an exemplary embodiment of the present specification, provided is a compound in which Chemical Formula 2-2 is any one of the following Chemical Formulae 2-2-A to 2-2-D.

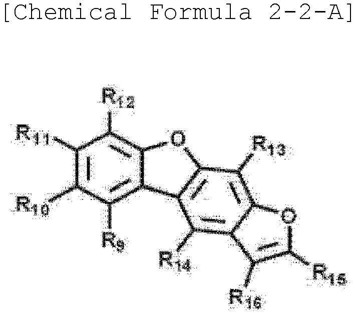

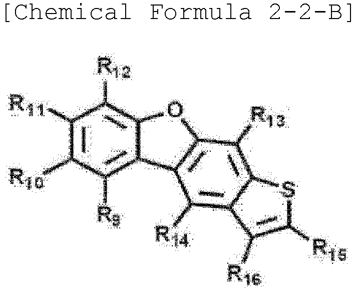

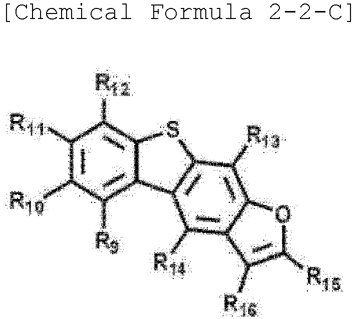

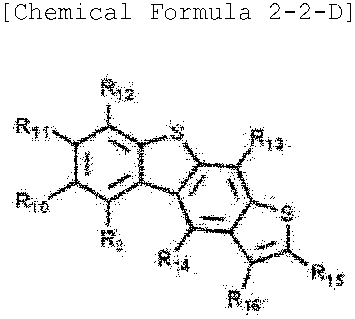

In Chemical Formulae 2-2-A to 2-2-D,
R₉ to R₁₆ are the same as those defined in Chemical Formulae 2-1 to 2-6.

According to an exemplary embodiment of the present specification, provided is a compound in which Chemical Formula 2-3 is any one of the following Chemical Formulae 2-3-A to 2-3-D.

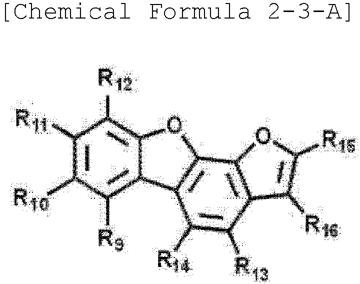

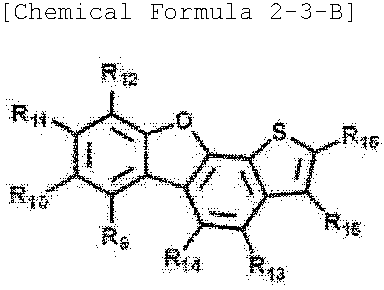

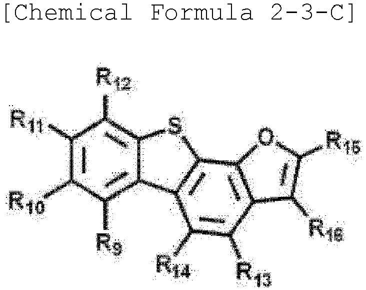

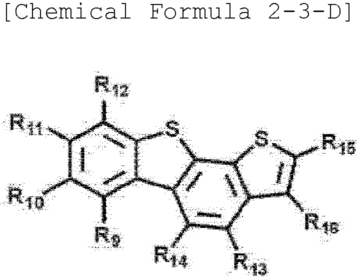

In Chemical Formulae 2-3-A to 2-3-D,
R₉ to R₁₆ are the same as those defined in Chemical Formulae 2-1 to 2-6.

According to an exemplary embodiment of the present specification, provided is a compound in which Chemical Formula 2-4 is any one of the following Chemical Formulae 2-4-A to 2-4-D.

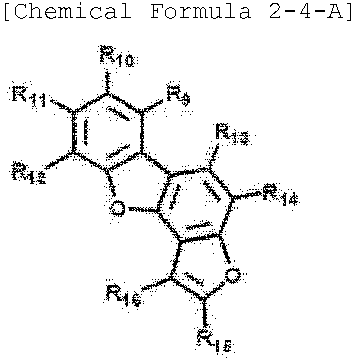

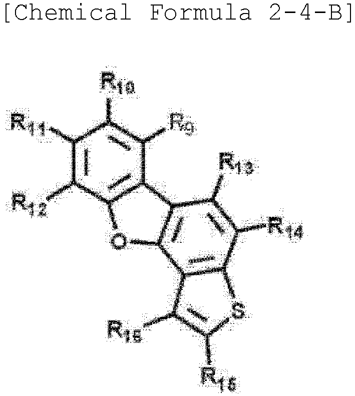

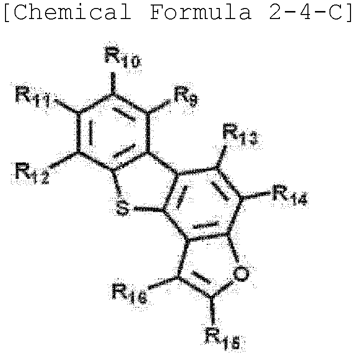

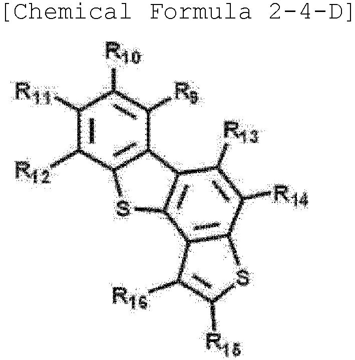

In Chemical Formulae 2-4-A to 2-4-D,
R₉ to R₁₆ are the same as those defined in Chemical Formulae 2-1 to 2-6.

According to an exemplary embodiment of the present specification, provided is a compound in which Chemical Formula 2-5 is any one of the following Chemical Formulae 2-5-A to 2-5-D.

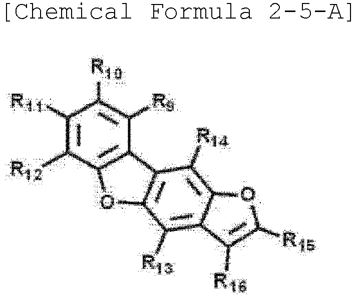

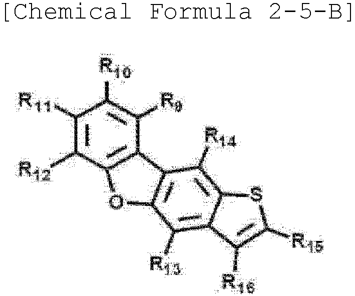

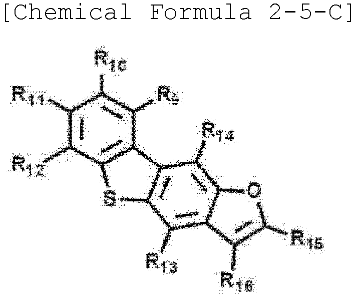

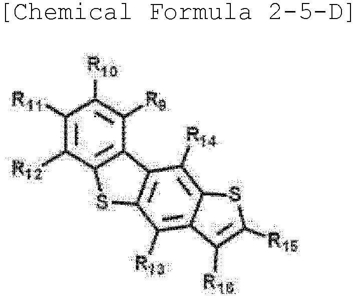

In Chemical Formulae 2-5-A to 2-5-D,
R₉ to R₁₆ are the same as those defined in Chemical Formulae 2-1 to 2-6.

According to an exemplary embodiment of the present specification, provided is a compound in which Chemical Formula 2-6 is any one of the following Chemical Formulae 2-6-A to 2-6-D.

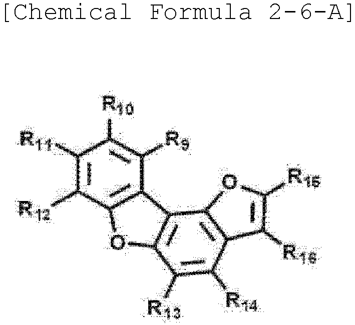

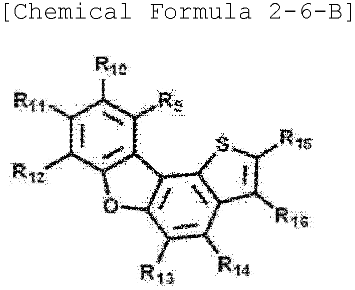

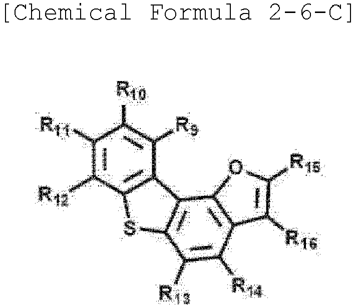

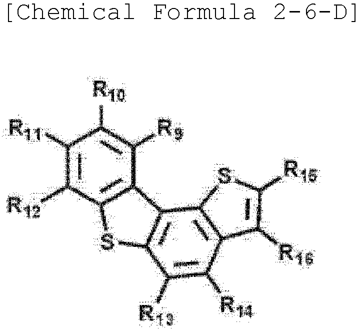

In Chemical Formulae 2-6-A to 2-6-D,
R₉ to R₁₆ are the same as those defined in Chemical Formulae 2-1 to 2-6.

According to an exemplary embodiment of the present specification, in Chemical Formula 1, when L is a direct bond, Q is Chemical Formula 2-2, both X and Y are O, and any one of R₉ to R₁₄ and R₁₆ is linked to L of Chemical Formula 1, if any one of Ar₁ and R₁₅ is an unsubstituted phenyl group, the other (the other in which any one of Ar₁ and R₁₅ is not an unsubstituted phenyl group) is a substituted or unsubstituted aryl group having 10 to 40 carbon atoms, or a substituted or unsubstituted heteroaryl group having 2 to 40 carbon atoms.

According to an exemplary embodiment of the present specification, in Chemical Formula 1, when L is a direct bond, Q is Chemical Formula 2-2, both X and Y are O, and any one of R₉ to R₁₄ and R₁₆ is linked to L of Chemical Formula 1, if any one of Ar₁ and R₁₅ is an unsubstituted phenyl group, the other is a substituted or unsubstituted aryl group having 10 to 20 carbon atoms, or a substituted or unsubstituted heteroaryl group having 2 to 20 carbon atoms.

According to an exemplary embodiment of the present specification, in Chemical Formula 1, when L is a direct bond, Q is Chemical Formula 2-2, both X and Y are O, and any one of R₉ to R₁₄ and R₁₆ is linked to L of Chemical Formula 1, if any one of Ar₁ and R₁₅ is an unsubstituted phenyl group, the other is a substituted or unsubstituted biphenyl group, a substituted or unsubstituted naphthyl group, a substituted or unsubstituted dibenzofuran group, or a substituted or unsubstituted dibenzothiophene group, the term 'substituted or unsubstituted' means being unsubstituted or substituted with one or more substituents selected from the group consisting of deuterium, a halogen group, an alkyl group having 1 to 60 carbon atoms, an aryl group having 6 to 60 carbon atoms, and a heteroaryl group having 2 to 60 carbon atoms, or the one or more adjacent substituents may be bonded to each other to form a ring.

According to an exemplary embodiment of the present specification, in Chemical Formula 1, when L is a direct bond, Q is Chemical Formula 2-2, both X and Y are O, and any one of R₉ to R₁₄ and R₁₆ is linked to L of Chemical Formula 1, if any one of Ar₁ and R₁₅ is an unsubstituted phenyl group, the other is a substituted or unsubstituted biphenyl group, a substituted or unsubstituted naphthyl group, a substituted or unsubstituted dibenzofuran group, or a substituted or unsubstituted dibenzothiophene group, the term 'substituted or unsubstituted' means being unsubstituted or substituted with one or more substituents selected from the group consisting of deuterium, a halogen group, an alkyl group having 1 to 40 carbon atoms, an aryl group having 6 to 40 carbon atoms, and a heteroaryl group having 2 to 40 carbon atoms, or the one or more adjacent substituents may be bonded to each other to form an aromatic hydrocarbon ring having 6 to 40 carbon atoms or an aromatic hetero ring having 2 to 40 carbon atoms.

According to an exemplary embodiment of the present specification, in Chemical Formula 1, when L is a direct bond, Q is Chemical Formula 2-2, both X and Y are O, and any one of R₉ to R₁₄ and R₁₆ is linked to L of Chemical Formula 1, if any one of Ar₁ and R₁₅ is an unsubstituted phenyl group, the other is a substituted or unsubstituted biphenyl group, a substituted or unsubstituted naphthyl group, a substituted or unsubstituted dibenzofuran group, or a substituted or unsubstituted dibenzothiophene group, the term 'substituted or unsubstituted' means being unsubstituted or substituted with one or more substituents selected from the group consisting of deuterium, a halogen group, an alkyl group having 1 to 30 carbon atoms, an aryl group having 6 to 30 carbon atoms, and a heteroaryl group having 2 to 30 carbon atoms, or the one or more adjacent substituents may be bonded to each other to form an aromatic hydrocarbon ring having 6 to 30 carbon atoms or an aromatic hetero ring having 2 to 30 carbon atoms.

According to an exemplary embodiment of the present specification, in Chemical Formula 1, when L is a direct bond, Q is Chemical Formula 2-2, both X and Y are O, and any one of R9 to R14 and R16 is linked to L of Chemical Formula 1, if any one of Ar1 and R15 is an unsubstituted phenyl group, the other is a substituted or unsubstituted biphenyl group, a substituted or unsubstituted naphthyl group, a substituted or unsubstituted dibenzofuran group, or a substituted or unsubstituted dibenzothiophene group, the term 'substituted or unsubstituted' means being unsubstituted or substituted with one or more substituents selected from the group consisting of deuterium, a halogen group, an alkyl group having 1 to 30 carbon atoms, an aryl group having 6 to 30 carbon atoms, and a heteroaryl group having 2 to 30 carbon atoms, or the one or more adjacent substituents may be bonded to each other to form a phenyl ring, a naphthyl ring, a benzofuran ring, or a benzothiophene ring.

According to an exemplary embodiment of the present specification, provided is a compound in which the other groups which are not linked to L of Chemical Formula 1 among R₉ to R₁₆ are the same as or different from each other, and are each independently hydrogen, deuterium, a substituted or unsubstituted aryl group, or a substituted or unsubstituted heteroaryl group.

According to an exemplary embodiment of the present specification, provided is a compound in which the other groups which are not linked to L of Chemical Formula 1 among R₉ to R₁₆ are the same as or different from each other, and are each independently hydrogen, deuterium, a substituted or unsubstituted aryl group having 6 to 60 carbon atoms, or a substituted or unsubstituted heteroaryl group having 2 to 60 carbon atoms.

According to an exemplary embodiment of the present specification, provided is a compound in which the other groups which are not linked to L of Chemical Formula 1 among R₉ to R₁₆ are the same as or different from each other, and are each independently hydrogen, deuterium, a substituted or unsubstituted aryl group having 6 to 40 carbon atoms, or a substituted or unsubstituted heteroaryl group having 2 to 40 carbon atoms.

According to an exemplary embodiment of the present specification, provided is a compound in which the other groups which are not linked to L of Chemical Formula 1 among R₉ to R₁₆ are the same as or different from each other, and are each independently hydrogen, deuterium, a substituted or unsubstituted aryl group having 6 to 30 carbon atoms, or a substituted or unsubstituted heteroaryl group having 2 to 30 carbon atoms.

According to an exemplary embodiment of the present specification, provided is a compound in which the other groups which are not linked to L of Chemical Formula 1 among R₉ to R₁₆ are the same as or different from each other, and are each independently hydrogen, deuterium, a substituted or unsubstituted aryl group having 6 to 20 carbon atoms, or a substituted or unsubstituted heteroaryl group having 2 to 20 carbon atoms.

According to an exemplary embodiment of the present specification, provided is a compound in which the other groups which are not linked to L of Chemical Formula 1 among R₉ to R₁₆ are the same as or different from each other, and are each independently hydrogen, deuterium, a substituted or unsubstituted phenyl group, a substituted or unsubstituted biphenyl group, a substituted or unsubstituted naphthyl group, a substituted or unsubstituted dibenzofuran group, or a substituted or unsubstituted dibenzothiophene group, and the term 'substituted or unsubstituted' means being unsubstituted or substituted with one or more substituents selected from the group consisting of deuterium, a halogen group, an alkyl group having 1 to 60 carbon atoms, an aryl group having 6 to 60 carbon atoms, and a heteroaryl group having 2 to 60 carbon atoms, or the one or more adjacent substituents are bonded to each other to form a ring.

According to an exemplary embodiment of the present specification, provided is a compound in which the other groups which are not linked to L of Chemical Formula 1 among R₉ to R₁₆ are the same as or different from each other, and are each independently hydrogen, deuterium, a substituted or unsubstituted phenyl group, a substituted or unsubstituted biphenyl group, a substituted or unsubstituted naphthyl group, a substituted or unsubstituted dibenzofuran group, or a substituted or unsubstituted dibenzothiophene group, and the term 'substituted or unsubstituted' means being unsubstituted or substituted with one or more substituents selected from the group consisting of deuterium, a halogen group, an alkyl group having 1 to 40 carbon atoms, an aryl group having 6 to 40 carbon atoms, and a heteroaryl group having 2 to 40 carbon atoms, or the one or more adjacent substituents are bonded to each other to form an aromatic hydrocarbon ring having 6 to 40 carbon atoms or an aromatic hetero ring having 2 to 40 carbon atoms.

According to an exemplary embodiment of the present specification, provided is a compound in which the other groups which are not linked to L of Chemical Formula 1 among R₉ to R₁₆ are the same as or different from each other, and are each independently hydrogen, deuterium, a substituted or unsubstituted phenyl group, a substituted or unsubstituted biphenyl group, a substituted or unsubstituted naphthyl group, a substituted or unsubstituted dibenzofuran group, or a substituted or unsubstituted dibenzothiophene group, and the term 'substituted or unsubstituted' means being unsubstituted or substituted with one or more substituents selected from the group consisting of deuterium, a halogen group, an alkyl group having 1 to 30 carbon atoms, an aryl group having 6 to 30 carbon atoms, and a heteroaryl group having 2 to 30 carbon atoms, or the one or more adjacent substituents are bonded to each other to form an aromatic hydrocarbon ring having 6 to 30 carbon atoms or an aromatic hetero ring having 2 to 30 carbon atoms.

According to an exemplary embodiment of the present specification, provided is a compound in which the other groups which are not linked to L of Chemical Formula 1 among R₉ to R₁₆ are the same as or different from each other, and are each independently hydrogen, deuterium, a substituted or unsubstituted phenyl group, a substituted or unsubstituted biphenyl group, a substituted or unsubstituted naphthyl group, a substituted or unsubstituted dibenzofuran group, or a substituted or unsubstituted dibenzothiophene group, and the term 'substituted or unsubstituted' means being unsubstituted or substituted with one or more substituents selected from the group consisting of deuterium, a halogen group, an alkyl group having 1 to 30 carbon atoms, an aryl group having 6 to 30 carbon atoms, and a heteroaryl group having 2 to 30 carbon atoms, or the one or more adjacent substituents are bonded to each other to form a phenyl ring, a naphthyl ring, a benzofuran ring, or a benzothiophene ring.

According to an exemplary embodiment of the present specification, the Chemical Formula 1 can be any one of the following compounds.

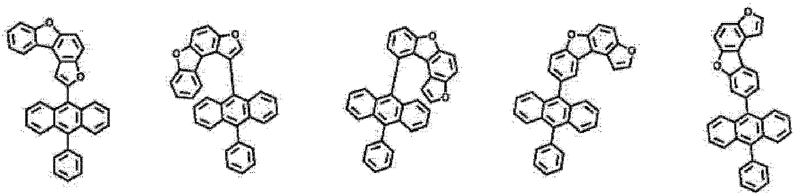

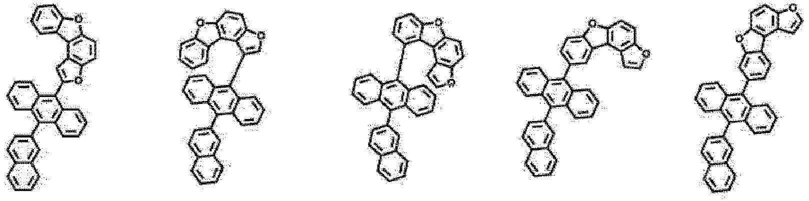

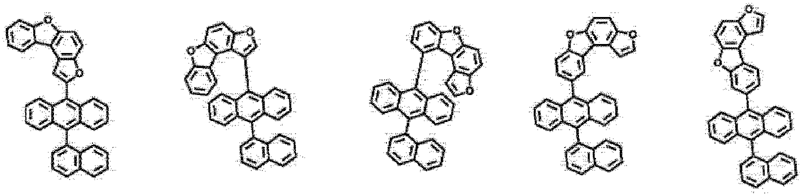

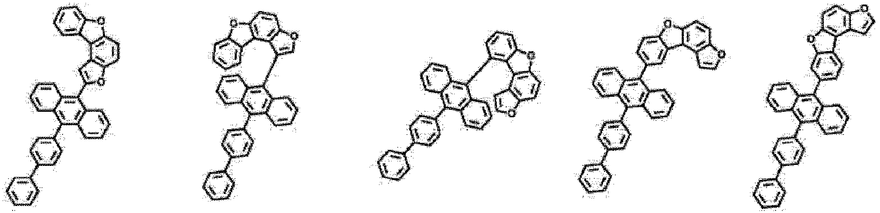

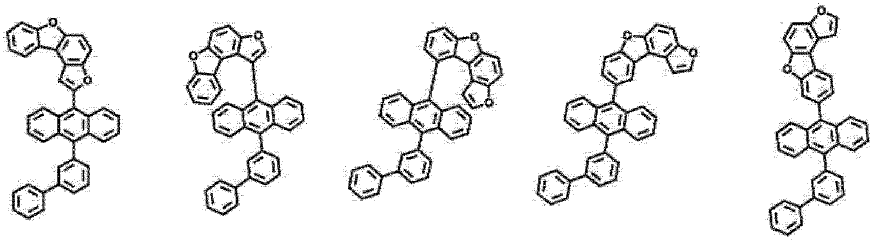

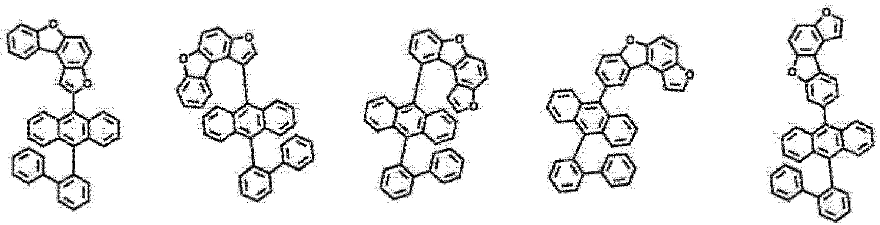

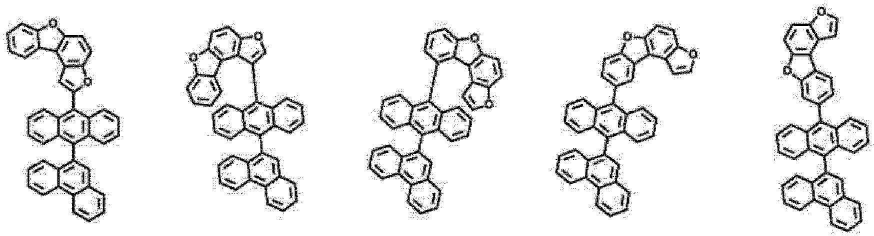

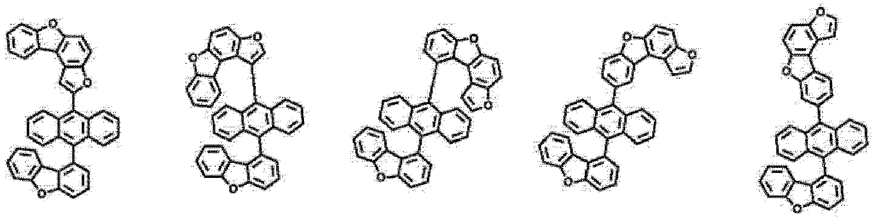

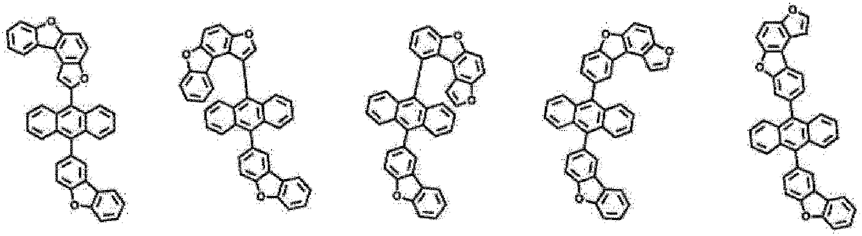

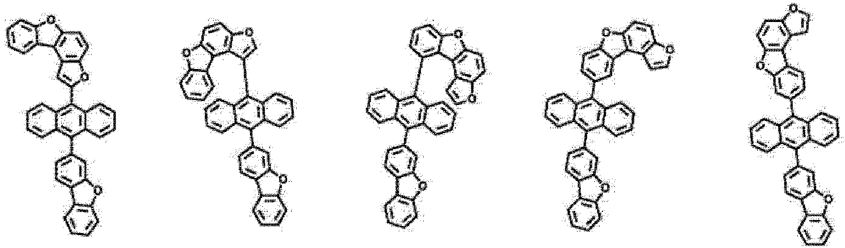

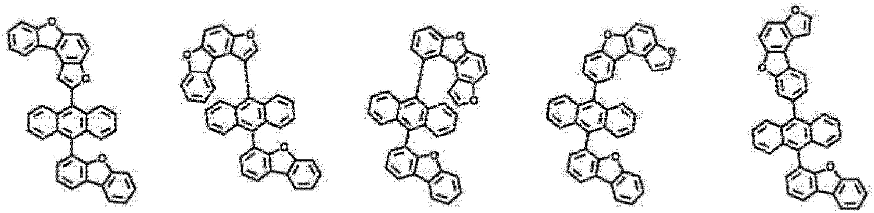

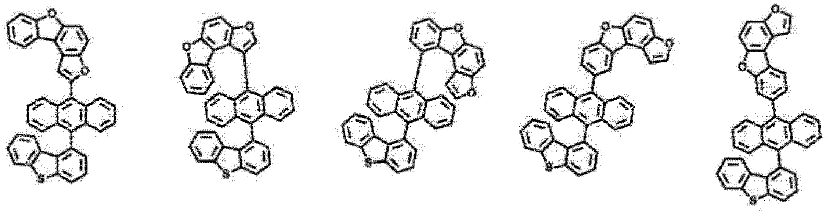

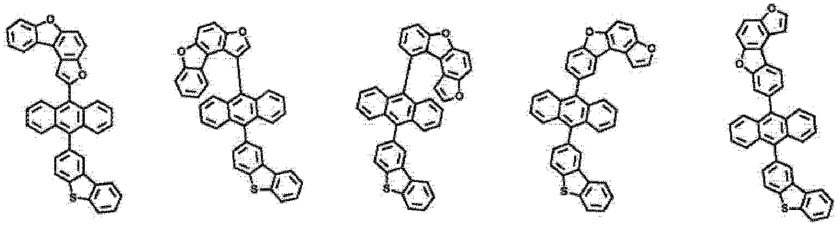

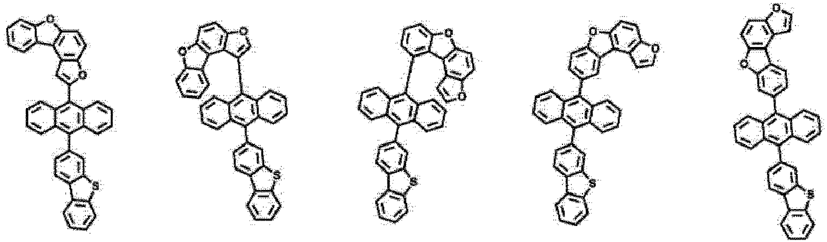

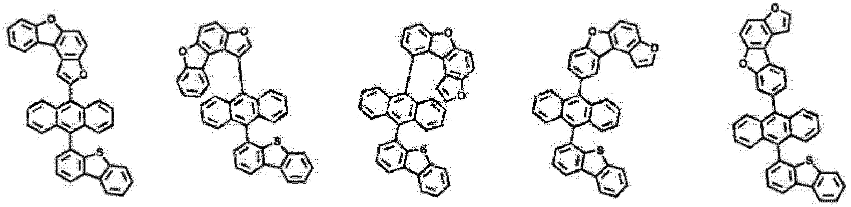

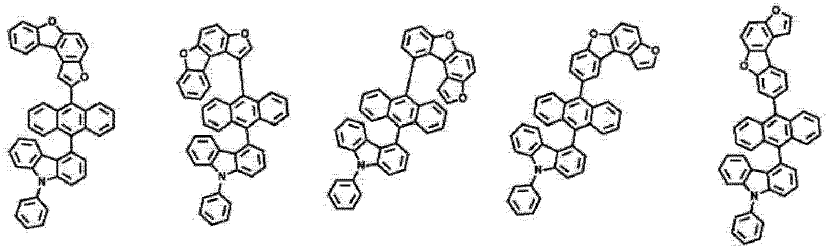

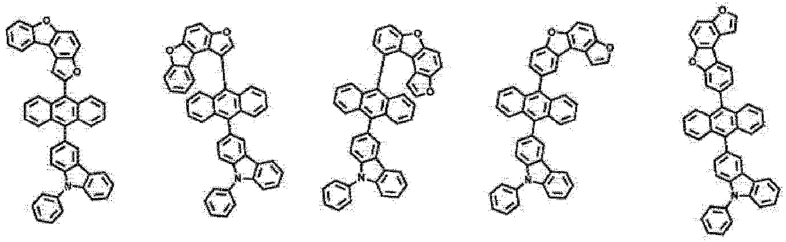

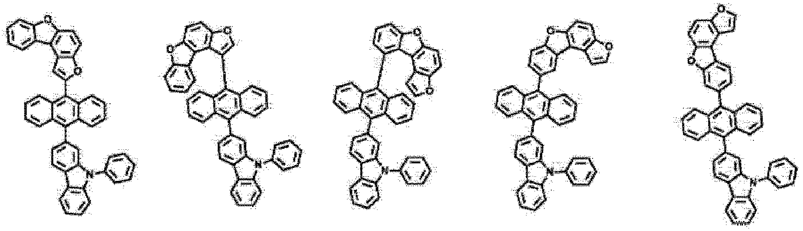

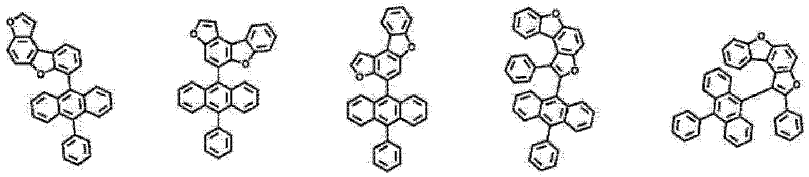

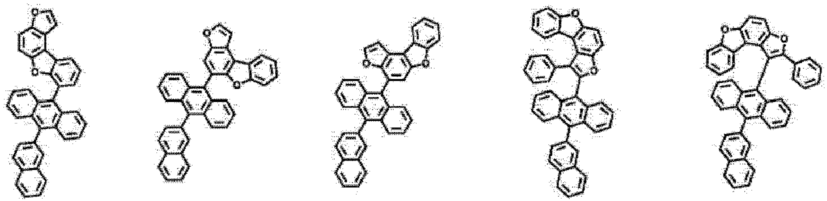

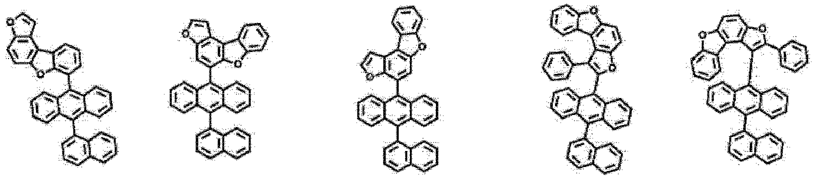

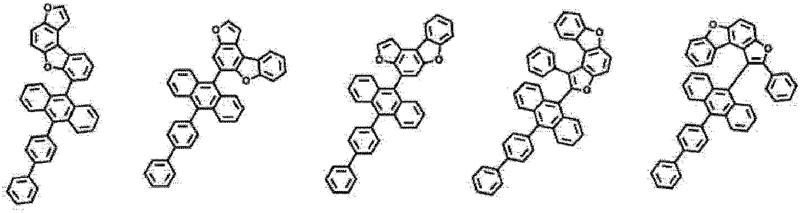

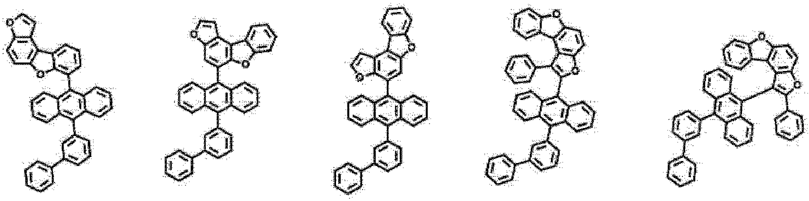

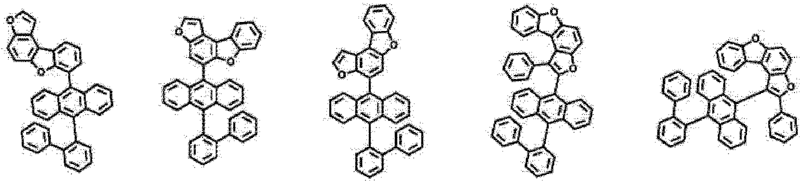

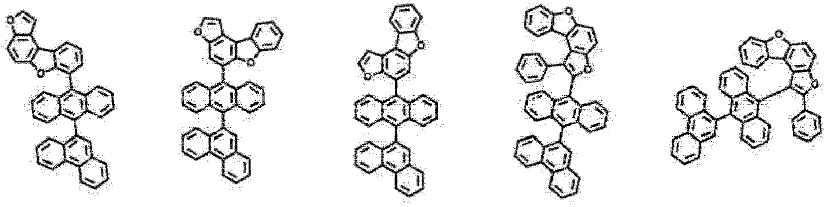

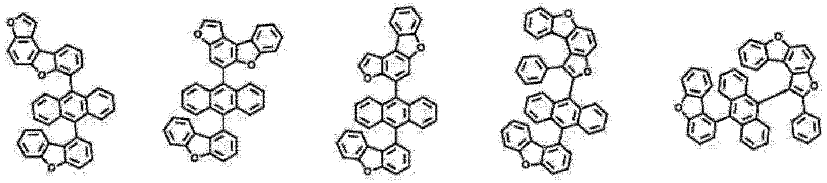

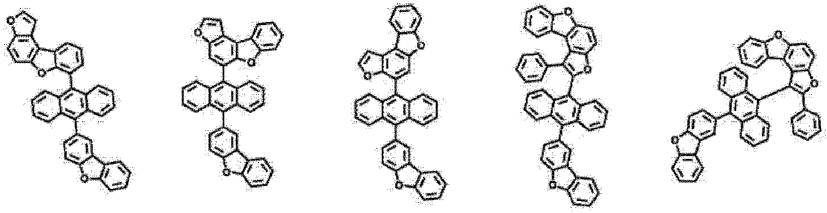

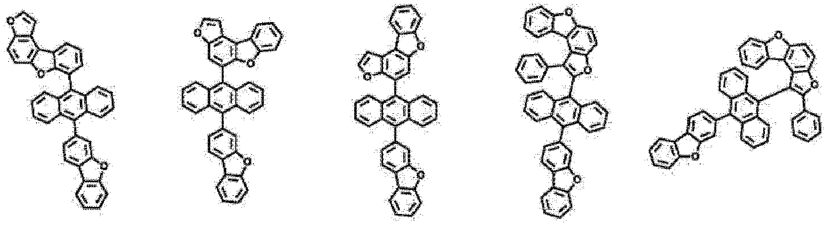

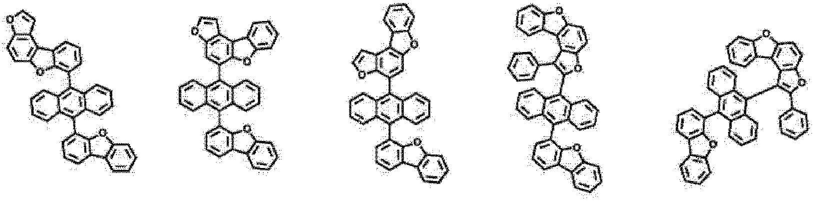

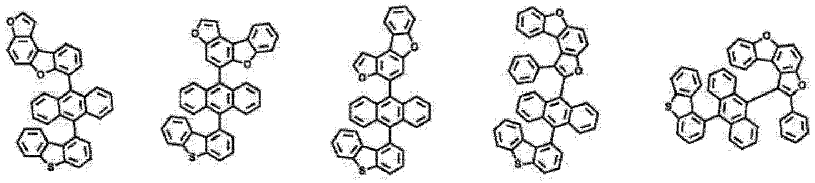

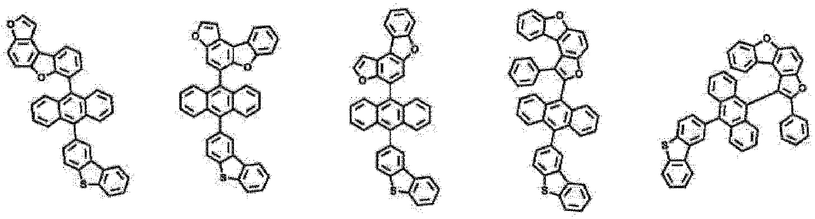

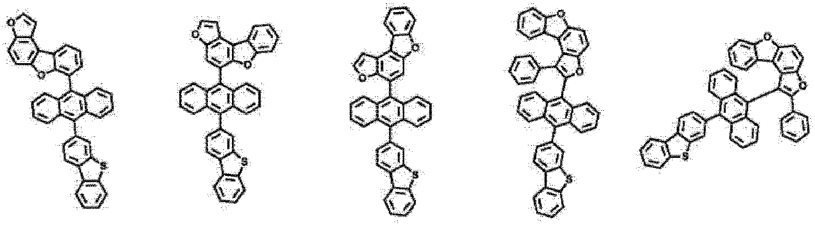

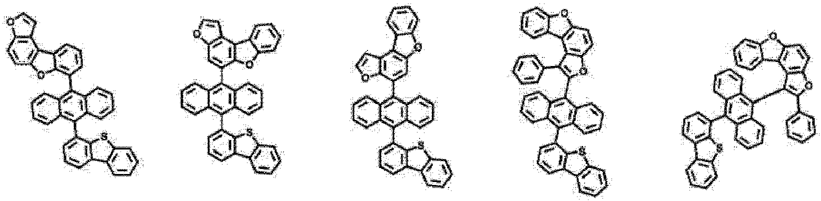

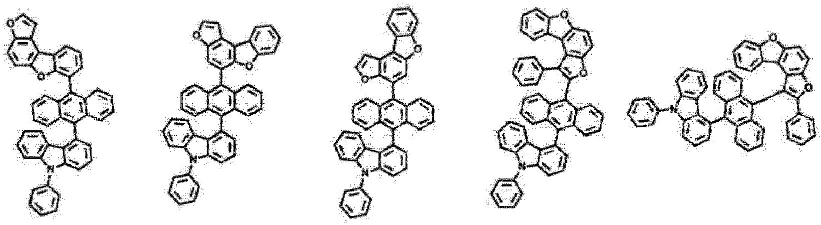

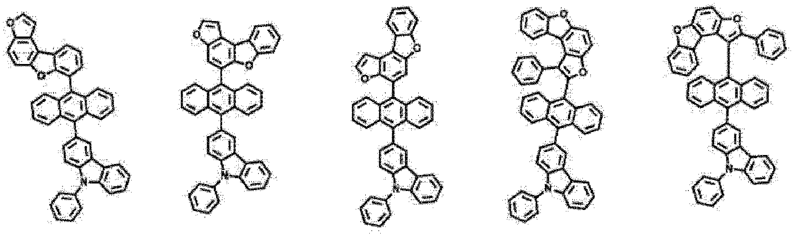

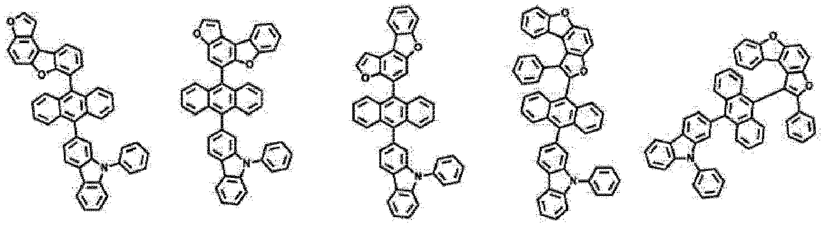

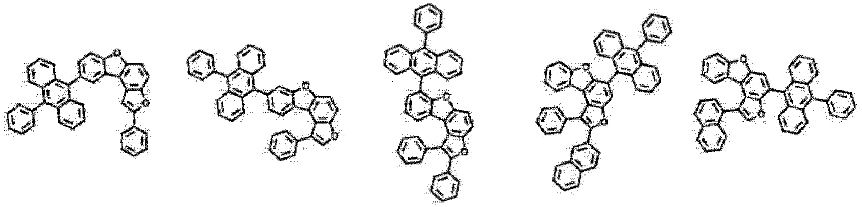

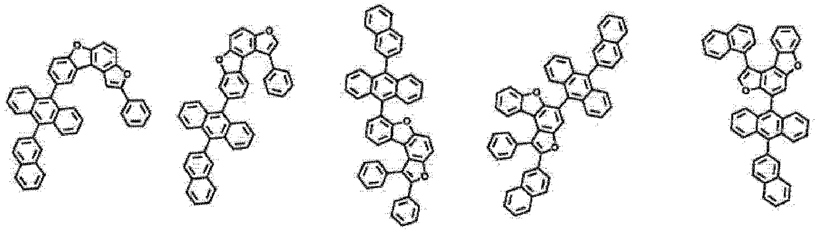

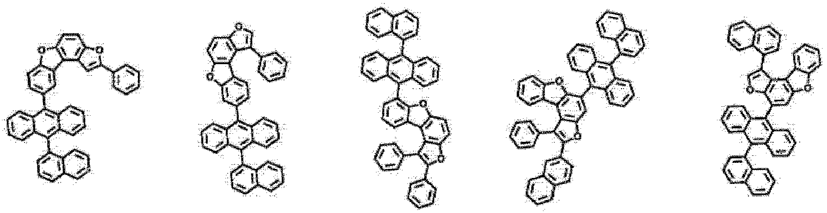

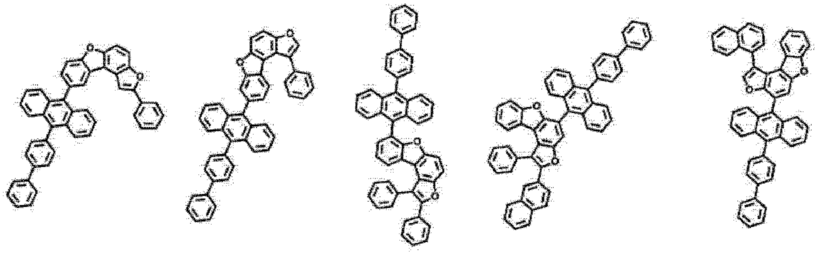

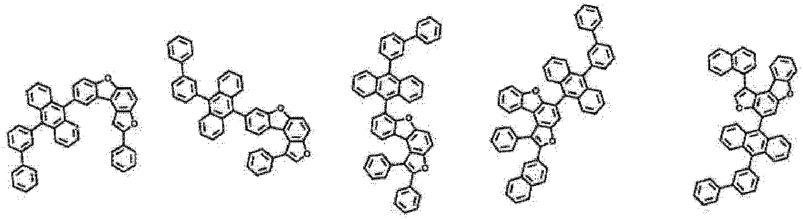

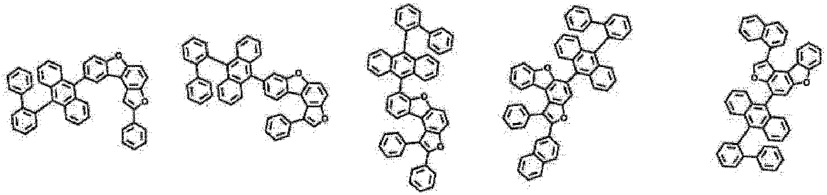

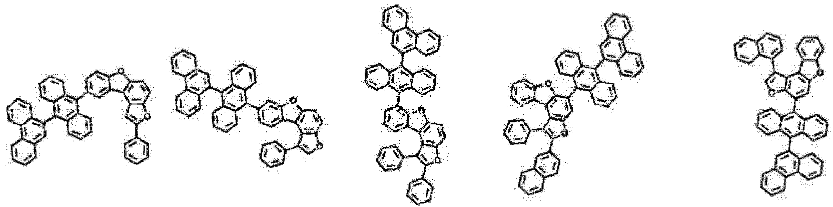

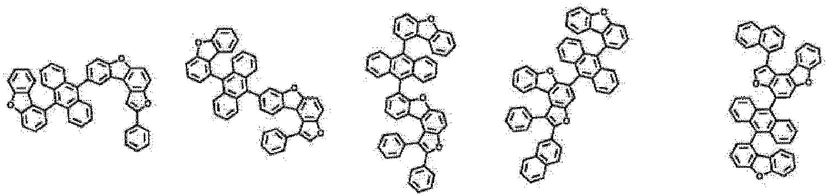

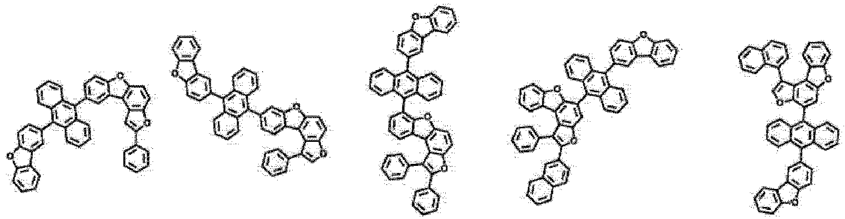

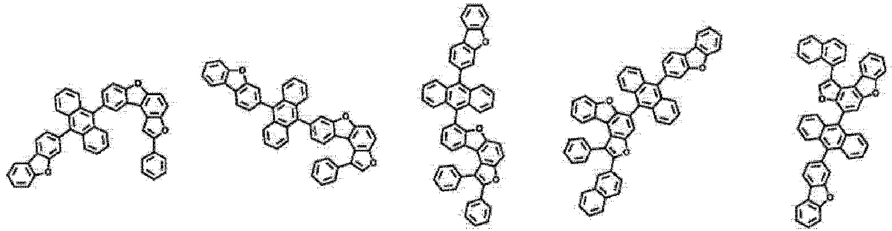

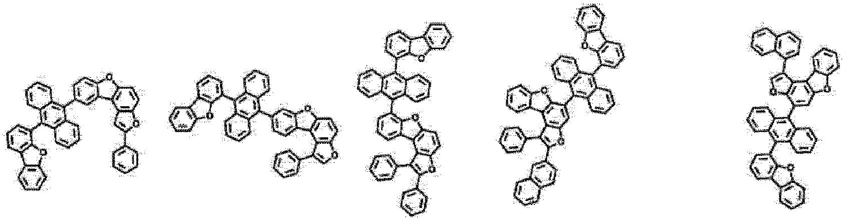

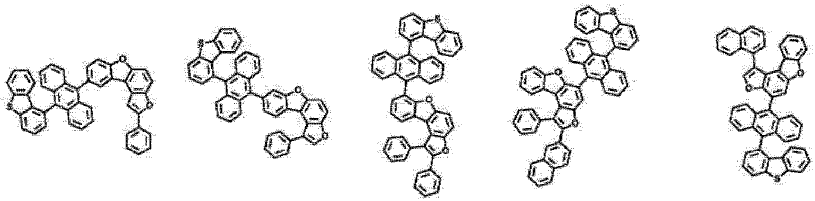

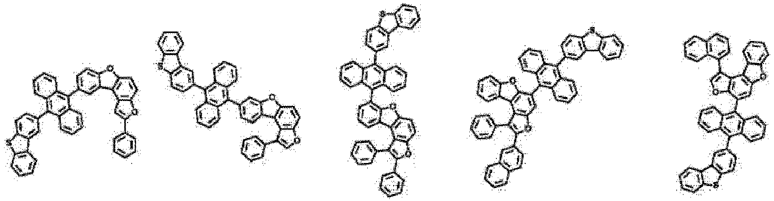

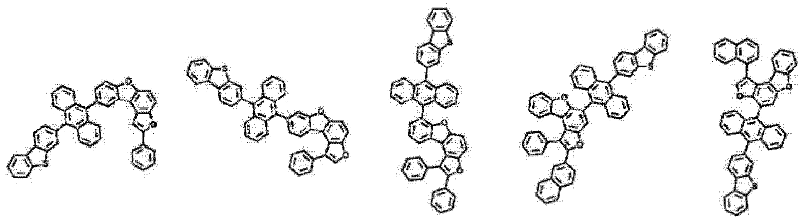

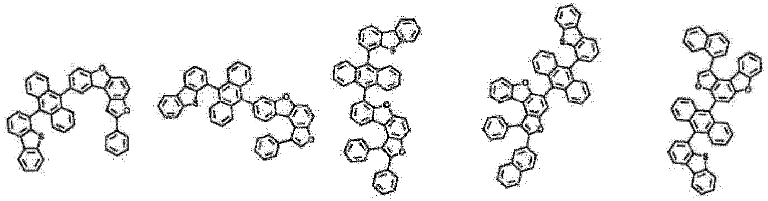

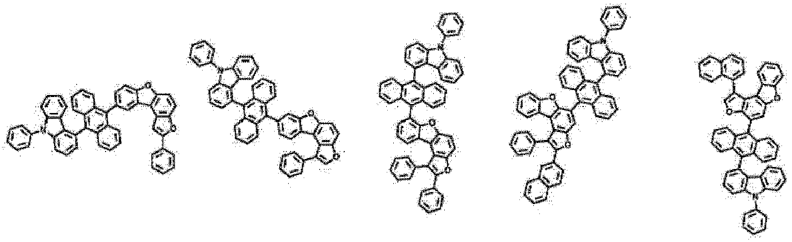

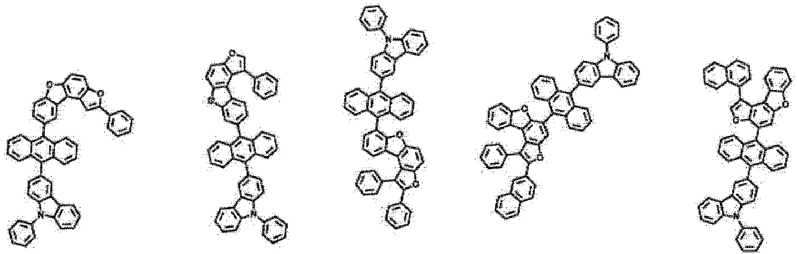

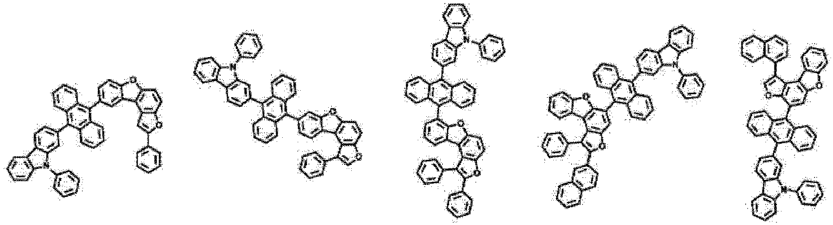

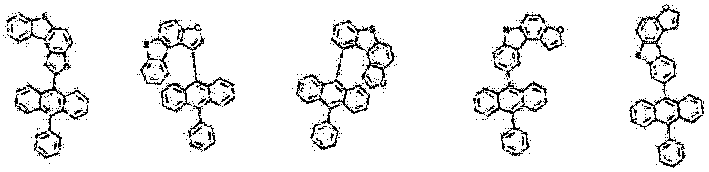

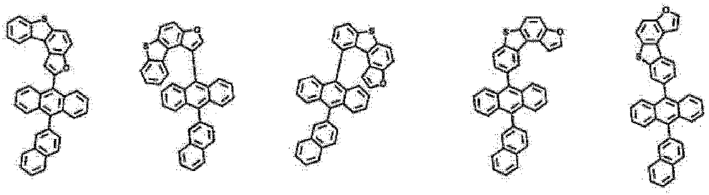

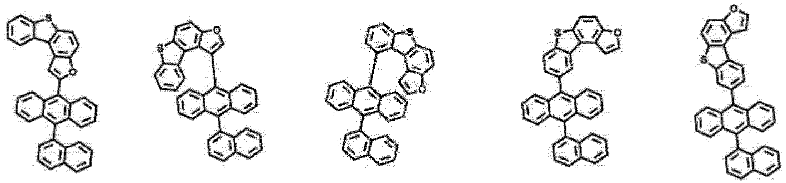

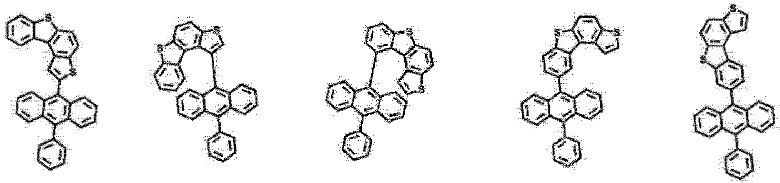

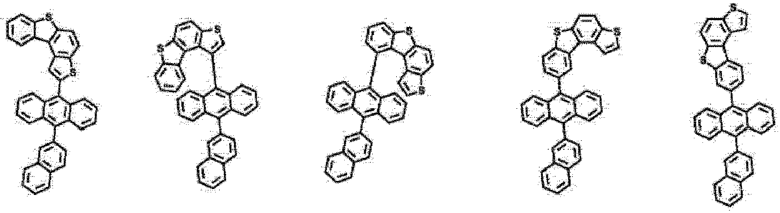

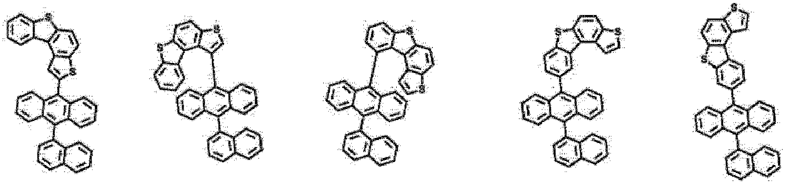

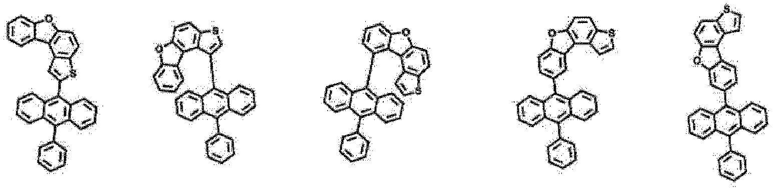

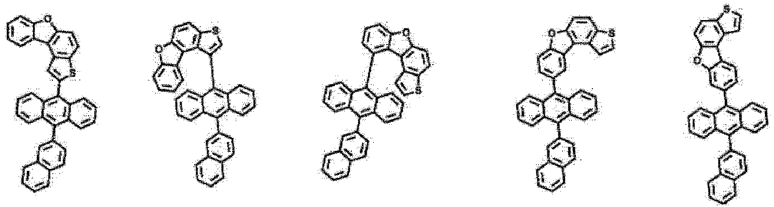

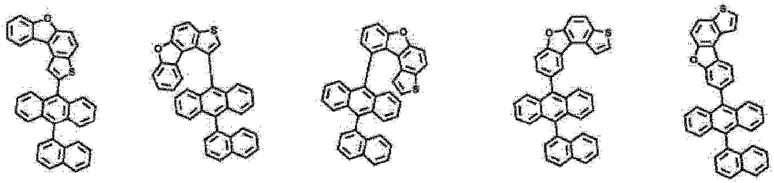

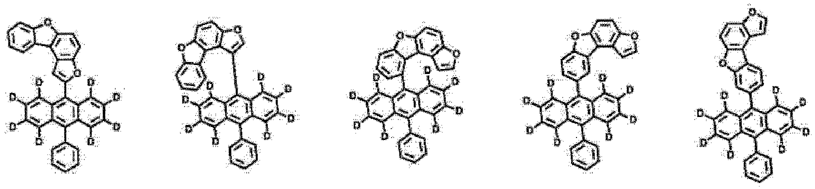

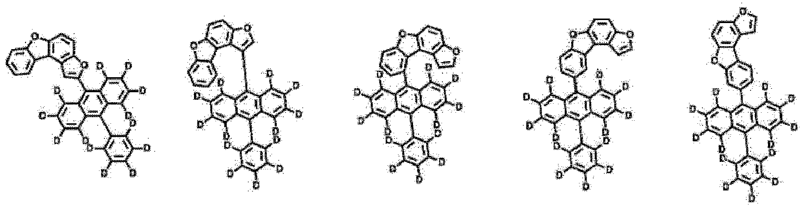

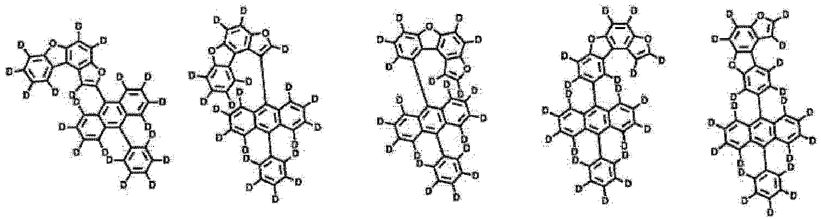

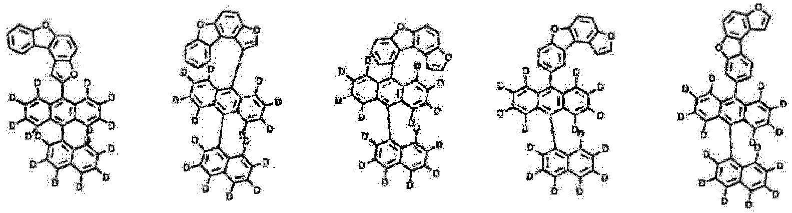

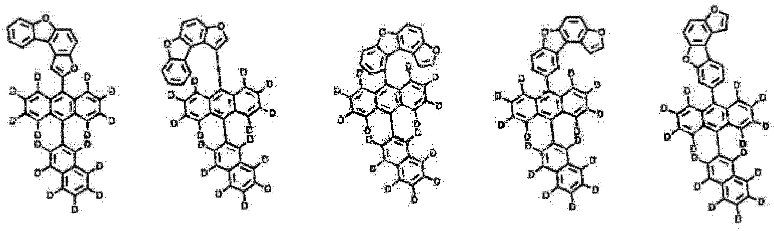

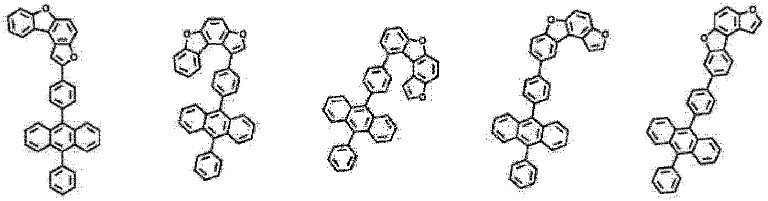

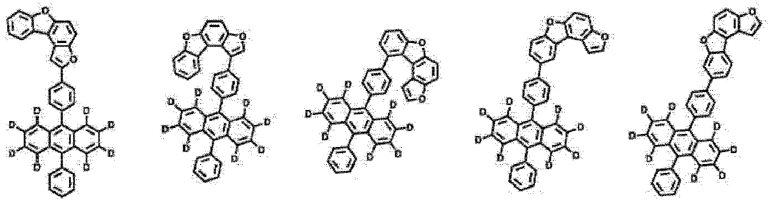

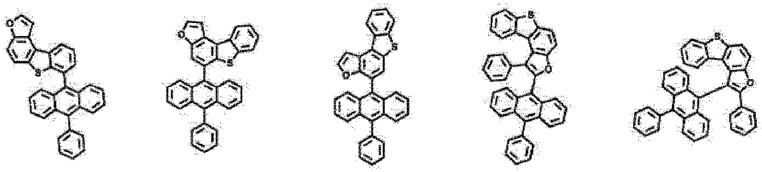

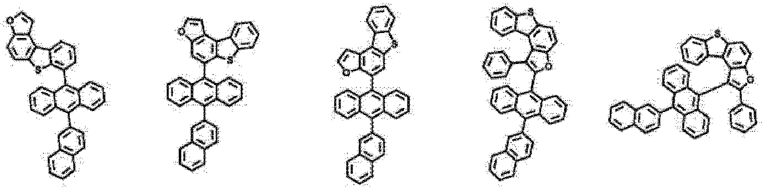

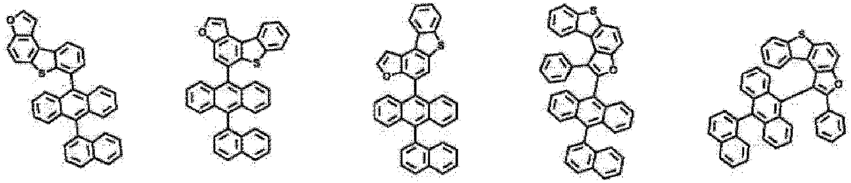

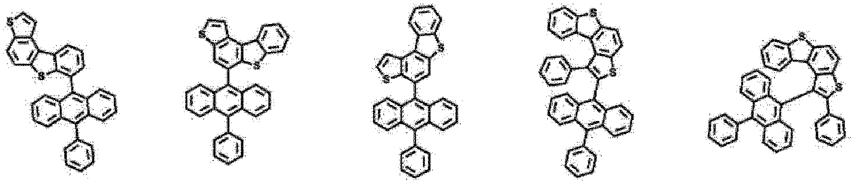

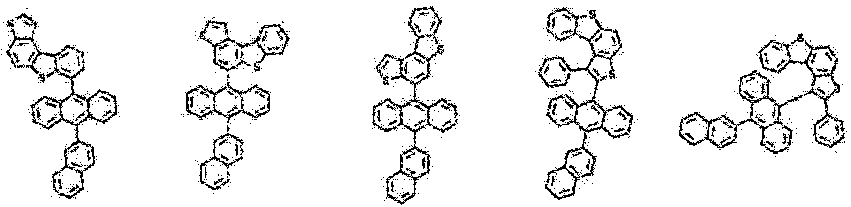

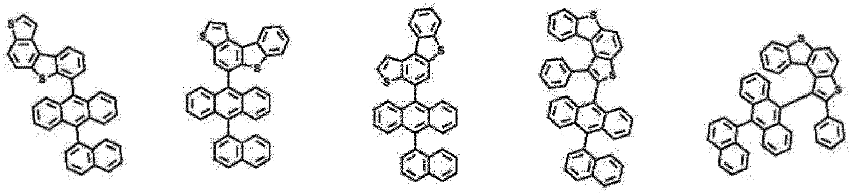

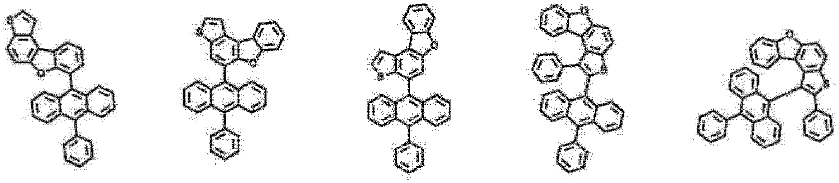

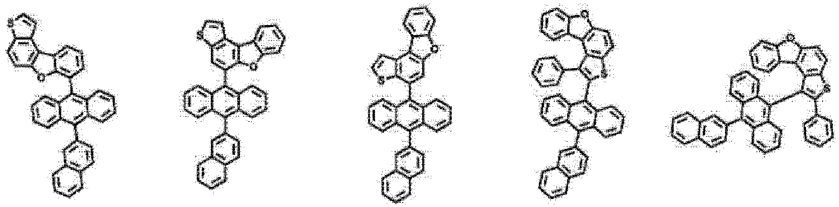

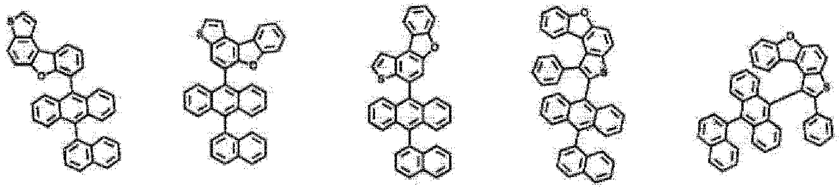

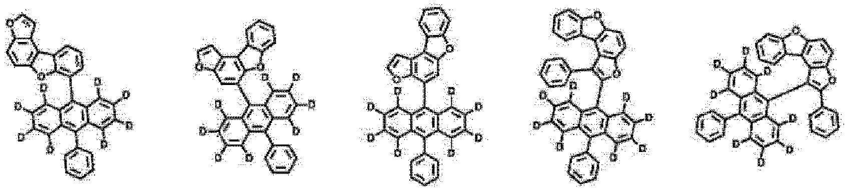

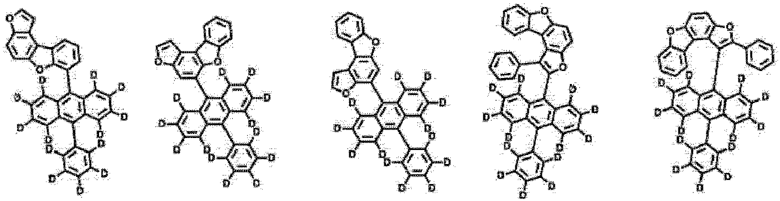

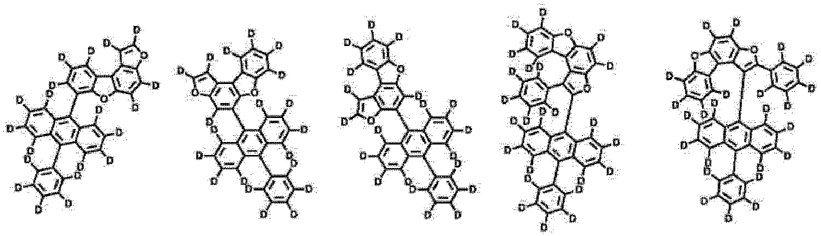

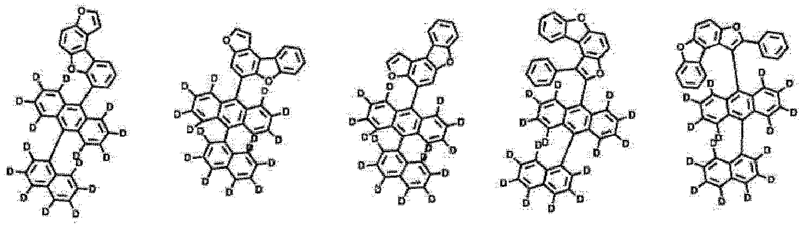

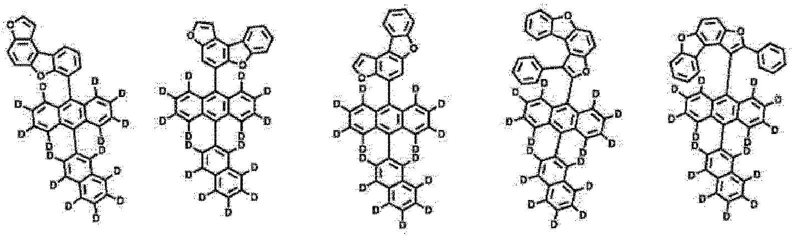

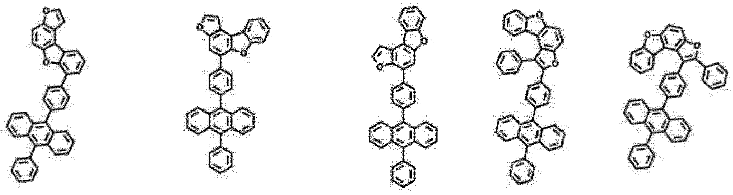

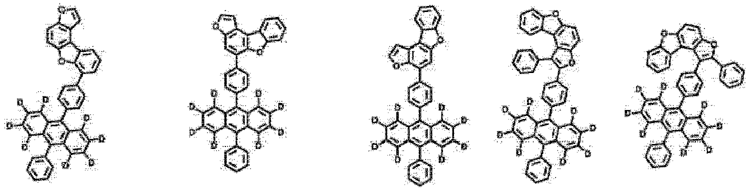

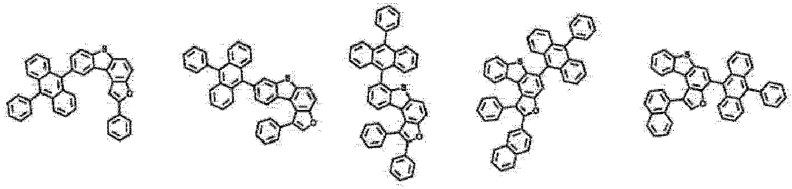

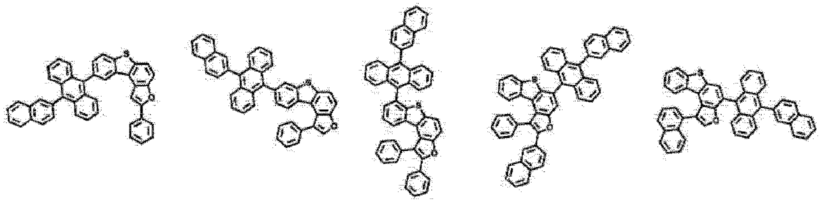

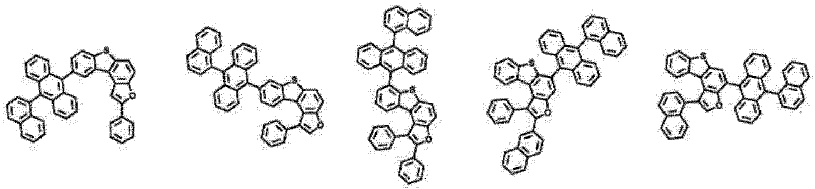

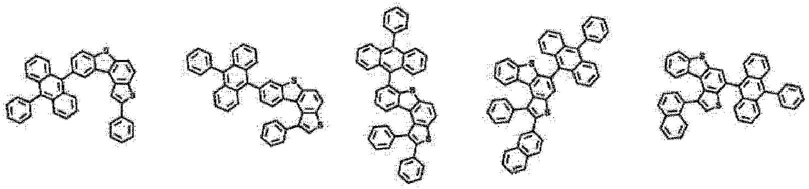

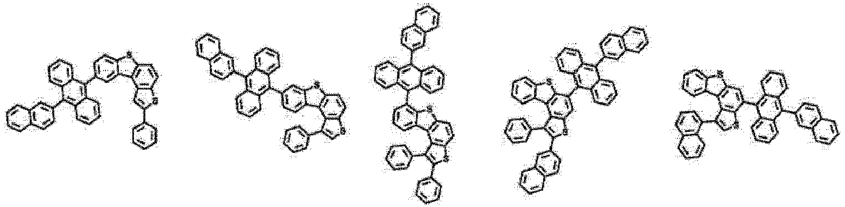

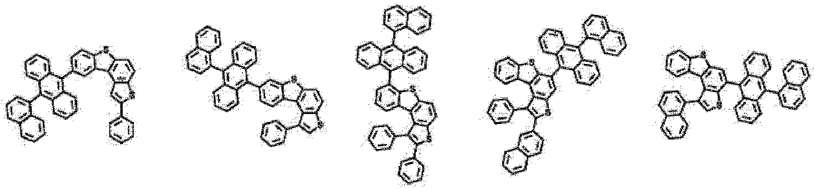

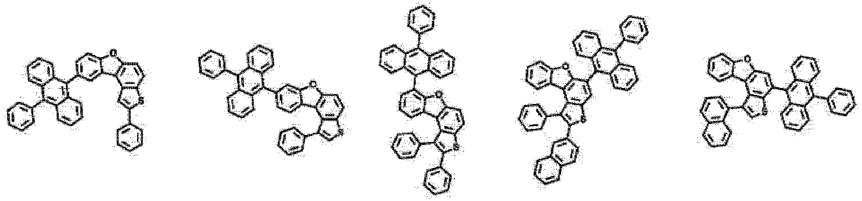

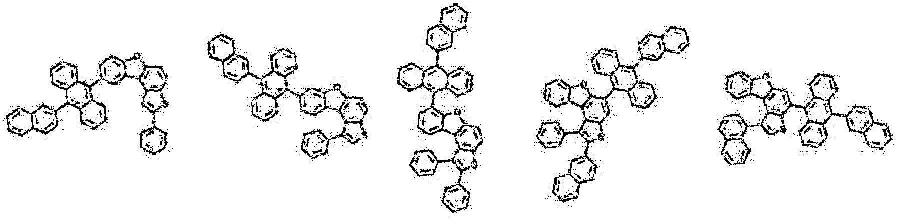

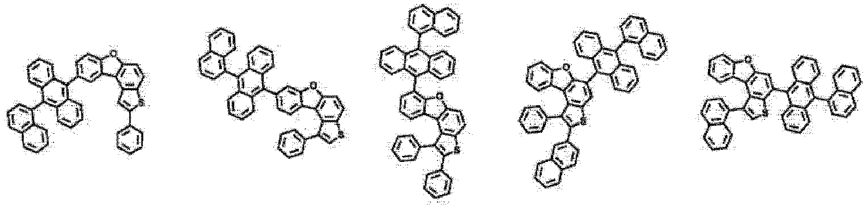

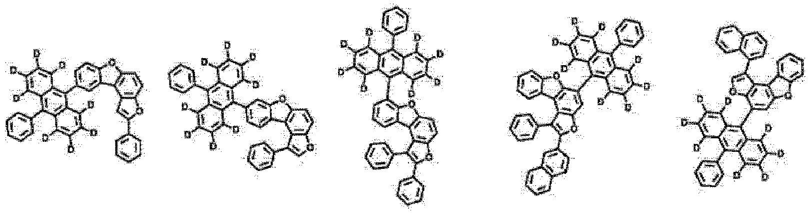

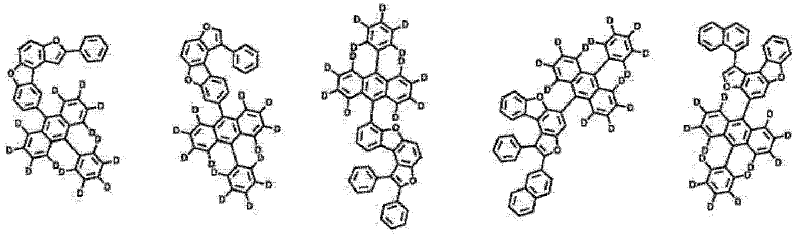

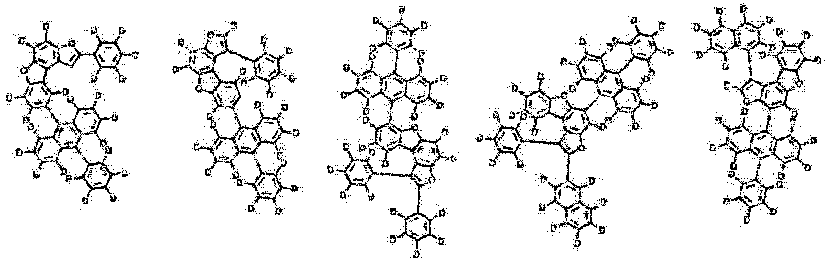

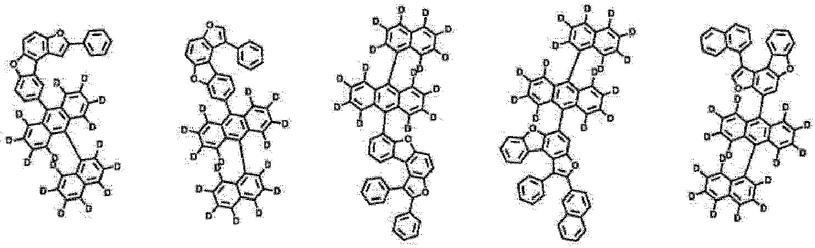

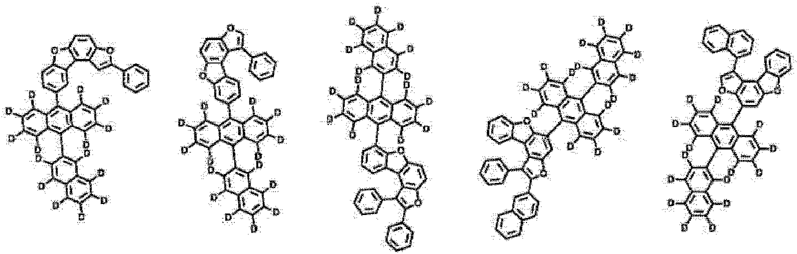

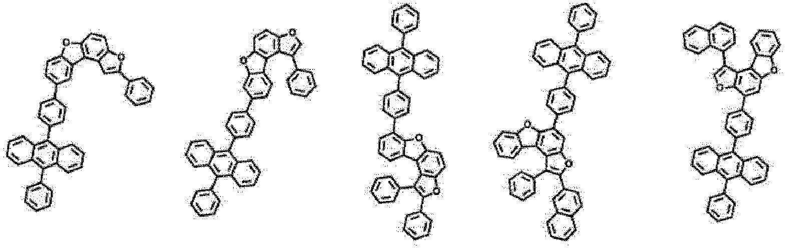

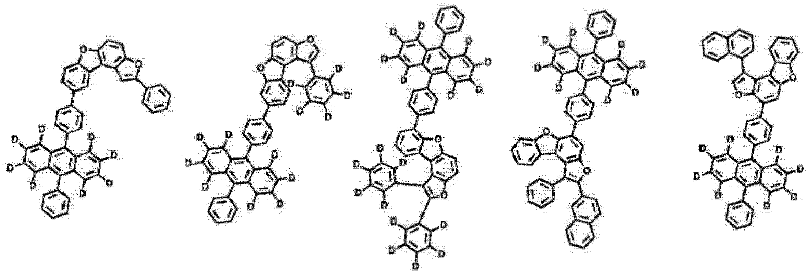

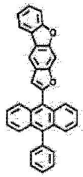

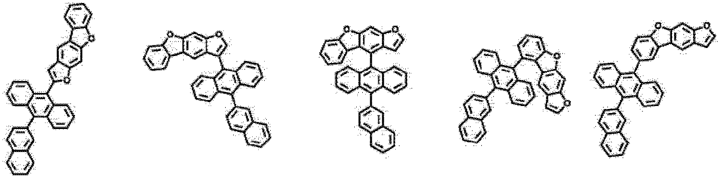

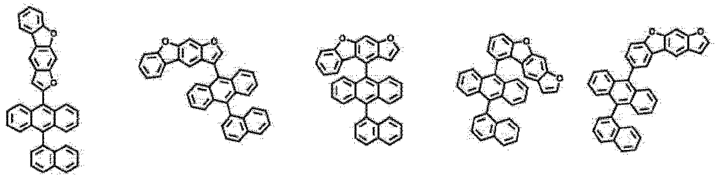

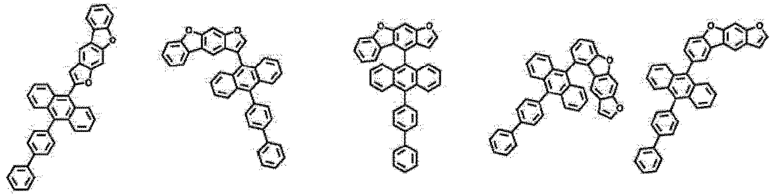

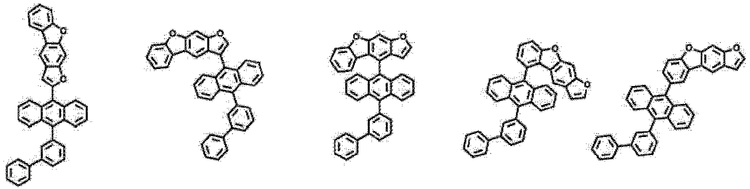

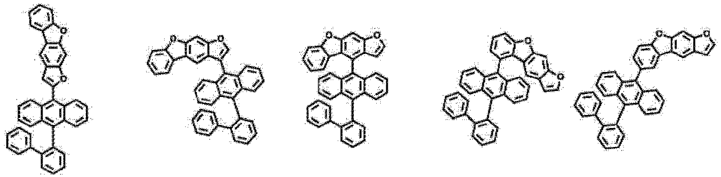

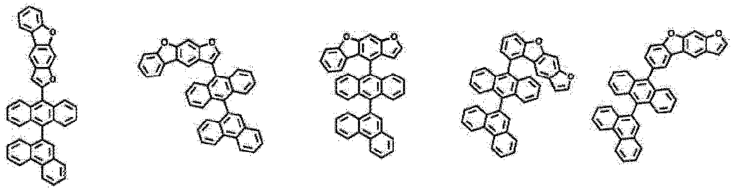

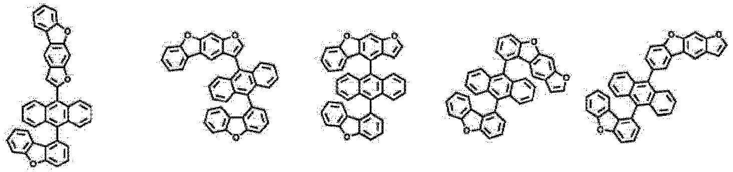

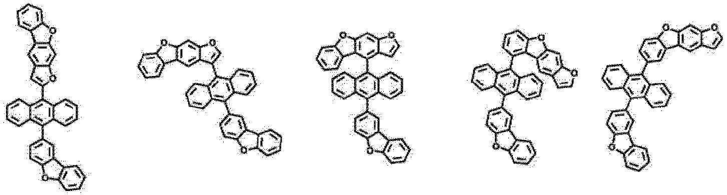

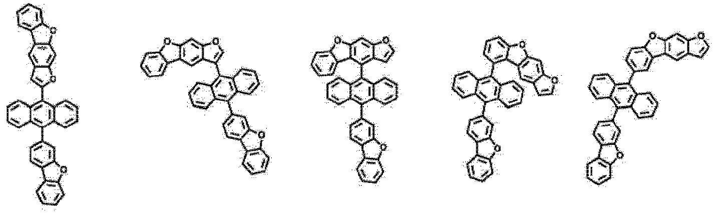

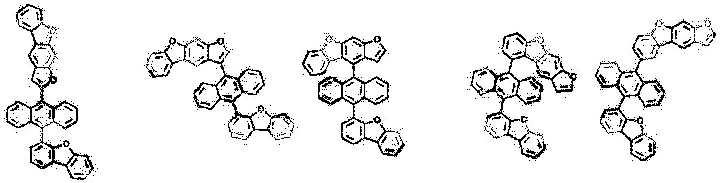

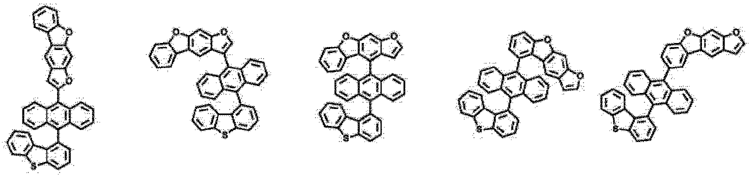

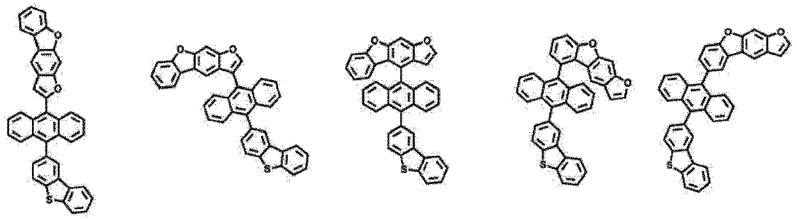

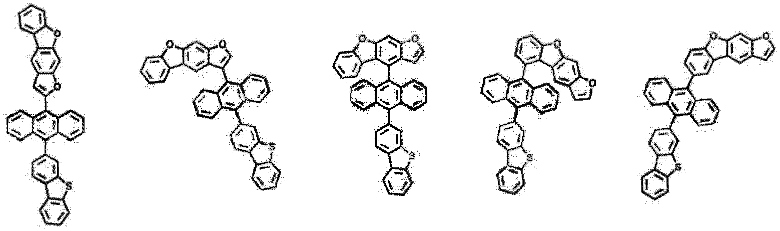

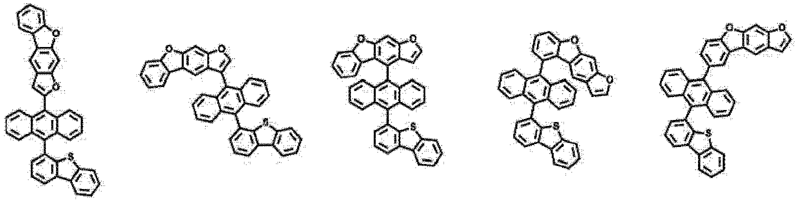

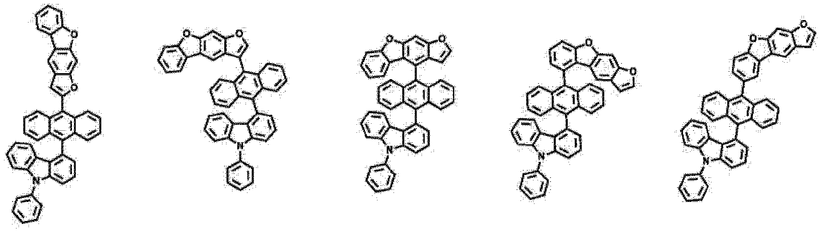

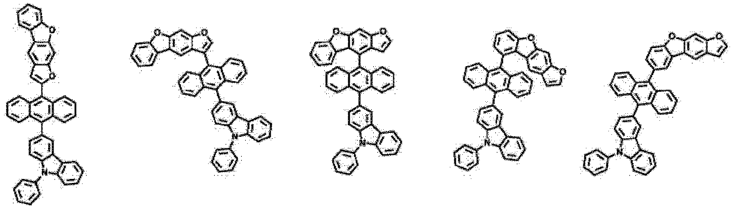

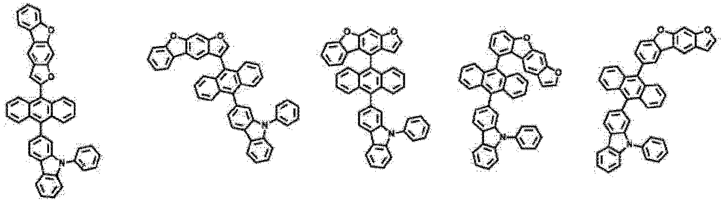

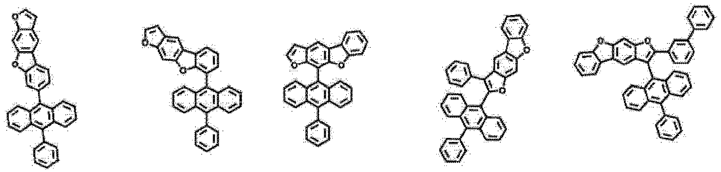

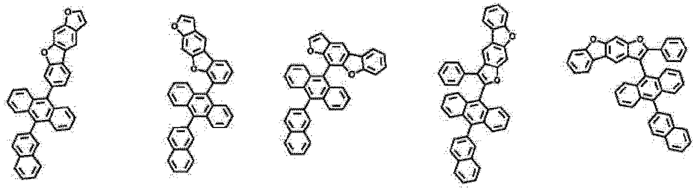

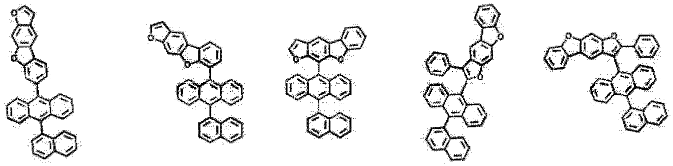

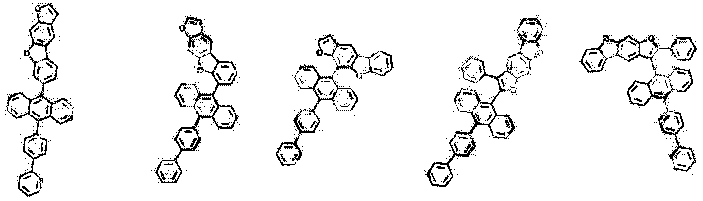

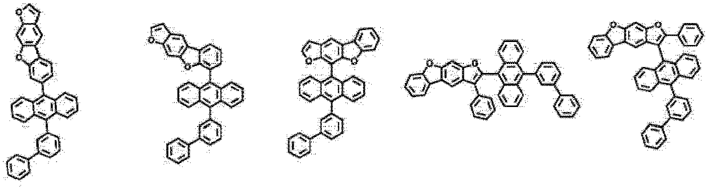

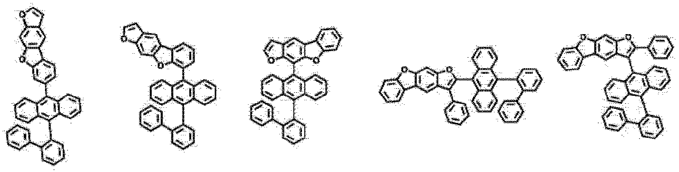

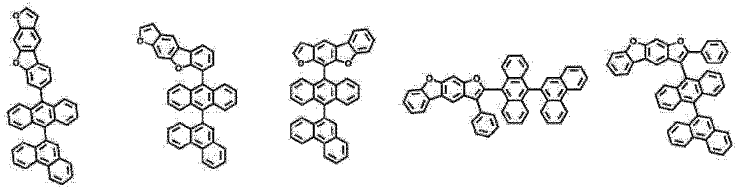

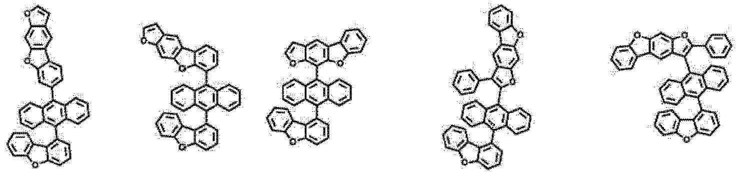

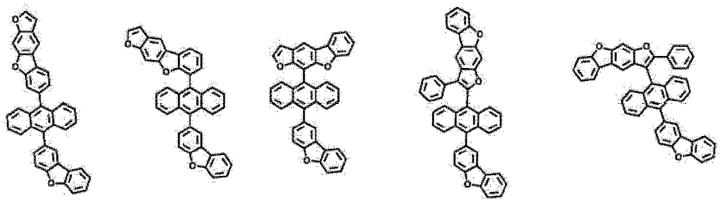

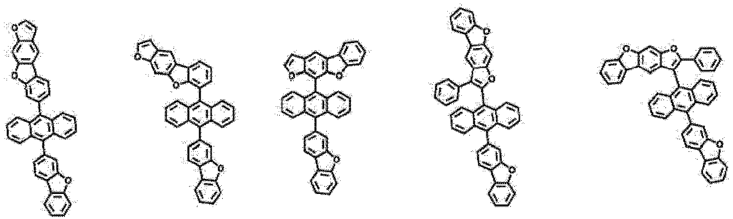

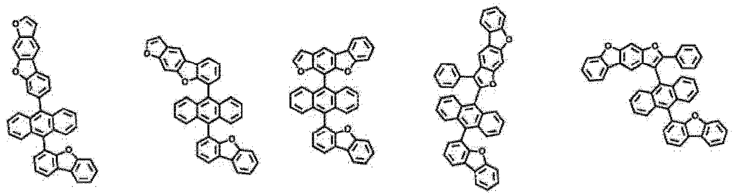

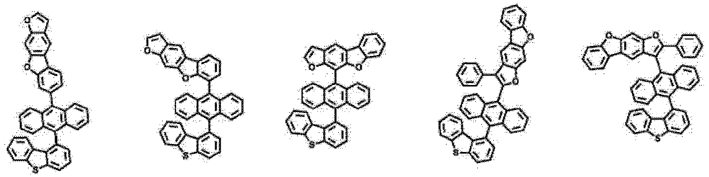

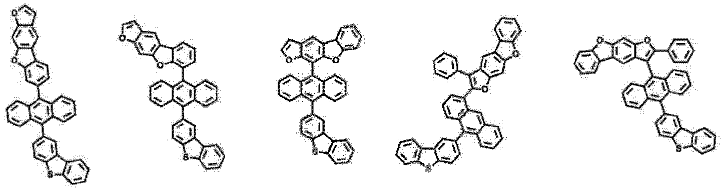

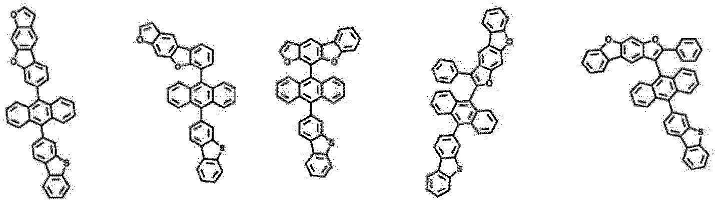

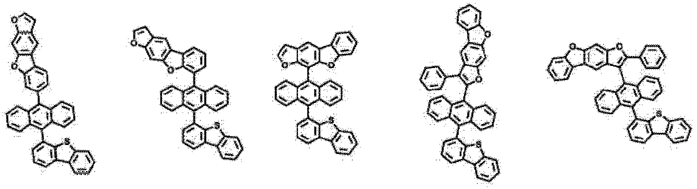

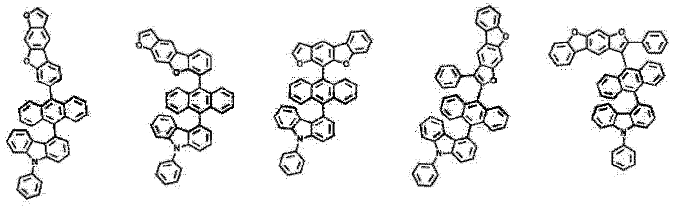

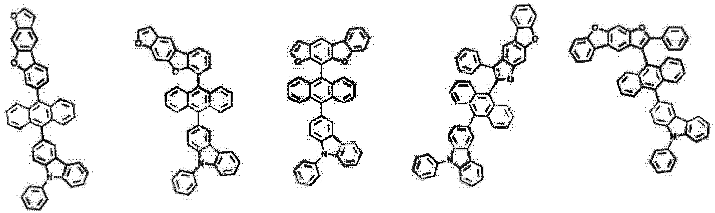

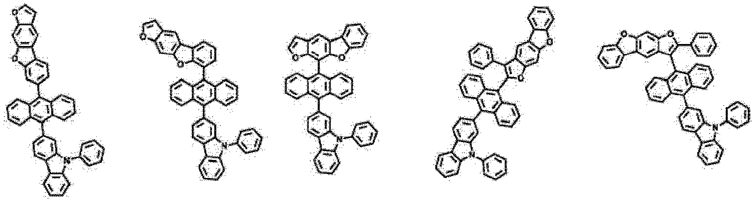

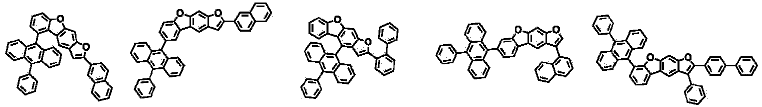

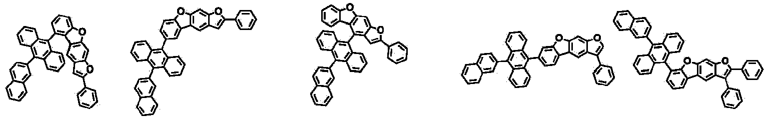

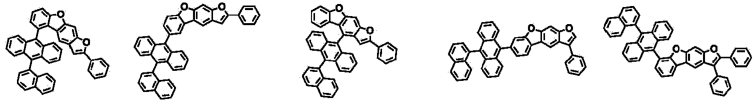

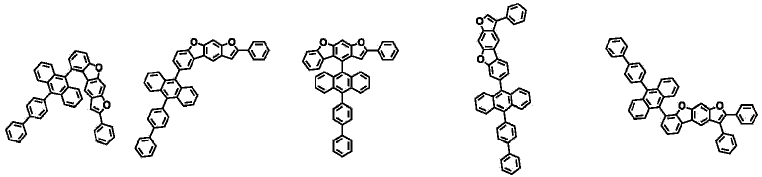

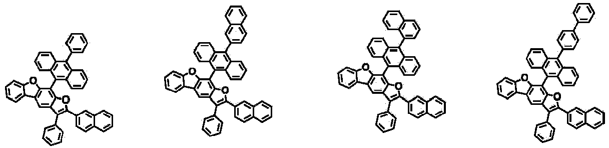

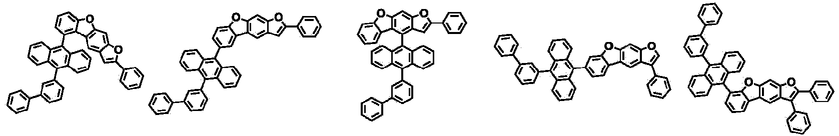

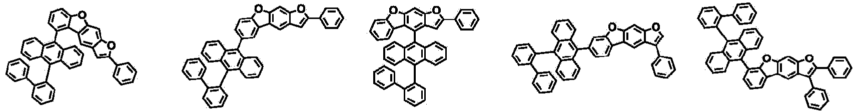

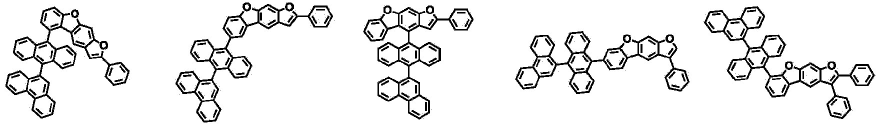

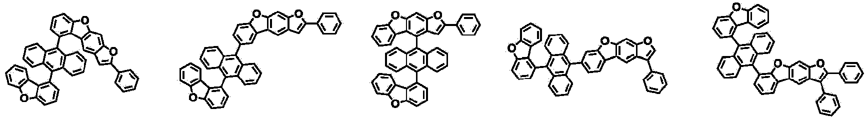

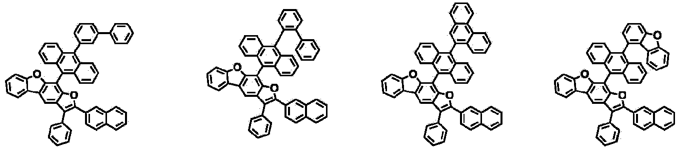

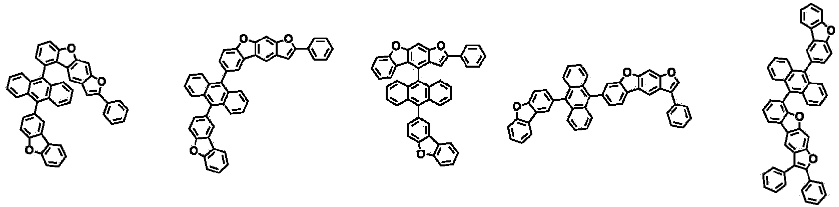

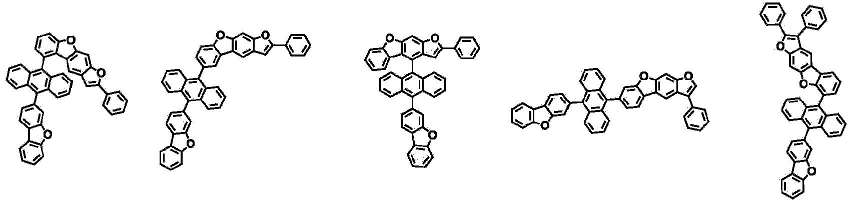

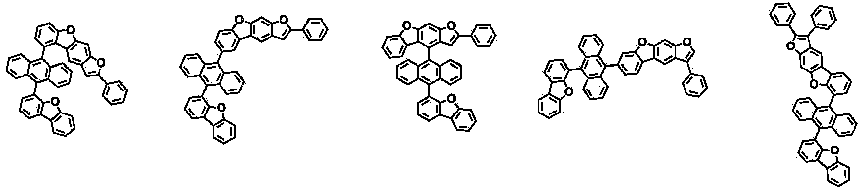

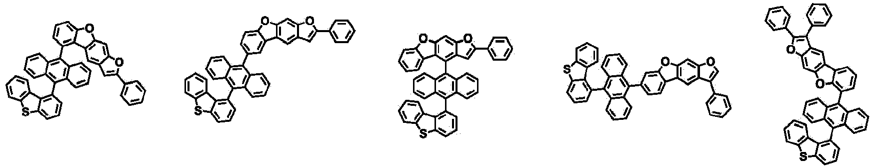

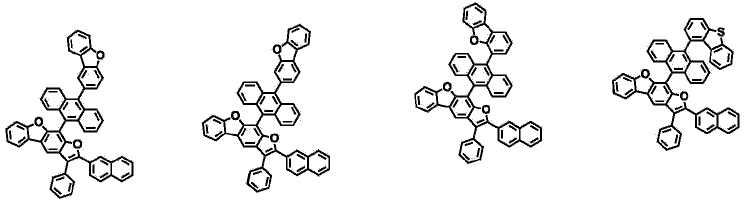

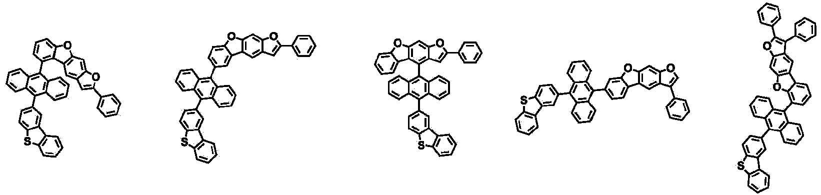

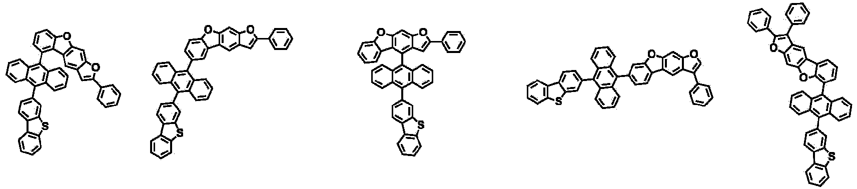

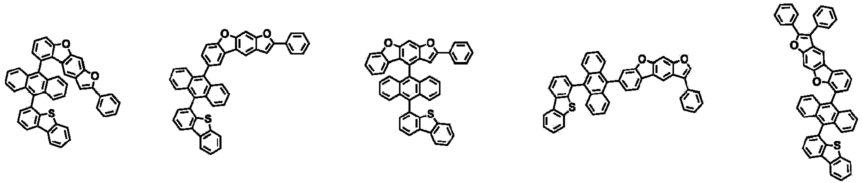

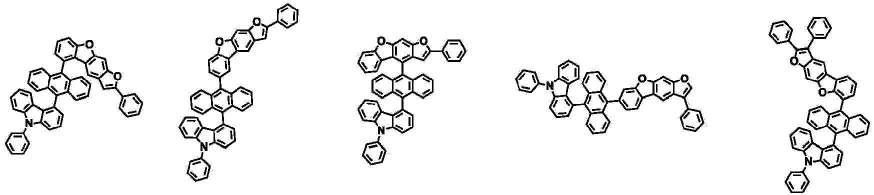

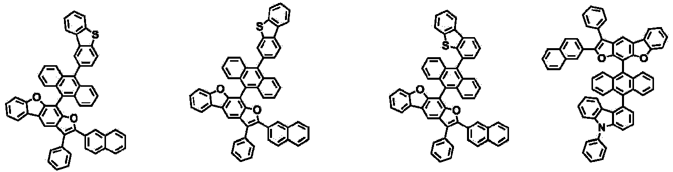

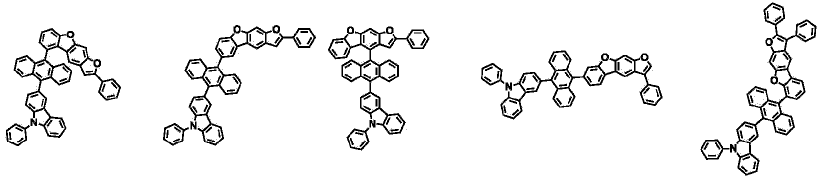

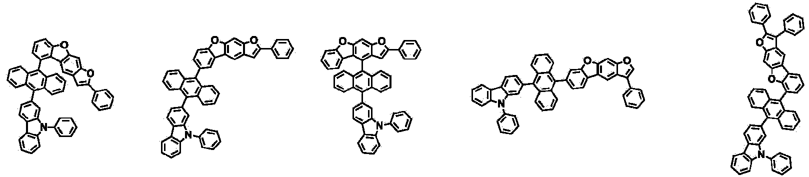

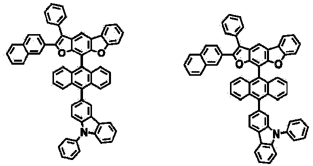

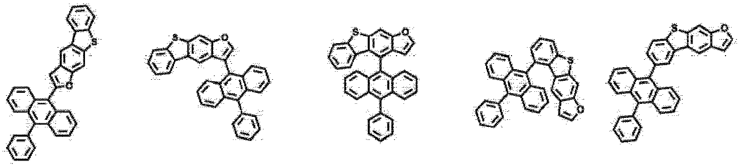

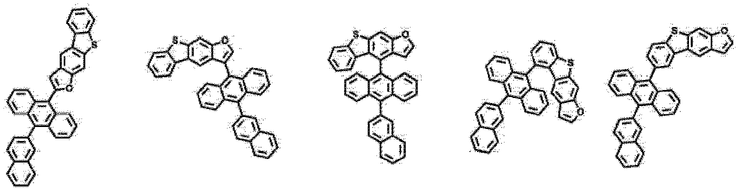

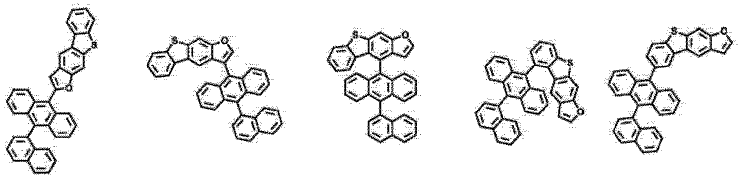

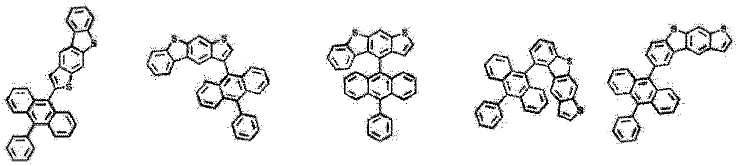

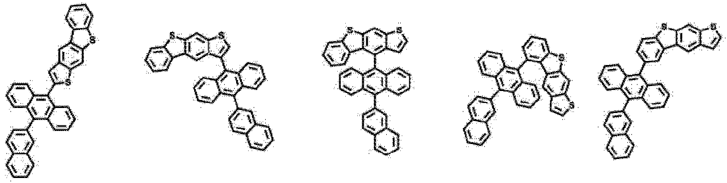

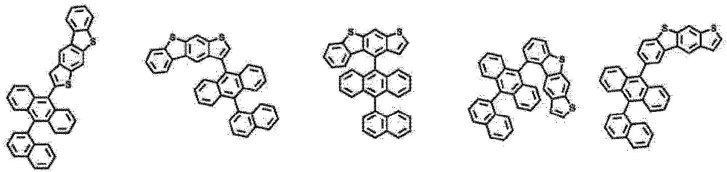

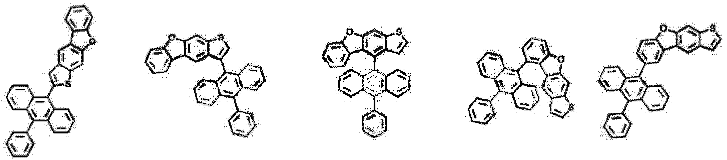

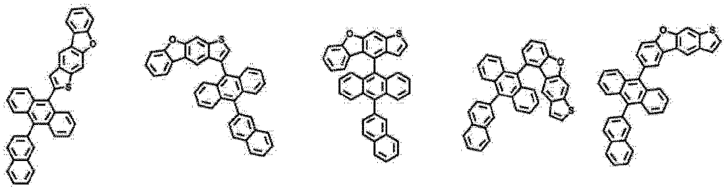

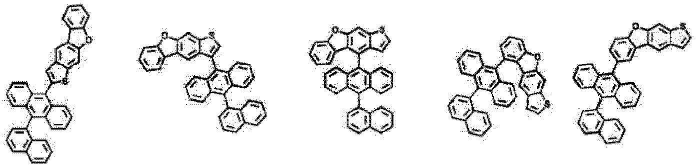

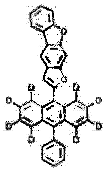

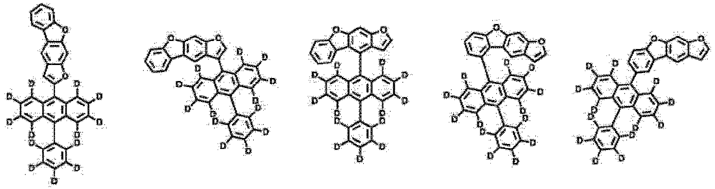

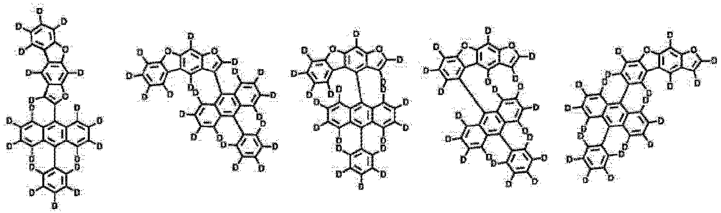

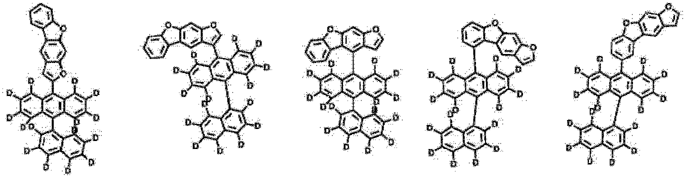

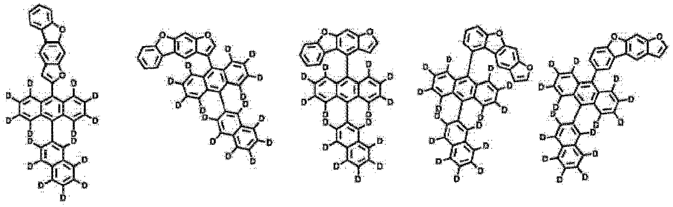

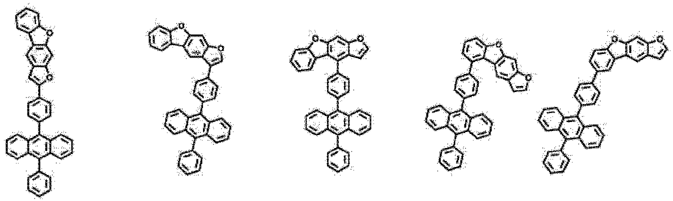

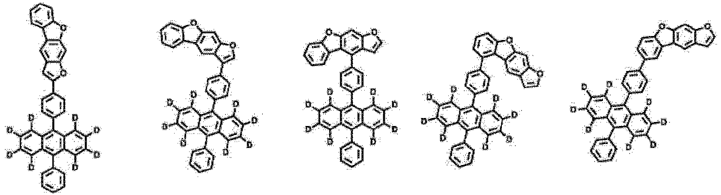

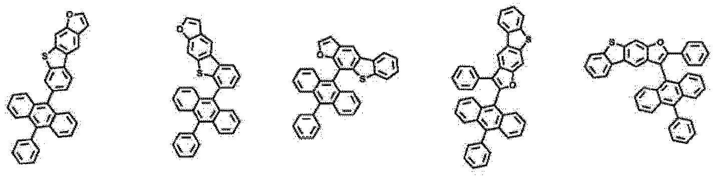

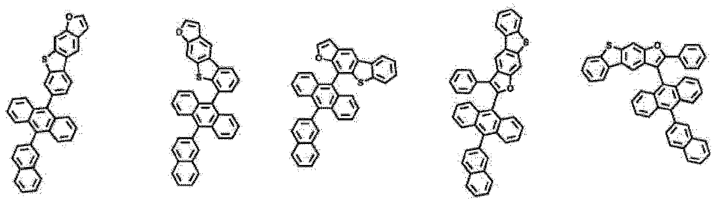

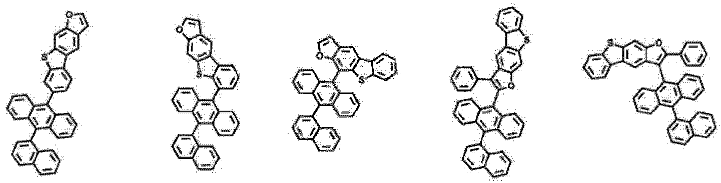

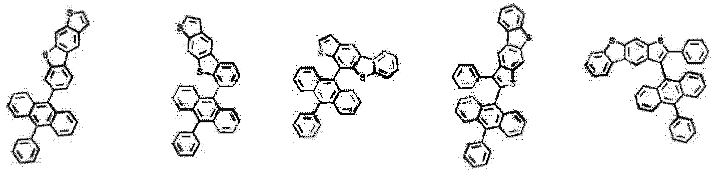

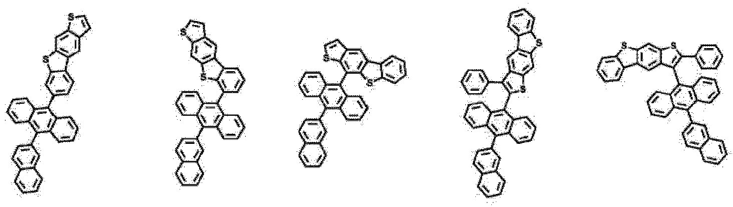

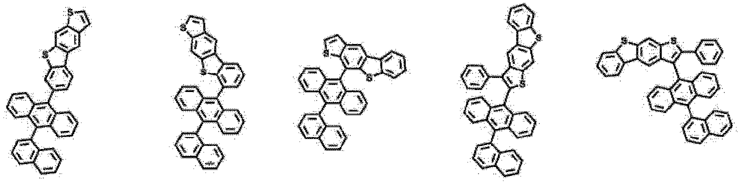

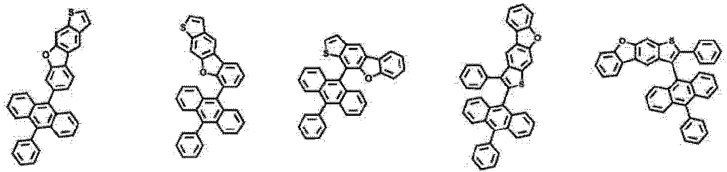

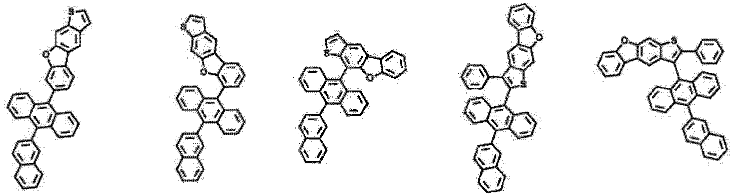

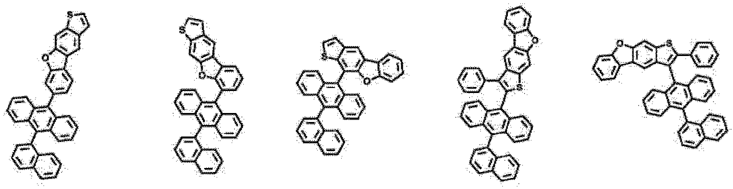

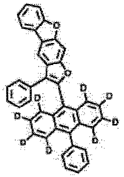

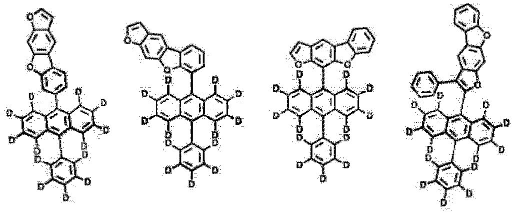

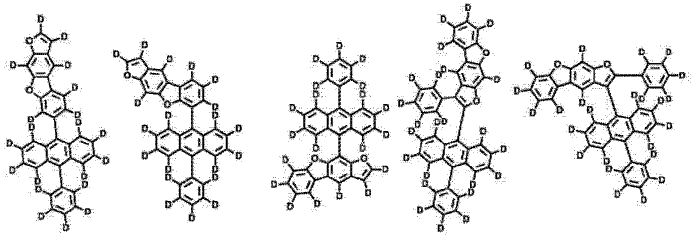

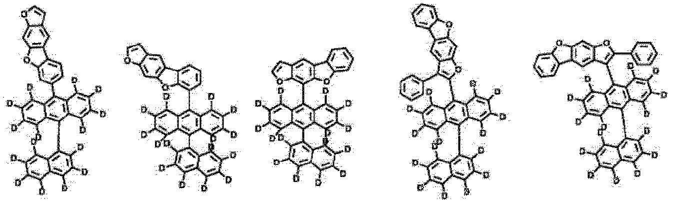

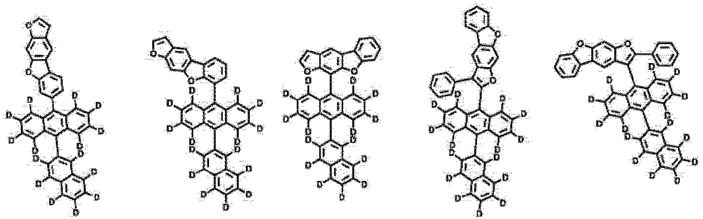

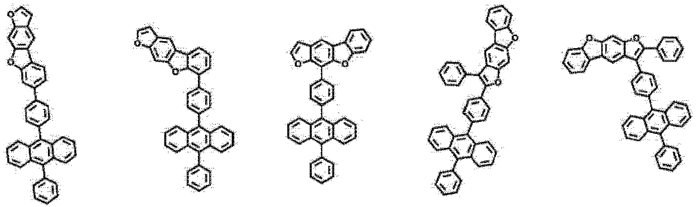

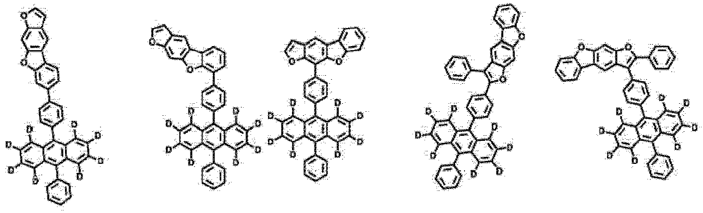

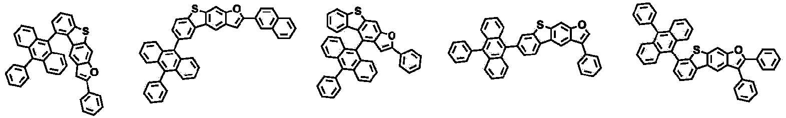

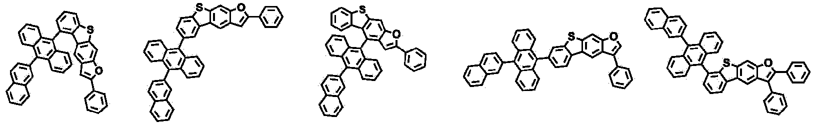

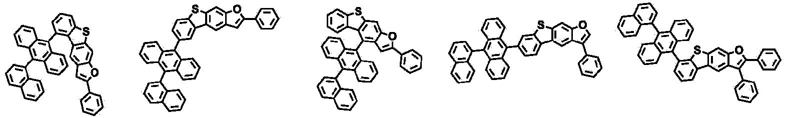

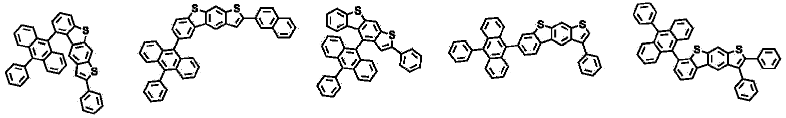

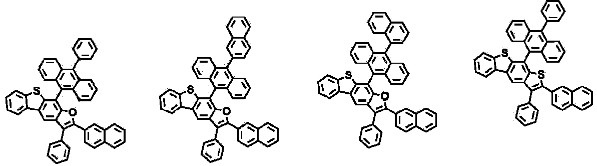

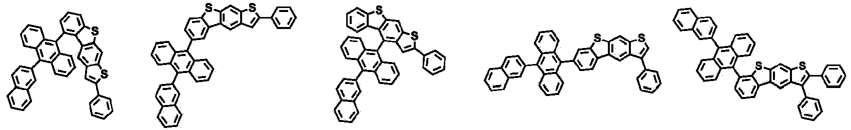

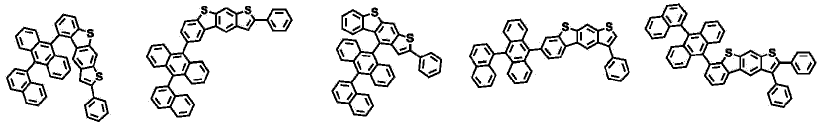

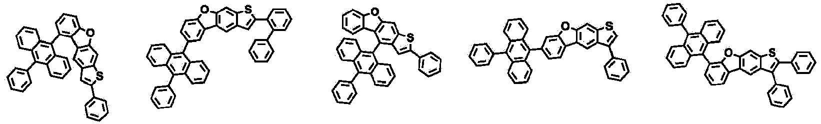

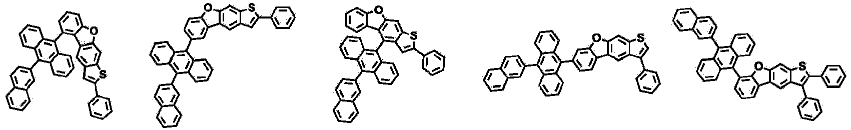

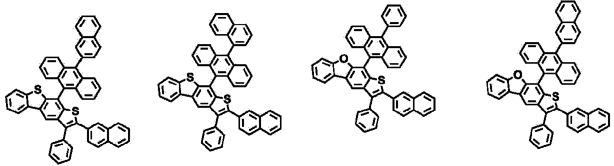

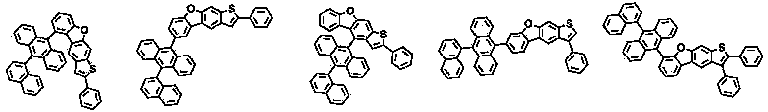

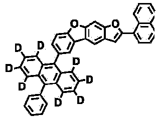

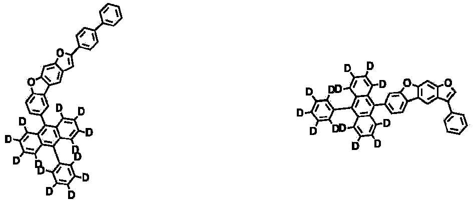

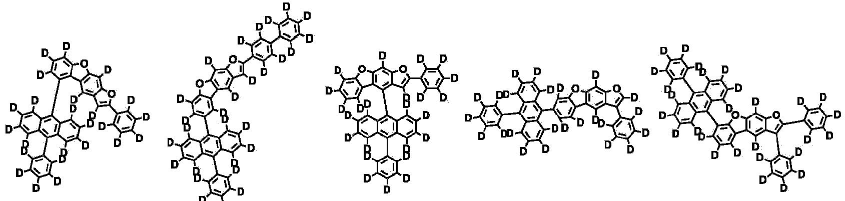

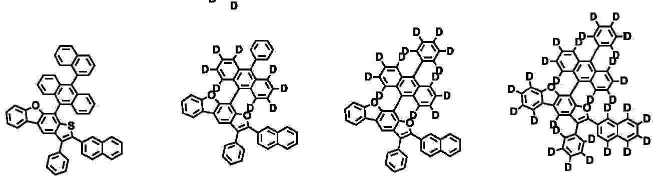

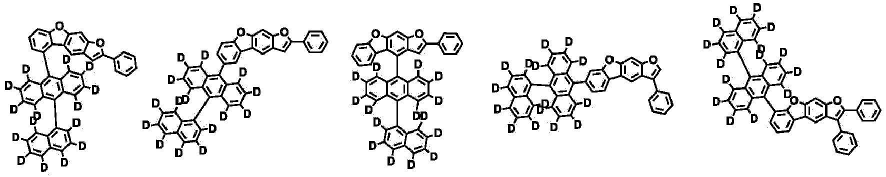

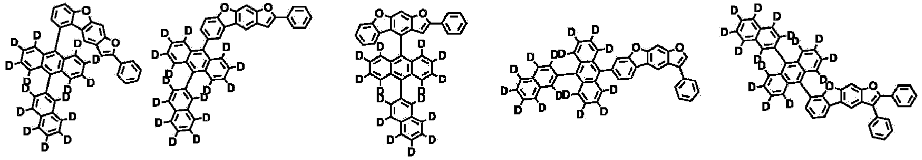

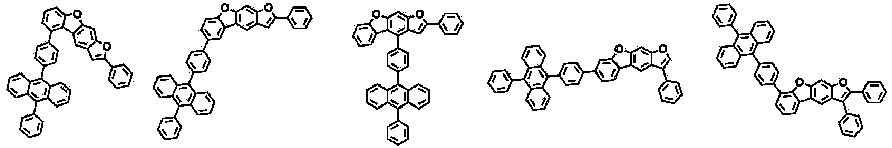

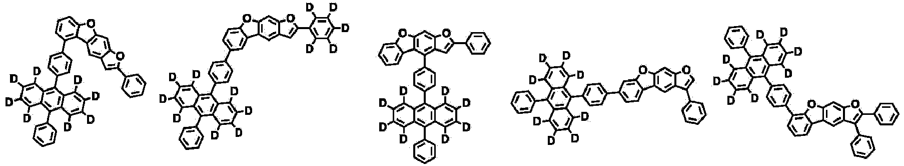

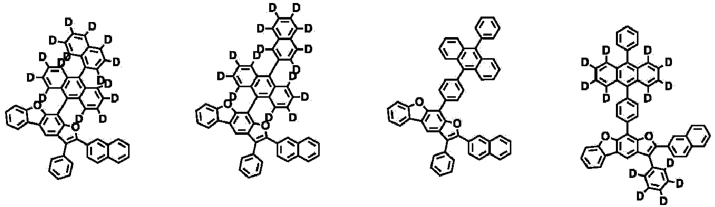

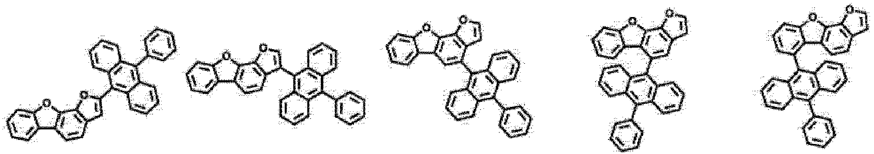

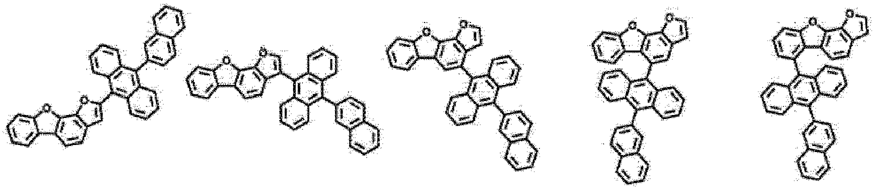

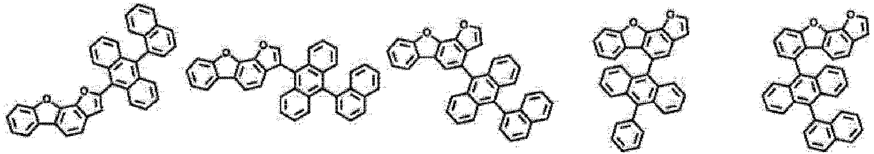

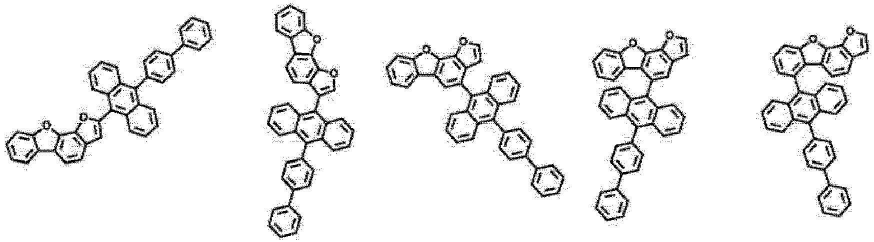

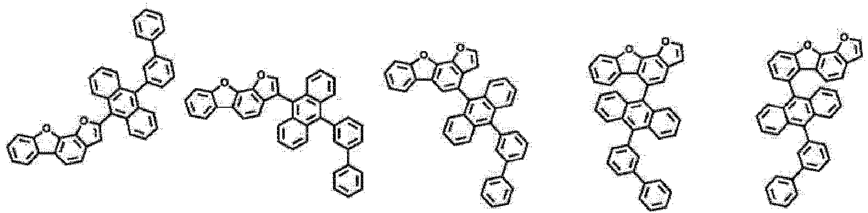

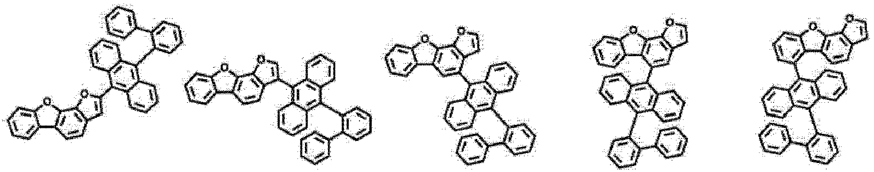

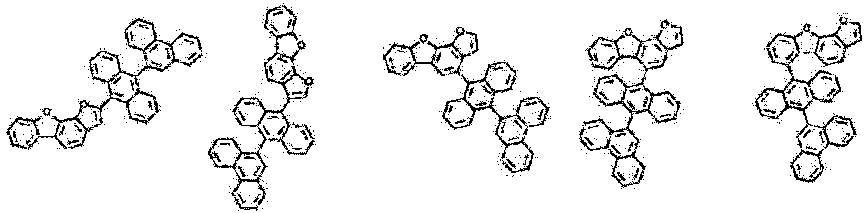

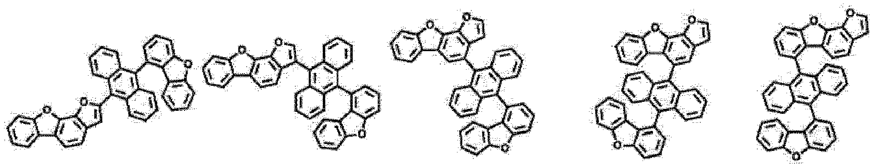

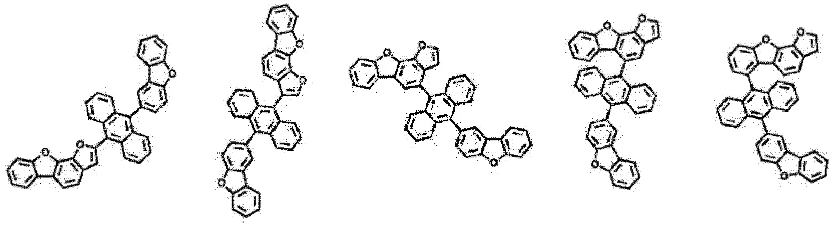

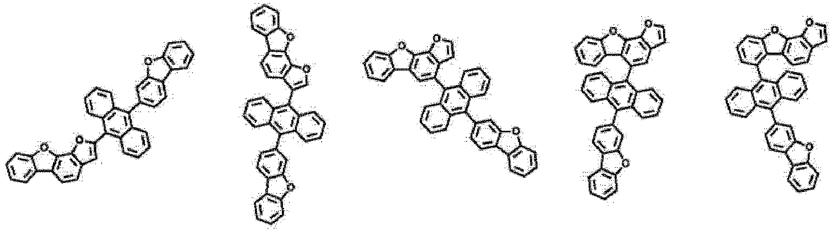

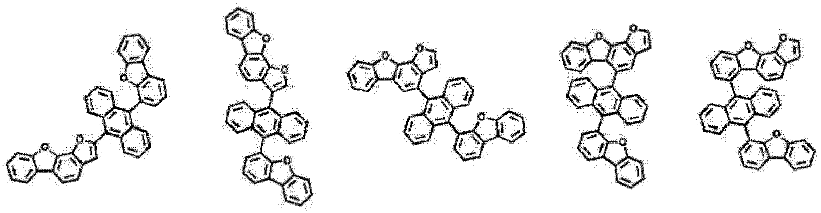

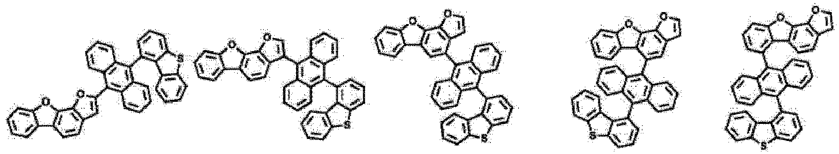

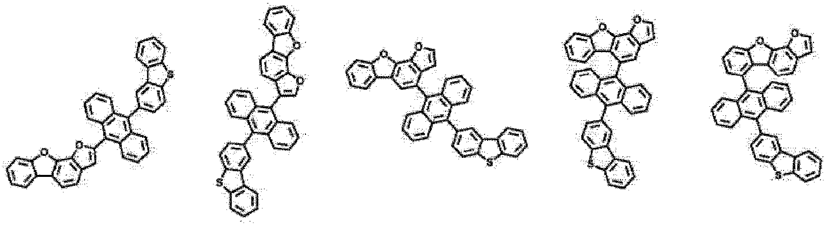

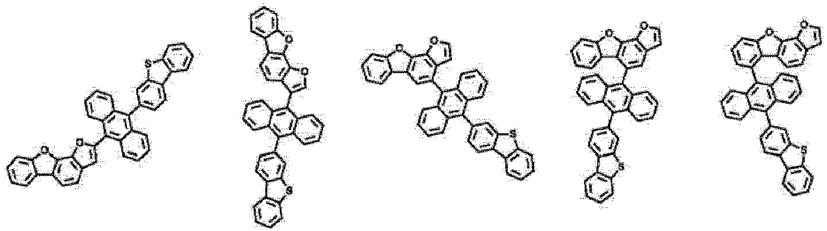

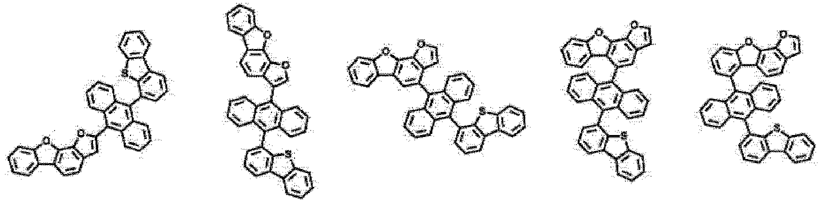

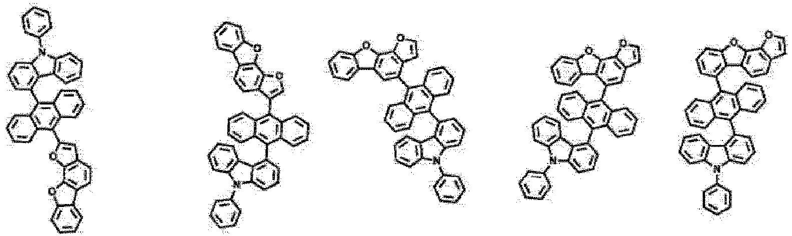

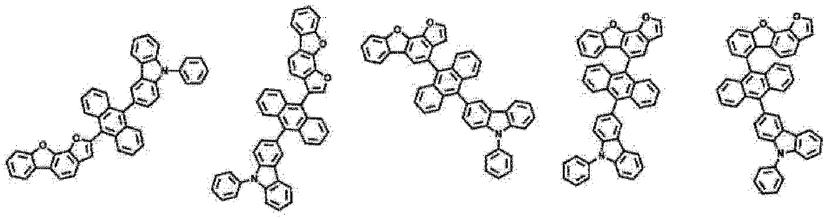

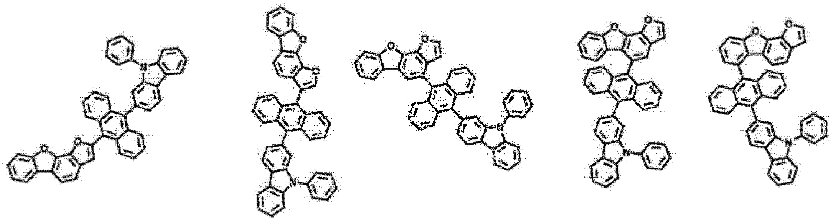

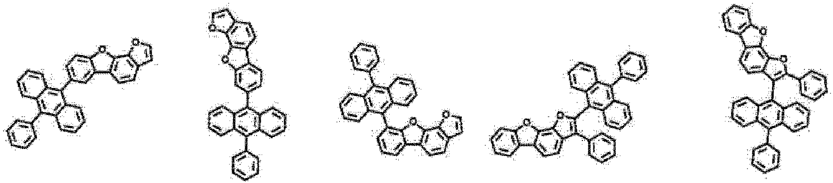

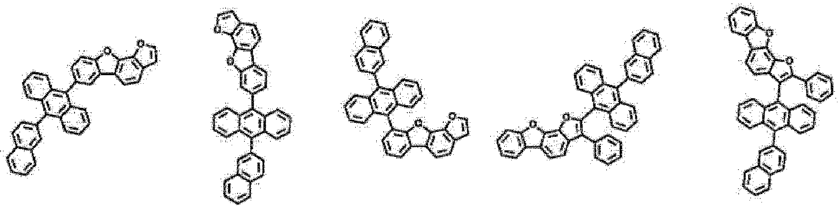

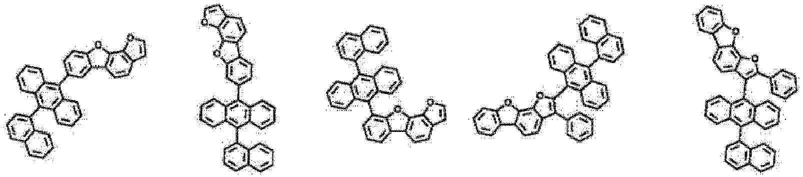

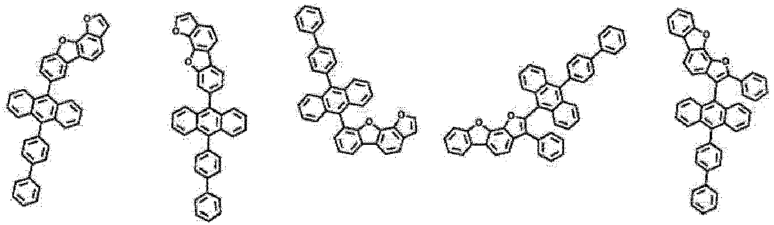

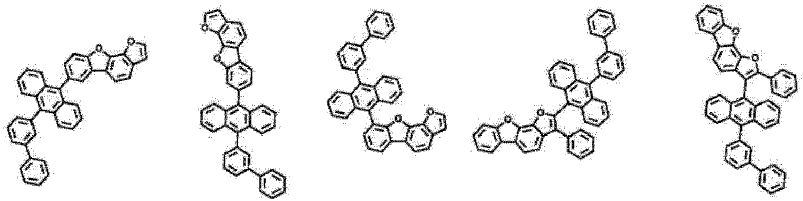

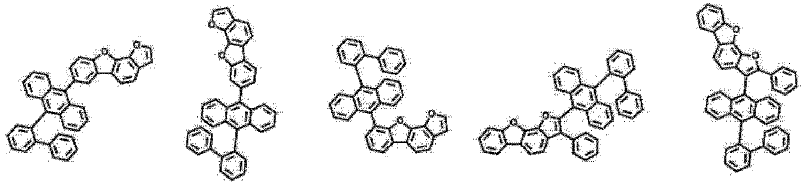

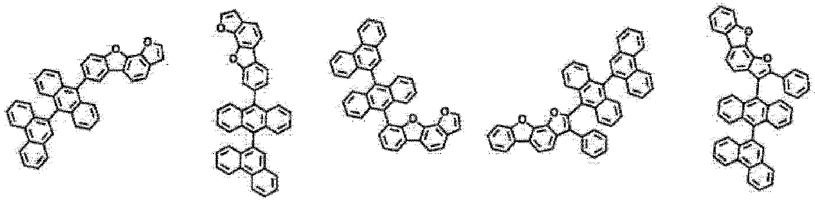

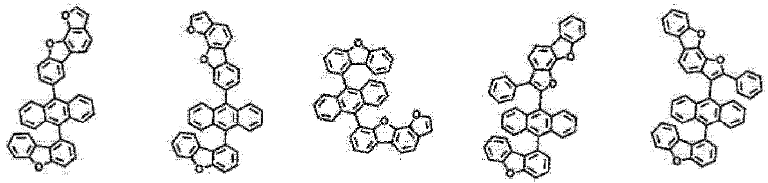

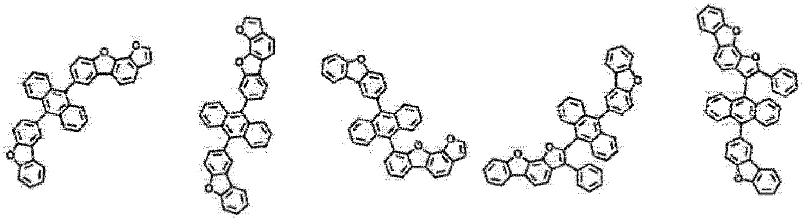

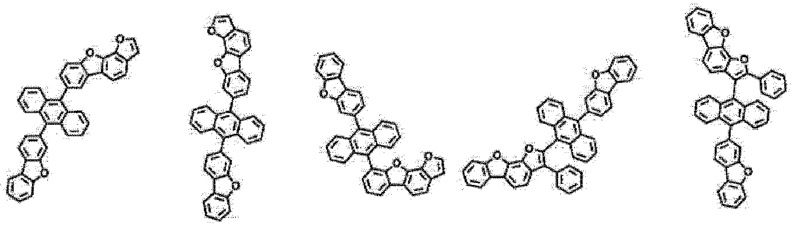

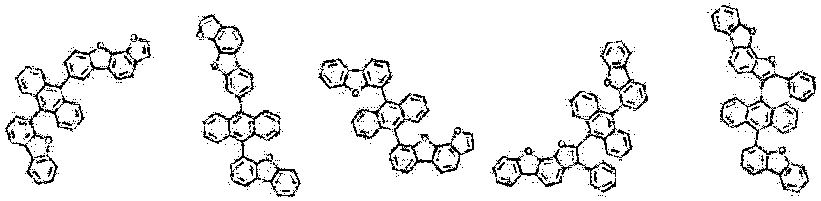

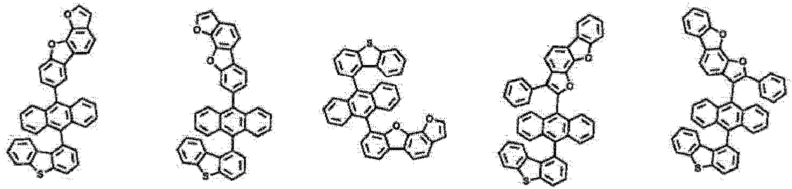

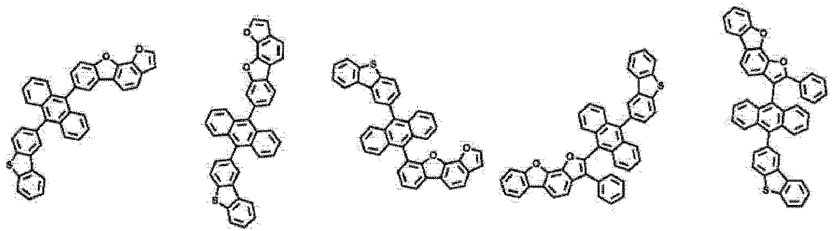

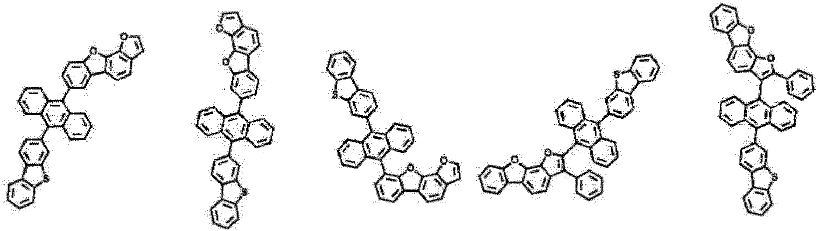

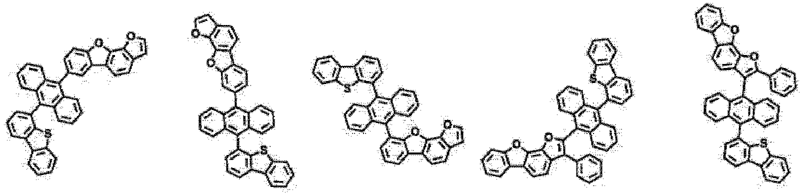

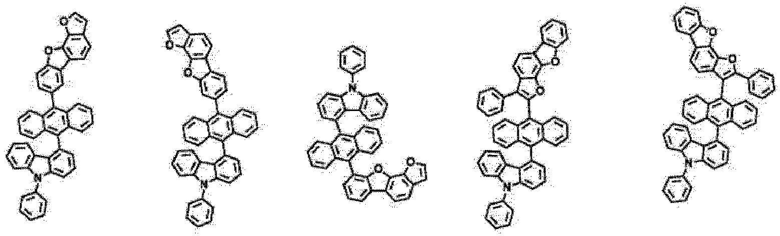

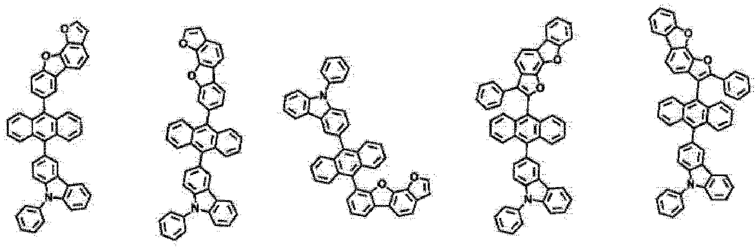

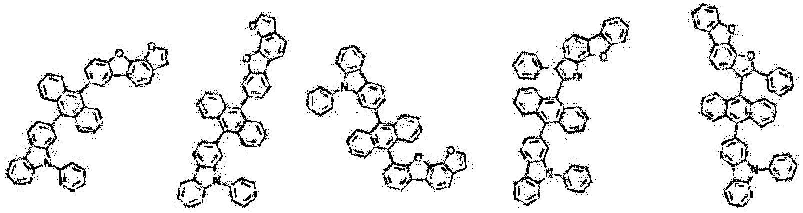

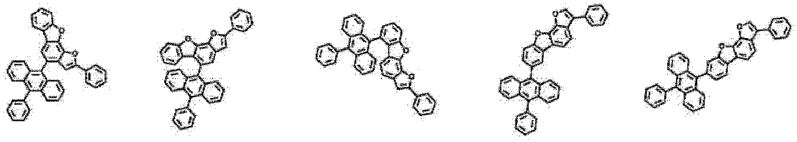

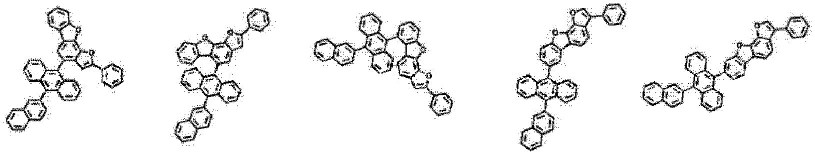

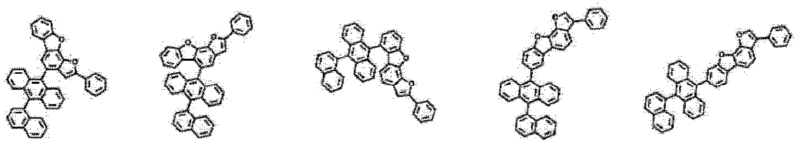

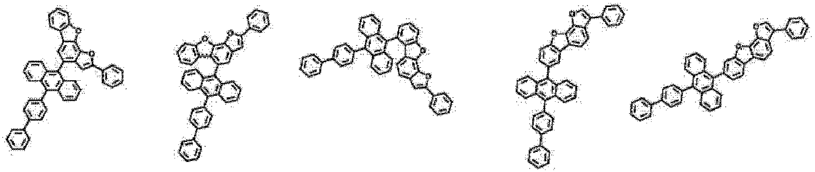

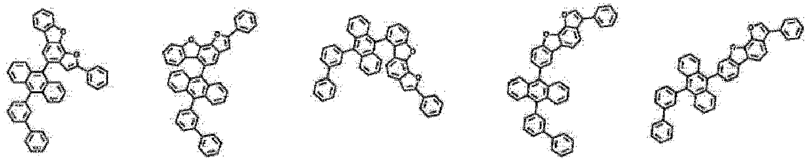

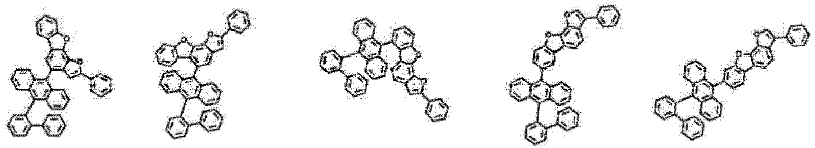

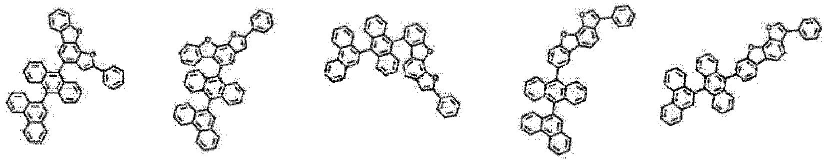

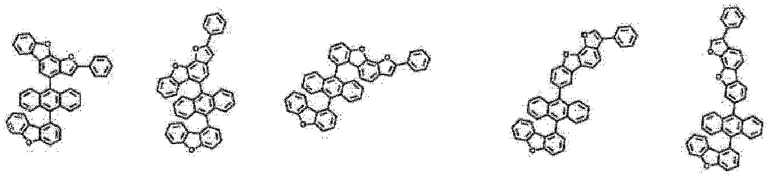

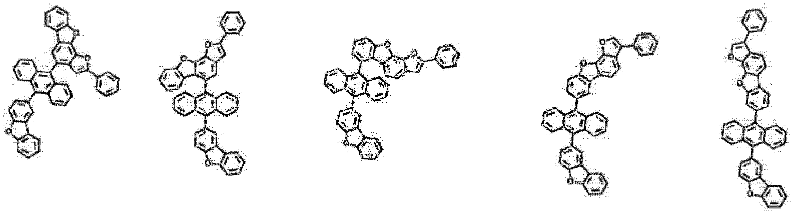

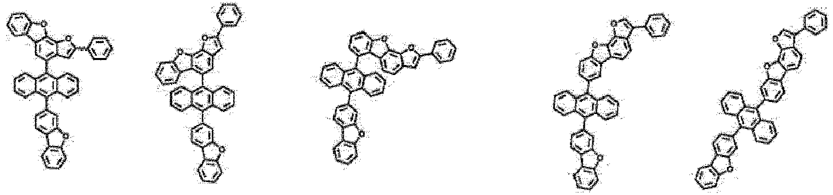

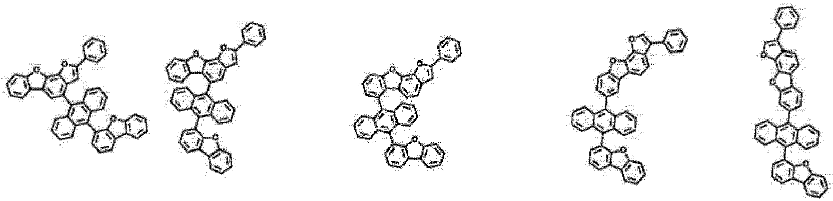

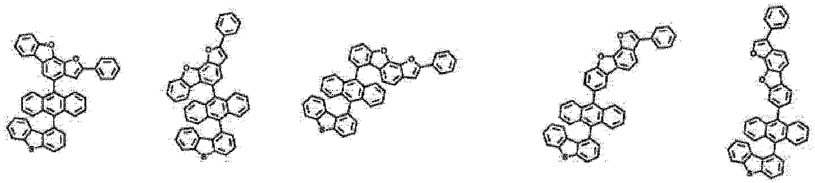

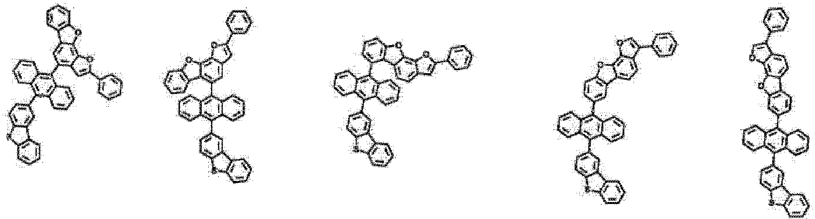

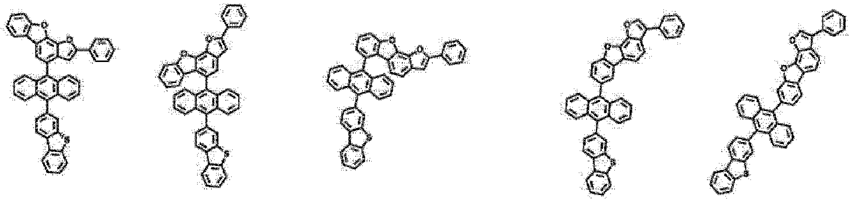

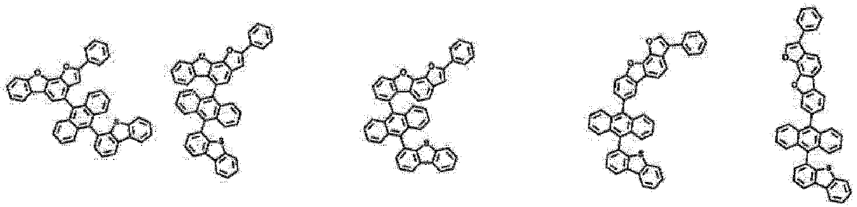

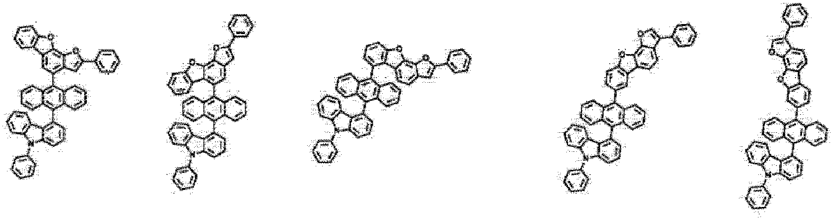

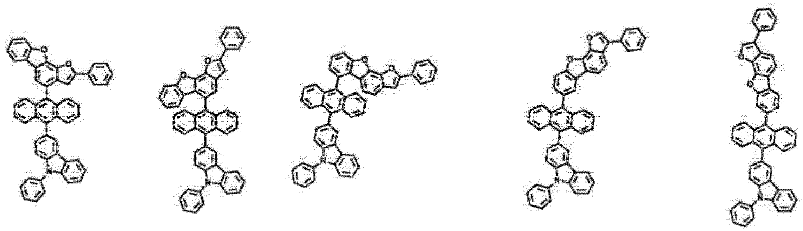

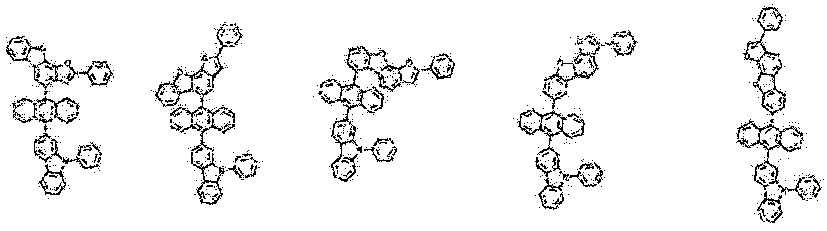

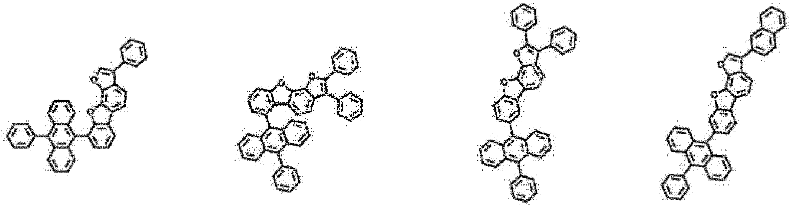

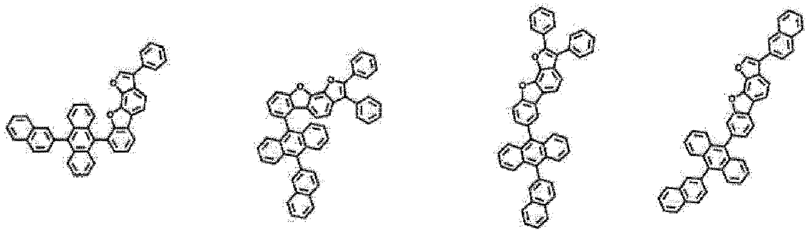

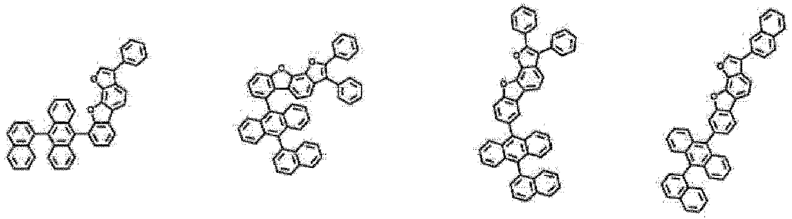

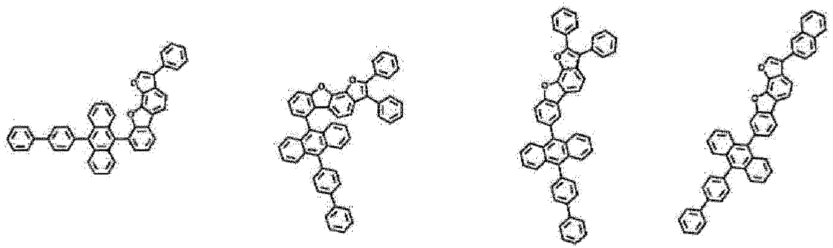

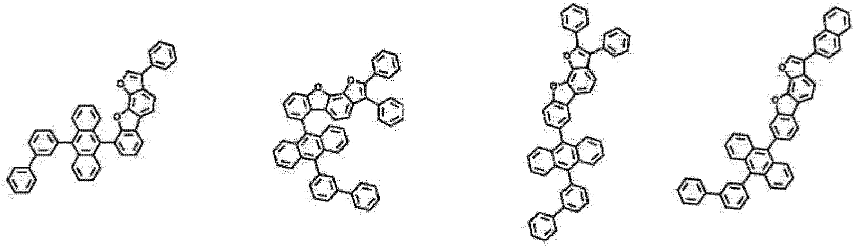

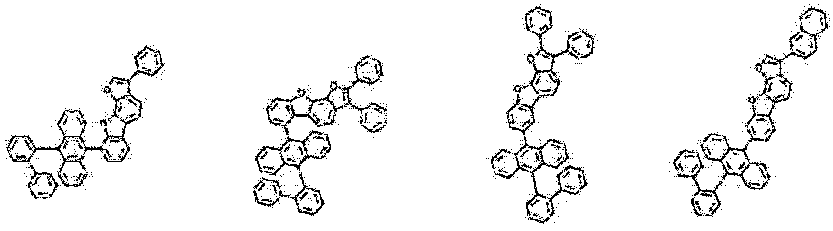

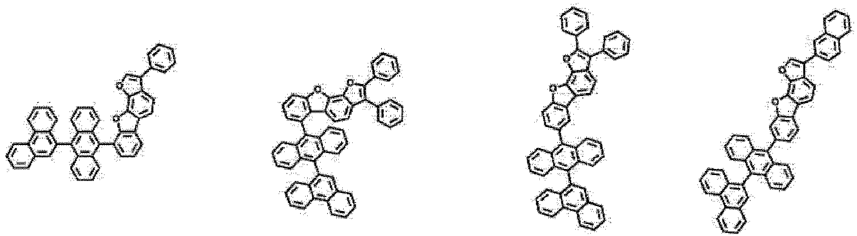

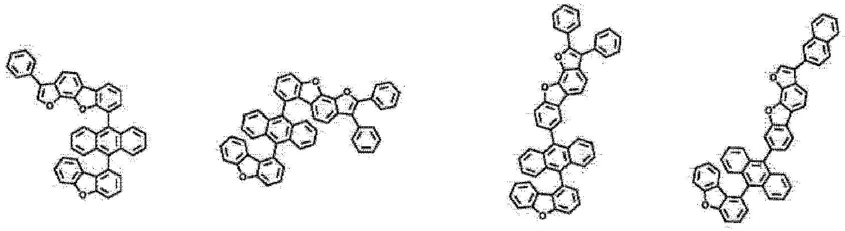

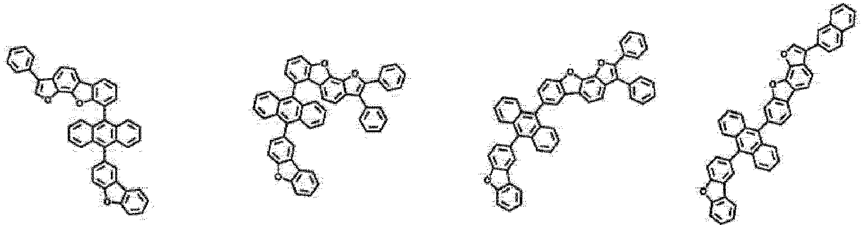

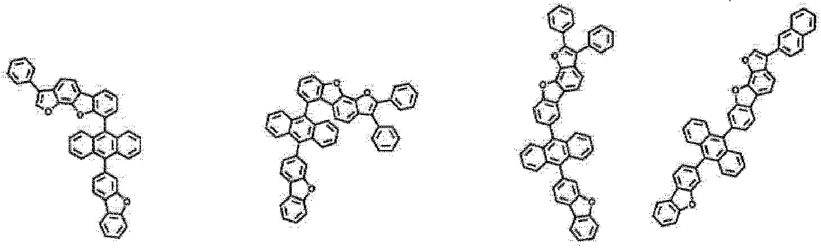

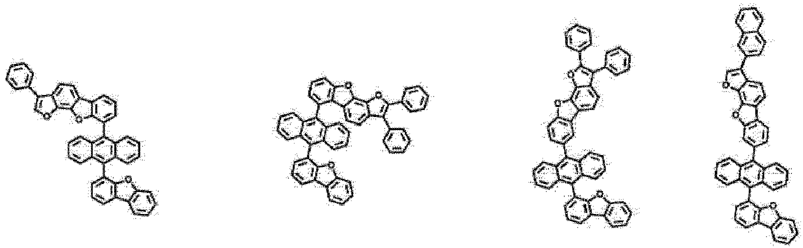

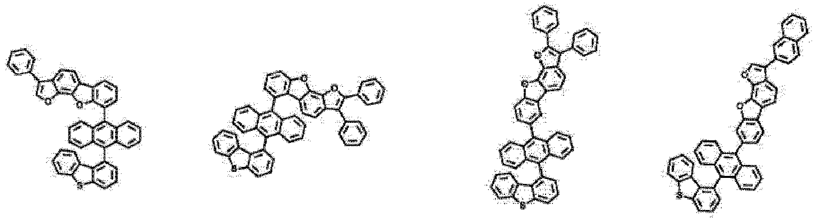

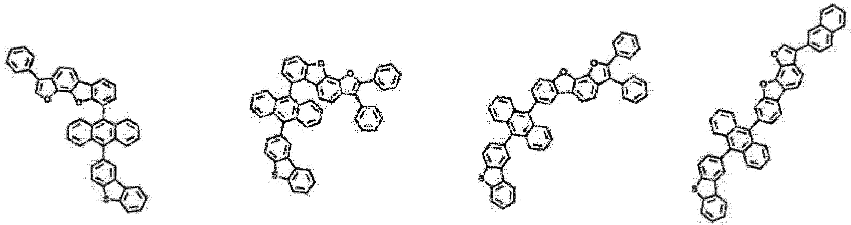

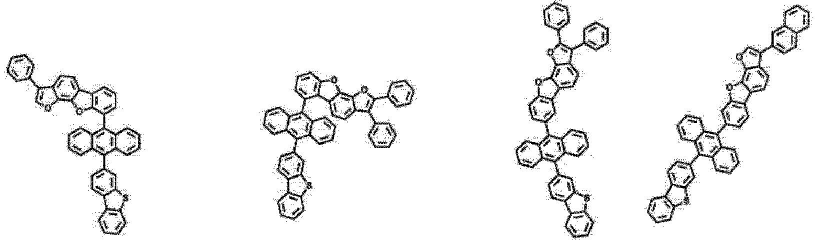

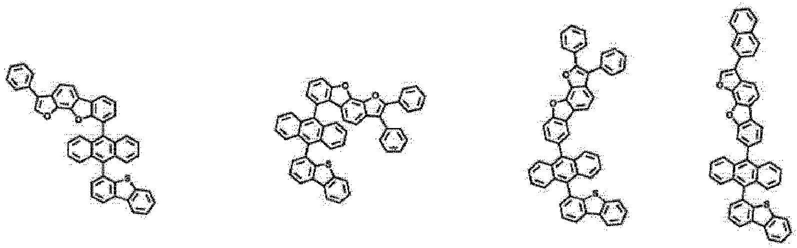

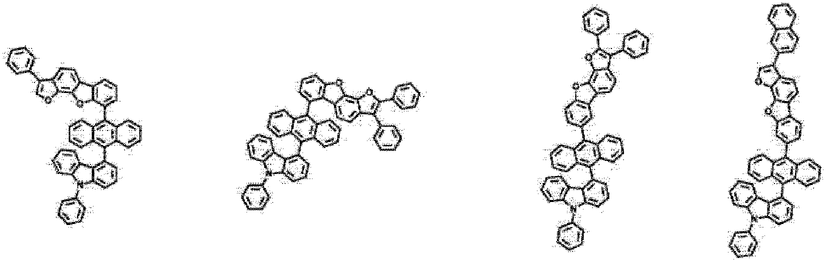

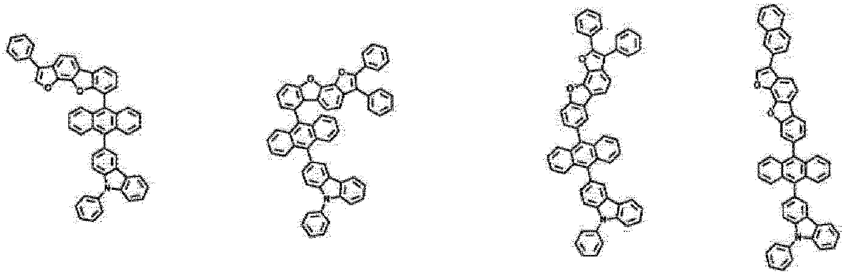

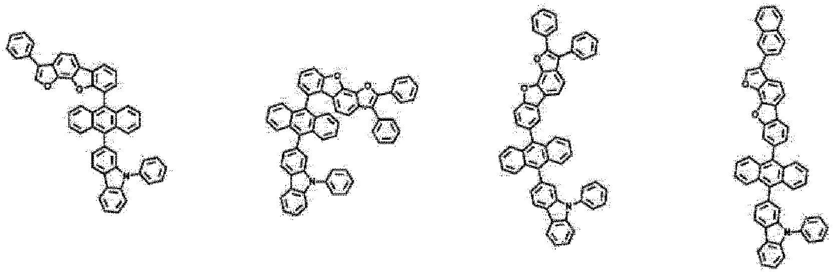

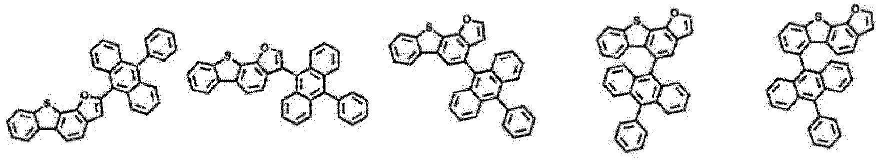

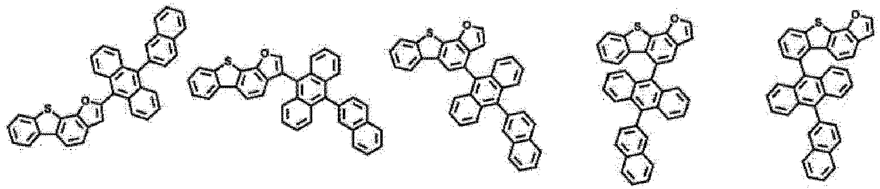

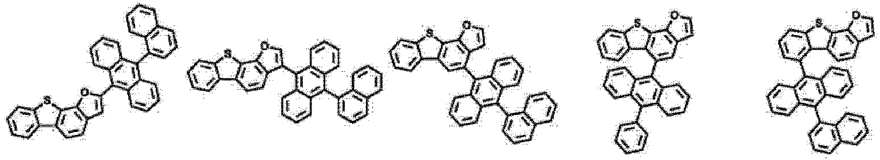

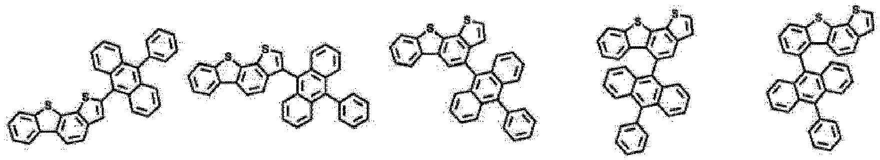

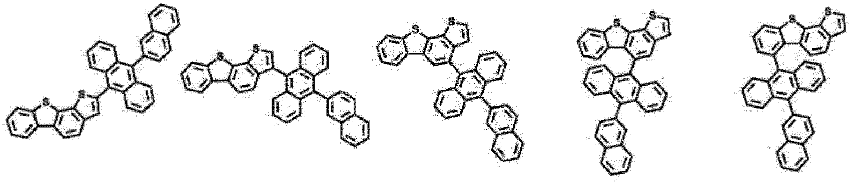

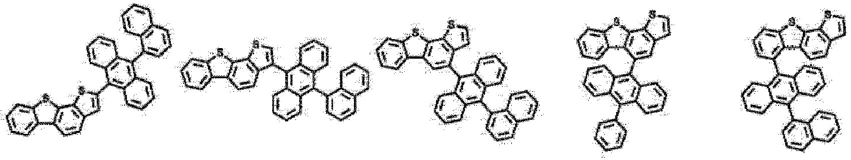

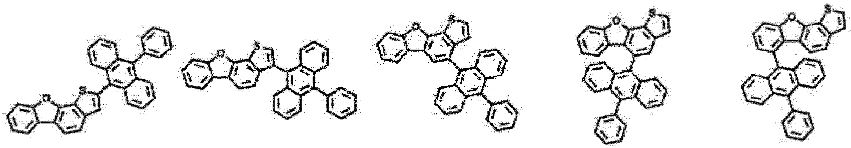

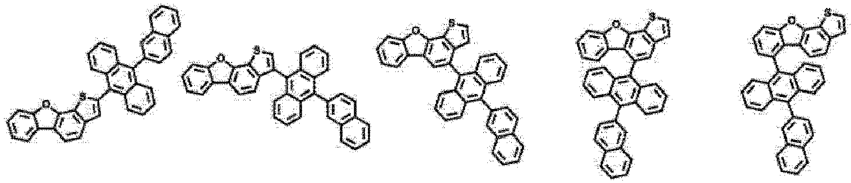

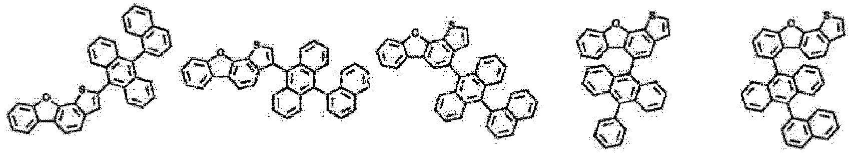

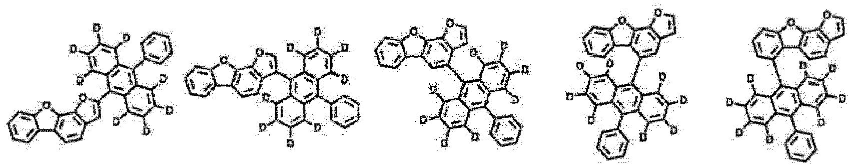

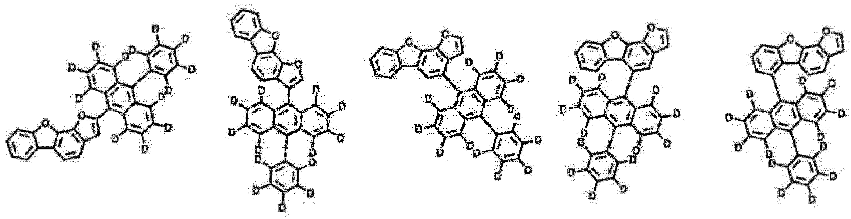

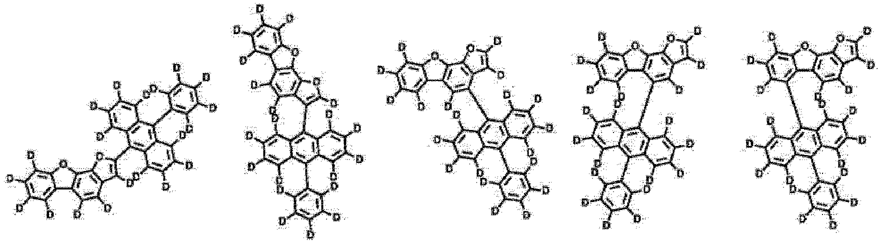

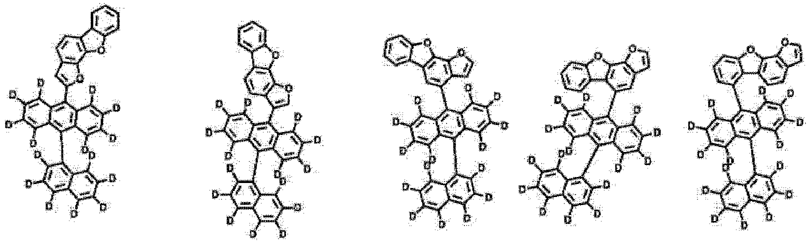

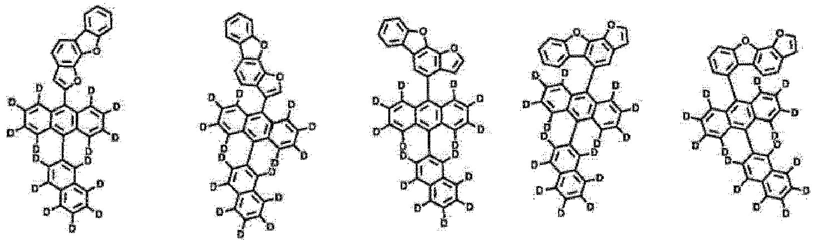

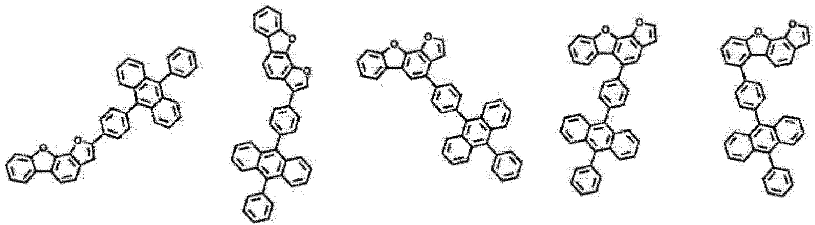

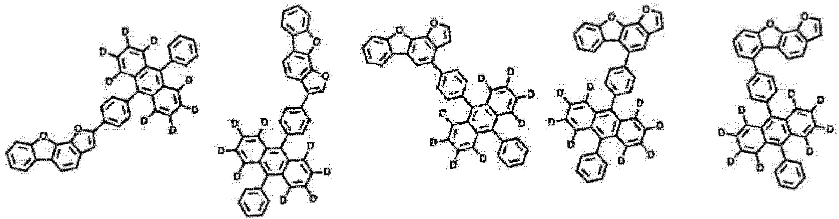

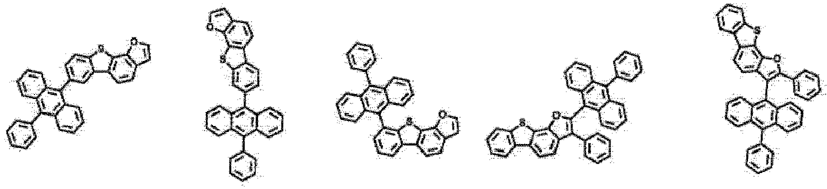

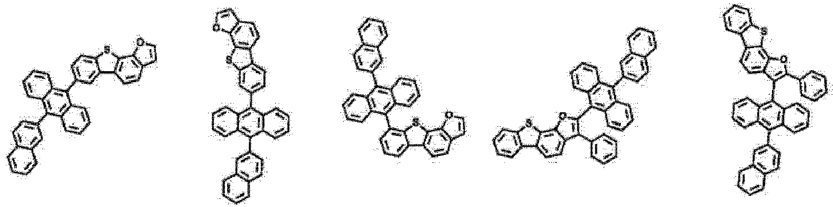

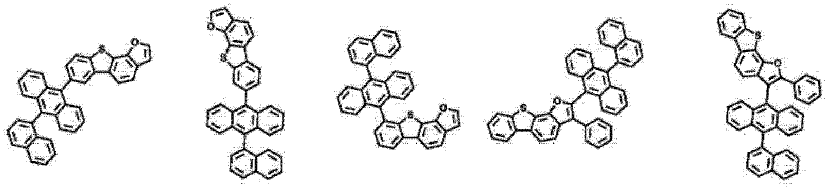

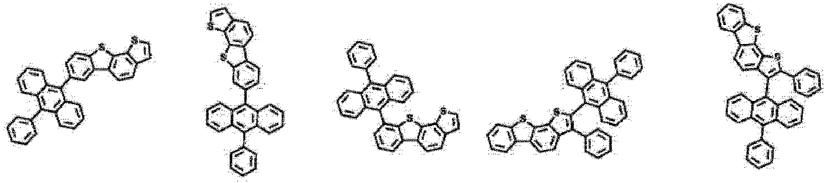

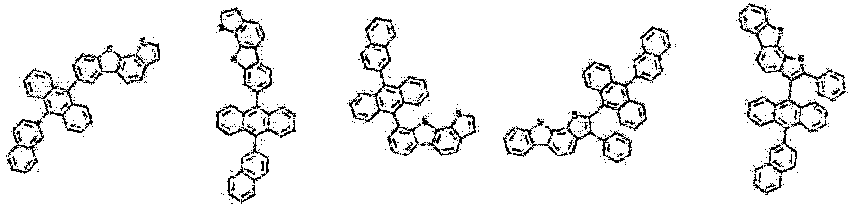

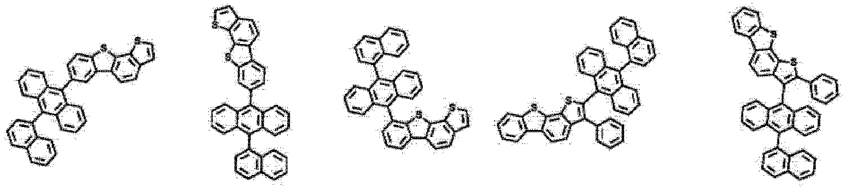

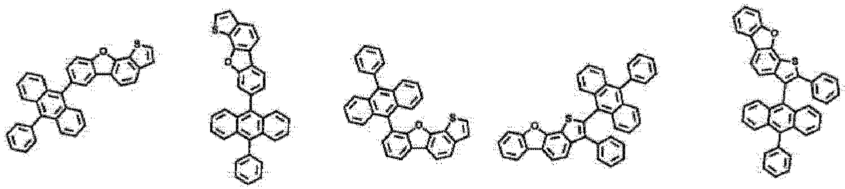

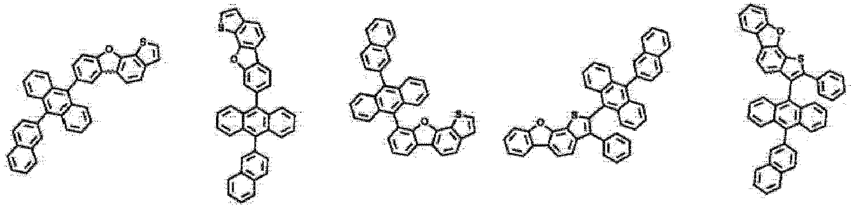

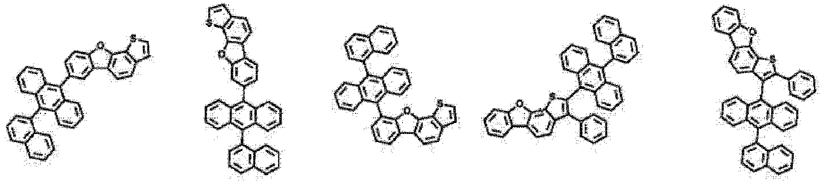

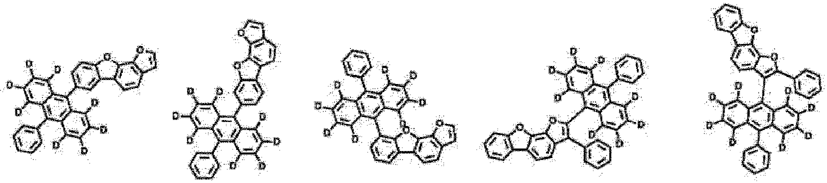

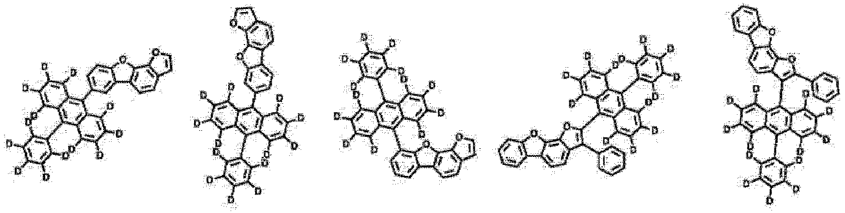

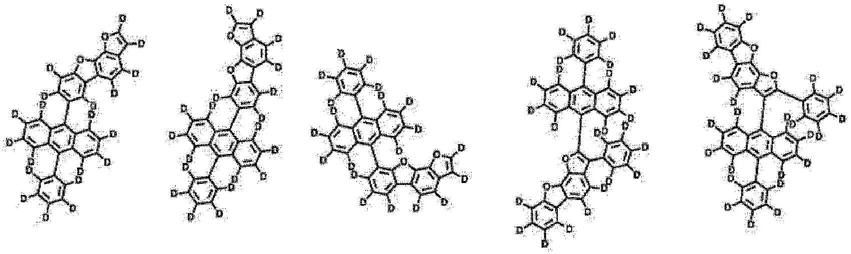

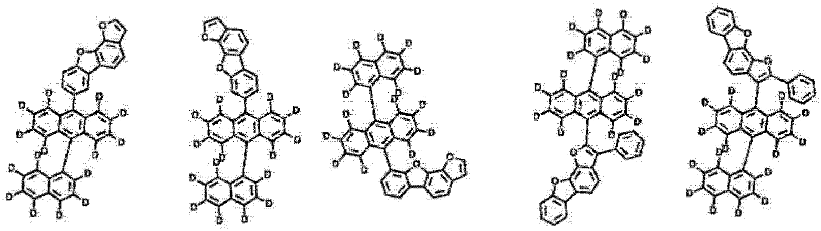

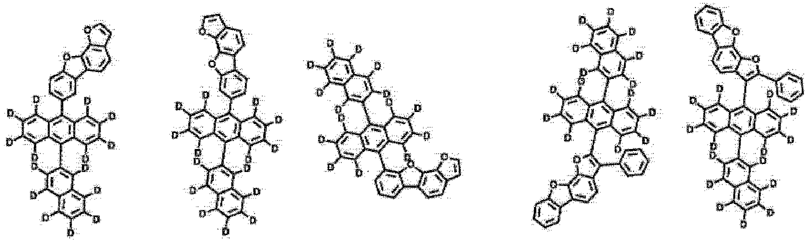

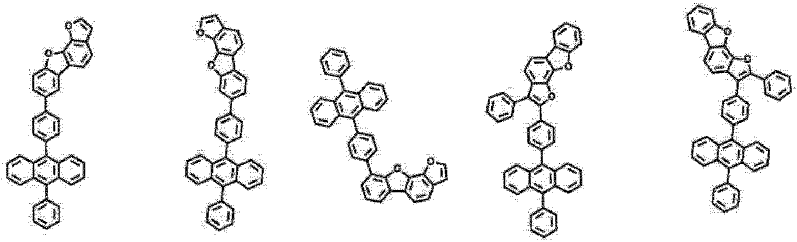

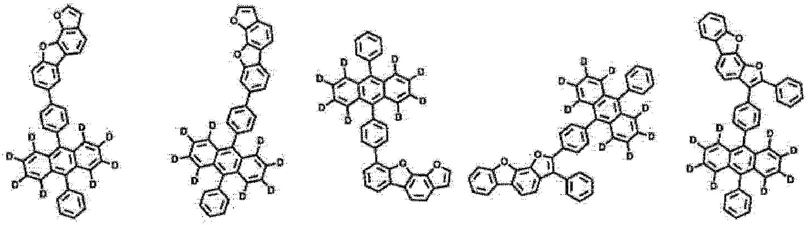

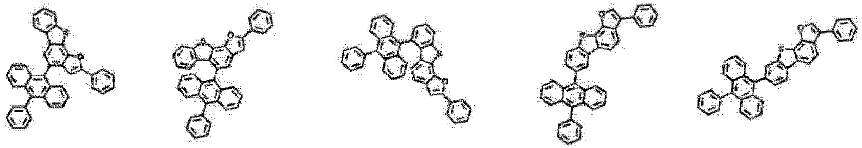

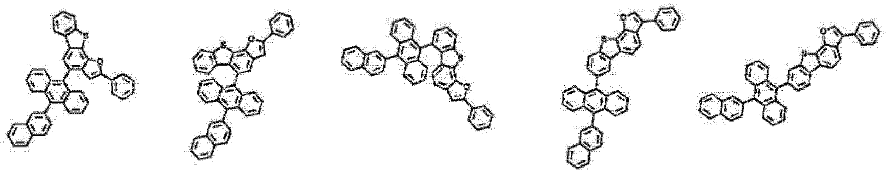

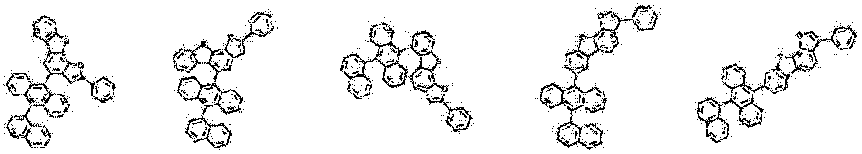

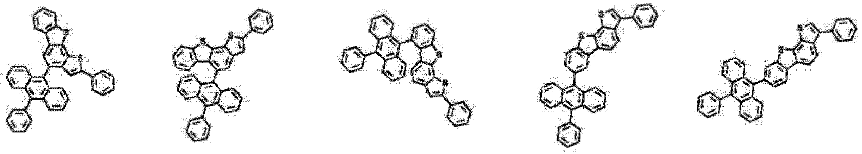

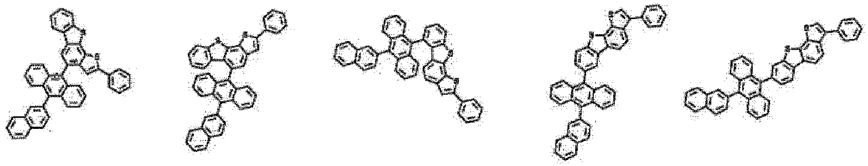

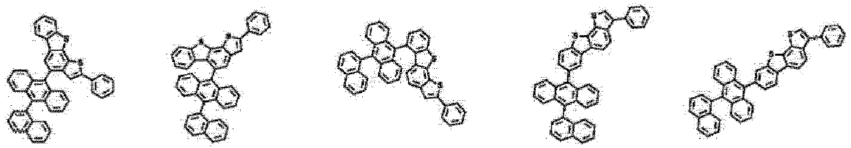

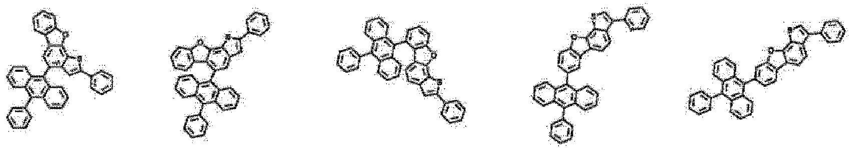

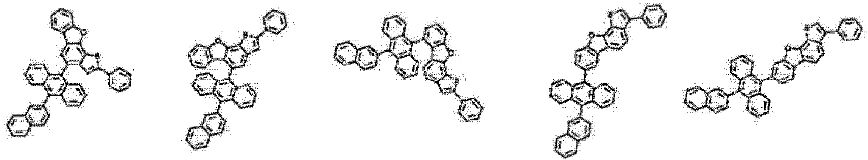

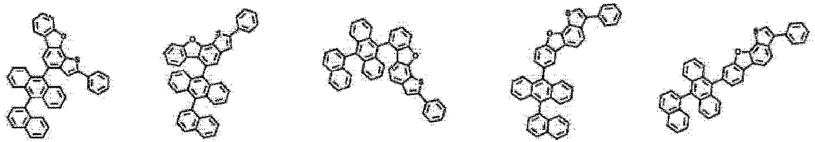

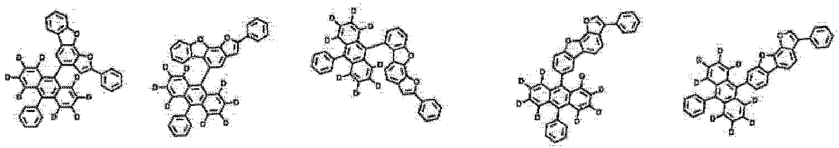

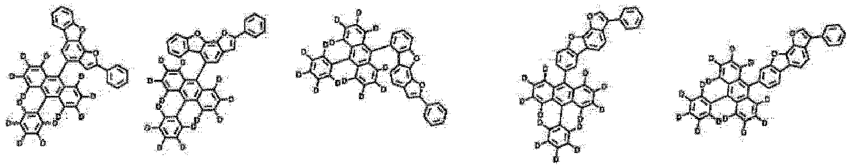

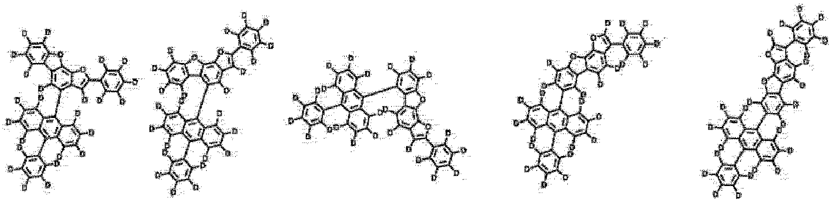

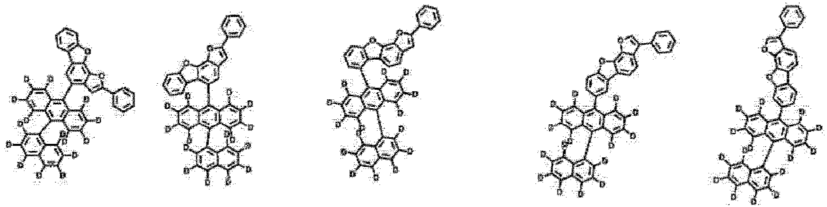

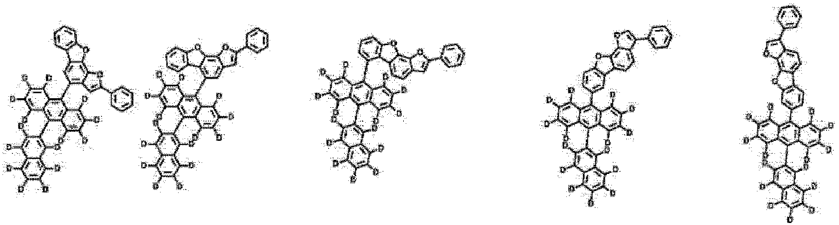

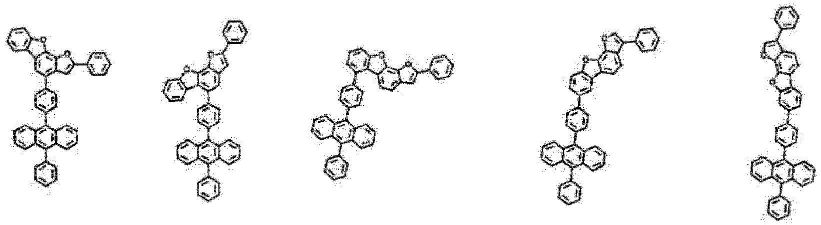

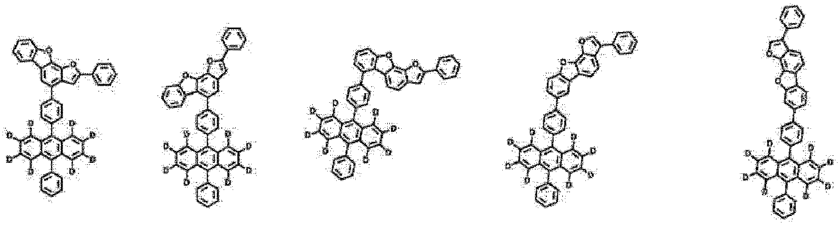

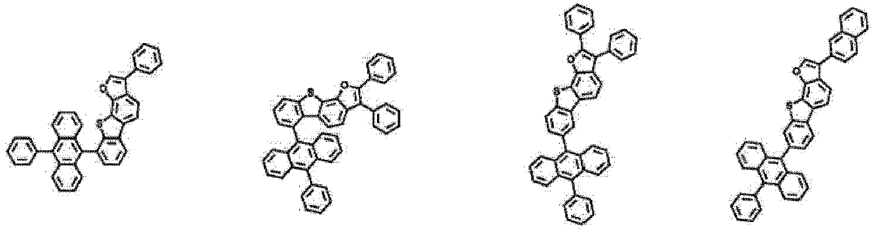

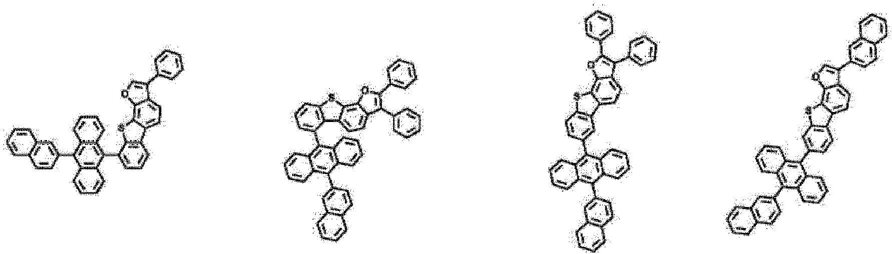

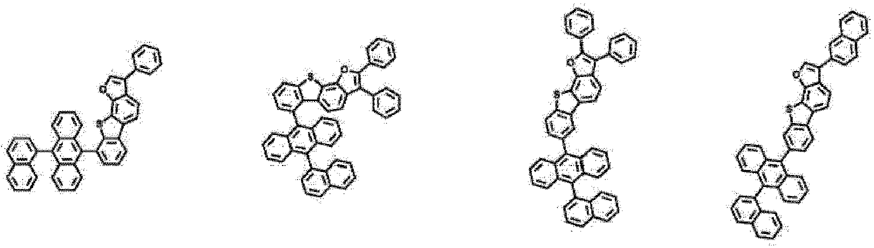

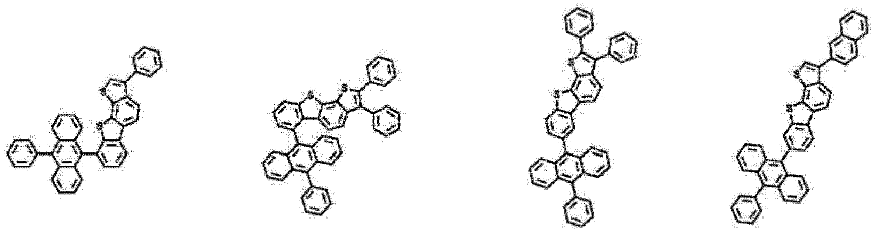

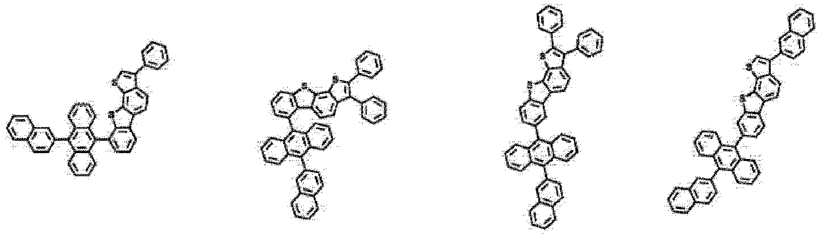

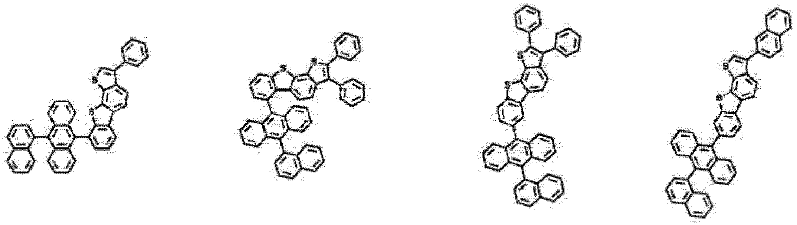

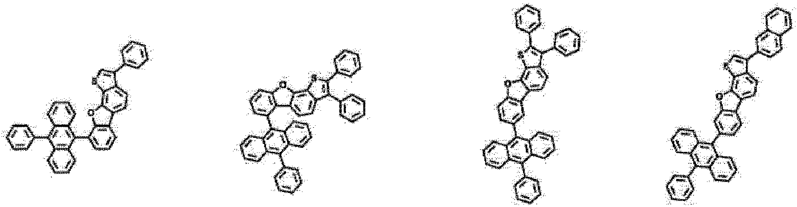

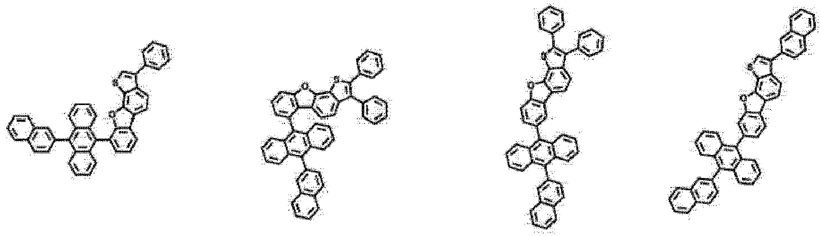

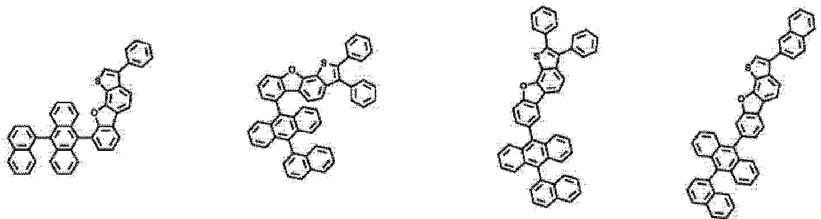

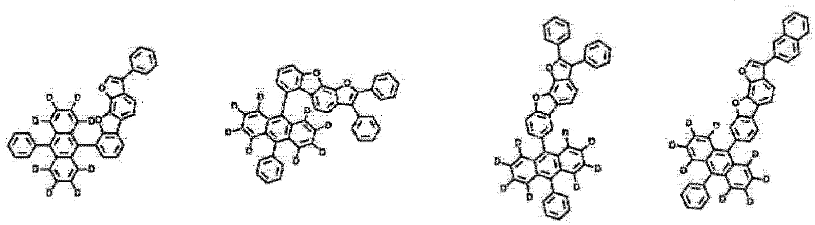

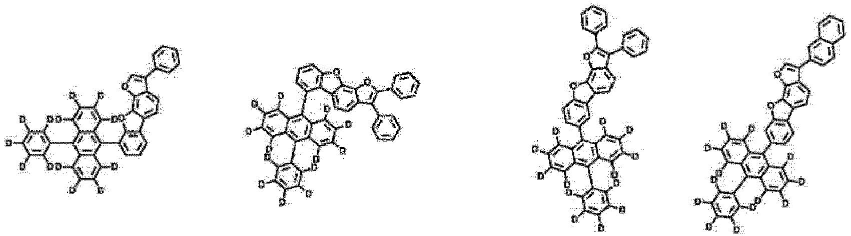

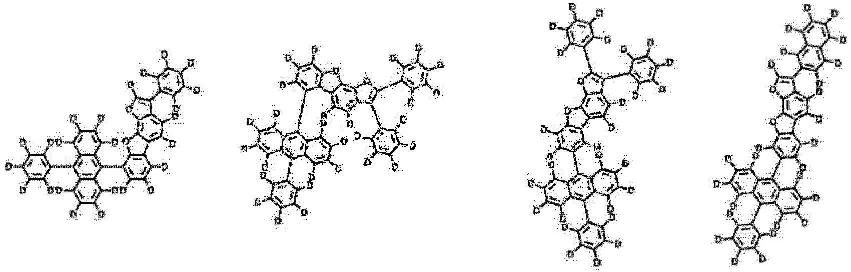

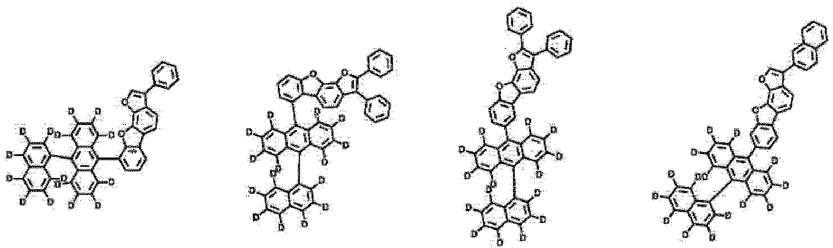

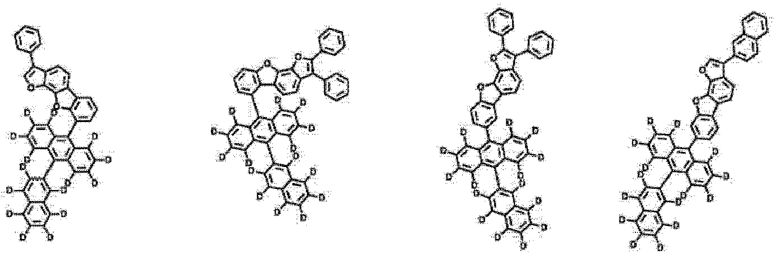

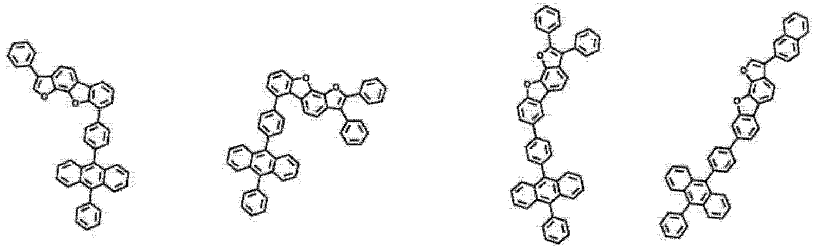

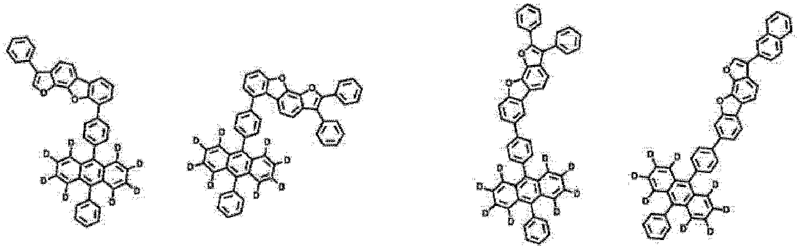

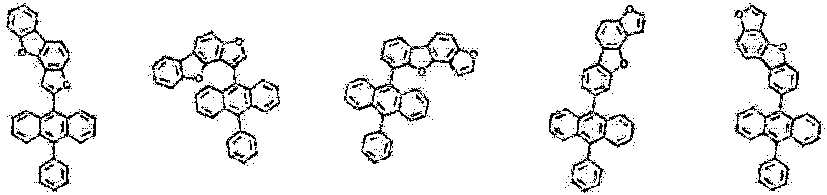

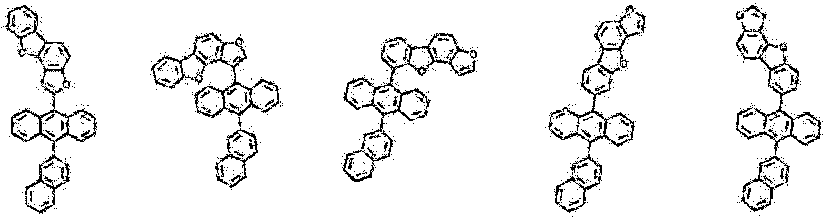

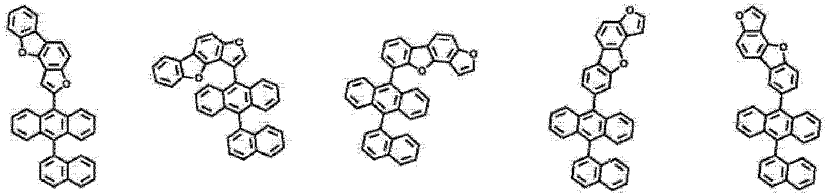

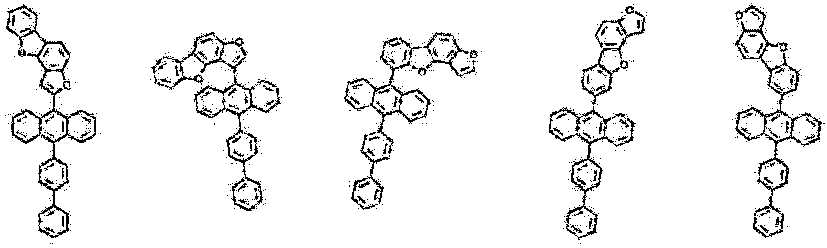

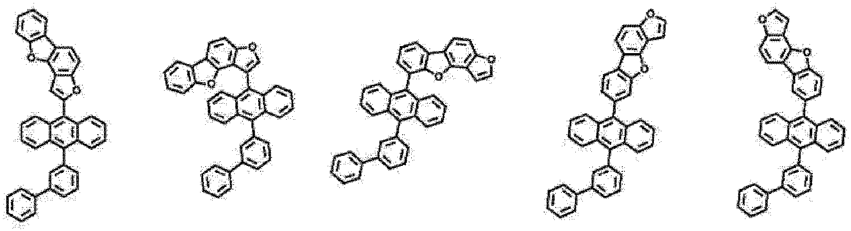

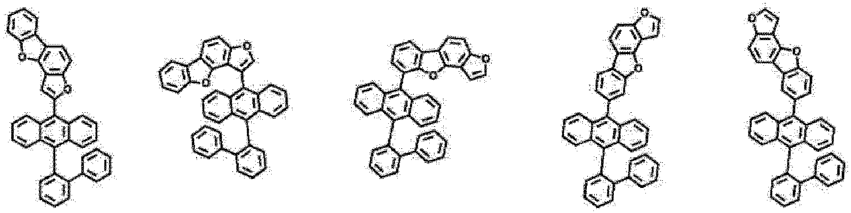

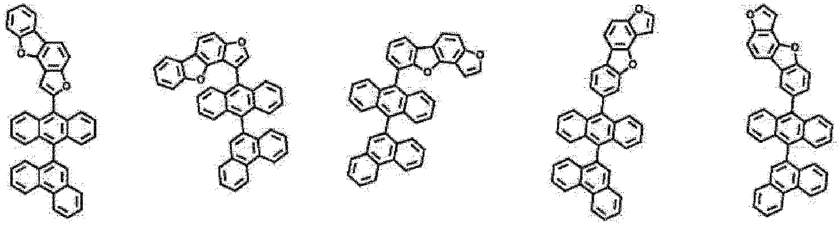

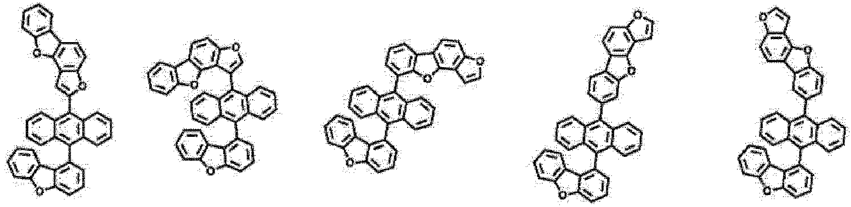

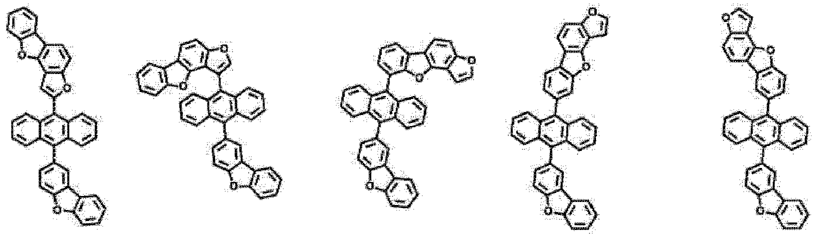

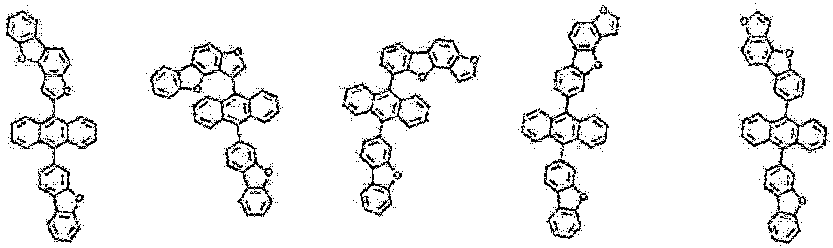

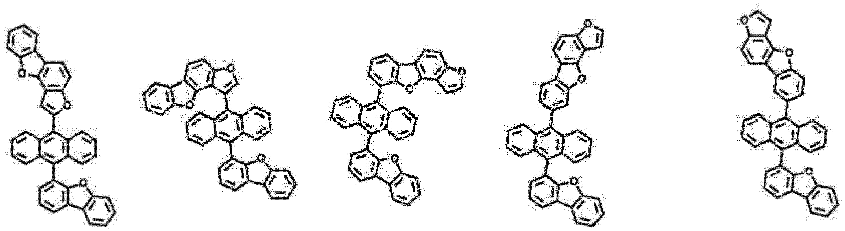

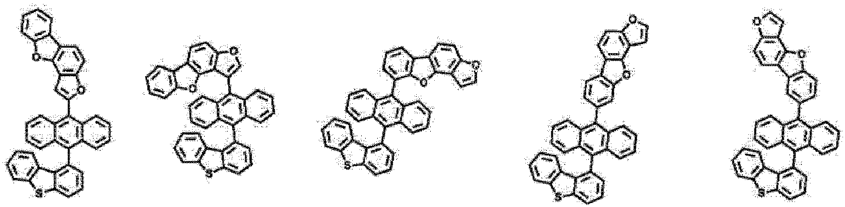

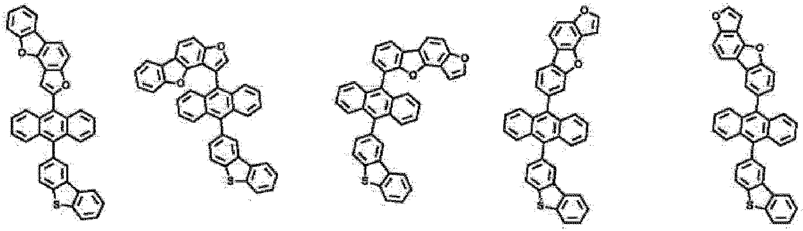

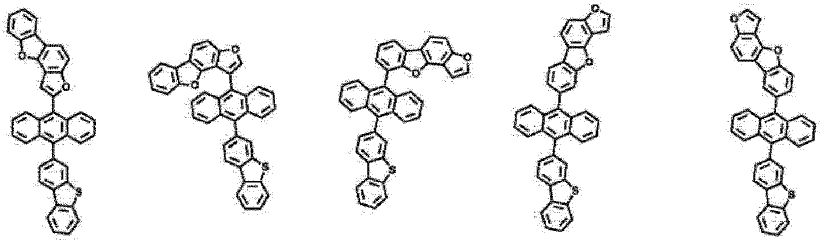

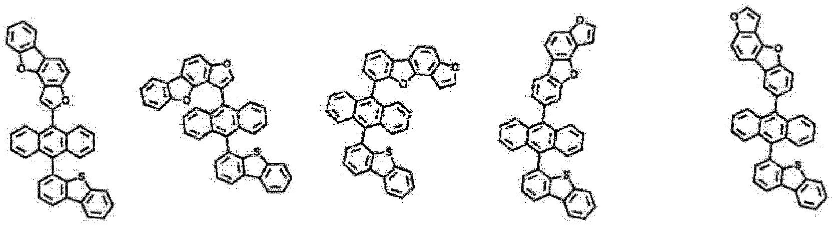

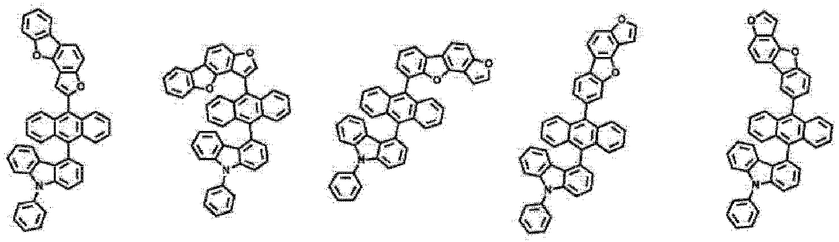

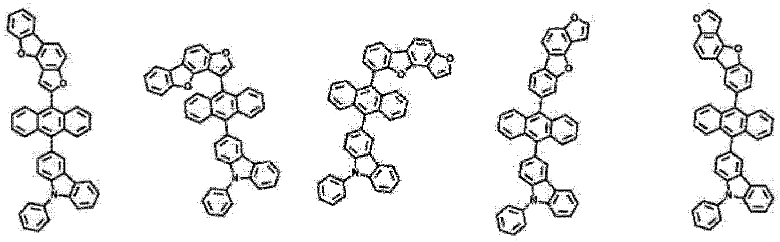

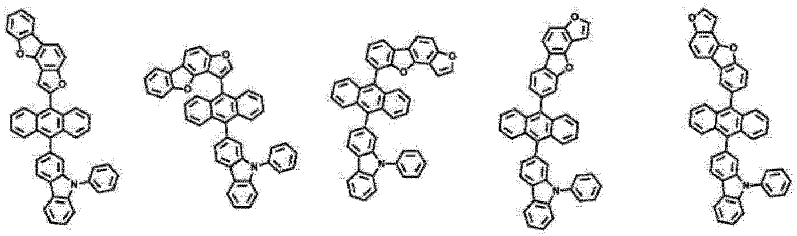

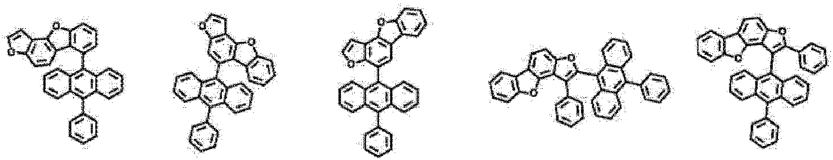

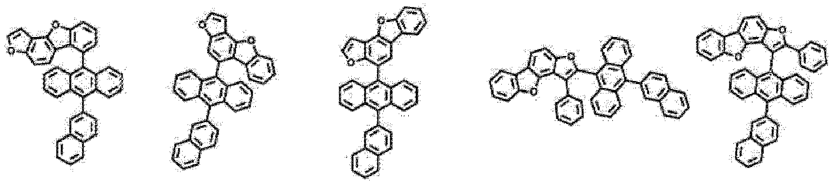

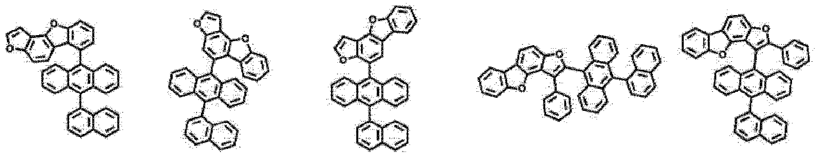

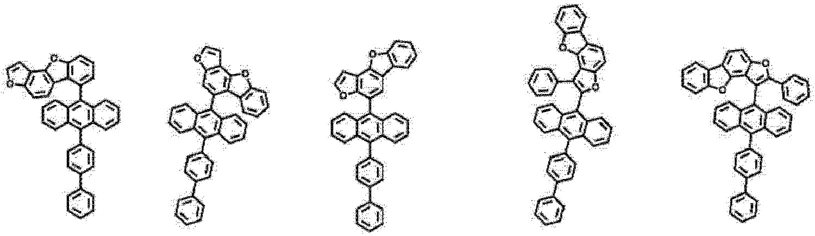

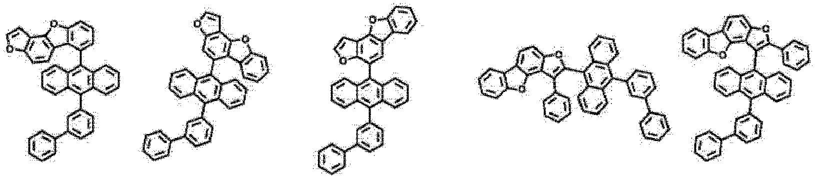

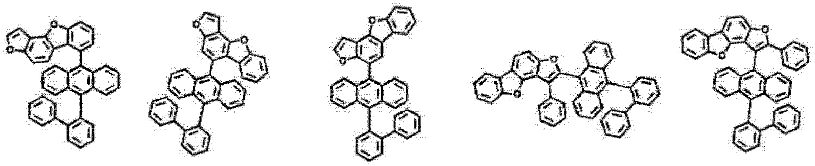

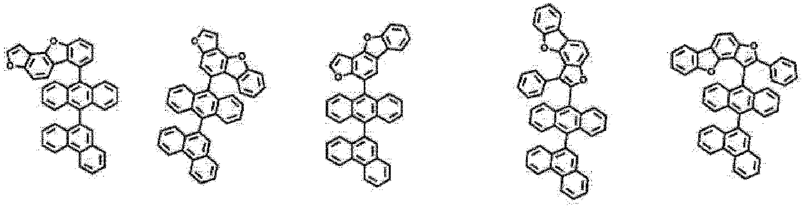

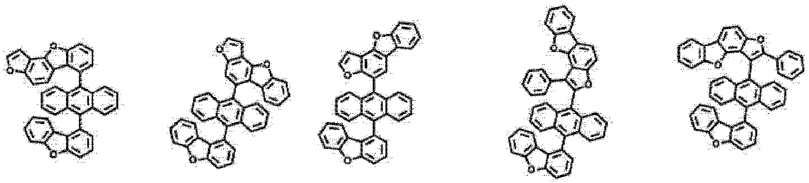

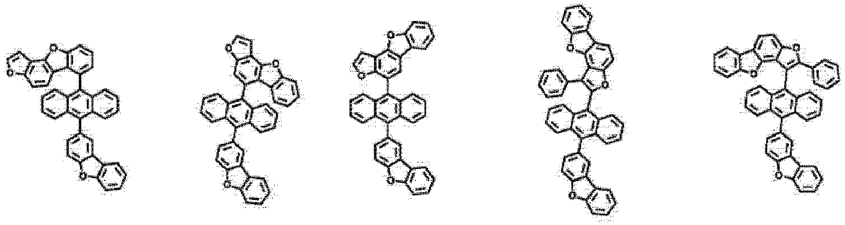

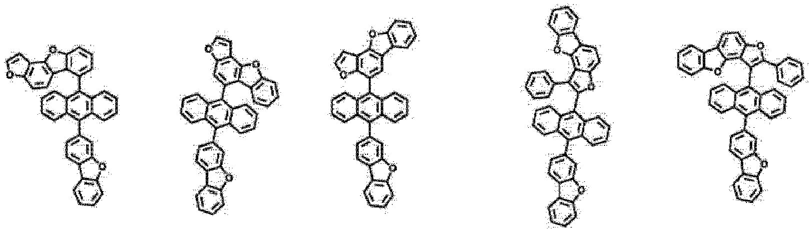

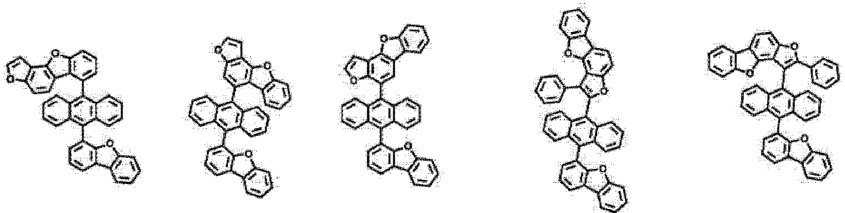

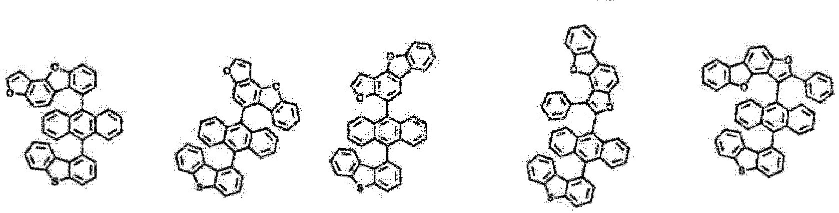

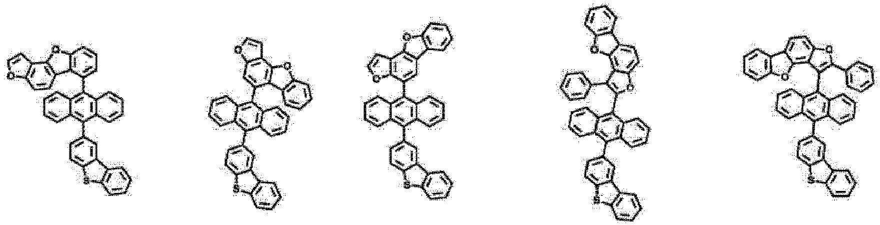

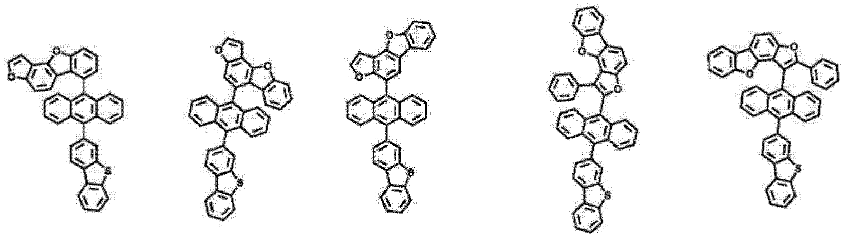

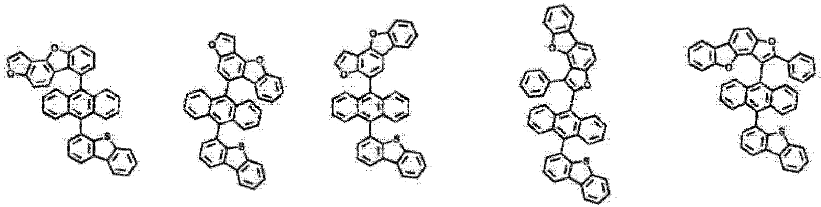

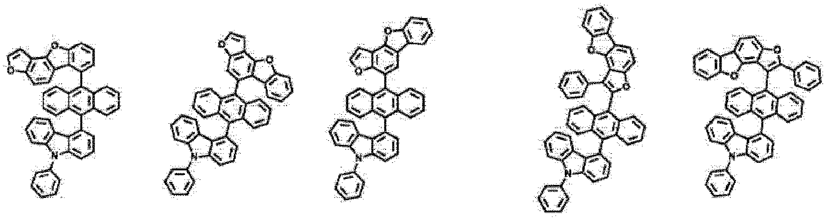

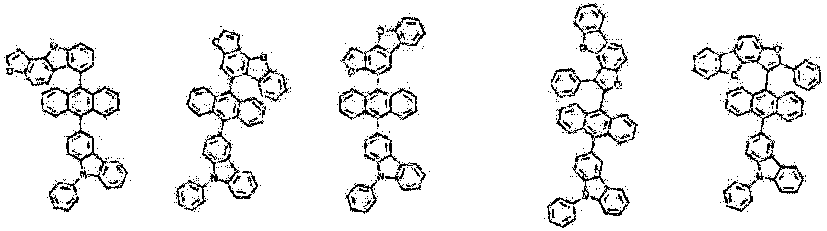

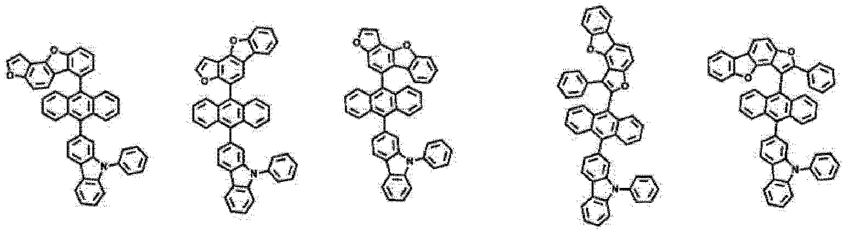

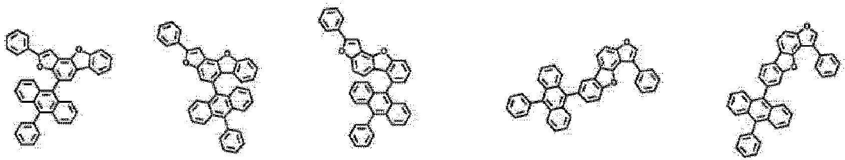

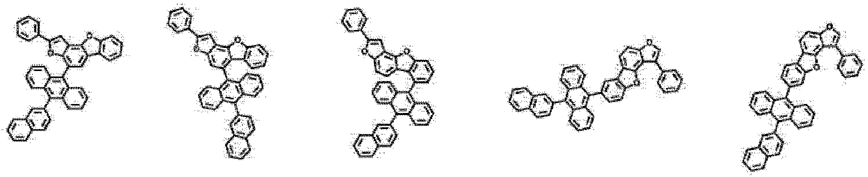

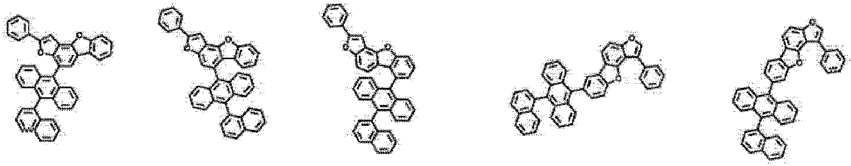

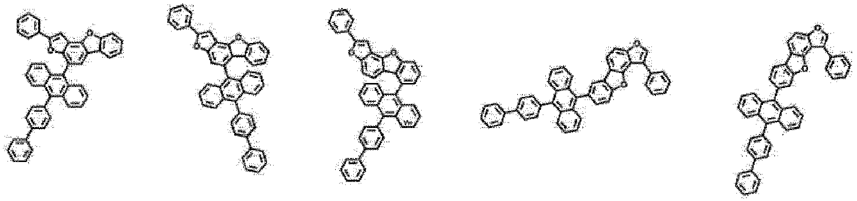

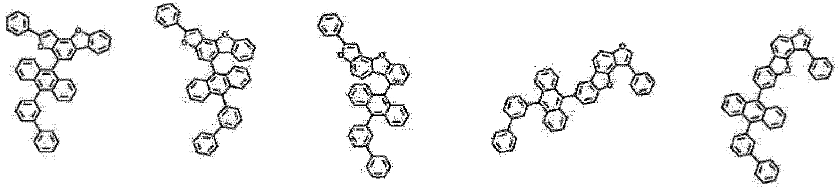

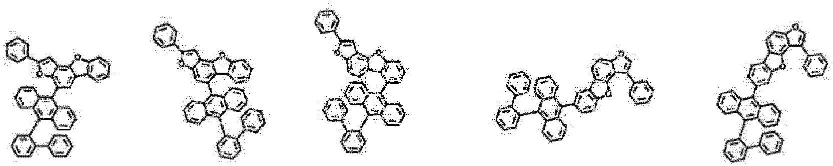

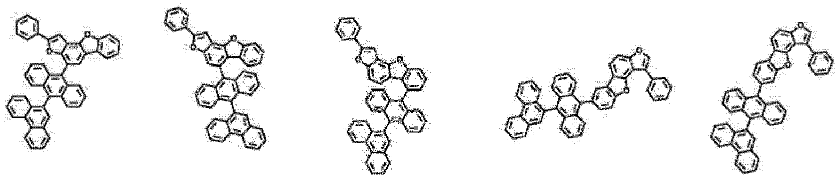

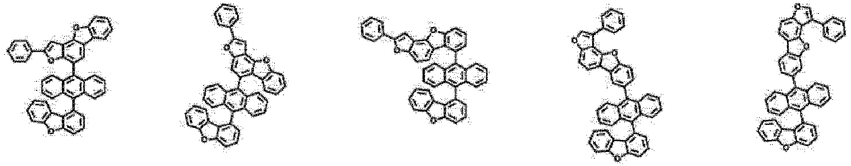

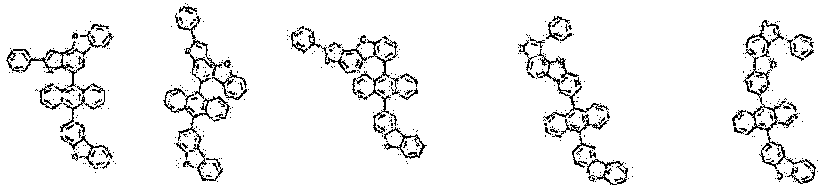

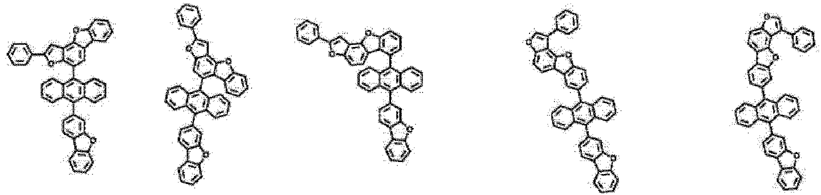

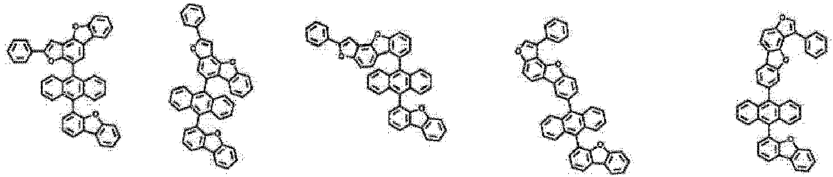

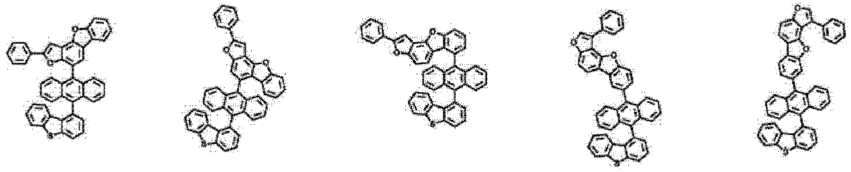

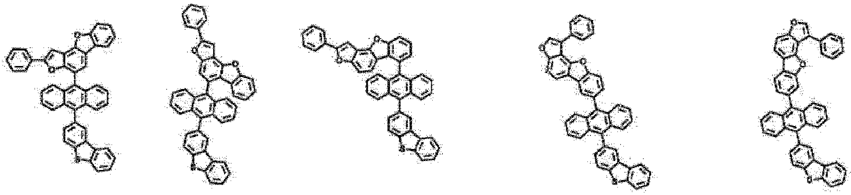

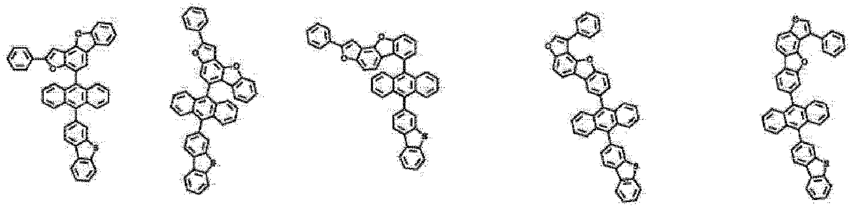

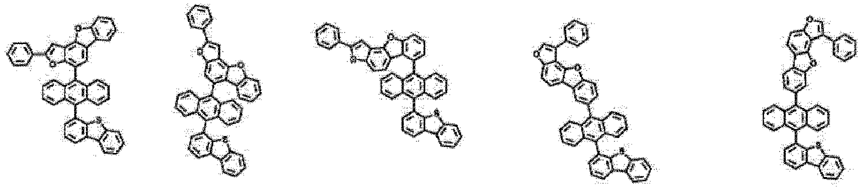

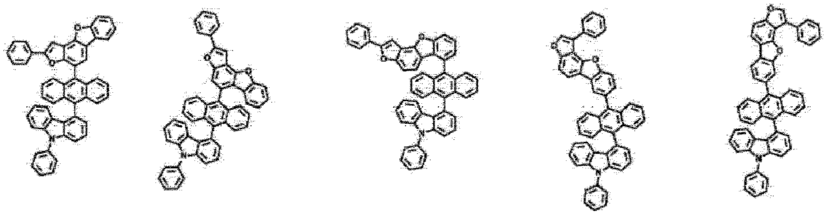

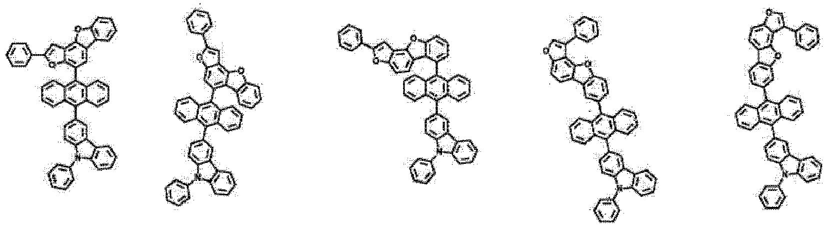

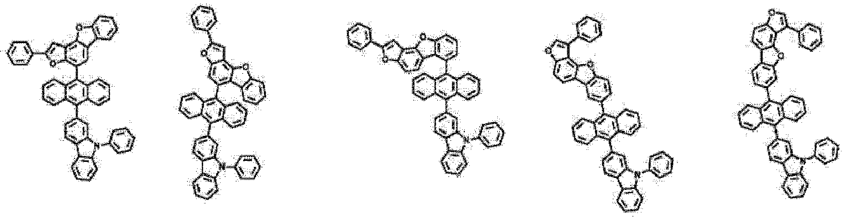

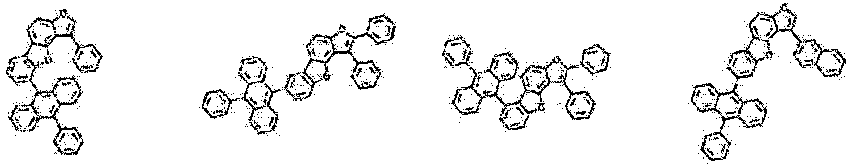

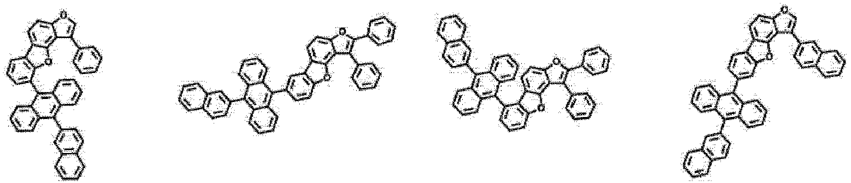

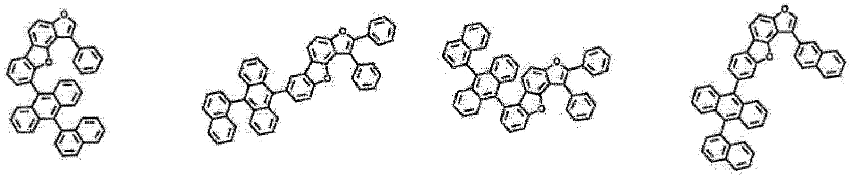

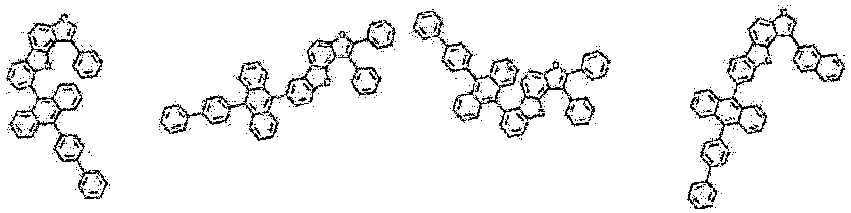

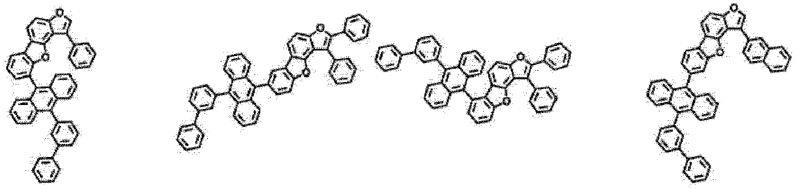

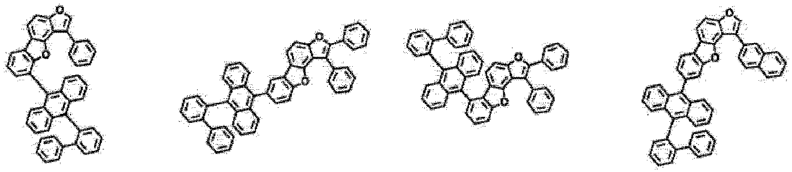

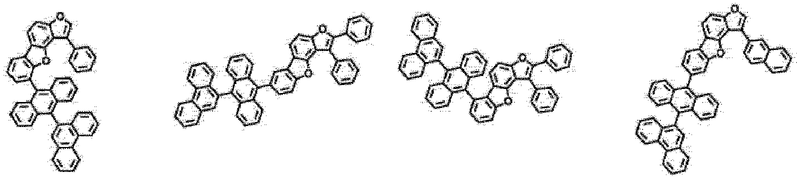

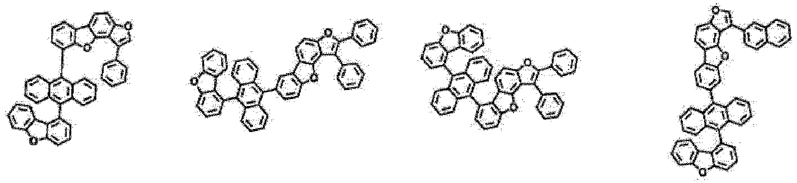

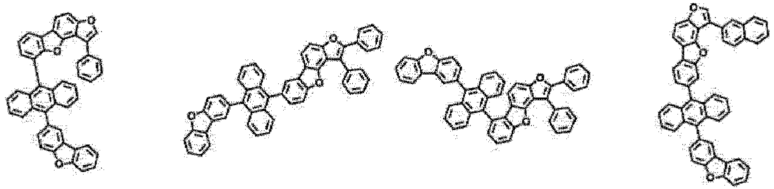

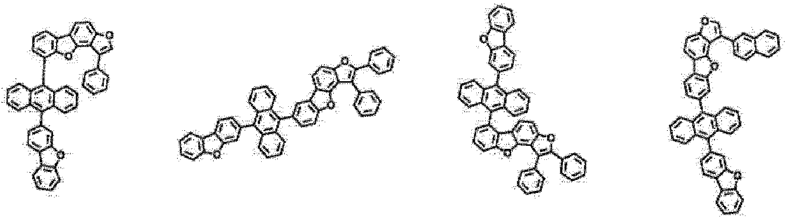

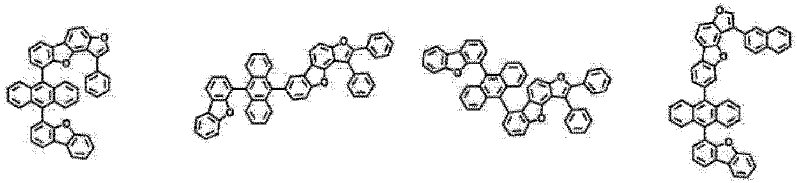

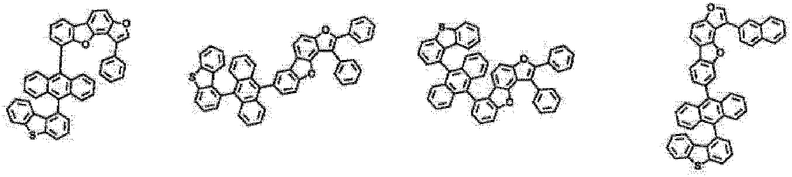

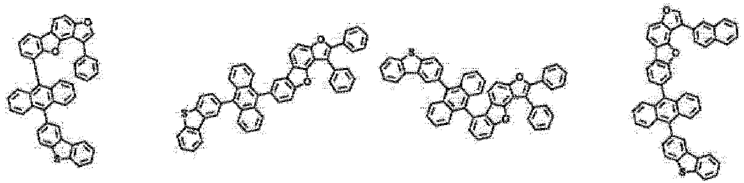

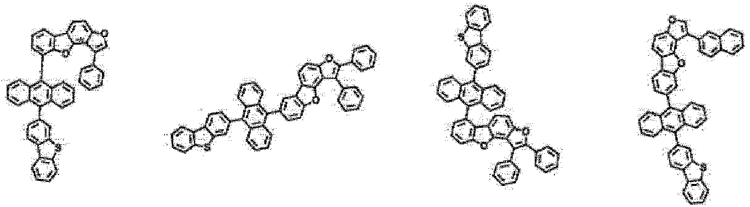

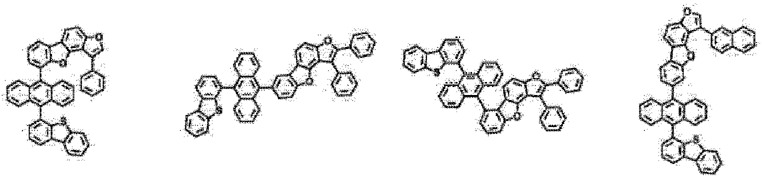

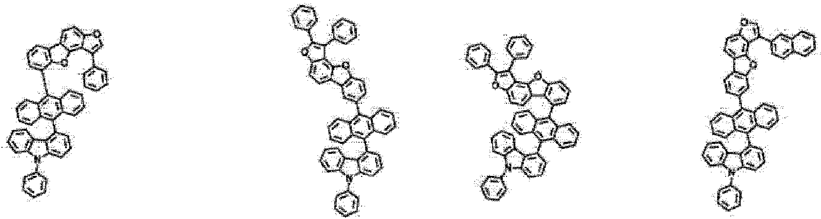

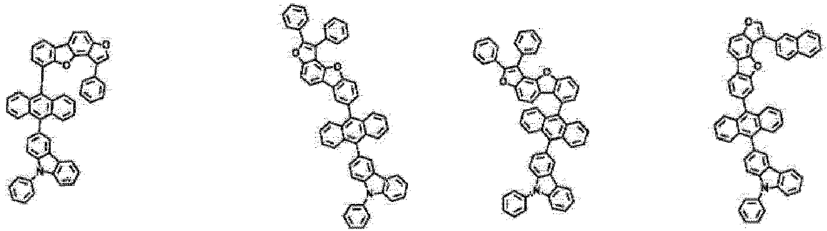

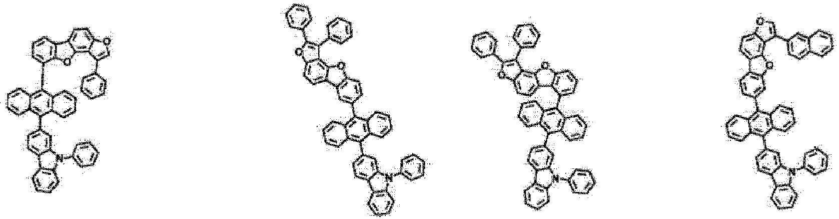

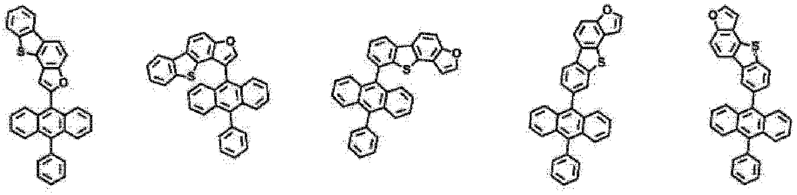

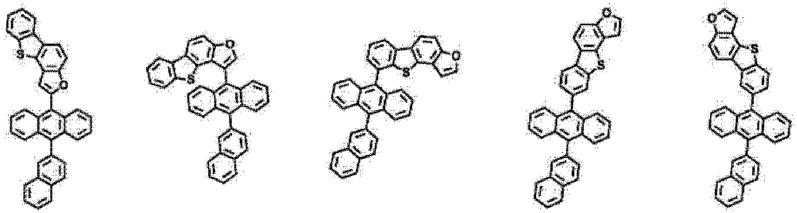

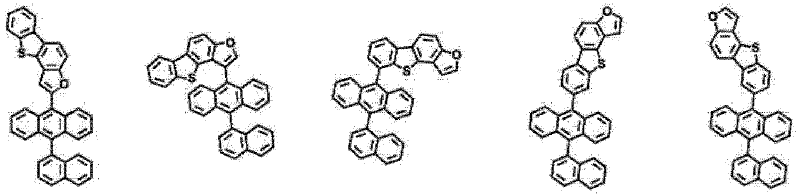

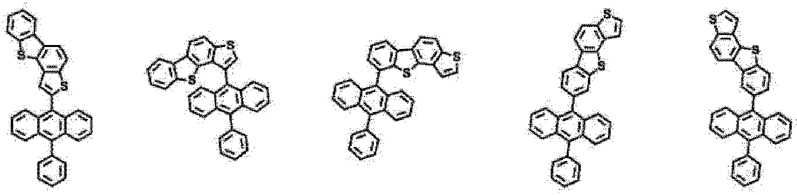

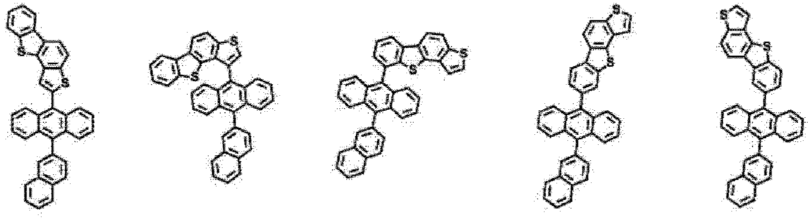

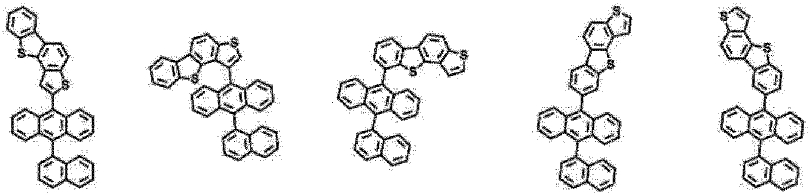

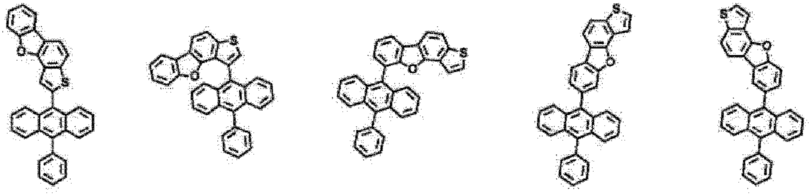

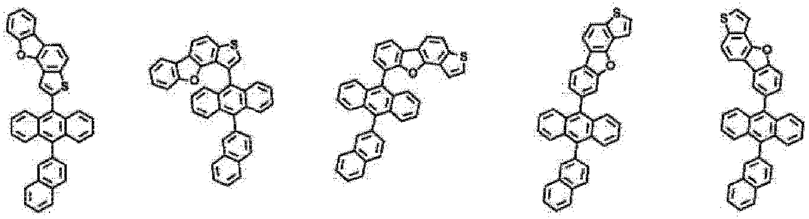

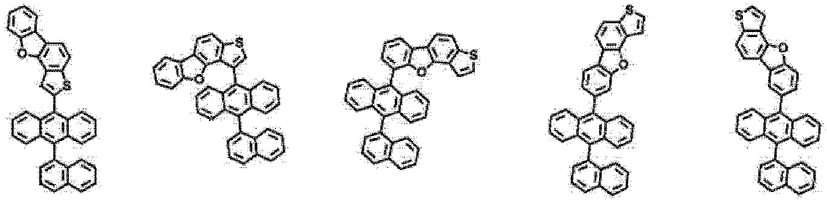

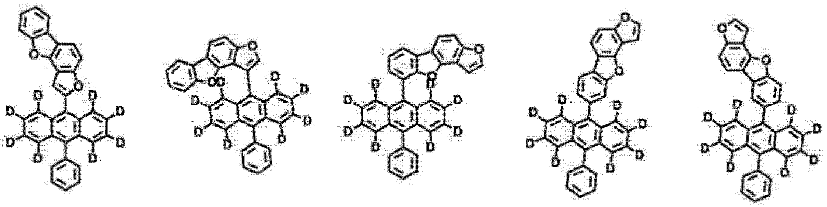

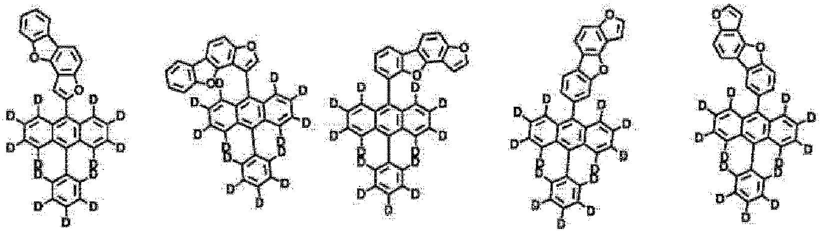

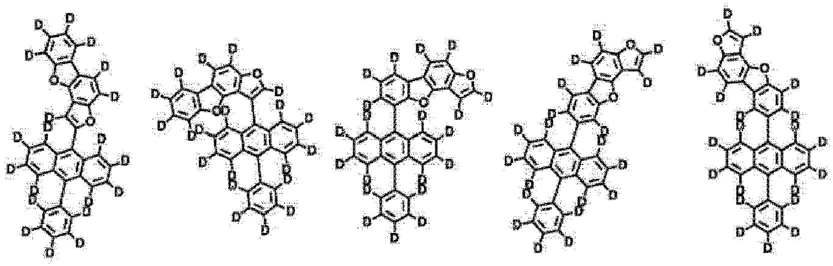

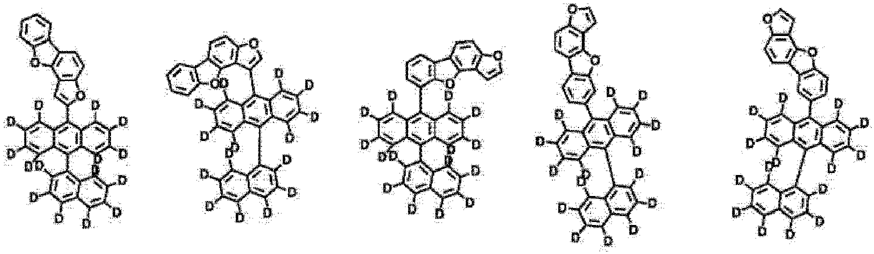

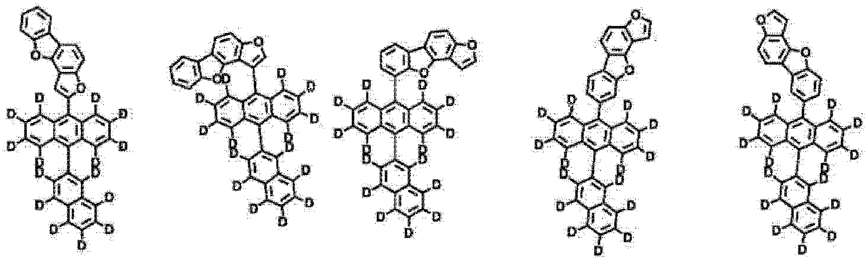

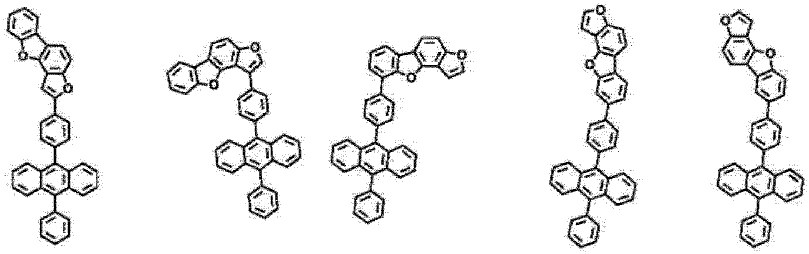

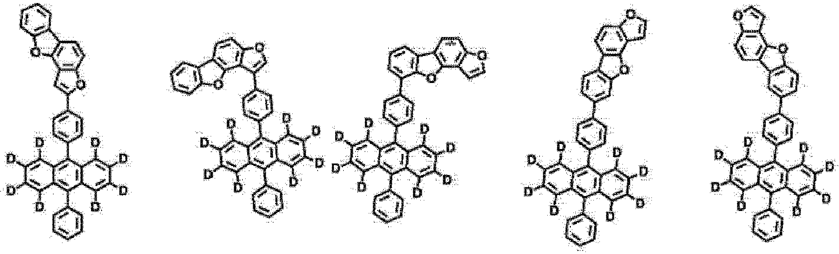

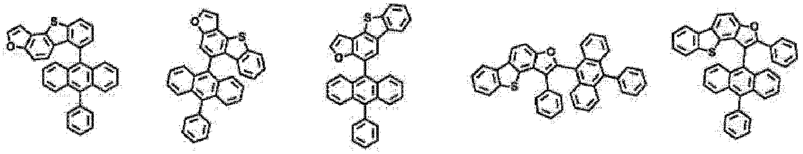

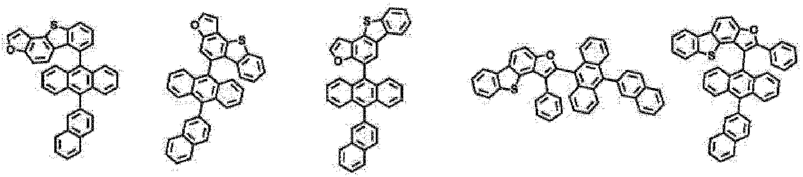

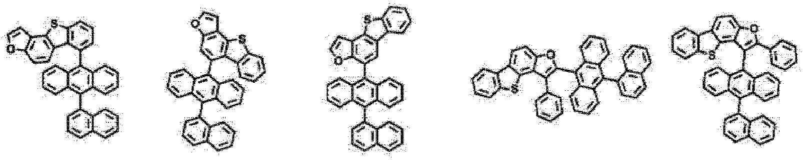

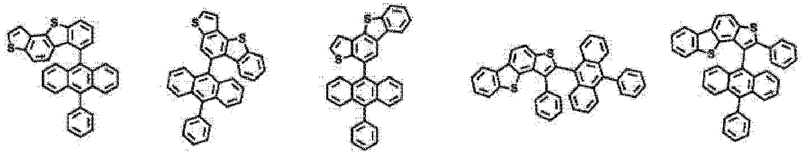

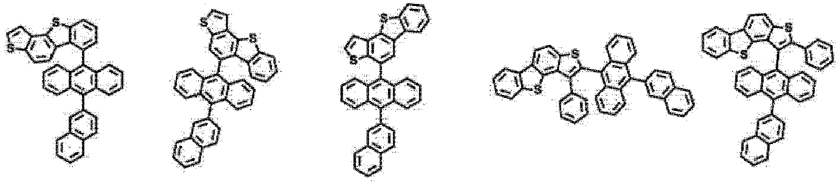

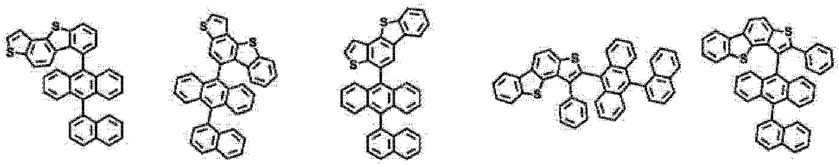

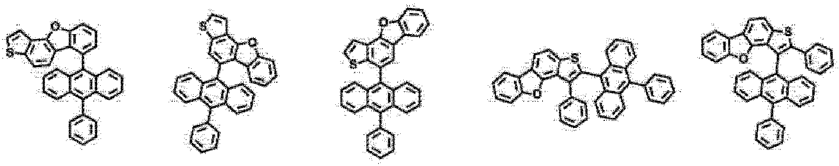

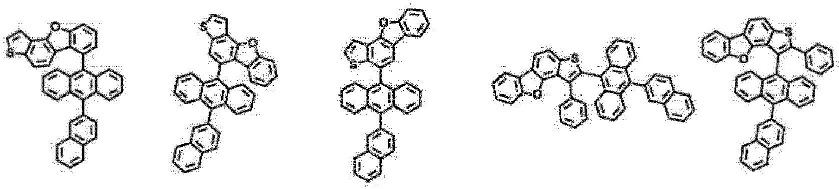

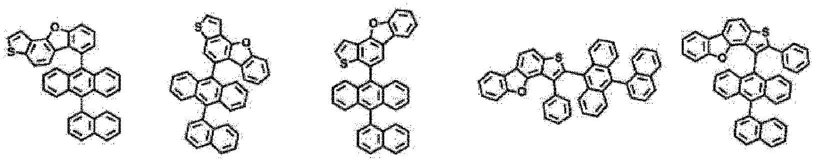

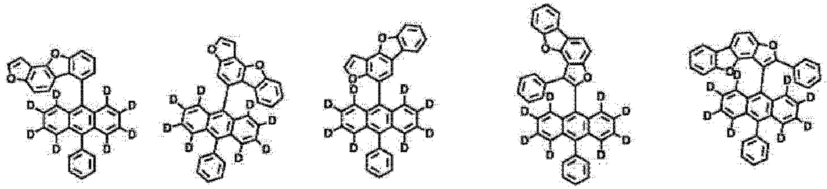

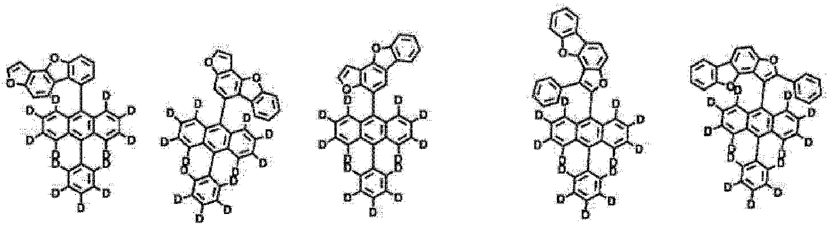

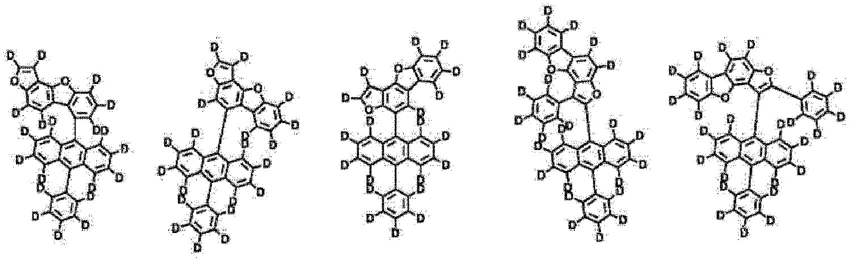

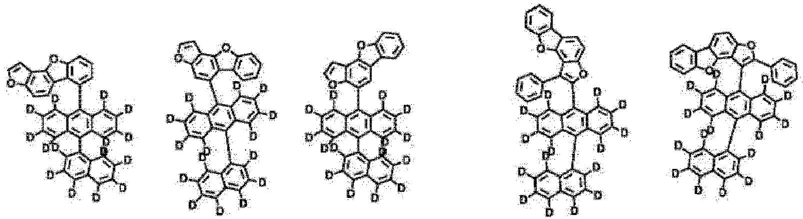

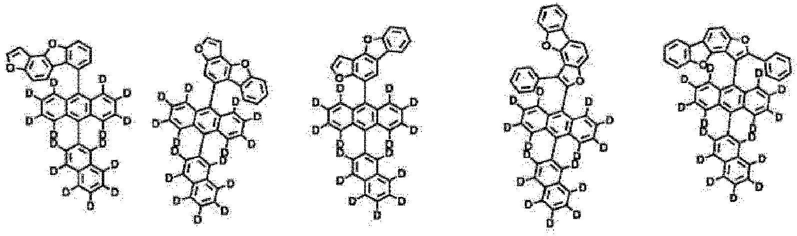

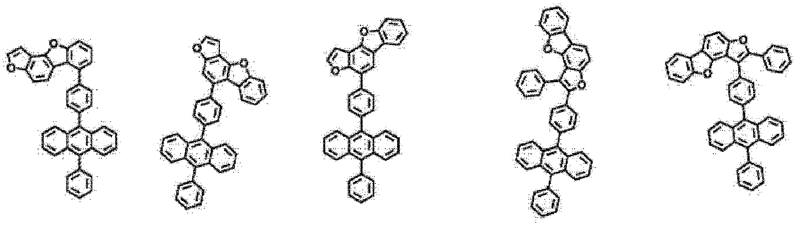

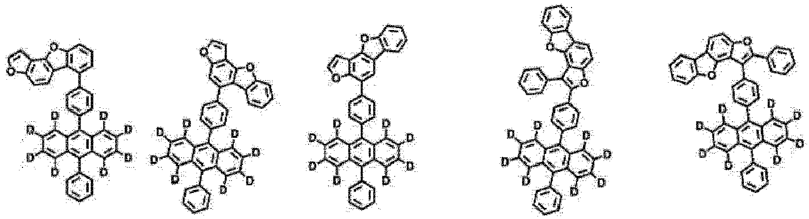

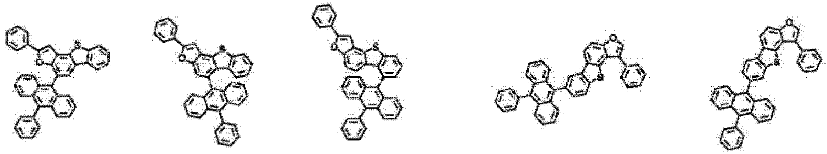

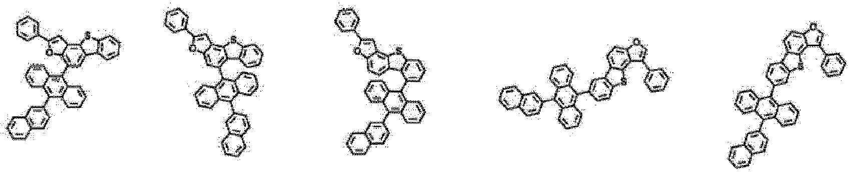

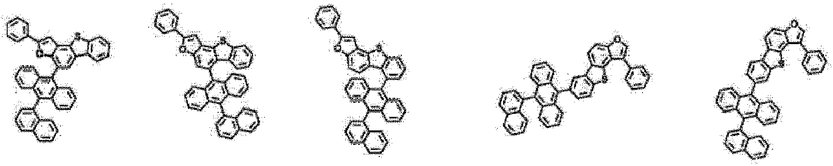

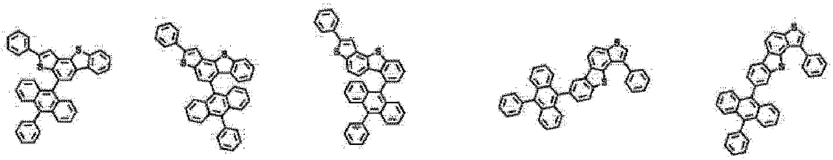

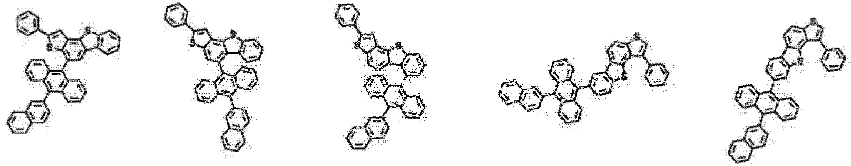

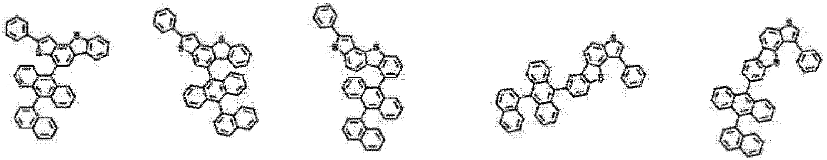

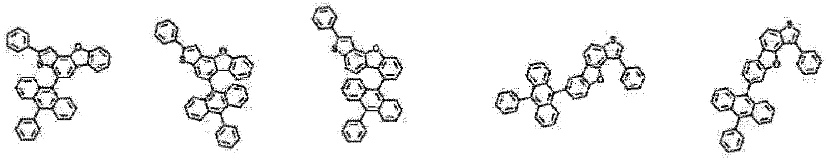

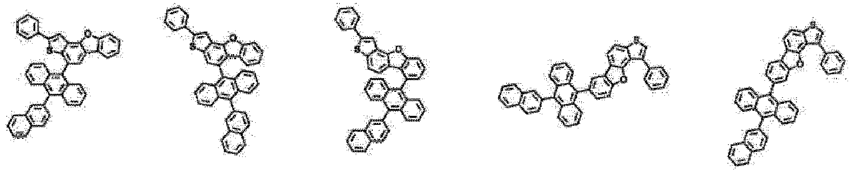

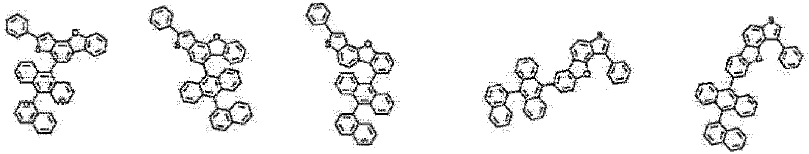

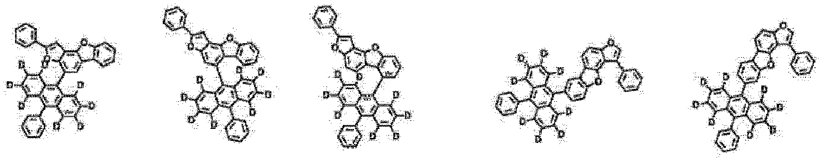

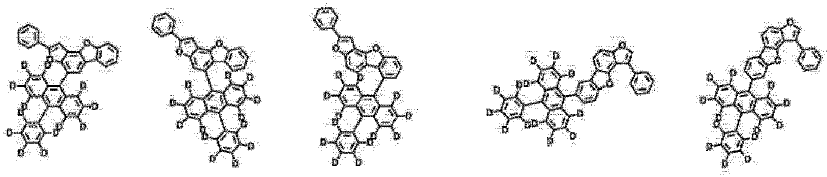

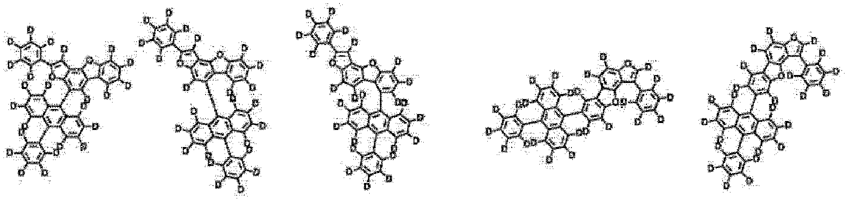

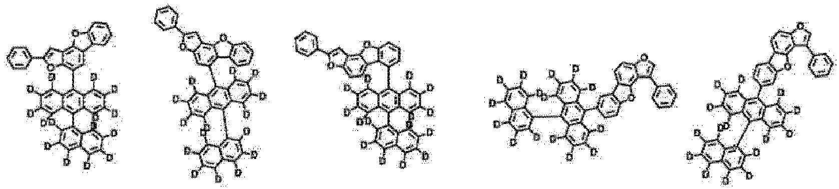

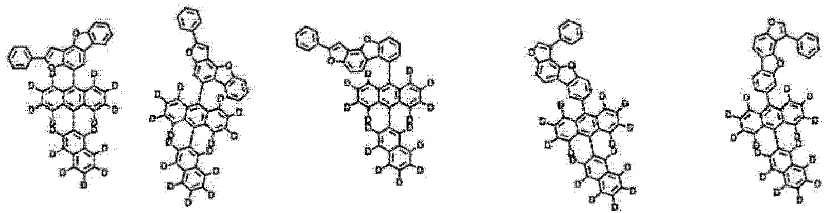

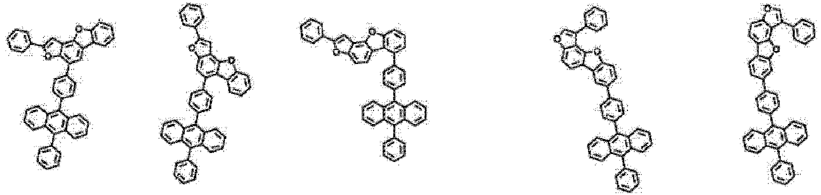

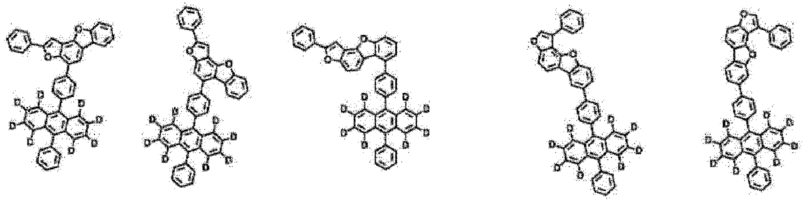

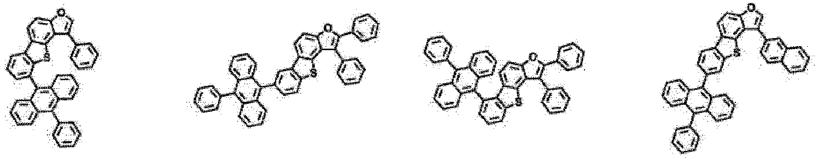

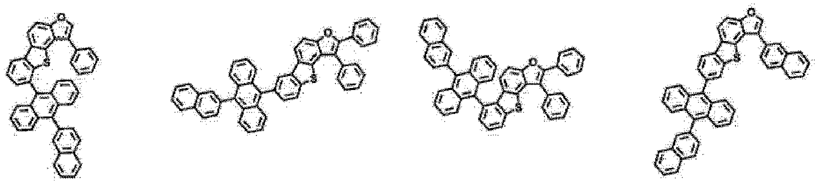

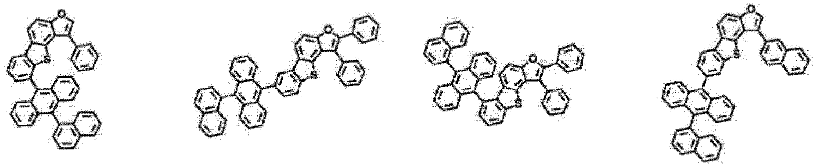

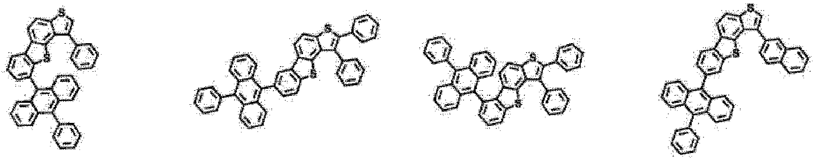

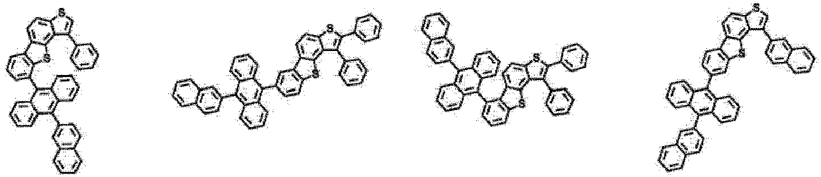

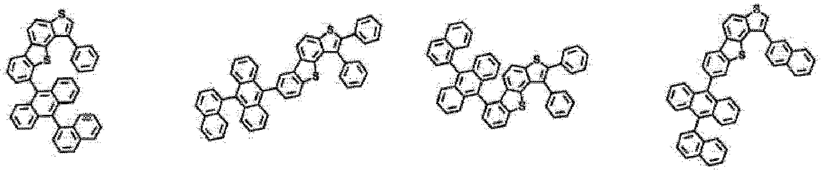

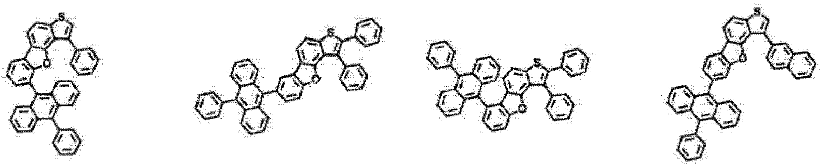

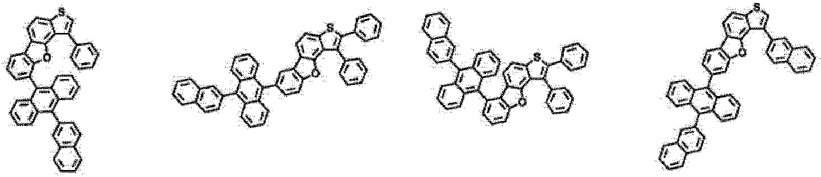

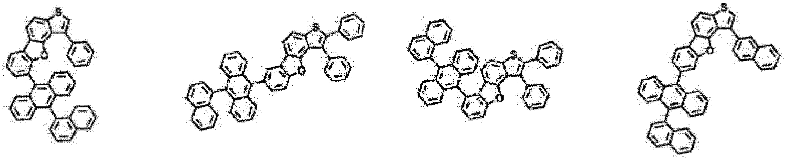

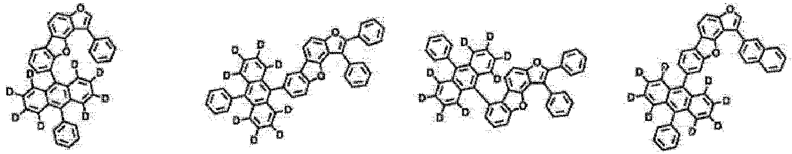

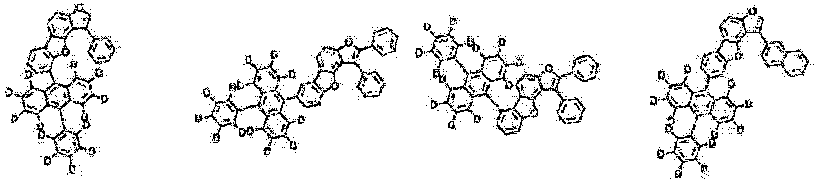

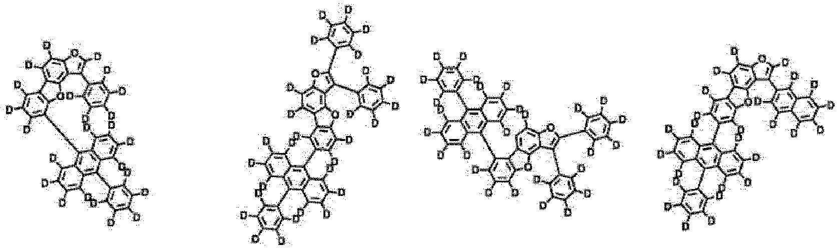

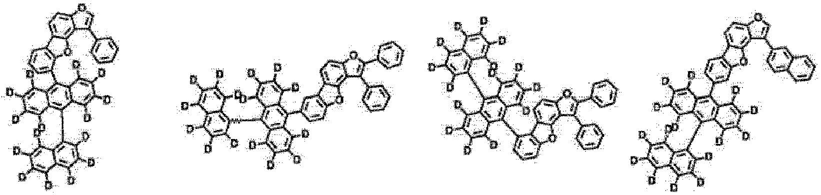

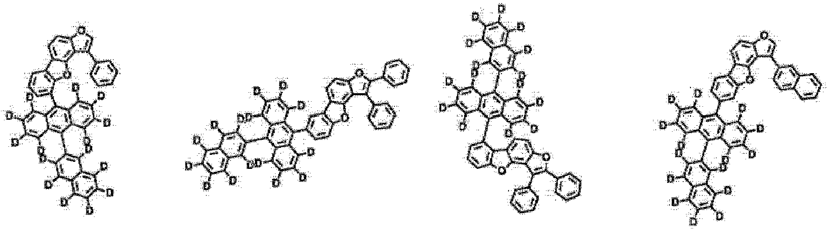

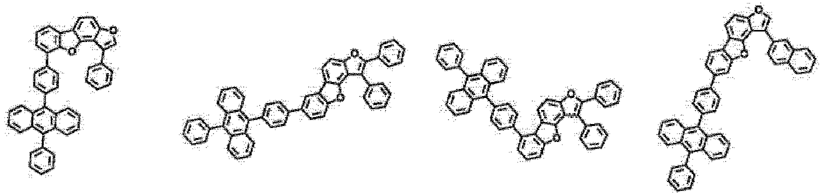

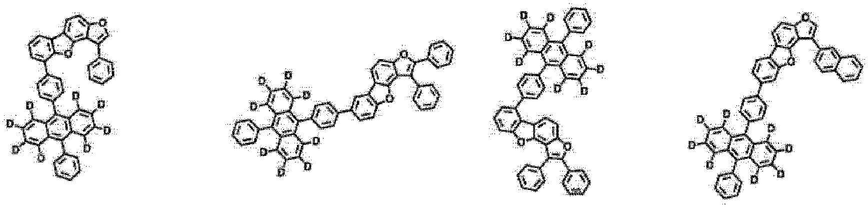

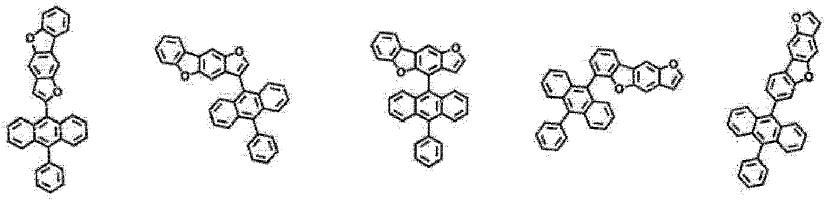

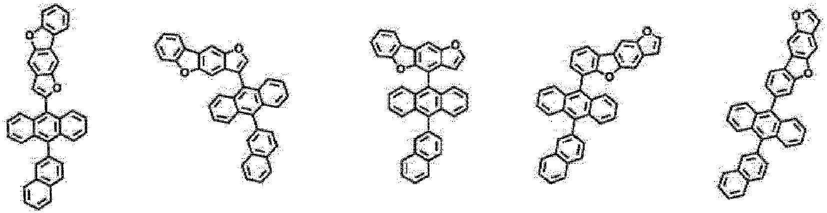

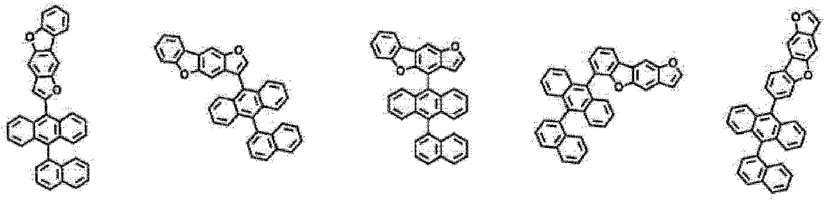

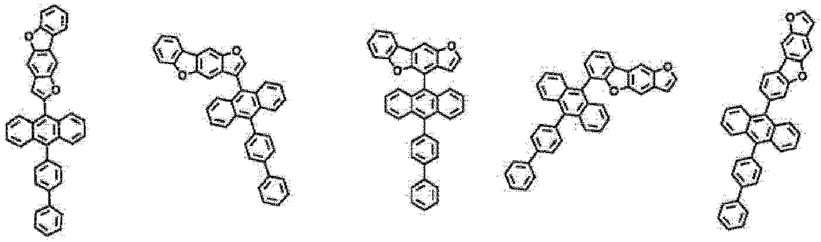

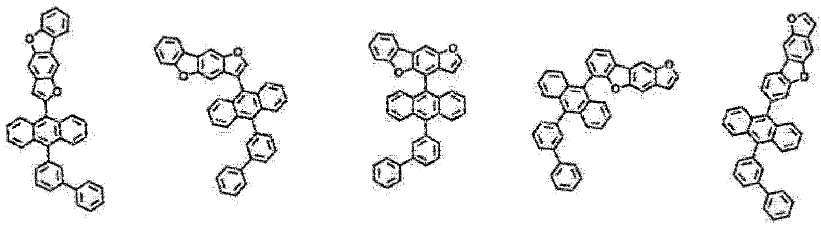

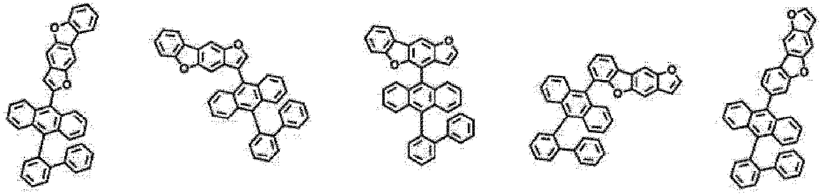

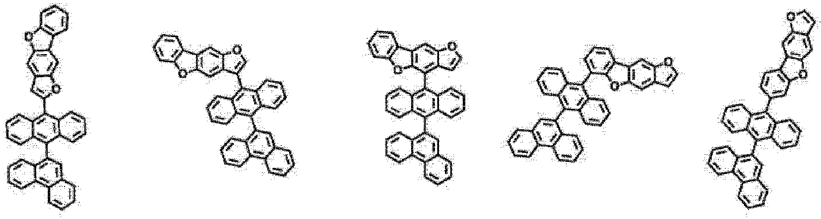

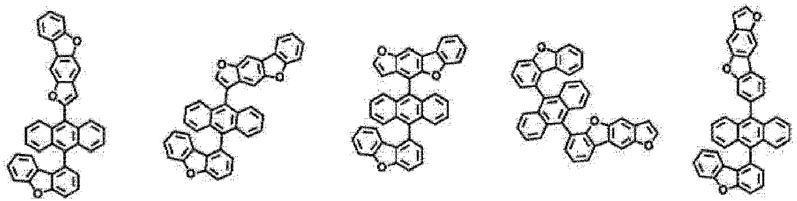

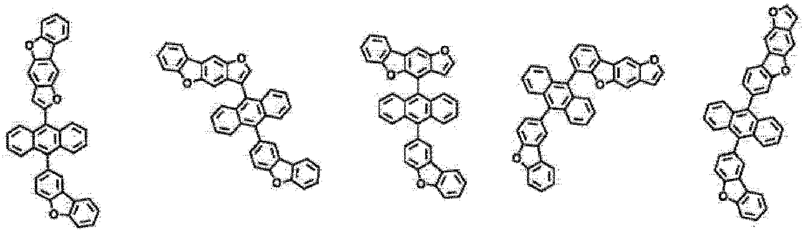

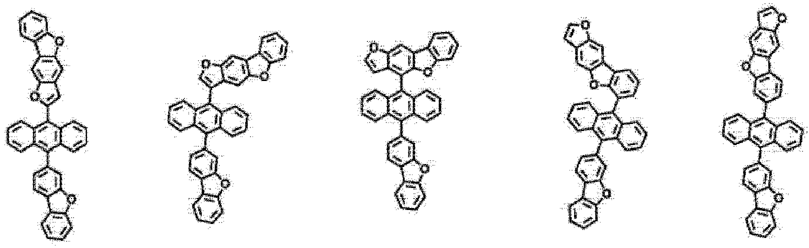

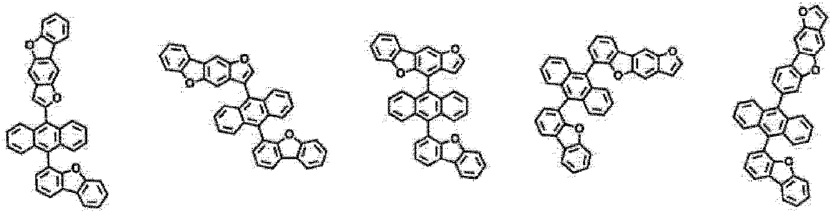

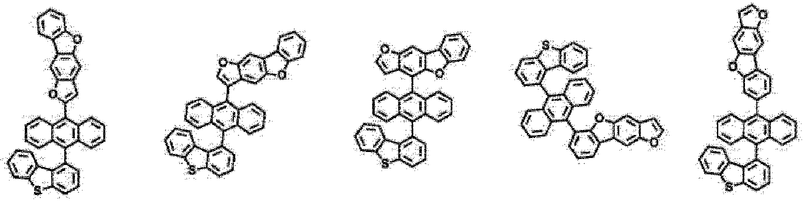

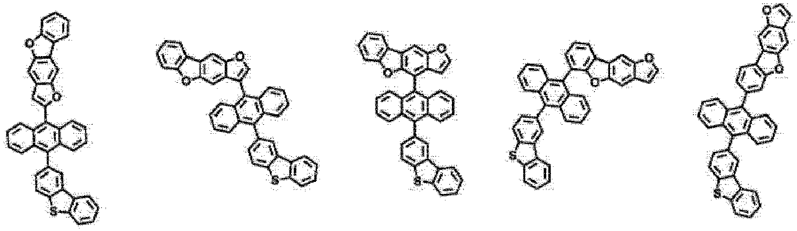

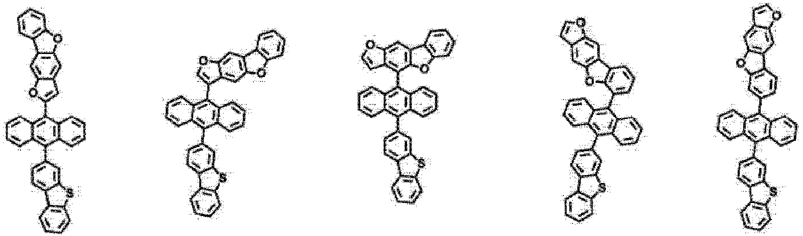

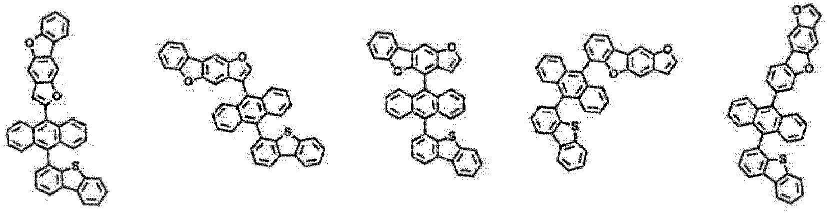

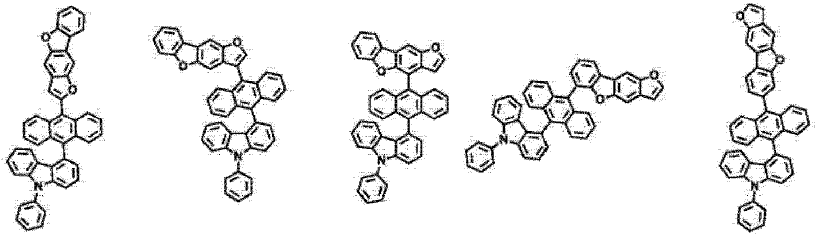

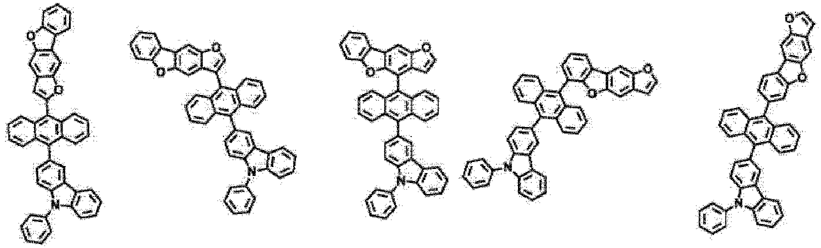

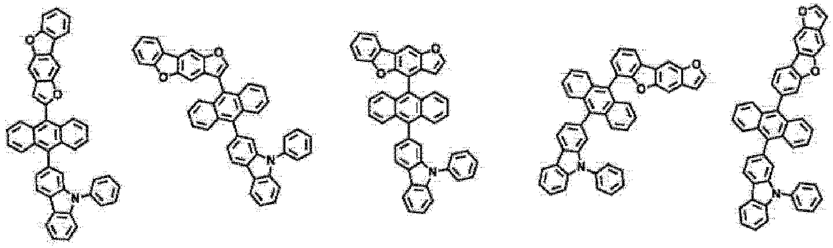

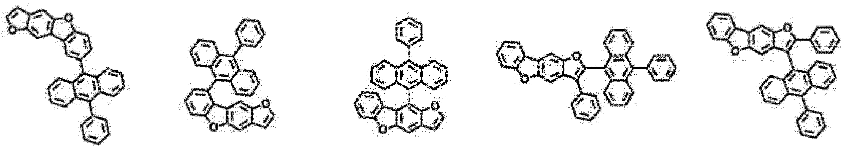

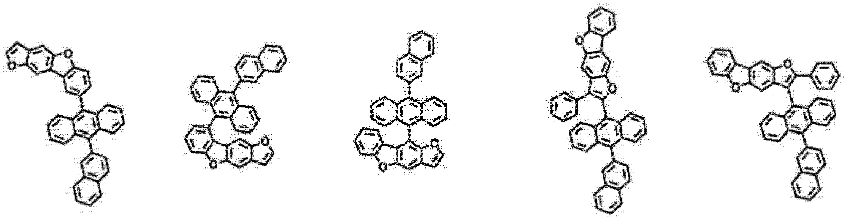

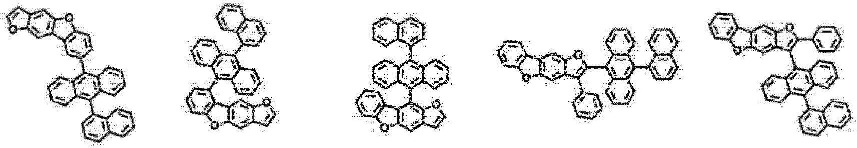

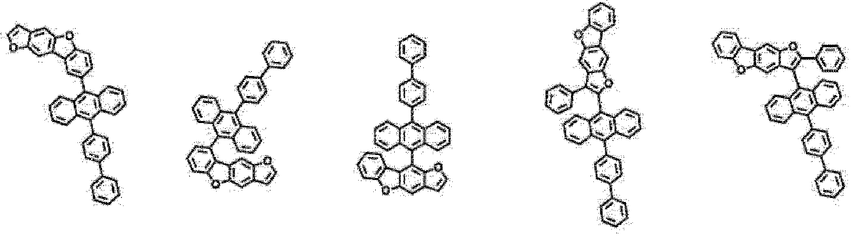

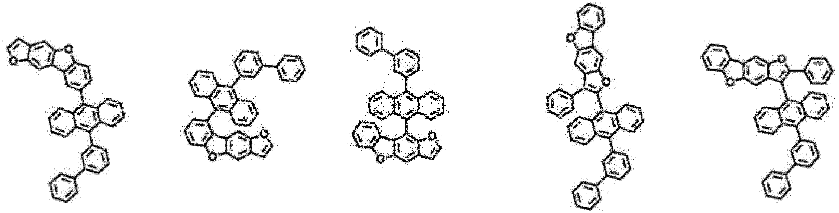

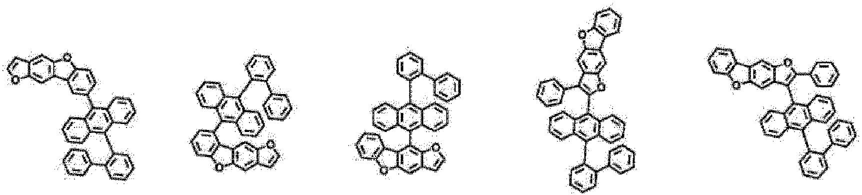

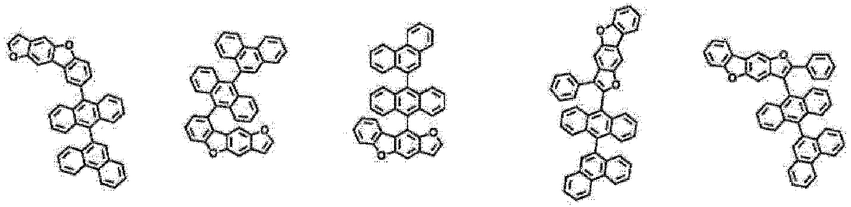

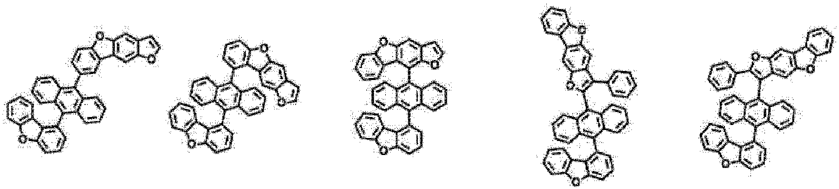

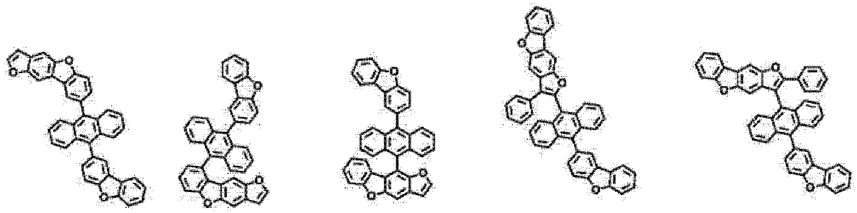

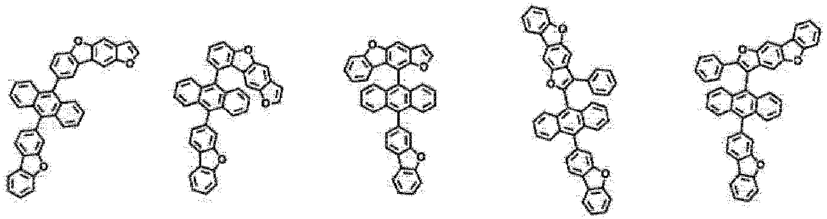

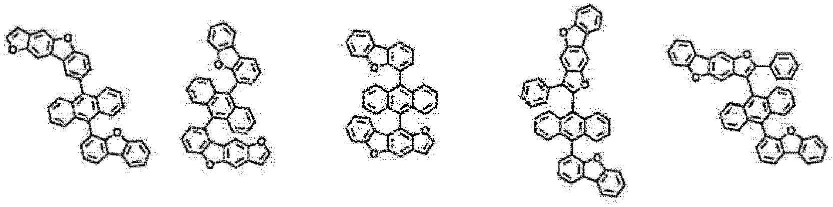

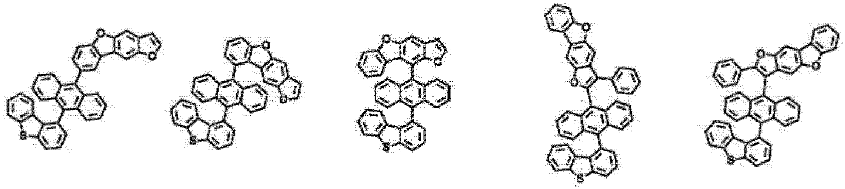

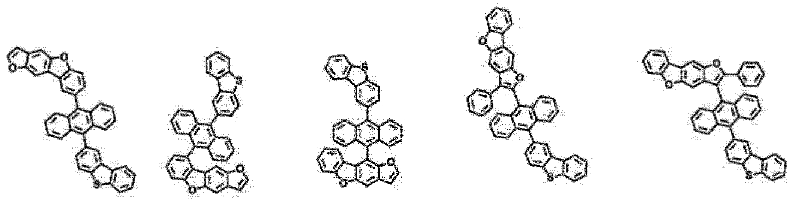

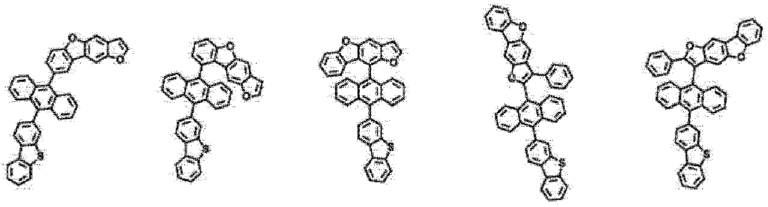

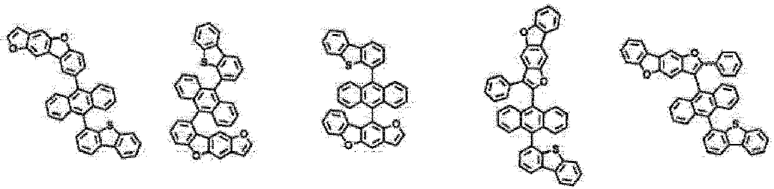

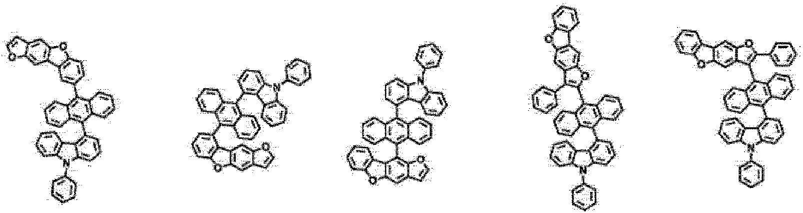

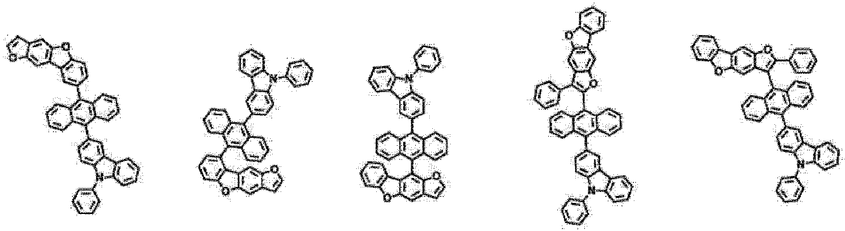

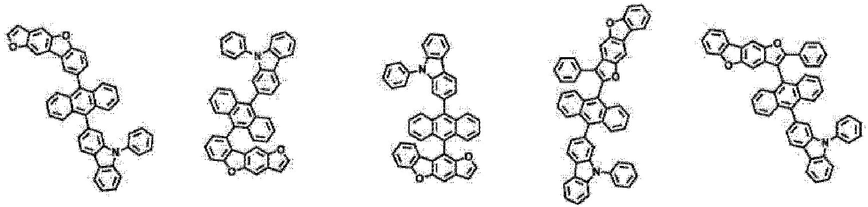

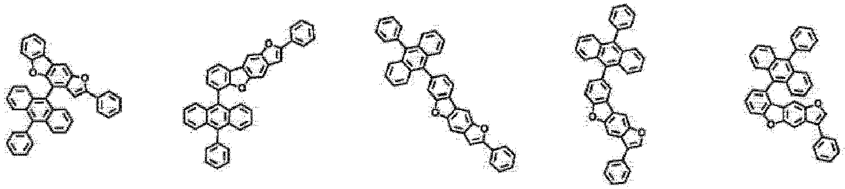

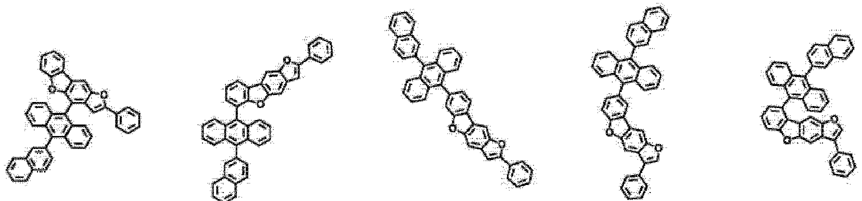

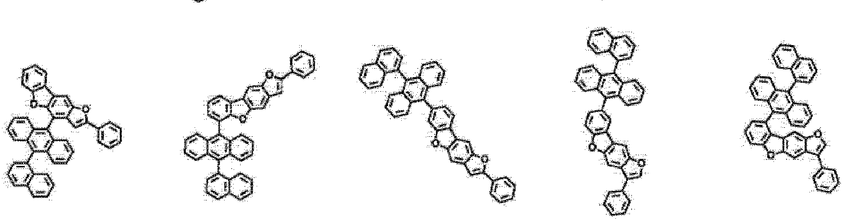

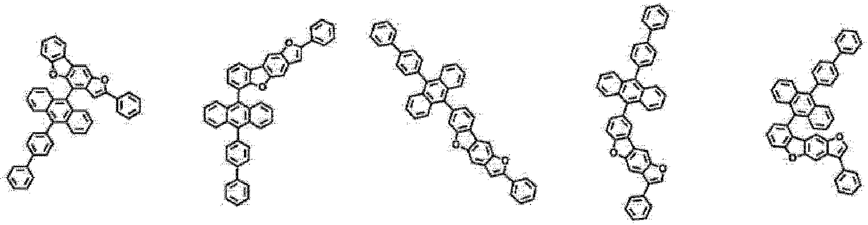

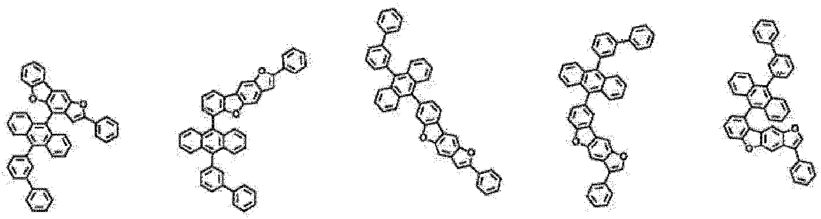

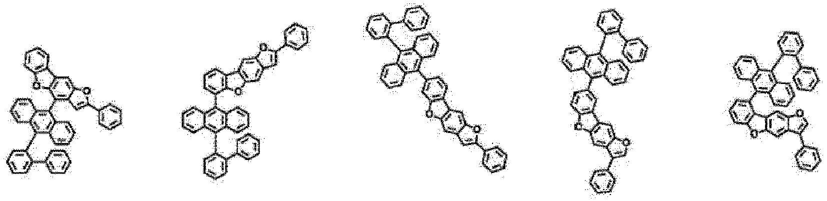

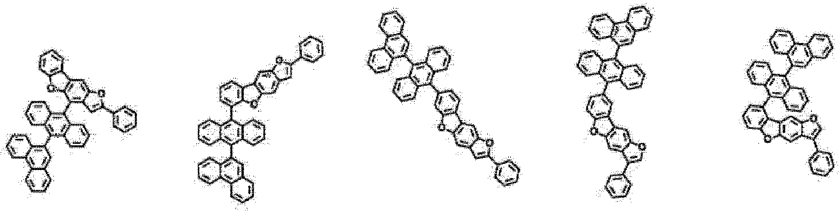

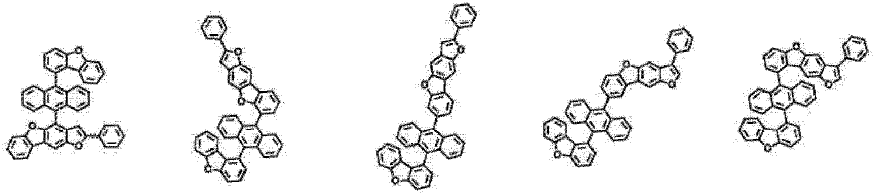

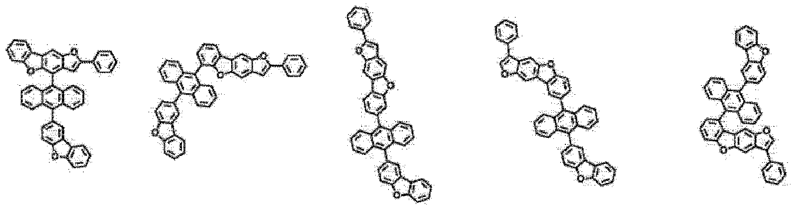

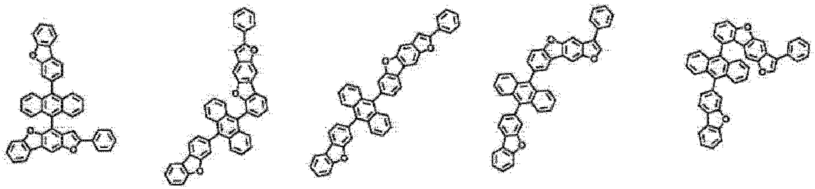

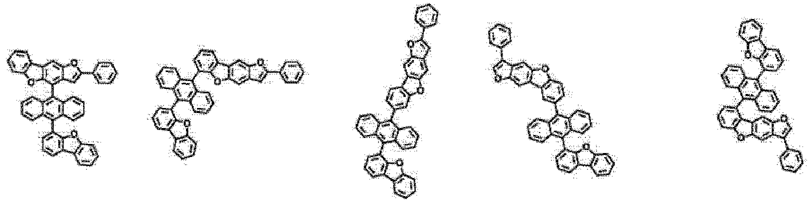

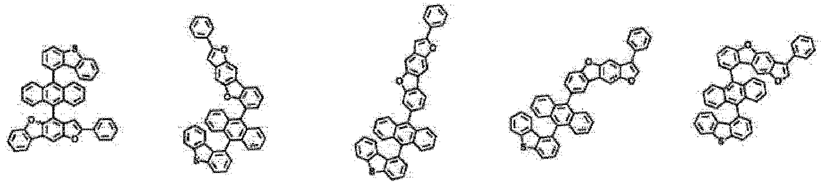

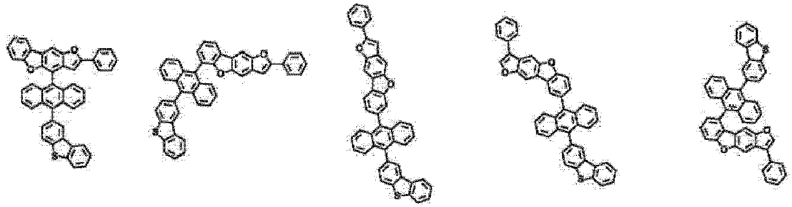

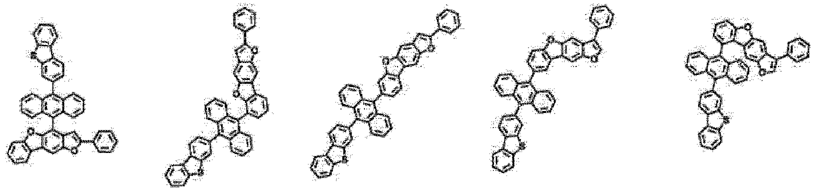

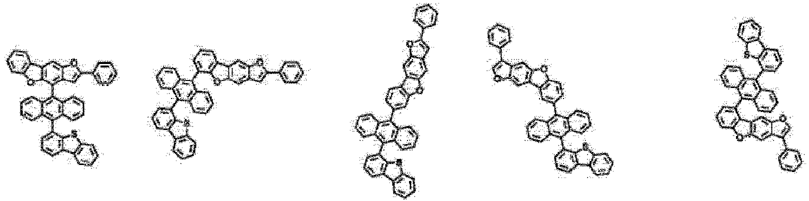

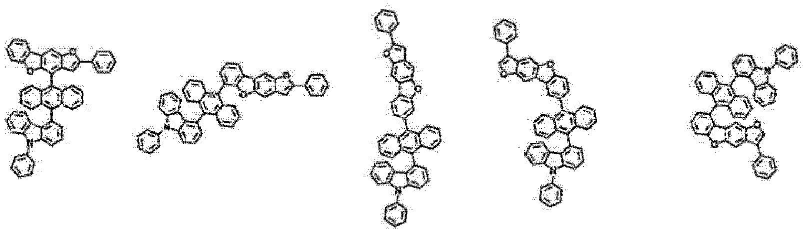

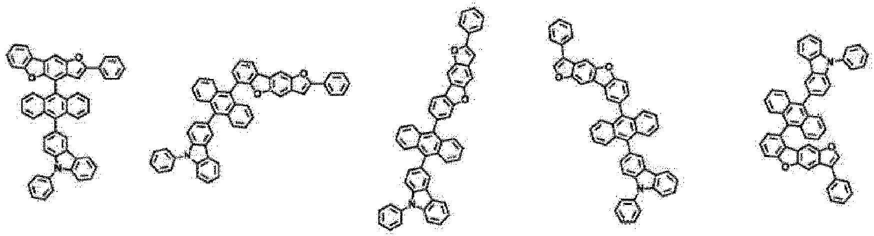

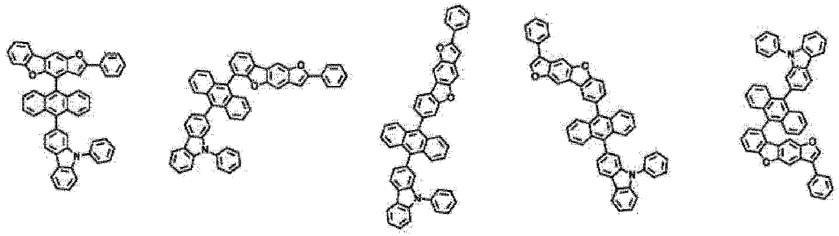

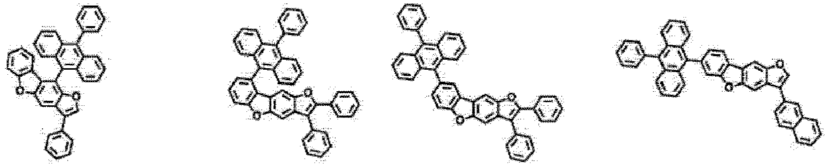

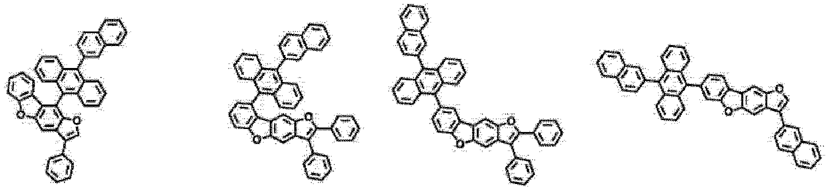

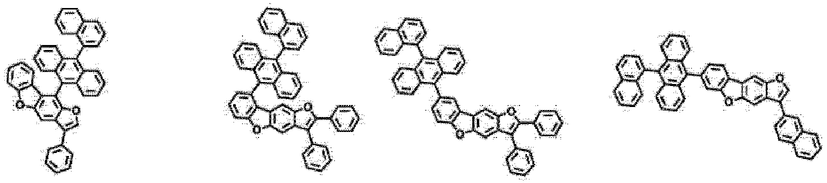

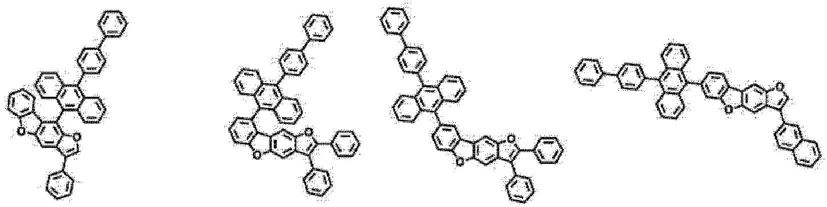

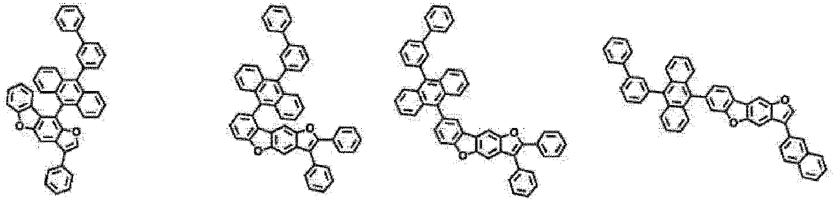

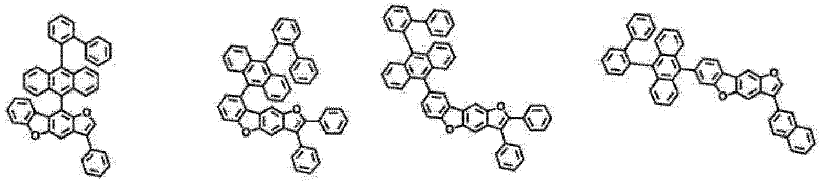

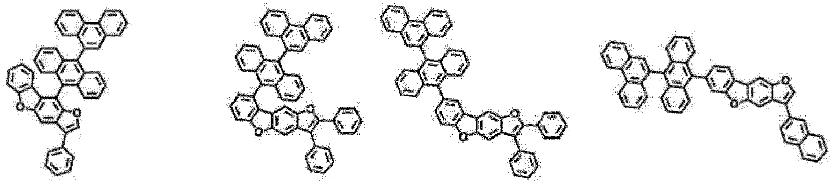

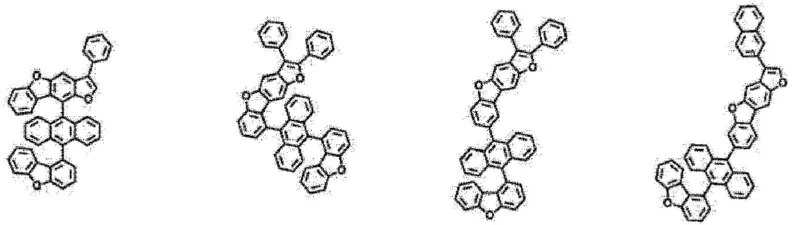

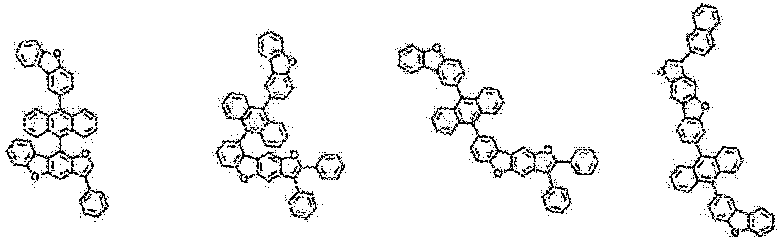

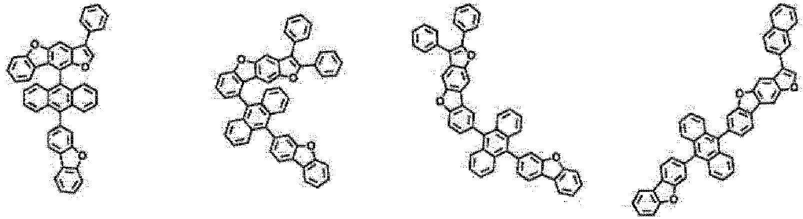

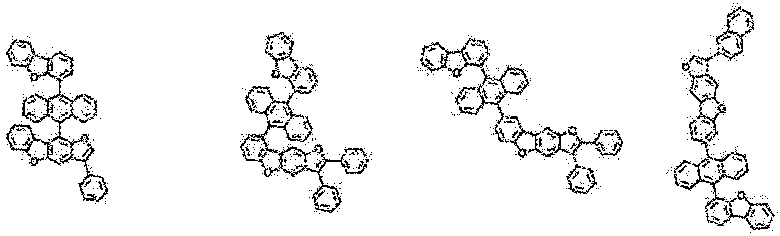

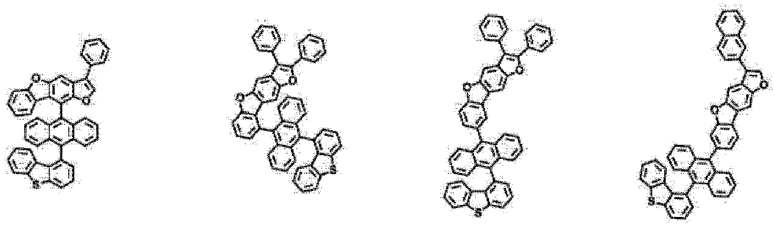

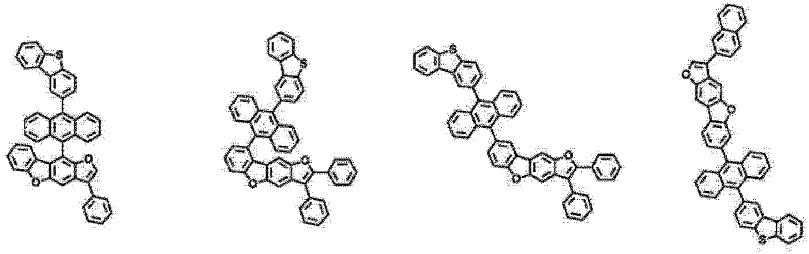

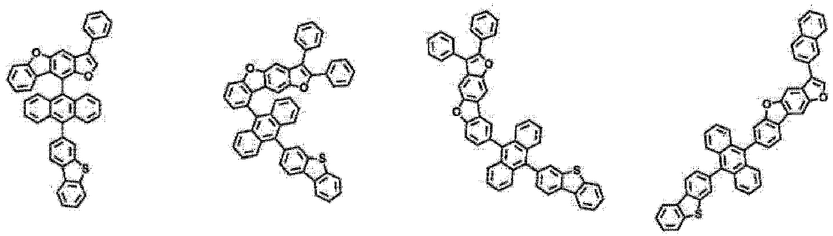

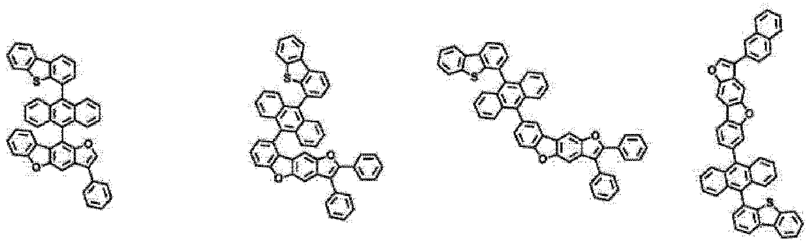

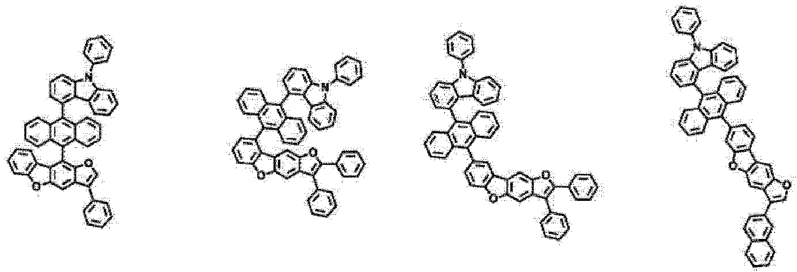

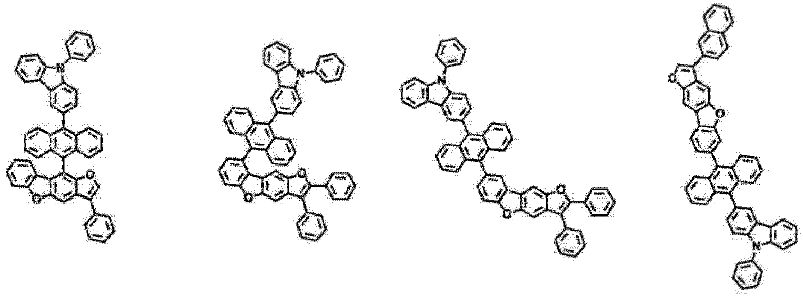

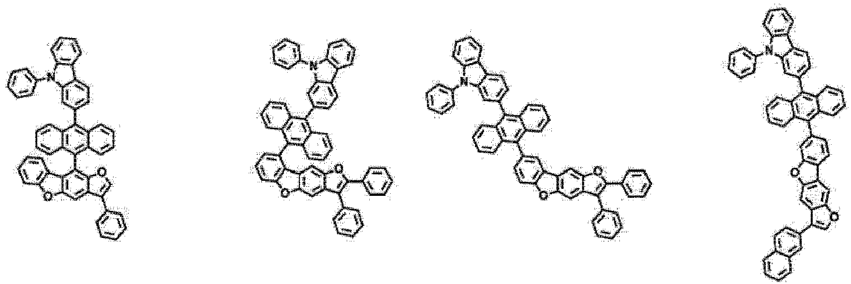

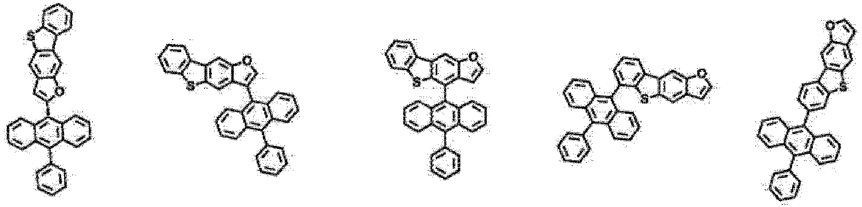

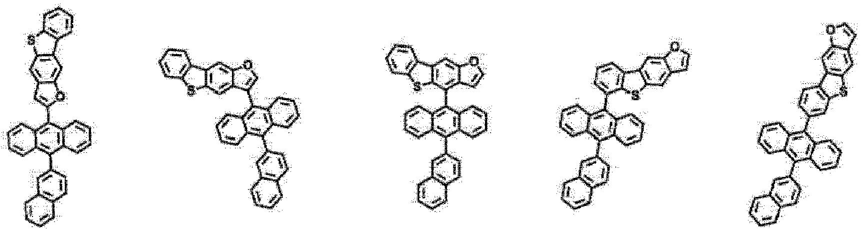

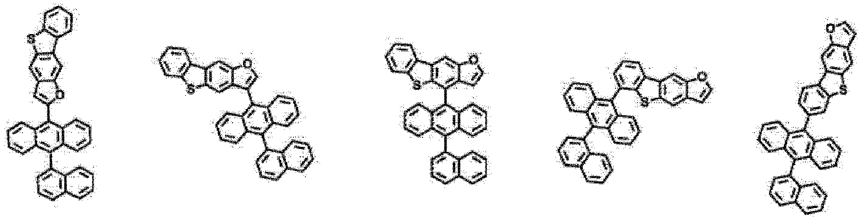

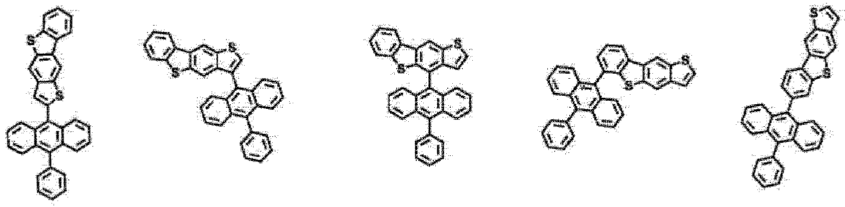

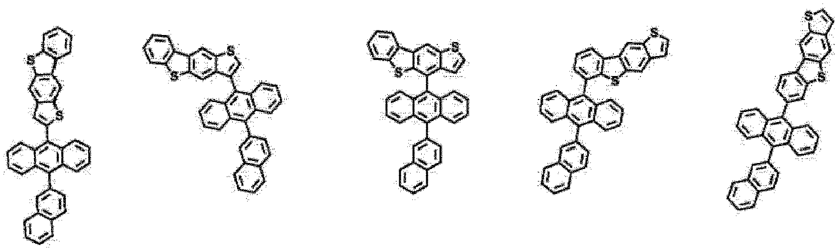

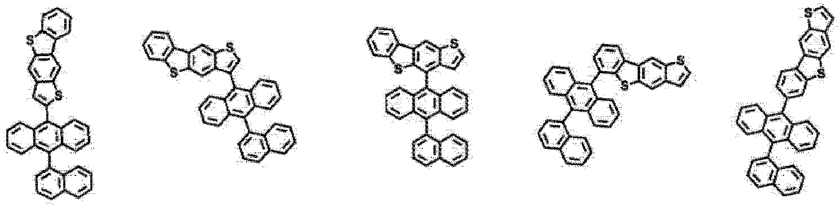

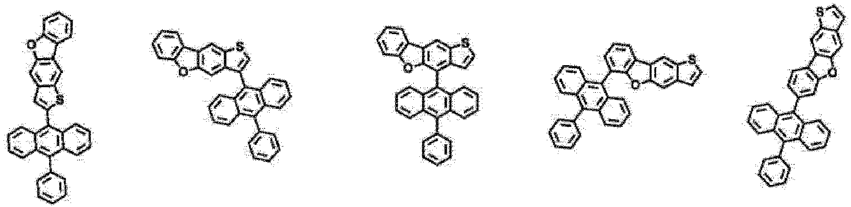

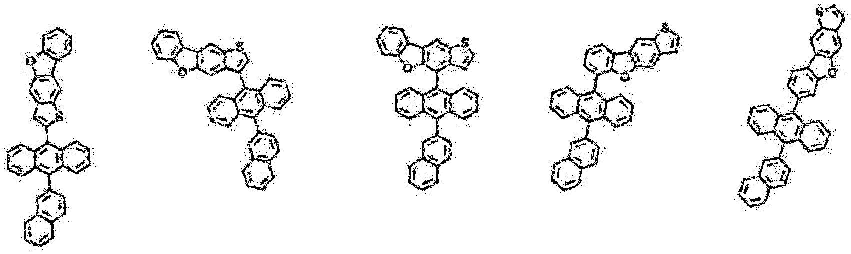

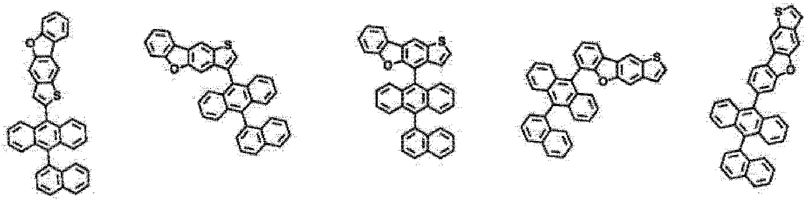

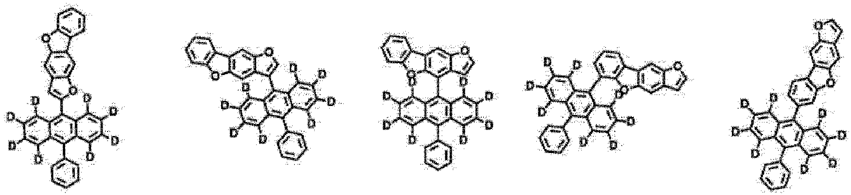

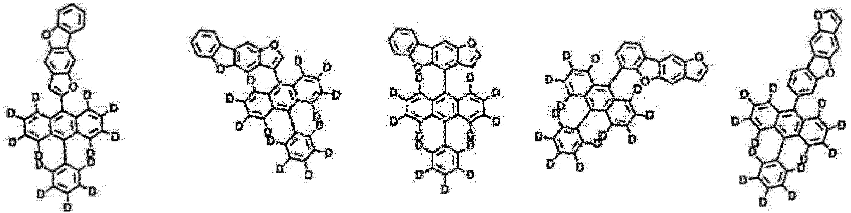

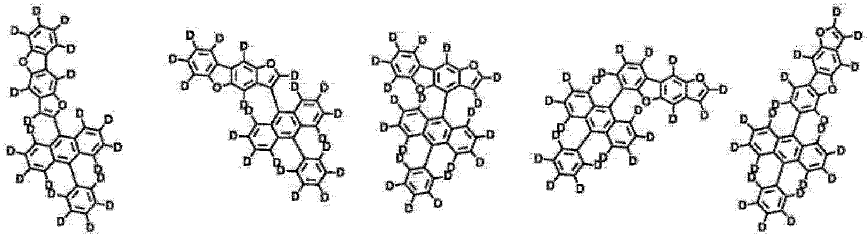

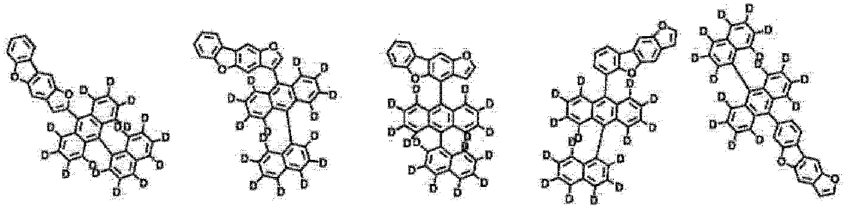

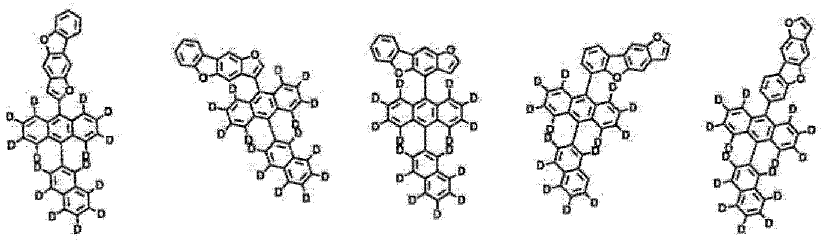

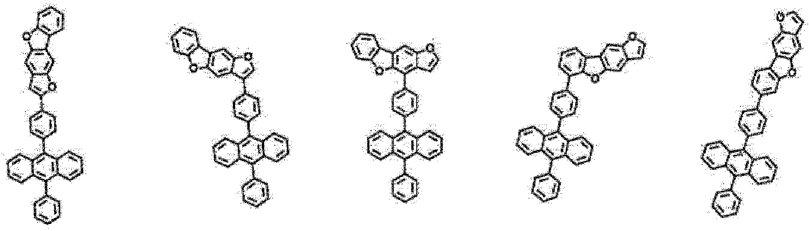

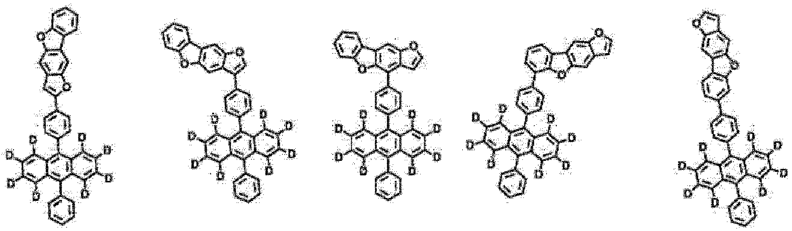

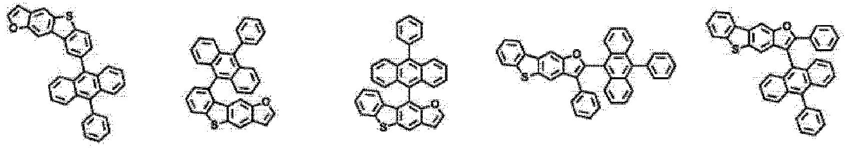

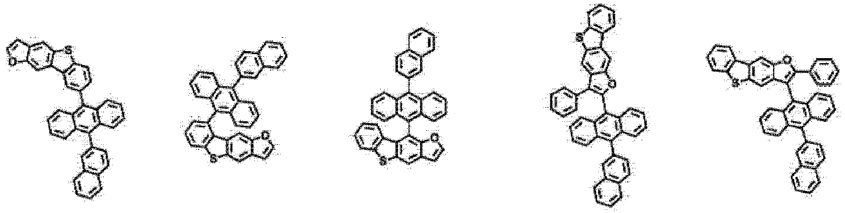

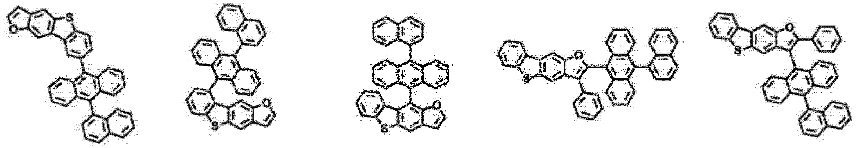

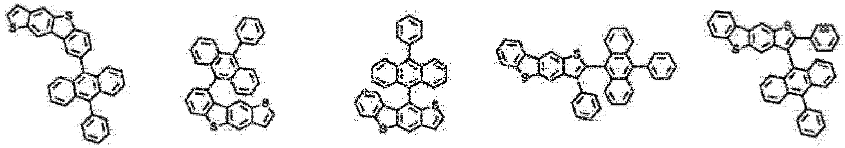

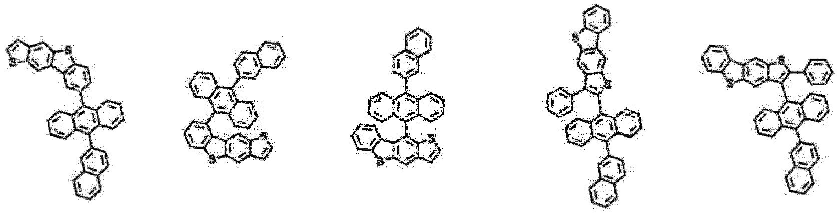

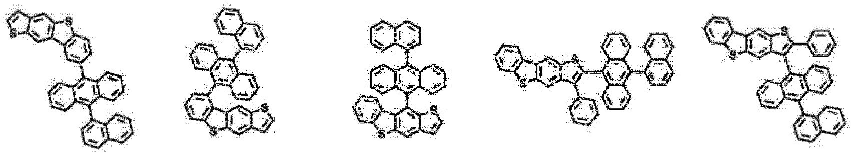

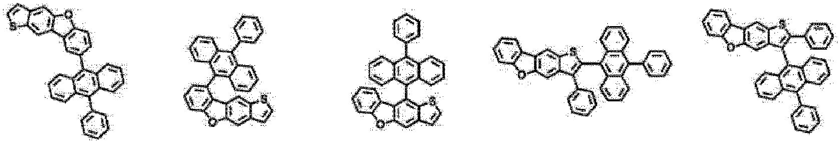

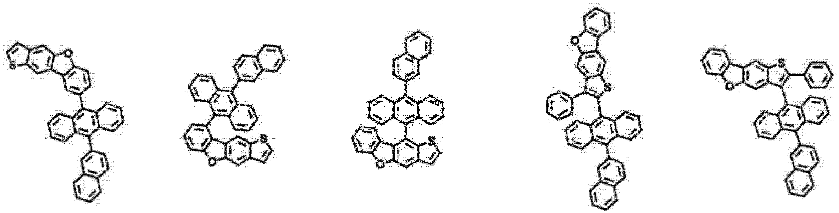

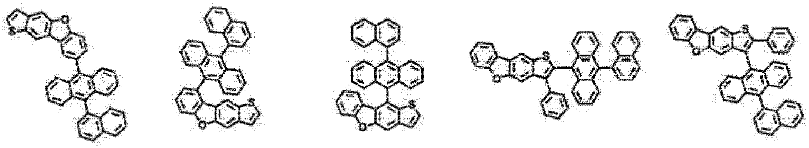

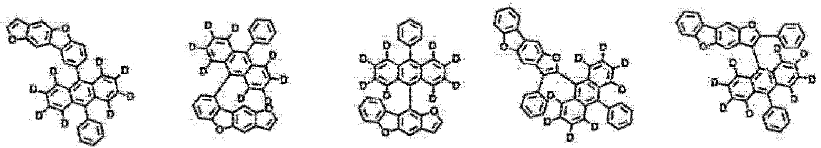

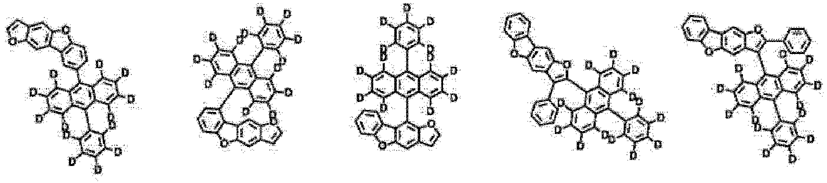

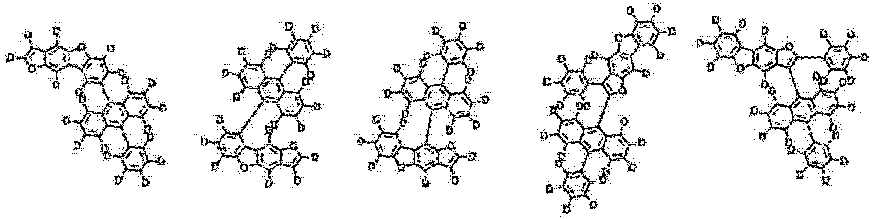

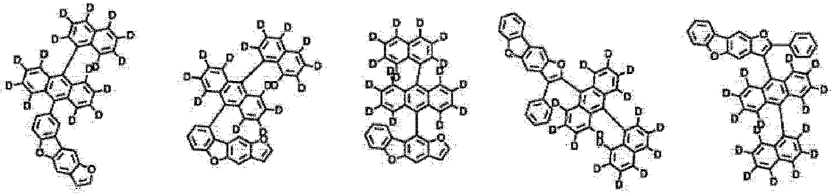

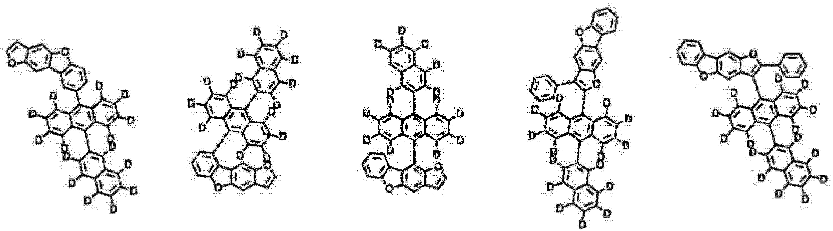

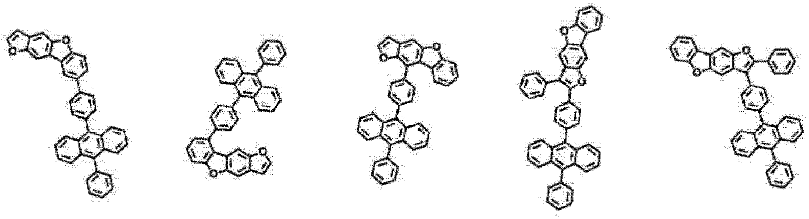

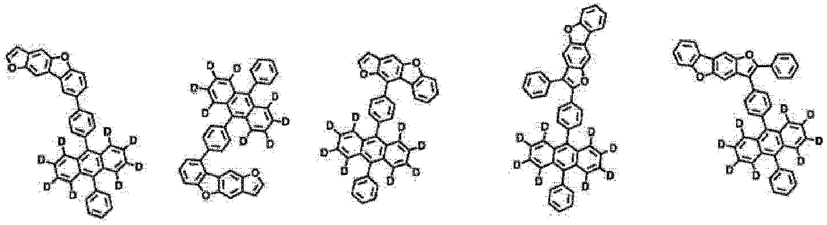

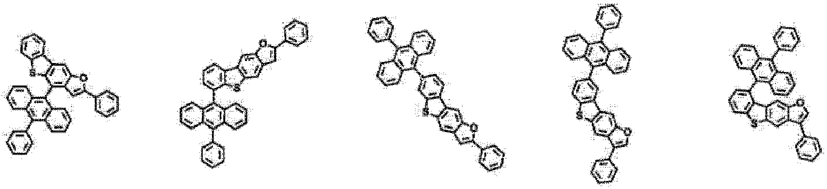

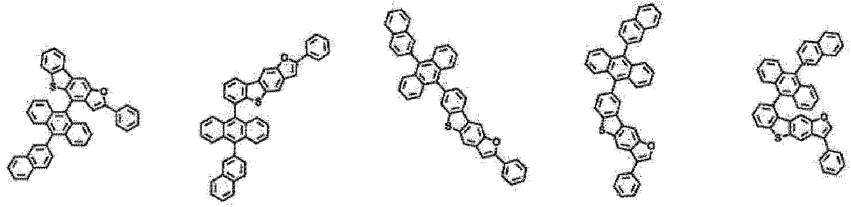

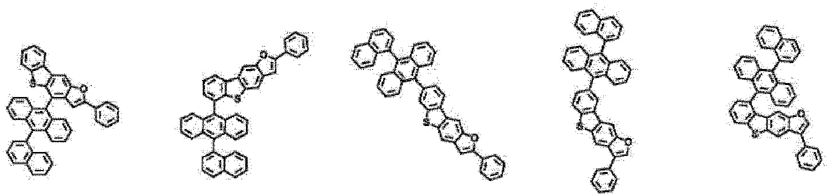

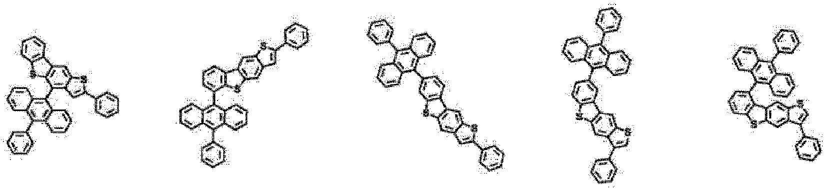

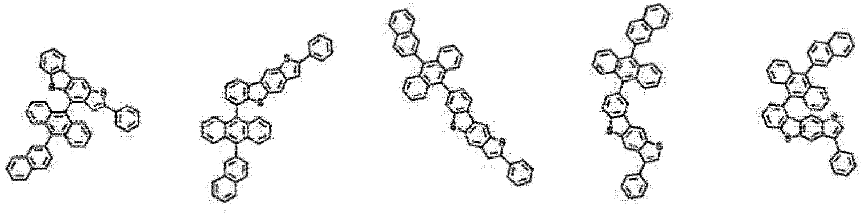

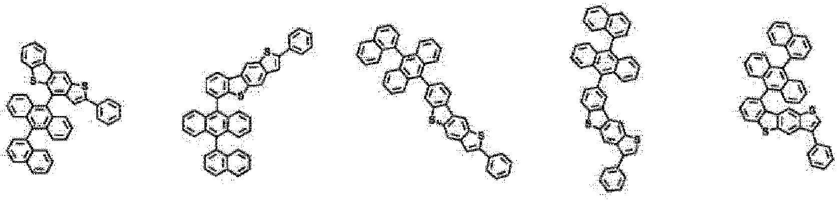

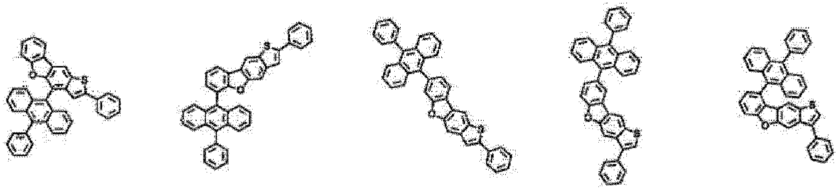

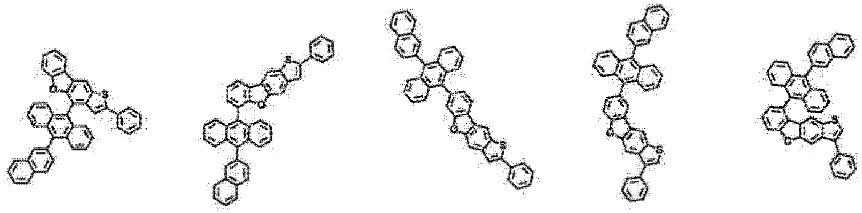

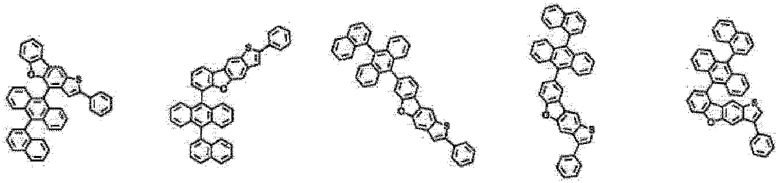

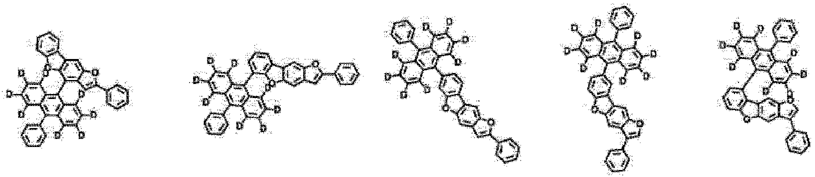

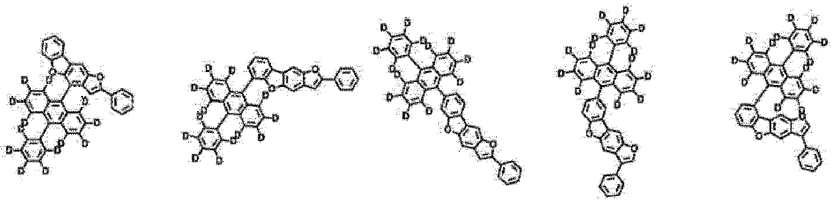

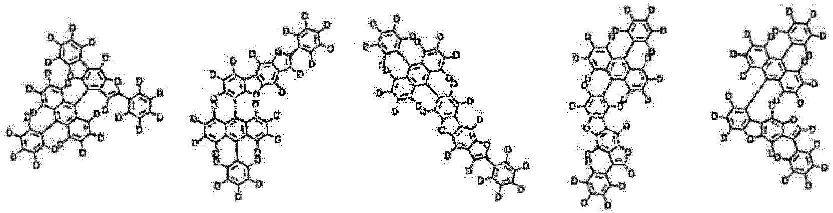

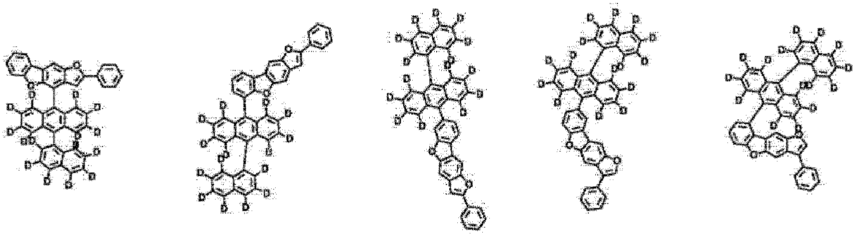

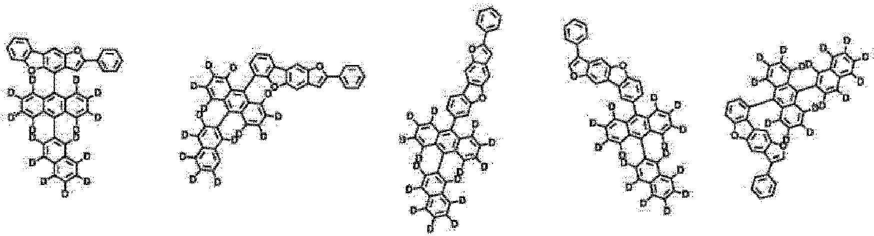

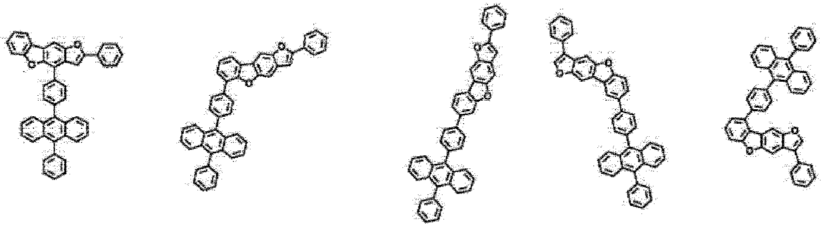

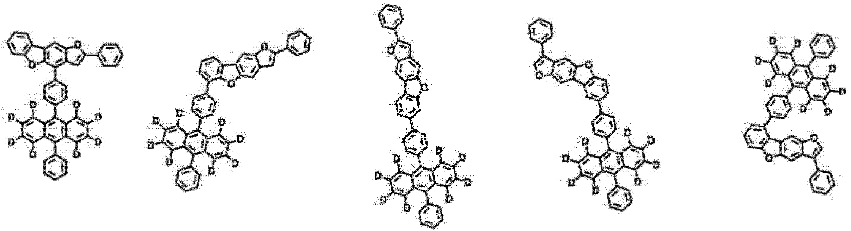

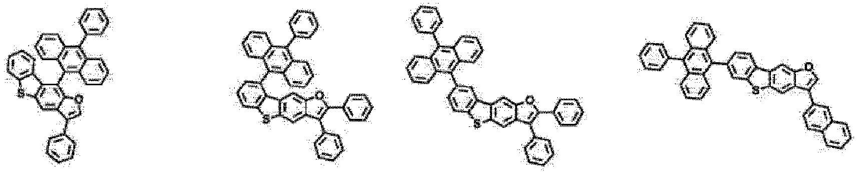

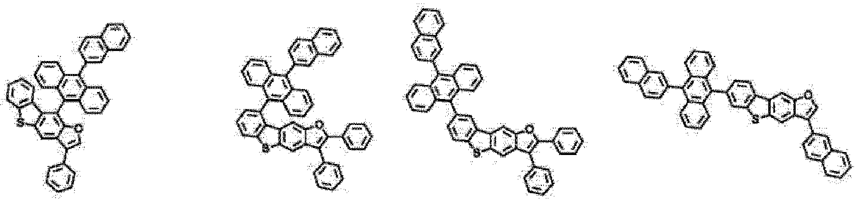

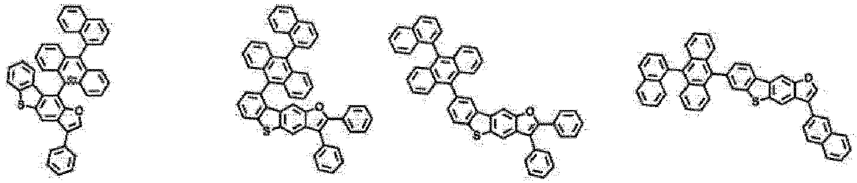

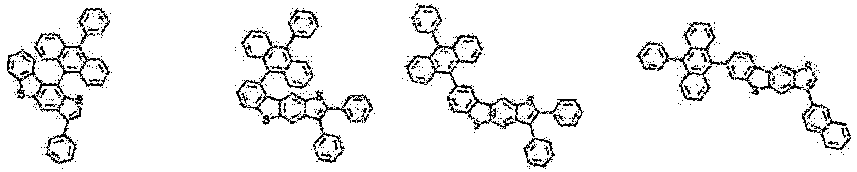

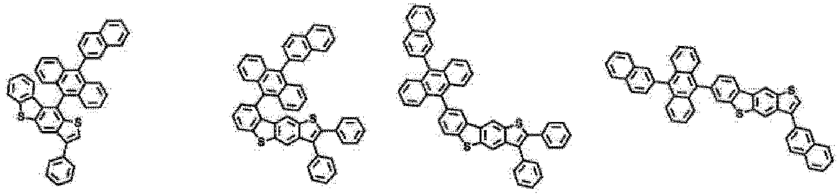

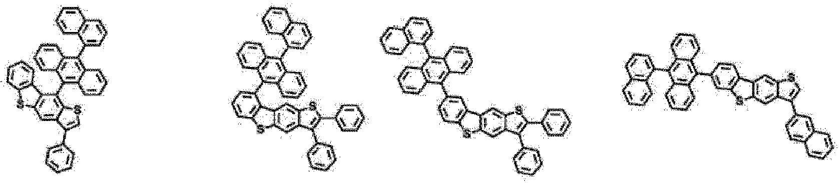

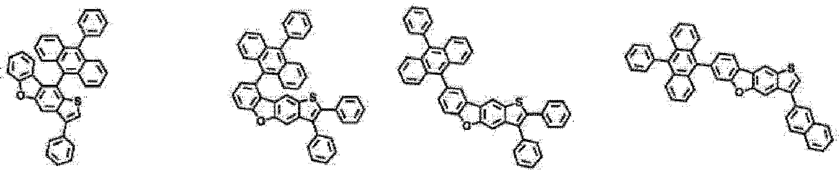

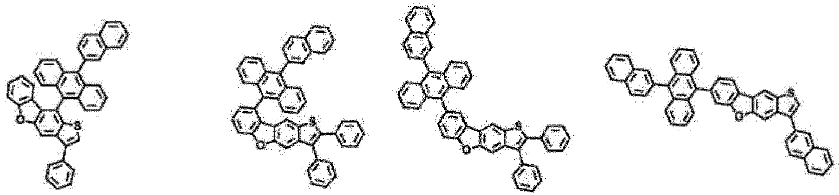

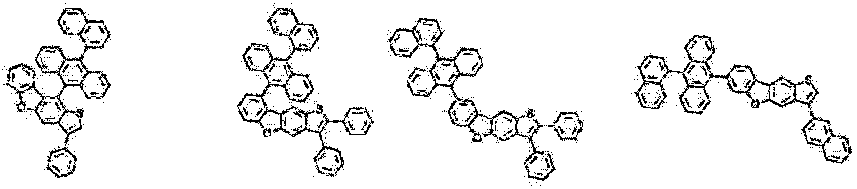

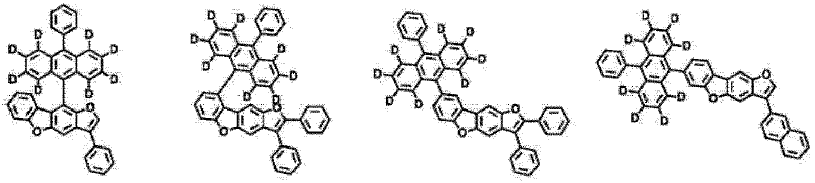

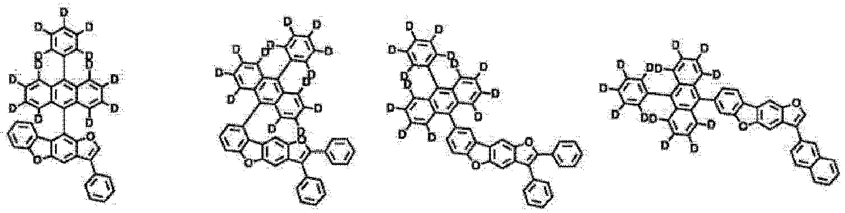

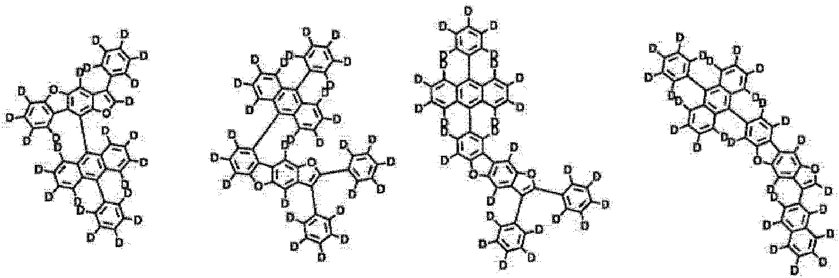

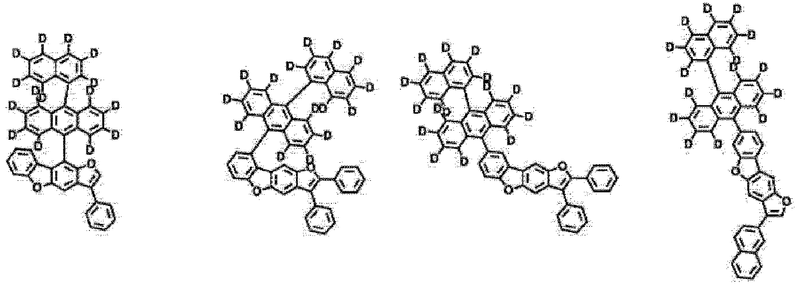

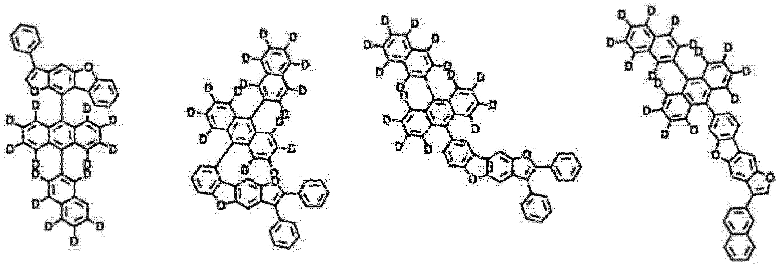

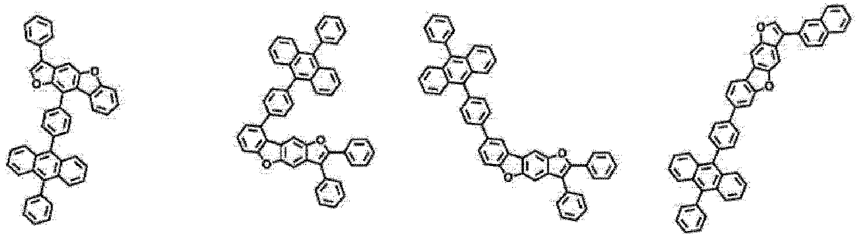

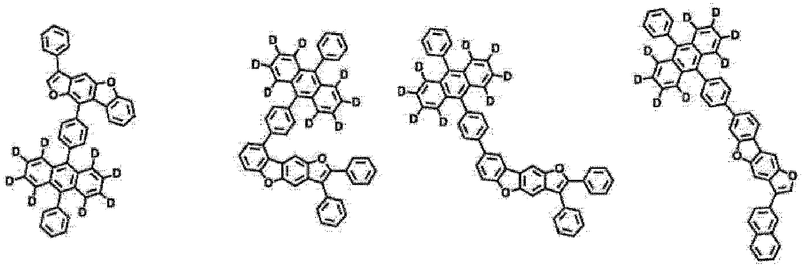

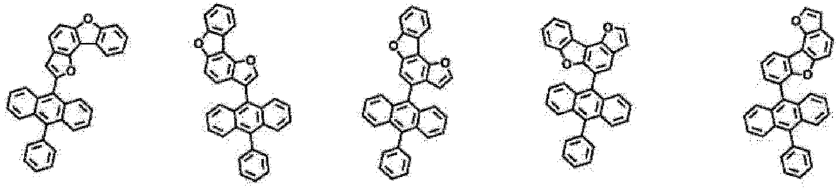

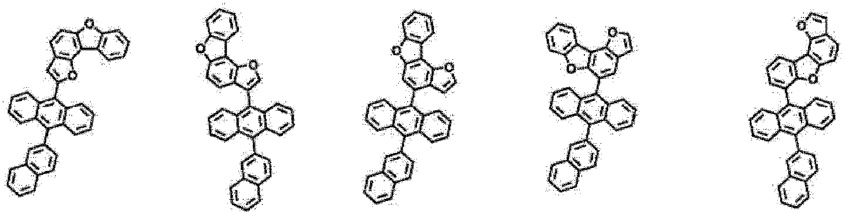

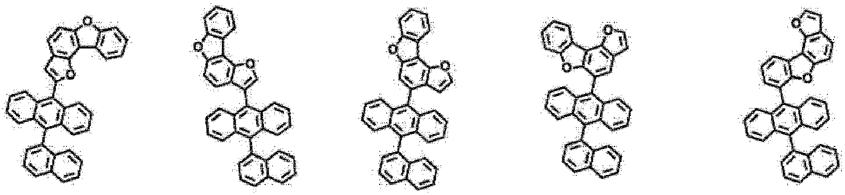

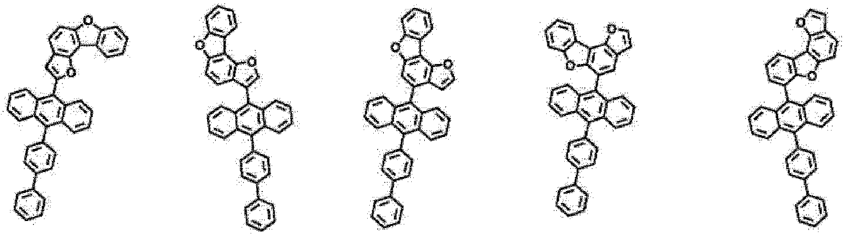

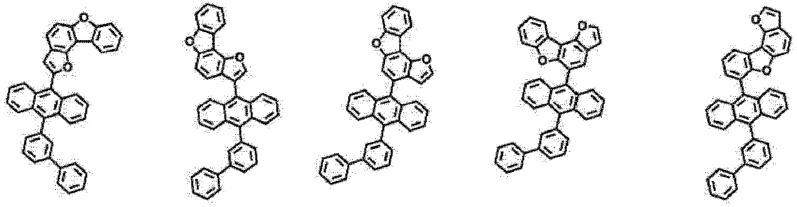

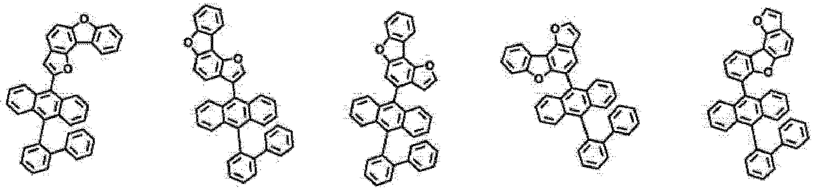

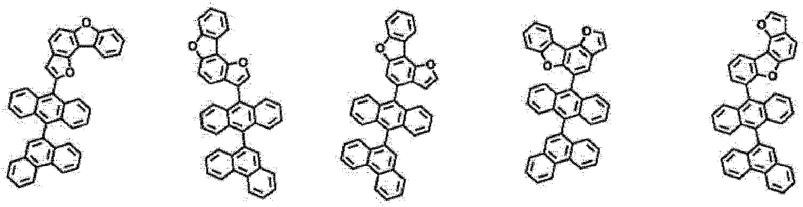

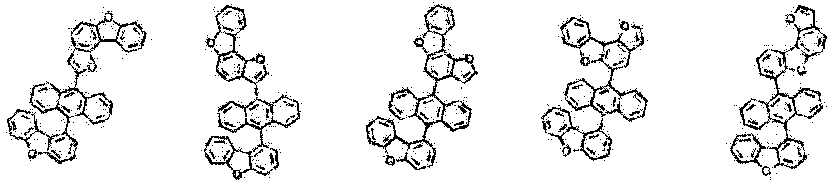

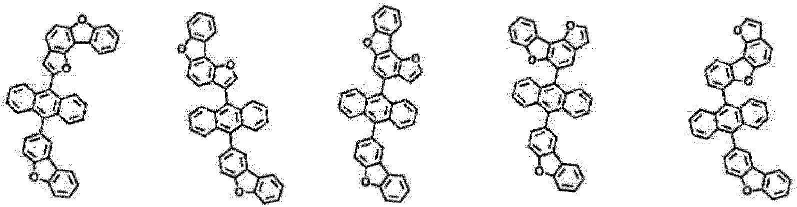

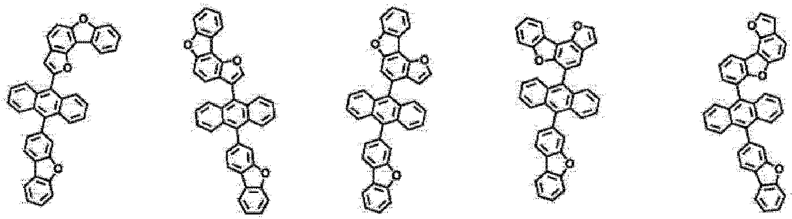

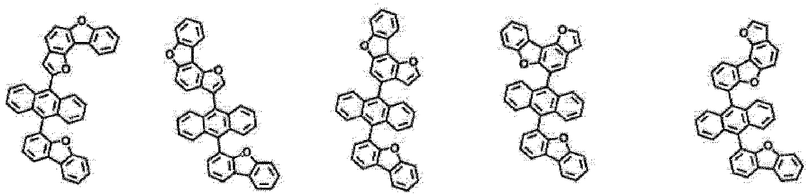

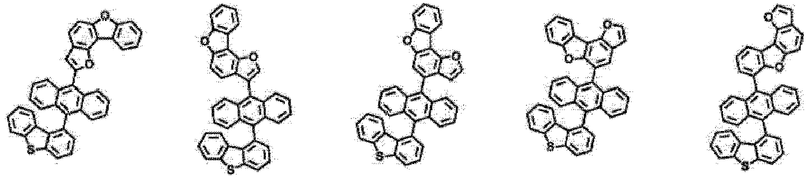

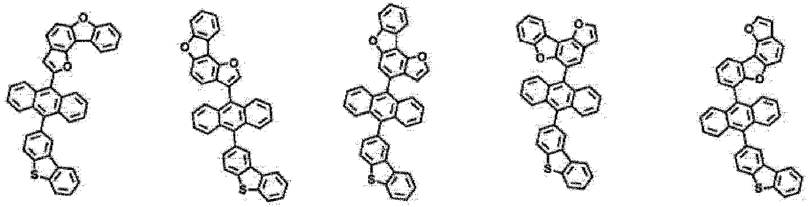

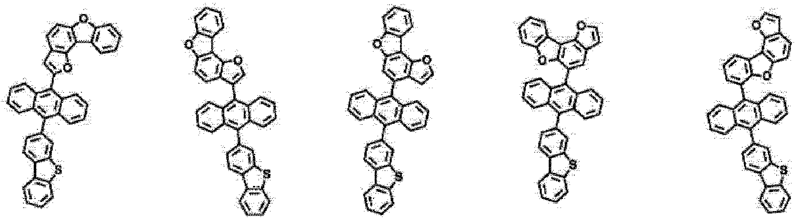

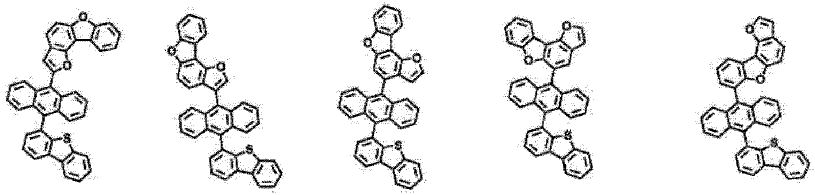

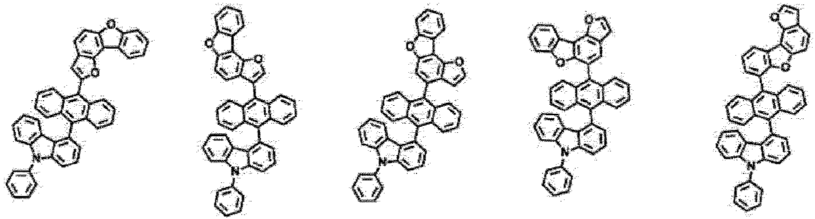

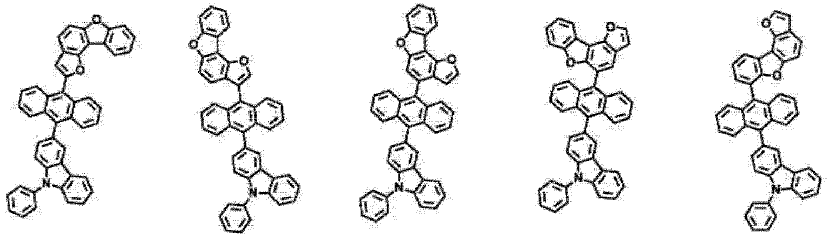

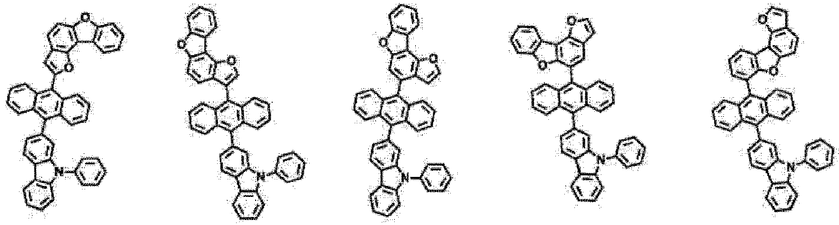

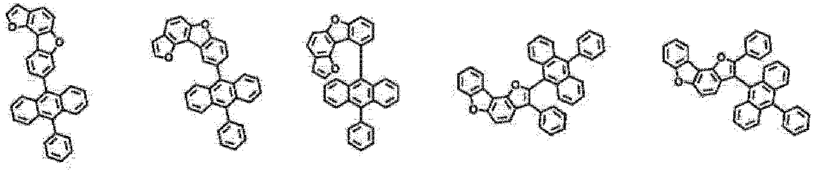

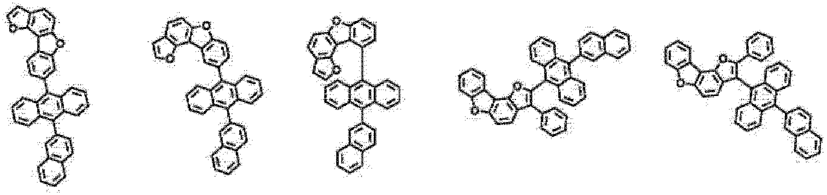

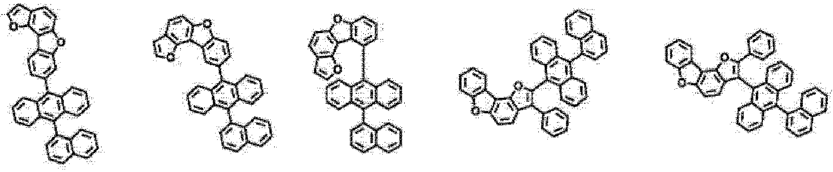

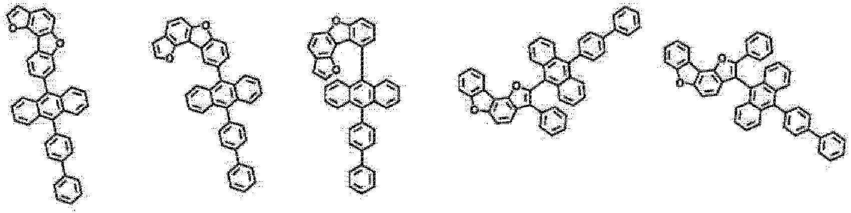

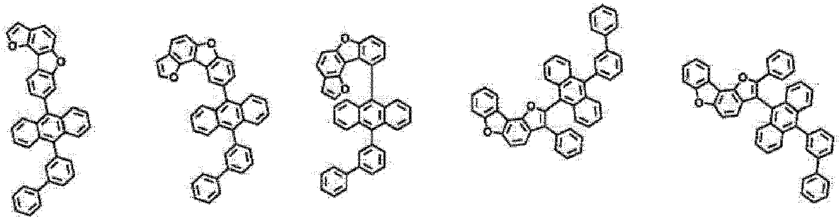

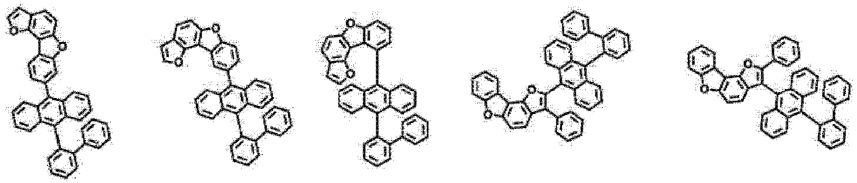

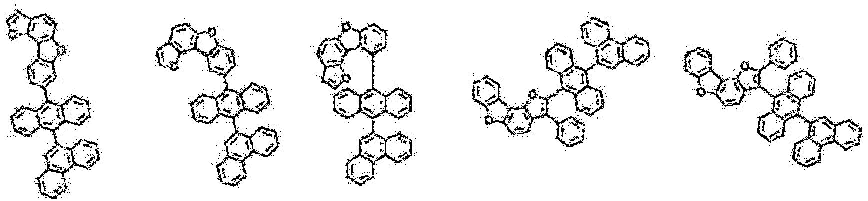

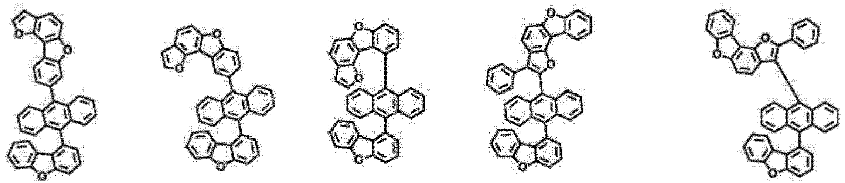

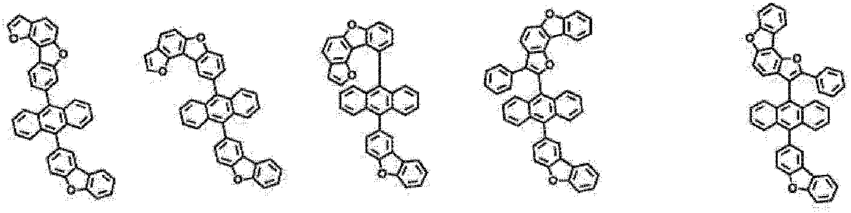

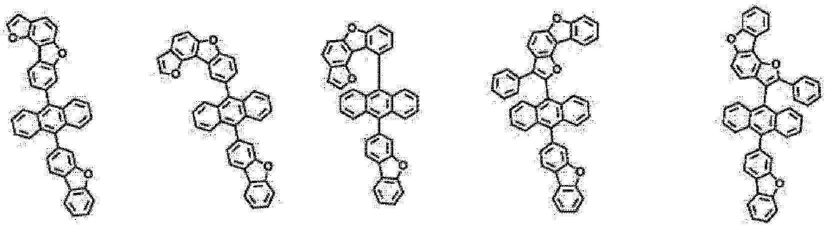

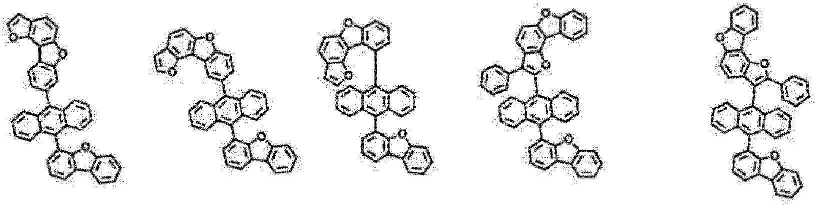

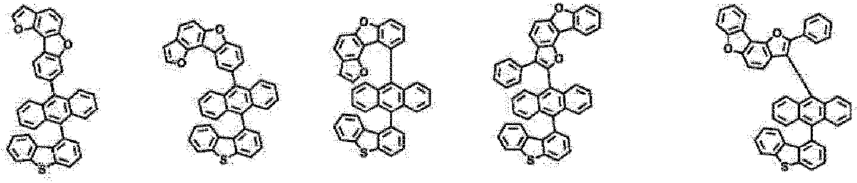

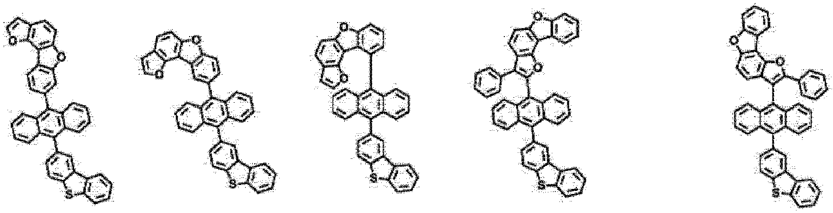

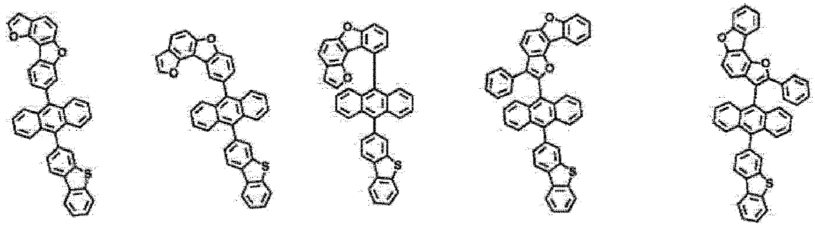

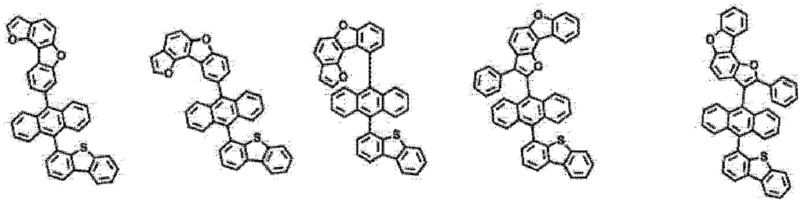

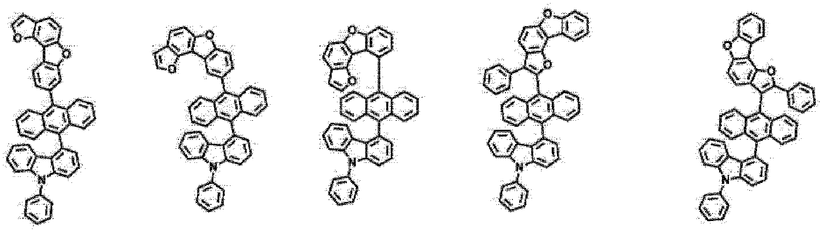

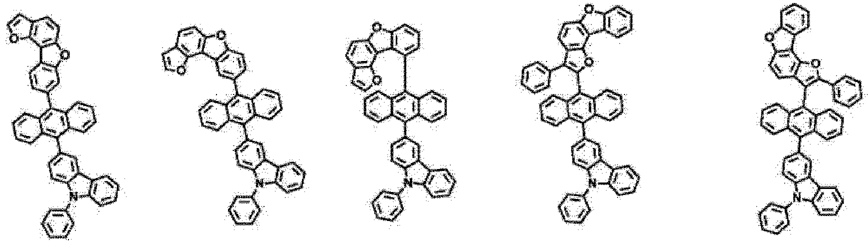

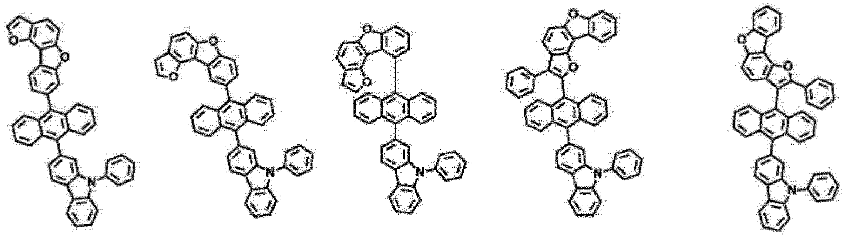

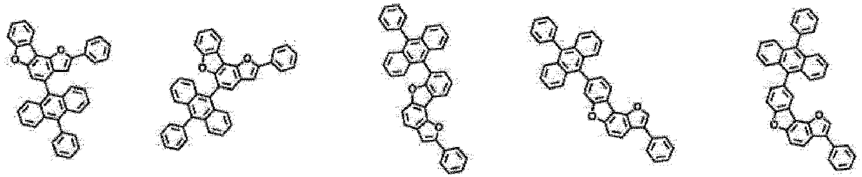

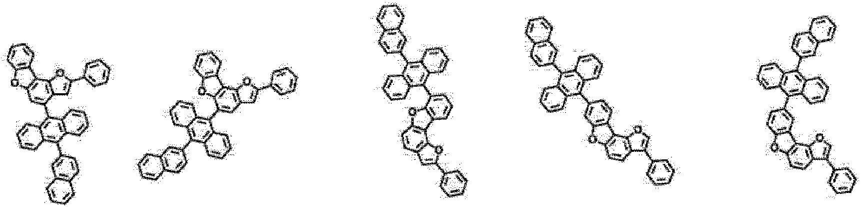

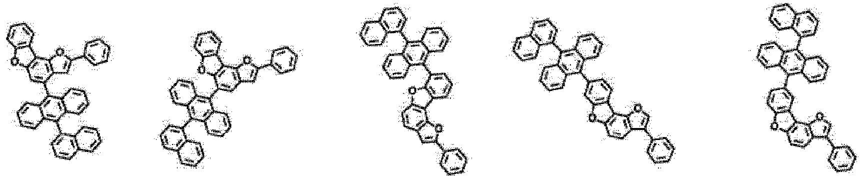

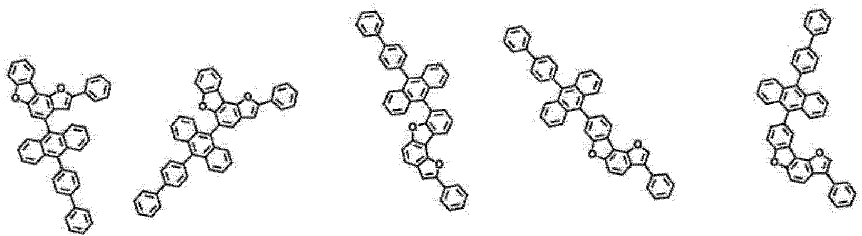

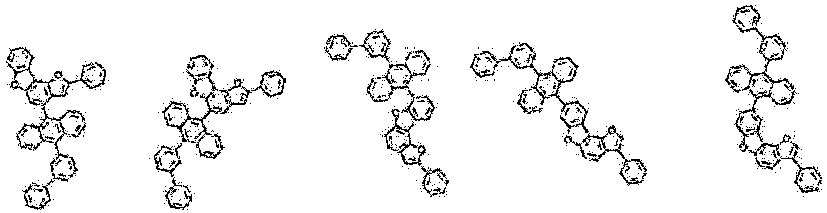

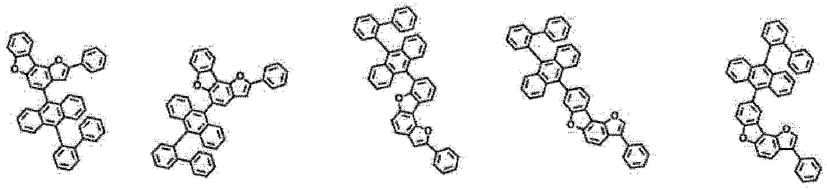

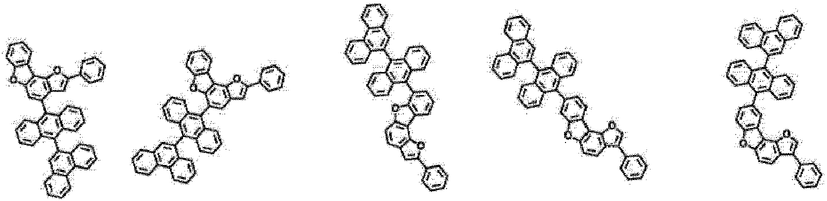

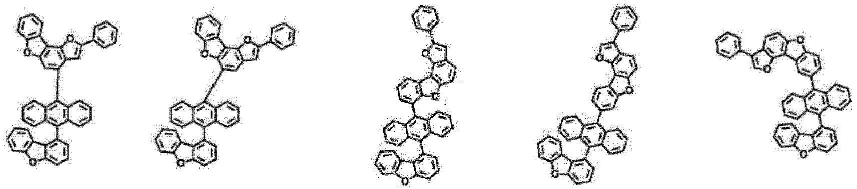

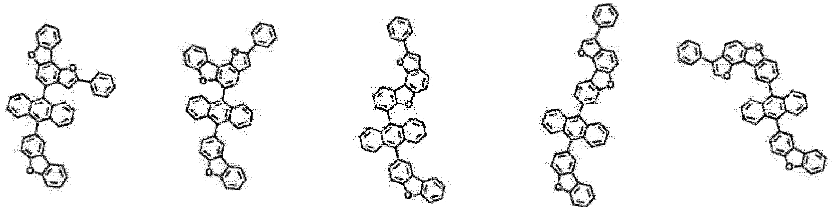

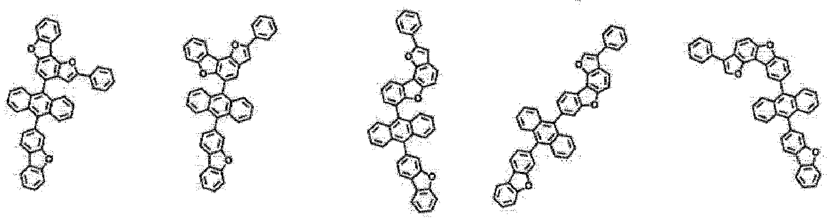

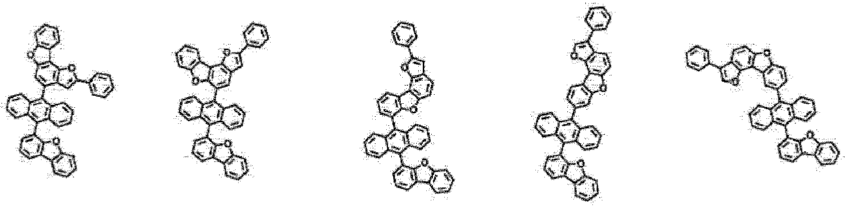

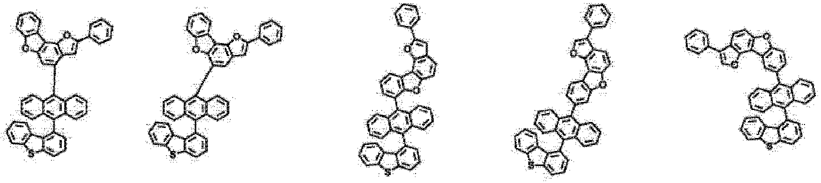

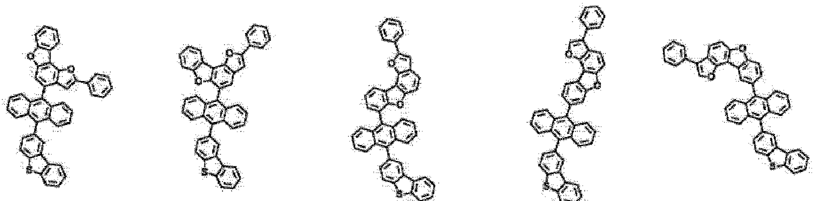

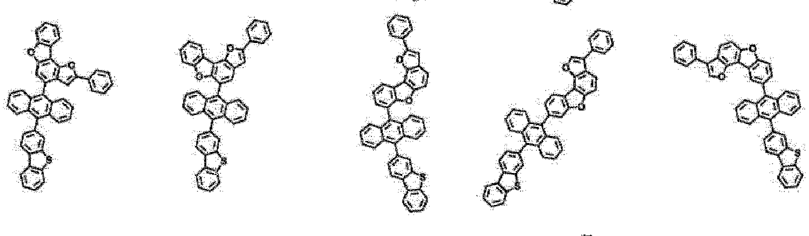

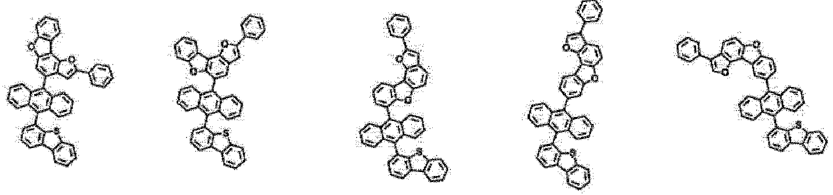

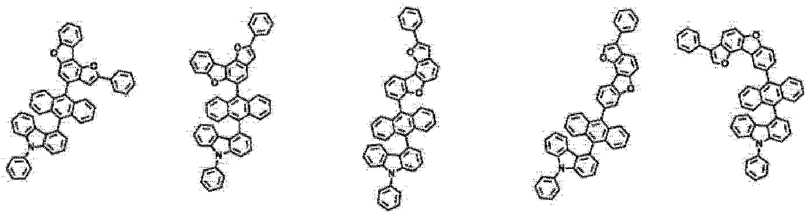

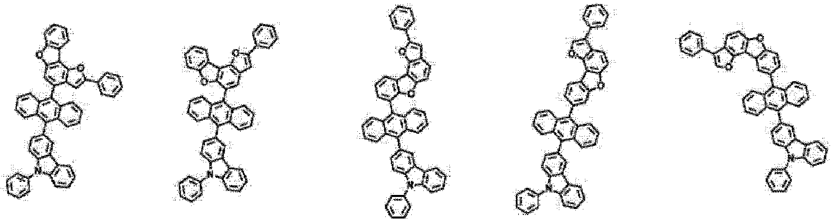

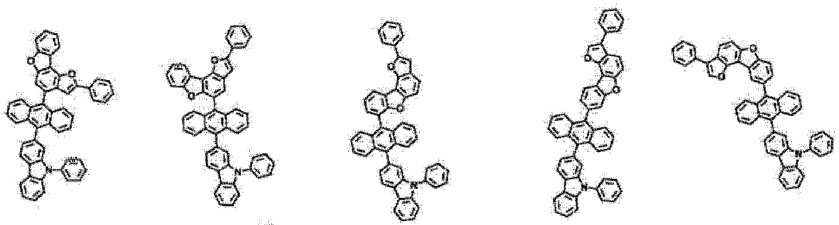

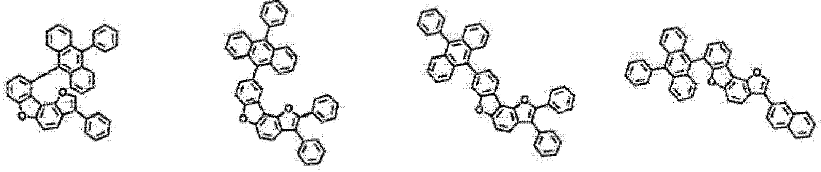

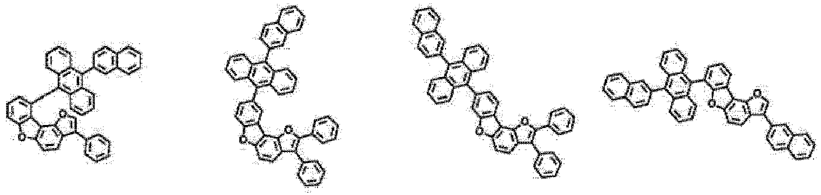

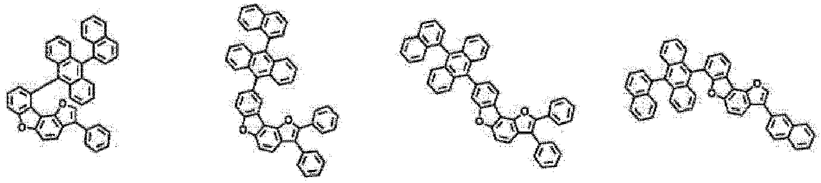

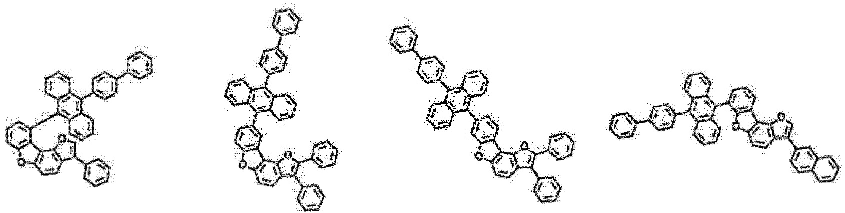

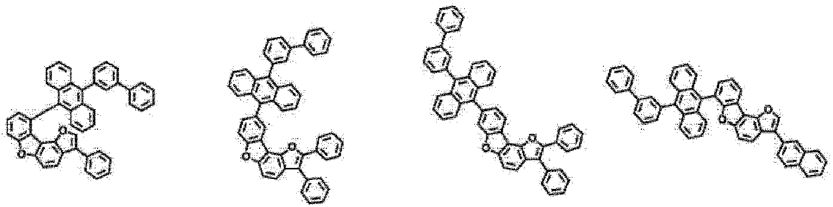

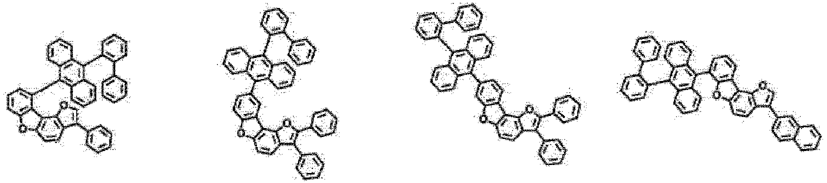

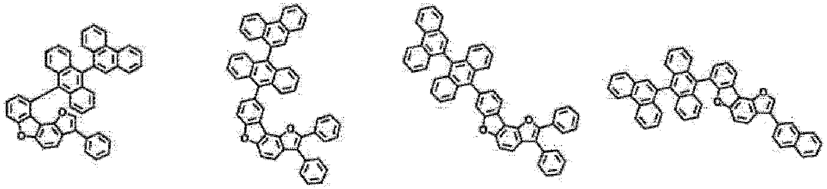

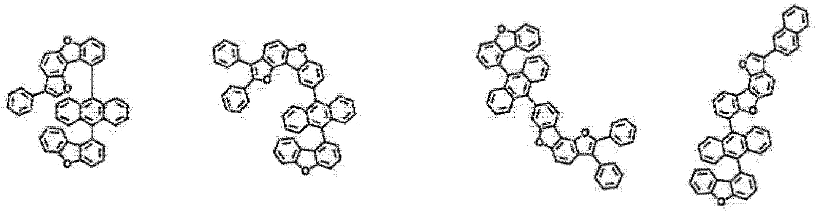

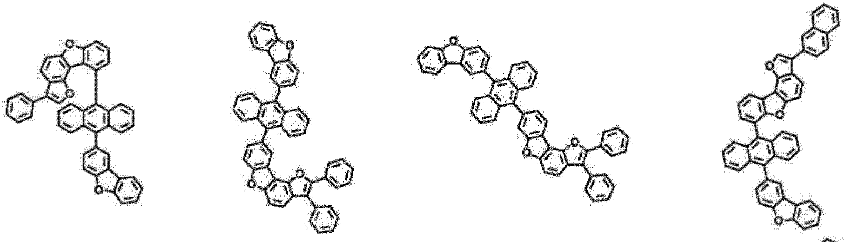

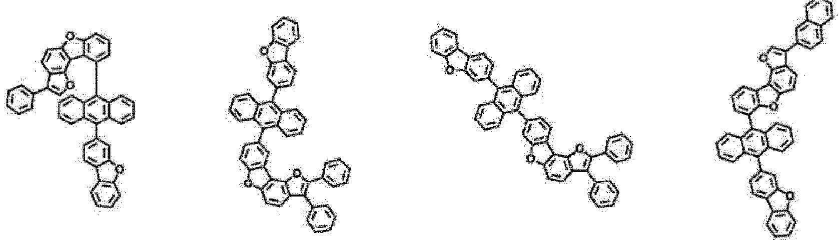

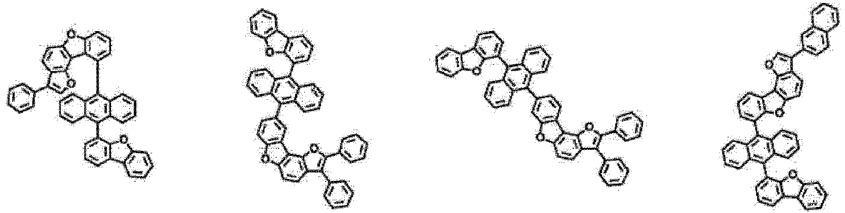

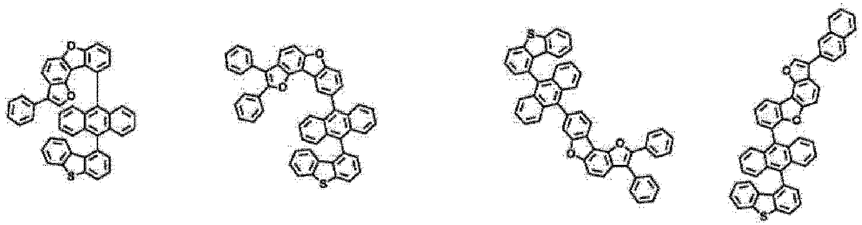

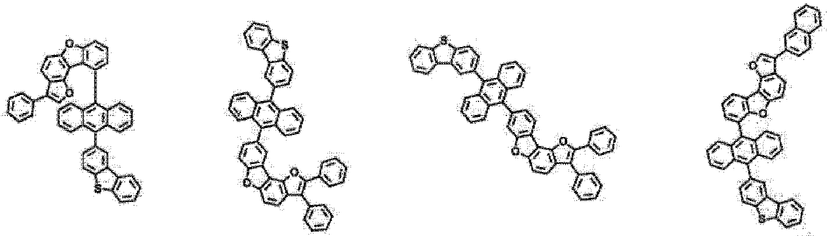

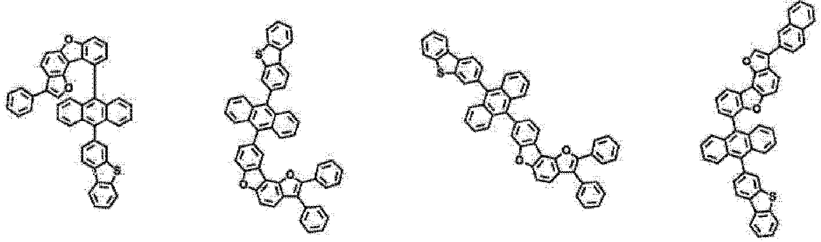

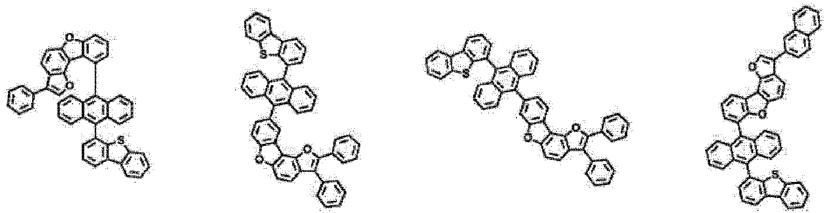

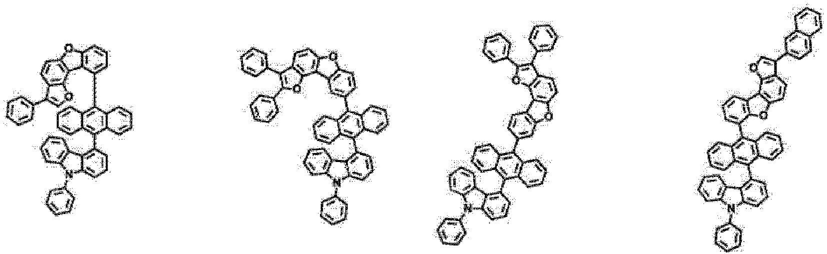

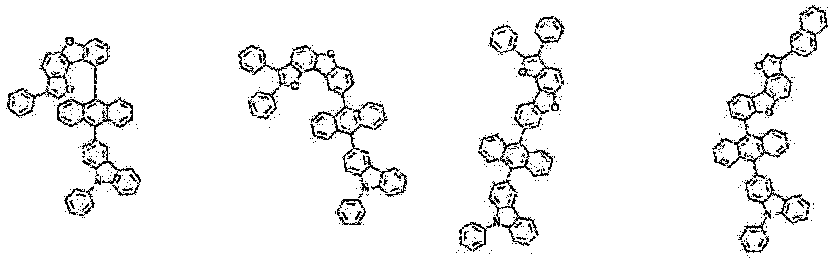

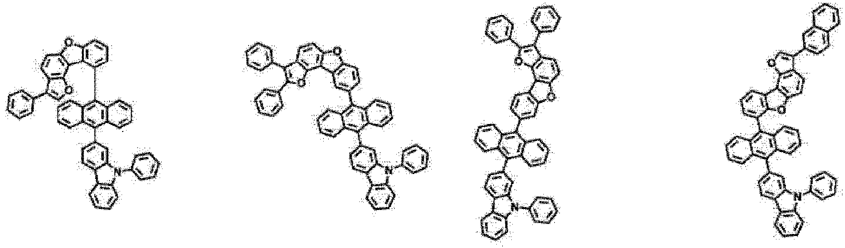

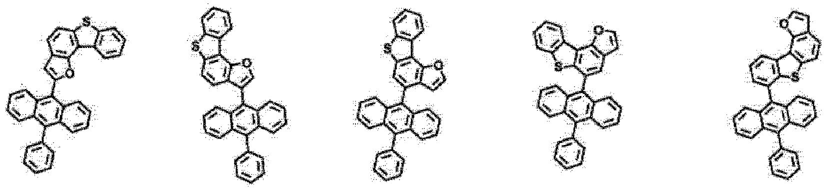

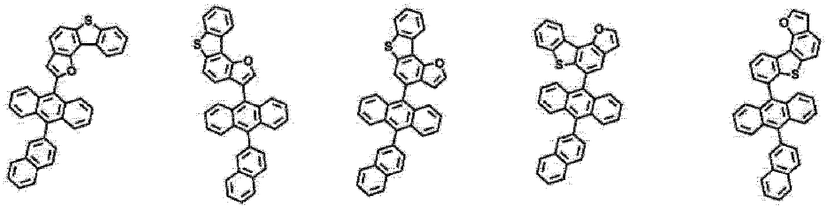

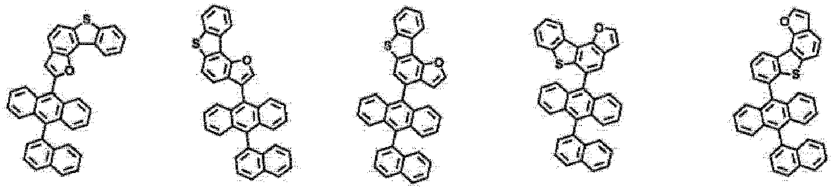

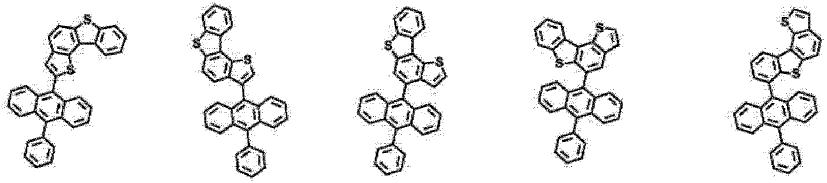

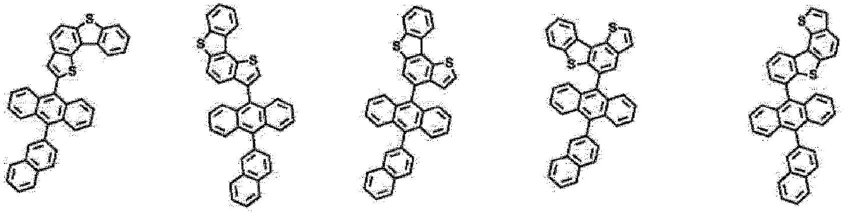

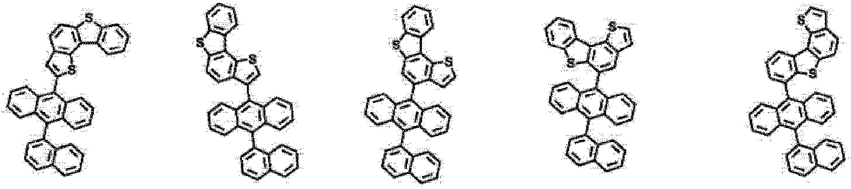

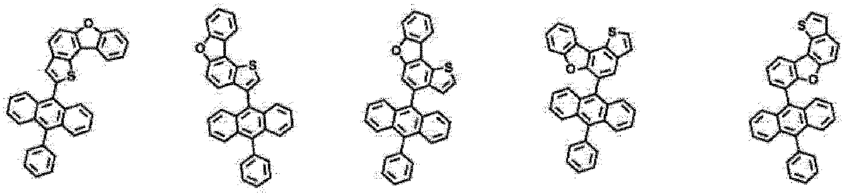

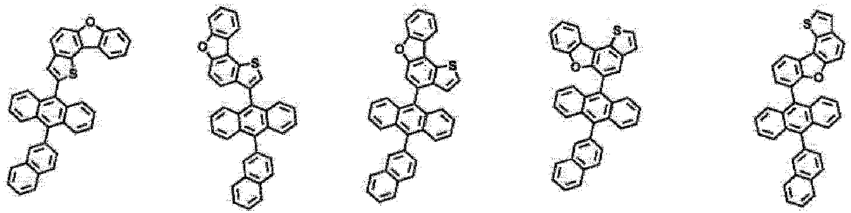

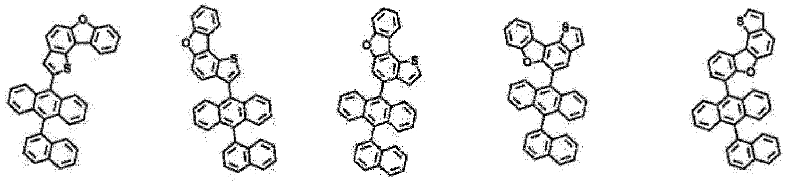

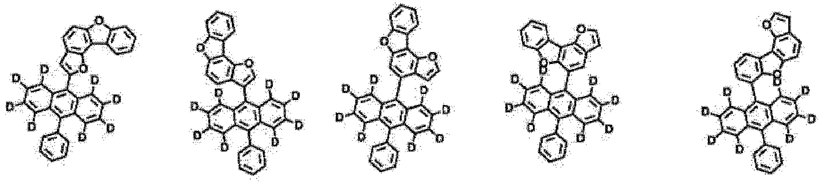

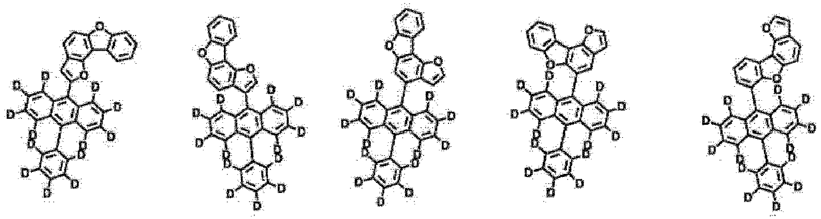

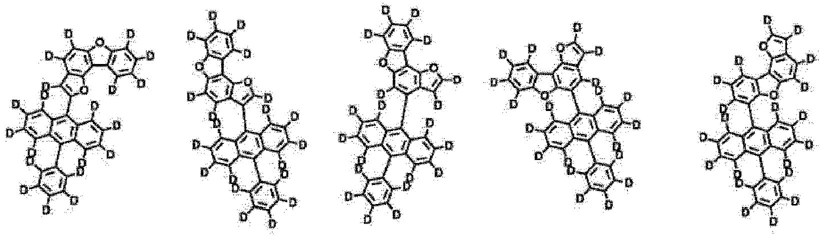

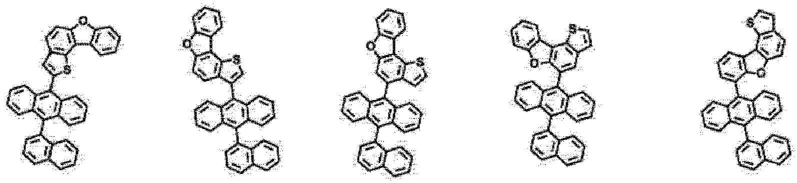

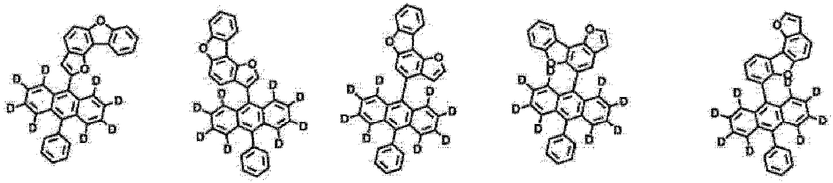

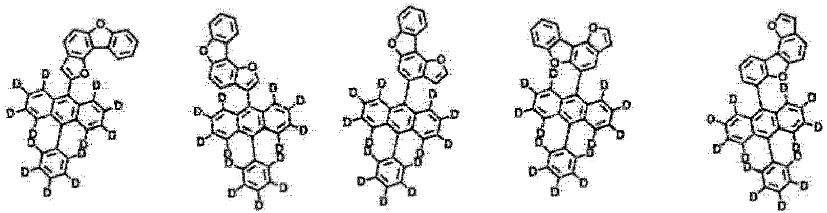

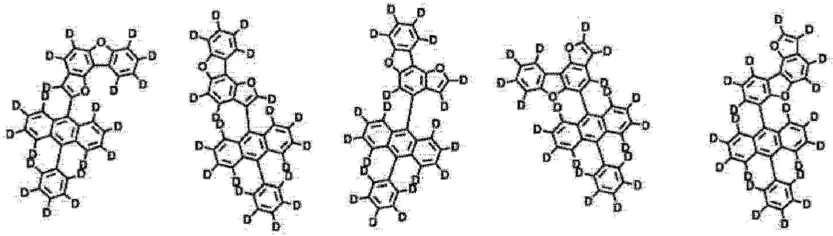

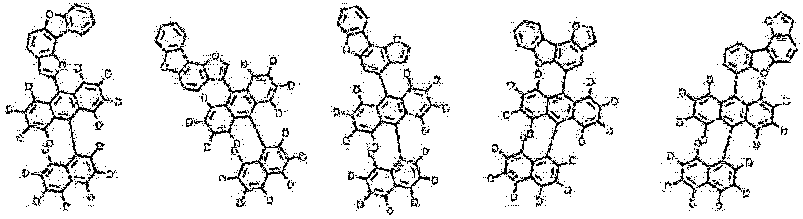

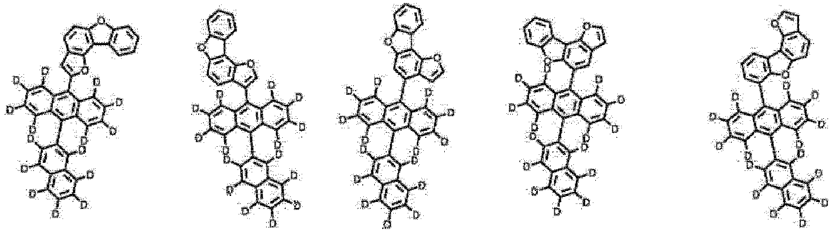

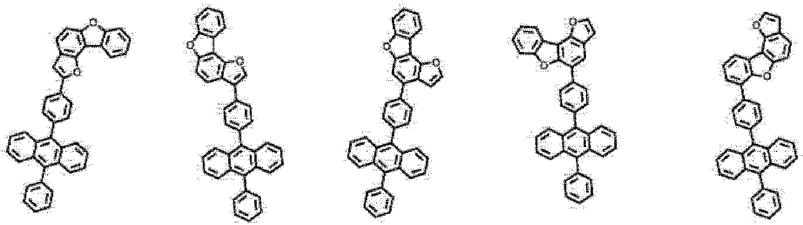

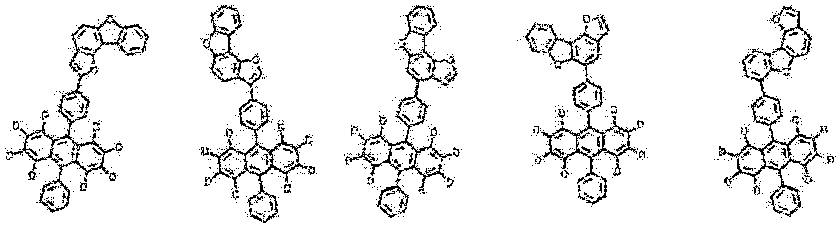

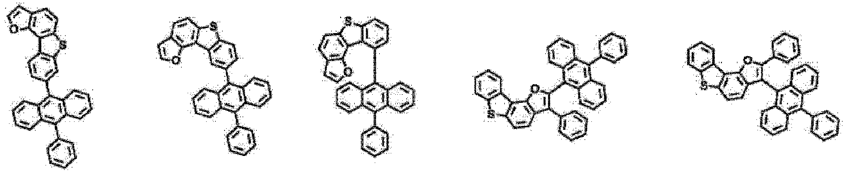

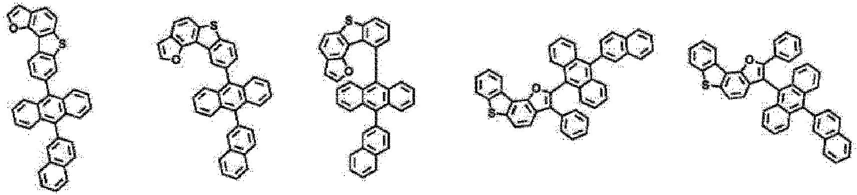

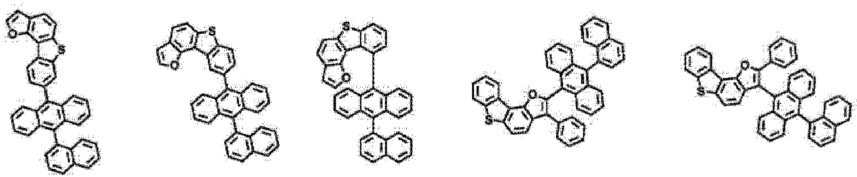

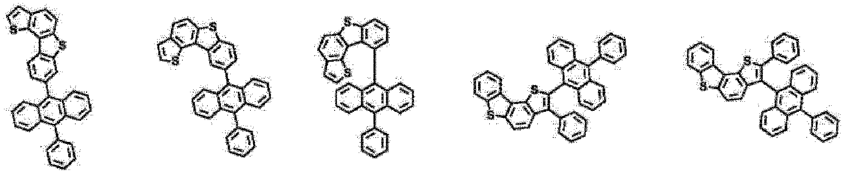

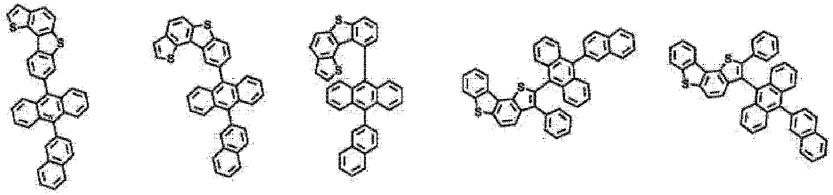

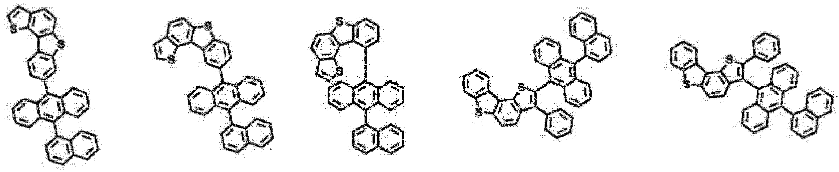

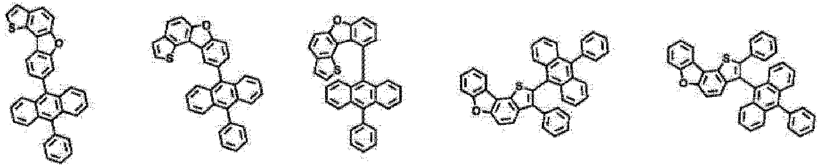

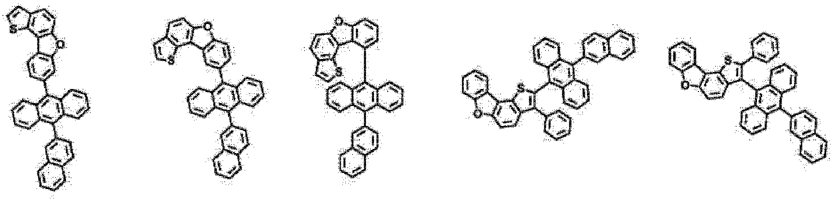

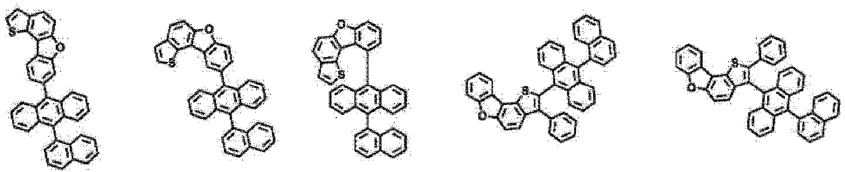

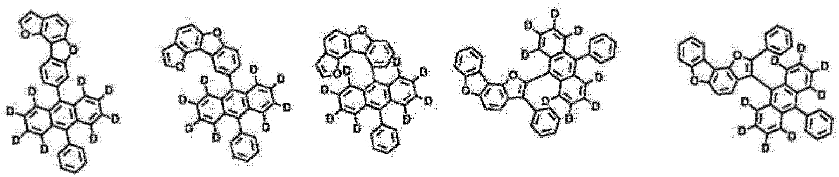

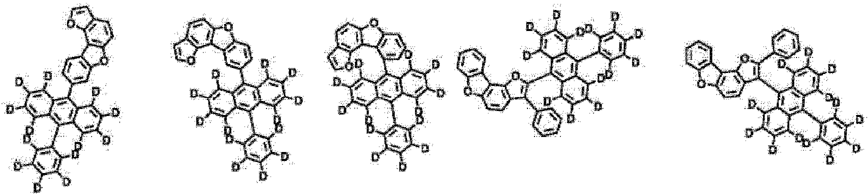

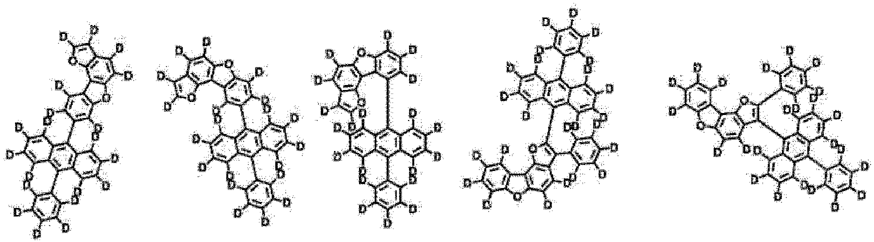

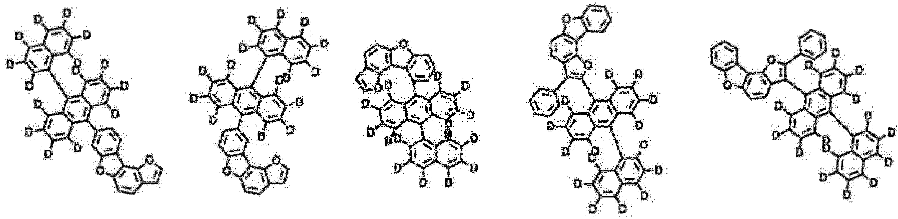

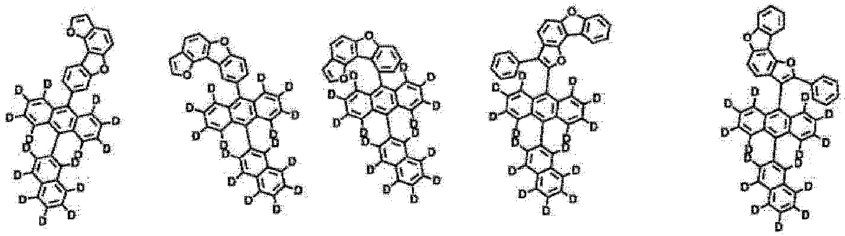

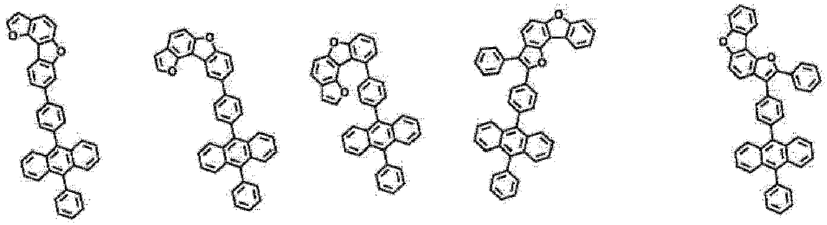

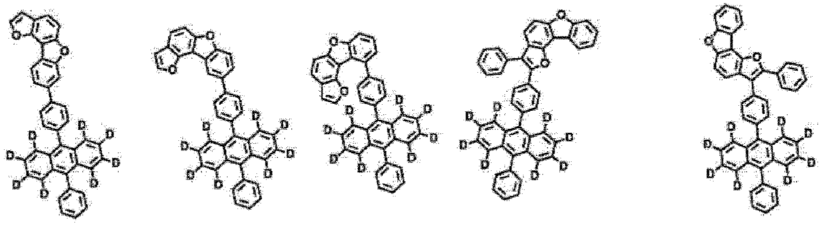

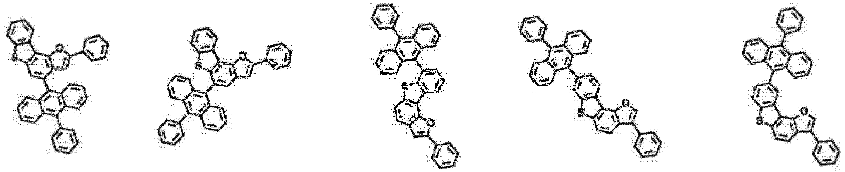

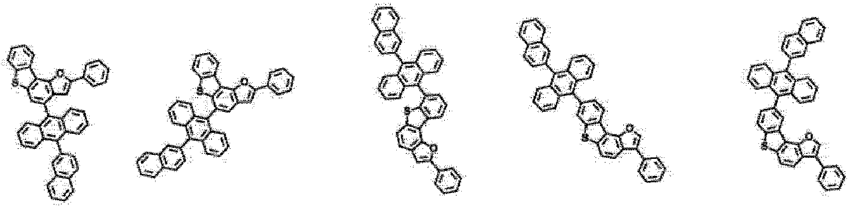

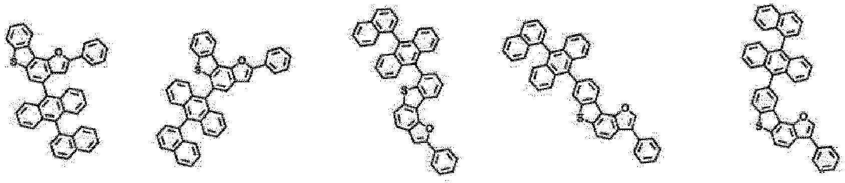

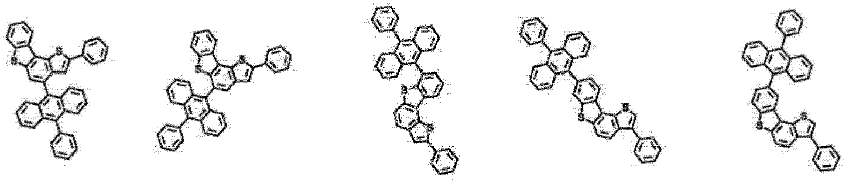

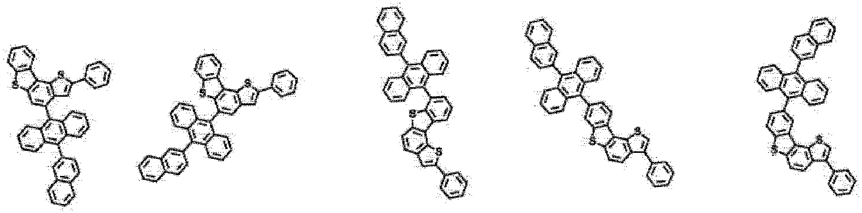

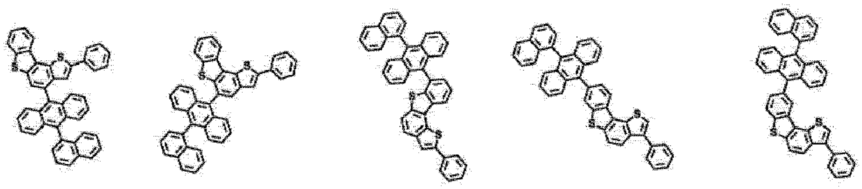

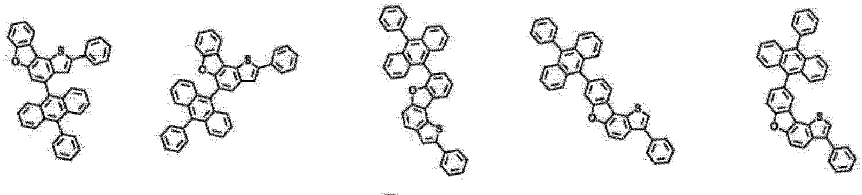

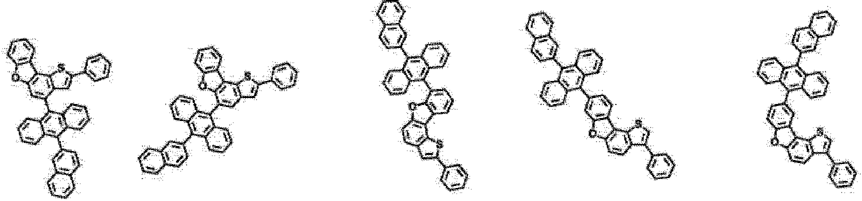

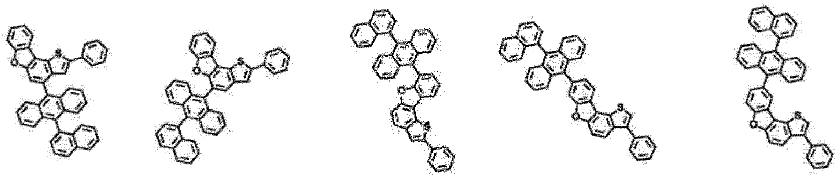

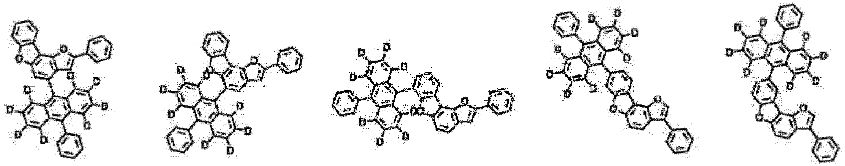

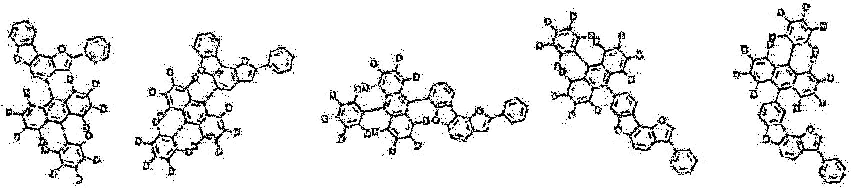

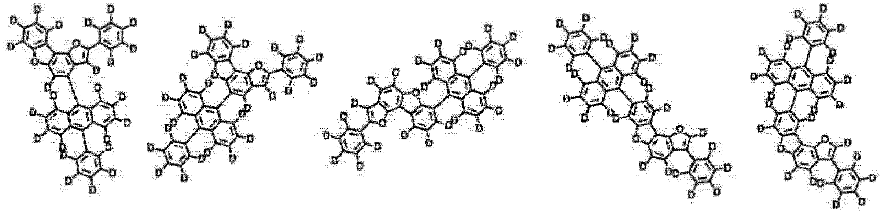

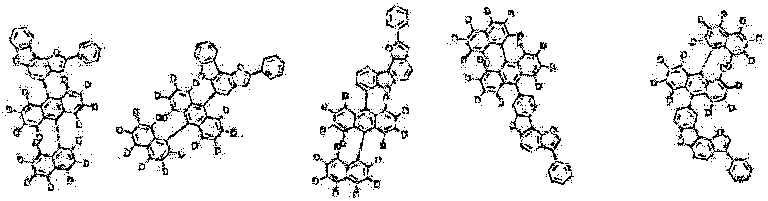

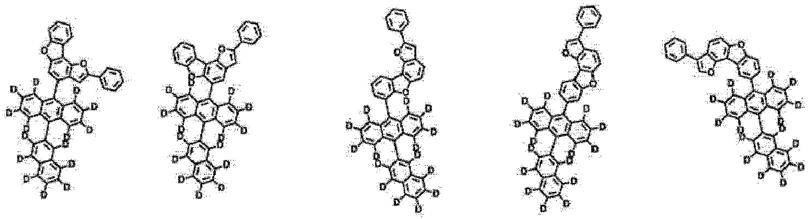

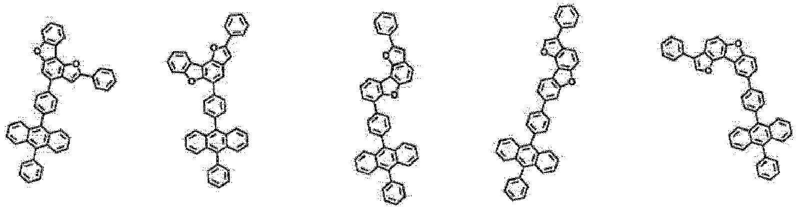

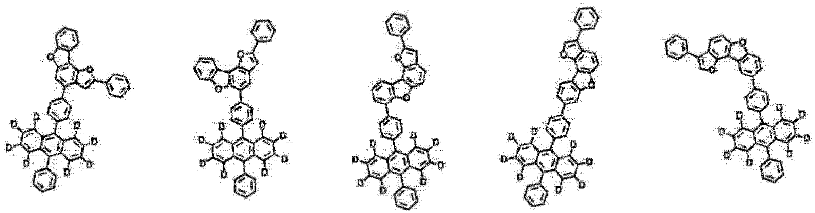

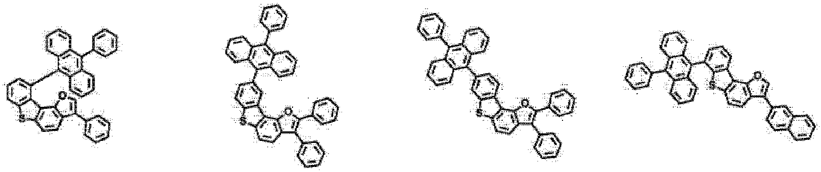

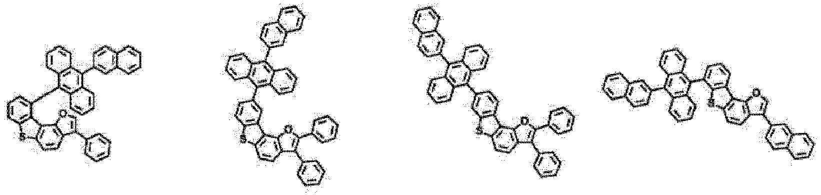

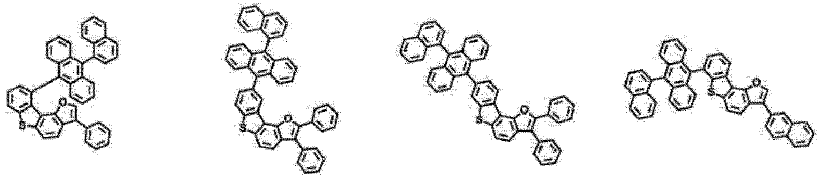

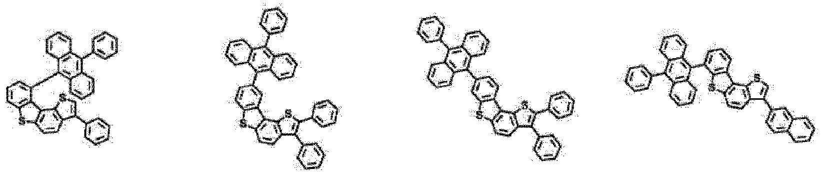

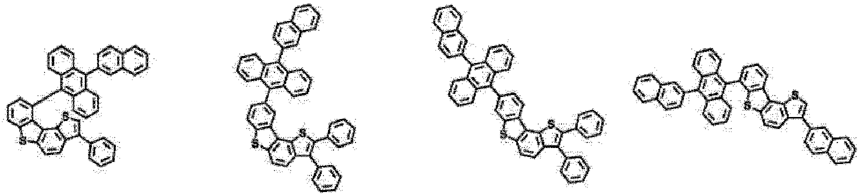

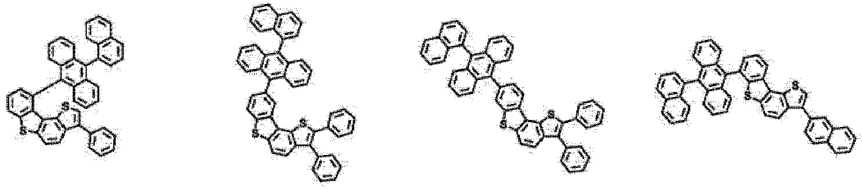

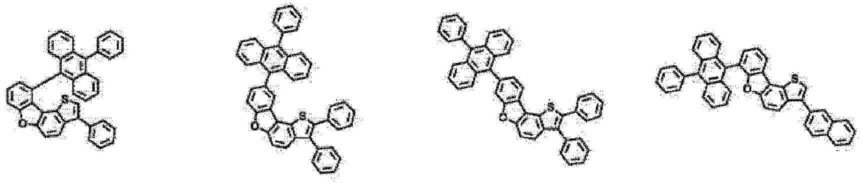

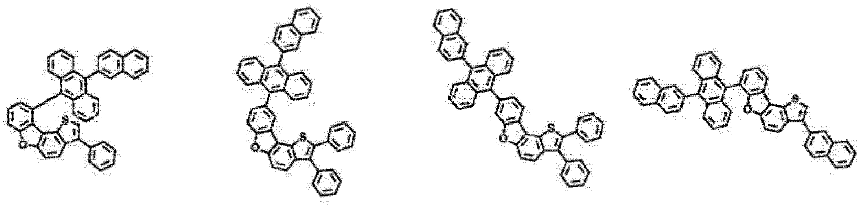

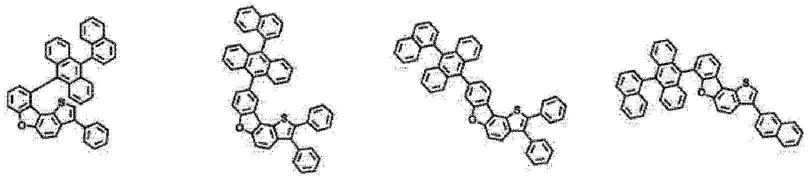

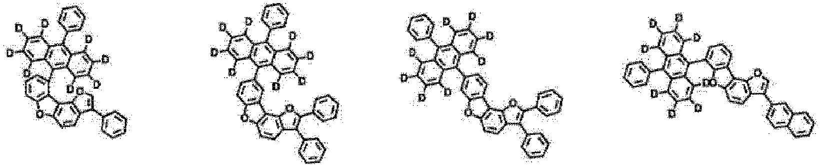

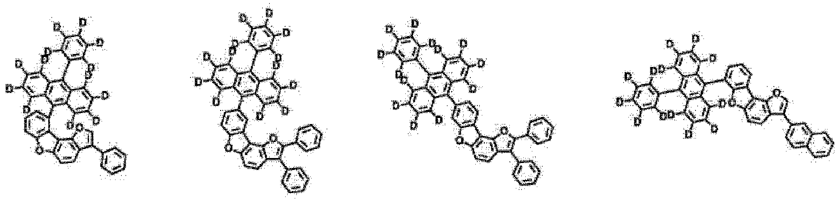

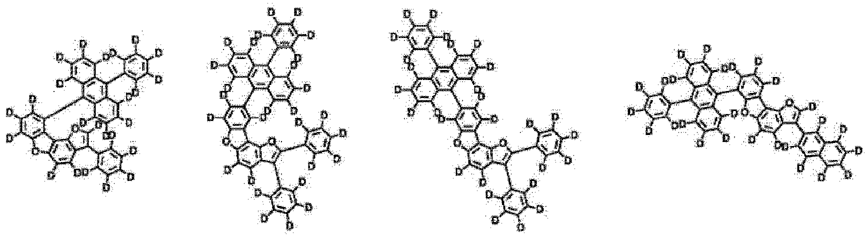

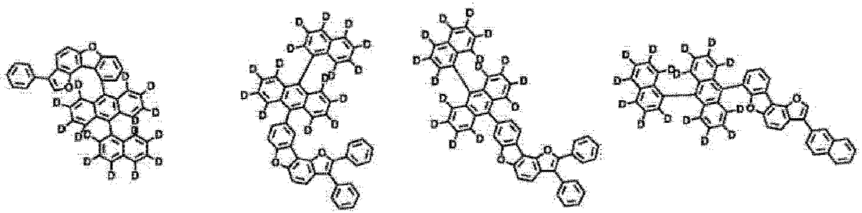

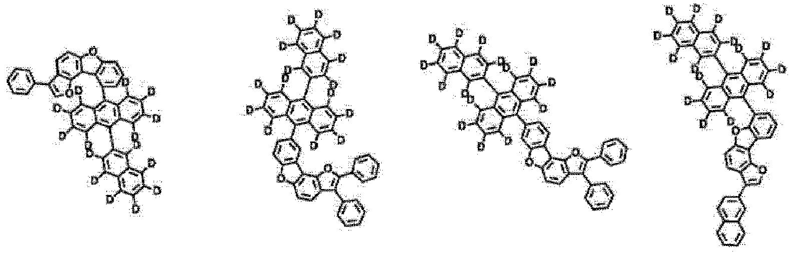

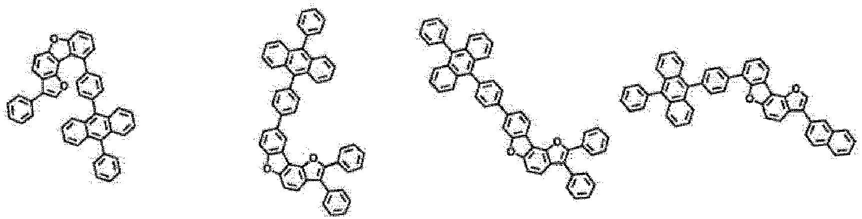

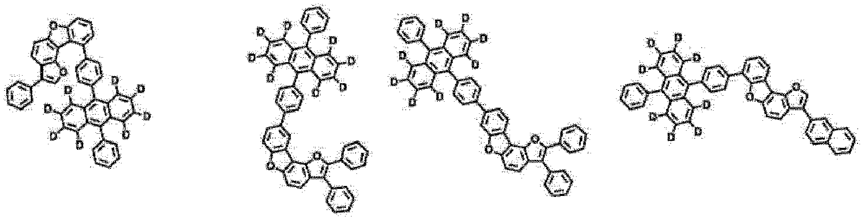

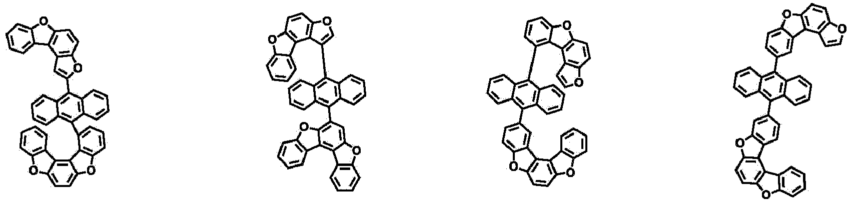

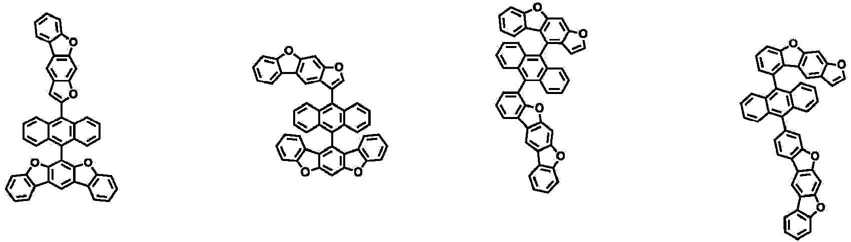

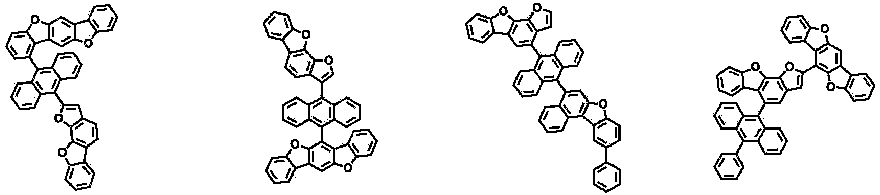

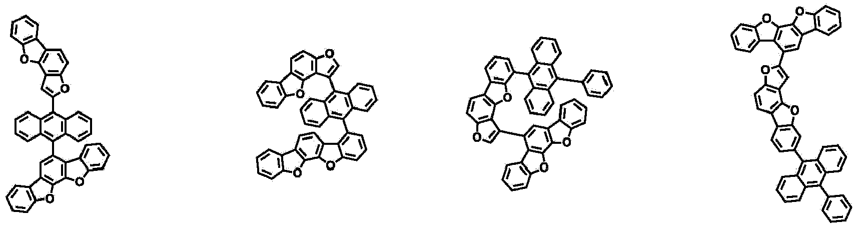

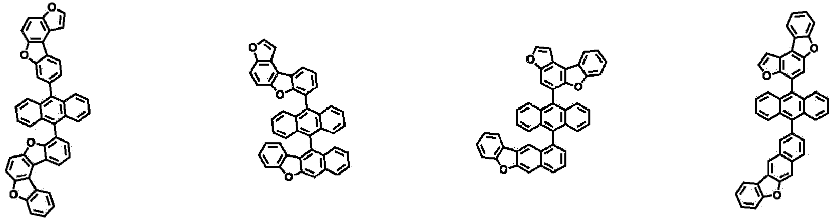

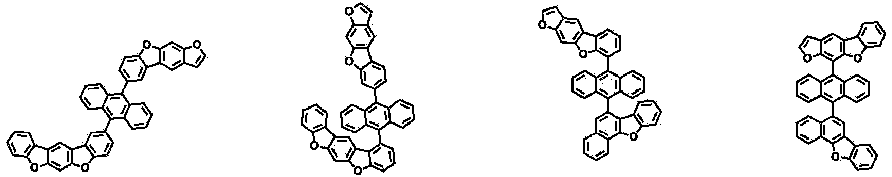

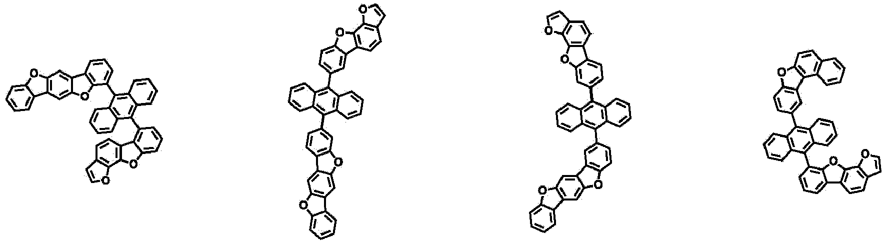

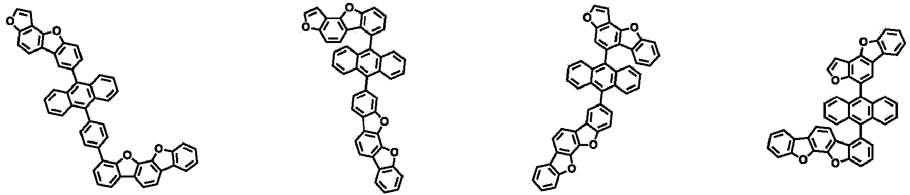

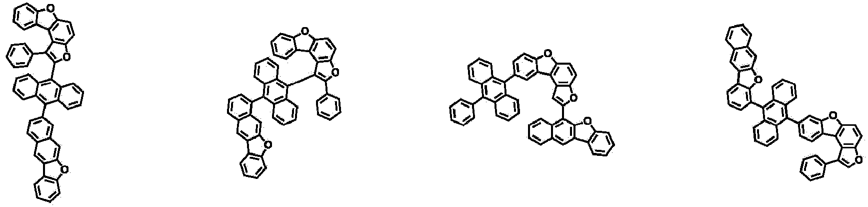

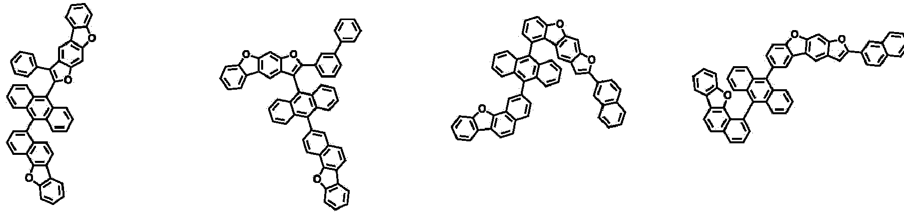

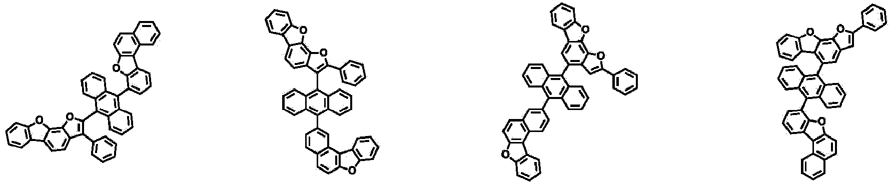

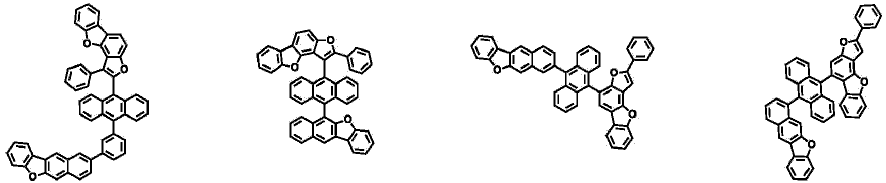

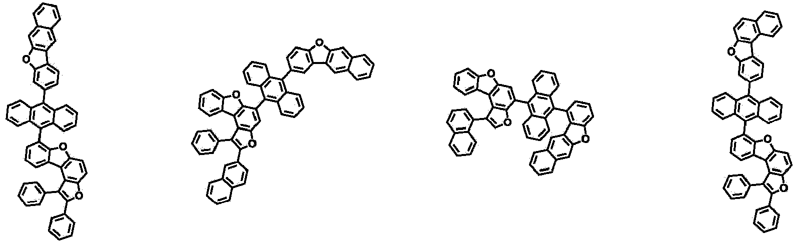

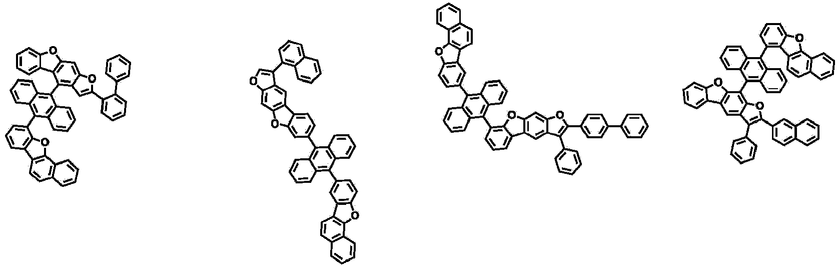

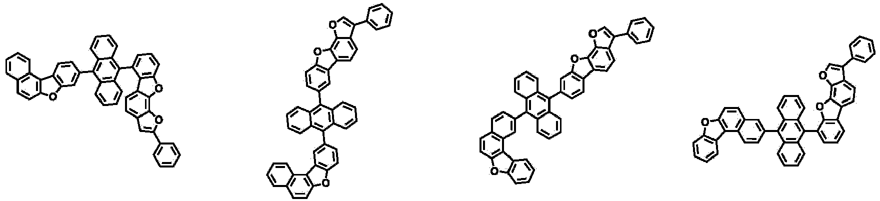

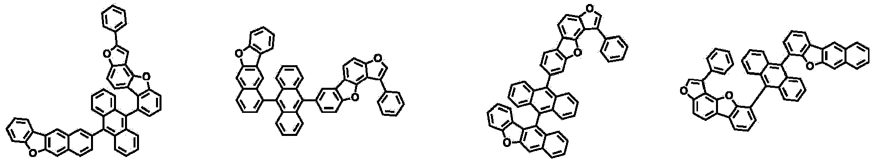

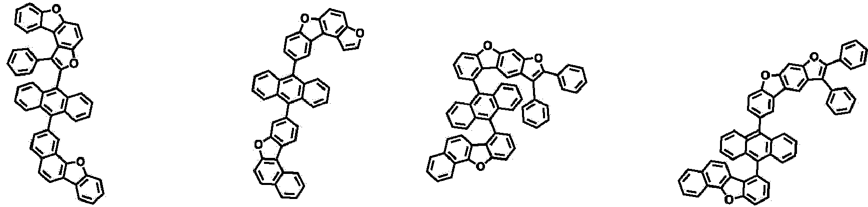

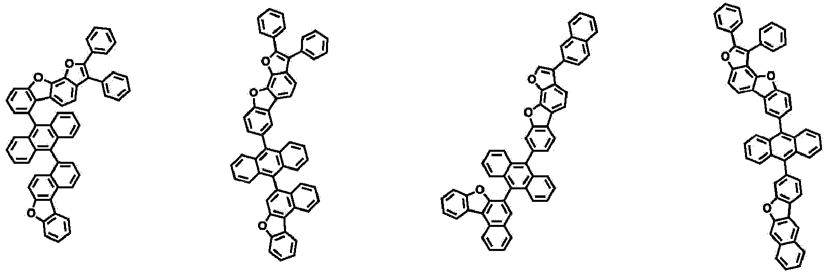

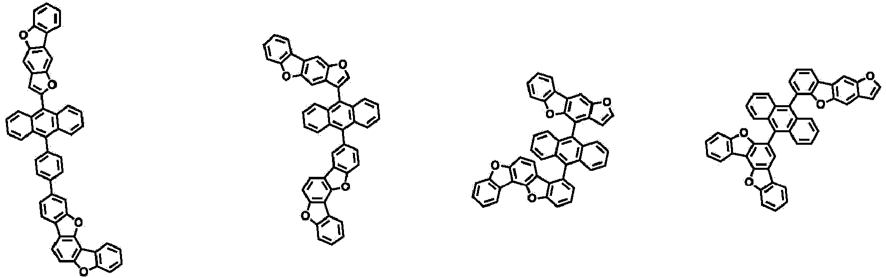

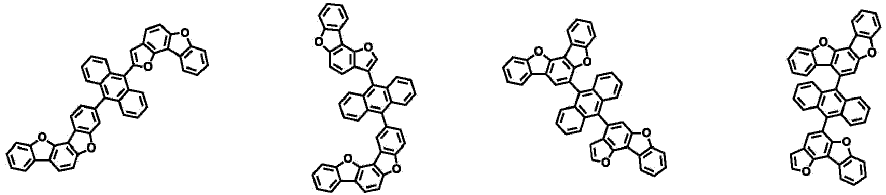

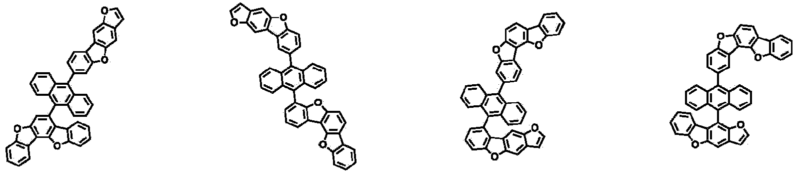

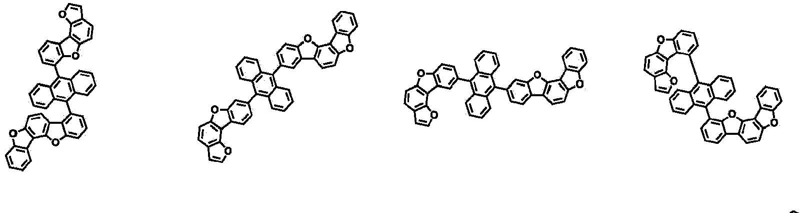

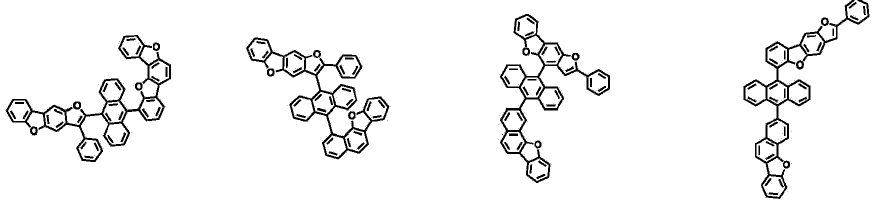

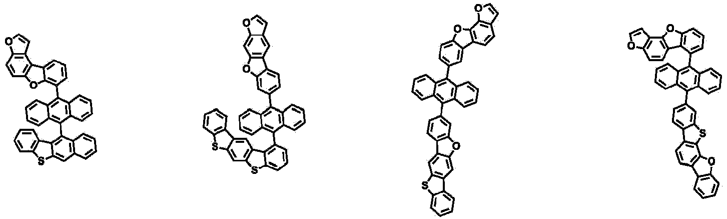

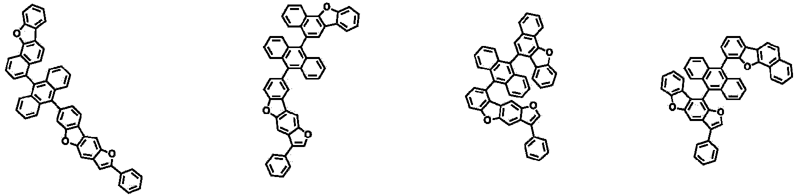

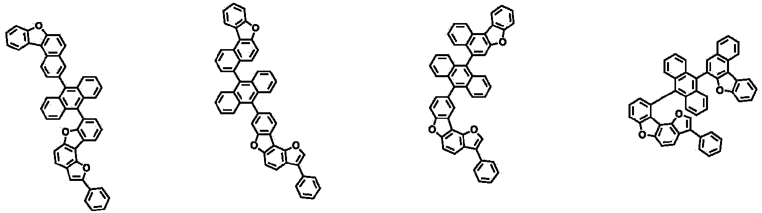

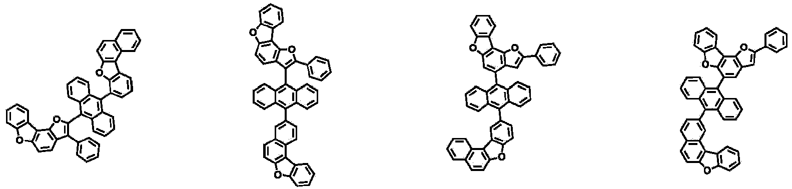

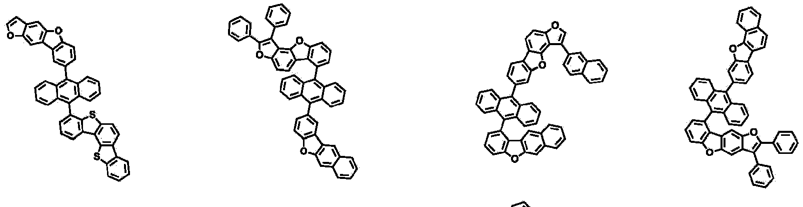

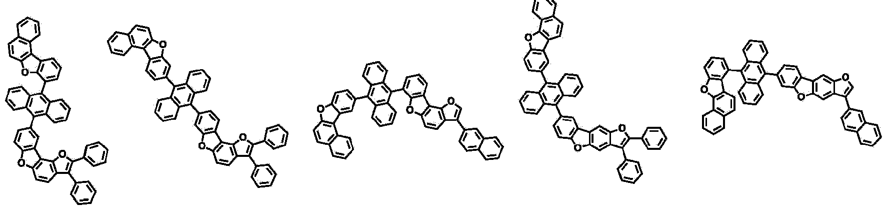

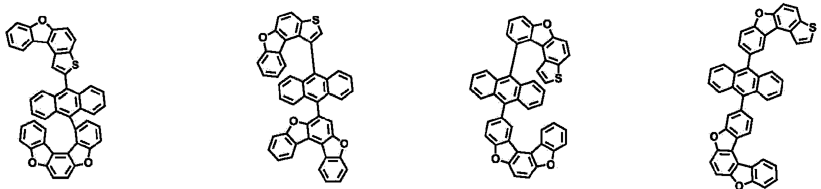

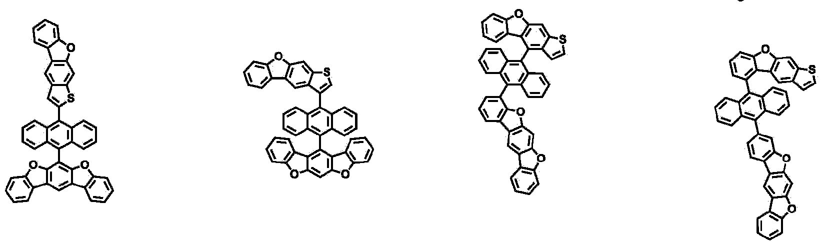

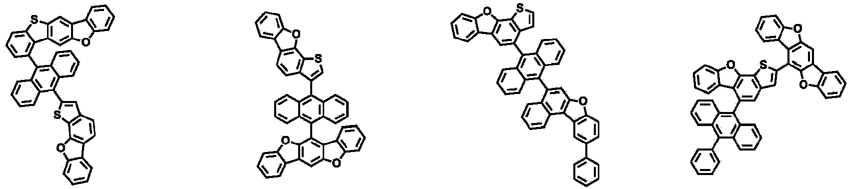

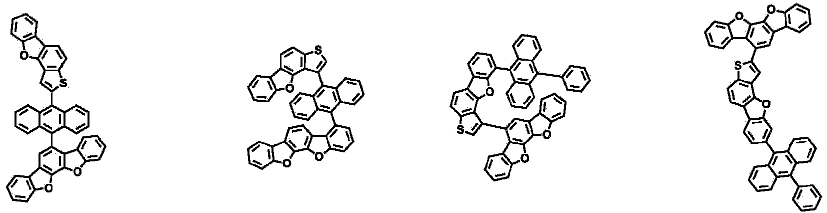

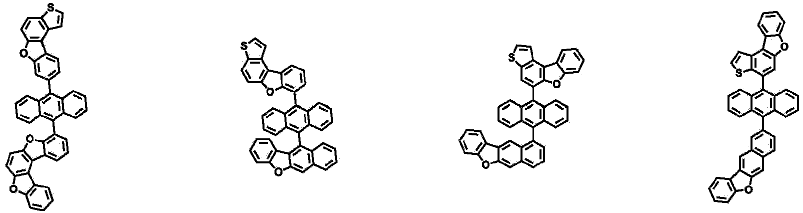

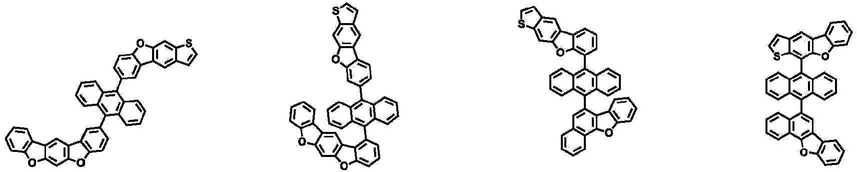

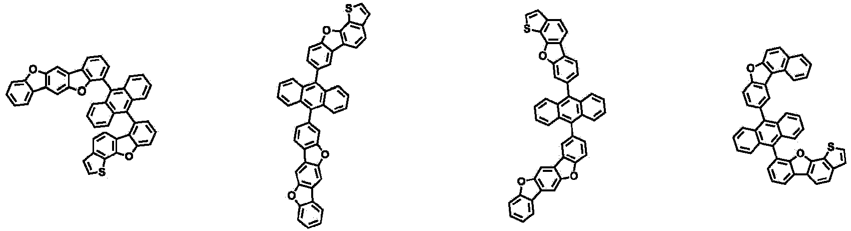

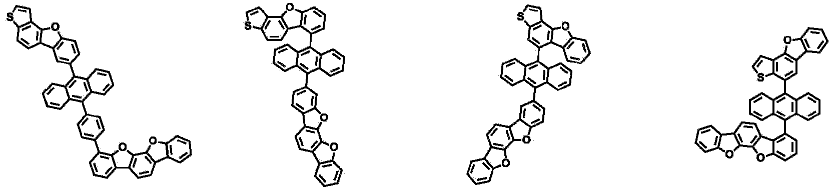

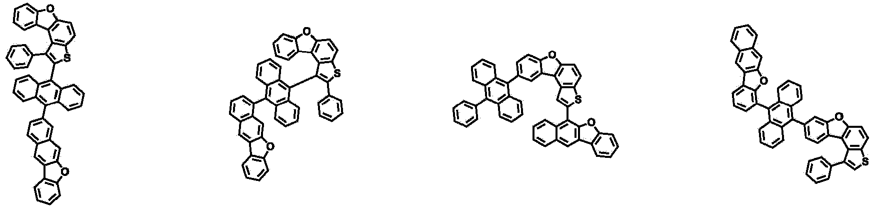

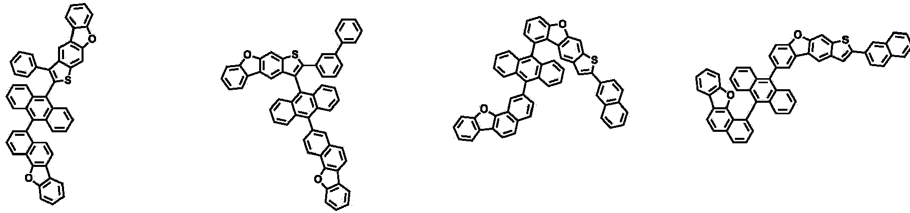

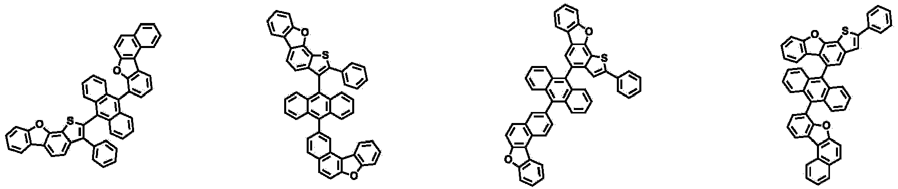

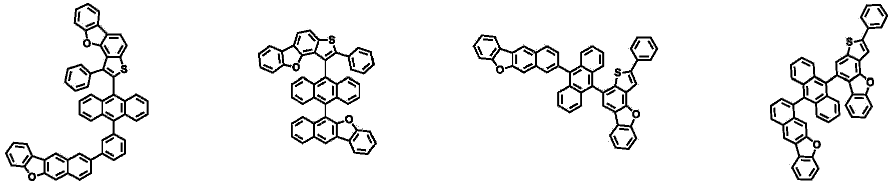

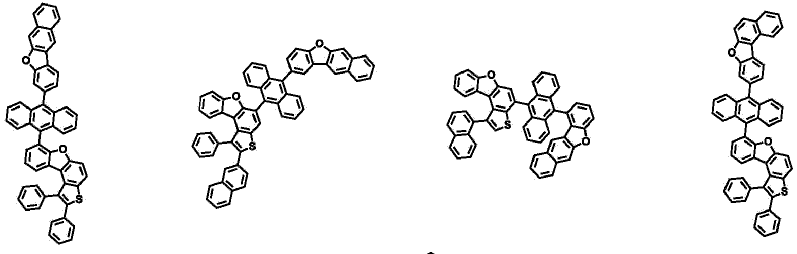

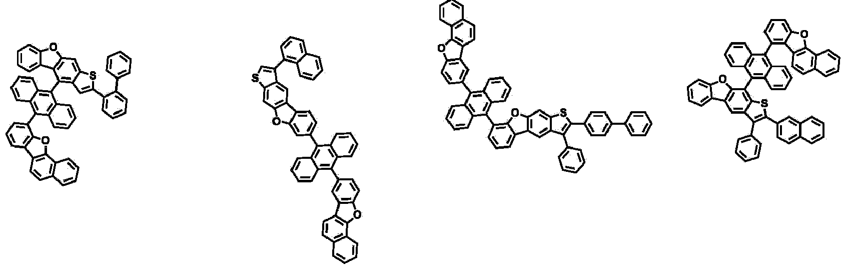

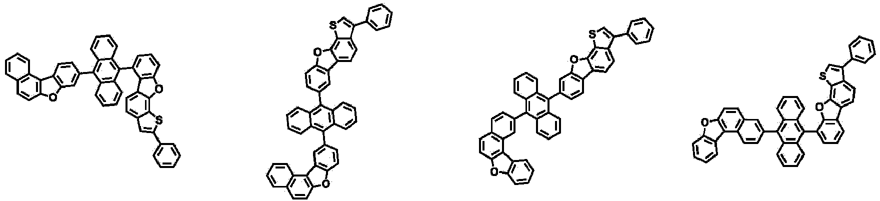

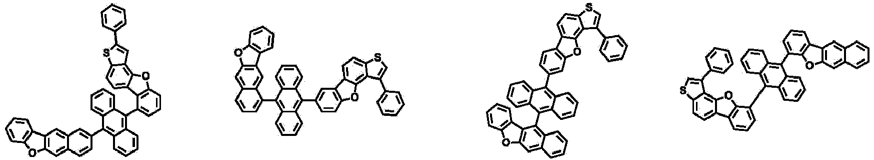

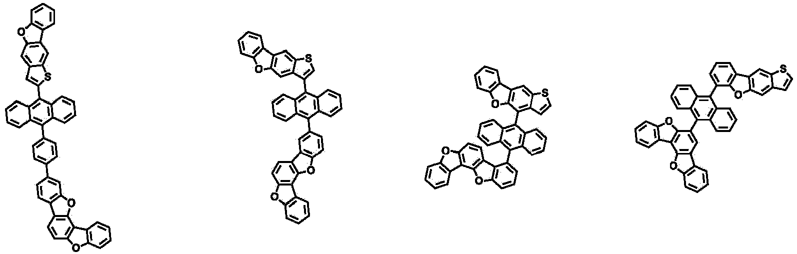

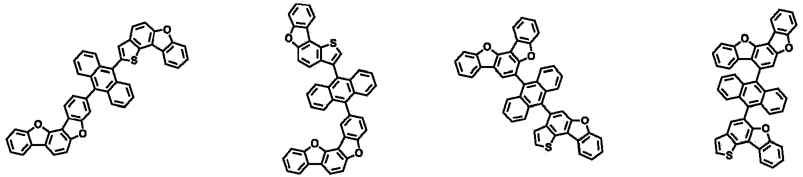

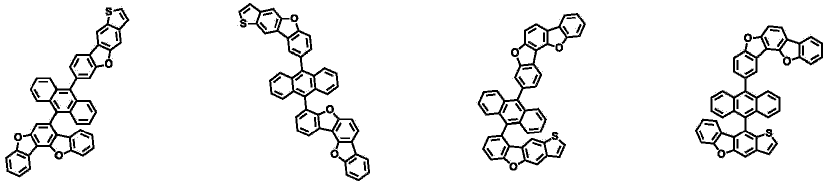

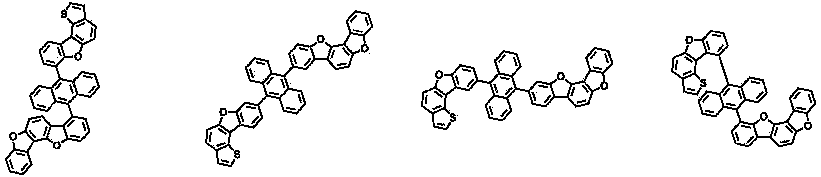

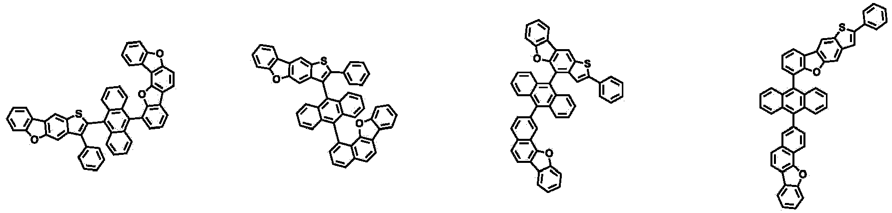

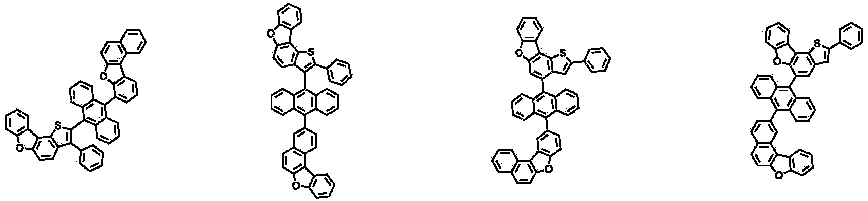

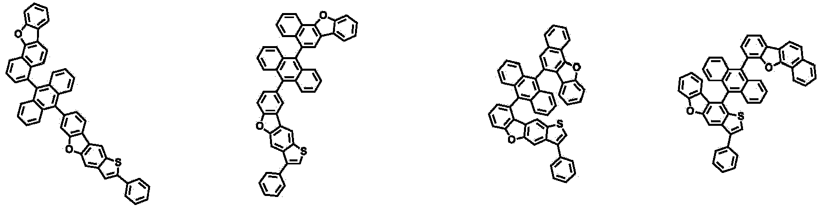

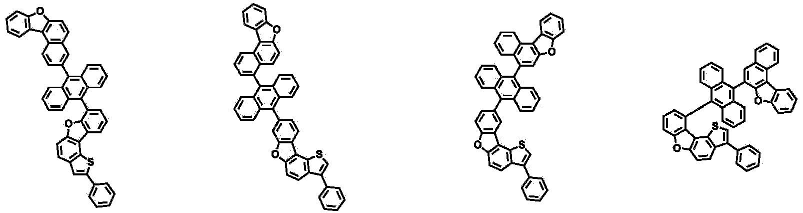

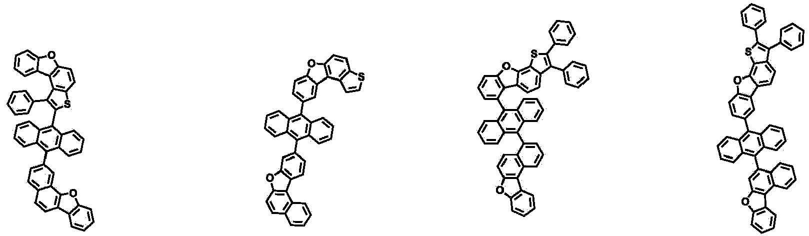

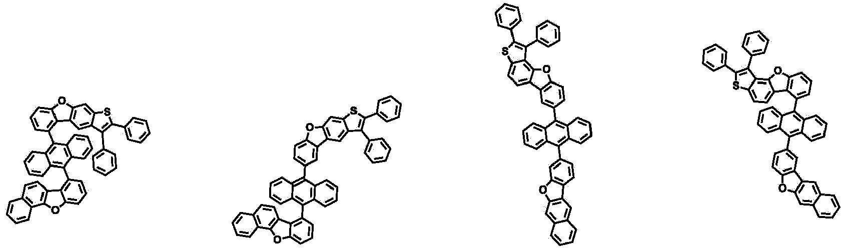

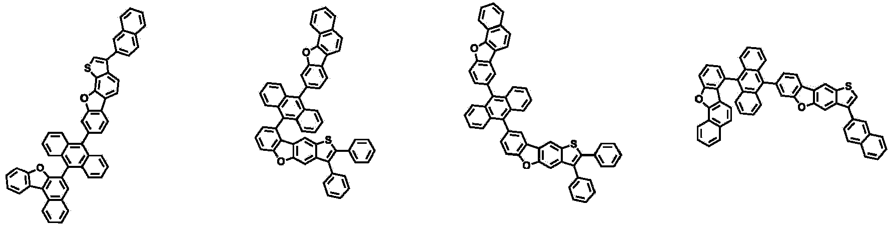

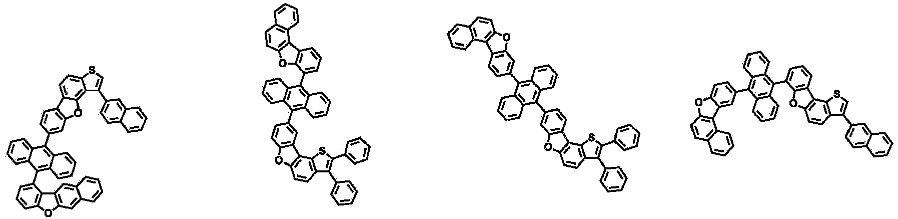

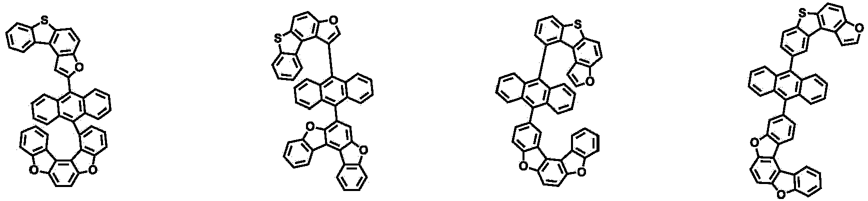

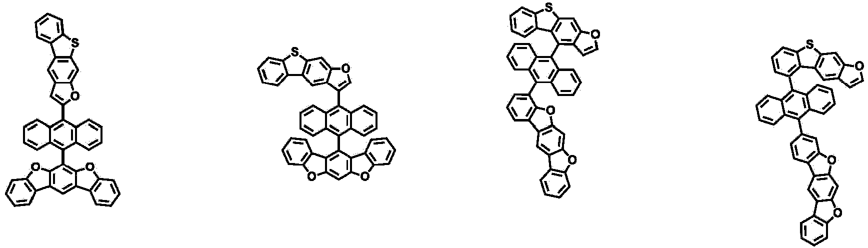

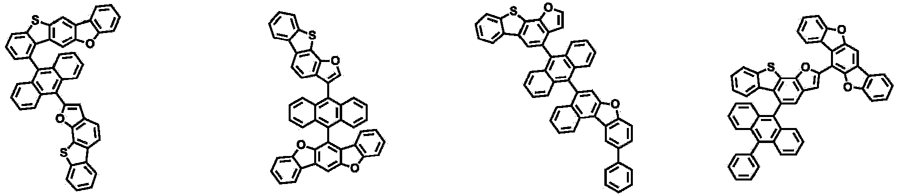

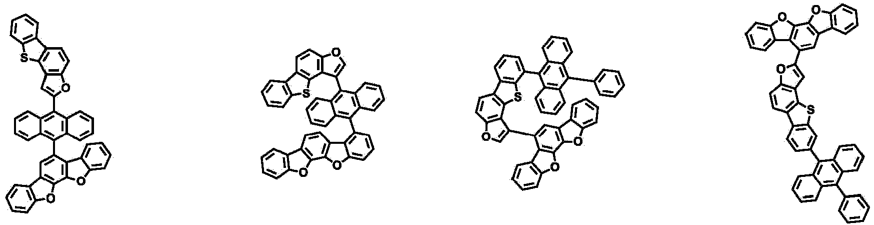

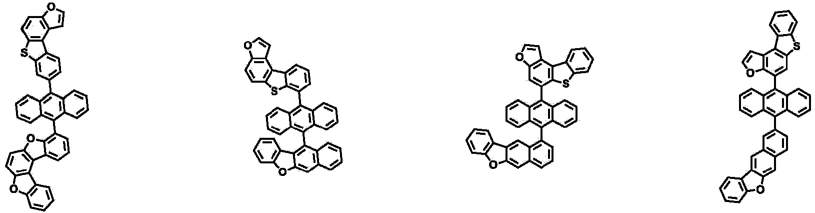

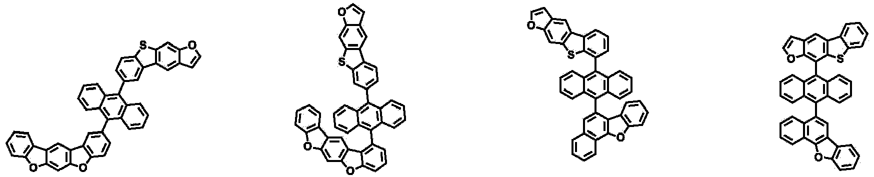

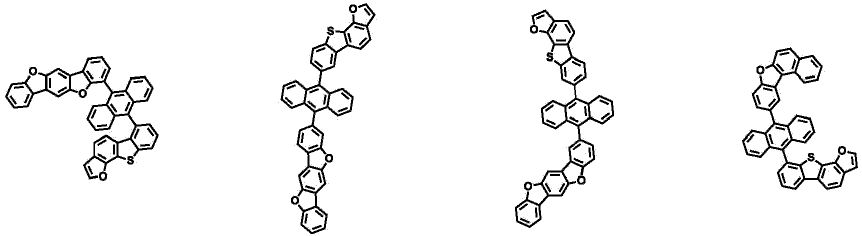

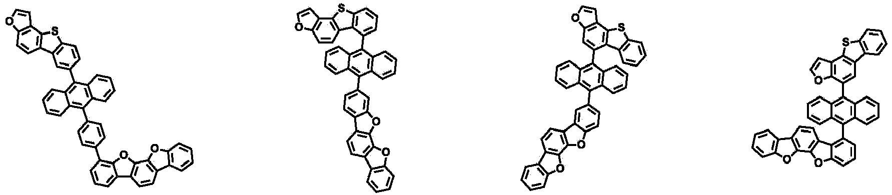

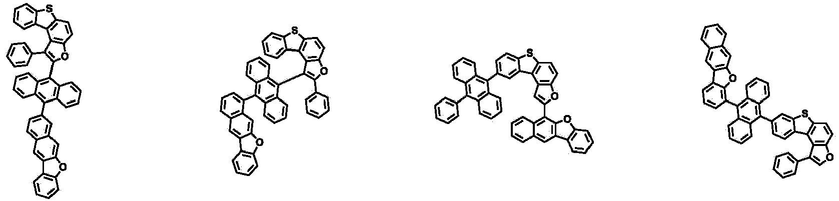

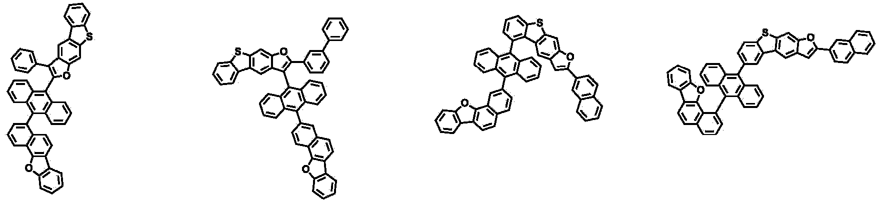

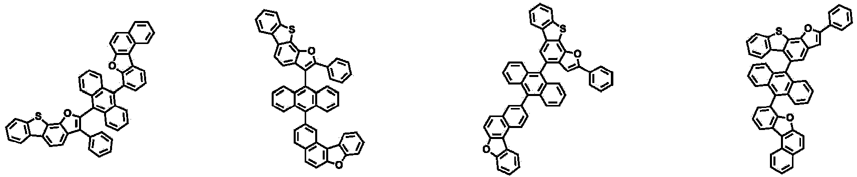

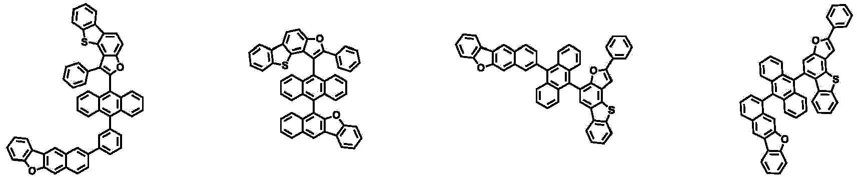

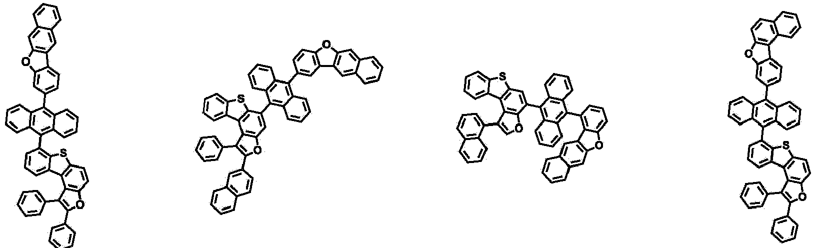

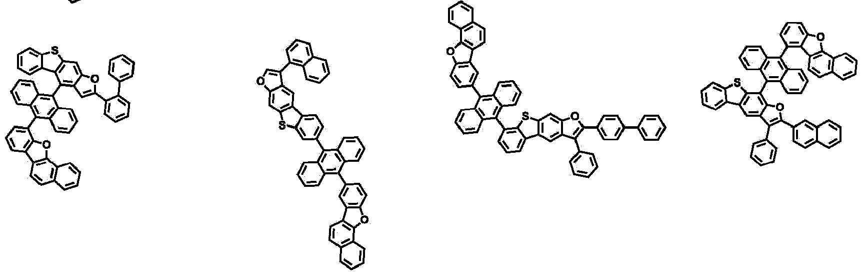

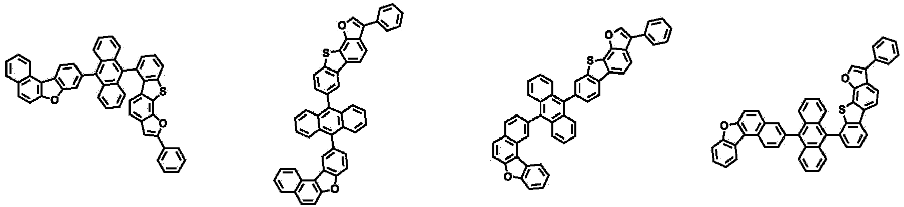

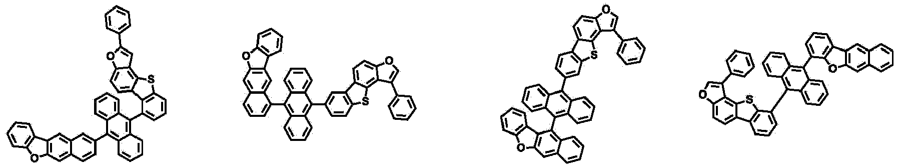

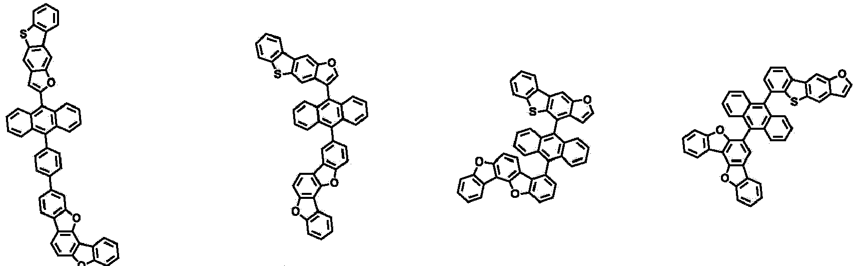

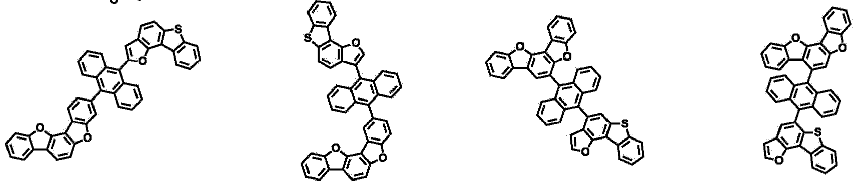

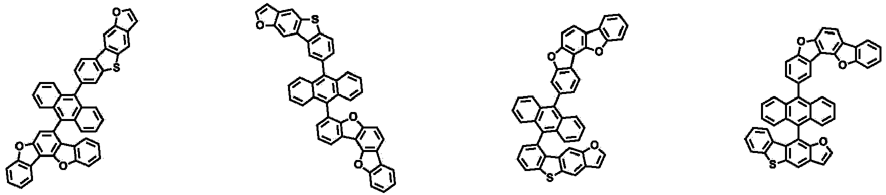

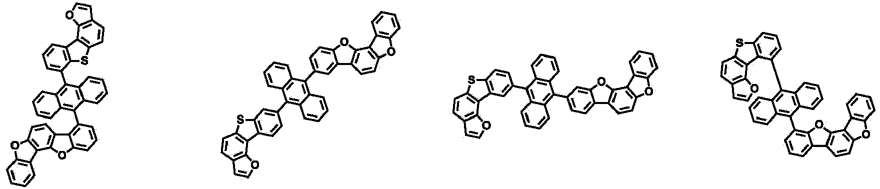

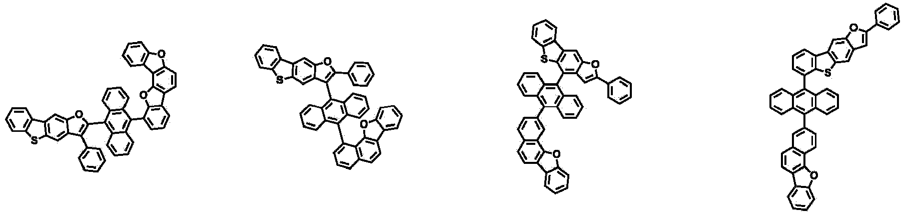

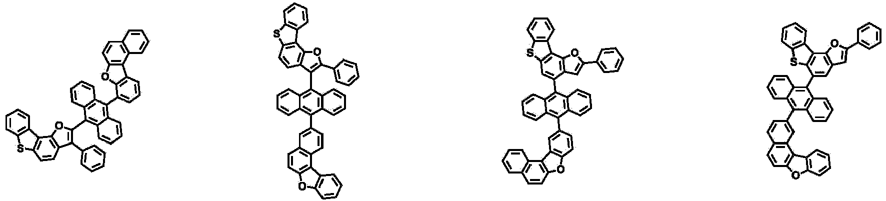

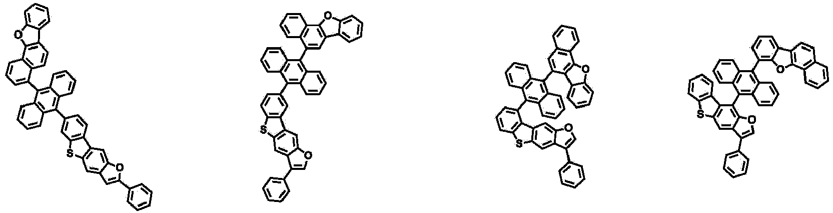

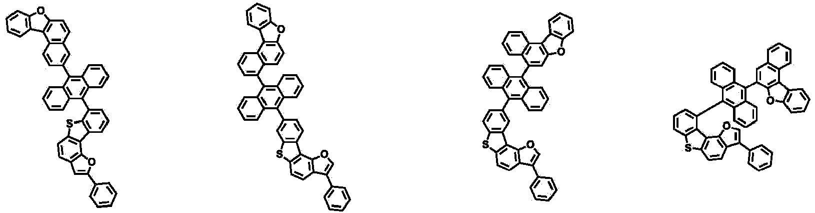

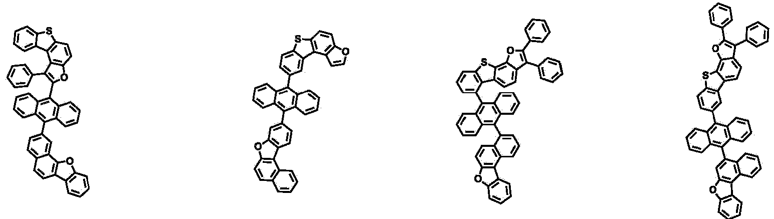

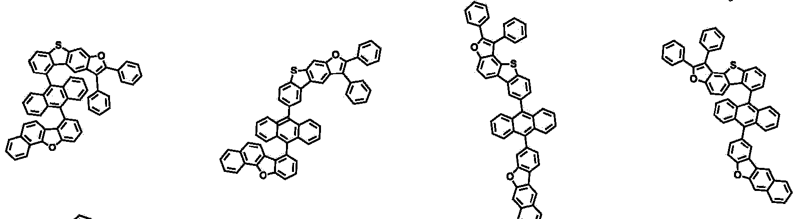

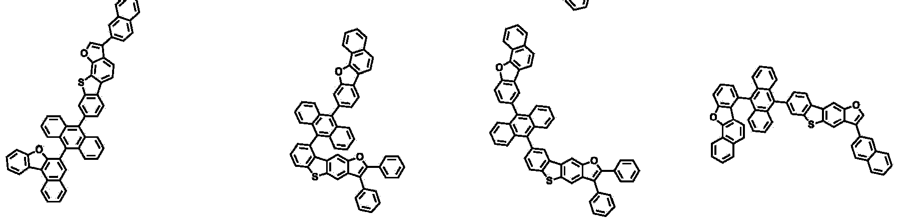

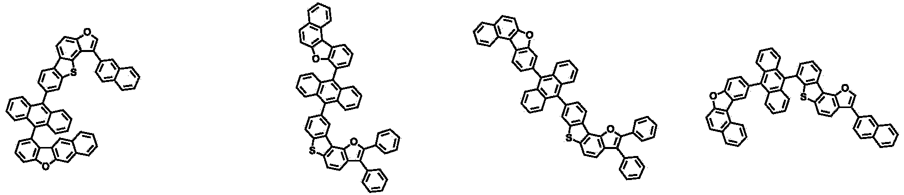

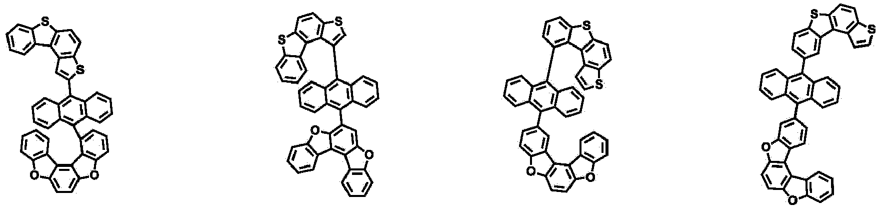

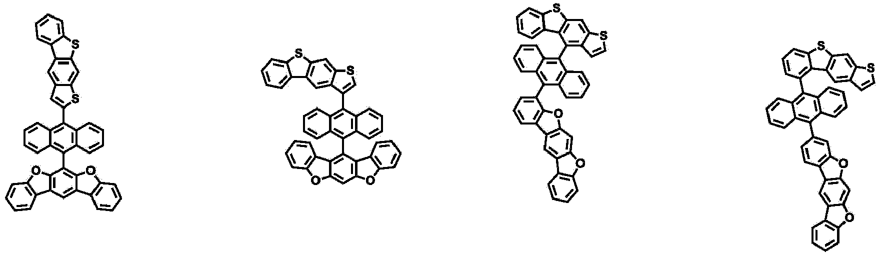

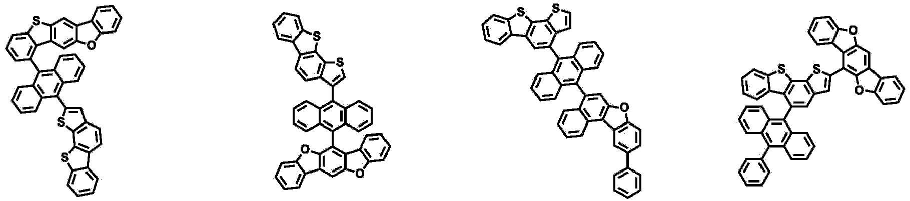

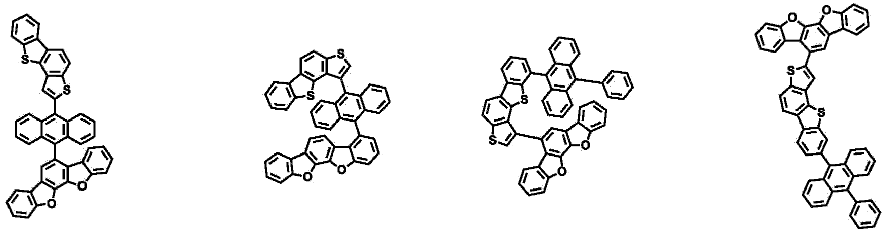

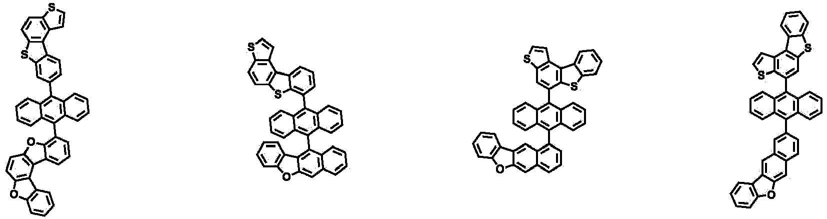

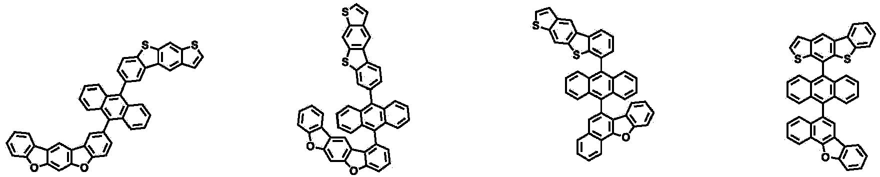

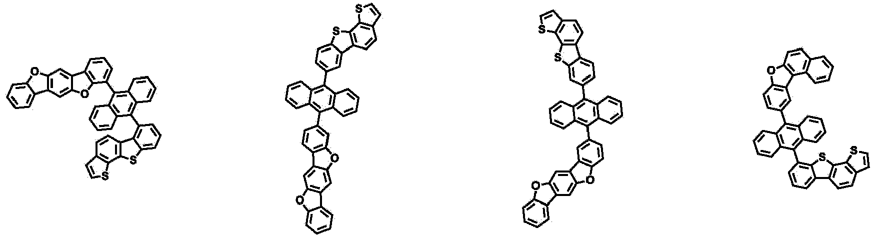

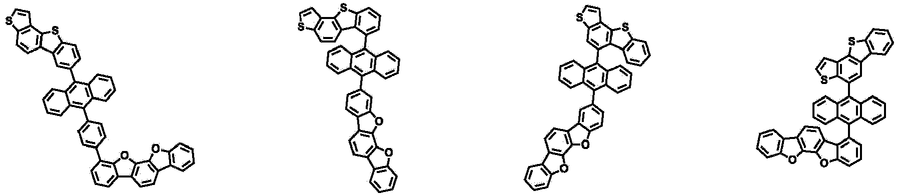

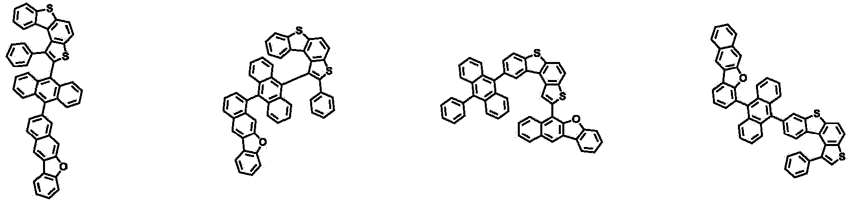

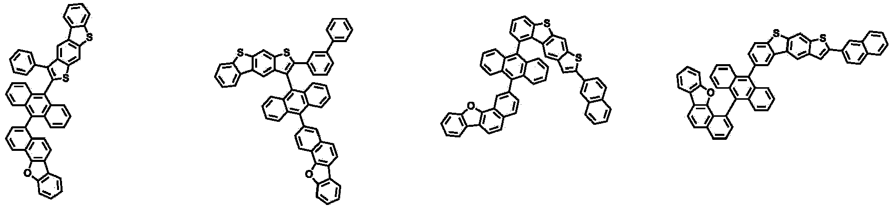

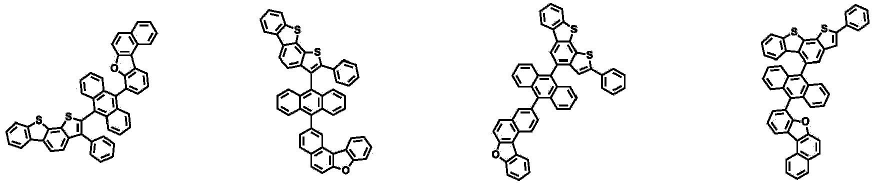

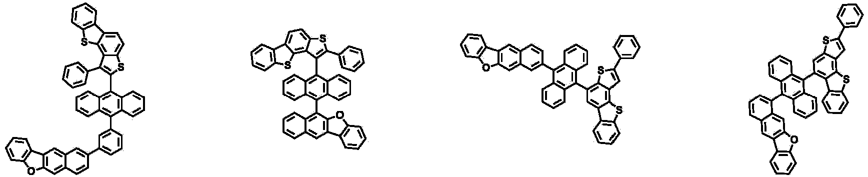

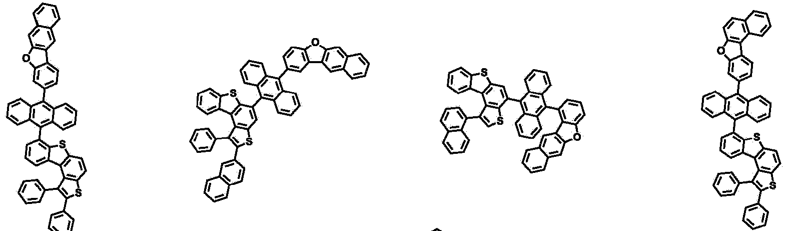

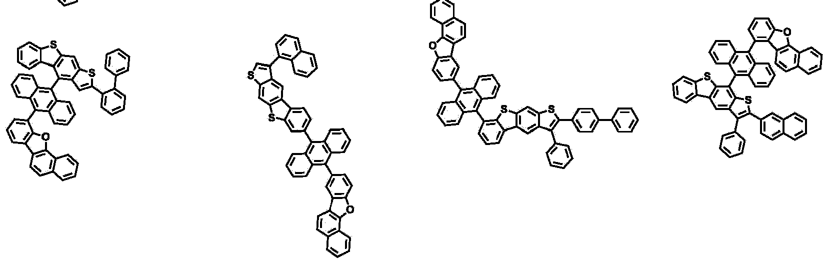

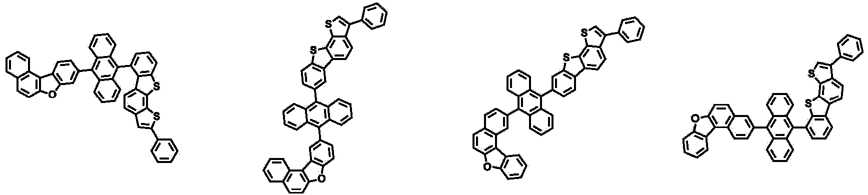

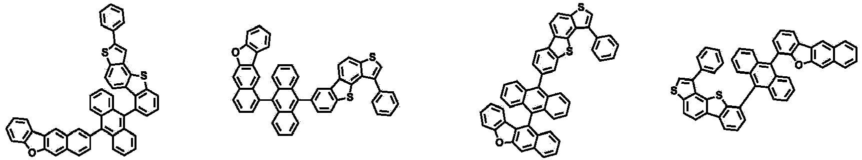

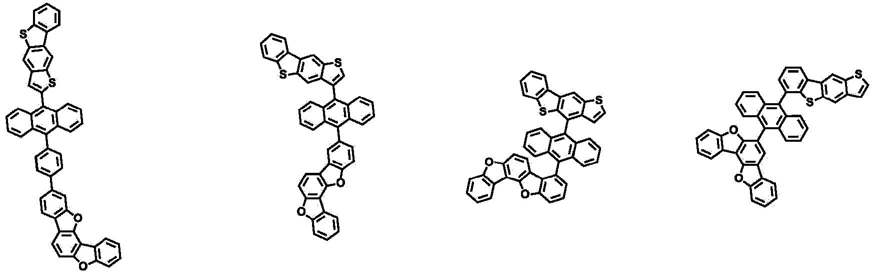

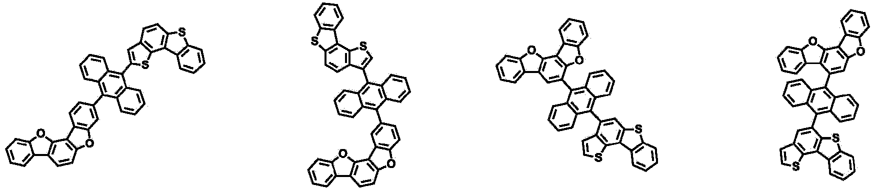

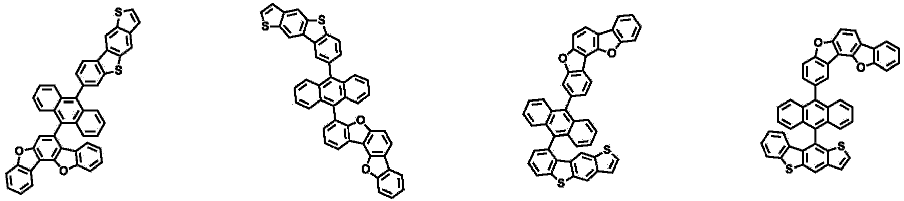

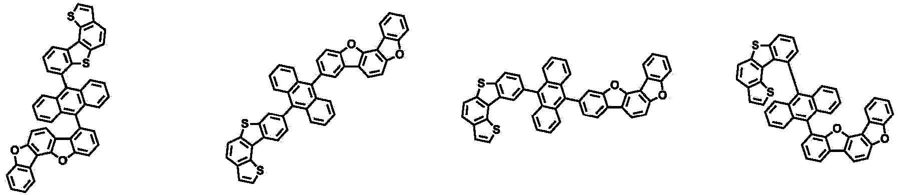

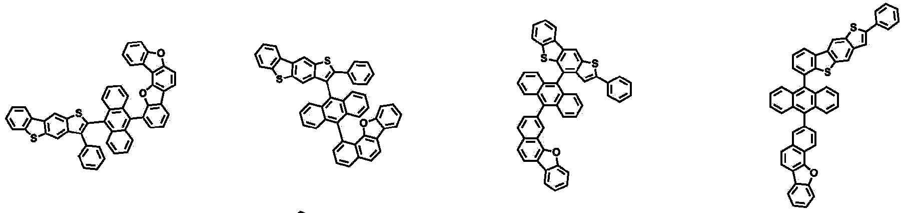

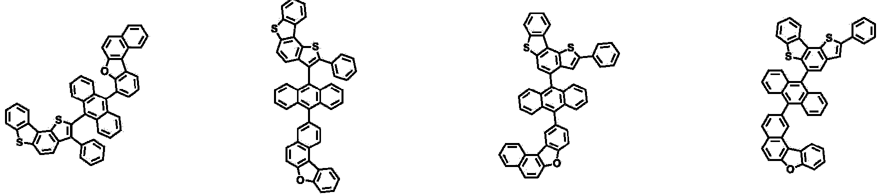

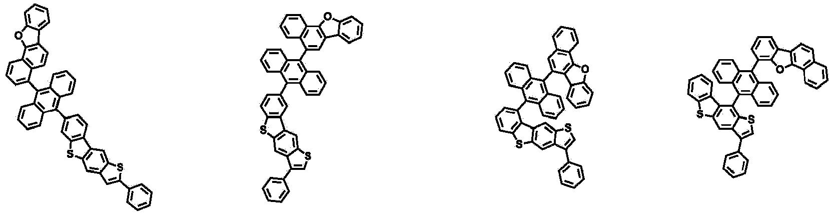

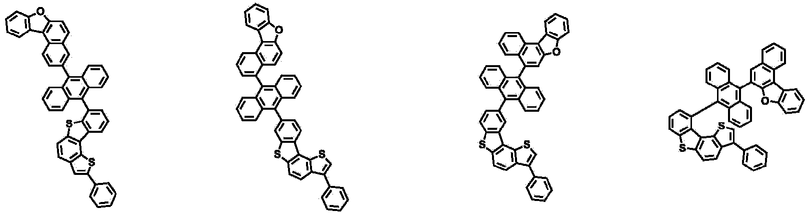

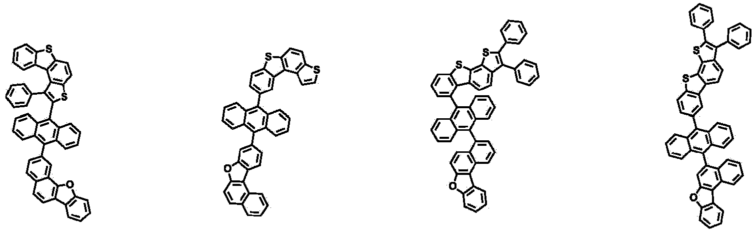

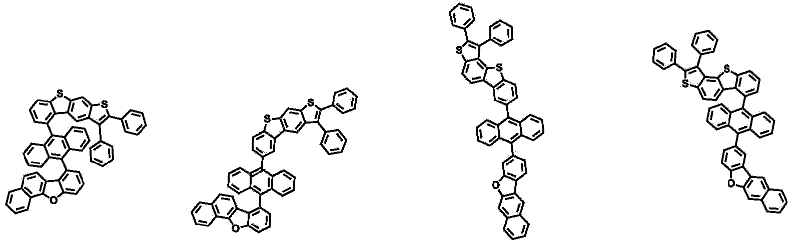

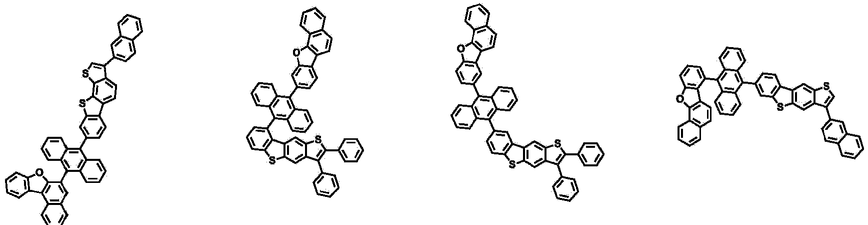

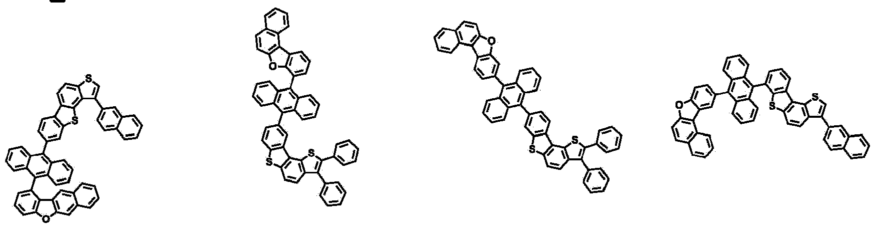

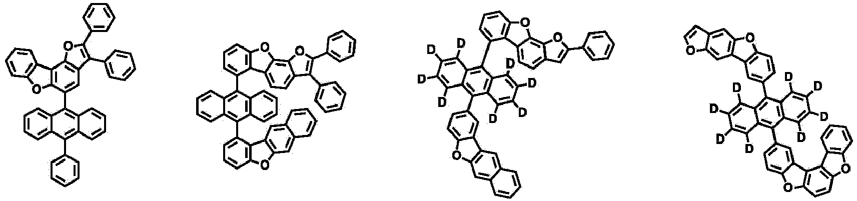

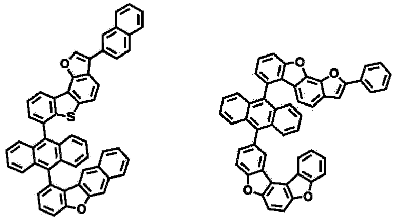

Compounds according to the above-described exemplary embodiments have stable forms in both oxidized and reduced states, and have high light emitting efficiency in converting excitons into light when the excitons are formed.

In addition, when the compound of the present invention is used in at least one layer of a hole transport layer, a hole injection layer, an electron transport layer, an electron injection layer, and a light emitting layer, it is possible to obtain an effect in which the driving voltage of the device is lowered, or the efficiency of the device is increased.

In particular, when the compound of the present invention is used as a blue host in the light emitting layer, it is possible to obtain an effect in which the driving voltage of the device is lowered, or the efficiency of the device is increased.

The substituents of the compound of Chemical Formula 1 may be bonded by a method known in the art according to the following Reaction Scheme 1, and the type and position of the substituent or the number of substituents may be changed according to the technology known in the art. Furthermore, Q, L and Arₗ mentioned in the following Reaction Scheme 1 are the same as those previously defined.

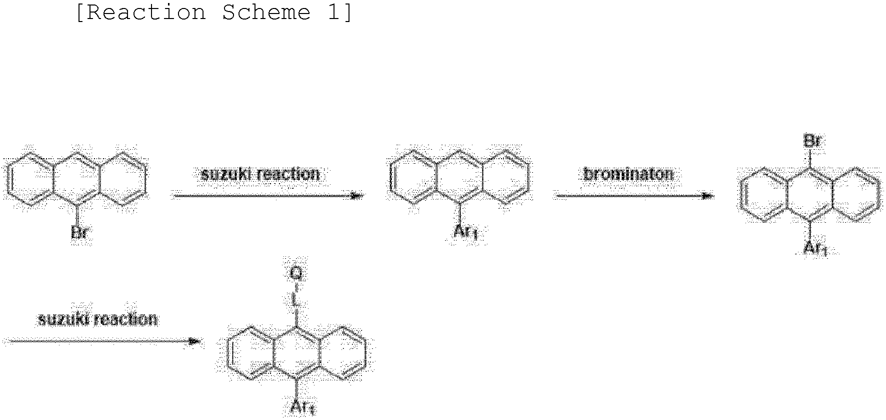

Further, in the present specification, a compound having inherent characteristics may be synthesized by introducing a heterocyclic group (Q) including two O's or S's into the anthracene core as described above. Specifically, a substituent usually used for a hole injection layer material, a hole transport layer material, a light emitting layer material, an electron transport layer material, an electron injection layer material, and the like, which are used when an organic light emitting device is manufactured, may be introduced into the core structure to synthesize a material which satisfies conditions required for each organic material layer.

In particular, in the present specification, it is possible to synthesize a material which satisfies conditions required for the light emitting layer by introducing into the anthracene core a heterocyclic group, which is usually used in the light emitting layer material used when an organic light emitting device is manufactured.

Further, in the present specification, compounds having various energy bandgaps may be synthesized due to the core structure described above. In addition, in the present invention, various substituents may be introduced into the core structure described above to adjust the HOMO and LUMO energy levels of a compound.

According to another exemplary embodiment of the present specification, provided is an organic light emitting device including: a first electrode; a second electrode provided so as to face the first electrode; and an organic material layer having one or more layers provided between the first electrode and the second electrode, in which one or more layers of the organic material layer include the compound according to any one of the exemplary embodiments.

Since the organic light emitting device according to the above-described exemplary embodiment includes a compound that has a stable form in both oxidation and reduction states, and uses a compound having high light emitting efficiency that converts excitons into light when the excitons are formed, it is possible to obtain an effect in which the driving voltage of the device is lowered, or the efficiency of the device is increased.

The organic light emitting device of the present invention may be manufactured using typical manufacturing methods and materials of an organic light emitting device, except that the above-described compound is used to form an organic material layer having one or more layers or a light emitting layer.

The compound may be formed as an organic material layer or a light emitting layer by not only a vacuum deposition method, but also a solution application method when an organic light emitting device is manufactured. Here, the solution application method means spin coating, dip coating, inkjet printing, screen printing, a spray method, roll coating, and the like, but is not limited thereto.

The organic material layer of the organic light emitting device of the present invention may be composed of a single-layered structure, but may be composed of a multi-layered structure in which two or more organic material layers are stacked. For example, the organic light emitting device of the present invention may have a structure including a hole injection layer, a hole transport layer, a light emitting layer, an electron transport layer, an electron injection layer, and the like as organic material layers. However, the structure of the organic light emitting device is not limited thereto, and may include a fewer or greater number of organic material layers.

According to another exemplary embodiment of the present specification, in the organic light emitting device, the organic material layer may include one or more layers of a hole transport layer, a hole injection layer, a light emitting layer, an electron injection layer, and an electron transport layer, and the one or more layers may include the compound of Chemical Formula 1.

According to still another exemplary embodiment of the present specification, provided is an organic light emitting device in which the organic material layer includes a light emitting layer, and the light emitting layer includes the compound.

According to yet another exemplary embodiment of the present specification, provided is an organic light emitting device in which the light emitting layer includes the compound as a blue host.

Since the organic light emitting device according to the above-described exemplary embodiment has a stable form in both oxidation and reduction states, and uses a compound having high light emitting efficiency that converts excitons into light when the excitons are formed, it is possible to obtain an effect in which the driving voltage of the device is lowered, or the efficiency of the device is increased.

In the organic light emitting device of the present specification, the organic material layer includes an electron transport layer, and the electron transport layer includes the compound of Chemical Formula 1.

In the organic light emitting device of the present specification, the organic material layer includes an electron injection layer, and the electron injection layer includes the compound of Chemical Formula 1.

In the organic light emitting device of the present specification, the organic material layer includes a light emitting layer, and the light emitting layer includes the compound of Chemical Formula 1.

According to another exemplary embodiment, the organic material layer includes a light emitting layer, and the light emitting layer may include the compound of Chemical Formula 1 as a host of the light emitting layer.

According to still another exemplary embodiment, the organic material layer includes a light emitting layer, and the light emitting layer may include the compound of Chemical Formula 1 as a phosphorescent host of the light emitting layer.

As still another example, the organic material layer includes a light emitting layer, and the light emitting layer includes the compound of Chemical Formula 1 as a host of the light emitting layer, and may further include a dopant.

As still another example, the organic material layer includes a light emitting layer, and the light emitting layer includes the compound of Chemical Formula 1 as a host of the light emitting layer, and may further include a dopant. The dopant may be included in an amount of 1 part by weight to 20 parts by weight, more preferably 1 part by weight to 5 parts by weight, with respect to 100 parts by weight of the host.

According to yet another exemplary embodiment, the organic material layer includes a light emitting layer, and the light emitting layer may include the compound of Chemical Formula 1 as a dopant of the light emitting layer.

In another exemplary embodiment, the organic material layer includes a light emitting layer, and the light emitting layer includes the compound of Chemical Formula 1 as a dopant of the light emitting layer, and may further include a host.

In still another exemplary embodiment, the organic material layer includes a light emitting layer, and the light emitting layer includes the compound of Chemical Formula 1 as a dopant of the light emitting layer, includes a fluorescent host or a phosphorescent host, and may include another organic compound, a metal, or a metal compound as a dopant.

As another example, the organic material layer includes a light emitting layer, the light emitting layer includes the compound of Chemical Formula 1 as a blue host of the light emitting layer, and includes a fluorescent host or a phosphorescent host, and the above-described compound may be used along with an iridium (Ir)-based dopant.

As still another example, the organic material layer includes a light emitting layer, the light emitting layer includes the compound of Chemical Formula 1 as a dopant of the light emitting layer, and includes a fluorescent host or a phosphorescent host, and the above-described compound may be used along with an iridium (Ir)-based dopant.

In the organic light emitting device of the present specification, the organic material layer may include a hole injection layer or a hole transport layer, and the hole injection layer or the hole transport layer may include the compound of Chemical Formula 1.

In the organic light emitting device of the present specification, the organic material layer includes an electron blocking layer, and the electron blocking layer may include the compound of Chemical Formula 1.

In the organic light emitting device of the present specification, the electron injection layer and/or the electron transport layer include(s) the compound of Chemical Formula 1 and a metal complex.

In the organic light emitting device of the present specification, the electron injection layer and/or the electron transport layer include(s) the compound of Chemical Formula 1 and lithium quinolate (LiQ).

In the organic light emitting device of the present specification, the electron injection layer and/or the electron transport layer may include the compound of Chemical Formula 1 and a metal complex at a weight ratio of 1:10 to 10:1.

In the organic light emitting device of the present specification, the electron injection layer and/or the electron transport layer may include the compound of Chemical Formula 1 and a metal complex at a weight ratio of 1:5 to 5:1.

In the organic light emitting device of the present specification, the electron injection layer and/or the electron transport layer may include the compound of Chemical Formula 1 and a metal complex at a weight ratio of 1:3 to 3:1.

In the organic light emitting device of the present specification, the electron injection layer and/or the electron transport layer may include the compound of Chemical Formula 1 and lithium quinolate at a weight ratio of 1:10 to 10:1.

In the organic light emitting device of the present specification, the electron injection layer and/or the electron transport layer may include the compound of Chemical Formula 1 and lithium quinolate at a weight ratio of 1:5 to 5:1.

In the organic light emitting device of the present specification, the electron injection layer and/or the electron transport layer may include the compound of Chemical Formula 1 and lithium quinolate at a weight ratio of 1:3 to 3:1.
(1) Anode/Hole transport layer/Light emitting layer/Cathode
(2) Anode/Hole injection layer/Hole transport layer/Light emitting layer/Cathode
(3) Anode/Hole injection layer/Hole buffer layer/Hole transport layer/Light emitting layer/Cathode
(4) Anode/Hole transport layer/Light emitting layer/Electron transport layer/Cathode
(5) Anode/Hole transport layer/Light emitting layer/Electron transport layer/Electron injection layer/Cathode
(6) Anode/Hole injection layer/Hole transport layer/Light emitting layer/Electron transport layer/Cathode
(7) Anode/Hole injection layer/Hole transport layer/Light emitting layer/Electron transport layer/Electron injection layer/Cathode
(8) Anode/Hole injection layer/Hole buffer layer/Hole transport layer/Light emitting layer/Electron transport layer/Cathode
(9) Anode/Hole injection layer/Hole buffer layer/Hole transport layer/Light emitting layer/Electron transport layer/Electron injection layer/Cathode
(10) Anode/Hole transport layer/Electron blocking layer/Light emitting layer/Electron transport layer/Cathode
(11) Anode/Hole transport layer/Electron blocking layer/Light emitting layer/Electron transport layer/Electron injection layer/Cathode
(12) Anode/Hole injection layer/Hole transport layer/Electron blocking layer/Light emitting layer/Electron transport layer/Cathode
(13) Anode/Hole injection layer/Hole transport layer/Electron blocking layer/Light emitting layer/Electron transport layer/Electron injection layer/Cathode
(14) Anode/Hole transport layer/Light emitting layer/Hole blocking layer/Electron transport layer/Cathode
(15) Anode/Hole transport layer/Light emitting layer/Hole blocking layer/Electron transport layer/Electron injection layer/Cathode
(16) Anode/Hole injection layer/Hole transport layer/Light emitting layer/Hole blocking layer/Electron transport layer/Cathode
(17) Anode/Hole injection layer/Hole transport layer/Light emitting layer/Hole blocking layer/Electron transport layer/Electron injection layer/Cathode
(18) Anode/Hole injection layer/Hole transport layer/Electron blocking layer/Light emitting layer/Hole blocking layer/Electron injection and transport layer/Cathode

The structure of the organic light emitting device of the present invention may have a structure illustrated in FIG. 1, but is not limited thereto.

FIG. 1 exemplifies the structure of an organic light emitting device in which an anode 2, a light emitting layer 3, and a cathode 4 are sequentially stacked on a substrate 1. In the structure described above, the compound of Chemical Formula 1 may be included in the organic material layer 3.

For example, the organic light emitting device according to the present invention may be manufactured by depositing a metal or a metal oxide having conductivity, or an alloy thereof on a substrate to form an anode, forming an organic material layer having one or more layers selected from the group consisting of a hole injection layer, a hole transport layer, a layer which transports and injects holes simultaneously (a hole transport and hole injection layer), a light emitting layer, an electron transport layer, an electron injection layer, and a layer which transports and injects electrons simultaneously (an electron transport and electron injection layer), thereon, and then depositing a material, which may be used as a cathode, thereon, by using a physical vapor deposition (PVD) method such as sputtering or e-beam evaporation. In addition to the method described above, an organic light emitting device may be made by sequentially depositing a cathode material, an organic material layer, and an anode material on a substrate.

The organic material layer may have a multi-layered structure including a hole injection layer, a hole transport layer, a light emitting layer, and an electron transport layer, and the like, but is not limited thereto and may have a single-layered structure. Further, the organic material layer may be manufactured to include a fewer number of layers by a method such as a solvent process, for example, spin coating, dip coating, doctor blading, screen printing, inkjet printing, or a thermal transfer method, using various polymer materials, instead of a deposition method.

The anode is an electrode which injects holes, and as an anode material, materials having a high work function are usually preferred so as to facilitate the injection of holes into an organic material layer. Specific examples of the anode material which may be used in the present invention include: a metal, such as vanadium, chromium, copper, zinc, and gold, or an alloy thereof; a metal oxide, such as zinc oxide, indium oxide, indium tin oxide (ITO), and indium zinc oxide (IZO); a combination of a metal and an oxide, such as ZnO : Al or SnO₂ : Sb; a conductive polymer, such as poly(3-methylthiophene), poly[3,4-(ethylene-1,2-dioxy)thiophene] (PEDOT), polypyrrole, and polyaniline; and the like, but are not limited thereto.

The cathode is an electrode which injects electrons, and as a cathode material, materials having a low work function are usually preferred so as to facilitate the injection of electrons into an organic material layer. Specific examples of the cathode material include: a metal such as magnesium, calcium, sodium, potassium, titanium, indium, yttrium, lithium, gadolinium, aluminum, silver, tin, and lead, or an alloy thereof; a multi-layer structured material, such as LiF/Al or LiO₂/Al; and the like, but are not limited thereto.

The hole injection layer is a layer which serves to facilitate the injection of holes from an anode to a light emitting layer, and a hole injection material is preferably a material which may proficiently accept holes from an anode at a low voltage, and the highest occupied molecular orbital (HOMO) of the hole injection material is preferably a value between the work function of the anode material and the HOMO of the neighboring organic material layer. Specific examples of the hole injection material include metal porphyrin, oligothiophene, arylamine-based organic materials, hexanitrile hexaazatriphenylene-based organic materials, quinacridone-based organic materials, perylene-based organic materials, anthraquinone, polyaniline-based and polythiophene-based conductive polymers, and the like, but are not limited thereto.

The hole injection layer may have a thickness of 1 nm to 150 nm. When the hole injection layer has a thickness of 1 nm or more, there is an advantage in that it is possible to prevent hole injection characteristics from deteriorating, and when the hole injection layer has a thickness of 150 nm or less, there is an advantage in that it is possible to prevent the driving voltage from being increased in order to improve the movement of holes due to the too thick hole injection layer.

An electron blocking layer may be provided between the hole transport layer and the light emitting layer. The electron blocking layer is a layer which may improve the service life and efficiency of the device by preventing holes injected from a hole injection layer from passing through a light emitting layer and entering an electron injection layer, and the compound of Chemical Formula 1 or a material known in the art may be used.

The light emitting layer may emit red, green, or blue light, and may be composed of a phosphorescent material or a fluorescent material. The light emitting material is a material which may receive holes and electrons from a hole transport layer and an electron transport layer, respectively, and combine the holes and the electrons to emit light in a visible ray region, and is preferably a material having high quantum efficiency for fluorescence or phosphorescence. Specific examples thereof include 8-hydroxy-quinoline aluminum complexes (Alq₃); carbazole-based compounds; dimerized styryl compounds; BAlq; 10-hydroxybenzoquinoline-metal compounds, benzoxazole-based, benzothiazole-based and benzimidazole-based compounds; poly(p-phenylenevinylene) (PPV)-based polymers; spiro compounds; a polyfluorene group, rubrene, and the like, but are not limited thereto.

Examples of the host material for the light emitting layer include fused aromatic ring derivatives, or hetero ring-containing compounds, and the like. Specifically, examples of the fused aromatic ring derivative include anthracene derivatives, pyrene derivatives, naphthalene derivatives, pentacene derivatives, phenanthrene compounds, fluoranthene compounds, and the like, and examples of the hetero ring-containing compound include carbazole derivatives, dibenzofuran derivatives, ladder-type furan compounds, pyrimidine derivatives, and the like, but the examples thereof are not limited thereto.

When the light emitting layer emits red light, it is possible to use a phosphorescent material such as bis(1-phenylisoquinoline)acetylacetonate iridium (PIQIr(acac)), bis(1-phenylquinoline)acetylacetonate iridium (PQIr(acac)), tris(1-phenylquinoline)iridium (PQIr), or octaethylporphyrin platinum (PtOEP), or a fluorescent material such as tris(8-hydroxyquinolino)aluminum (Alq₃) as a light emitting dopant, but the light emitting dopant is not limited thereto. When the light emitting layer emits green light, it is possible to use a phosphorescent material such as fac tris(2-phenylpyridine)iridium (Ir(ppy)₃), or a fluorescent material such as tris(8-hydroxyquinolino)aluminum (Alq₃), as the light emitting dopant, but the light emitting dopant is not limited thereto. When the light emitting layer emits blue light, it is possible to use a phosphorescent material such as (4,6-F2ppy)₂Irpic, or a fluorescent material such as spiro-DPVBi, spiro-6P, distyryl benzene(DSB), distyryl arylene (DSA), a PFO-based polymer or a PPV-based polymer as the light emitting dopant, but the light emitting dopant is not limited thereto.

A hole blocking layer may be provided between the electron transport layer and the light emitting layer, and the hole blocking layer is a layer which blocks holes from reaching a negative electrode, and may be generally formed under the same conditions as those of the hole injection layer. Specifically, examples of a hole blocking material include triazine derivatives, phenanthroline derivatives, BCP, and the like, but are not limited to these, and materials known in the art may be used.

The electron transport layer may serve to facilitate the transport of electrons. An electron transport material is suitably a material having high electron mobility which may proficiently accept electrons from a negative electrode and transfer the electrons to a light emitting layer. Specific examples thereof include: Al complexes of 8-hydroxyquinoline; complexes including Alq₃; organic radical compounds; hydroxyflavone-metal complexes; and the like, but are not limited thereto.

The electron transport layer may have a thickness of 1 nm to 50 nm. When the electron transport layer has a thickness of 1 nm or more, there is an advantage in that it is possible to prevent electron transport characteristics from deteriorating, and when the electron transport layer has a thickness of 50 nm or less, there is an advantage in that it is possible to prevent the driving voltage from being increased in order to improve the movement of electrons due to the too thick electron transport layer.

The electron injection layer may serve to facilitate the injection of electrons. An electron injection material is preferably a compound which has a capability of transporting electrons, an effect of injecting electrons from a negative electrode, and an excellent effect of injecting electrons into a light emitting layer or a light emitting material, prevents excitons produced from a light emitting layer from moving to a hole injection layer, and is also excellent in the ability to form a thin film. Specific examples thereof include fluorenone, anthraquinodimethane, diphenoquinone, thiopyran dioxide, oxazole, oxadiazole, triazole, imidazole, perylenetetracarboxylic acid, fluorenylidene methane, anthrone, and the like, and derivatives thereof, metal complex compounds, nitrogen-containing 5-membered ring derivatives, and the like, but are not limited thereto.

Examples of the metal complex compounds include 8-hydroxyquinolinato lithium, bis(8-hydroxyquinolinato) zinc, bis(8-hydroxyquinolinato) copper, bis(8-hydroxyquinolinato) manganese, tris(8-hydroxyquinolinato) aluminum, tris(2-methyl-8-hydroxyquinolinato) aluminum, tris(8-hydroxyquinolinato) gallium, bis(10-hydroxybenzo[h]quinolinato) beryllium, bis(10-hydroxybenzo[h]quinolinato) zinc, bis(2-methyl-8-quinolinato) chlorogallium, bis(2-methyl-8-quinolinato) (o-cresolato) gallium, bis(2-methyl-8-quinolinato) (1-naphtholato) aluminum, bis(2-methyl-8-quinolinato) (2-naphtholato) gallium, and the like, but are not limited thereto.

The organic light emitting device according to the present invention may be a top emission type, a bottom emission type, or a dual emission type according to the material to be used.

### [Mode for Invention]

Hereinafter, the present specification will be described in detail with reference to Examples for specifically describing the present specification. However, the Examples according to the present specification may be modified in various forms, and it is not interpreted that the scope of the present application is limited to the Examples described in detail below. The Examples of the present application are provided to explain the present specification more completely to a person with ordinary skill in the art.

### <Preparation Examples> Preparation of precursor of Q Preparation Example 1.

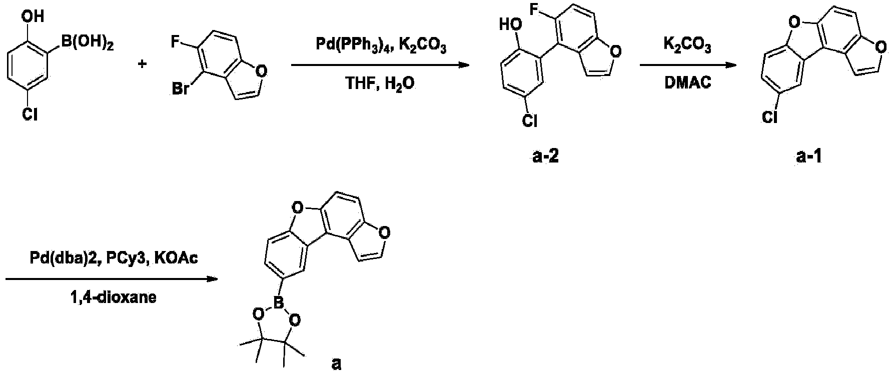

### 1) Preparation of Chemical Formula a-2

Under a nitrogen atmosphere, (5-chloro-2-hydroxyphenyl)boronic acid (100 g, 580.1 mmol) and 4-bromo-5-fluorobenzofuran (124.7 g, 580.1 mmol) were added to 2,000 ml of THF, and the resulting solution was stirred and refluxed. Thereafter, potassium carbonate (320.7 g, 2320.6 mmol) was dissolved in 962 ml of water, the resulting solution was introduced thereinto, the resulting mixture was sufficiently stirred, and then tetrakis(triphenylphosphine)-palladium(0) (20.1 g, 17.4 mmol) was introduced thereinto. After reacting for 9 hours, the resulting product was cooled to room temperature, an organic layer and an aqueous layer were separated, and then the organic layer was distilled. The distilled organic layer was again dissolved in chloroform, the solution was washed twice with water, and then the organic layer was separated, anhydrous magnesium sulfate was added thereto, and the resulting mixture was stirred, and then filtered, and the filtrate was distilled under reduced pressure. The concentrated compound was purified by silica gel column chromatography to prepare 106.7 g of Compound a-2. (Yield 70%, MS: [M+H]+= 264)

### 2) Preparation of Chemical Formula a-1

Under a nitrogen atmosphere, a-2 (106.7 g, 406.2 mmol) was added to 2134 ml of DMAC, and the resulting solution was stirred under reflux. Thereafter, potassium carbonate (168.4 g, 1218.7 mmol) was introduced thereinto, and the resulting mixture was stirred. After reacting for 1 hour, the resulting product was cooled to room temperature, and 2.5 L of water was added to solidify the product, followed by filtration. The product was again dissolved in chloroform, the solution was washed twice with water, and then the organic layer was separated, anhydrous magnesium sulfate was added thereto, and the resulting mixture was stirred, and then filtered, and the filtrate was distilled under reduced pressure. The concentrated compound was purified by silica gel column chromatography to prepare 69g of Compound a-1.
(Yield 70%, MS: [M+H]+= 244)

### 3) Preparation of Chemical Formula a

Under a nitrogen atmosphere, a-1 (69 g, 284.3 mmol) and bis(pinacolato)diboron (86.6 g, 341.2 mmol) were stirred under reflux in 1380 ml of 1,4-dioxane. Thereafter, potassium acetate (41.9 g, 426.5mmol) was introduced thereinto, the resulting mixture was sufficiently stirred, and then bis(dibenzylideneacetone)palladium(0) (4.9 g, 8.5 mmol) and tricyclohexylphosphine (4.8 g, 17.1 mmol) were introduced thereinto. After reacting for 7 hours, the resulting product was cooled to room temperature, an organic layer was separated using chloroform and water, and then the organic layer was distilled. The distilled organic layer was again dissolved in chloroform, the solution was washed twice with water, and then the organic layer was separated, anhydrous magnesium sulfate was added thereto, and the resulting mixture was stirred, and then filtered, and the filtrate was distilled under reduced pressure. The concentrated compound was purified by silica gel column chromatography to prepare 70.3 g of Compound a. (Yield 74%, MS: [M+H]+= 335)

### Preparation Example 2.

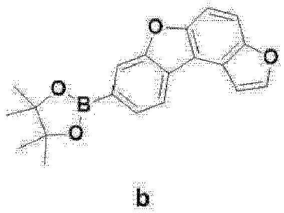

### 1) Preparation of Chemical Formula b

Chemical Formula b was synthesized in the same manner as in the method of preparing Chemical Formula a using (4-chloro-2-hydroxyphenyl)boronic acid instead of (5-chloro-2-hydroxyphenyl)boronic acid.

### Preparation Example 3.

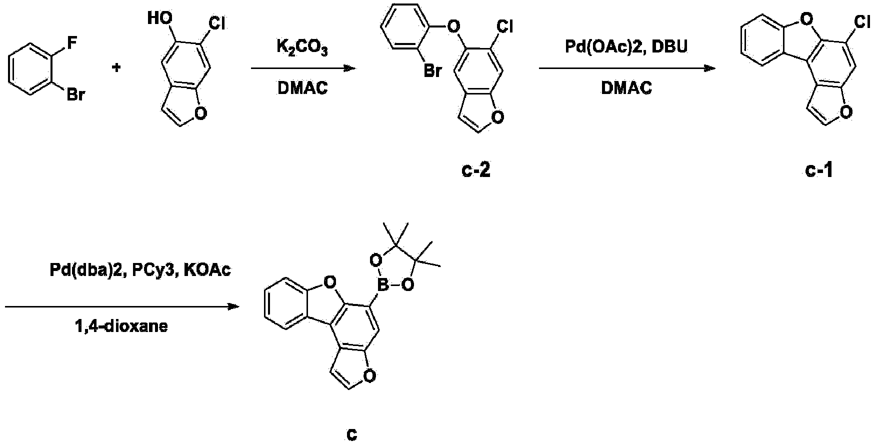

### 1) Preparation of Chemical Formula c-2

Under a nitrogen atmosphere, 1-bromo-2-fluorobenzene (60 g, 342.9 mmol) and 6-chlorobenzofuran-5-ol (57.8 g, 342.9 mmol) were added to 1,200 ml of DMAC, and the resulting solution was stirred under reflux. Thereafter, potassium carbonate (142.2g, 1028.6mmol) was introduced thereinto, and the resulting mixture was stirred. After reacting for 1 hour, the resulting product was cooled to room temperature, and 2.5 L of water was poured to solidify the product, followed by filtration. The product was again dissolved in chloroform, the solution was washed twice with water, and then the organic layer was separated, anhydrous magnesium sulfate was added thereto, and the resulting mixture was stirred, and then filtered, and the filtrate was distilled under reduced pressure. The concentrated compound was purified by silica gel column chromatography to prepare 75.4 g of Compound c-2. (Yield 68%, MS: [M+H]+= 325)

### 2) Preparation of Chemical Formula c-1

Under a nitrogen atmosphere, c-2 (75.4 g, 233 mmol) was added to 754 ml of DMAC, and the resulting solution was stirred and refluxed. Thereafter, DBU(212.9 g, 1398.2 mmol) and palladium(II) acetate (1.6 g, 7 mmol) were introduced thereinto. After reacting for 8 hours, the resulting product was cooled to room temperature, and 2.5 L of water was poured to solidify the product, followed by filtration. The product was again dissolved in chloroform, the solution was washed twice with water, and then the organic layer was separated, anhydrous magnesium sulfate was added thereto, and the resulting mixture was stirred, and then filtered, and the filtrate was distilled under reduced pressure. The concentrated compound was purified by silica gel column chromatography to prepare 24.3g of Compound c-1. (Yield 43%, MS: [M+H]+= 244)

### 3) Preparation of Chemical Formula c

Chemical Formula c was synthesized in the same manner as in the method of preparing Chemical Formula a using c-1 instead of a-1.

### Preparation Example 4.

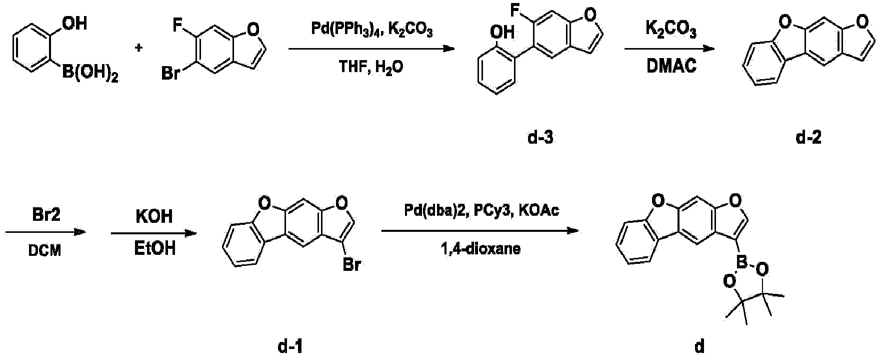

### 1) Preparation of Chemical Formula d-3

Chemical Formula d-3 was synthesized in the same manner as in the method of preparing Chemical Formula a-2 using (2-hydroxyphenyl)boronic acid instead of (5-chloro-2-hydroxyphenyl)boronic acid and using 5-bromo-6-fluorobenzofuran instead of 4-bromo-5-fluorobenzofuran.

### 2) Preparation of Chemical Formula d-2

Chemical Formula d-2 was synthesized in the same manner as in the method of preparing Chemical Formula a-1 using d-3 instead of a-2.

### 3) Preparation of Chemical Formula d-1

Under a nitrogen atmosphere, d-2 (30 g, 144.1 mmol) was added to 600 ml of dichloromethane, and the resulting mixture was stirred at -10°C. Thereafter, bromine (25.3 g, 158.5 mmol) was slowly added dropwise thereto, and the resulting mixture was stirred for 1 hour. After the reaction was completed, extraction was conducted using chloroform, Na₂S₂O₃, and water, and then an organic layer was distilled, and again dissolved in ethanol, the resulting solution was added dropwise to 300 ml of an ethanol solution saturated with KOH at 10°C, and then the resulting mixture was reacted under reflux for 2 hours. After the reaction was completed, ethanol was distilled, extraction was conducted using ethyl acetate and brine, anhydrous magnesium sulfate was added to the organic layer, the resulting mixture was stirred, and then filtered to distill the filtrate under reduced pressure. The concentrated compound was purified by silica gel column chromatography to prepare 29 g of Compound d-1. (Yield 70%, MS: [M+H]+= 288)

### 4) Preparation of Chemical Formula d

Chemical Formula d was synthesized in the same manner as in the method of preparing Chemical Formula a using d-1 instead of a-1.

### Preparation Example 5.

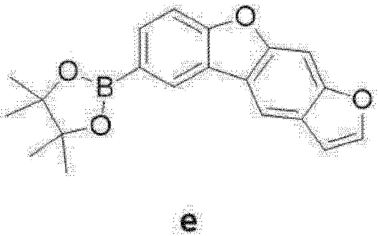

### 1) Preparation of Chemical Formula e

Chemical Formula e was synthesized in the same manner as in the method of preparing Chemical Formula a using 5-bromo-6-fluorobenzofuran instead of 4-bromo-5-fluorobenzofuran.

### Preparation Example 7.

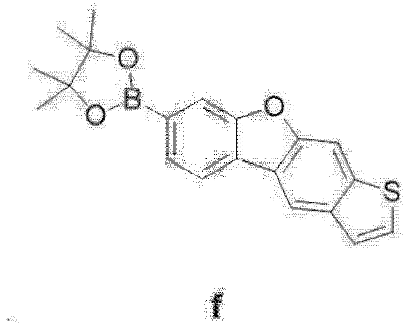

### 1) Preparation of Chemical Formula f

Chemical Formula f was synthesized in the same manner as in the method of preparing Chemical Formula a using (4-chloro-2-hydroxyphenyl)boronic acid instead of (5-chloro-2-hydroxyphenyl)boronic acid and using 5-bromo-6-fluorobenzo[b]thiophene instead of 4-bromo-5-fluorobenzofuran.

### Preparation Example 8.

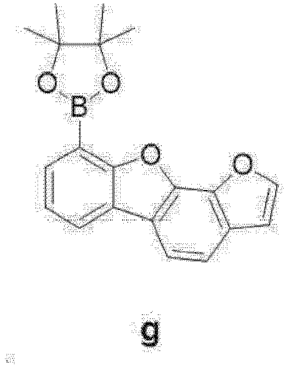

### 1) Preparation of Chemical Formula g

Chemical Formula g was synthesized in the same manner as in the method of preparing Chemical Formula a using (3-chloro-2-hydroxyphenyl)boronic acid instead of (5-chloro-2-hydroxyphenyl)boronic acid and using 6-bromo-7-fluorobenzofuran instead of 4-bromo-5-fluorobenzofuran.

### Preparation Example 9.

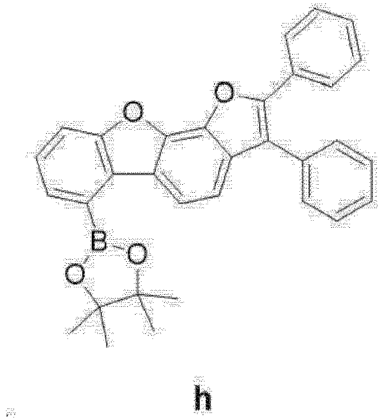

### 1) Preparation of Chemical Formula h

Chemical Formula h was synthesized in the same manner as in the method of preparing Chemical Formula c using 2-bromo-1-chloro-3-fluorobenzene instead of 1-bromo-2-fluorobenzene and using 2,3-diphenylbenzofuran-7-ol instead of 6-chlorobenzofuran-5-ol.

### Preparation Example 10.

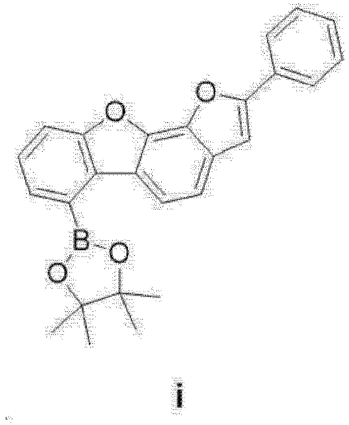

### 1) Preparation of Chemical Formula i

Chemical Formula i was synthesized in the same manner as in the method of preparing Chemical Formula c using 2-bromo-1-chloro-3-fluorobenzene instead of 1-bromo-2-fluorobenzene and using 2-phenylbenzofuran-7-ol instead of 6-chlorobenzofuran-5-ol.

### Preparation Example 11.

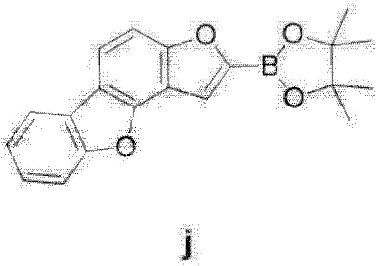

### 1) Preparation of Chemical Formula j

Chemical Formula j was synthesized in the same manner as in the method of preparing Chemical Formula c using 2-bromobenzofuran-4-ol instead of 6-chlorobenzofuran-5-ol.

### Preparation Example 12.

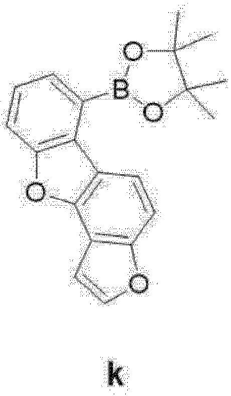

### 1) Preparation of Chemical Formula k

Chemical Formula k was synthesized in the same manner as in the method of preparing Chemical Formula a using (2-chloro-6-hydroxyphenyl)boronic acid instead of (5-chloro-2-hydroxyphenyl)boronic acid and using 5-bromo-4-fluorobenzofuran instead of 4-bromo-5-fluorobenzofuran.

### Preparation Example 13.

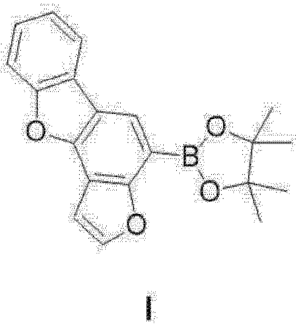

### 1) Preparation of Chemical Formula l

Chemical Formula 1 was synthesized in the same manner as in the method of preparing Chemical Formula c using 7-chloro-4-fluorobenzofuran instead of 1-bromo-2-fluorobenzene and using 2-bromophenol instead of 6-chlorobenzofuran-5-ol.

### Preparation Example 14.

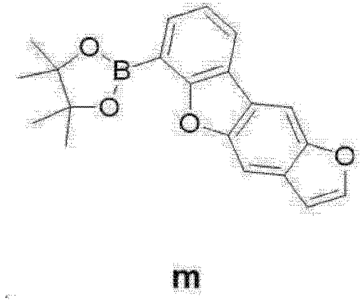

### 1) Preparation of Chemical Formula m

Chemical Formula m was synthesized in the same manner as in the method of preparing Chemical Formula a using (3-chloro-2-hydroxyphenyl)boronic acid instead of (5-chloro-2-hydroxyphenyl)boronic acid and using 6-bromo-5-fluorobenzofuran instead of 4-bromo-5-fluorobenzofuran.

### Preparation Example 15.

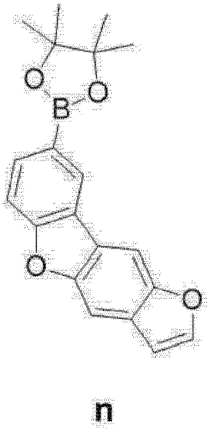

### 1) Preparation of Chemical Formula n

Chemical Formula n was synthesized in the same manner as in the method of preparing Chemical Formula a using 6-bromo-5-fluorobenzofuran instead of 4-bromo-5-fluorobenzofuran.

### Preparation Example 16.

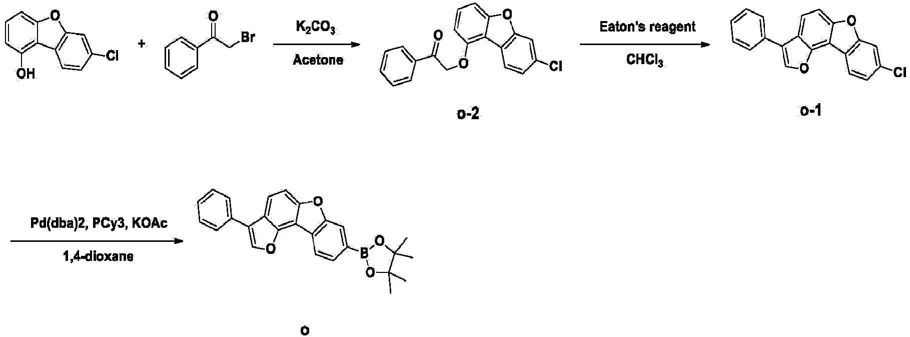

### 1) Preparation of Chemical Formula o-2

7-chlorodibenzo[b,d]furan-1-ol (60 g, 274.4 mmol) and 2-bromo-1-phenylethan-1-one (54.6 g, 274.4 mmol) were added to 600 ml of acetone, and the resulting mixture was stirred. Thereafter, potassium carbonate (56.9 g, 411.6 mmol) was introduced thereinto, and the resulting mixture was stirred. After reacting for 9 hours, the resulting product was distilled, extraction was conducted using chloroform and water, and then an organic layer was separated, anhydrous magnesium sulfate was added thereto, and the resulting mixture was stirred, and then filtered to distill the filtrate under reduced pressure. The concentrated compound was purified by silica gel column chromatography to prepare 66.5 g of Compound o-2. (Yield 72%, MS: [M+H]+= 338)

### 2) Preparation of Chemical Formula o-1

o-2 (66.5 g, 197.5 mmol) was added to 665 ml of chloroform, and the resulting mixture was stirred. Thereafter, phosphorus pentoxide was introduced thereinto in an amount of 7.7 wt.% in methanesulfonic acid (Eaton's reagent, 112.1 g, 394.9 mmol), and the resulting mixture was stirred at 40°C. After reacting for 7 hours, the resulting product was cooled to room temperature, an organic layer was separated using chloroform, NaHCO₃, and water, and then washed twice with water, the organic layer was again separated, anhydrous magnesium sulfate was added thereto, the resulting mixture was stirred, and then filtered to distill the filtrate under reduced pressure. The concentrated compound was purified by silica gel column chromatography to prepare 19.5g of Compound o-1. (Yield 31%, MS: [M+H]+= 320)

### 3) Preparation of Chemical Formula o

Chemical Formula o was synthesized in the same manner as in the method of preparing Chemical Formula a using o-1 instead of a-1.

### Preparation Example 17.

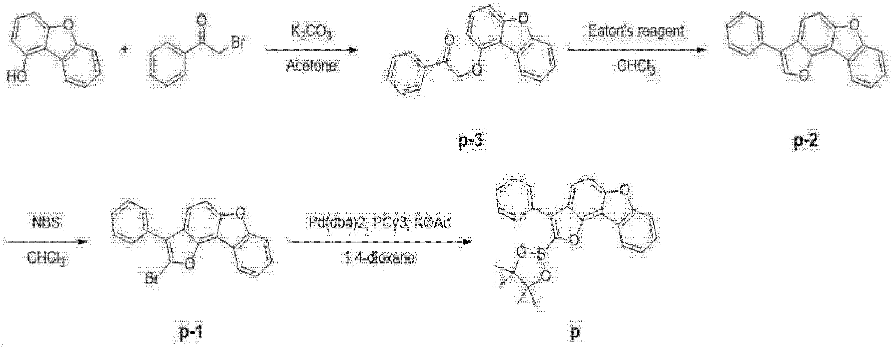

### 1) Preparation of Chemical Formula p-3

Chemical Formula p-3 was synthesized in the same manner as in the method of preparing Chemical Formula o-2 using dibenzo[b,d]furan-1-ol instead of 7-chlorodibenzo[b,d]furan-1-ol.

### 2) Preparation of Chemical Formula p-2

Chemical Formula p-2 was synthesized in the same manner as in the method of preparing Chemical Formula o-1 using p-3 instead of o-2.

### 3) Preparation of Chemical Formula p-1

Under a nitrogen atmosphere, p-2 (30 g, 105.5 mmol) and N-bromosuccinimide (19.7 g, 110.8 mmol) were added to 600 ml of chloroform, and the resulting mixture was stirred at room temperature. After reacting for 3 hours, an organic layer was separated using chloroform and water, and then anhydrous magnesium sulfate was added thereto, the resulting mixture was stirred, and then filtered to distill the filtrate under reduced pressure. The concentrated compound was purified by silica gel column chromatography to prepare 23.8 g of Compound p-1. (Yield 62%, MS: [M+H]+= 364)

### 4) Preparation of Chemical Formula p

Chemical Formula p was synthesized in the same manner as in the method of preparing Chemical Formula a using p-1 instead of a-1.

### Preparation Example 18.

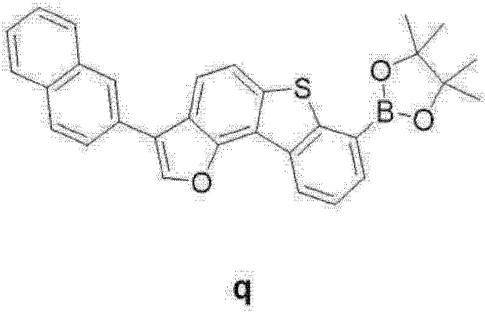

### 1) Preparation of Chemical Formula q

Chemical Formula q was synthesized in the same manner as in the method of preparing Chemical Formula o using 6-bromodibenzo[b,d]thiophen-1-ol instead of 7-chlorodibenzo[b,d]furan-1-ol and using 2-bromo-1-(naphthalen-2-yl)ethan-1-one instead of 2-bromo-1-phenylethan-1-one.

### Preparation Example 19.

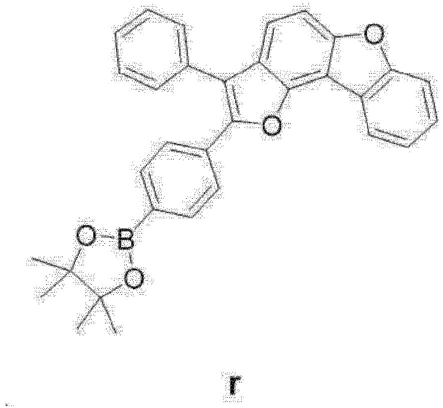

### 1) Preparation of Chemical Formula r

Chemical Formula r was synthesized in the same manner as in the method of preparing Chemical Formula o using dibenzo[b,d]furan-1-ol instead of 7-chlorodibenzo[b,d]furan-1-ol and using 2-bromo-2-(4-chlorophenyl)-1-phenylethan-1-one instead of 2-bromo-1-phenylethan-1-one.

### Preparation Example 20.

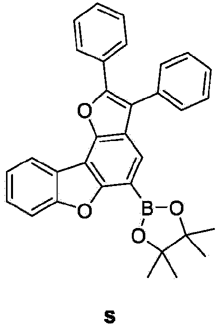

### 1) Preparation of Chemical Formula s

Chemical Formula s was synthesized in the same manner as in the method of preparing Chemical Formula o using 4-chlorodibenzo[b,d]furan-1-ol instead of 7-chlorodibenzo[b,d]furan-1-ol and using 2-bromo-1,2-diphenylethan-1-one instead of 2-bromo-1-phenylethan-1-one.

### Preparation Example 21.

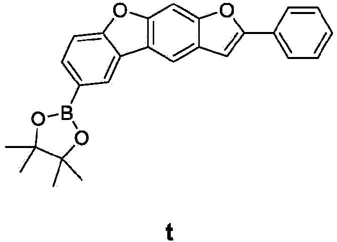

### 1) Preparation of Chemical Formula t

Chemical Formula t was synthesized in the same manner as in the method of preparing Chemical Formula c using 2-bromo-4-chloro-1-fluorobenzene instead of 1-bromo-2-fluorobenzene and using 2-phenylbenzofuran-6-ol instead of 6-chlorobenzofuran-5-ol.

### <Synthesis Examples> Synthesis of compound of Chemical Formula 1

### Synthesis Example 1.

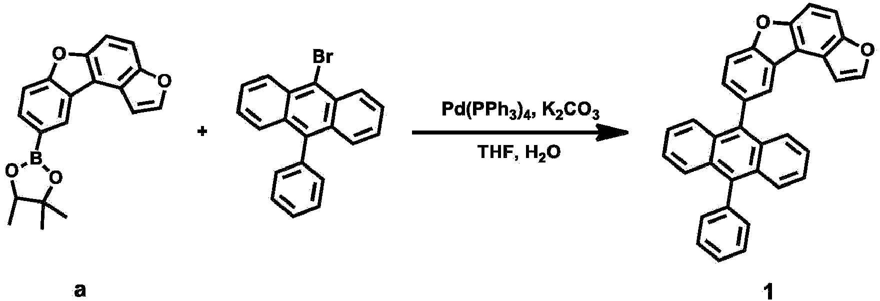

Under a nitrogen atmosphere, Compound a (15 g, 44.9 mmol) and 9-bromo-10-phenylanthracene (15 g, 44.9 mmol) were added to 300 ml of THF, and the resulting solution was stirred and refluxed. Thereafter, potassium carbonate (24.8 g, 179.5 mmol) was dissolved in 74 ml of water, the resulting solution was introduced thereinto, the resulting mixture was sufficiently stirred, and then tetrakis(triphenylphosphine)palladium(0) (1.6 g, 1.3 mmol) was introduced thereinto. After reacting for 9 hours, the resulting product was cooled to room temperature, an organic layer and an aqueous layer were separated, and then the organic layer was distilled. The distilled organic layer was again dissolved in chloroform, the solution was washed twice with water, and then the organic layer was separated, anhydrous magnesium sulfate was added thereto, and the resulting mixture was stirred, and then filtered, and the filtrate was distilled under reduced pressure. The concentrated compound was purified by silica gel column chromatography to prepare 7 g of Compound 1. (Yield 34%, MS: [M+H]+= 462)

### Synthesis Example 2.

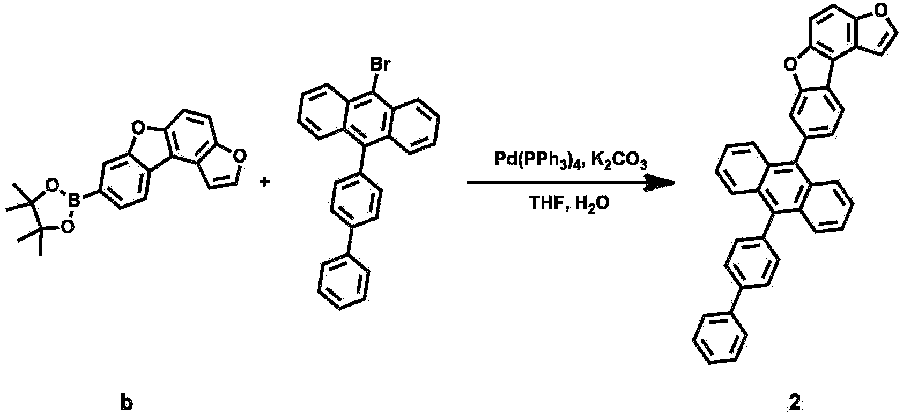

Under a nitrogen atmosphere, Compound b (15 g, 44.9 mmol) and 9-([1,1'-biphenyl]-4-yl)-10-bromoanthracene (18.4 g, 44.9 mmol) were added to 300 ml of THF, and the resulting solution was stirred and refluxed. Thereafter, potassium carbonate (24.8 g, 179.5 mmol) was dissolved in 74 ml of water, the resulting solution was introduced thereinto, the resulting mixture was sufficiently stirred, and then tetrakis(triphenylphosphine)palladium(0) (1.6 g, 1.3 mmol) was introduced thereinto. After reacting for 11 hours, the resulting product was cooled to room temperature, an organic layer and an aqueous layer were separated, and then the organic layer was distilled. The distilled organic layer was again dissolved in chloroform, the solution was washed twice with water, and then the organic layer was separated, anhydrous magnesium sulfate was added thereto, and the resulting mixture was stirred, and then filtered, and the filtrate was distilled under reduced pressure. The concentrated compound was purified by silica gel column chromatography to prepare 11.8g of Compound 2. (Yield 49%, MS: [M+H]+= 538)

### Synthesis Example 3.

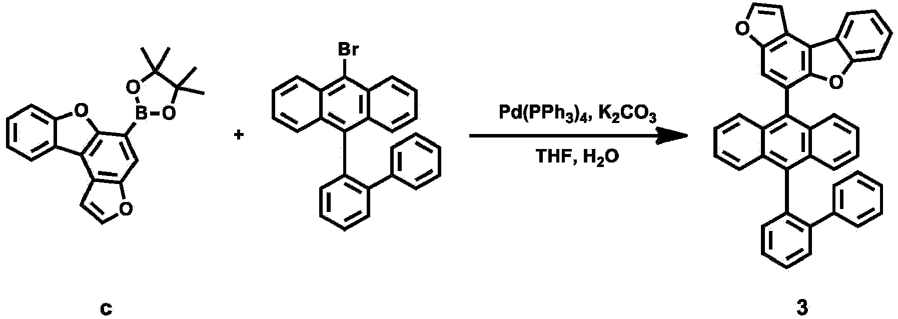

Under a nitrogen atmosphere, Compound c (15 g, 44.9 mmol) and 9-([1,1'-biphenyl]-2-yl)-10-bromoanthracene (18.4 g, 44.9 mmol) were added to 300 ml of THF, and the resulting solution was stirred and refluxed. Thereafter, potassium carbonate (24.8 g, 179.5 mmol) was dissolved in 74 ml of water, the resulting solution was introduced thereinto, the resulting mixture was sufficiently stirred, and then tetrakis(triphenylphosphine)palladium(0) (1.6 g, 1.3 mmol) was introduced thereinto. After reacting for 12 hours, the resulting product was cooled to room temperature, an organic layer and an aqueous layer were separated, and then the organic layer was distilled. The distilled organic layer was again dissolved in chloroform, the solution was washed twice with water, and then the organic layer was separated, anhydrous magnesium sulfate was added thereto, and the resulting mixture was stirred, and then filtered, and the filtrate was distilled under reduced pressure. The concentrated compound was purified by silica gel column chromatography to prepare 8.9g of Compound 3. (Yield 37%, MS: [M+H]+= 538)

### Synthesis Example 4.

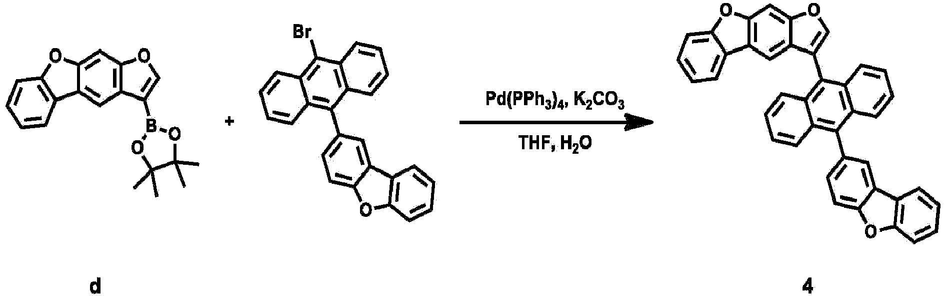

Under a nitrogen atmosphere, Compound d (15 g, 44.9 mmol) and 2-(10-bromoanthracen-9-yl)dibenzo[b,d]furan (19 g, 44.9 mmol) were added to 300 ml of THF, and the resulting solution was stirred and refluxed. Thereafter, potassium carbonate (24.8 g, 179.5 mmol) was dissolved in 74 ml of water, the resulting solution was introduced thereinto, the resulting mixture was sufficiently stirred, and then tetrakis(triphenylphosphine)palladium(0) (1.6 g, 1.3 mmol) was introduced thereinto. After reacting for 12 hours, the resulting product was cooled to room temperature, an organic layer and an aqueous layer were separated, and then the organic layer was distilled. The distilled organic layer was again dissolved in chloroform, the solution was washed twice with water, and then the organic layer was separated, anhydrous magnesium sulfate was added thereto, and the resulting mixture was stirred, and then filtered, and the filtrate was distilled under reduced pressure. The concentrated compound was purified by silica gel column chromatography to prepare 12.4g of Compound 4. (Yield 50%, MS: [M+H]+= 552)

### Synthesis Example 5.

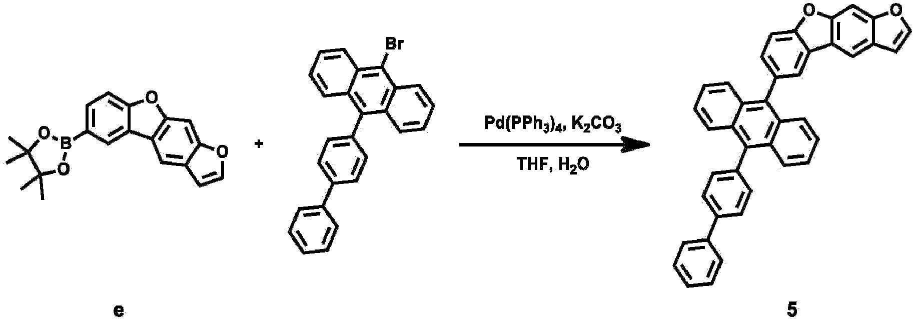

Under a nitrogen atmosphere, Compound e (15 g, 44.9 mmol) and 9-([1,1'-biphenyl]-4-yl)-10-bromoanthracene (18.4 g, 44.9 mmol) were added to 300 ml of THF, and the resulting solution was stirred and refluxed. Thereafter, potassium carbonate (24.8 g, 179.5 mmol) was dissolved in 74 ml of water, the resulting solution was introduced thereinto, the resulting mixture was sufficiently stirred, and then tetrakis(triphenylphosphine)palladium(0) (1.6 g, 1.3 mmol) was introduced thereinto. After reacting for 9 hours, the resulting product was cooled to room temperature, an organic layer and an aqueous layer were separated, and then the organic layer was distilled. The distilled organic layer was again dissolved in chloroform, the solution was washed twice with water, and then the organic layer was separated, anhydrous magnesium sulfate was added thereto, and the resulting mixture was stirred, and then filtered, and the filtrate was distilled under reduced pressure. The concentrated compound was purified by silica gel column chromatography to prepare 9.6g of Compound 5. (Yield 40%, MS: [M+H]+= 538)

### Synthesis Example 6.

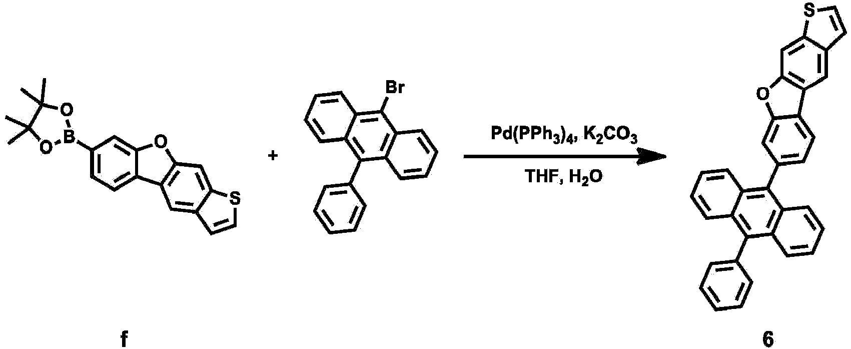

Under a nitrogen atmosphere, Compound f (15 g, 42.8 mmol) and 9-bromo-10-phenylanthracene (14.3 g, 42.8 mmol) were added to 300 ml of THF, and the resulting solution was stirred and refluxed. Thereafter, potassium carbonate (23.7 g, 171.3 mmol) was dissolved in 71 ml of water, the resulting solution was introduced thereinto, the resulting mixture was sufficiently stirred, and then tetrakis(triphenylphosphine)palladium(0) (1.5 g, 1.3 mmol) was introduced thereinto. After reacting for 11 hours, the resulting product was cooled to room temperature, an organic layer and an aqueous layer were separated, and then the organic layer was distilled. The distilled organic layer was again dissolved in chloroform, the solution was washed twice with water, and then the organic layer was separated, anhydrous magnesium sulfate was added thereto, and the resulting mixture was stirred, and then filtered, and the filtrate was distilled under reduced pressure. The concentrated compound was purified by silica gel column chromatography to prepare 9.8g of Compound 6. (Yield 48%, MS: [M+H]+= 478)

### Synthesis Example 7.

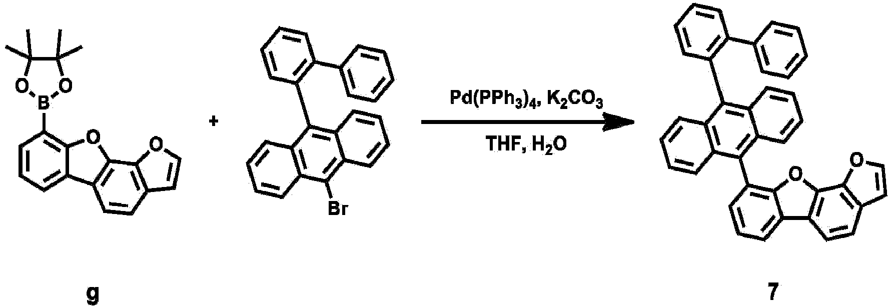

Under a nitrogen atmosphere, Compound g (15 g, 44.9 mmol) and 9-([1,1'-biphenyl]-2-yl)-10-bromoanthracene (18.4 g, 44.9 mmol) were added to 300 ml of THF, and the resulting solution was stirred and refluxed. Thereafter, potassium carbonate (24.8 g, 179.5 mmol) was dissolved in 74 ml of water, the resulting solution was introduced thereinto, the resulting mixture was sufficiently stirred, and then tetrakis(triphenylphosphine)palladium(0) (1.6 g, 1.3 mmol) was introduced thereinto. After reacting for 11 hours, the resulting product was cooled to room temperature, an organic layer and an aqueous layer were separated, and then the organic layer was distilled. The distilled organic layer was again dissolved in chloroform, the solution was washed twice with water, and then the organic layer was separated, anhydrous magnesium sulfate was added thereto, and the resulting mixture was stirred, and then filtered, and the filtrate was distilled under reduced pressure. The concentrated compound was purified by silica gel column chromatography to prepare 9.4g of Compound 7. (Yield 39%, MS: [M+H]+= 538)

### Synthesis Example 8.

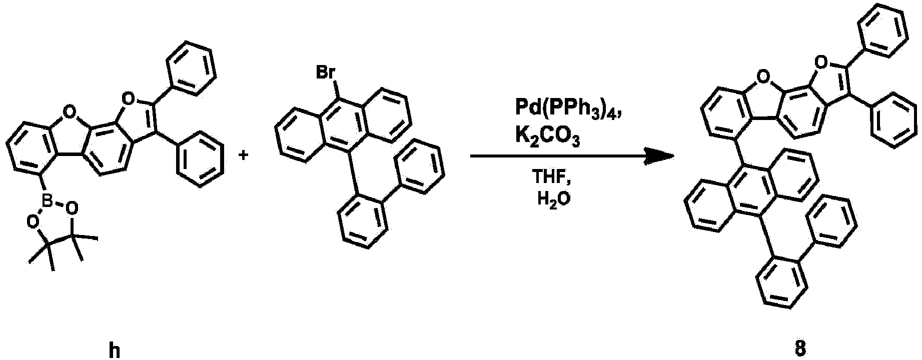

Under a nitrogen atmosphere, Compound h (15 **g,** 30.8 mmol) and 9-([1,1'-biphenyl]-2-yl)-10-bromoanthracene (12.6 **g,** 30.8 mmol) were added to 300 ml of THF, and the resulting solution was stirred and refluxed. Thereafter, potassium carbonate (17 g, 123.4 mmol) was dissolved in 51 ml of water, the resulting solution was introduced thereinto, the resulting mixture was sufficiently stirred, and then tetrakis(triphenylphosphine)palladium(0) (1.1 g, 0.9 mmol) was introduced thereinto. After reacting for 9 hours, the resulting product was cooled to room temperature, an organic layer and an aqueous layer were separated, and then the organic layer was distilled. The distilled organic layer was again dissolved in chloroform, the solution was washed twice with water, and then the organic layer was separated, anhydrous magnesium sulfate was added thereto, and the resulting mixture was stirred, and then filtered, and the filtrate was distilled under reduced pressure. The concentrated compound was purified by silica gel column chromatography to prepare 7.4g of Compound 8. (Yield 35%, MS: [M+H]+= 690)

### Synthesis Example 9.

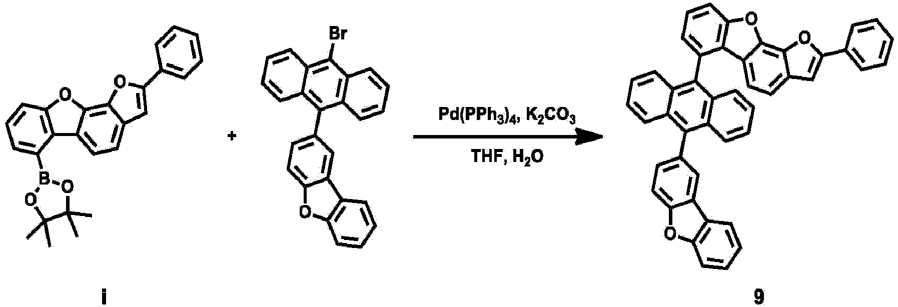

Under a nitrogen atmosphere, Compound i (15 g, 36.6 mmol) and 2-(10-bromoanthracen-9-yl)dibenzo[b,d]furan (15.5 g, 36.6 mmol) were added to 300 ml of THF, and the resulting solution was stirred and refluxed. Thereafter, potassium carbonate (20.2 g, 146.2 mmol) was dissolved in 61 ml of water, the resulting solution was introduced thereinto, the resulting mixture was sufficiently stirred, and then tetrakis(triphenylphosphine)palladium(0) (1.3 g, 1.1 mmol) was introduced thereinto. After reacting for 9 hours, the resulting product was cooled to room temperature, an organic layer and an aqueous layer were separated, and then the organic layer was distilled. The distilled organic layer was again dissolved in chloroform, the solution was washed twice with water, and then the organic layer was separated, anhydrous magnesium sulfate was added thereto, and the resulting mixture was stirred, and then filtered, and the filtrate was distilled under reduced pressure. The concentrated compound was purified by silica gel column chromatography to prepare 9.4g of Compound 9. (Yield 41%, MS: [M+H]+= 628)

### Synthesis Example 10.

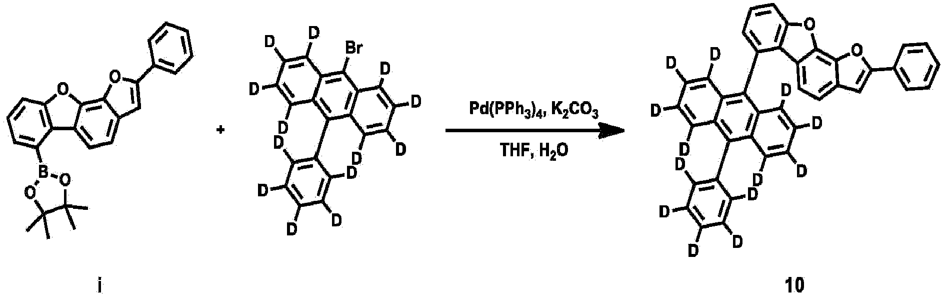

Under a nitrogen atmosphere, Compound i (15 g, 36.6 mmol) and 9-bromo-10-(phenyl-d5)anthracene-1,2,3,4,5,6,7,8-d8 (12.7 g, 36.6 mmol) were added to 300 ml of THF, and the resulting solution was stirred and refluxed. Thereafter, potassium carbonate (20.2 g, 146.2 mmol) was dissolved in 61 ml of water, the resulting solution was introduced thereinto, the resulting mixture was sufficiently stirred, and then tetrakis(triphenylphosphine)palladium(0) (1.3 g, 1.1 mmol) was introduced thereinto. After reacting for 11 hours, the resulting product was cooled to room temperature, an organic layer and an aqueous layer were separated, and then the organic layer was distilled. The distilled organic layer was again dissolved in chloroform, the solution was washed twice with water, and then the organic layer was separated, anhydrous magnesium sulfate was added thereto, and the resulting mixture was stirred, and then filtered, and the filtrate was distilled under reduced pressure. The concentrated compound was purified by silica gel column chromatography to prepare 9g of Compound 10. (Yield 45%, MS: [M+H]+= 551)

### Synthesis Example 11.

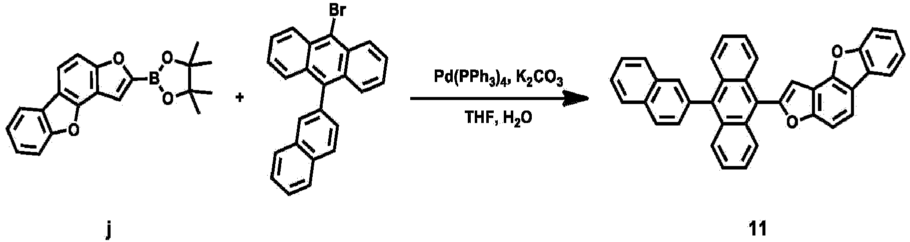

Under a nitrogen atmosphere, Compound j (15 g, 44.9 mmol) and 9-bromo-10-(naphthalen-2-yl)anthracene (17.2 g, 44.9 mmol) were added to 300 ml of THF, and the resulting solution was stirred and refluxed. Thereafter, potassium carbonate (24.8 g, 179.5 mmol) was dissolved in 74 ml of water, the resulting solution was introduced thereinto, the resulting mixture was sufficiently stirred, and then tetrakis(triphenylphosphine)palladium(0) (1.6 g, 1.3 mmol) was introduced thereinto. After reacting for 9 hours, the resulting product was cooled to room temperature, an organic layer and an aqueous layer were separated, and then the organic layer was distilled. The distilled organic layer was again dissolved in chloroform, the solution was washed twice with water, and then the organic layer was separated, anhydrous magnesium sulfate was added thereto, and the resulting mixture was stirred, and then filtered, and the filtrate was distilled under reduced pressure. The concentrated compound was purified by silica gel column chromatography to prepare 8.7g of Compound 11. (Yield 38%, MS: [M+H]+= 512)

### Synthesis Example 12.

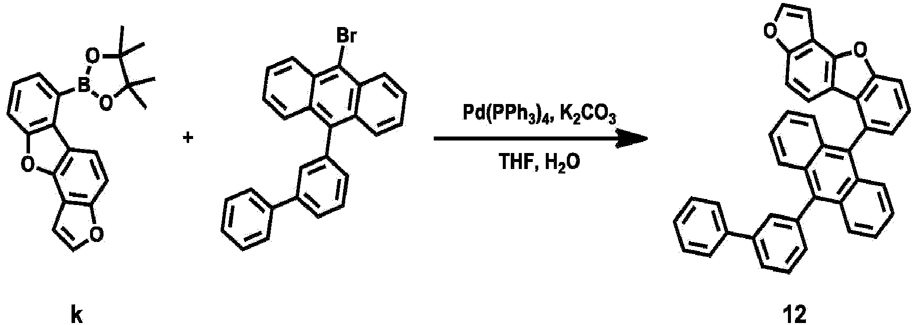

Under a nitrogen atmosphere, Compound k (15 g, 44.9 mmol) and 9-([1,1'-biphenyl]-3-yl)-10-bromoanthracene (18.4 g, 44.9 mmol) were added to 300 ml of THF, and the resulting solution was stirred and refluxed. Thereafter, potassium carbonate (24.8 g, 179.5 mmol) was dissolved in 74 ml of water, the resulting solution was introduced thereinto, the resulting mixture was sufficiently stirred, and then tetrakis(triphenylphosphine)palladium(0) (1.6 g, 1.3 mmol) was introduced thereinto. After reacting for 10 hours, the resulting product was cooled to room temperature, an organic layer and an aqueous layer were separated, and then the organic layer was distilled. The distilled organic layer was again dissolved in chloroform, the solution was washed twice with water, and then the organic layer was separated, anhydrous magnesium sulfate was added thereto, and the resulting mixture was stirred, and then filtered, and the filtrate was distilled under reduced pressure. The concentrated compound was purified by silica gel column chromatography to prepare 8.7g of Compound 12. (Yield 36%, MS: [M+H]+= 538)

### Synthesis Example 13.

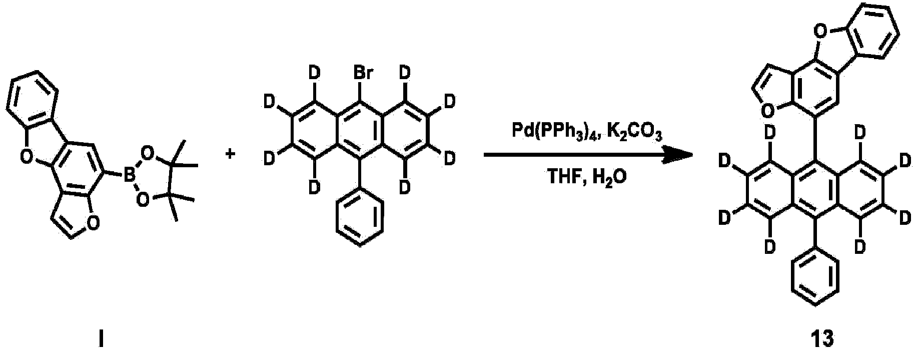

Under a nitrogen atmosphere, Compound l (15 g, 44.9 mmol) and 9-bromo-10-phenylanthracene-1,2,3,4,5,6,7,8-d8 (15.3 g, 44.9 mmol) were added to 300 ml of THF, and the resulting solution was stirred and refluxed. Thereafter, potassium carbonate (24.8 g, 179.5 mmol) was dissolved in 74 ml of water, the resulting solution was introduced thereinto, the resulting mixture was sufficiently stirred, and then tetrakis(triphenylphosphine)palladium(0) (1.6 g, 1.3 mmol) was introduced thereinto. After reacting for 11 hours, the resulting product was cooled to room temperature, an organic layer and an aqueous layer were separated, and then the organic layer was distilled. The distilled organic layer was again dissolved in chloroform, the solution was washed twice with water, and then the organic layer was separated, anhydrous magnesium sulfate was added thereto, and the resulting mixture was stirred, and then filtered, and the filtrate was distilled under reduced pressure. The concentrated compound was purified by silica gel column chromatography to prepare 9.9g of Compound 13. (Yield 47%, MS: [M+H]+= 470)

### Synthesis Example 14.

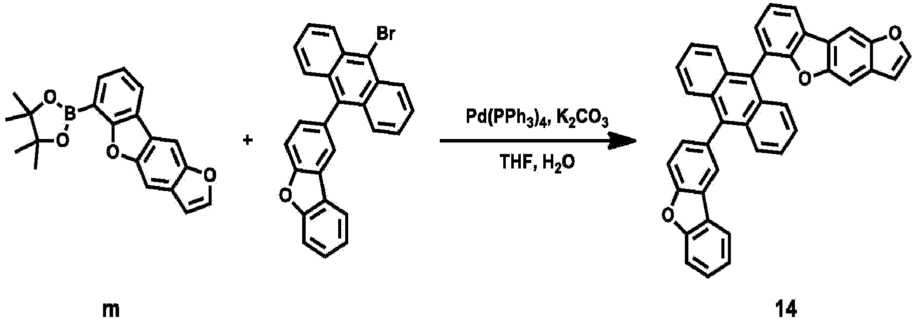

Under a nitrogen atmosphere, Compound m (15 g, 44.9 mmol) and 2-(10-bromoanthracen-9-yl)dibenzo[b,d]furan (19 g, 44.9 mmol) were added to 300 ml of THF, and the resulting solution was stirred and refluxed. Thereafter, potassium carbonate (24.8 g, 179.5 mmol) was dissolved in 74 ml of water, the resulting solution was introduced thereinto, the resulting mixture was sufficiently stirred, and then tetrakis(triphenylphosphine)palladium(0) (1.6 g, 1.3 mmol) was introduced thereinto. After reacting for 9 hours, the resulting product was cooled to room temperature, an organic layer and an aqueous layer were separated, and then the organic layer was distilled. The distilled organic layer was again dissolved in chloroform, the solution was washed twice with water, and then the organic layer was separated, anhydrous magnesium sulfate was added thereto, and the resulting mixture was stirred, and then filtered, and the filtrate was distilled under reduced pressure. The concentrated compound was purified by silica gel column chromatography to prepare 12.4g of Compound 14. (Yield 50%, MS: [M+H]+= 552)

### Synthesis Example 15.

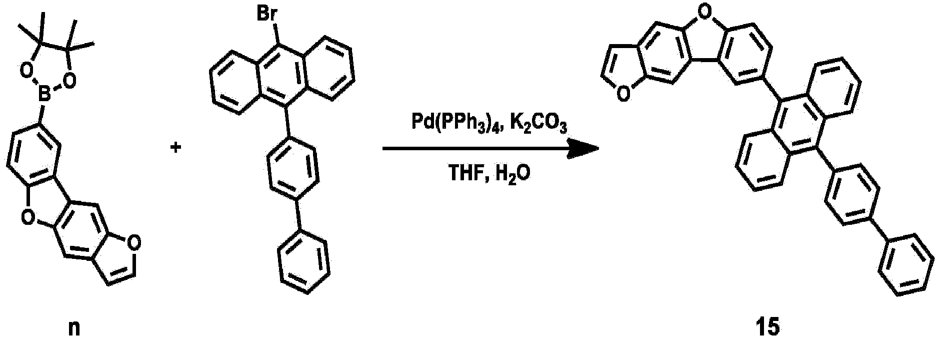

Under a nitrogen atmosphere, Compound n (15 g, 44.9 mmol) and 9-([1,1'-biphenyl]-4-yl)-10-bromoanthracene (18.4 g, 44.9 mmol) were added to 300 ml of THF, and the resulting solution was stirred and refluxed. Thereafter, potassium carbonate (24.8 g, 179.5 mmol) was dissolved in 74 ml of water, the resulting solution was introduced thereinto, the resulting mixture was sufficiently stirred, and then tetrakis(triphenylphosphine)palladium(0) (1.6 g, 1.3 mmol) was introduced thereinto. After reacting for 12 hours, the resulting product was cooled to room temperature, an organic layer and an aqueous layer were separated, and then the organic layer was distilled. The distilled organic layer was again dissolved in chloroform, the solution was washed twice with water, and then the organic layer was separated, anhydrous magnesium sulfate was added thereto, and the resulting mixture was stirred, and then filtered, and the filtrate was distilled under reduced pressure. The concentrated compound was purified by silica gel column chromatography to prepare 10.4g of Compound 15. (Yield 43%, MS: [M+H]+= 538)

### Synthesis Example 16.

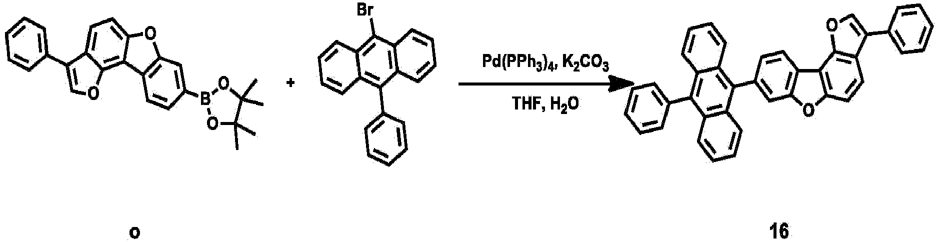

Under a nitrogen atmosphere, Compound o (15 g, 36.6 mmol) and 9-bromo-10-phenylanthracene (12.2 g, 36.6 mmol) were added to 300 ml of THF, and the resulting solution was stirred and refluxed. Thereafter, potassium carbonate (20.2 g, 146.2 mmol) was dissolved in 61 ml of water, the resulting solution was introduced thereinto, the resulting mixture was sufficiently stirred, and then tetrakis(triphenylphosphine)palladium(0) (1.3 g, 1.1 mmol) was introduced thereinto. After reacting for 9 hours, the resulting product was cooled to room temperature, an organic layer and an aqueous layer were separated, and then the organic layer was distilled. The distilled organic layer was again dissolved in chloroform, the solution was washed twice with water, and then the organic layer was separated, anhydrous magnesium sulfate was added thereto, and the resulting mixture was stirred, and then filtered, and the filtrate was distilled under reduced pressure. The concentrated compound was purified by silica gel column chromatography to prepare 8.4g of Compound 16. (Yield 43%, MS: [M+H]+= 538)

### Synthesis Example 17.

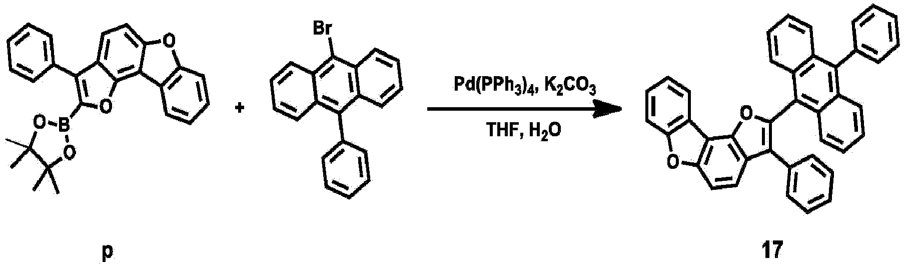

Under a nitrogen atmosphere, Compound p (15 g, 36.6 mmol) and 9-bromo-10-phenylanthracene (12.2 g, 36.6 mmol) were added to 300 ml of THF, and the resulting solution was stirred and refluxed. Thereafter, potassium carbonate (20.2 g, 146.2 mmol) was dissolved in 61 ml of water, the resulting solution was introduced thereinto, the resulting mixture was sufficiently stirred, and then tetrakis(triphenylphosphine)palladium(0) (1.3 g, 1.1 mmol) was introduced thereinto. After reacting for 11 hours, the resulting product was cooled to room temperature, an organic layer and an aqueous layer were separated, and then the organic layer was distilled. The distilled organic layer was again dissolved in chloroform, the solution was washed twice with water, and then the organic layer was separated, anhydrous magnesium sulfate was added thereto, and the resulting mixture was stirred, and then filtered, and the filtrate was distilled under reduced pressure. The concentrated compound was purified by silica gel column chromatography to prepare 12.4g of Compound 17. (Yield 63%, MS: [M+H]+= 538)

### Synthesis Example 18.

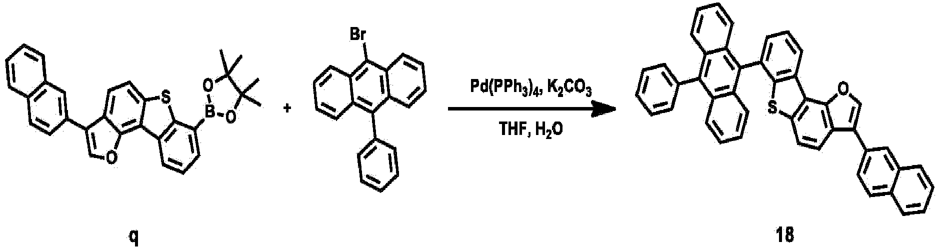

Under a nitrogen atmosphere, Compound q (15 g, 31.5 mmol) and 9-bromo-10-phenylanthracene (10.5 g, 31.5 mmol) were added to 300 ml of THF, and the resulting solution was stirred and refluxed. Thereafter, potassium carbonate (17.4 g, 125.9 mmol) was dissolved in 52 ml of water, the resulting solution was introduced thereinto, the resulting mixture was sufficiently stirred, and then tetrakis(triphenylphosphine)palladium(0) (1.1 g, 0.9 mmol) was introduced thereinto. After reacting for 9 hours, the resulting product was cooled to room temperature, an organic layer and an aqueous layer were separated, and then the organic layer was distilled. The distilled organic layer was again dissolved in chloroform, the solution was washed twice with water, and then the organic layer was separated, anhydrous magnesium sulfate was added thereto, and the resulting mixture was stirred, and then filtered, and the filtrate was distilled under reduced pressure. The concentrated compound was purified by silica gel column chromatography to prepare 11.6g of Compound 18. (Yield 61%, MS: [M+H]+= 604)

### Synthesis Example 19.

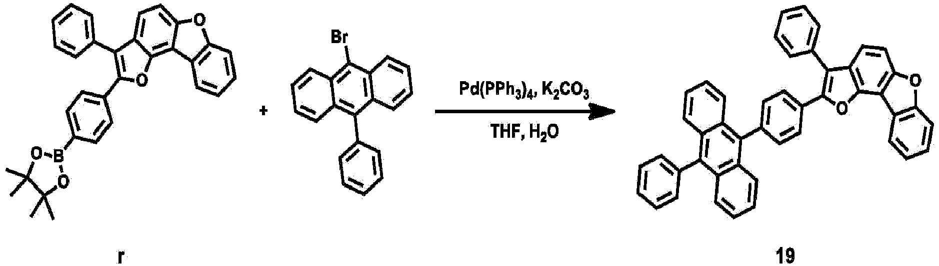

Under a nitrogen atmosphere, Compound r (15 g, 30.8 mmol) and 9-bromo-10-phenylanthracene (10.3 g, 30.8 mmol) were added to 300 ml of THF, and the resulting solution was stirred and refluxed. Thereafter, potassium carbonate (17 g, 123.4 mmol) was dissolved in 51 ml of water, the resulting solution was introduced thereinto, the resulting mixture was sufficiently stirred, and then tetrakis(triphenylphosphine)palladium(0) (1.1 g, 0.9 mmol) was introduced thereinto. After reacting for 11 hours, the resulting product was cooled to room temperature, an organic layer and an aqueous layer were separated, and then the organic layer was distilled. The distilled organic layer was again dissolved in chloroform, the solution was washed twice with water, and then the organic layer was separated, anhydrous magnesium sulfate was added thereto, and the resulting mixture was stirred, and then filtered, and the filtrate was distilled under reduced pressure. The concentrated compound was purified by silica gel column chromatography to prepare 9.6g of Compound 19. (Yield 51%, MS: [M+H]+= 614)

### Synthesis Example 20.

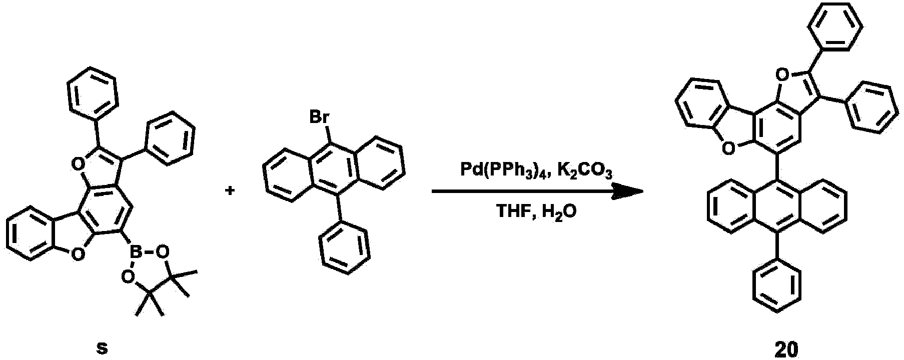

Under a nitrogen atmosphere, Compound s (15 g, 30.8 mmol) and 9-bromo-10-phenylanthracene (10.3 g, 30.8 mmol) were added to 300 ml of THF, and the resulting solution was stirred and refluxed. Thereafter, potassium carbonate (17 g, 123.4 mmol) was dissolved in 51 ml of water, the resulting solution was introduced thereinto, the resulting mixture was sufficiently stirred, and then tetrakis(triphenylphosphine)palladium(0) (1.1 g, 0.9 mmol) was introduced thereinto. After reacting for 10 hours, the resulting product was cooled to room temperature, an organic layer and an aqueous layer were separated, and then the organic layer was distilled. The distilled organic layer was again dissolved in chloroform, the solution was washed twice with water, and then the organic layer was separated, anhydrous magnesium sulfate was added thereto, and the resulting mixture was stirred, and then filtered, and the filtrate was distilled under reduced pressure. The concentrated compound was purified by silica gel column chromatography to prepare 7.9 g of Compound 20. (Yield 42%, MS: [M+H]+= 614)

### Synthesis Example 21.

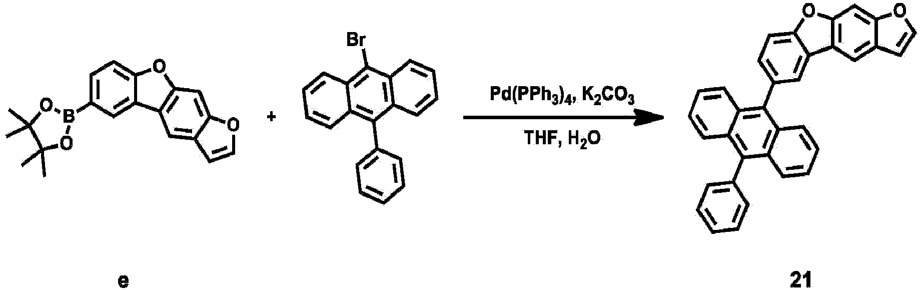

Under a nitrogen atmosphere, Compound e (15 g, 44.9 mmol) and 9-bromo-10-phenylanthracene (15 g, 44.9 mmol) were added to 300 ml of THF, and the resulting solution was stirred and refluxed. Thereafter, potassium carbonate (18.6 g, 134.7 mmol) was dissolved in 56 ml of water, the resulting solution was introduced thereinto, the resulting mixture was sufficiently stirred, and then tetrakis(triphenylphosphine)palladium(0) (1 g, 0.9 mmol) was introduced thereinto. After reacting for 10 hours, the resulting product was cooled to room temperature, an organic layer and an aqueous layer were separated, and then the organic layer was distilled. The distilled organic layer was again dissolved in chloroform, the solution was washed twice with water, and then the organic layer was separated, anhydrous magnesium sulfate was added thereto, and the resulting mixture was stirred, and then filtered, and the filtrate was distilled under reduced pressure. The concentrated compound was purified by silica gel column chromatography to prepare 10.3g of Compound 21. (Yield 50%, MS: [M+H]+= 461)

### Synthesis Example 22.

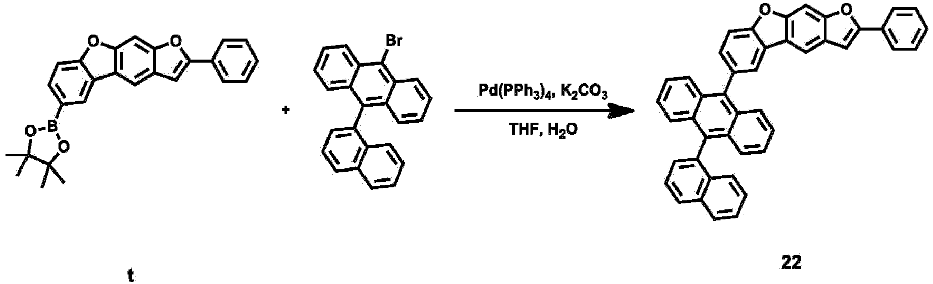

Under a nitrogen atmosphere, Compound t (15 g, 36.6 mmol) and 9-bromo-10-(naphthalen-1-yl)anthracene (14 g, 36.6 mmol) were added to 300 ml of THF, and the resulting solution was stirred and refluxed. Thereafter, potassium carbonate (15.2 g, 109.7 mmol) was dissolved in 45 ml of water, the resulting solution was introduced thereinto, the resulting mixture was sufficiently stirred, and then tetrakis(triphenylphosphine)palladium(0) (0.8 g, 0.7 mmol) was introduced thereinto. After reacting for 11 hours, the resulting product was cooled to room temperature, an organic layer and an aqueous layer were separated, and then the organic layer was distilled. The distilled organic layer was again dissolved in chloroform, the solution was washed twice with water, and then the organic layer was separated, anhydrous magnesium sulfate was added thereto, and the resulting mixture was stirred, and then filtered, and the filtrate was distilled under reduced pressure. The concentrated compound was purified by silica gel column chromatography to prepare 11.4g of Compound 22. (Yield 53%, MS: [M+H]+= 587)

### Synthesis Example 23.

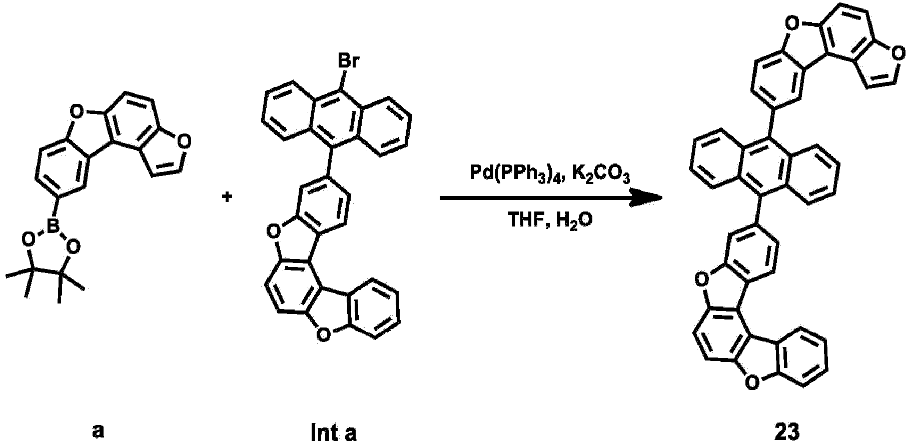

Under a nitrogen atmosphere, Compound a (15 g, 44.9 mmol) and Int a (23 g, 44.9 mmol) were added to 300 ml of THF, and the resulting solution was stirred and refluxed. Thereafter, potassium carbonate (18.6 g, 134.7 mmol) was dissolved in 56 ml of water, the resulting solution was introduced thereinto, the resulting mixture was sufficiently stirred, and then tetrakis(triphenylphosphine)palladium(0) (1 g, 0.9 mmol) was introduced thereinto. After reacting for 5 hours, the resulting product was cooled to room temperature, an organic layer and an aqueous layer were separated, and then the organic layer was distilled. The distilled organic layer was again dissolved in chloroform, the solution was washed twice with water, and then the organic layer was separated, anhydrous magnesium sulfate was added thereto, and the resulting mixture was stirred, and then filtered, and the filtrate was distilled under reduced pressure. The concentrated compound was purified by silica gel column chromatography to prepare 11.5g of Compound 23.
(Yield 40%, MS: [M+H]+= 641)

### Synthesis Example 24.

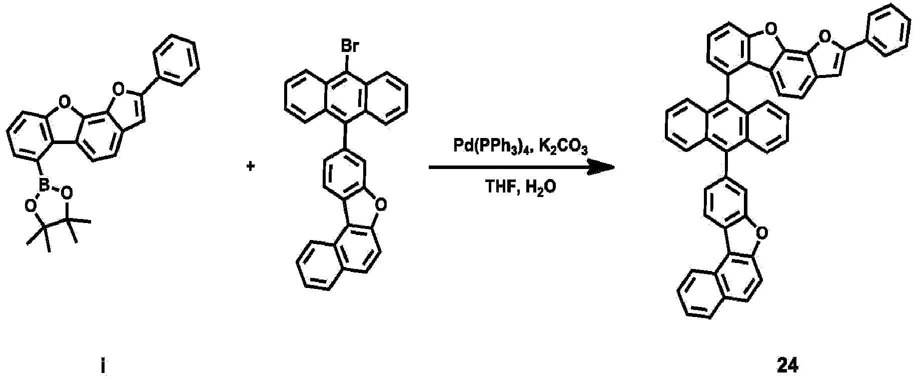

Under a nitrogen atmosphere, Compound i (15 g, 36.6 mmol) and 9-(10-bromoanthracen-9-yl)naphtho[2,1-b]benzofuran (17.3 g, 36.6 mmol) were added to 300 ml of THF, and the resulting solution was stirred and refluxed. Thereafter, potassium carbonate (15.2 g, 109.7 mmol) was dissolved in 45 ml of water, the resulting solution was introduced thereinto, the resulting mixture was sufficiently stirred, and then tetrakis(triphenylphosphine)palladium(0) (0.8 g, 0.7 mmol) was introduced thereinto. After reacting for 12 hours, the resulting product was cooled to room temperature, an organic layer and an aqueous layer were separated, and then the organic layer was distilled. The distilled organic layer was again dissolved in chloroform, the solution was washed twice with water, and then the organic layer was separated, anhydrous magnesium sulfate was added thereto, and the resulting mixture was stirred, and then filtered, and the filtrate was distilled under reduced pressure. The concentrated compound was purified by silica gel column chromatography to prepare 10.9g of Compound 24. (Yield 44%, MS: [M+H]+= 677)

### Synthesis Example 25.

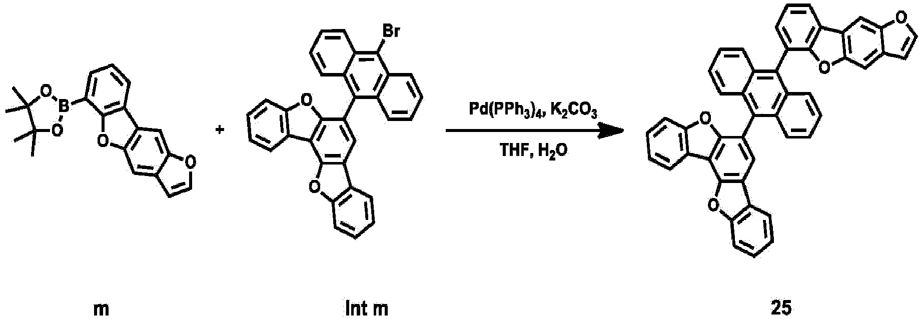

Under a nitrogen atmosphere, Compound m (15 g, 44.9 mmol) and Int m (23 g, 44.9 mmol) were added to 300 ml of THF, and the resulting solution was stirred and refluxed. Thereafter, potassium carbonate (18.6 g, 134.7 mmol) was dissolved in 56 ml of water, the resulting solution was introduced thereinto, the resulting mixture was sufficiently stirred, and then tetrakis(triphenylphosphine)palladium(0) (1 g, 0.9 mmol) was introduced thereinto. After reacting for 6 hours, the resulting product was cooled to room temperature, an organic layer and an aqueous layer were separated, and then the organic layer was distilled. The distilled organic layer was again dissolved in chloroform, the solution was washed twice with water, and then the organic layer was separated, anhydrous magnesium sulfate was added thereto, and the resulting mixture was stirred, and then filtered, and the filtrate was distilled under reduced pressure. The concentrated compound was purified by silica gel column chromatography to prepare 14.1g of Compound 25.
(Yield 49%, MS: [M+H]+= 641)

### Synthesis Example 26.

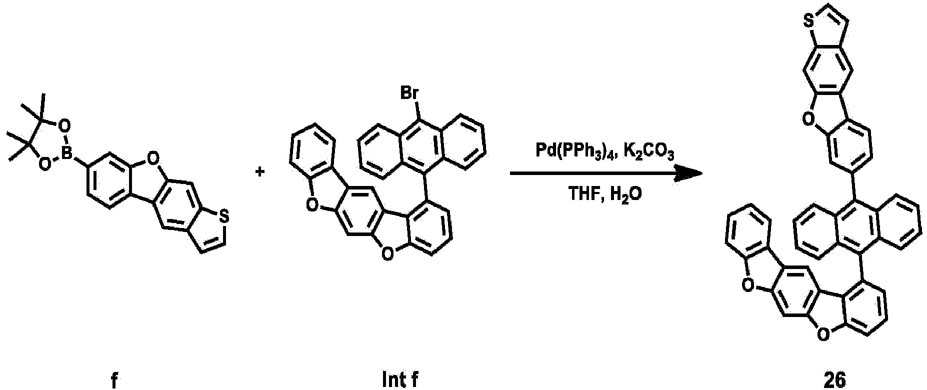

Under a nitrogen atmosphere, Compound f (15 g, 42.8 mmol) and Int f (22 g, 42.8 mmol) were added to 300 ml of THF, and the resulting solution was stirred and refluxed. Thereafter, potassium carbonate (17.8 g, 128.5 mmol) was dissolved in 53 ml of water, the resulting solution was introduced thereinto, the resulting mixture was sufficiently stirred, and then tetrakis(triphenylphosphine)palladium(0) (1 g, 0.9 mmol) was introduced thereinto. After reacting for 3 hours, the resulting product was cooled to room temperature, an organic layer and an aqueous layer were separated, and then the organic layer was distilled. The distilled organic layer was again dissolved in chloroform, the solution was washed twice with water, and then the organic layer was separated, anhydrous magnesium sulfate was added thereto, and the resulting mixture was stirred, and then filtered, and the filtrate was distilled under reduced pressure. The concentrated compound was purified by silica gel column chromatography to prepare 12.1 g of Compound 26.
(Yield 43%, MS: [M+H]+= 657)

### Synthesis Example 27.

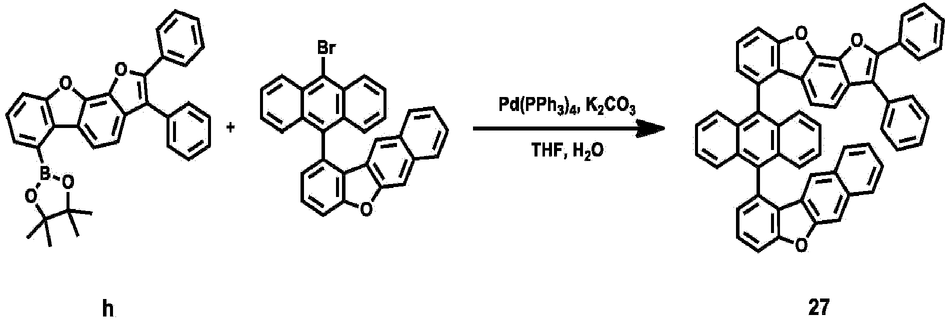

Under a nitrogen atmosphere, Compound h (15 g, 30.8 mmol) and 1-(10-bromoanthracen-9-yl)naphtho[2,3-b]benzofuran (14.6 g, 30.8 mmol) were added to 300 ml of THF, and the resulting solution was stirred and refluxed. Thereafter, potassium carbonate (12.8 g, 92.5 mmol) was dissolved in 38 ml of water, the resulting solution was introduced thereinto, the resulting mixture was sufficiently stirred, and then tetrakis(triphenylphosphine)palladium(0) (0.7 g, 0.6 mmol) was introduced thereinto. After reacting for 3 hours, the resulting product was cooled to room temperature, an organic layer and an aqueous layer were separated, and then the organic layer was distilled. The distilled organic layer was again dissolved in chloroform, the solution was washed twice with water, and then the organic layer was separated, anhydrous magnesium sulfate was added thereto, and the resulting mixture was stirred, and then filtered, and the filtrate was distilled under reduced pressure. The concentrated compound was purified by silica gel column chromatography to prepare 10.2g of Compound 27. (Yield 44%, MS: [M+H]+= 753)

### Synthesis Example 28.

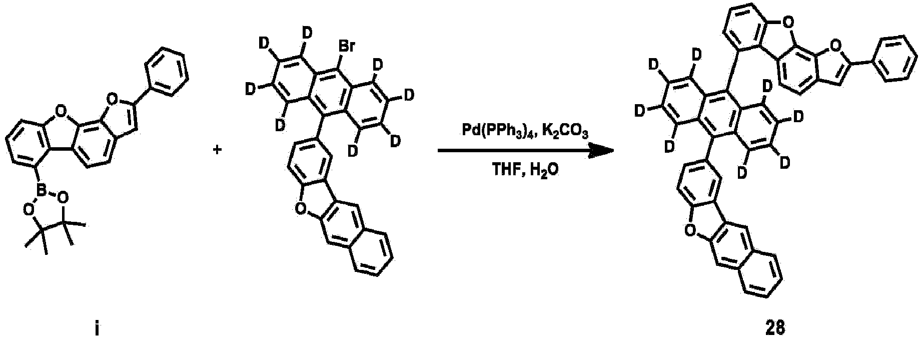

Under a nitrogen atmosphere, Compound i (15 g, 36.6 mmol) and 2-(10-bromoanthracen-9-yl-1,2,3,4,5,6,7,8-d8)naphtho[2,3-b]benzofuran (17.6 g, 36.6 mmol) were added to 300 ml of THF, and the resulting solution was stirred and refluxed. Thereafter, potassium carbonate (15.2 g, 109.7 mmol) was dissolved in 45 ml of water, the resulting solution was introduced thereinto, the resulting mixture was sufficiently stirred, and then tetrakis(triphenylphosphine)palladium(0) (0.8 g, 0.7 mmol) was introduced thereinto. After reacting for 11 hours, the resulting product was cooled to room temperature, an organic layer and an aqueous layer were separated, and then the organic layer was distilled. The distilled organic layer was again dissolved in chloroform, the solution was washed twice with water, and then the organic layer was separated, anhydrous magnesium sulfate was added thereto, and the resulting mixture was stirred, and then filtered, and the filtrate was distilled under reduced pressure. The concentrated compound was purified by silica gel column chromatography to prepare 14.8g of Compound 28. (Yield 59%, MS: [M+H]+= 685)

### Synthesis Example 29.

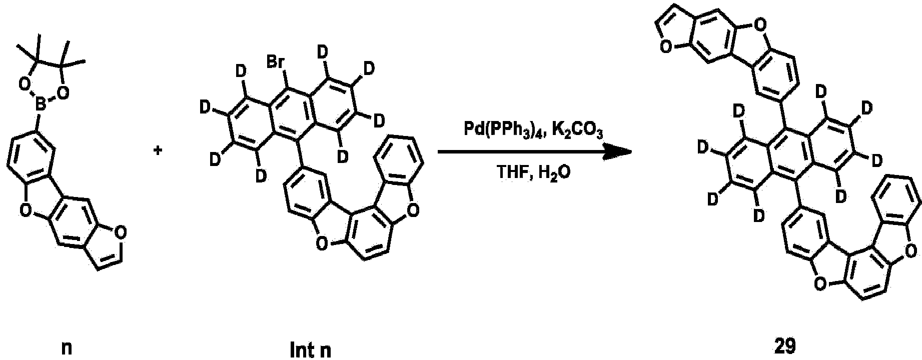

Under a nitrogen atmosphere, Compound n (15 g, 44.9 mmol) and Int n (23.4 g, 44.9 mmol) were added to 300 ml of THF, and the resulting solution was stirred and refluxed. Thereafter, potassium carbonate (18.6 g, 134.7 mmol) was dissolved in 56 ml of water, the resulting solution was introduced thereinto, the resulting mixture was sufficiently stirred, and then tetrakis(triphenylphosphine)palladium(0) (1 g, 0.9 mmol) was introduced thereinto. After reacting for 10 hours, the resulting product was cooled to room temperature, an organic layer and an aqueous layer were separated, and then the organic layer was distilled. The distilled organic layer was again dissolved in chloroform, the solution was washed twice with water, and then the organic layer was separated, anhydrous magnesium sulfate was added thereto, and the resulting mixture was stirred, and then filtered, and the filtrate was distilled under reduced pressure. The concentrated compound was purified by silica gel column chromatography to prepare 16.9g of Compound 29.
(Yield 58%, MS: [M+H]+= 649)

### Synthesis Example 30.

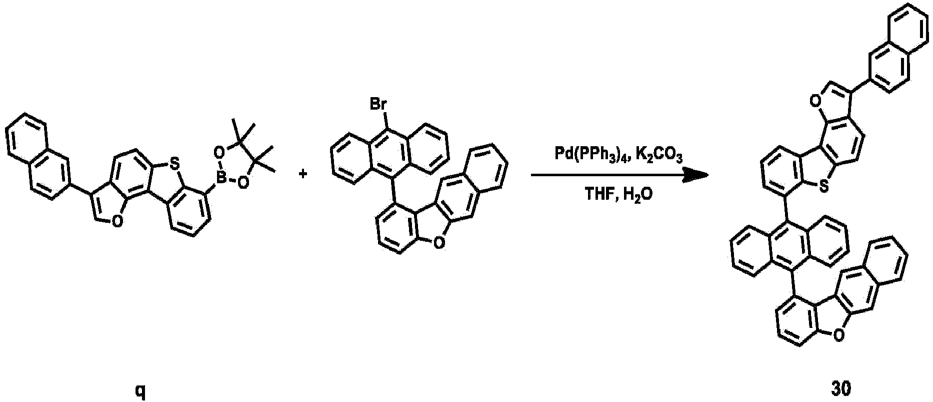

Under a nitrogen atmosphere, Compound q (15 g, 31.5 mmol) and 1-(10-bromoanthracen-9-yl)naphtho[2,3-b]benzofuran (14.9 g, 31.5 mmol) were added to 300 ml of THF, and the resulting solution was stirred and refluxed. Thereafter, potassium carbonate (13.1 g, 94.5 mmol) was dissolved in 39 ml of water, the resulting solution was introduced thereinto, the resulting mixture was sufficiently stirred, and then tetrakis(triphenylphosphine)palladium(0) (0.7 g, 0.6 mmol) was introduced thereinto. After reacting for 11 hours, the resulting product was cooled to room temperature, an organic layer and an aqueous layer were separated, and then the organic layer was distilled. The distilled organic layer was again dissolved in chloroform, the solution was washed twice with water, and then the organic layer was separated, anhydrous magnesium sulfate was added thereto, and the resulting mixture was stirred, and then filtered, and the filtrate was distilled under reduced pressure. The concentrated compound was purified by silica gel column chromatography to prepare 10.5g of Compound 30. (Yield 45%, MS: [M+H]+= 743)

### <Examples>

### Example 1.

A glass substrate thinly coated with indium tin oxide (ITO) to have a thickness of 150 nm was put into distilled water in which a detergent was dissolved, and ultrasonically washed. In this case, a product manufactured by the Fischer Co., was used as the detergent, and distilled water twice filtered using a filter manufactured by Millipore Co., was used as the distilled water. After the ITO was washed for 30 minutes, ultrasonic washing was repeated twice by using distilled water for 10 minutes. After the washing using distilled water was completed, ultrasonic washing was conducted by using isopropyl alcohol, acetone, and methanol solvents, and the resulting product was dried and then transported to a plasma washing machine. Furthermore, the substrate was washed using nitrogen plasma for 5 minutes, and then was transported to a vacuum deposition machine. The following compound HAT-CN was thermally vacuum-deposited to have a thickness of 5 nm on the ITO transparent electrode thus prepared, thereby forming a hole injection layer. Subsequently, the following compound HTL1 was thermally vacuum-deposited to have a thickness of 100 nm, and then the following compound HTL2 was thermally vacuum-deposited to have a thickness of 10 nm, thereby forming a hole transport layer. Subsequently, Compound 1 as a host and the following compound BD-A as a dopant (weight ratio 95:5) were simultaneously vacuum-deposited, thereby forming a light emitting layer having a thickness of 20 nm. Subsequently, the following compound ETL was vacuum-deposited to have a thickness of 20 nm, thereby forming an electron transport layer. Subsequently, LiF was vacuum-deposited to have a thickness of 0.5 nm, thereby forming an electron injection layer. Subsequently, aluminum was deposited to have a thickness of 100 nm to form a negative electrode, thereby manufacturing an organic light emitting device.

The structures of the compounds used in the examples are as follows.

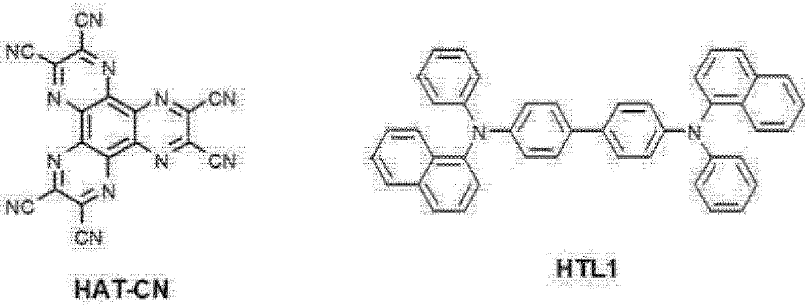

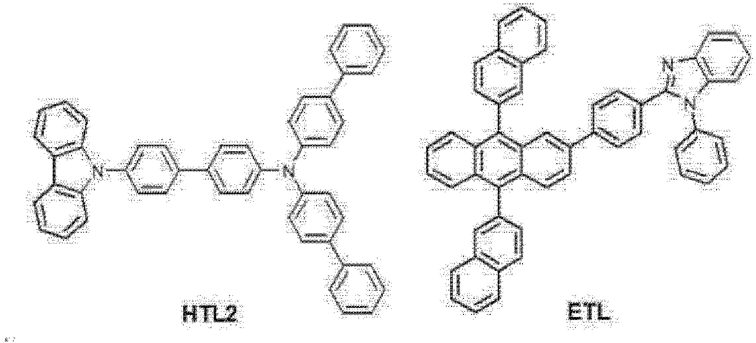

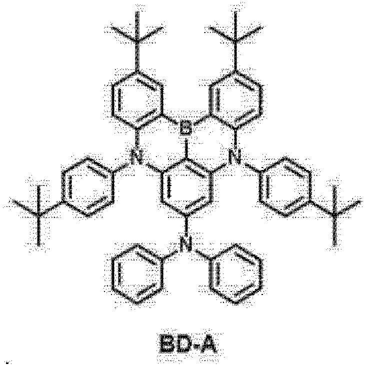

### Examples 1 to 30

Organic light emitting devices were manufactured in the same manner as in Example 1, except that in Example 1, compounds described in the following Table 1 were used instead of Compound 1 as the light emitting layer.

### Comparative Examples 1 to 5

Organic light emitting devices were manufactured in the same manner as in Example 1, except that in Example 1, compounds described in the following Table 1 were used instead of Compound 1 as the light emitting layer. The compounds of BH-A, BH-B, BH-C, BH-D and BH-E used in Comparative Examples 1 to 5 in the following Table 1 are as follows.

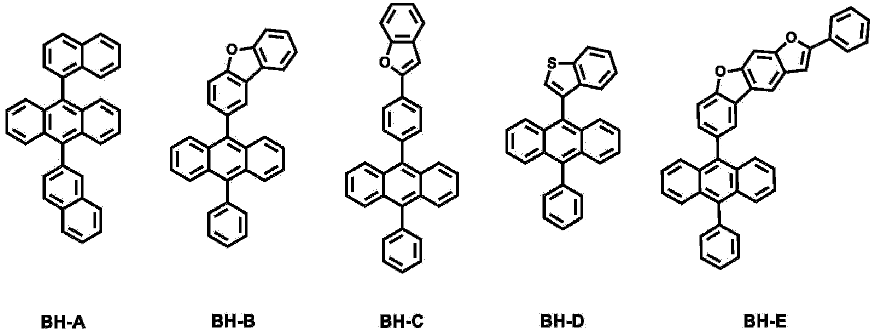

In the organic light emitting devices manufactured in Examples 1 to 30 and Comparative Examples 1 to 5, the driving voltage and the light emitting efficiency were measured at a current density of 10 mA/cm², and a time (LT) for reaching a 95% value compared to the initial luminance was measured at a current density of 20 mA/cm², and the results are shown in the following Table 1.

**[Table 1]**

| | Compound | Value measured at 10 mA/cm² | | Color coordinate (x, y) | LT (T95%) |
|---|---|---|---|---|---|
| | (Light emitting layer host) | Vop | Cd/A | | |
| Example 1 | Compound 1 | 4.09 | 6.92 | (0.145, 0.043) | 220 |
| Example 2 | Compound 2 | 4.11 | 6.88 | (0.144, 0.044) | 186 |
| Example 3 | Compound 3 | 4.12 | 6.63 | (0.145, 0.044) | 177 |
| Example 4 | Compound 4 | 4.2 | 6.71 | (0.145, 0.044) | 200 |
| Example 5 | Compound 5 | 4.18 | 6.58 | (0.146, 0.045) | 180 |
| Example 6 | Compound 6 | 4.23 | 6.44 | (0.144, 0.043) | 163 |
| Example 7 | Compound 7 | 4.16 | 6.65 | (0.145, 0.044) | 193 |
| Example 8 | Compound 8 | 4.09 | 6.7 | (0.146, 0.045) | 173 |
| Example 9 | Compound 9 | 4.08 | 6.87 | (0.145, 0.045) | 210 |
| Example 10 | Compound 10 | 4.1 | 6.91 | (0.145, 0.046) | 270 |
| Example 11 | Compound 11 | 4.11 | 6.56 | (0.143, 0.045) | 223 |
| Example 12 | Compound 12 | 4.25 | 6.35 | (0.144, 0.045) | 199 |
| Example 13 | Compound 13 | 4.2 | 6.57 | (0.143, 0.047) | 273 |
| Example 14 | Compound 14 | 4.23 | 6.33 | (0.144, 0.048) | 167 |
| Example 15 | Compound 15 | 4.2 | 6.57 | (0.143, 0.046) | 166 |
| Example 16 | Compound 16 | 4.29 | 6.4 | (0.143, 0.042) | 156 |
| Example 17 | Compound 17 | 4.15 | 6.77 | (0.142, 0.047) | 188 |
| Example 18 | Compound 18 | 4.3 | 6.7 | (0.145, 0.044) | 157 |
| Example 19 | Compound 19 | 4.11 | 6.67 | (0.144, 0.043) | 191 |
| Example 20 | Compound 20 | 4.14 | 6.80 | (0.144, 0.044) | 231 |
| Example 21 | Compound 21 | 4.10 | 6.75 | (0.143, 0.045) | 225 |
| Example 22 | Compound 22 | 4.11 | 6.71 | (0.143, 0.044) | 246 |
| Example 23 | Compound 23 | 4.07 | 6.80 | (0.145, 0.047) | 240 |
| Example 24 | Compound 24 | 4.22 | 6.48 | (0.145, 0.043) | 190 |
| Example 25 | Compound 25 | 4.08 | 6.39 | (0.146, 0.045) | 167 |
| Example 26 | Compound 26 | 4.19 | 6.44 | (0.144, 0.047) | 155 |
| Example 27 | Compound 27 | 4.20 | 6.50 | (0.142, 0.047) | 168 |
| Example 28 | Compound 28 | 4.13 | 6.90 | (0.144, 0.045) | 273 |
| Example 29 | Compound 29 | 4.06 | 6.93 | (0.144, 0.046) | 275 |
| Example 30 | Compound 30 | 4.16 | 6.70 | (0.145, 0.045) | 180 |
| Comparative Example 1 | BH-A | 4.61 | 4.31 | (0.144, 0.043) | 119 |
| Comparative Example 2 | BH-B | 4.65 | 5.64 | (0.145, 0.045) | 97 |
| Comparative Example 3 | BH-C | 4.50 | 6 | (0.145, 0.045) | 121 |
| Comparative Example 4 | BH-D | 4.64 | 5.99 | (0.146, 0.046) | 108 |
| Comparative Example 5 | BH-E | 4.4 | 6.03 | (0.143, 0.045) | 113 |

According to Table 1, the organic light emitting devices of Examples 1 to 30 using the compound of Chemical Formula 1 of the present invention exhibited excellent characteristics in terms of efficiency, driving voltage and/or stability. Specifically, Examples 1 to 30 exhibited lower voltage and/or higher efficiency characteristics than the organic light emitting devices of Comparative Examples 1 to 5, and exhibited longer service life characteristics than the organic light emitting devices of Comparative Examples 1 to 5 even in terms of service life.

Furthermore, Examples 10, 13, 28 and 29 showed that the service life was additionally improved by deuterium substitution.

Although the preferred exemplary embodiments of the present invention have been described above, the present invention is not limited thereto, and various modifications can be made and carried out within the scopes of the claims and the detailed description of the invention, and also fall within the scope of the invention.

## Claims

1. A compound of Chemical Formula 1: 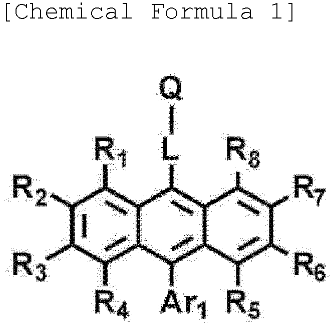 wherein, in Chemical Formula 1:
R₁ to R₈ are the same as or different from each other, and are each independently hydrogen, deuterium, a halogen group, a substituted or unsubstituted alkyl group, a substituted or unsubstituted cycloalkyl group, a substituted or unsubstituted aryl group, or a substituted or unsubstituted heteroaryl group;
Ar₁ is a substituted or unsubstituted aryl group, or a substituted or unsubstituted heteroaryl group;
L is a direct bond or a substituted or unsubstituted arylene group;
Q is any one of the groups of the following Chemical Formulae 2-1 to 2-6, 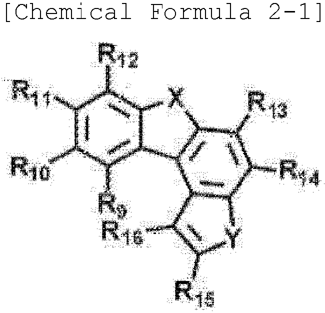 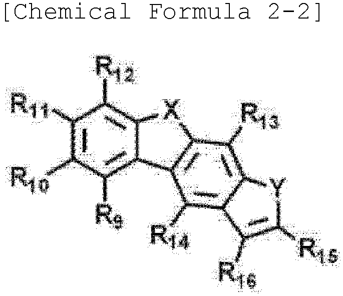 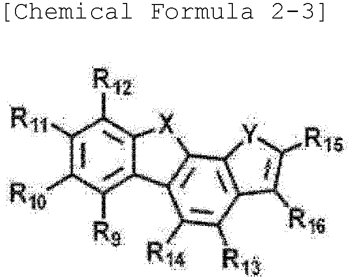 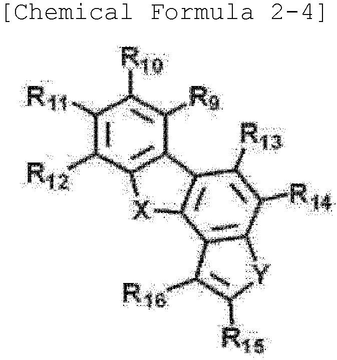 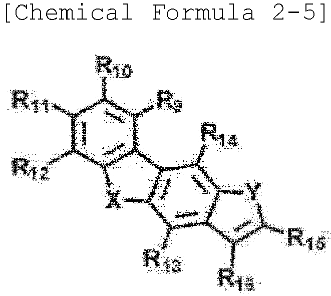 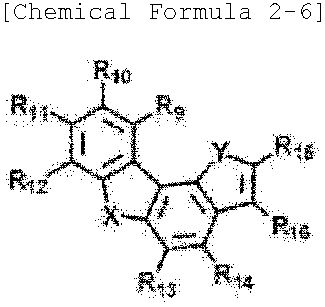
wherein in Chemical Formulae 2-1 to 2-6:
X and Y are the same as or different from each other, and are each independently O or S;
any one of R₉ to R₁₆ is linked to L of Chemical Formula 1;
the other groups which are not linked to L of Chemical Formula 1 among R₉ to R₁₆ are the same as or different from each other, and are each independently hydrogen, deuterium, a halogen, a cyano group, a silyl group, a substituted or unsubstituted alkyl group, a substituted or unsubstituted cycloalkyl group, a substituted or unsubstituted aryl group, or a substituted or unsubstituted heteroaryl group,
provided that in Chemical Formula 1, when L is a direct bond, Q is Chemical Formula 2-2, both X and Y are O, and any one of R₉ to R₁₄ and R₁₆ is linked to L of Chemical Formula 1, if any one of Ar₁ and R₁₅ is an unsubstituted phenyl group, the other is a substituted or unsubstituted aryl group having 10 to 60 carbon atoms, or a substituted or unsubstituted heteroaryl group having 2 to 60 carbon atoms.

2. The compound of claim 1, wherein R₁ to R₈ are the same as or different from each other, and are each independently hydrogen or deuterium.

3. The compound of claim 1, wherein the Ar₁ is a substituted or unsubstituted phenyl group, a substituted or unsubstituted biphenyl group; a substituted or unsubstituted naphthyl group, a substituted or unsubstituted phenanthrene group, a substituted or unsubstituted dibenzofuran group, a substituted or unsubstituted dibenzothiophene group, or a substituted or unsubstituted carbazole group; and
the term 'substituted or unsubstituted' means being unsubstituted or substituted with one or more substituents selected from the group consisting of deuterium, a halogen group, an alkyl group having 1 to 60 carbon atoms, an aryl group having 6 to 60 carbon atoms, and a heteroaryl group having 2 to 60 carbon atoms, or one or more adjacent substituents are bonded to each other to form a ring.

4. The compound of claim 1, wherein L is a direct bond; a phenylene group which is unsubstituted or substituted with deuterium; or a naphthylene group which is unsubstituted or substituted with deuterium.

5. The compound of claim 1, wherein Chemical Formula 2-1 is any one of the following Chemical Formulae 2-1-A to 2-1-D: 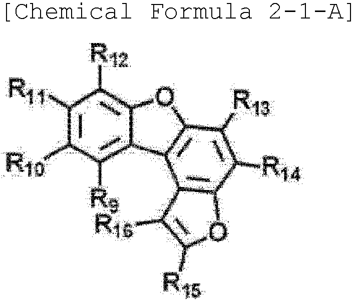 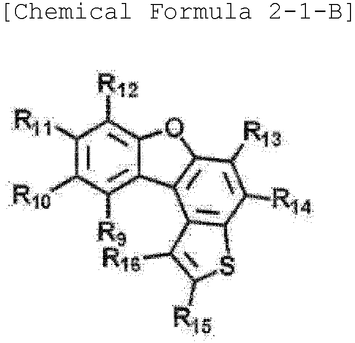 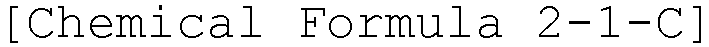 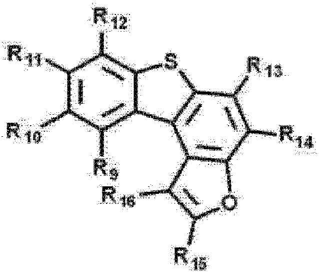 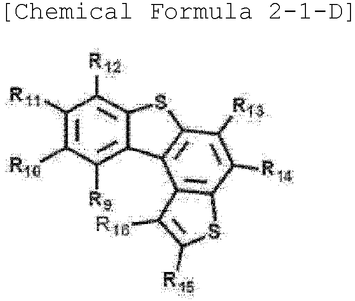 wherein in Chemical Formulae 2-1-A to 2-1-D,
R₉ to R₁₆ are the same as those defined in Chemical Formulae 2-1 to 2-6.

6. The compound of claim 1, wherein Chemical Formula 2-2 is any one of the following Chemical Formulae 2-2-A to 2-2-D: 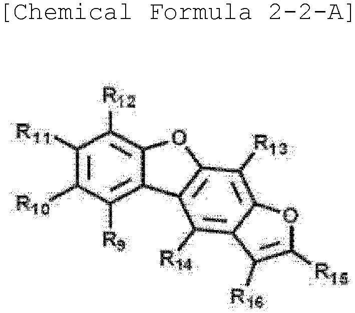 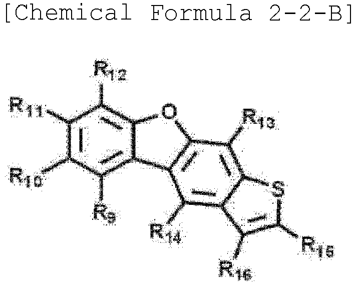 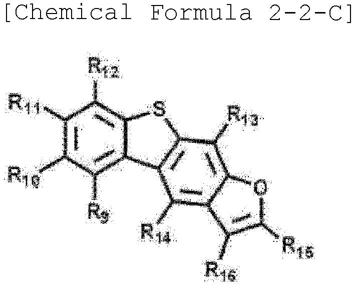 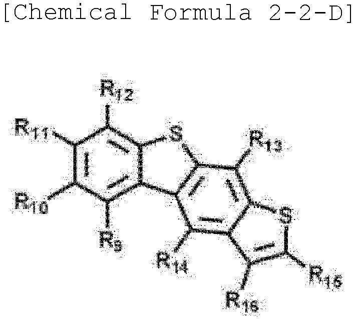 wherein in Chemical Formulae 2-2-A to 2-2-D,
R₉ to R₁₆ are the same as those defined in Chemical Formulae 2-1 to 2-6.

7. The compound of claim 1, wherein Chemical Formula 2-3 is any one of the following Chemical Formulae 2-3-A to 2-3-D: 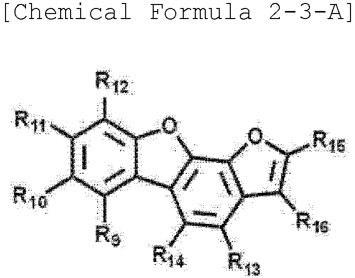 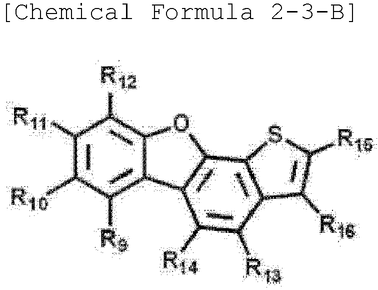 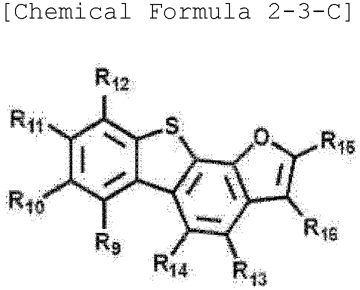 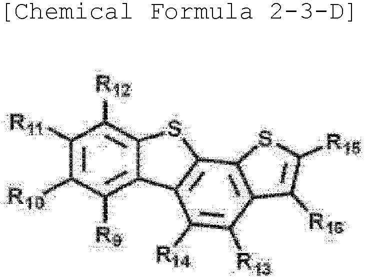 wherein in Chemical Formulae 2-3-A to 2-3-D,
R₉ to R₁₆ are the same as those defined in Chemical Formulae 2-1 to 2-6.

8. The compound of claim 1, wherein Chemical Formula 2-4 is any one of the following Chemical Formulae 2-4-A to 2-4-D: 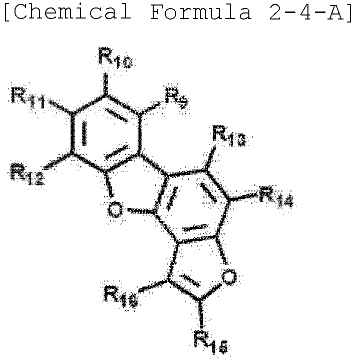 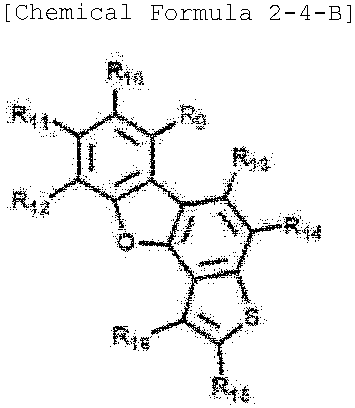 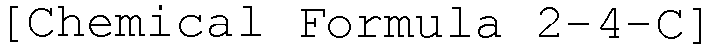 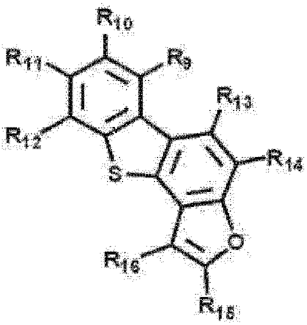 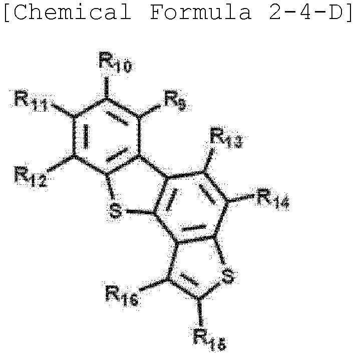 wherein in Chemical Formulae 2-4-A to 2-4-D,
R₉ to R₁₆ are the same as those defined in Chemical Formulae 2-1 to 2-6.

9. The compound of claim 1, wherein Chemical Formula 2-5 is any one of the following Chemical Formulae 2-5-A to 2-5-D: 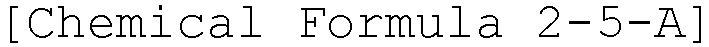 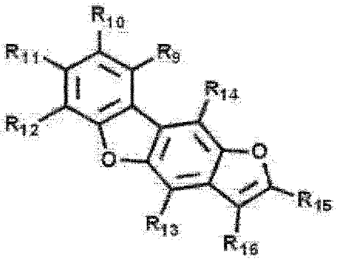 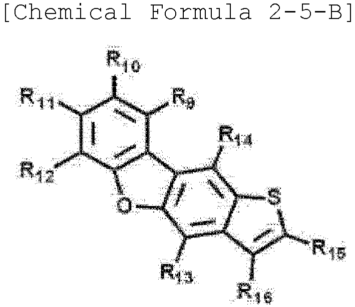 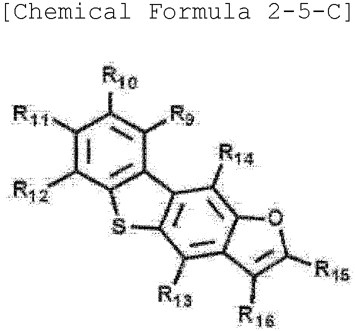 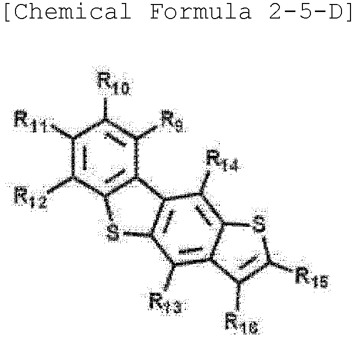 wherein in Chemical Formulae 2-5-A to 2-5-D,
R₉ to R₁₆ are the same as those defined in Chemical Formulae 2-1 to 2-6.

10. The compound of claim 1, wherein Chemical Formula 2-6 is any one of the following Chemical Formulae 2-6-A to 2-6-D: 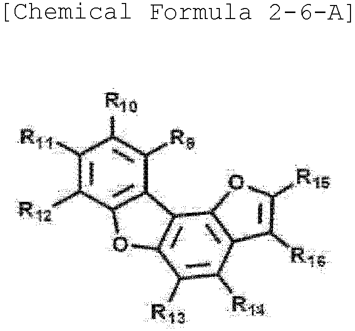 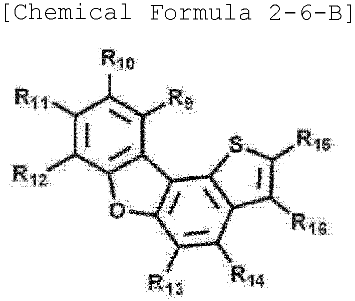 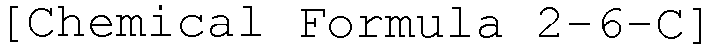 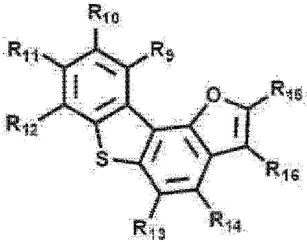 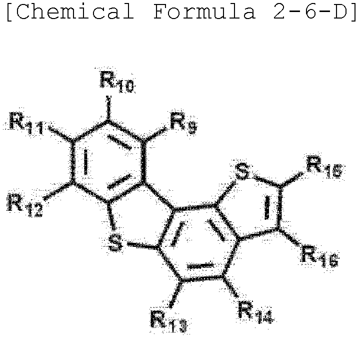 wherein in Chemical Formulae 2-6-A to 2-6-D,
R₉ to R₁₆ are the same as those defined in Chemical Formulae 2-1 to 2-6.

11. The compound of claim 1, wherein in Chemical Formula 1, when L is a direct bond, Q is Chemical Formula 2-2, both X and Y are O, and any one of R₉ to R₁₄ and R₁₆ is linked to L of Chemical Formula 1, if any one of Ar₁ and R₁₅ is an unsubstituted phenyl group, the other is a substituted or unsubstituted biphenyl group, a substituted or unsubstituted naphthyl group, a substituted or unsubstituted dibenzofuran group, or a substituted or unsubstituted dibenzothiophene group, and
the term 'substituted or unsubstituted' means being unsubstituted or substituted with one or more substituents selected from the group consisting of deuterium, a halogen group, an alkyl group having 1 to 60 carbon atoms, an aryl group having 6 to 60 carbon atoms, and a heteroaryl group having 2 to 60 carbon atoms, or the one or more adjacent substituents are bonded to each other to form a ring.

12. The compound of claim 1, wherein the other groups which are not linked to L of Chemical Formula 1 among R₉ to R₁₆ are the same as or different from each other, and are each independently hydrogen, deuterium, a substituted or unsubstituted phenyl group, a substituted or unsubstituted biphenyl group, a substituted or unsubstituted naphthyl group, a substituted or unsubstituted dibenzofuran group, or a substituted or unsubstituted dibenzothiophene group, and
the term 'substituted or unsubstituted' means being substituted with one or more substituents selected from the group consisting of deuterium, a halogen group, an alkyl group having 1 to 60 carbon atoms; an aryl group having 6 to 60 carbon atoms; and a heteroaryl group having 2 to 60 carbon atoms, or the one or more adjacent substituents are bonded to each other to form a ring.

13. The compound of claim 1, wherein the compound of Chemical Formula 1 is any one of the following compounds:

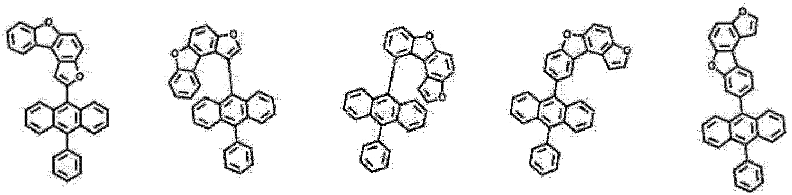

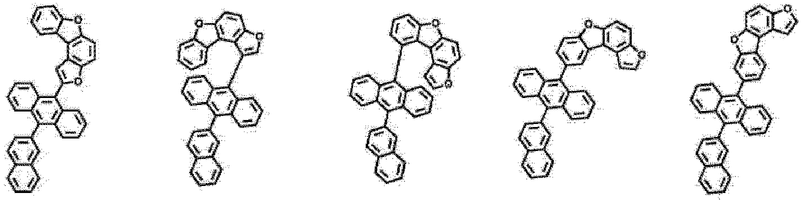

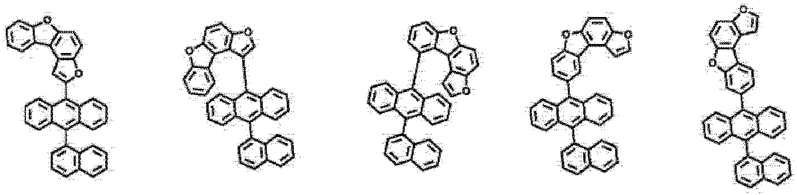

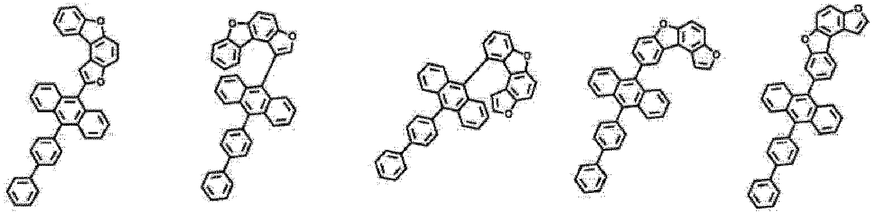

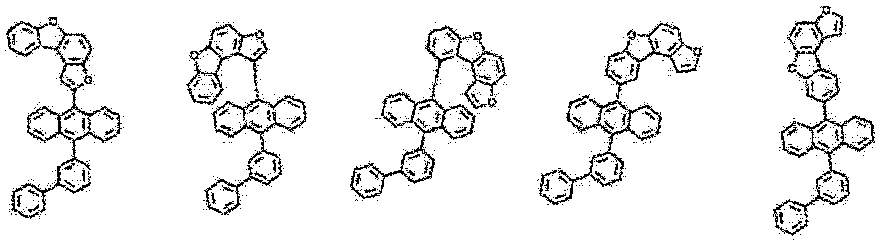

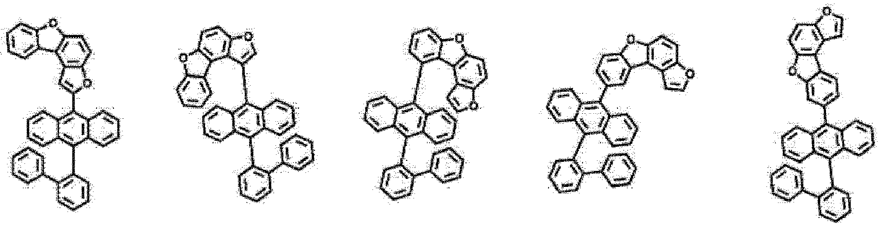

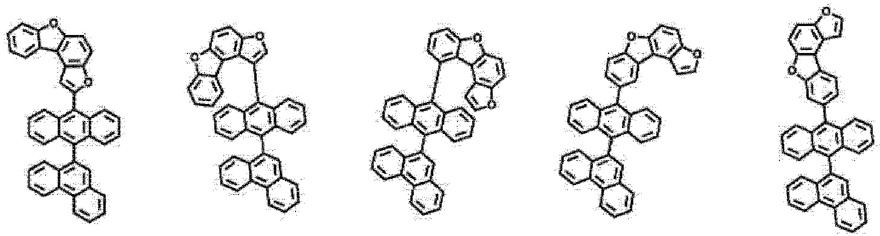

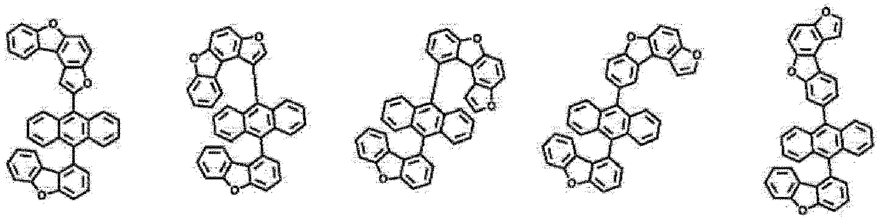

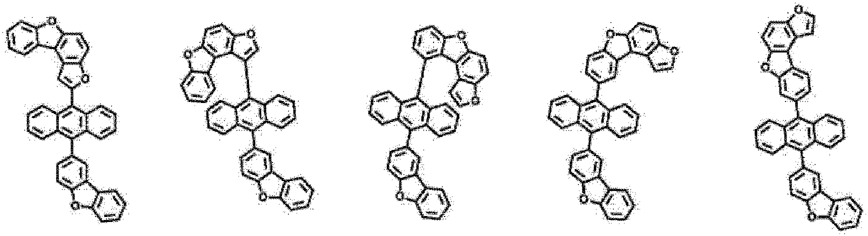

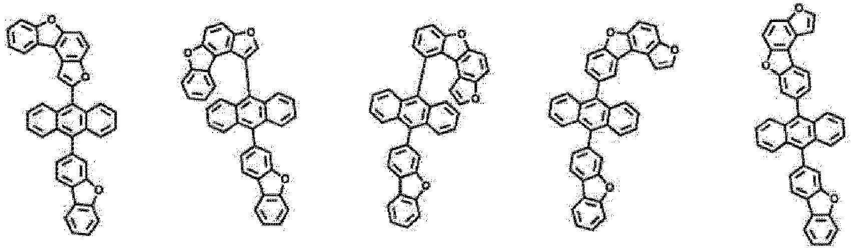

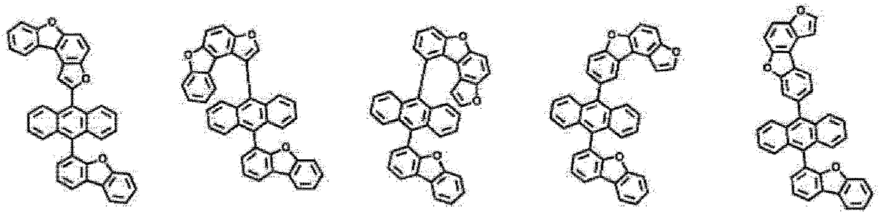

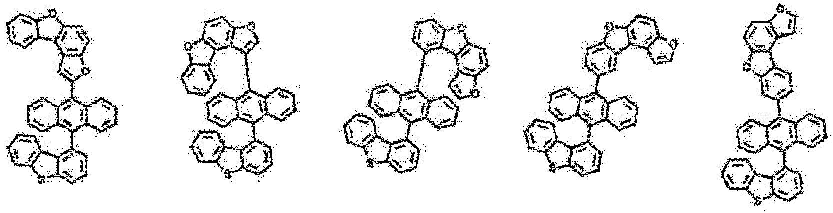

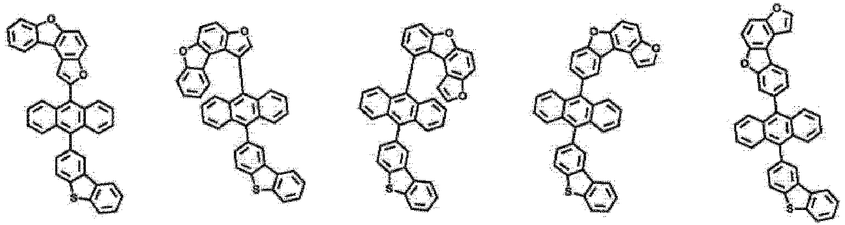

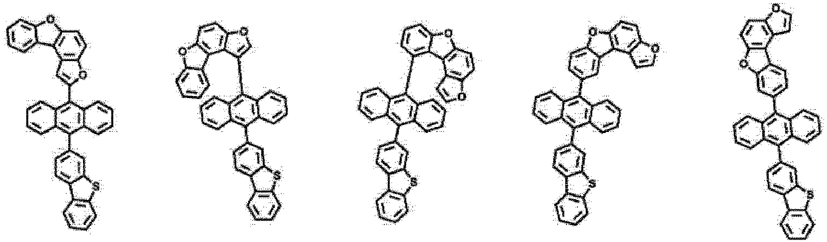

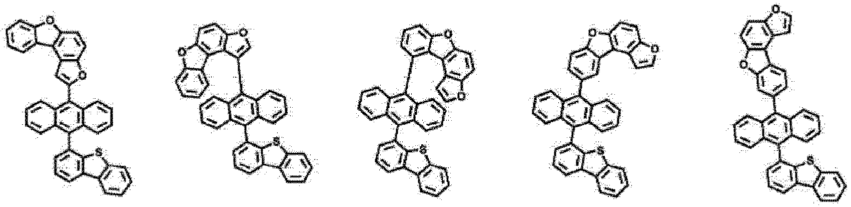

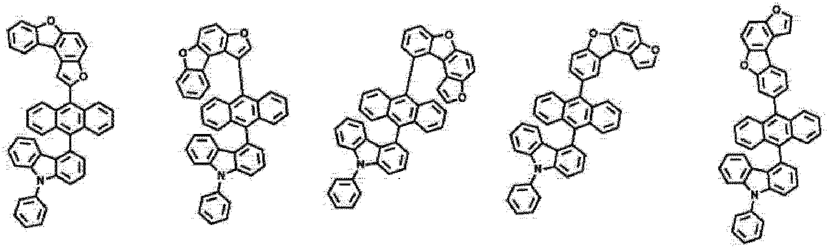

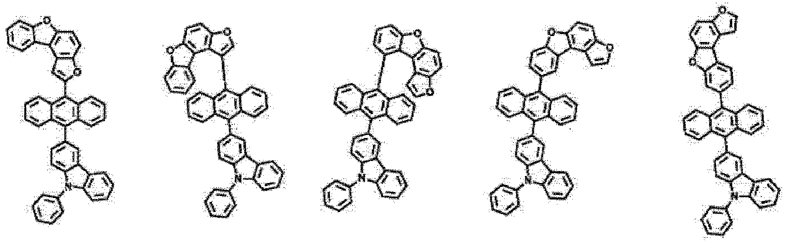

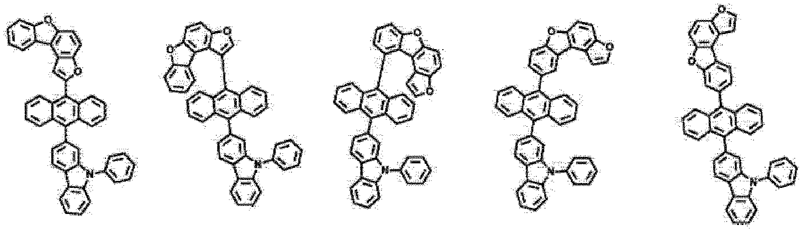

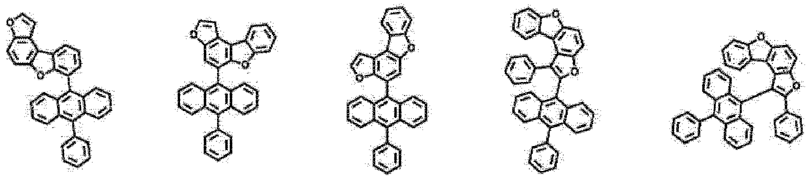

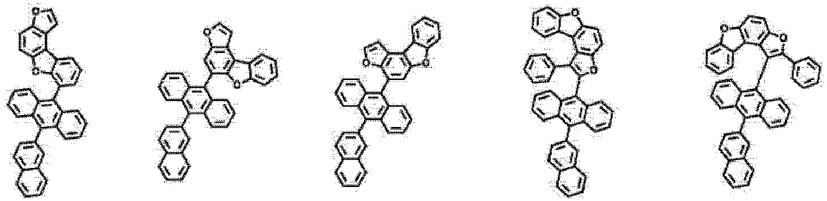

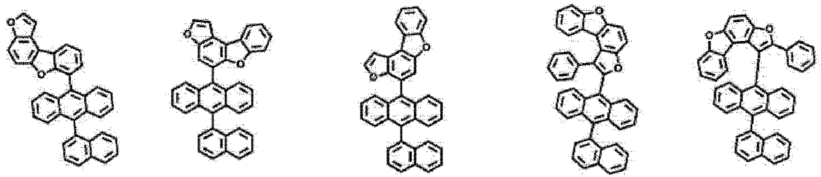

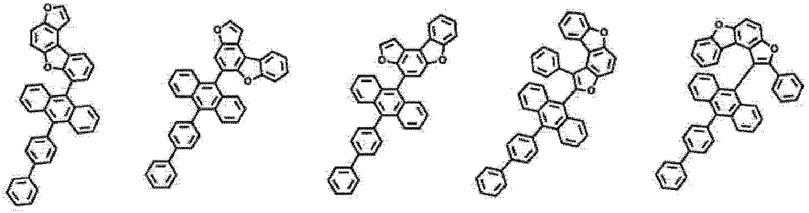

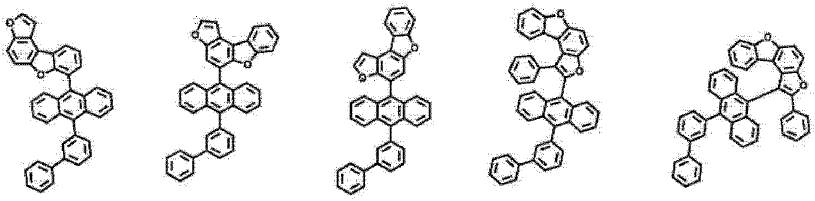

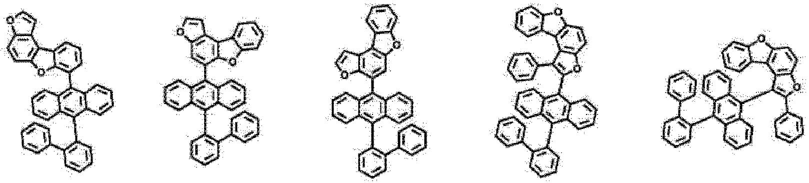

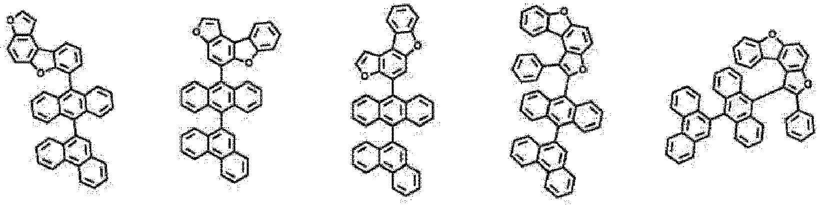

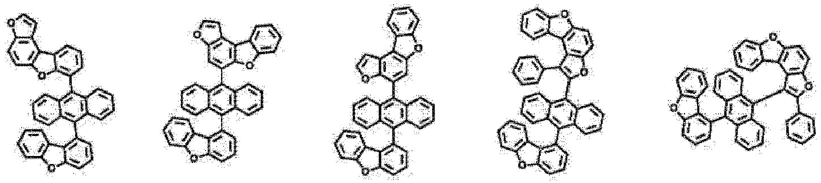

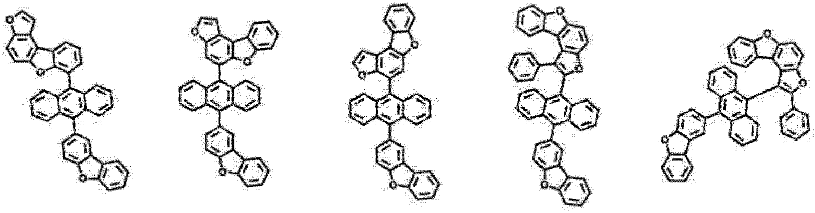

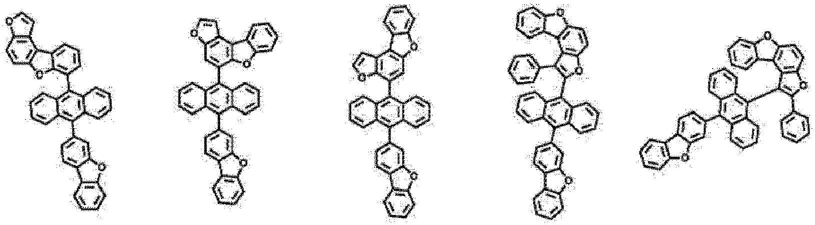

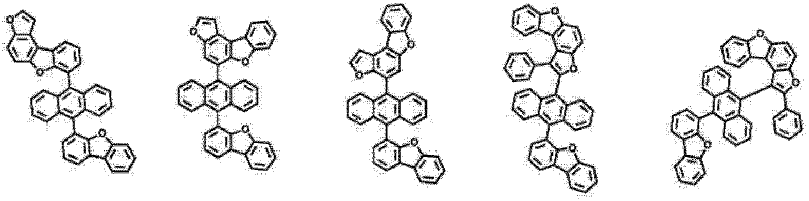

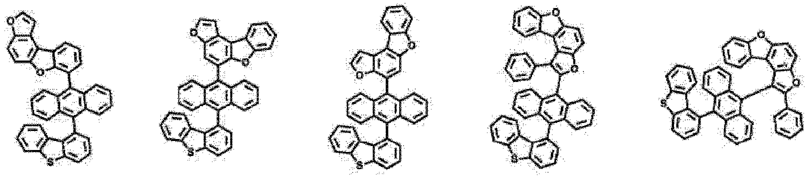

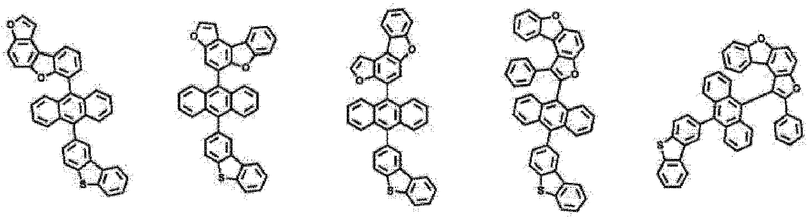

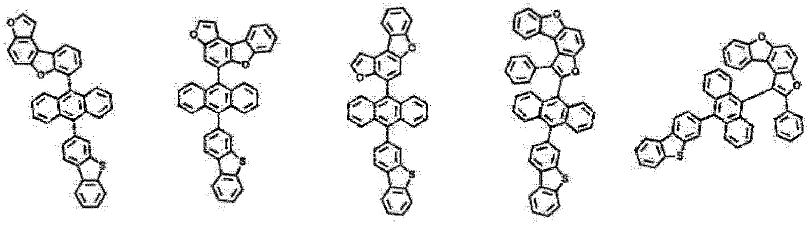

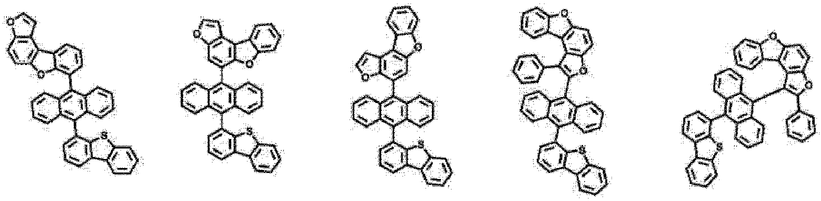

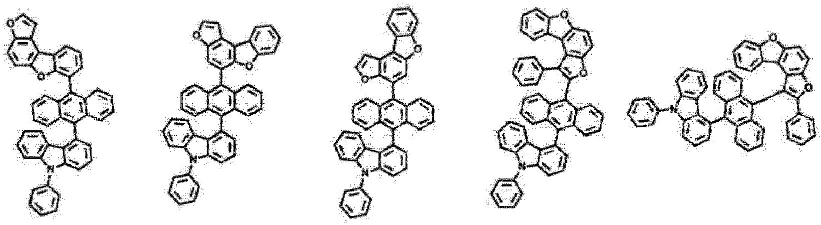

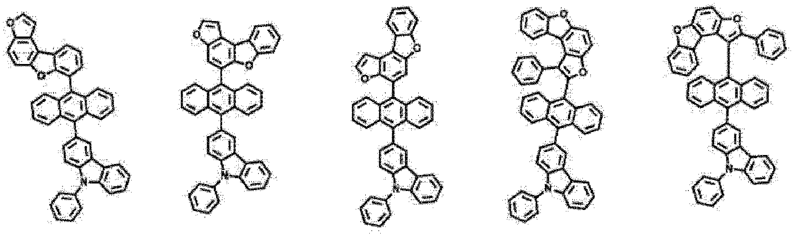

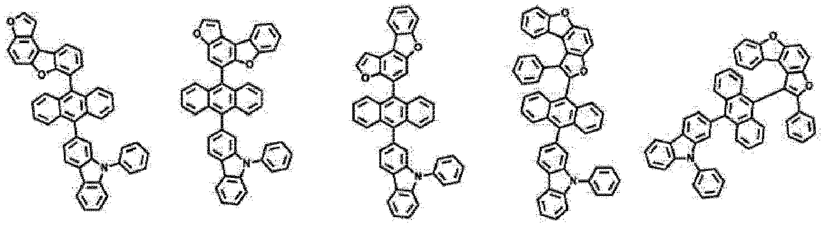

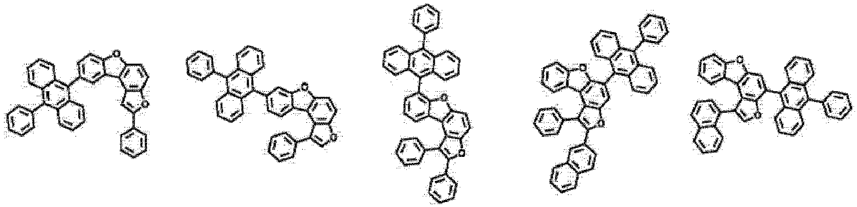

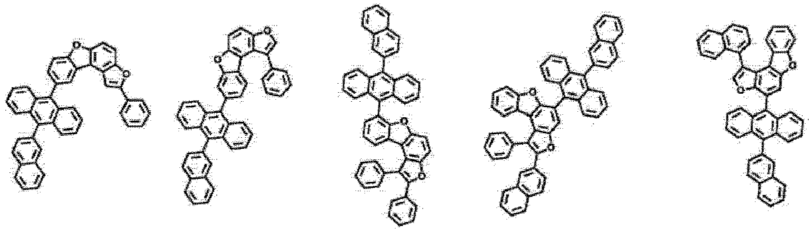

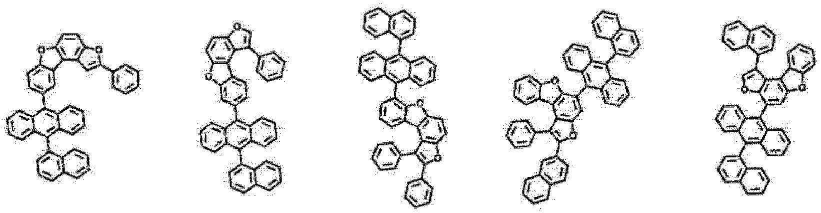

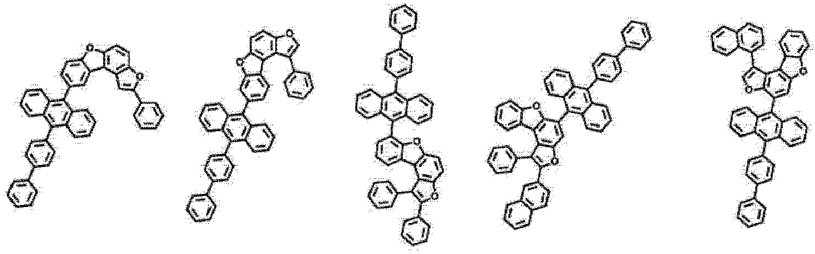

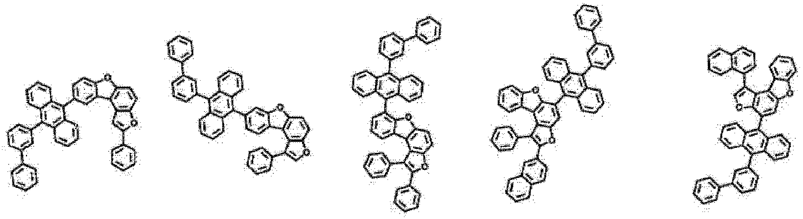

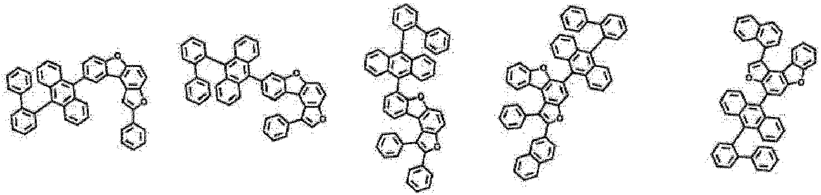

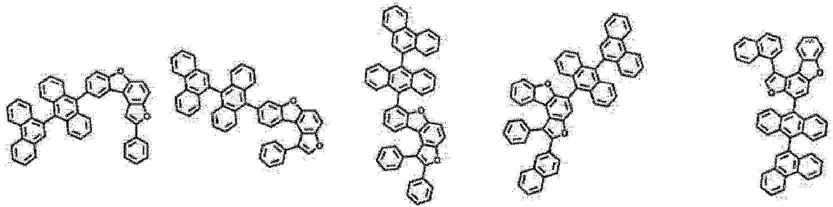

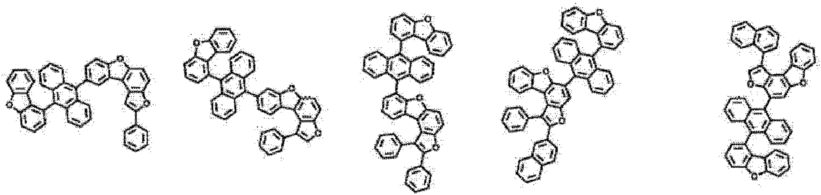

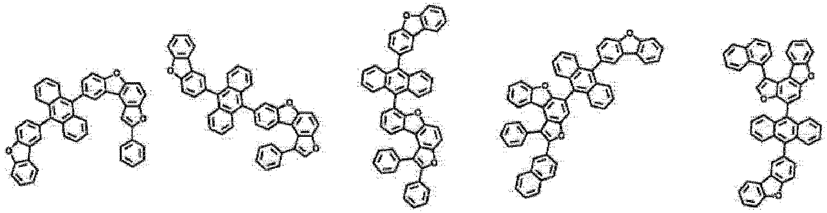

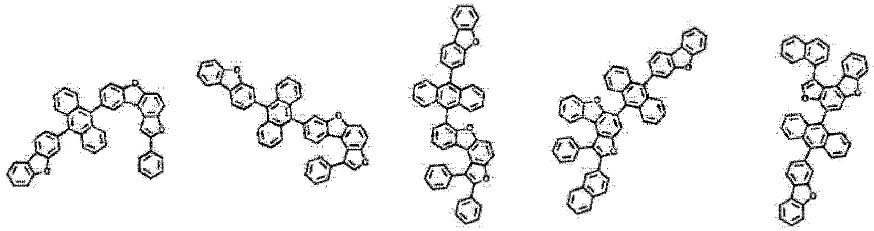

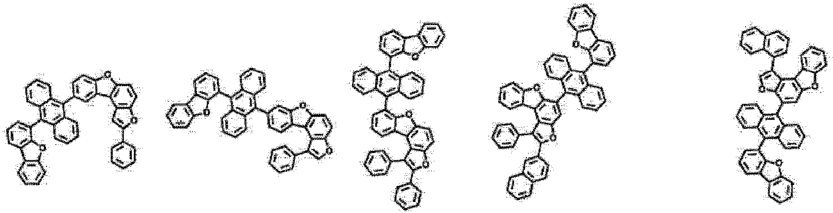

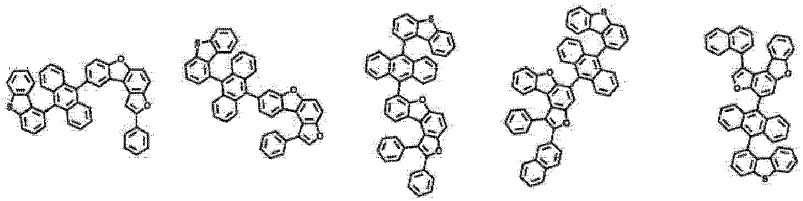

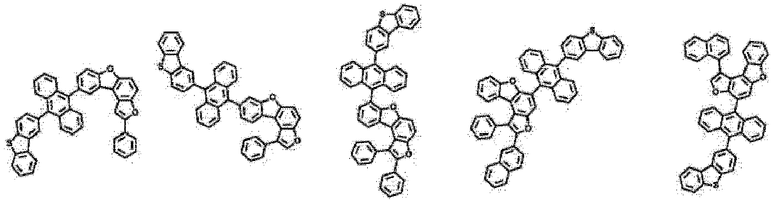

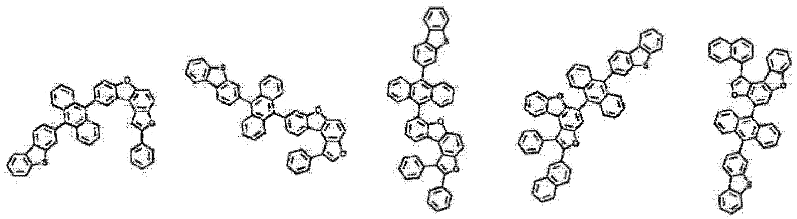

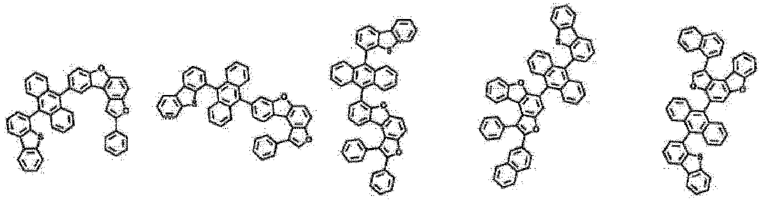

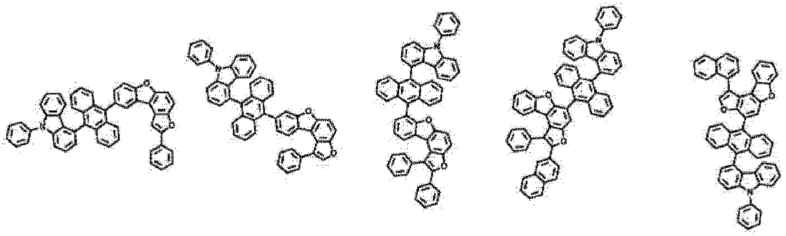

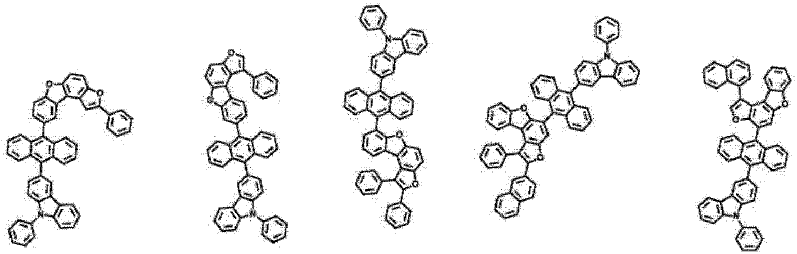

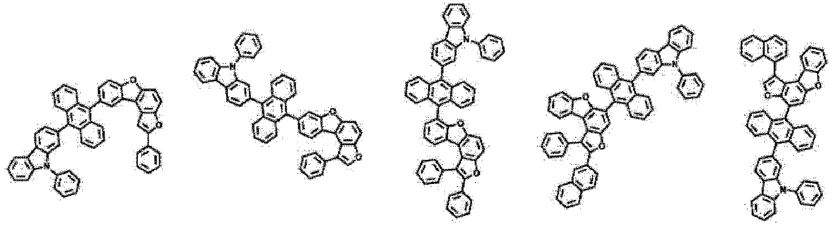

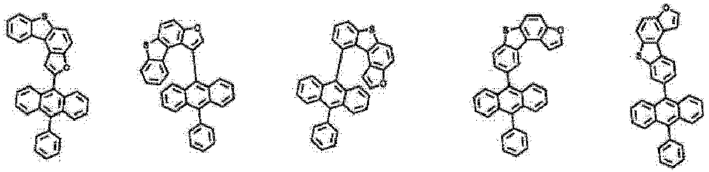

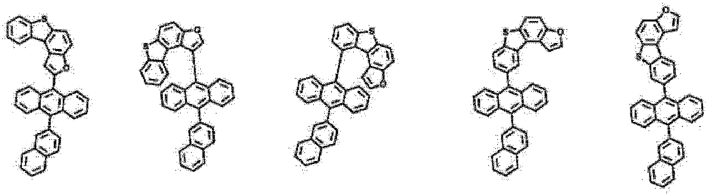

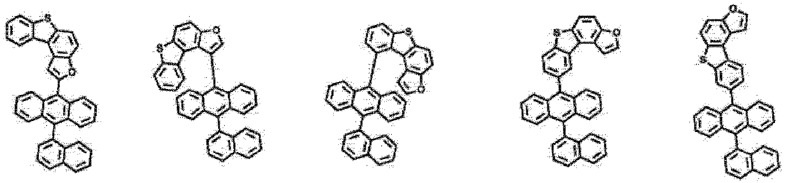

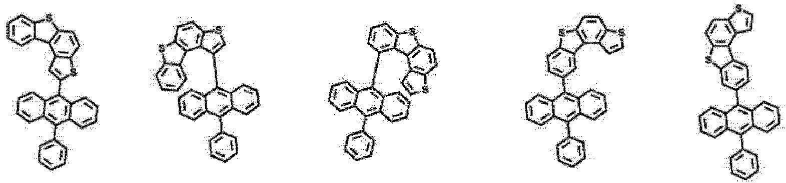

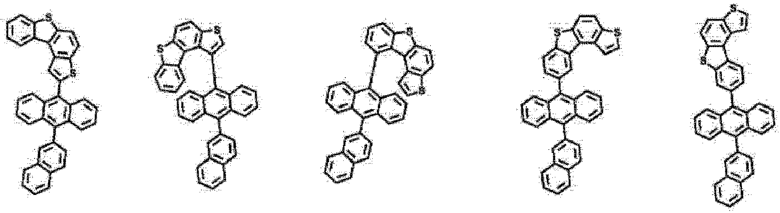

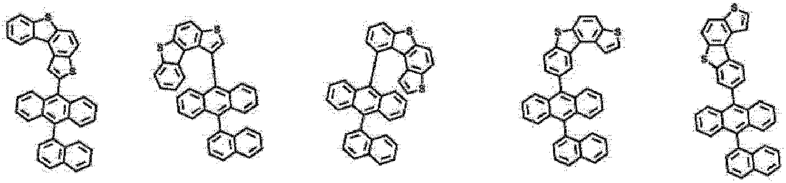

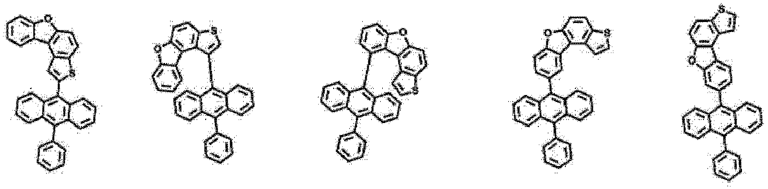

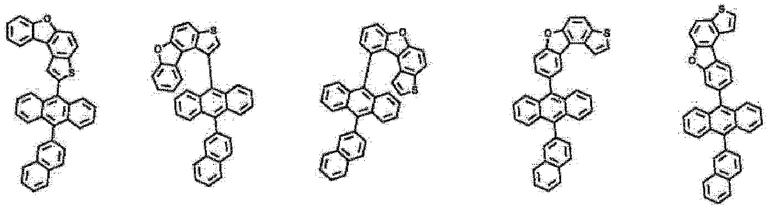

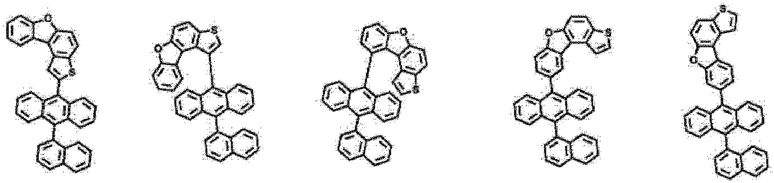

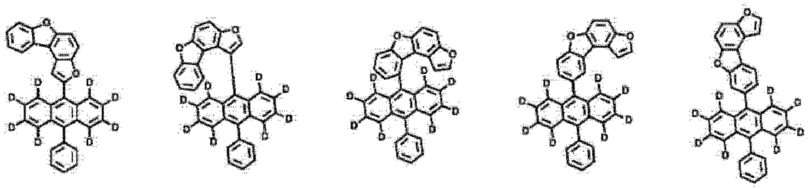

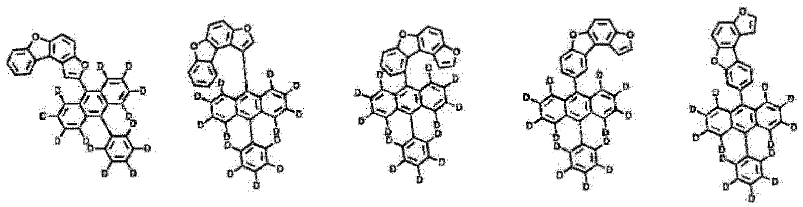

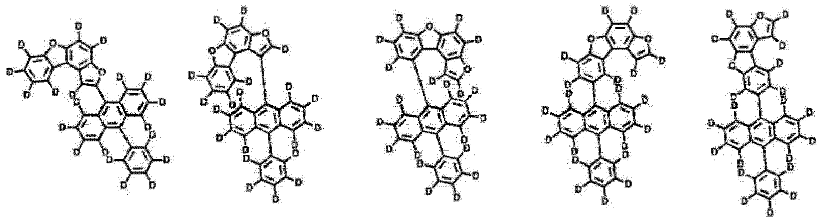

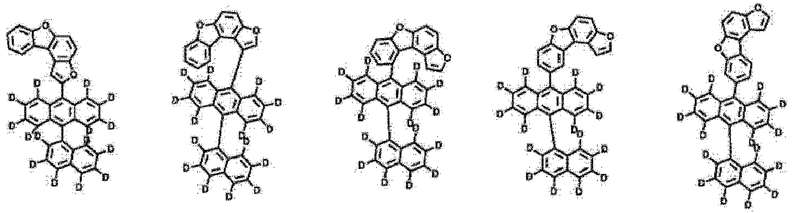

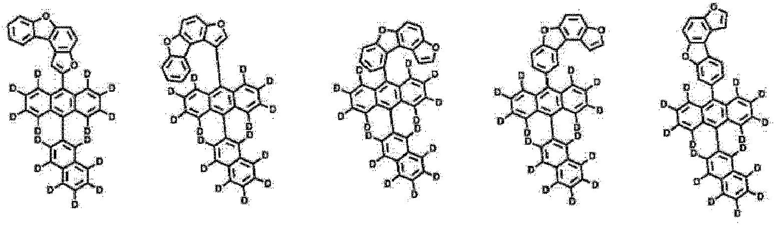

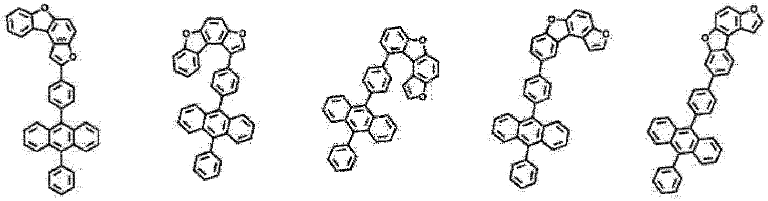

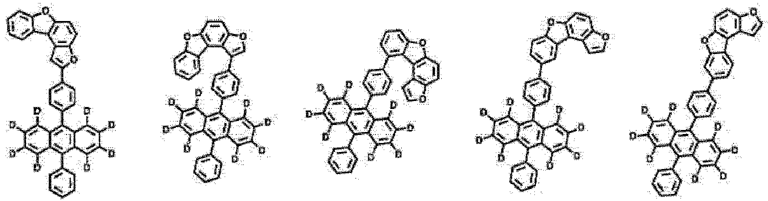

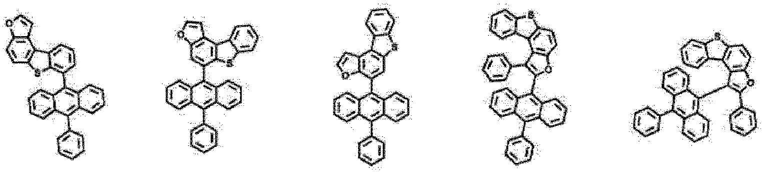

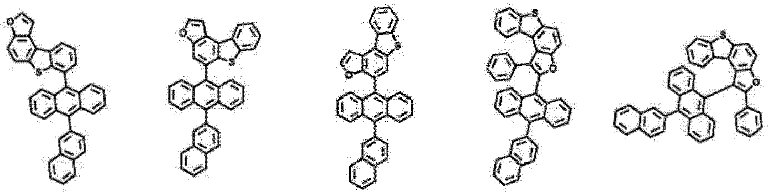

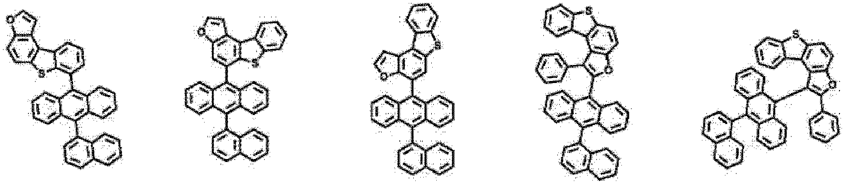

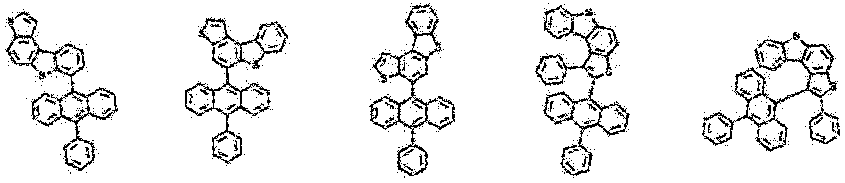

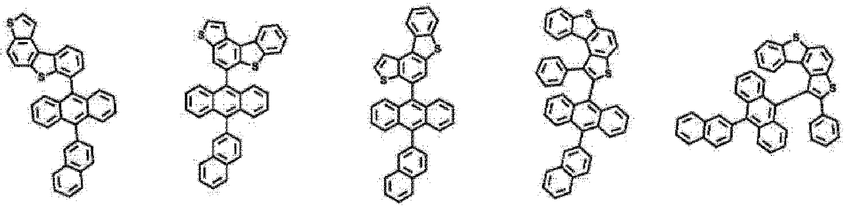

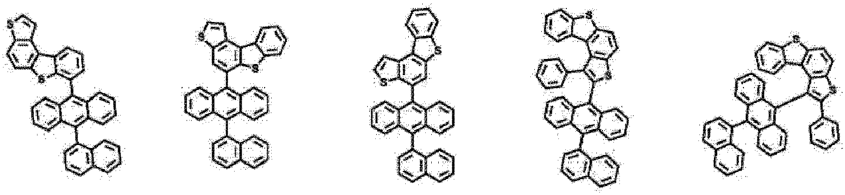

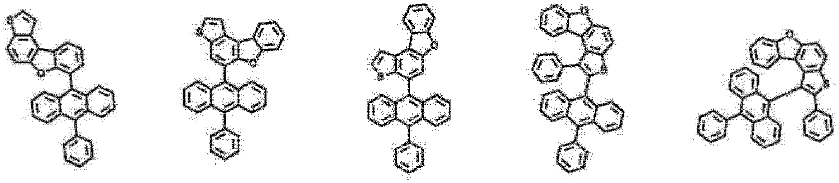

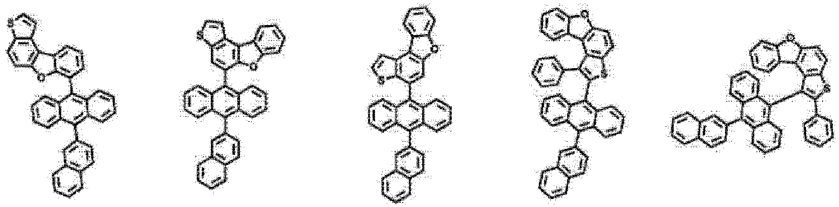

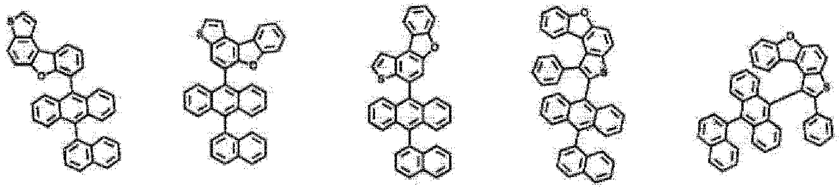

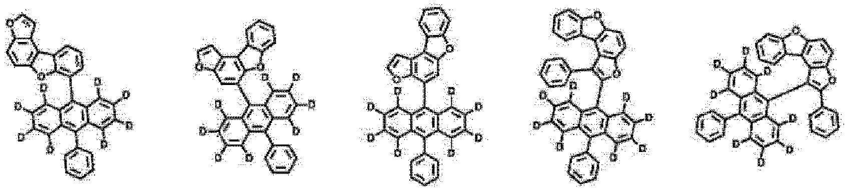

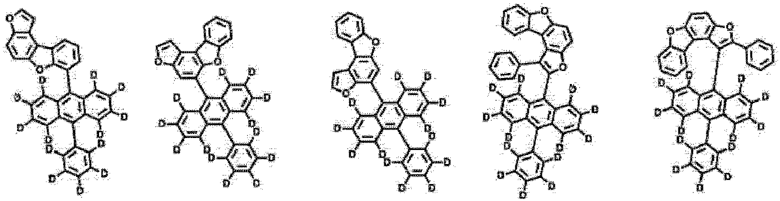

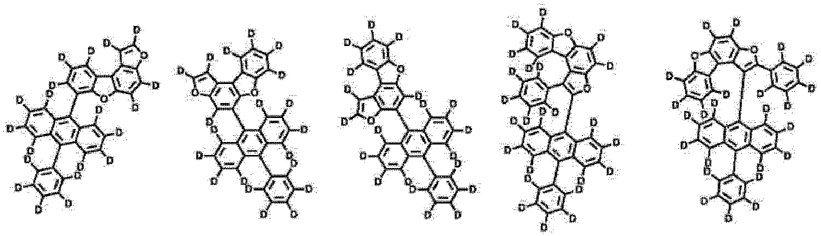

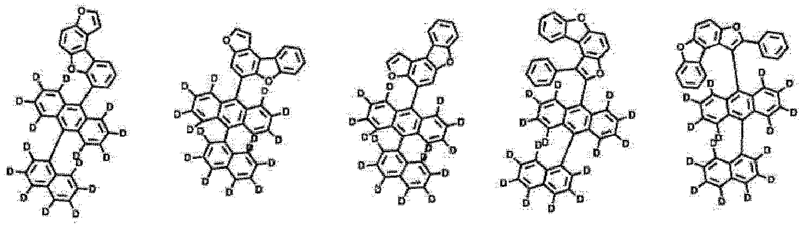

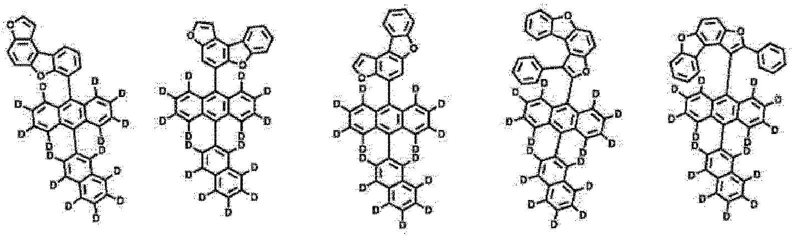

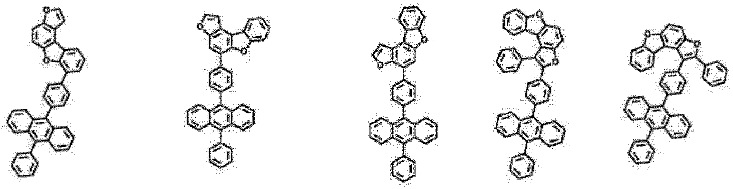

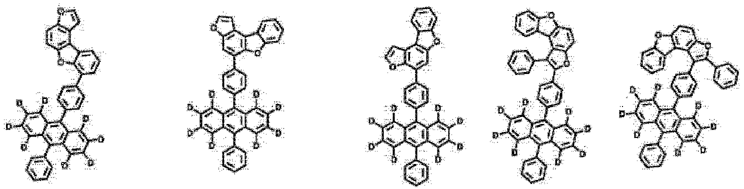

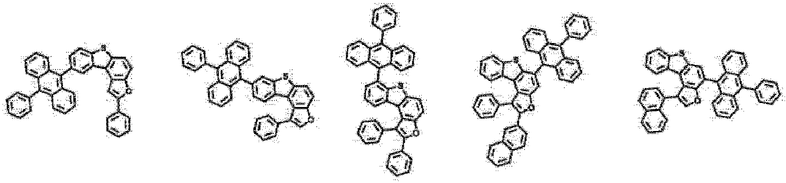

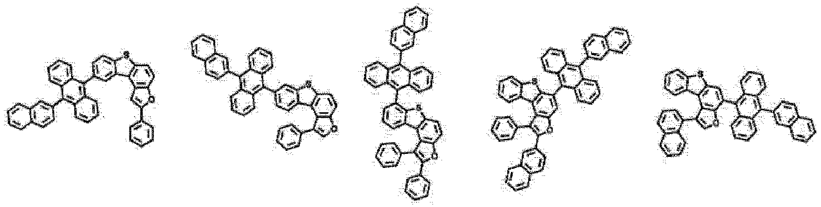

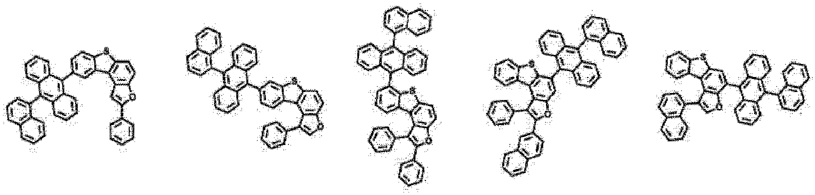

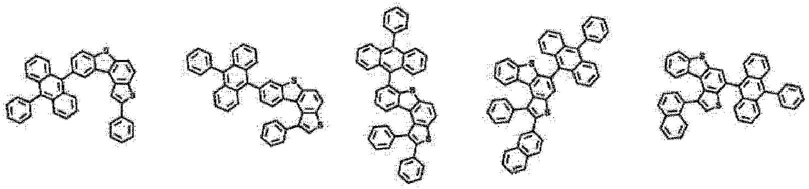

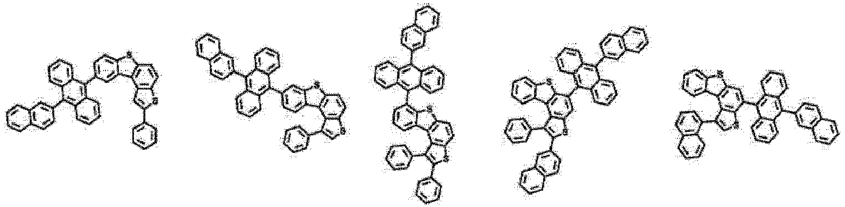

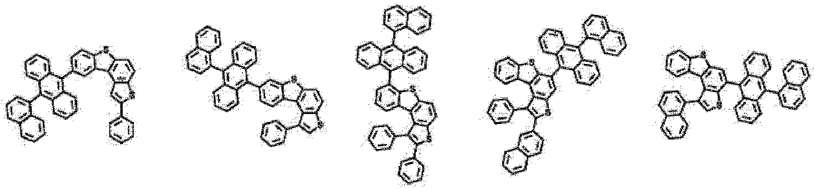

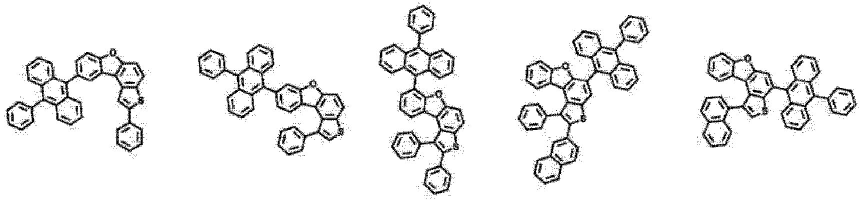

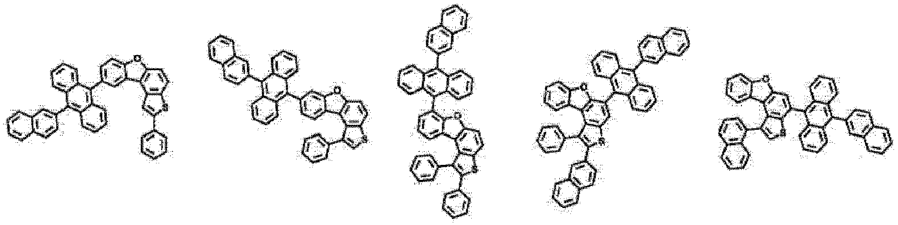

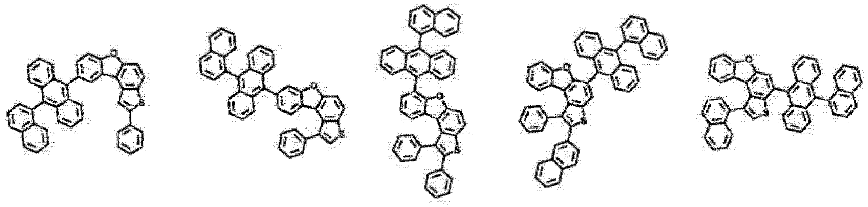

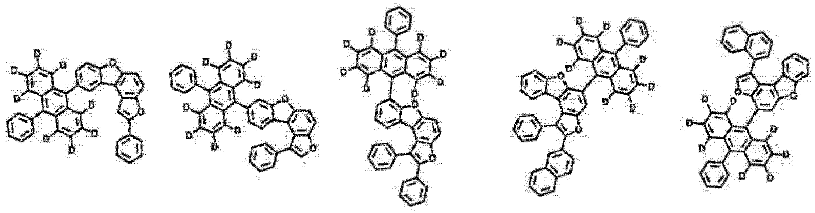

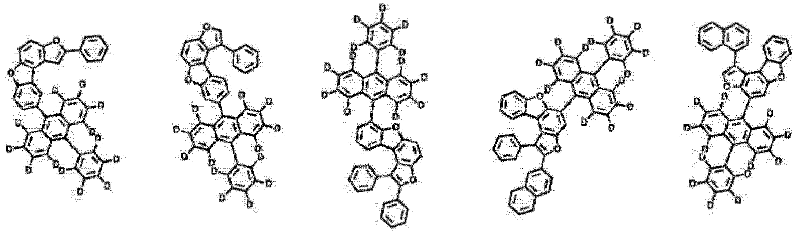

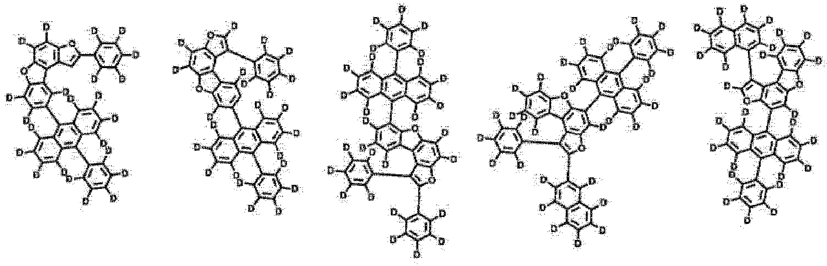

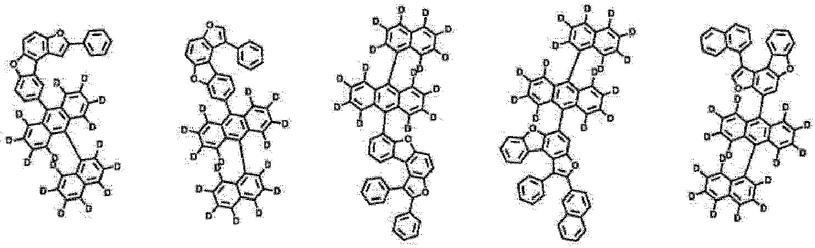

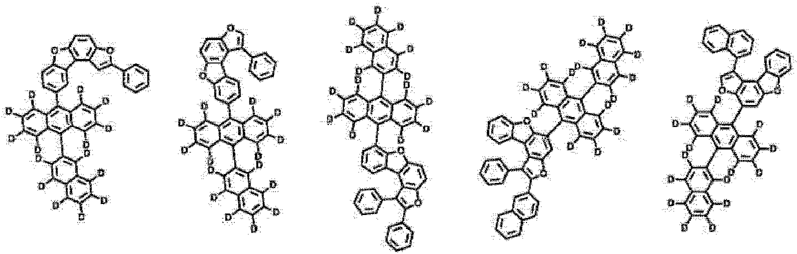

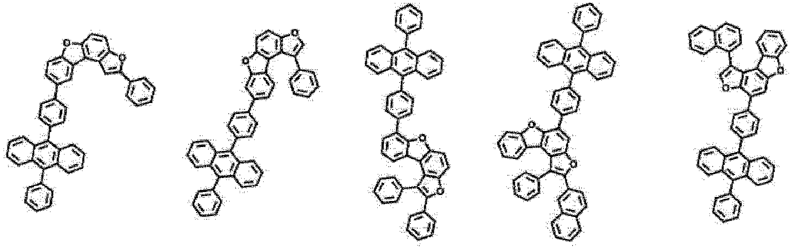

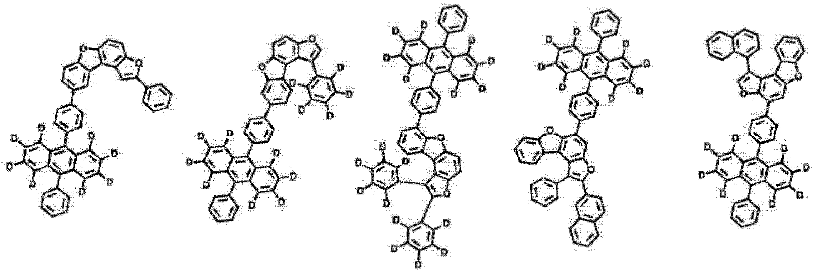

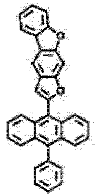

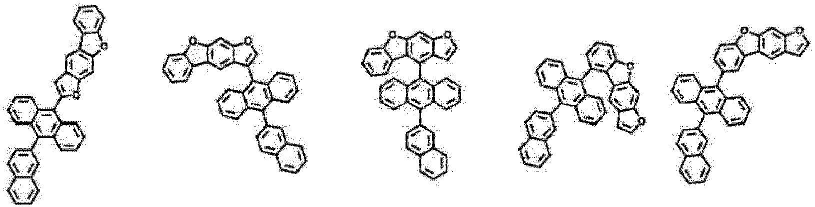

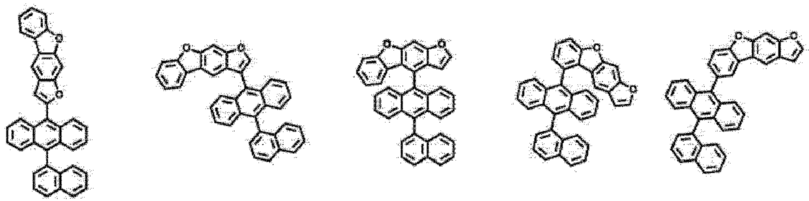

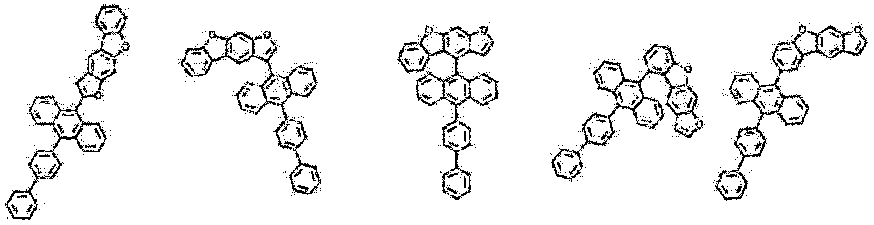

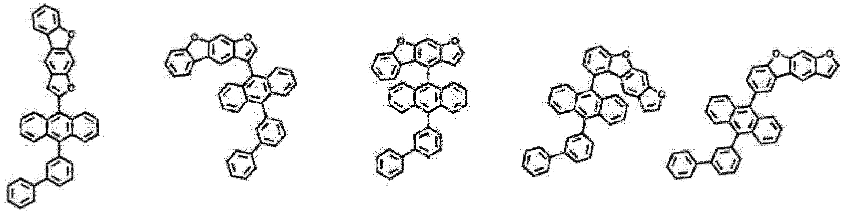

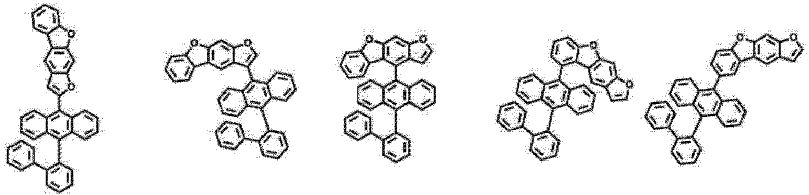

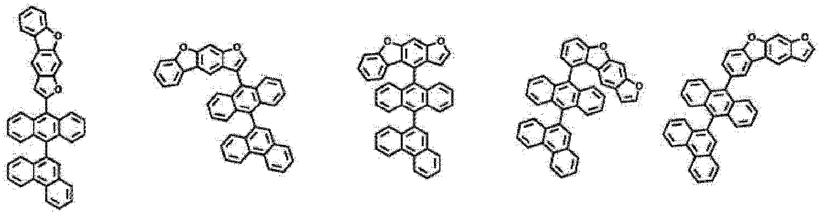

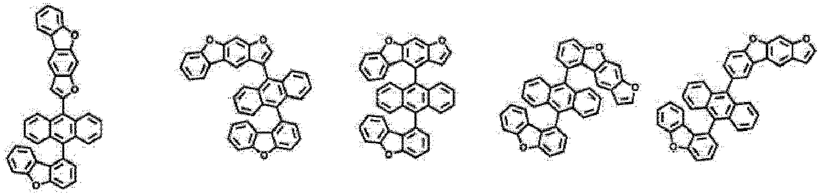

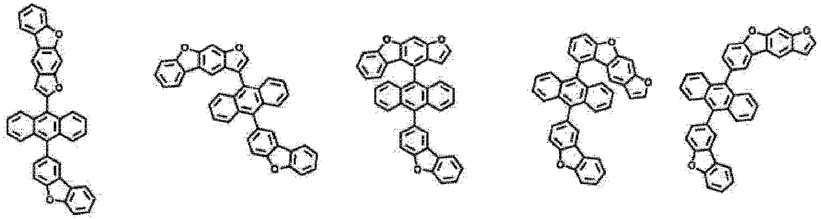

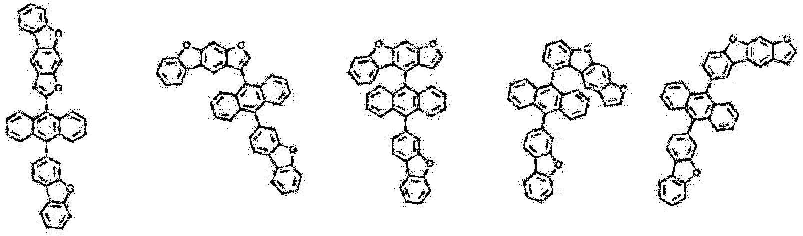

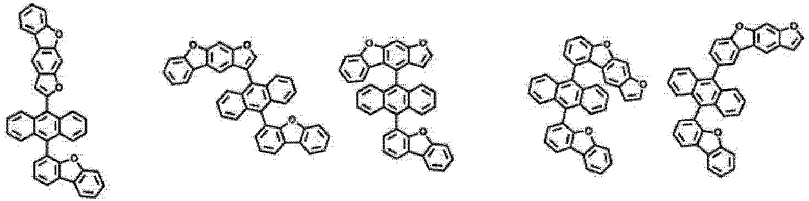

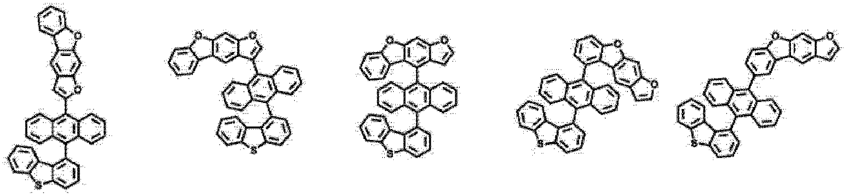

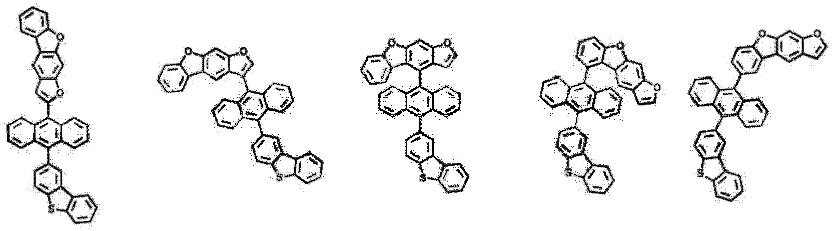

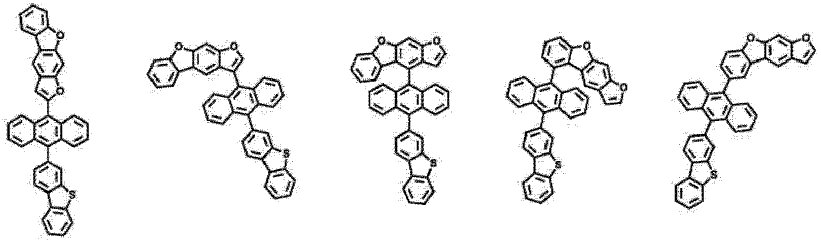

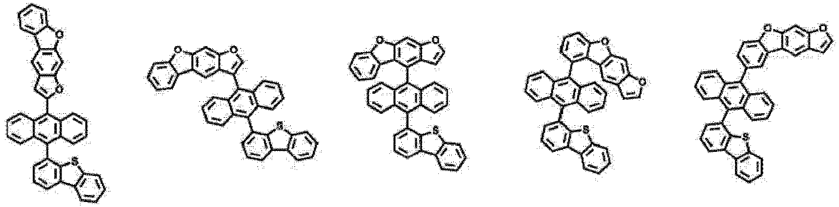

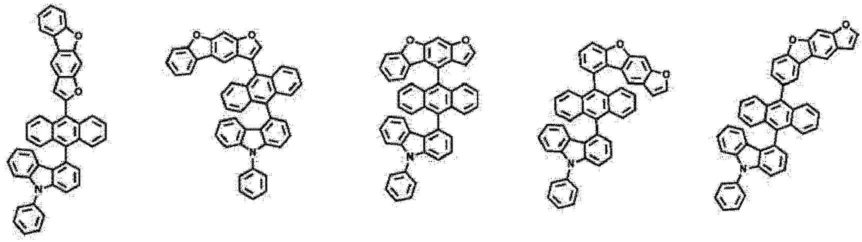

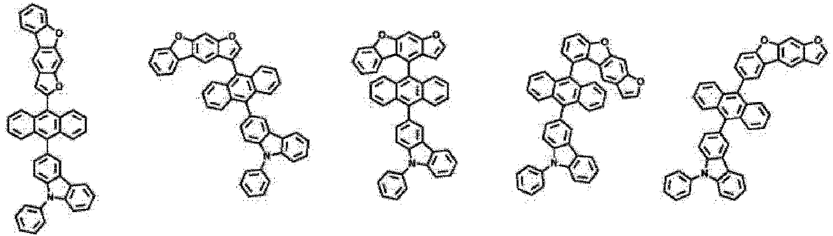

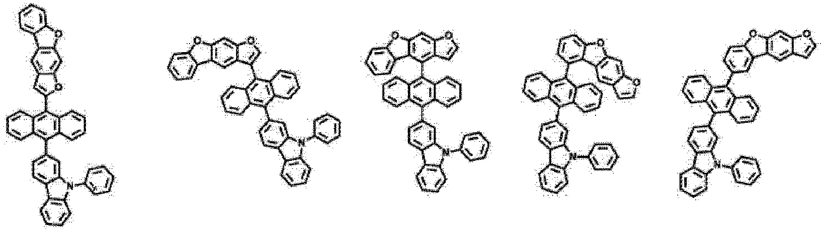

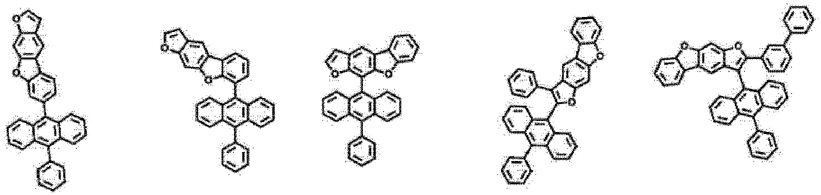

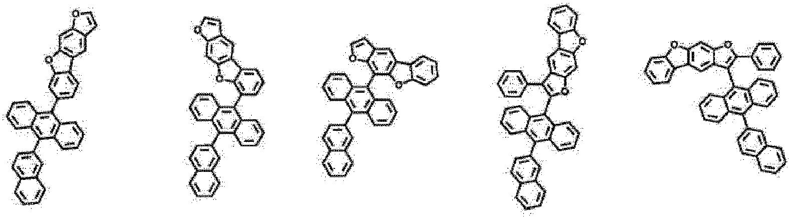

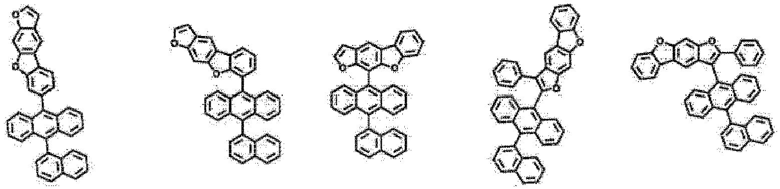

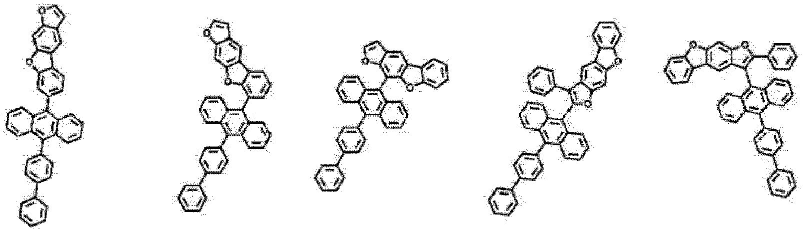

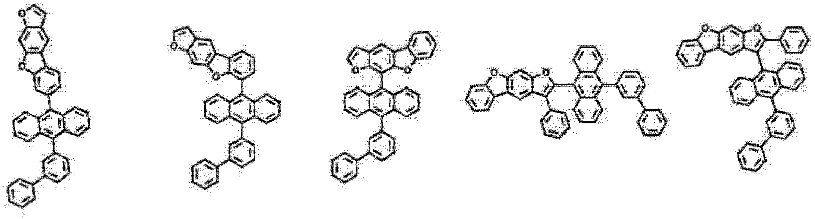

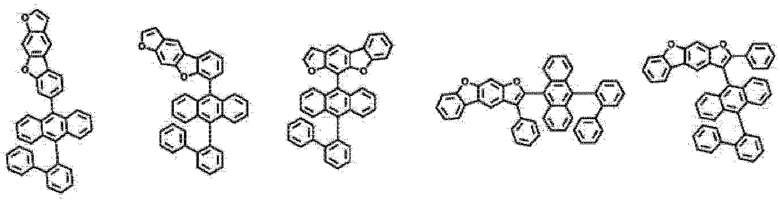

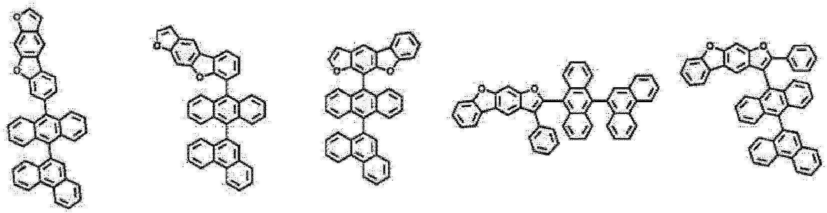

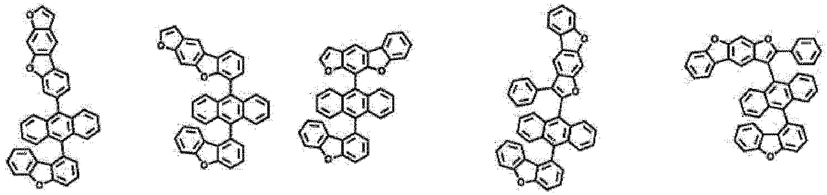

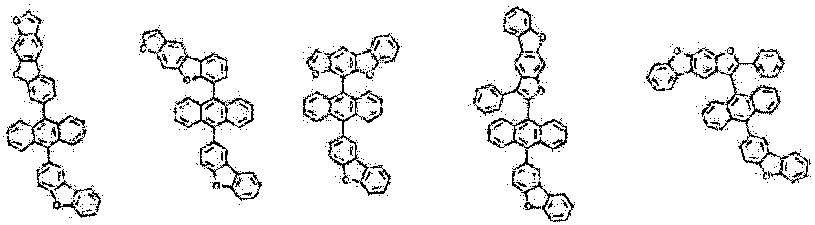

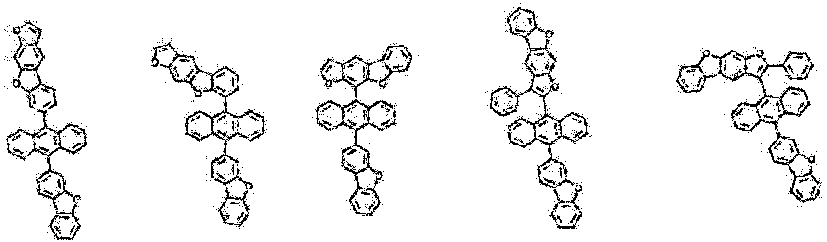

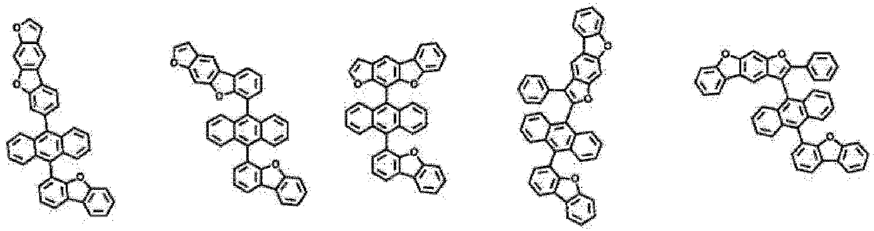

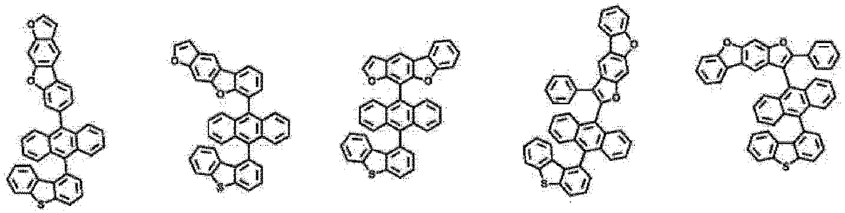

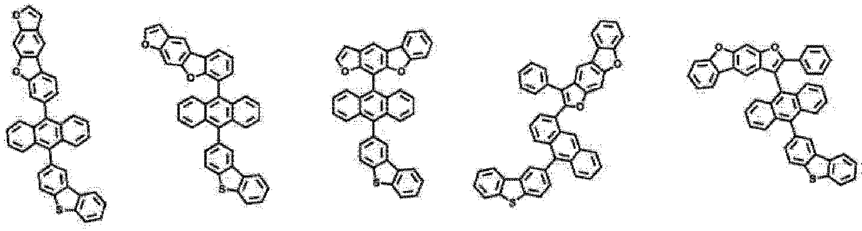

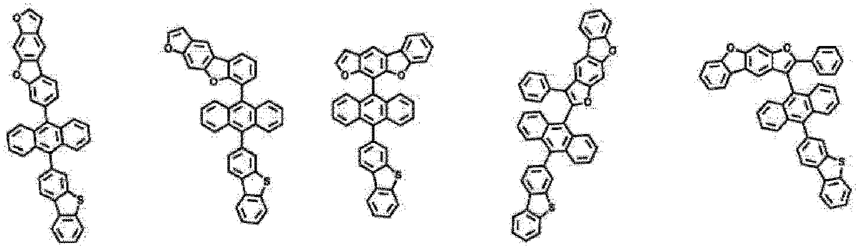

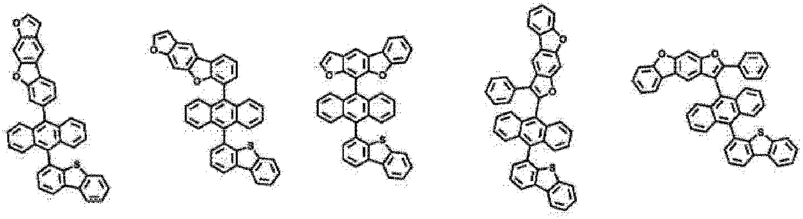

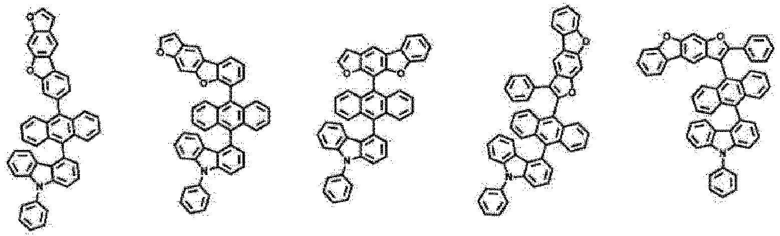

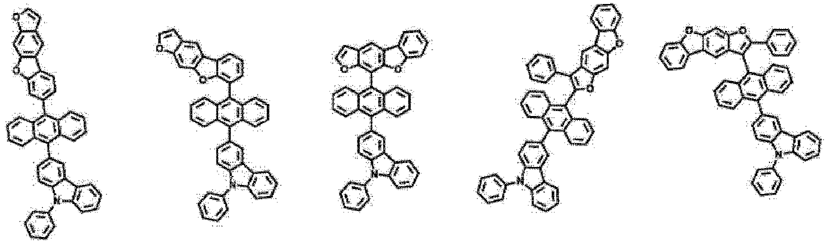

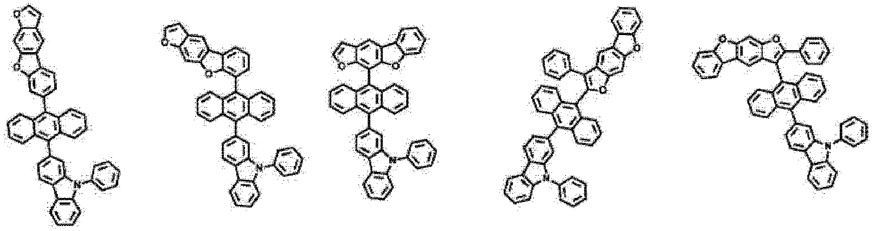

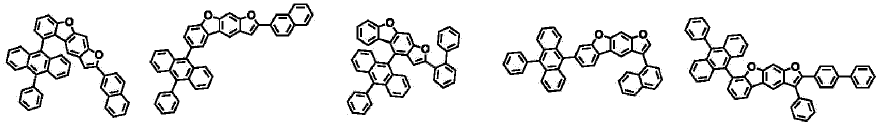

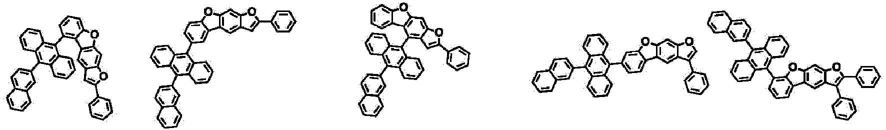

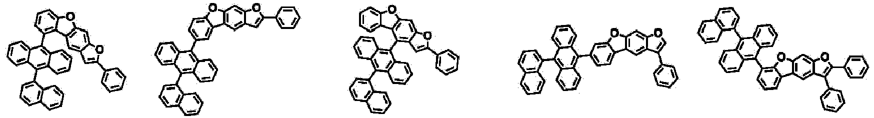

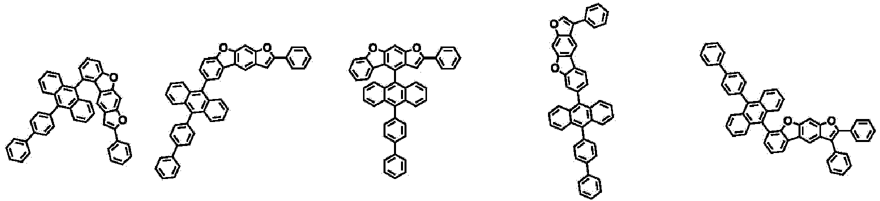

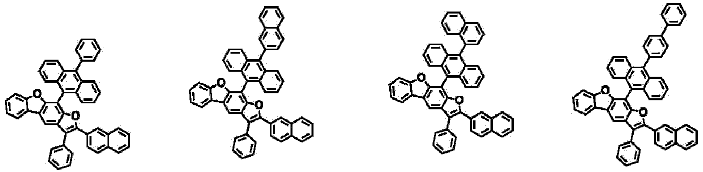

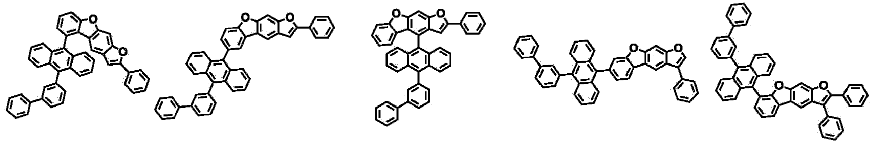

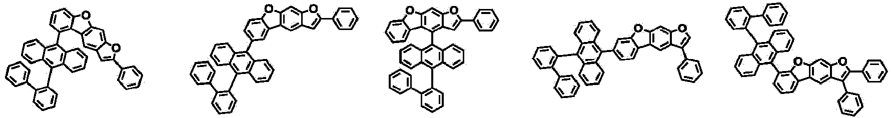

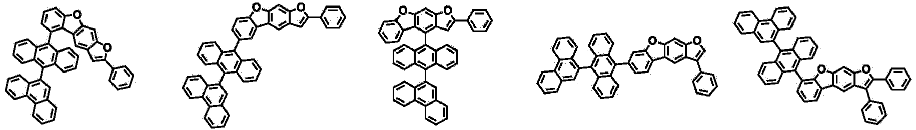

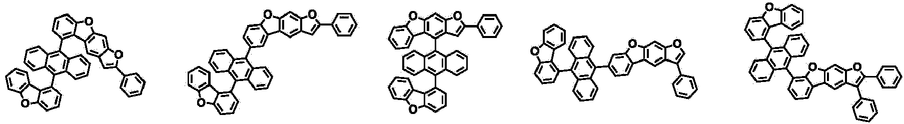

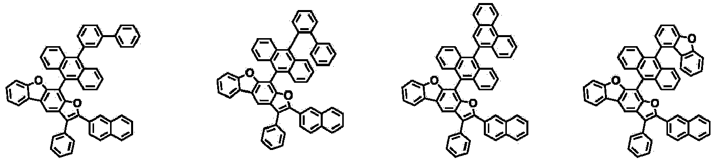

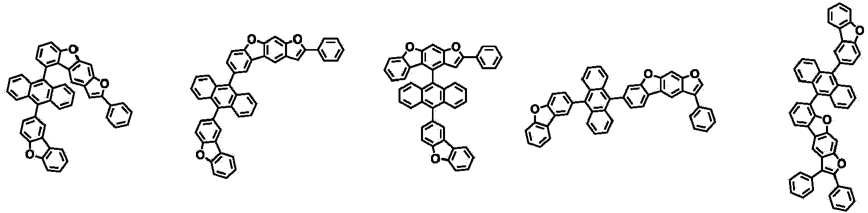

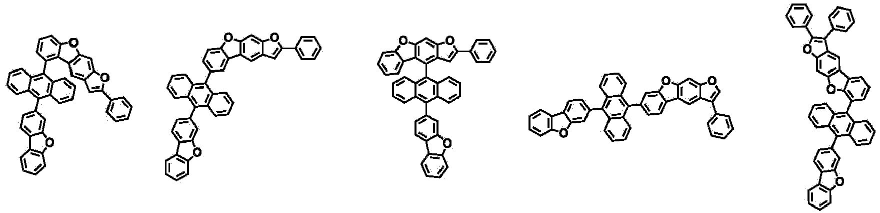

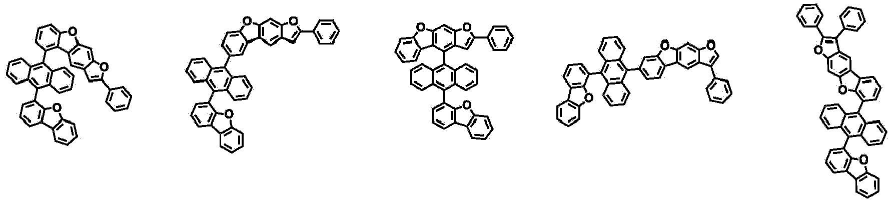

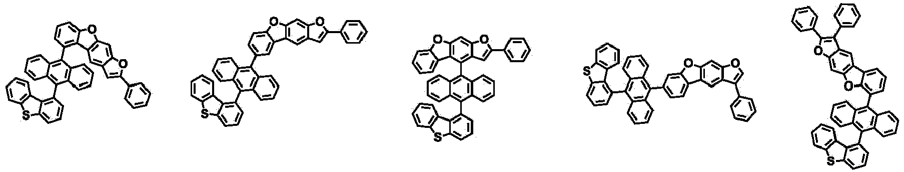

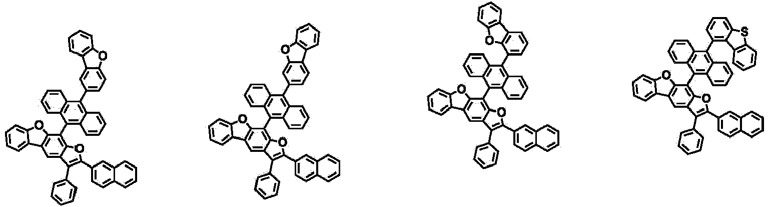

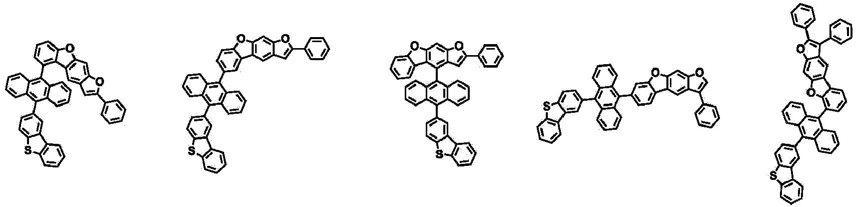

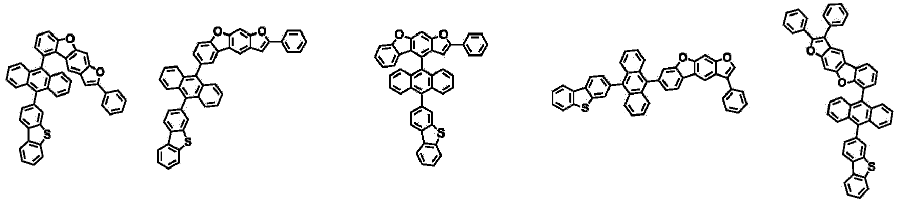

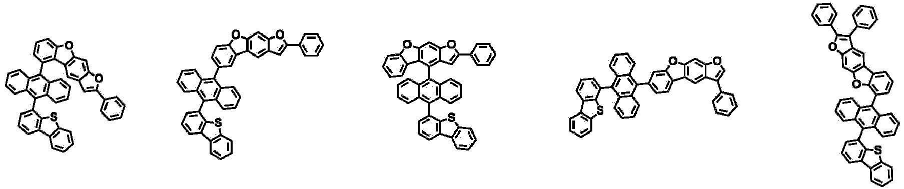

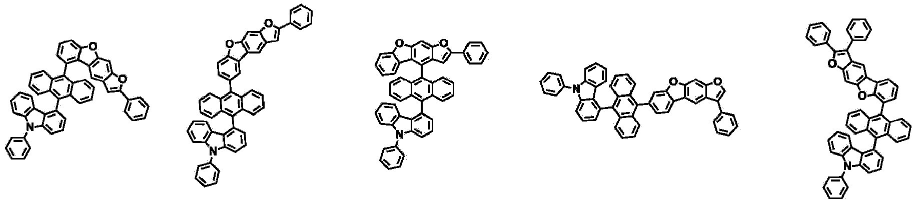

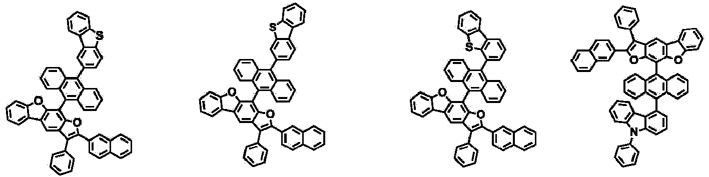

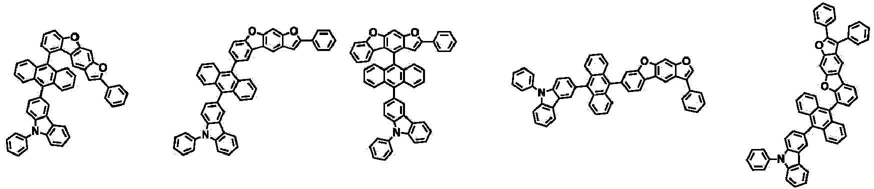

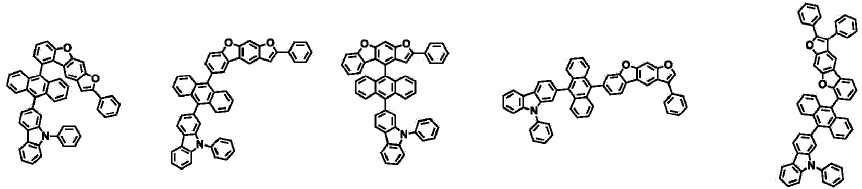

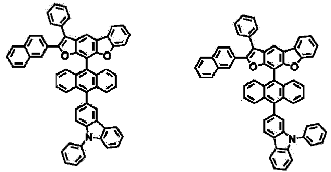

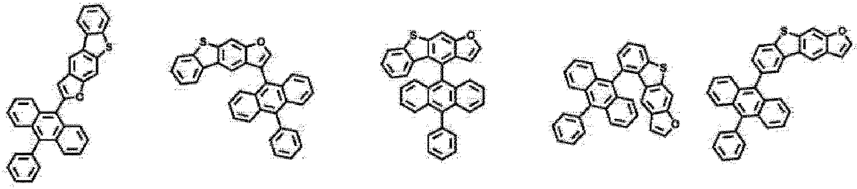

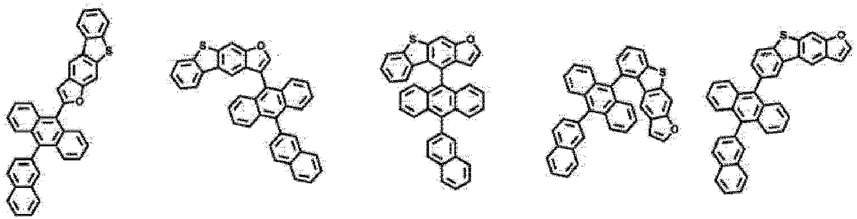

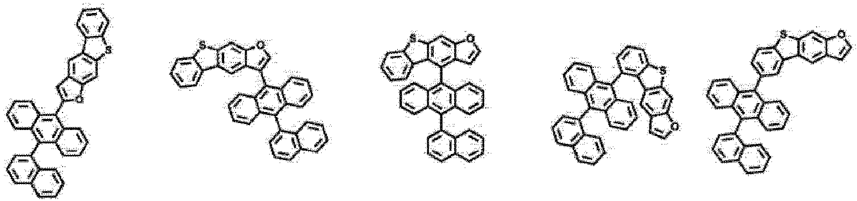

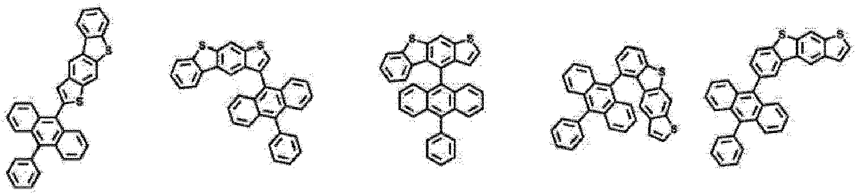

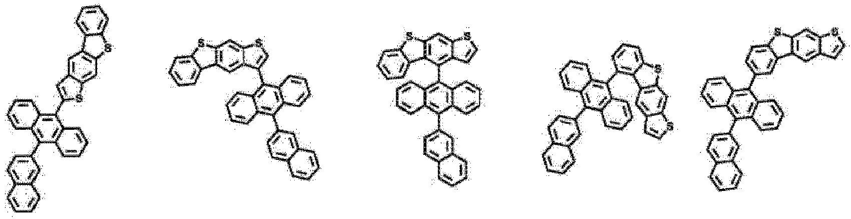

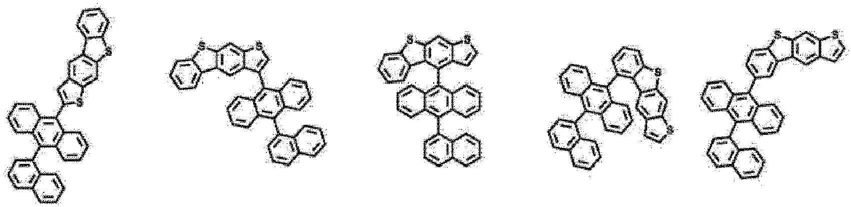

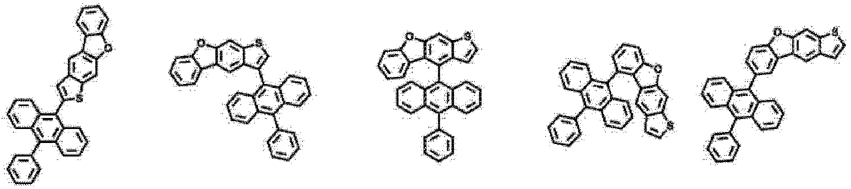

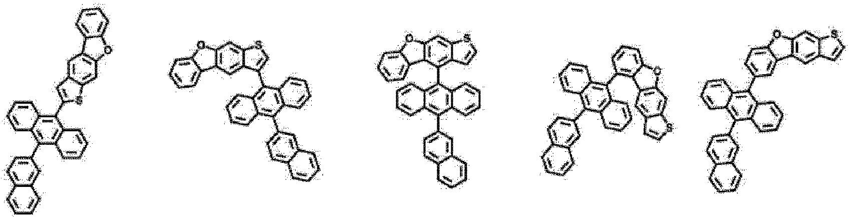

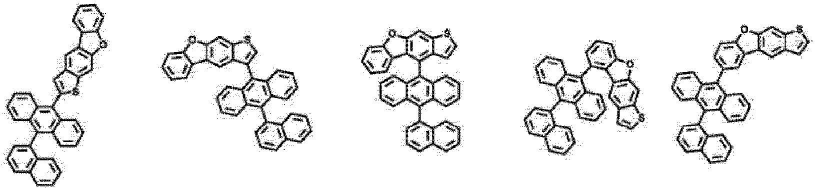

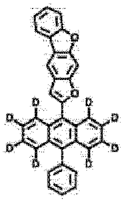

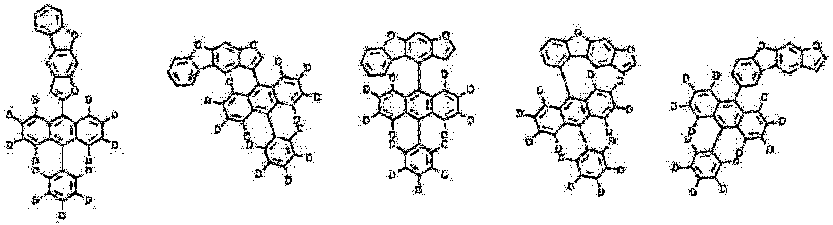

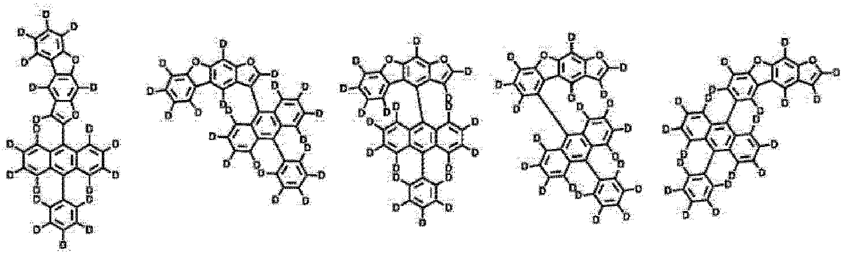

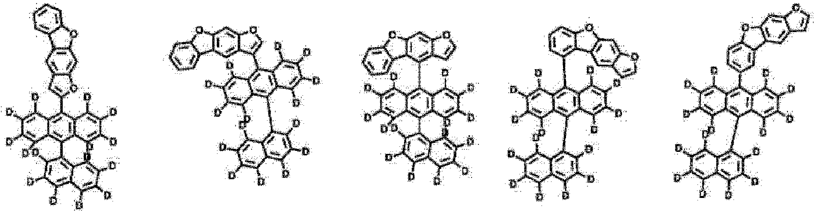

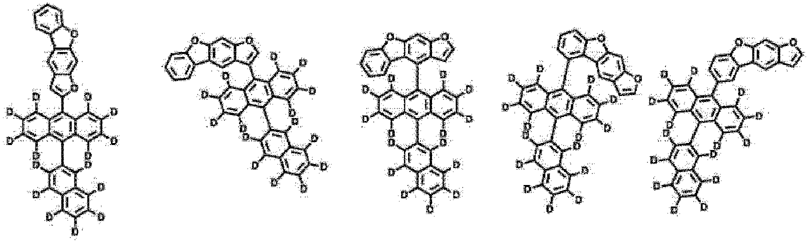

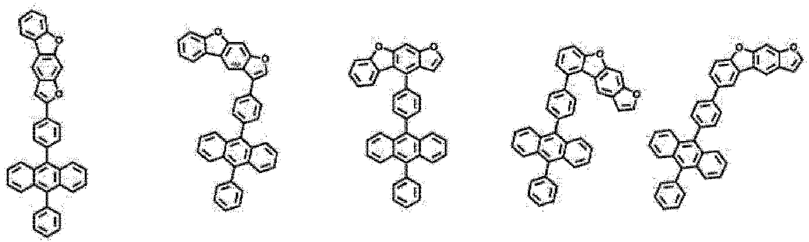

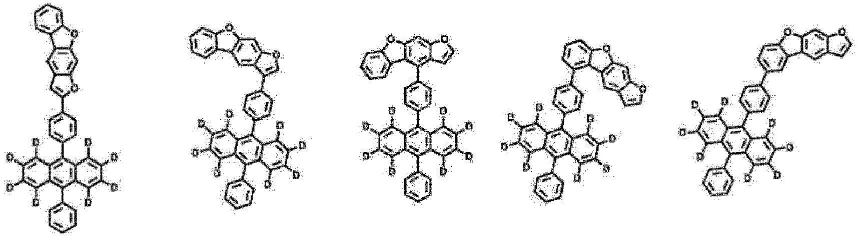

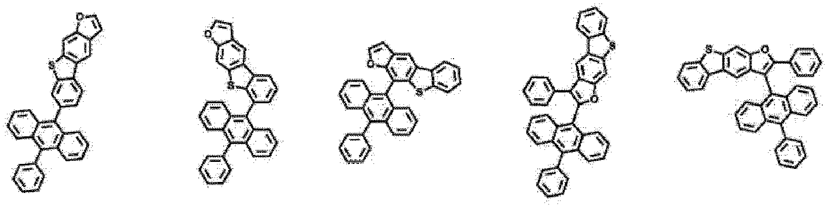

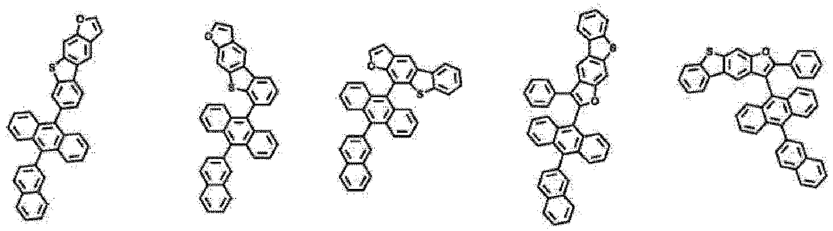

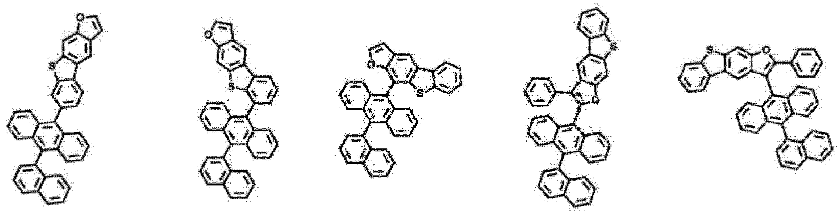

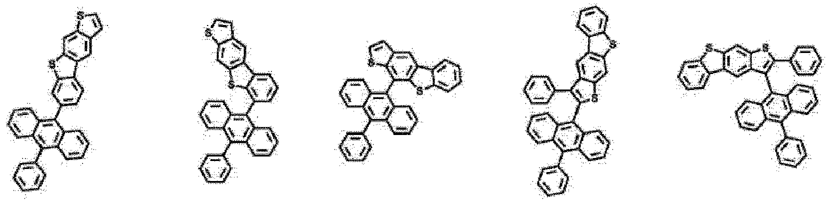

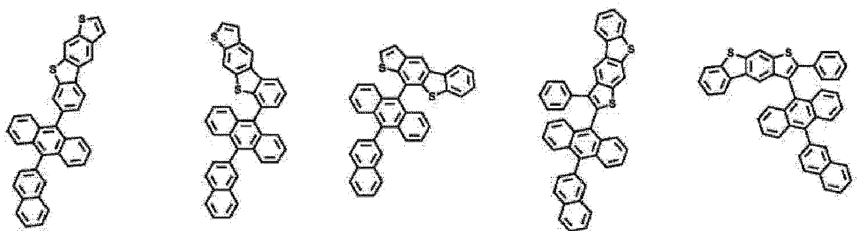

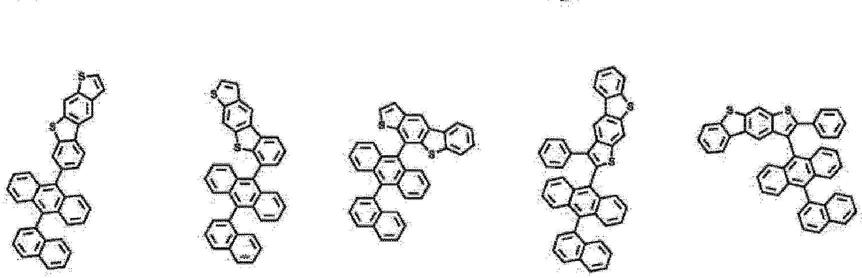

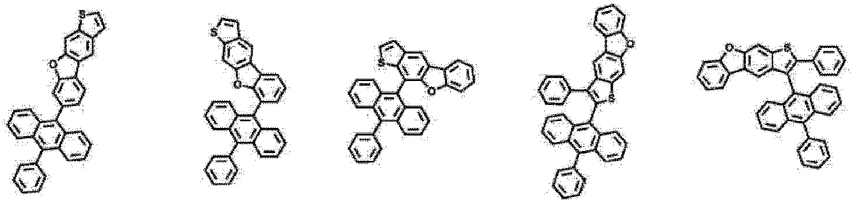

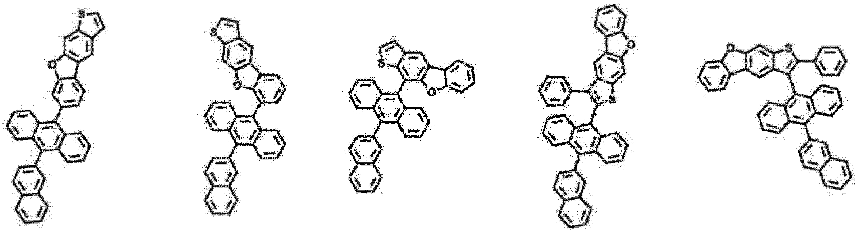

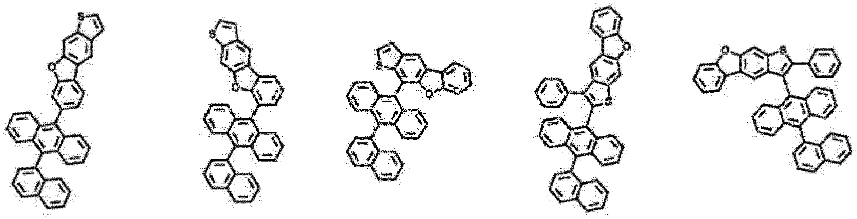

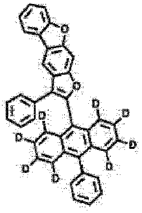

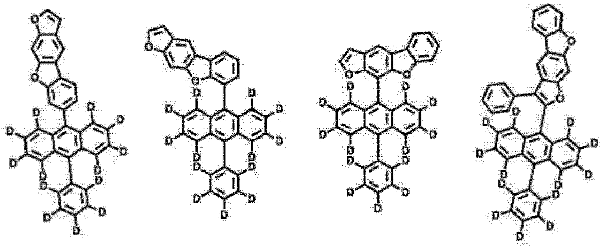

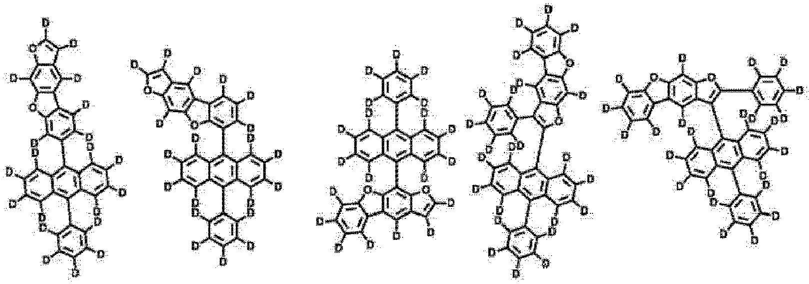

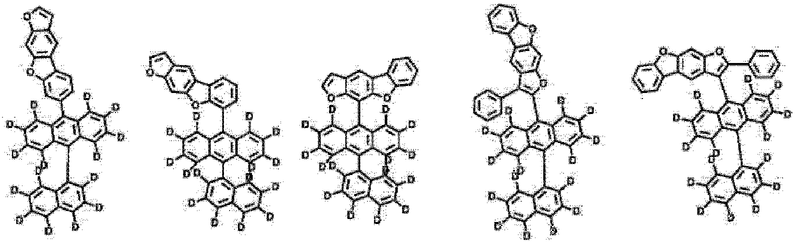

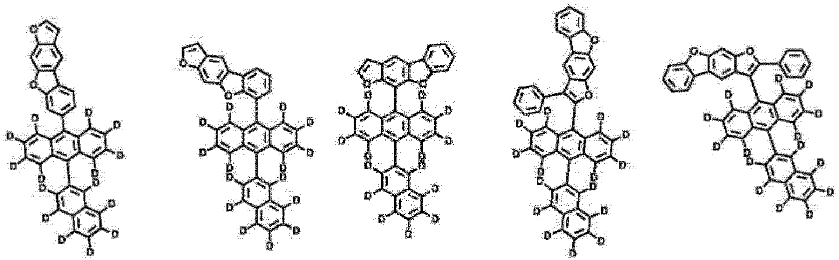

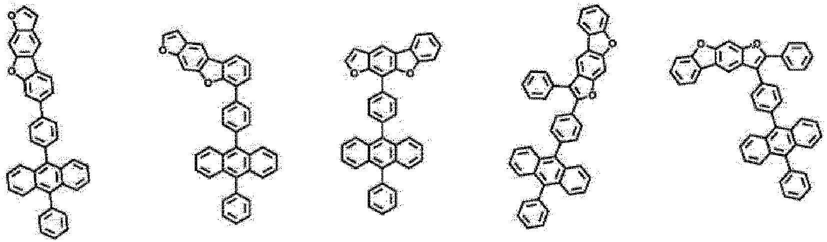

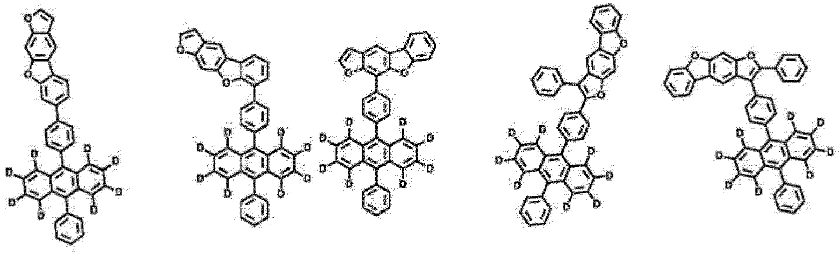

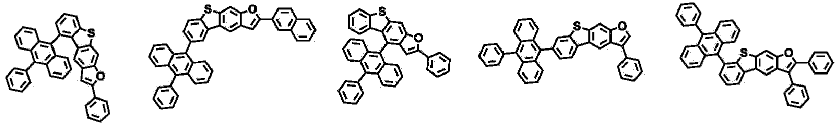

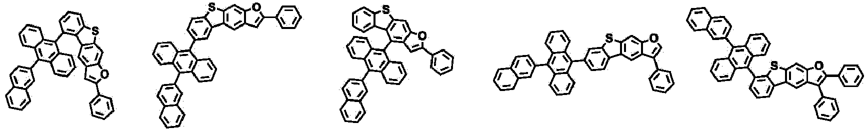

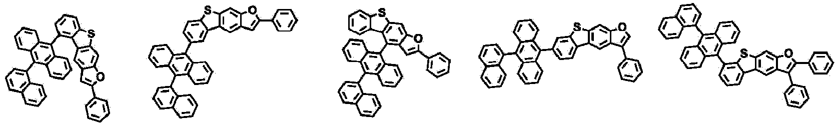

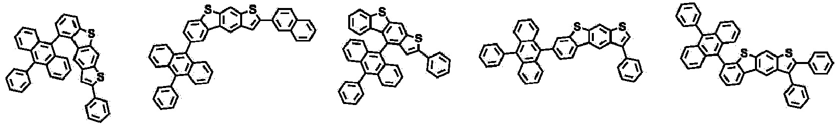

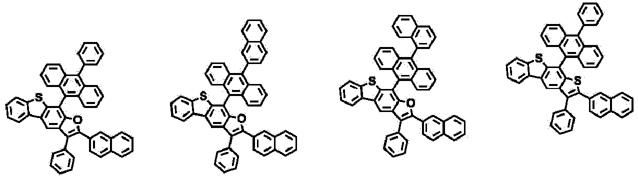

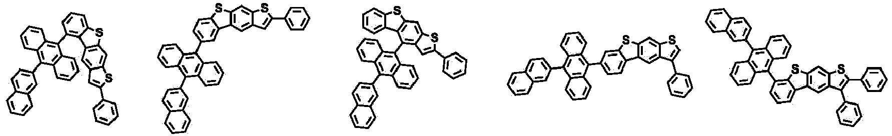

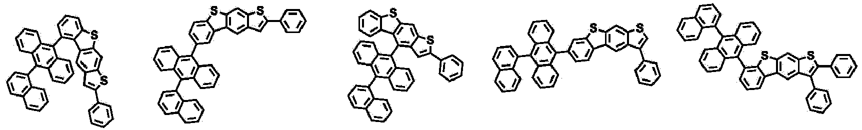

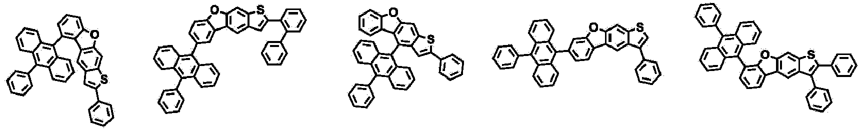

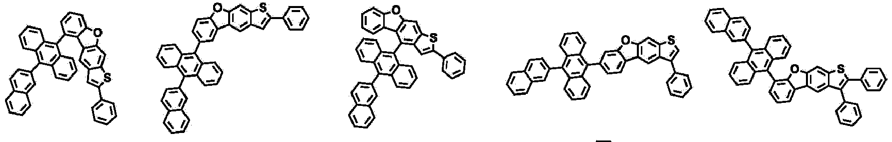

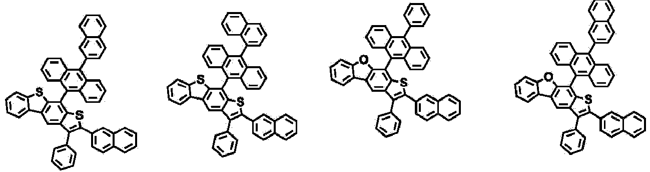

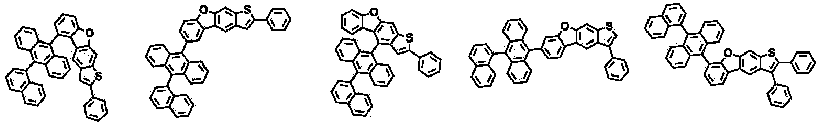

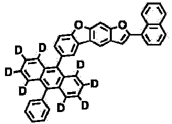

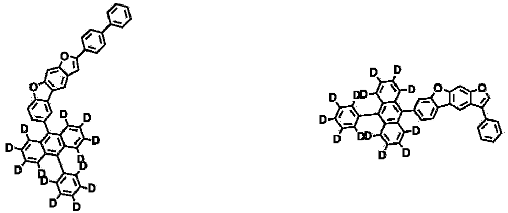

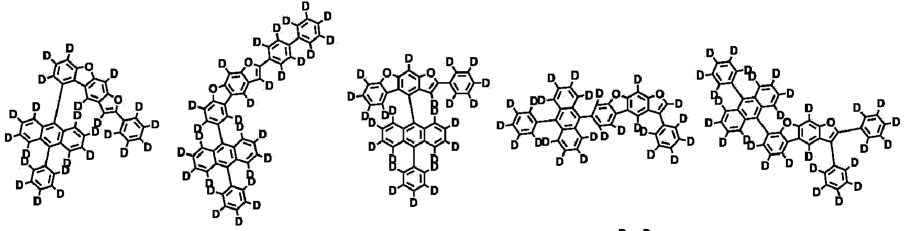

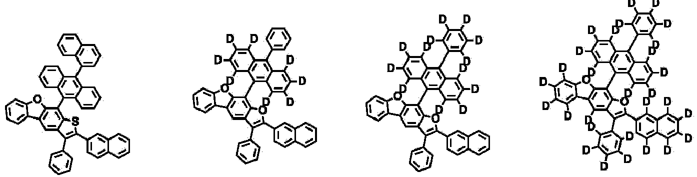

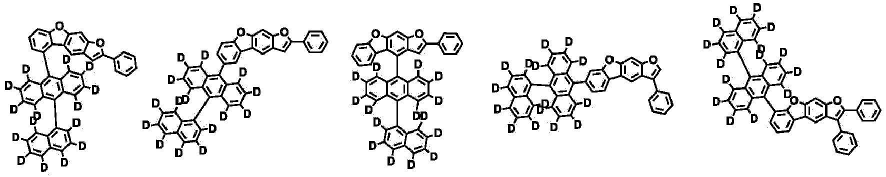

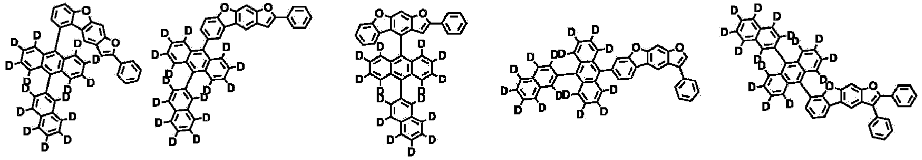

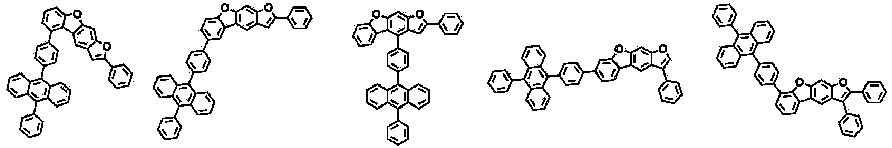

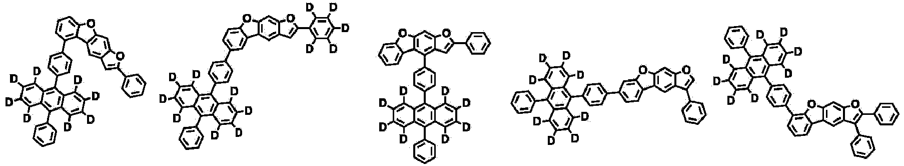

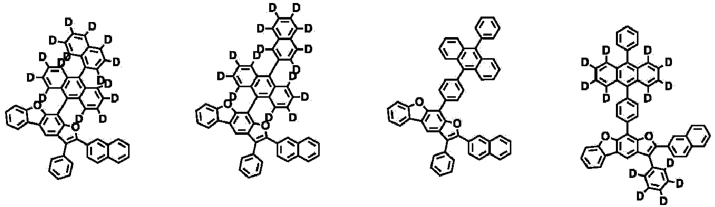

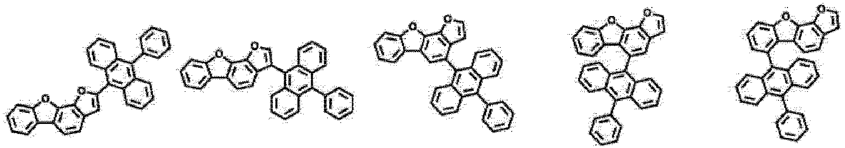

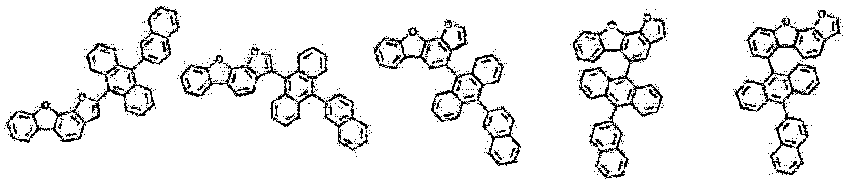

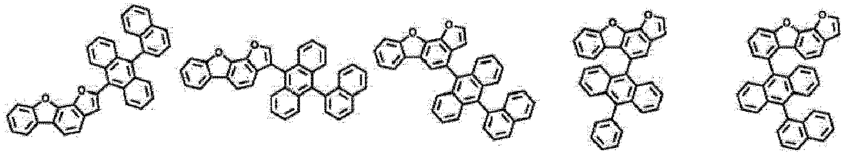

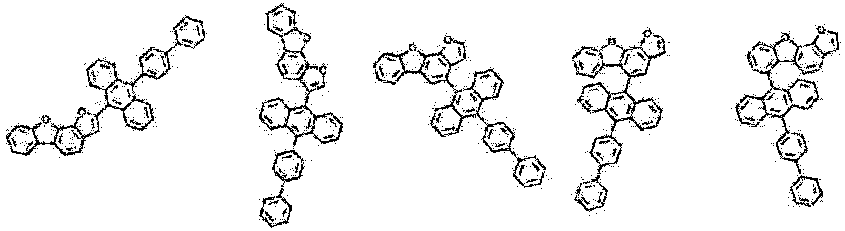

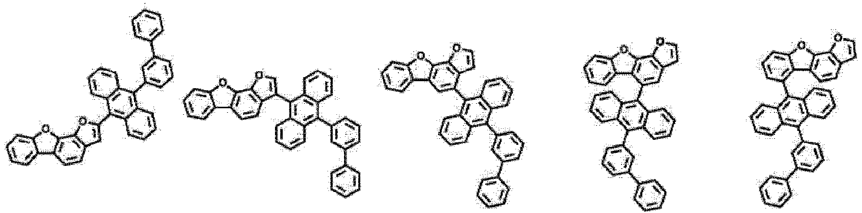

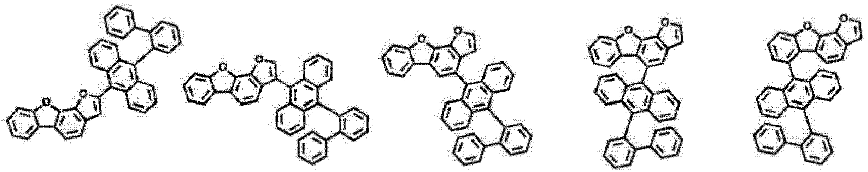

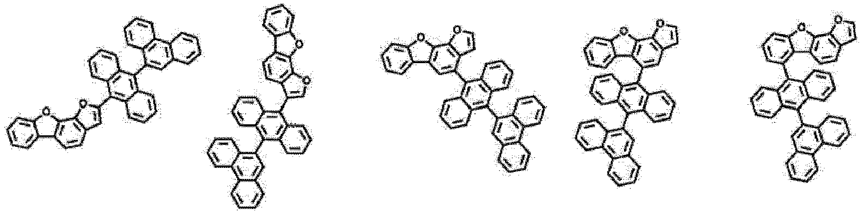

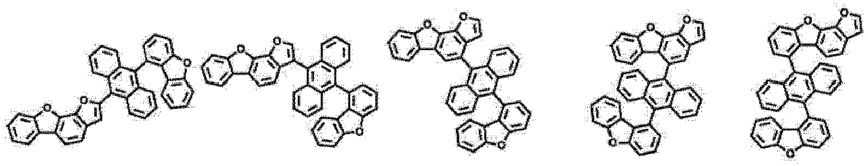

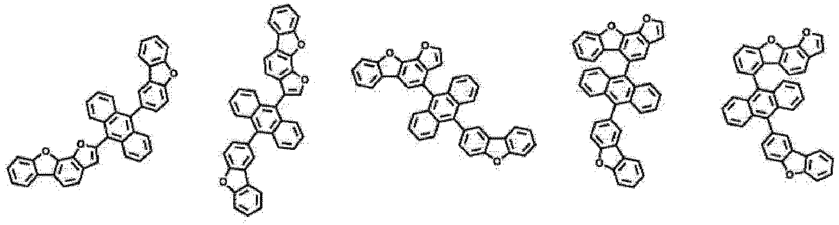

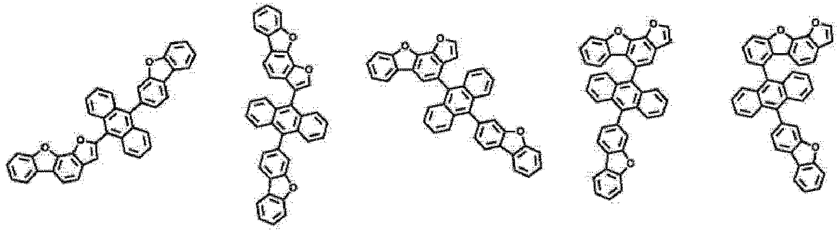

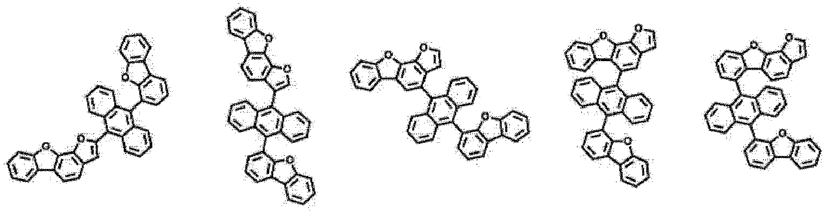

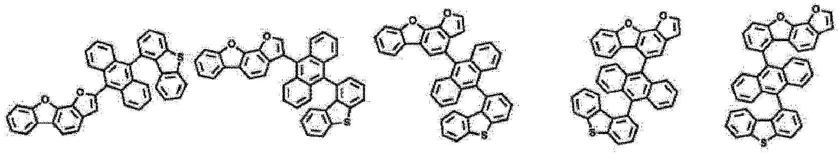

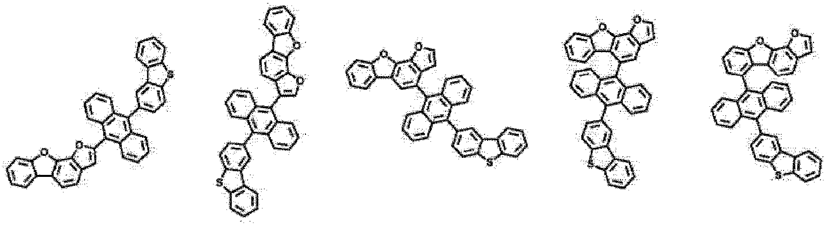

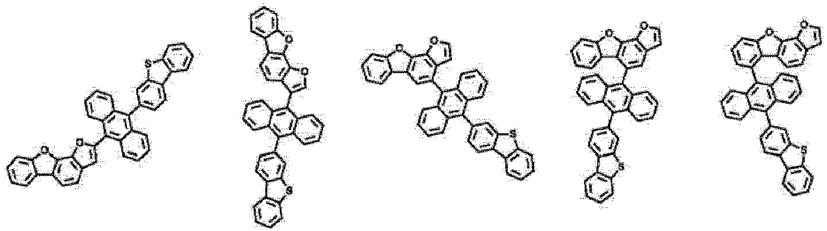

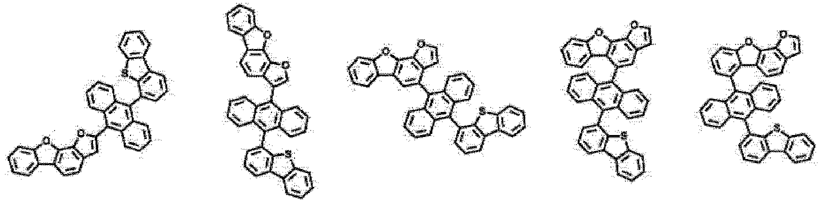

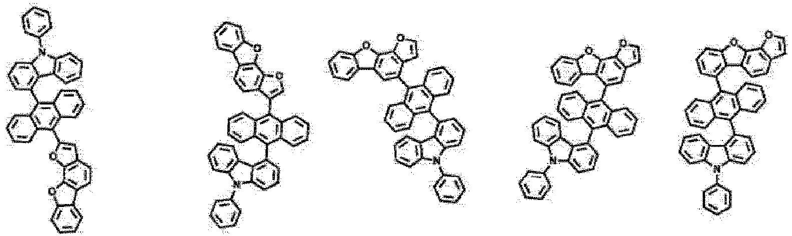

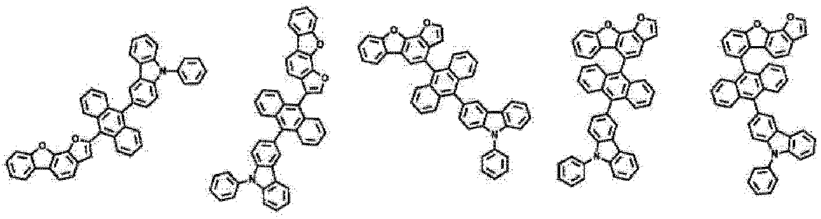

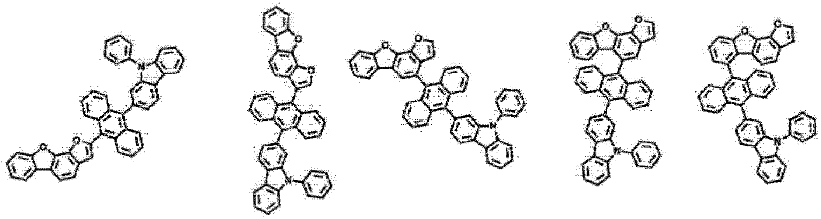

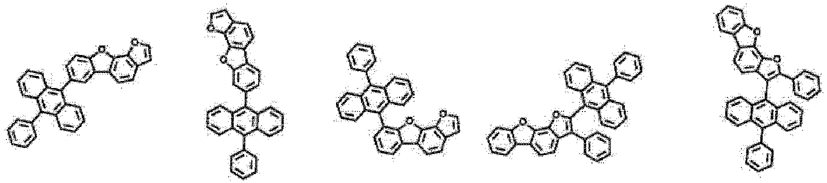

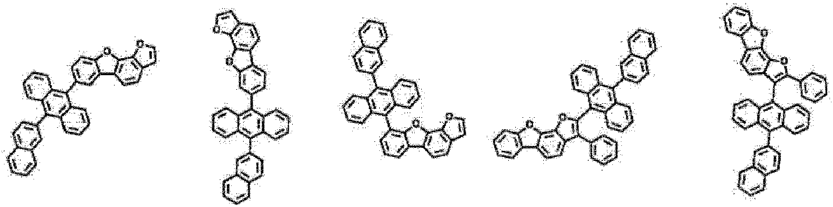

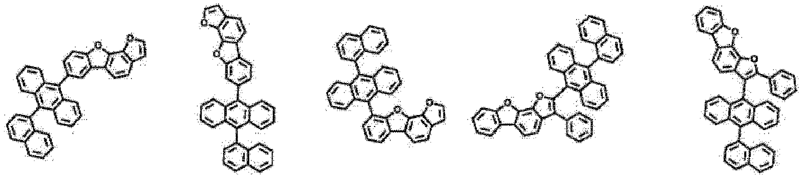

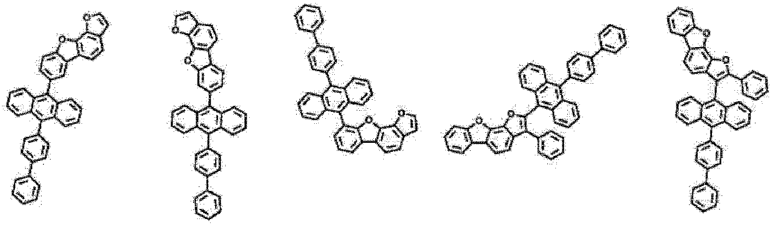

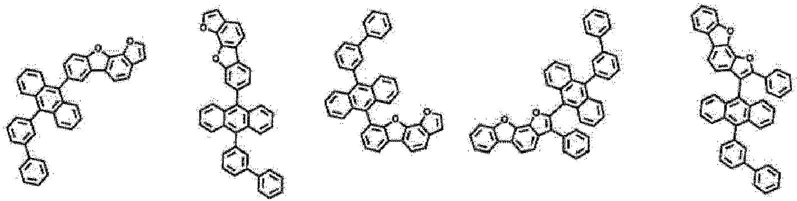

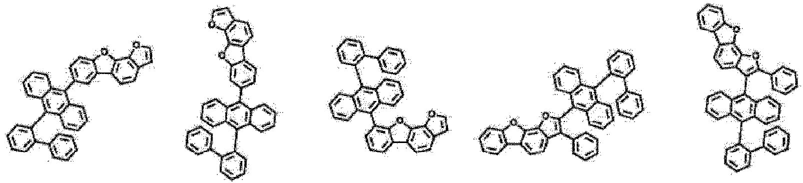

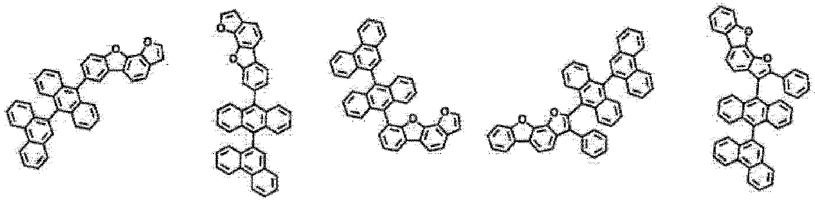

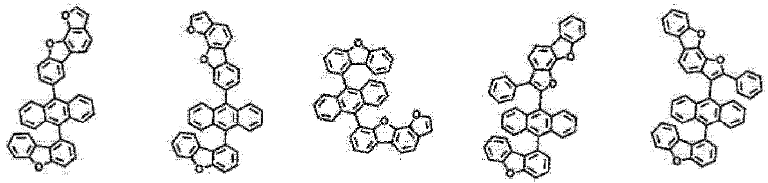

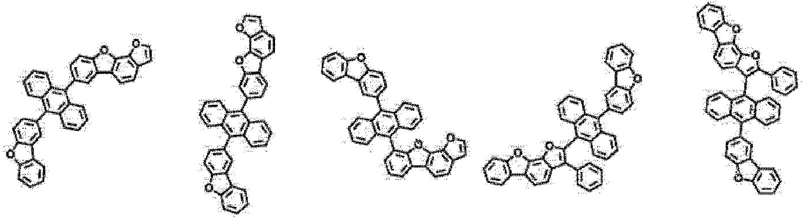

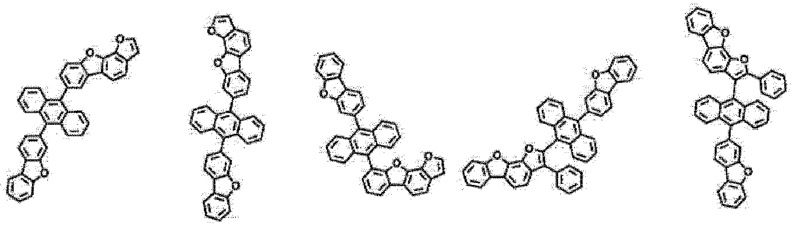

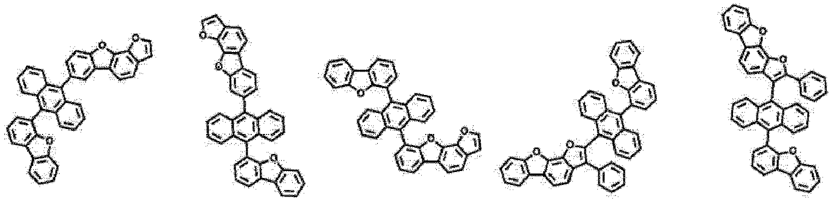

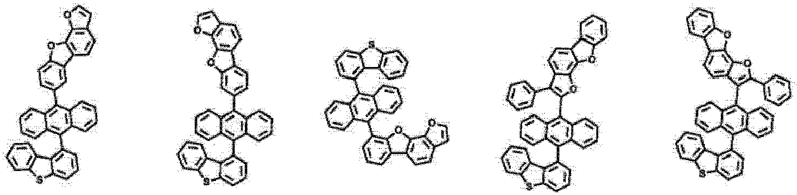

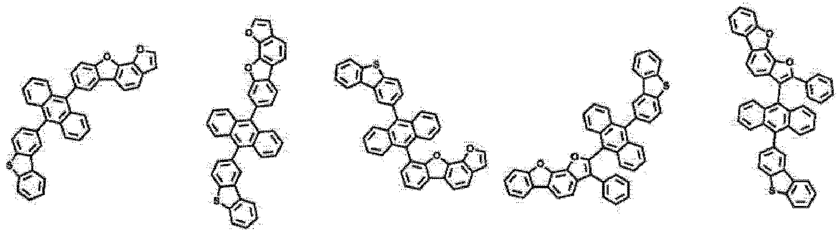

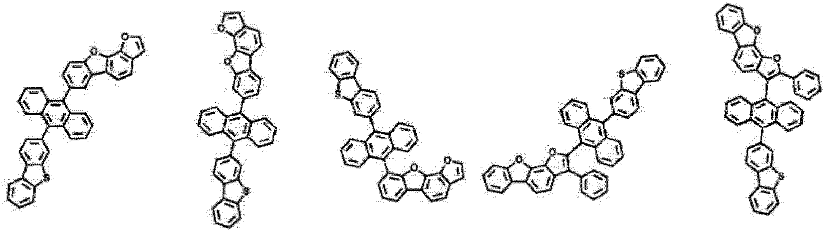

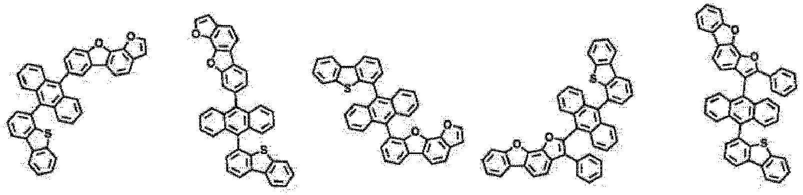

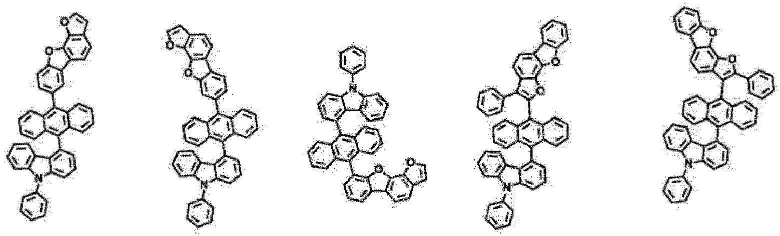

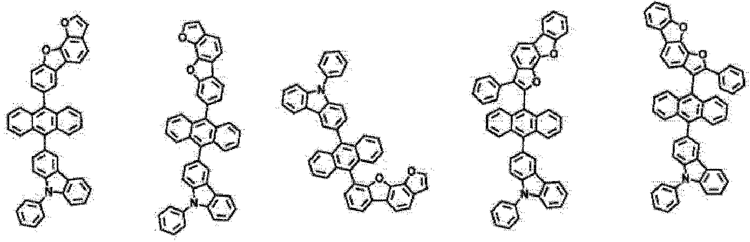

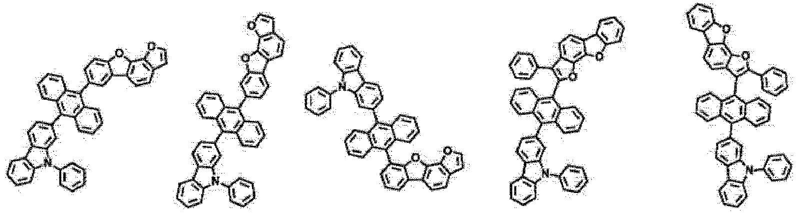

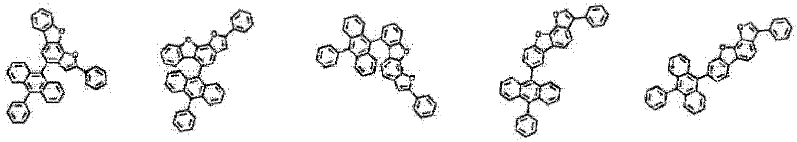

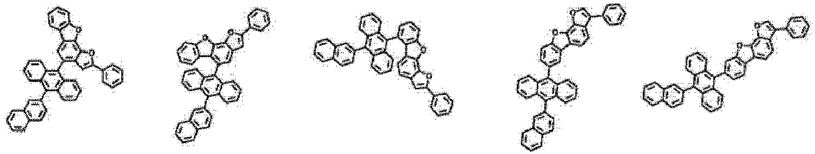

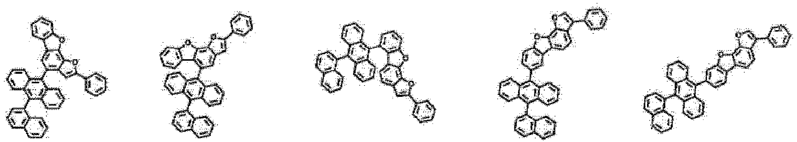

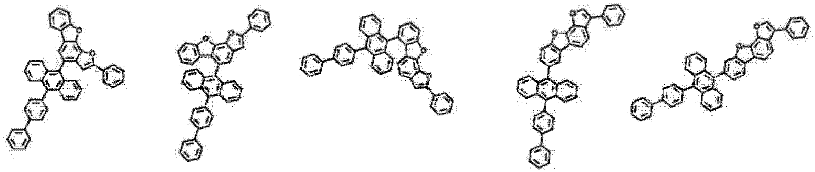

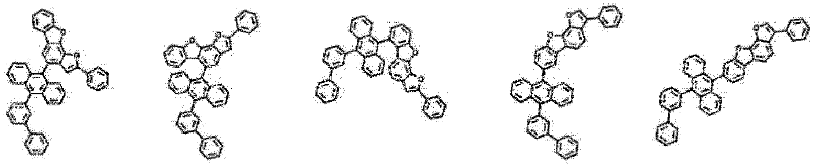

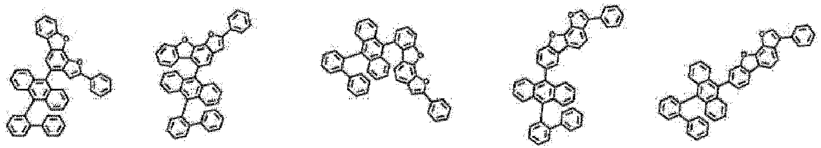

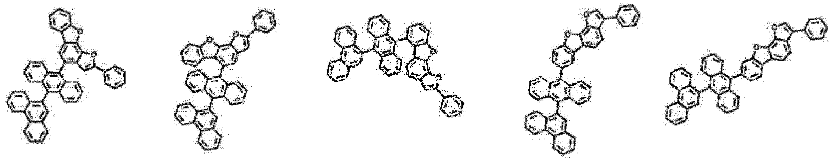

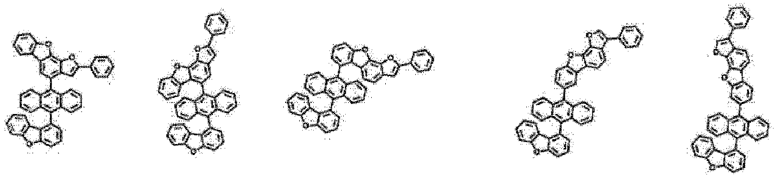

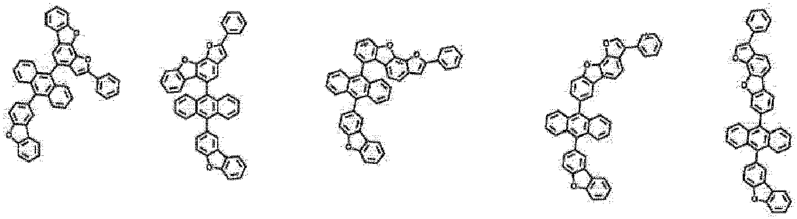

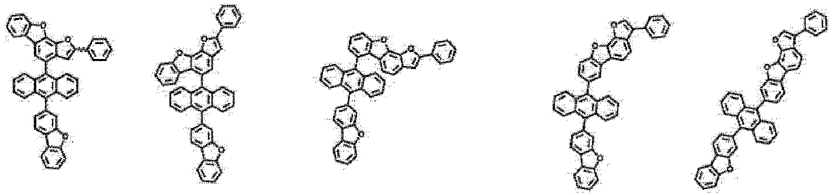

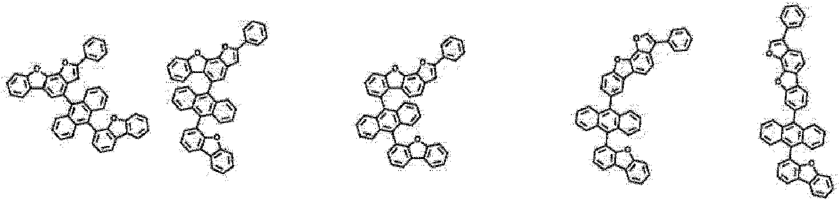

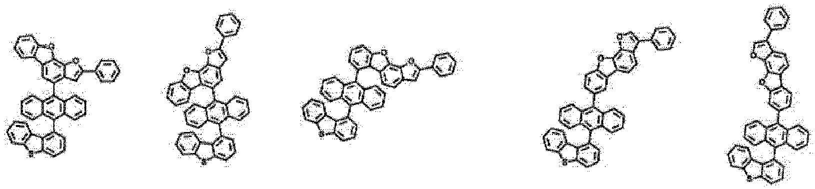

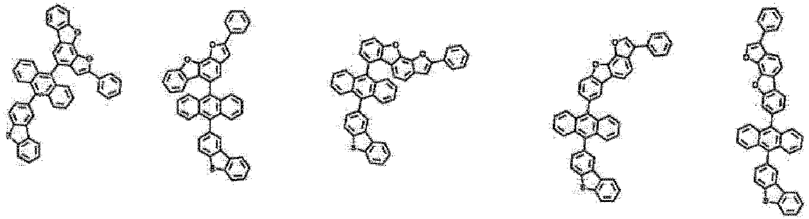

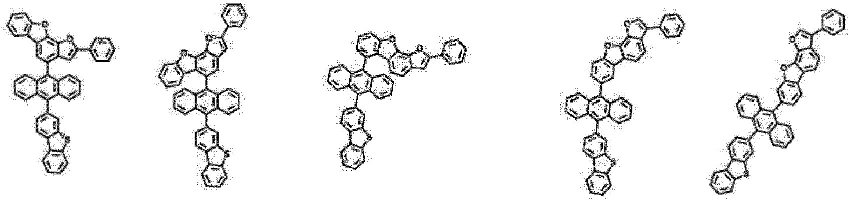

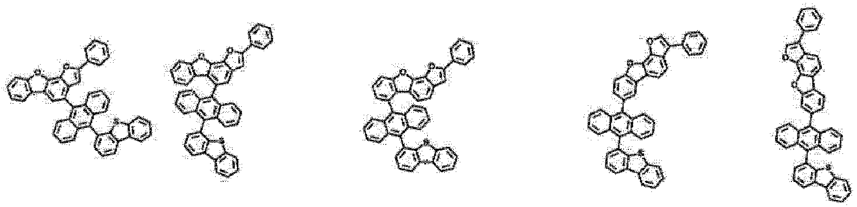

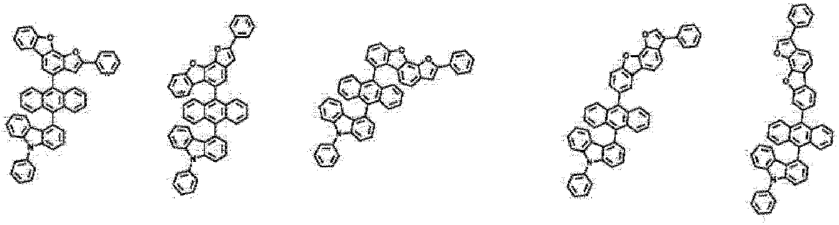

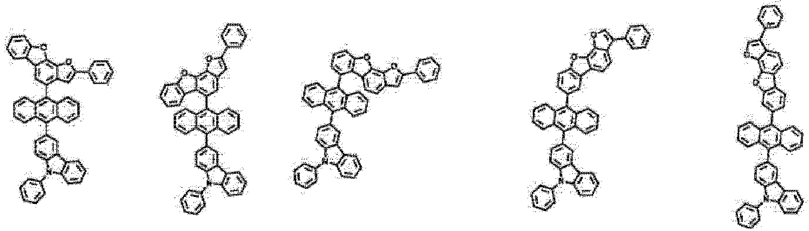

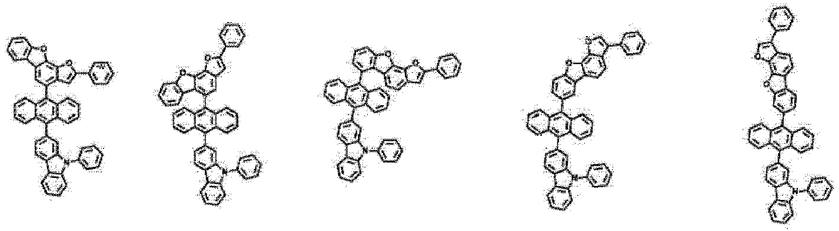

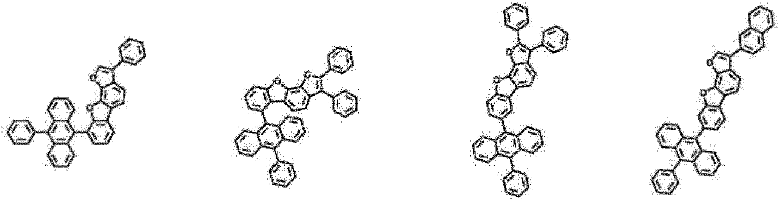

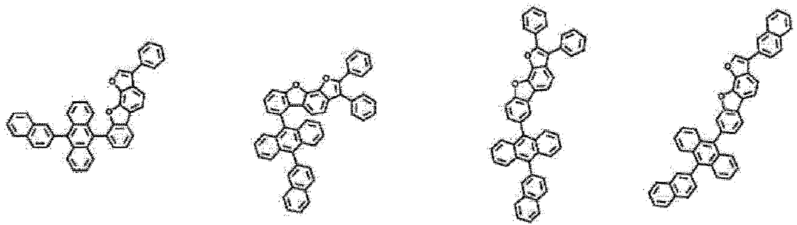

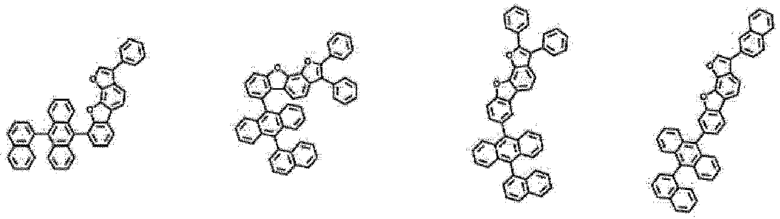

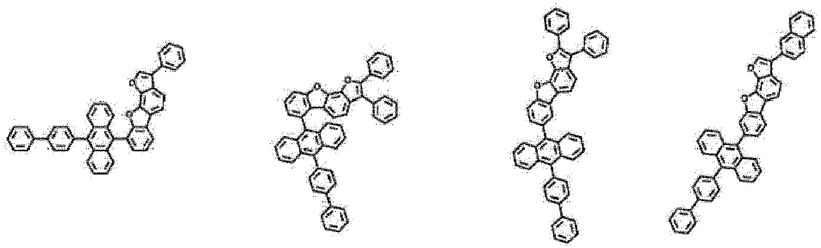

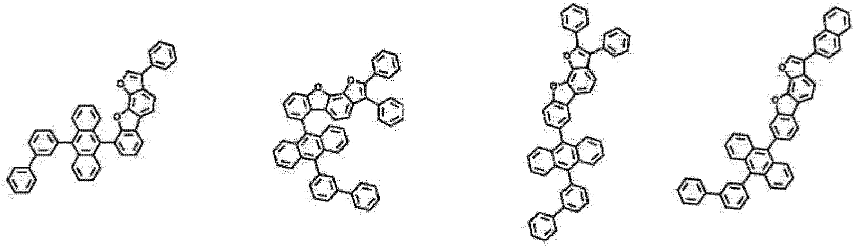

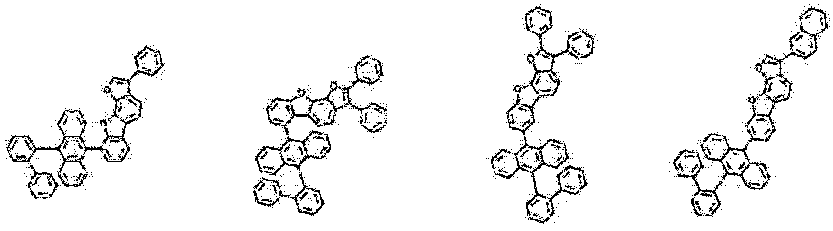

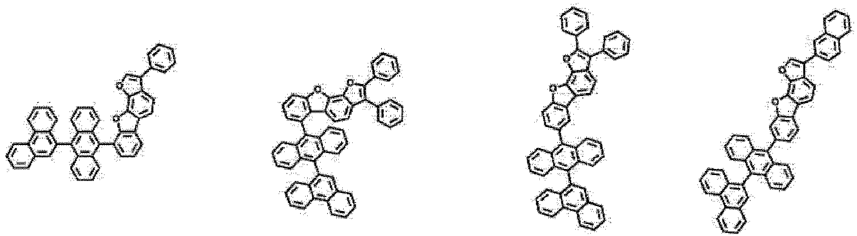

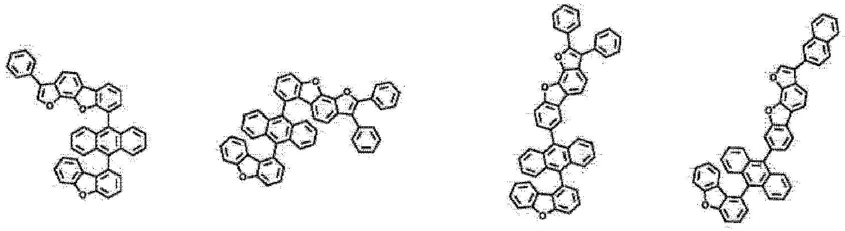

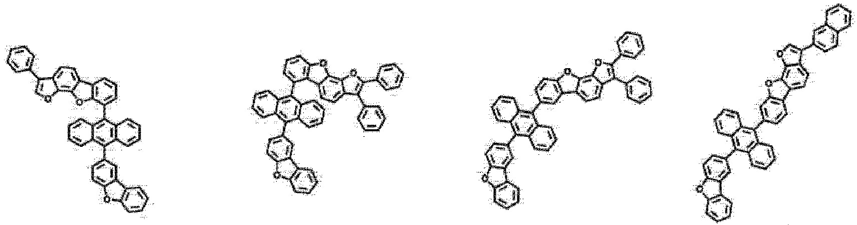

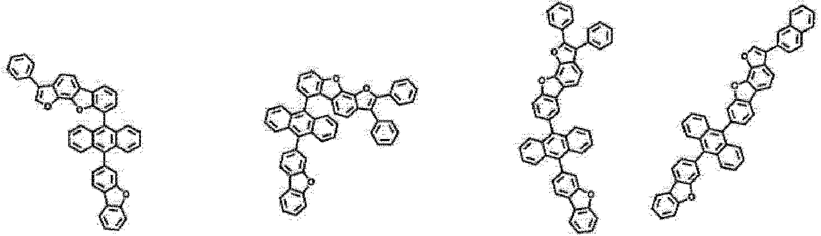

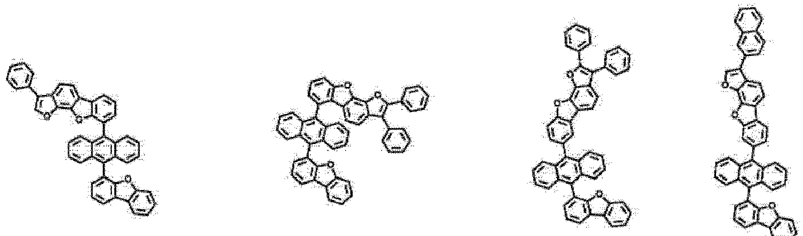

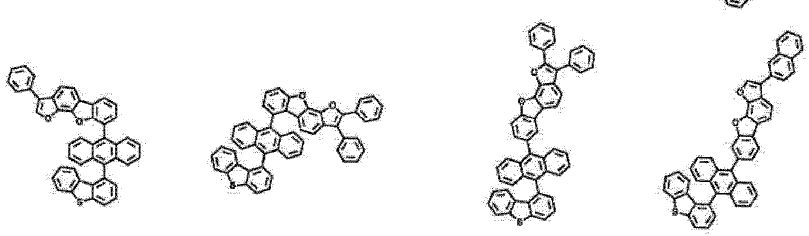

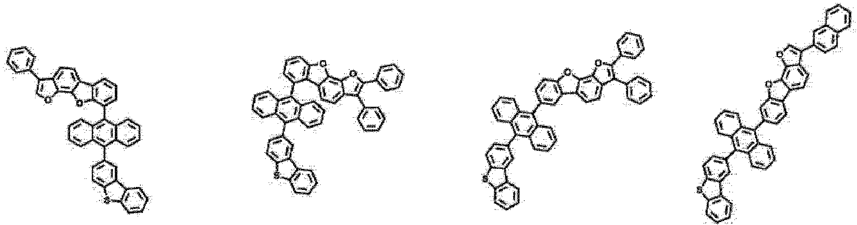

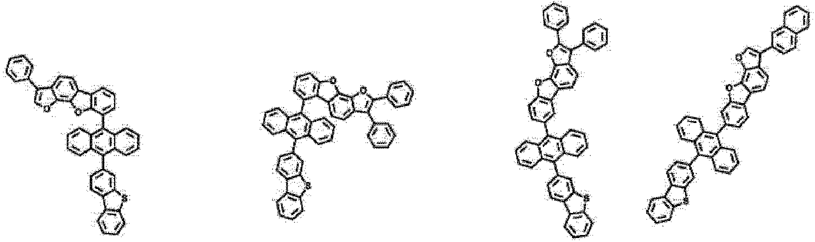

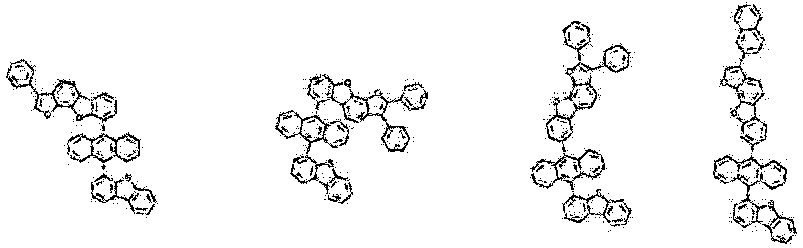

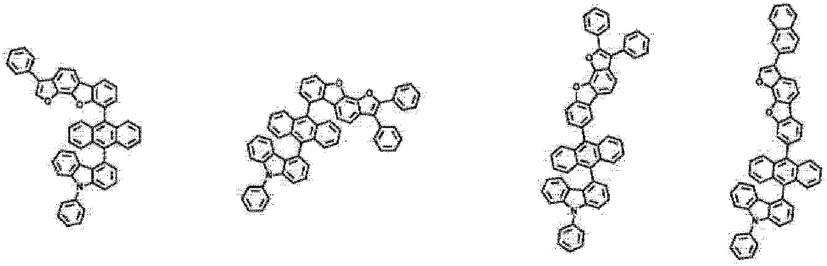

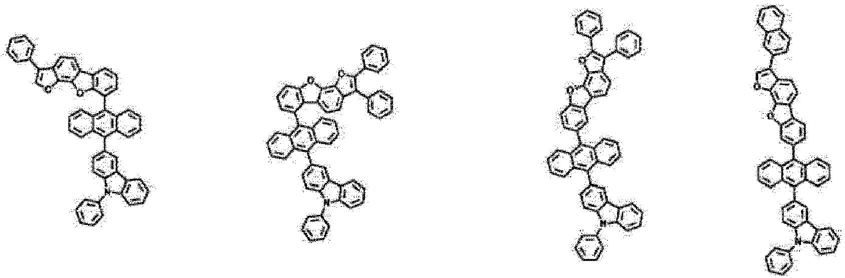

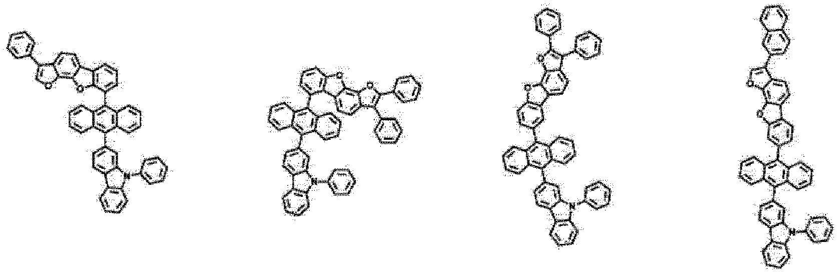

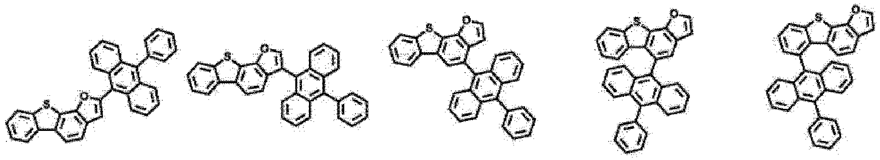

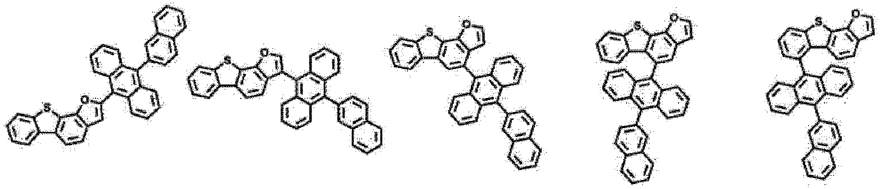

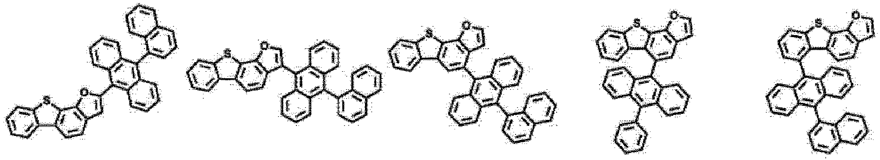

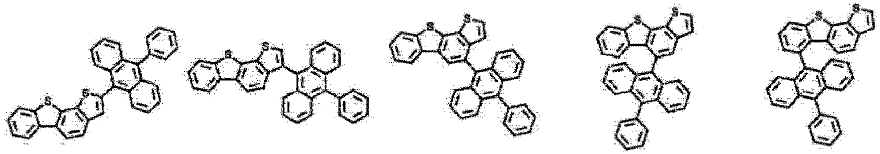

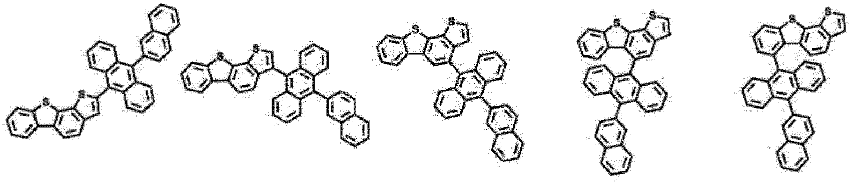

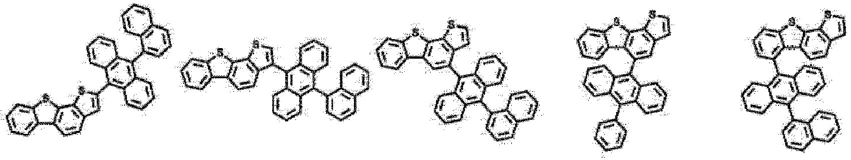

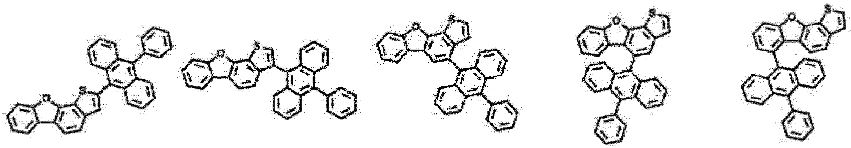

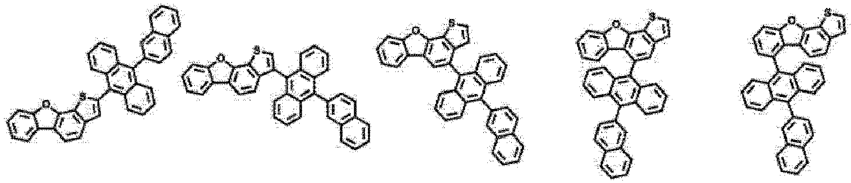

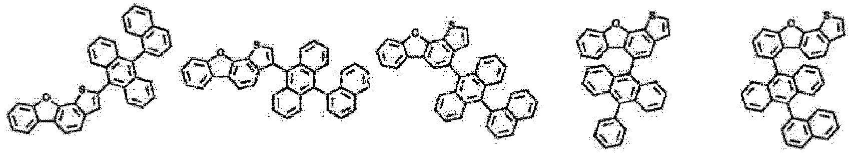

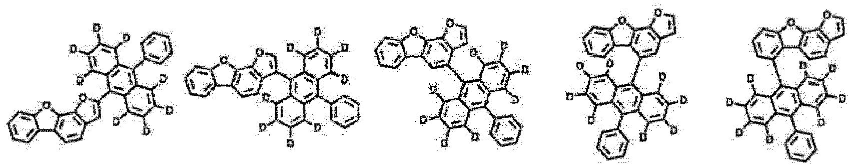

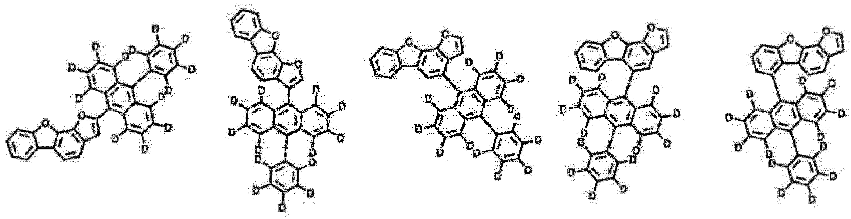

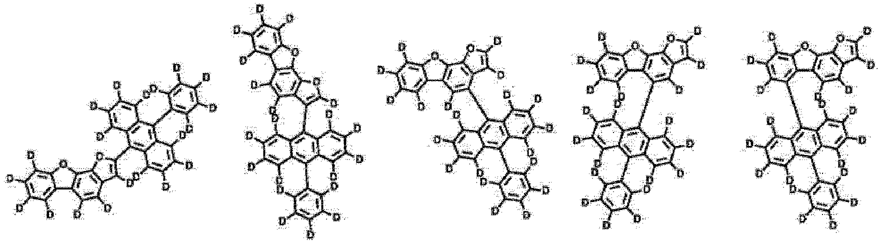

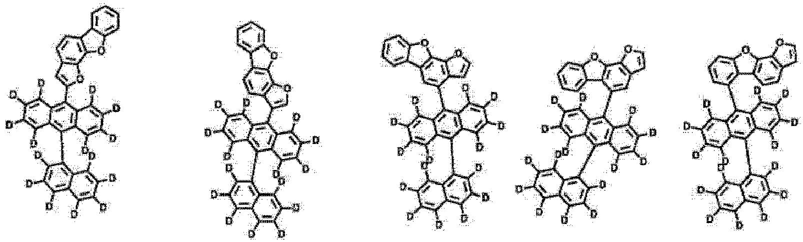

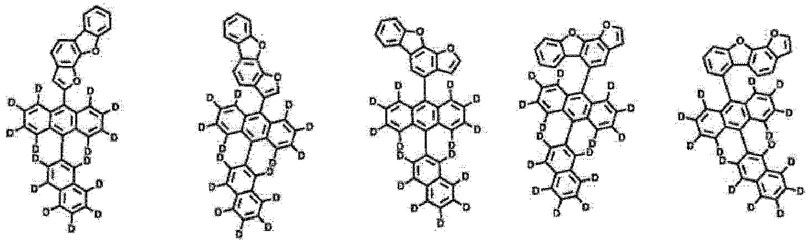

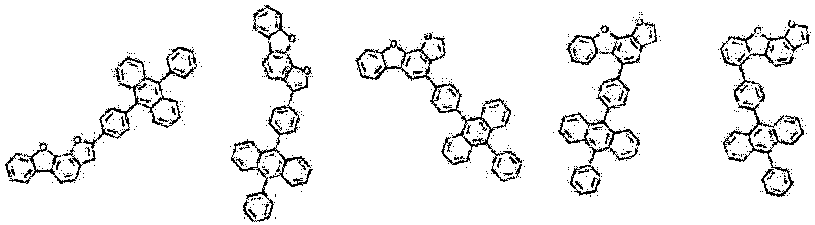

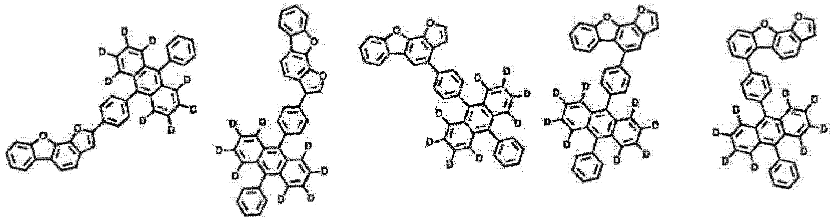

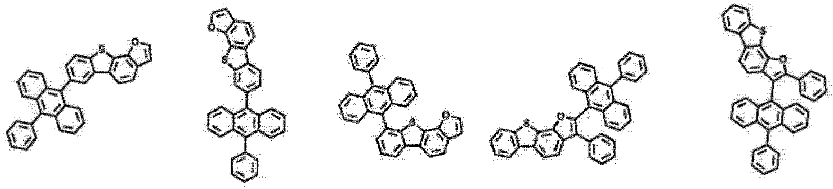

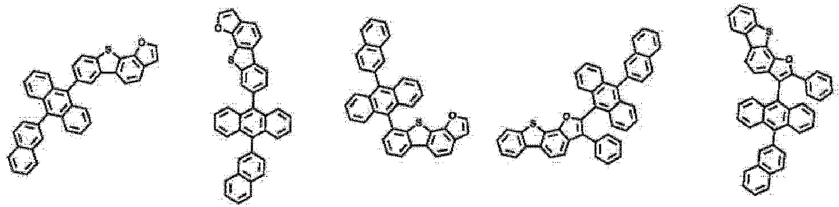

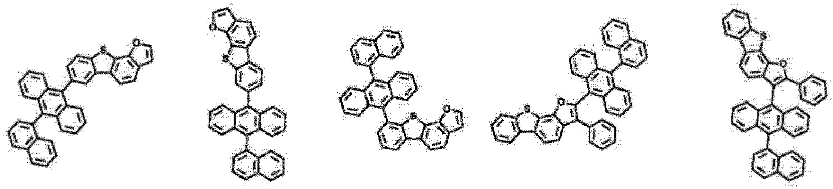

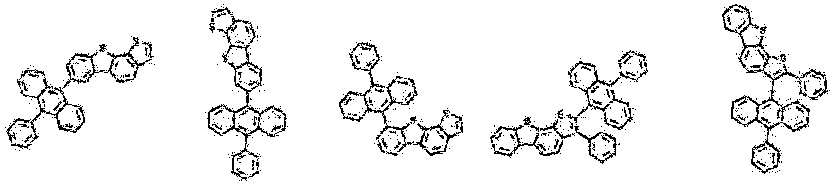

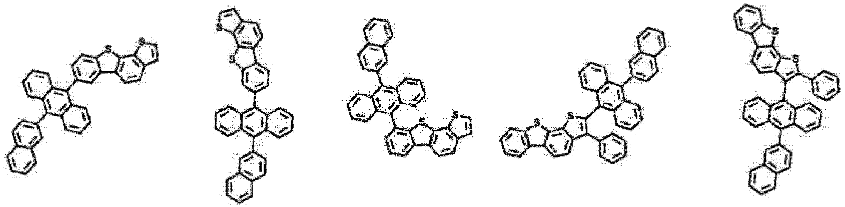

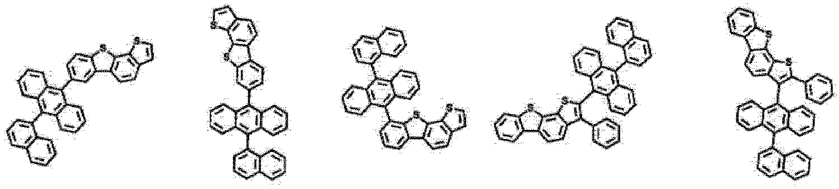

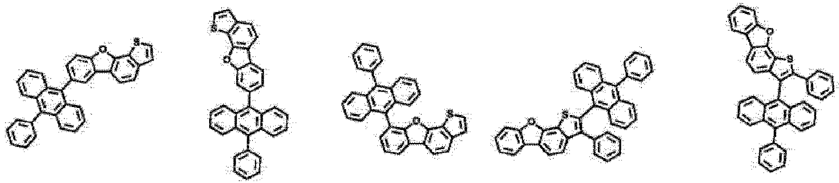

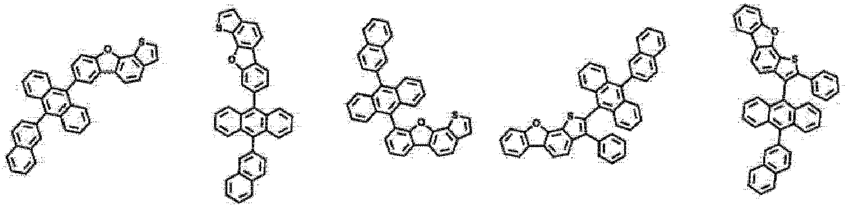

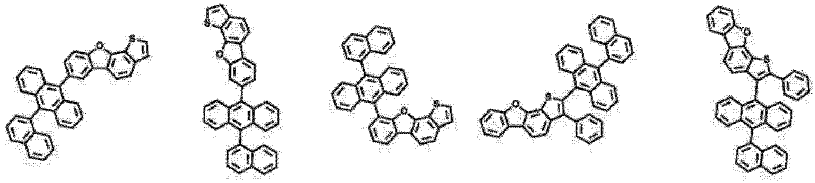

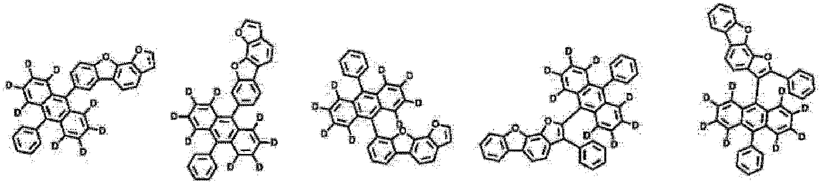

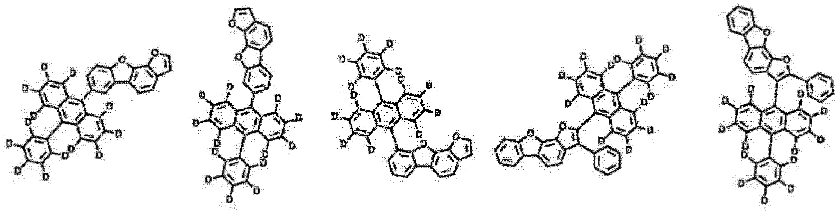

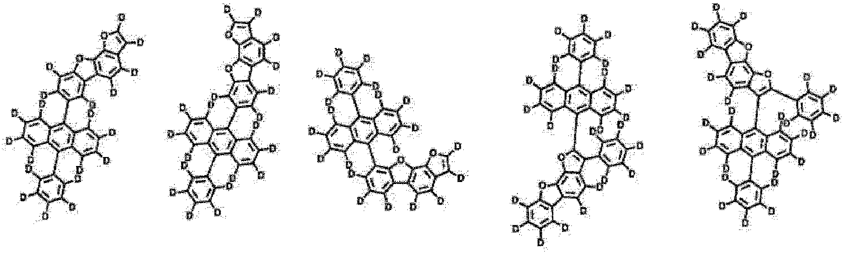

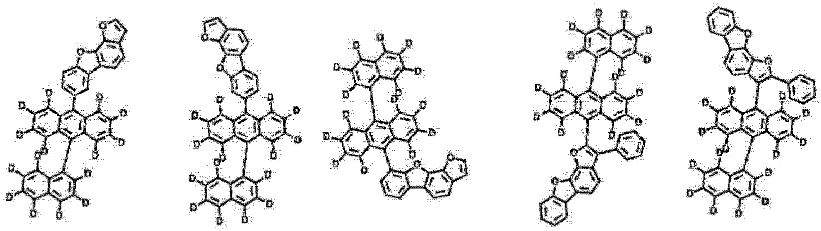

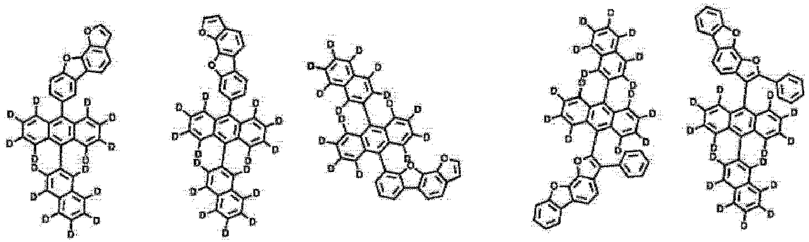

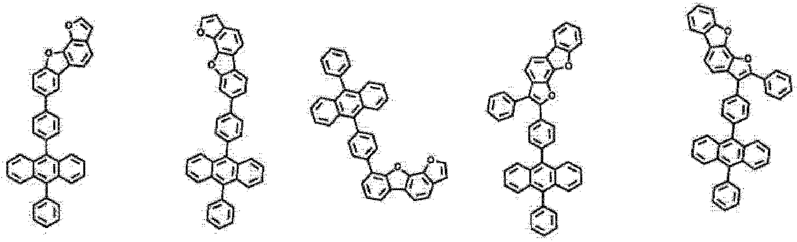

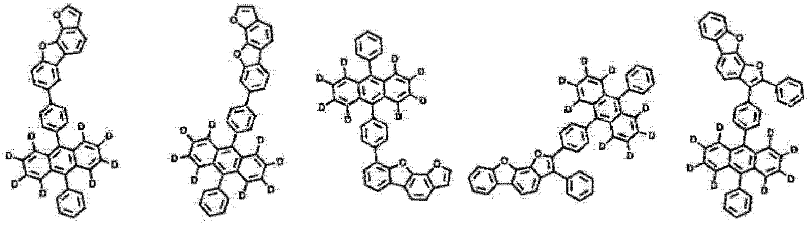

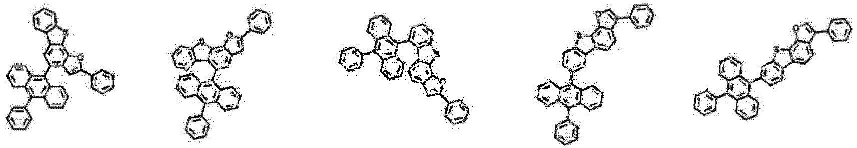

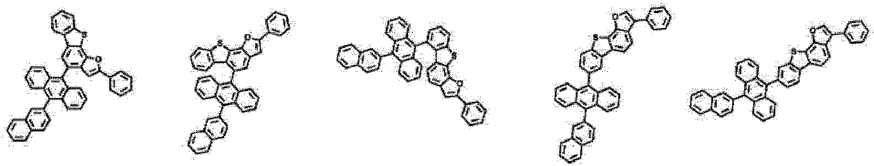

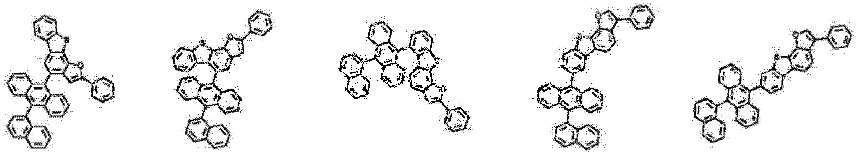

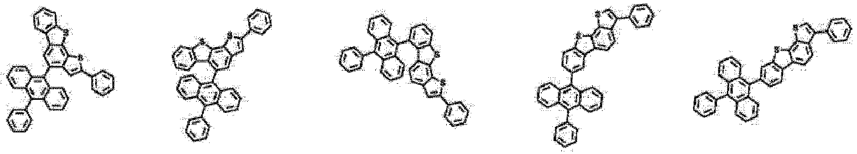

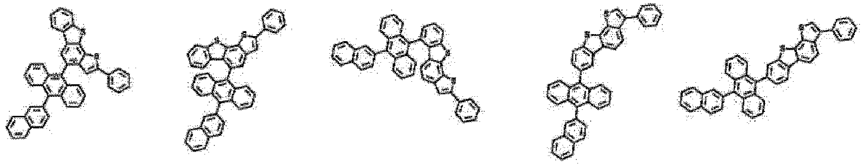

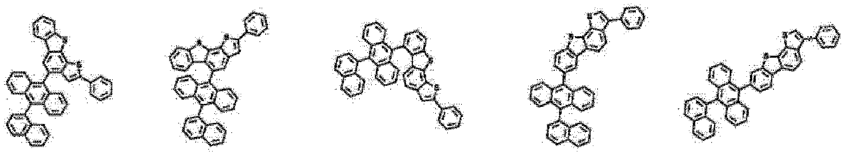

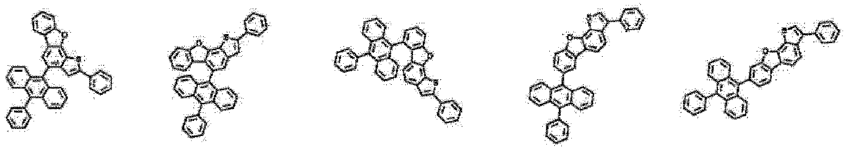

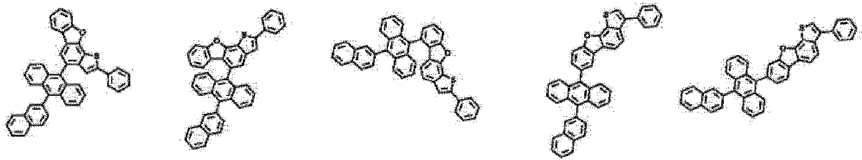

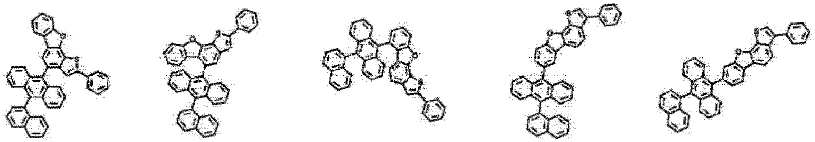

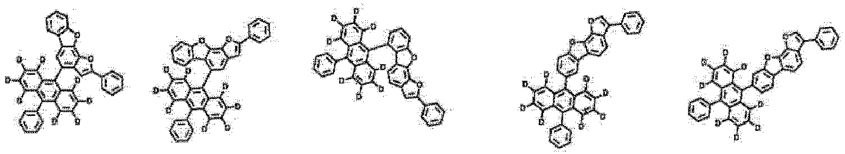

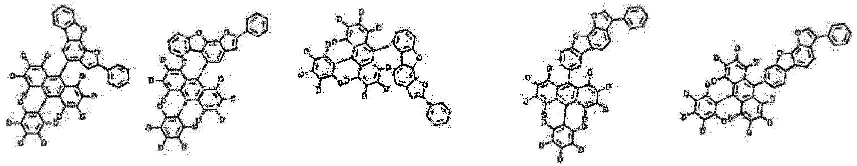

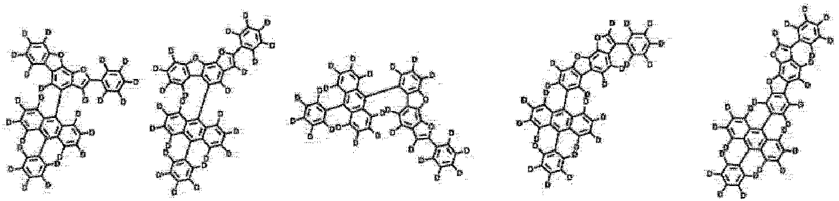

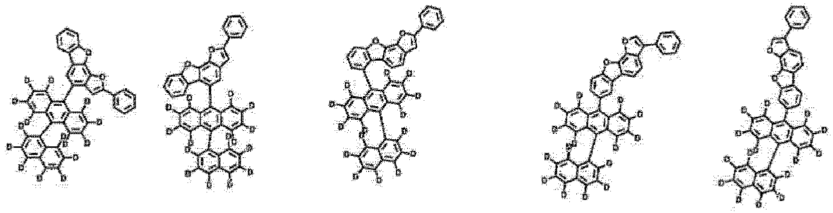

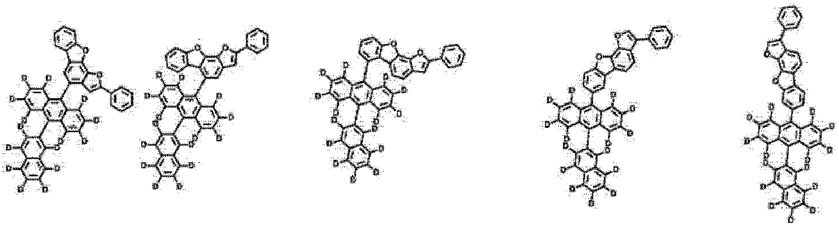

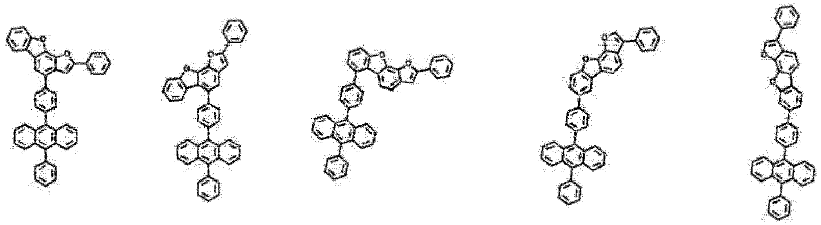

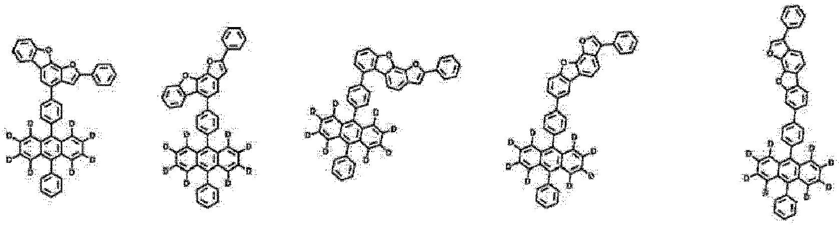

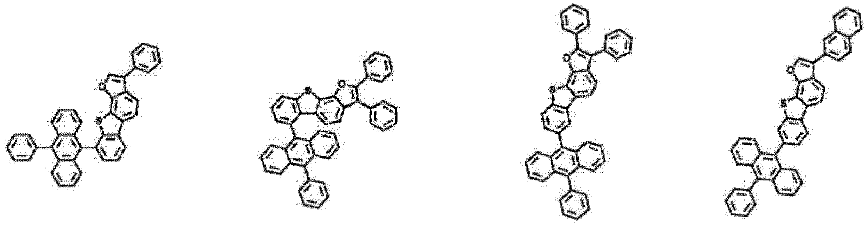

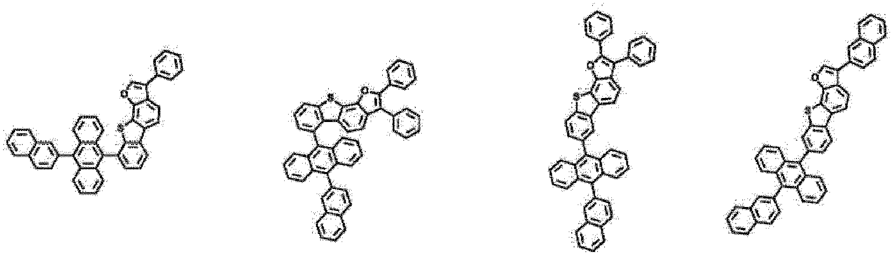

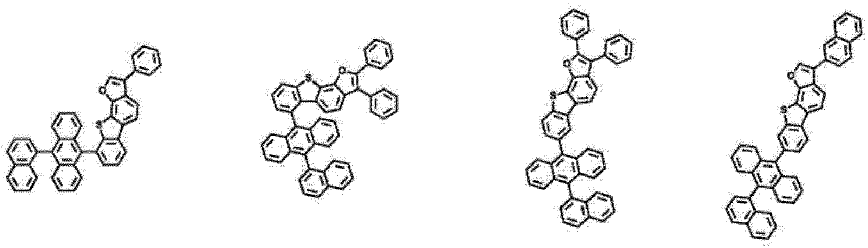

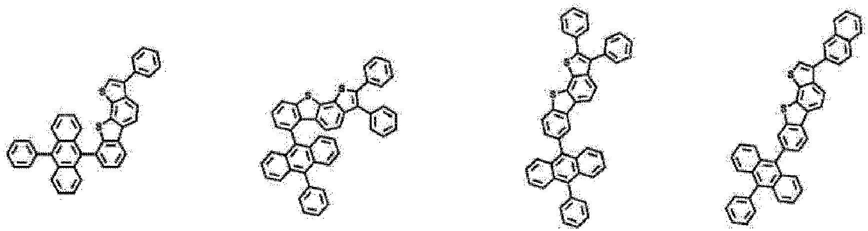

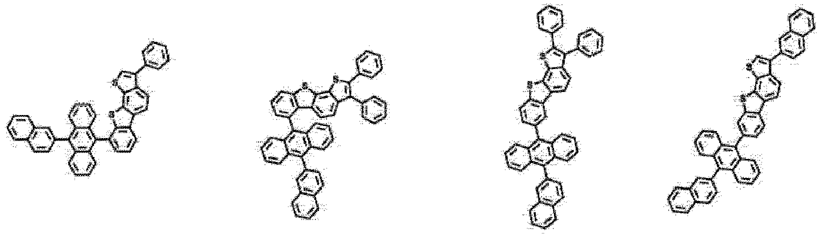

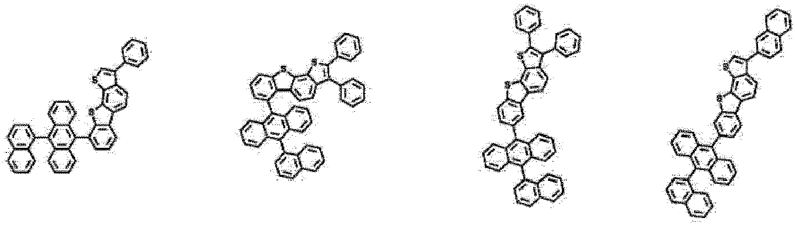

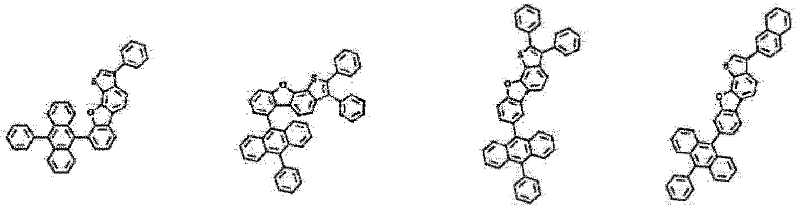

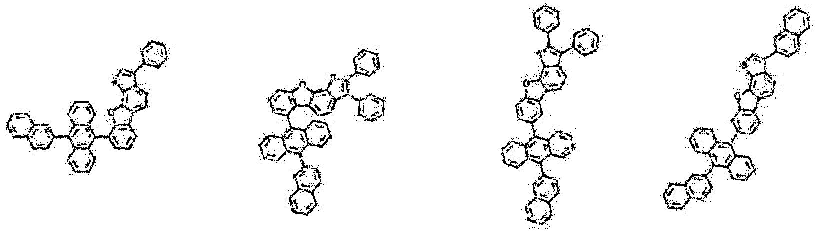

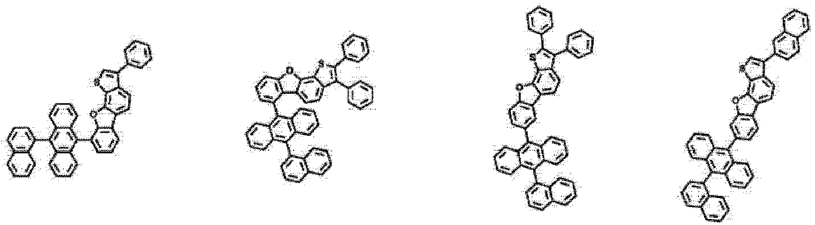

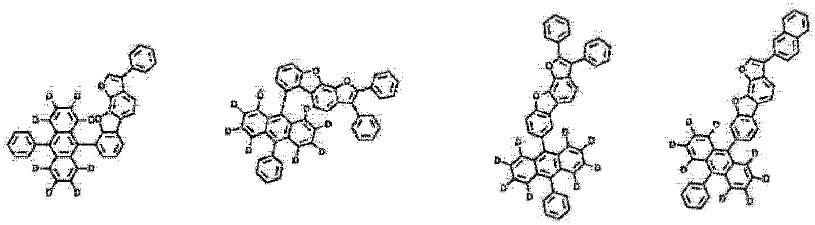

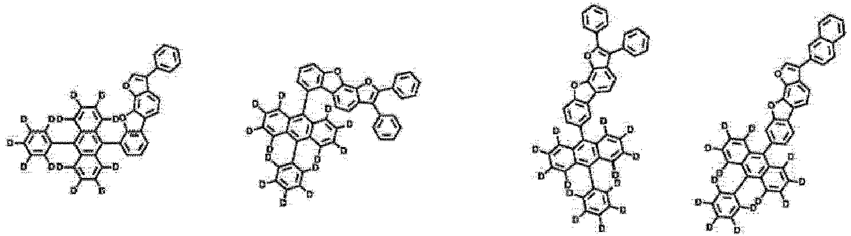

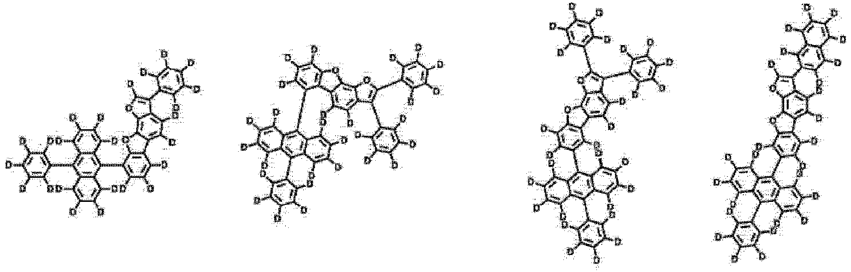

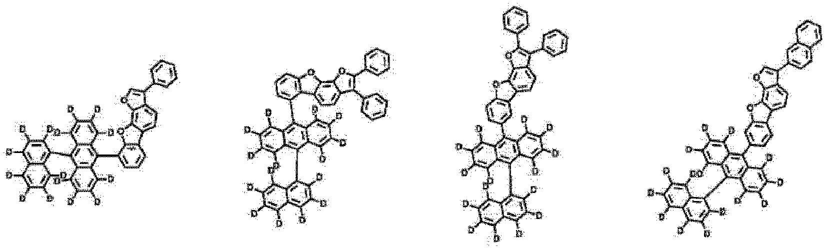

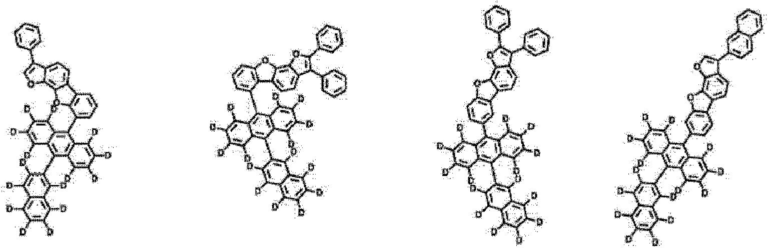

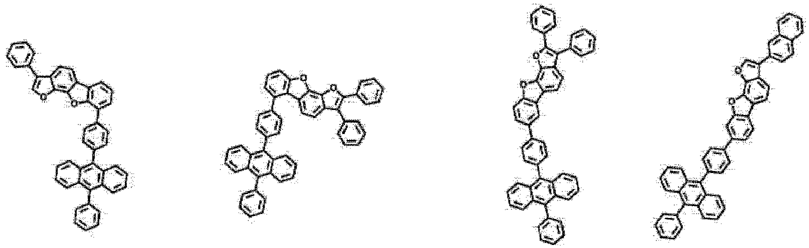

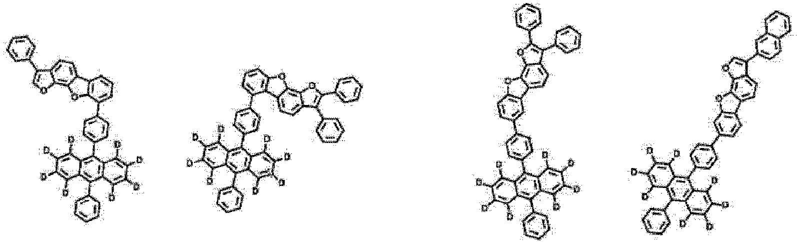

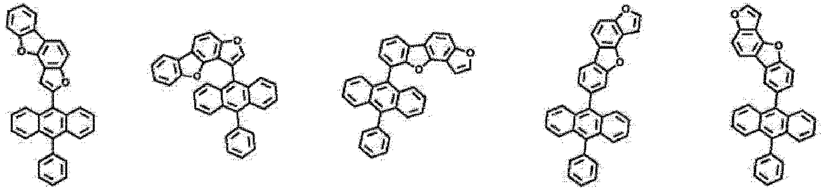

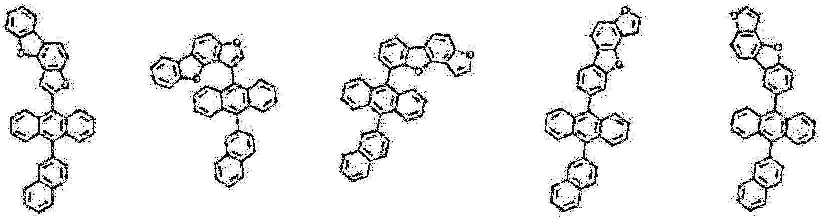

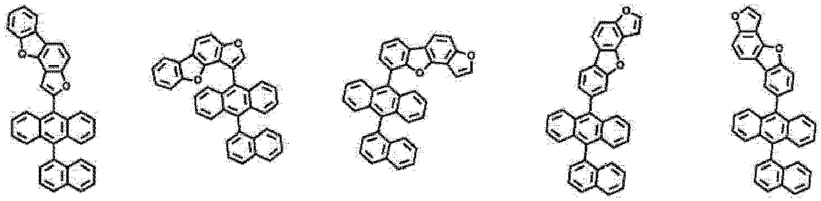

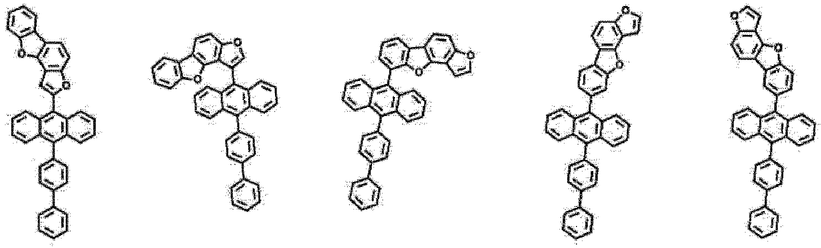

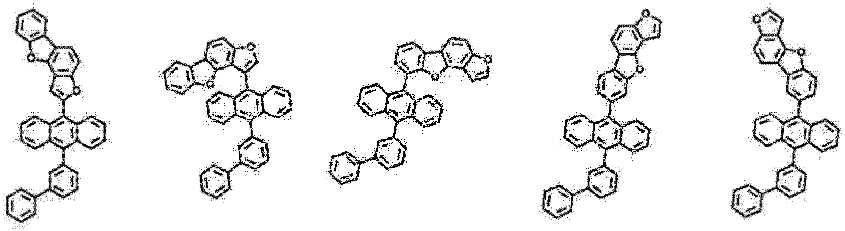

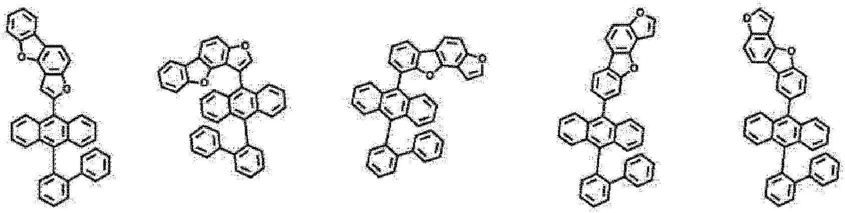

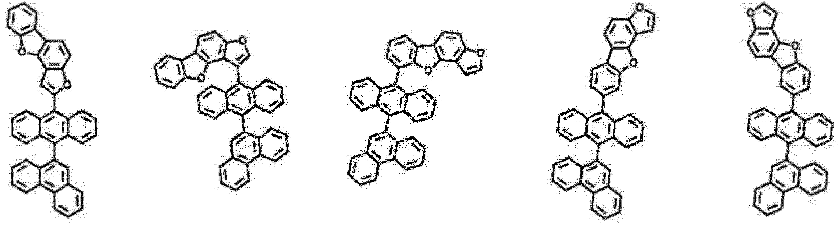

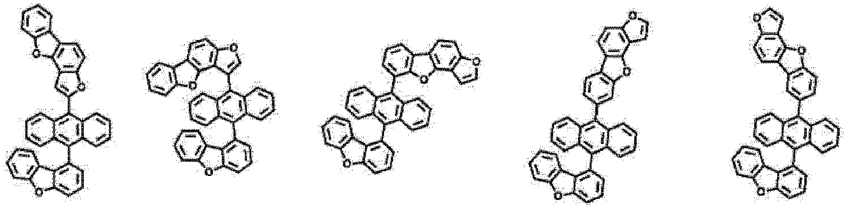

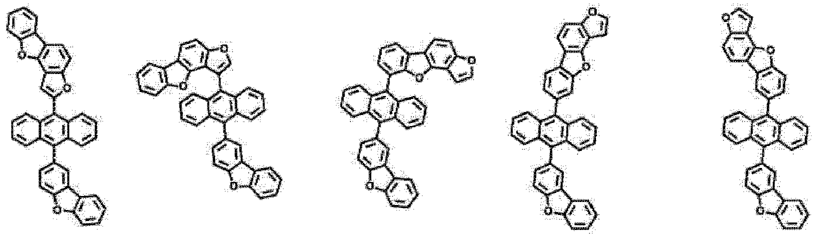

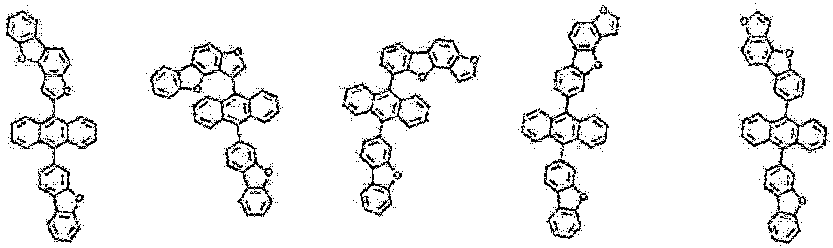

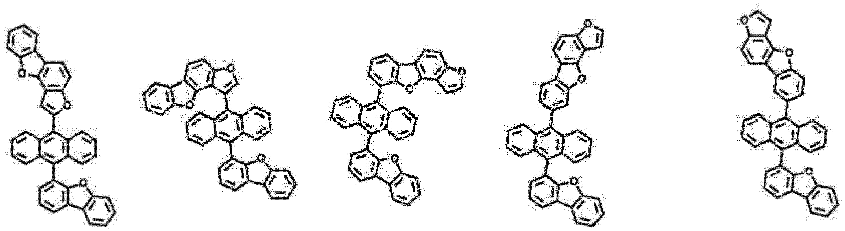

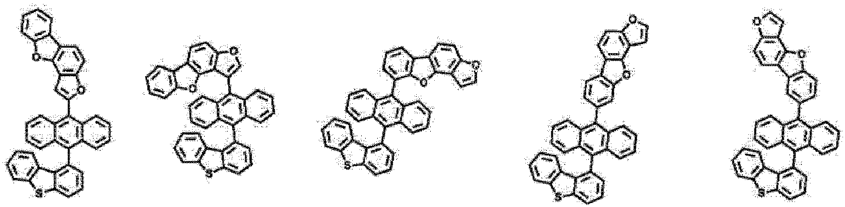

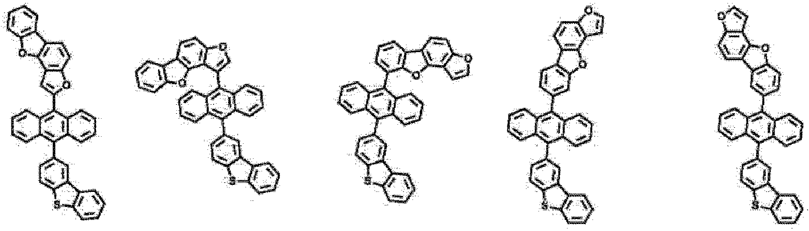

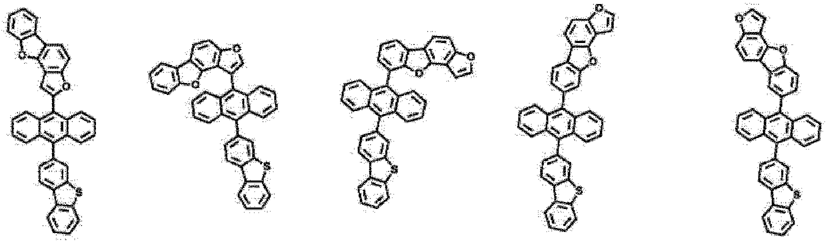

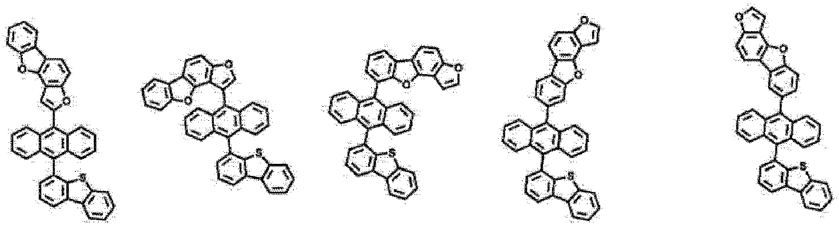

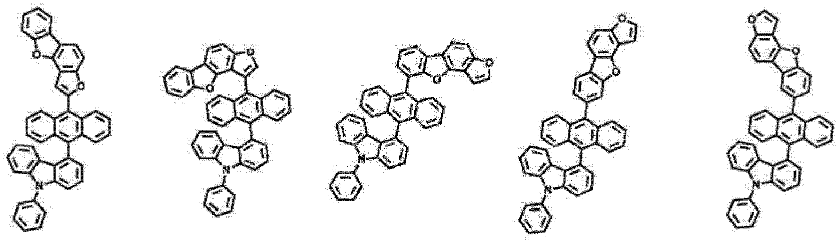

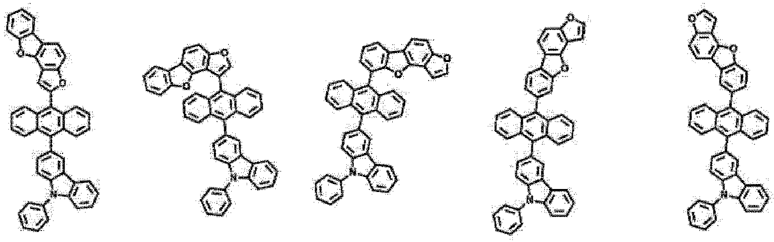

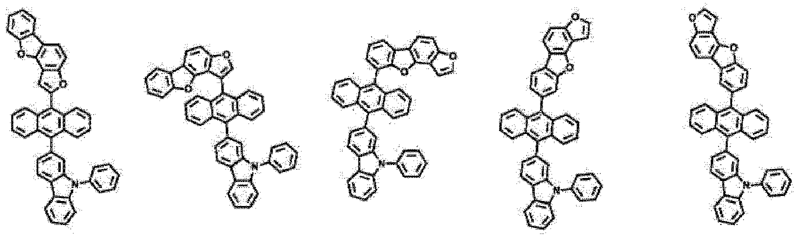

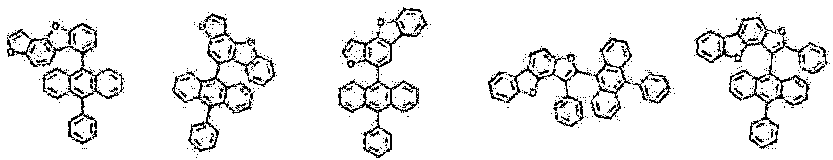

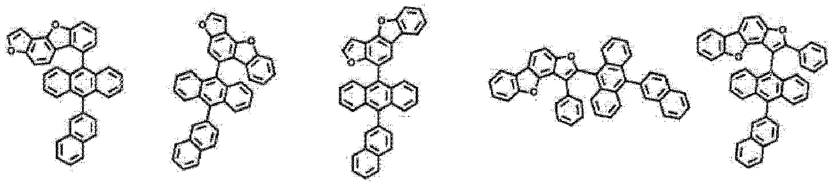

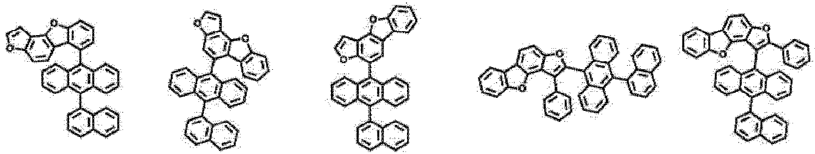

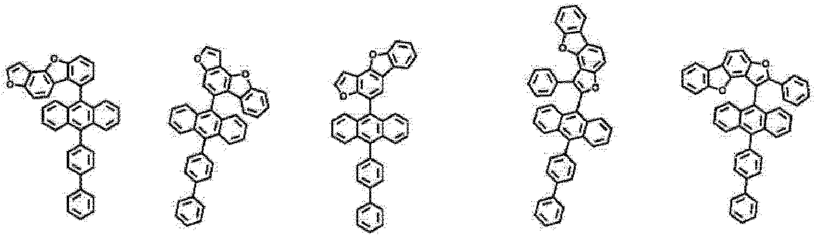

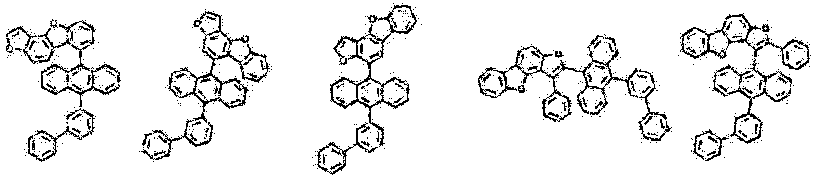

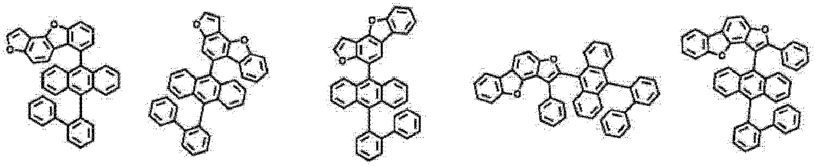

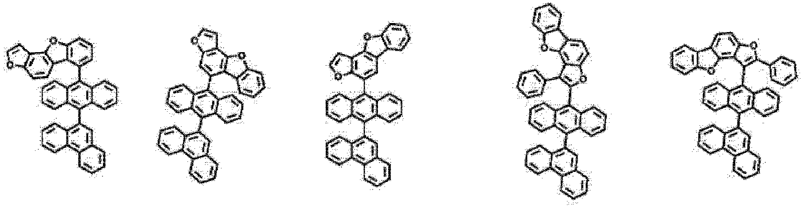

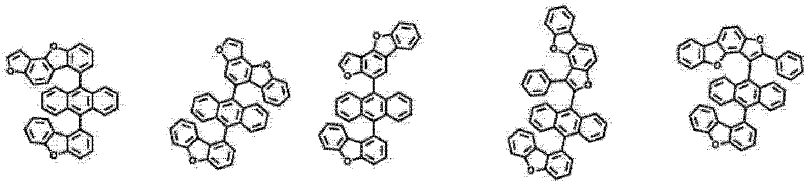

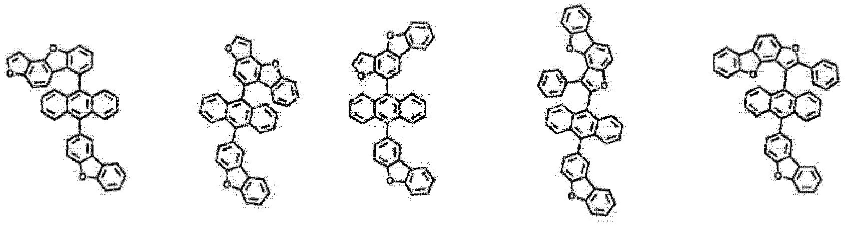

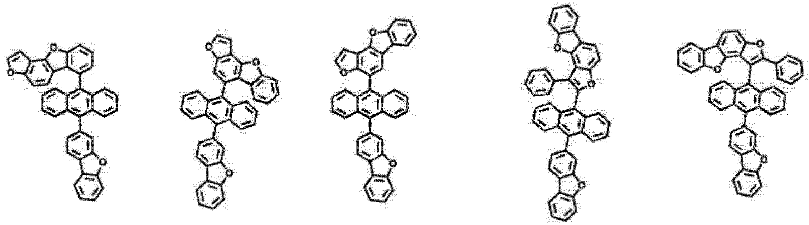

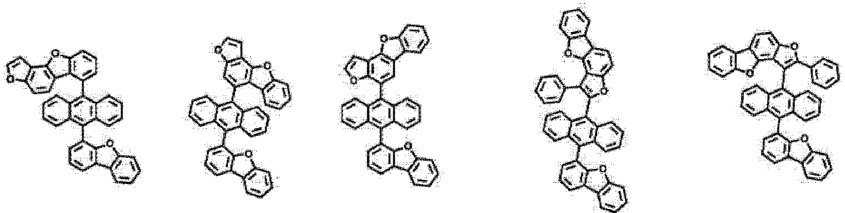

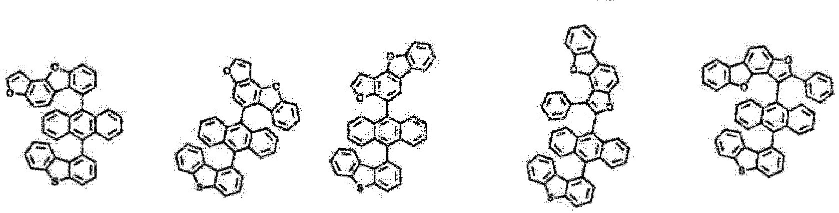

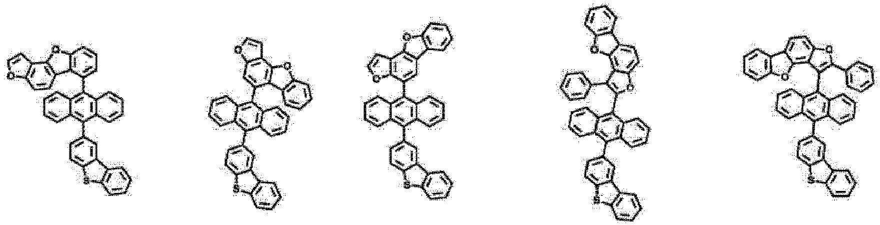

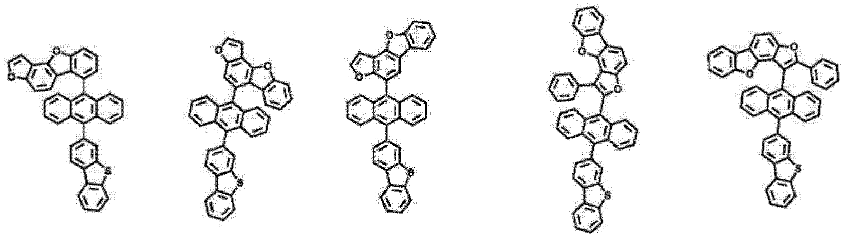

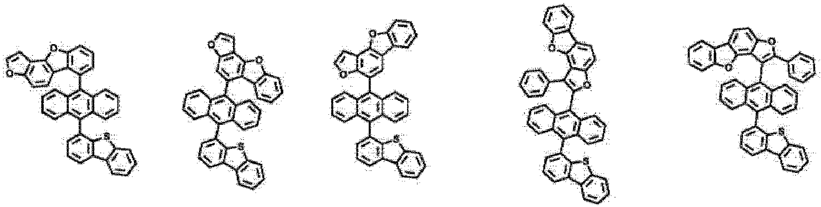

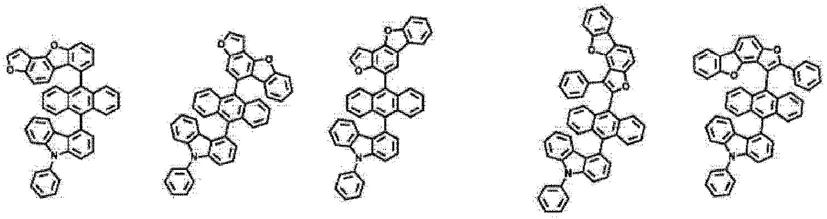

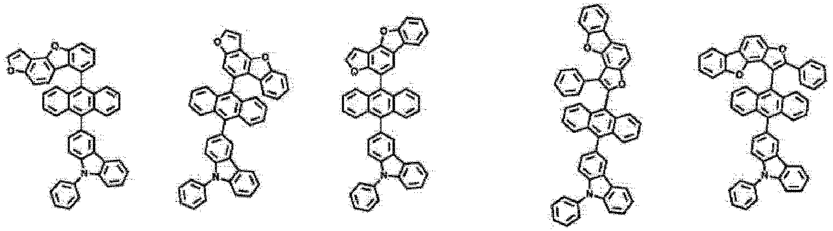

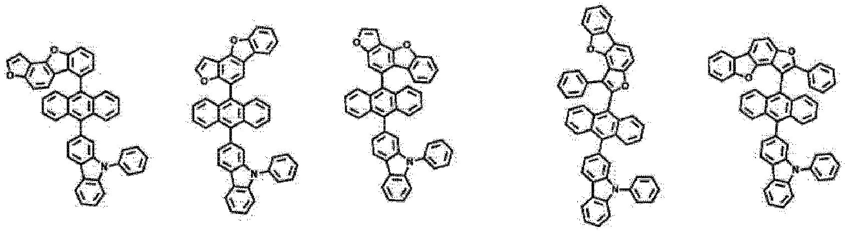

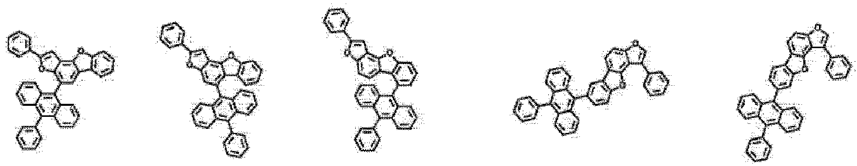

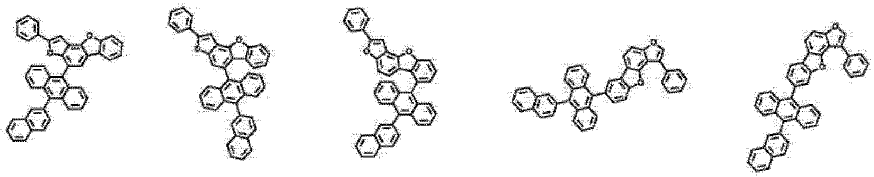

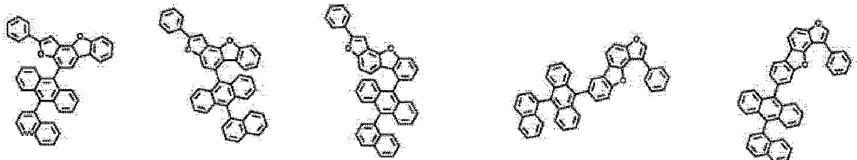

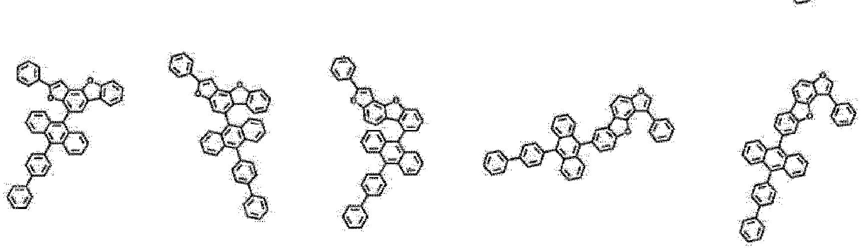

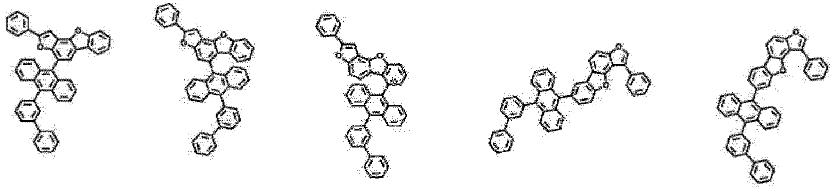

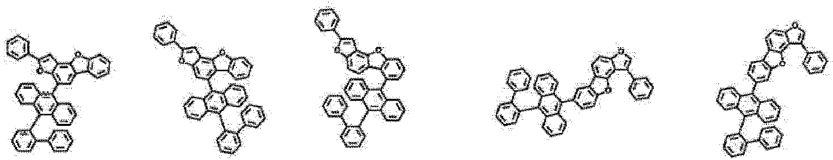

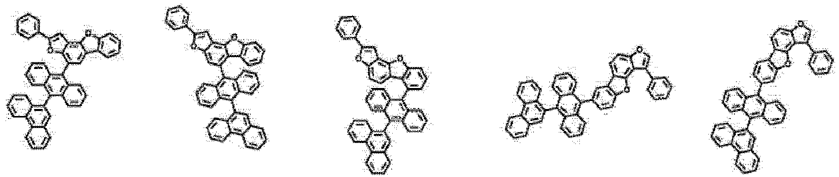

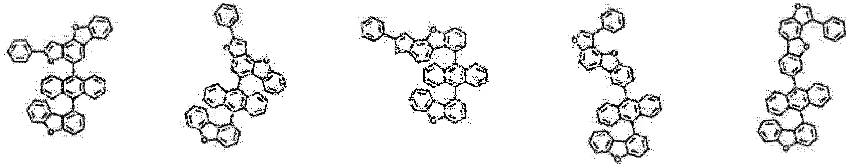

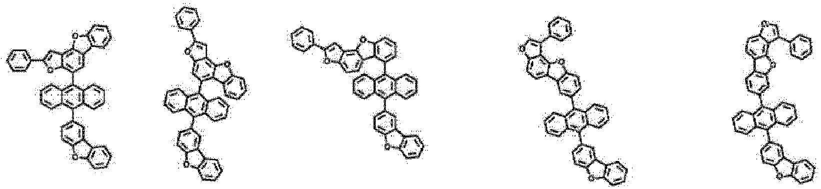

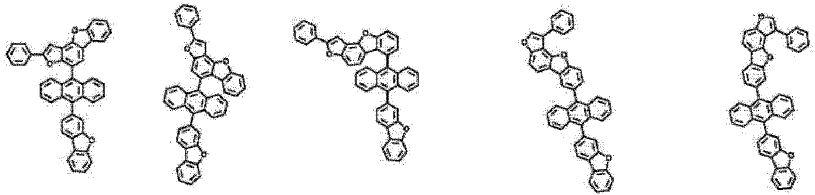

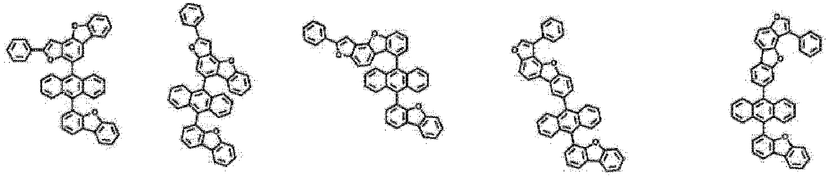

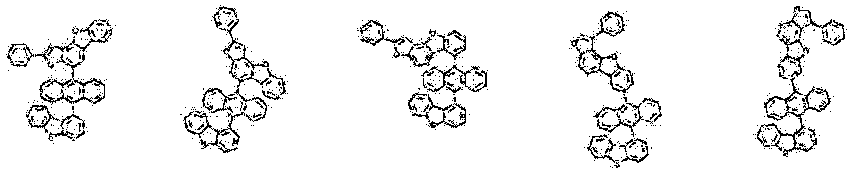

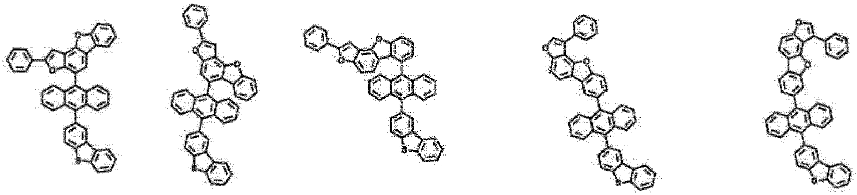

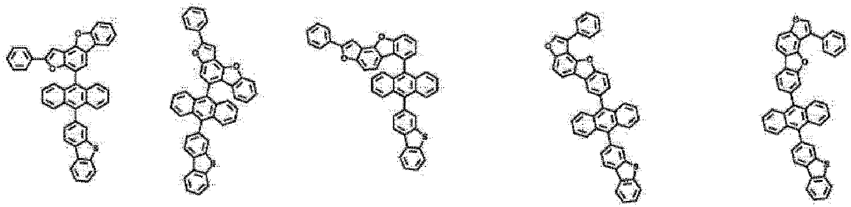

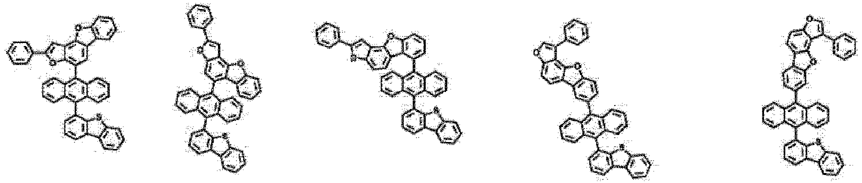

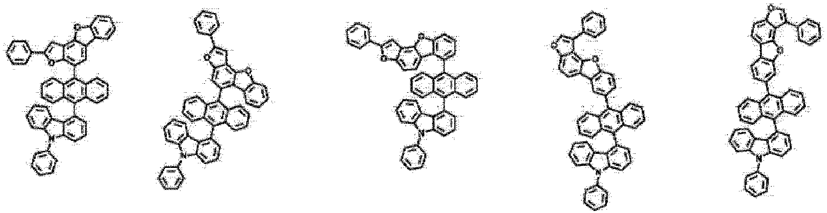

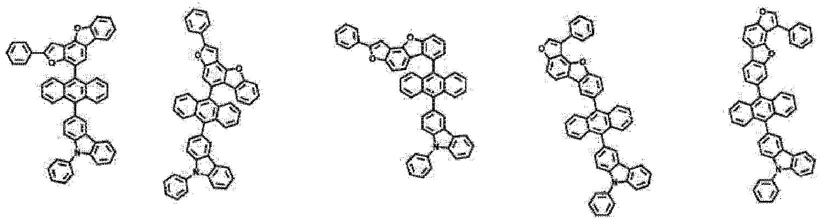

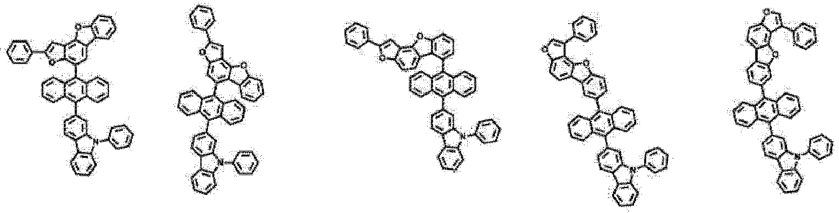

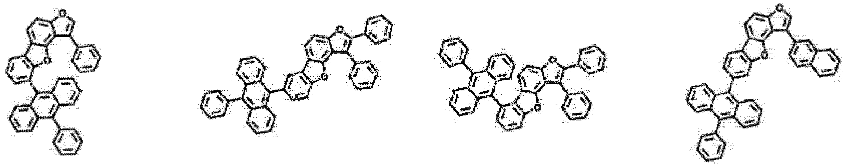

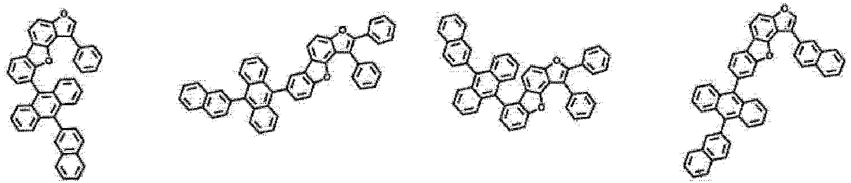

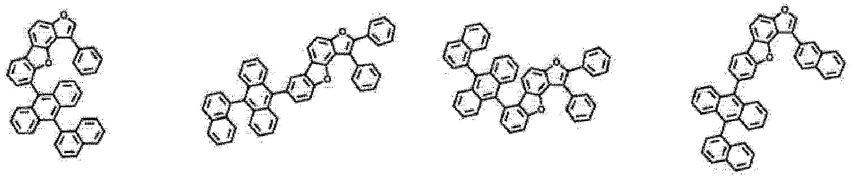

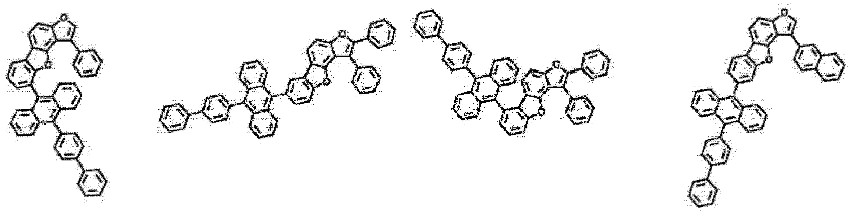

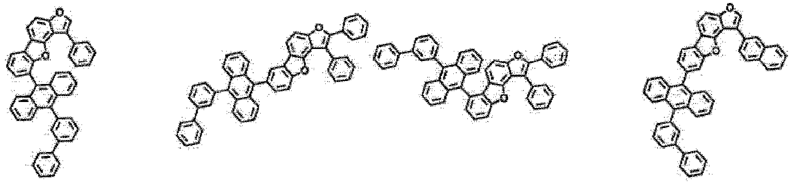

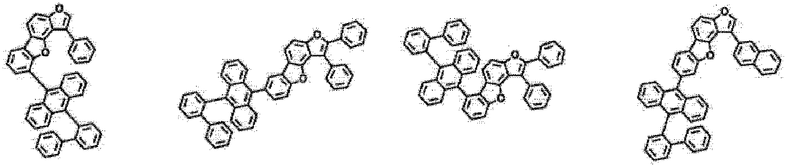

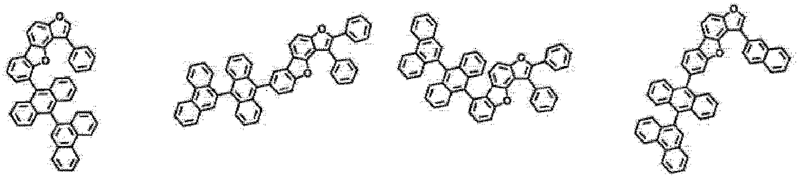

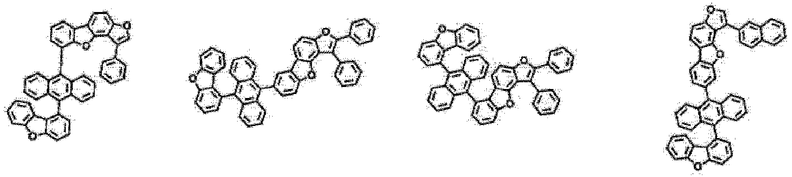

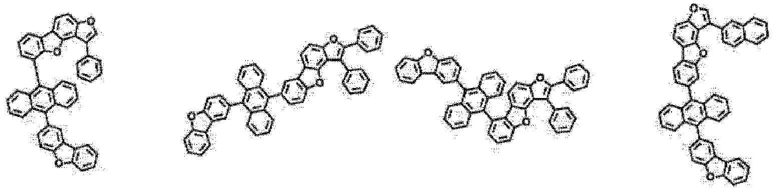

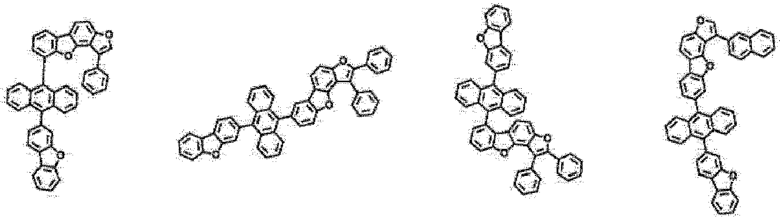

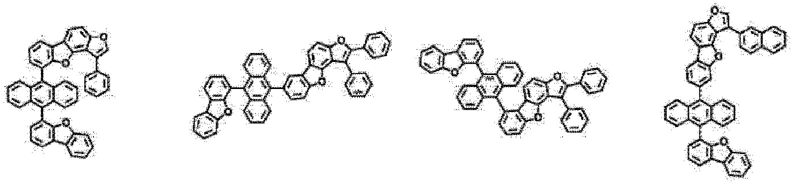

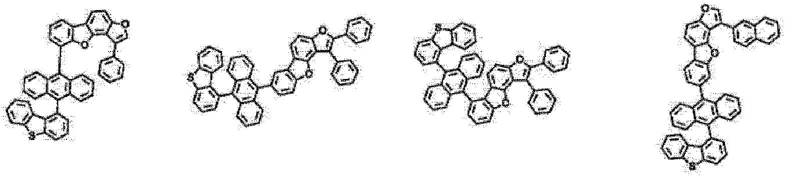

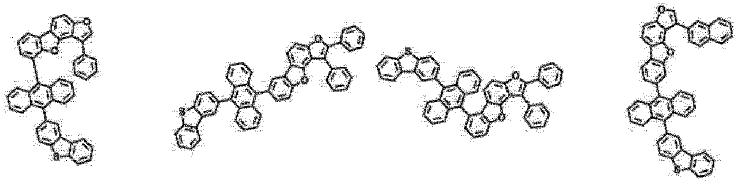

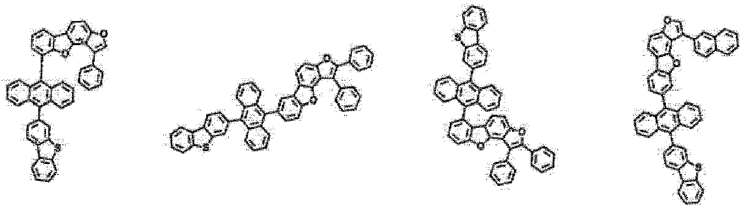

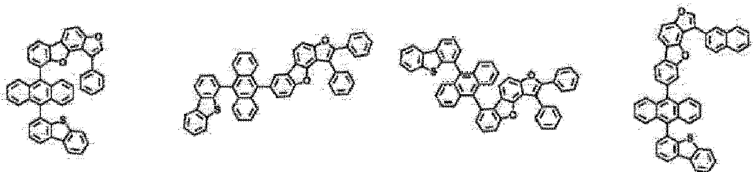

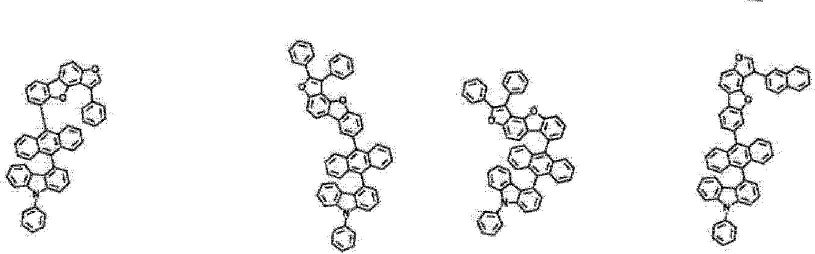

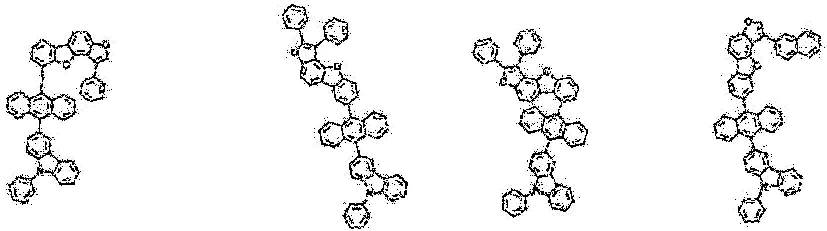

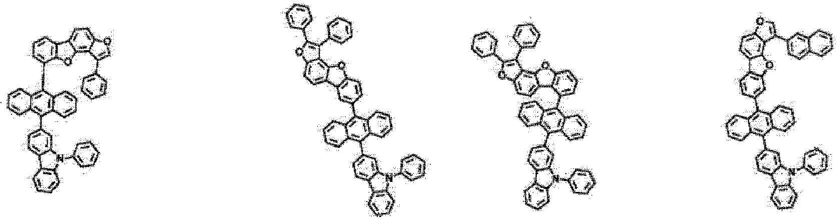

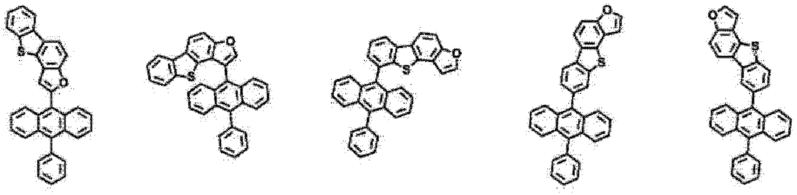

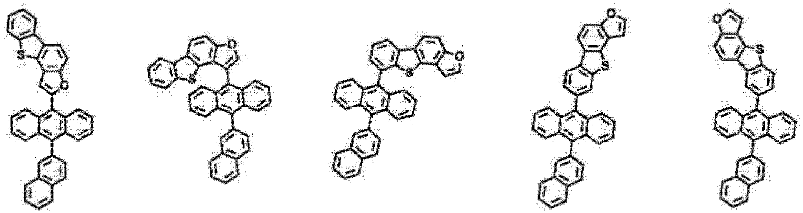

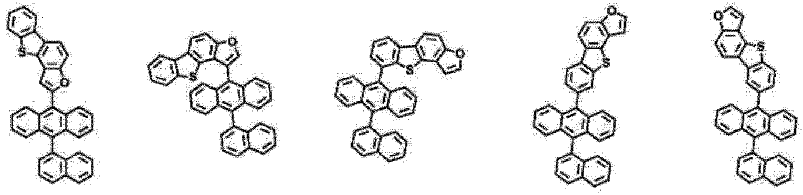

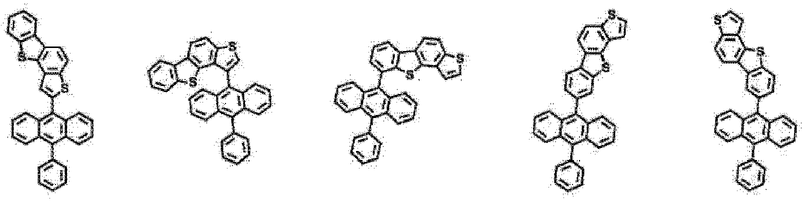

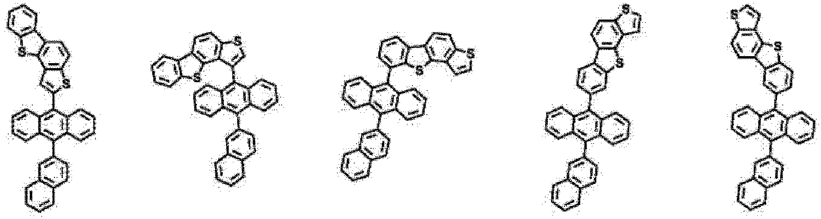

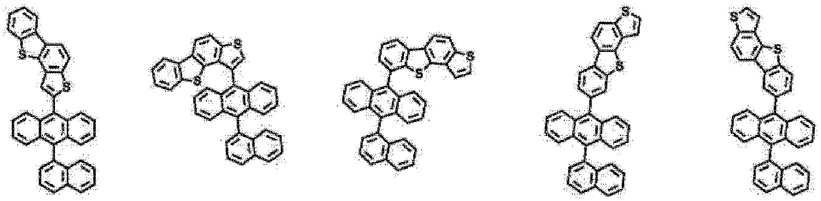

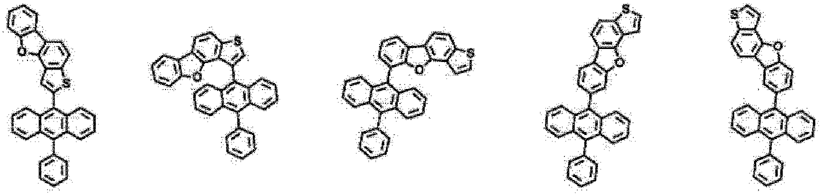

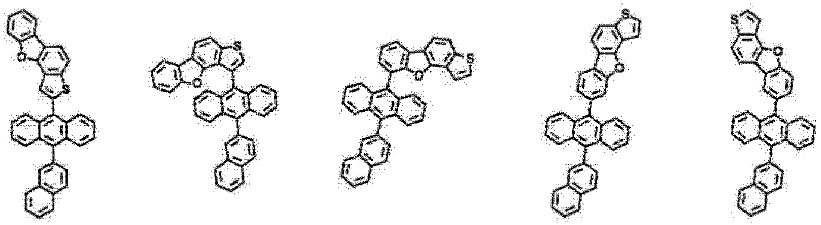

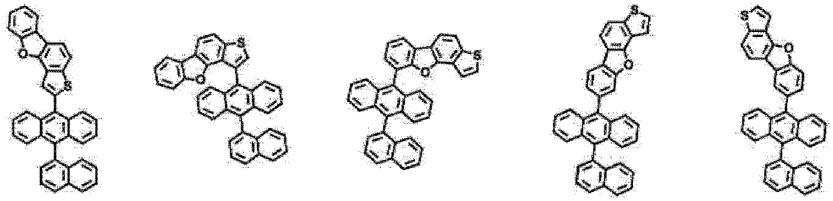

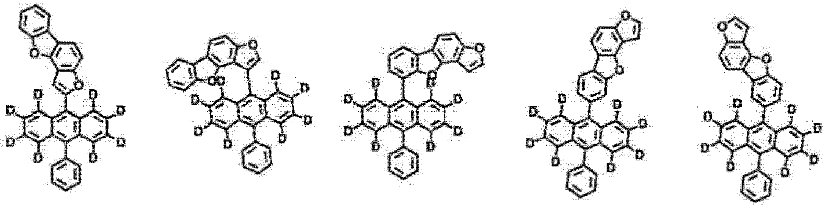

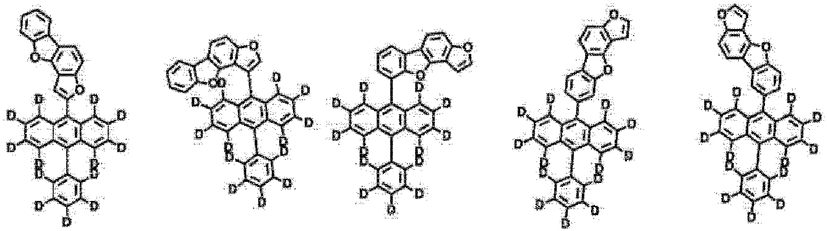

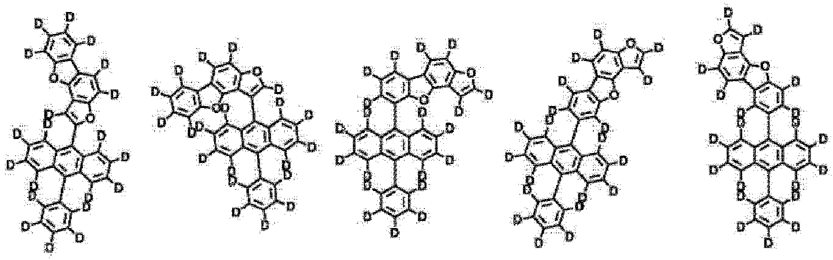

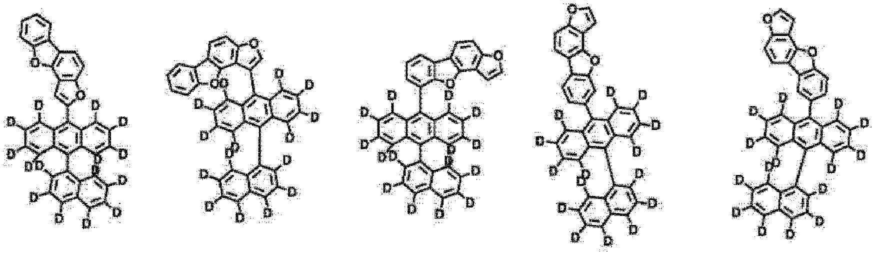

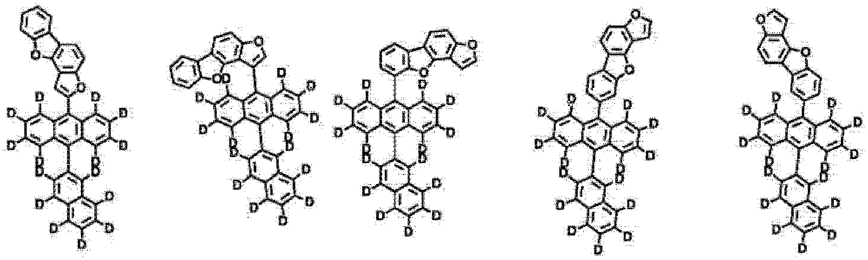

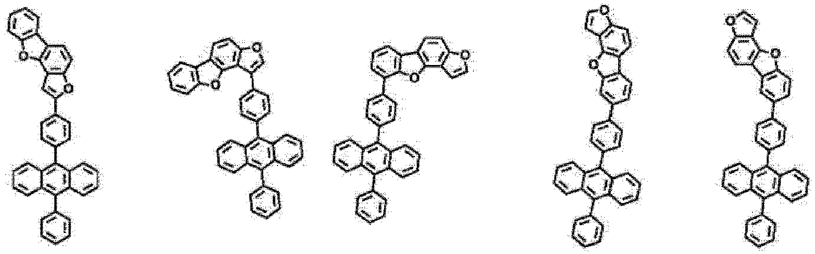

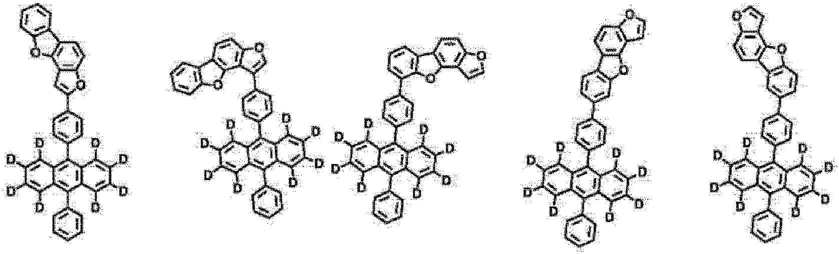

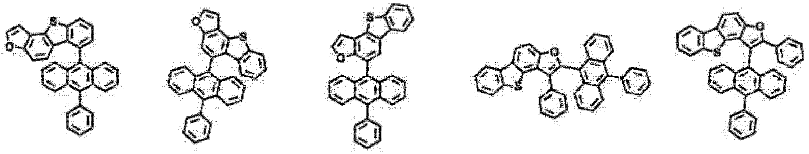

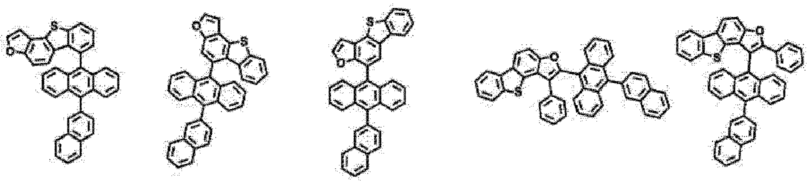

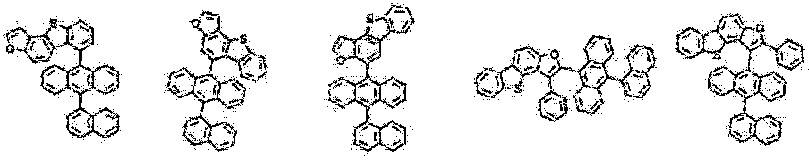

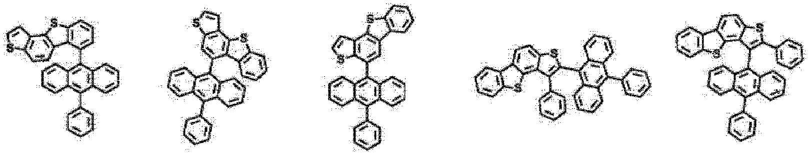

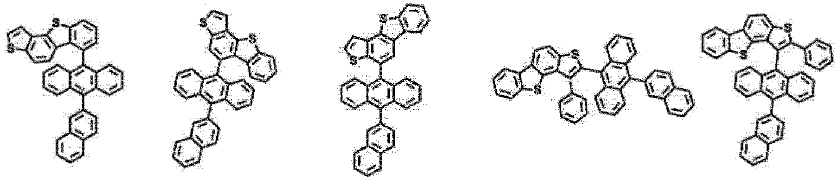

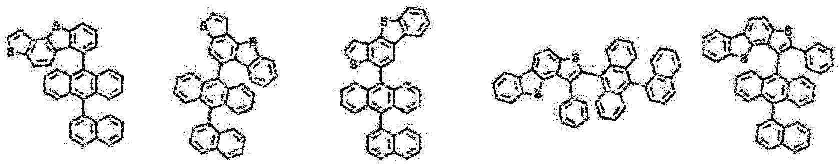

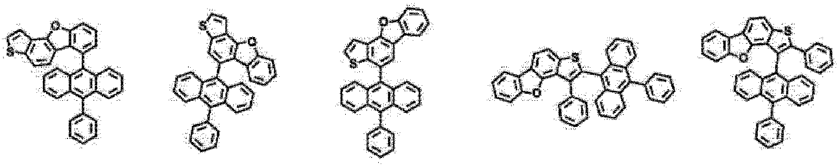

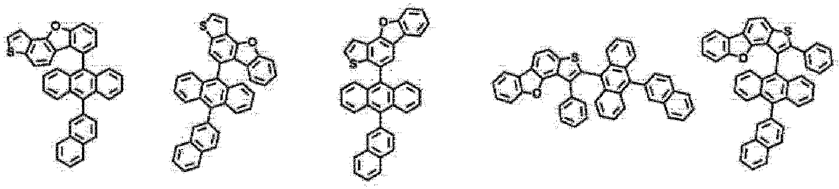

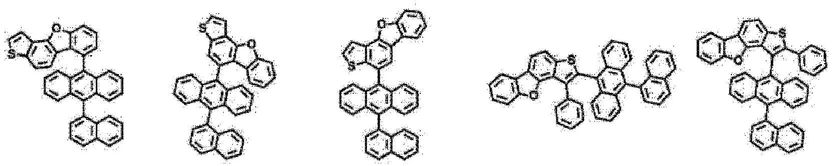

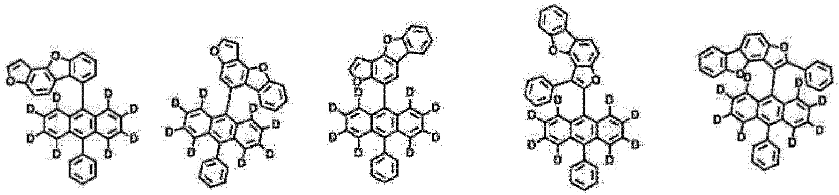

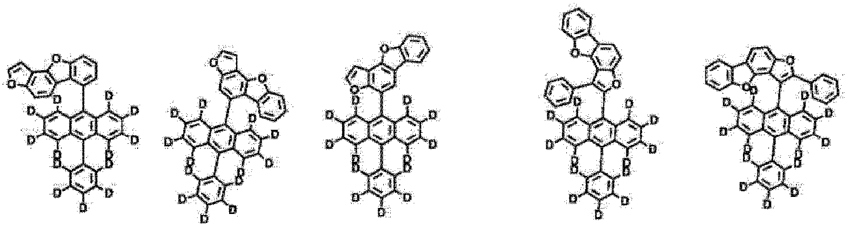

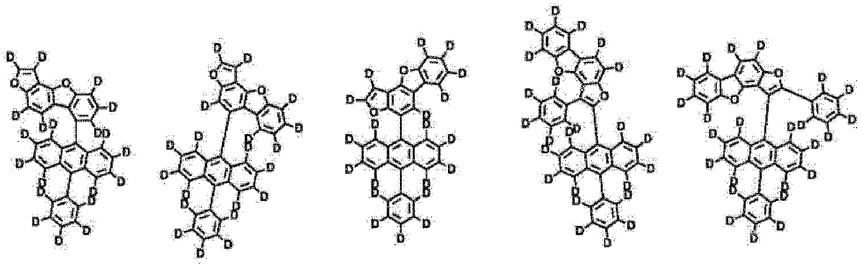

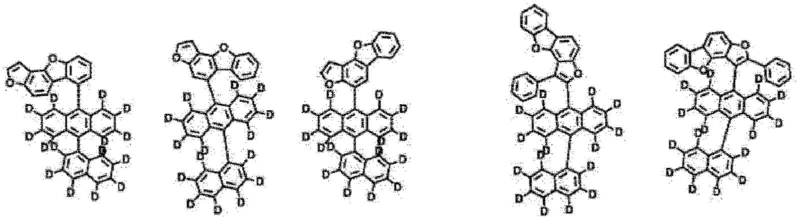

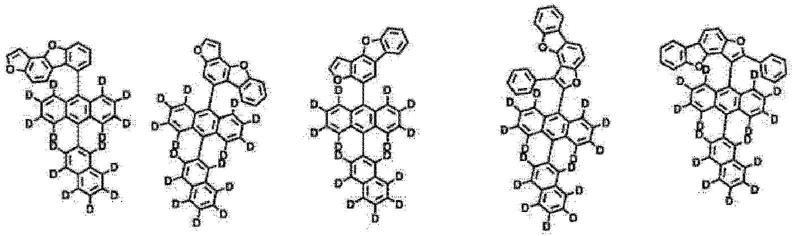

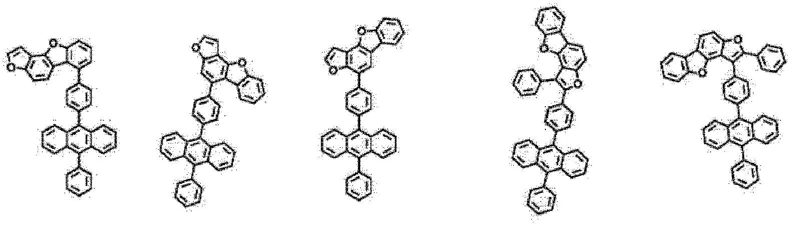

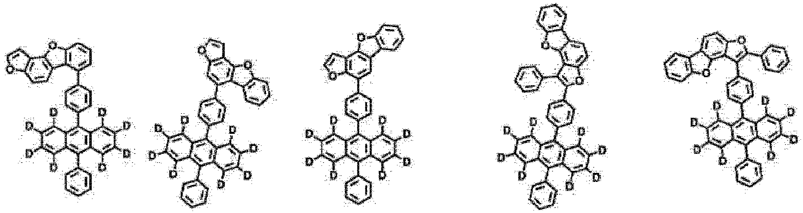

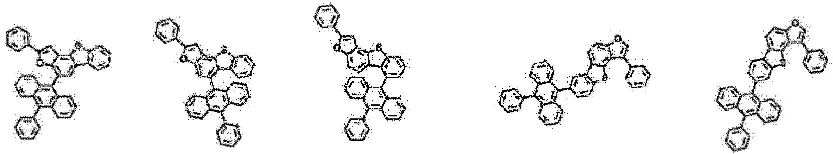

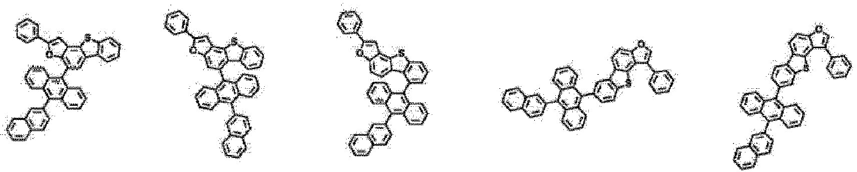

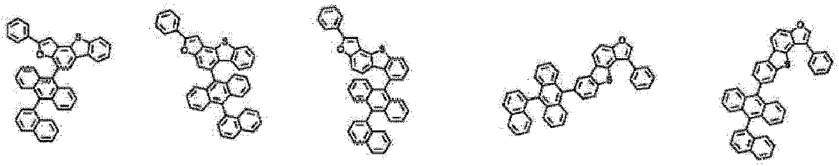

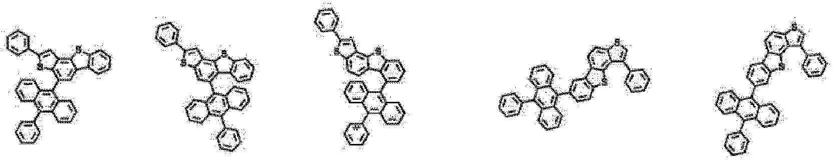

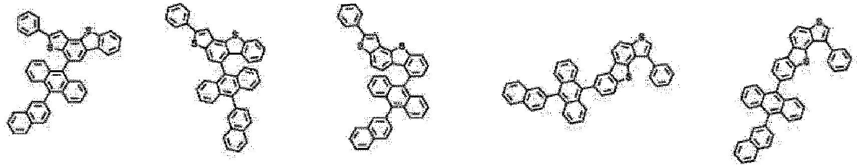

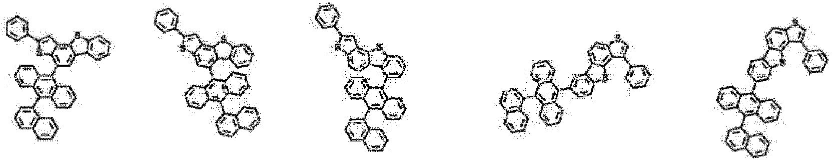

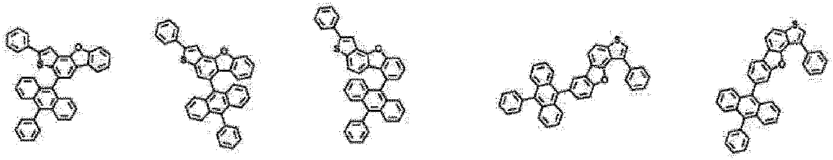

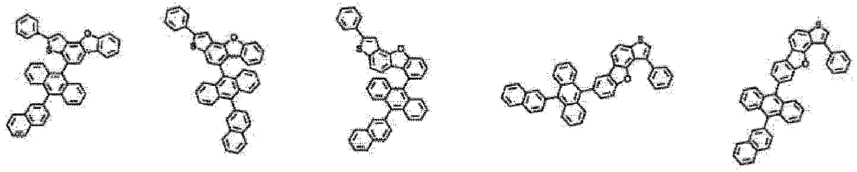

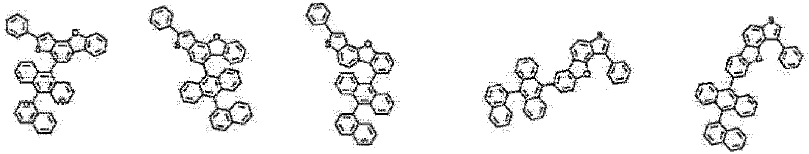

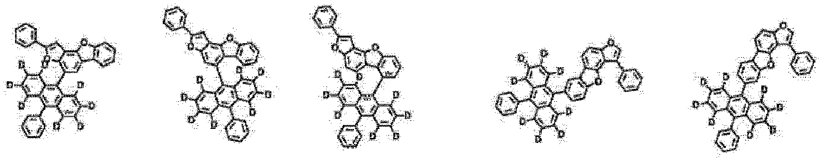

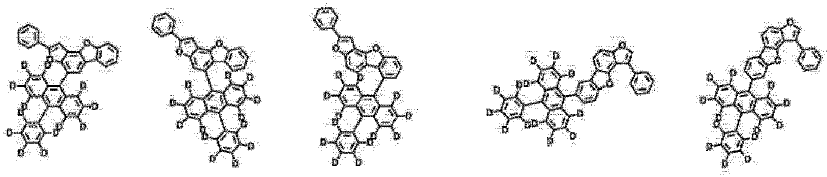

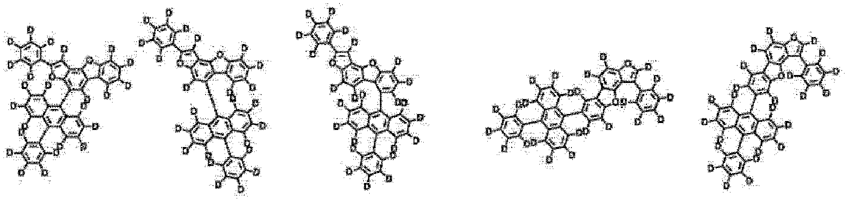

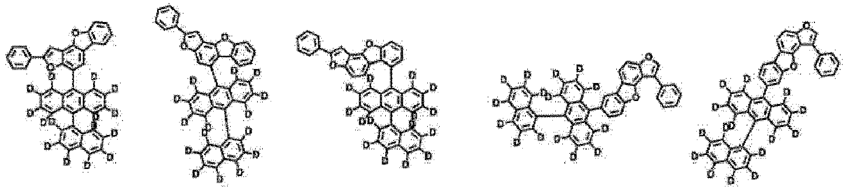

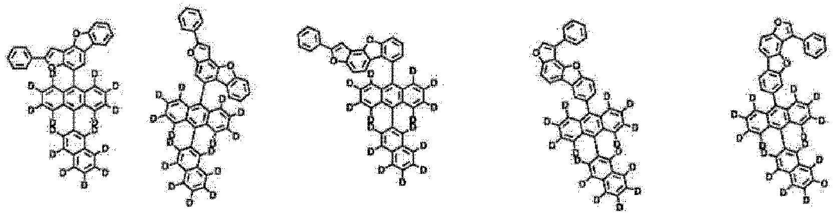

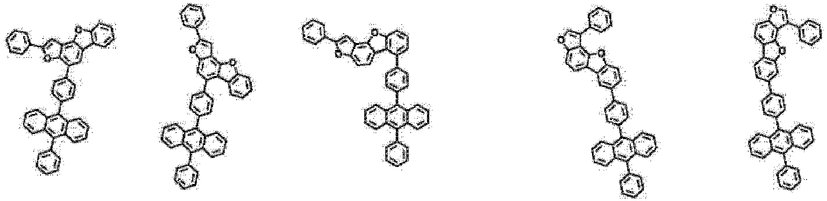

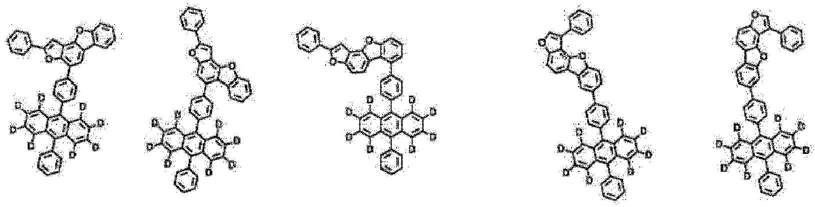

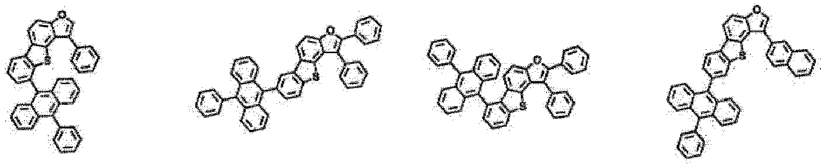

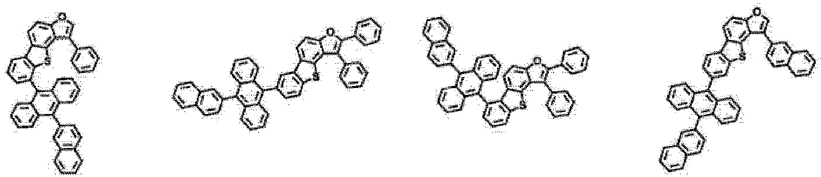

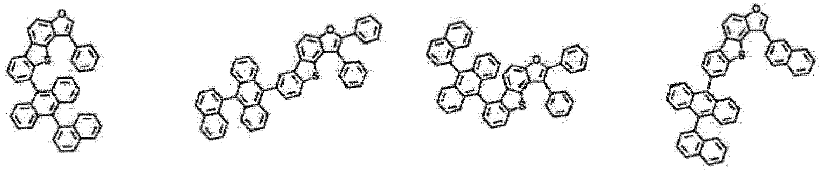

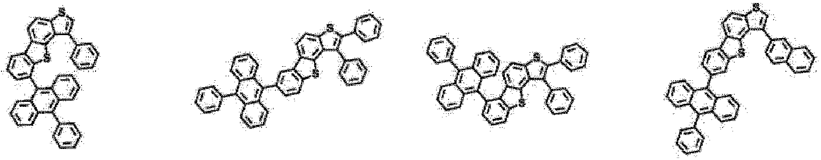

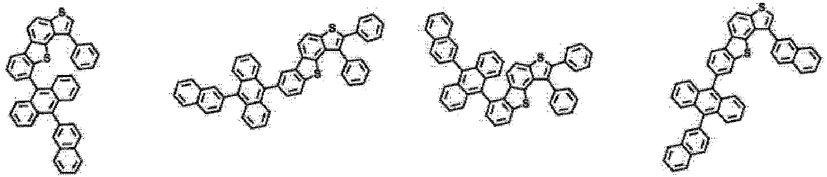

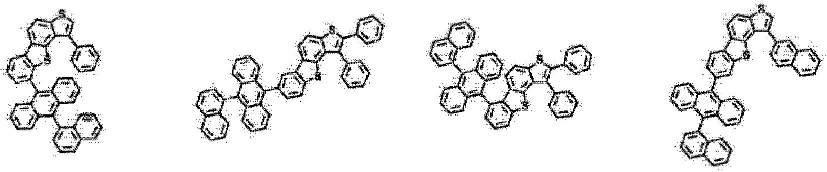

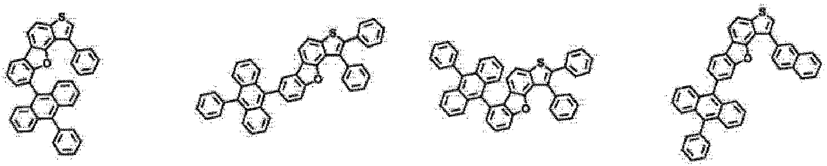

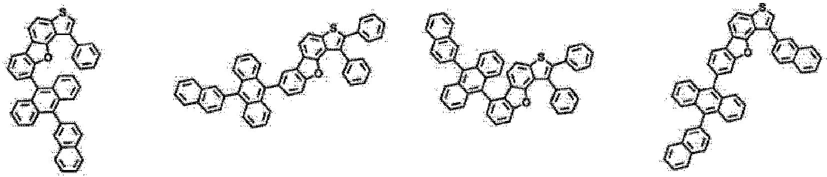

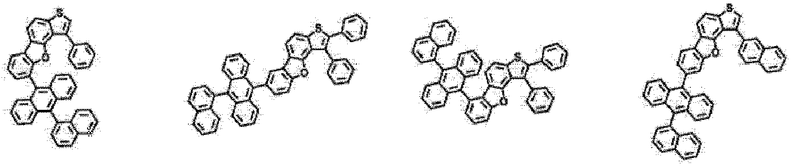

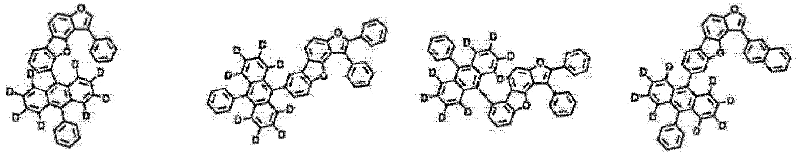

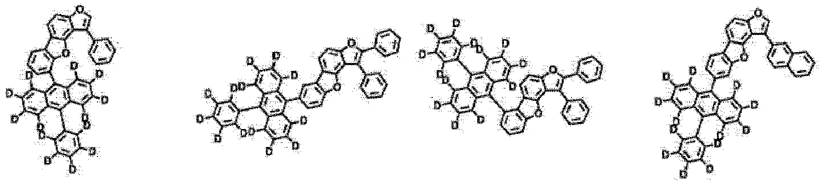

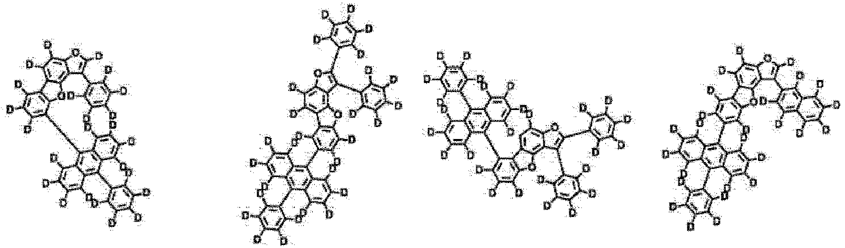

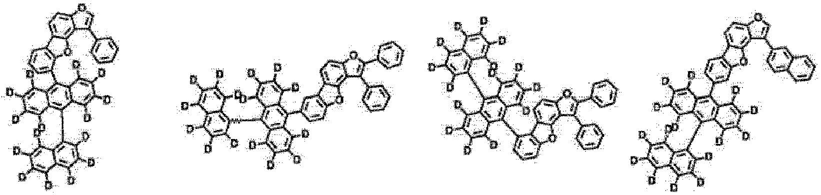

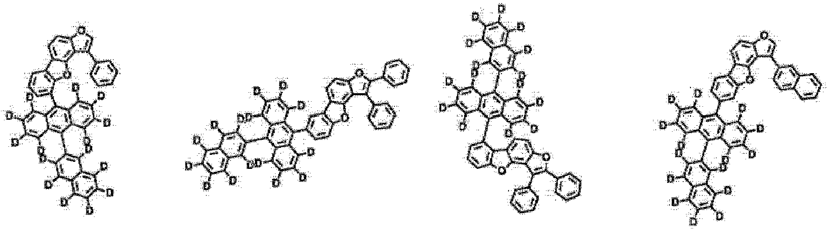

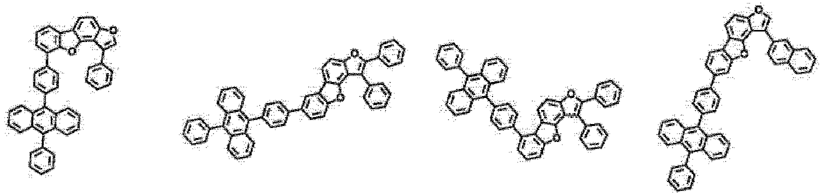

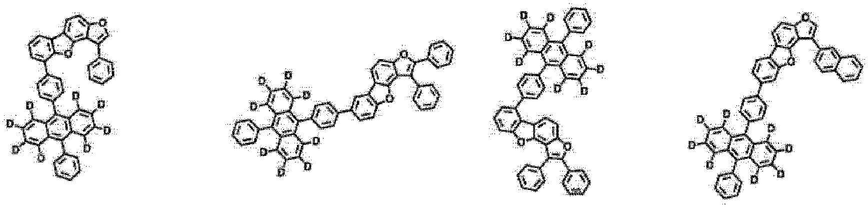

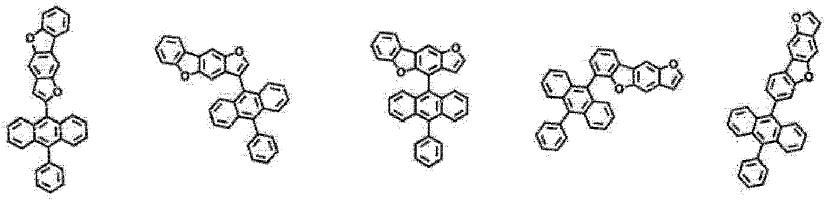

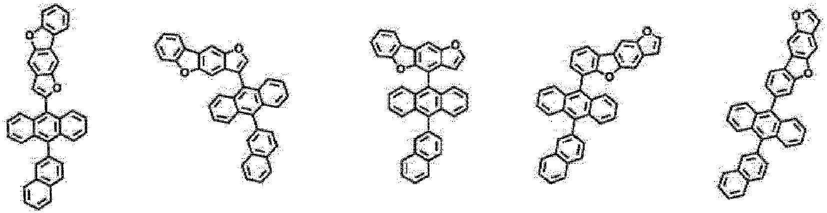

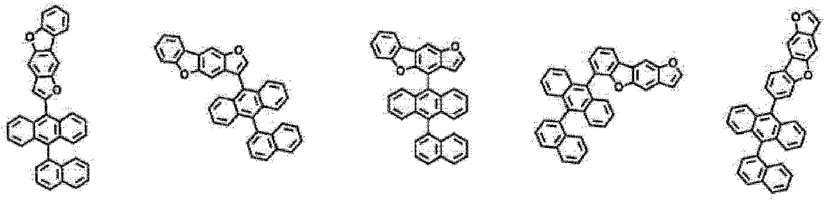

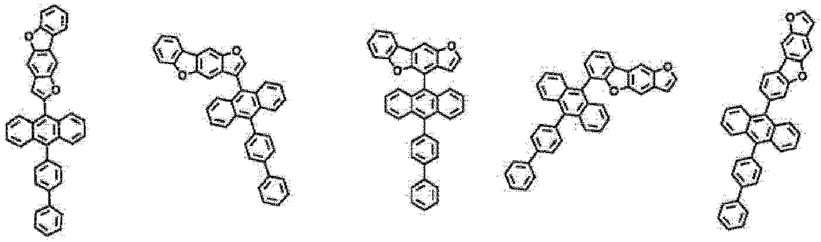

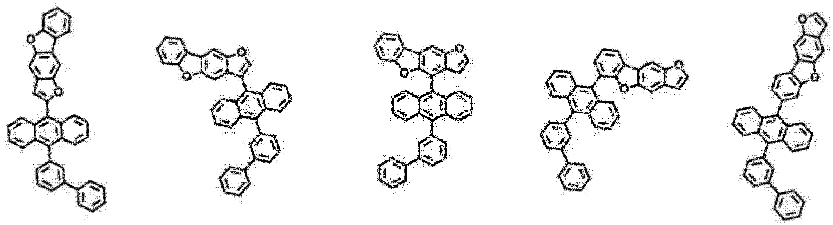

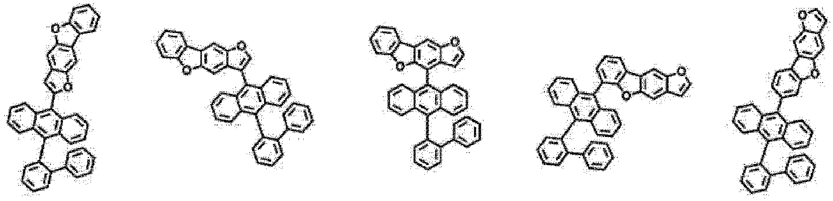

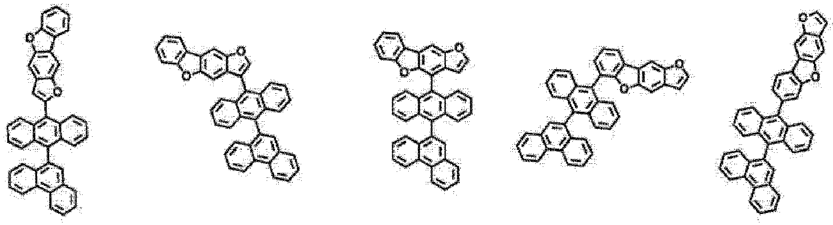

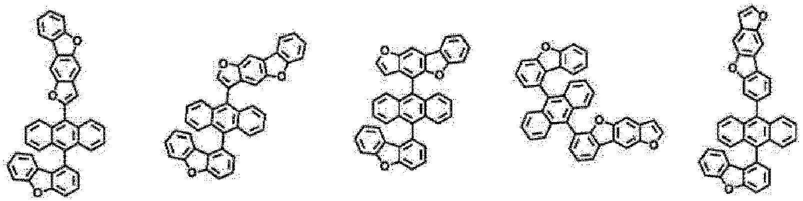

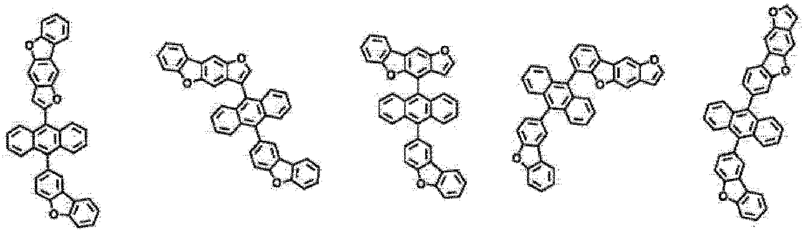

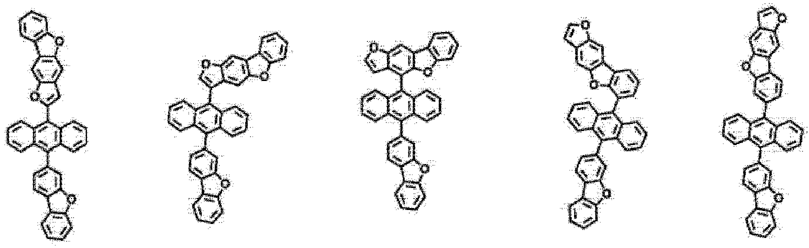

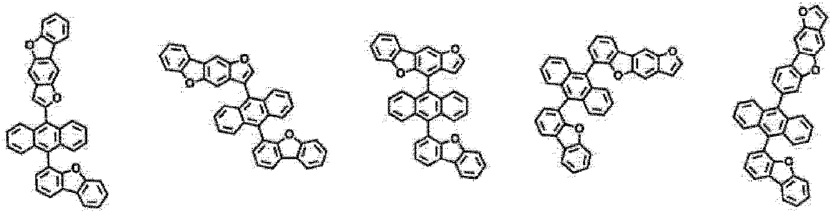

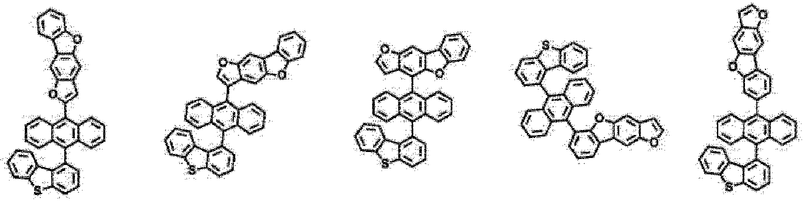

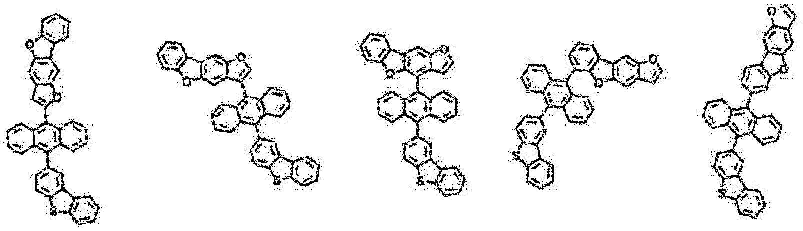

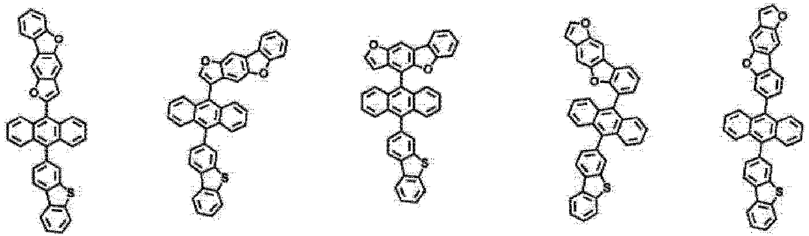

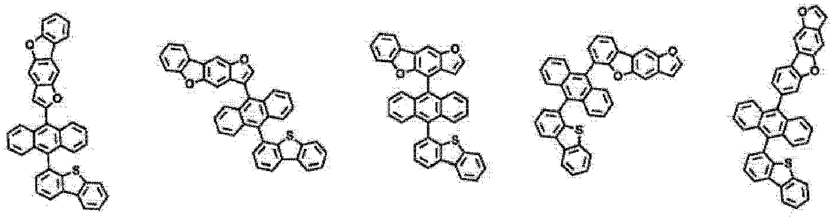

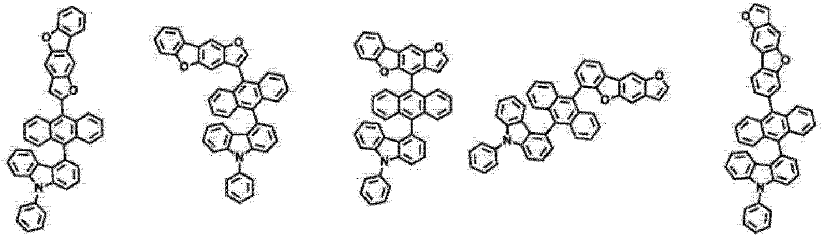

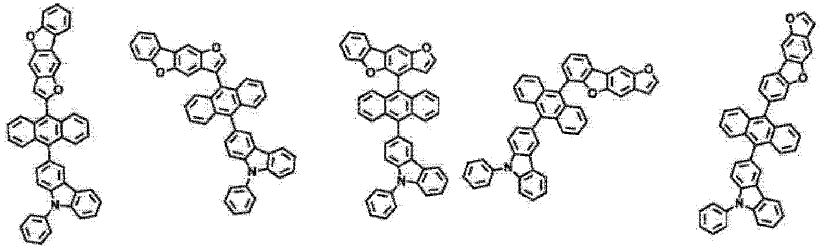

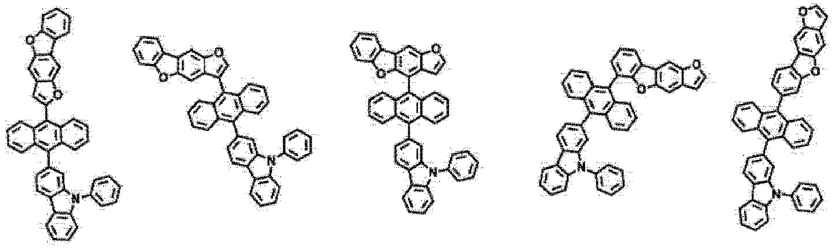

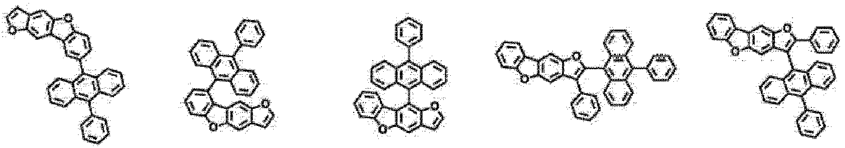

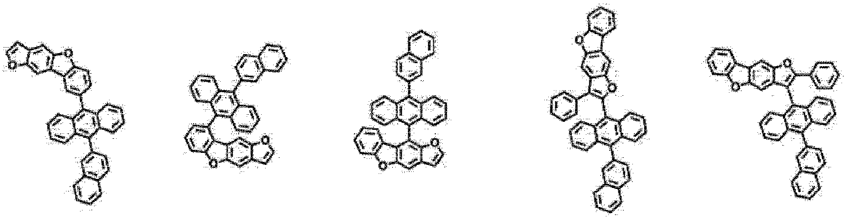

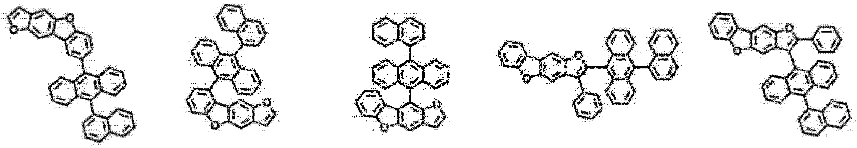

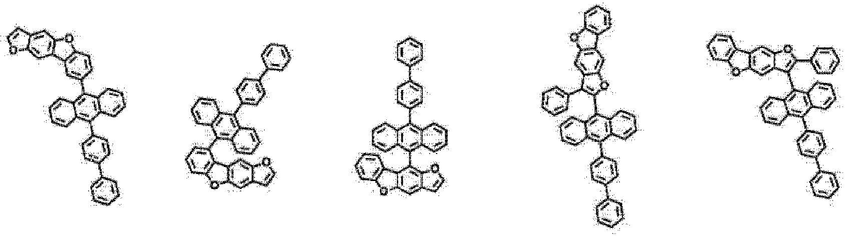

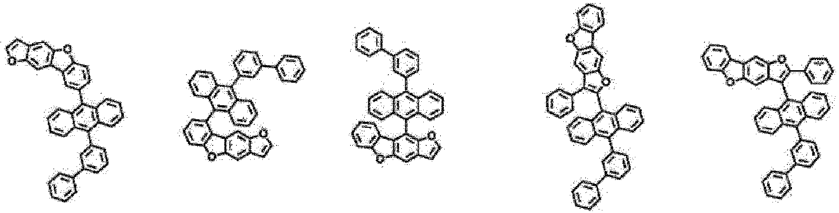

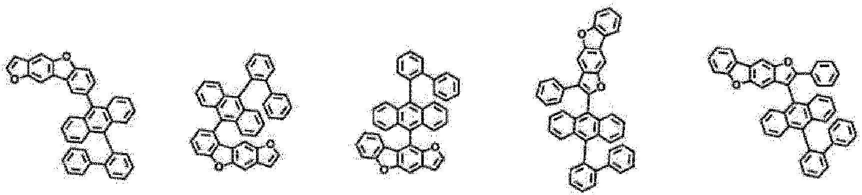

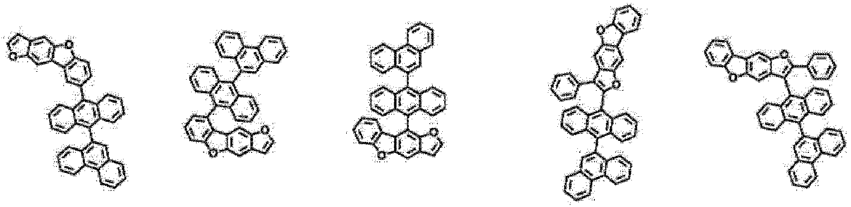

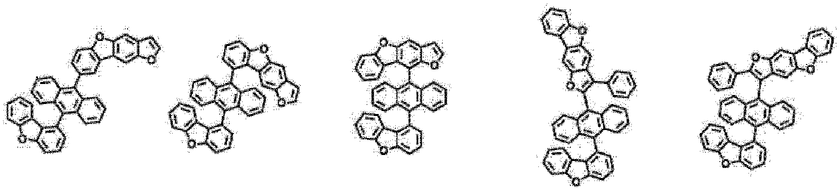

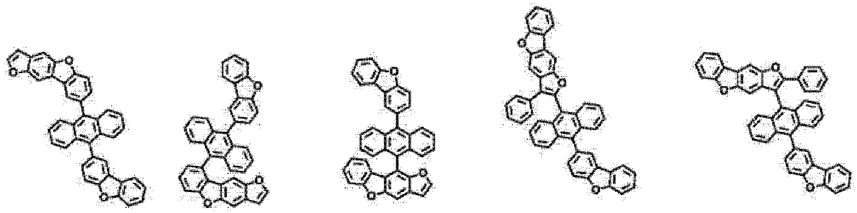

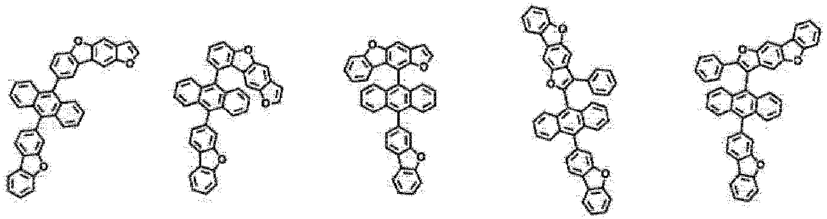

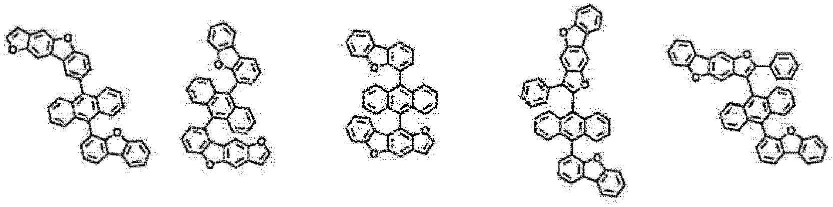

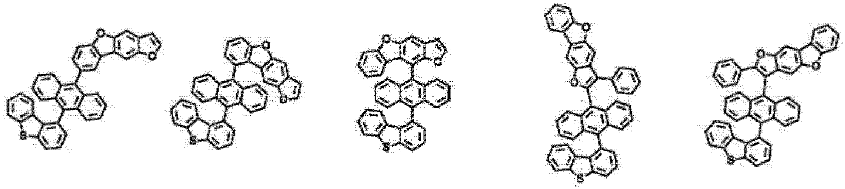

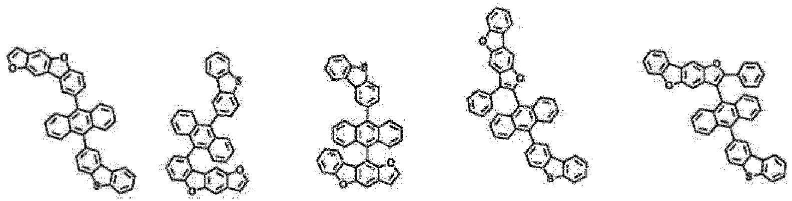

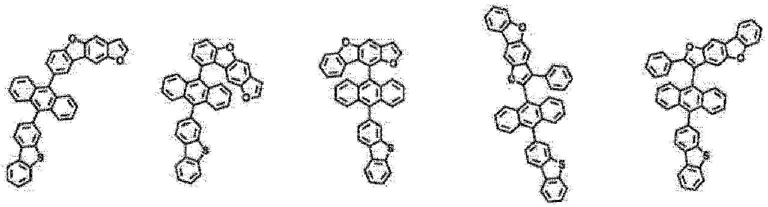

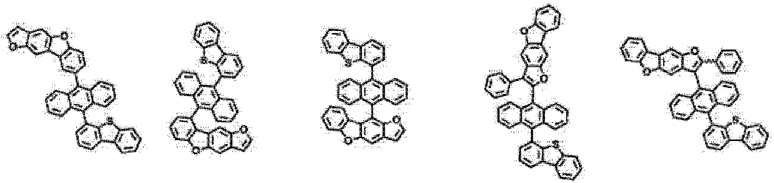

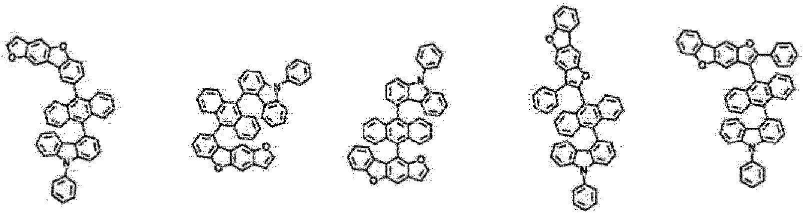

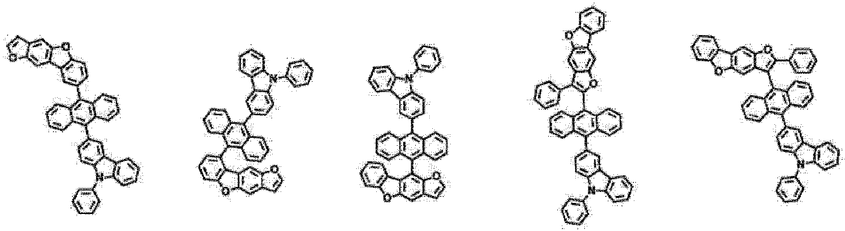

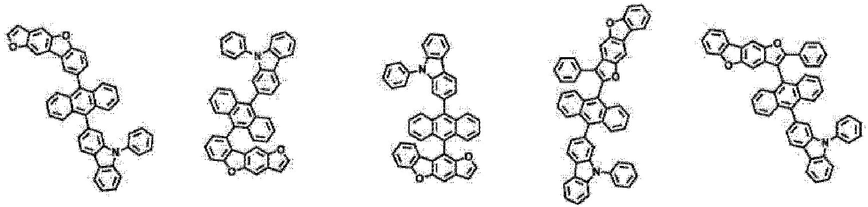

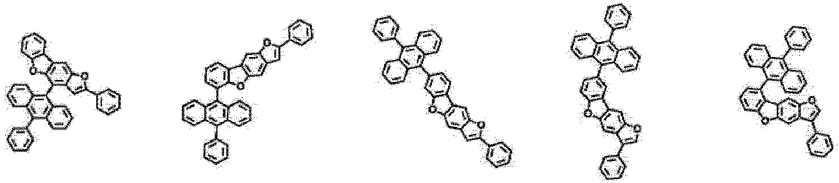

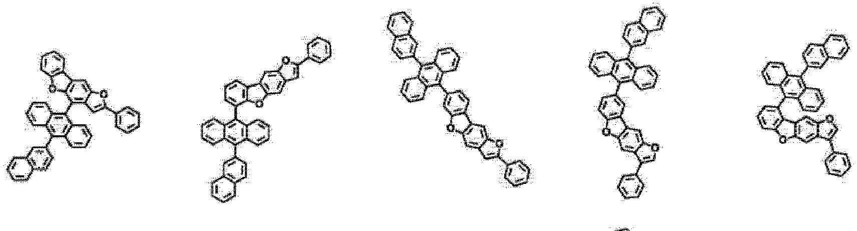

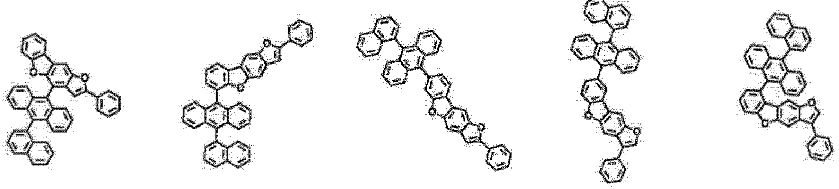

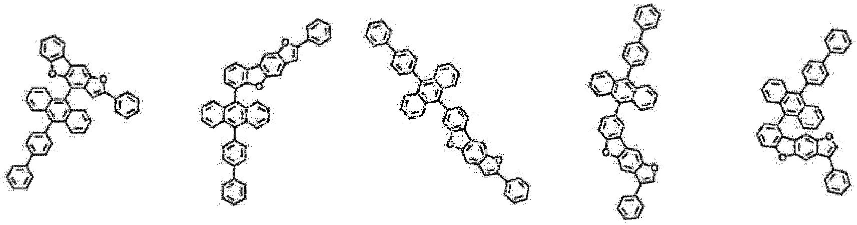

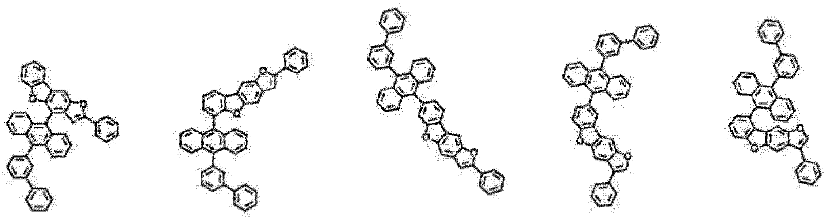

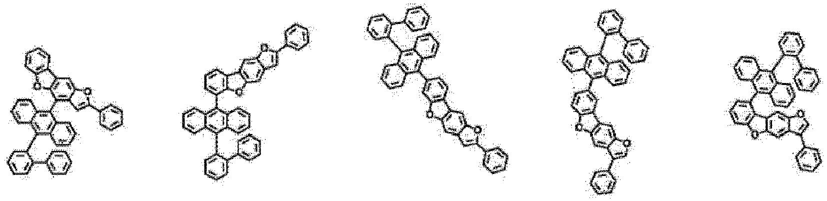

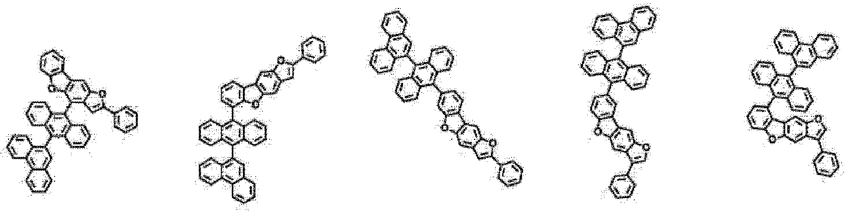

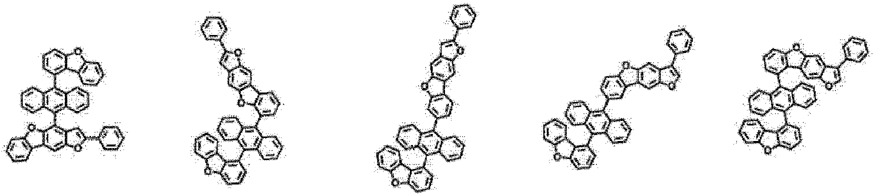

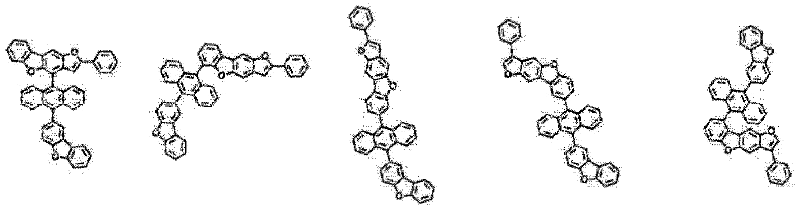

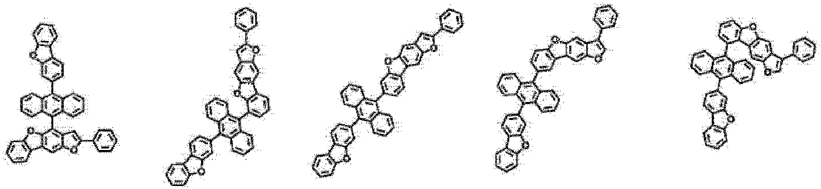

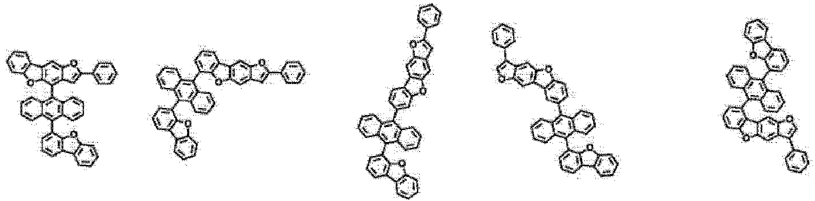

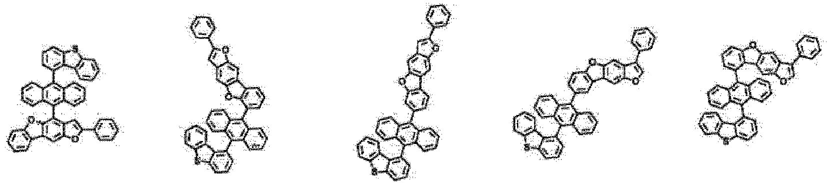

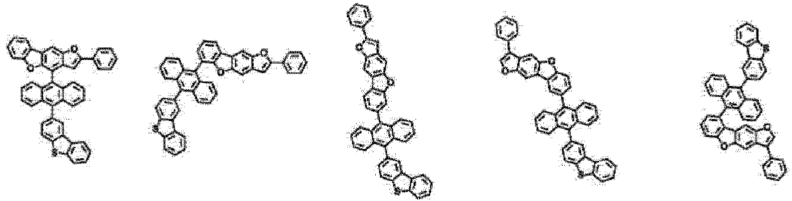

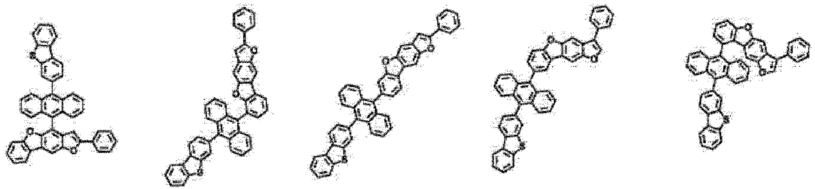

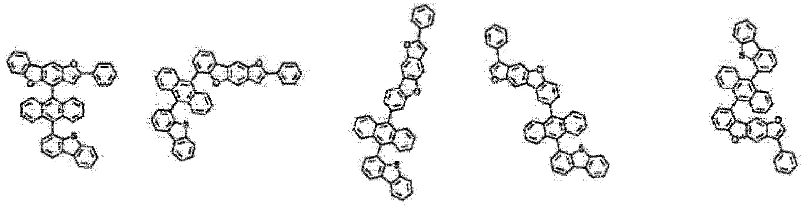

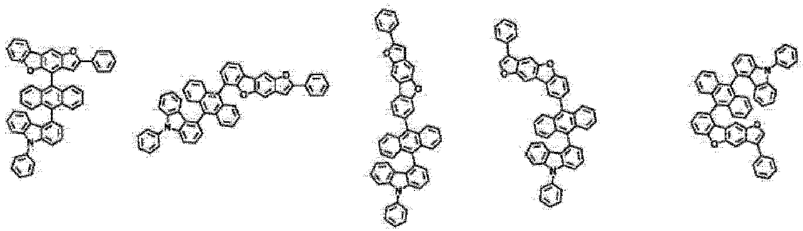

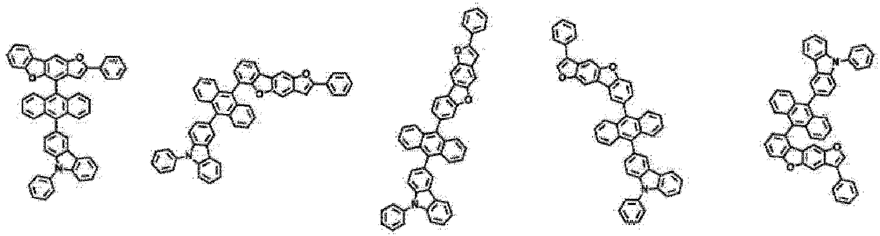

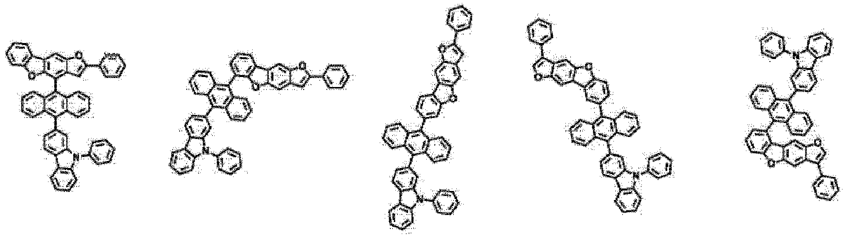

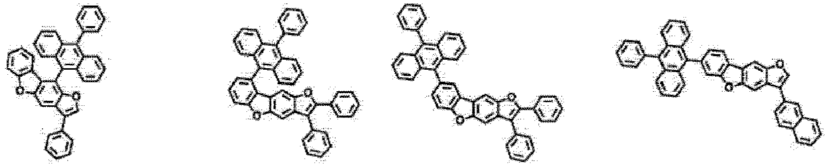

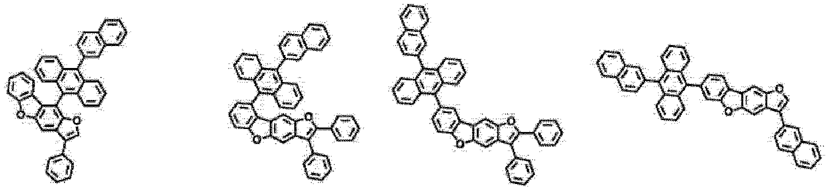

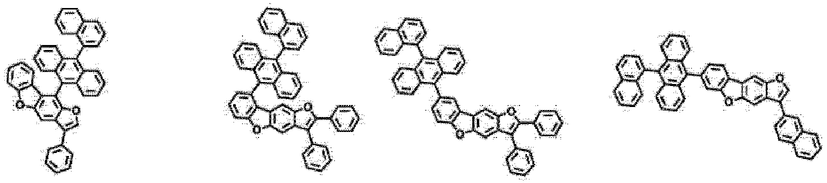

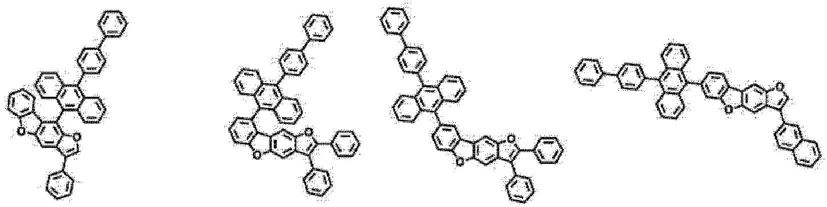

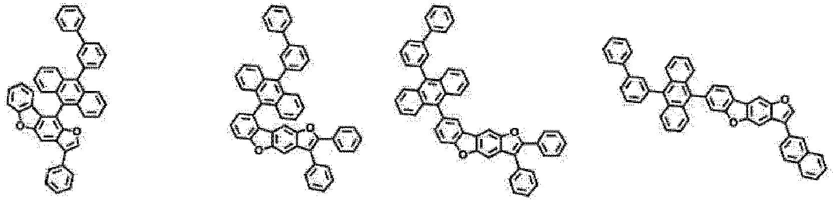

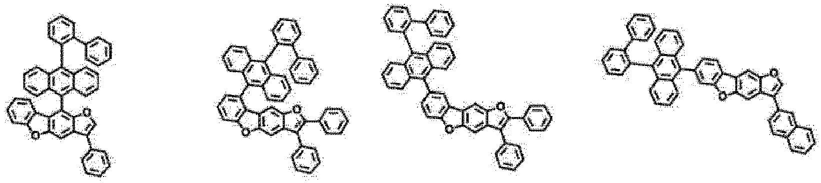

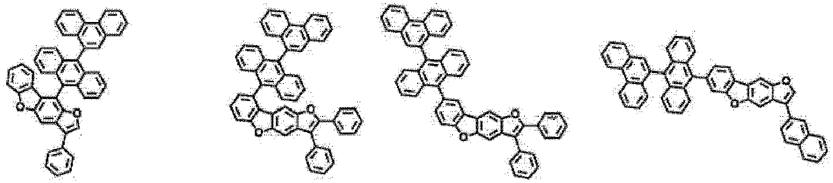

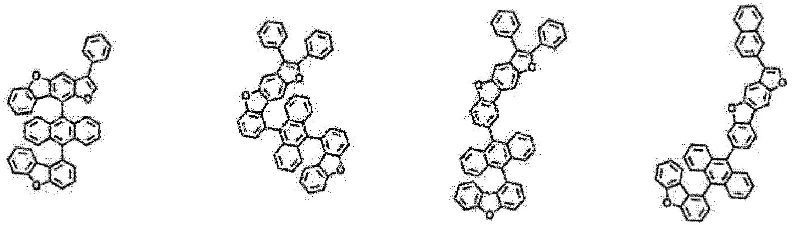

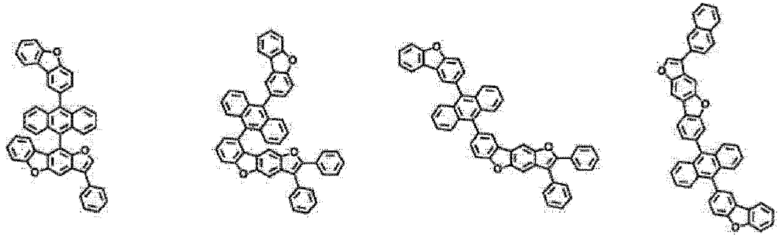

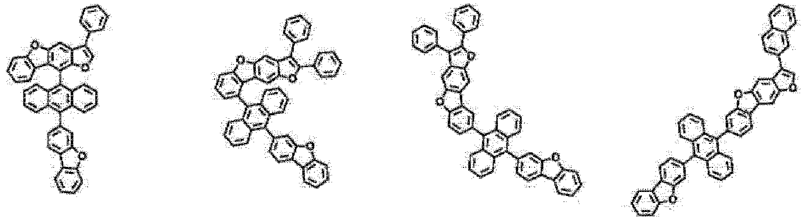

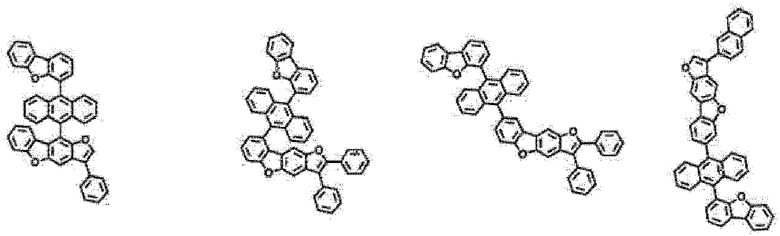

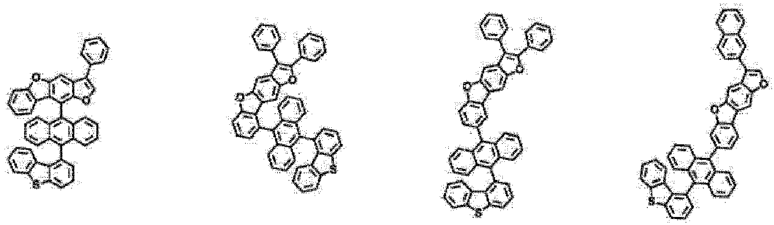

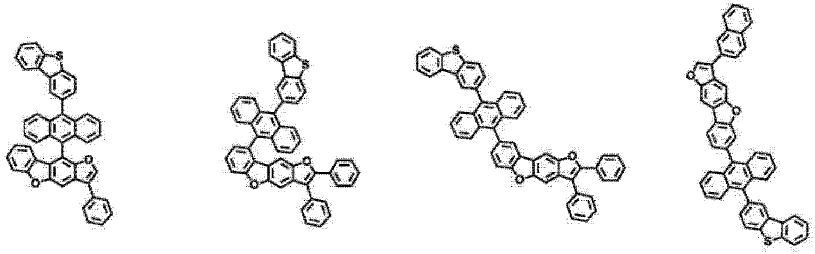

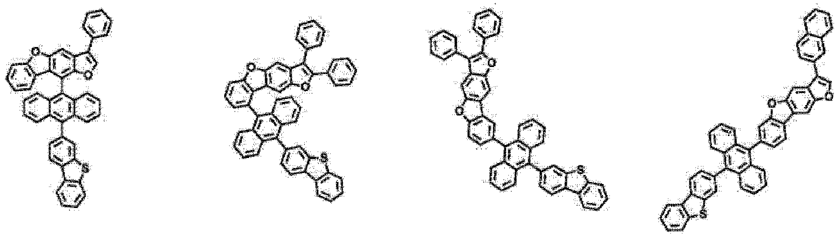

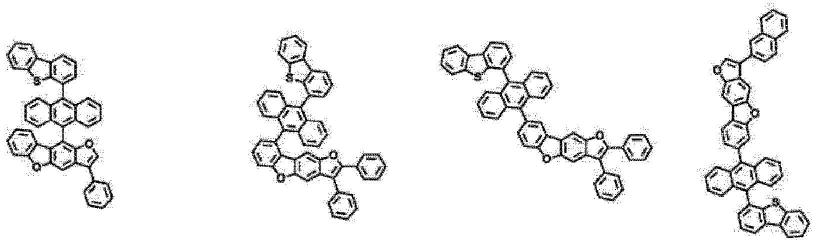

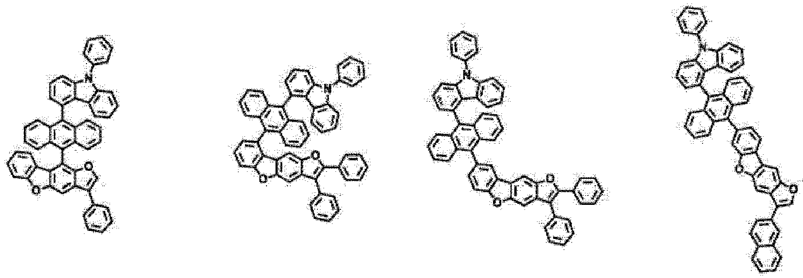

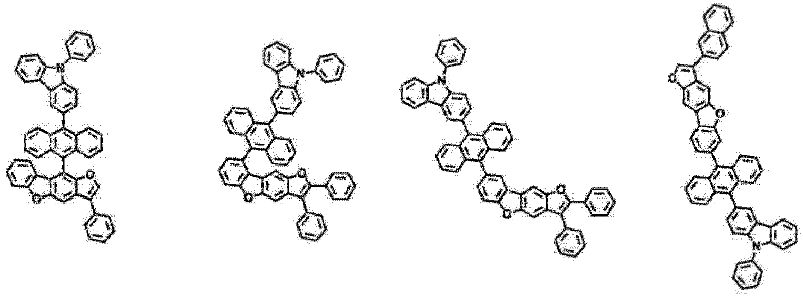

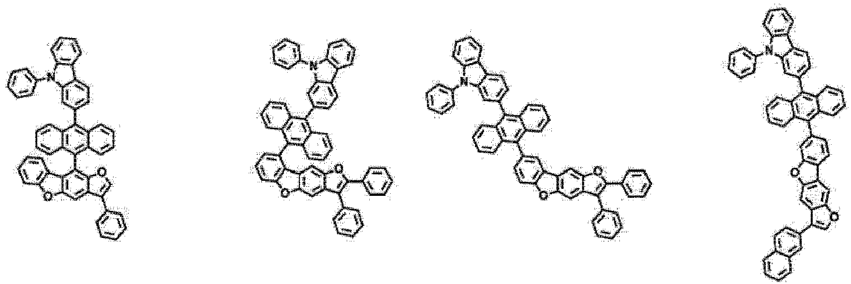

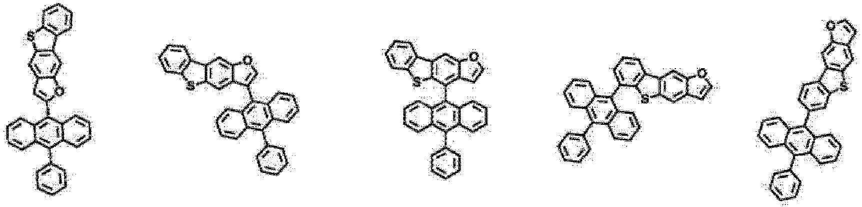

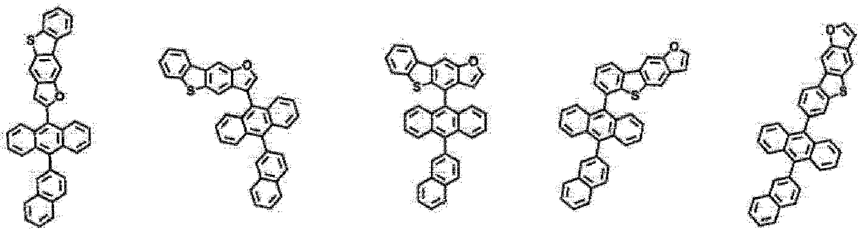

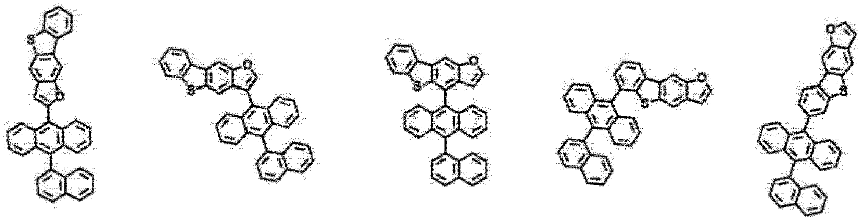

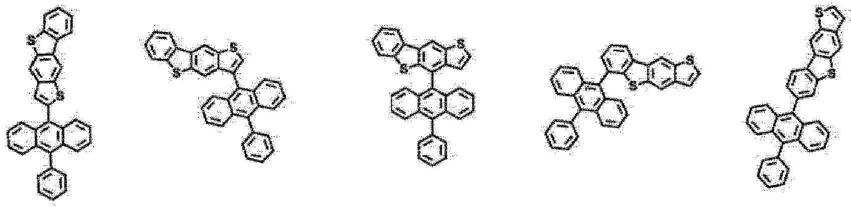

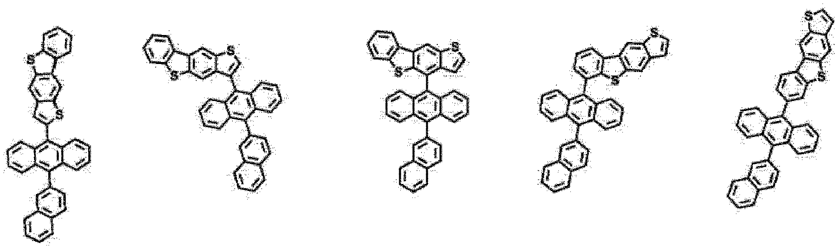

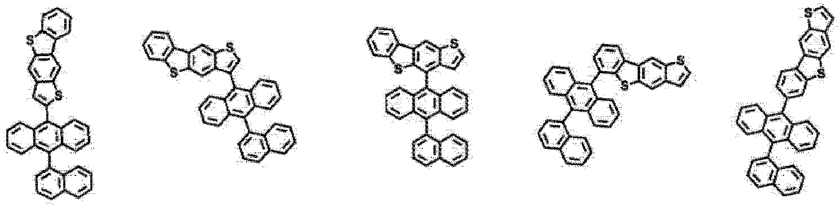

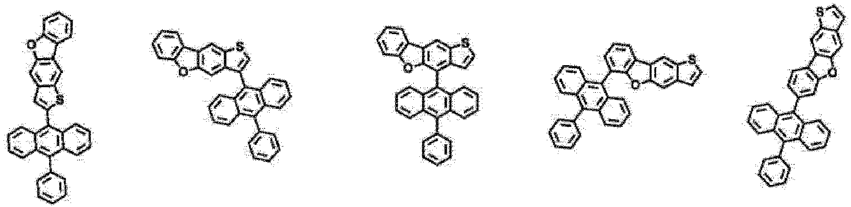

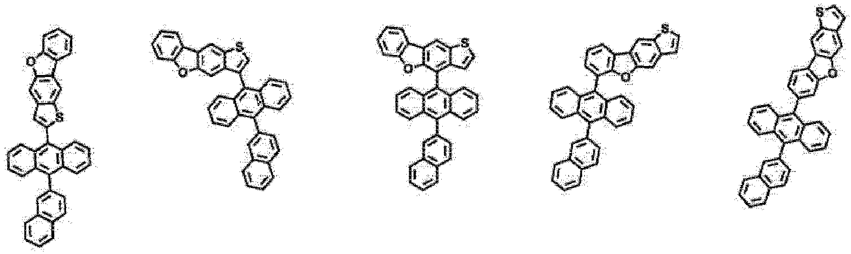

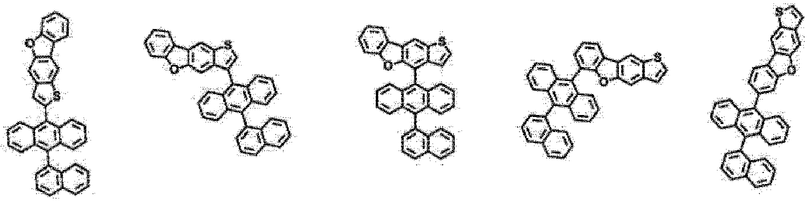

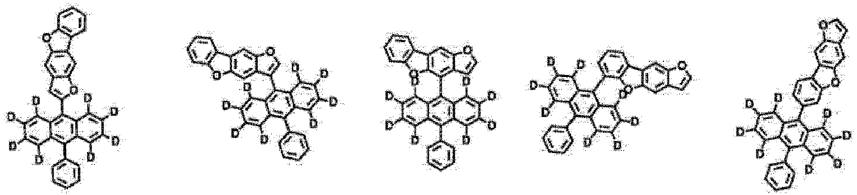

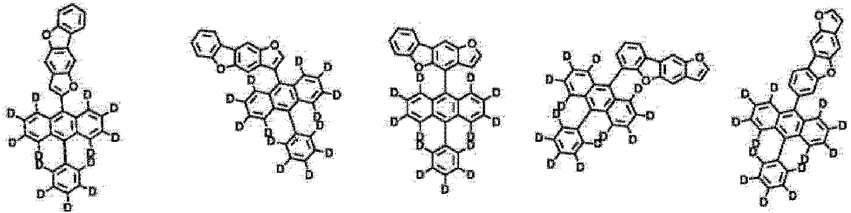

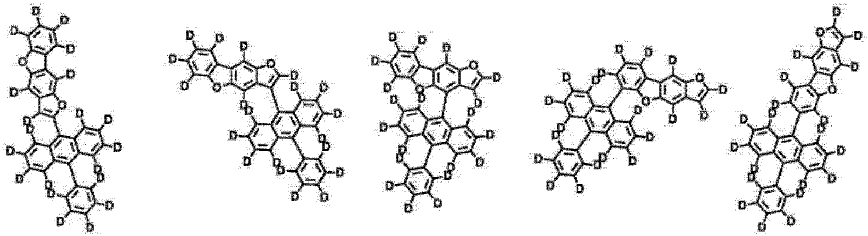

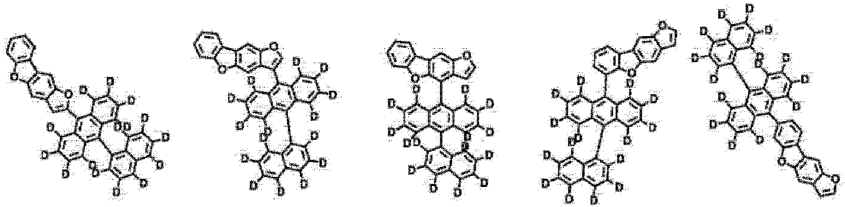

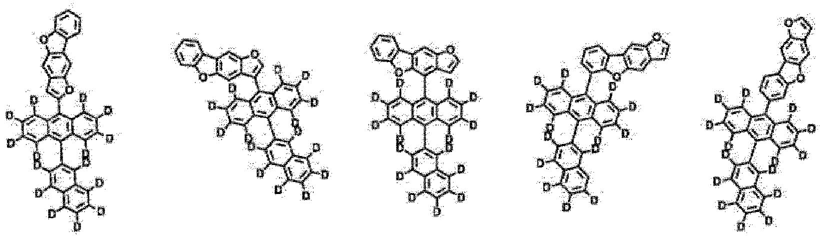

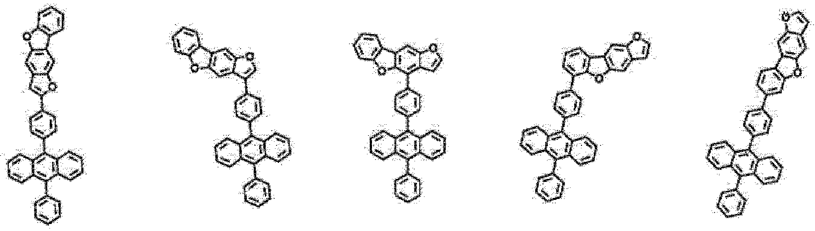

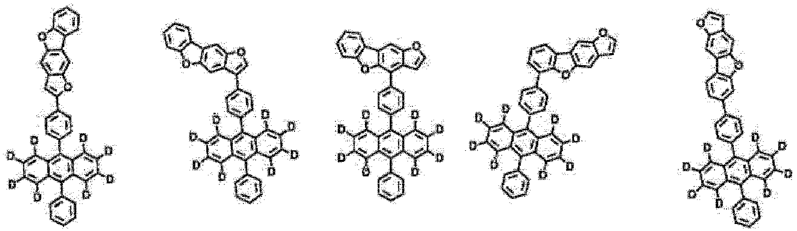

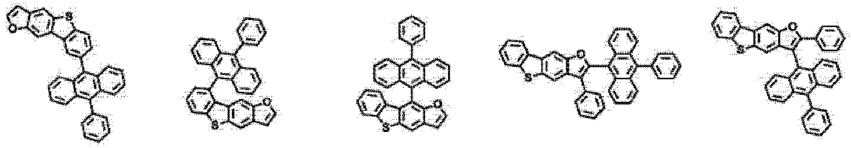

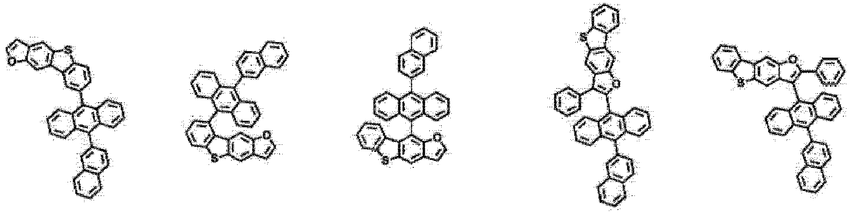

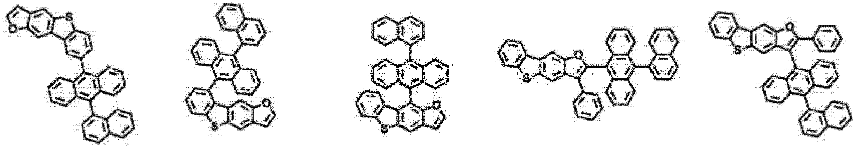

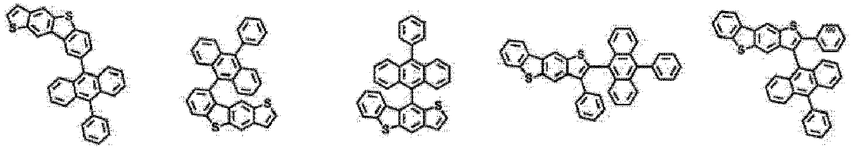

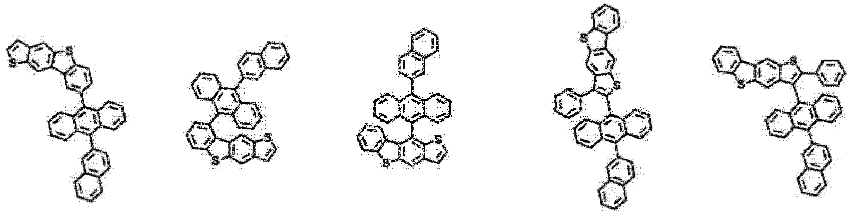

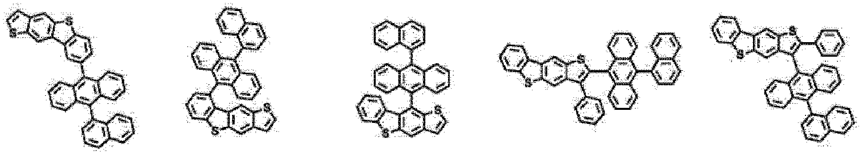

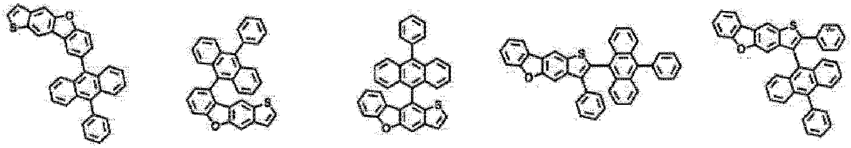

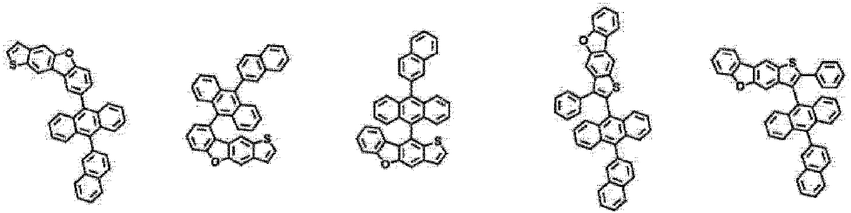

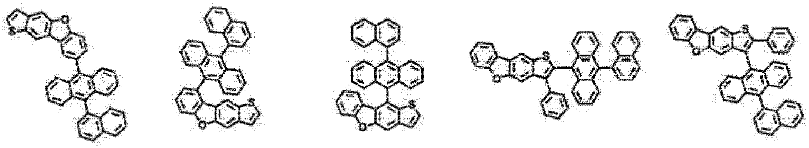

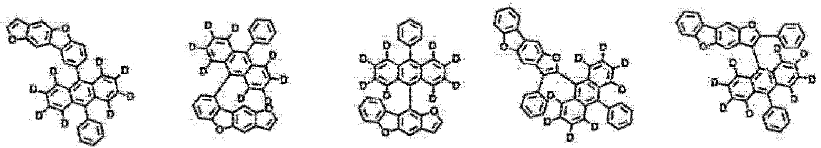

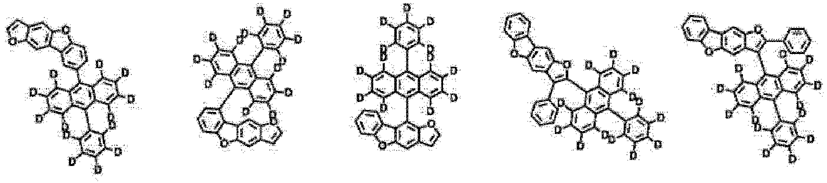

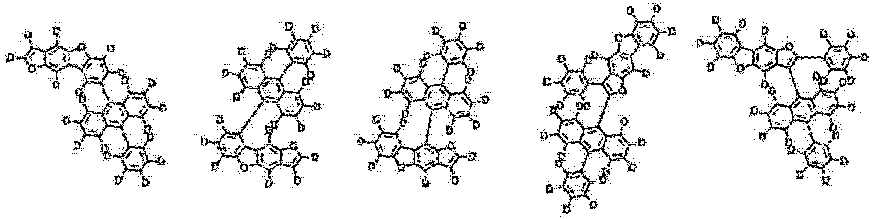

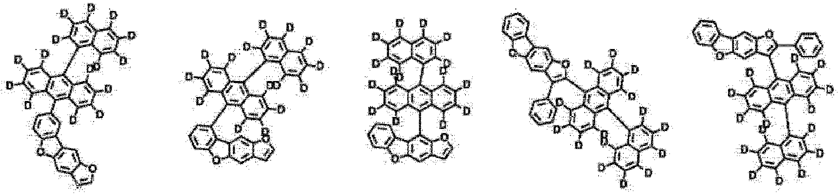

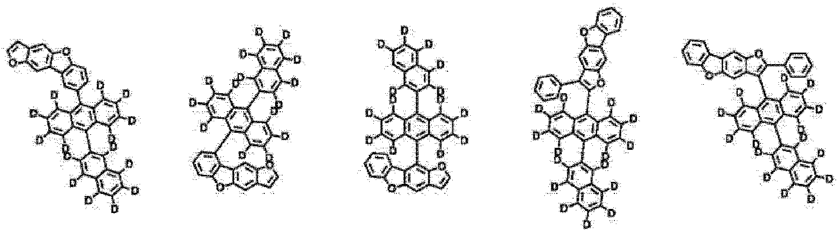

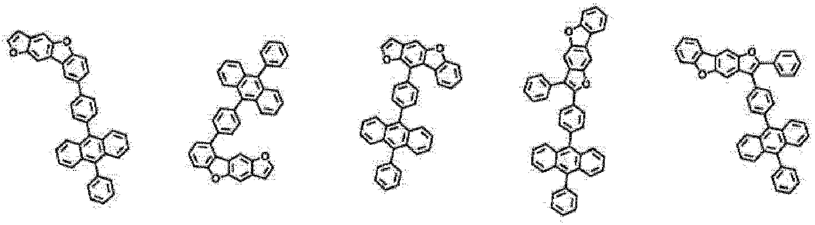

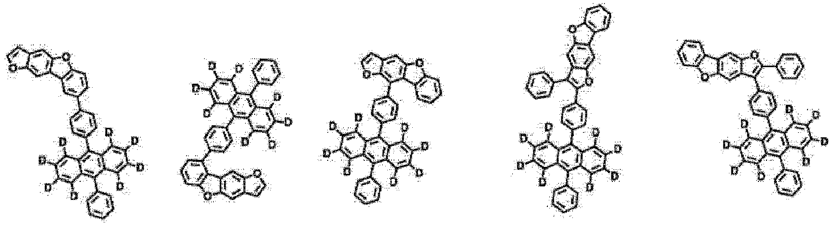

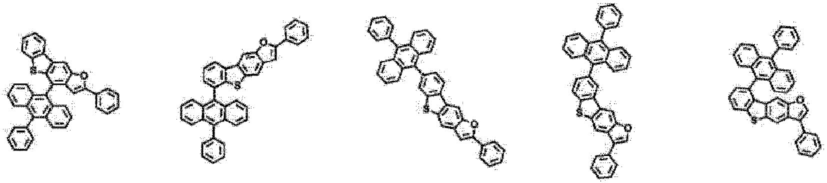

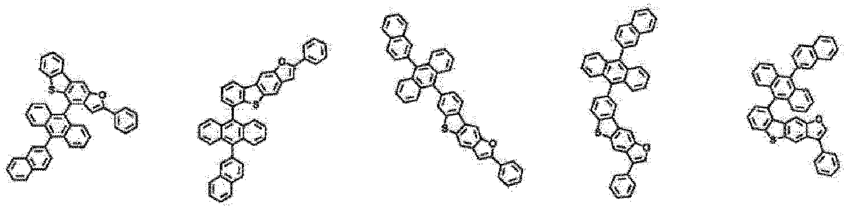

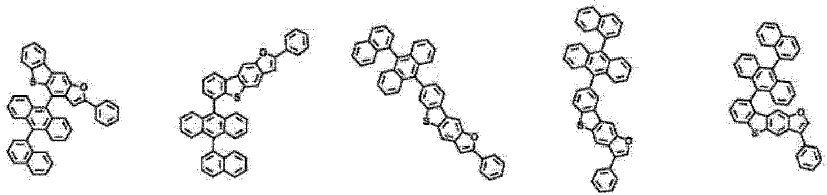

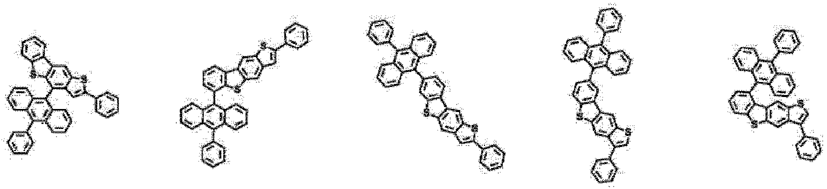

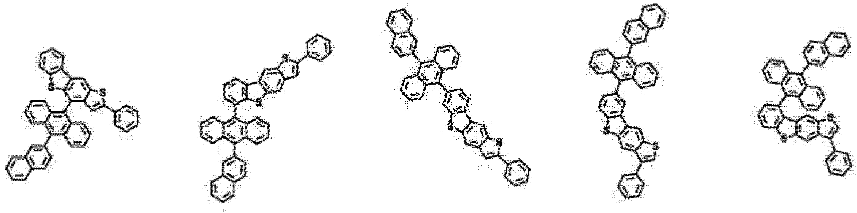

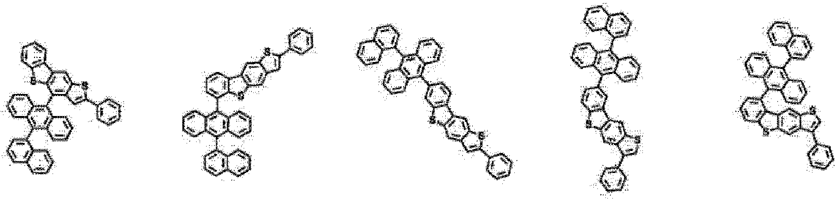

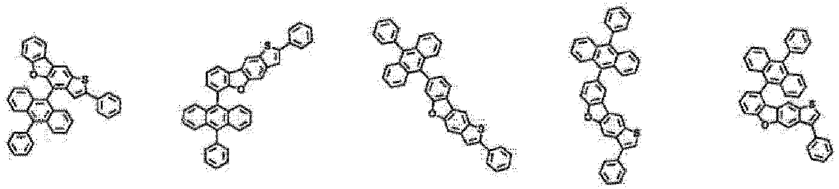

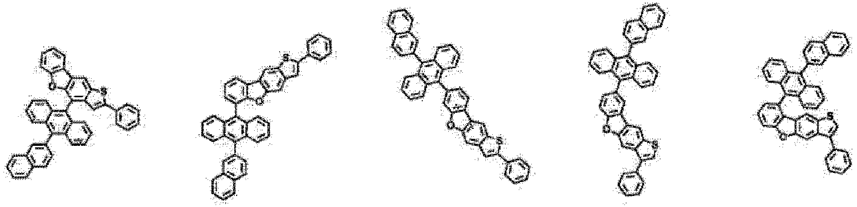

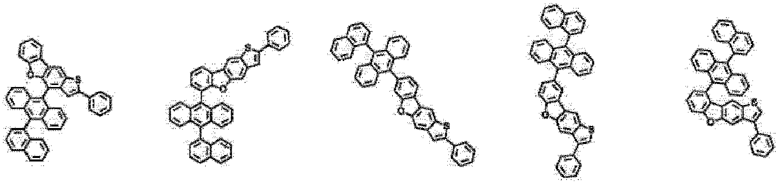

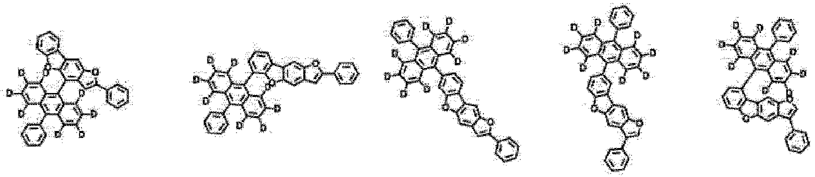

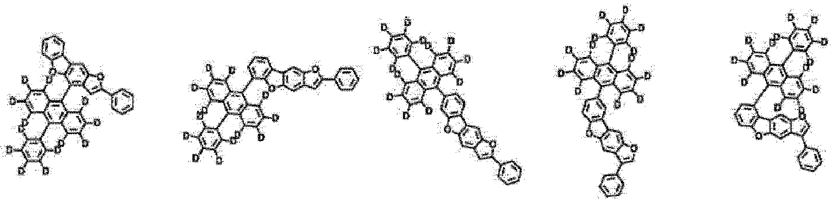

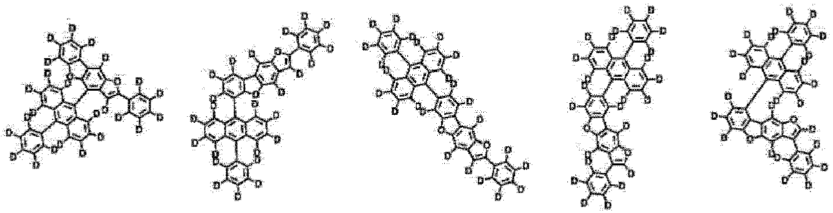

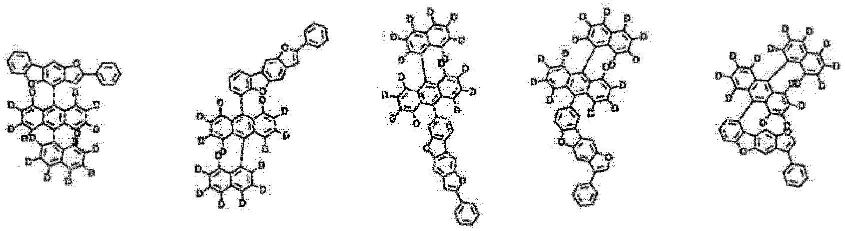

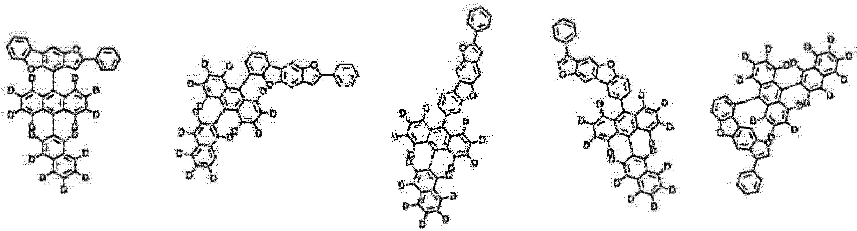

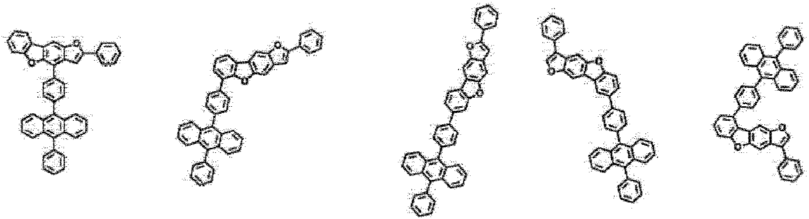

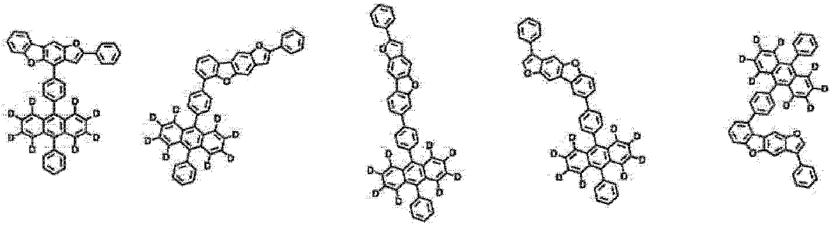

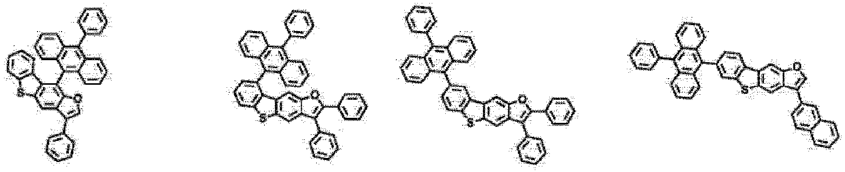

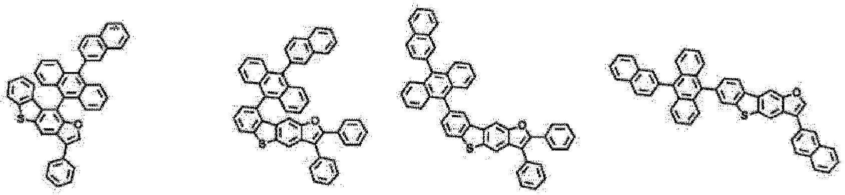

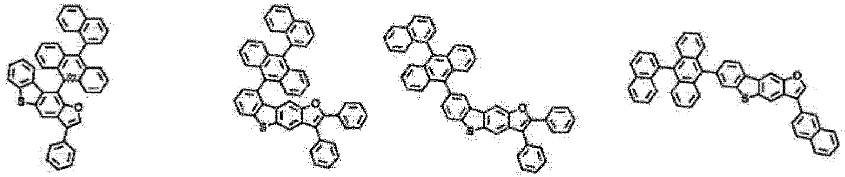

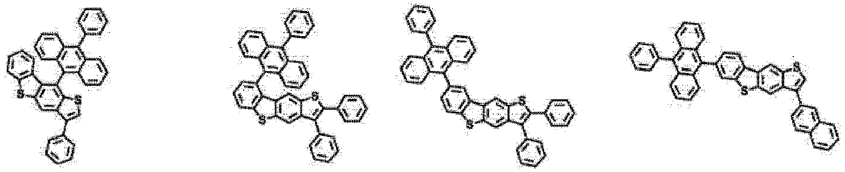

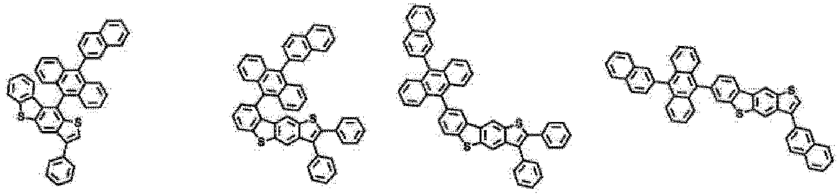

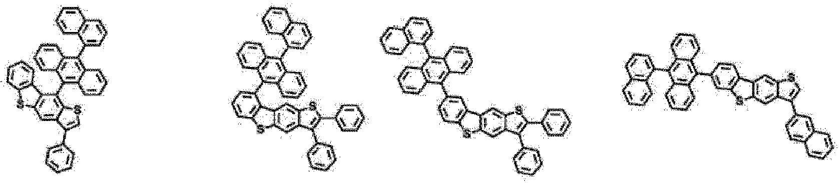

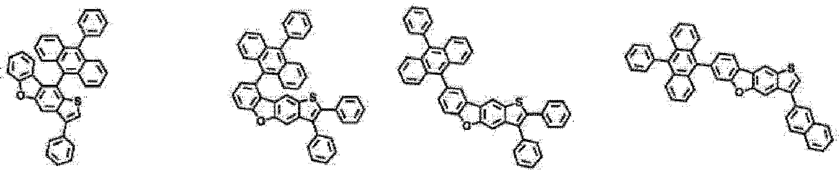

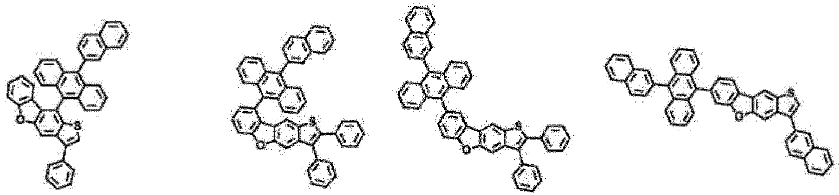

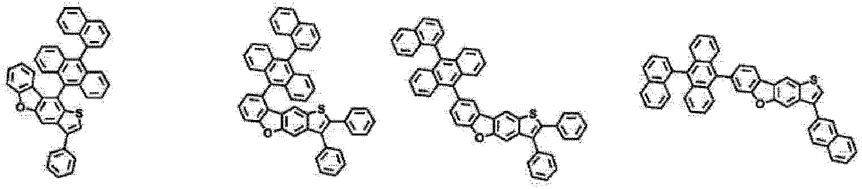

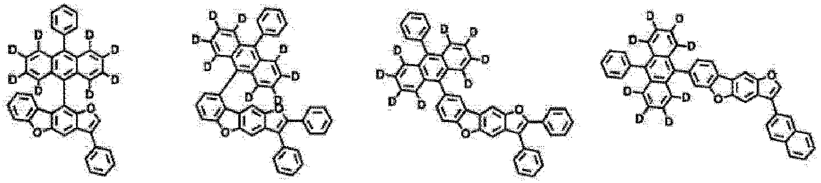

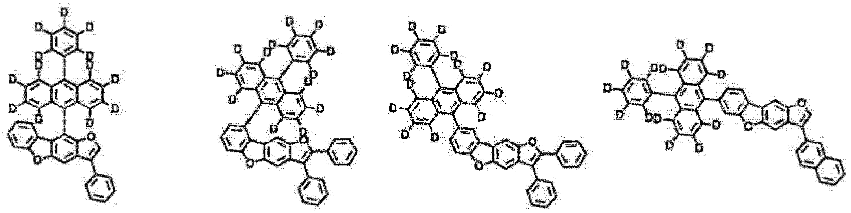

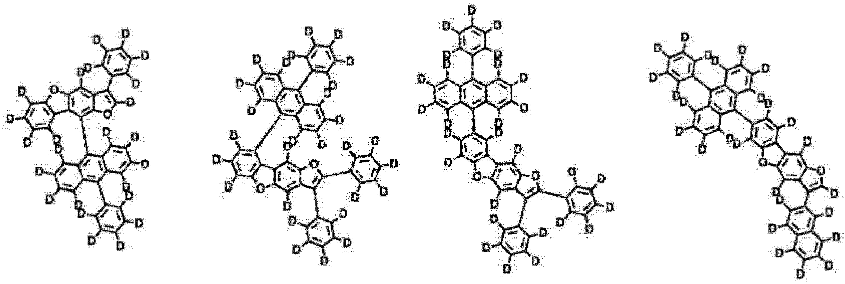

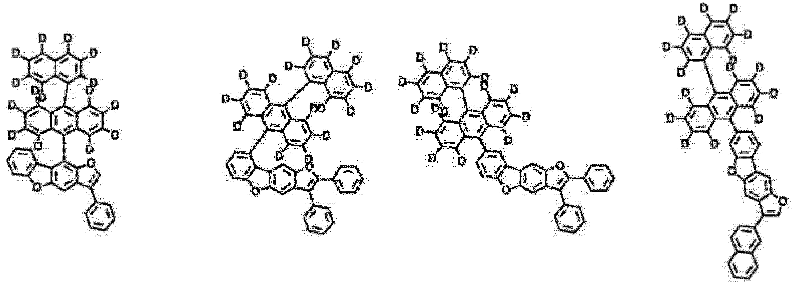

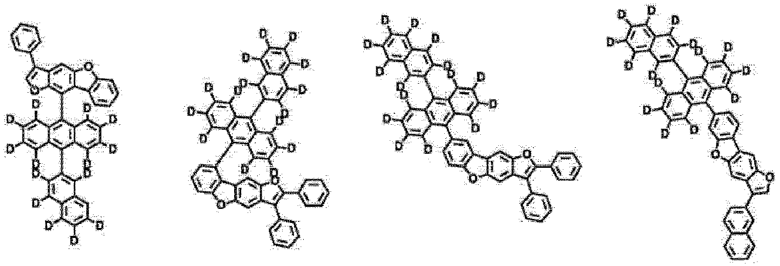

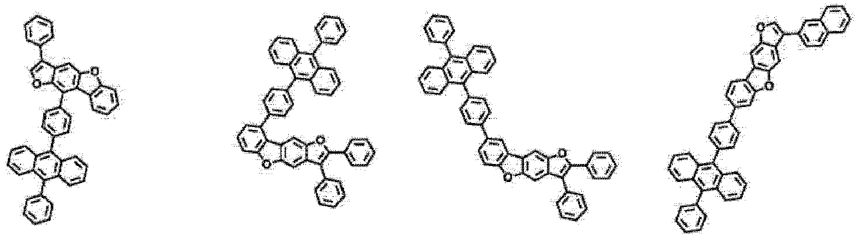

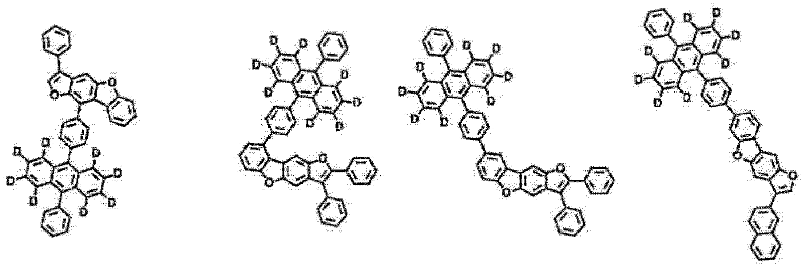

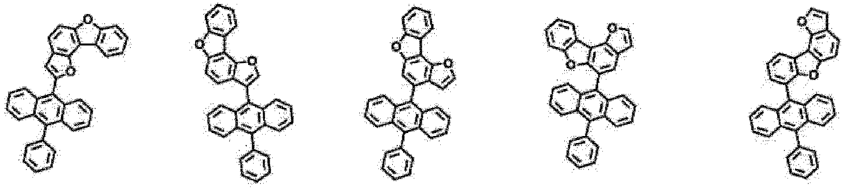

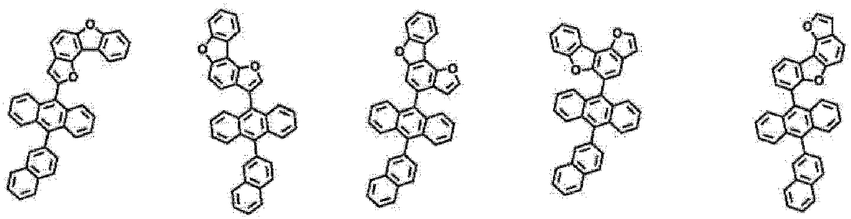

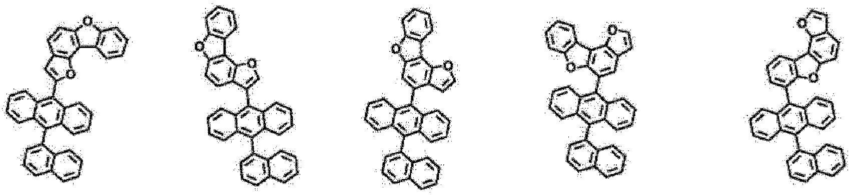

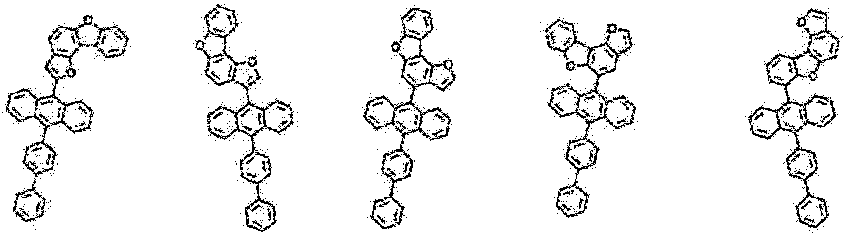

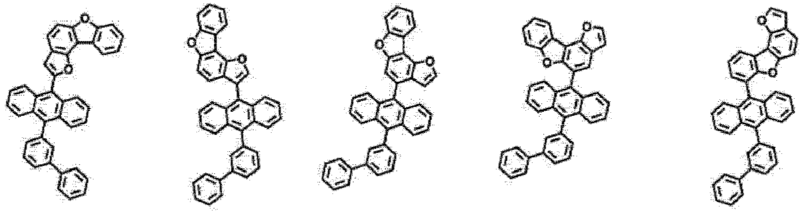

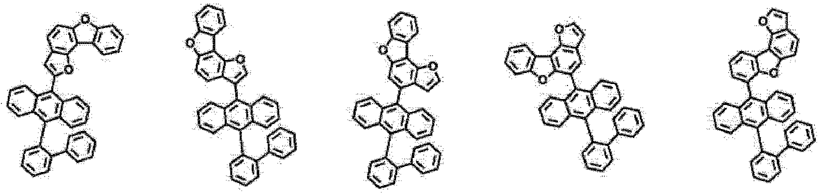

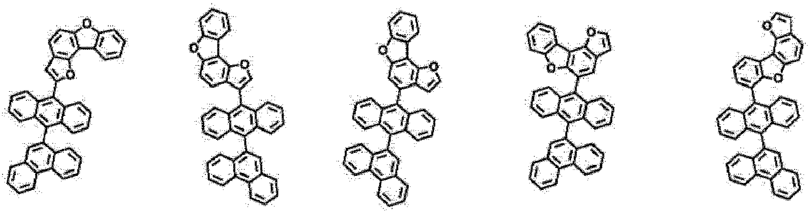

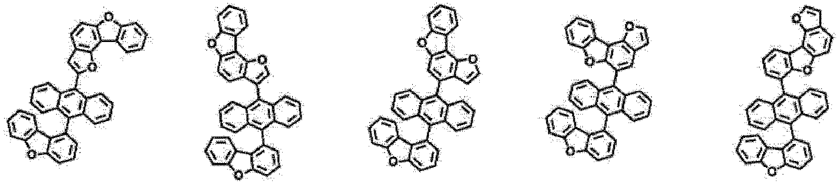

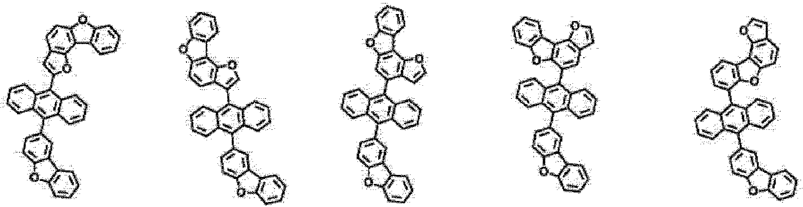

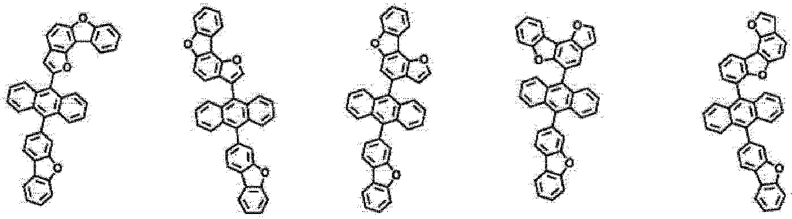

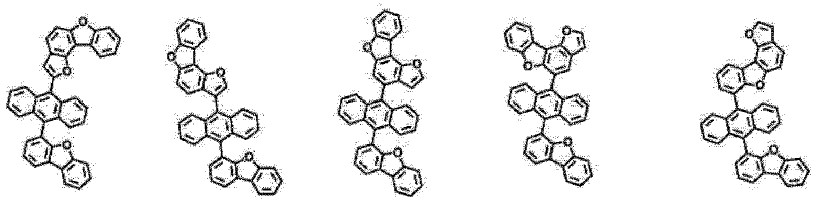

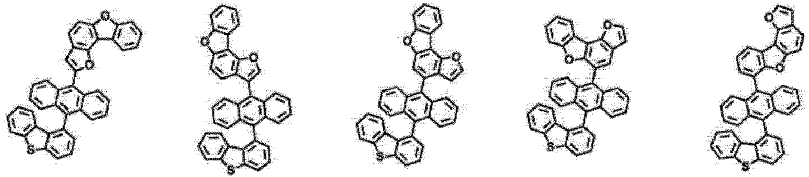

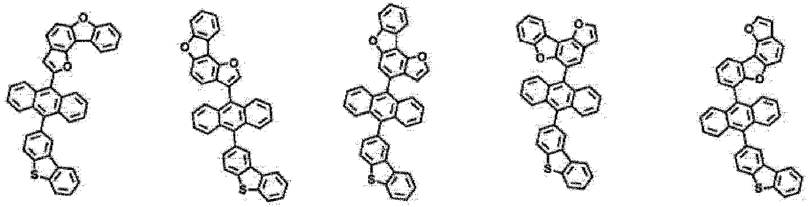

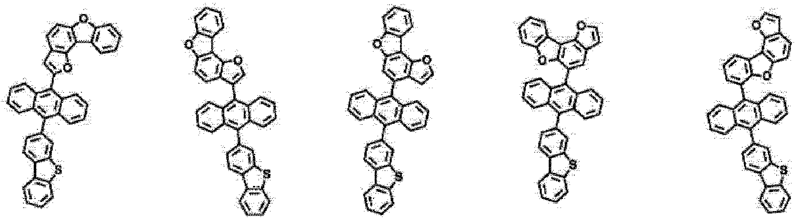

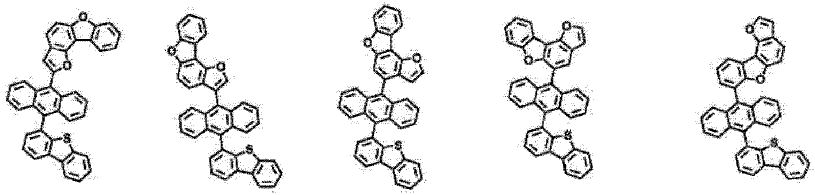

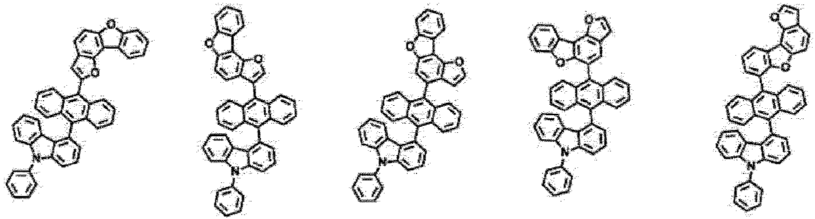

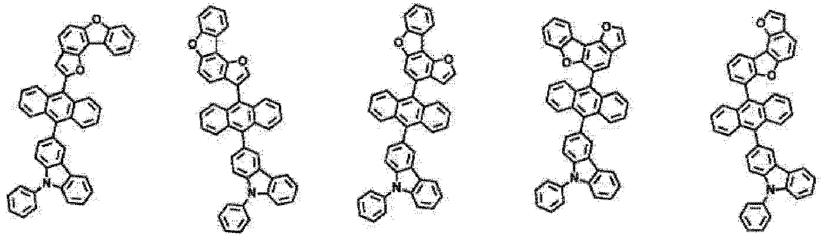

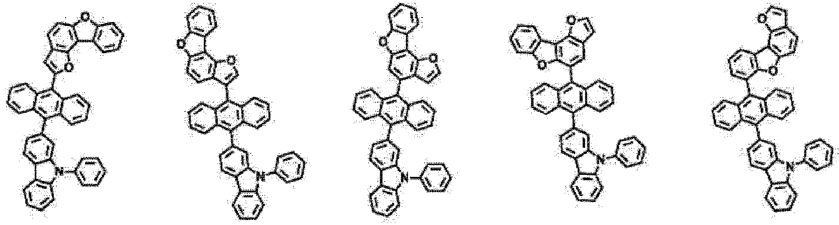

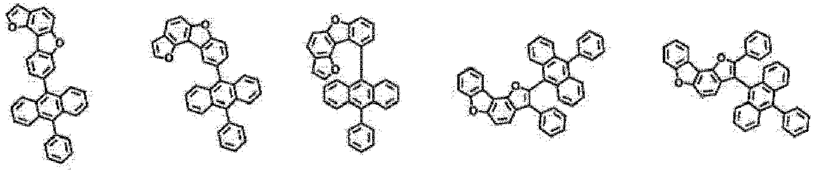

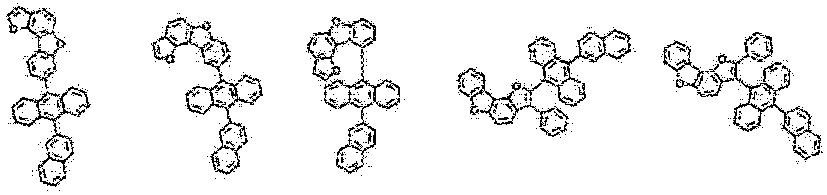

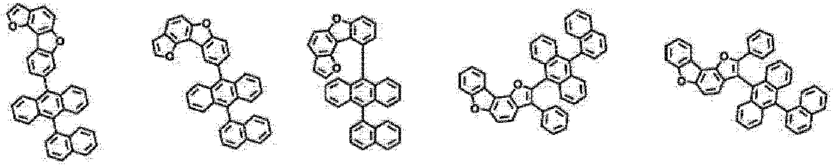

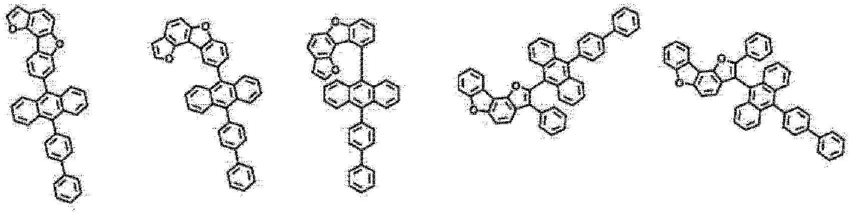

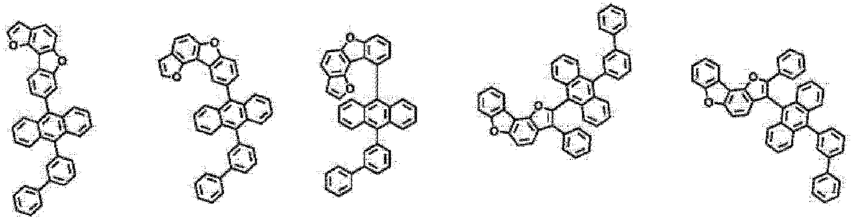

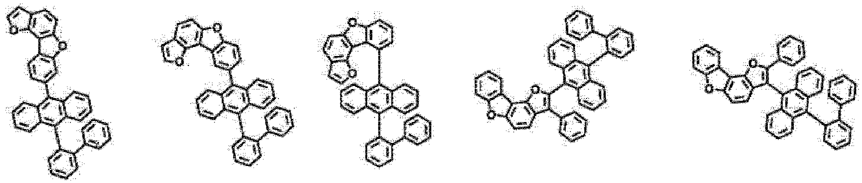

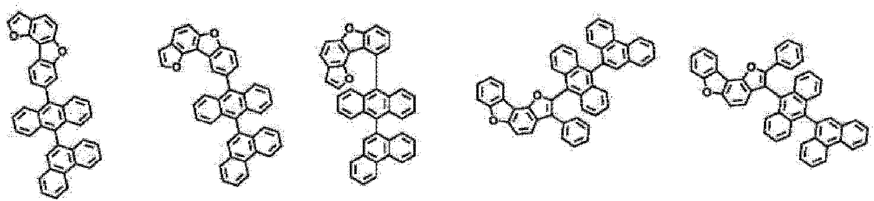

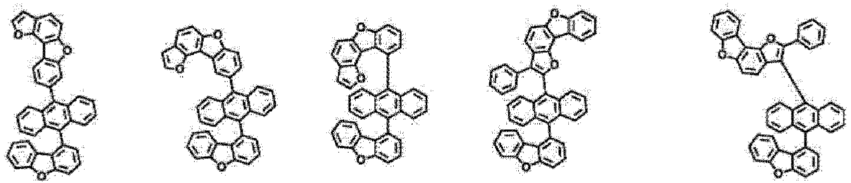

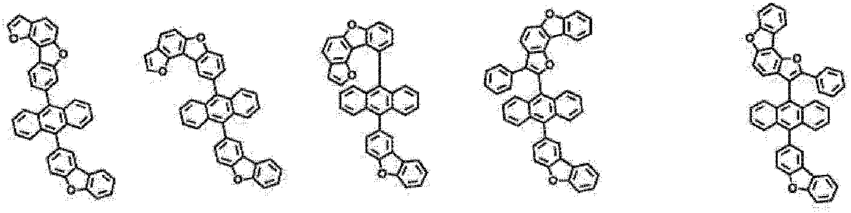

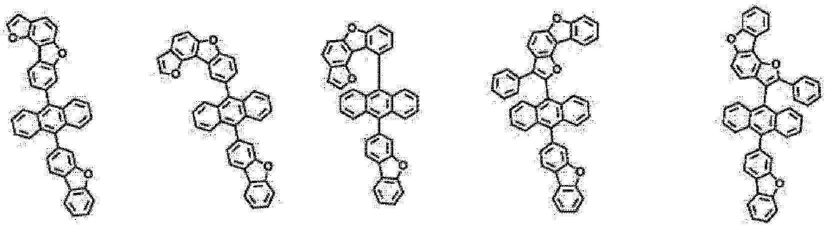

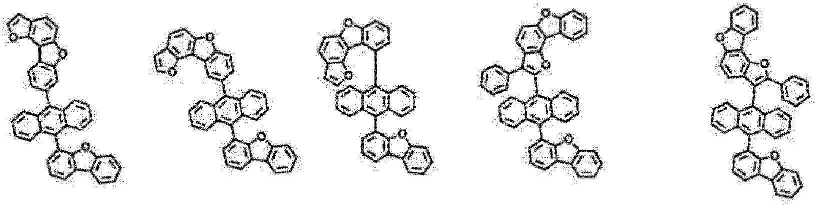

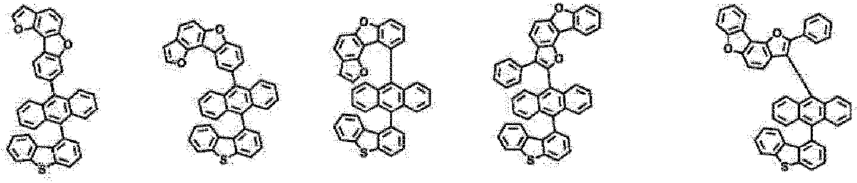

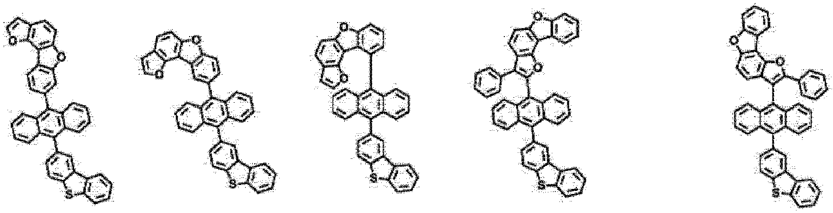

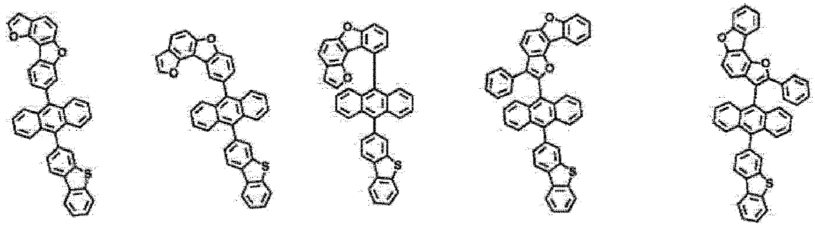

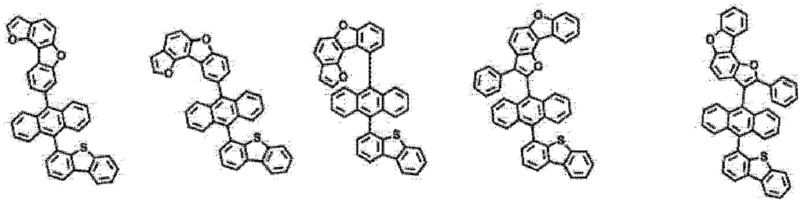

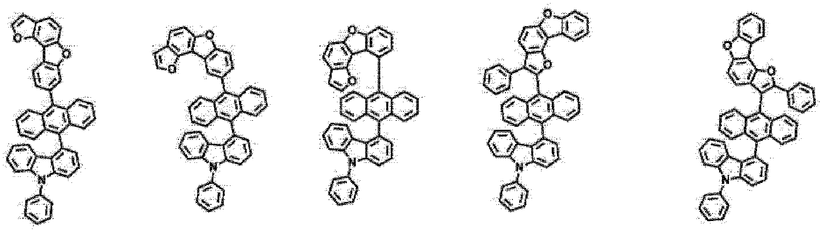

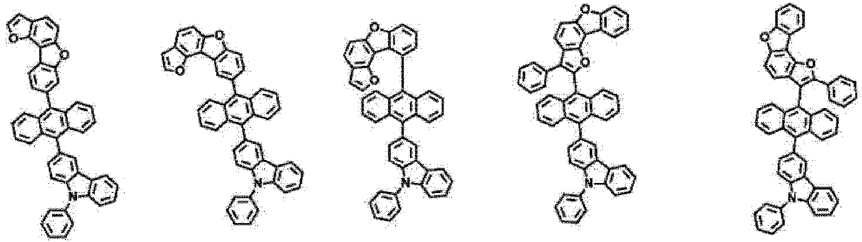

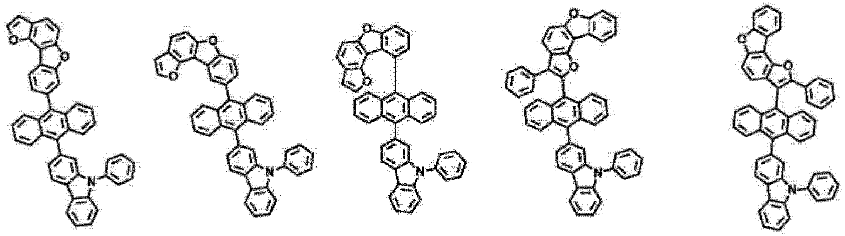

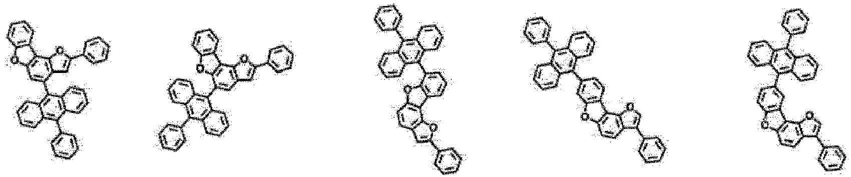

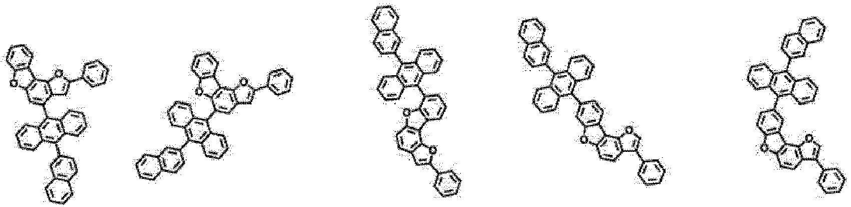

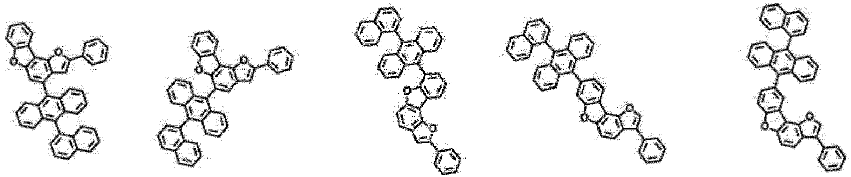

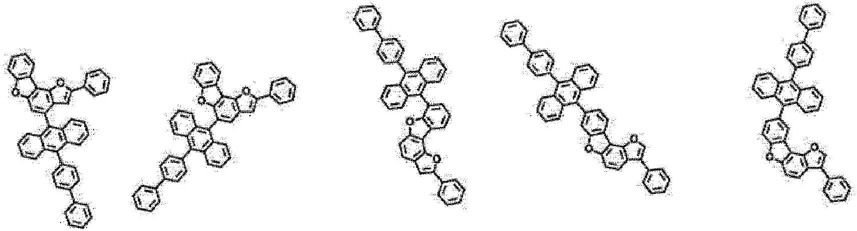

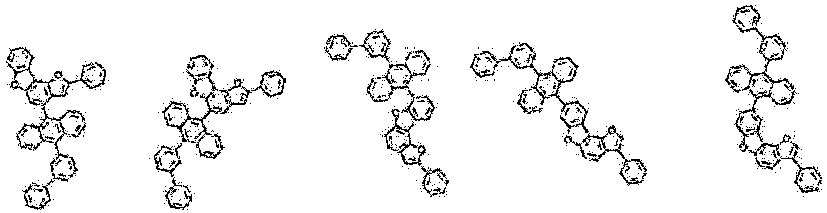

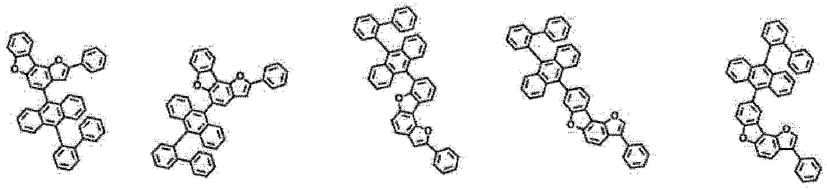

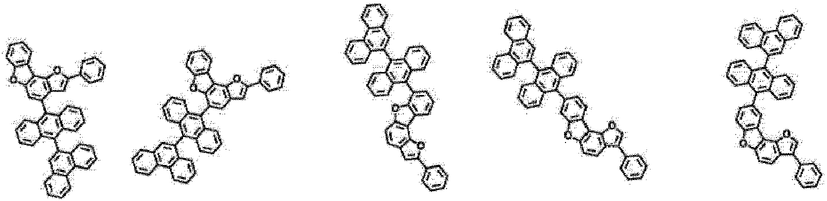

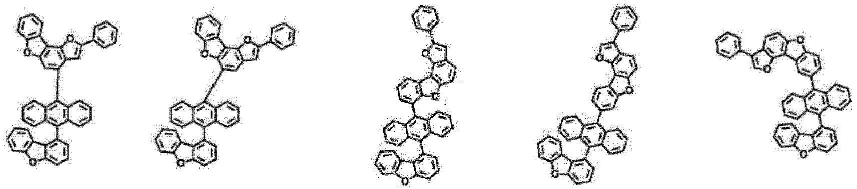

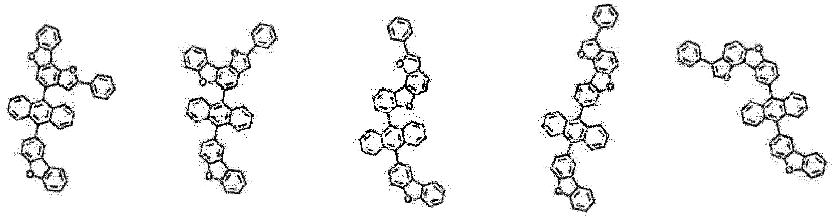

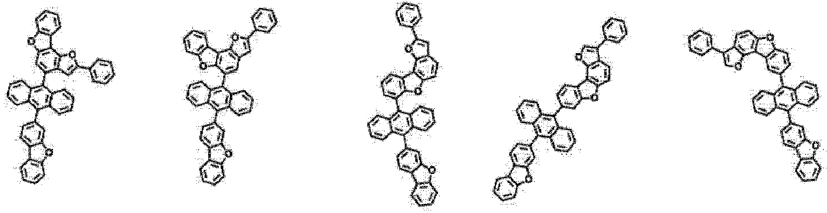

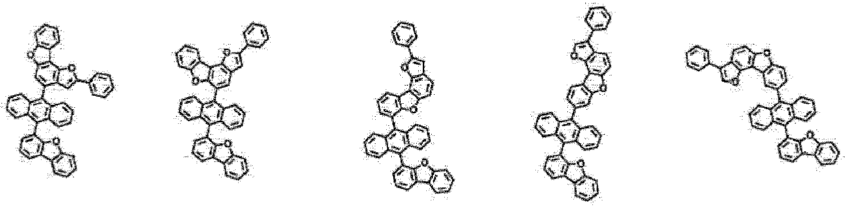

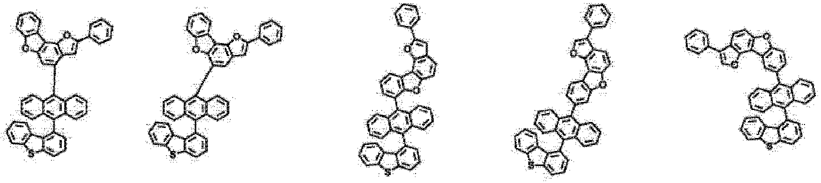

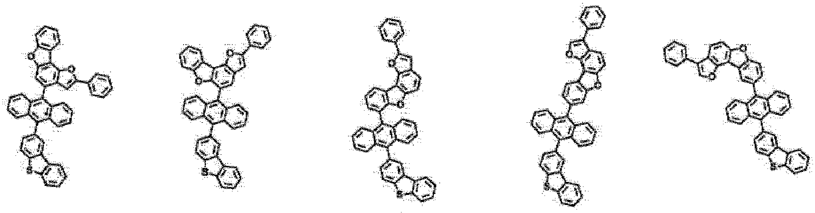

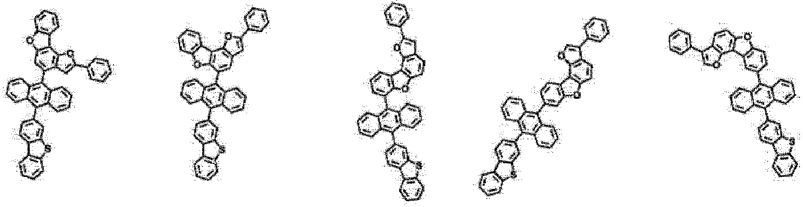

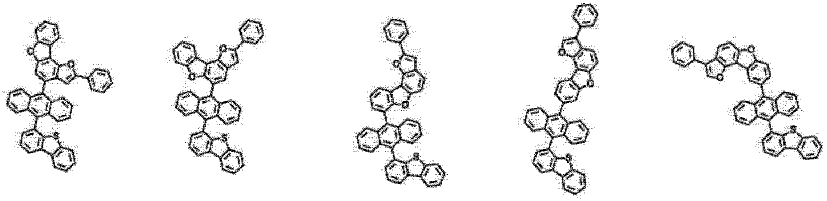

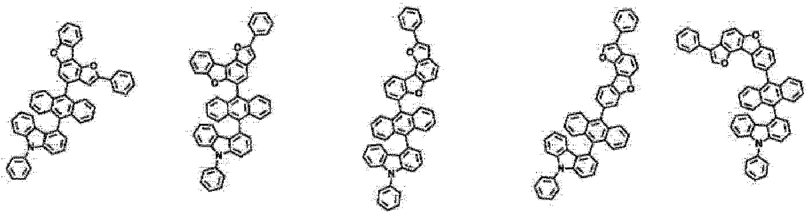

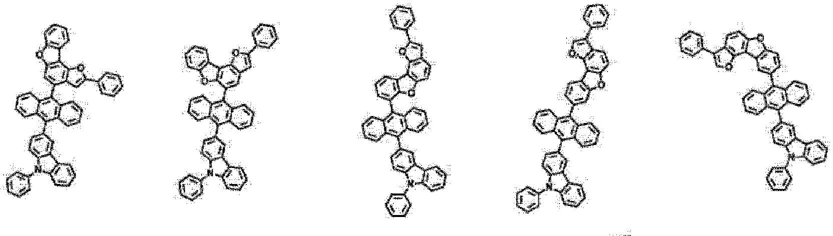

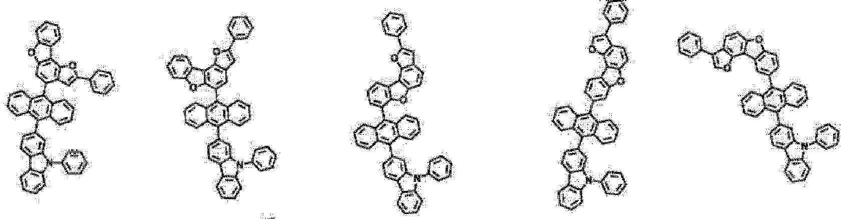

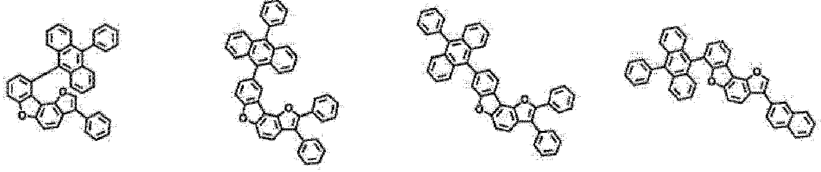

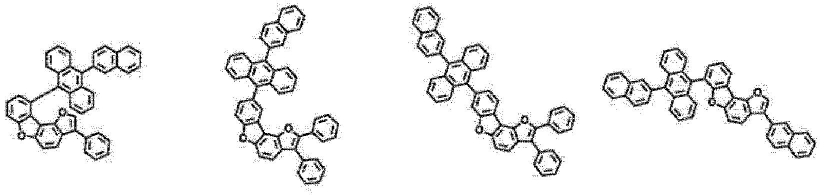

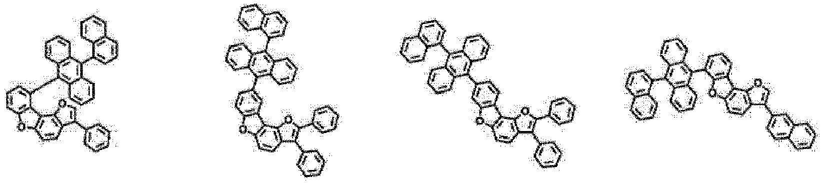

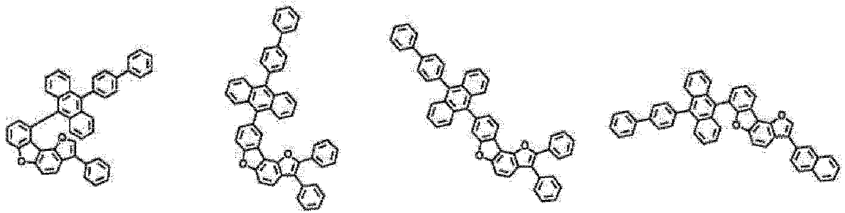

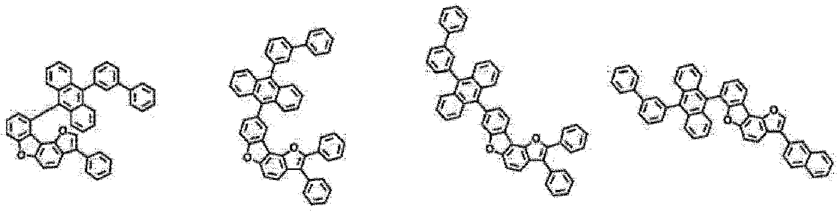

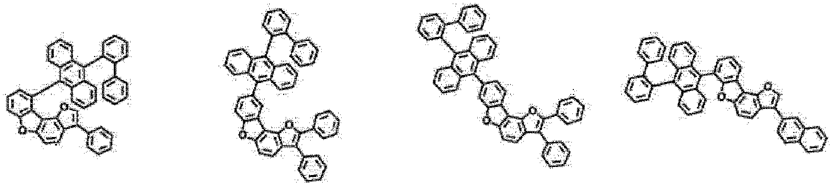

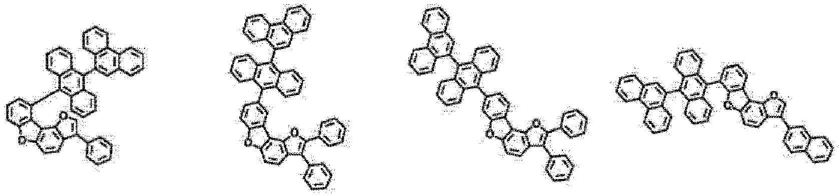

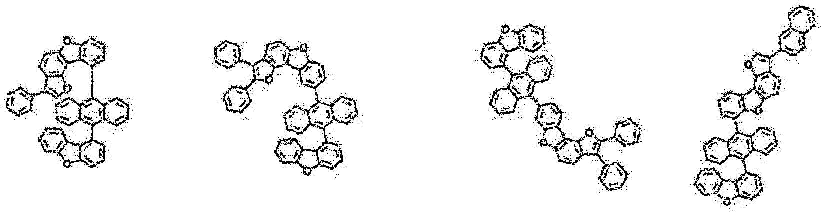

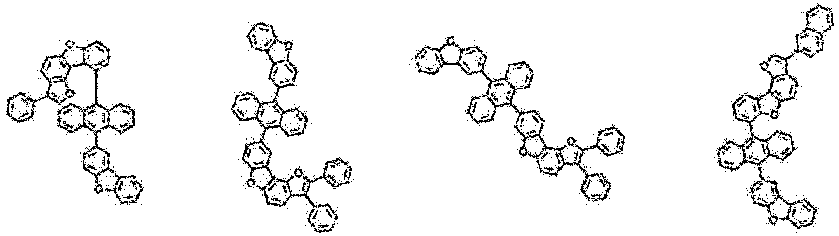

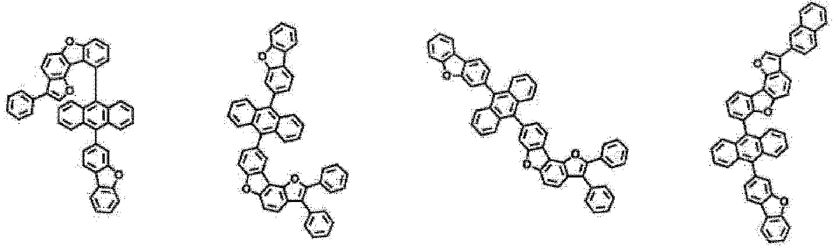

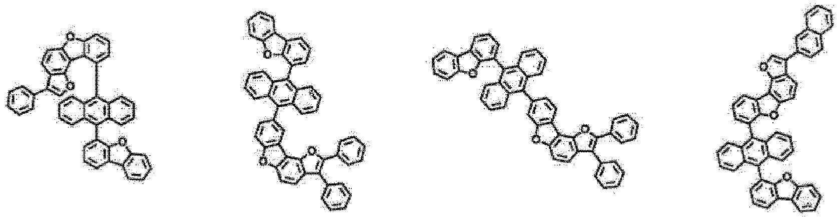

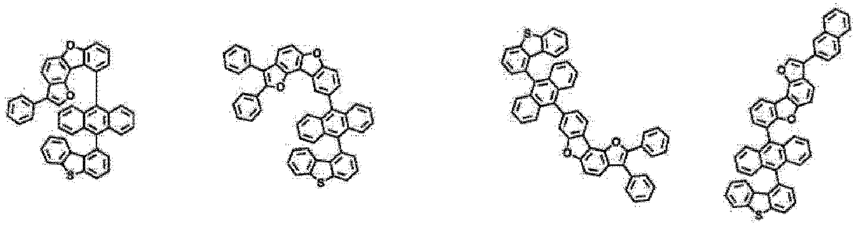

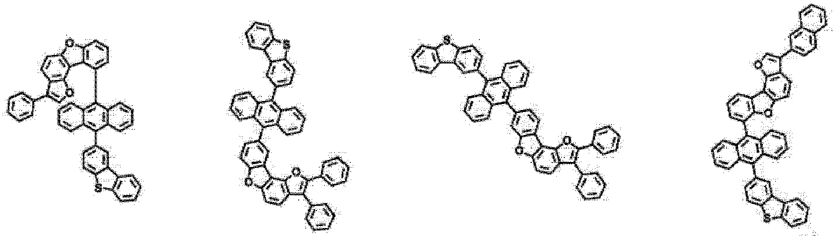

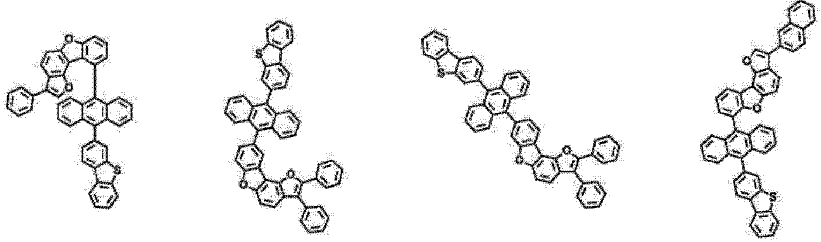

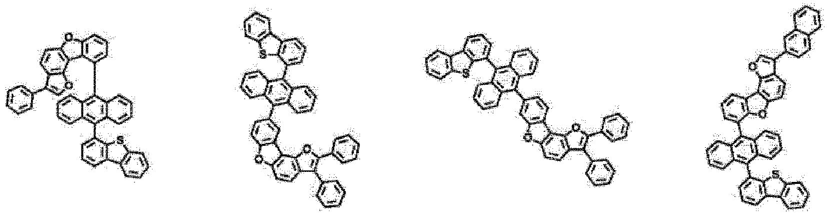

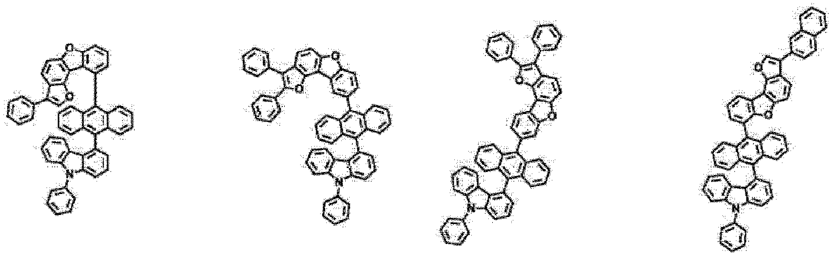

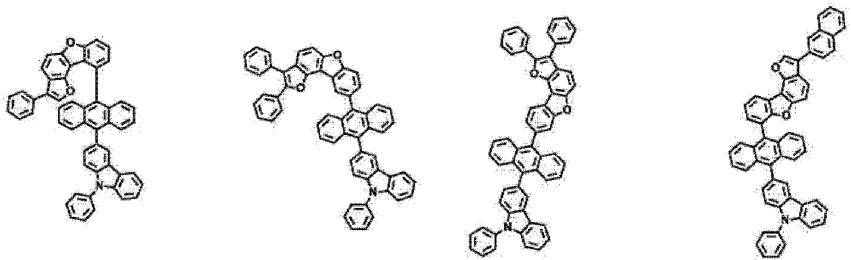

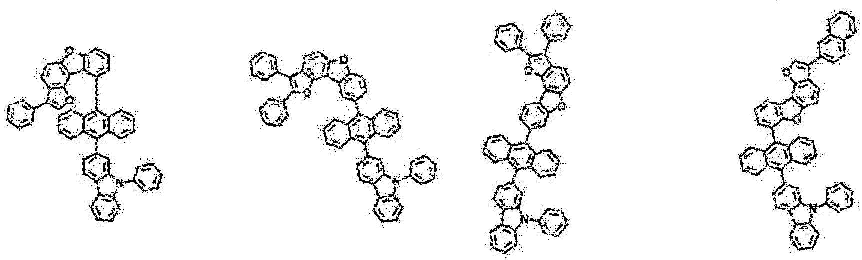

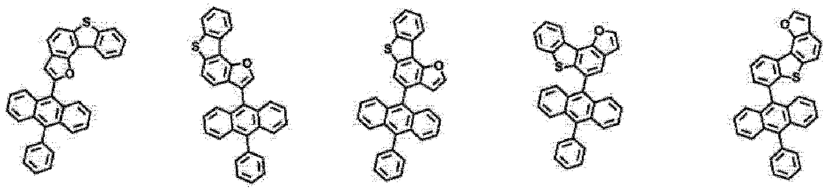

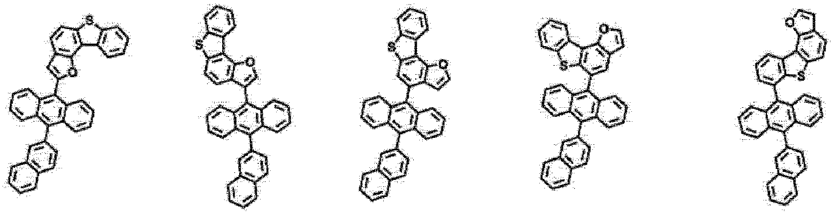

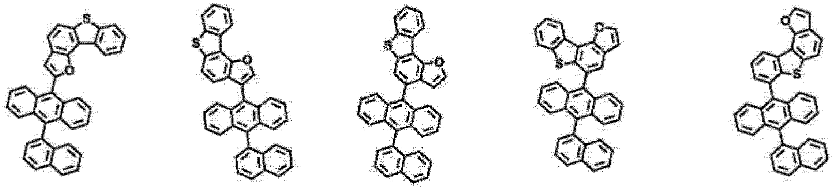

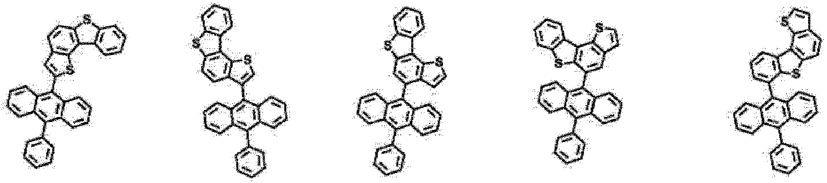

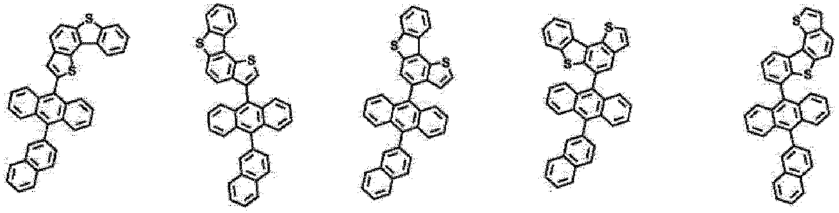

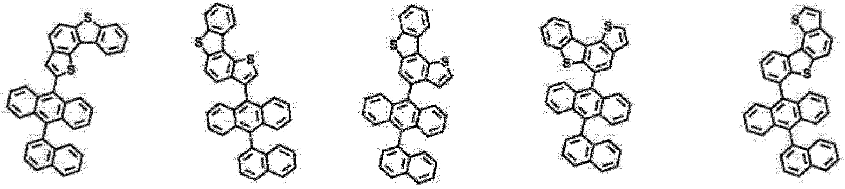

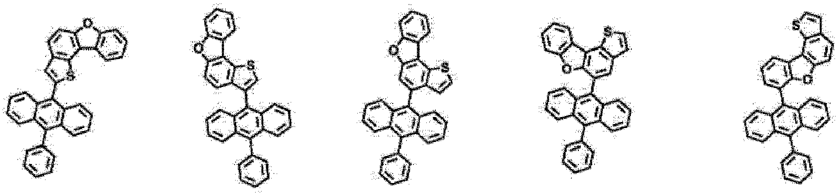

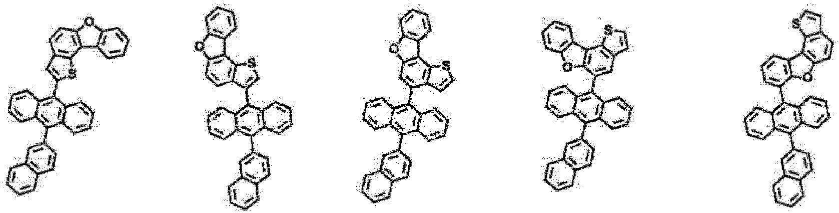

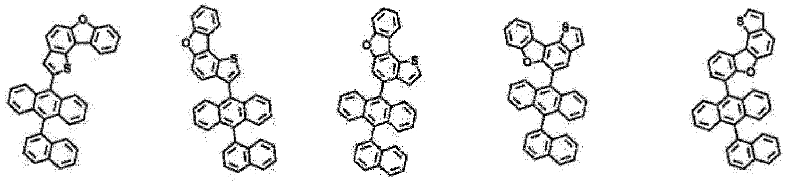

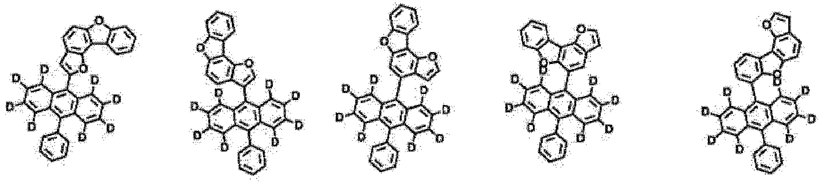

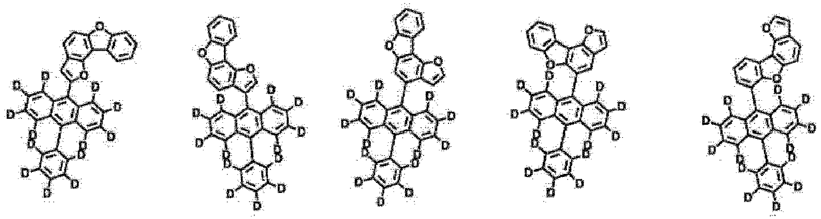

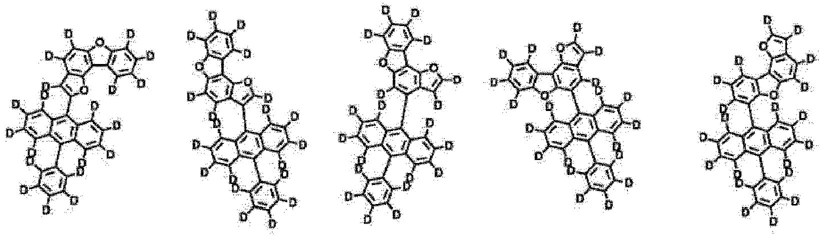

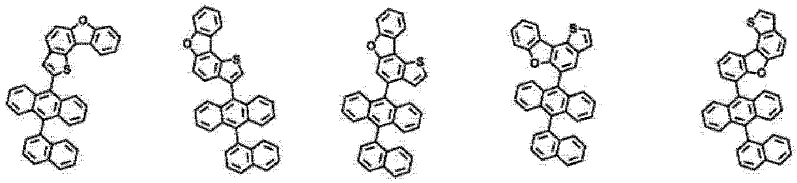

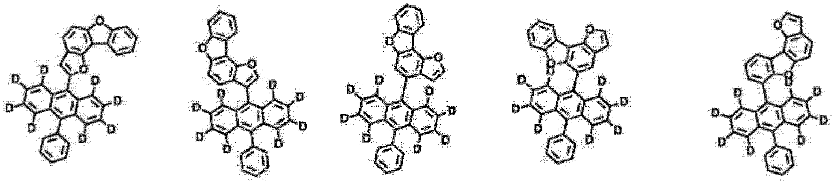

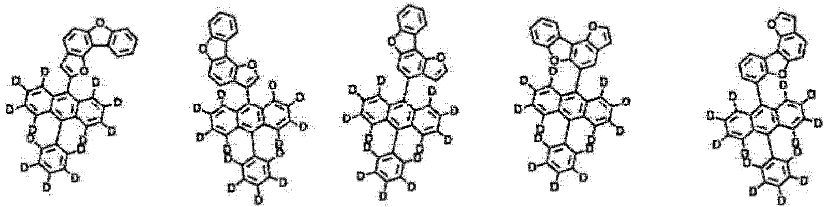

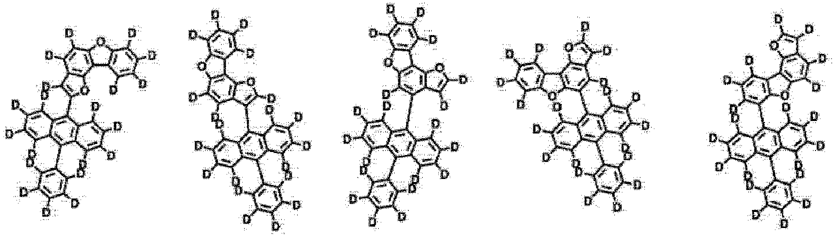

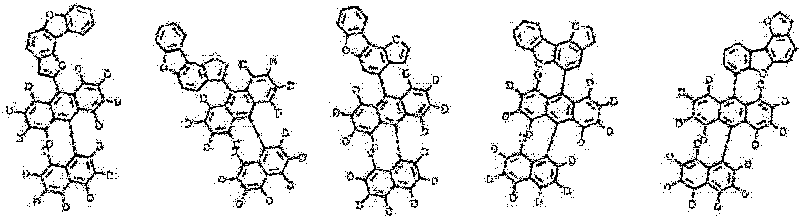

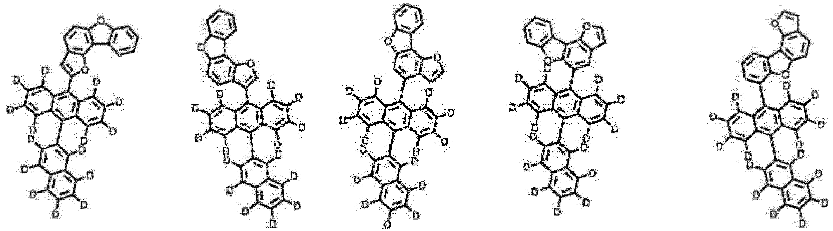

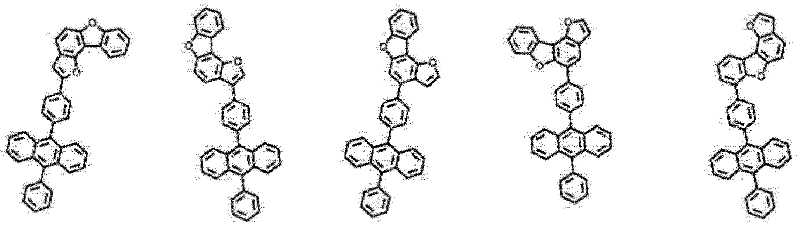

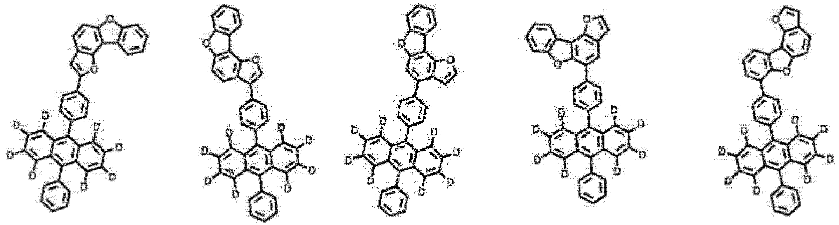

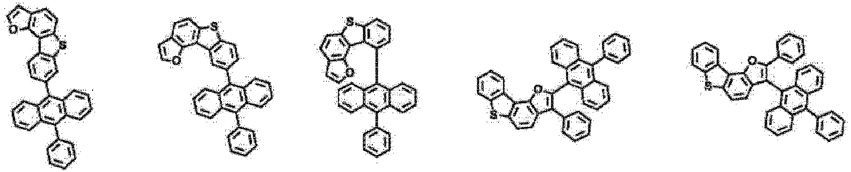

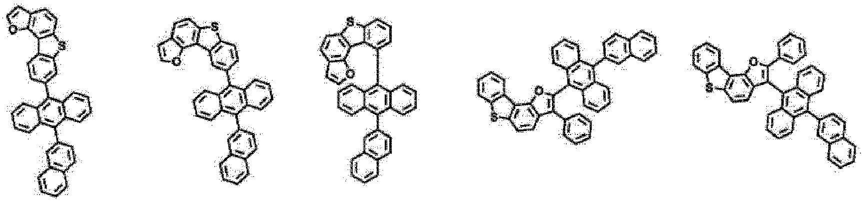

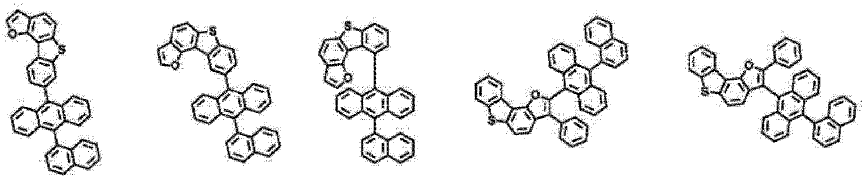

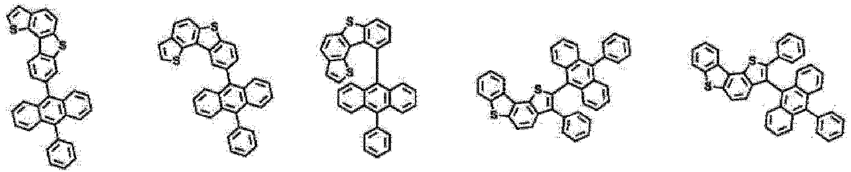

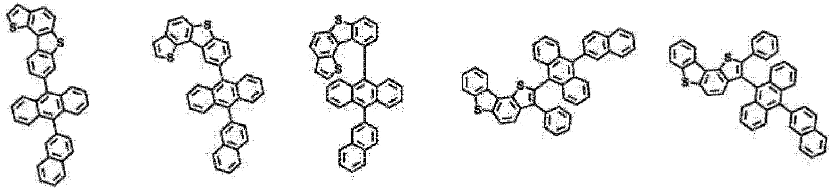

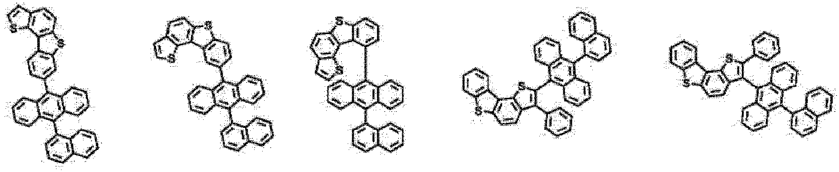

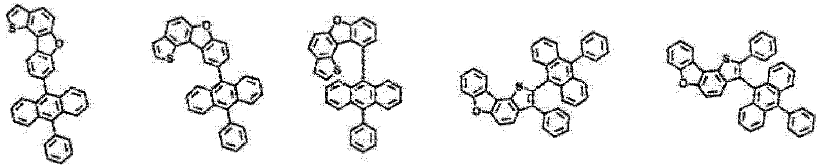

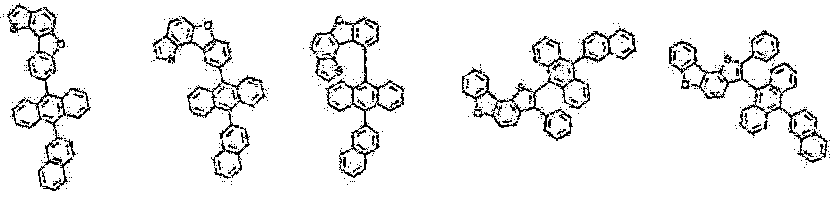

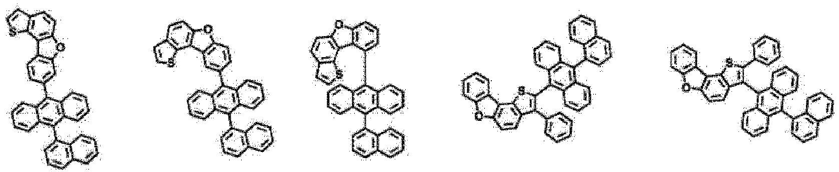

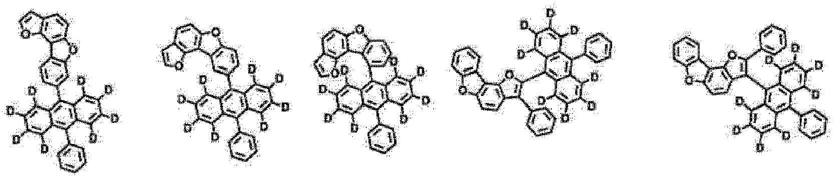

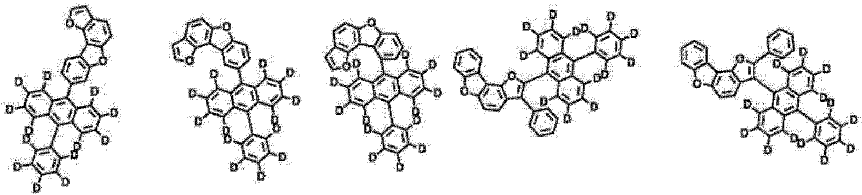

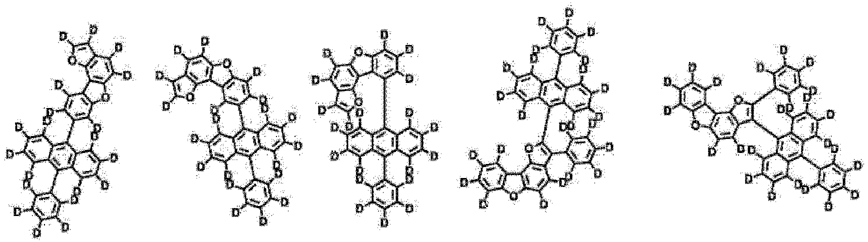

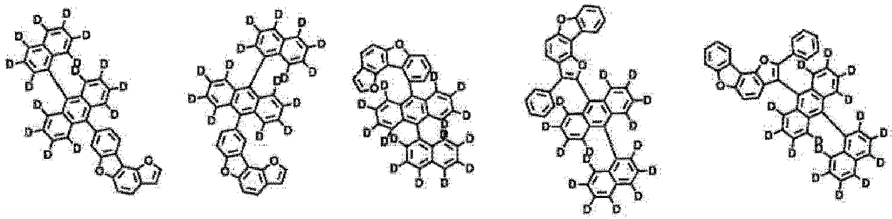

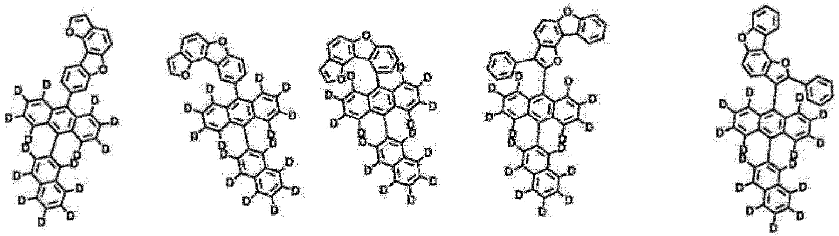

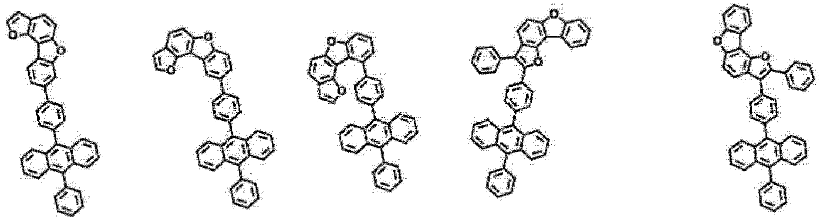

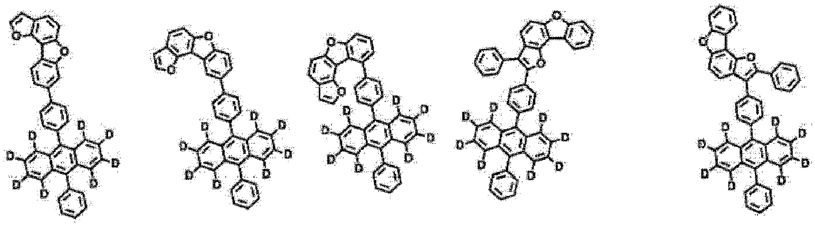

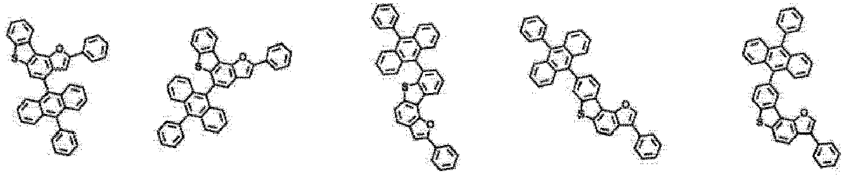

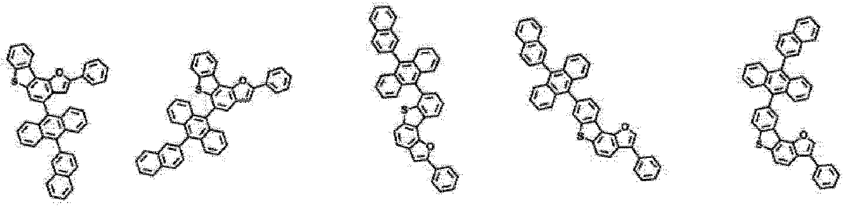

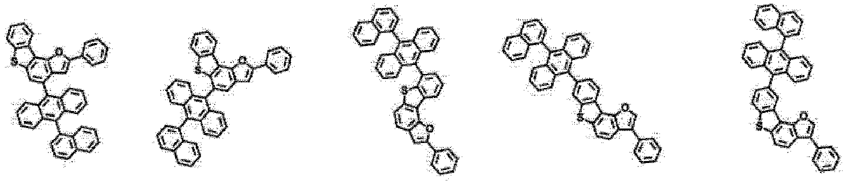

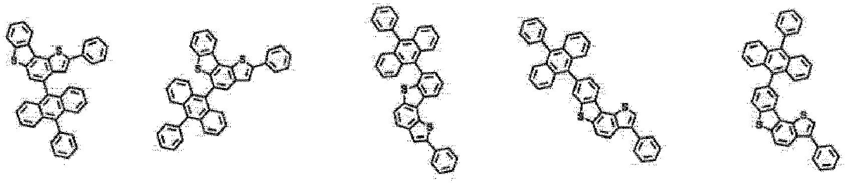

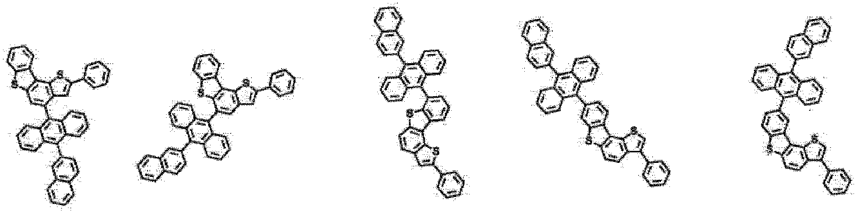

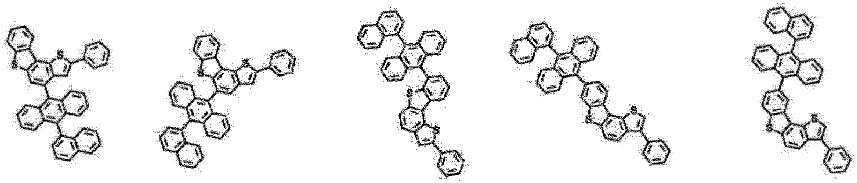

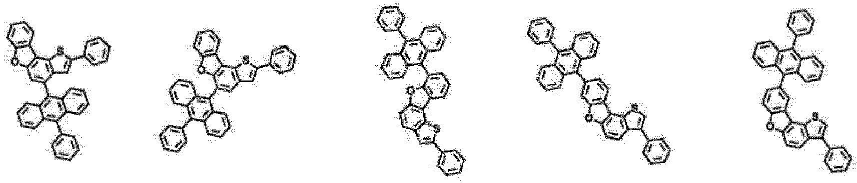

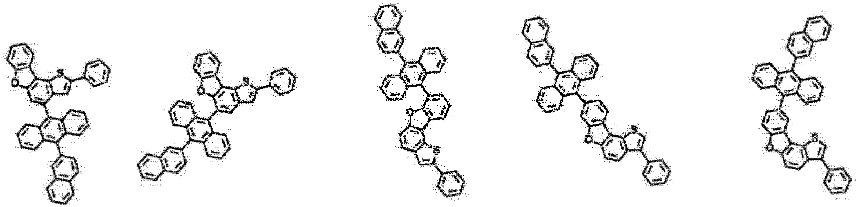

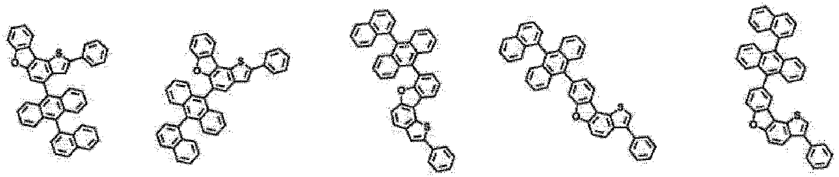

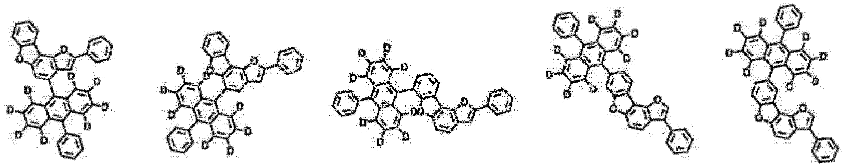

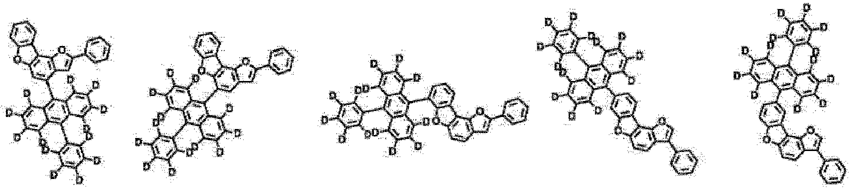

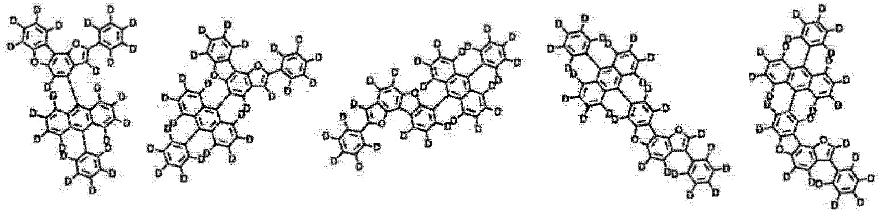

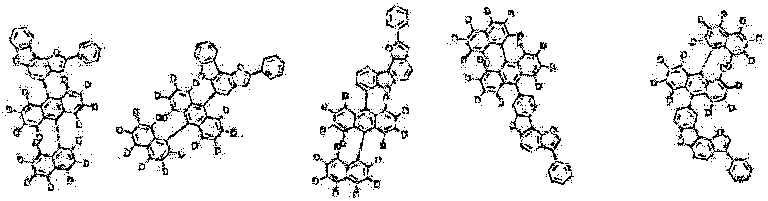

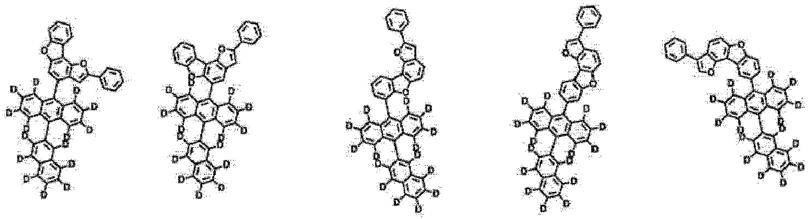

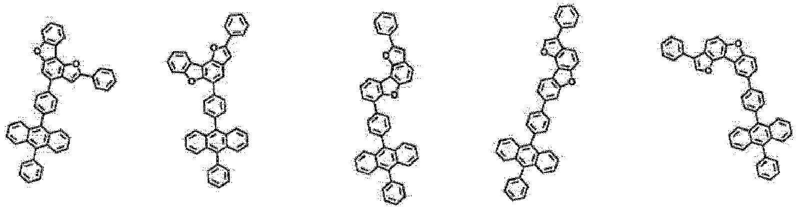

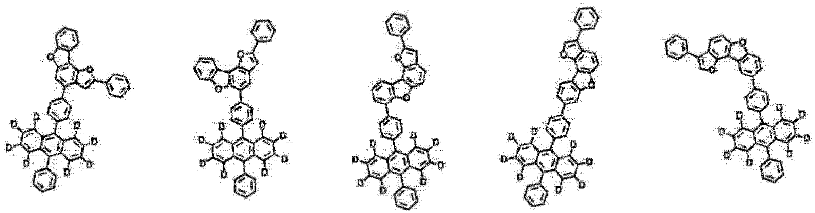

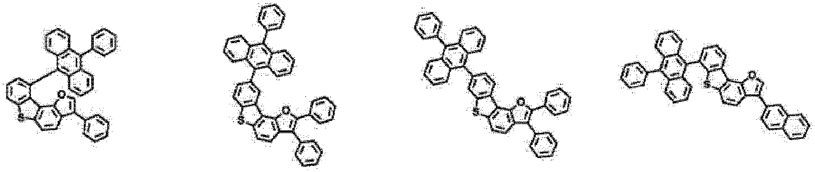

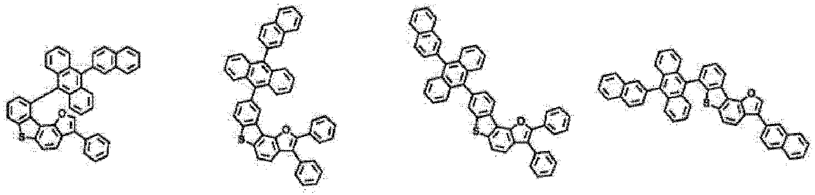

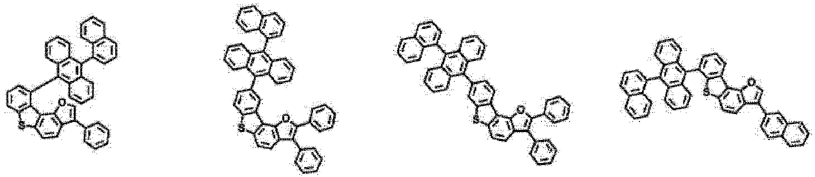

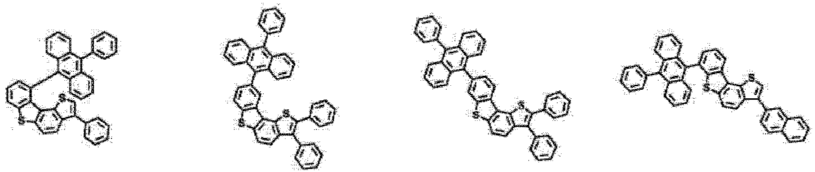

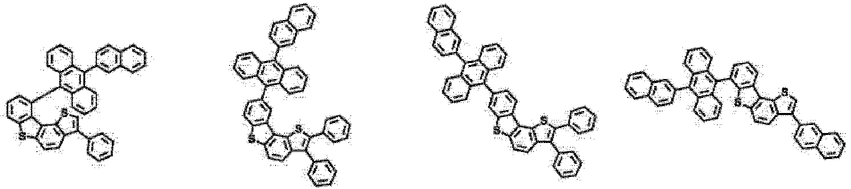

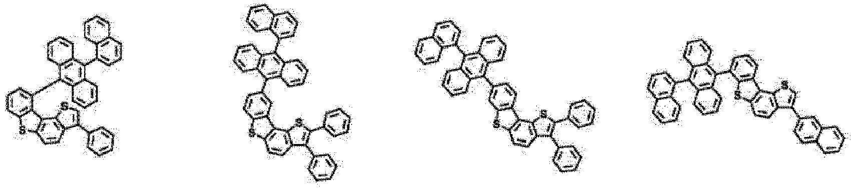

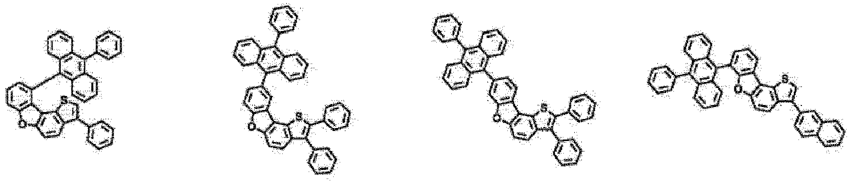

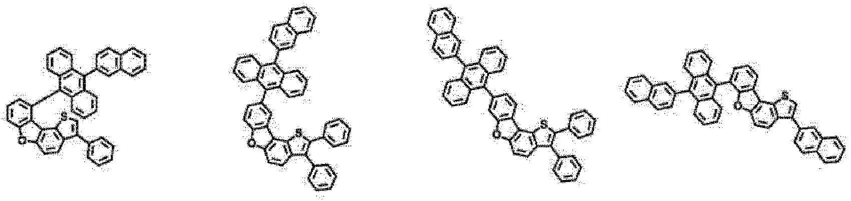

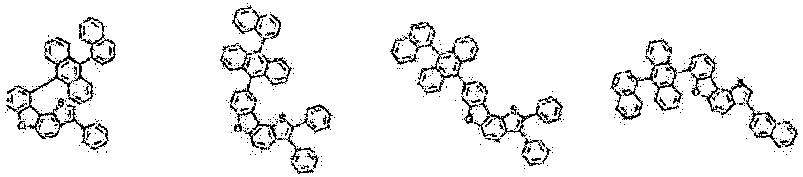

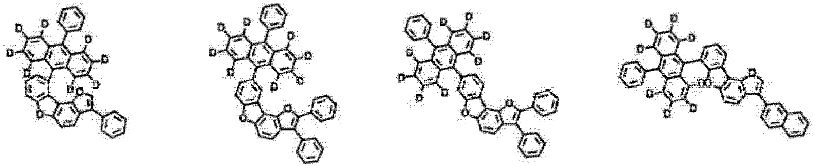

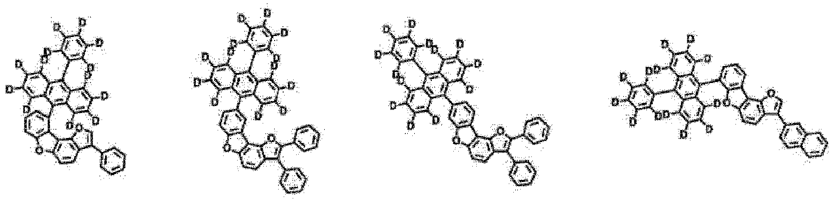

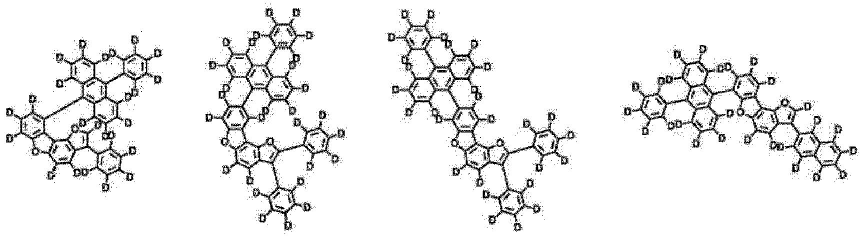

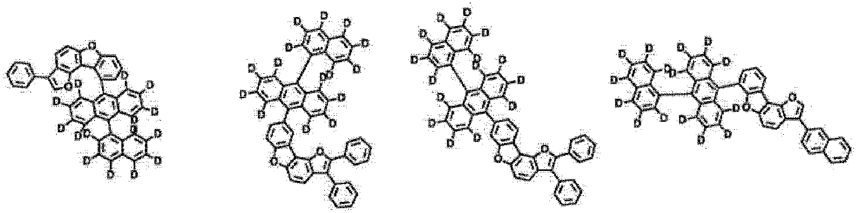

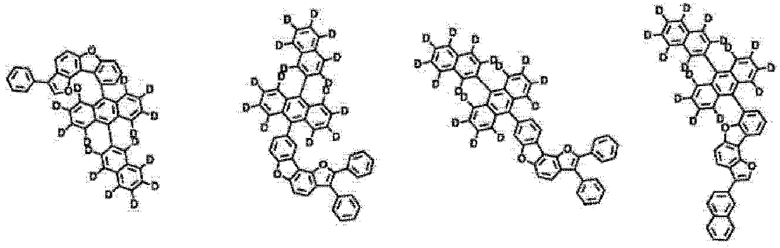

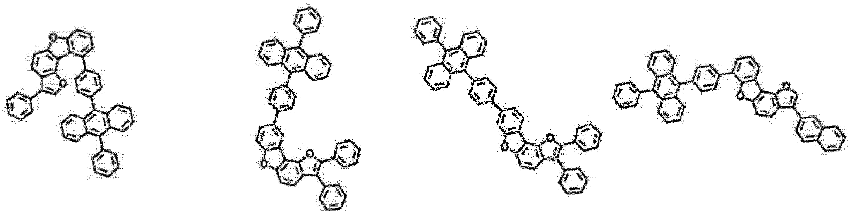

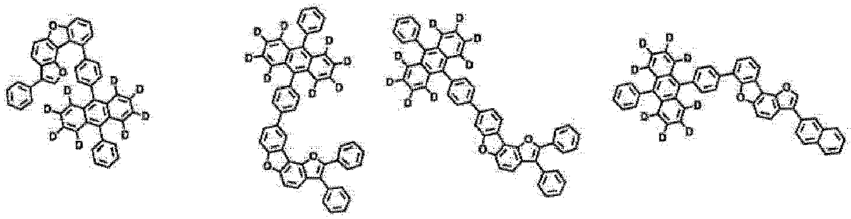

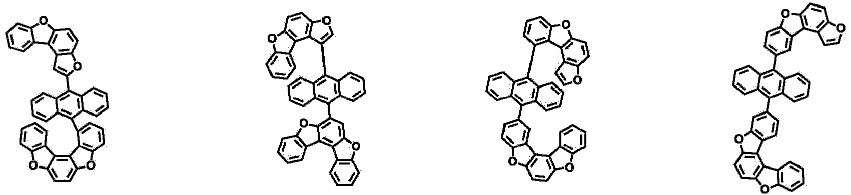

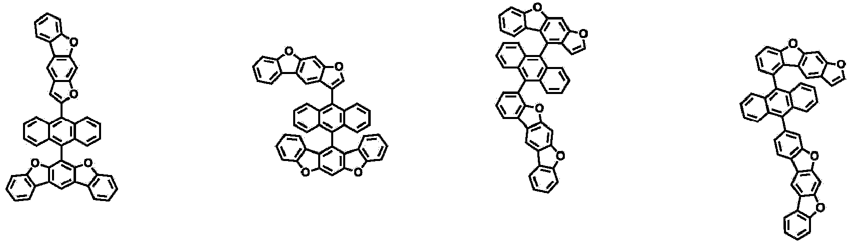

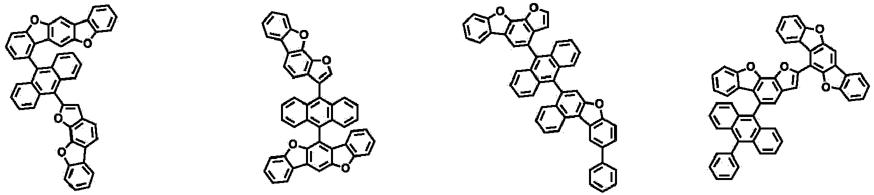

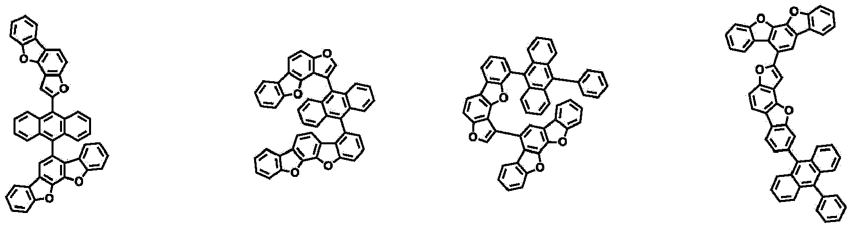

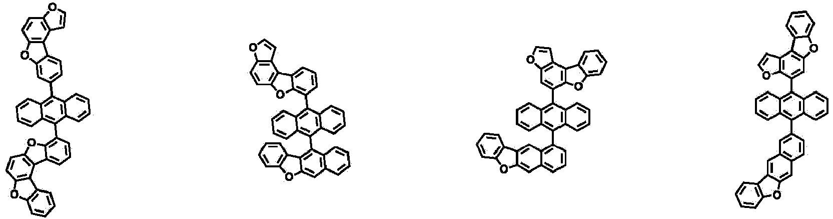

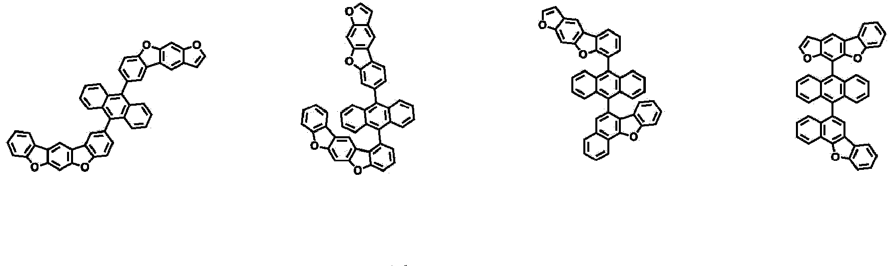

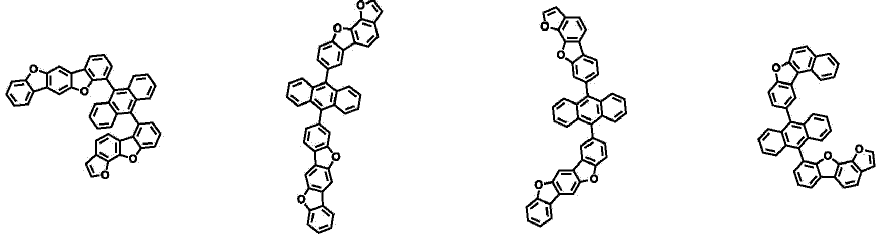

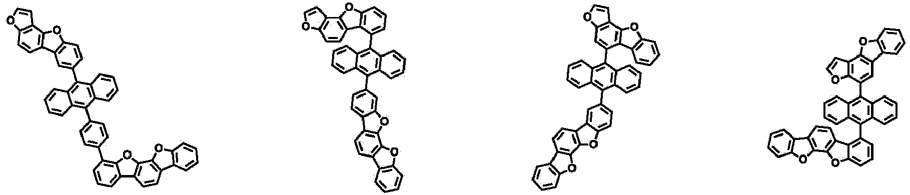

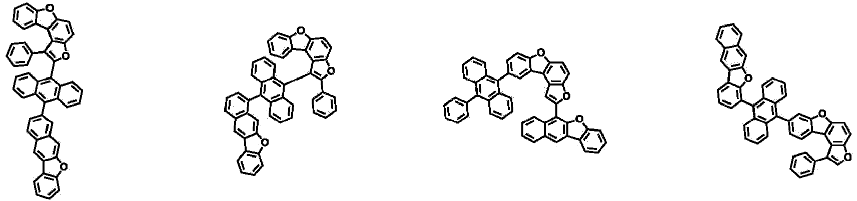

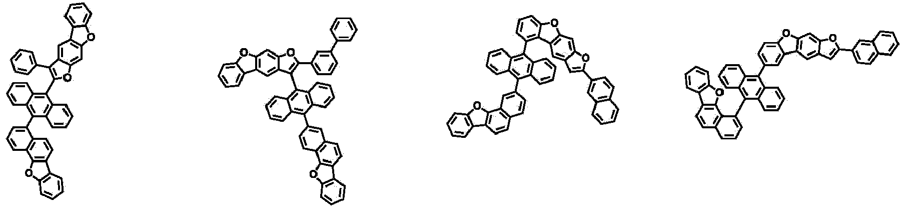

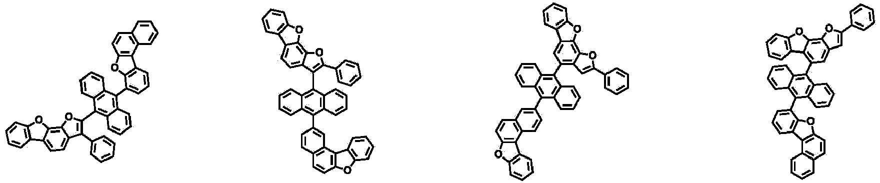

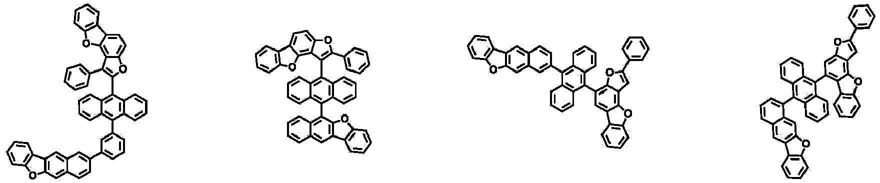

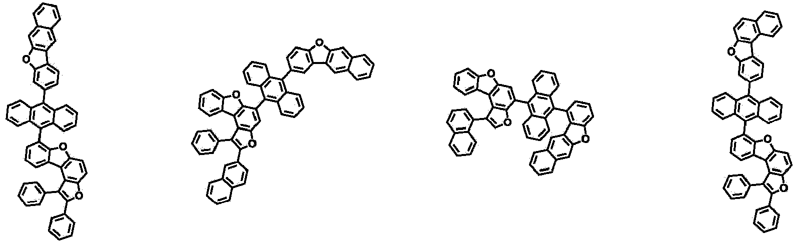

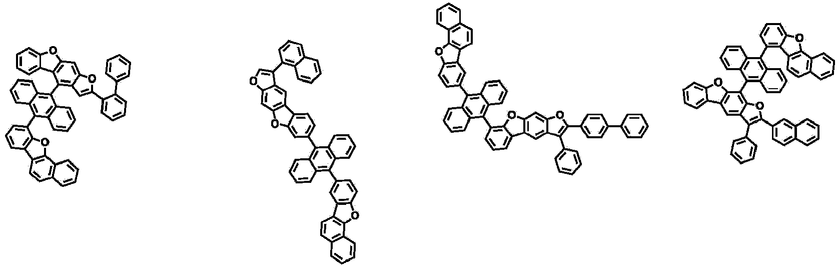

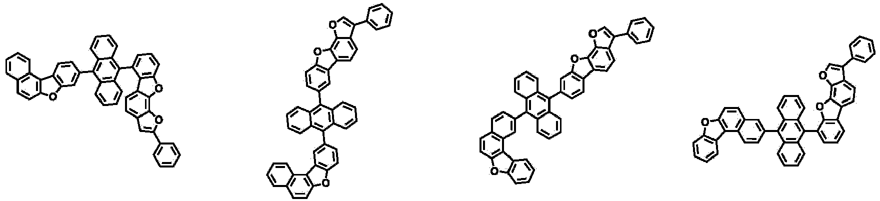

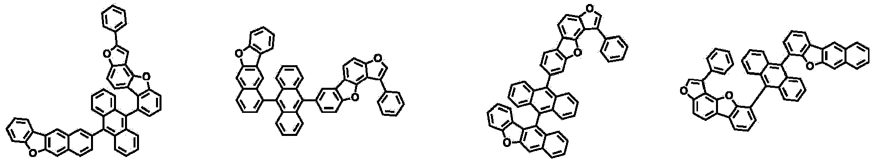

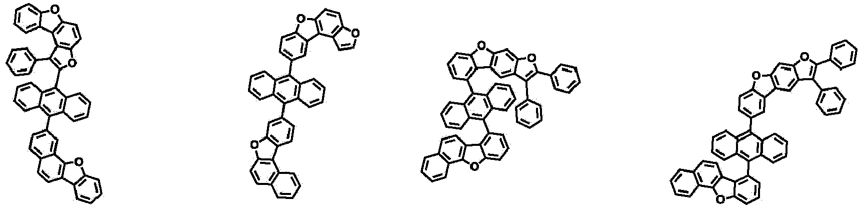

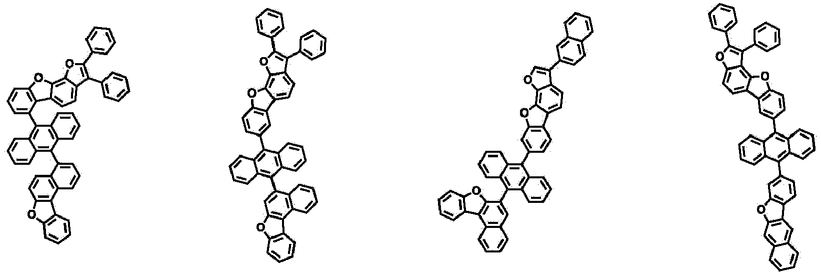

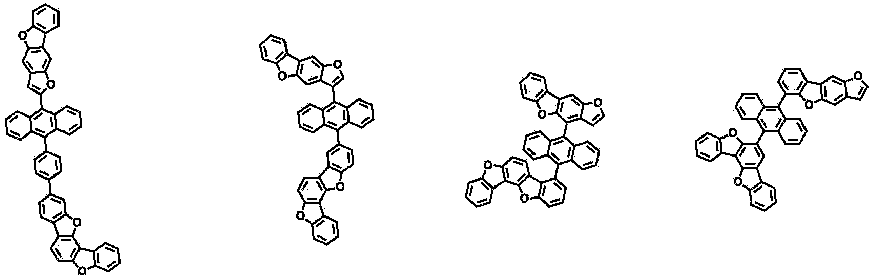

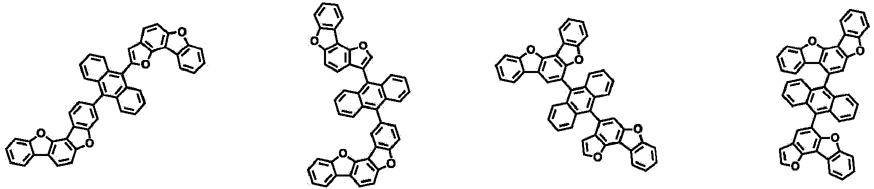

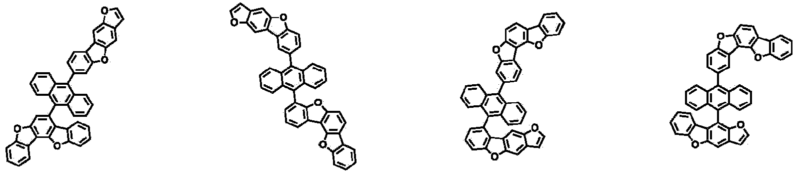

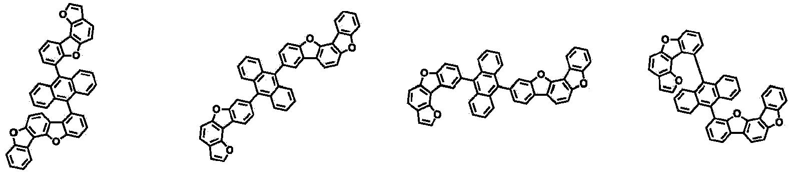

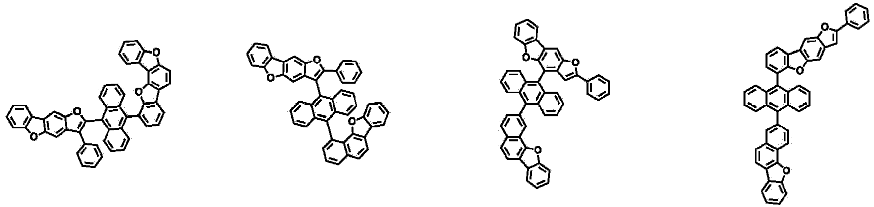

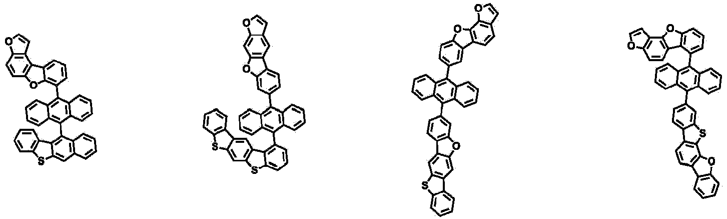

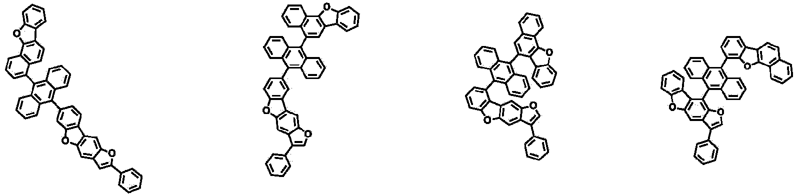

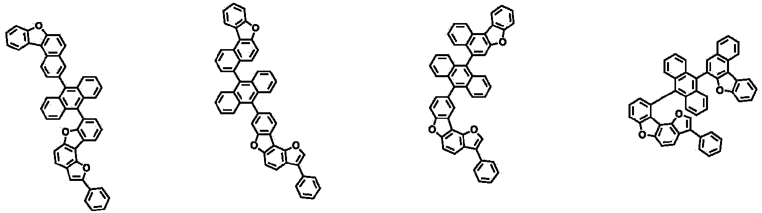

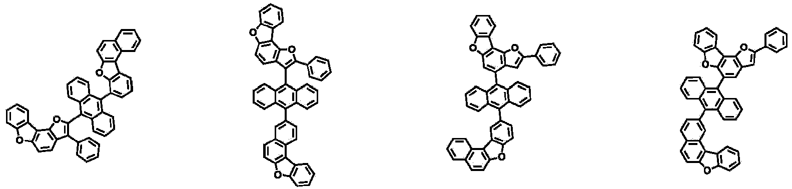

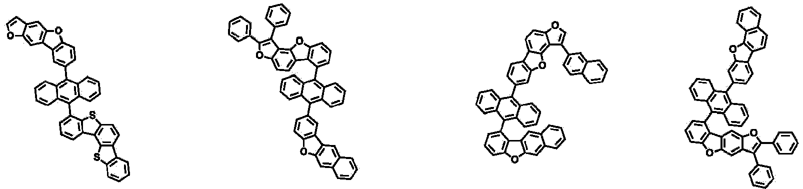

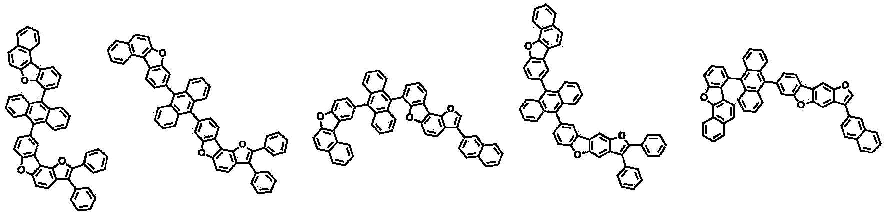

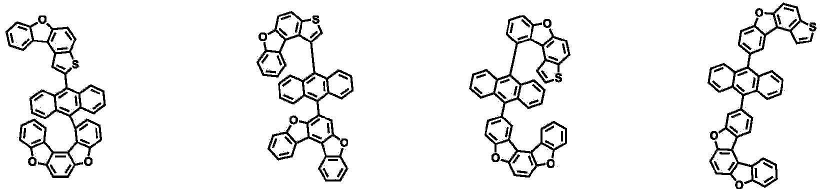

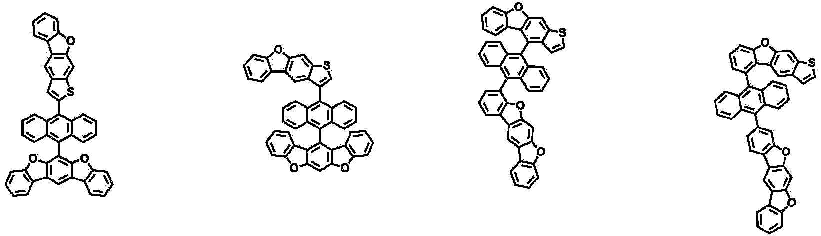

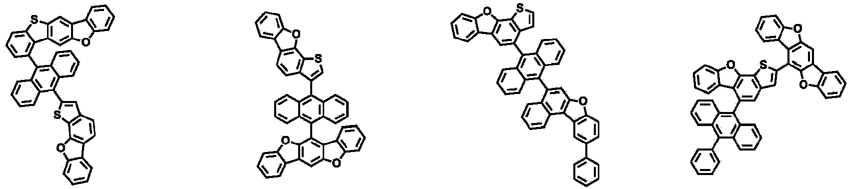

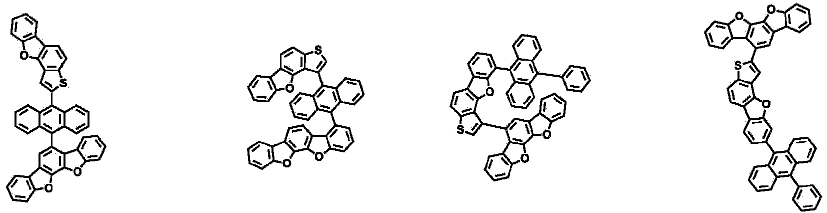

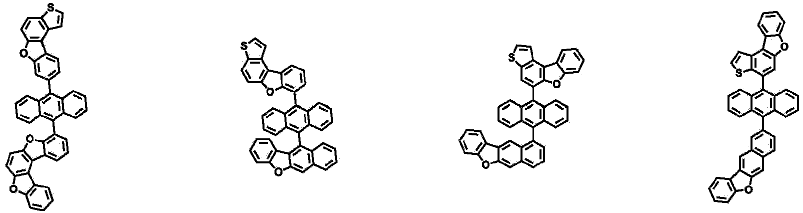

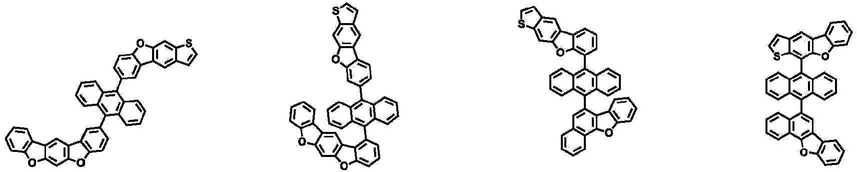

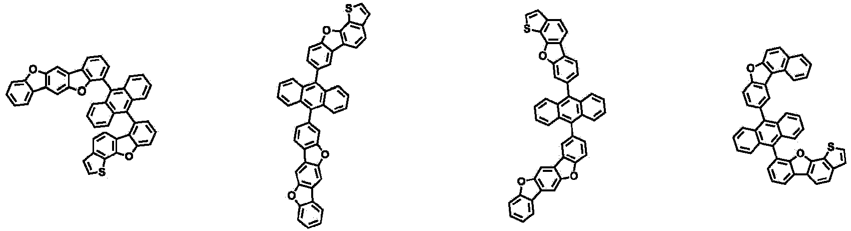

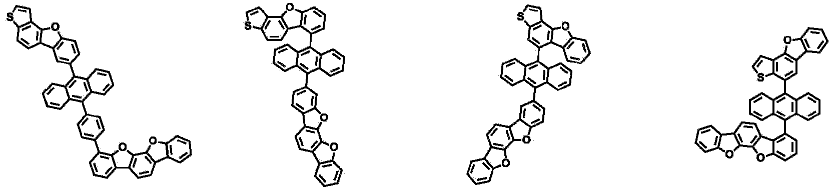

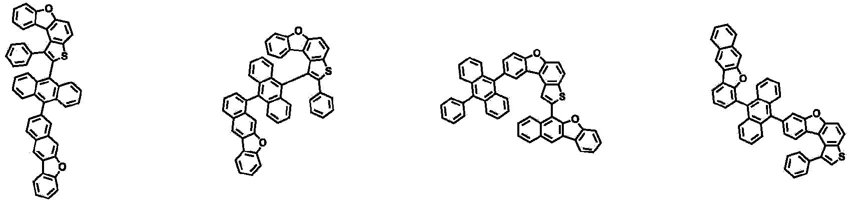

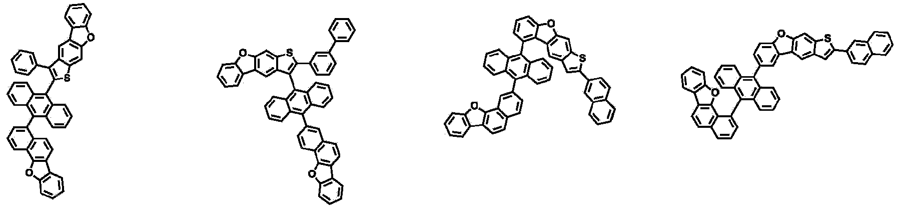

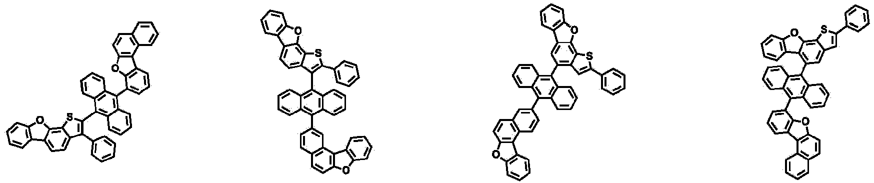

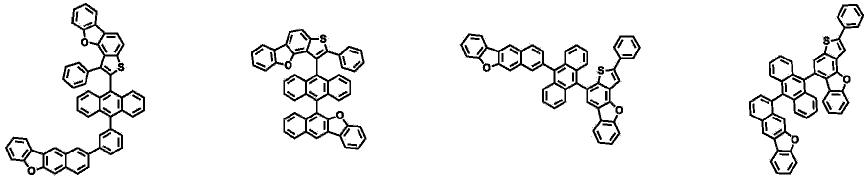

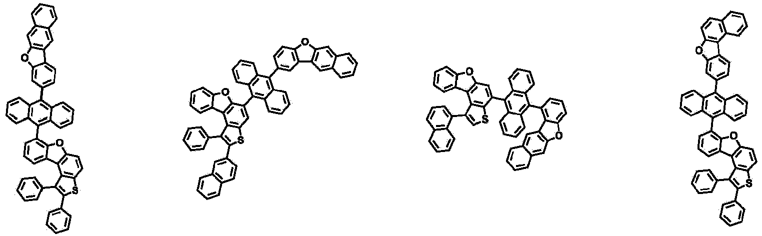

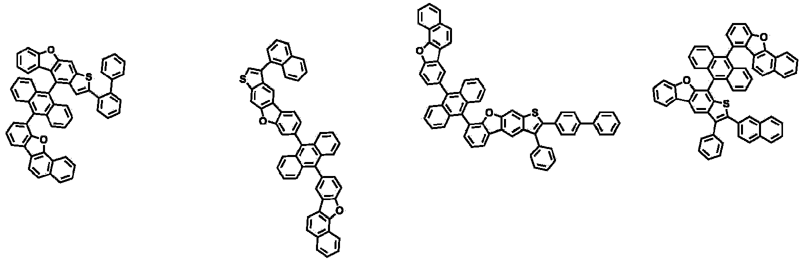

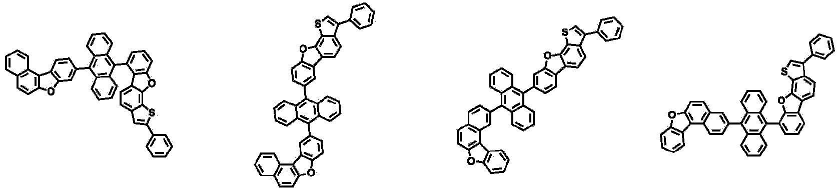

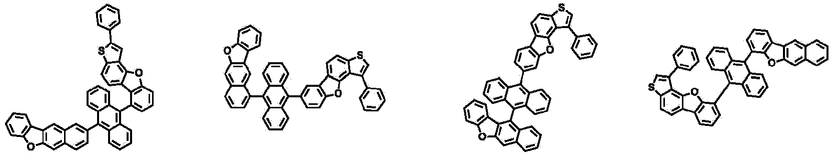

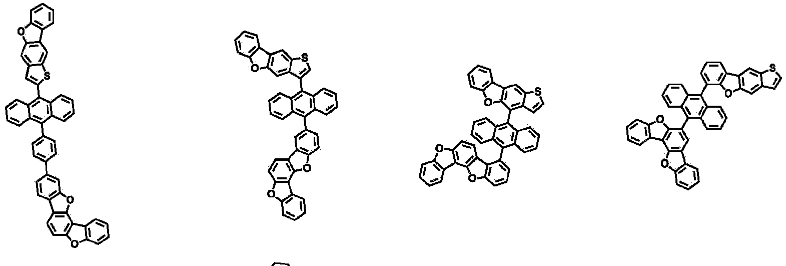

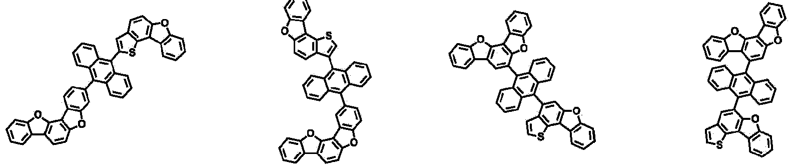

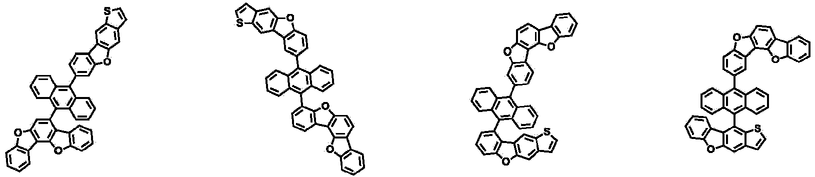

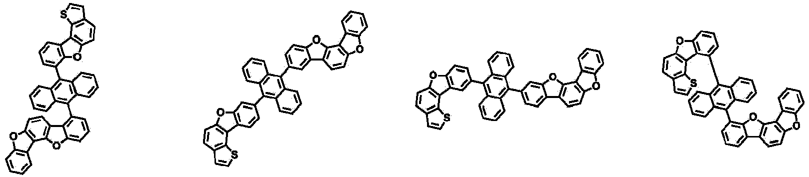

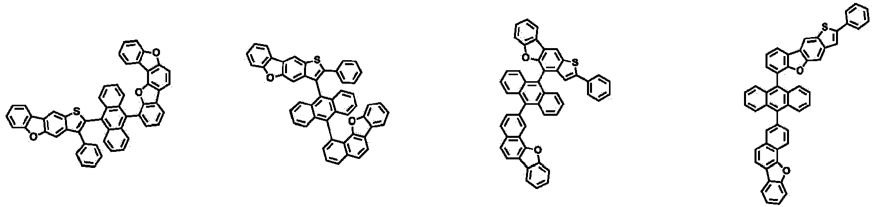

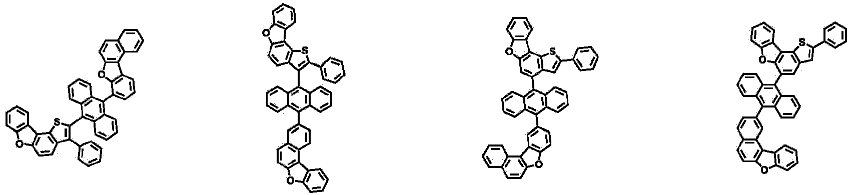

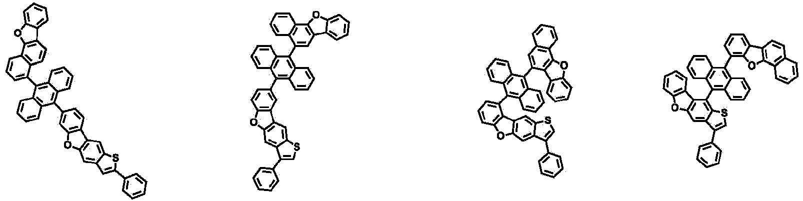

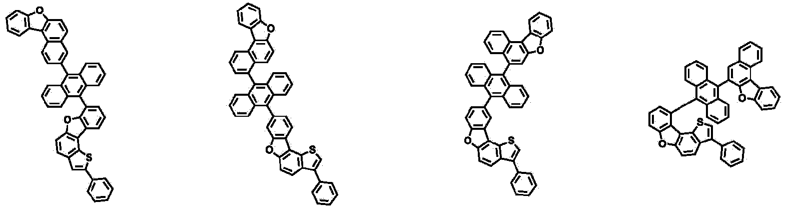

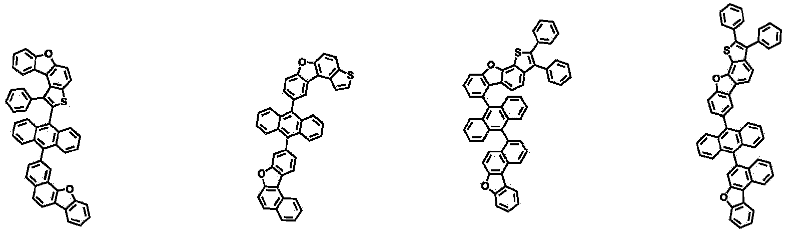

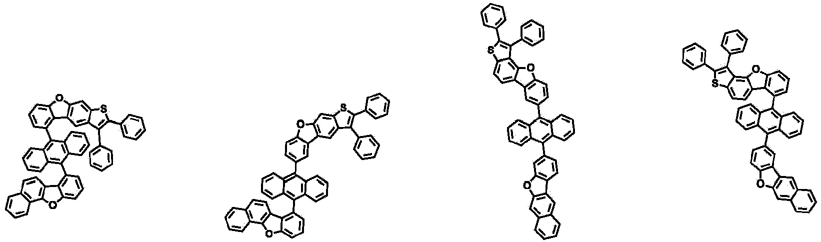

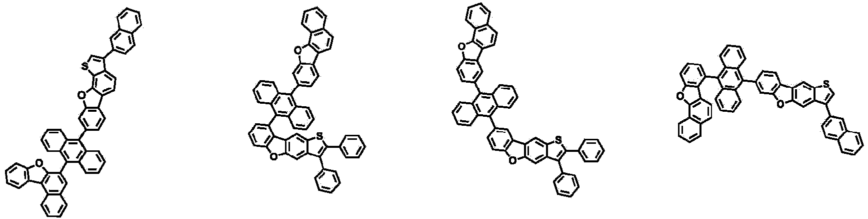

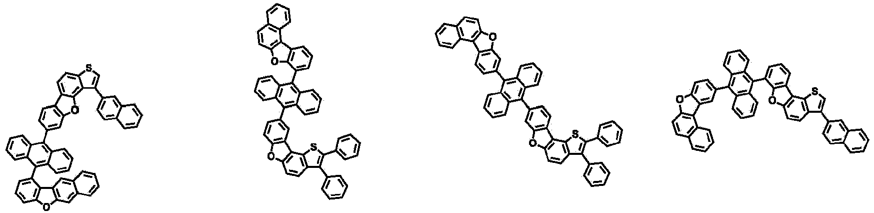

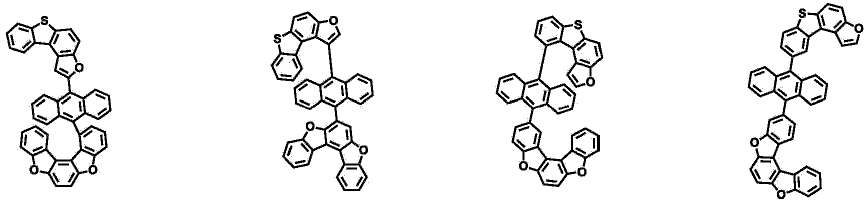

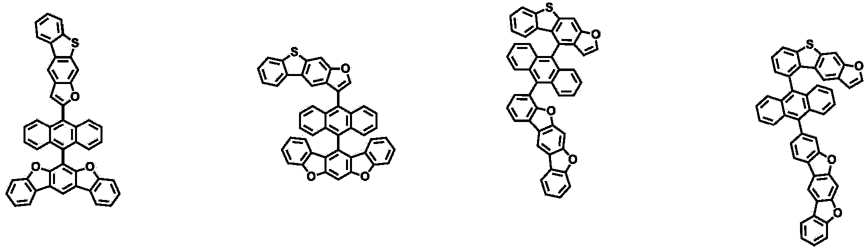

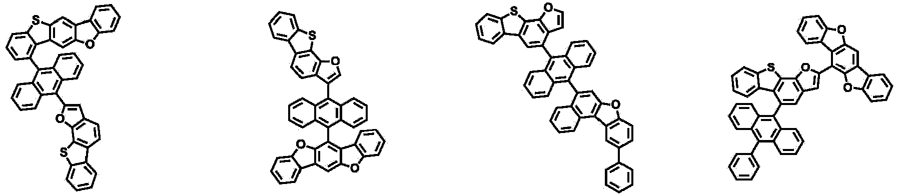

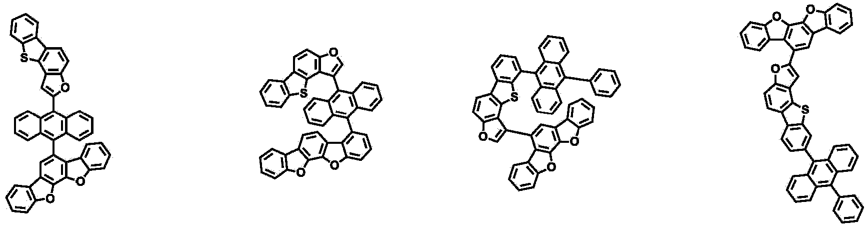

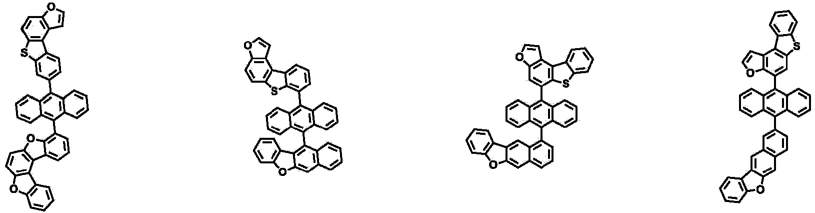

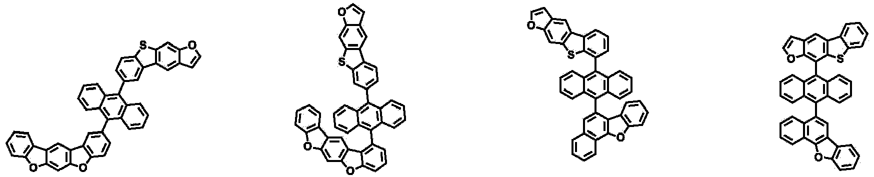

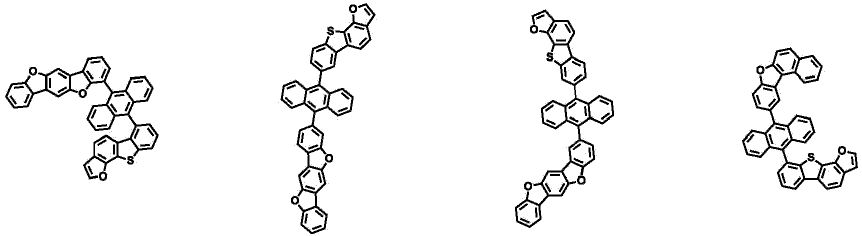

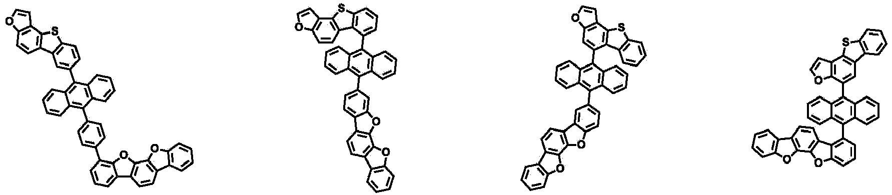

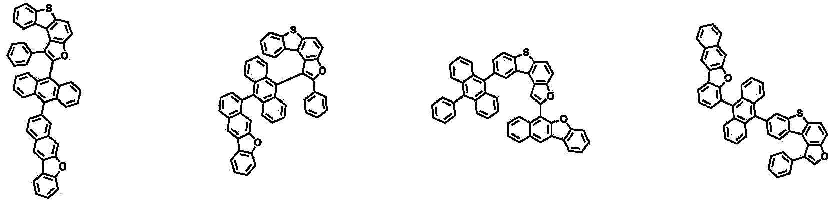

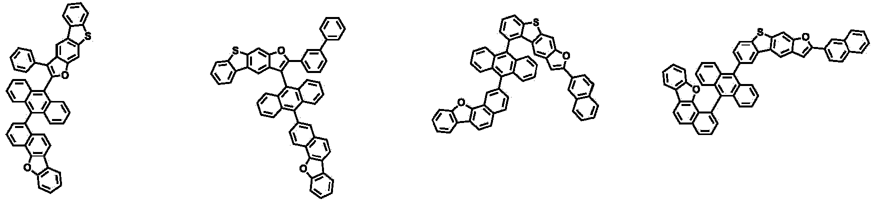

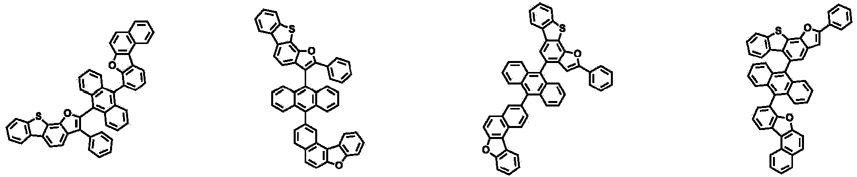

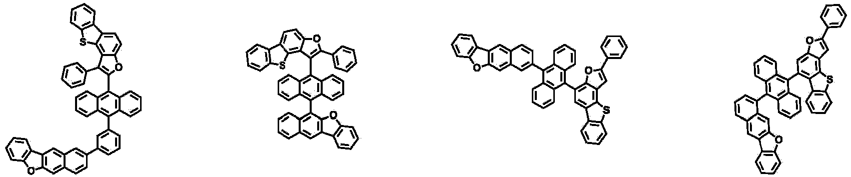

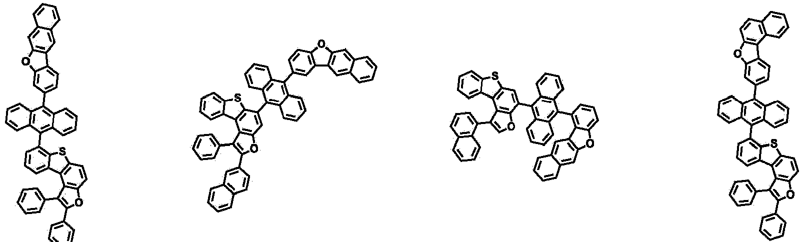

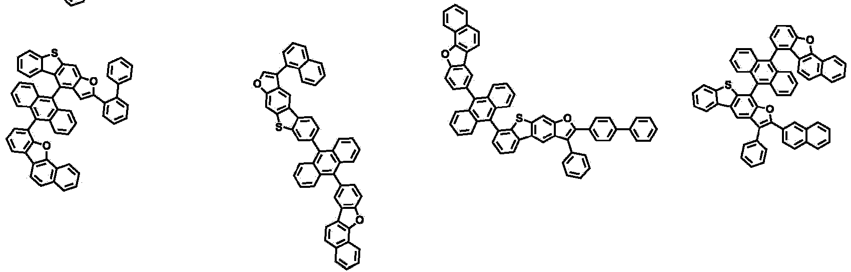

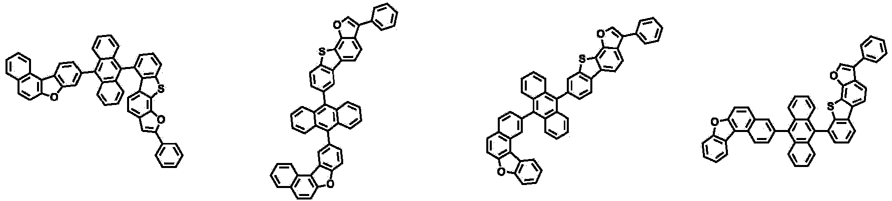

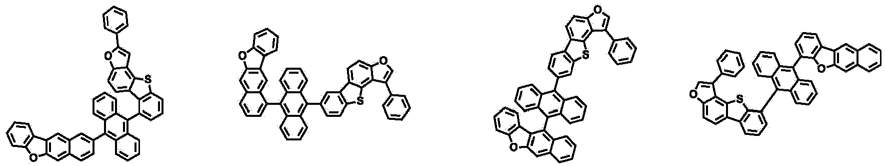

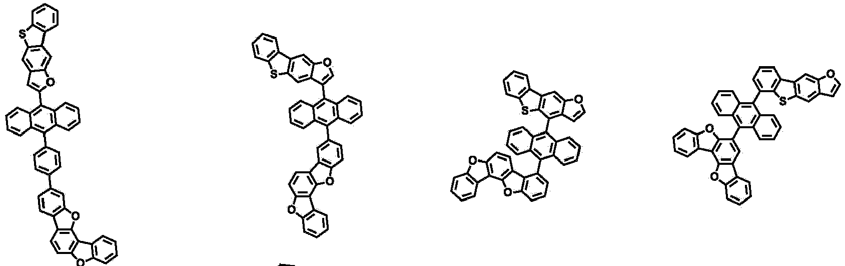

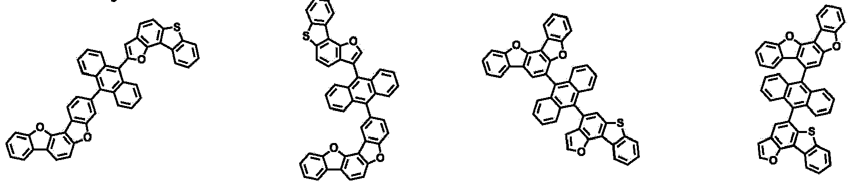

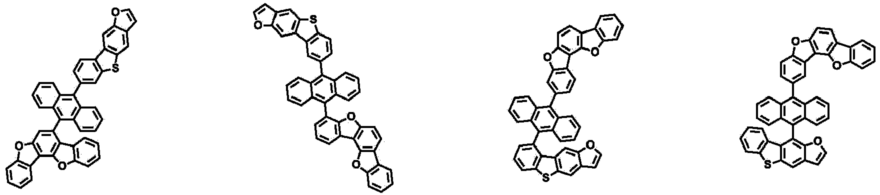

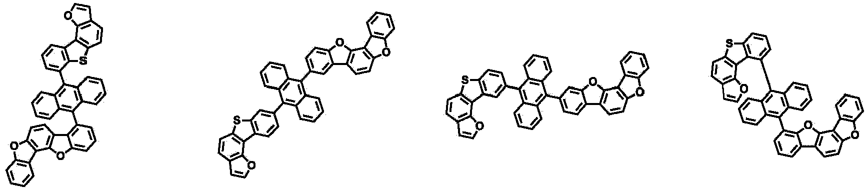

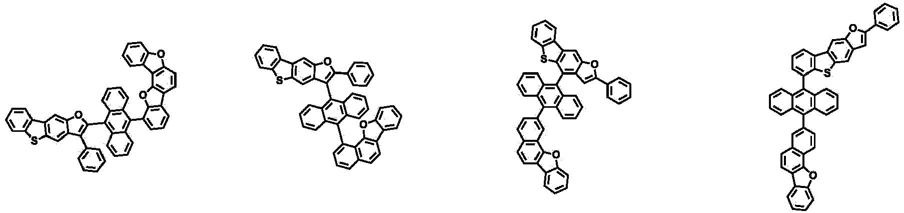

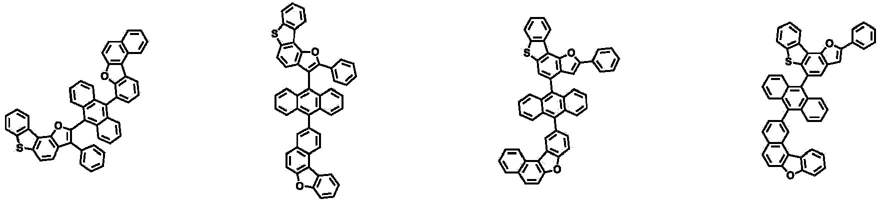

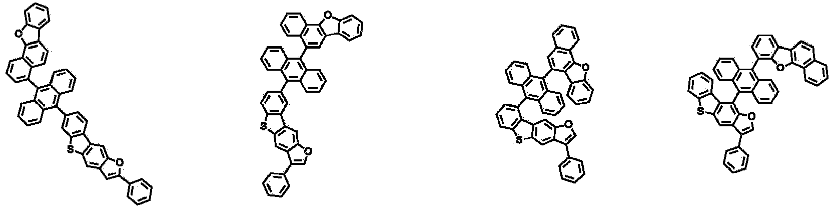

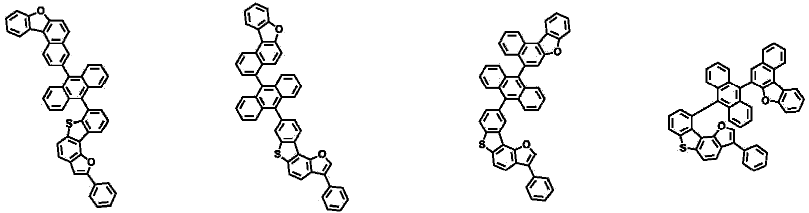

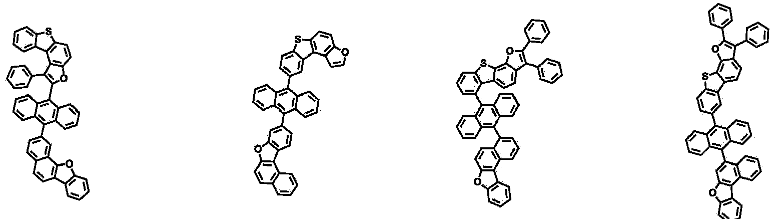

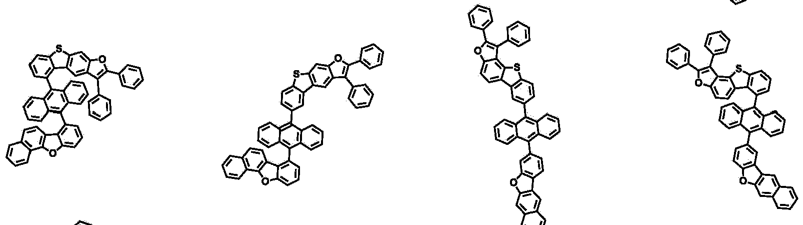

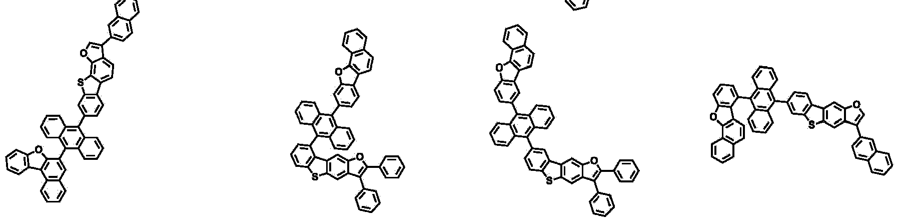

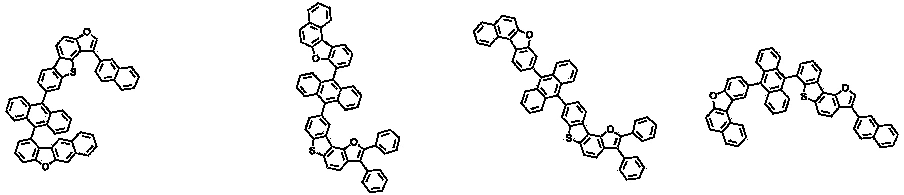

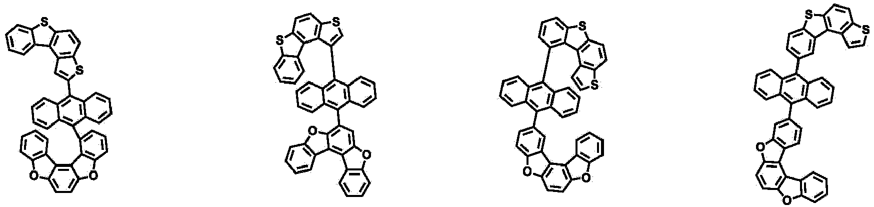

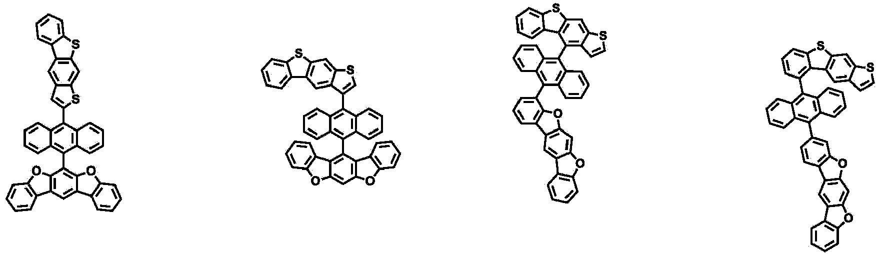

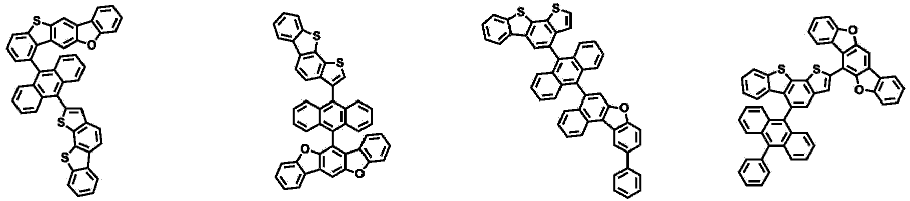

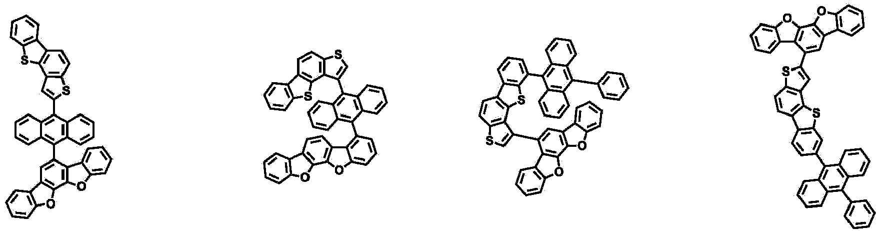

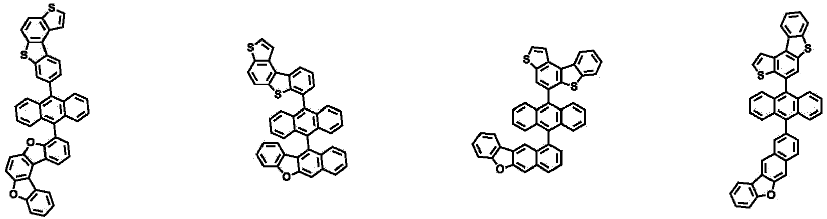

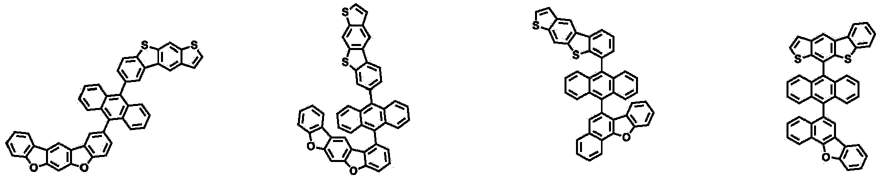

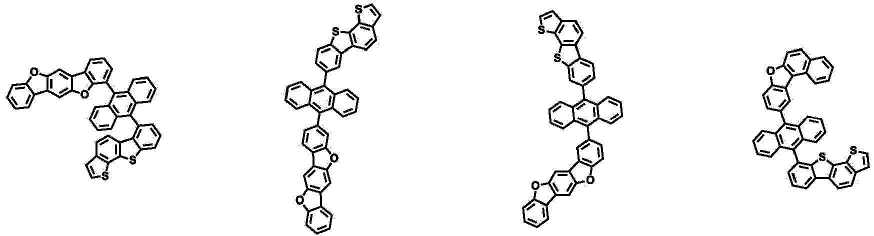

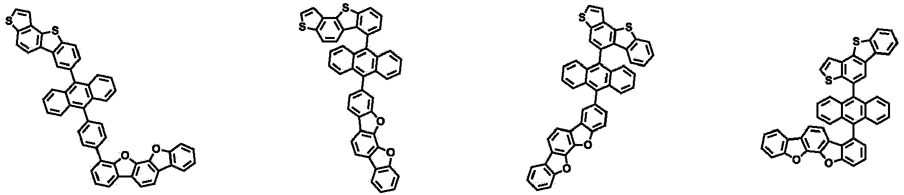

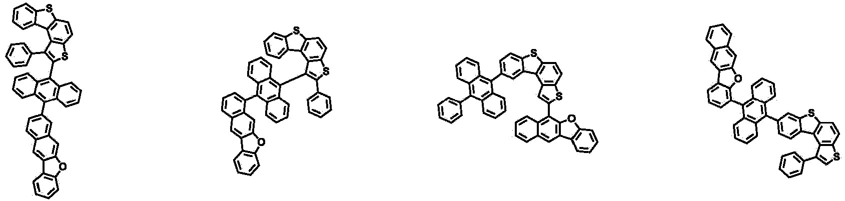

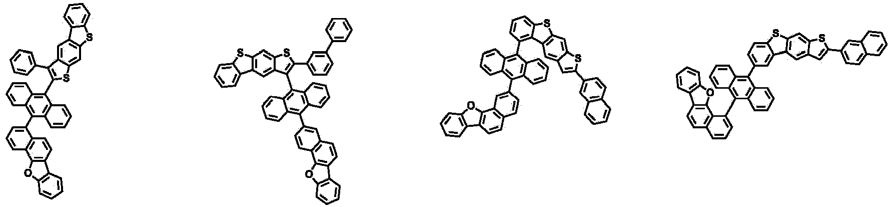

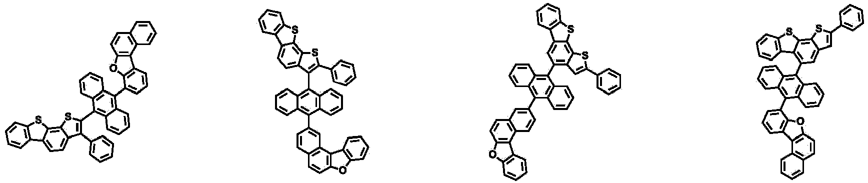

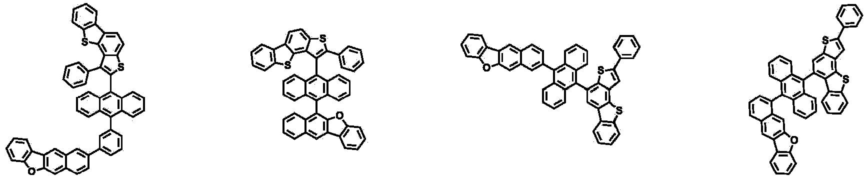

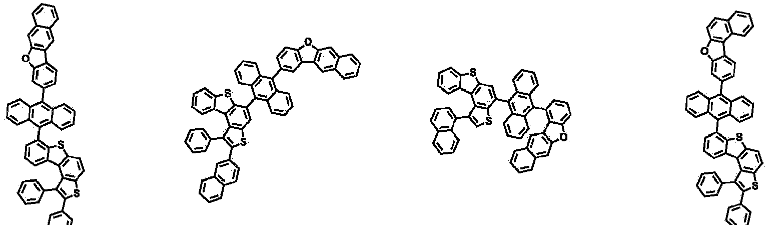

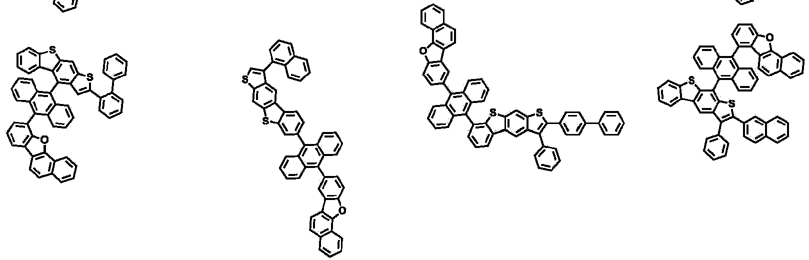

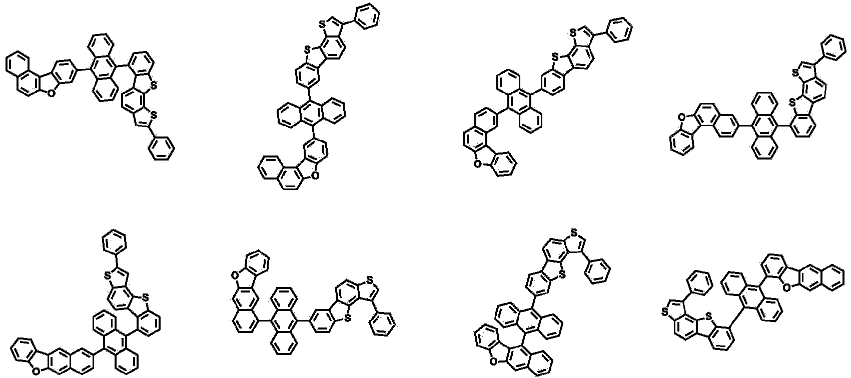

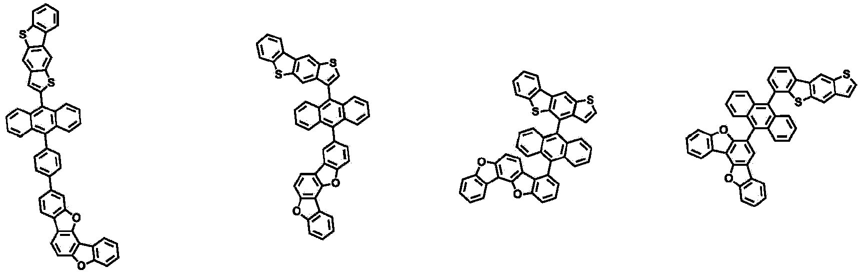

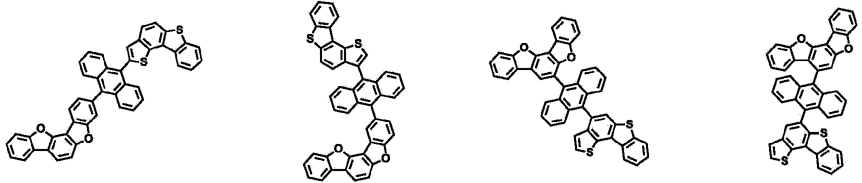

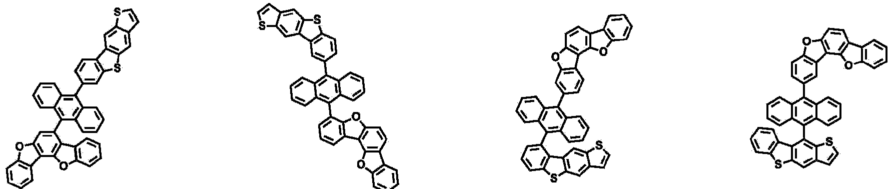

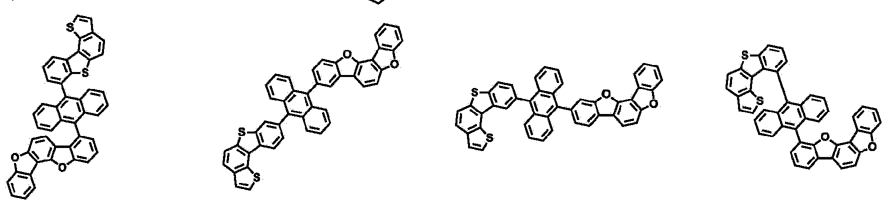

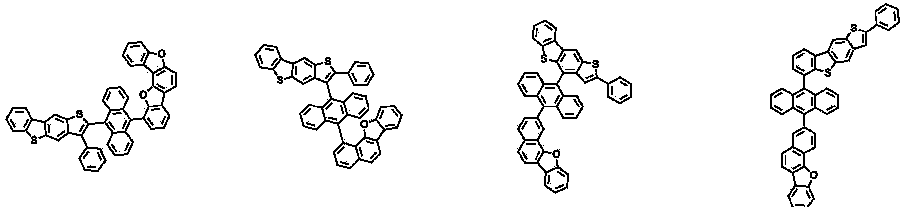

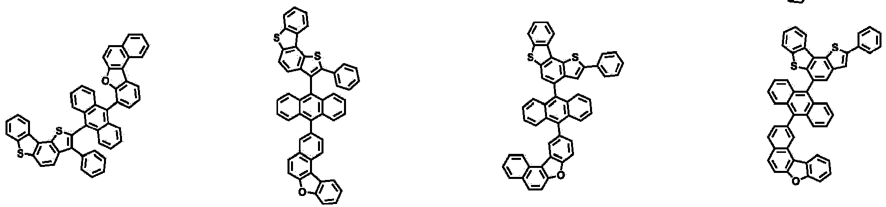

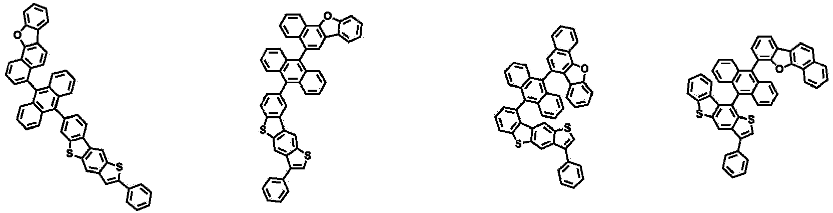

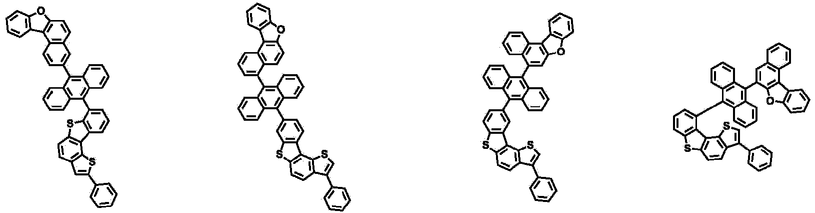

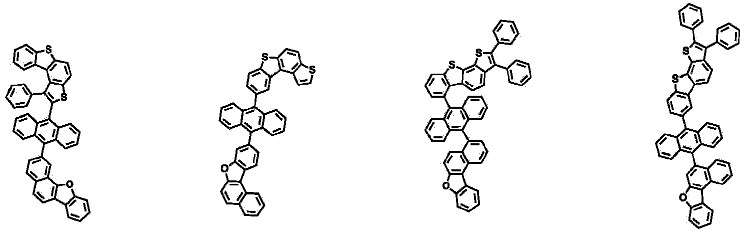

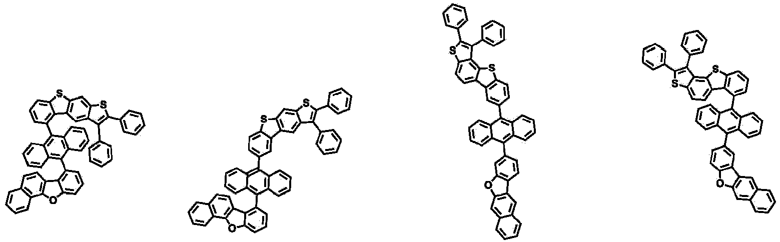

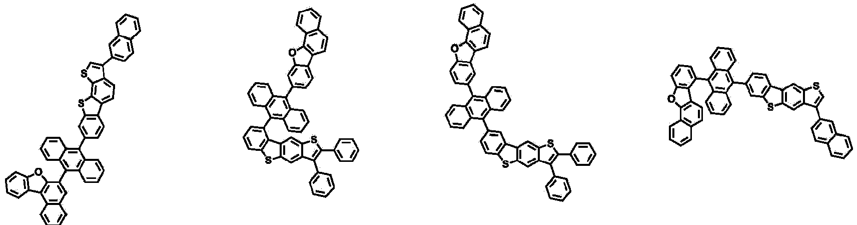

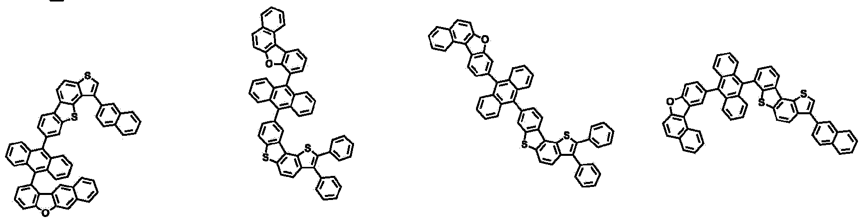

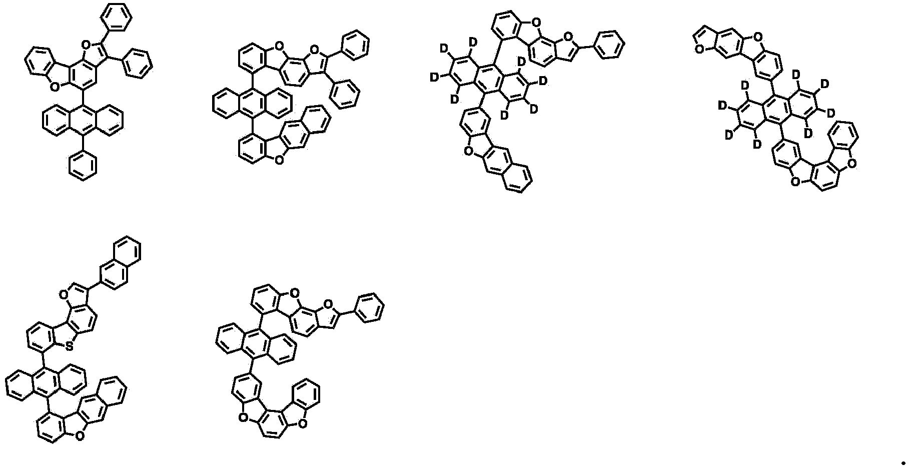

14. An organic light emitting device comprising:
a first electrode;
a second electrode provided to face the first electrode; and
an organic material layer having one or more layers provided between the first electrode and the second electrode,
wherein one or more layers of the organic material layer comprise the compound according to any one of claims 1 to 13.

15. The organic light emitting device of claim 14, wherein the organic material layer comprises a light emitting layer, and the light emitting layer comprises the compound.

## Patentansprüche

1. Verbindung der chemischen Formel 1: 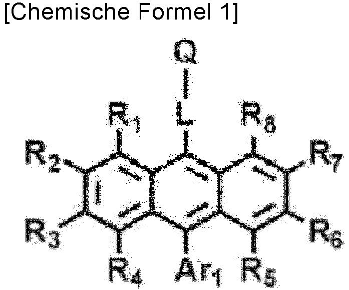 wobei, in der chemischen Formel 1:
R₁ bis R₈ gleich oder verschieden voneinander sind und jeweils unabhängig voneinander Wasserstoff, Deuterium, eine Halogengruppe, eine substituierte oder unsubstituierte Alkylgruppe, eine substituierte oder unsubstituierte Cycloalkylgruppe, eine substituierte oder unsubstituierte Arylgruppe oder eine substituierte oder unsubstituierte Heteroarylgruppe sind;
Ar₁ eine substituierte oder unsubstituierte Arylgruppe, oder eine substituierte oder unsubstituierte Heteroarylgruppe ist;
L eine direkte Bindung oder eine substituierte oder unsubstituierte Arylengruppe ist;
Q eine der Gruppen der folgenden chemischen Formeln 2-1 bis 2-6 ist, 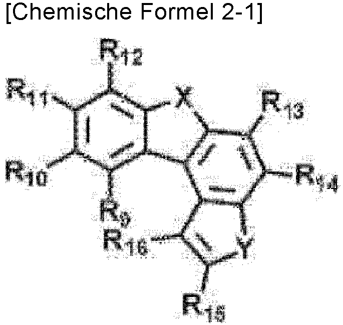 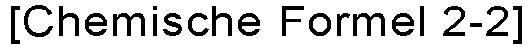 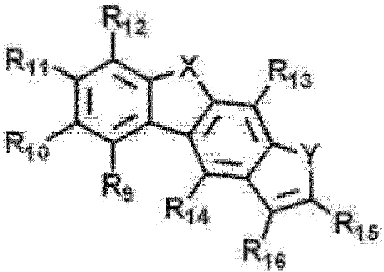 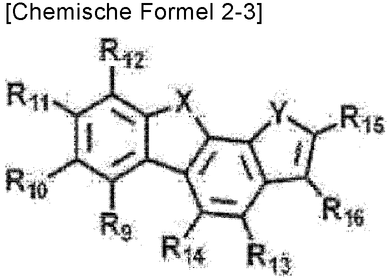 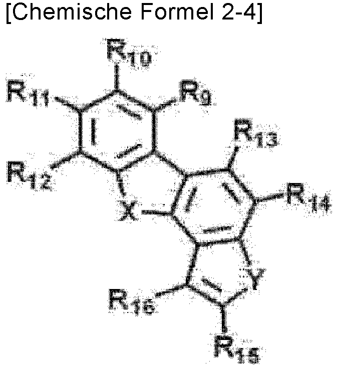 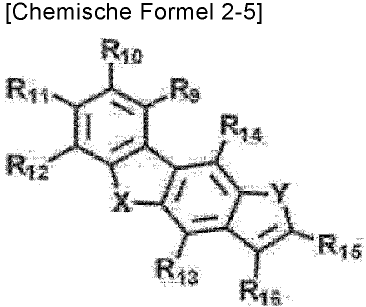 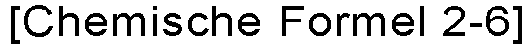 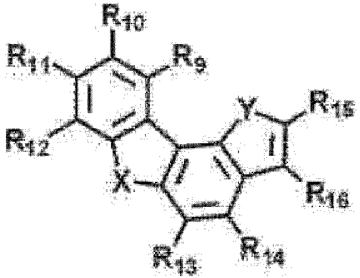
wobei in den chemischen Formeln 2-1 bis 2-6:
X und Y gleich oder verschieden voneinander sind und jeweils unabhängig voneinander 0 oder S sind;
einer von R₉ bis R₁₆ mit L der chemischen Formel 1 verbunden ist;
die anderen Gruppen unter R₉ bis R₁₆, die nicht mit L der chemischen Formel 1 verbunden sind, gleich oder verschieden voneinander sind und jeweils unabhängig voneinander Wasserstoff, Deuterium, ein Halogen, eine Cyanogruppe, eine Silylgruppe, eine substituierte oder unsubstituierte Alkylgruppe, eine substituierte oder unsubstituierte Cycloalkylgruppe, eine substituierte oder unsubstituierte Arylgruppe oder eine substituierte oder unsubstituierte Heteroarylgruppe sind,
mit der Maßgabe, dass in der chemischen Formel 1, wenn L eine direkte Bindung ist, Q die chemische Formel 2-2 ist, sowohl X als auch Y 0 sind und eines von R₉ bis R₁₄ und R₁₆ mit L der chemischen Formel 1 verbunden ist, wenn eines von Ar₁ und R₁₅ eine unsubstituierte Phenylgruppe ist, das andere eine substituierte oder unsubstituierte Arylgruppe mit 10 bis 60 Kohlenstoffatomen oder eine substituierte oder unsubstituierte Heteroarylgruppe mit 2 bis 60 Kohlenstoffatomen ist.

2. Verbindung nach Anspruch 1, wobei R₁ bis R₈ gleich oder verschieden voneinander sind und jeweils unabhängig voneinander Wasserstoff oder Deuterium sind.

3. Verbindung nach Anspruch 1, wobei Ar₁ eine substituierte oder unsubstituierte Phenylgruppe, eine substituierte oder unsubstituierte Biphenylgruppe; eine substituierte oder unsubstituierte Naphthylgruppe, eine substituierte oder unsubstituierte Phenanthrengruppe, eine substituierte oder unsubstituierte Dibenzofurangruppe, eine substituierte oder unsubstituierte Dibenzothiophengruppe oder eine substituierte oder unsubstituierte Carbazolgruppe ist; und
der Begriff "substituiert oder unsubstituiert" bedeutet, dass sie unsubstituiert oder mit einem oder mehreren Substituenten substituiert sind, die aus der Gruppe ausgewählt sind, die aus Deuterium, einer Halogengruppe, einer Alkylgruppe, die 1 bis 60 Kohlenstoffatome aufweist, einer Arylgruppe, die 6 bis 60 Kohlenstoffatome aufweist, und einer Heteroarylgruppe, die 2 bis 60 Kohlenstoffatome aufweist, besteht, oder dass ein oder mehrere benachbarte Substituenten aneinander gebunden sind, um einen Ring zu bilden.

4. Verbindung nach Anspruch 1, wobei L eine direkte Bindung; eine Phenylengruppe, die unsubstituiert oder mit Deuterium substituiert ist; oder eine Naphthylengruppe, die unsubstituiert oder mit Deuterium substituiert ist, ist.

5. Verbindung nach Anspruch 1, wobei die chemische Formel 2-1 eine der folgenden chemischen Formeln 2-1-A bis 2-1-D ist: 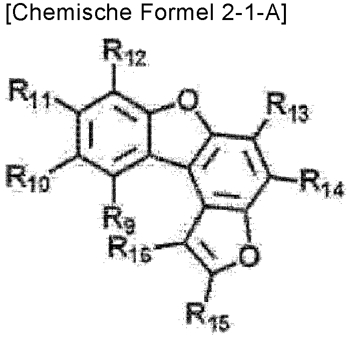 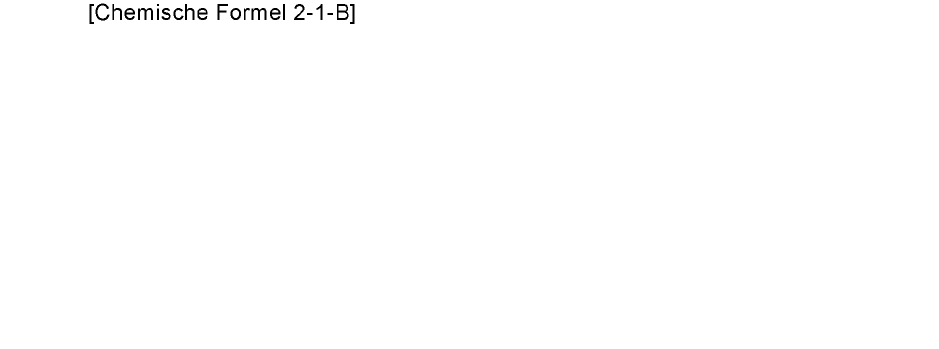 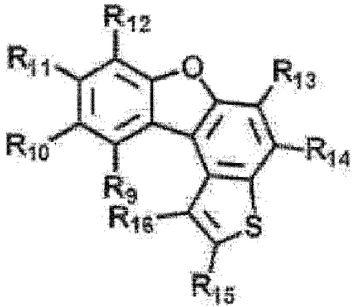 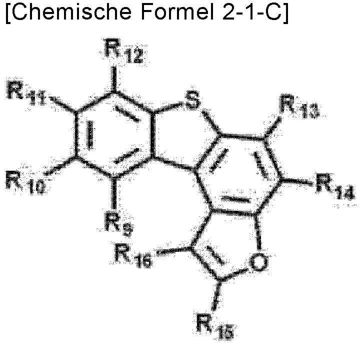 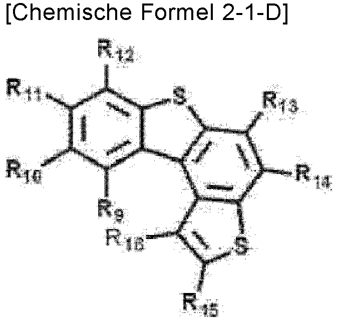 wobei in den chemischen Formeln 2-1-A bis 2-1-D,
R₉ bis R₁₆ die gleichen sind, wie in den chemischen Formeln 2-1 bis 2-6 definiert.

6. Verbindung nach Anspruch 1, wobei die chemische Formel 2-2 eine der folgenden chemischen Formeln 2-2-A bis 2-2-D ist: 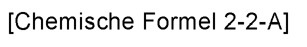 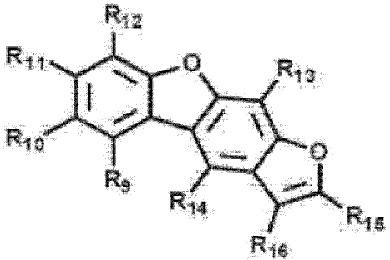 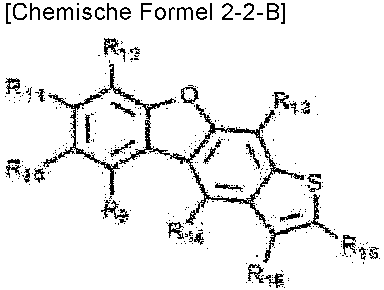 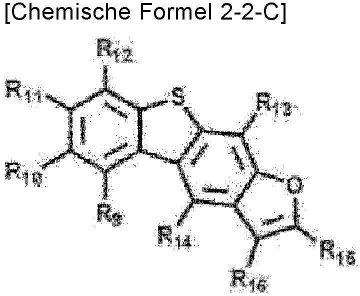 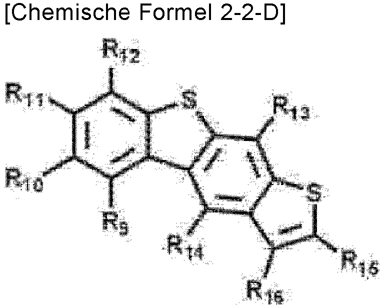 wobei in den chemischen Formeln 2-2-A bis 2-2-D,
R₉ bis R₁₆ die gleichen sind, wie in den chemischen Formeln 2-1 bis 2-6 definiert.

7. Verbindung nach Anspruch 1, wobei die chemische Formel 2-3 eine der folgenden chemischen Formeln 2-3-A bis 2-3-D ist: 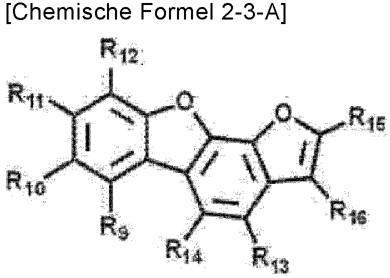 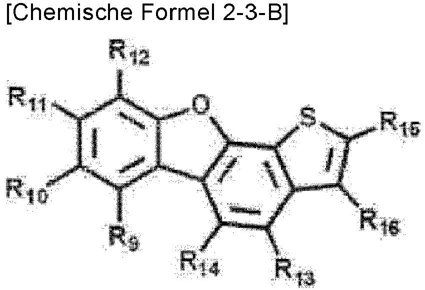 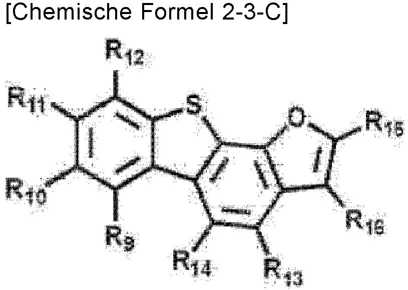 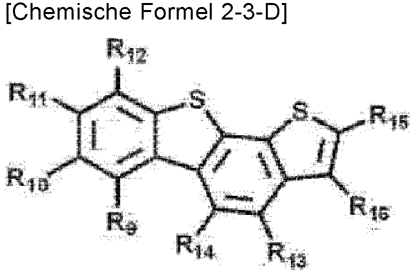 wobei in den chemischen Formeln 2-3-A bis 2-3-D,
R₉ bis R₁₆ die gleichen sind, wie in den chemischen Formeln 2-1 bis 2-6 definiert.

8. Verbindung nach Anspruch 1, wobei die chemische Formel 2-4 eine der folgenden chemischen Formeln 2-4-A bis 2-4-D ist: 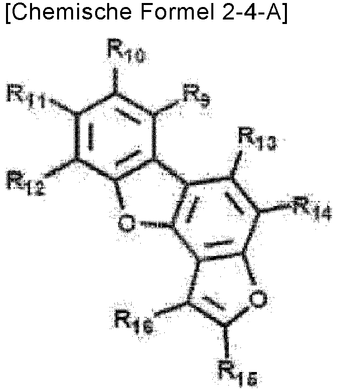 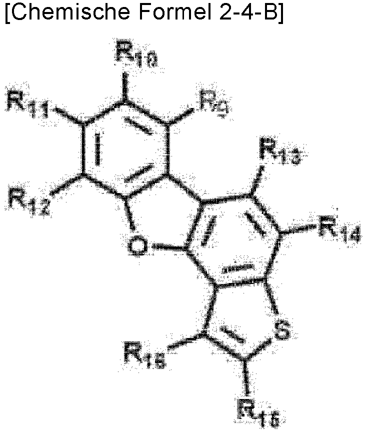 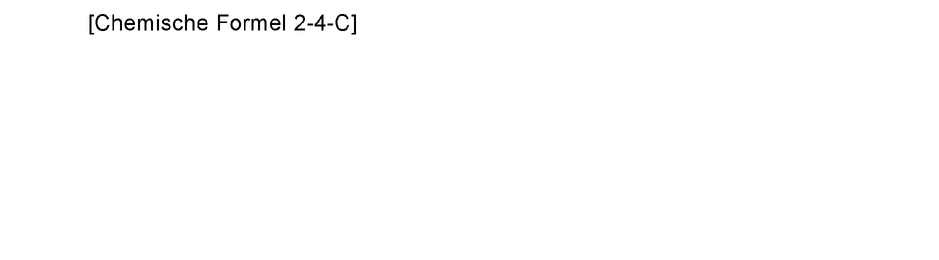 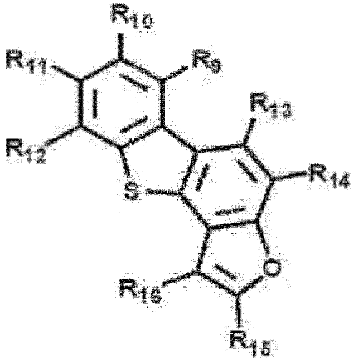 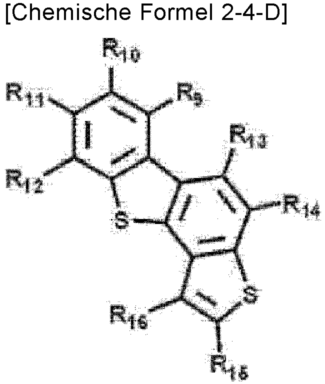 wobei in den chemischen Formeln 2-4-A bis 2-4-D,
R₉ bis R₁₆ die gleichen sind, wie in den chemischen Formeln 2-1 bis 2-6 definiert.

9. Verbindung nach Anspruch 1, wobei die chemische Formel 2-5 eine der folgenden chemischen Formeln 2-5-A bis 2-5-D ist: 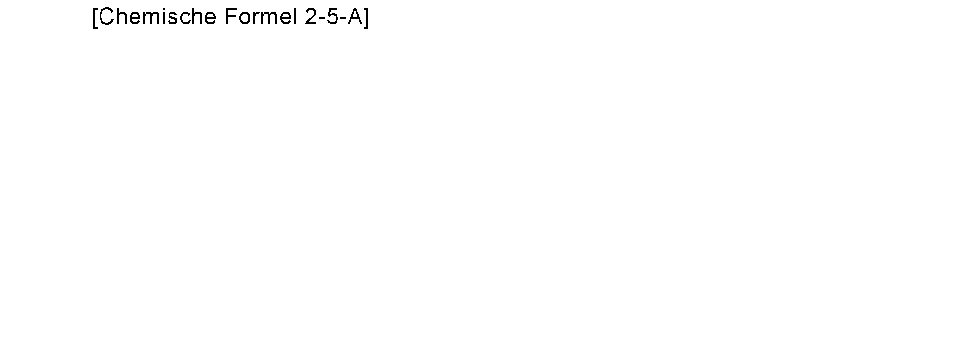 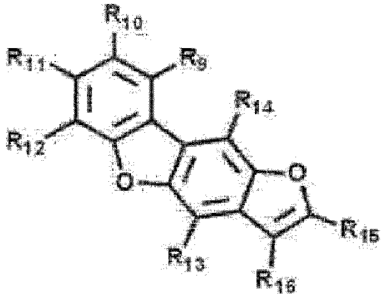 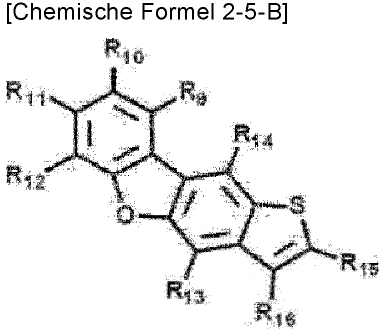 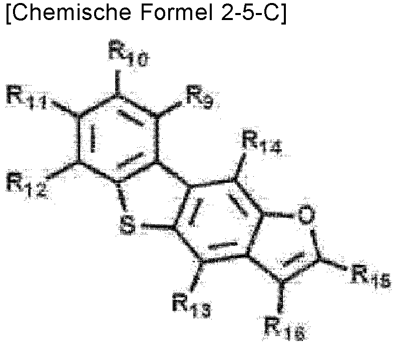 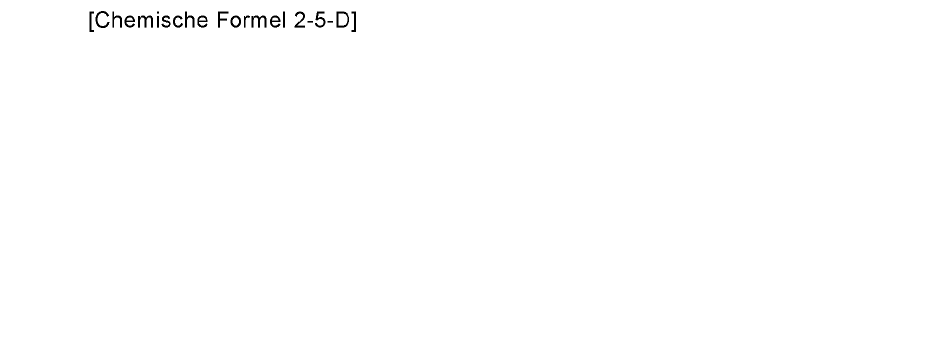 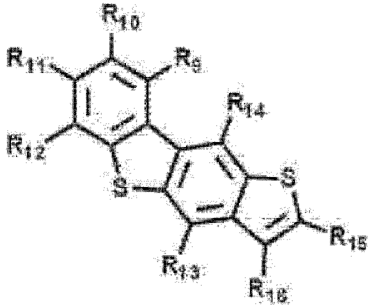 wobei in den chemischen Formeln 2-5-A bis 2-5-D,
R₉ bis R₁₆ die gleichen sind, wie in den chemischen Formeln 2-1 bis 2-6 definiert.

10. Verbindung nach Anspruch 1, wobei die chemische Formel 2-6 eine der folgenden chemischen Formeln 2-6-A bis 2-6-D ist: 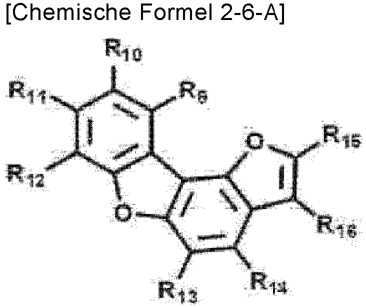 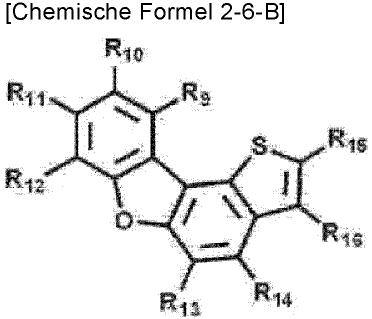 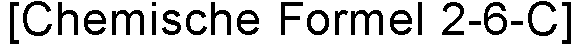 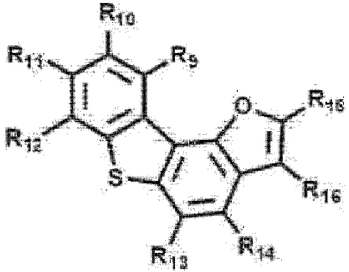 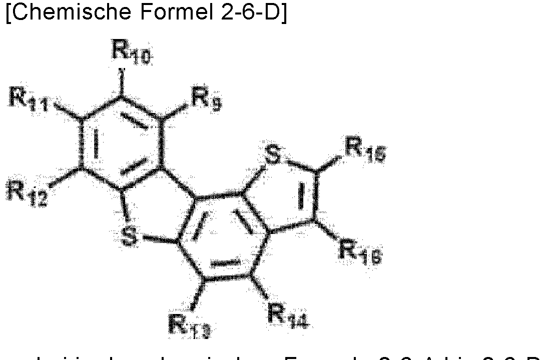 wobei in den chemischen Formeln 2-6-A bis 2-6-D
R₉ bis R₁₆ die gleichen sind, wie in den chemischen Formeln 2-1 bis 2-6 definiert.

11. Verbindung nach Anspruch 1, wobei in der chemischen Formel 1, wenn L eine direkte Bindung ist, Q die chemische Formel 2-2 ist, sowohl X als auch Y 0 sind und einer von R₉ bis R₁₄ und R16 mit L der chemischen Formel 1 verbunden ist, wenn einer von Ar₁ und R₁₅ eine unsubstituierte Phenylgruppe ist, der andere eine substituierte oder unsubstituierte Biphenylgruppe, eine substituierte oder unsubstituierte Naphthylgruppe, eine substituierte oder unsubstituierte Dibenzofurangruppe oder eine substituierte oder unsubstituierte Dibenzothiophengruppe ist, und
der Begriff "substituiert oder unsubstituiert" unsubstituiert oder mit einem oder mehreren Substituenten substituiert bedeutet, die aus der Gruppe ausgewählt sind, die aus Deuterium, einer Halogengruppe, einer Alkylgruppe, die 1 bis 60 Kohlenstoffatome aufweist, einer Arylgruppe, die 6 bis 60 Kohlenstoffatome aufweist, und einer Heteroarylgruppem die 2 bis 60 Kohlenstoffatome aufweist, besteht, oder bedeutet, dass ein oder mehrere benachbarte Substituenten aneinander gebunden sind, um einen Ring zu bilden.

12. Verbindung nach Anspruch 1, wobei die anderen Gruppen unter R₉ bis R₁₆, die nicht mit L der chemischen Formel 1 verlinkt sind, gleich oder verschieden voneinander sind und jeweils unabhängig voneinander Wasserstoff, Deuterium, eine substituierte oder unsubstituierte Phenylgruppe, eine substituierte oder unsubstituierte Biphenylgruppe, eine substituierte oder unsubstituierte Naphthylgruppe, eine substituierte oder unsubstituierte Dibenzofurangruppe oder eine substituierte oder unsubstituierte Dibenzothiophengruppe sind, und
der Begriff "substituiert oder unsubstituiert" mit einem oder mehreren Substituenten substituiert bedeutet, die aus der Gruppe ausgewählt sind, die aus Deuterium, einer Halogengruppe, einer Alkylgruppe, die 1 bis 60 Kohlenstoffatome aufweist; einer Arylgruppe, die 6 bis 60 Kohlenstoffatome aufweist; und einer Heteroarylgruppe, die 2 bis 60 Kohlenstoffatome aufweist, besteht, oder bedeutet, dass ein oder mehrere benachbarte Substituenten aneinander gebunden sind, um einen Ring zu bilden.

13. Verbindung nach Anspruch 1, wobei die Verbindung der chemischen Formel 1 eine der folgenden Verbindungen ist:

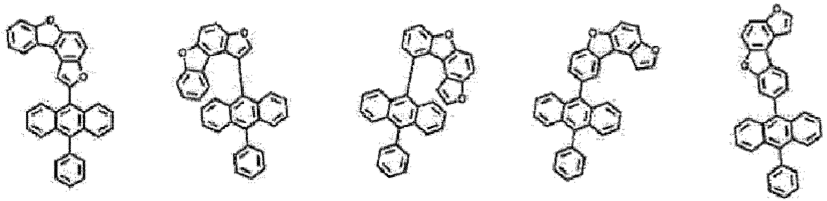

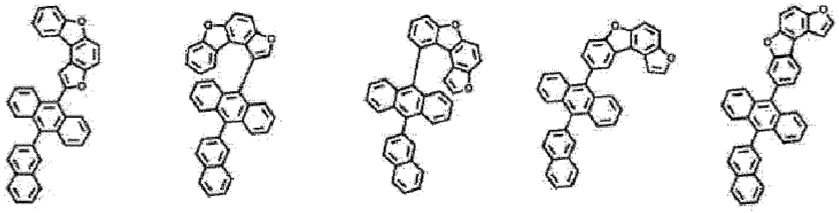

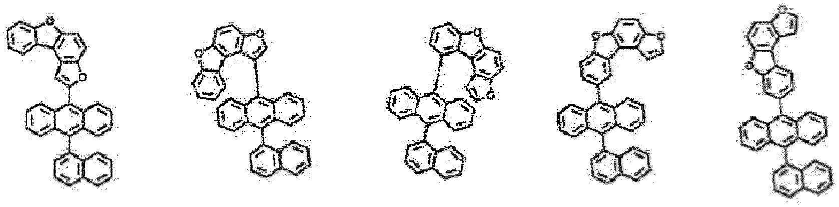

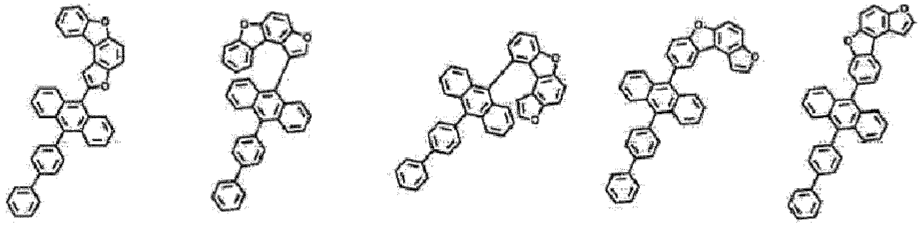

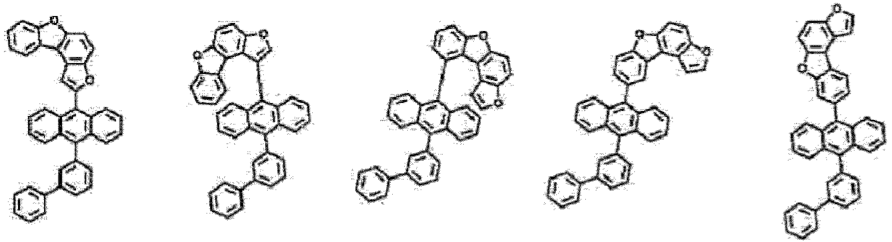

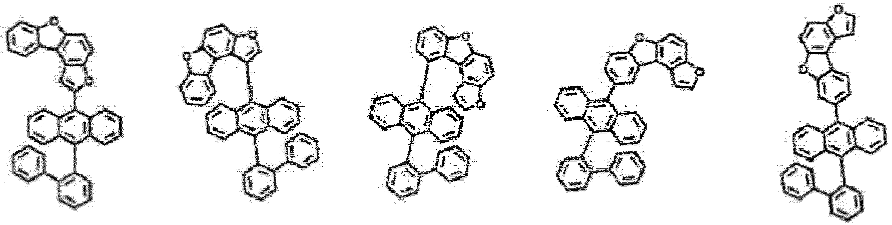

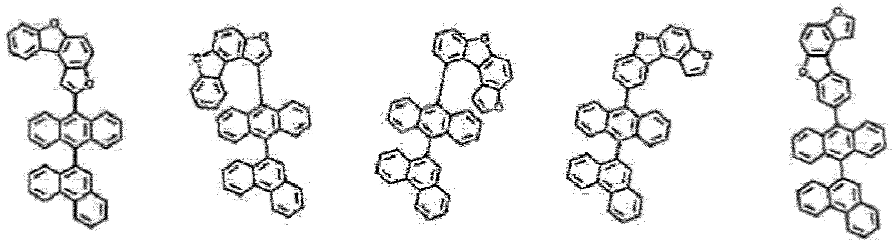

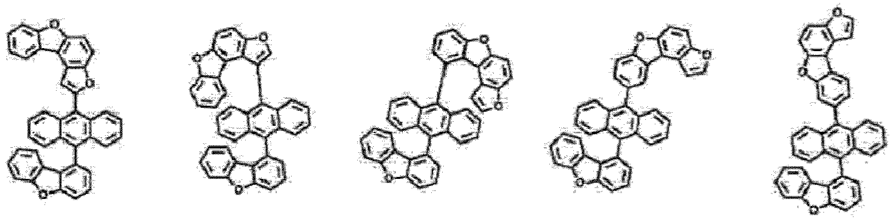

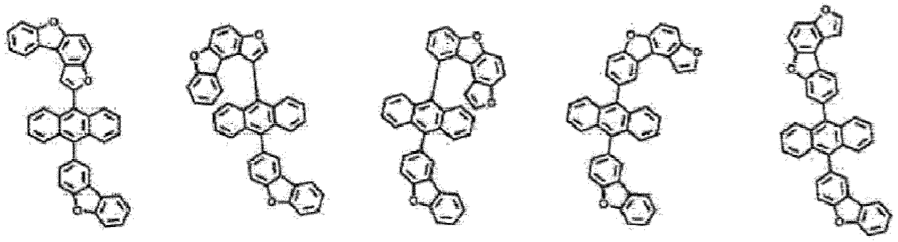

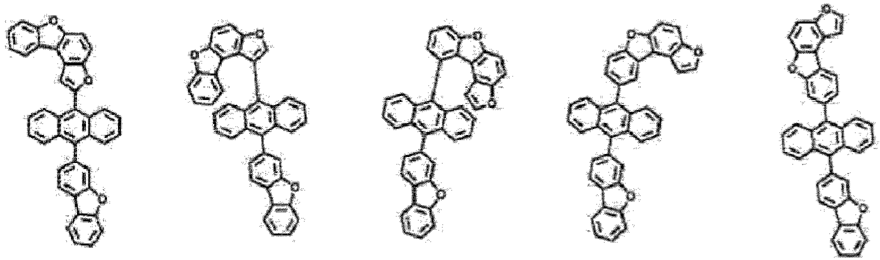

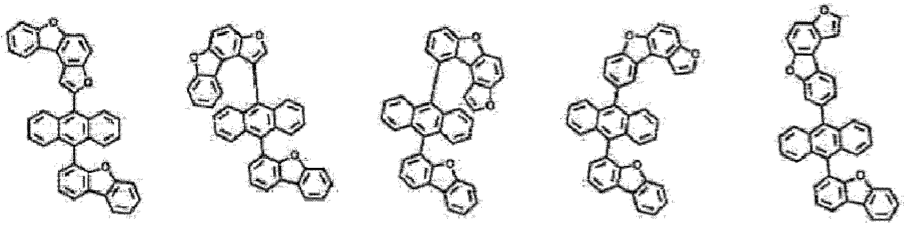

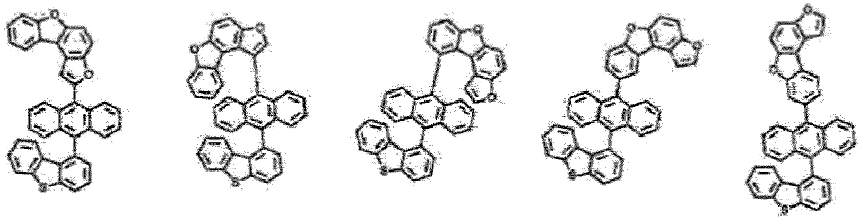

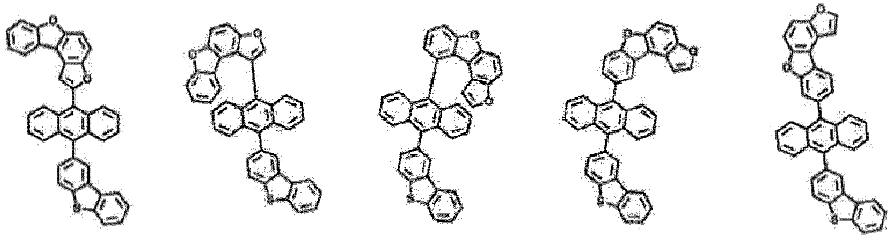

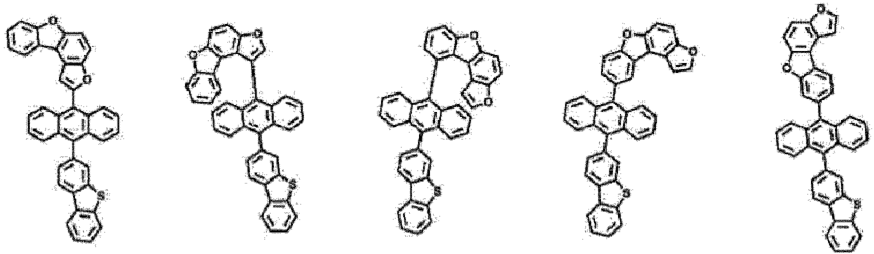

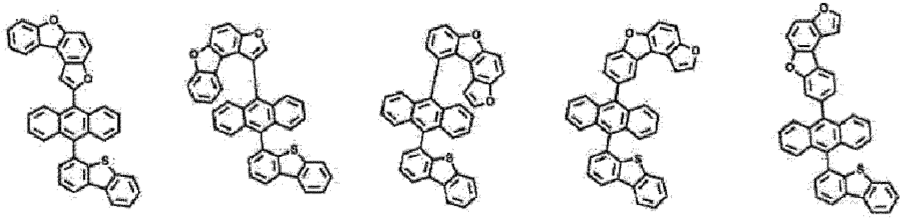

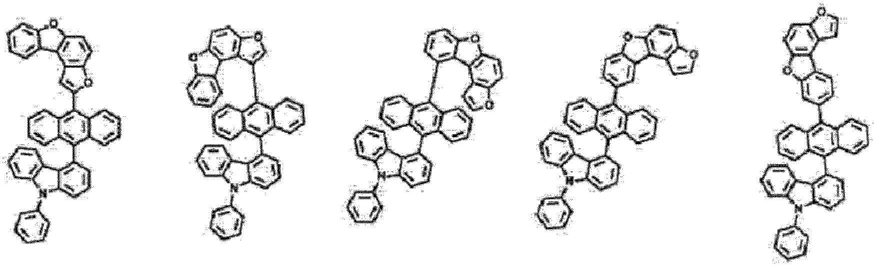

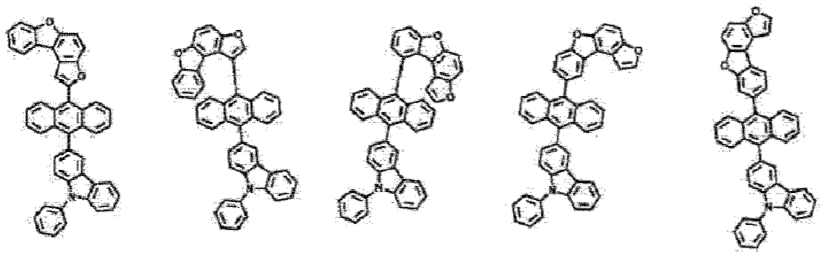

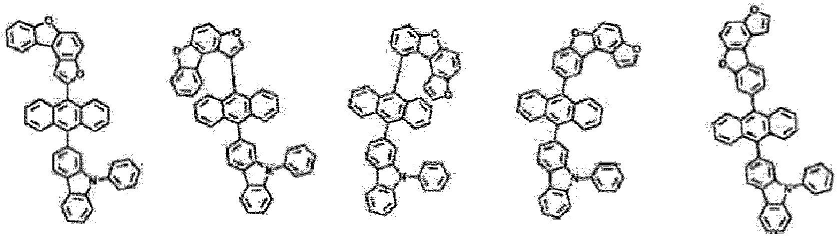

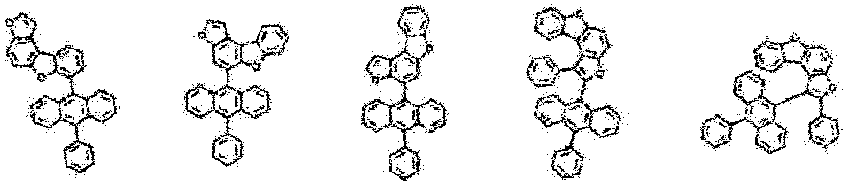

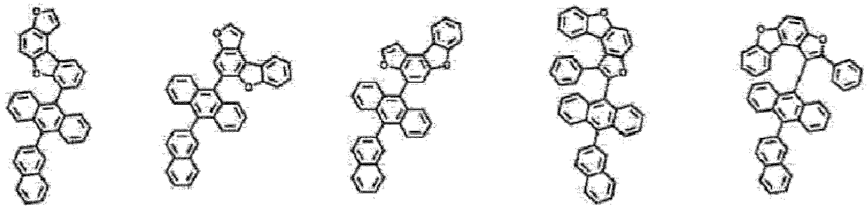

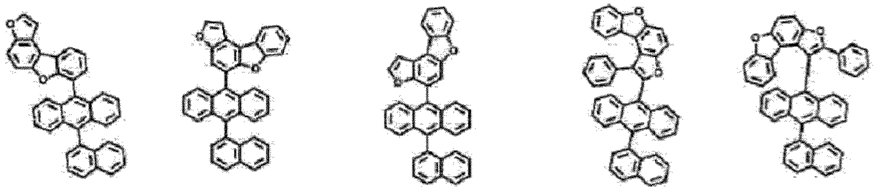

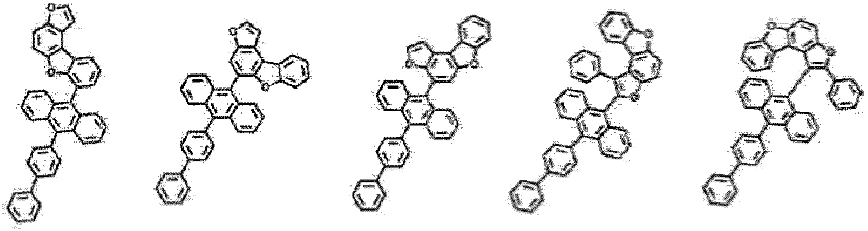

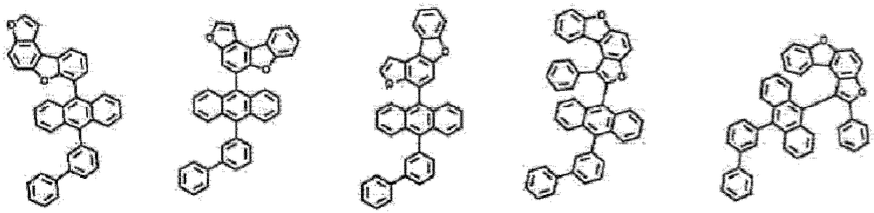

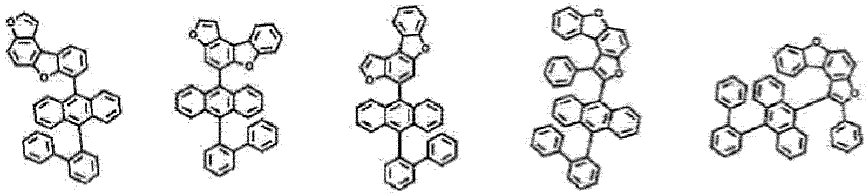

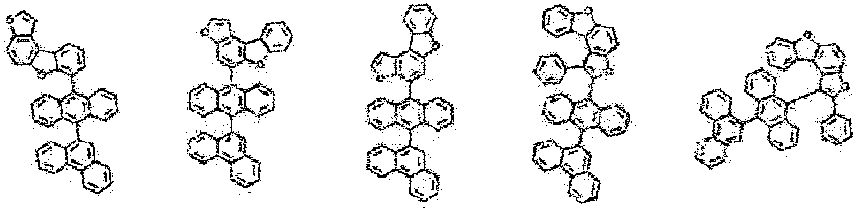

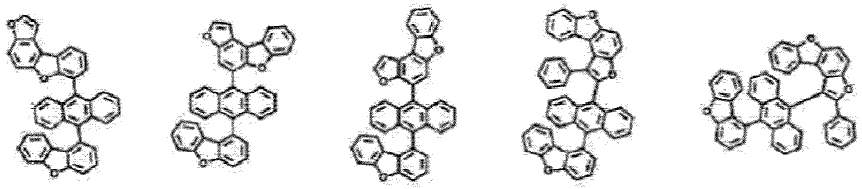

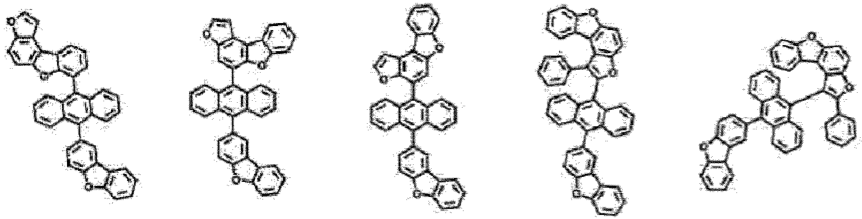

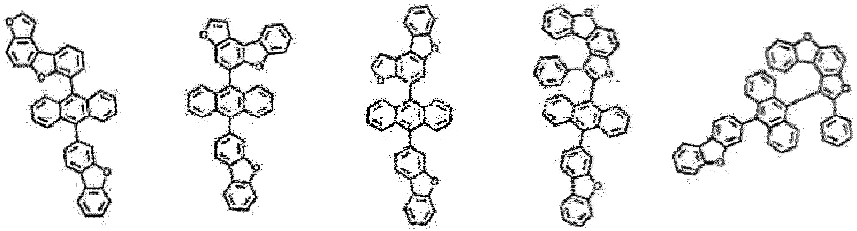

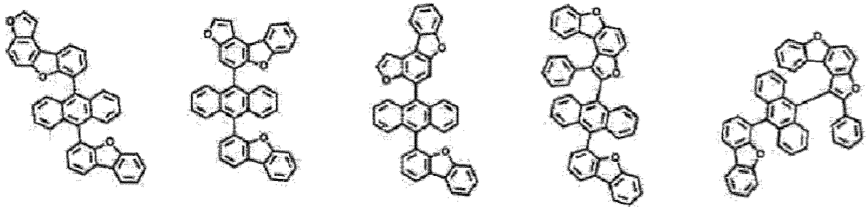

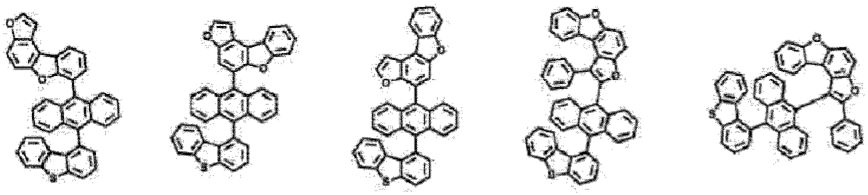

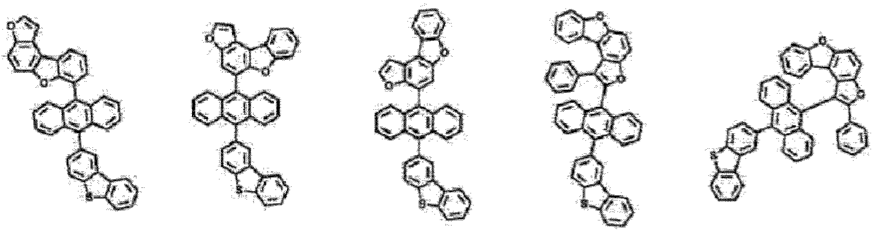

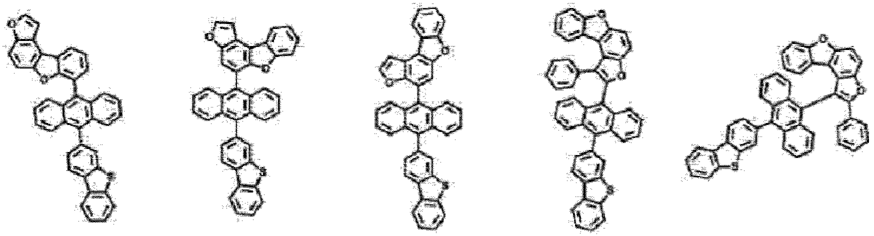

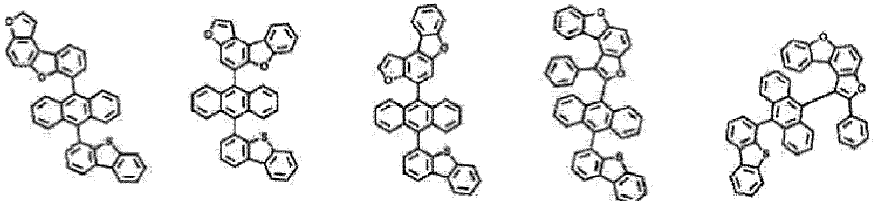

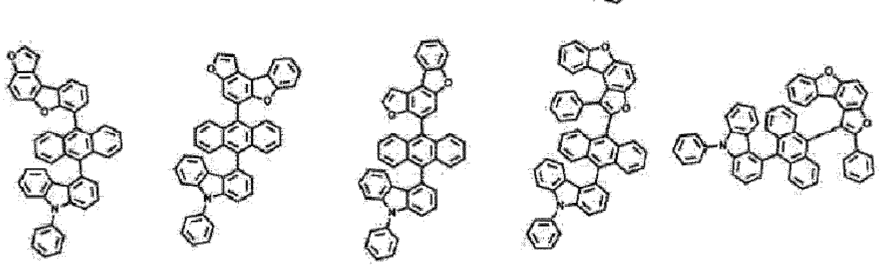

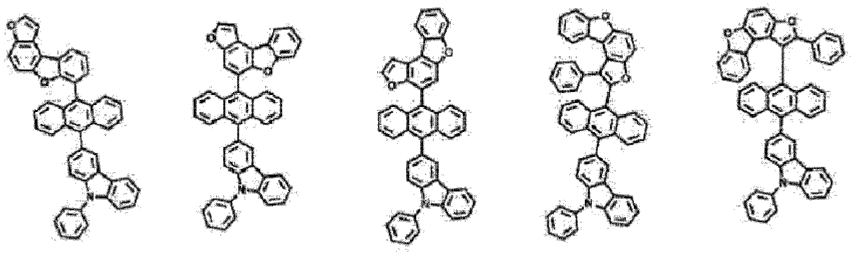

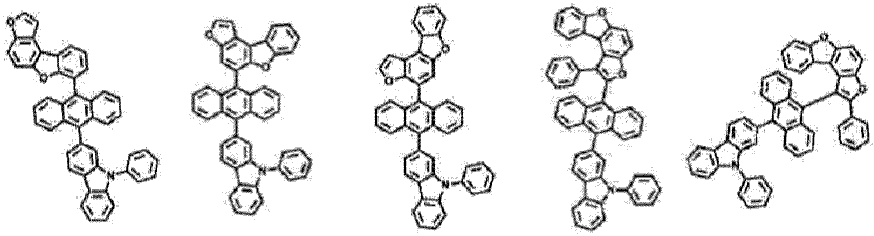

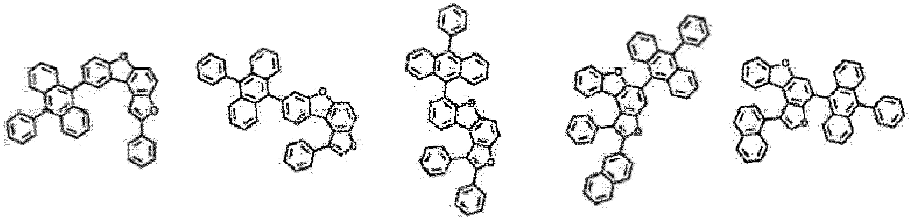

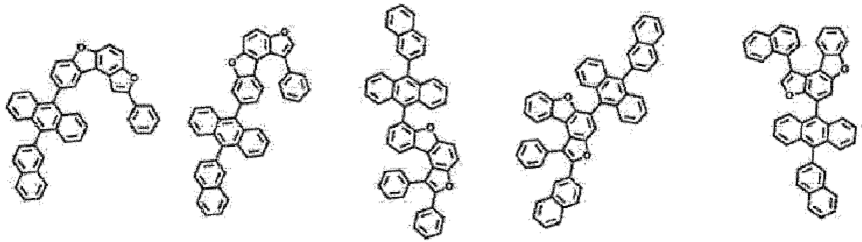

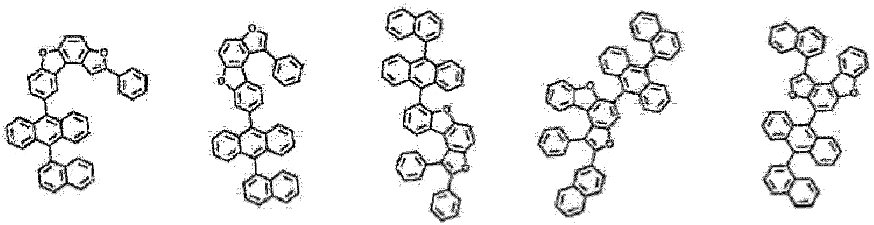

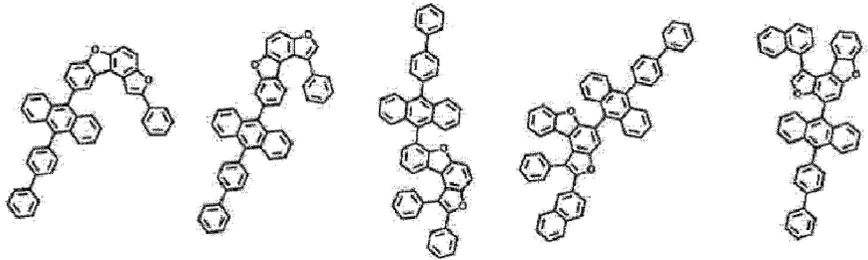

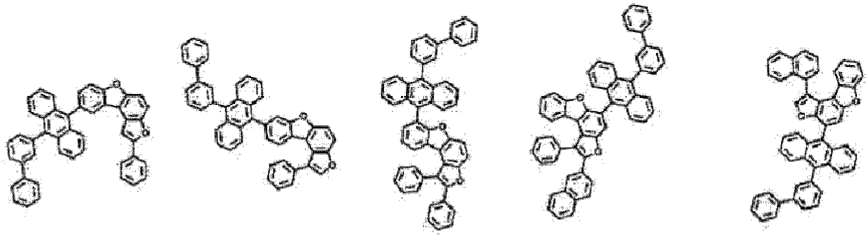

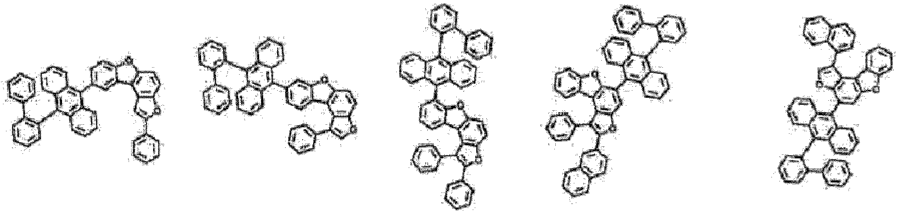

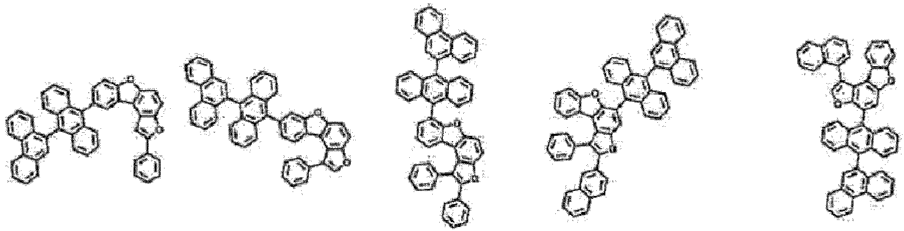

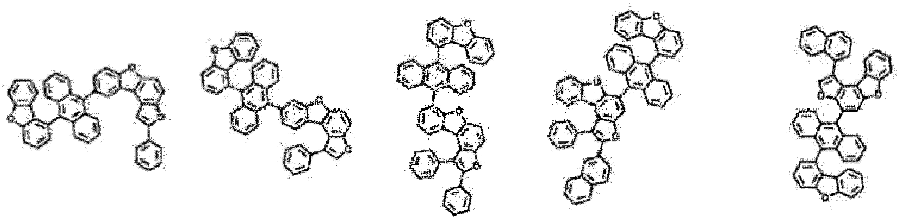

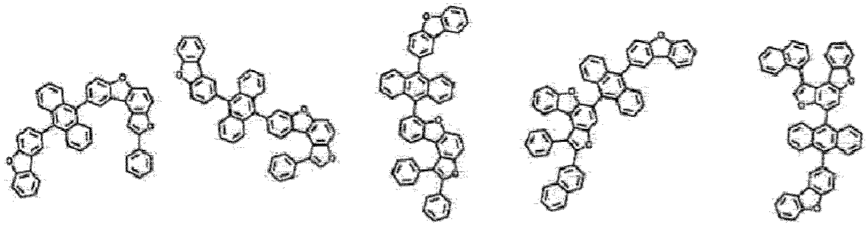

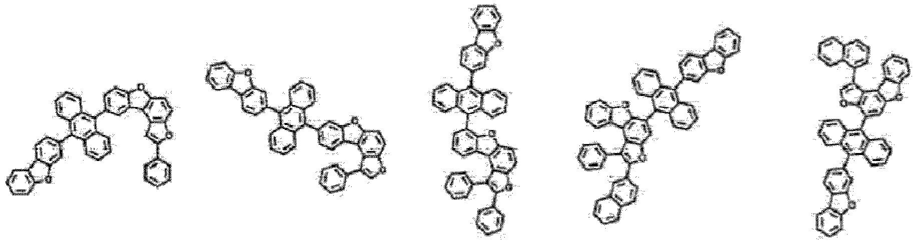

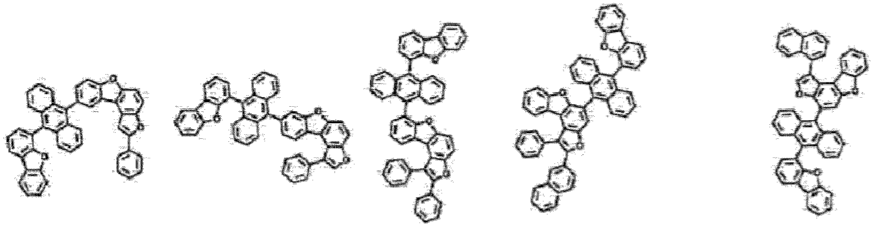

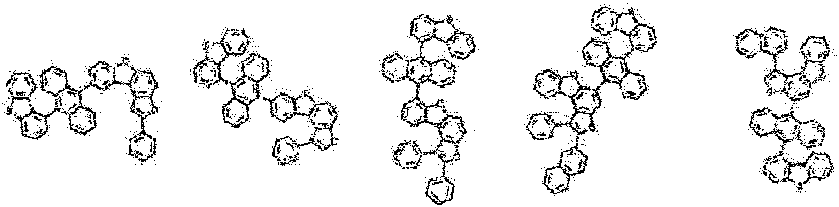

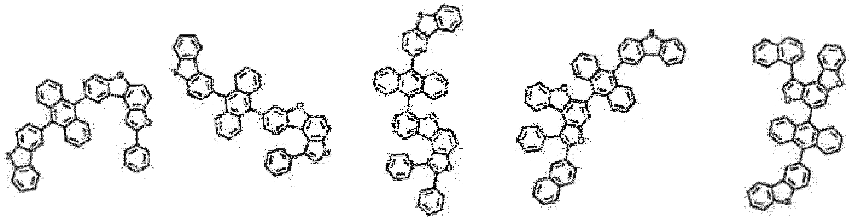

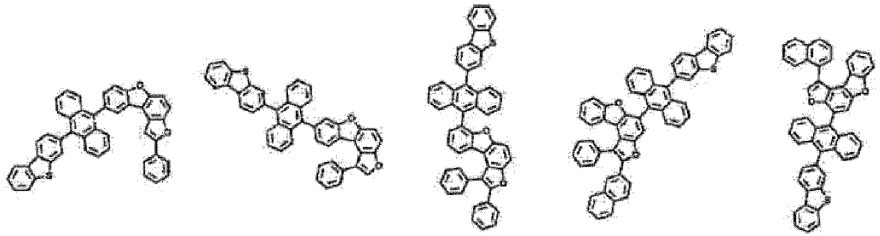

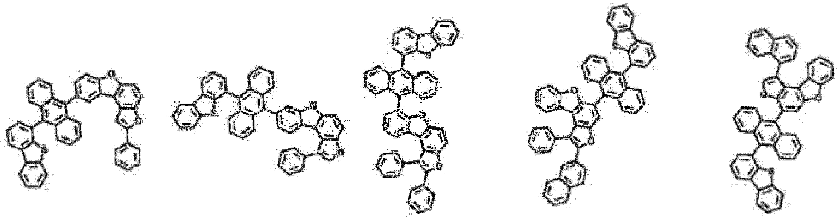

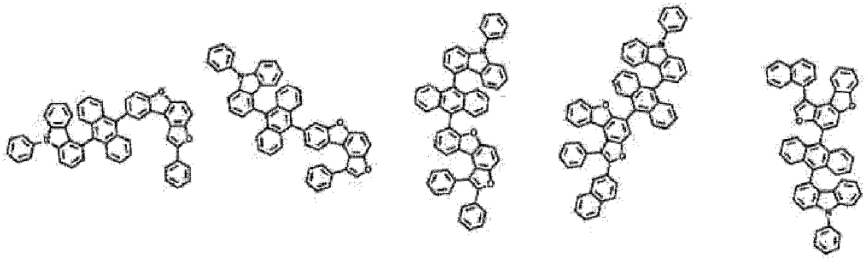

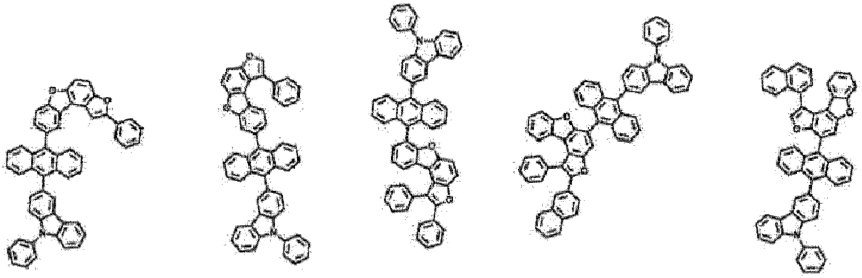

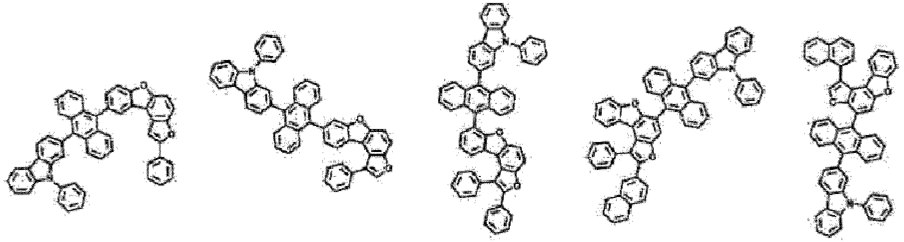

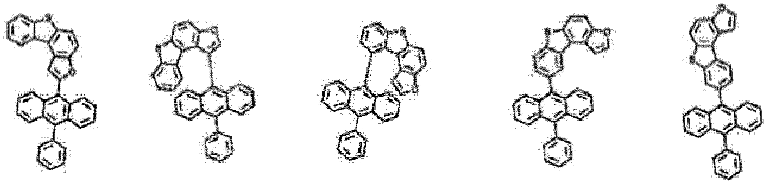

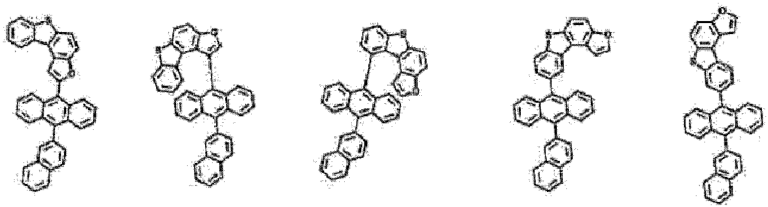

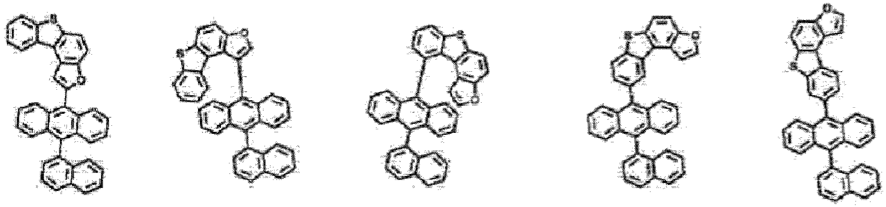

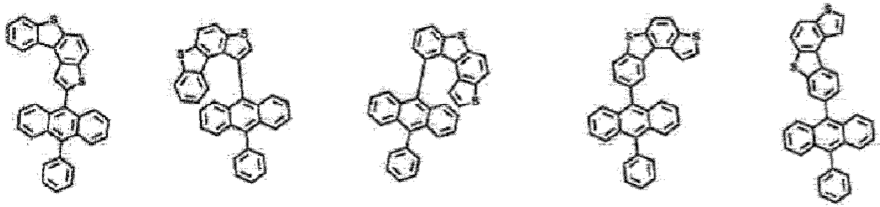

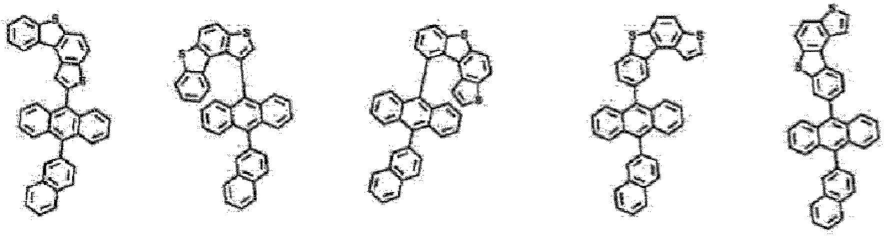

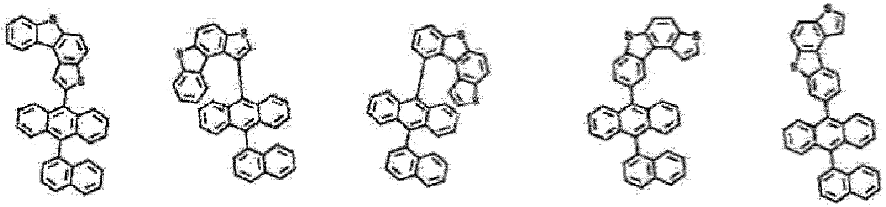

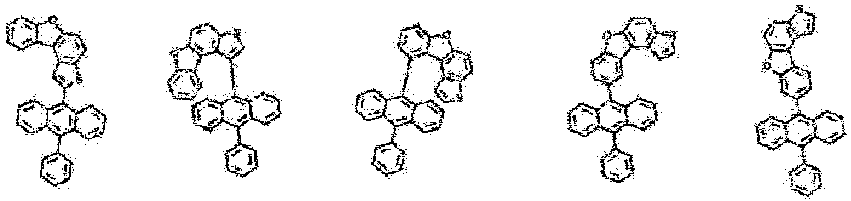

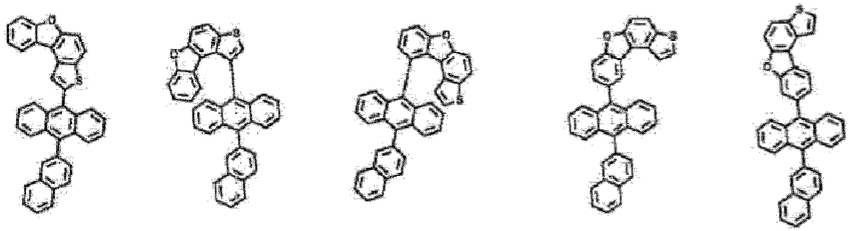

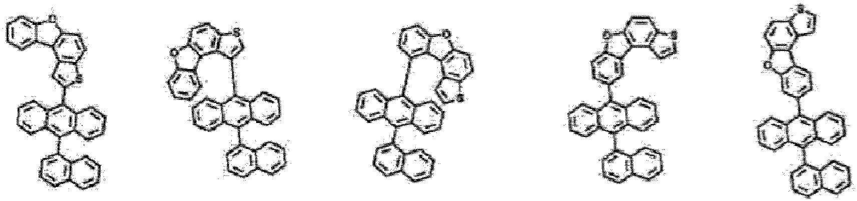

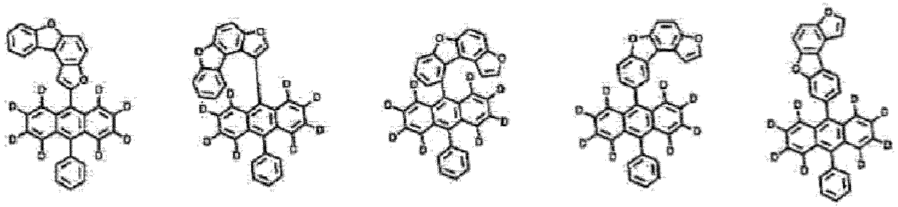

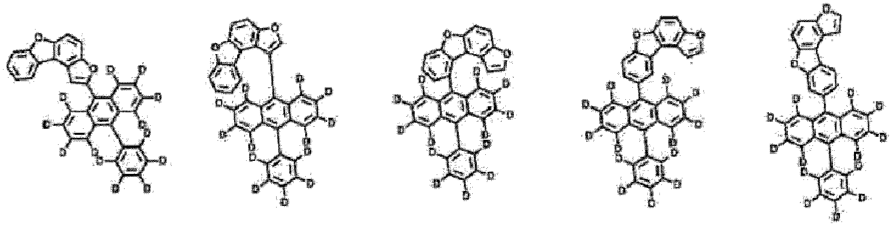

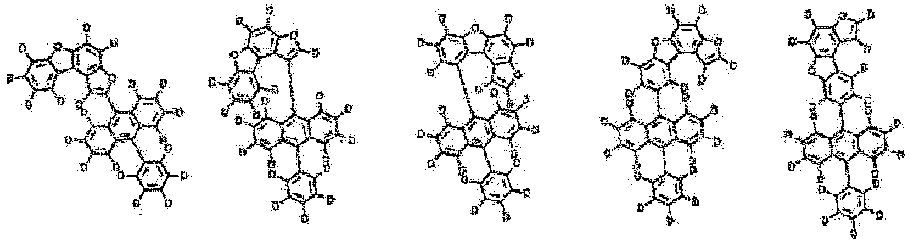

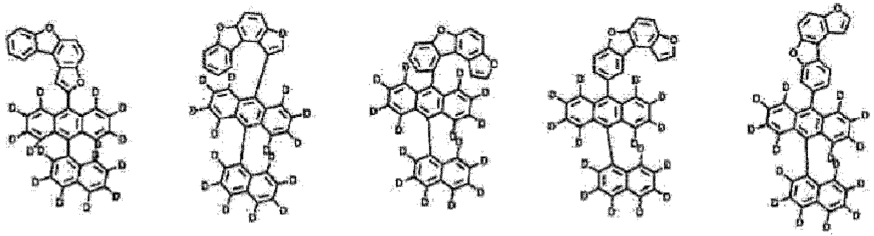

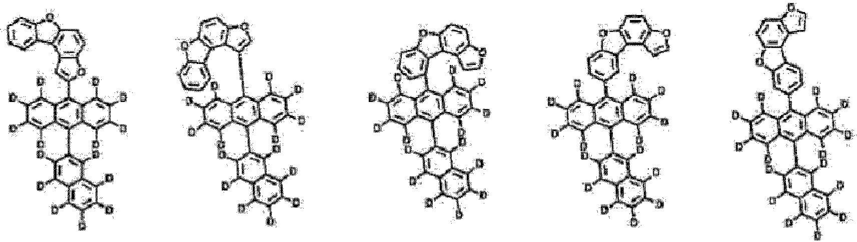

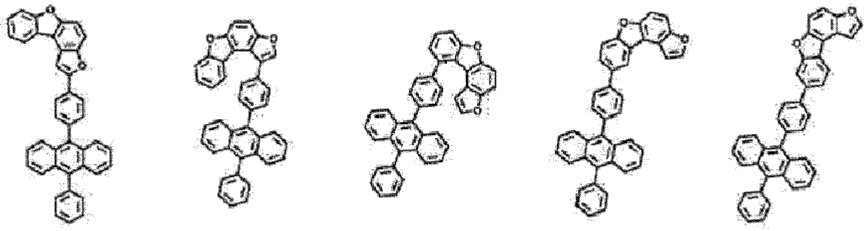

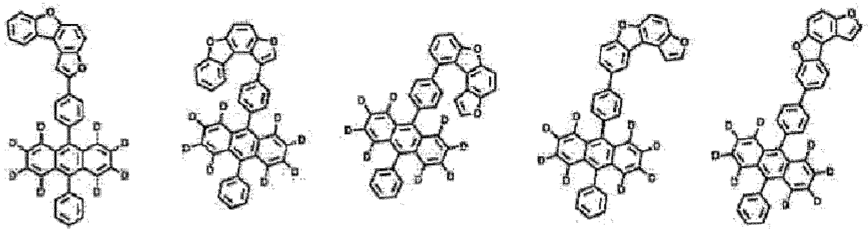

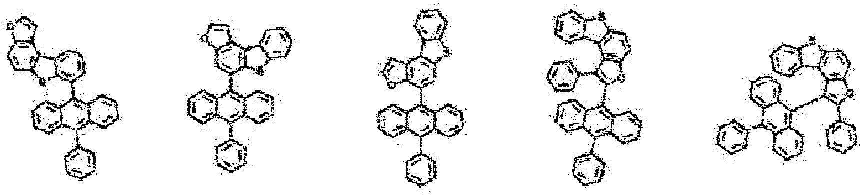

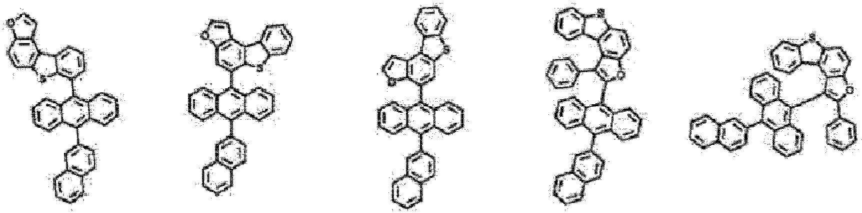

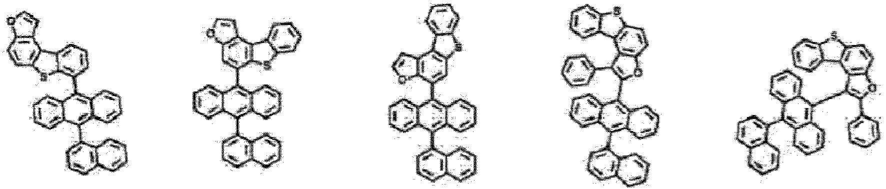

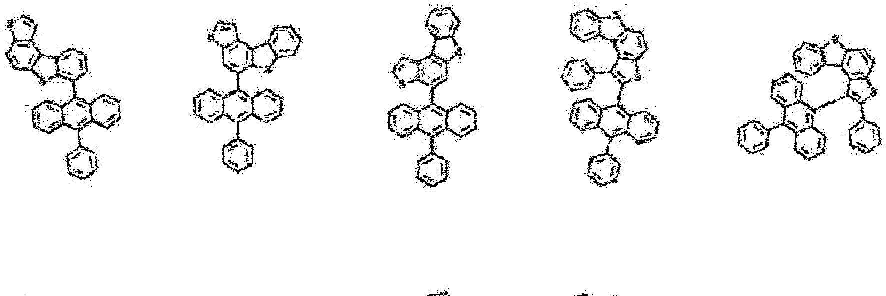

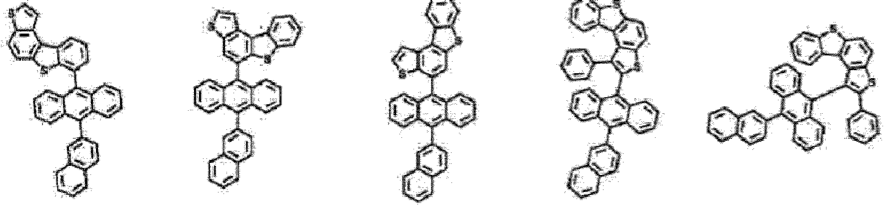

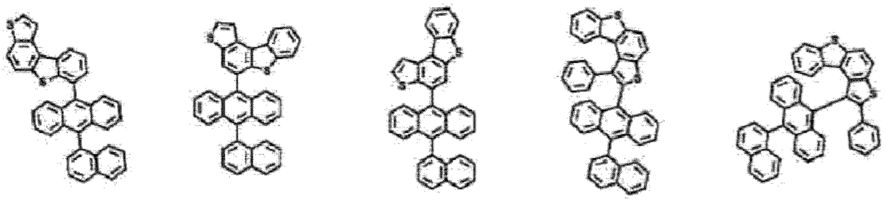

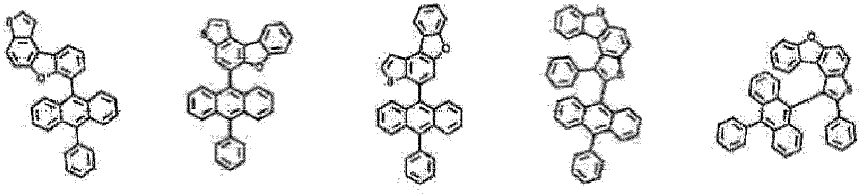

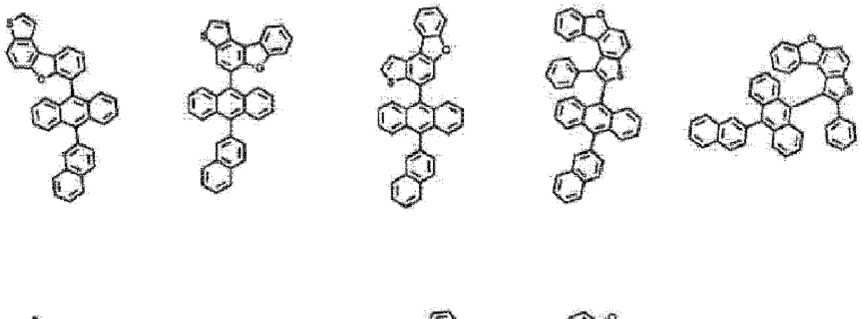

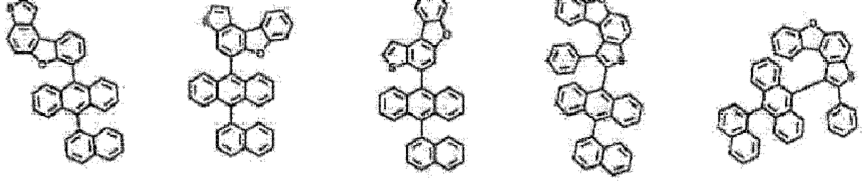

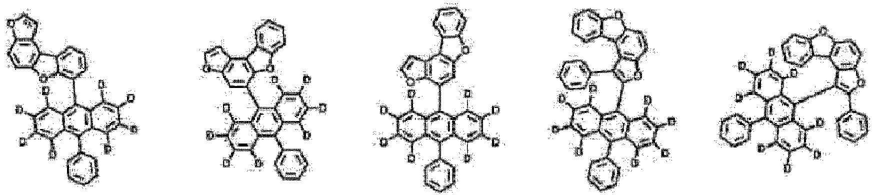

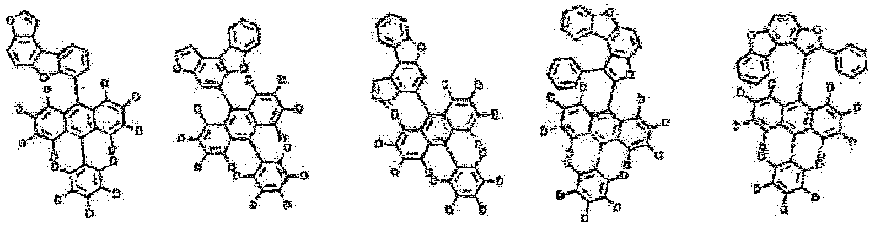

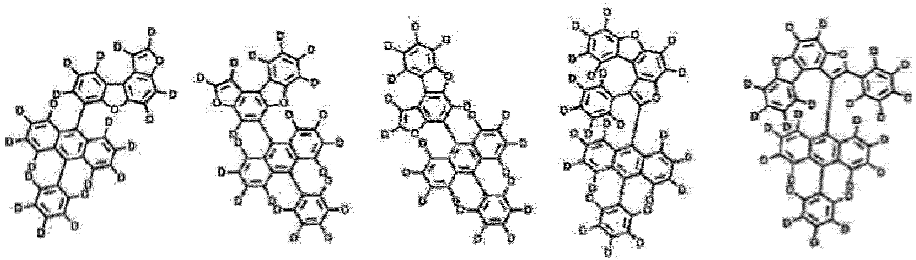

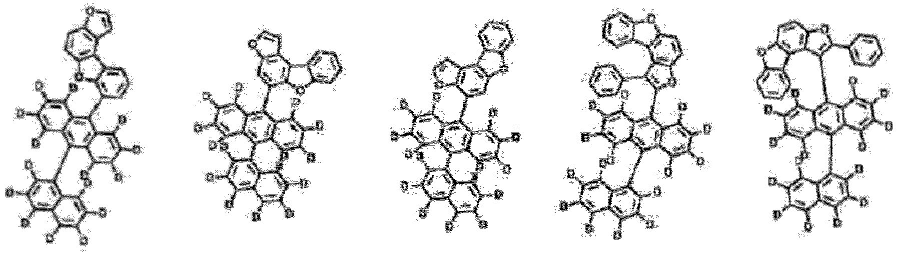

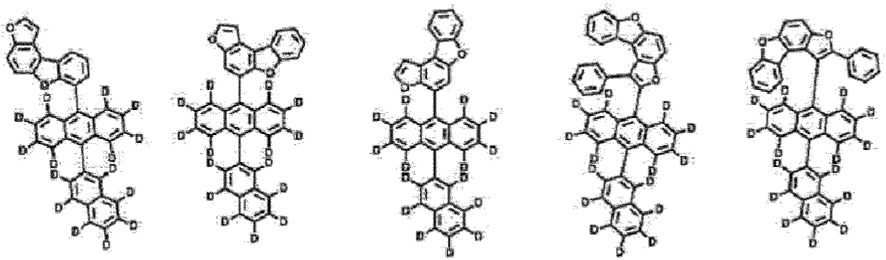

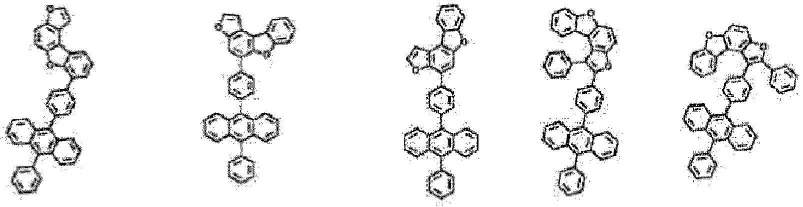

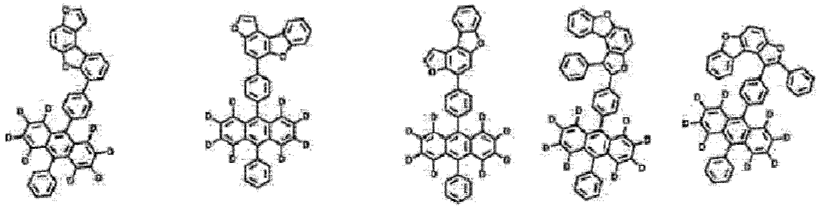

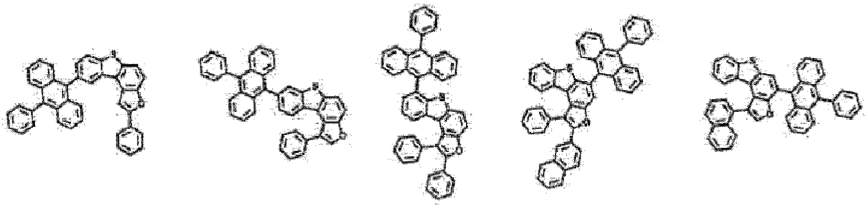

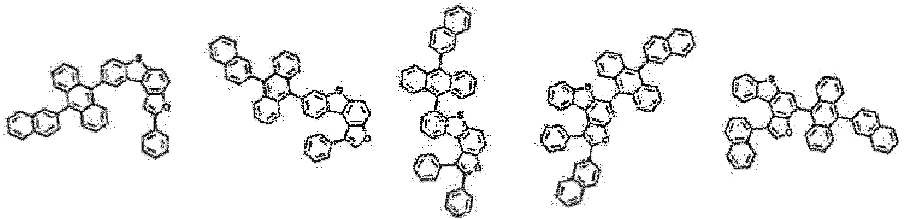

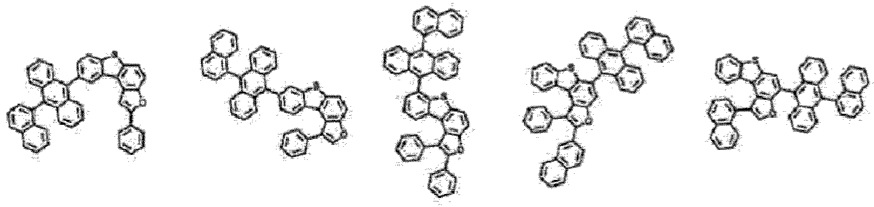

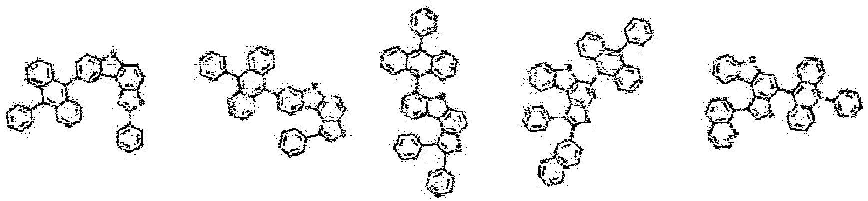

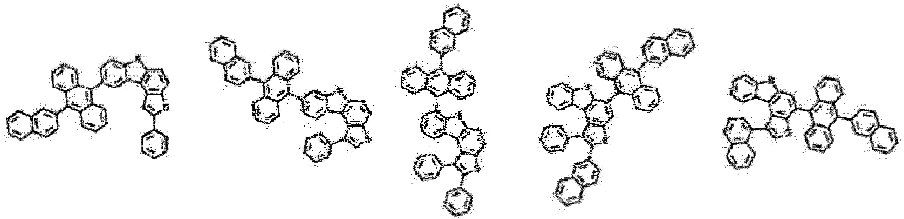

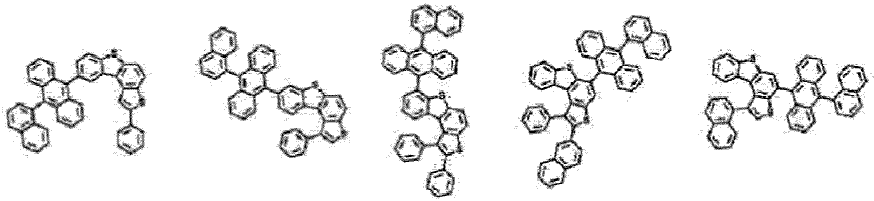

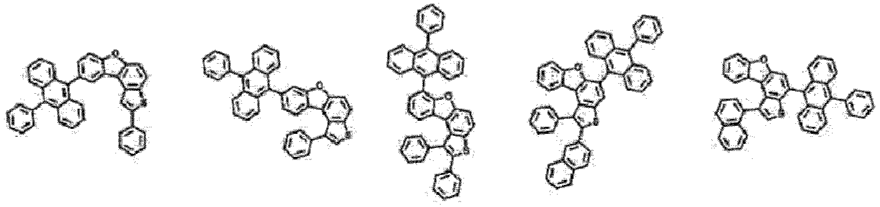

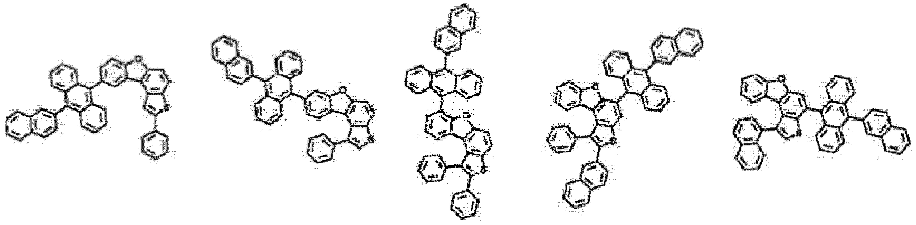

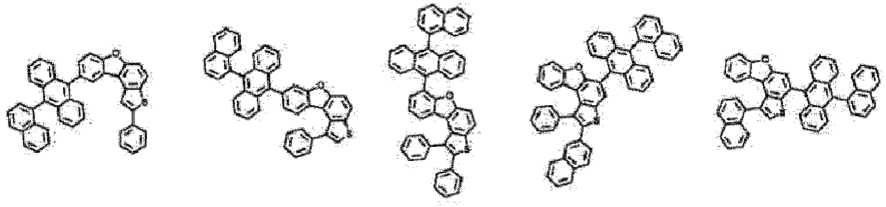

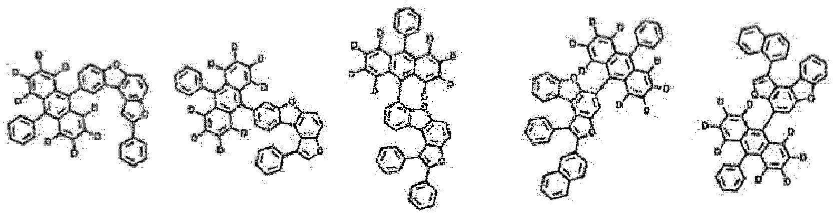

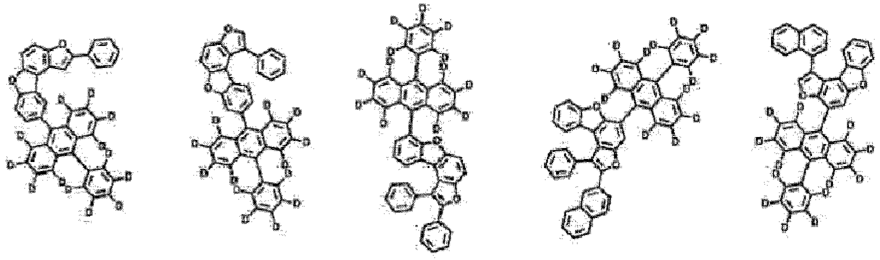

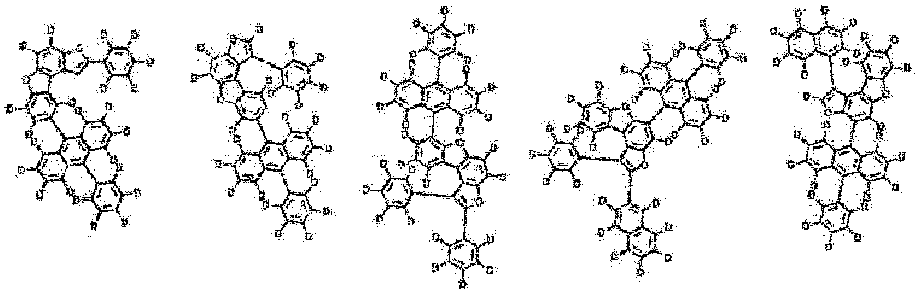

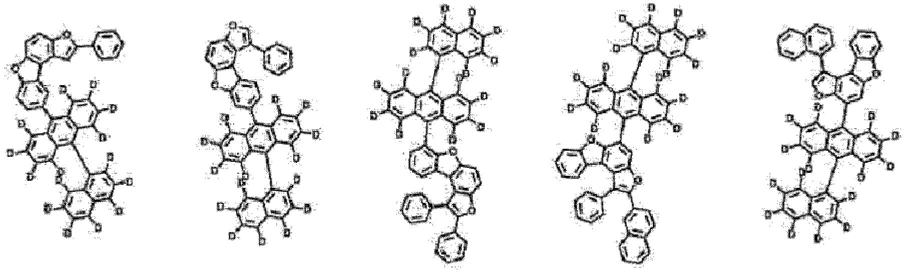

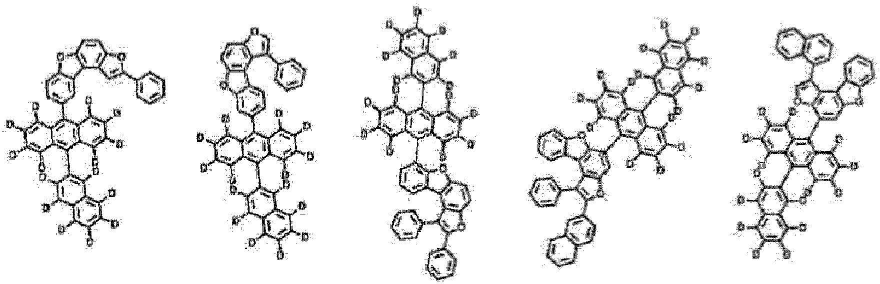

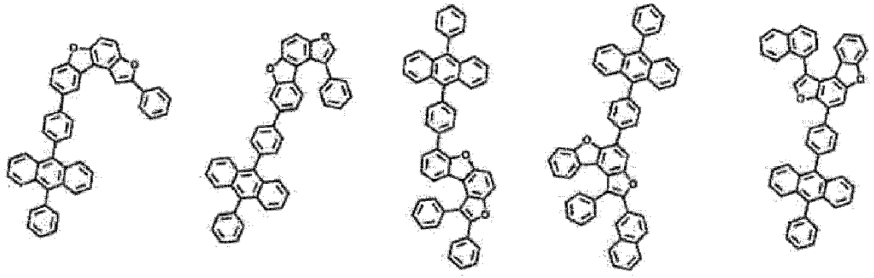

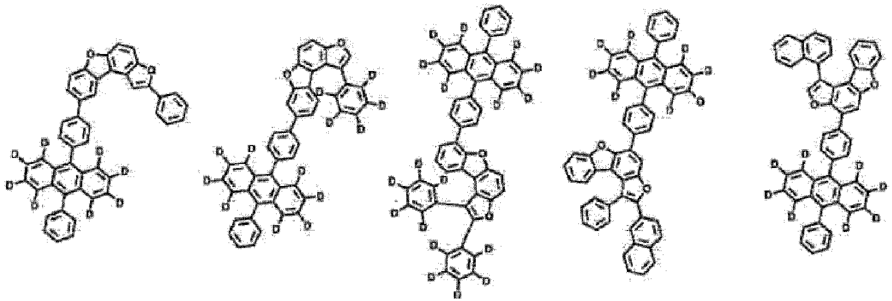

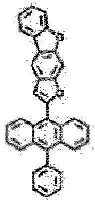

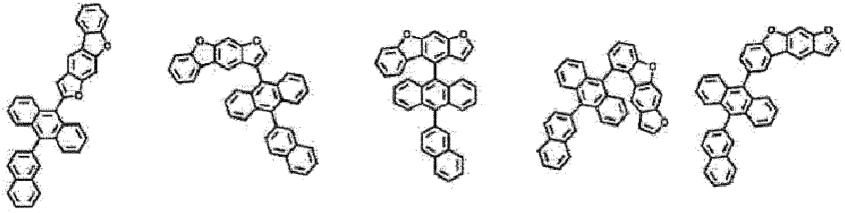

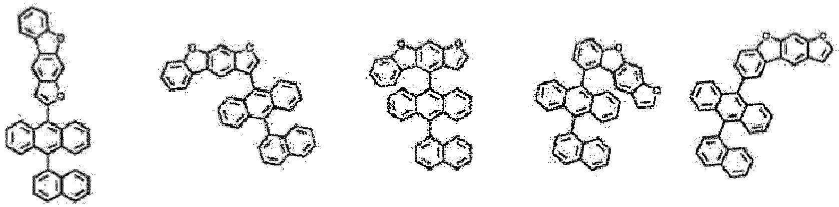

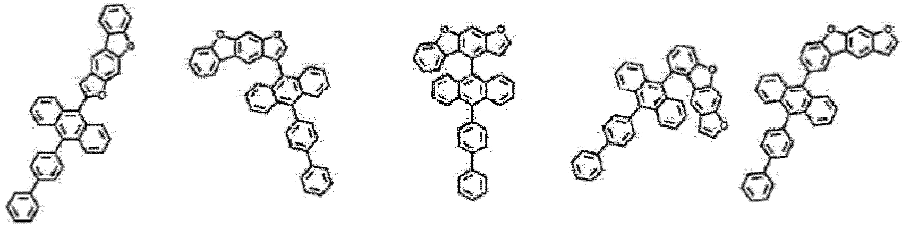

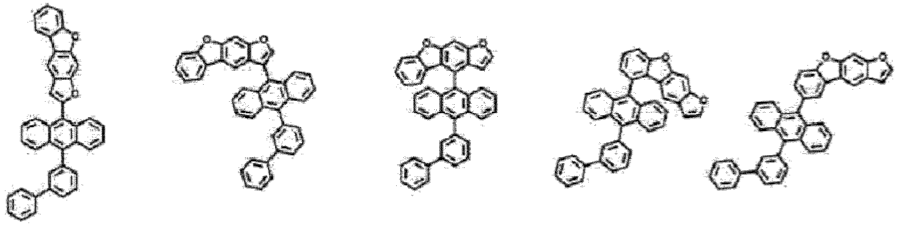

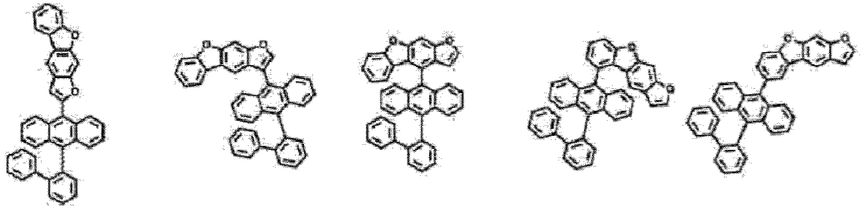

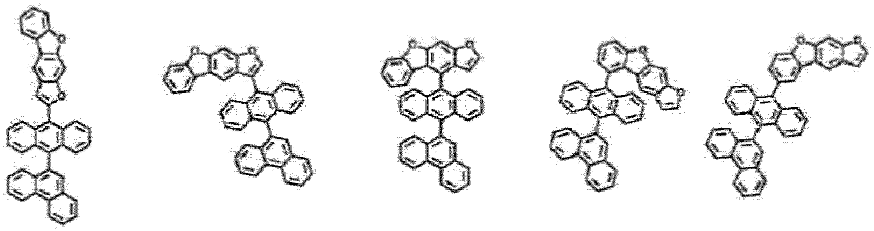

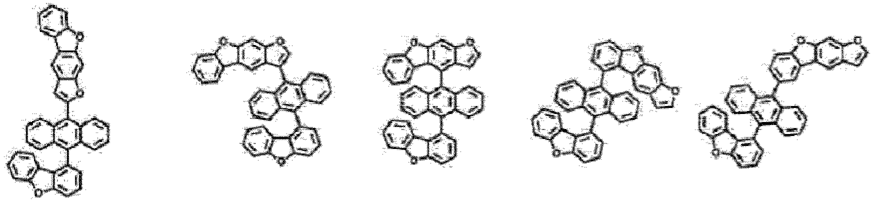

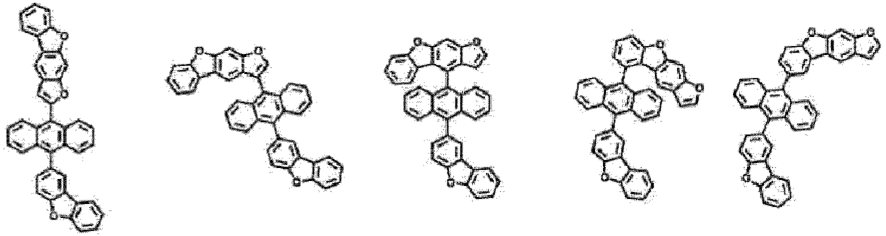

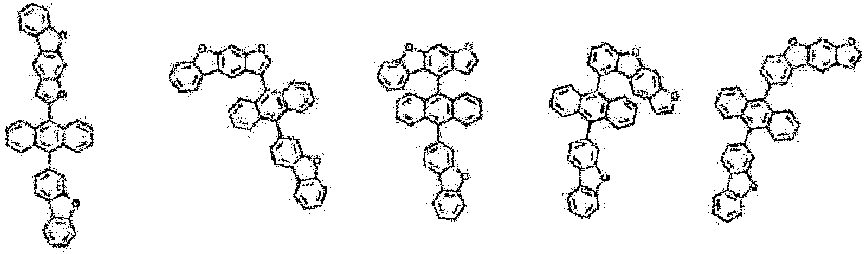

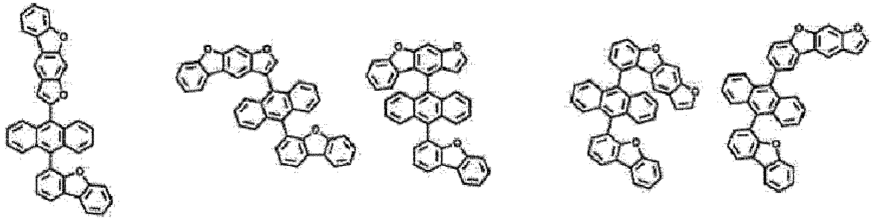

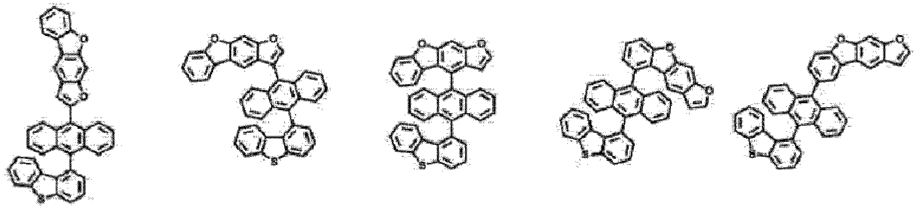

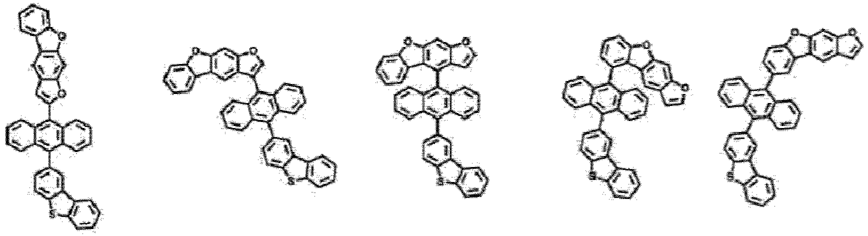

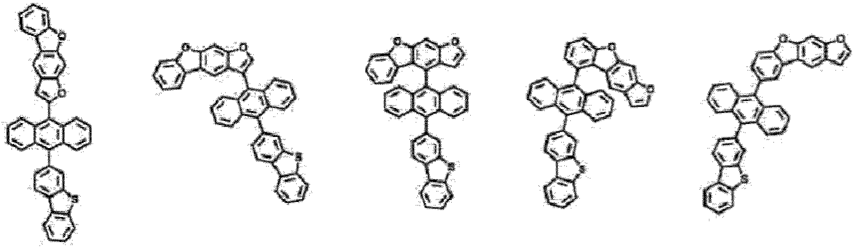

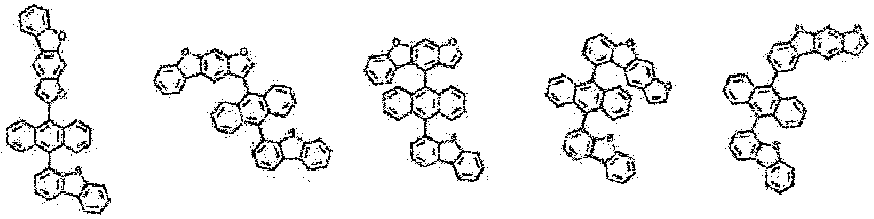

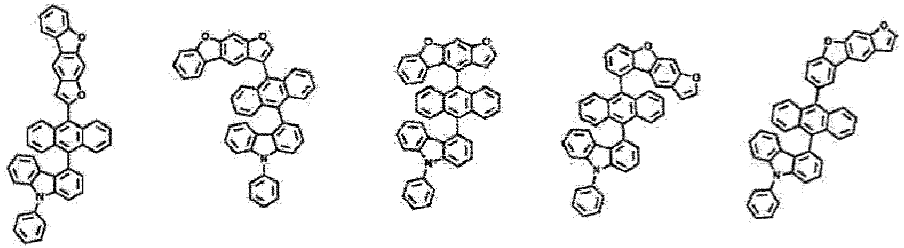

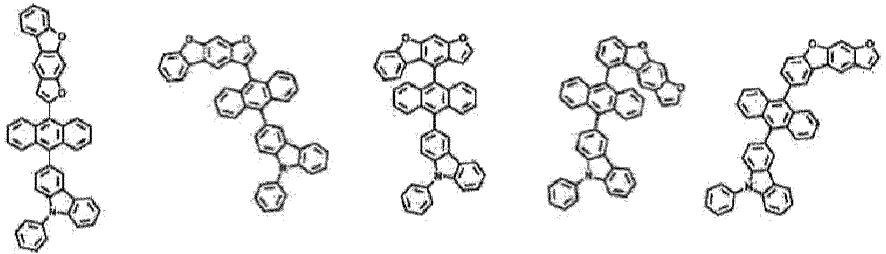

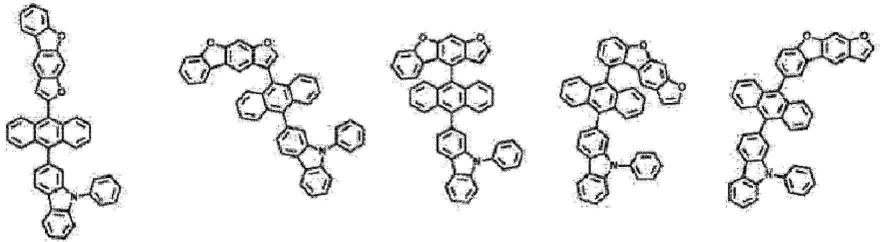

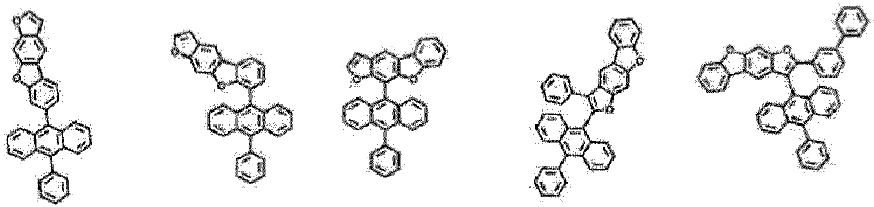

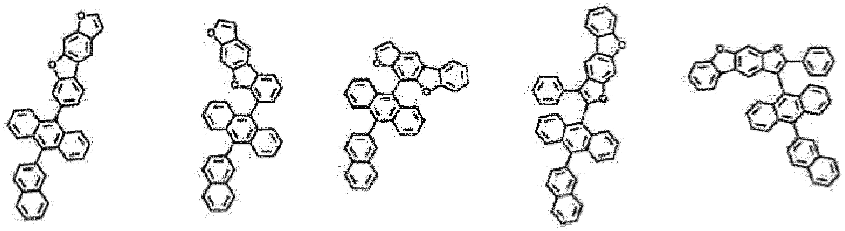

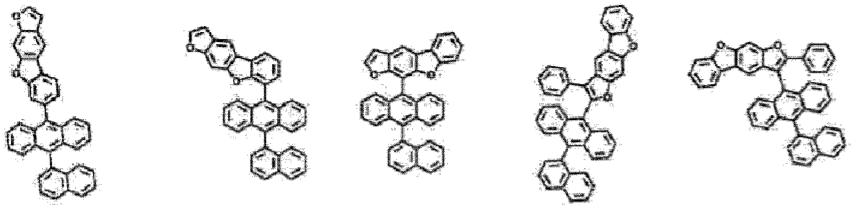

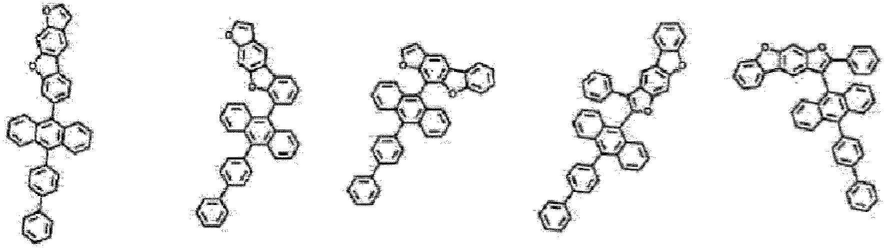

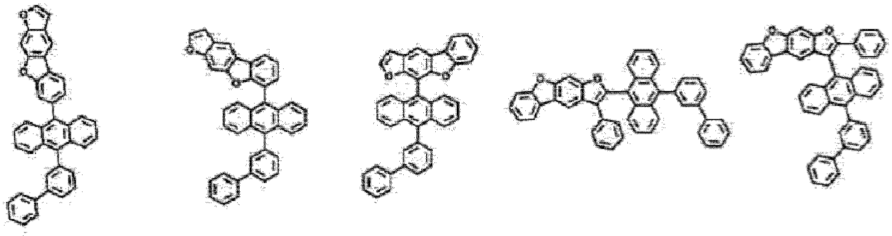

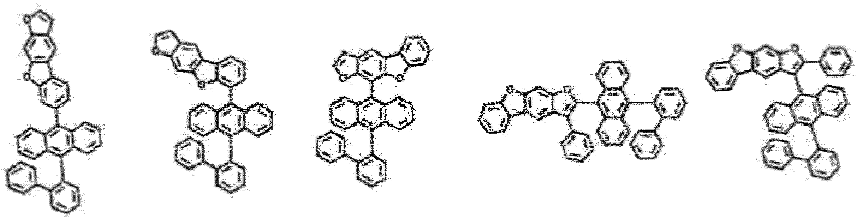

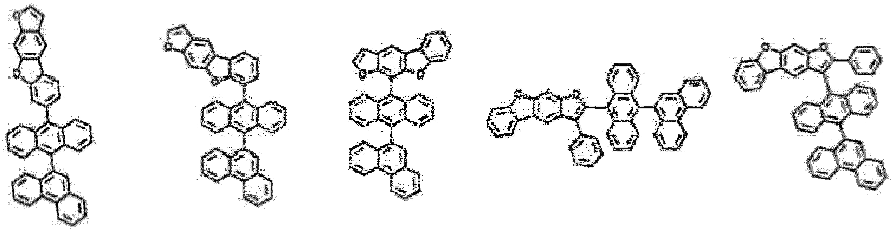

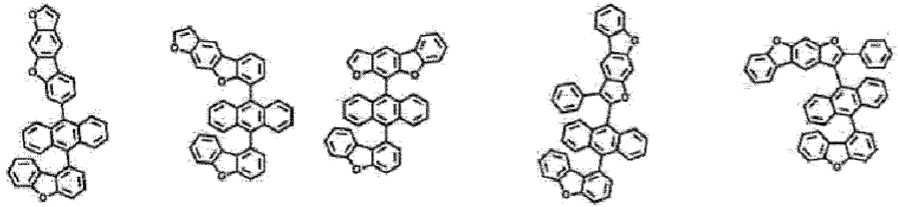

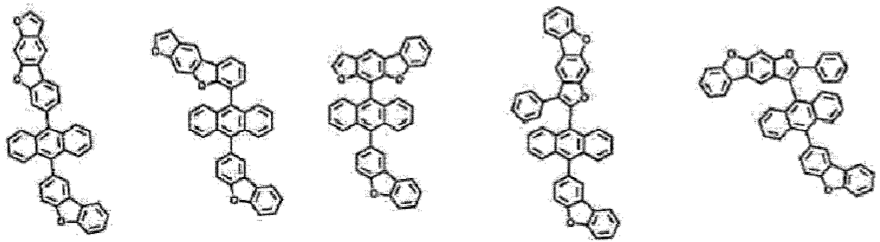

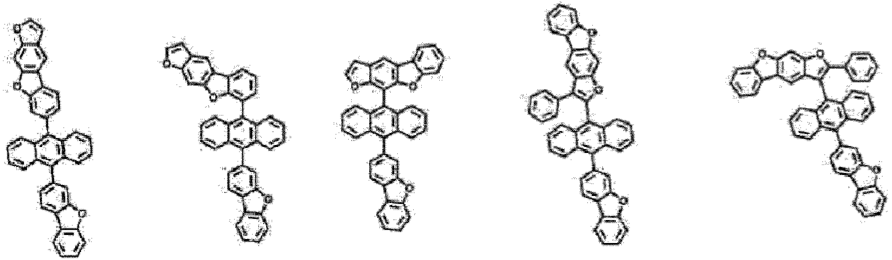

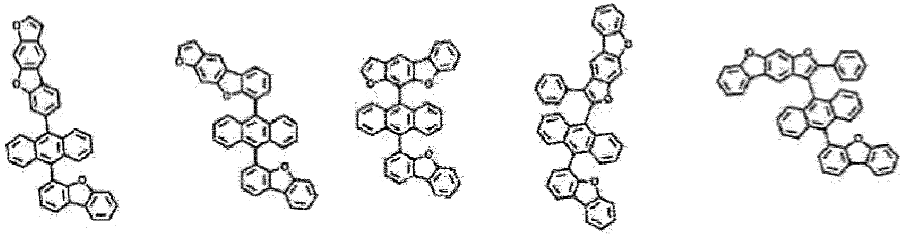

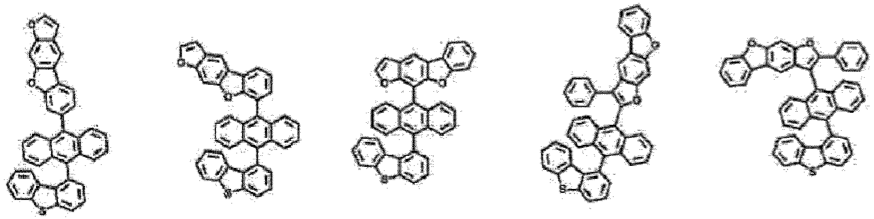

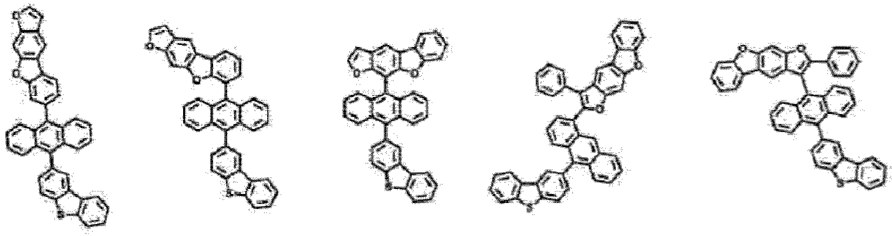

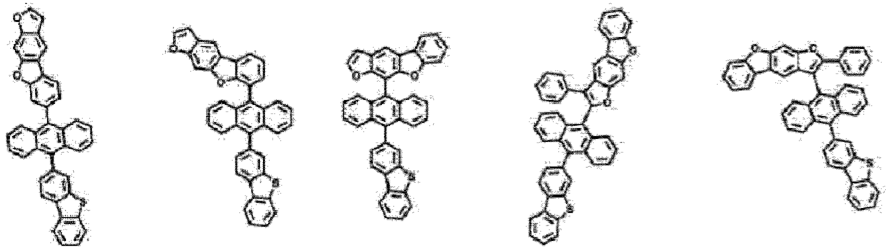

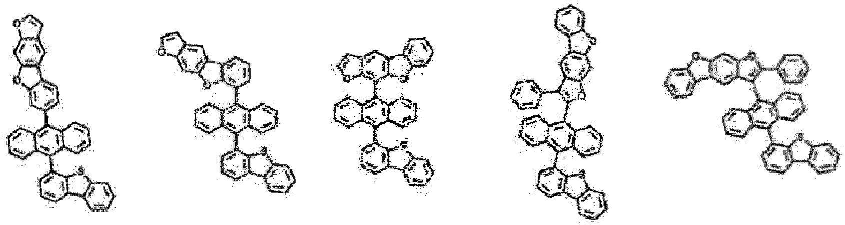

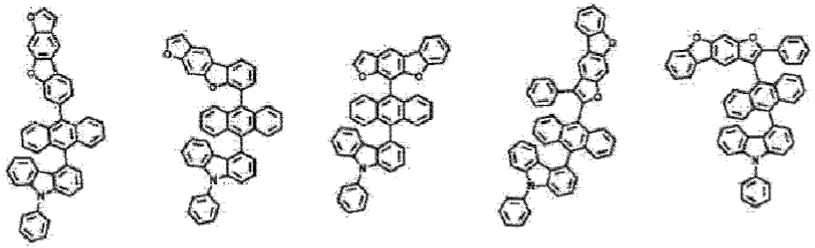

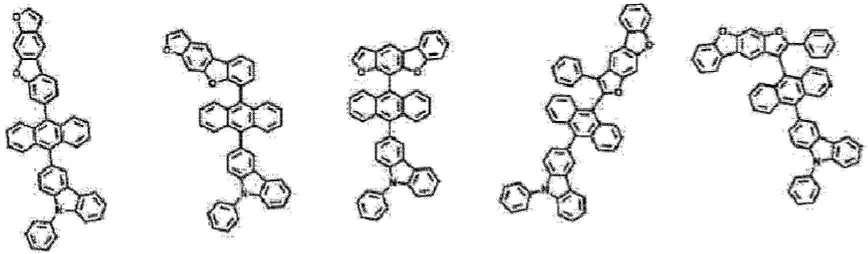

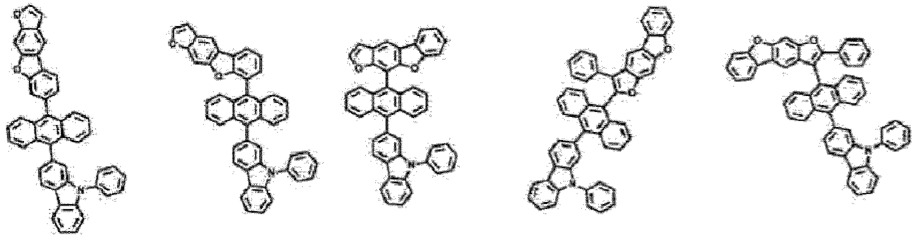

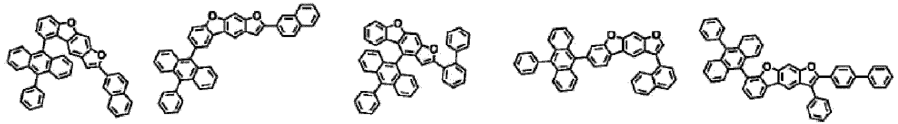

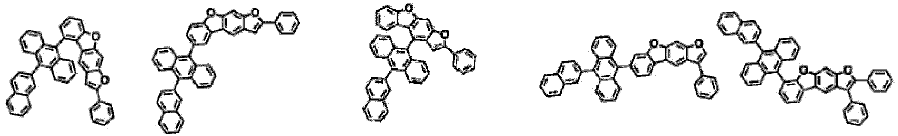

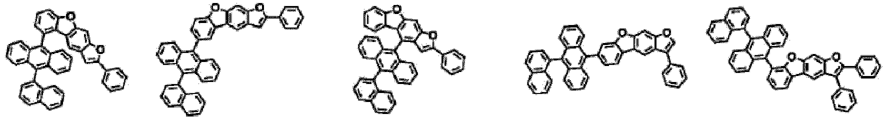

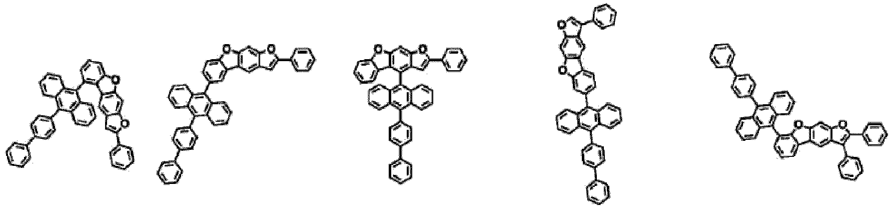

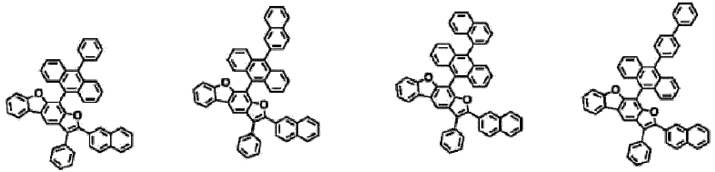

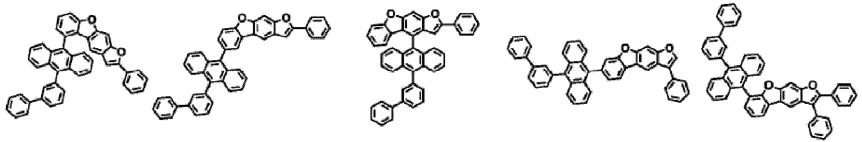

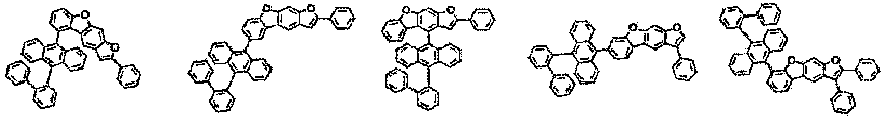

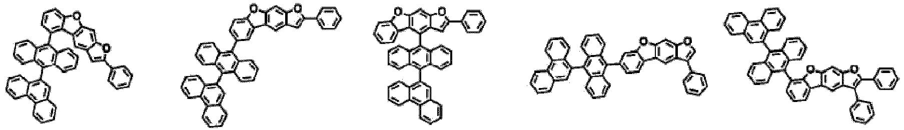

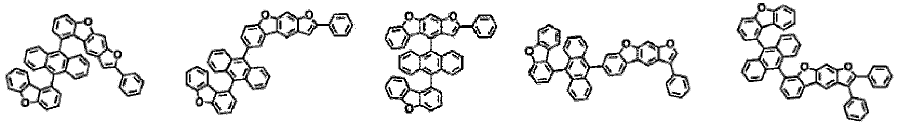

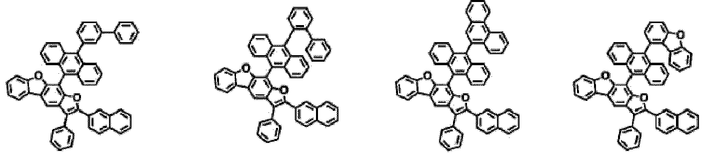

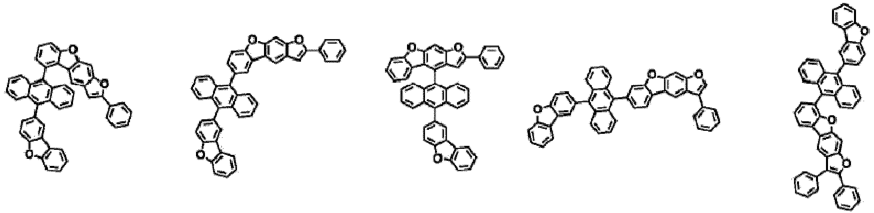

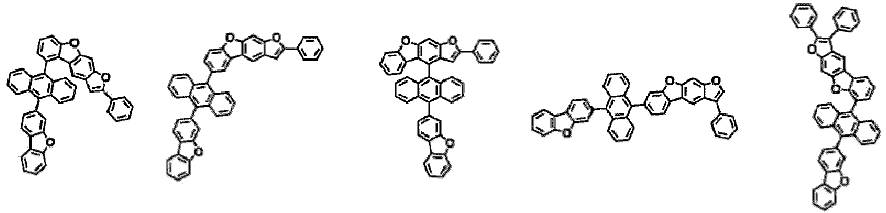

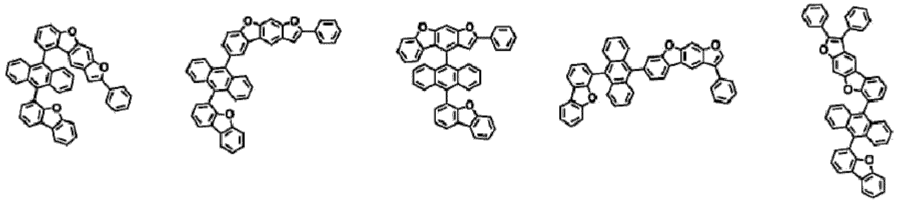

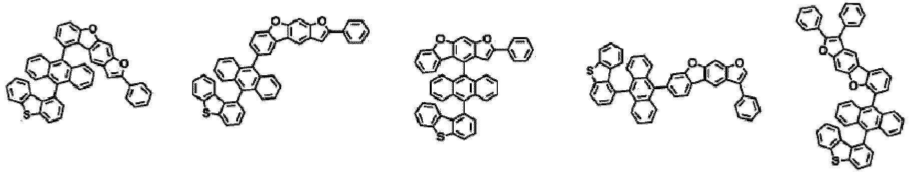

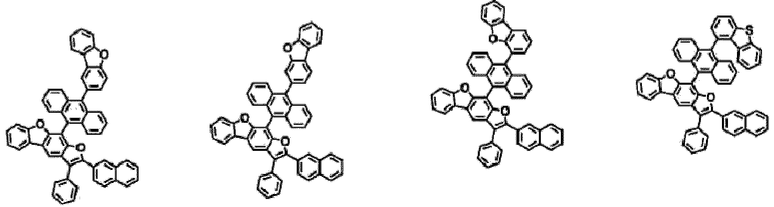

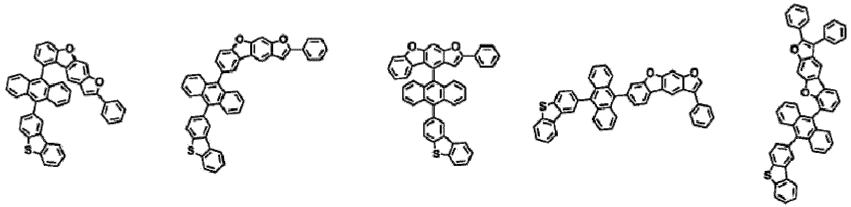

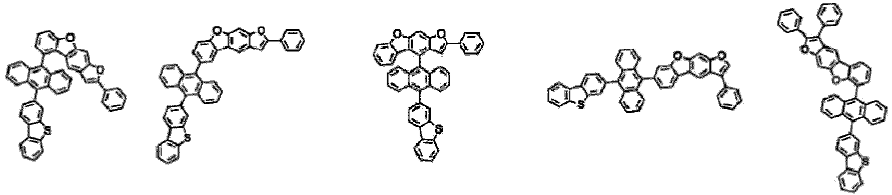

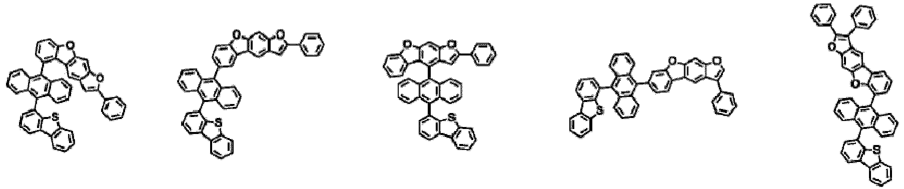

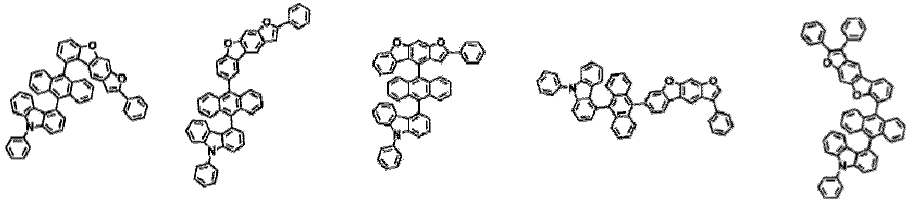

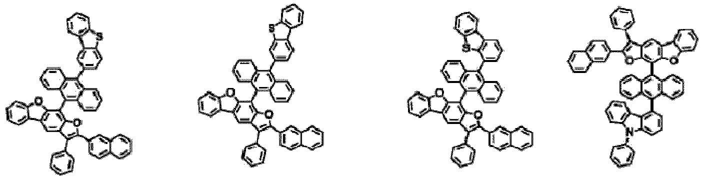

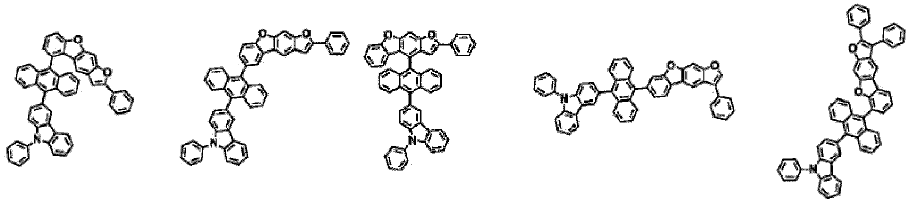

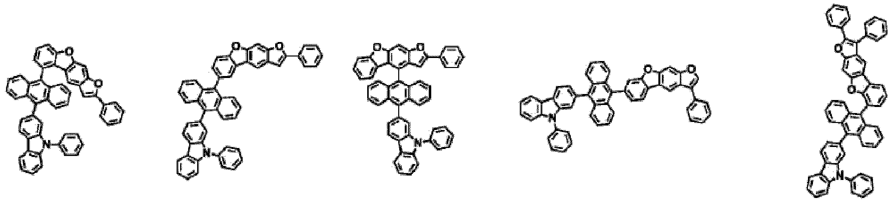

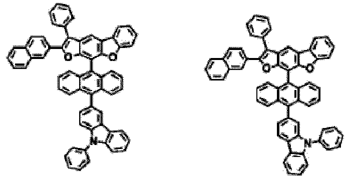

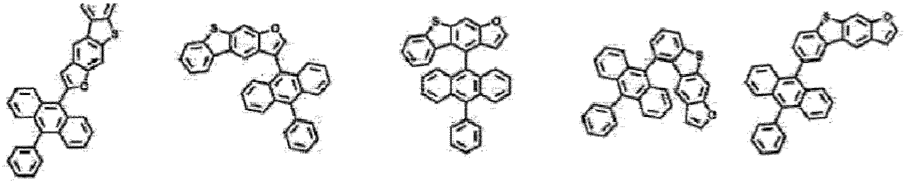

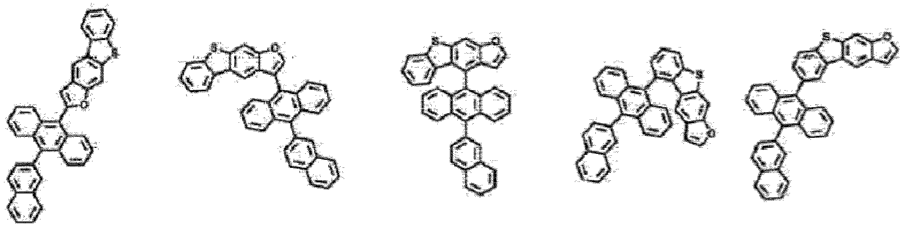

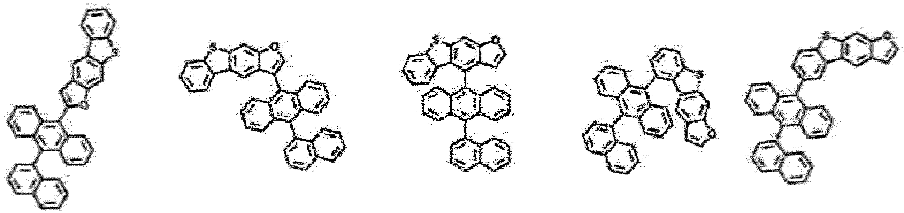

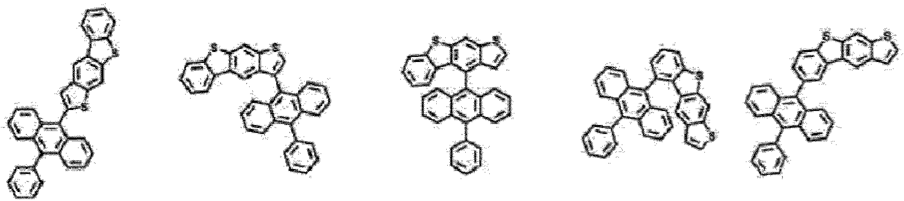

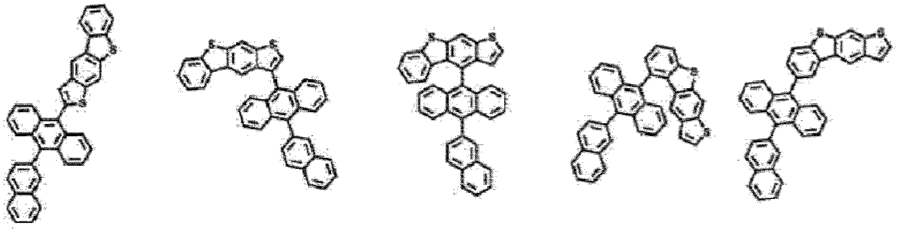

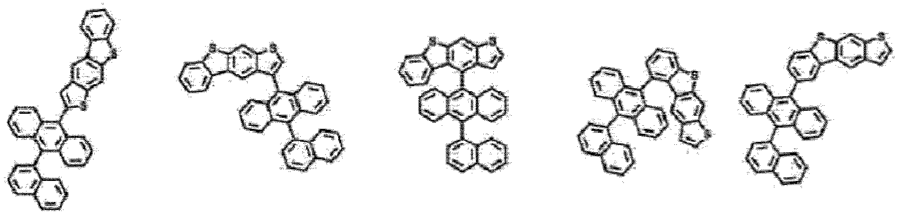

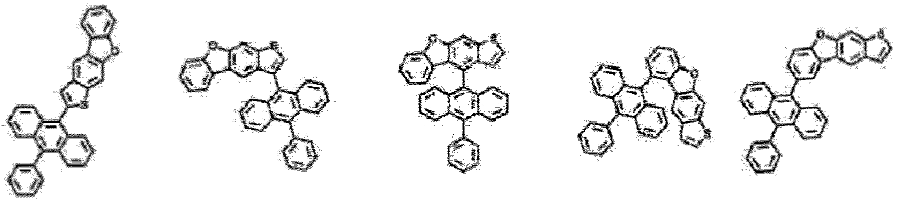

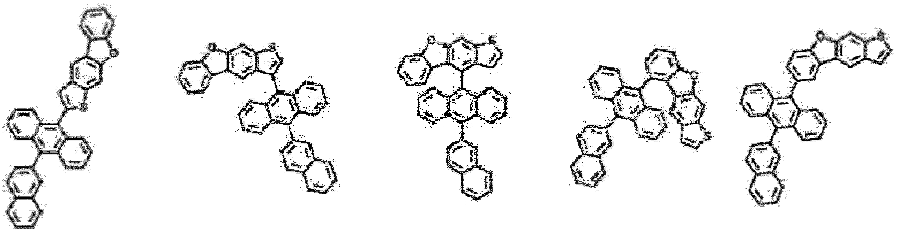

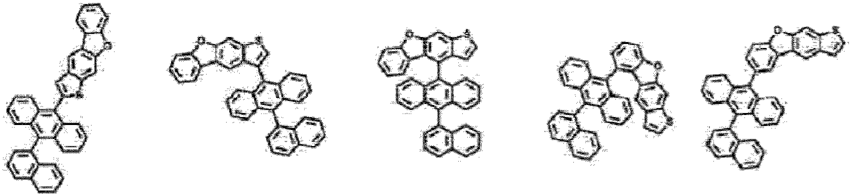

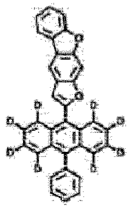

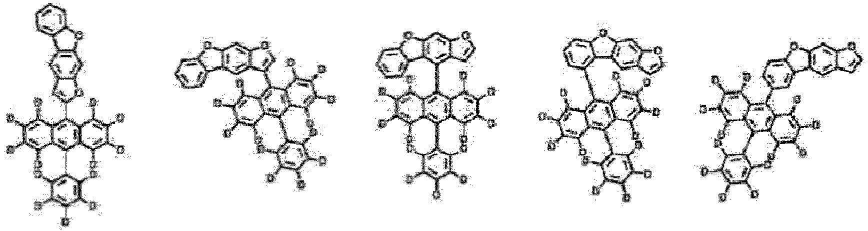

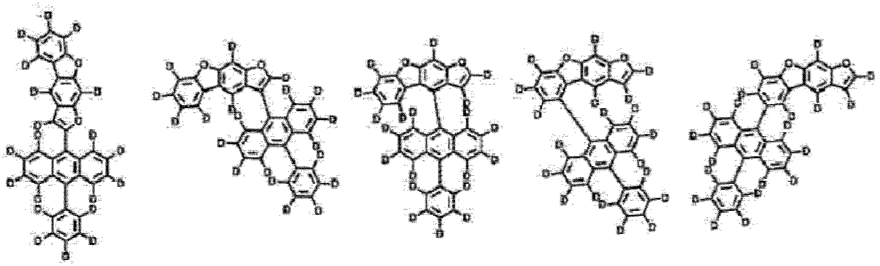

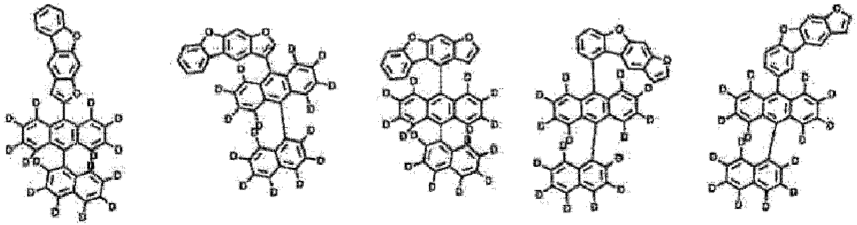

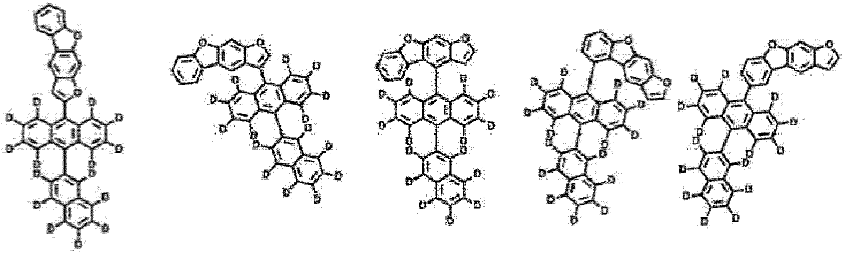

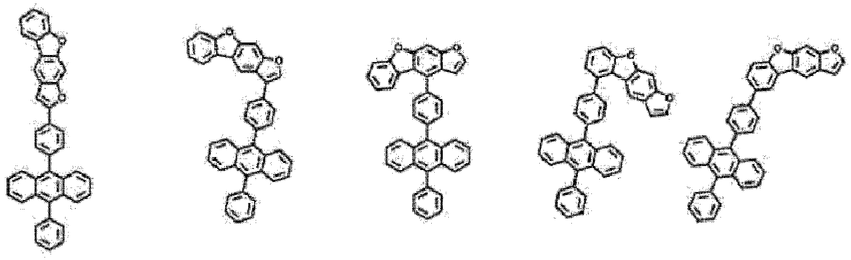

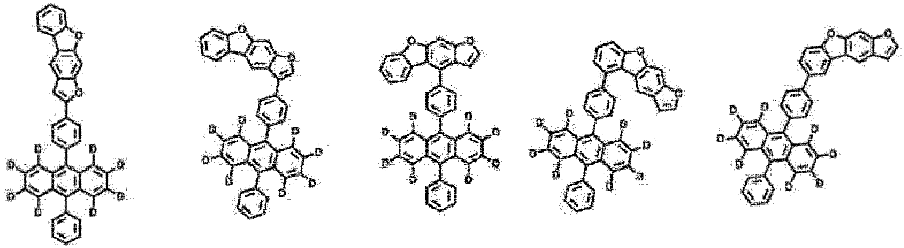

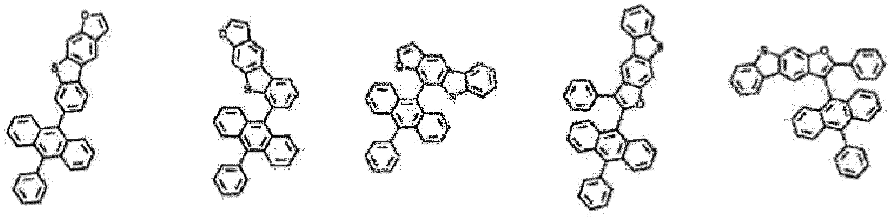

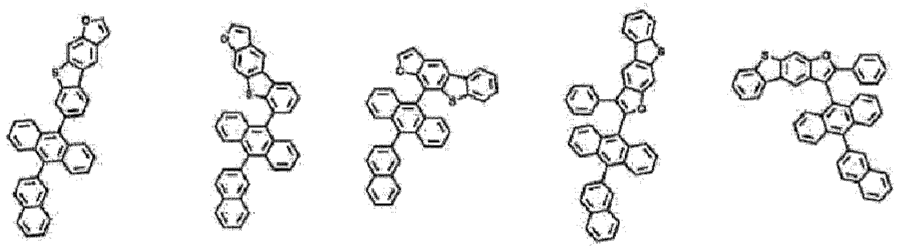

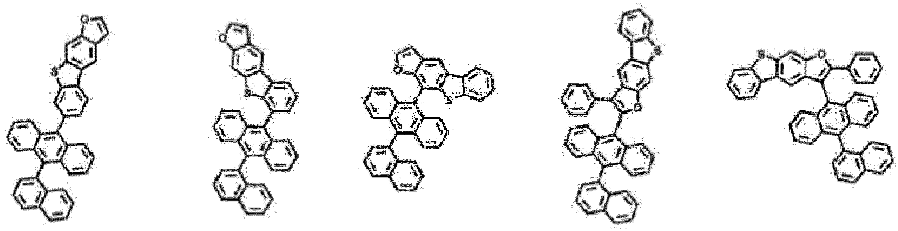

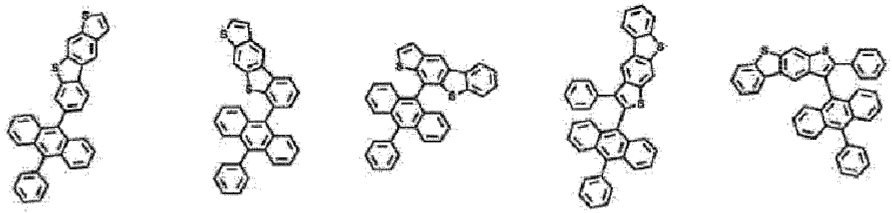

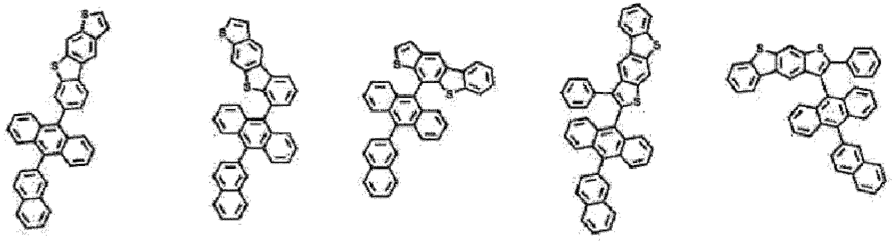

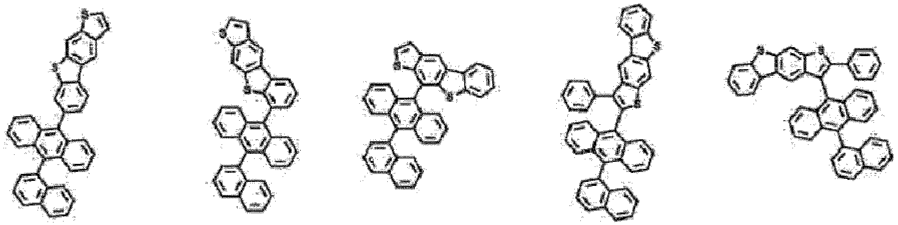

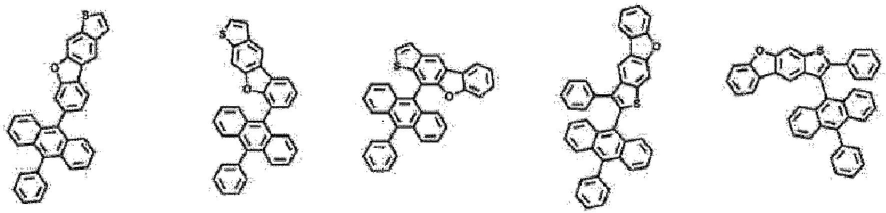

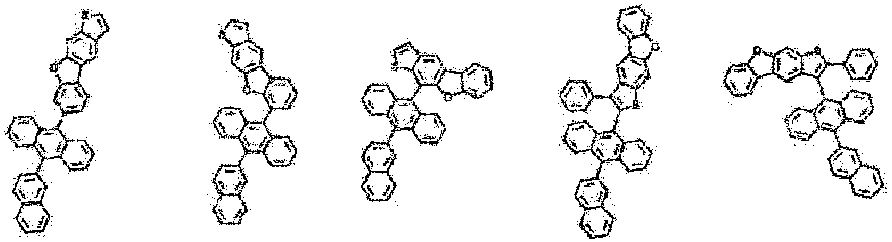

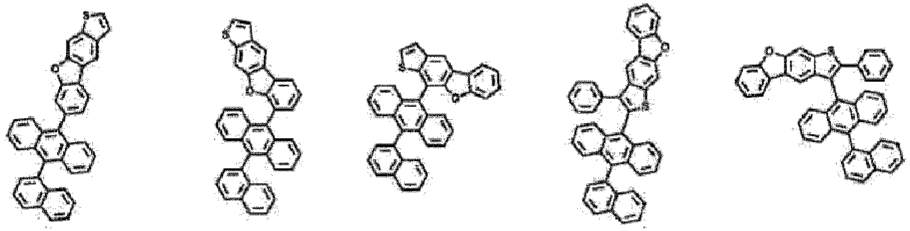

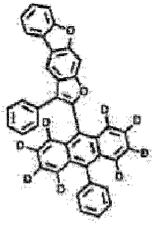

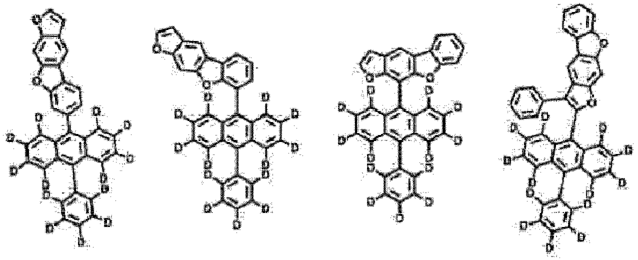

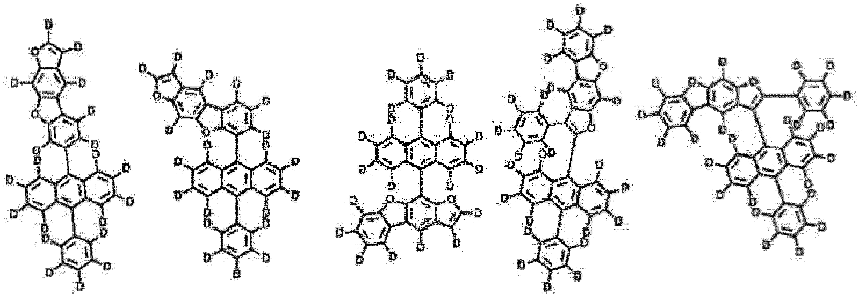

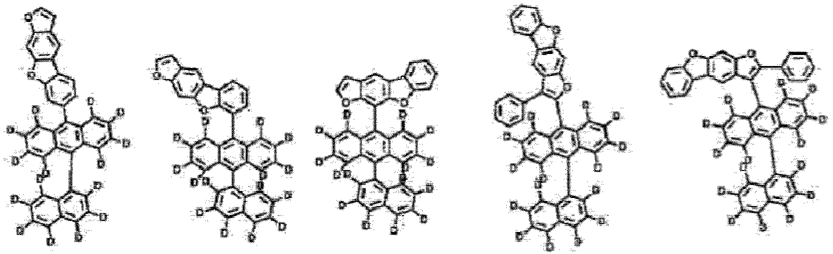

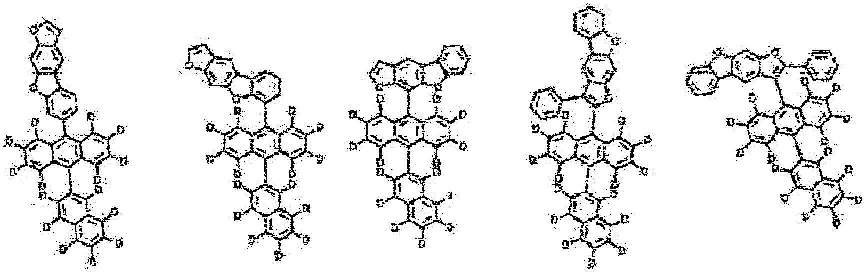

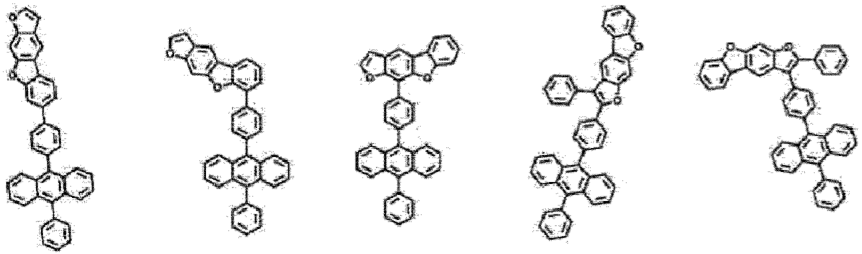

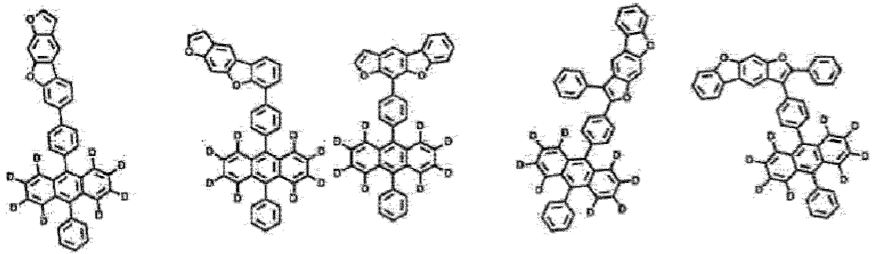

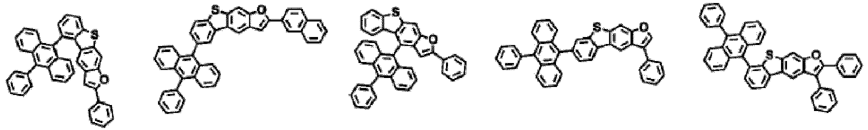

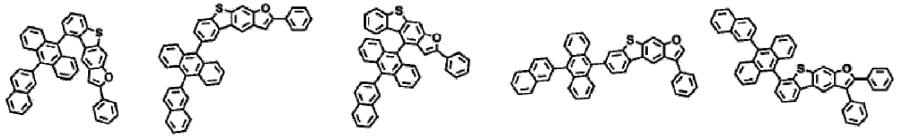

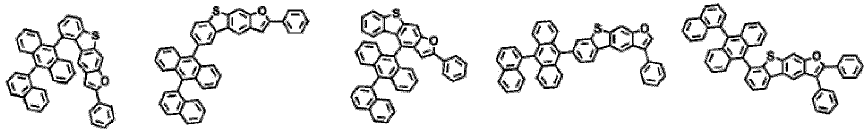

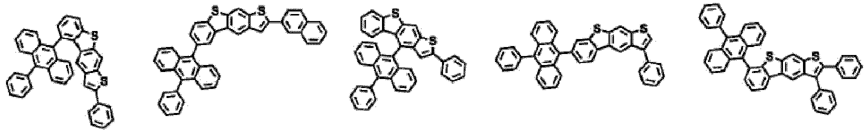

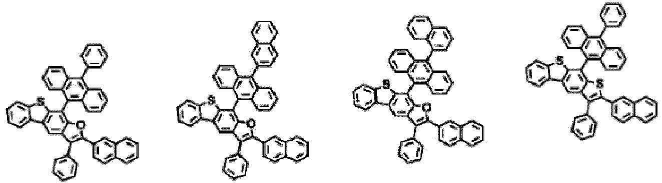

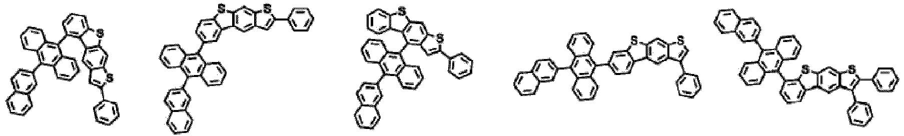

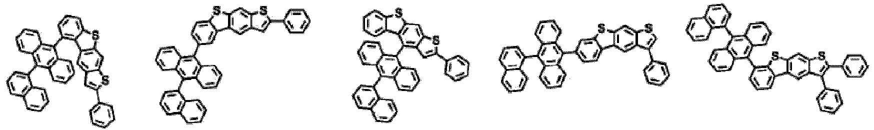

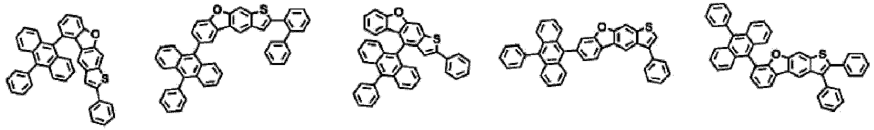

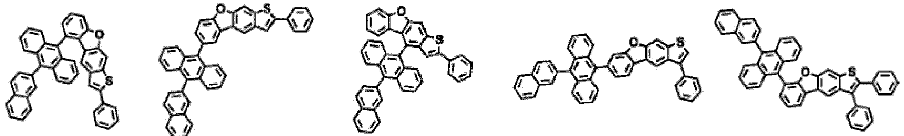

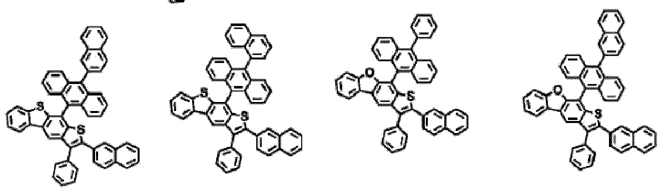

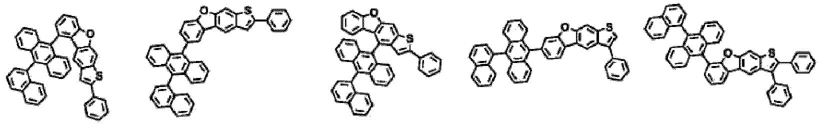

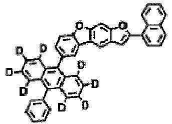

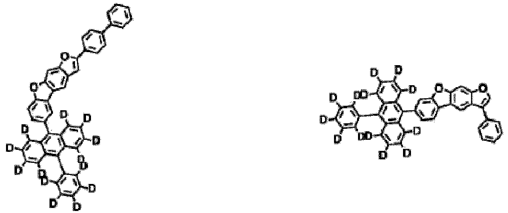

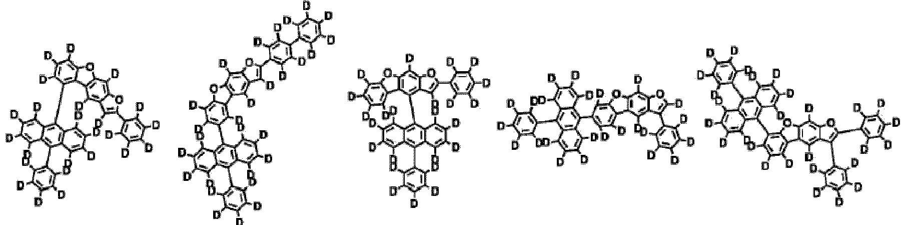

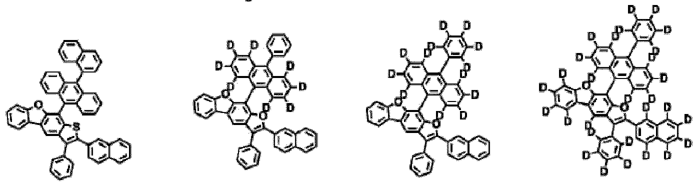

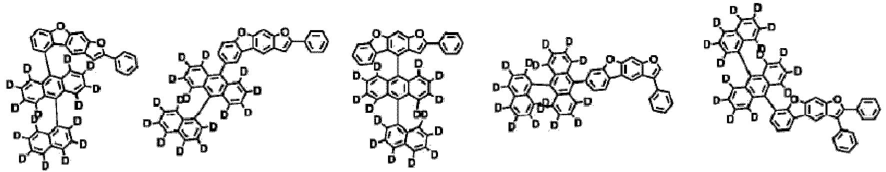

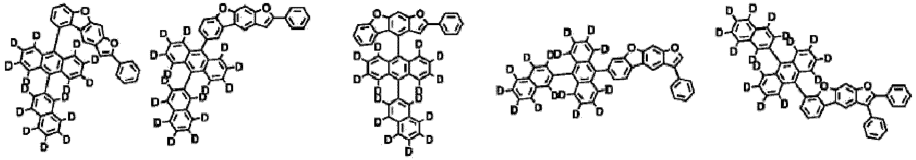

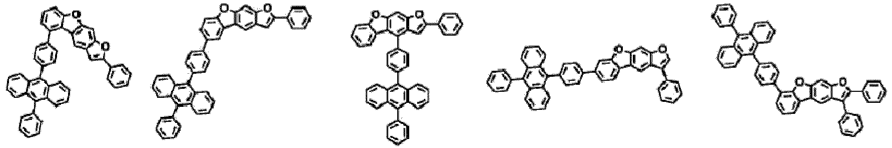

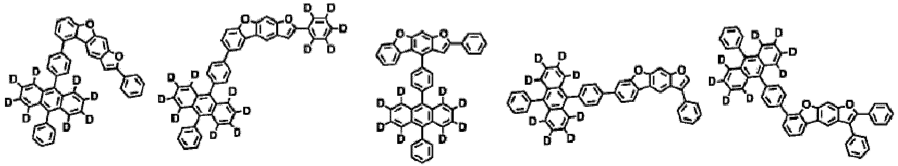

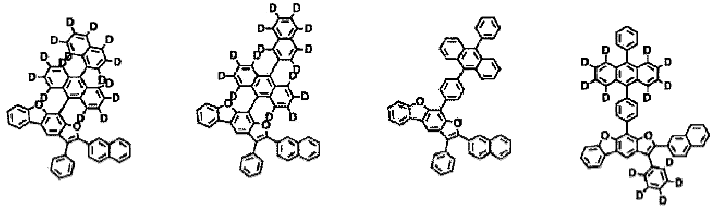

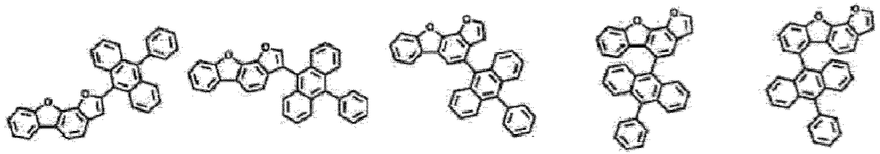

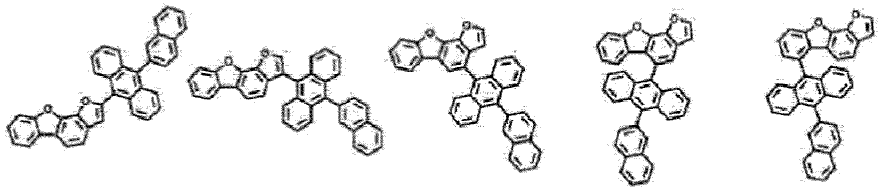

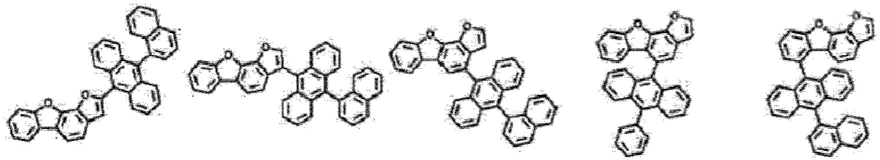

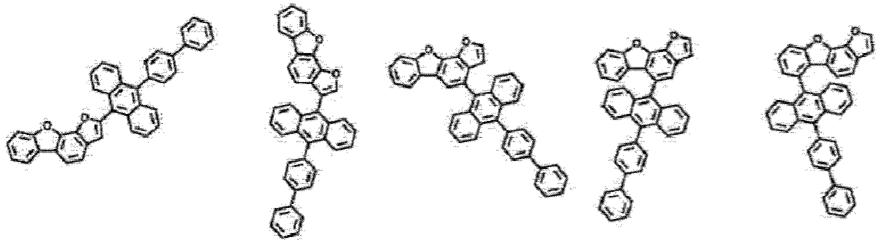

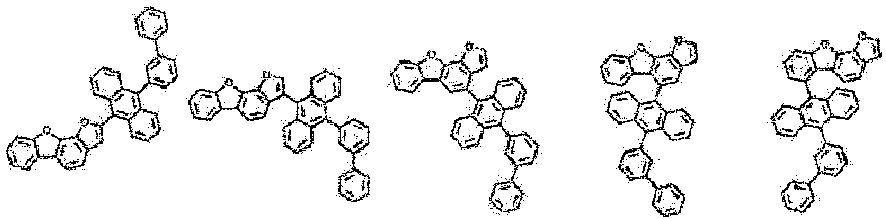

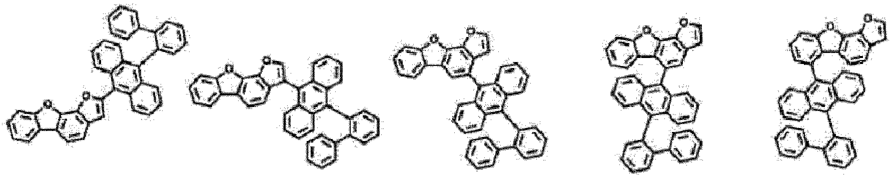

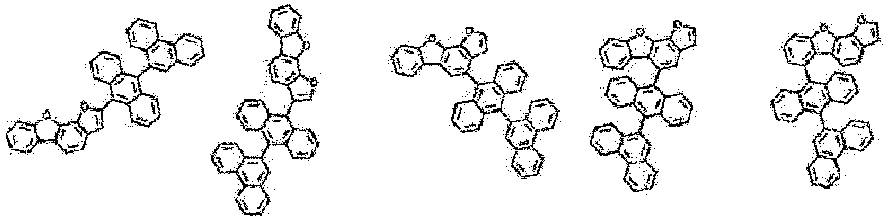

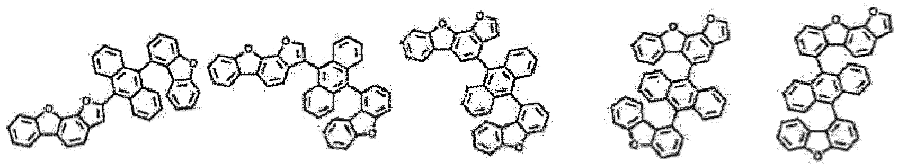

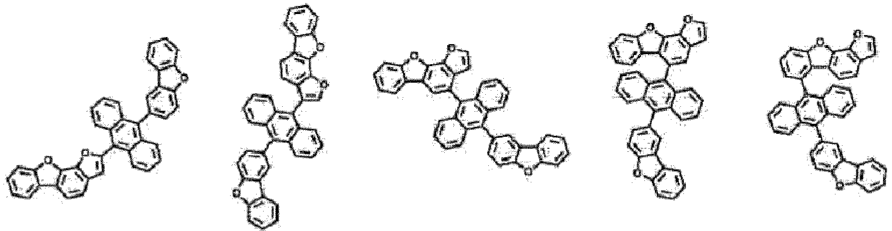

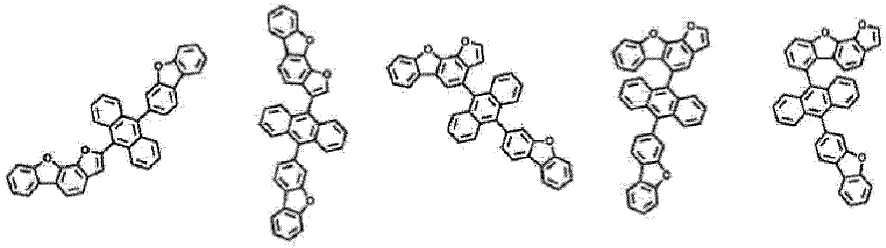

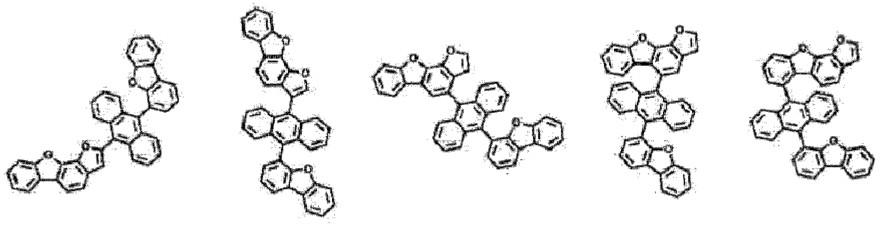

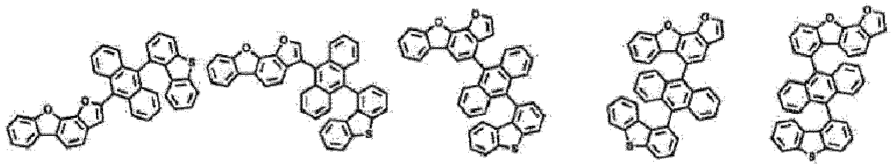

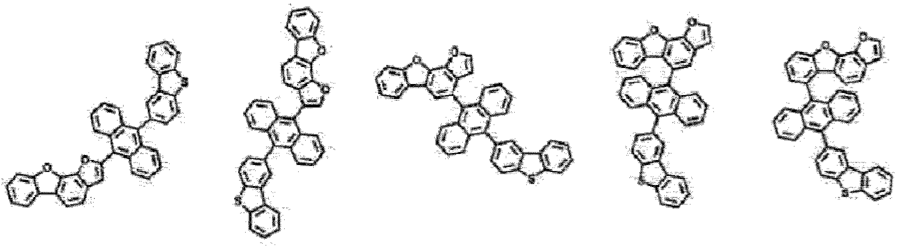

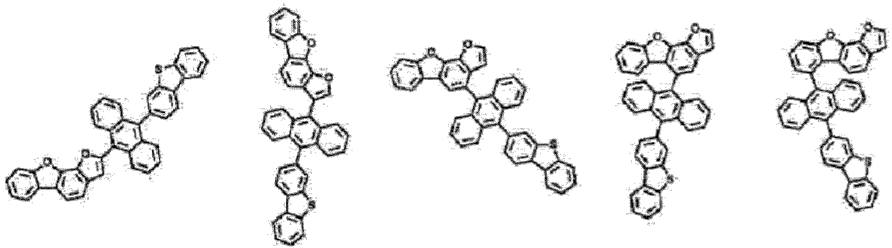

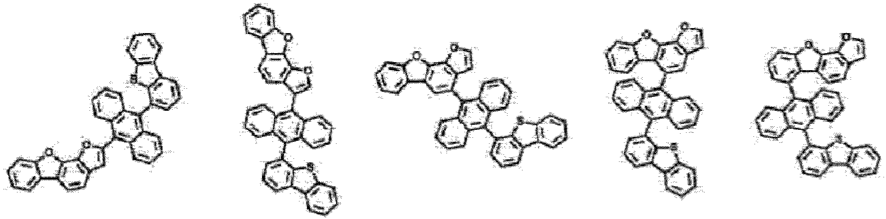

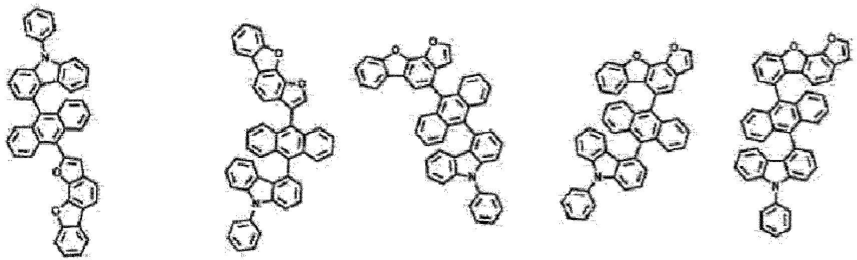

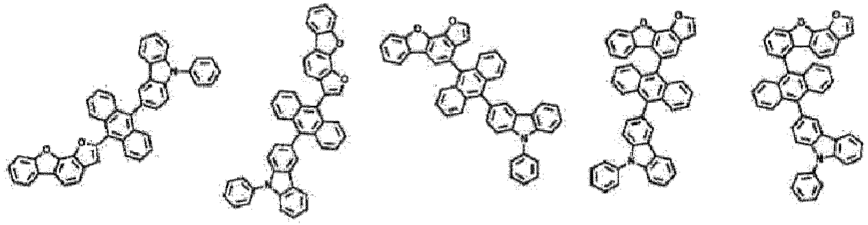

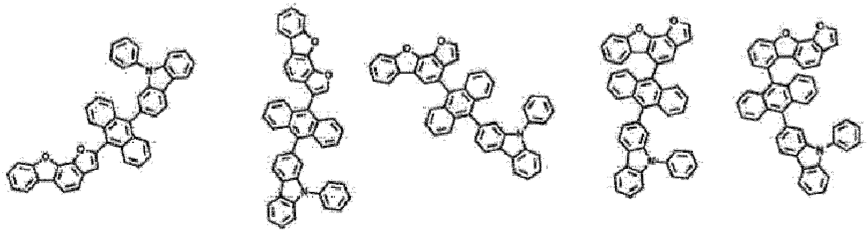

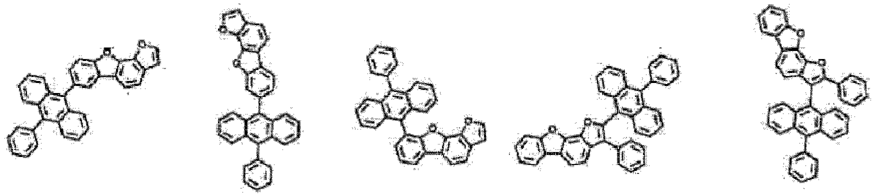

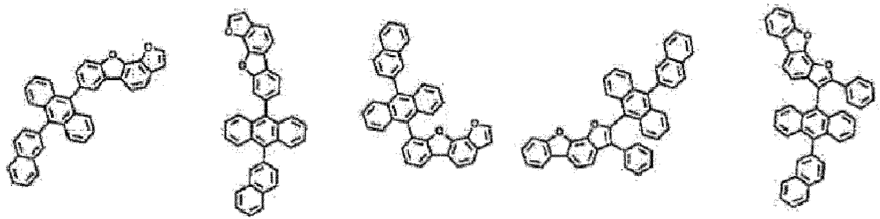

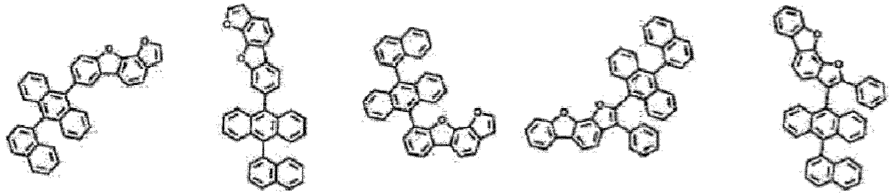

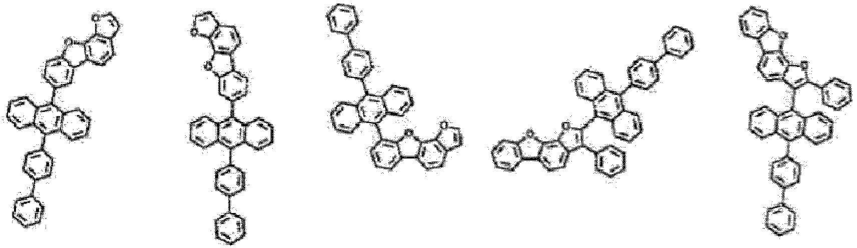

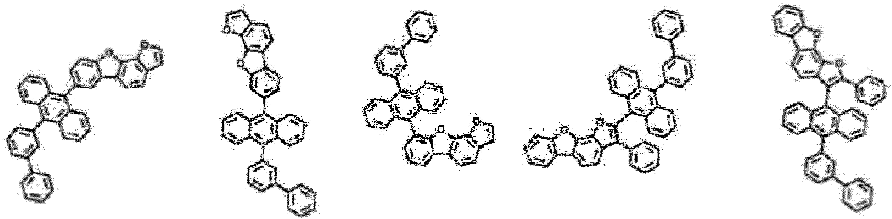

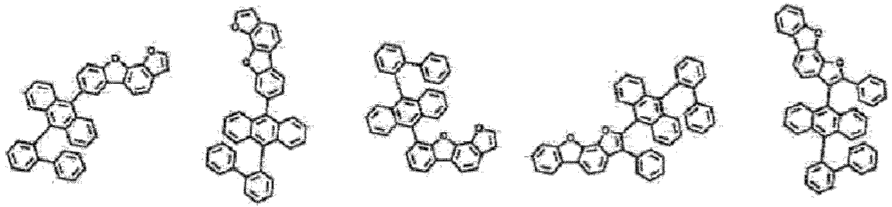

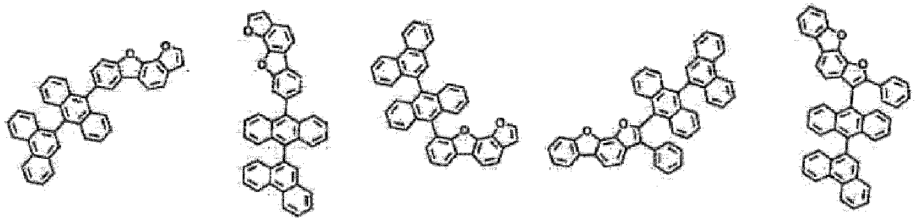

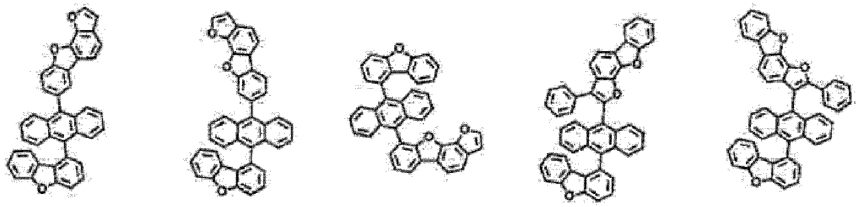

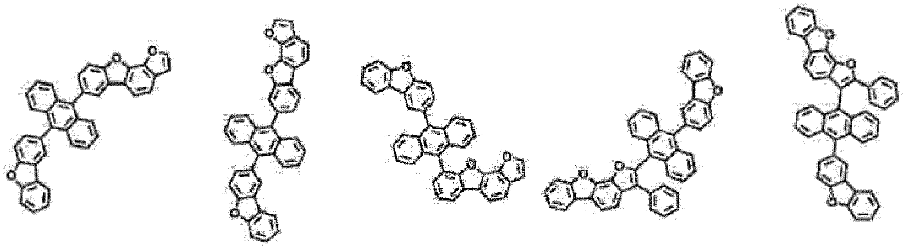

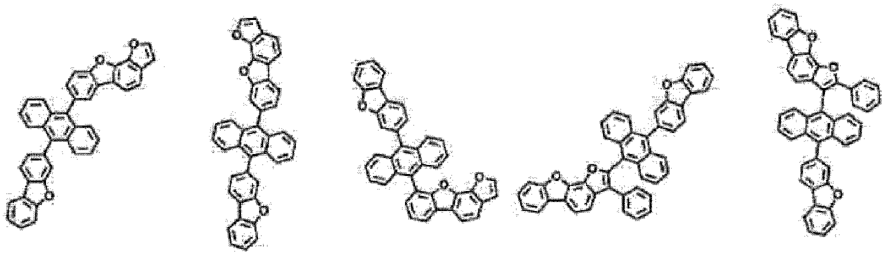

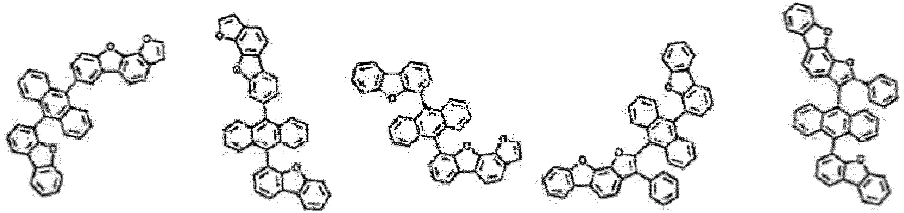

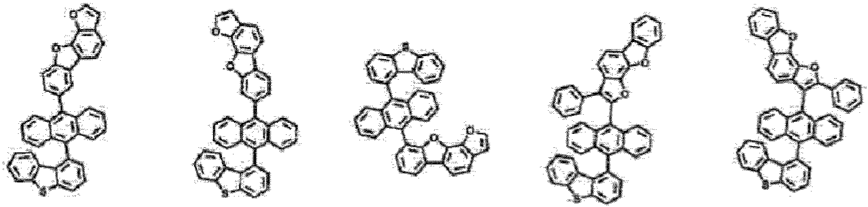

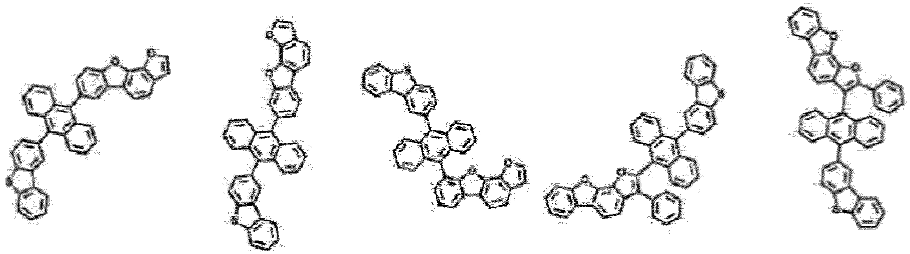

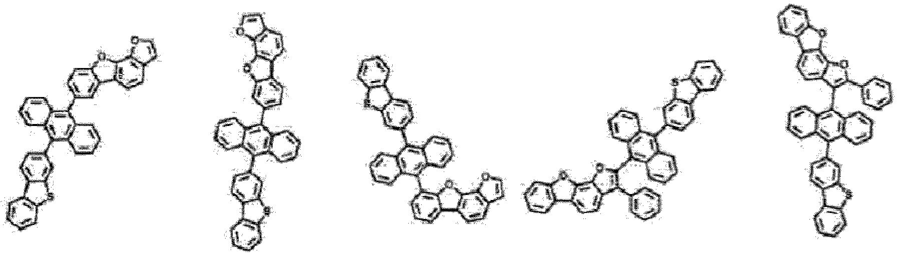

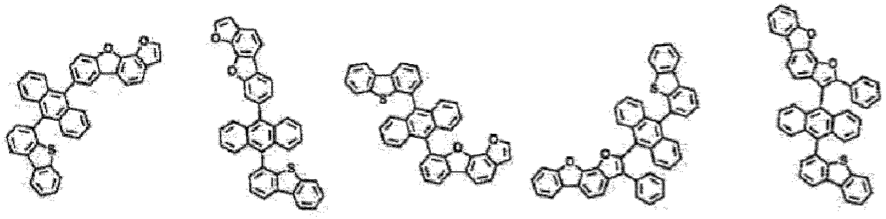

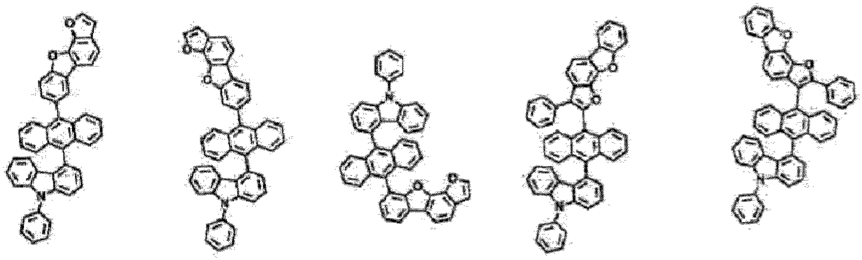

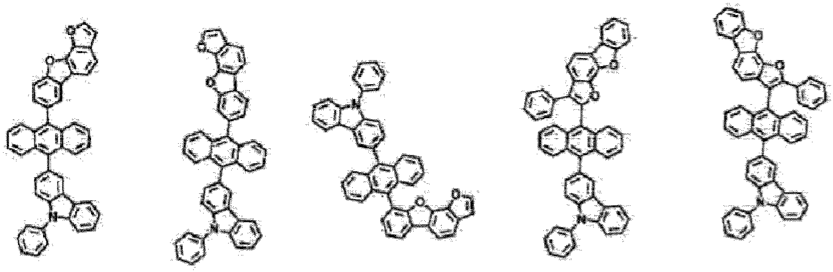

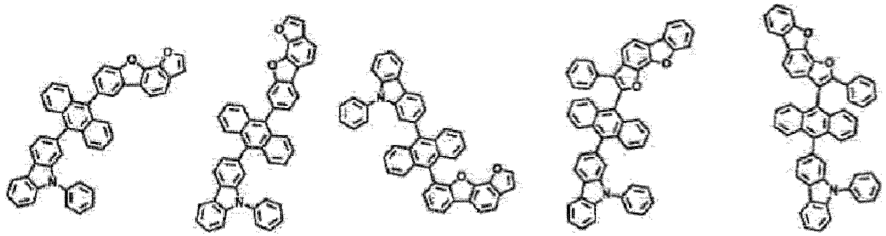

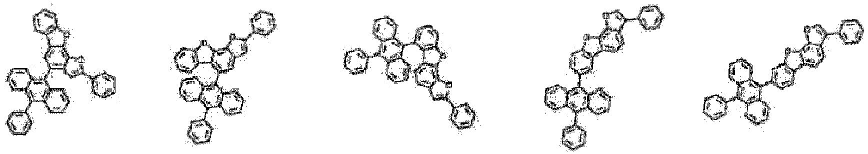

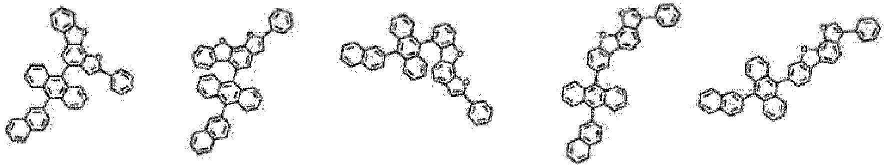

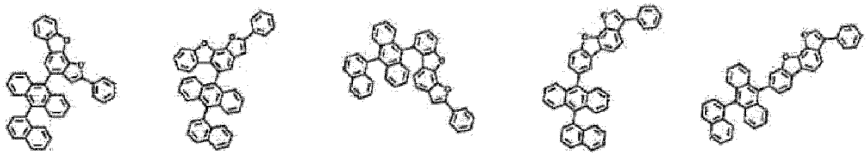

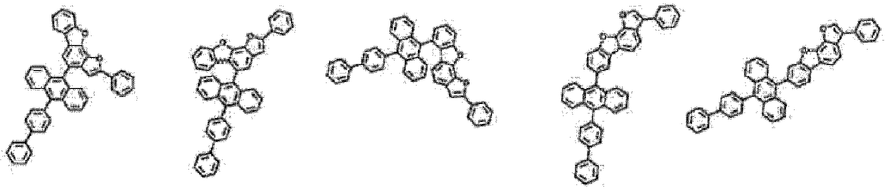

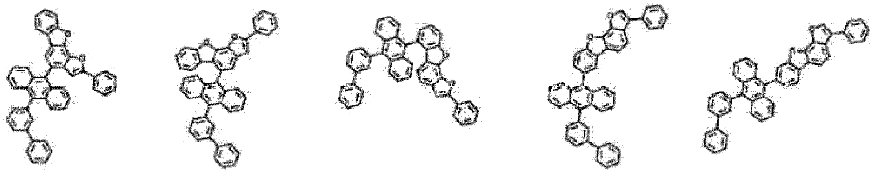

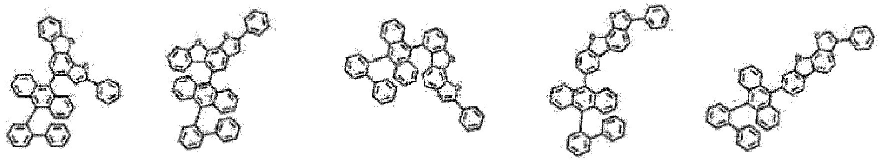

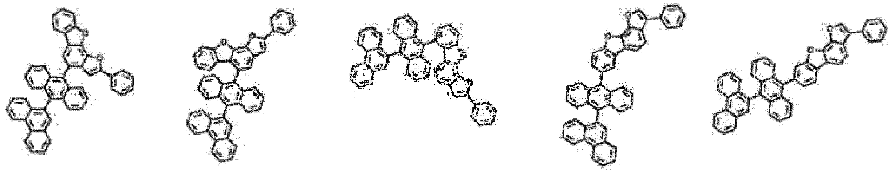

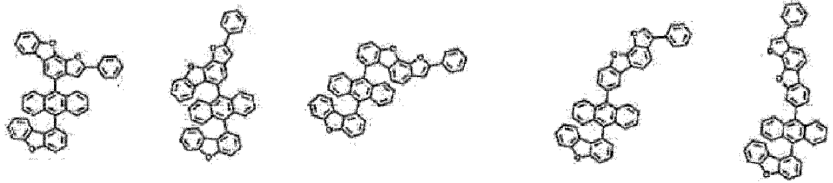

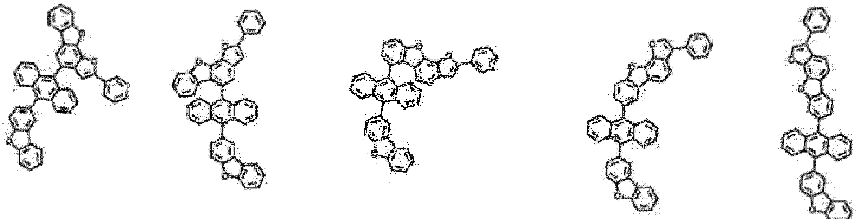

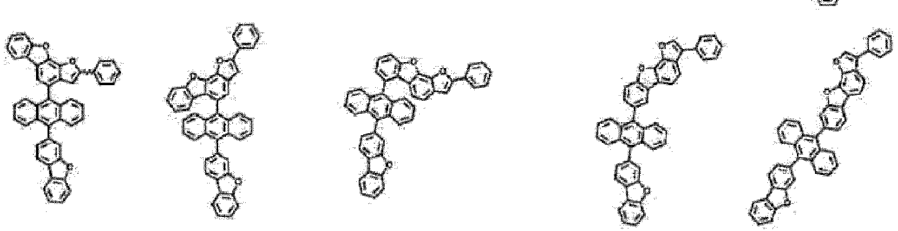

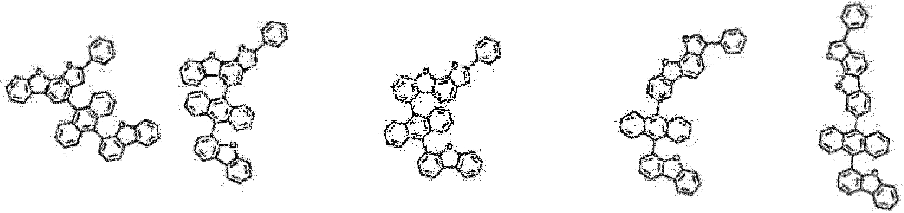

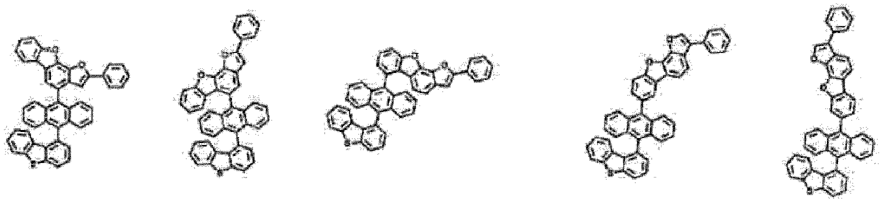

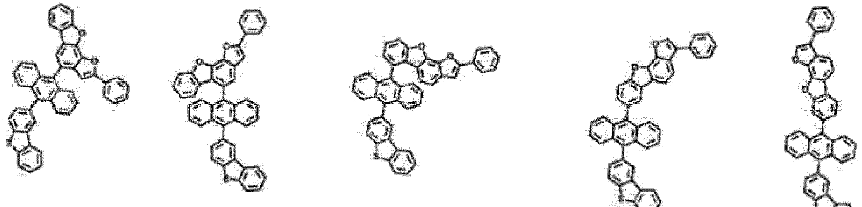

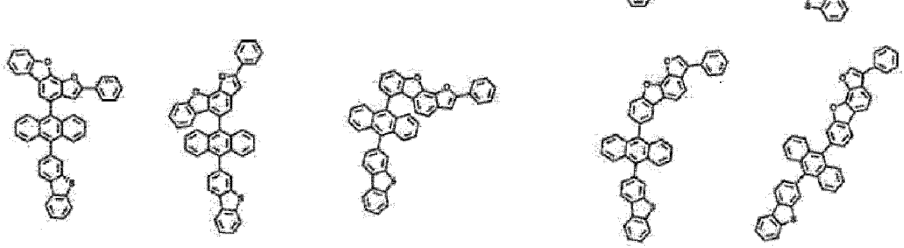

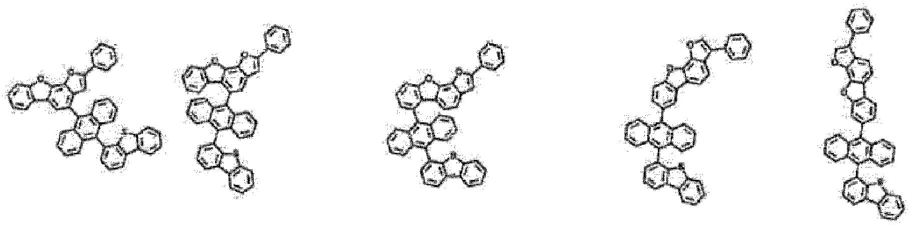

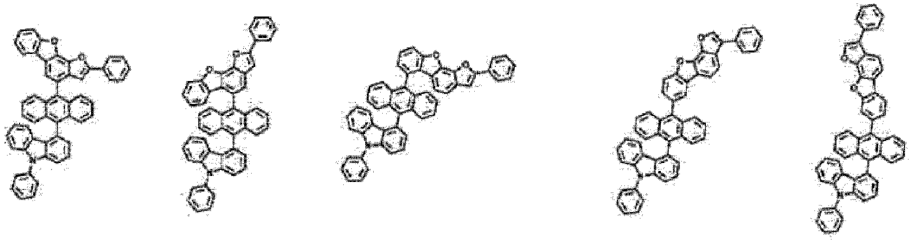

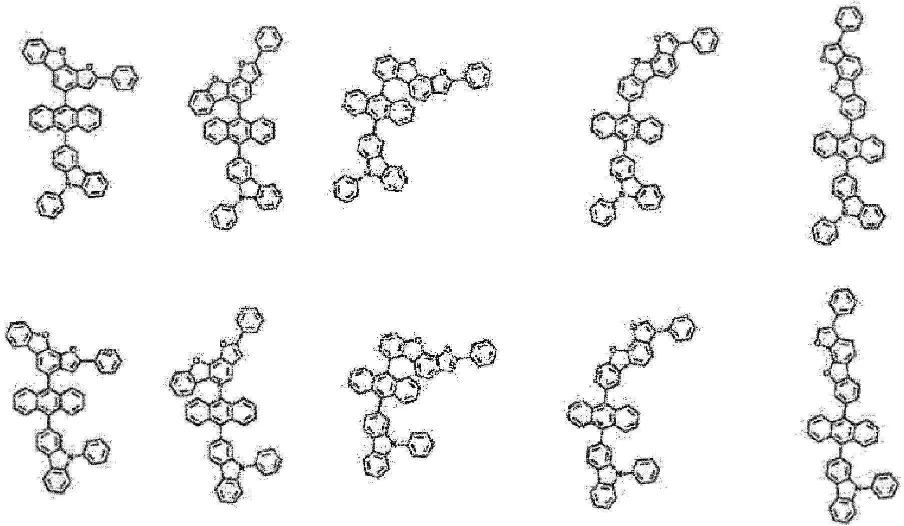

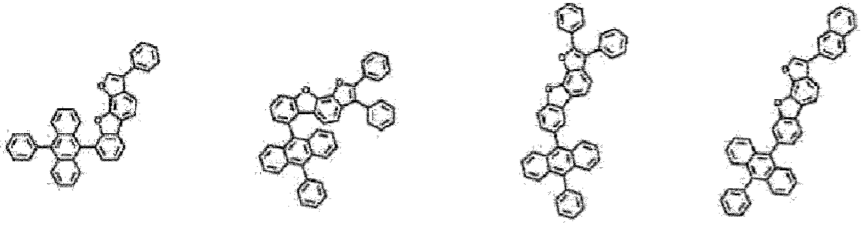

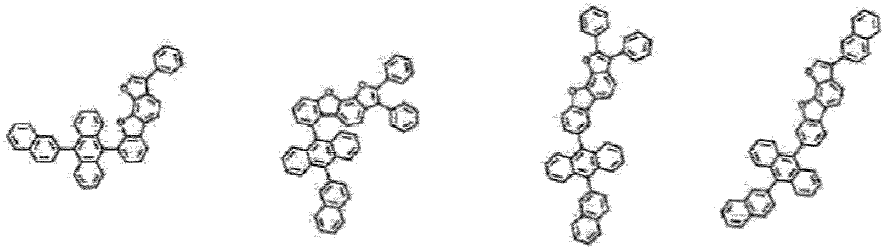

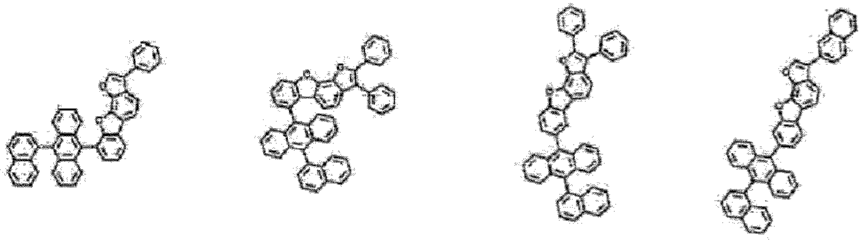

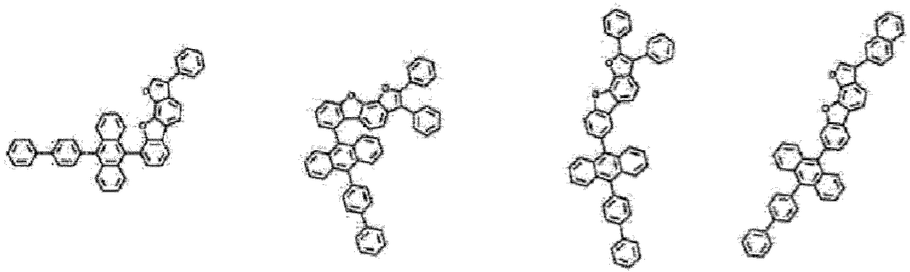

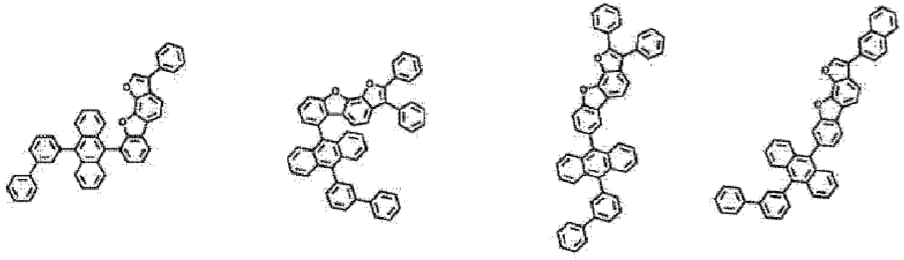

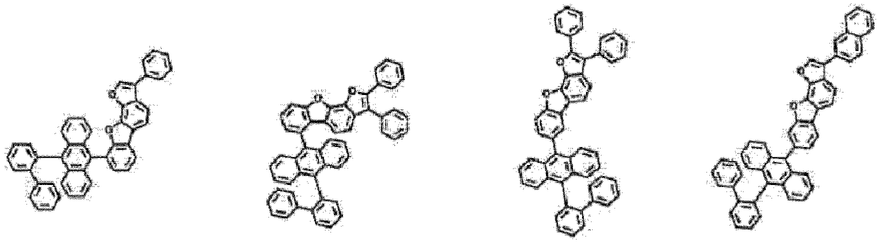

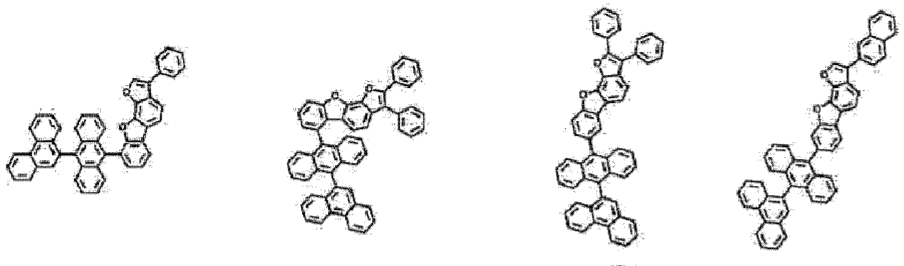

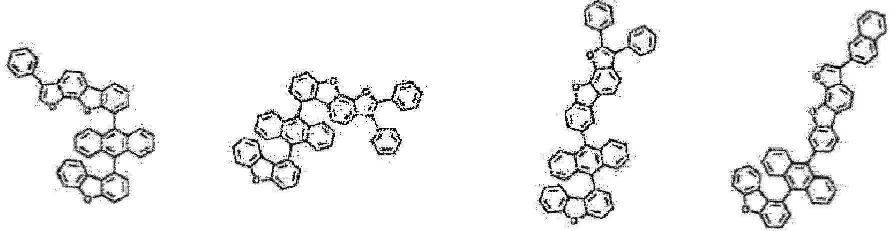

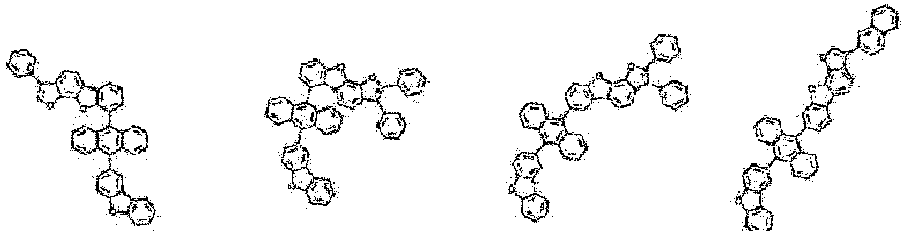

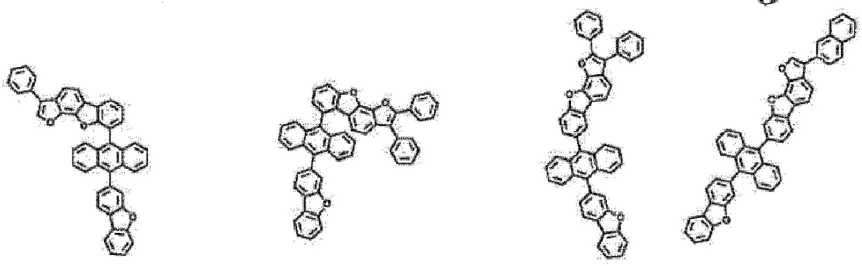

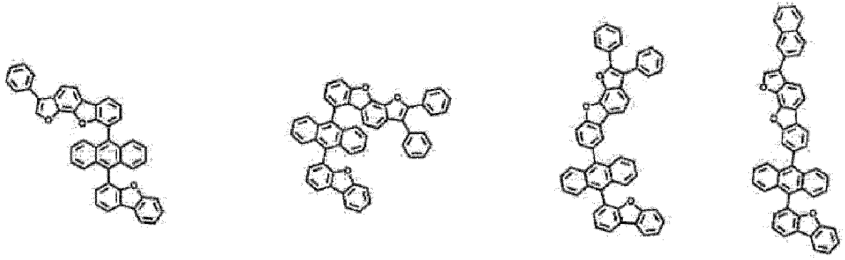

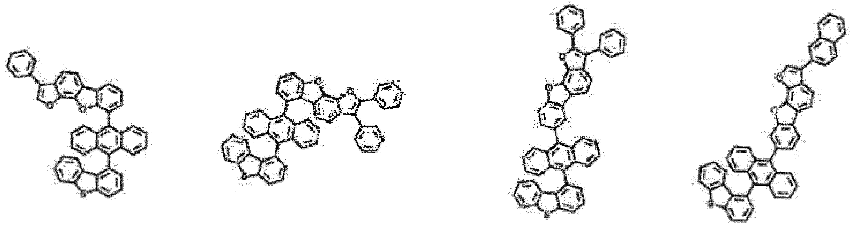

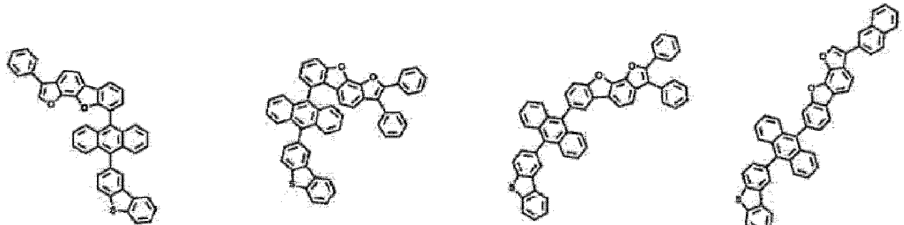

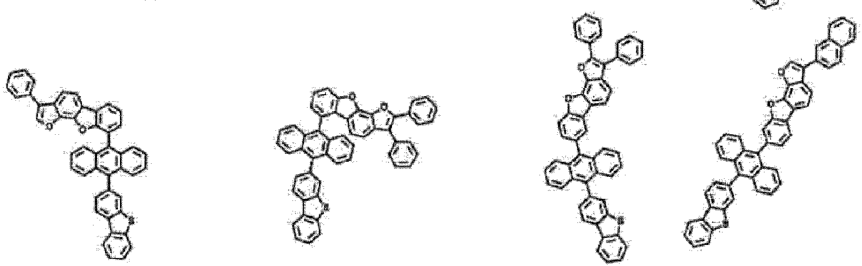

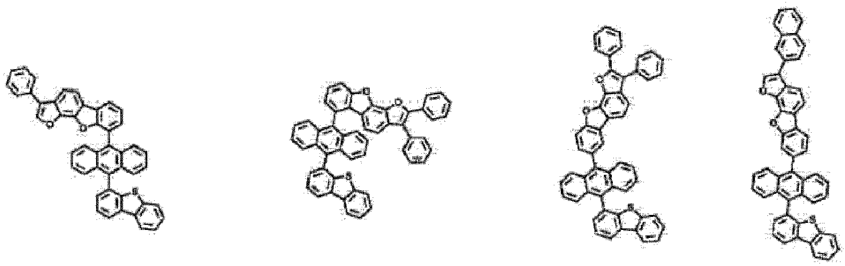

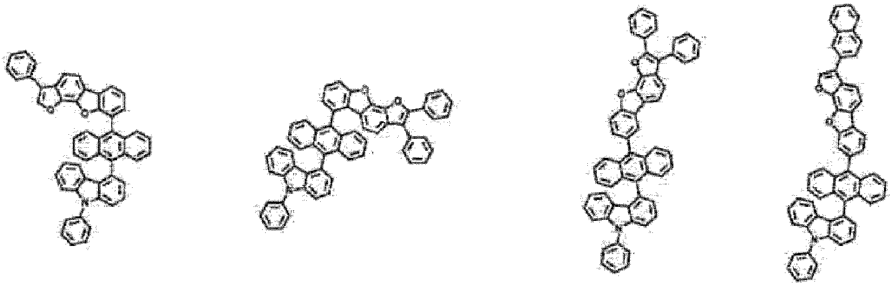

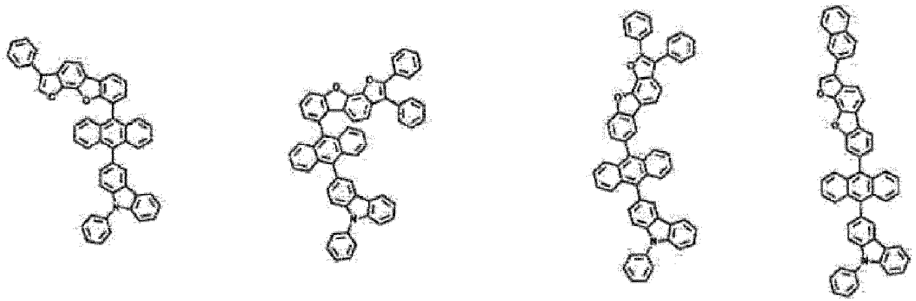

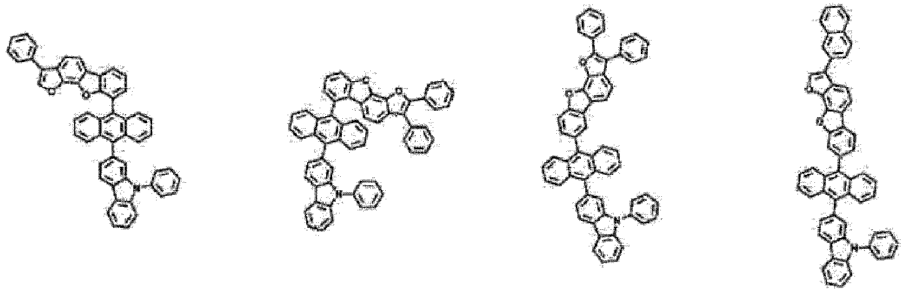

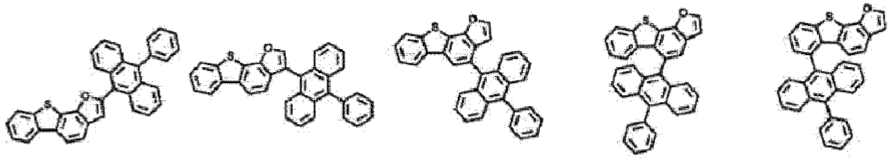

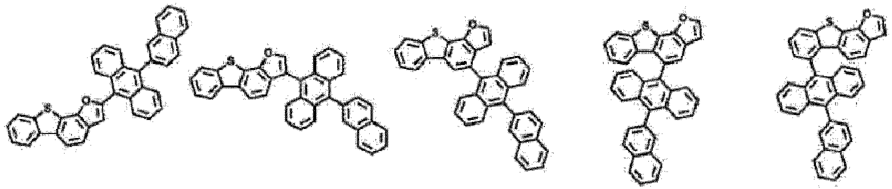

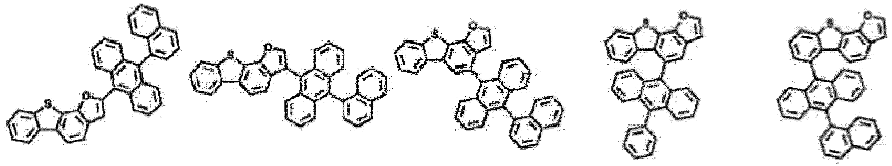

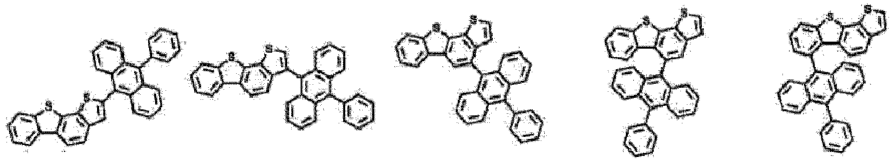

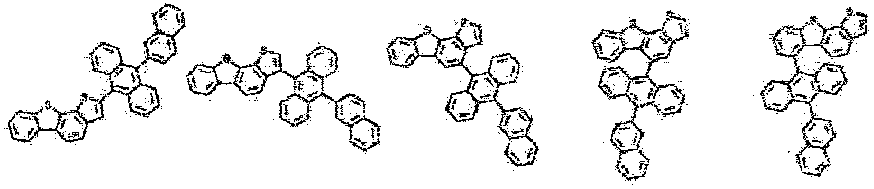

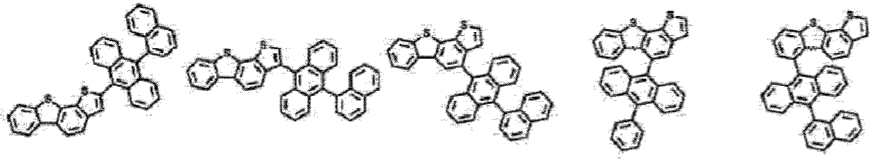

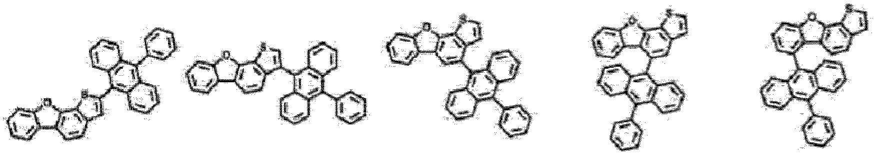

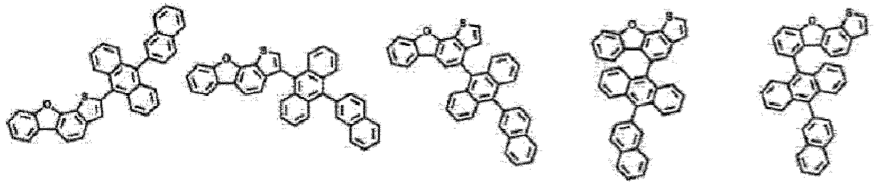

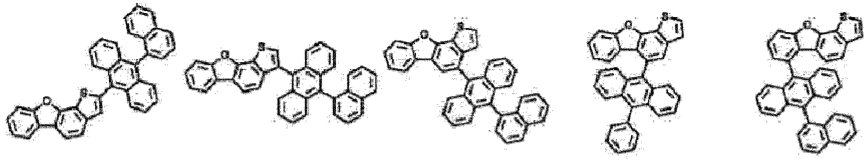

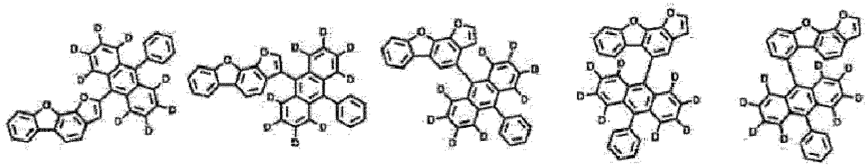

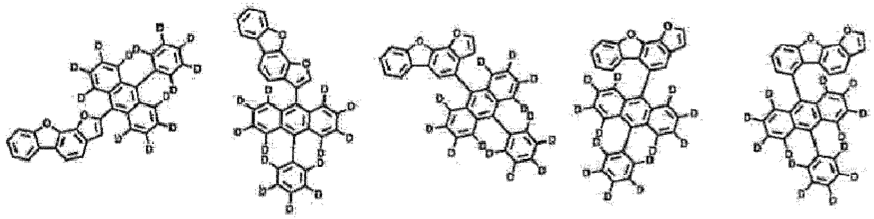

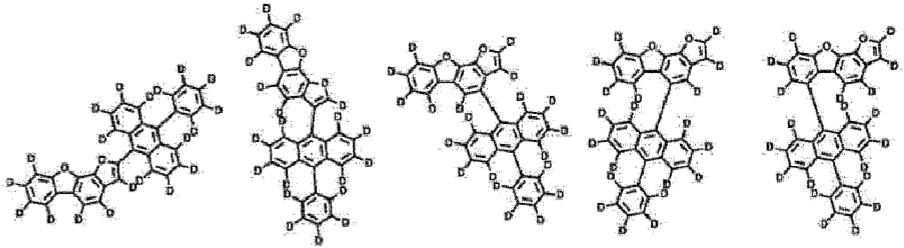

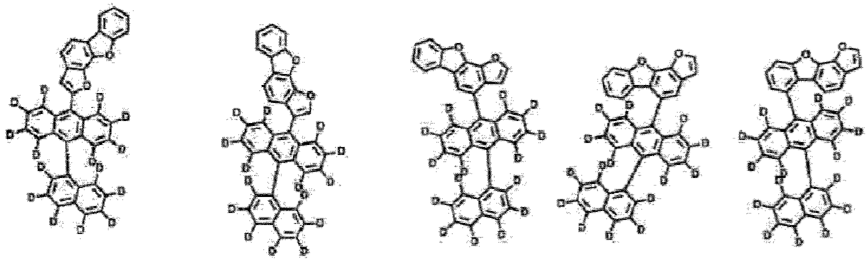

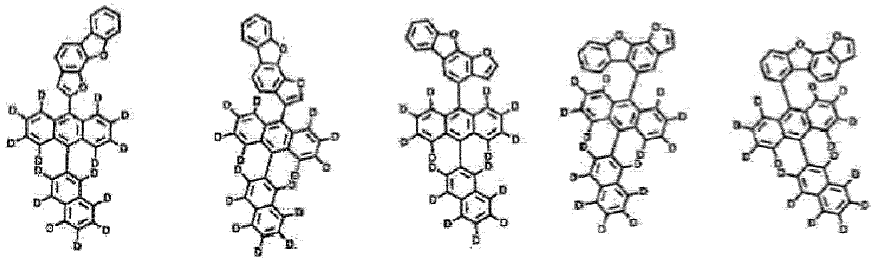

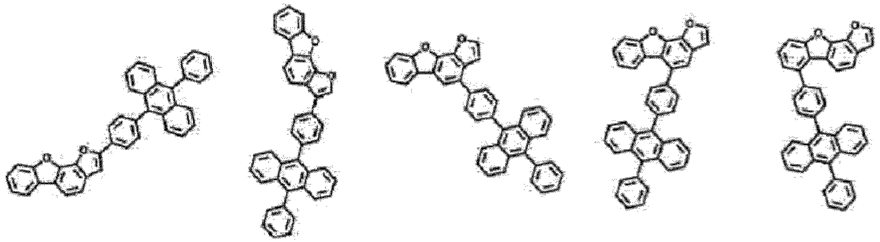

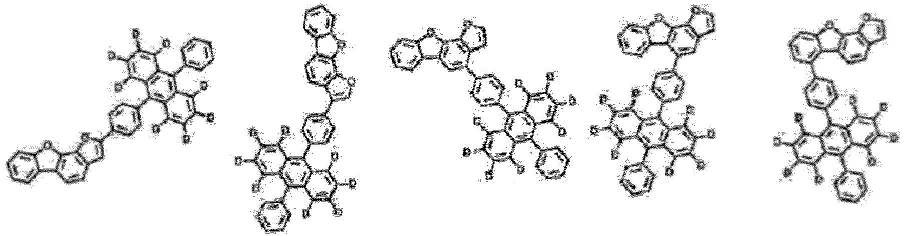

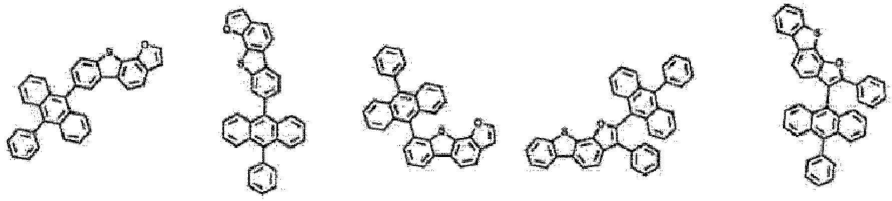

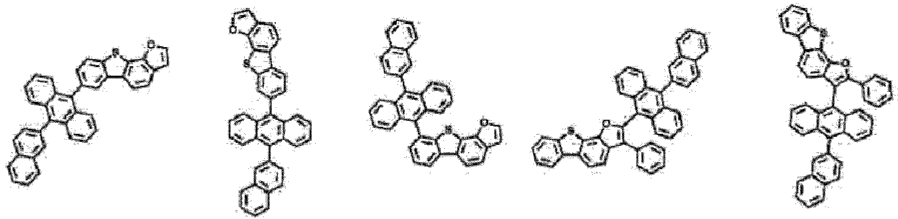

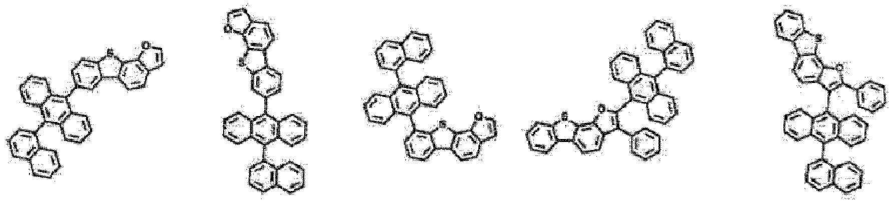

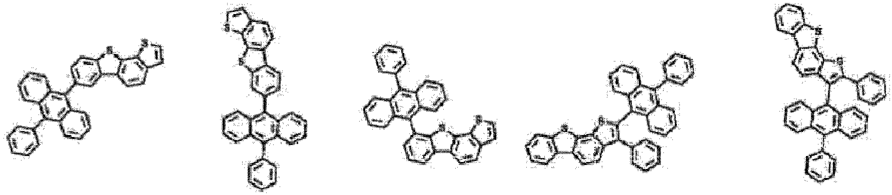

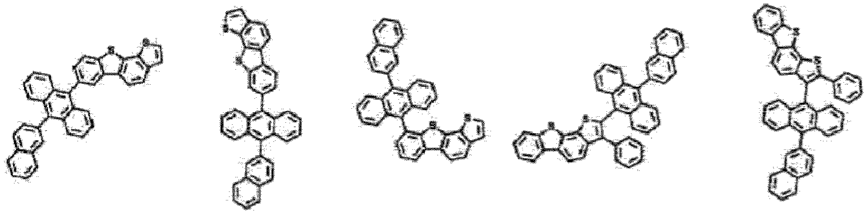

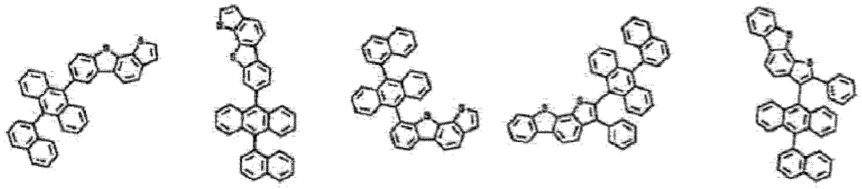

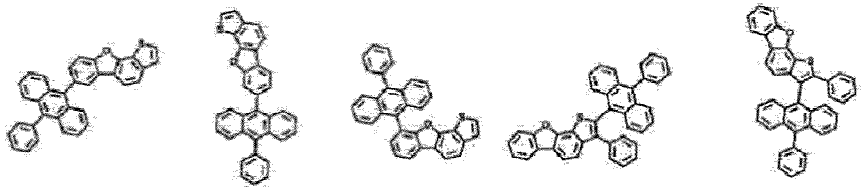

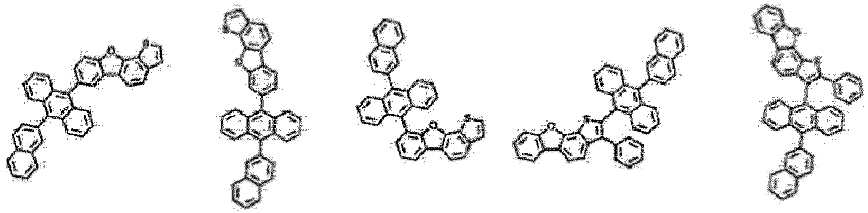

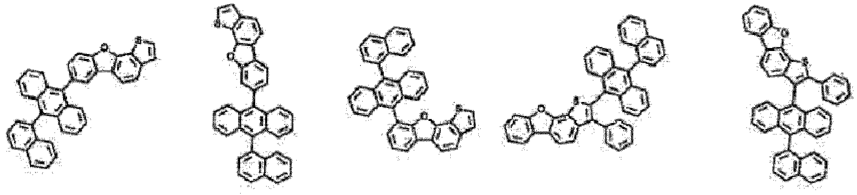

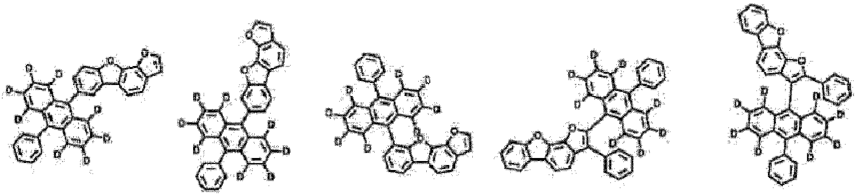

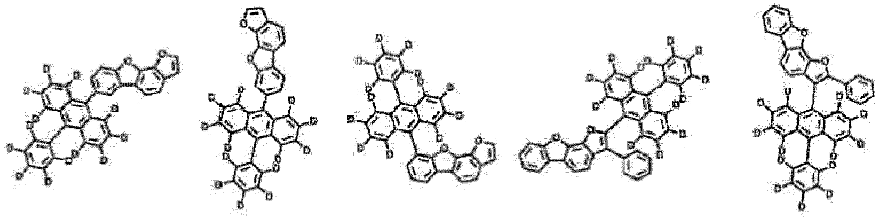

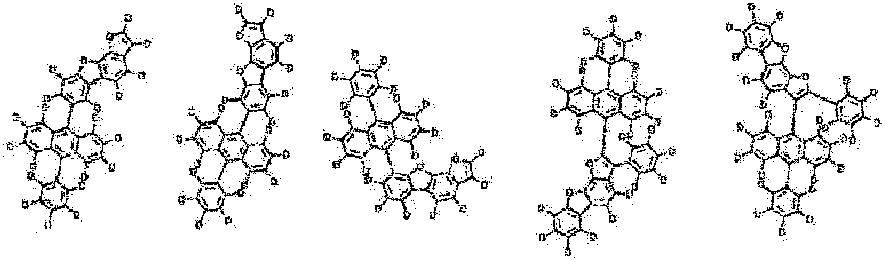

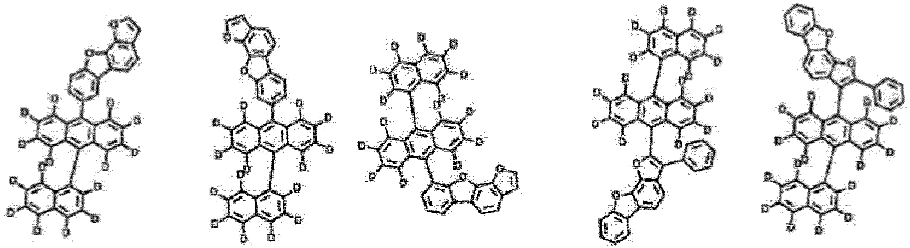

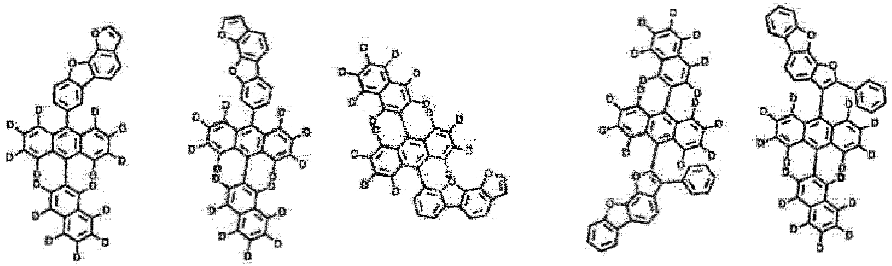

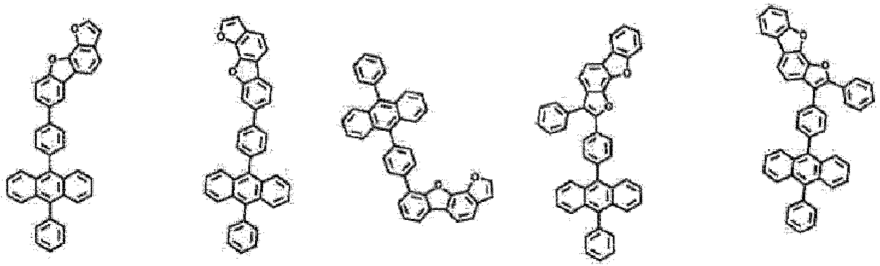

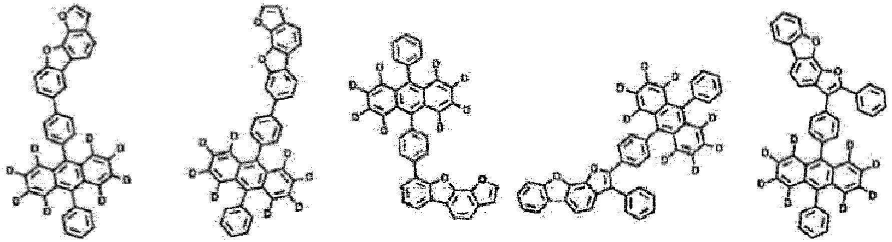

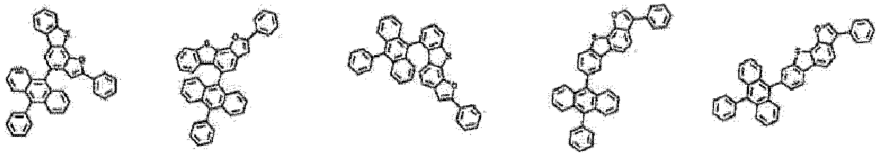

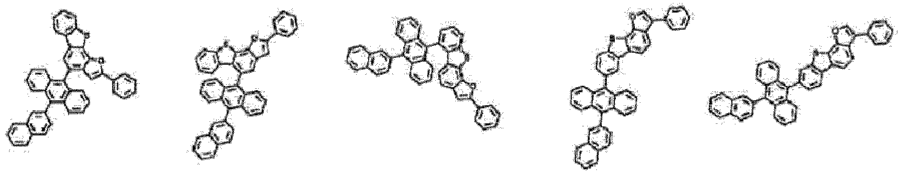

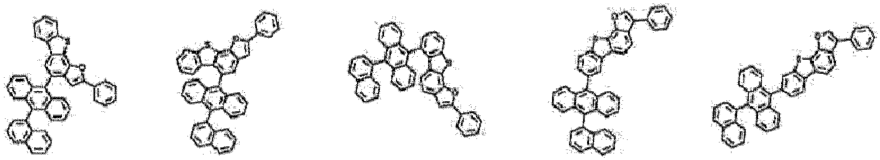

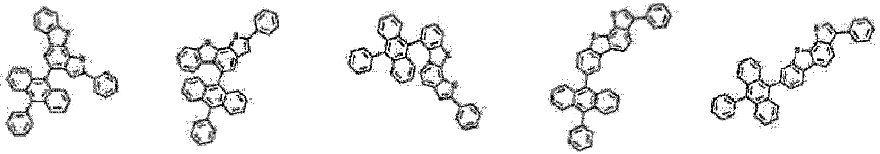

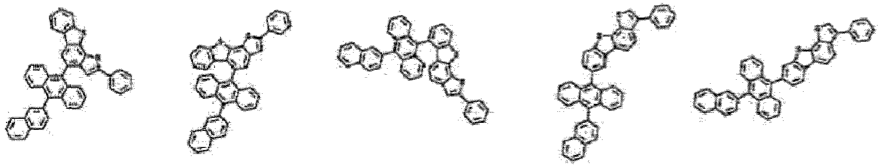

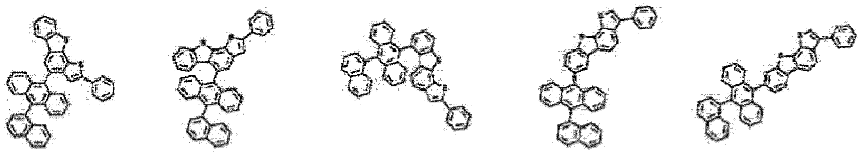

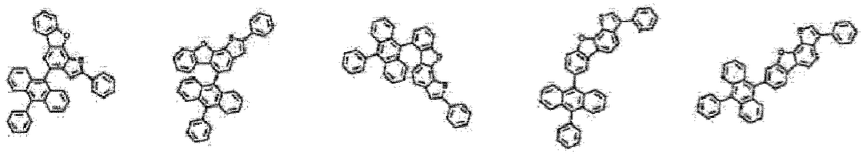

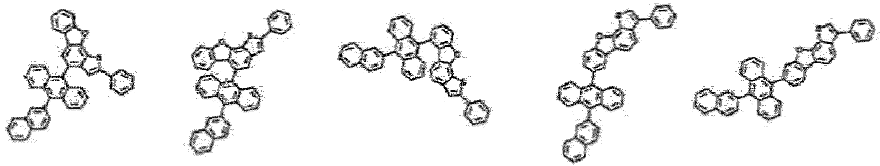

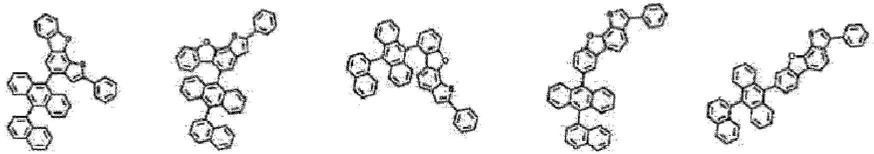

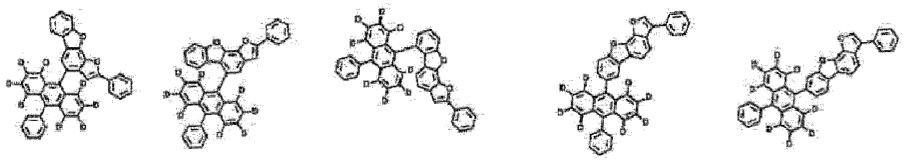

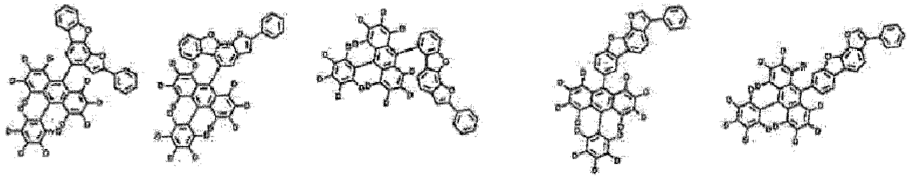

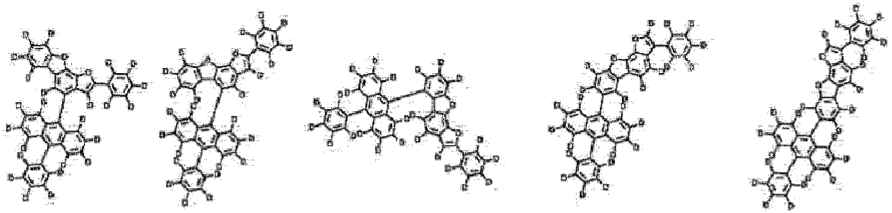

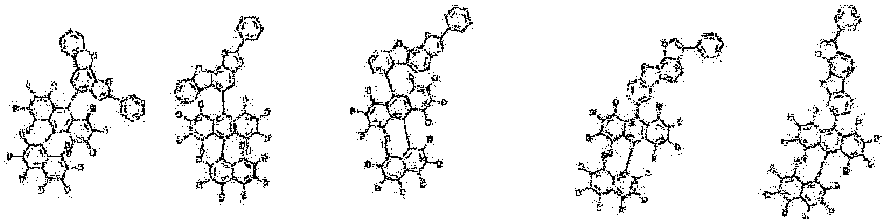

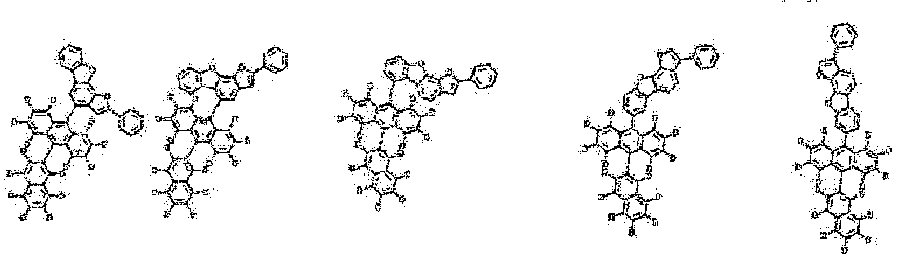

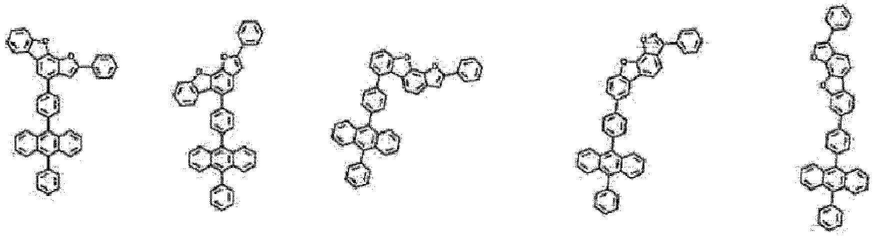

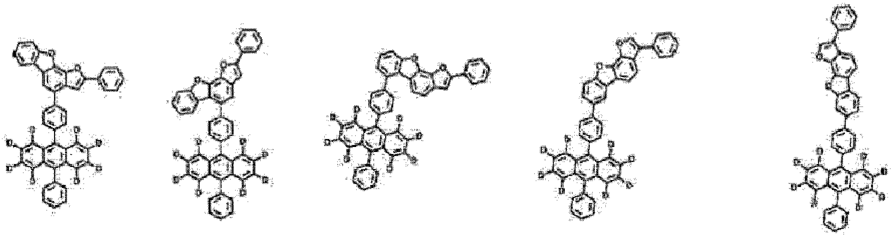

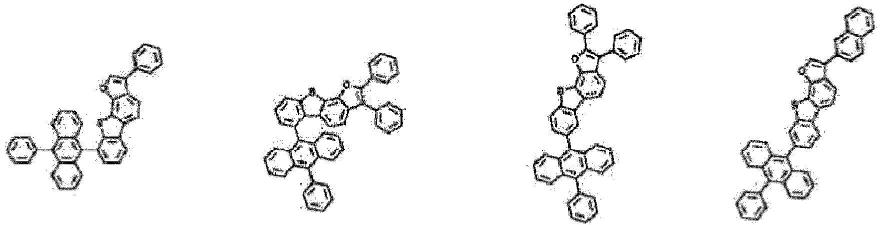

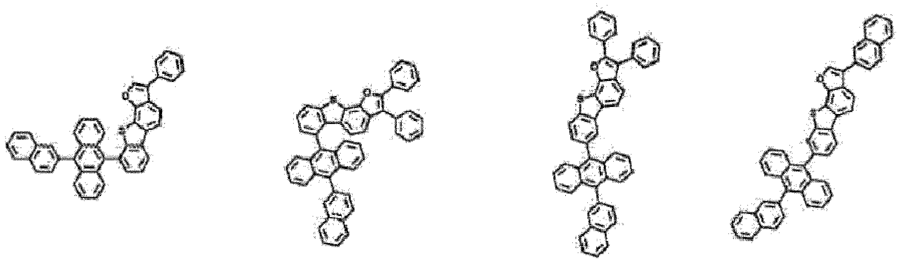

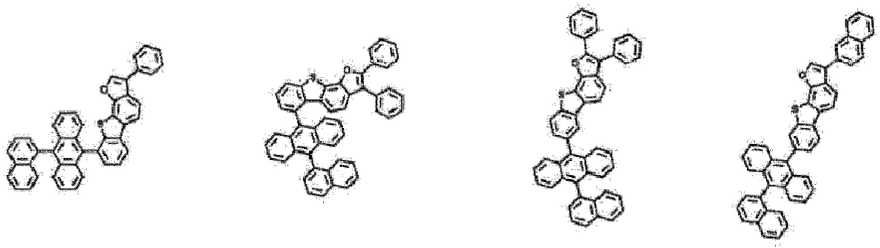

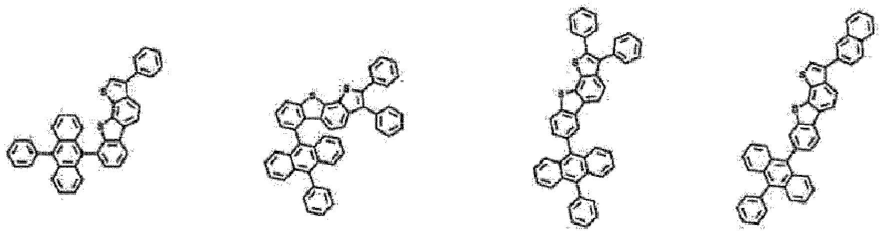

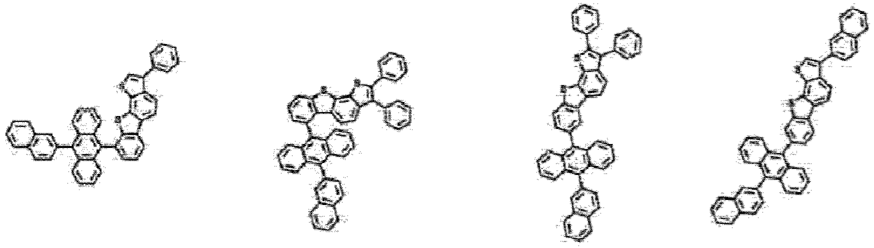

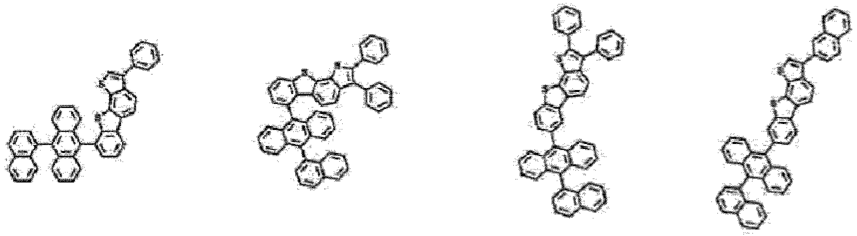

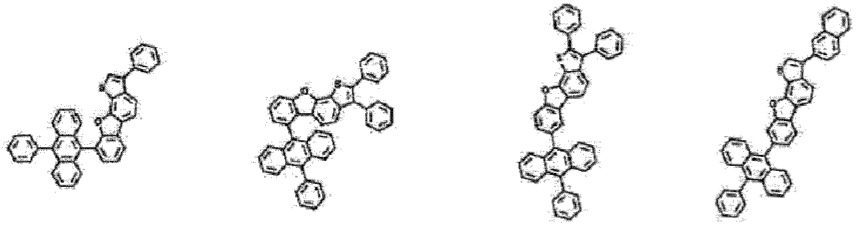

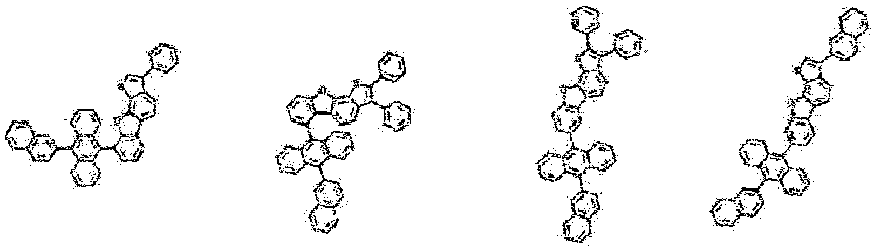

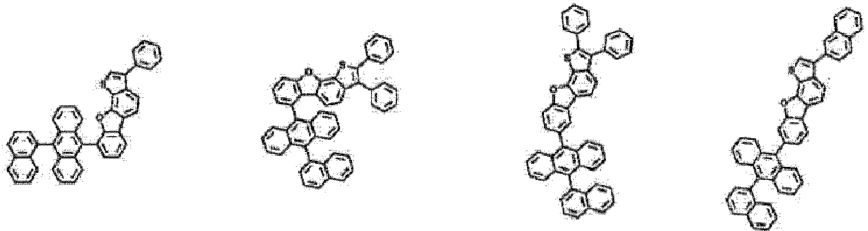

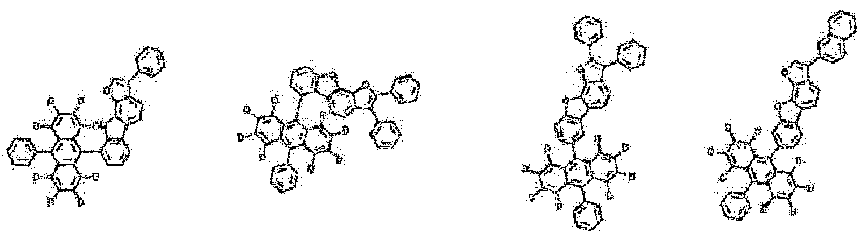

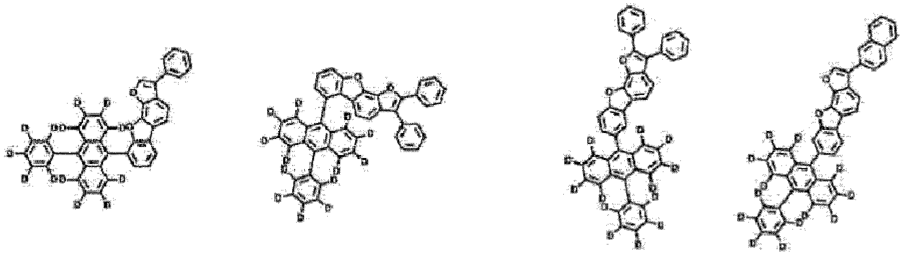

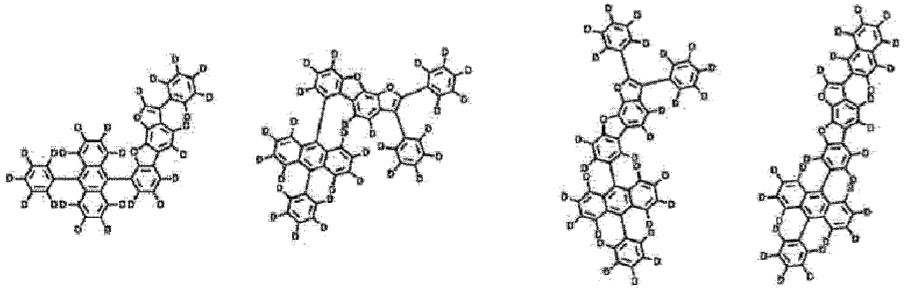

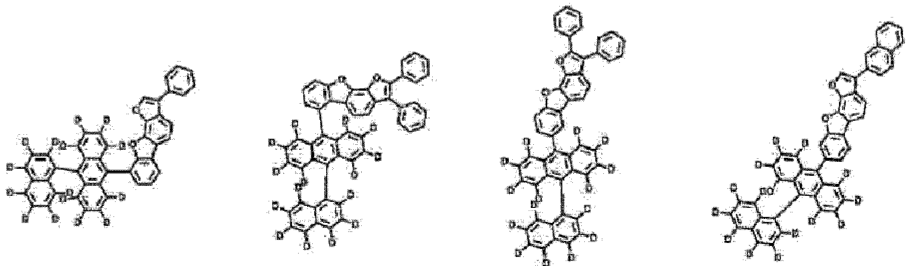

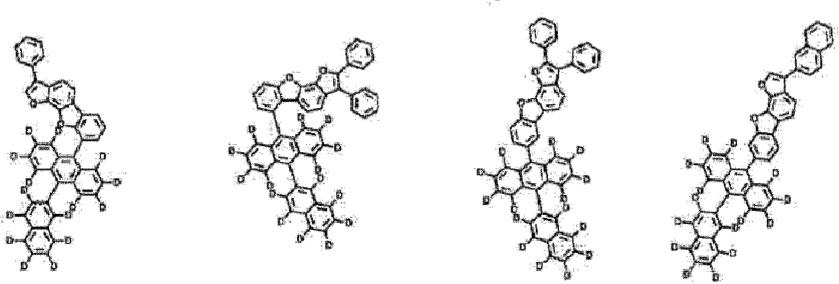

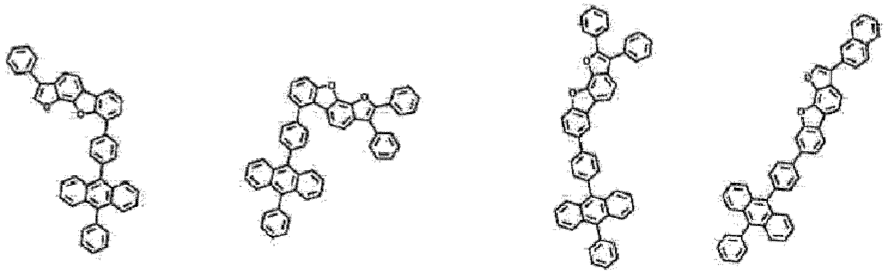

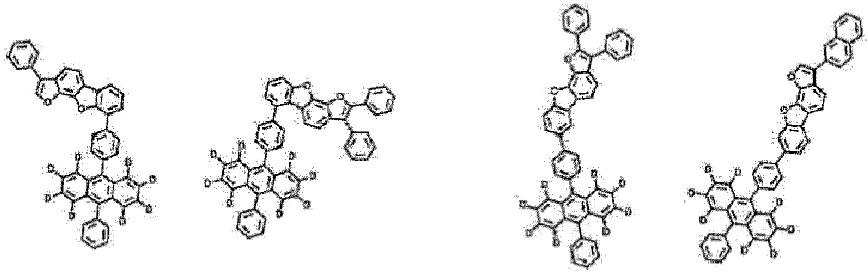

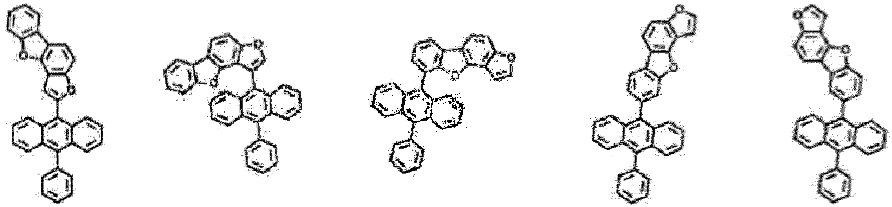

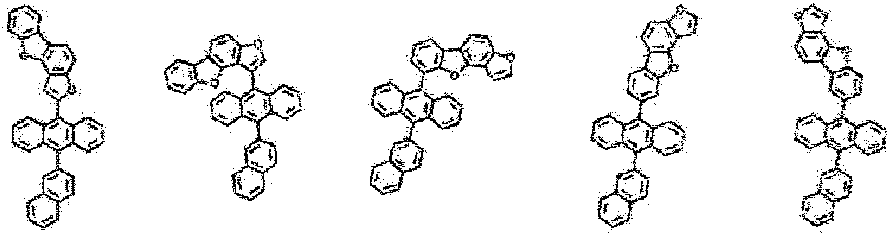

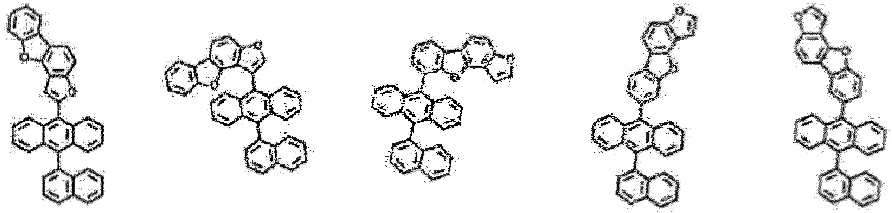

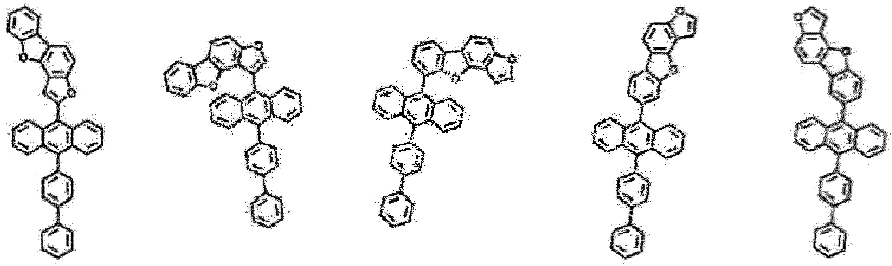

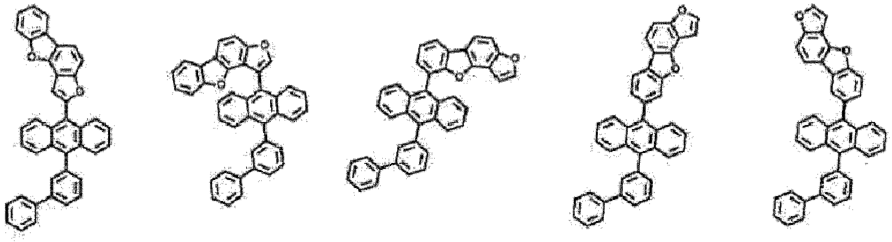

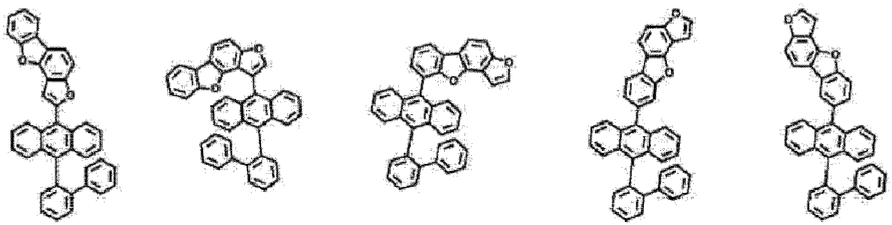

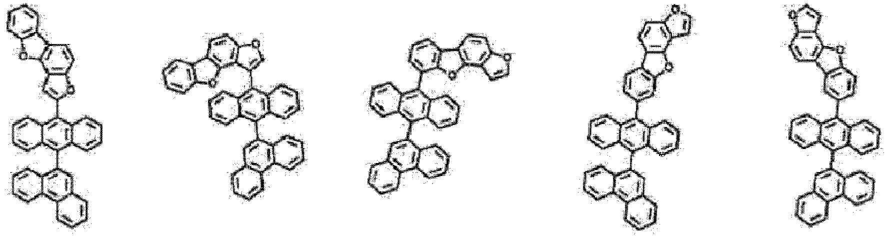

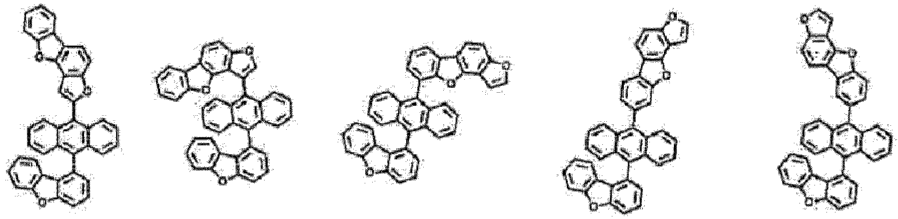

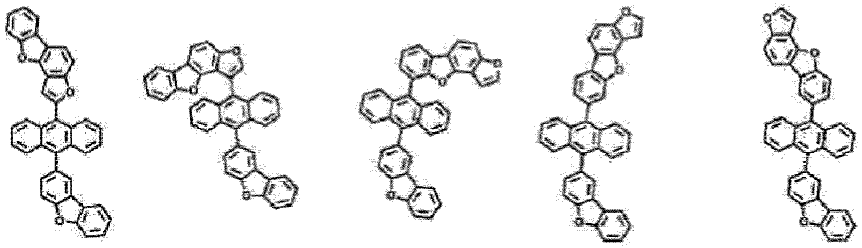

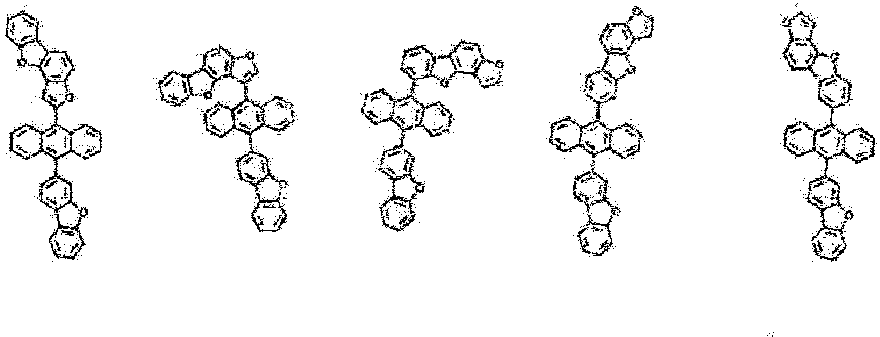

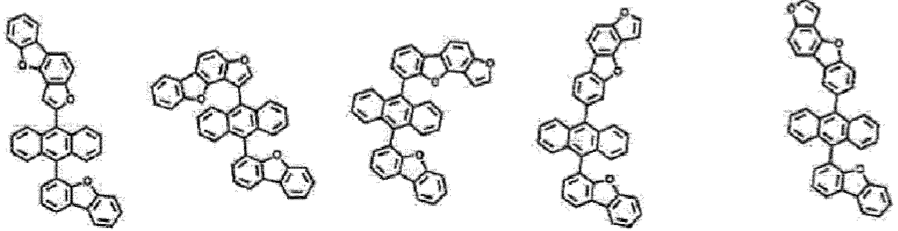

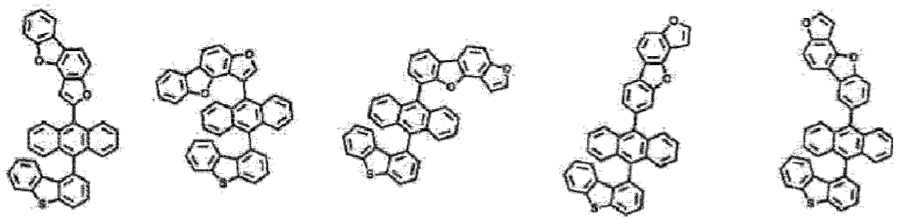

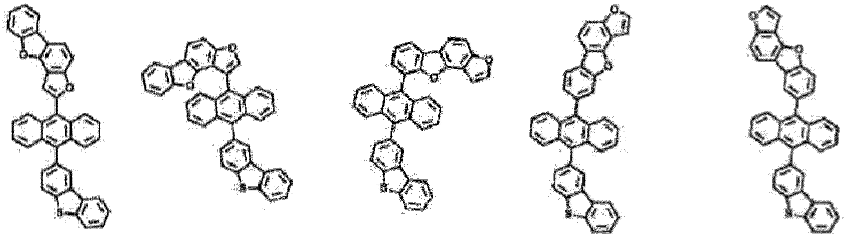

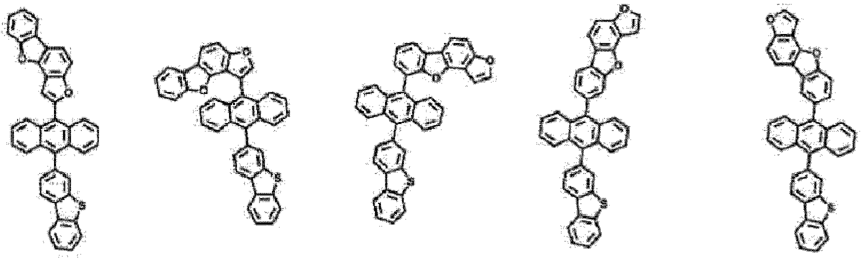

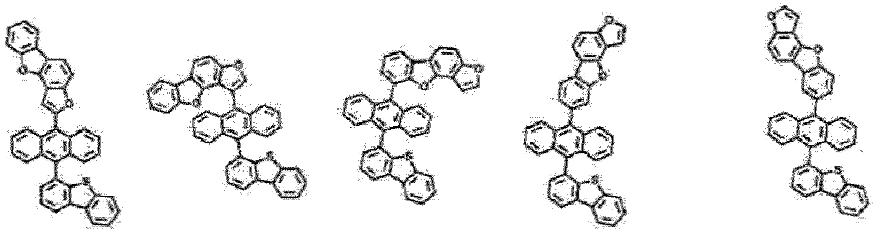

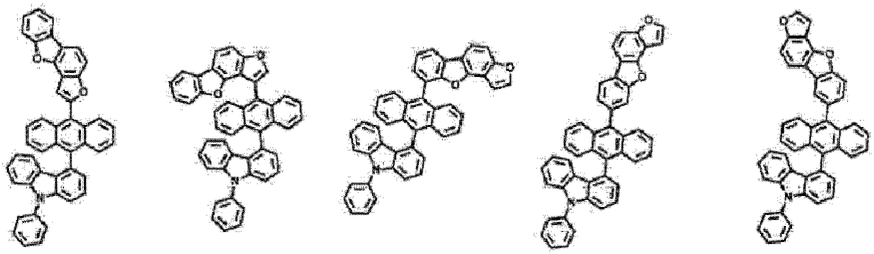

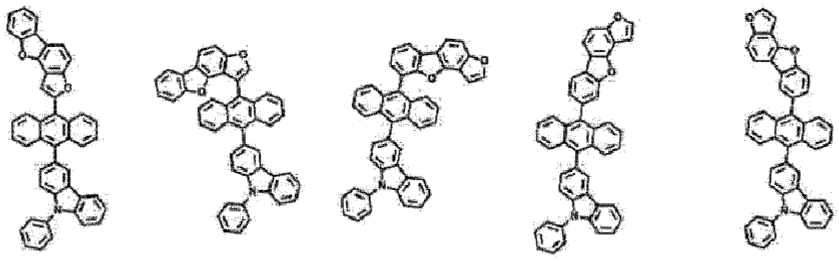

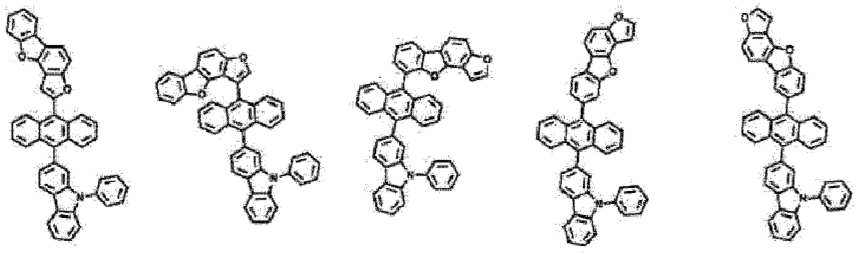

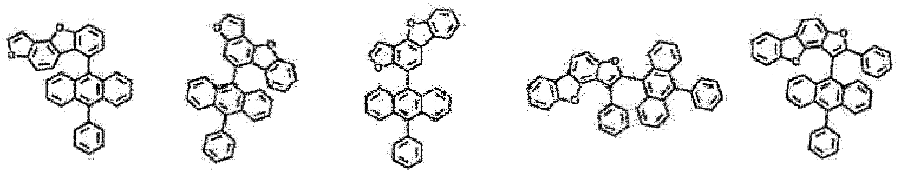

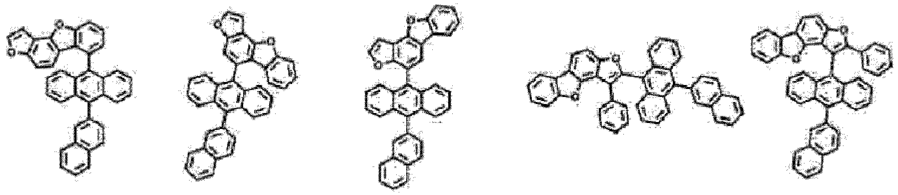

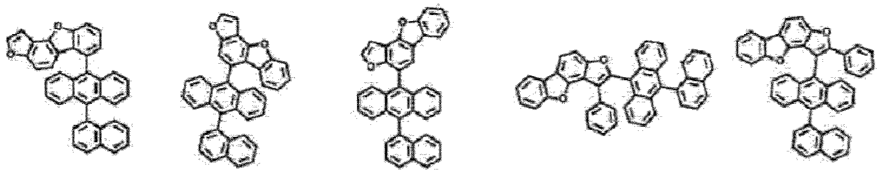

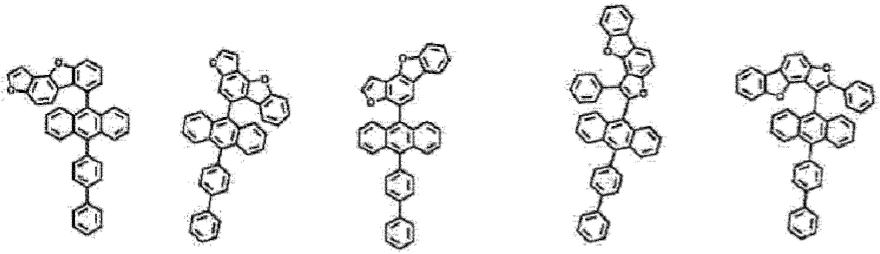

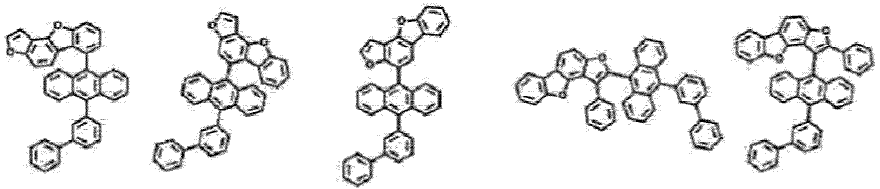

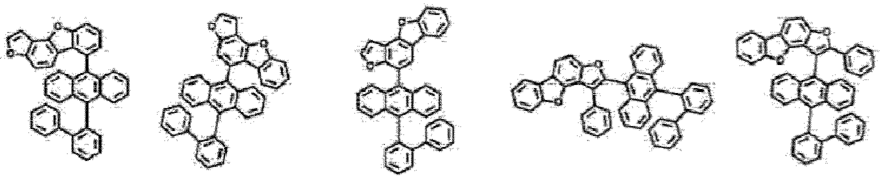

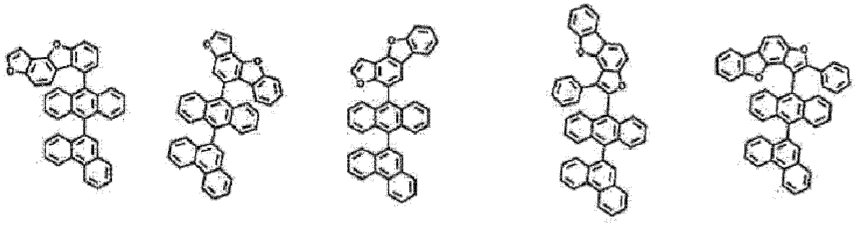

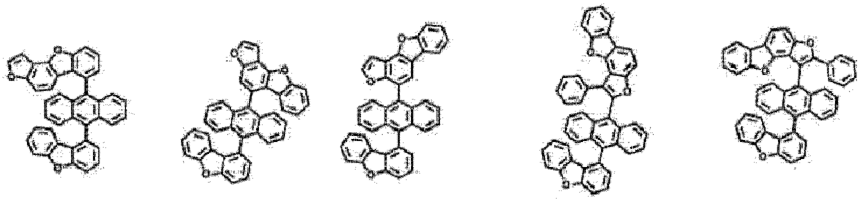

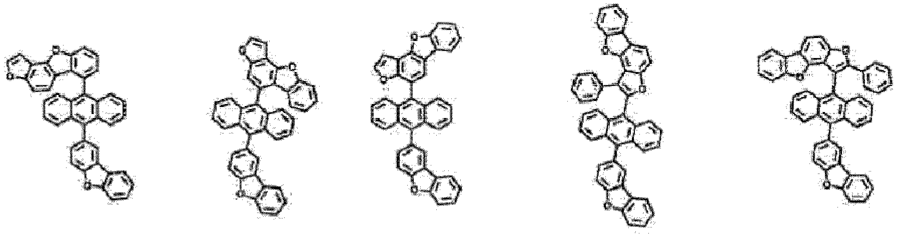

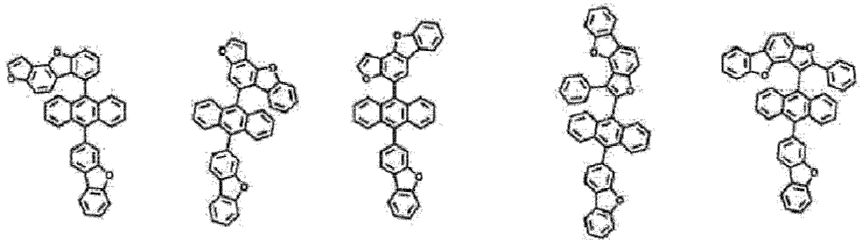

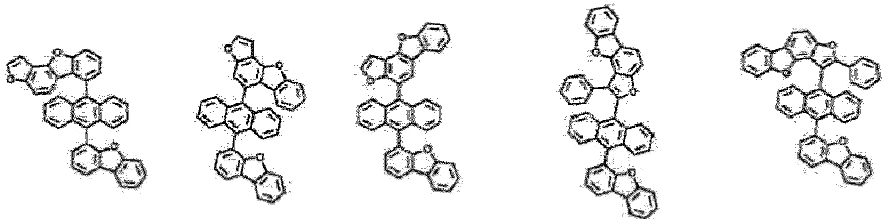

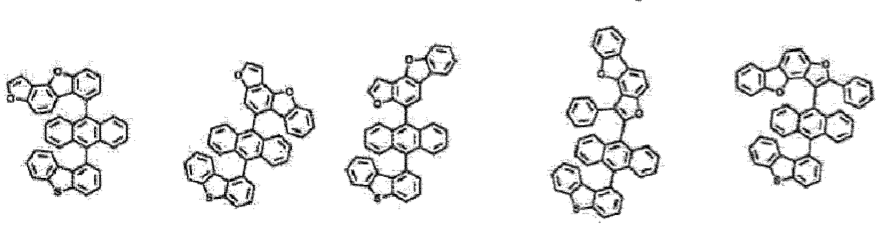

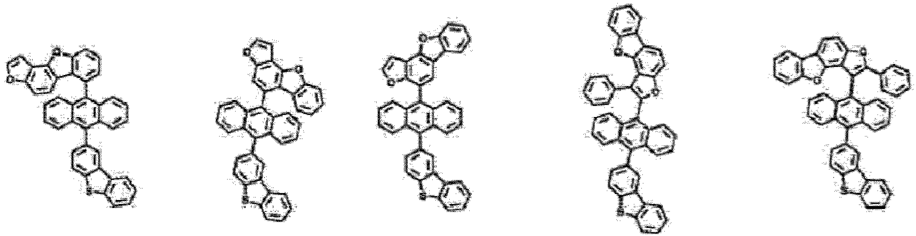

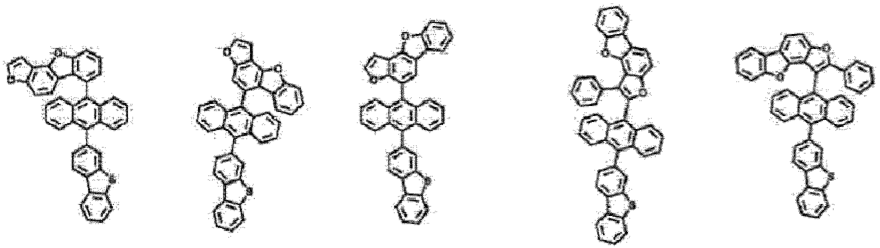

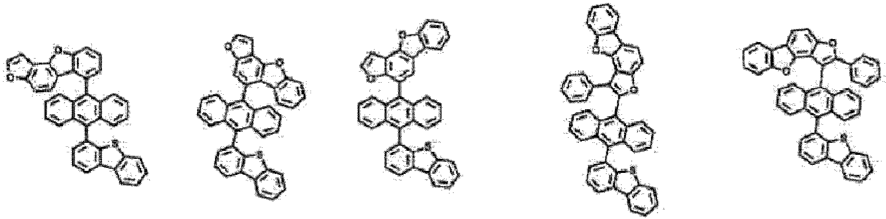

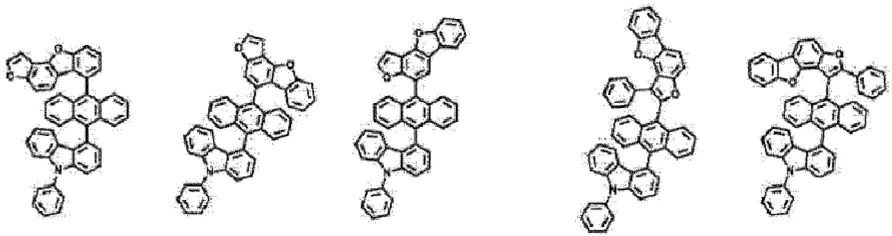

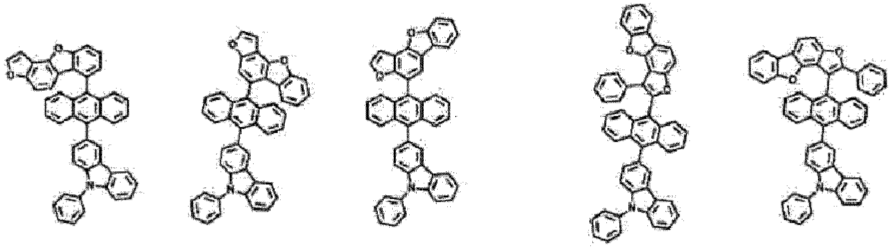

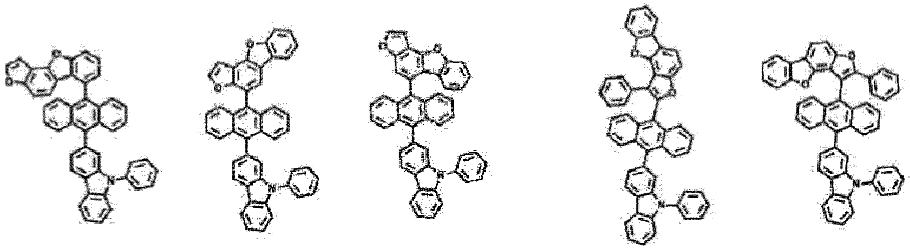

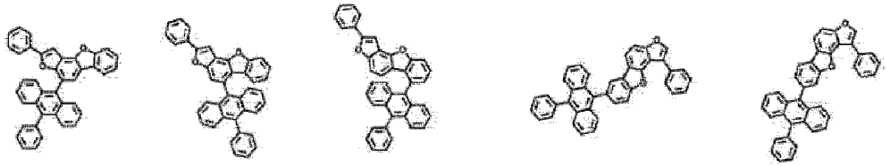

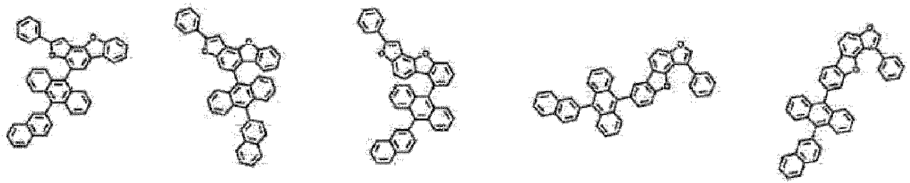

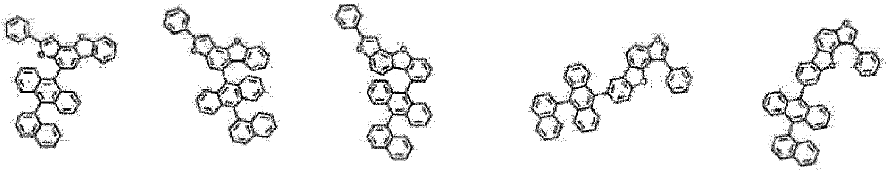

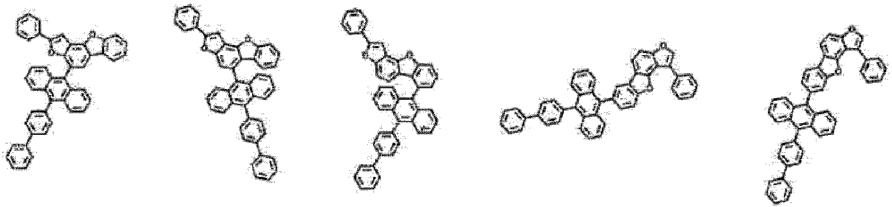

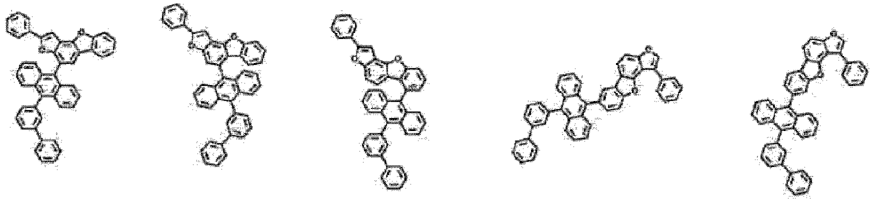

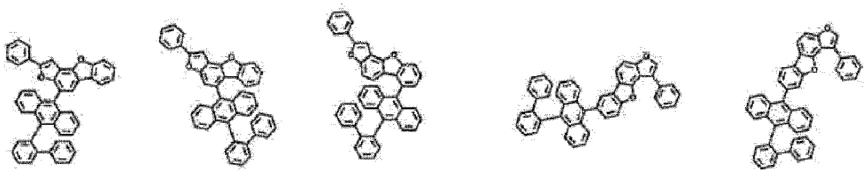

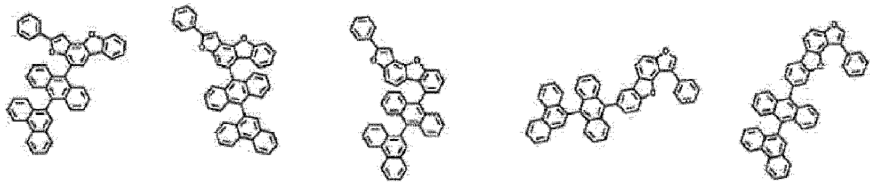

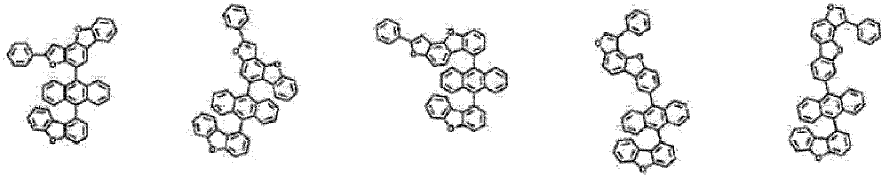

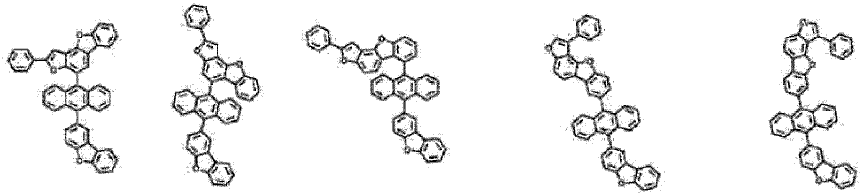

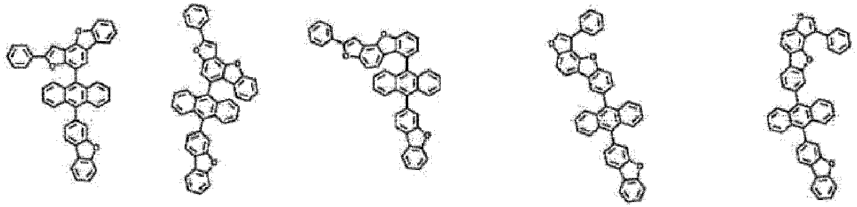

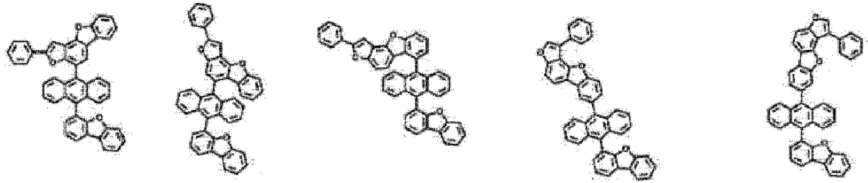

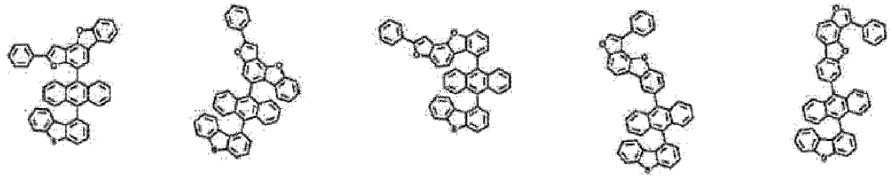

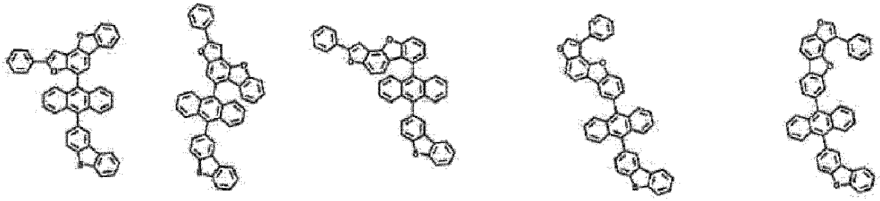

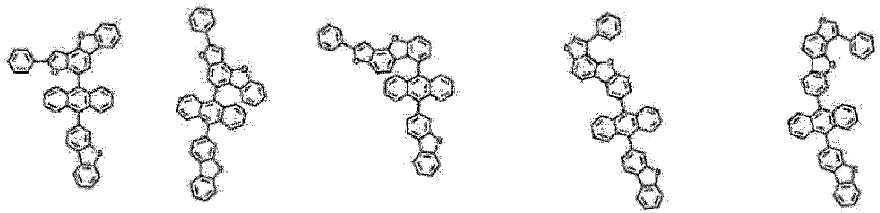

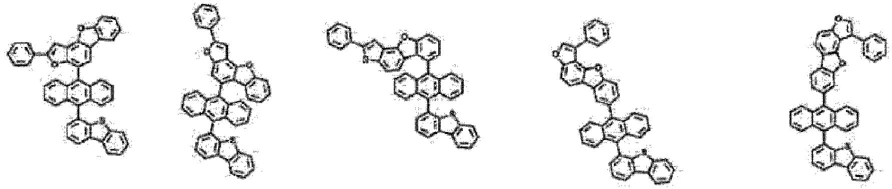

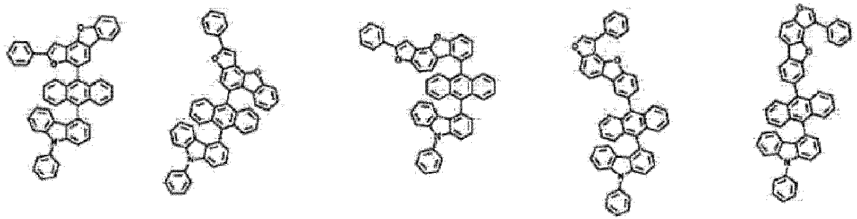

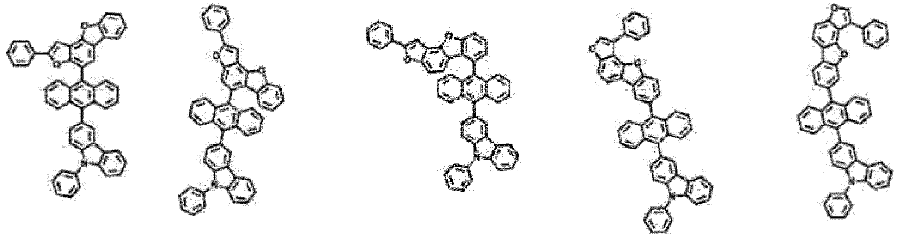

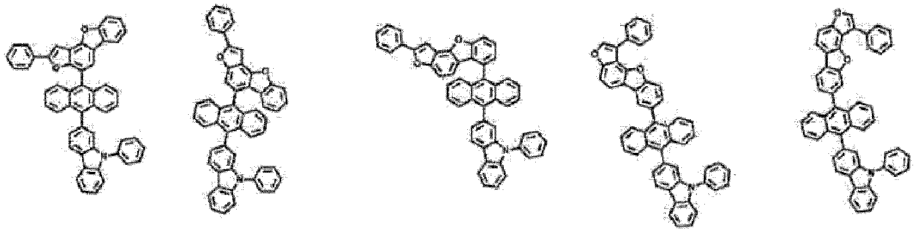

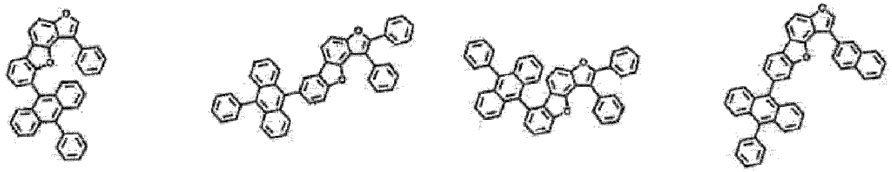

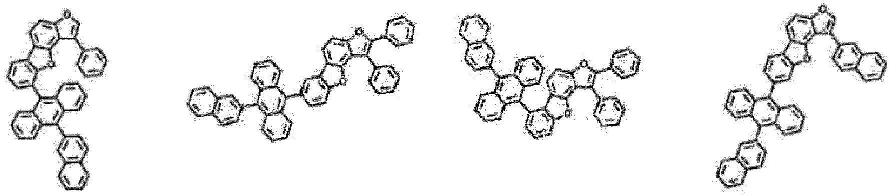

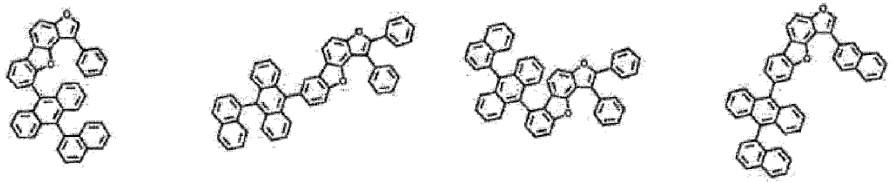

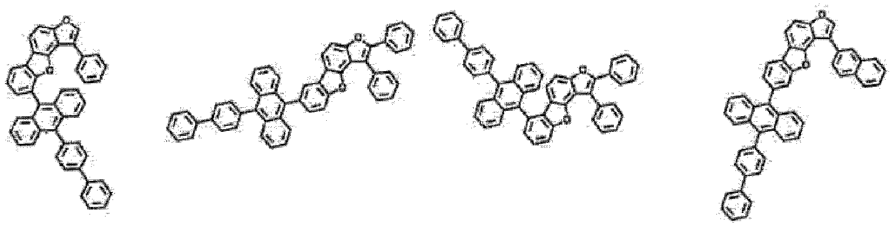

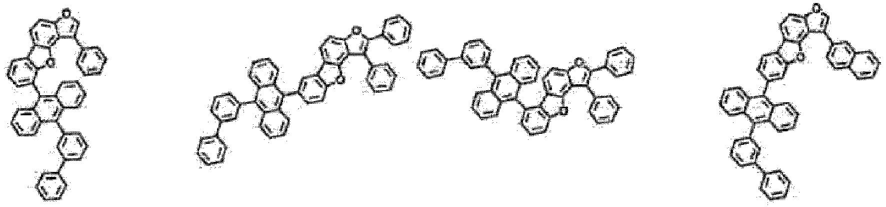

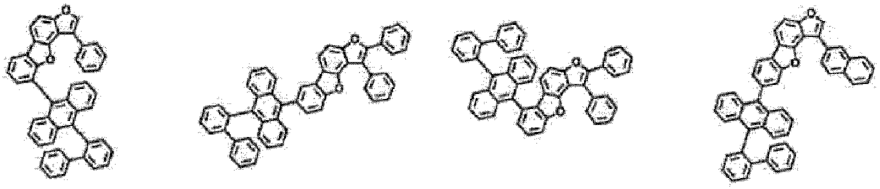

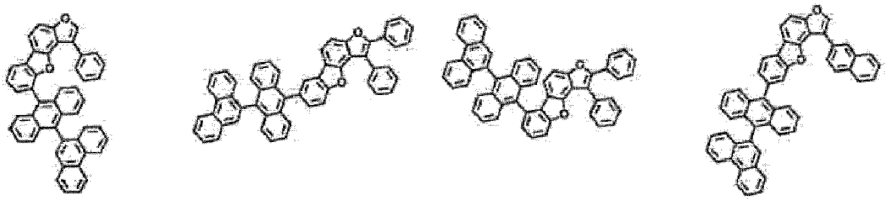

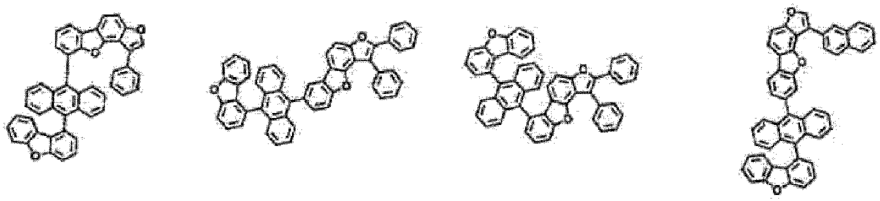

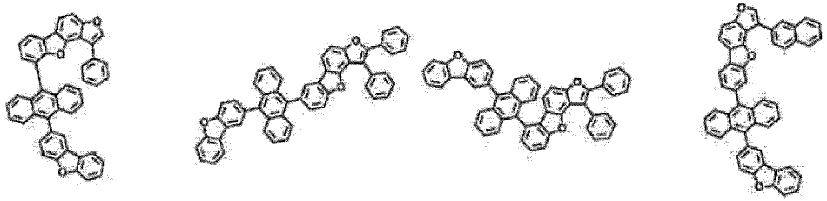

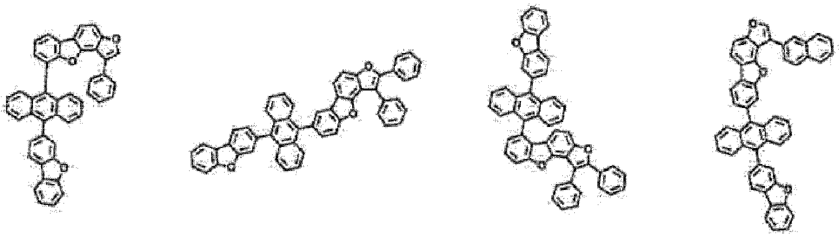

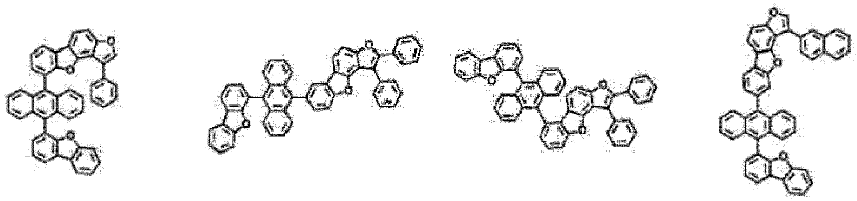

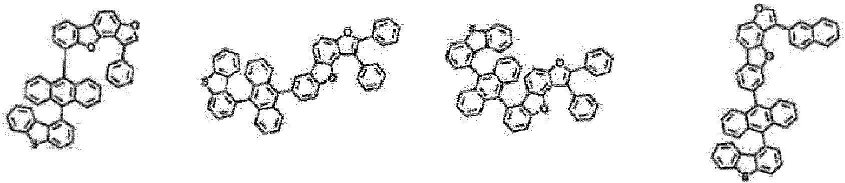

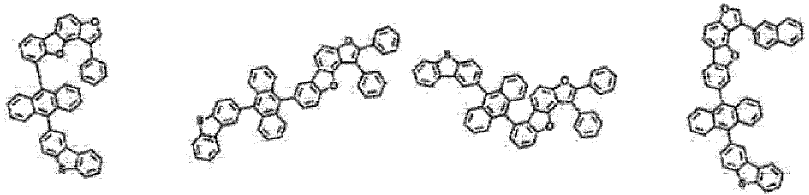

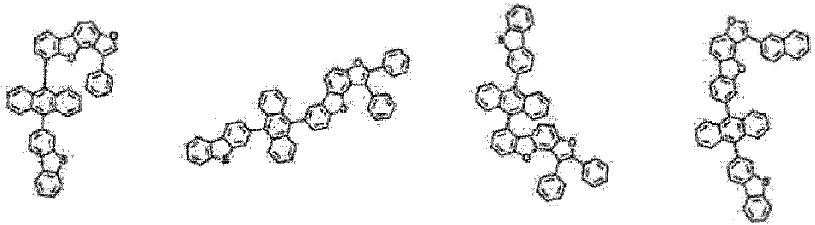

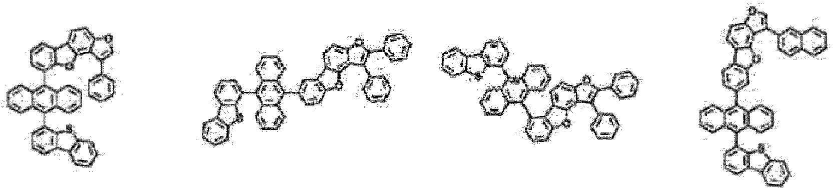

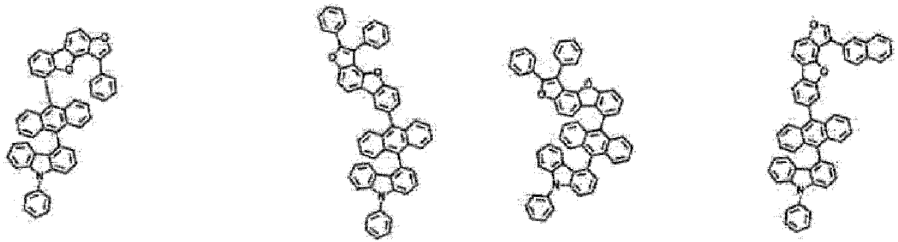

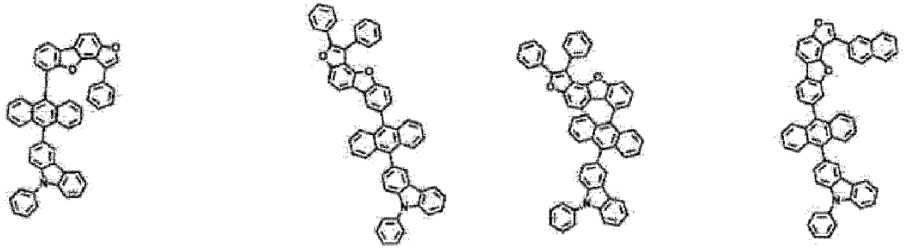

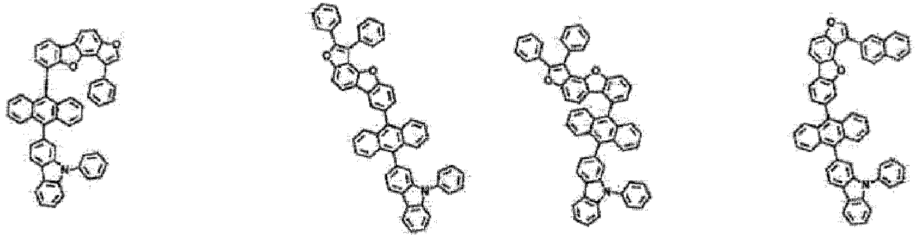

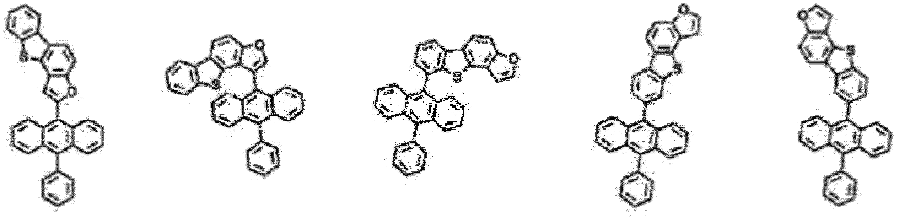

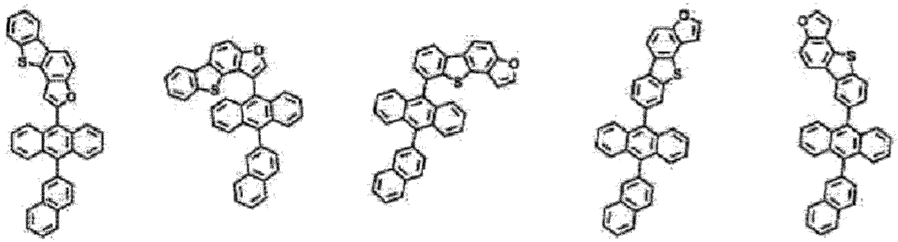

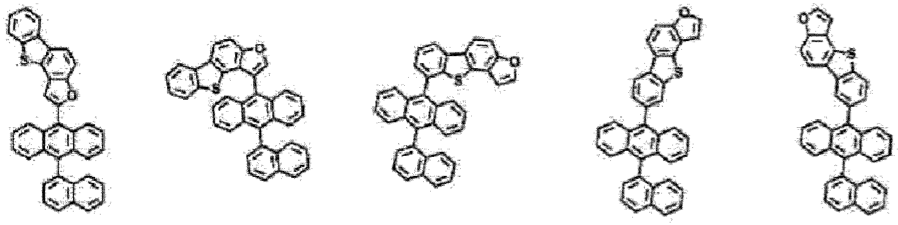

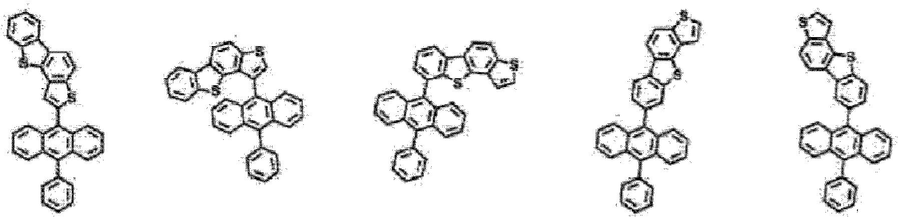

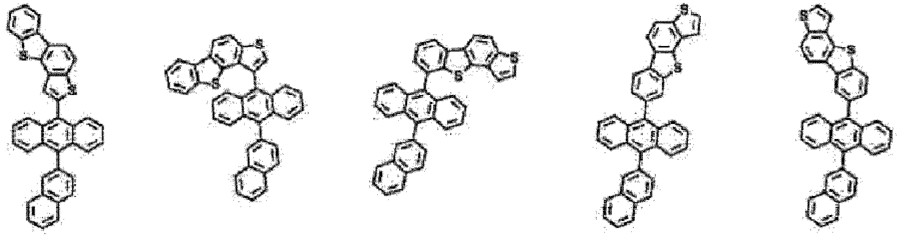

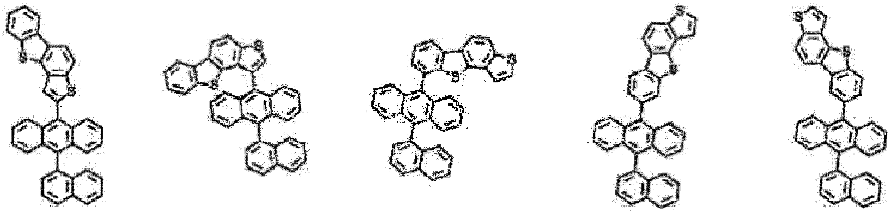

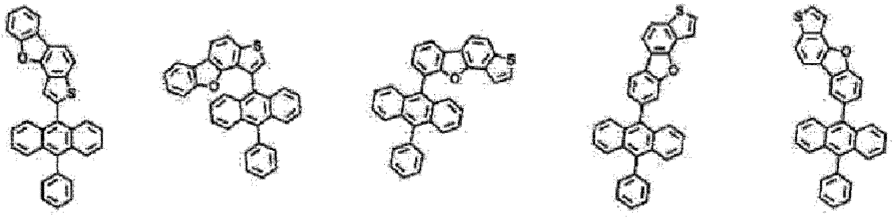

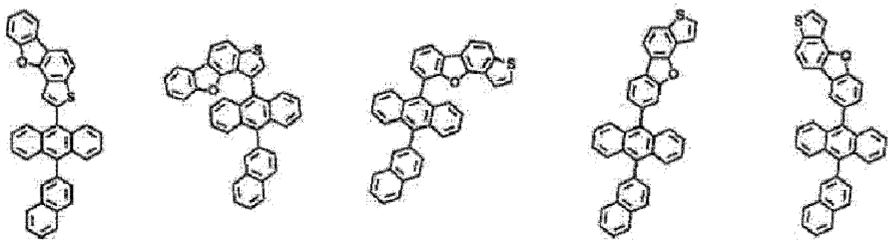

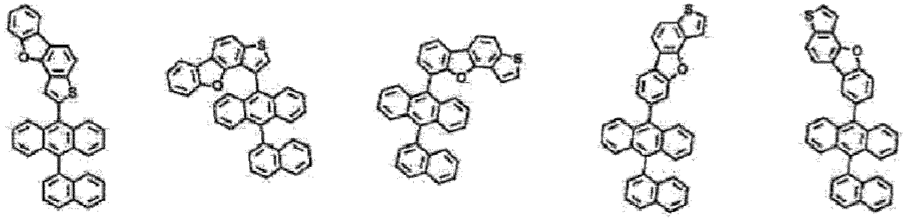

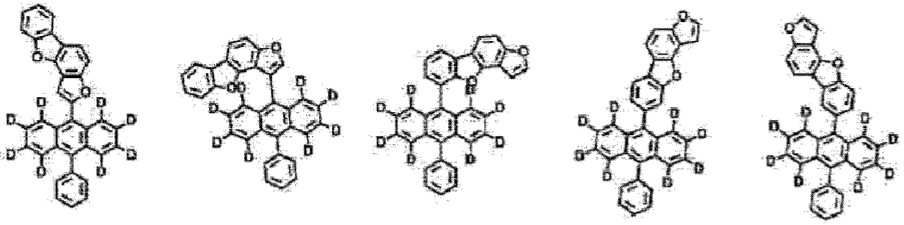

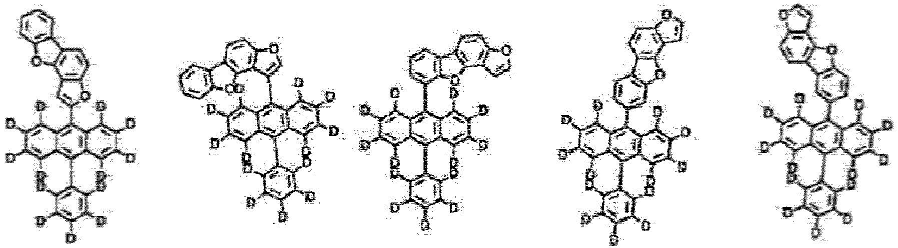

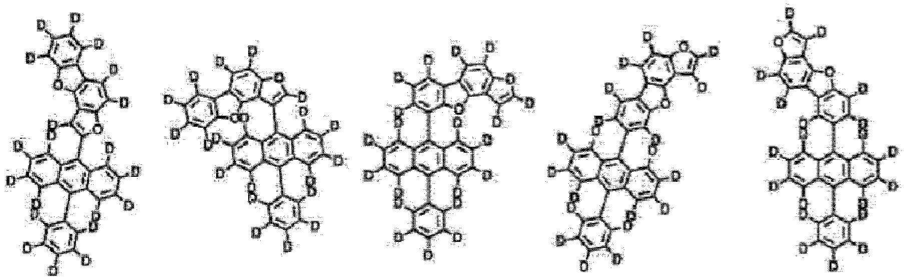

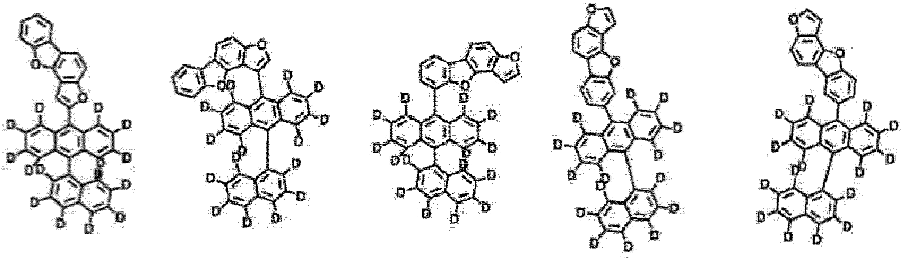

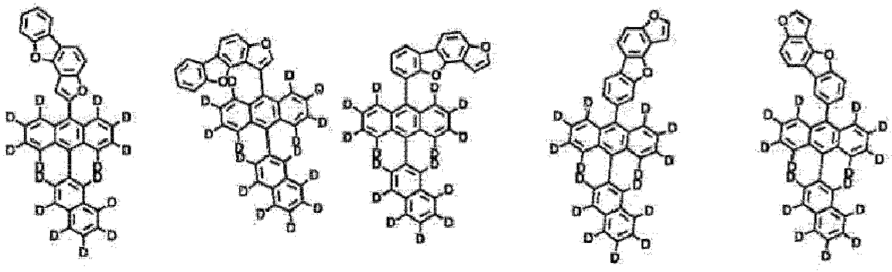

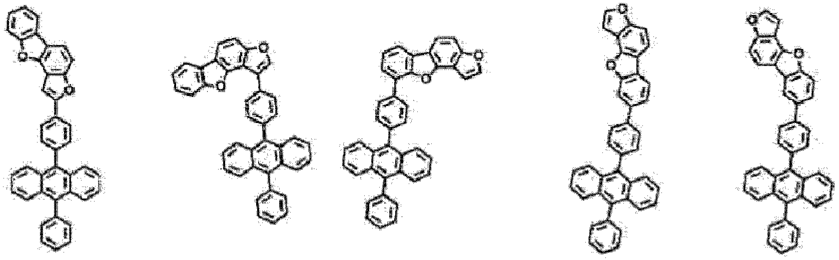

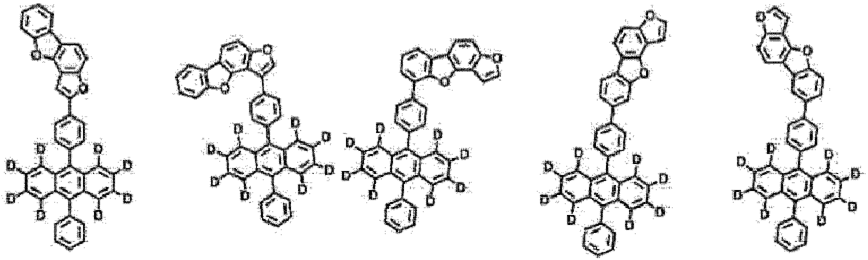

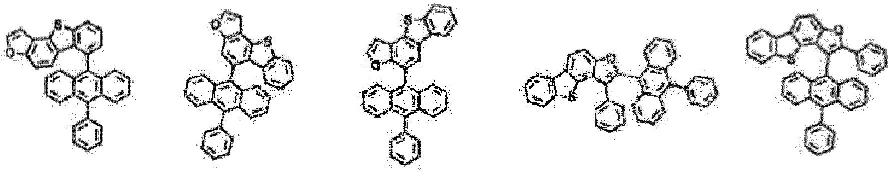

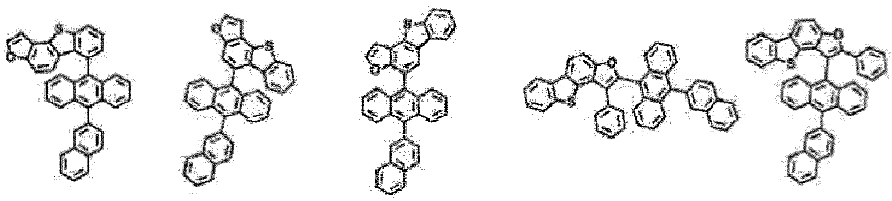

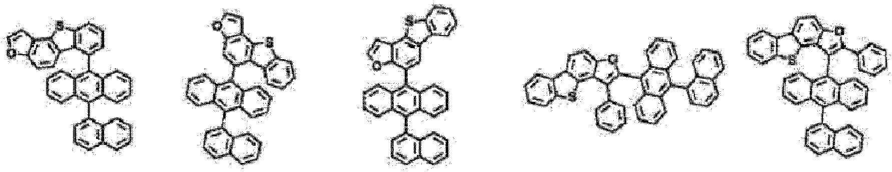

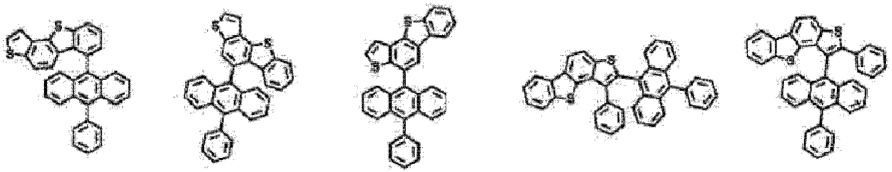

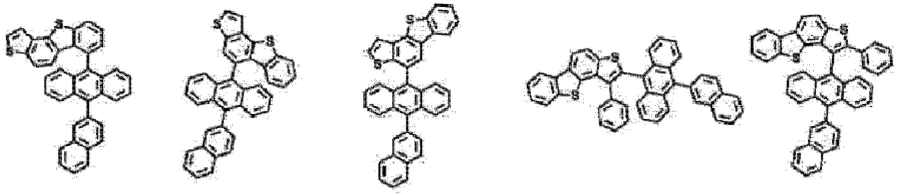

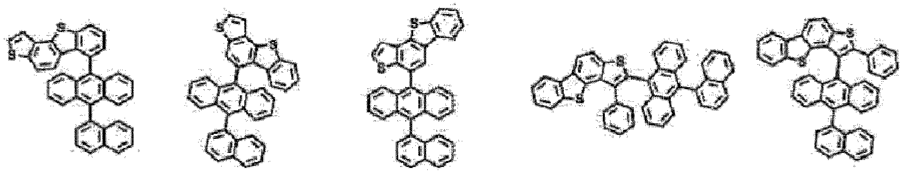

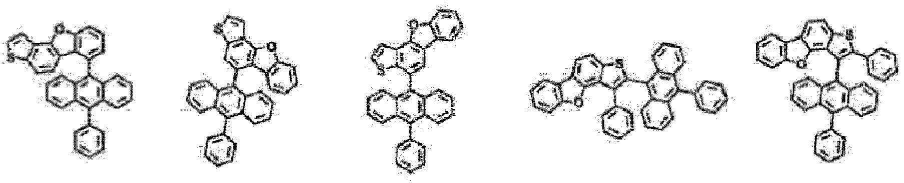

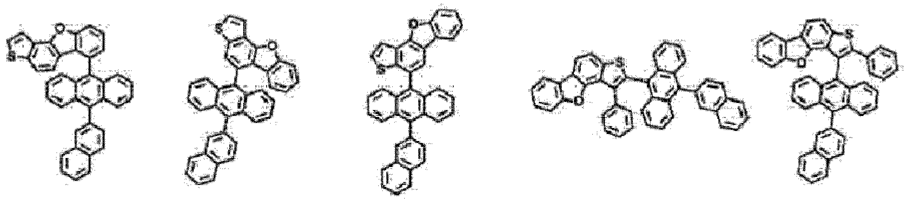

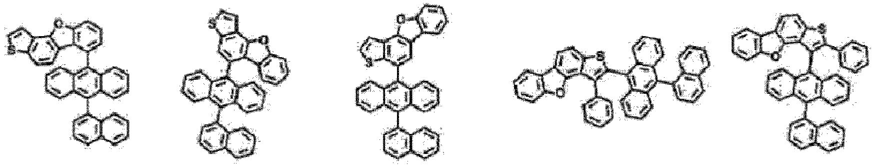

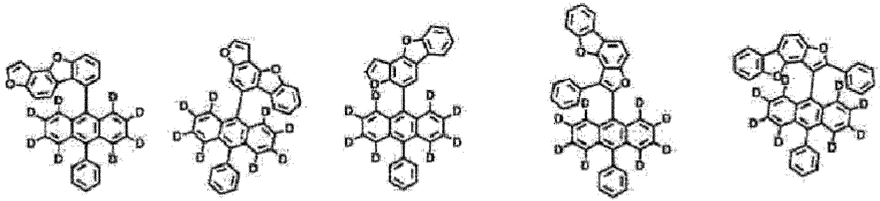

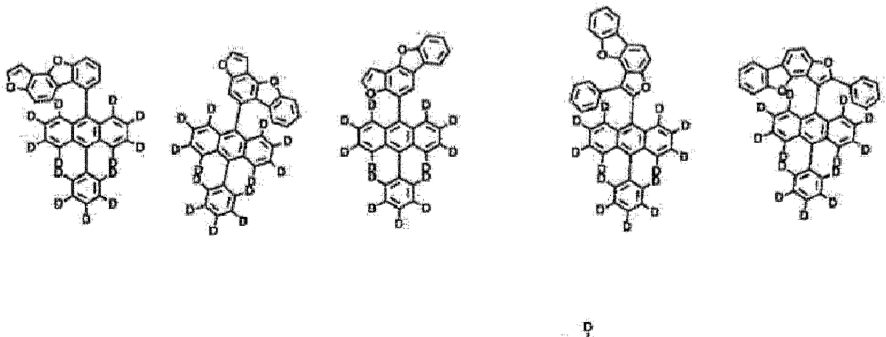

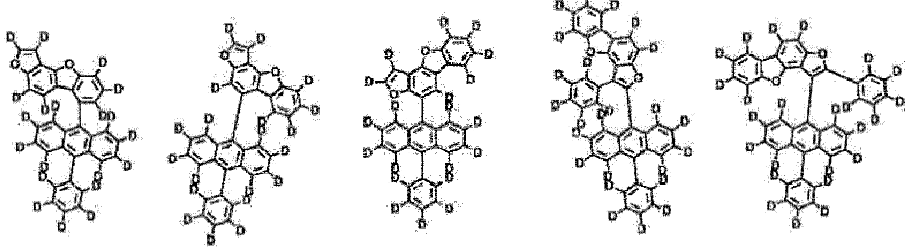

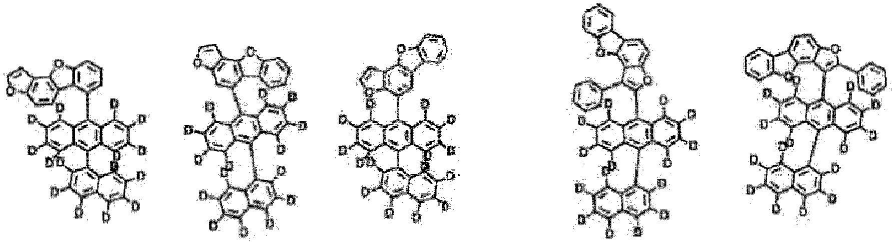

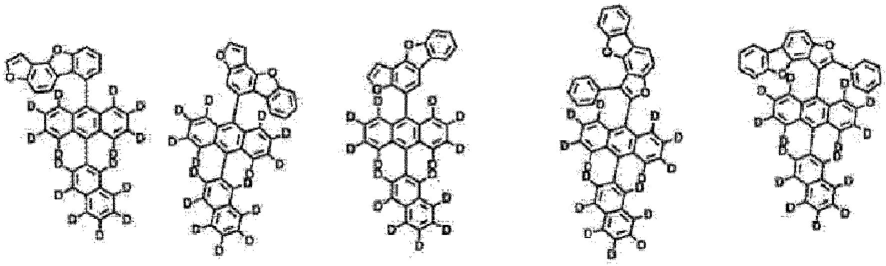

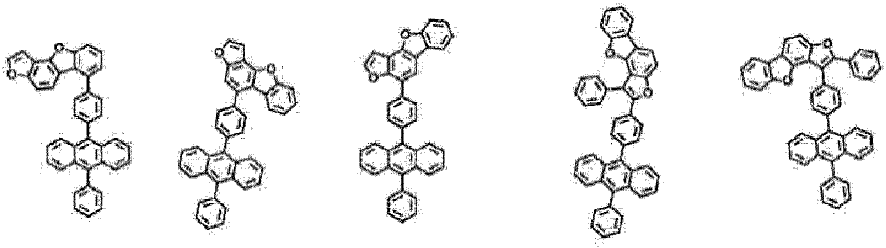

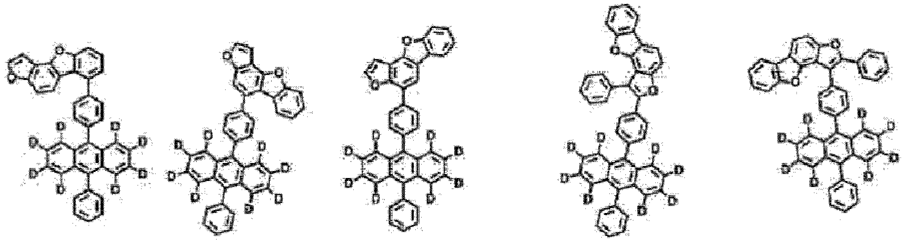

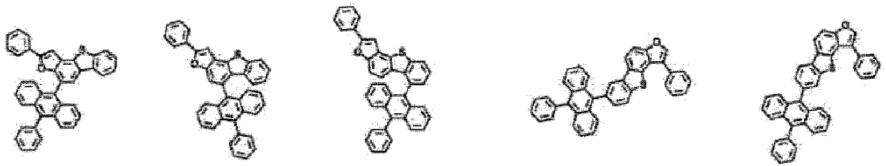

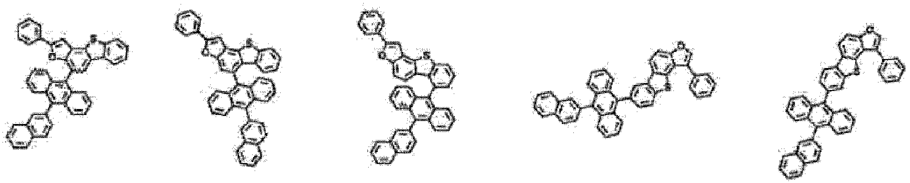

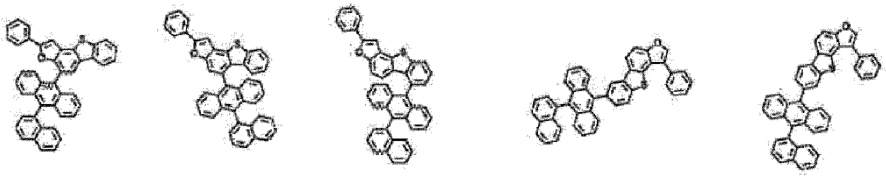

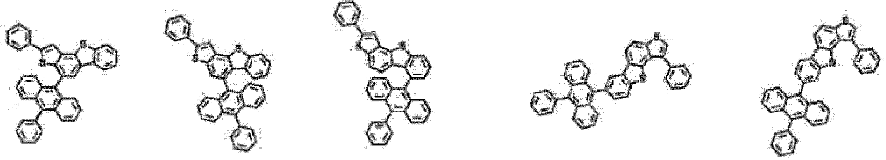

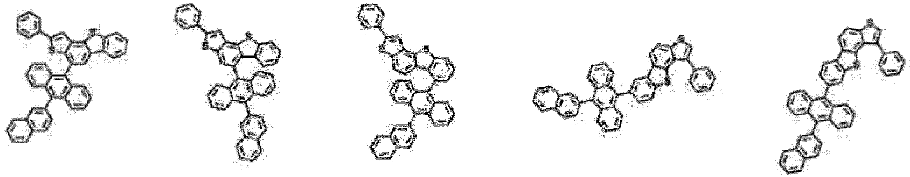

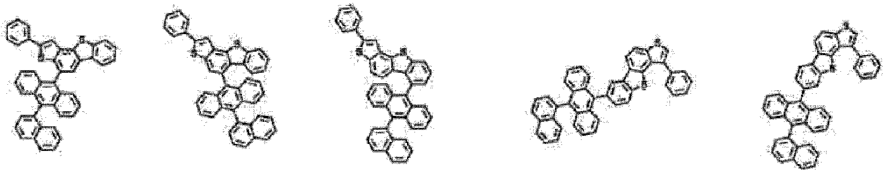

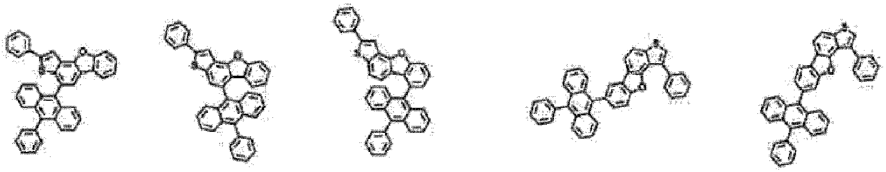

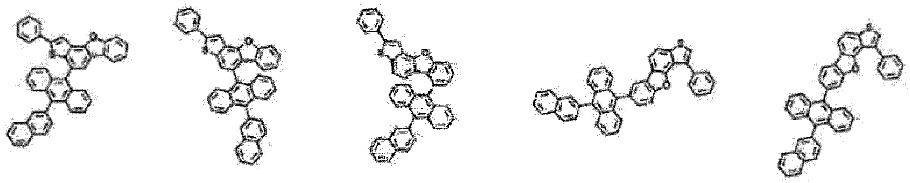

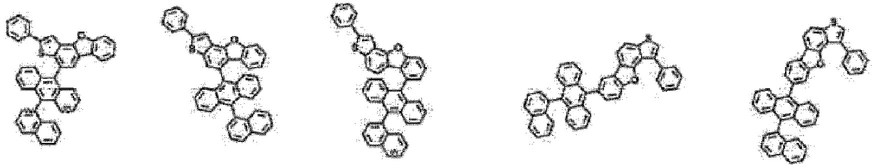

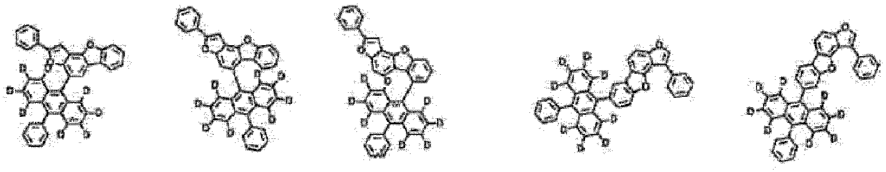

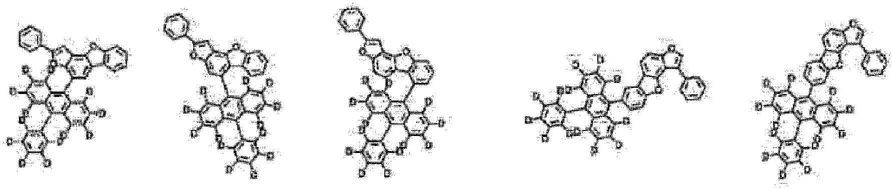

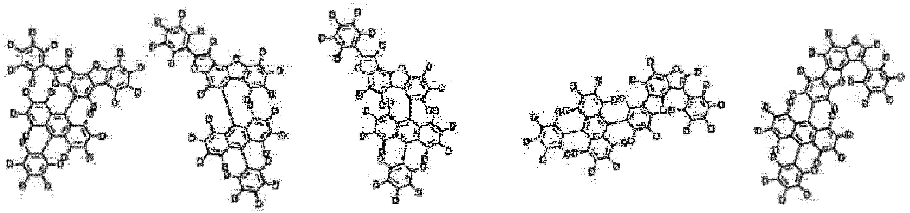

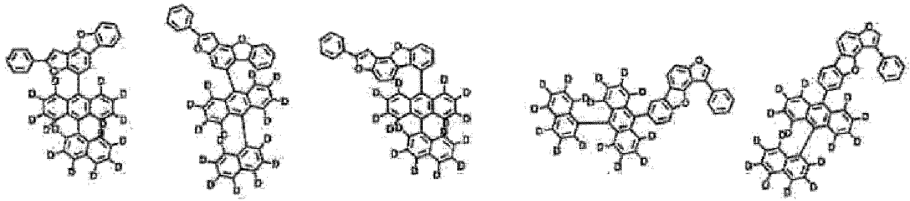

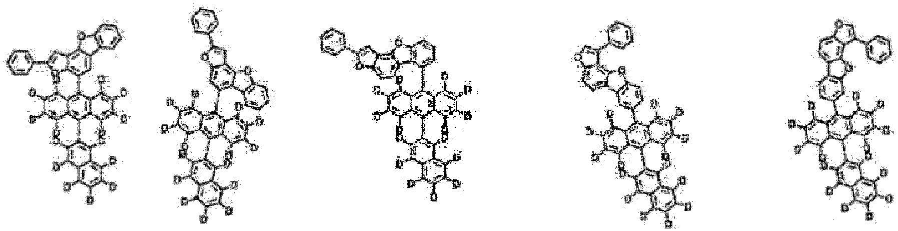

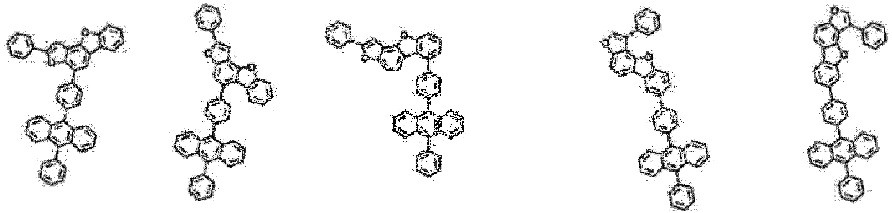

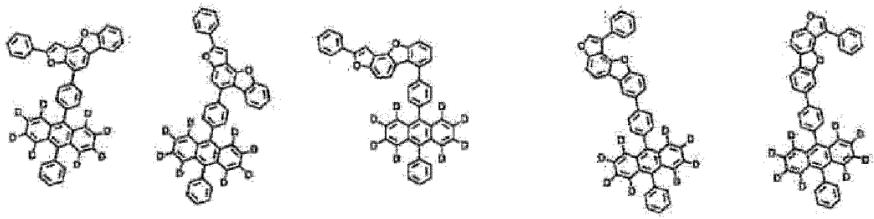

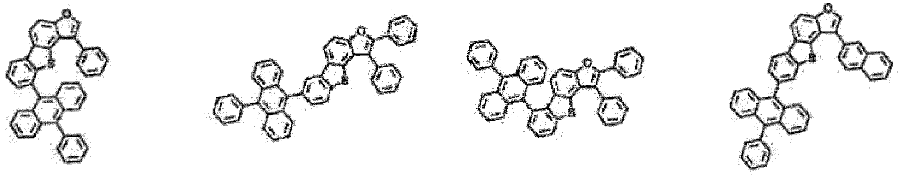

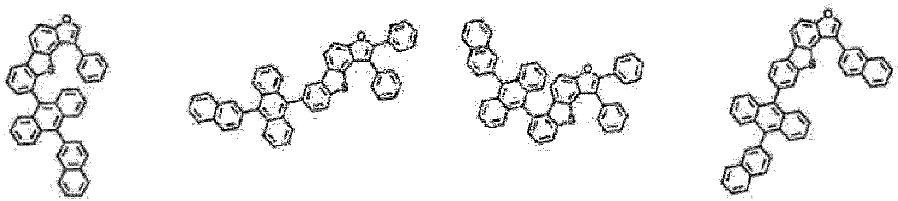

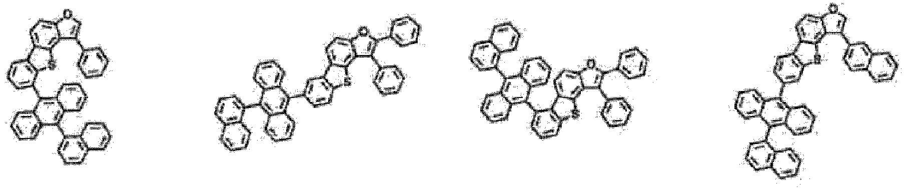

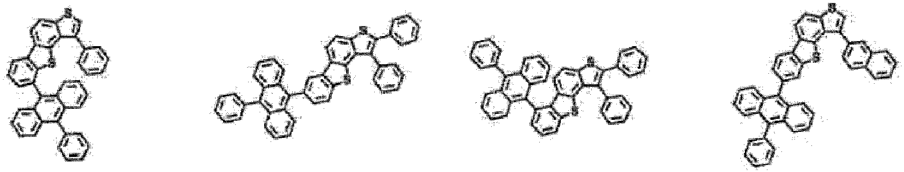

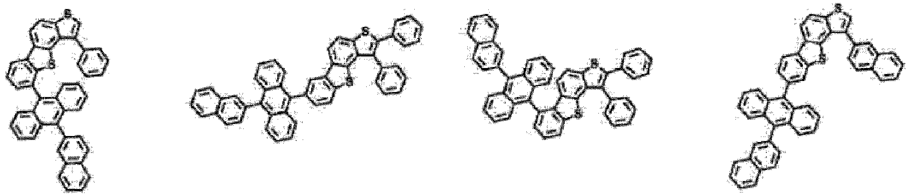

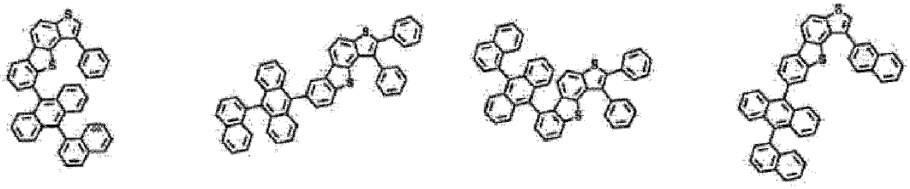

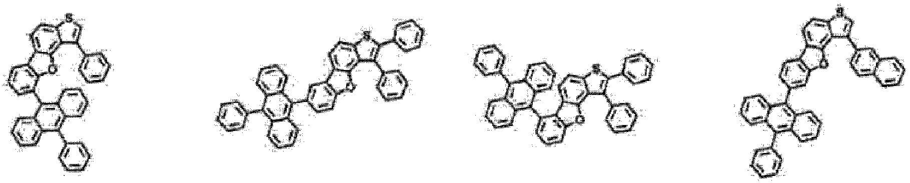

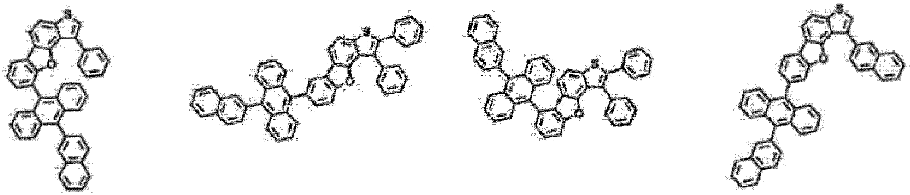

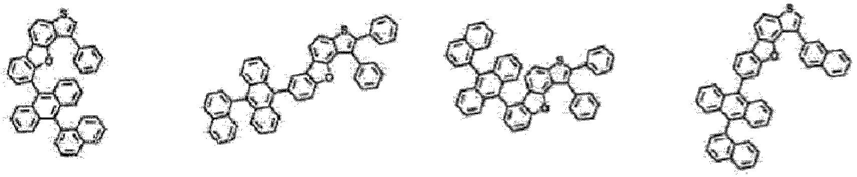

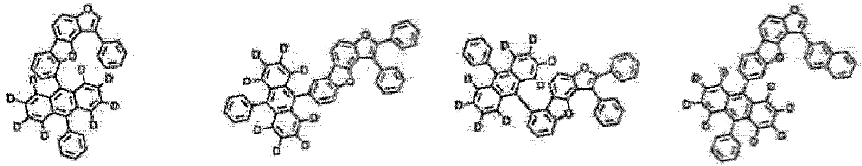

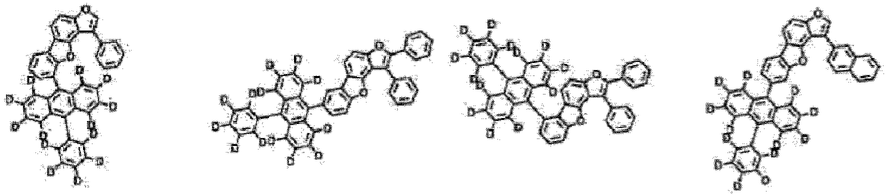

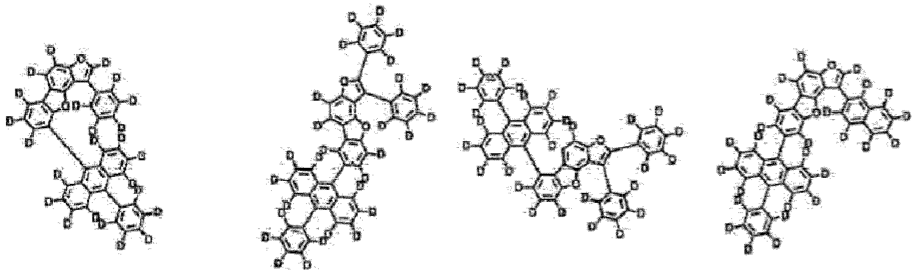

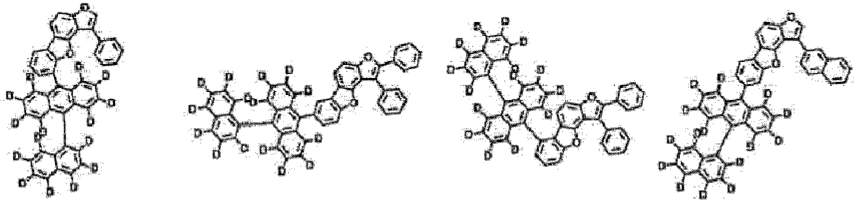

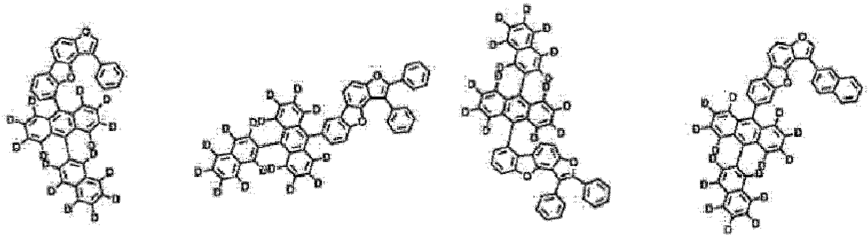

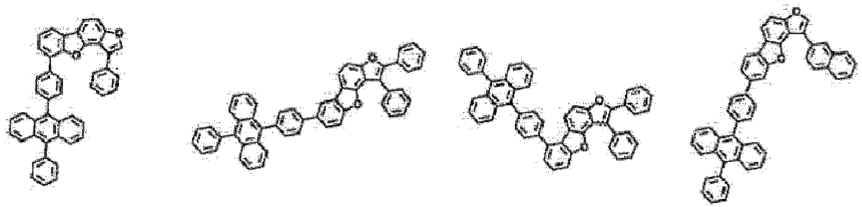

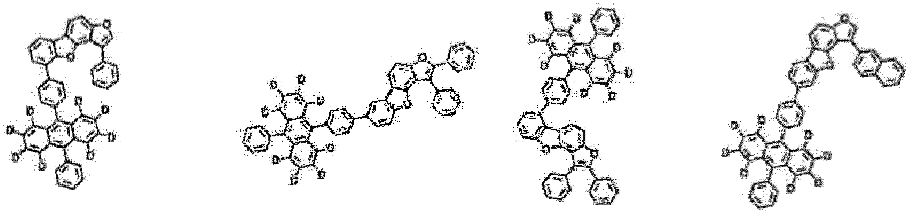

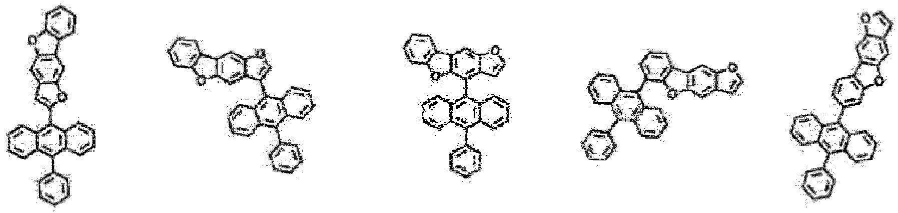

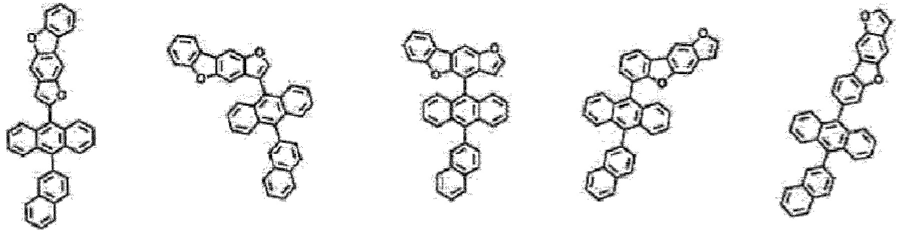

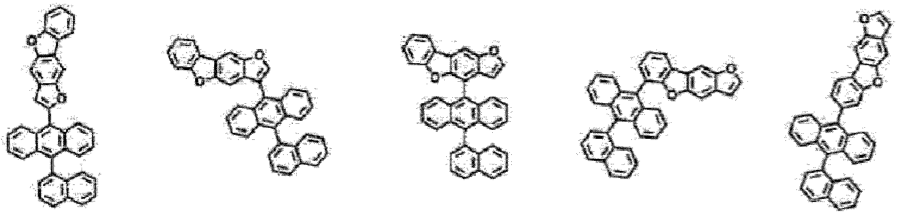

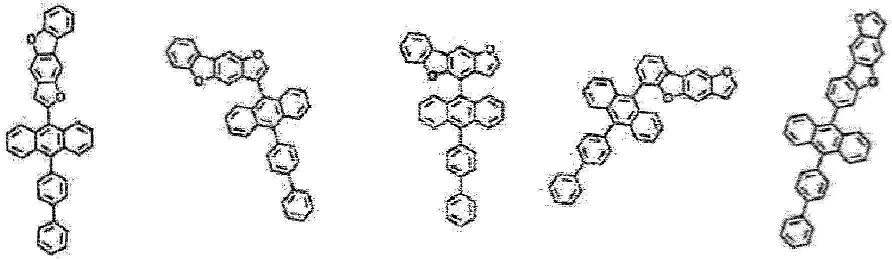

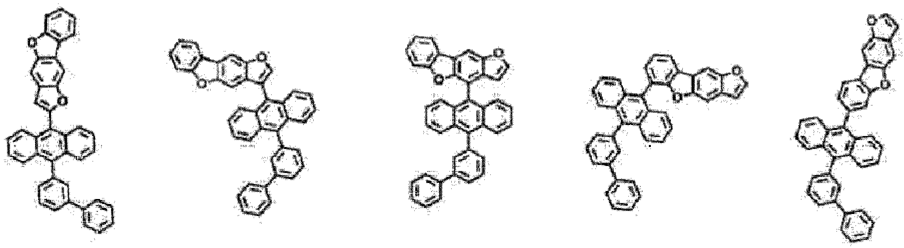

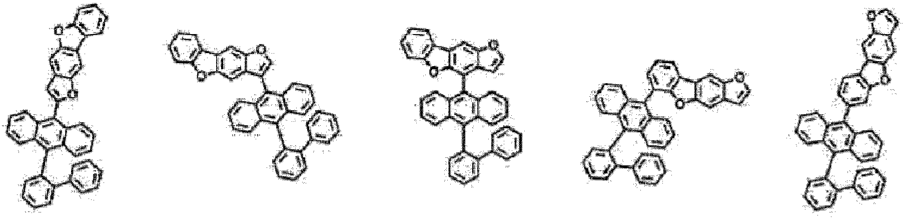

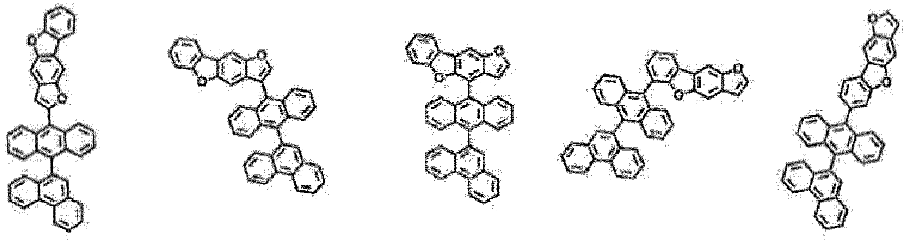

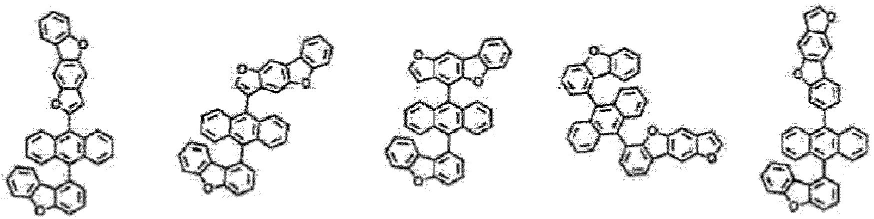

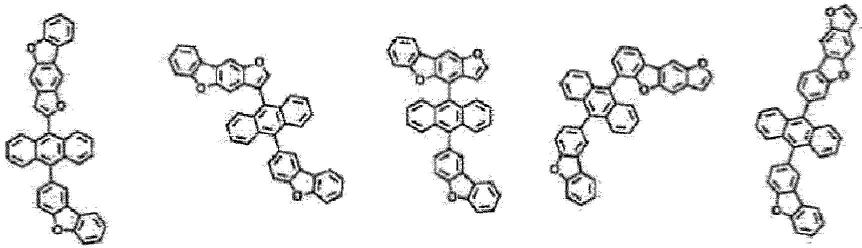

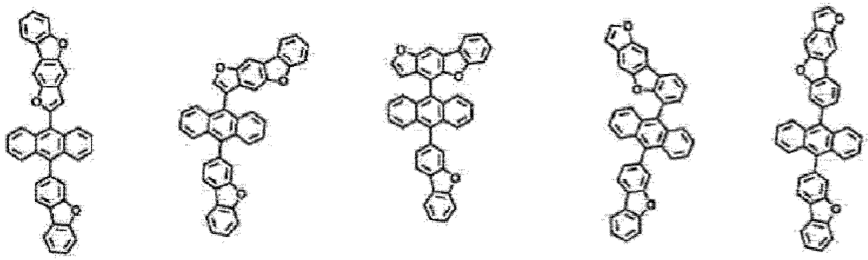

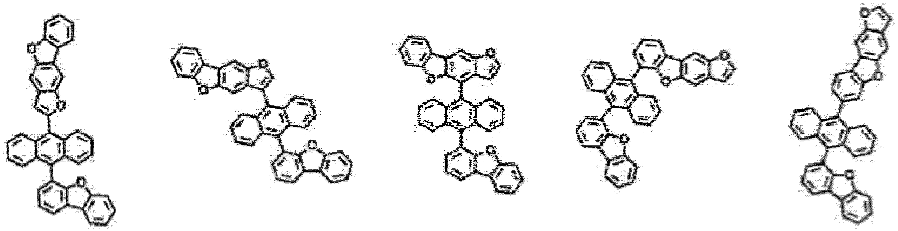

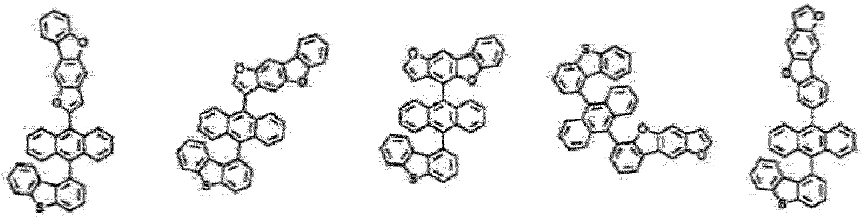

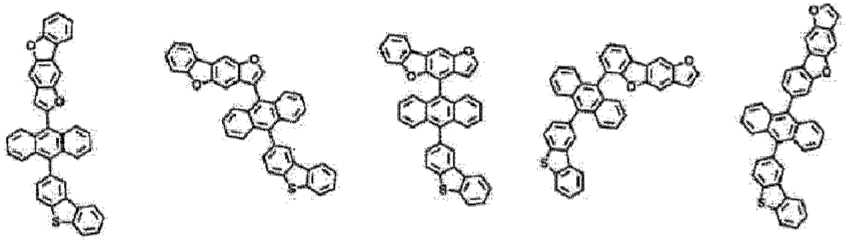

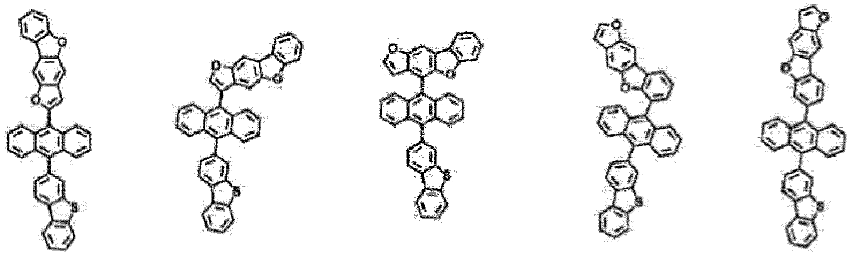

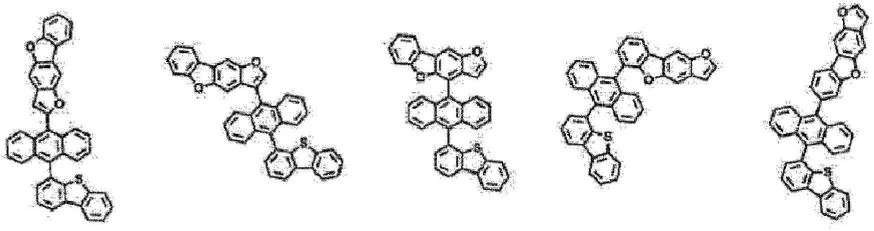

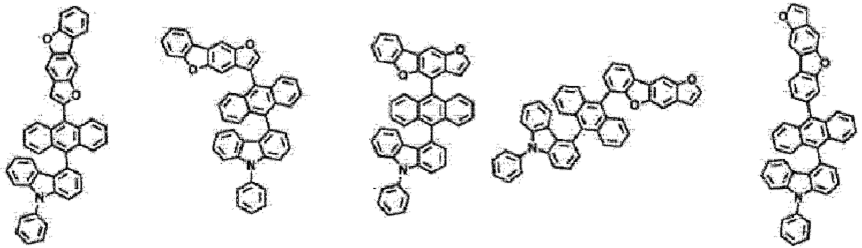

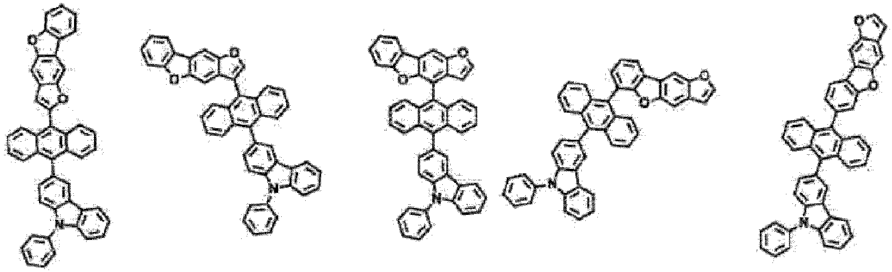

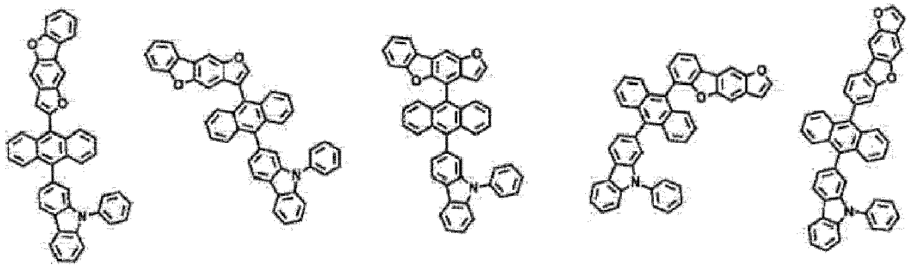

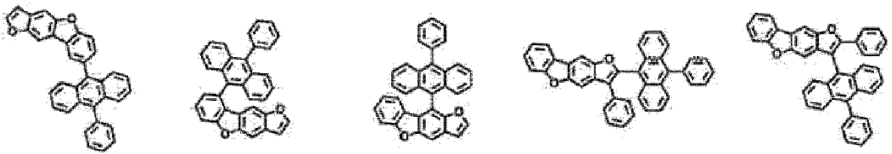

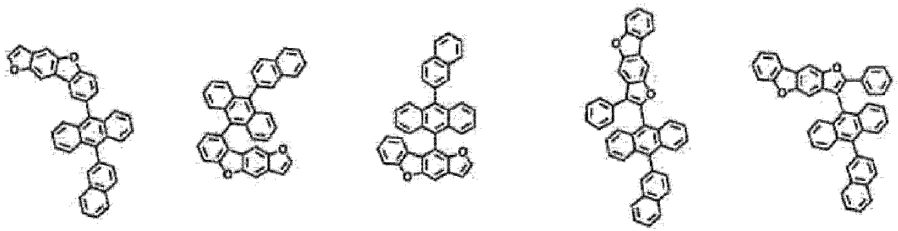

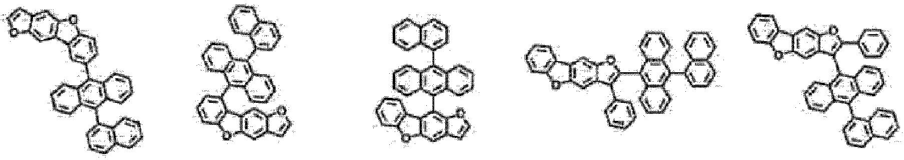

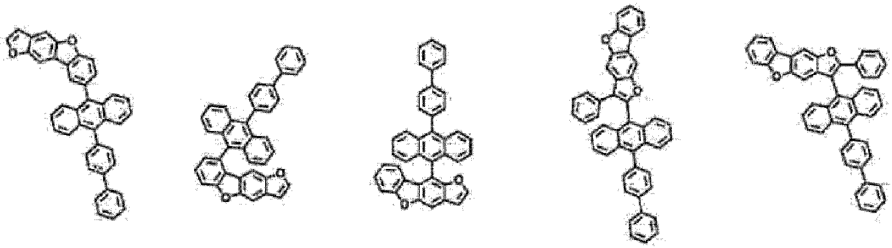

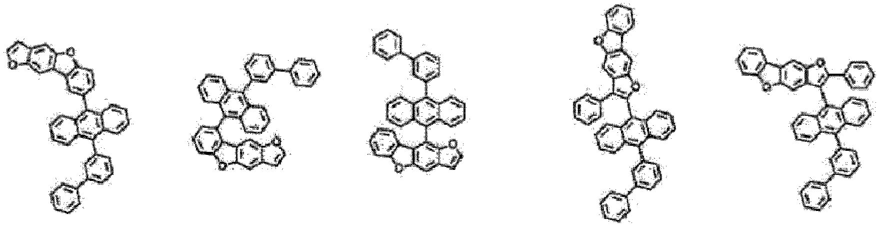

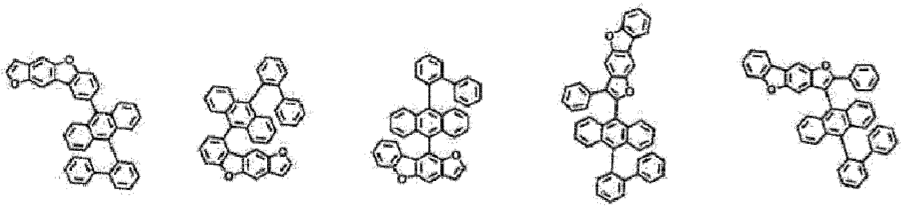

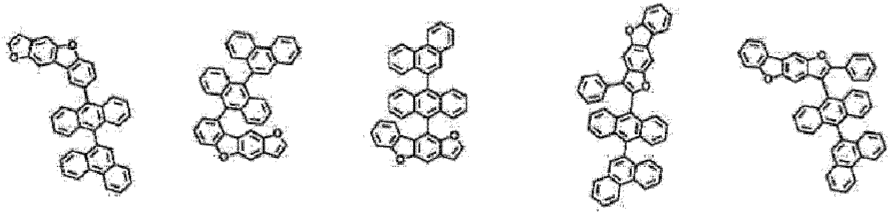

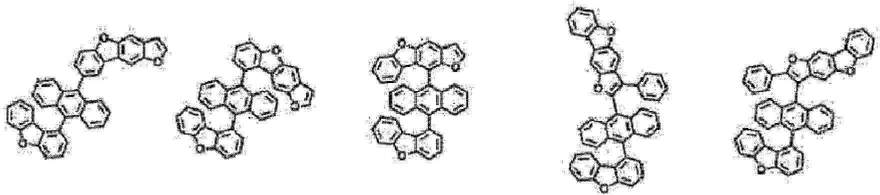

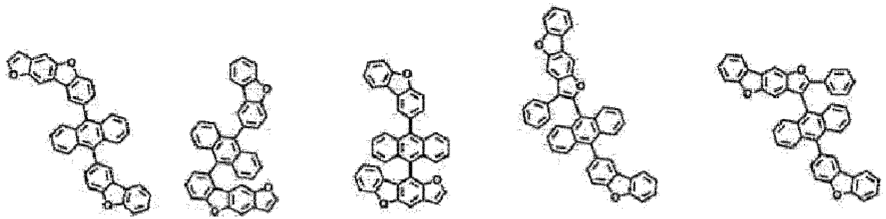

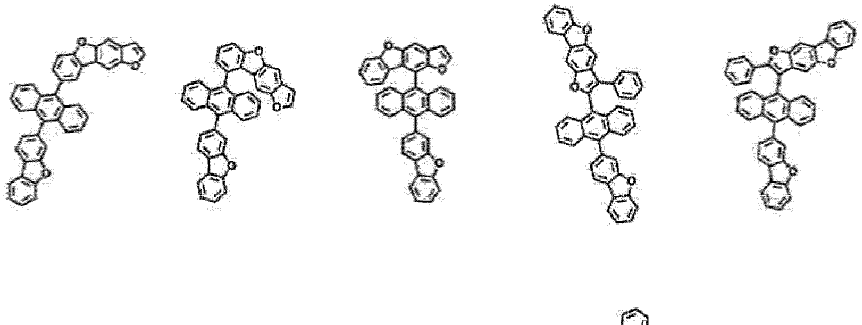

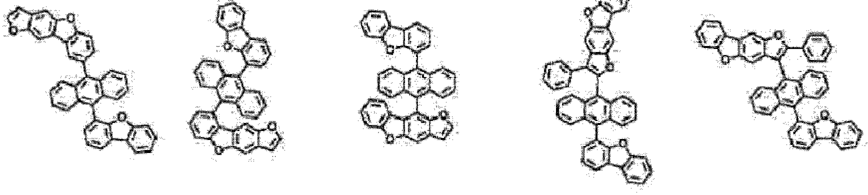

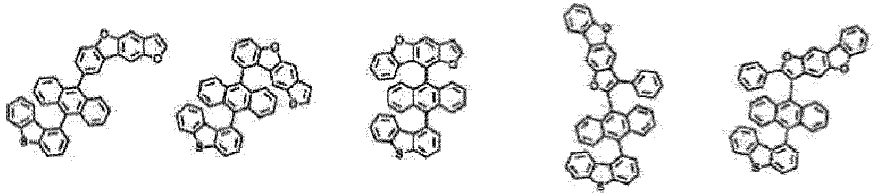

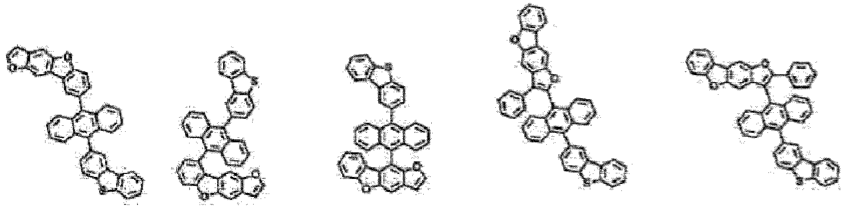

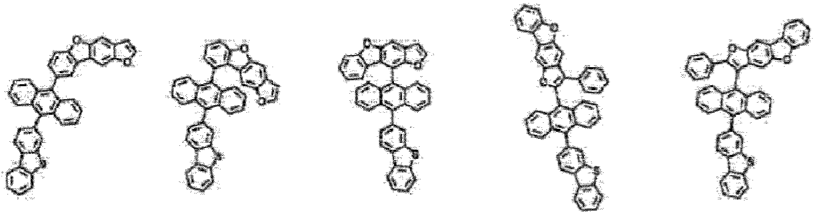

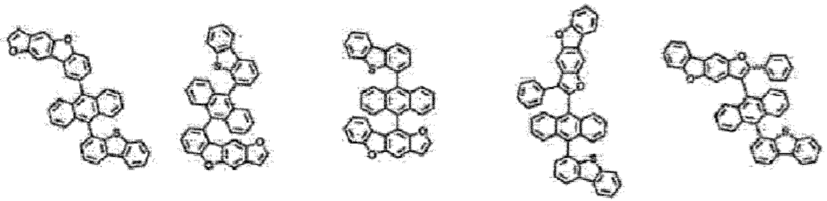

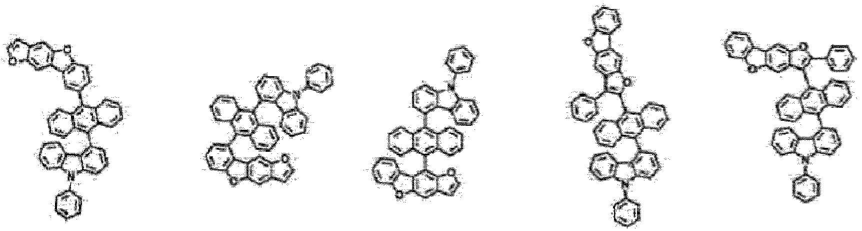

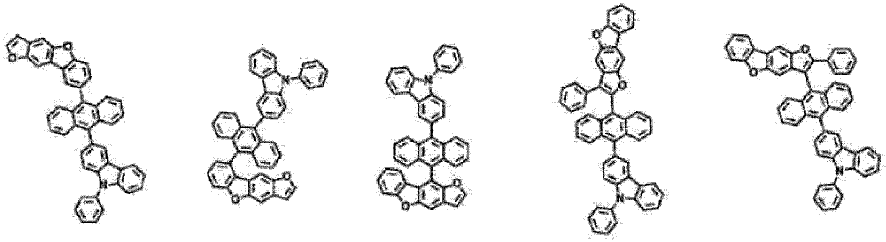

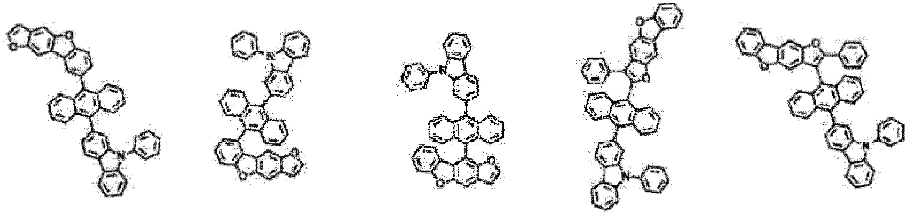

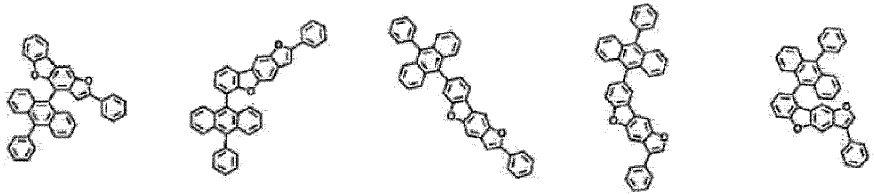

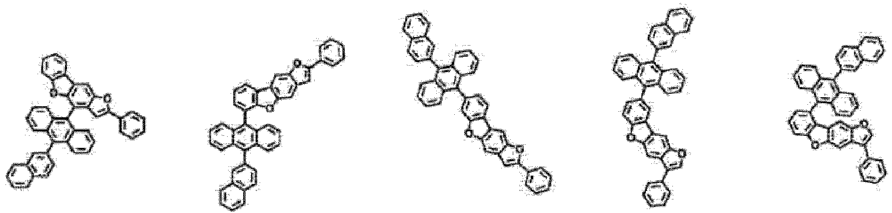

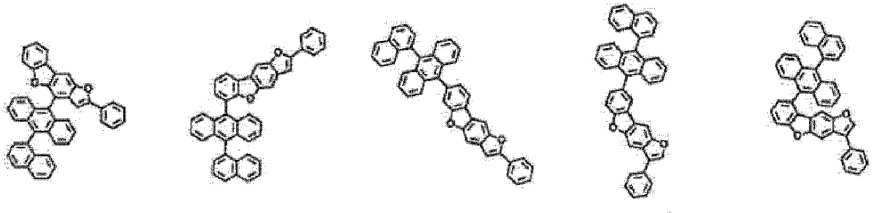

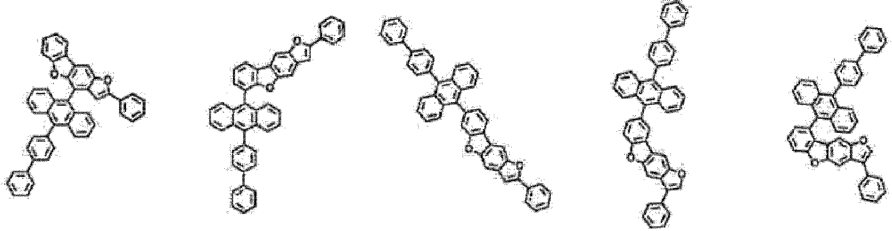

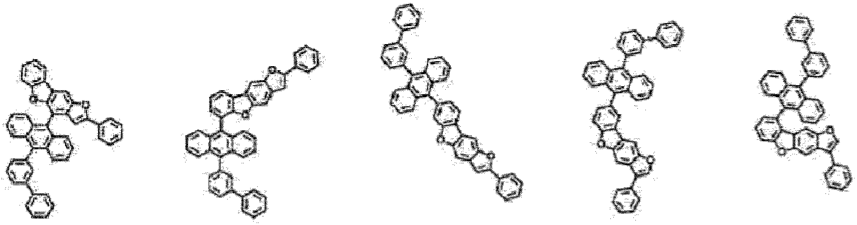

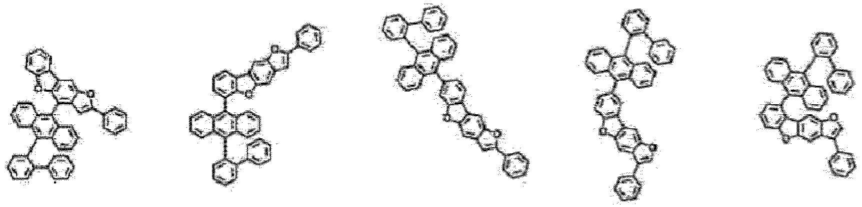

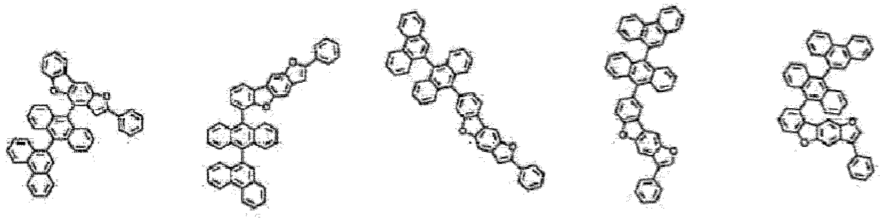

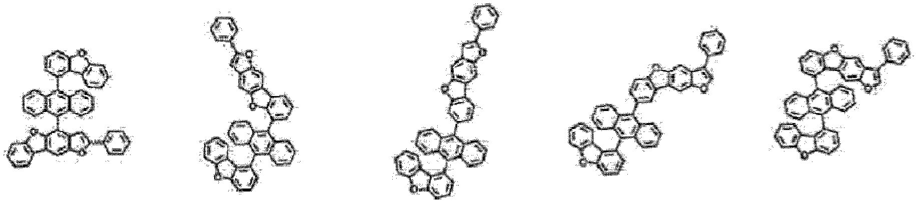

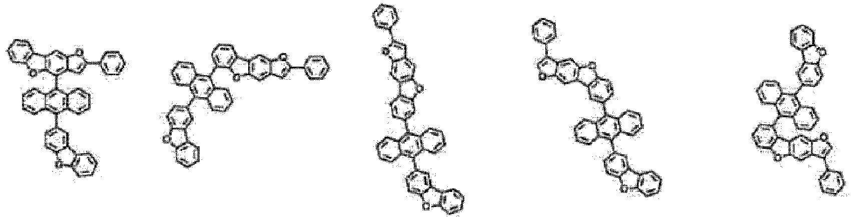

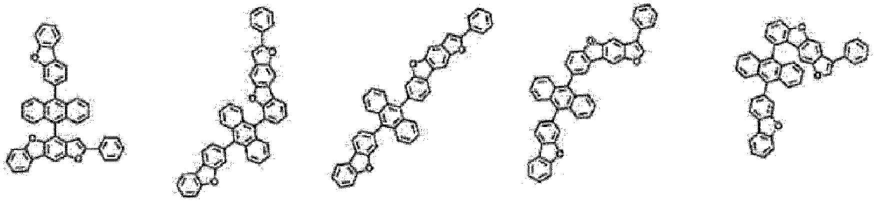

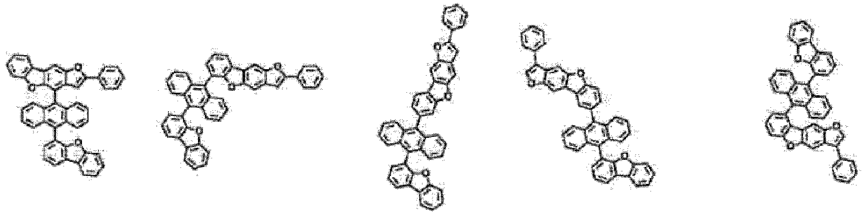

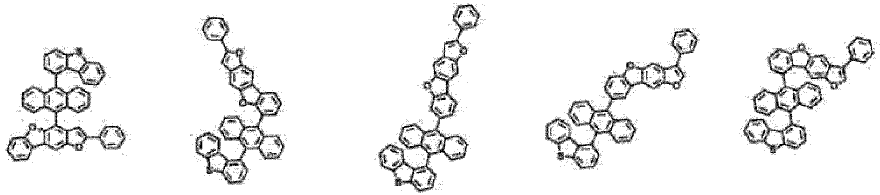

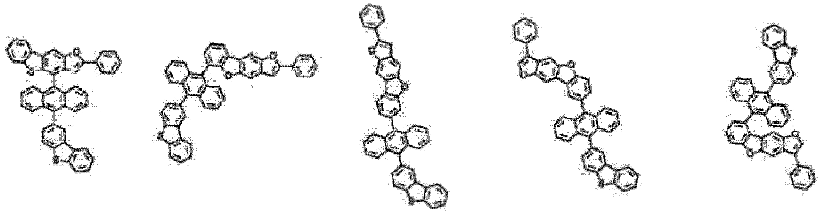

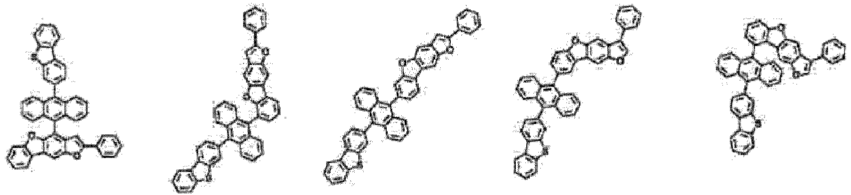

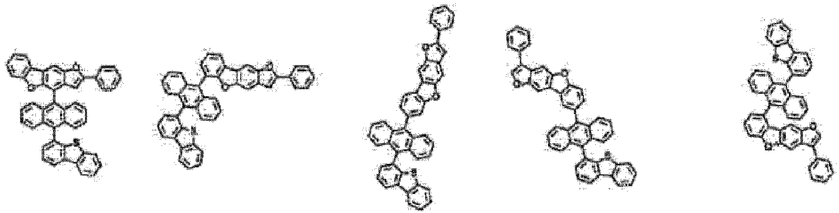

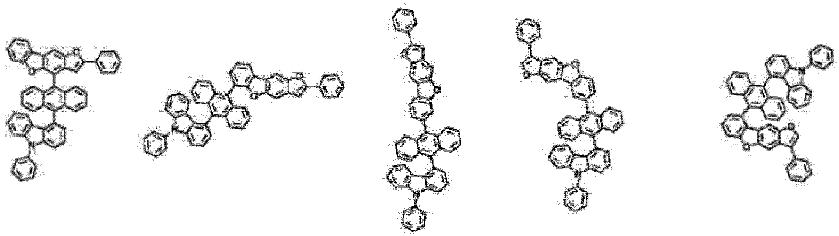

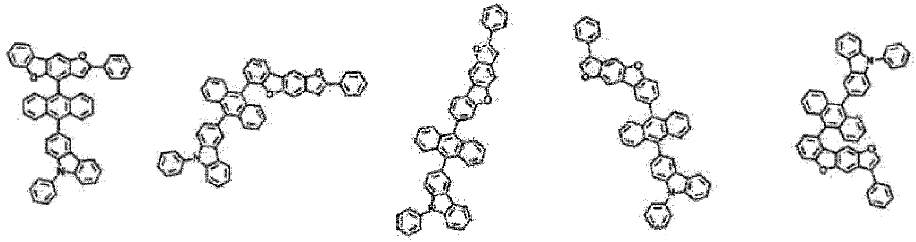

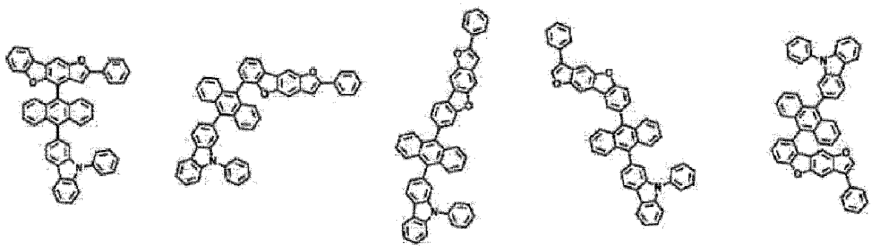

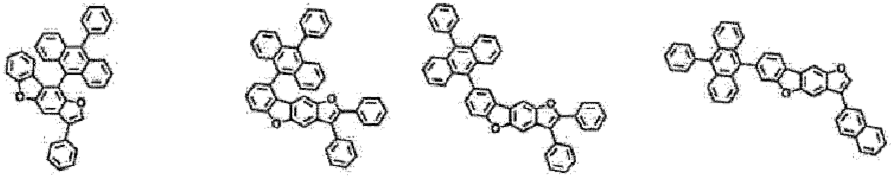

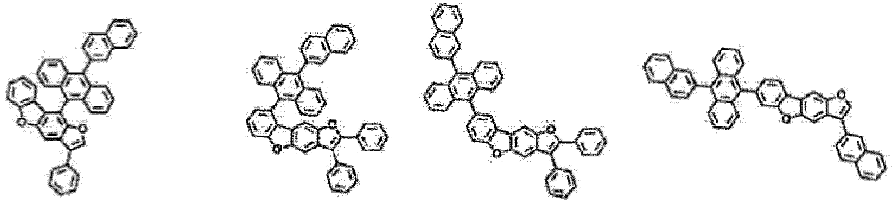

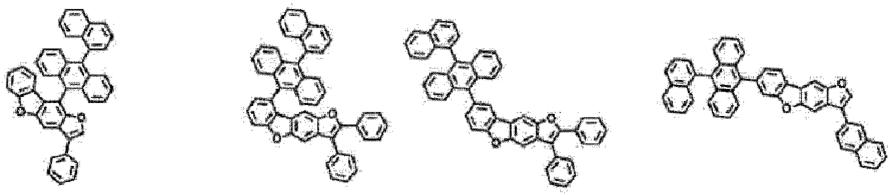

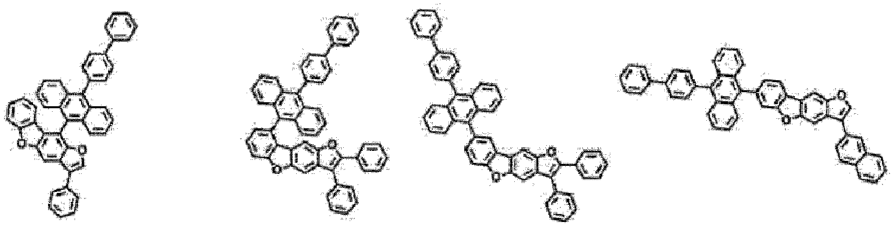

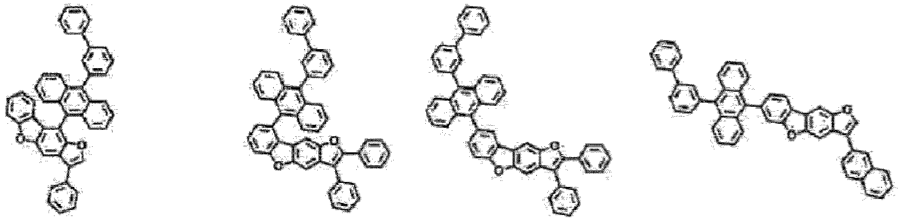

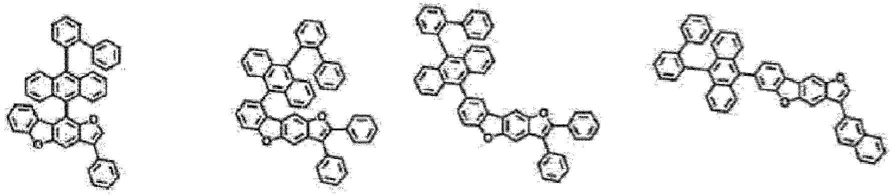

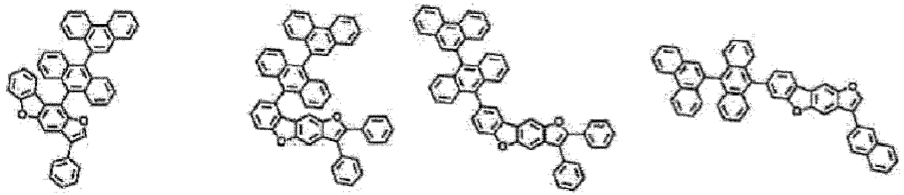

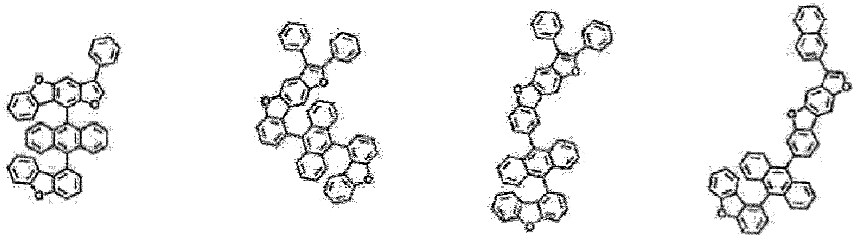

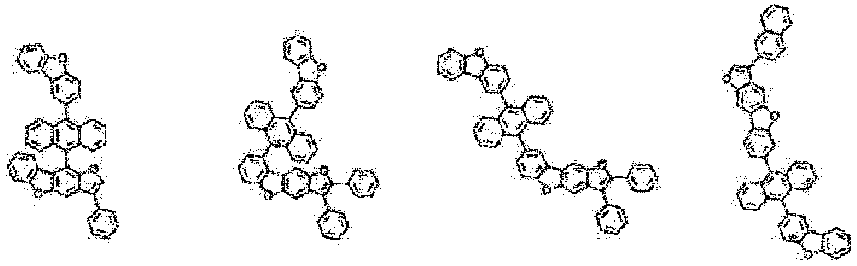

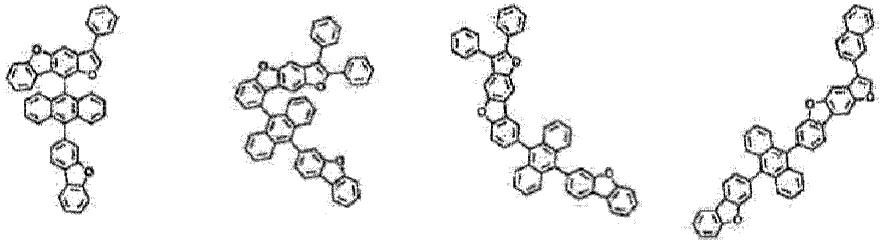

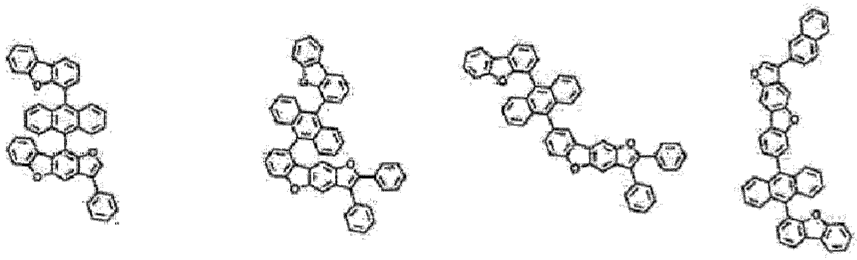

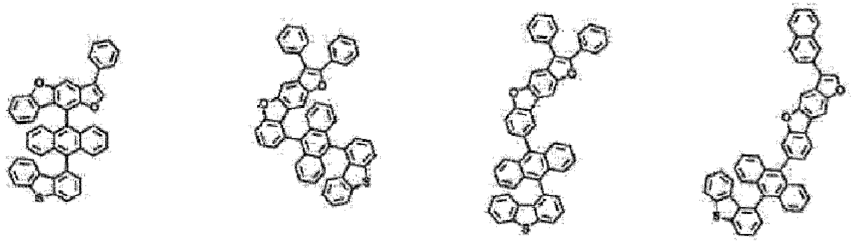

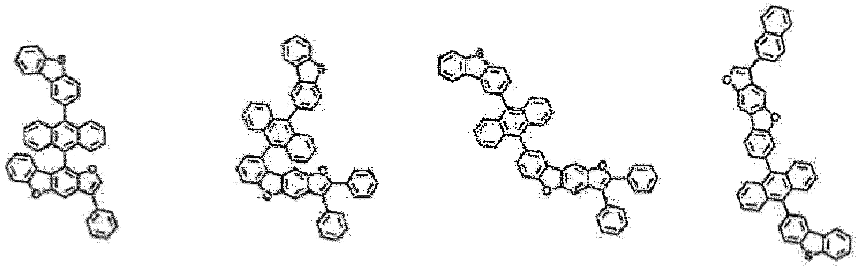

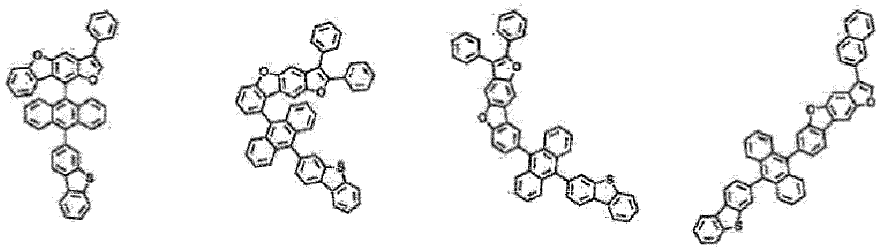

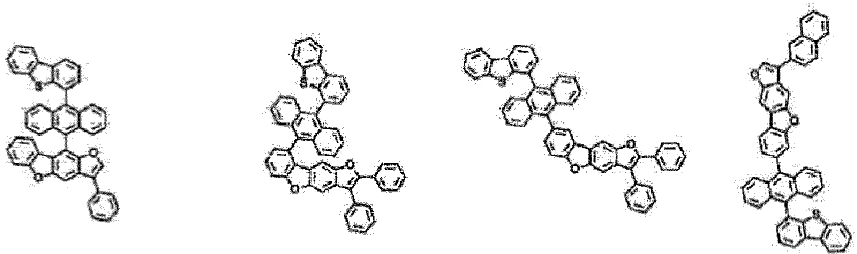

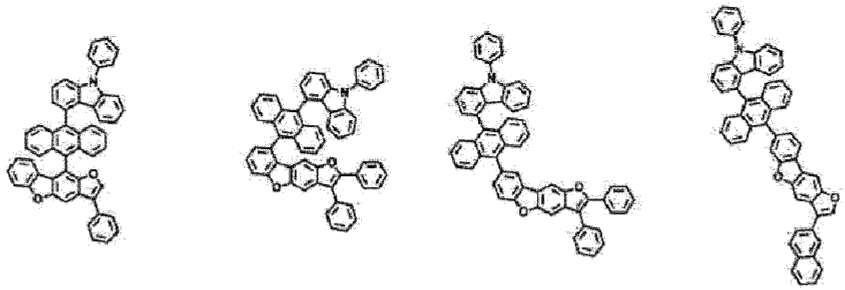

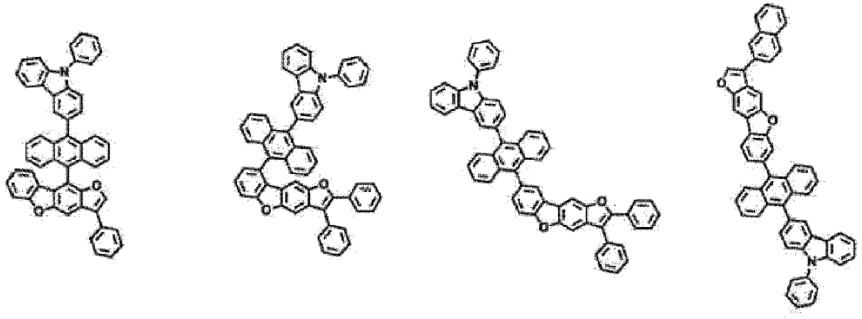

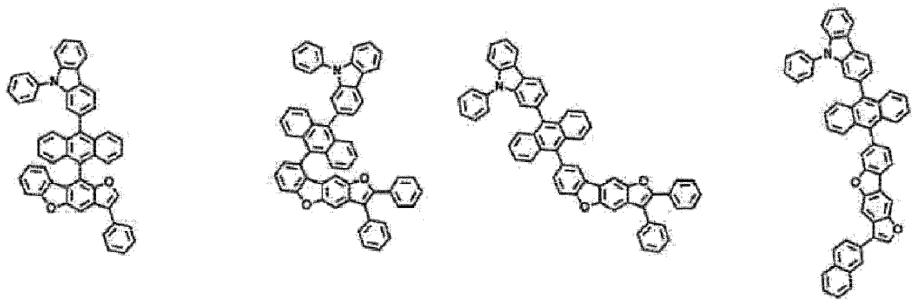

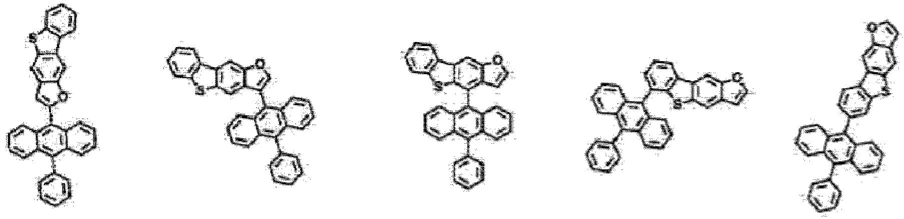

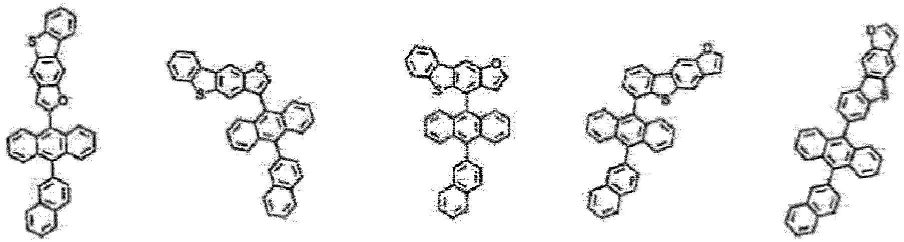

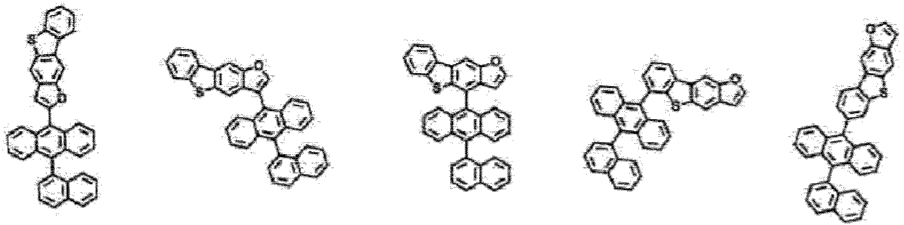

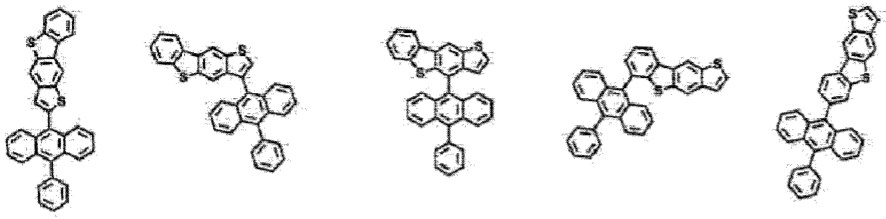

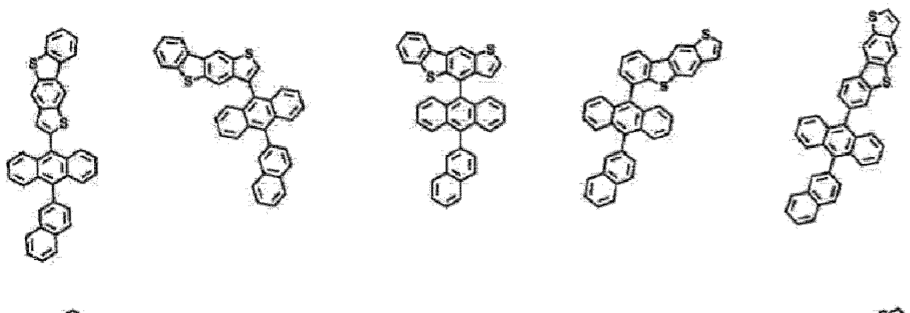

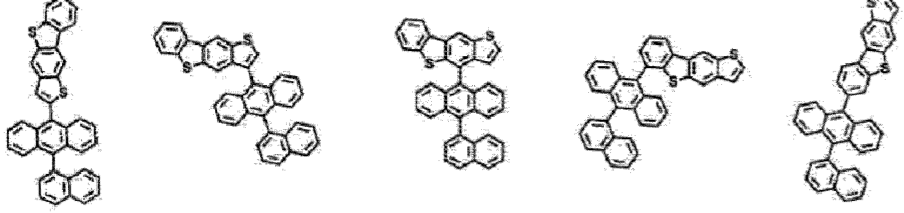

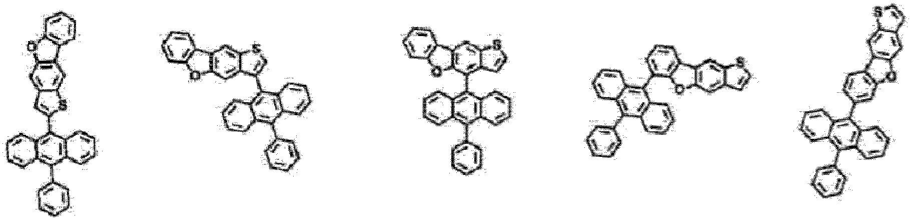

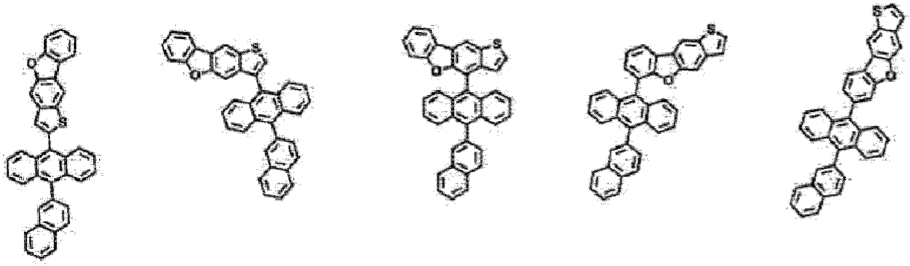

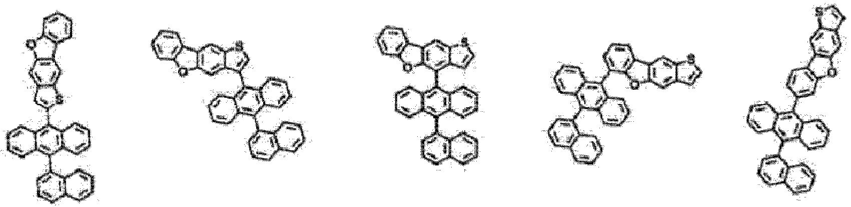

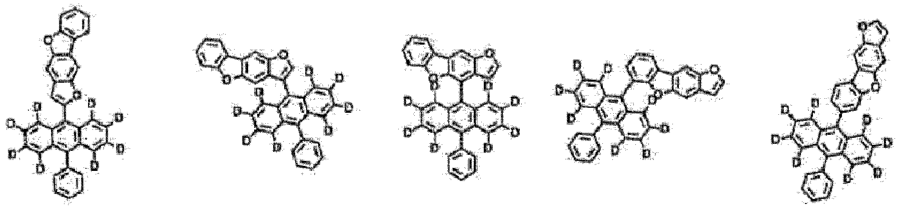

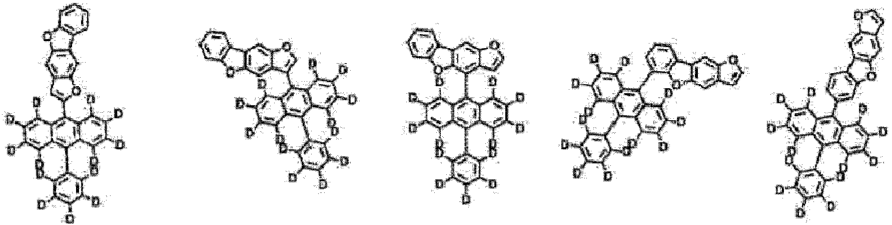

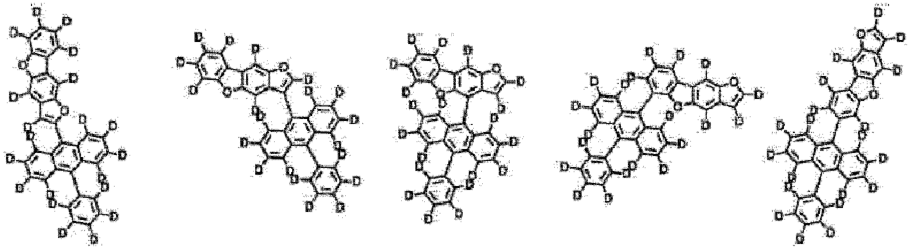

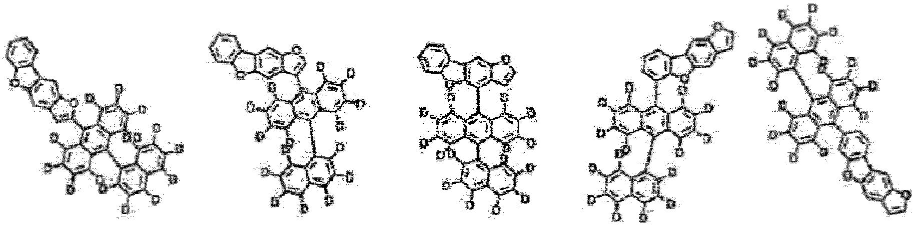

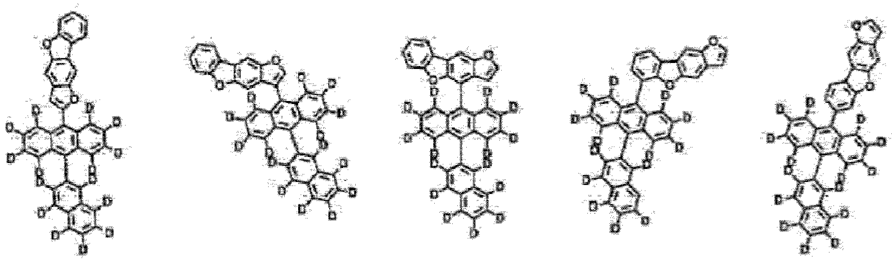

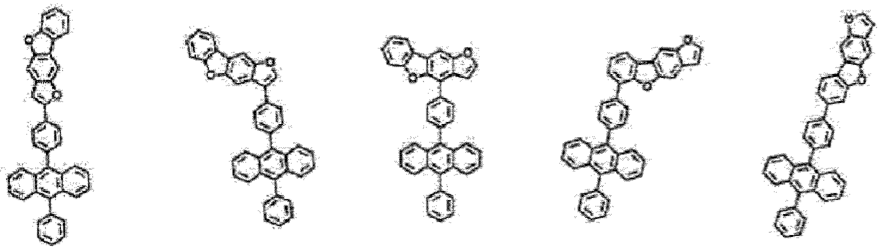

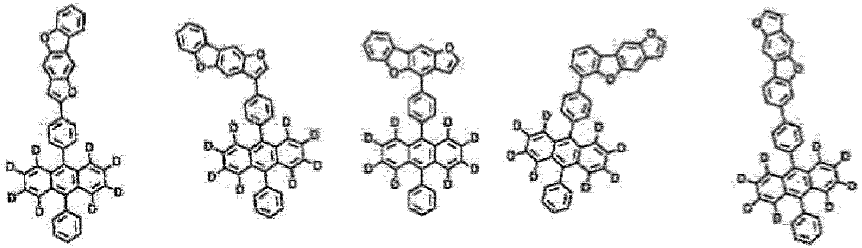

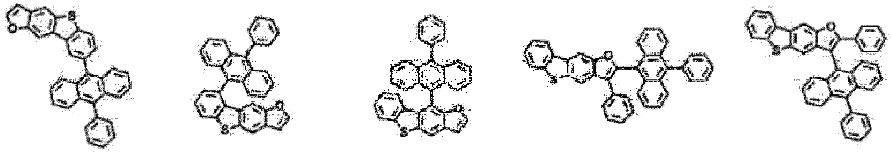

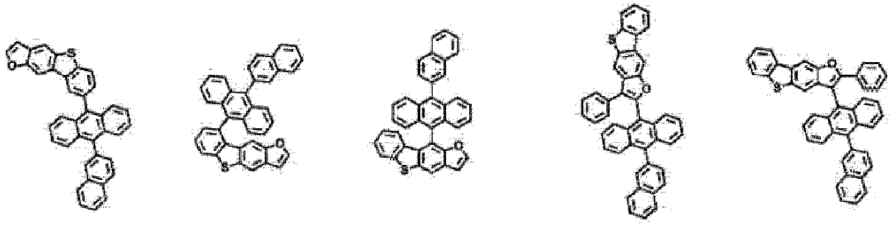

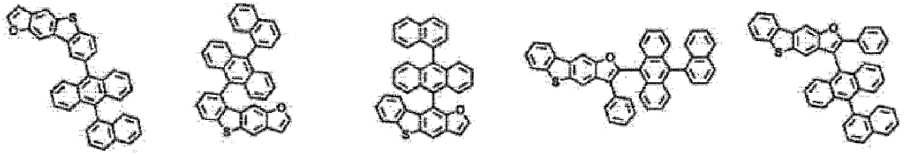

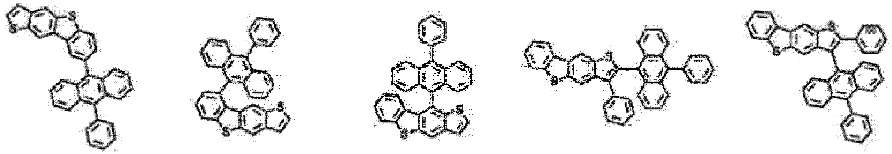

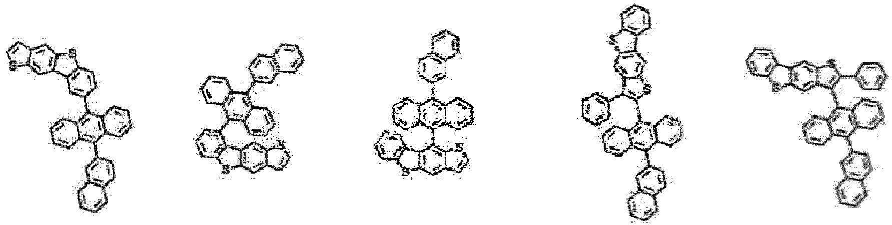

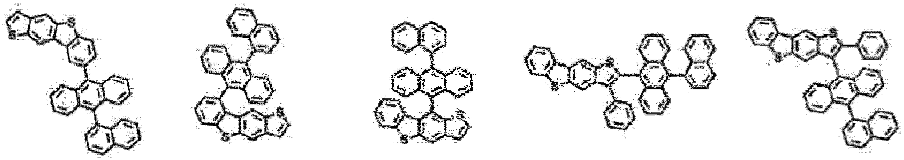

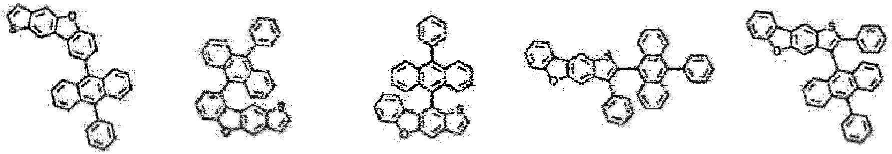

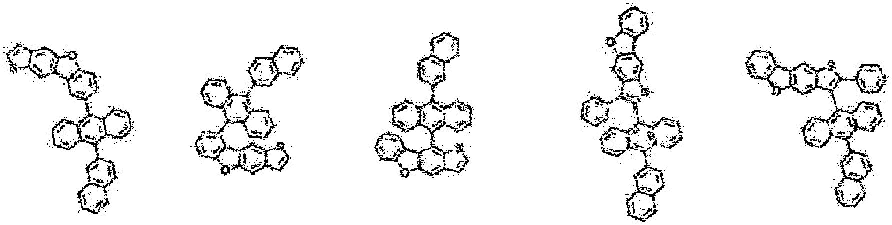

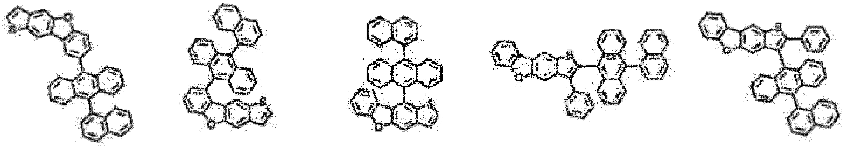

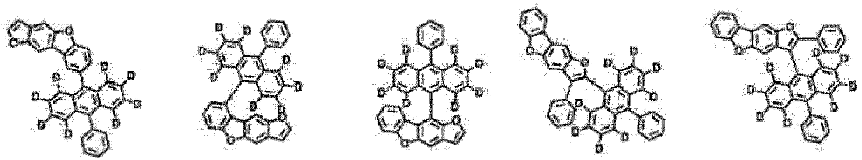

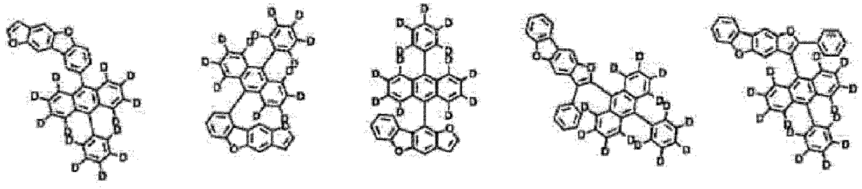

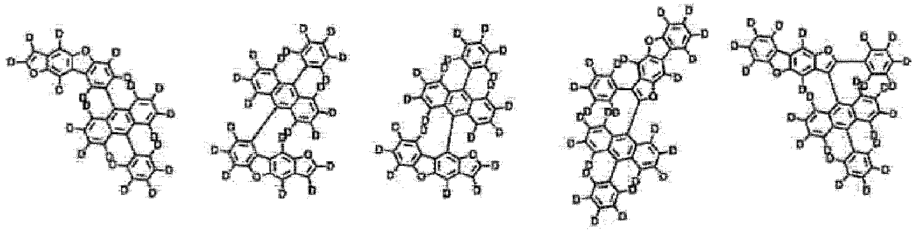

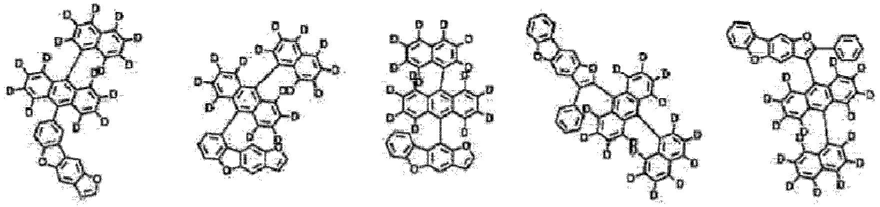

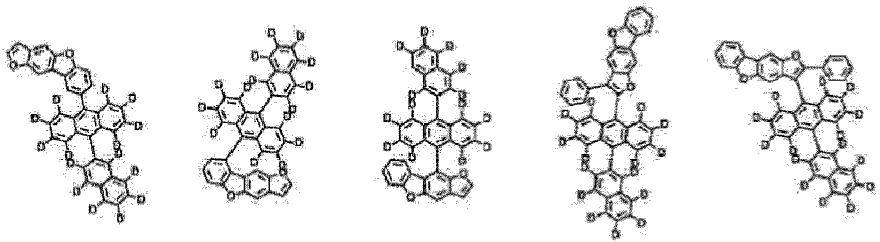

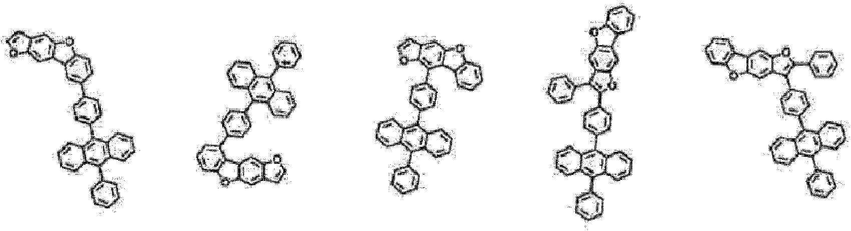

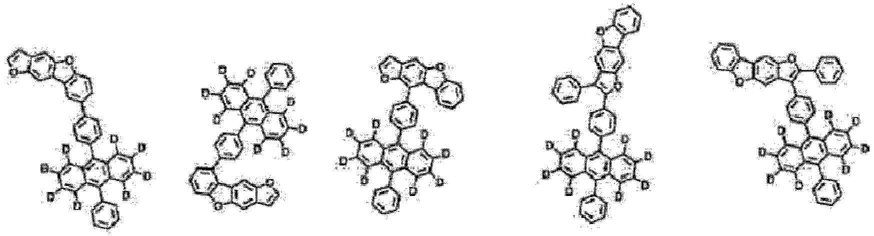

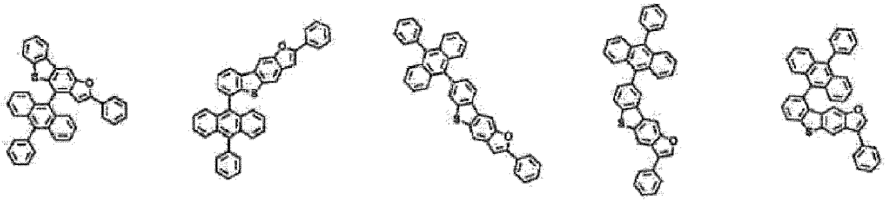

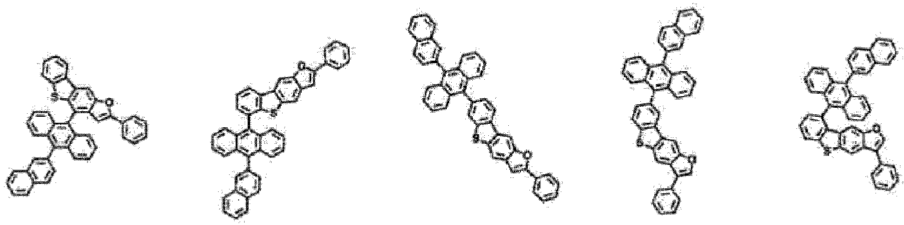

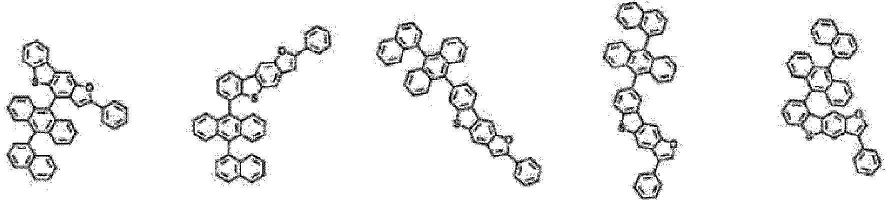

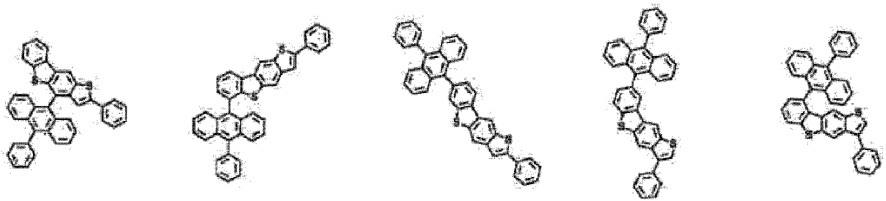

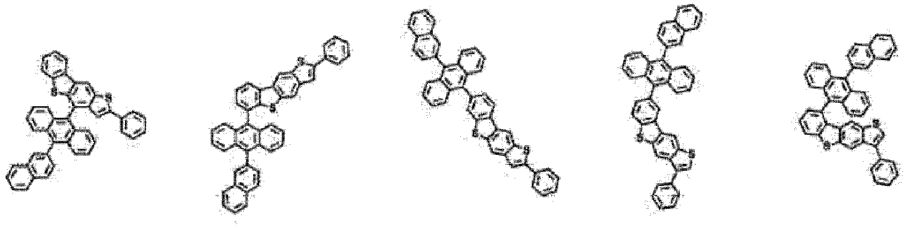

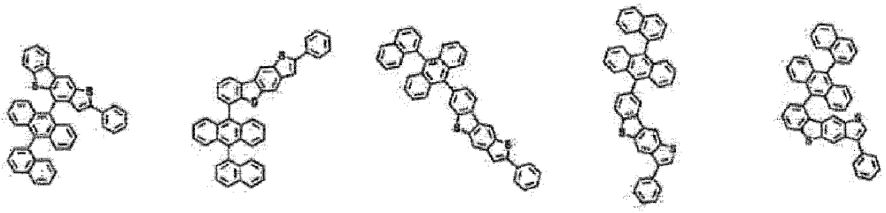

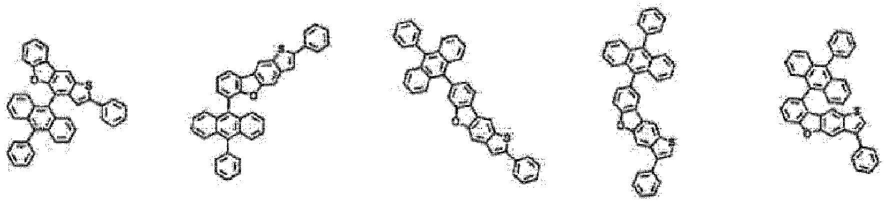

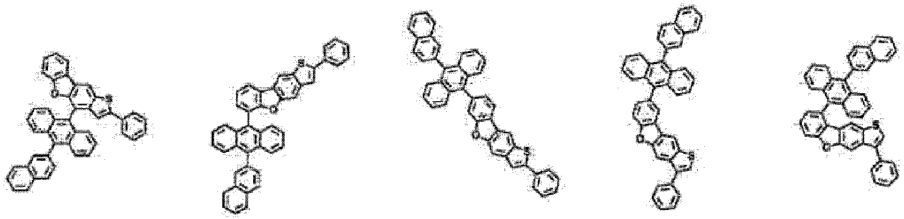

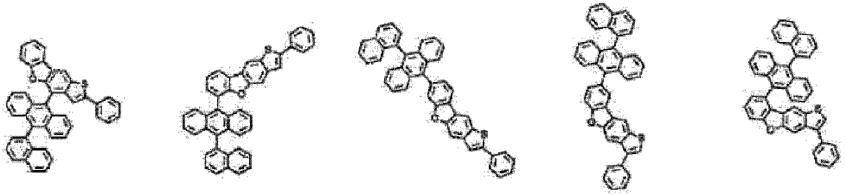

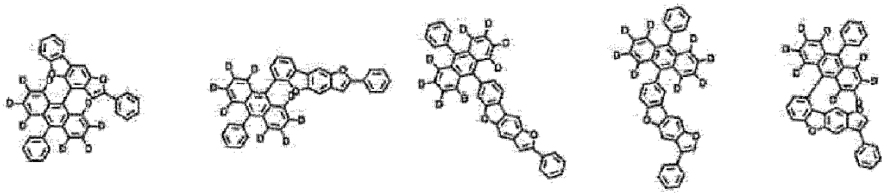

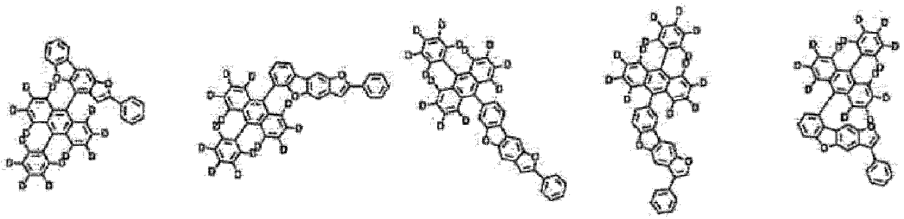

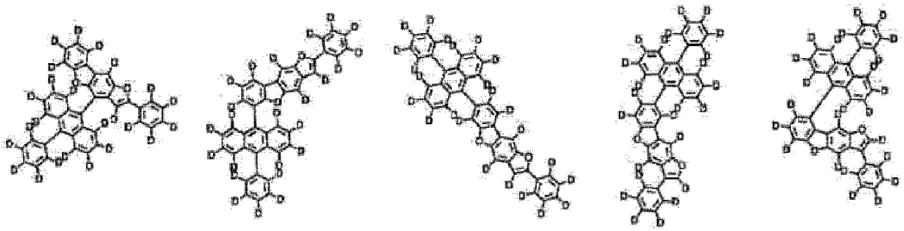

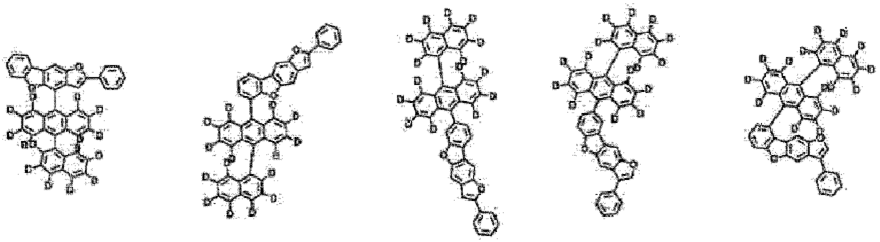

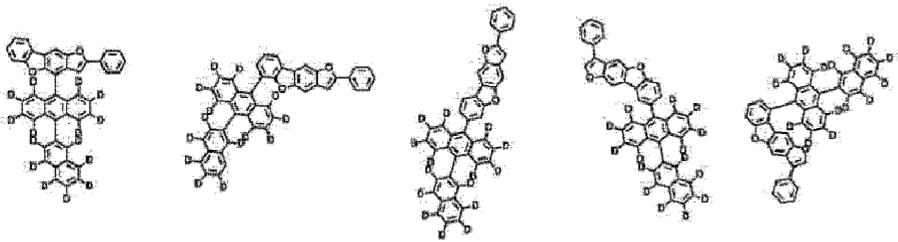

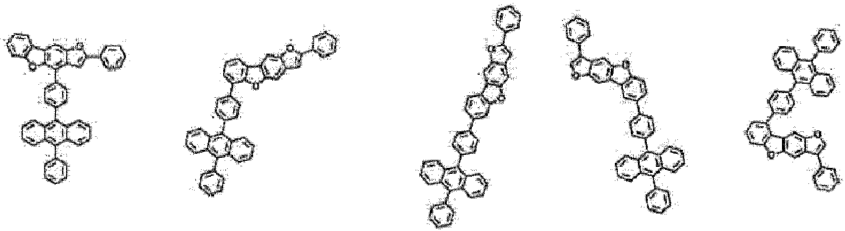

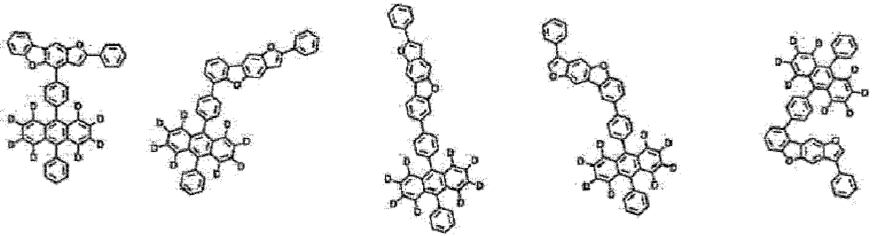

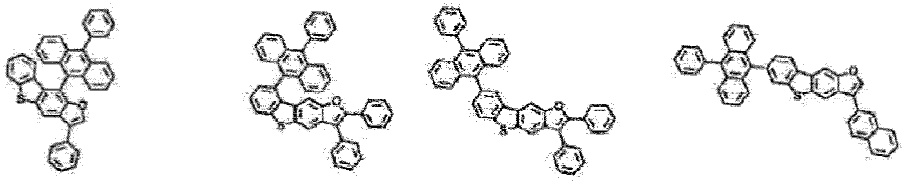

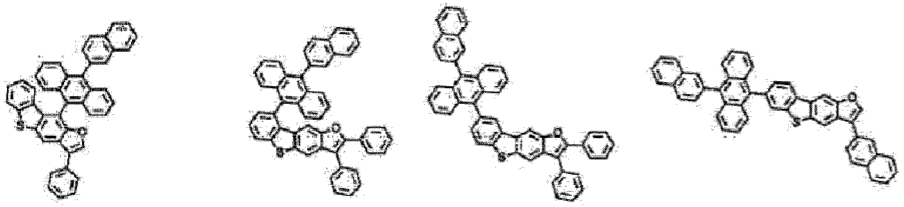

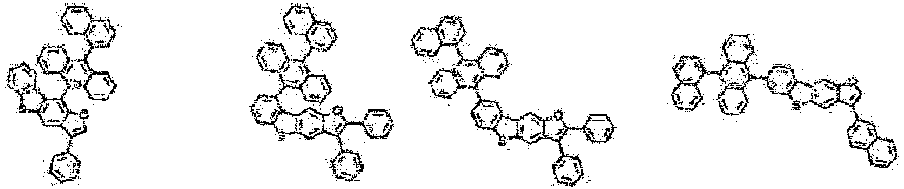

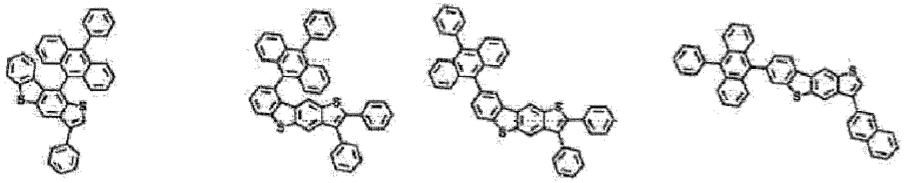

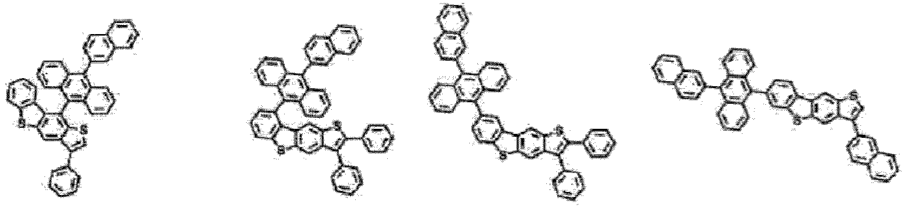

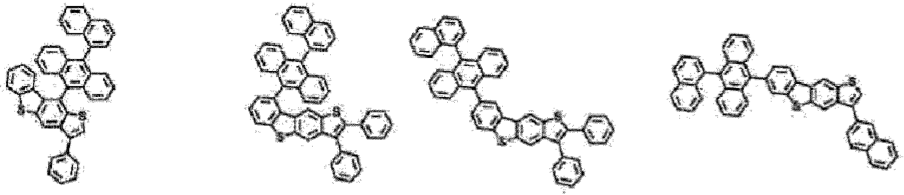

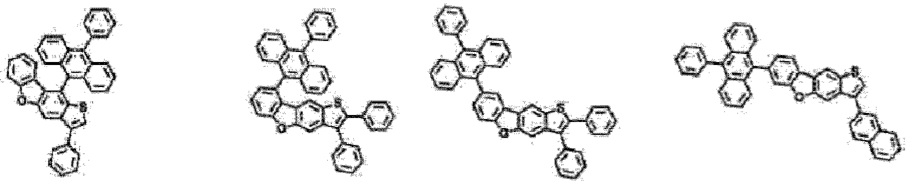

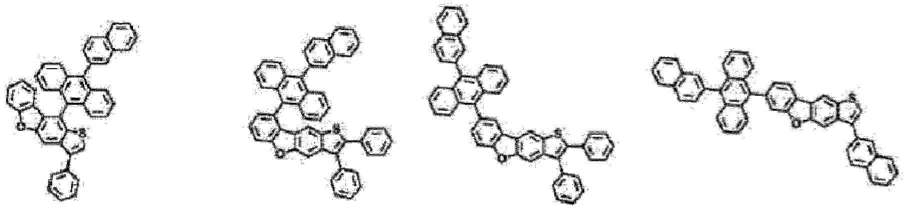

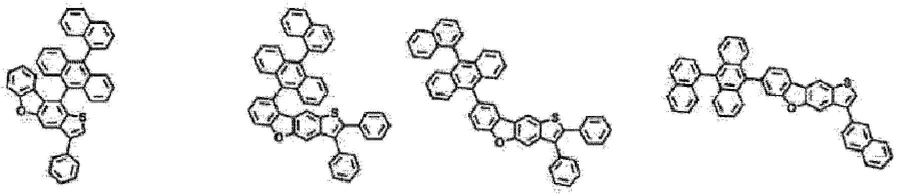

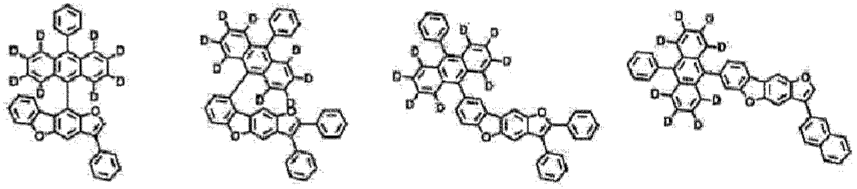

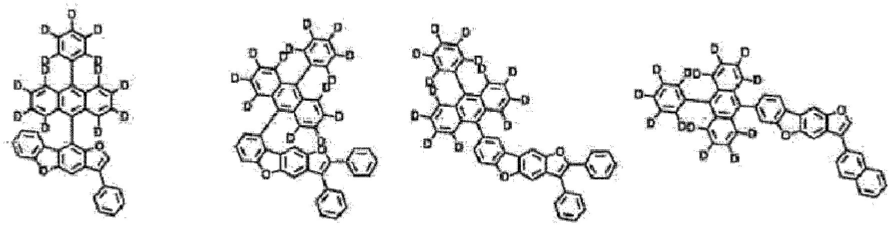

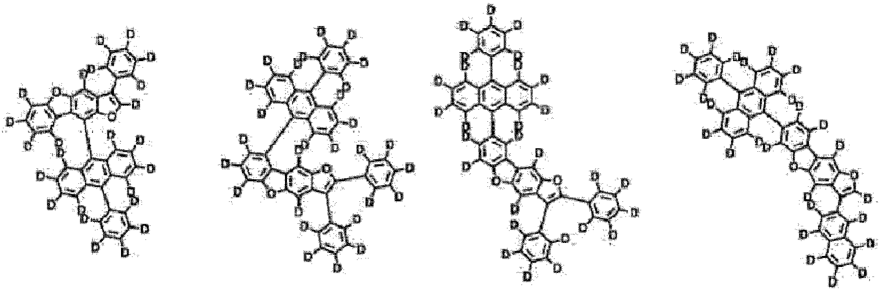

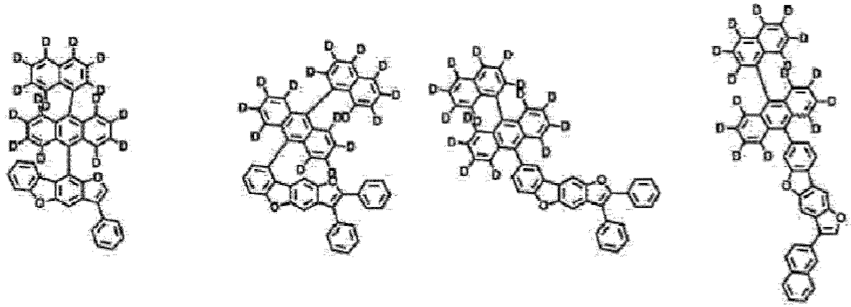

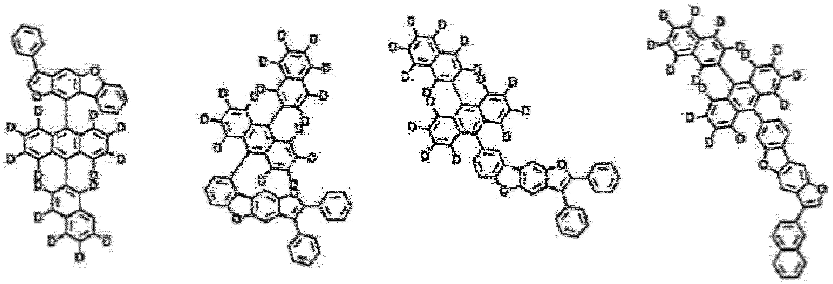

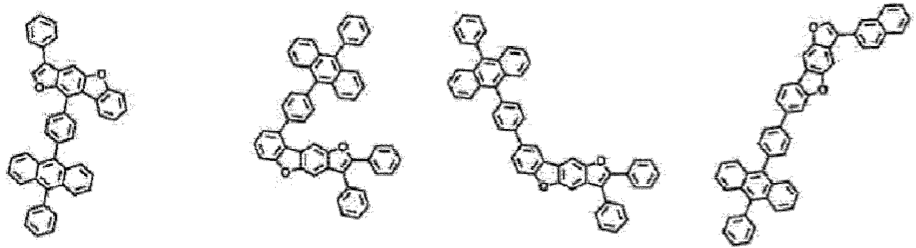

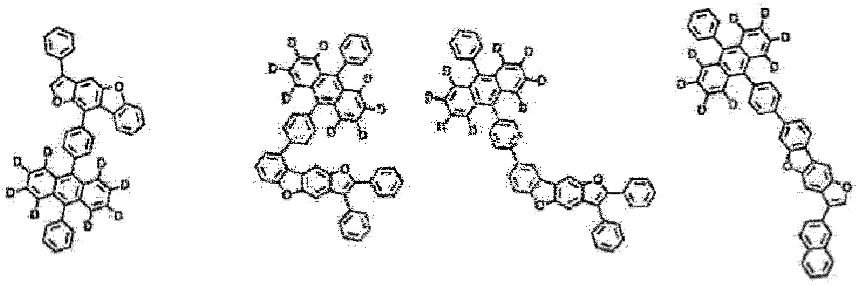

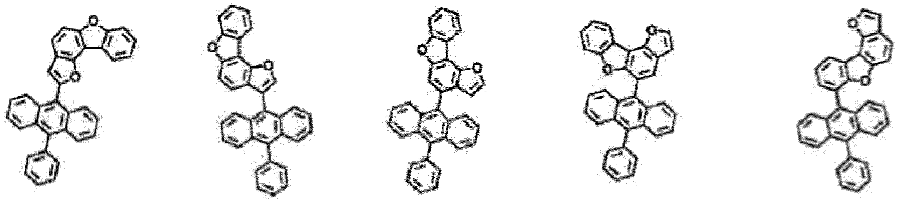

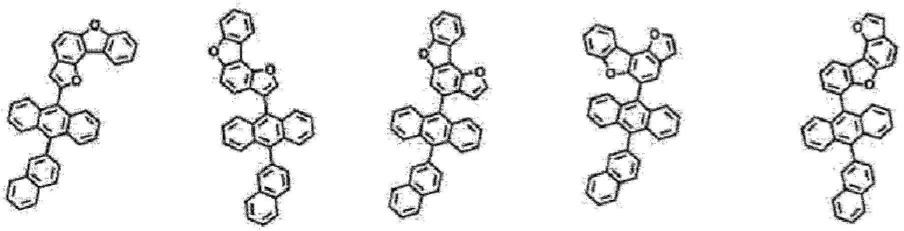

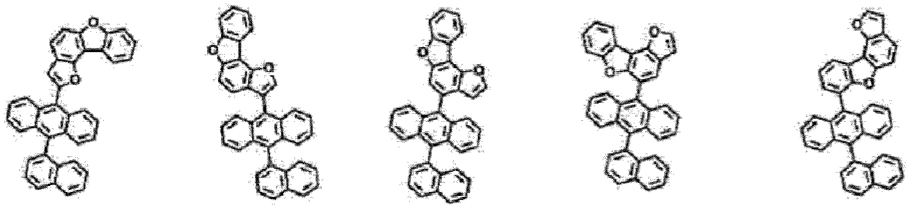

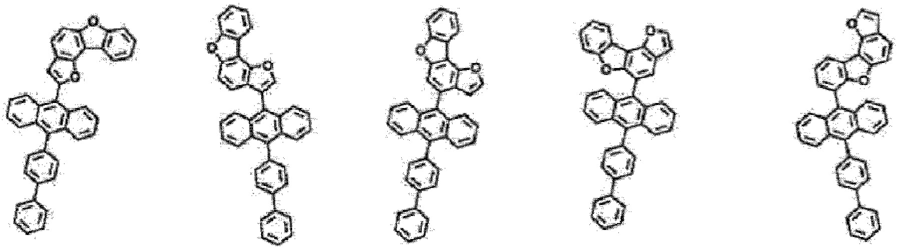

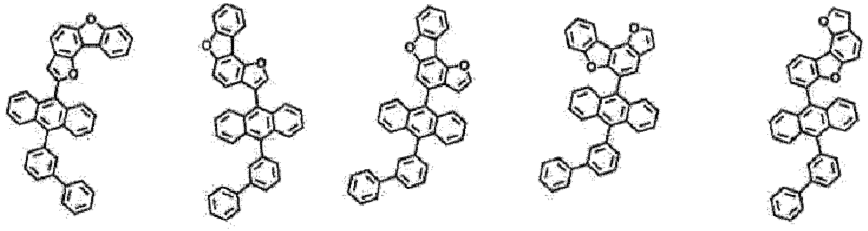

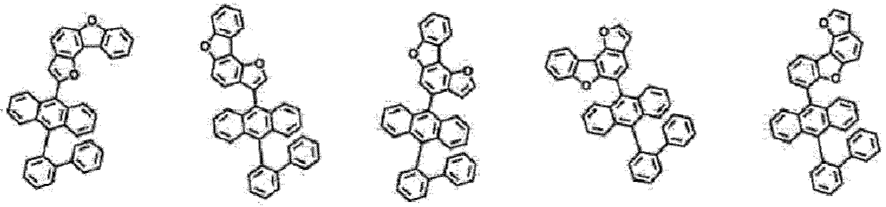

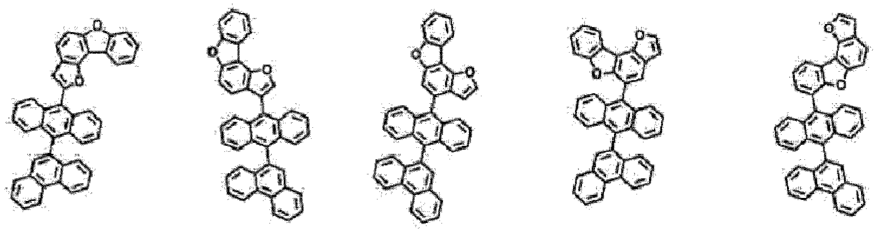

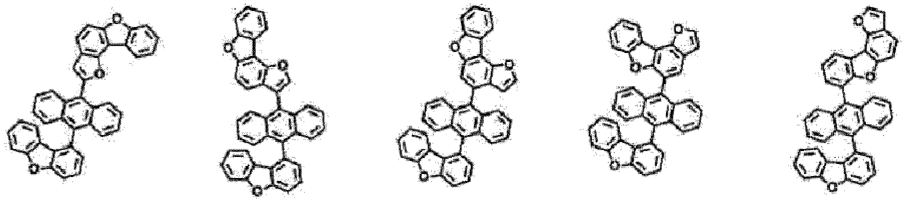

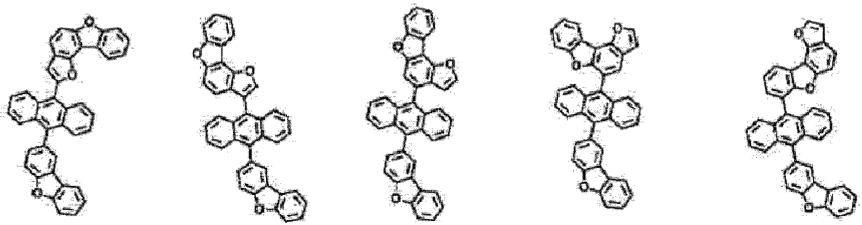

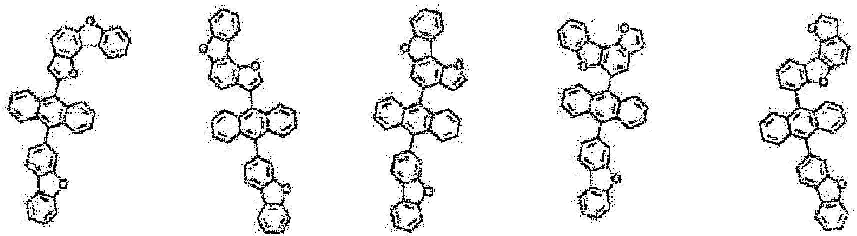

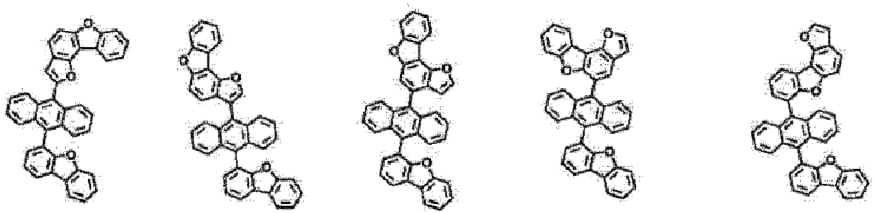

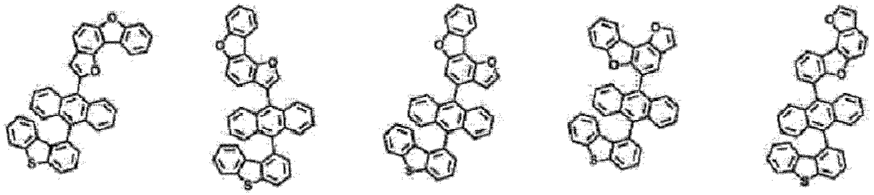

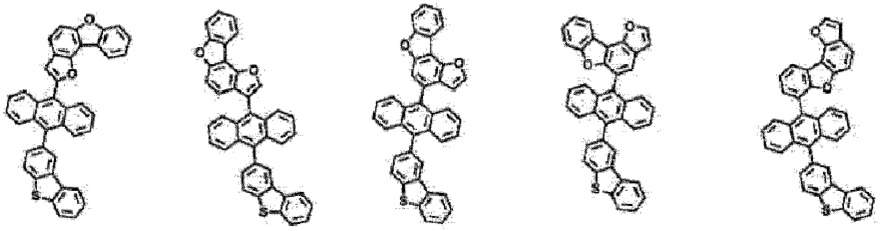

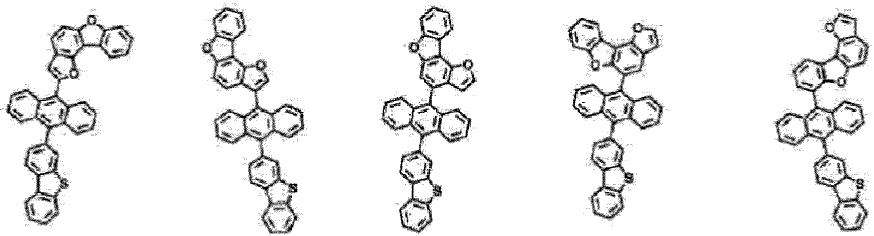

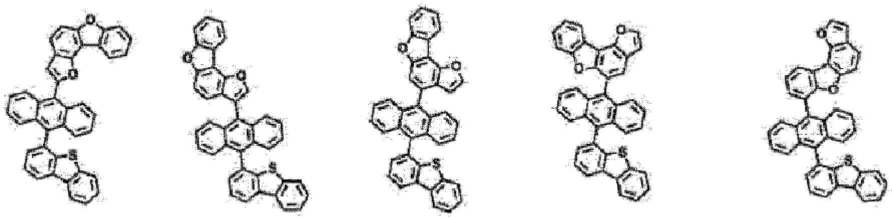

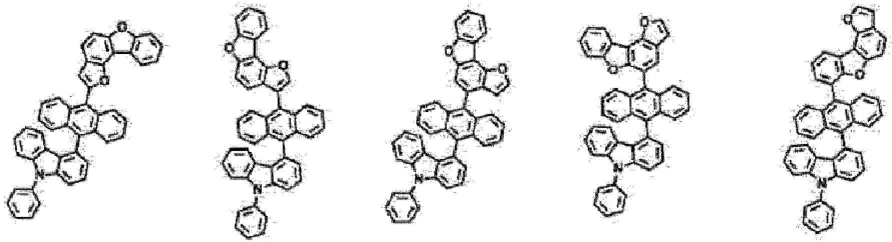

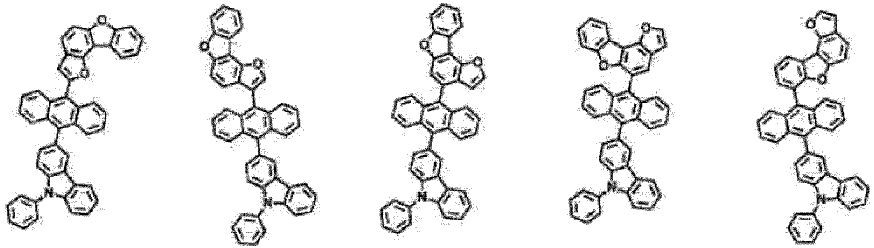

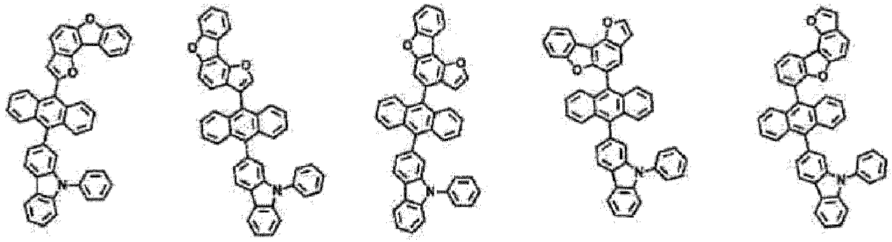

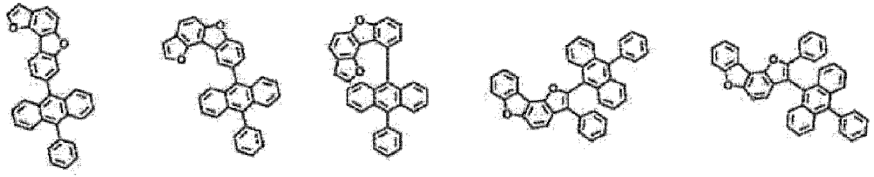

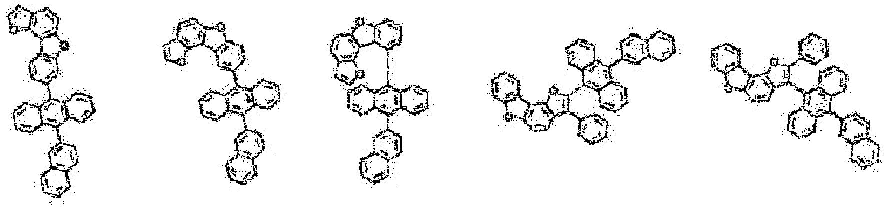

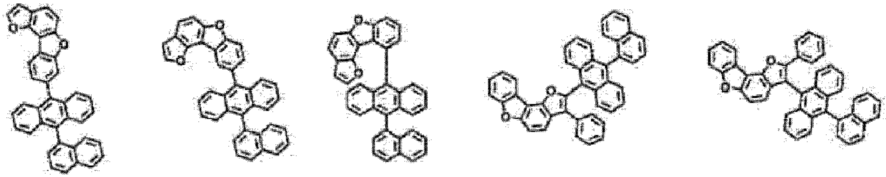

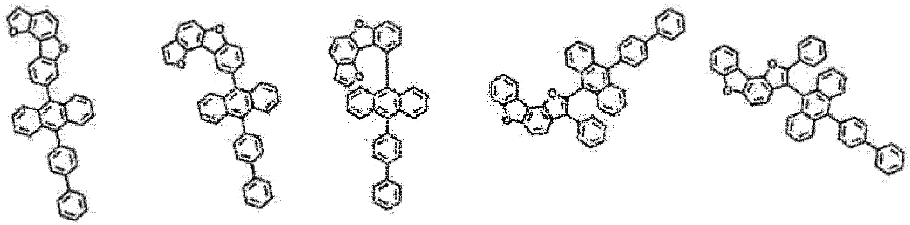

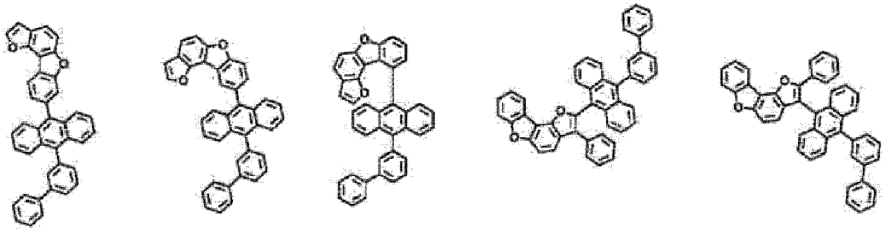

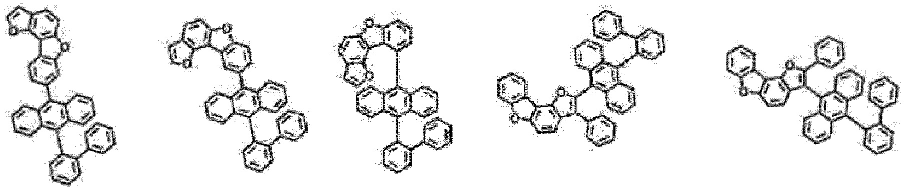

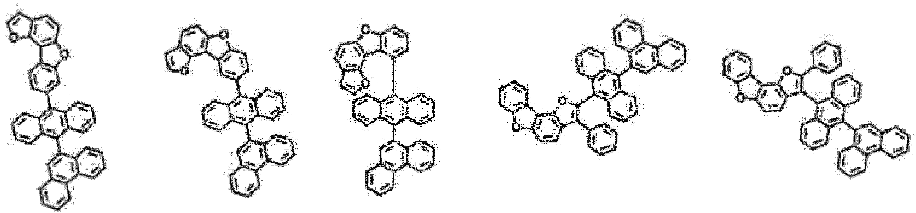

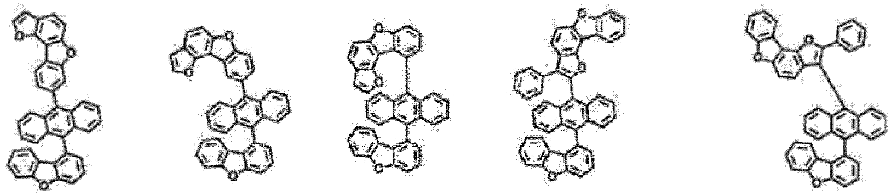

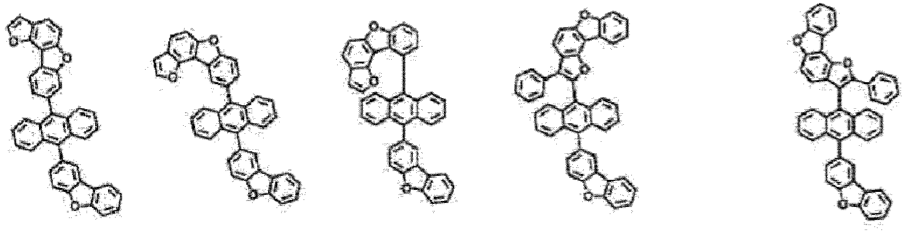

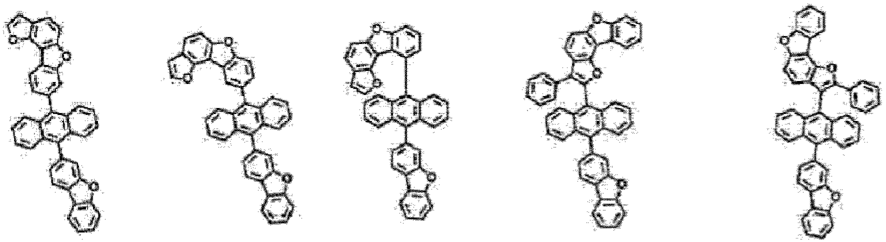

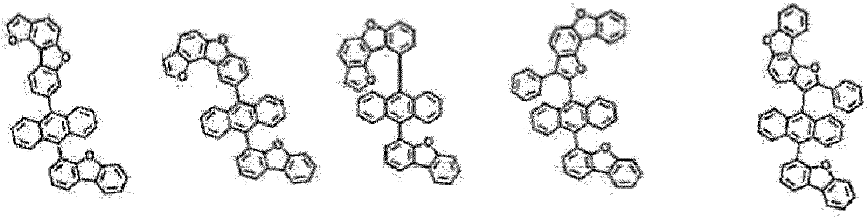

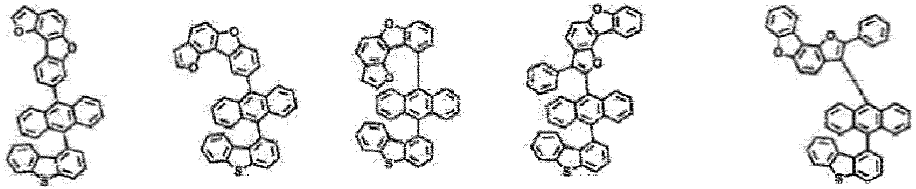

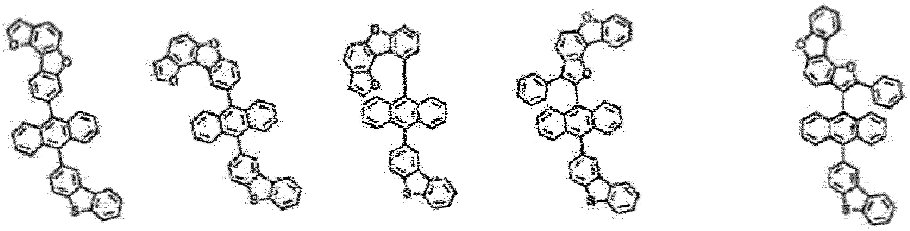

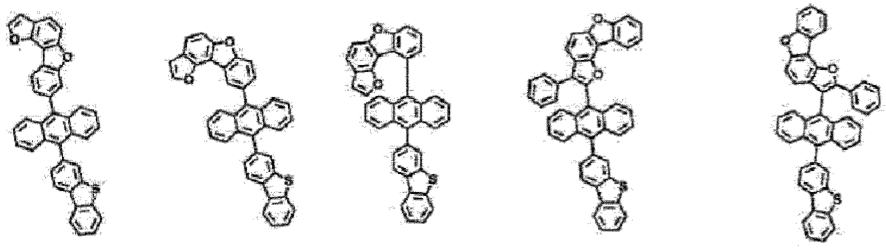

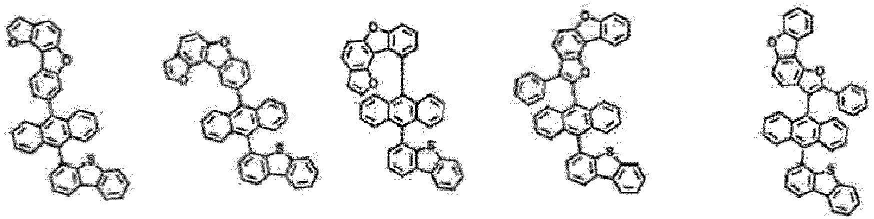

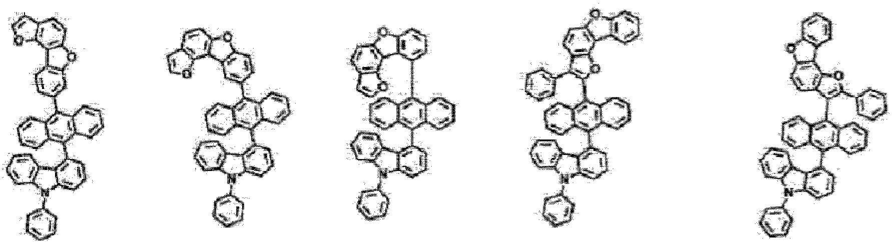

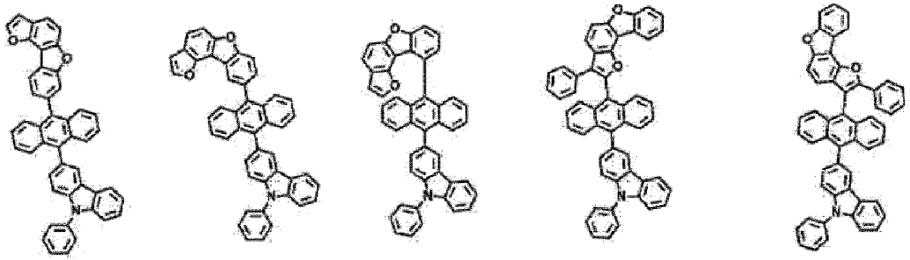

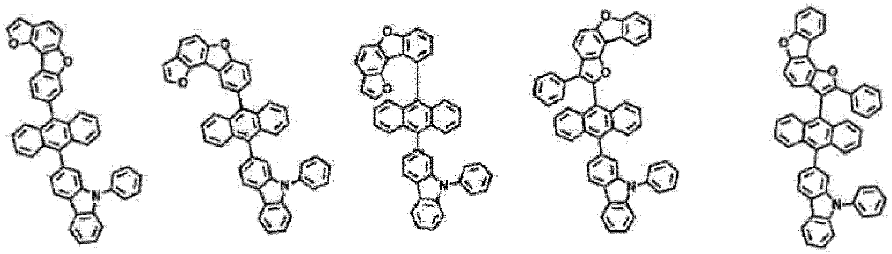

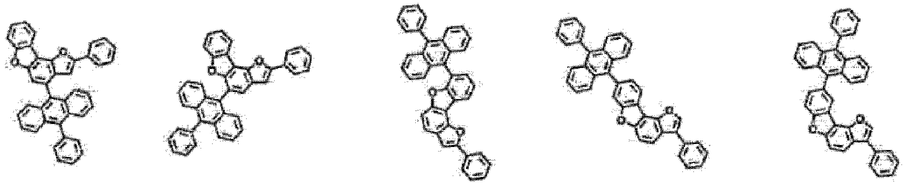

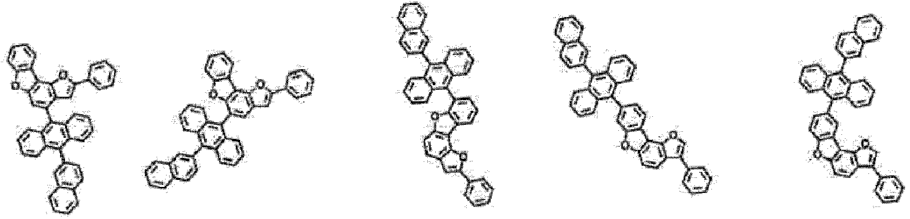

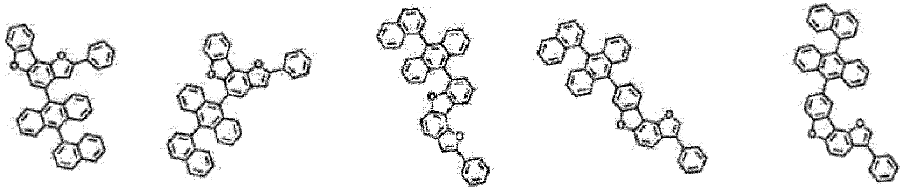

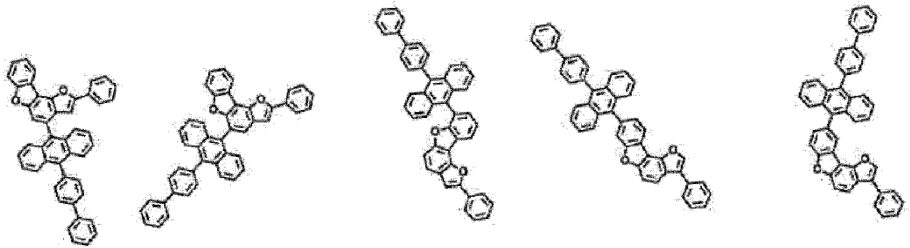

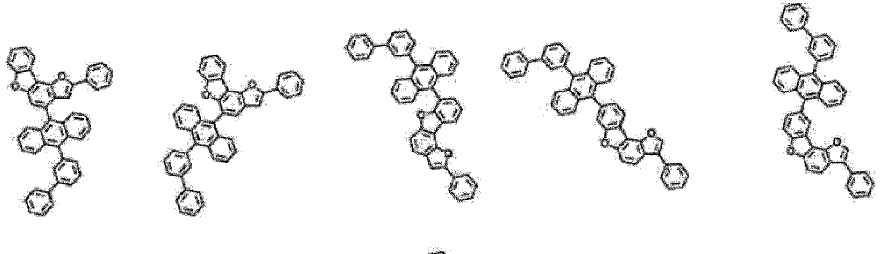

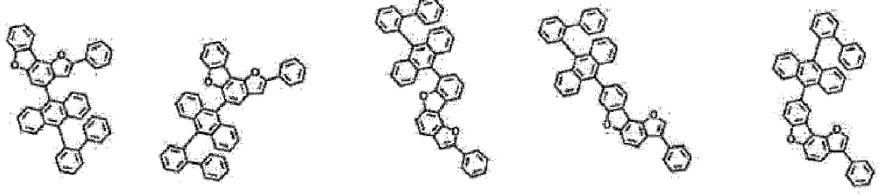

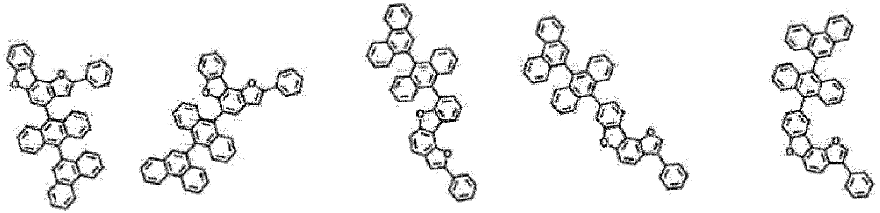

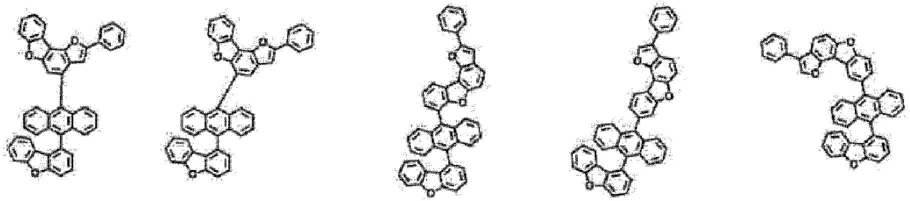

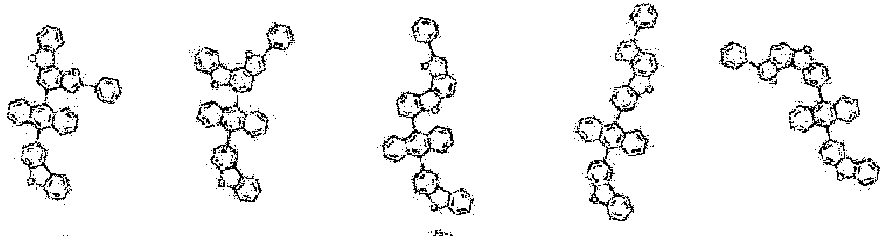

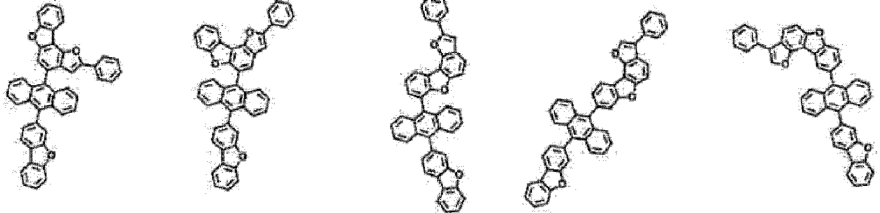

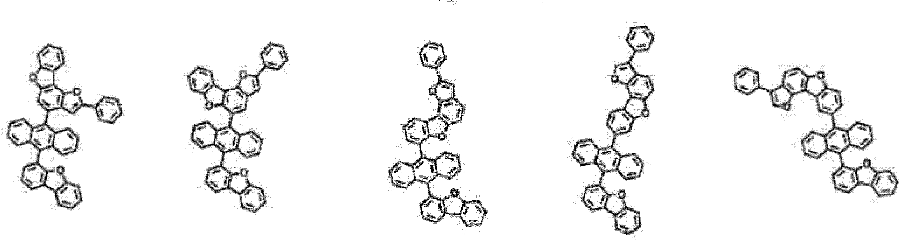

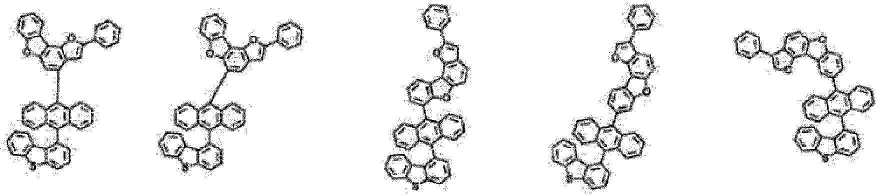

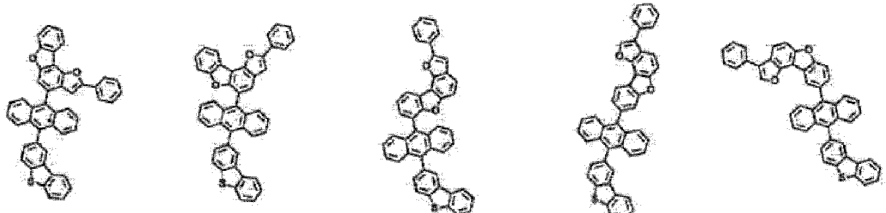

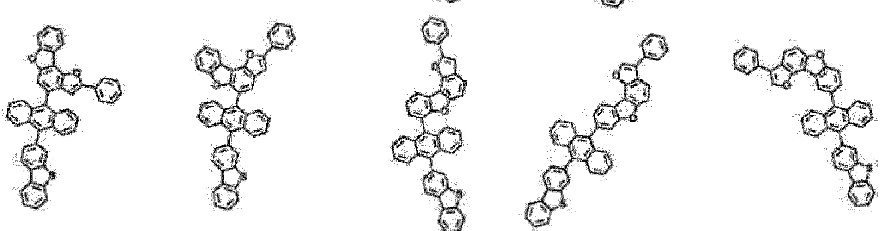

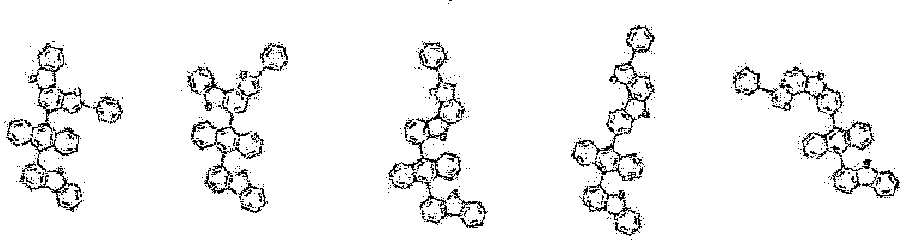

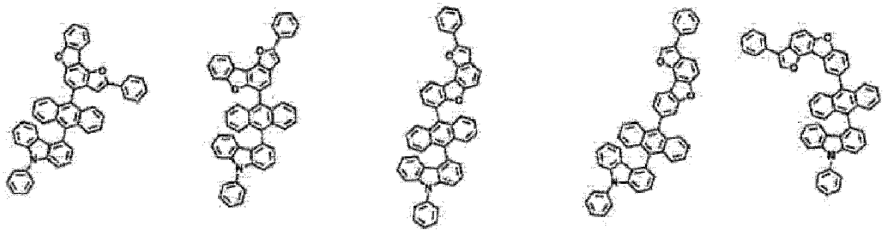

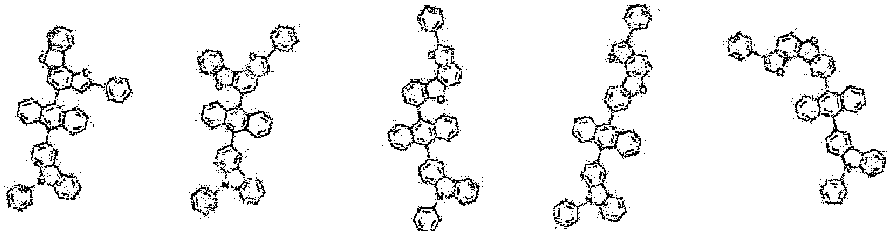

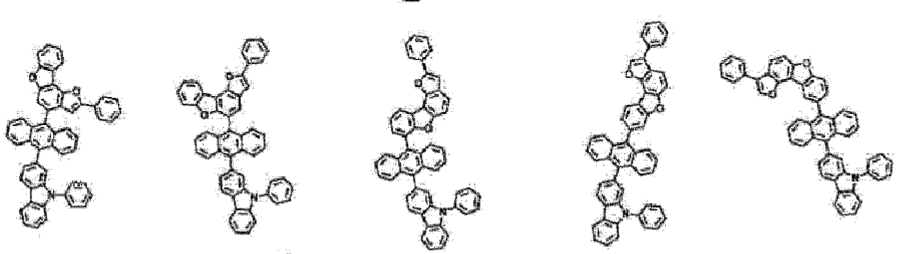

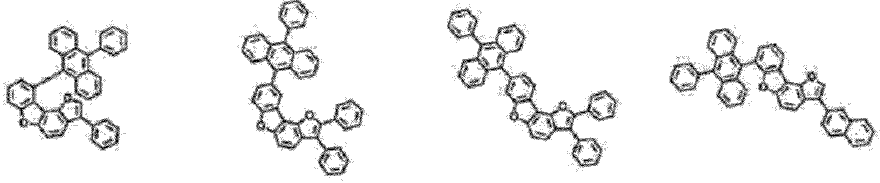

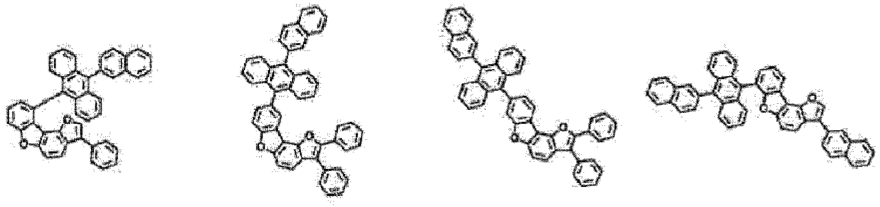

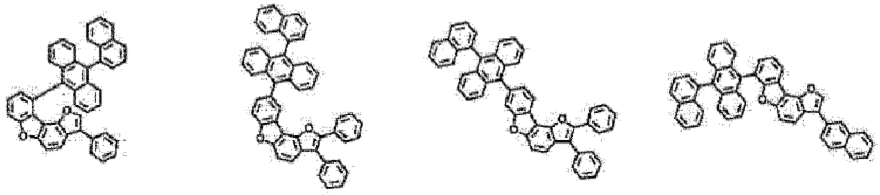

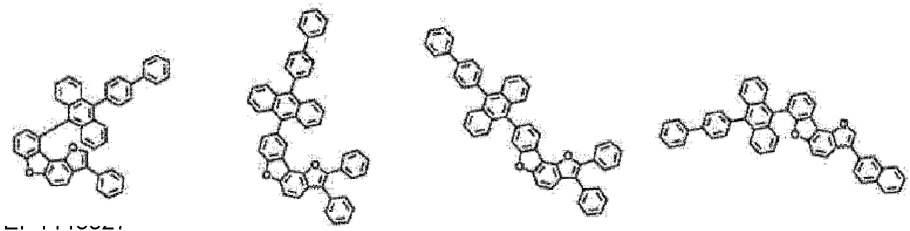

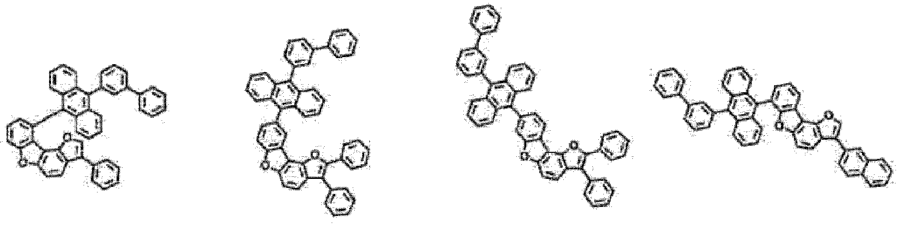

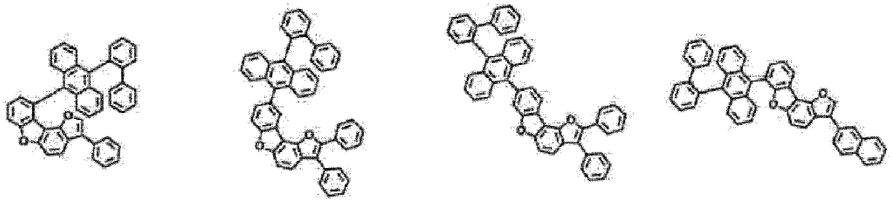

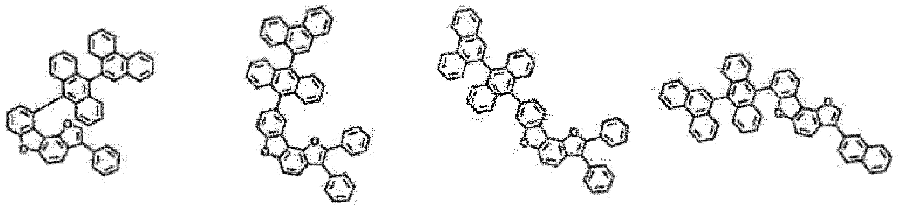

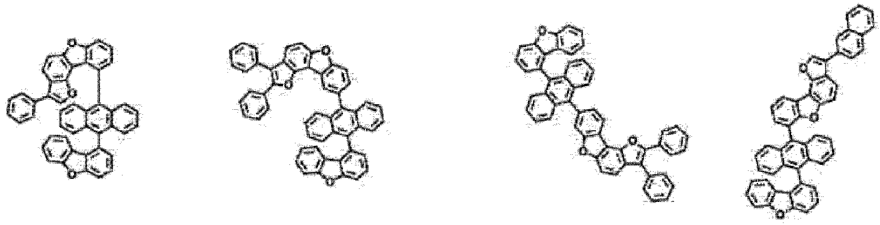

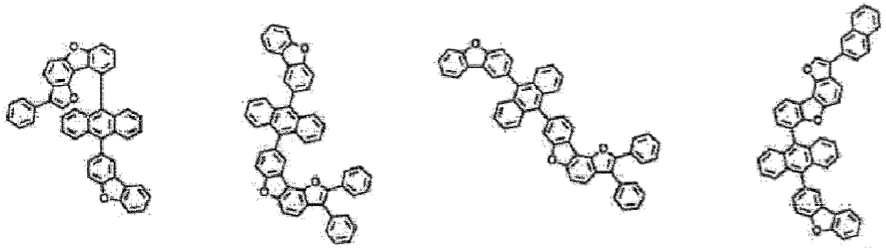

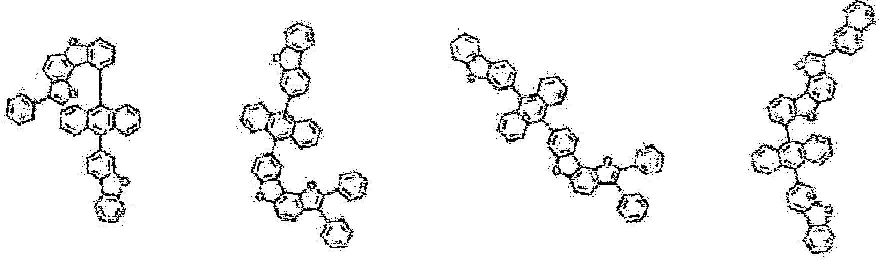

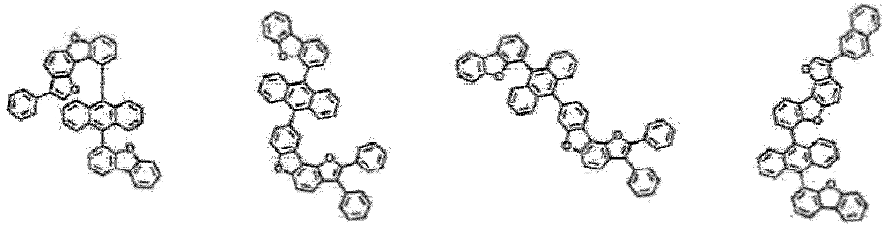

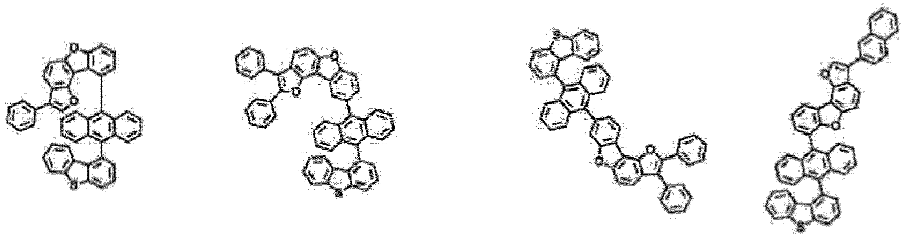

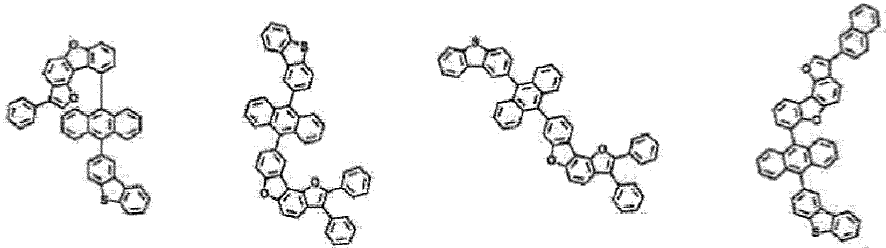

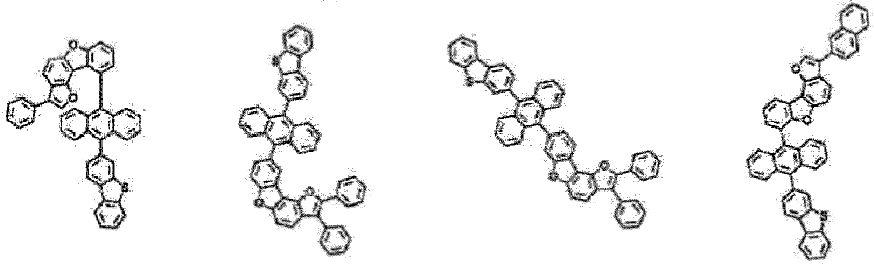

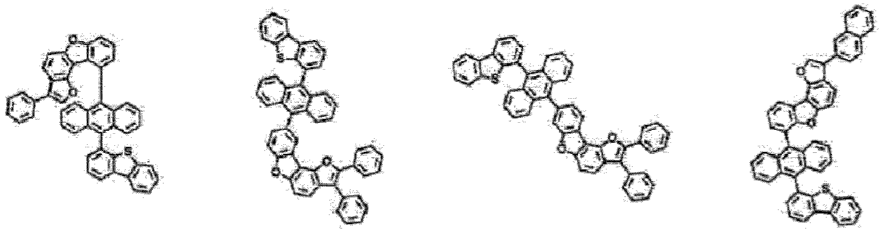

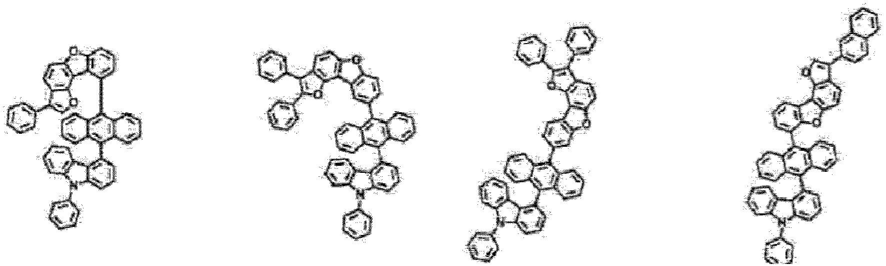

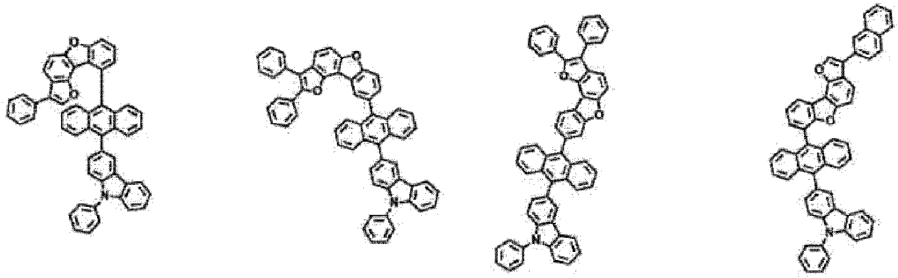

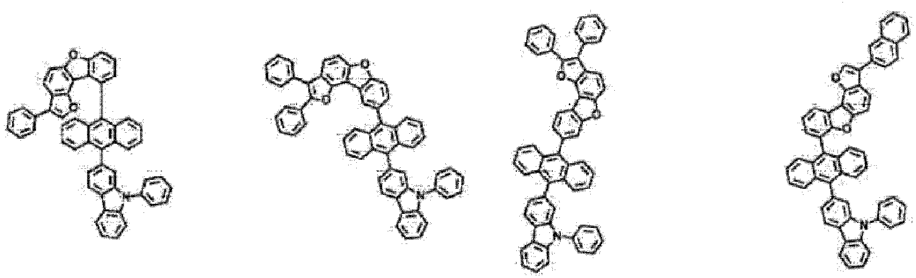

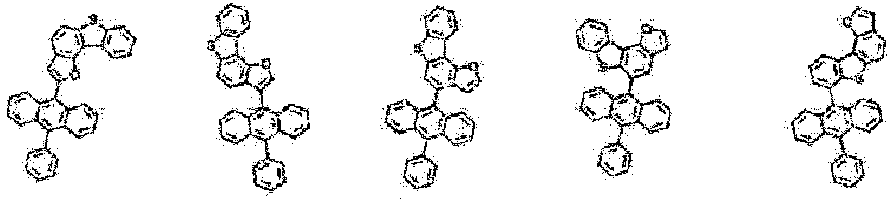

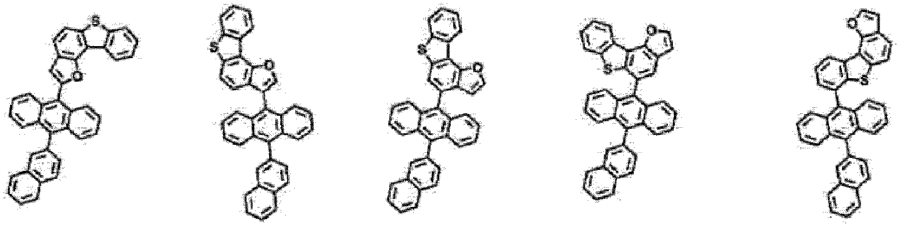

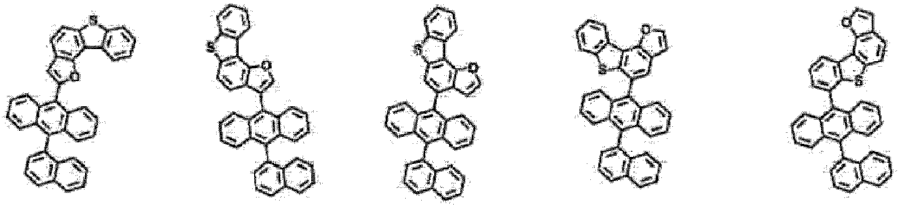

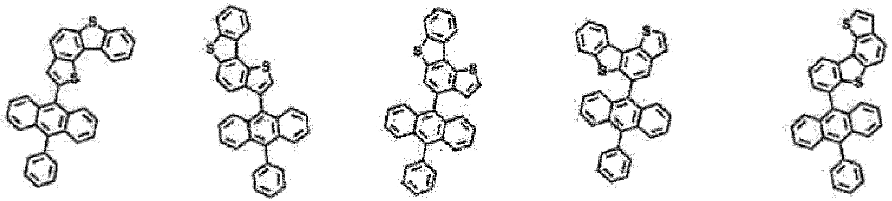

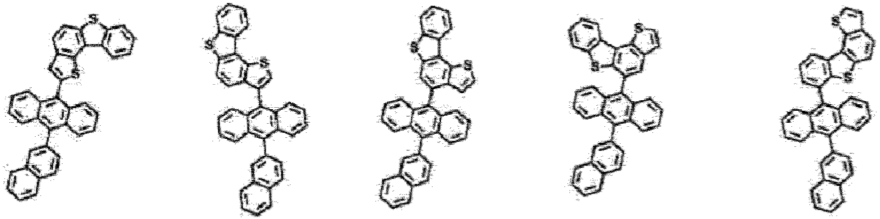

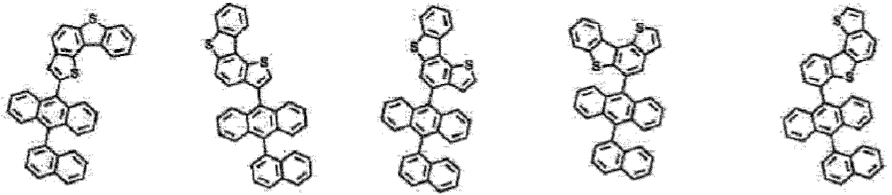

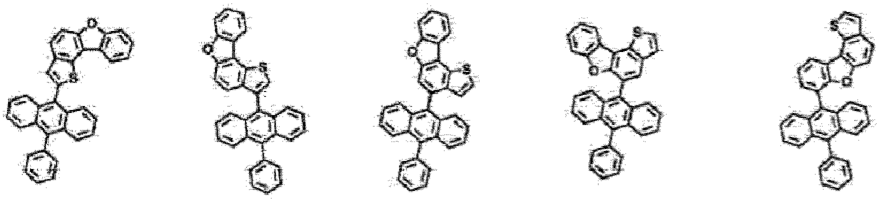

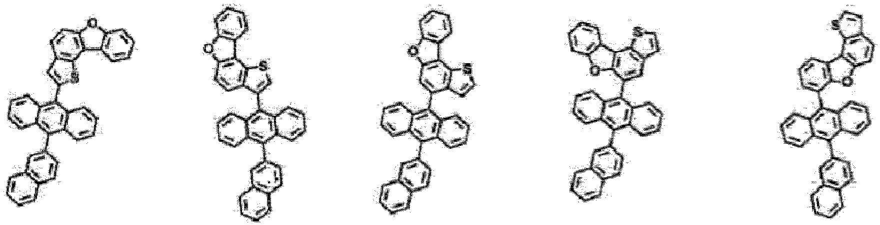

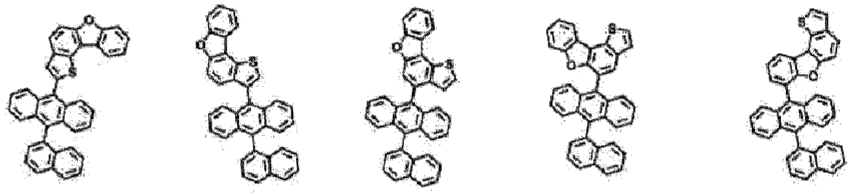

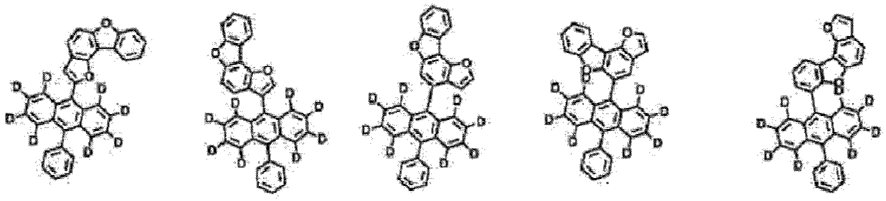

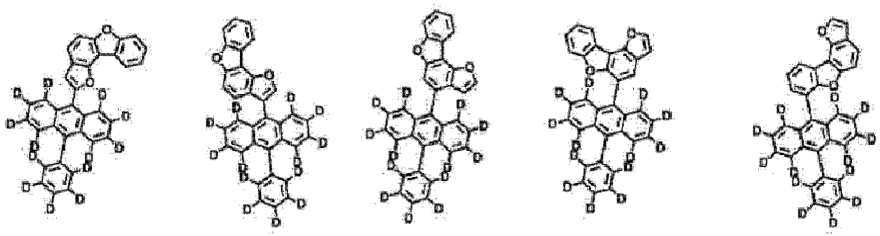

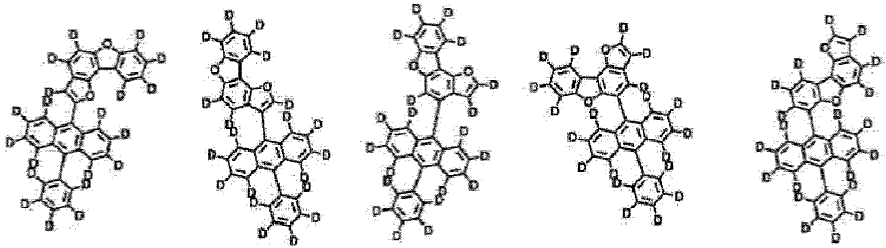

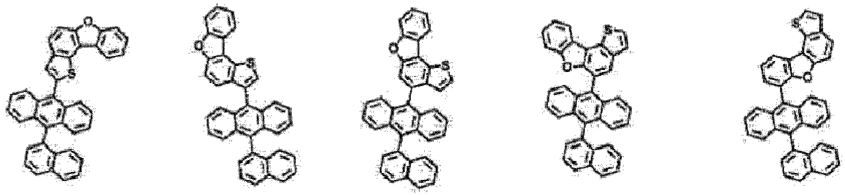

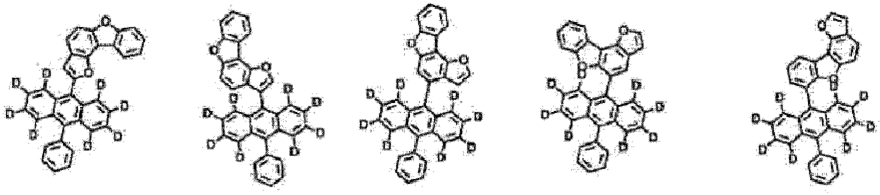

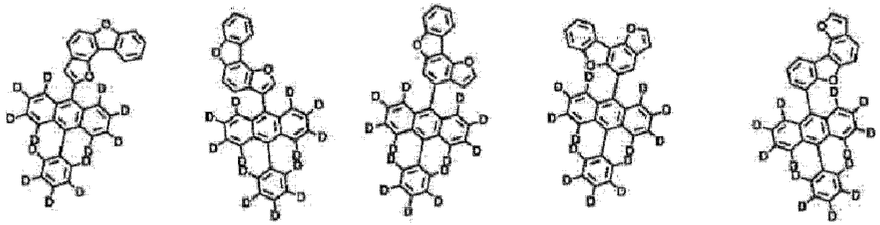

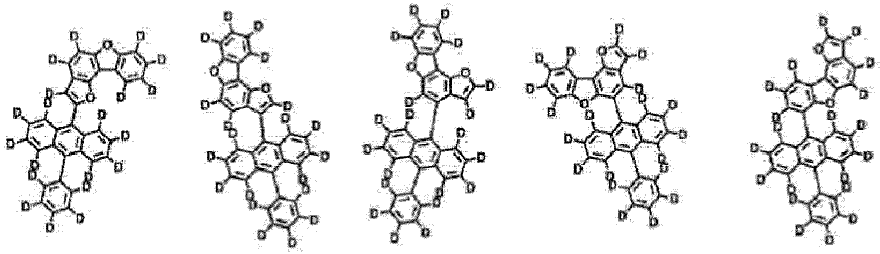

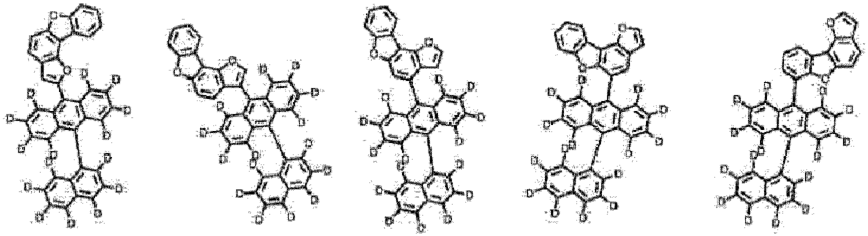

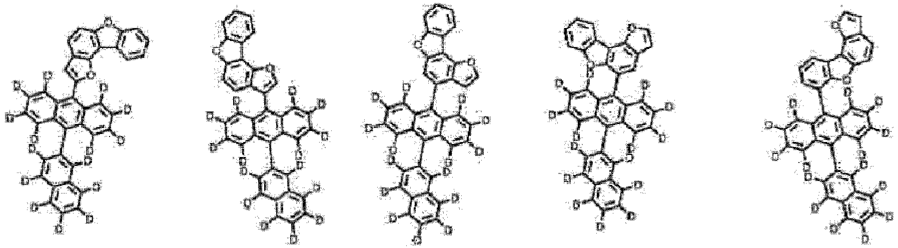

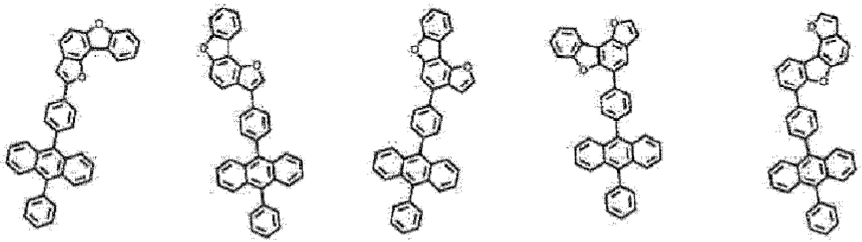

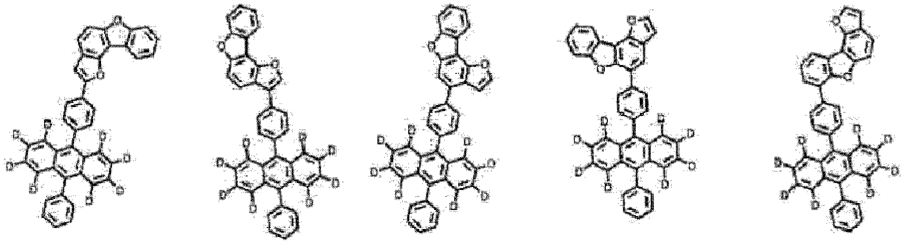

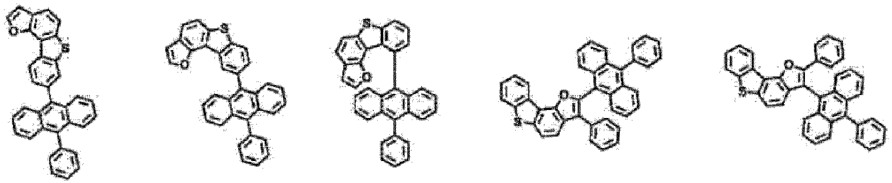

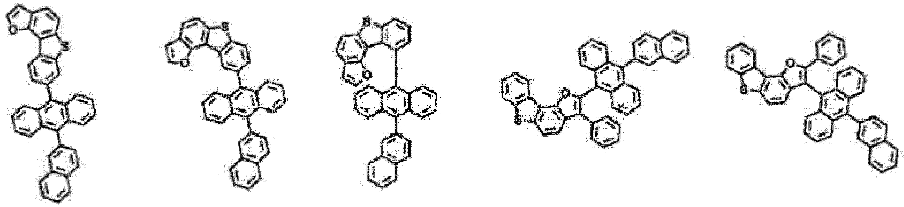

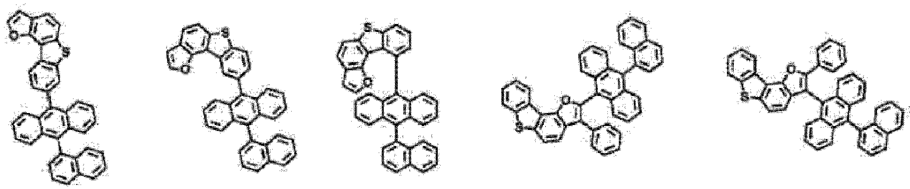

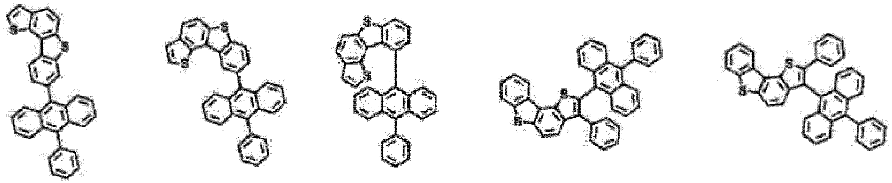

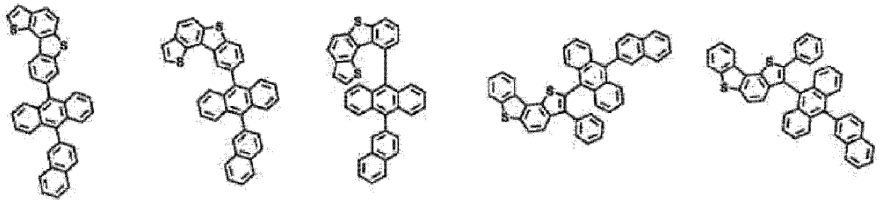

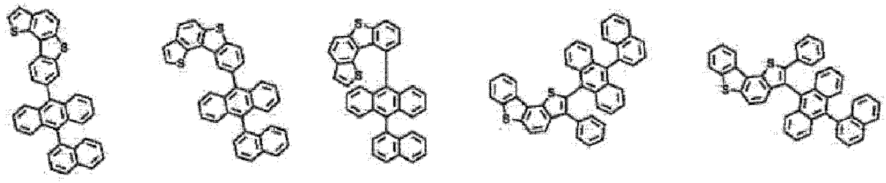

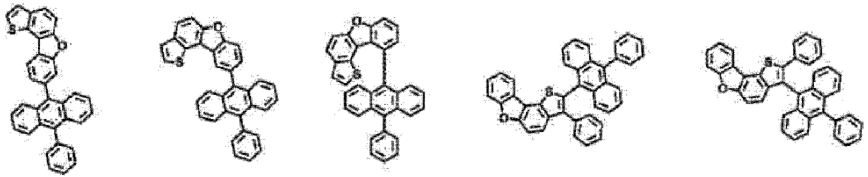

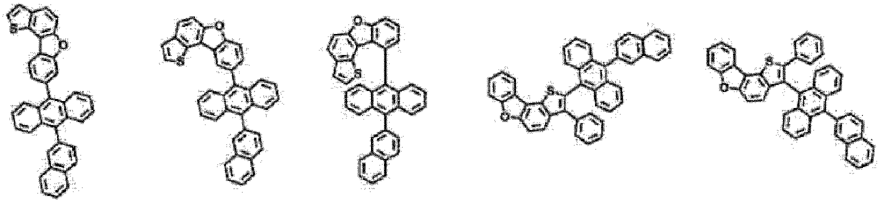

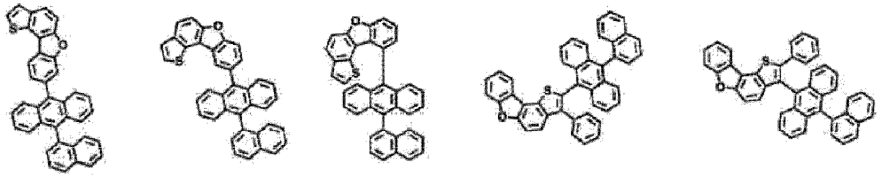

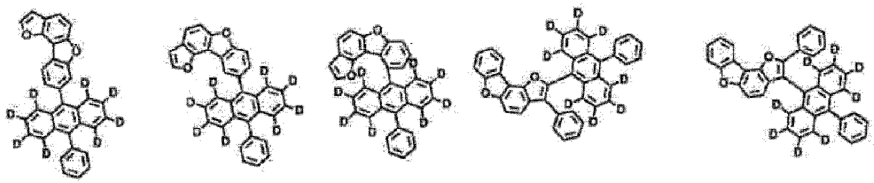

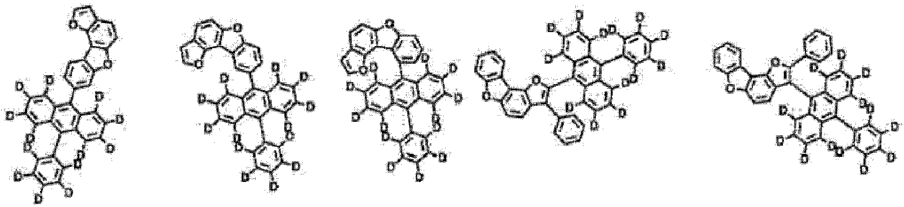

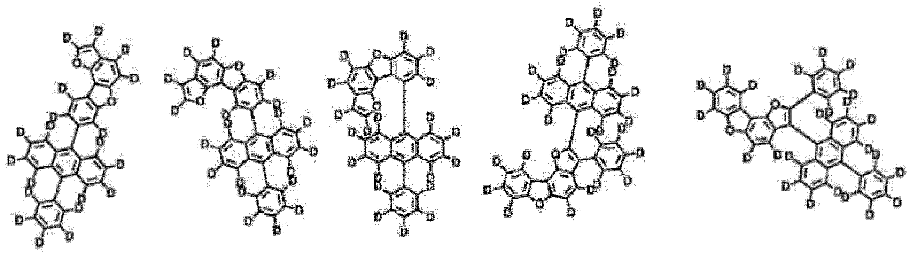

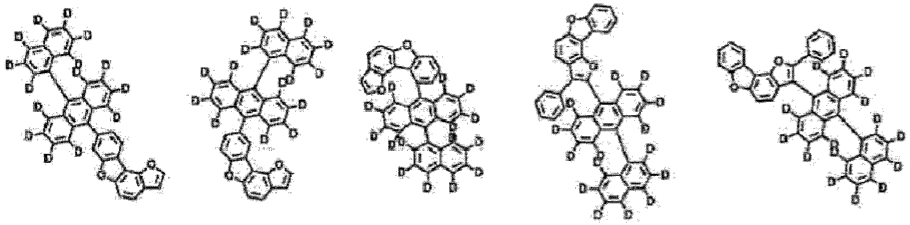

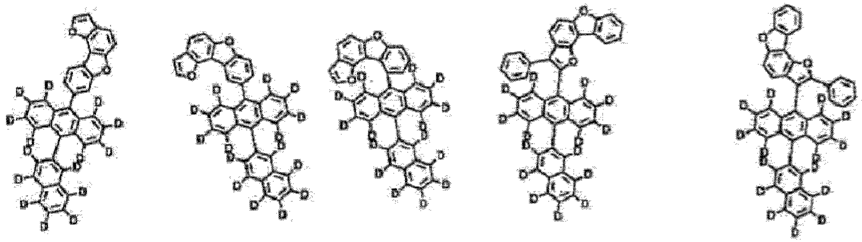

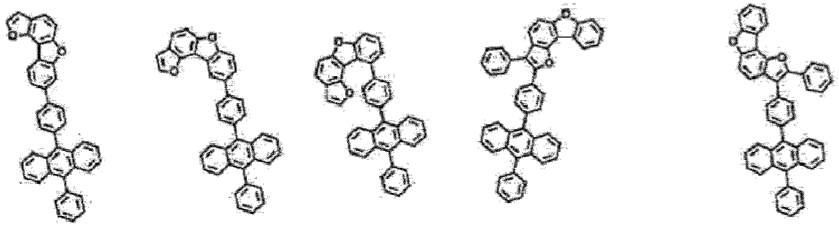

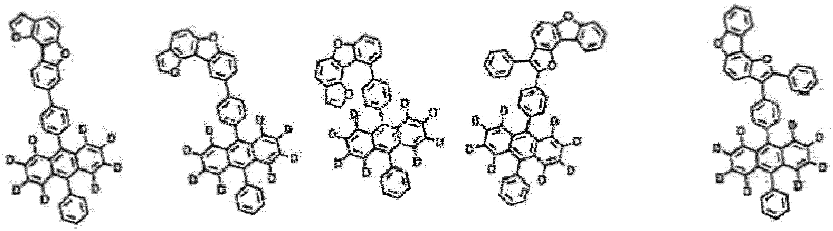

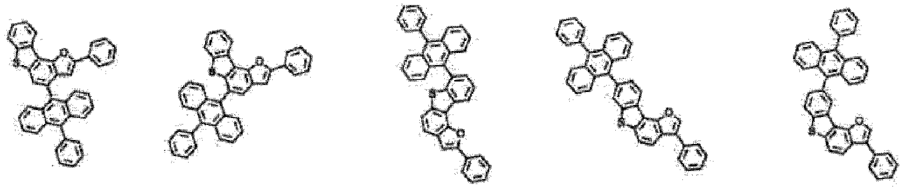

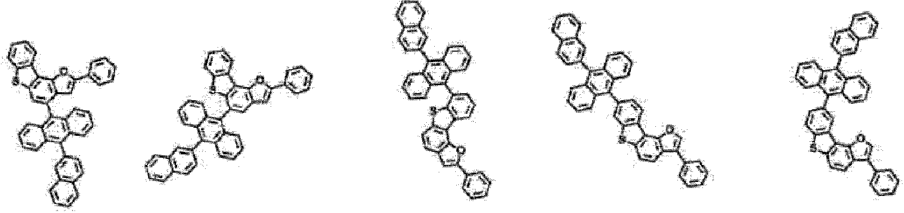

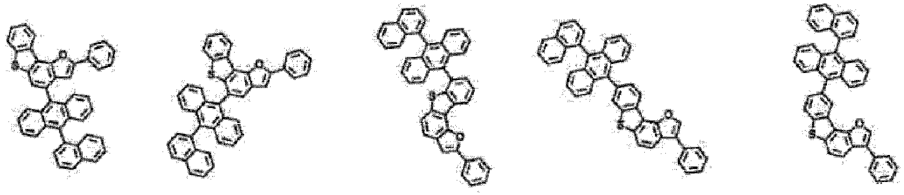

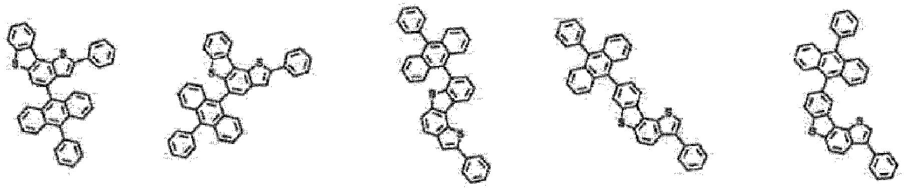

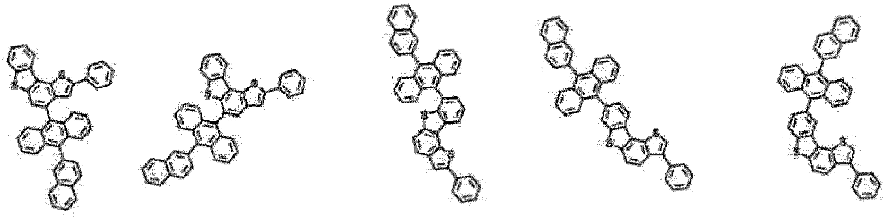

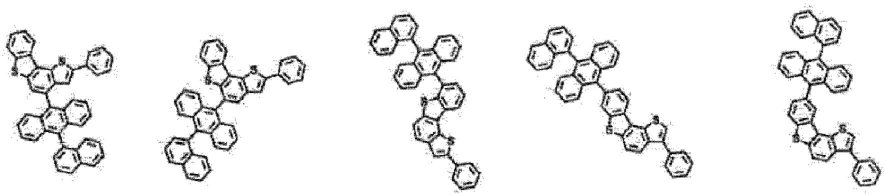

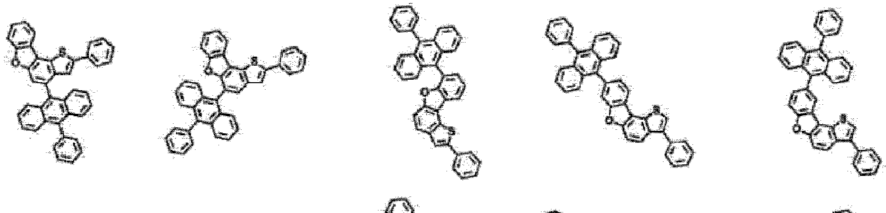

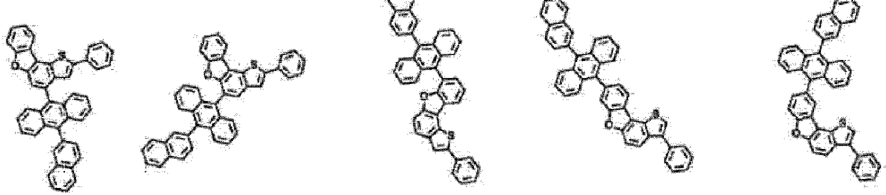

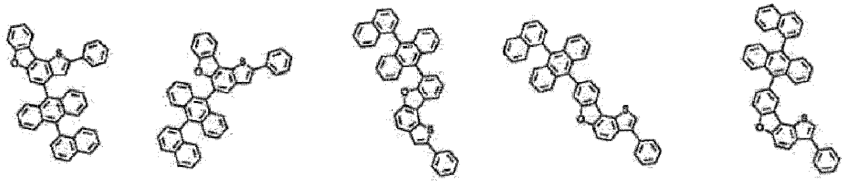

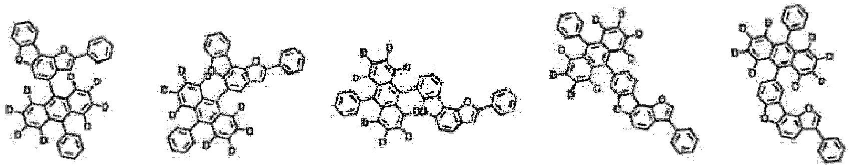

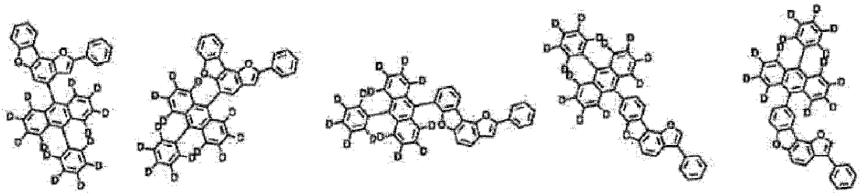

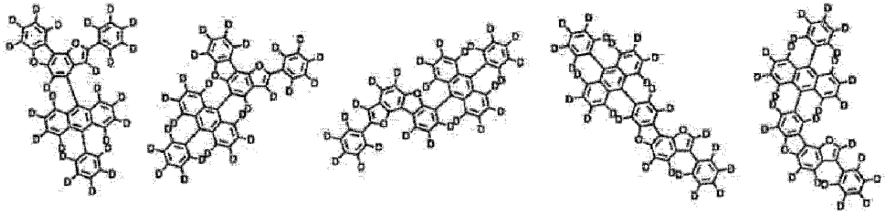

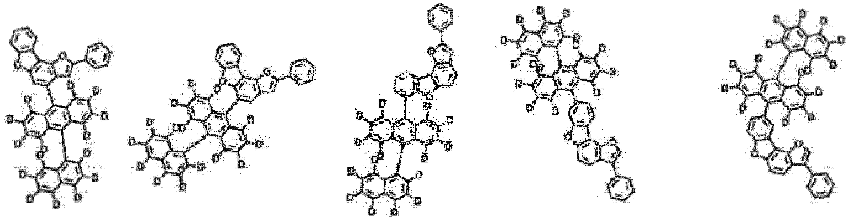

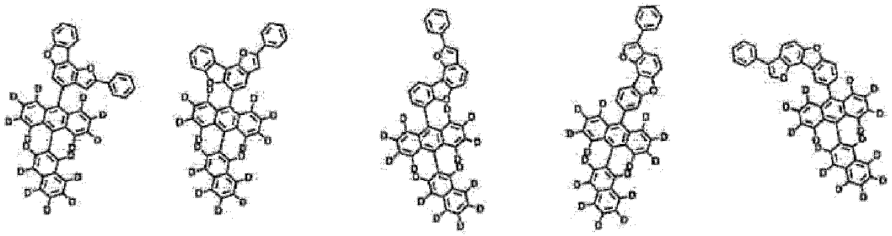

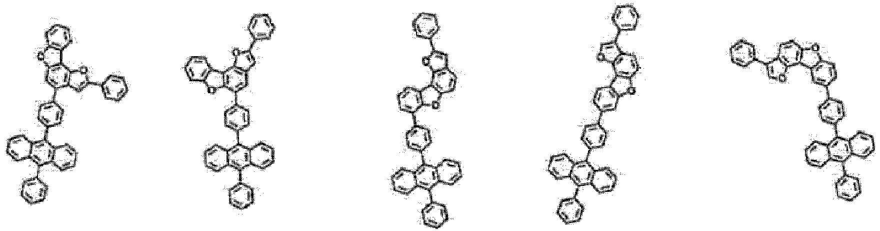

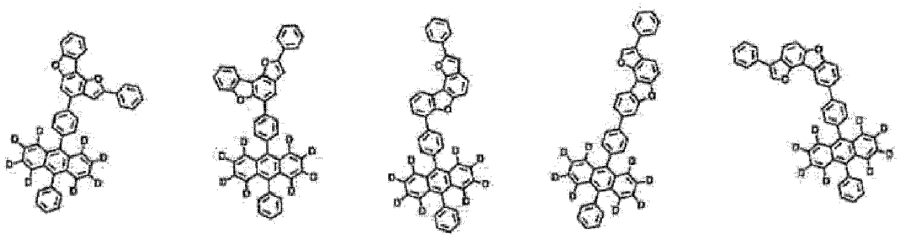

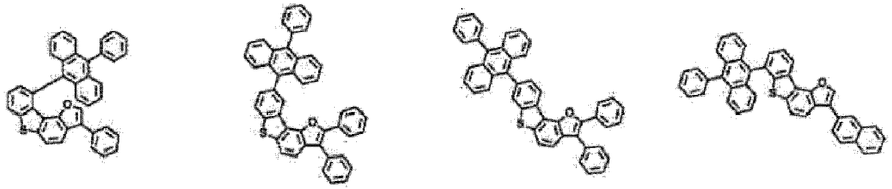

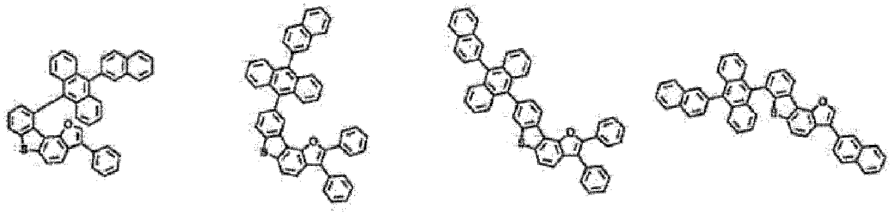

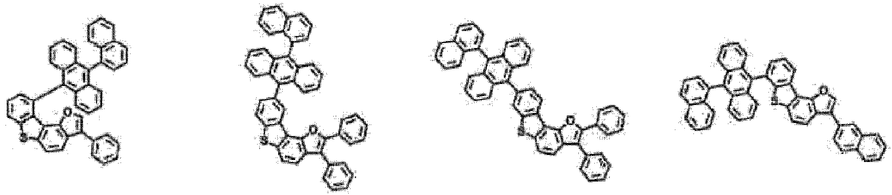

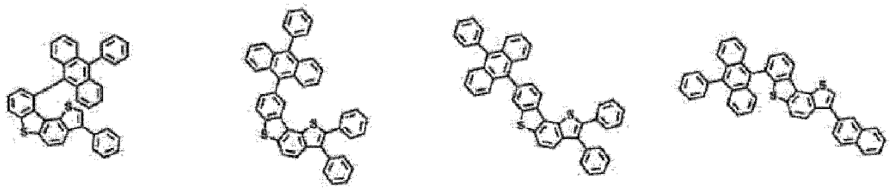

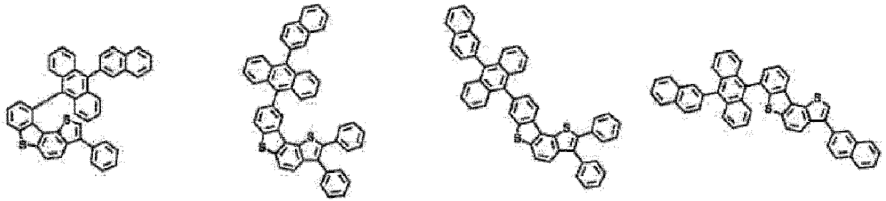

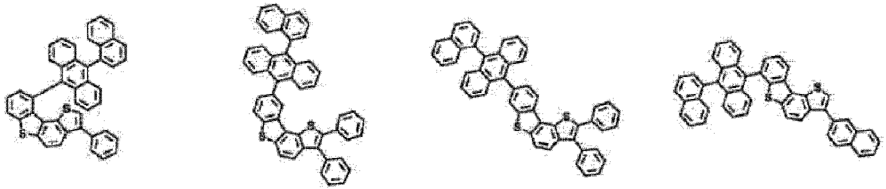

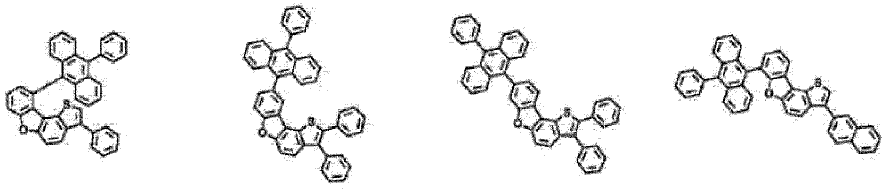

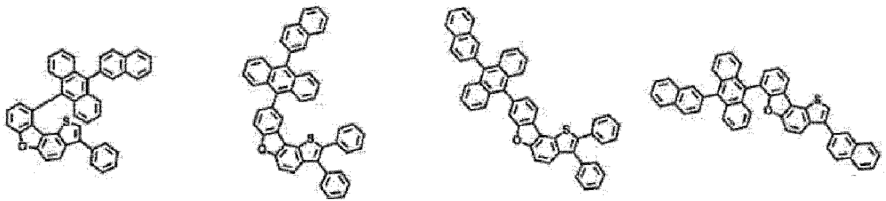

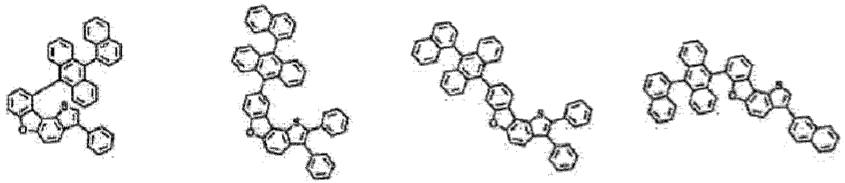

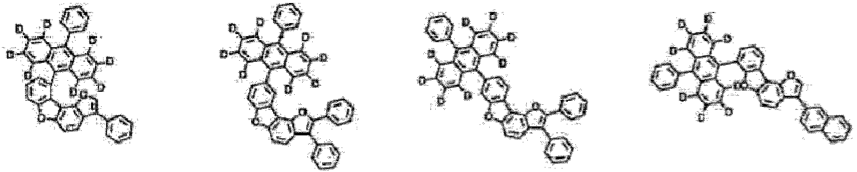

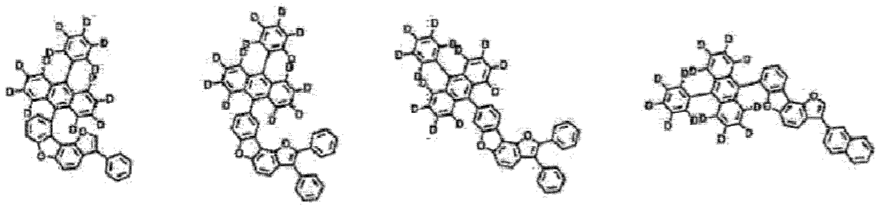

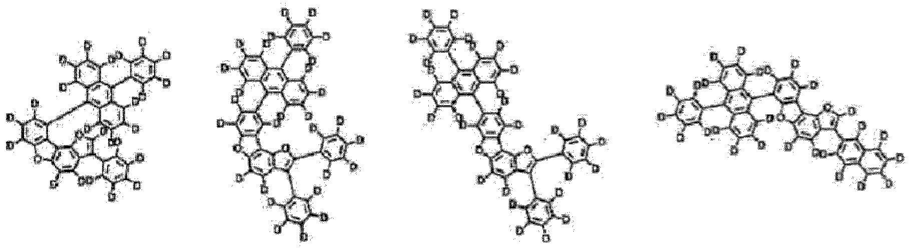

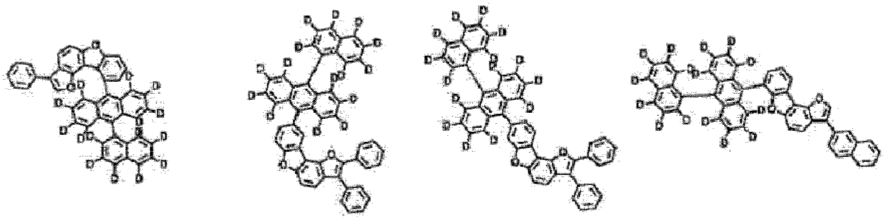

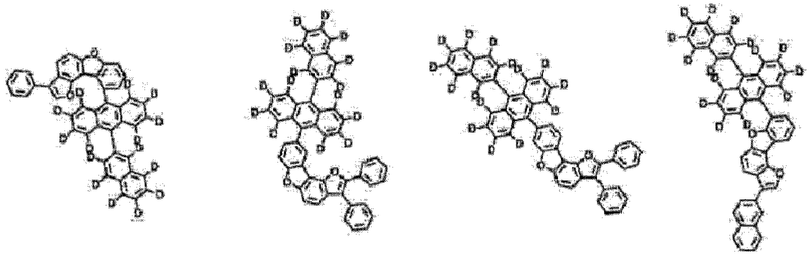

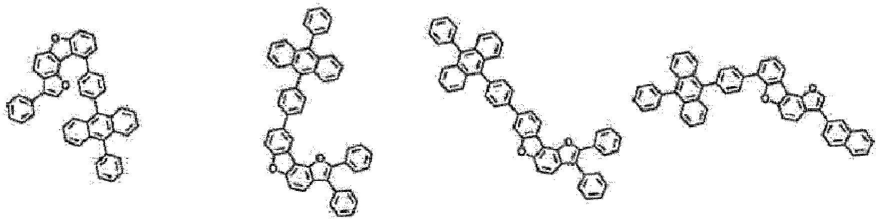

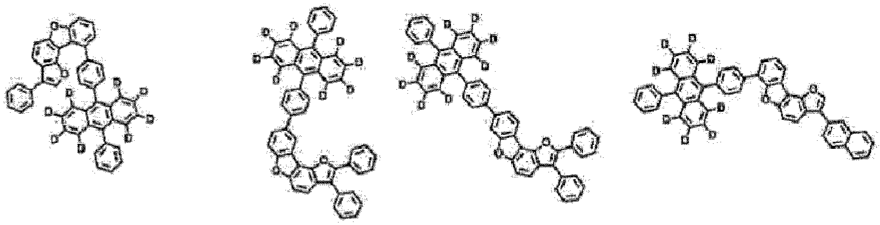

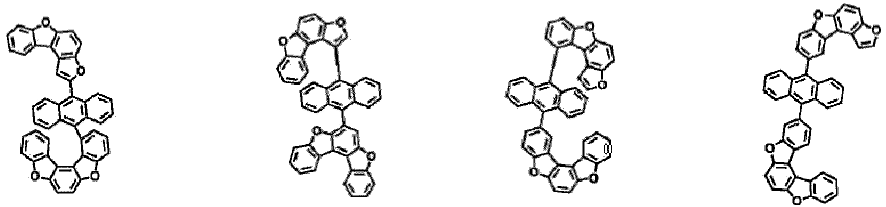

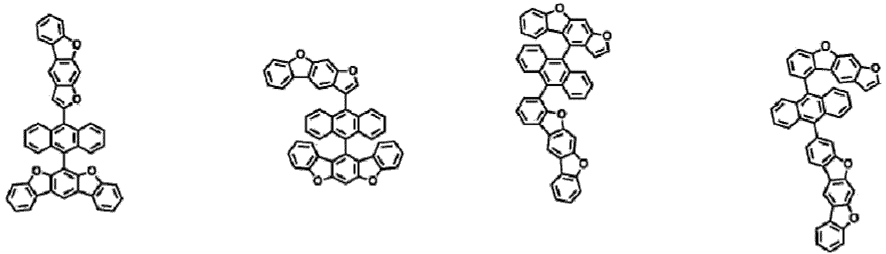

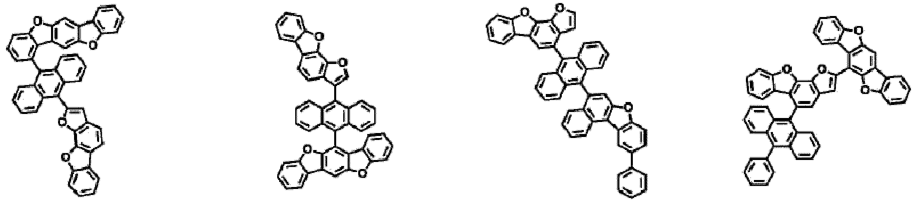

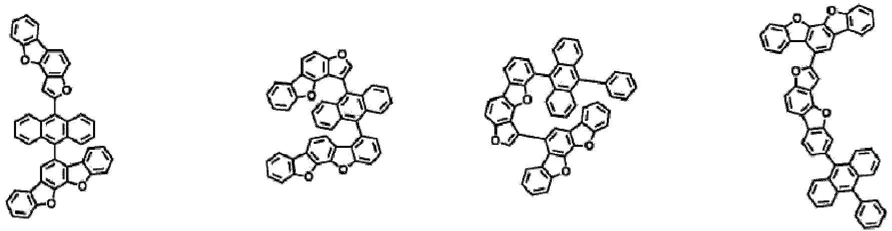

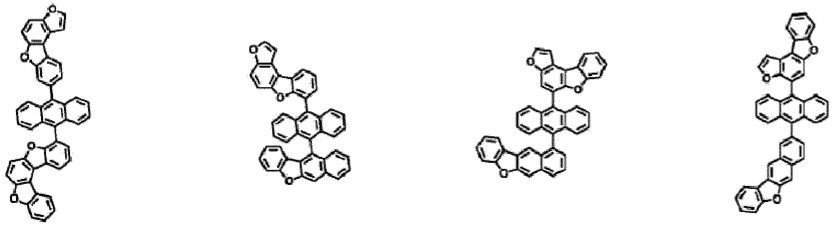

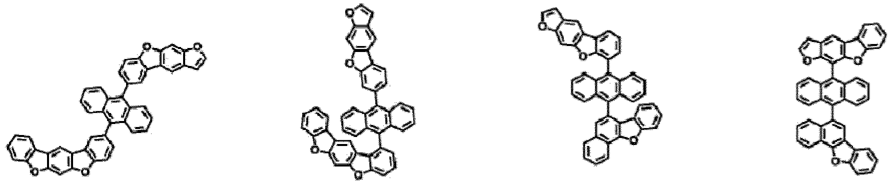

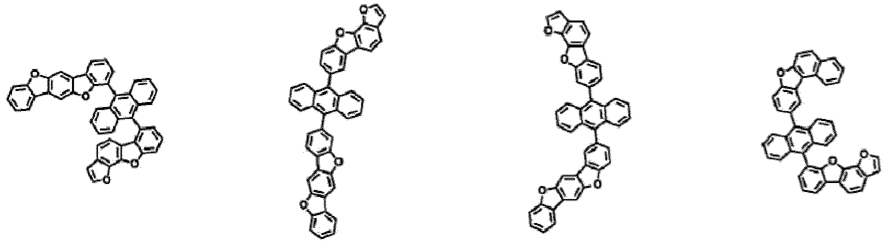

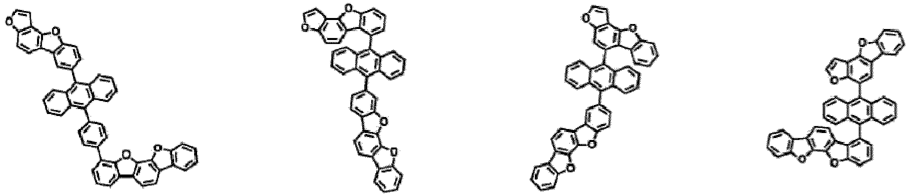

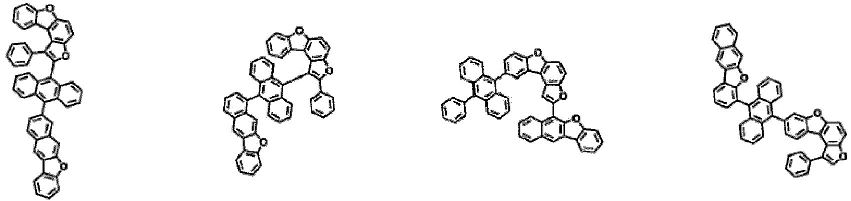

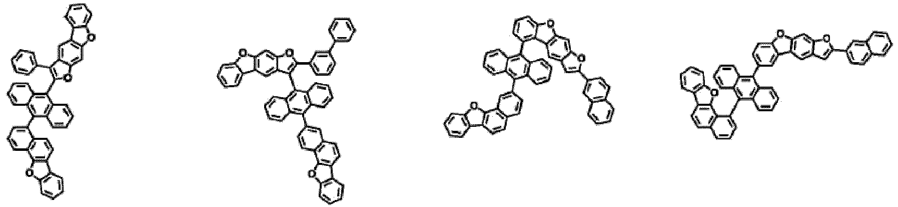

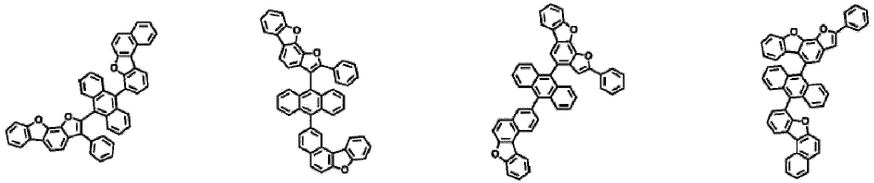

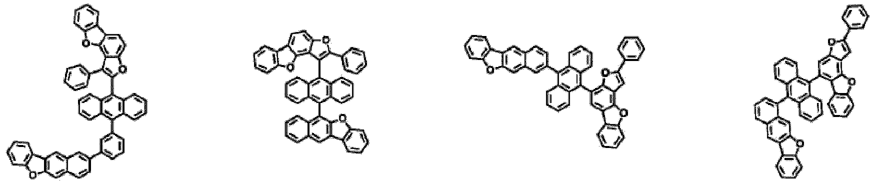

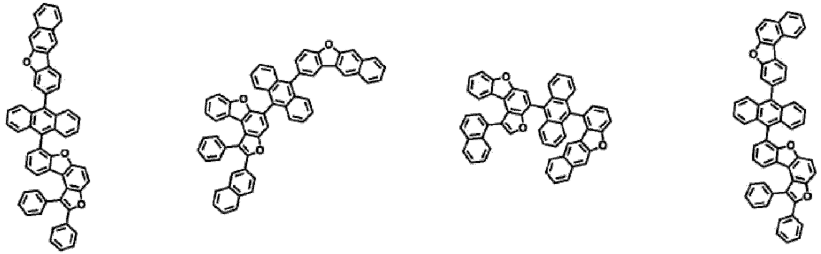

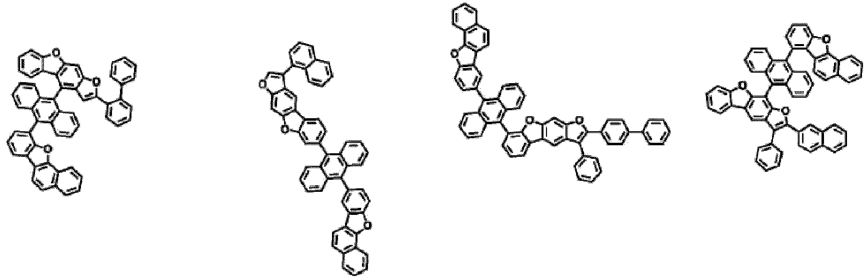

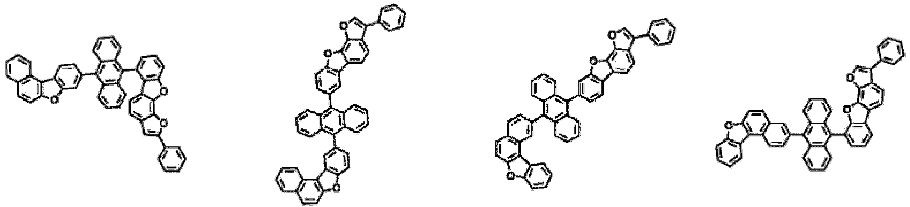

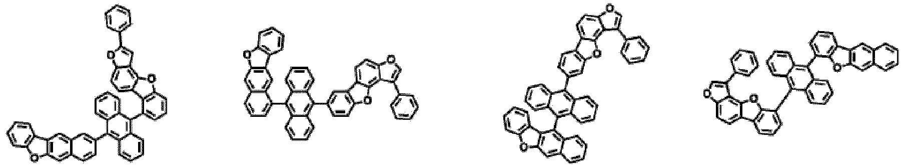

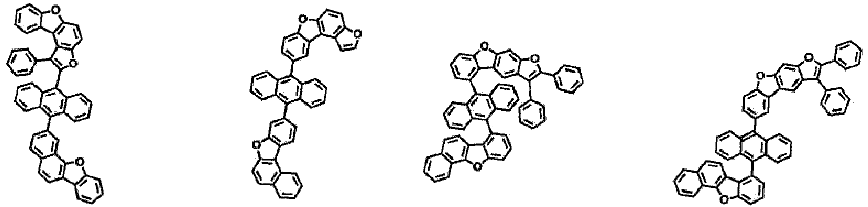

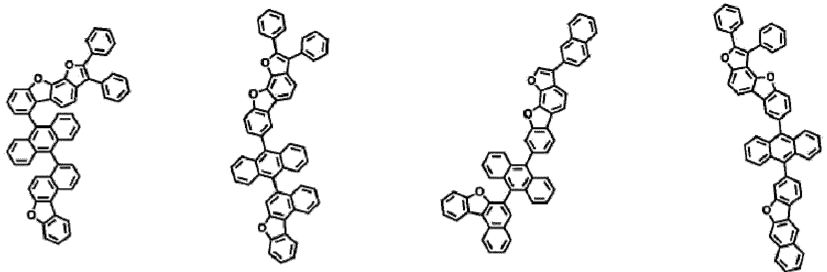

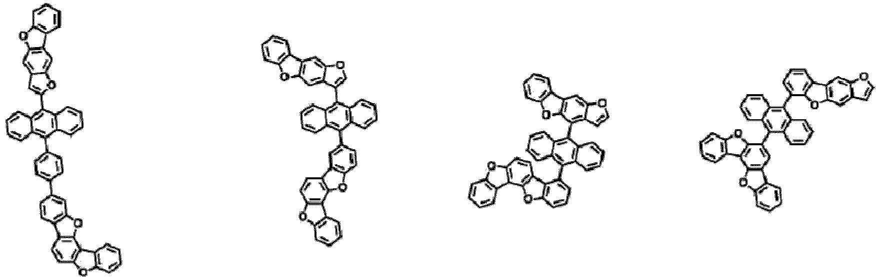

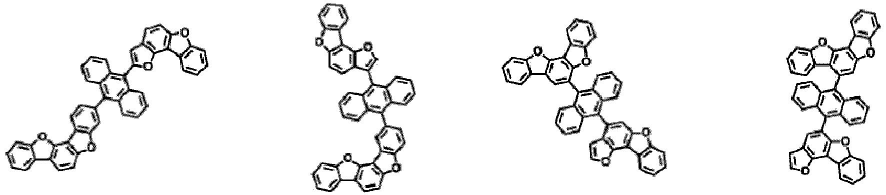

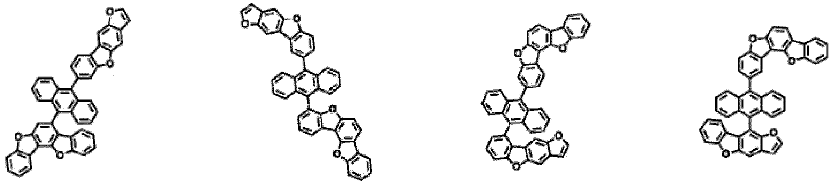

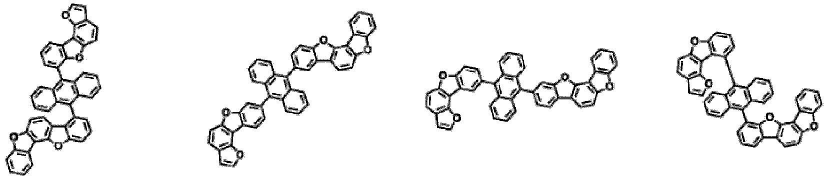

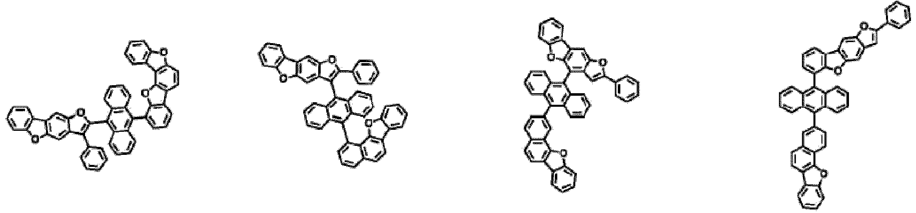

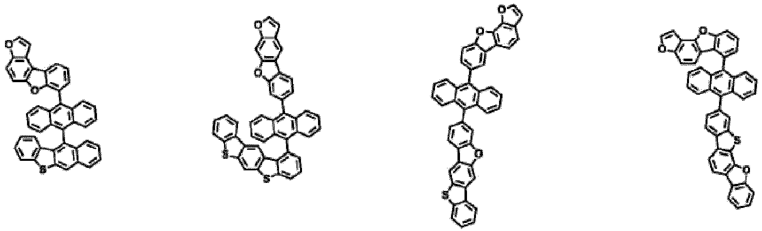

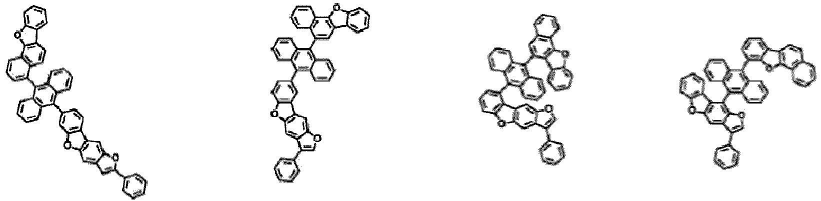

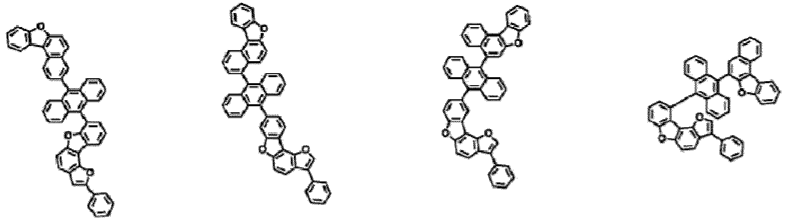

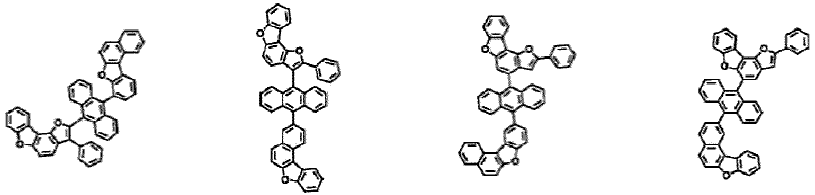

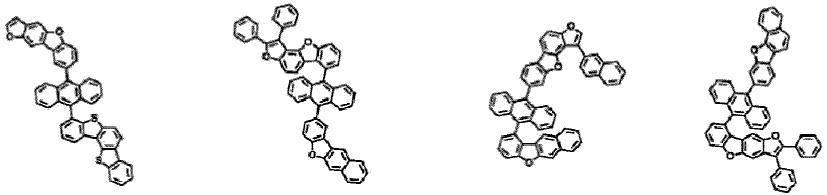

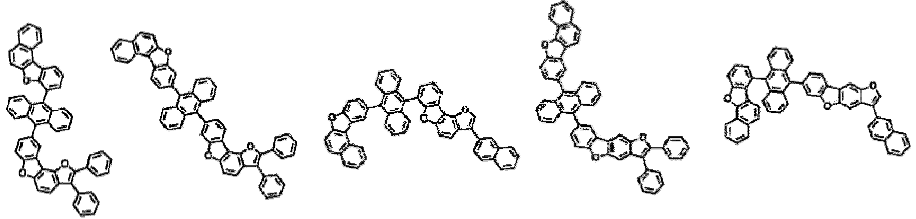

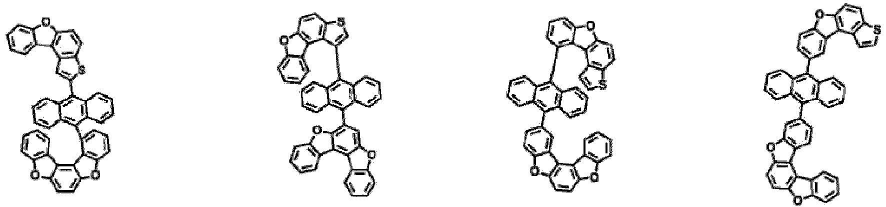

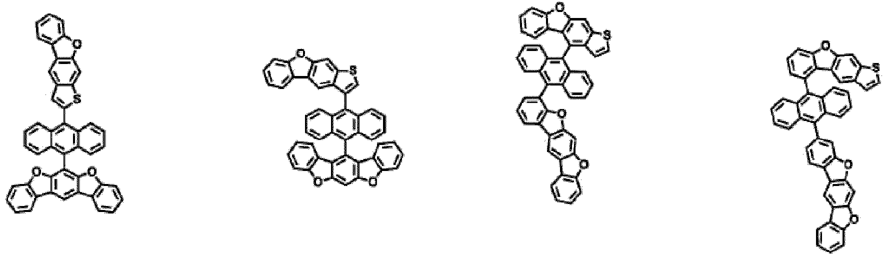

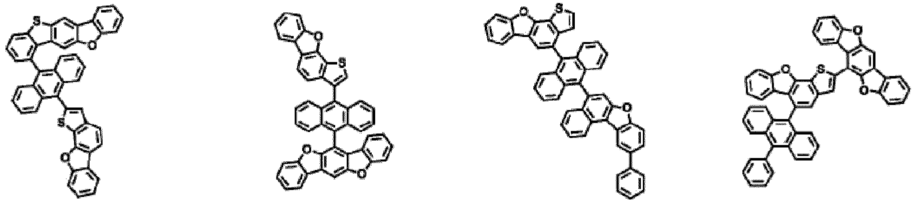

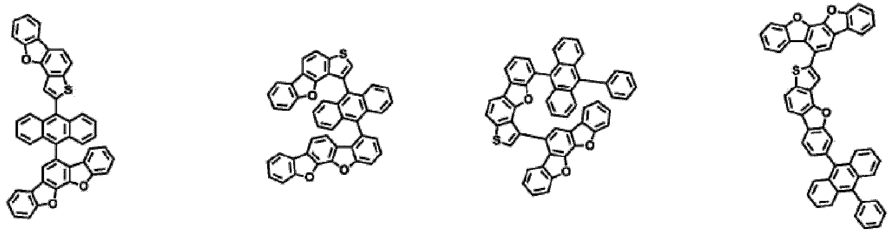

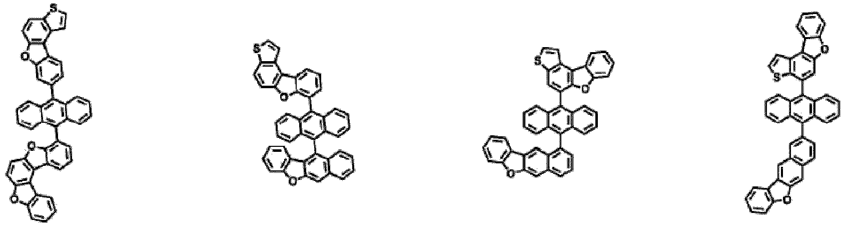

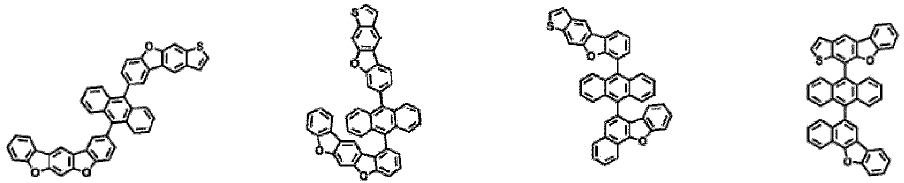

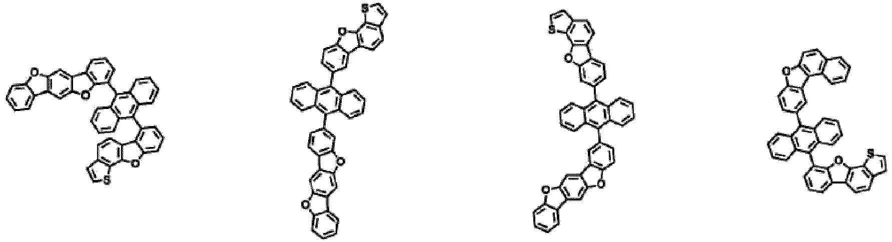

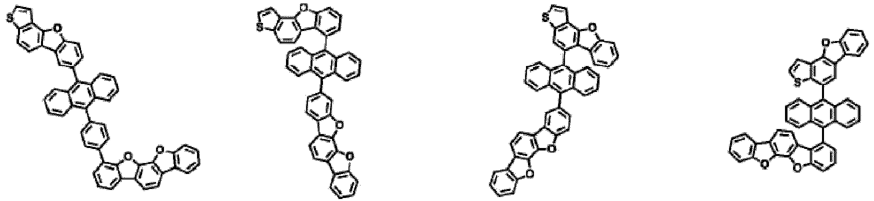

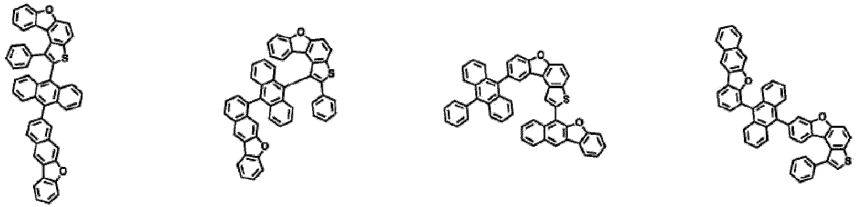

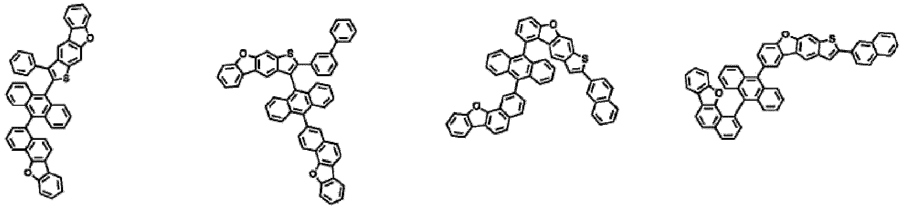

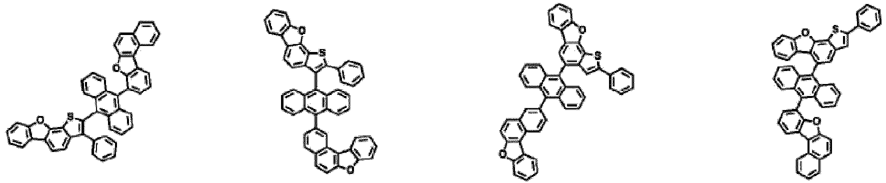

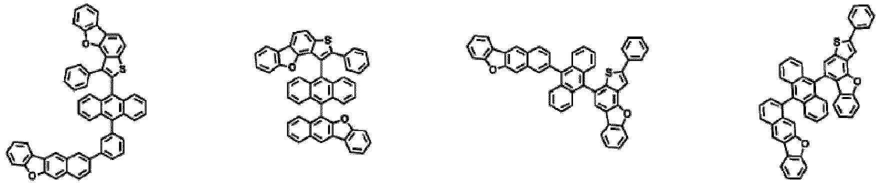

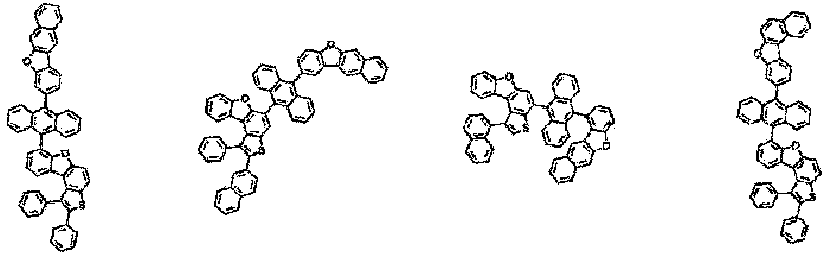

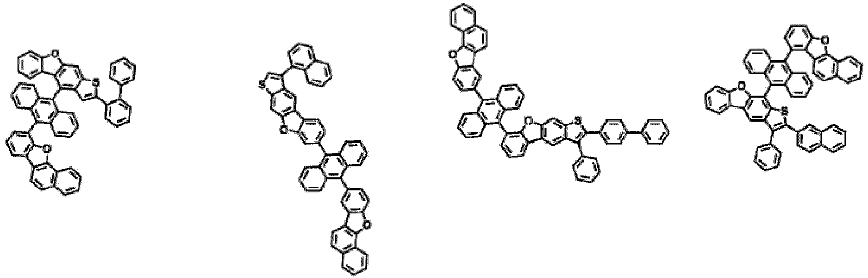

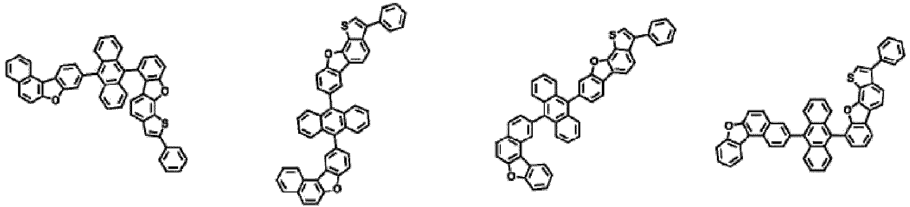

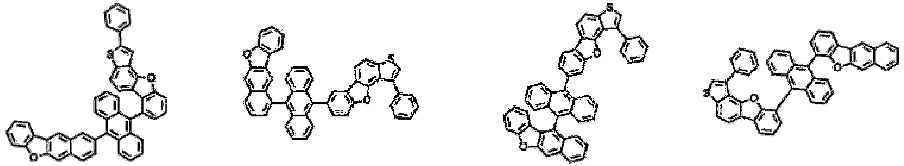

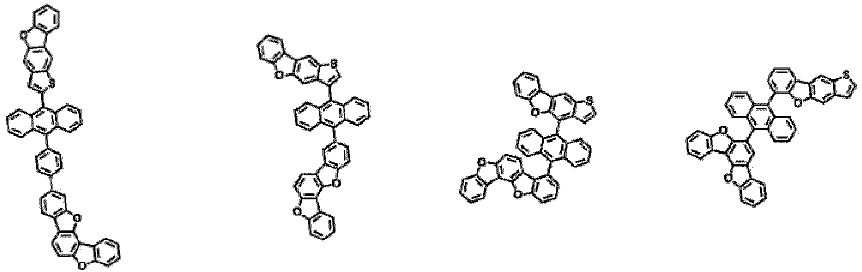

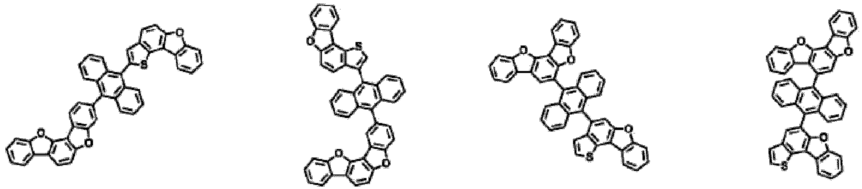

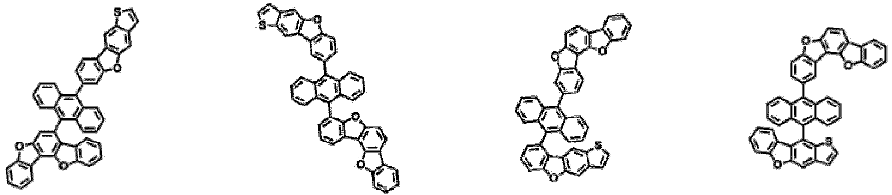

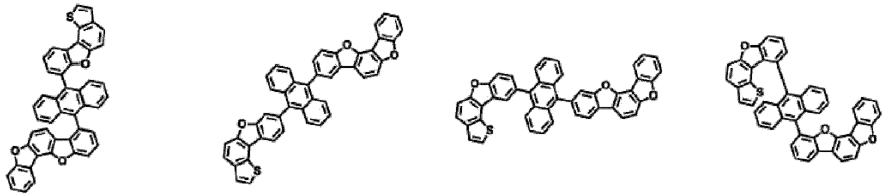

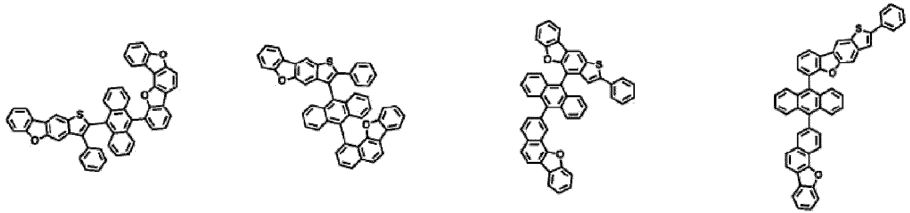

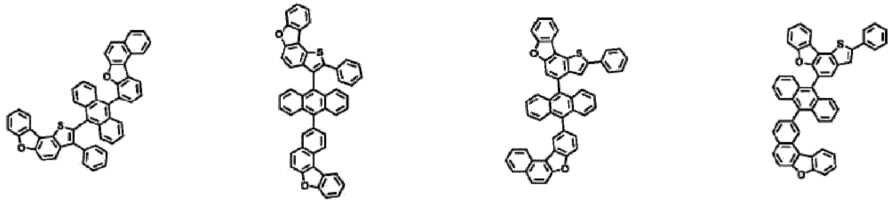

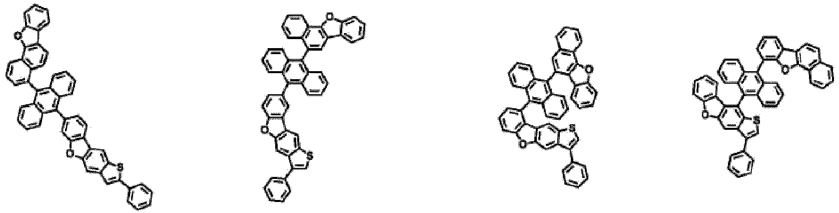

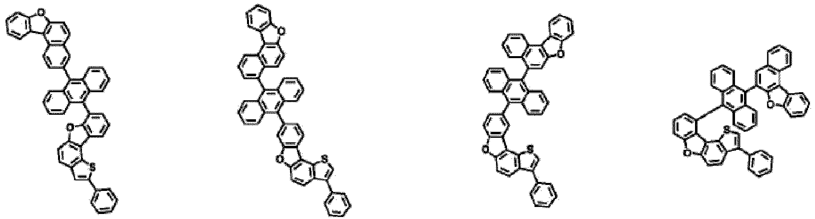

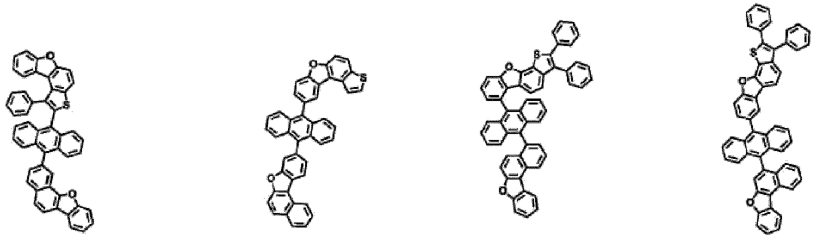

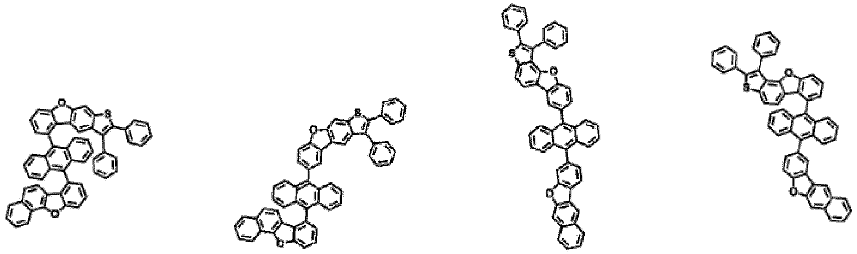

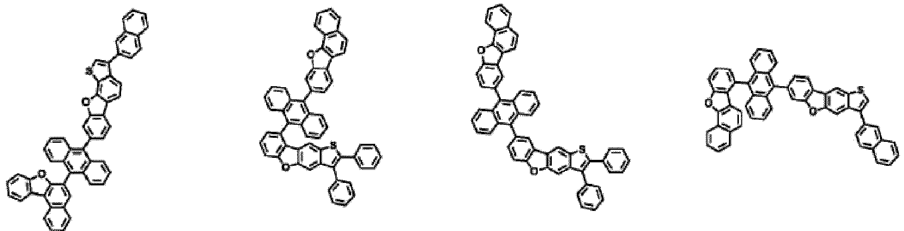

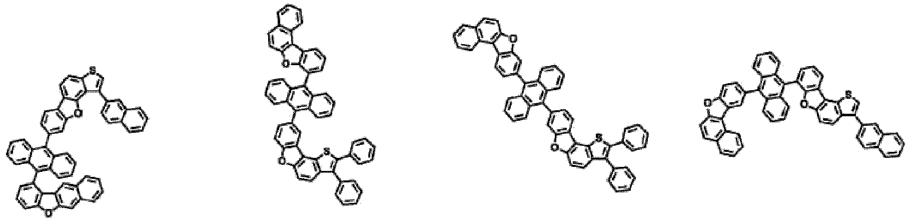

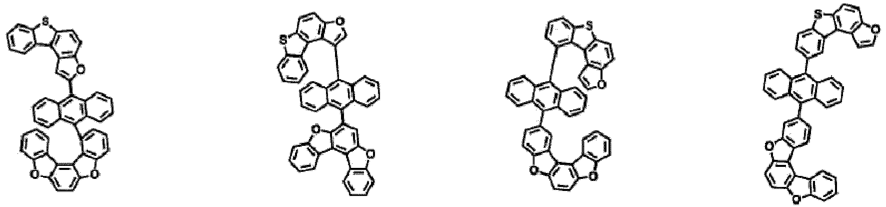

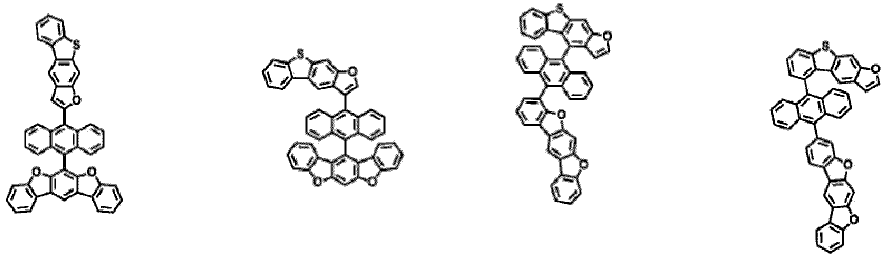

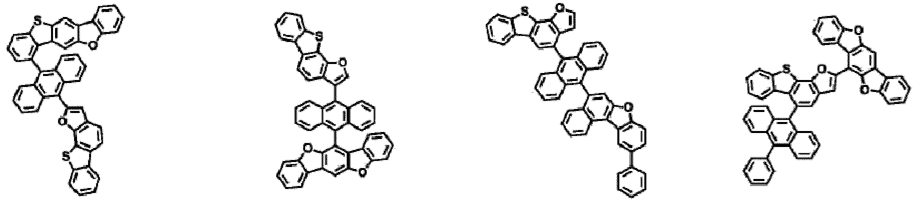

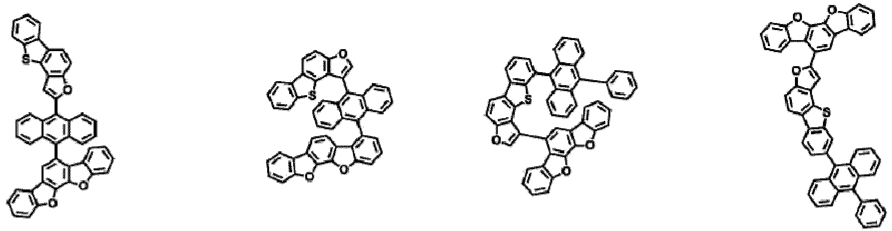

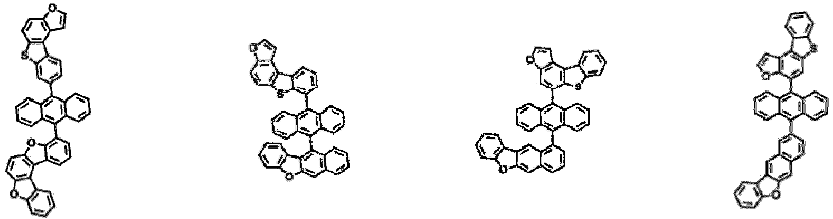

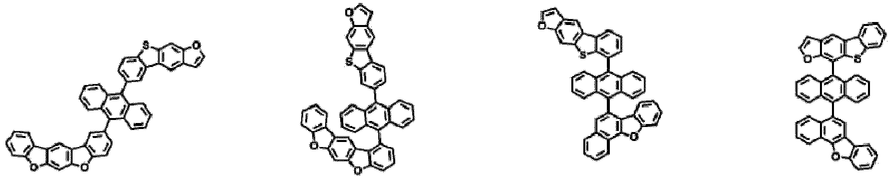

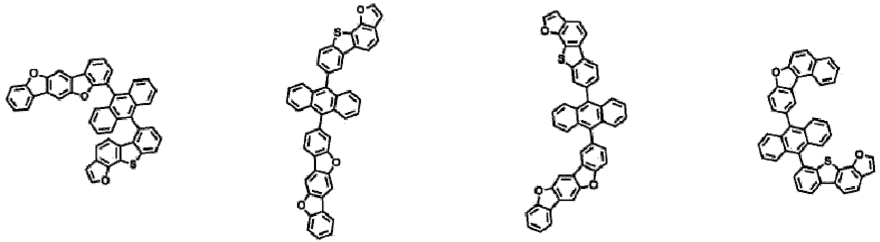

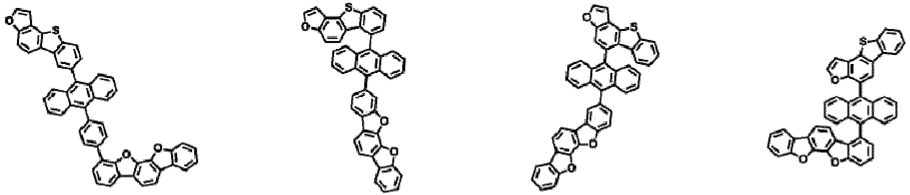

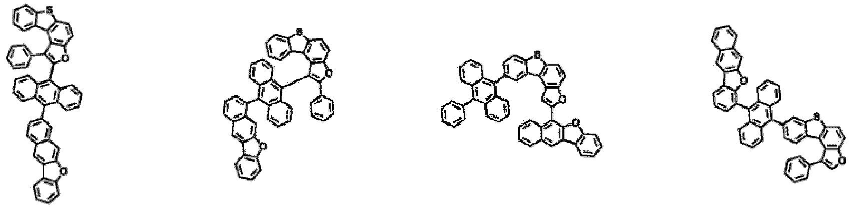

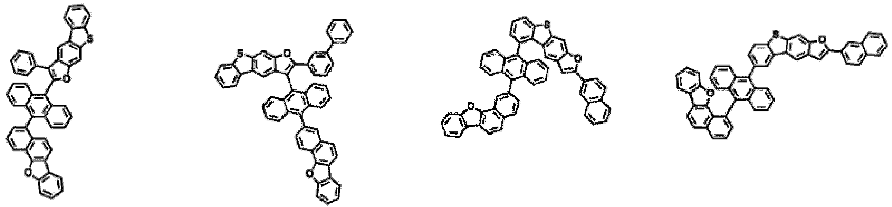

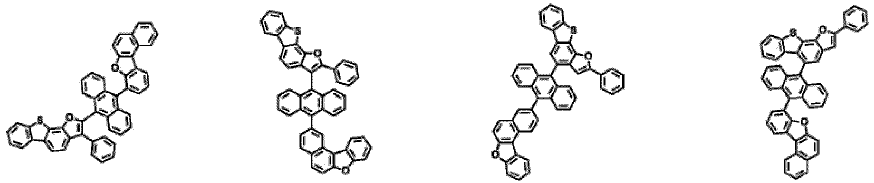

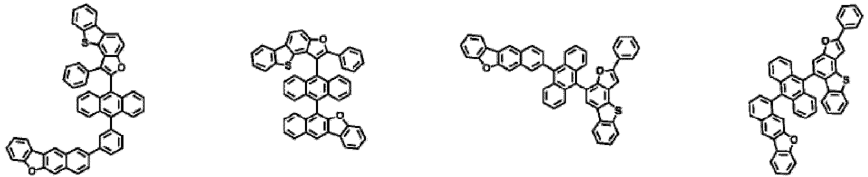

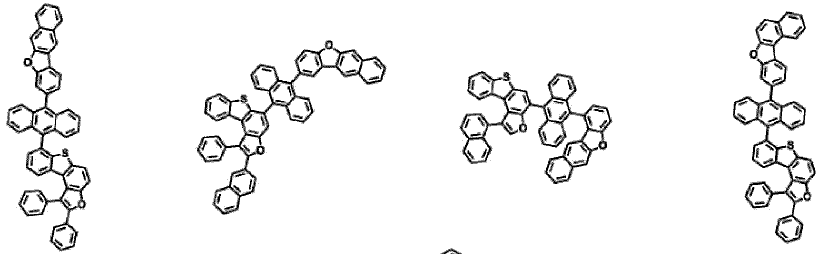

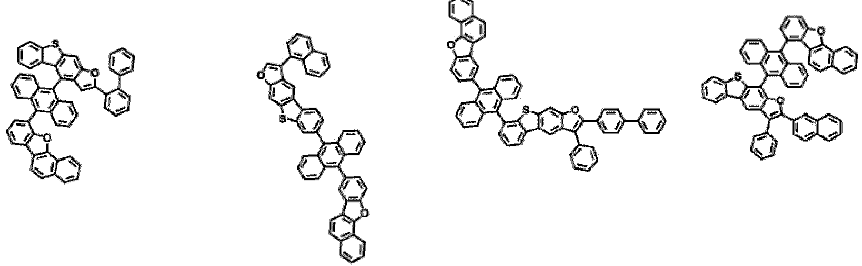

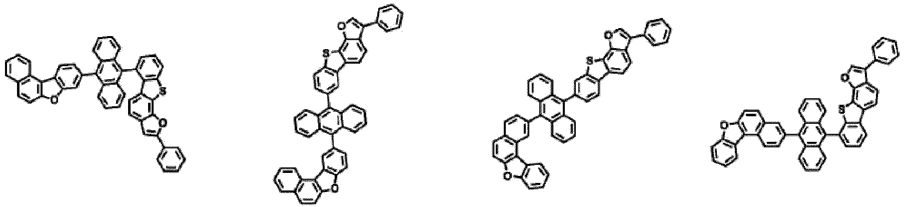

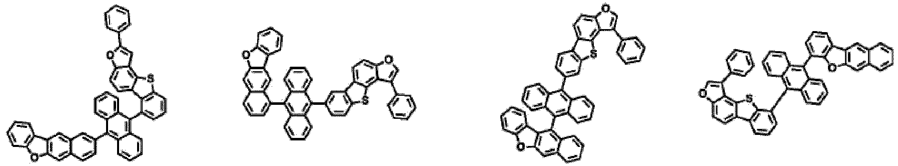

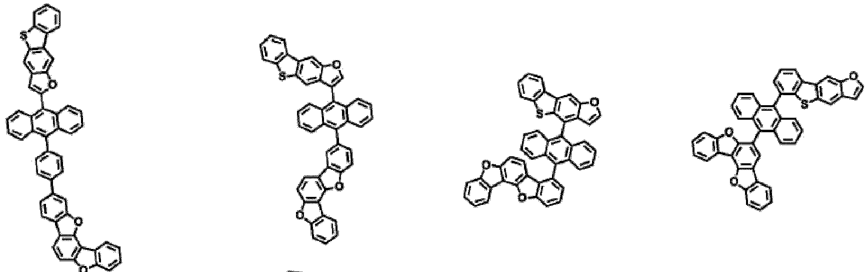

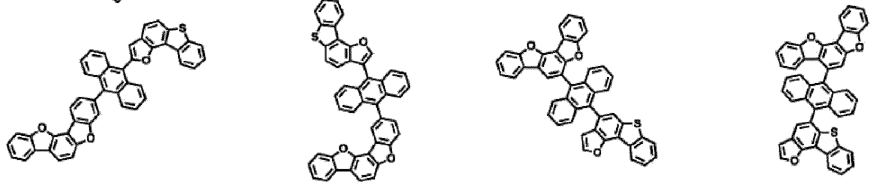

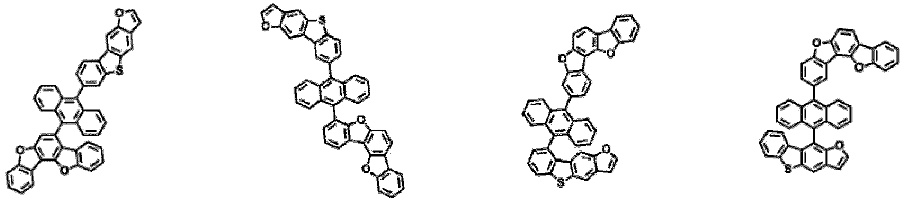

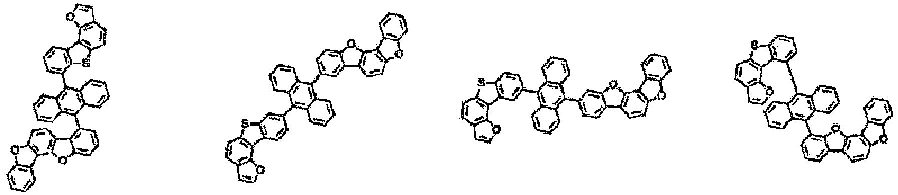

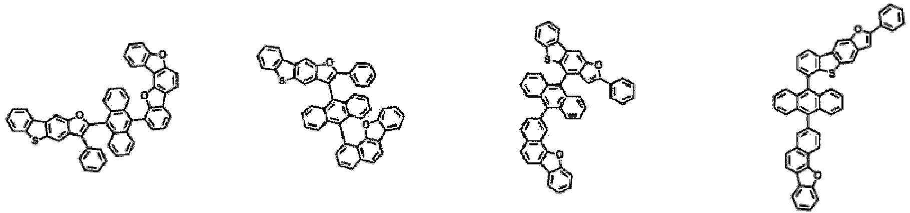

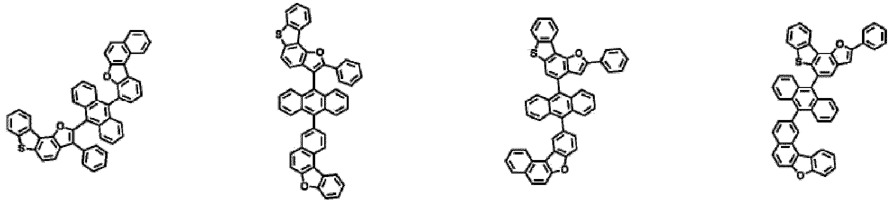

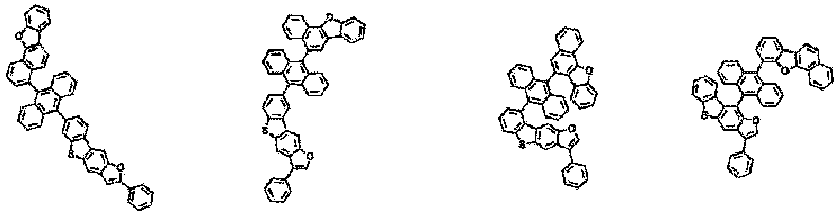

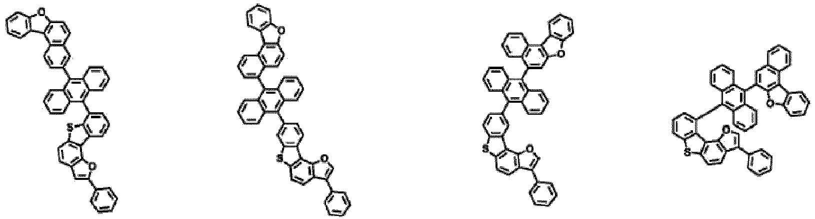

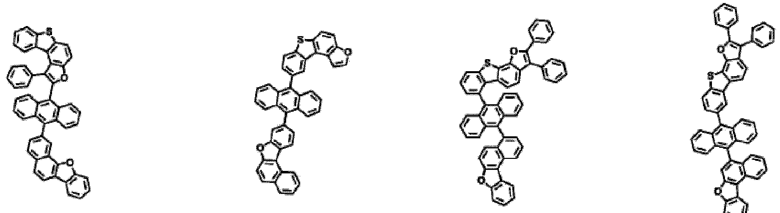

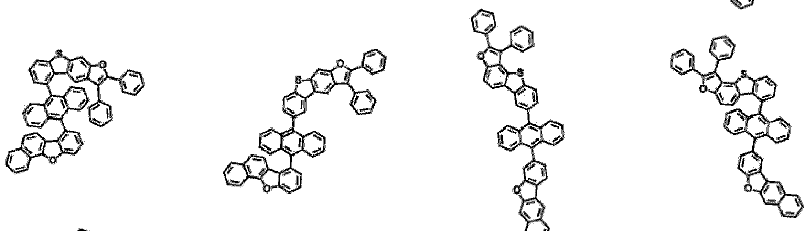

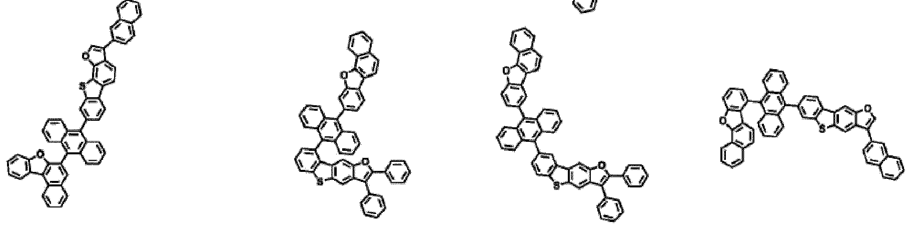

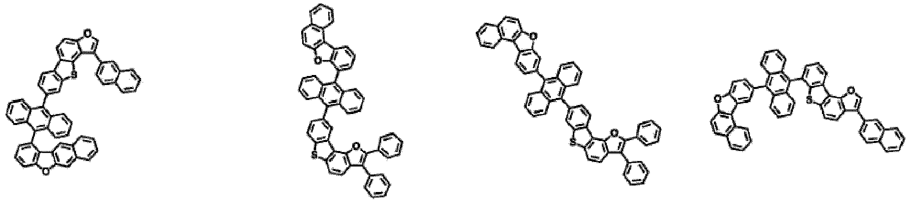

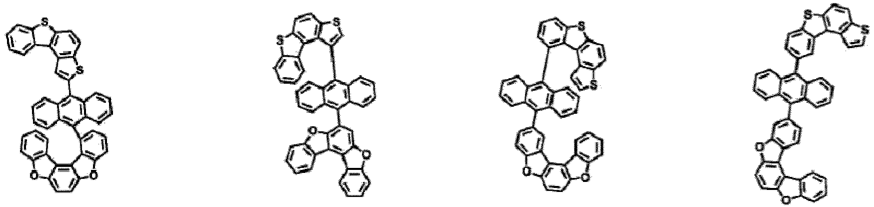

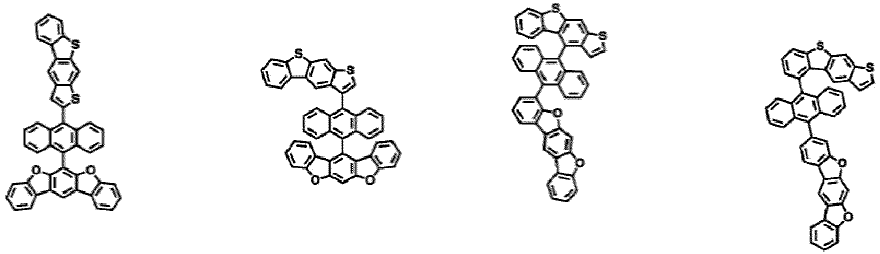

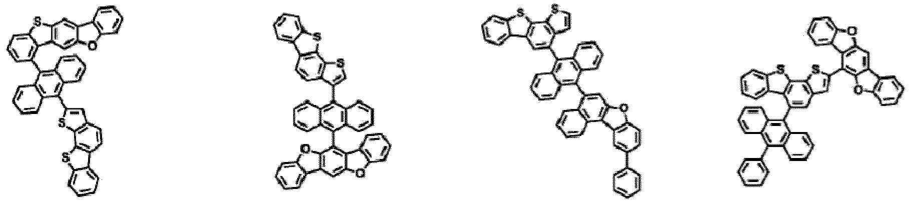

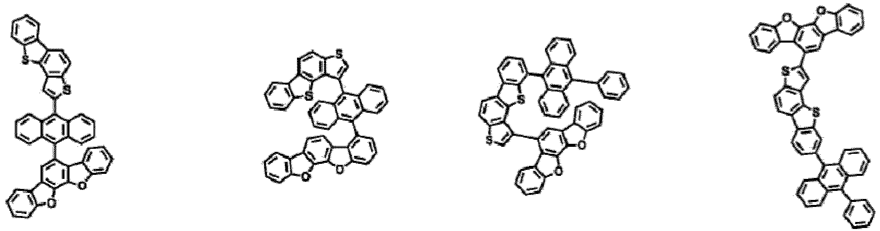

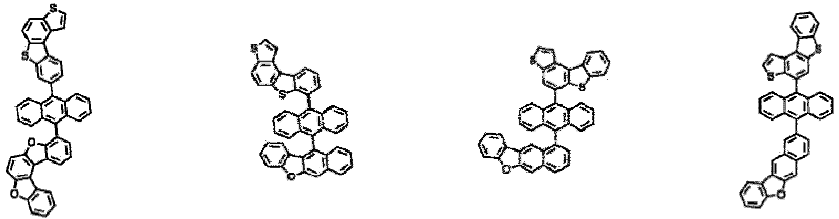

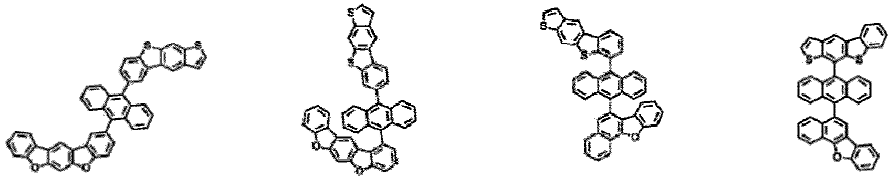

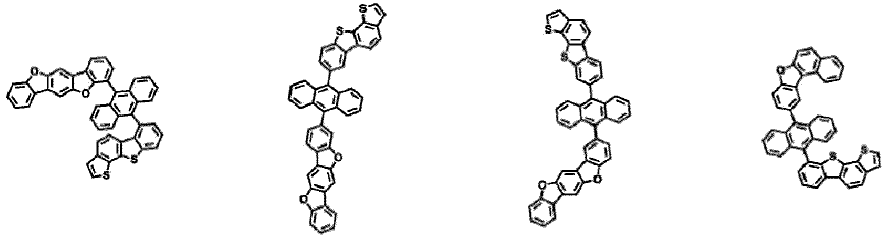

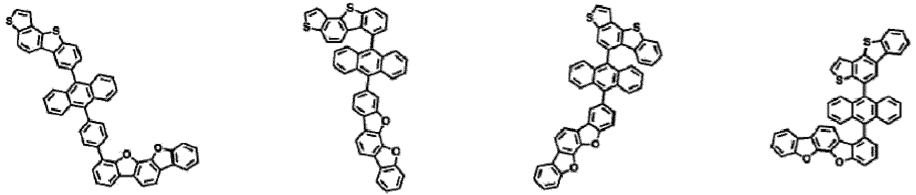

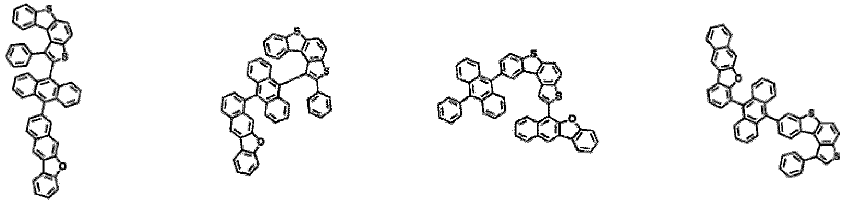

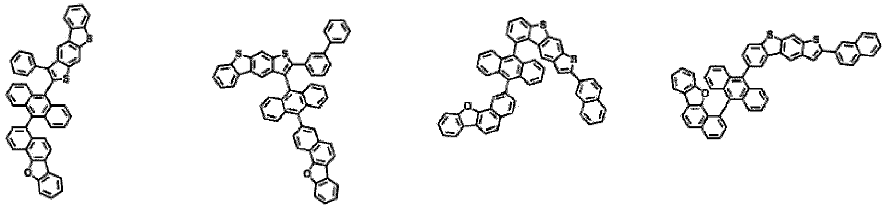

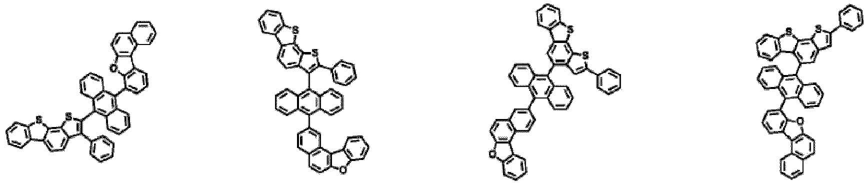

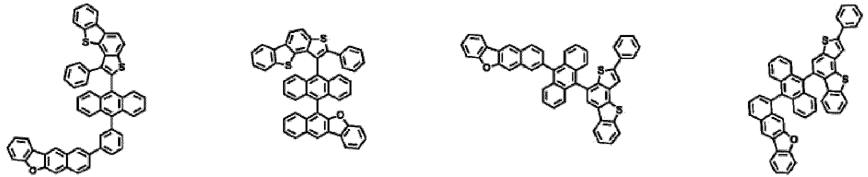

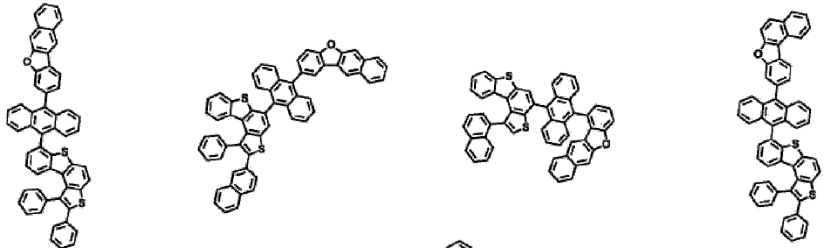

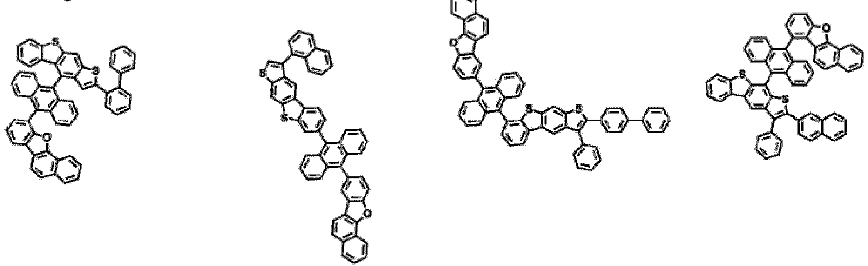

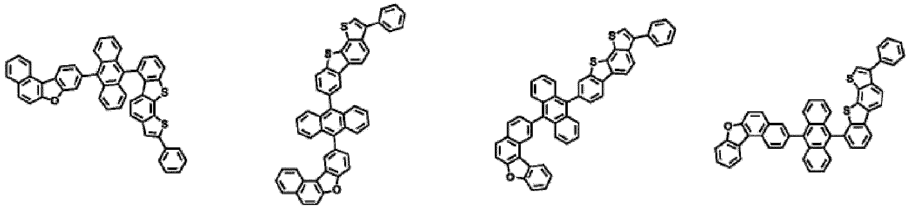

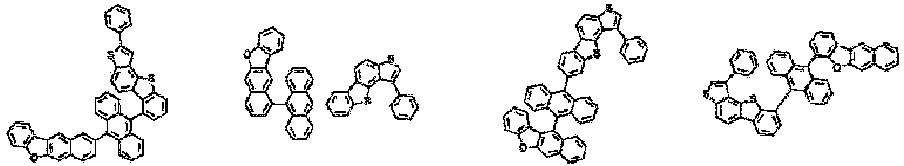

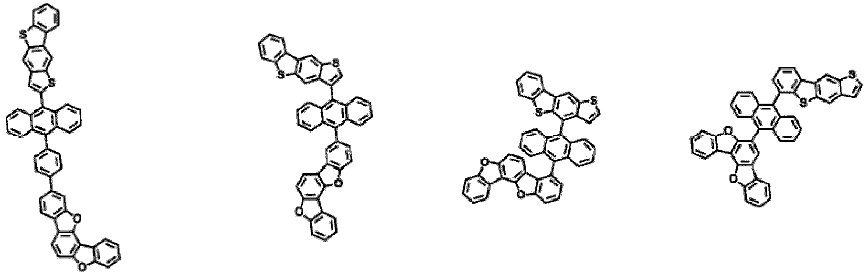

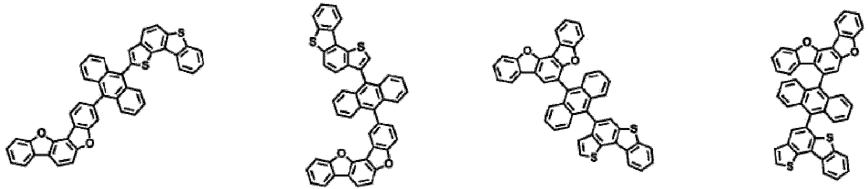

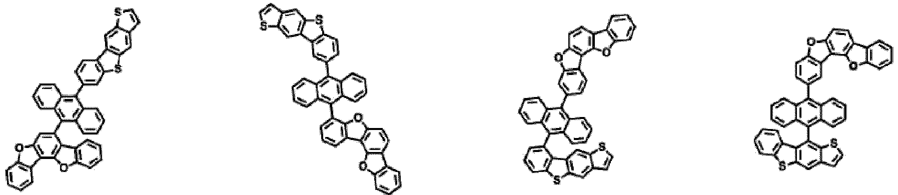

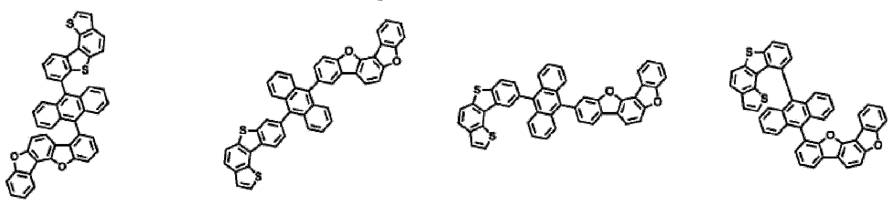

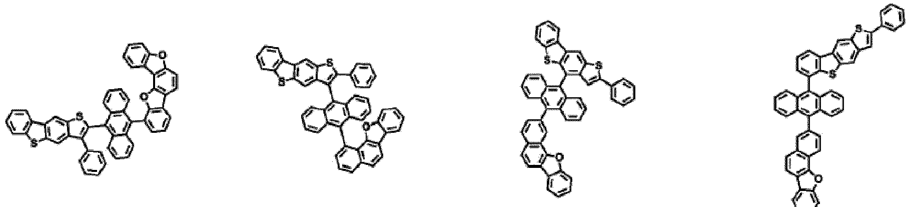

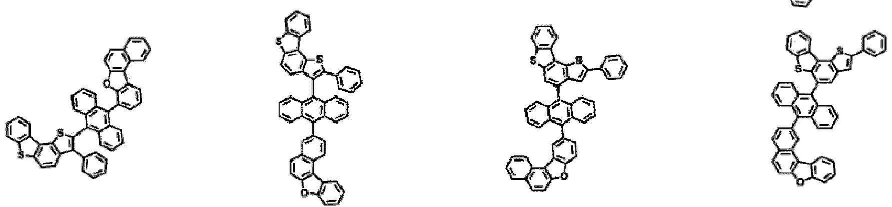

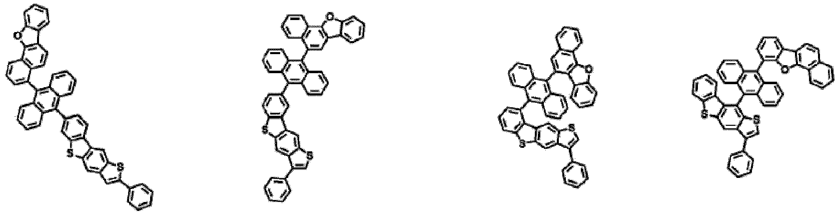

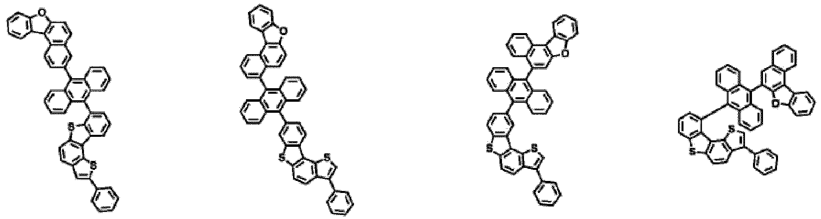

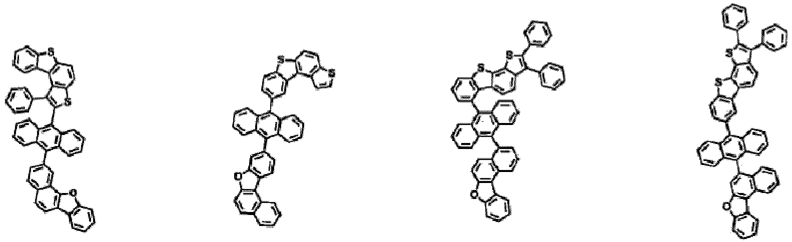

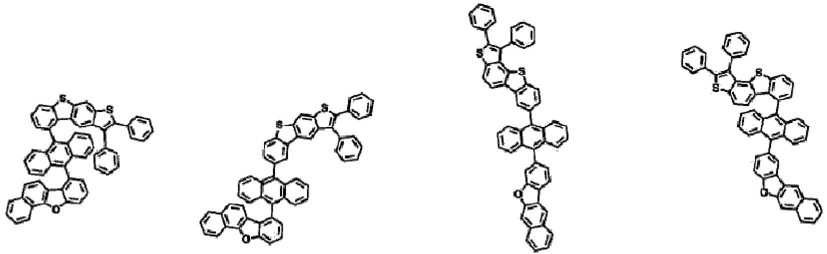

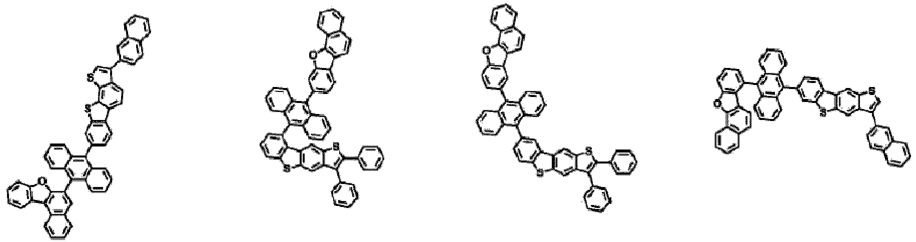

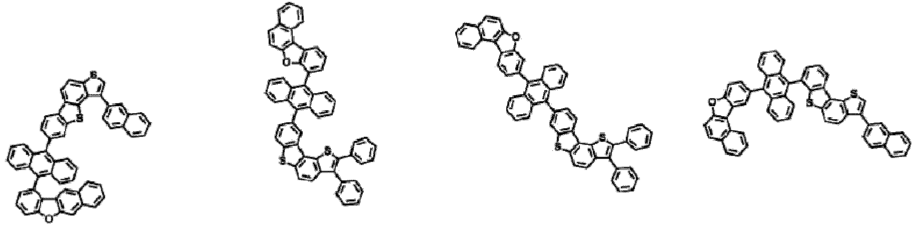

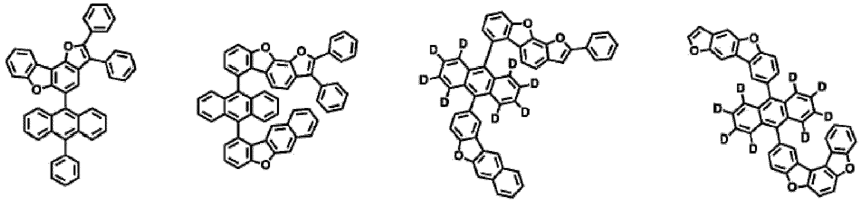

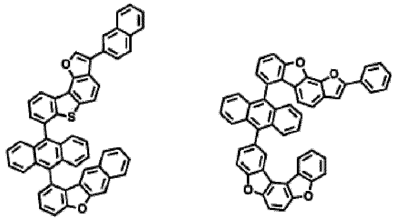

14. Organische lichtemittierende Vorrichtung, umfassend:
eine erste Elektrode;
eine zweite Elektrode, die bereitgestellt ist, um der ersten Elektrode gegenüberzuliegen; und
eine Schicht aus organischem Material, das eine oder mehrere Schichten aufweist, zwischen der ersten Elektrode und der zweiten Elektrode bereitgestellt ist,
wobei eine oder mehrere Schichten der organischen Materialschicht die Verbindung nach einem der Ansprüche 1 bis 13 umfassen.

15. Organische lichtemittierende Vorrichtung nach Anspruch 14, wobei die organische Materialschicht eine lichtemittierende Schicht umfasst und die lichtemittierende Schicht die Verbindung umfasst.

## Revendications

1. Composé de formule chimique 1 : 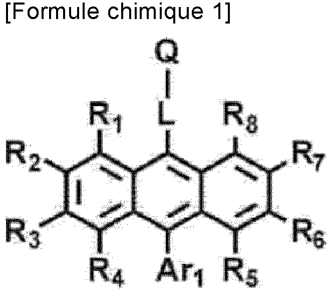 dans lequel, dans la formule chimique 1 :
R₁ à R₈ sont identiques les uns aux autres ou différents les uns des autres, et sont chacun indépendamment de l'hydrogène, du deutérium, un groupe halogène, un groupe alkyle substitué ou non substitué, un groupe cycloalkyle substitué ou non substitué, un groupe aryle substitué ou non substitué ou un groupe hétéroaryle substitué ou non substitué ;
Ar₁ est un groupe aryle substitué ou non substitué ou un groupe hétéroaryle substitué ou non substitué ;
L est une liaison directe ou un groupe arylène substitué ou non substitué ;
Q est l'un quelconque des groupes des formules chimiques 2-1 à 2-6 suivantes, 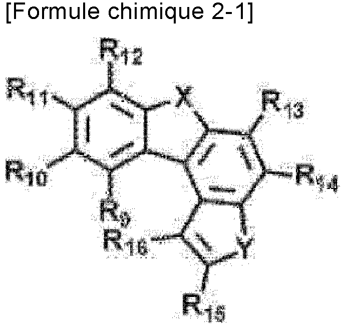 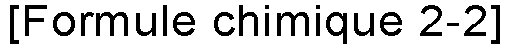 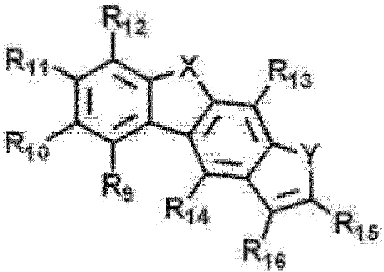 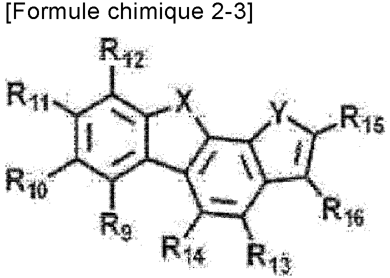 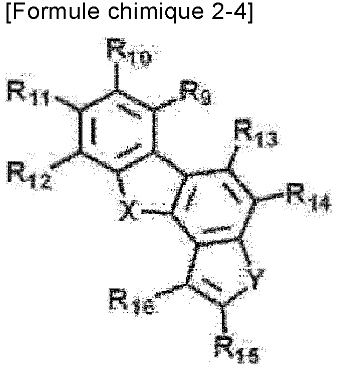 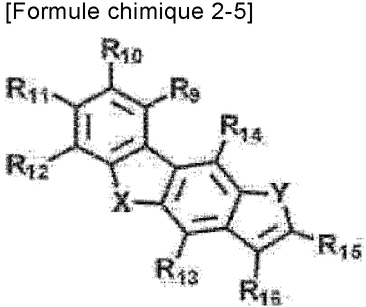 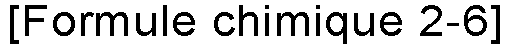 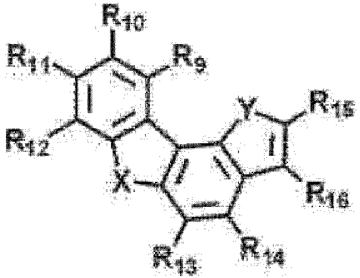
dans lequel, dans les formules chimiques 2-1 à 2-6 :
X et Y sont identiques l'un à l'autre ou différents l'un de l'autre, et sont chacun indépendamment 0 ou S ;
l'un quelconque des R₉ à R₁₆ est lié à L de la formule chimique 1 ; les autres groupes qui ne sont pas liés à L de la formule chimique 1 parmi R₉ à R₁₆ sont identiques les uns aux autres ou différents les uns des autres, et sont chacun indépendamment de l'hydrogène, du deutérium, un halogène, un groupe cyano, un groupe silyle, un groupe alkyle substitué ou non substitué, un groupe cycloalkyle substitué ou non substitué, un groupe aryle substitué ou non substitué ou un groupe hétéroaryle substitué ou non substitué,
à condition que dans la formule chimique 1, lorsque L est une liaison directe, Q est la formule chimique 2-2, X et Y sont tous les deux 0, et l'un quelconque des R₉ à R₁₄ et R₁₆ est lié à L de la formule chimique 1, si l'un quelconque de Ar₁ et R₁₅ est un groupe phényle non substitué, l'autre est un groupe aryle substitué ou non substitué présentant 10 à 60 atomes de carbone, ou un groupe hétéroaryle substitué ou non substitué présentant 2 à 60 atomes de carbone.

2. Composé selon la revendication 1, dans lequel R₁ à R₈ sont identiques les uns aux autres ou différents les uns des autres, et sont chacun indépendamment de l'hydrogène ou du deutérium.

3. Composé selon la revendication 1, dans lequel le Ar₁ est un groupe phényle substitué ou non substitué, un groupe biphényle substitué ou non substitué, un groupe naphtyle substitué ou non substitué, un groupe phénanthrène substitué ou non substitué, un groupe dibenzofurane substitué ou non substitué, un groupe dibenzothiophène substitué ou non substitué ou un groupe carbazole substitué ou non substitué ; et
le terme 'substitué ou non substitué' signifie étant non substitué ou substitué par un ou plusieurs substituants sélectionnés dans le groupe consistant en deutérium, un groupe halogène, un groupe alkyle présentant 1 à 60 atomes de carbone, un groupe aryle présentant 6 à 60 atomes de carbone et un groupe hétéroaryle présentant 2 à 60 atomes de carbone, ou un ou plusieurs substituants adjacents sont liés les uns aux autres pour former un cycle.

4. Composé selon la revendication 1, dans lequel L est une liaison directe ; un groupe phénylène qui est non substitué ou substitué par du deutérium ; ou un groupe naphtylène qui est non substitué ou substitué par du deutérium.

5. Composé selon la revendication 1, dans lequel la formule chimique 2-1 est l'une quelconque des formules chimiques 2-1-A à 2-1-D suivantes : 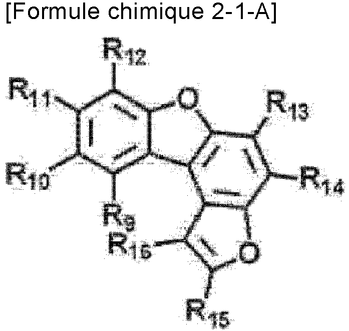 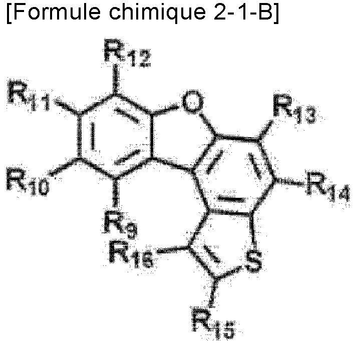 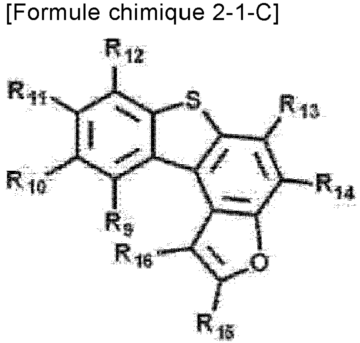 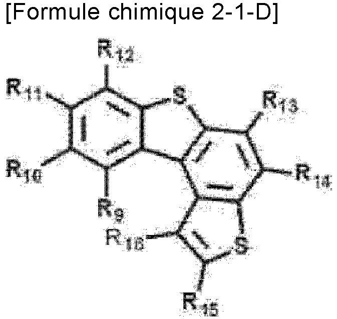 dans lequel, dans les formules chimiques 2-1-A à 2-1-D,
R₉ à R₁₆ sont identiques à ceux définis dans les formules chimiques 2-1 à 2-6.

6. Composé selon la revendication 1, dans lequel la formule chimique 2-2 est l'une quelconque des formules chimiques 2-2-A à 2-2-D suivantes : 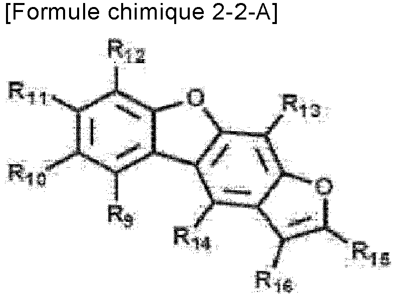 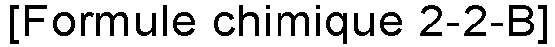 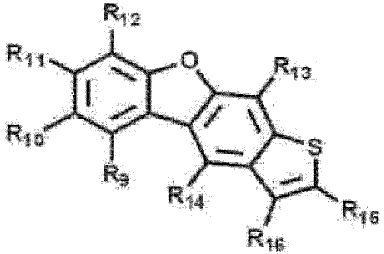 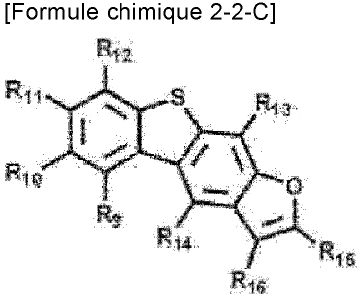 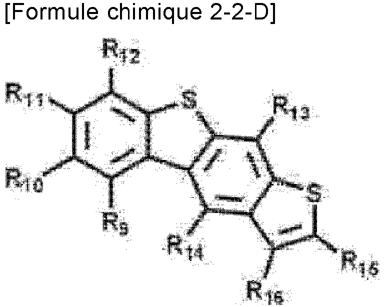 dans lequel, dans les formules chimiques 2-2-A à 2-2-D,
R₉ à R₁₆ sont identiques à ceux définis dans les formules chimiques 2-1 à 2-6.

7. Composé selon la revendication 1, dans lequel la formule chimique 2-3 est l'une quelconque des formules chimiques 2-3-A à 2-3-D suivantes : 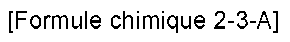 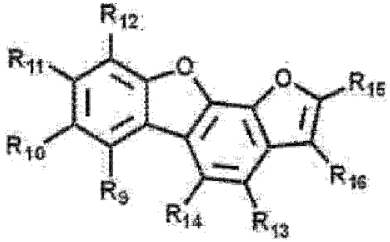 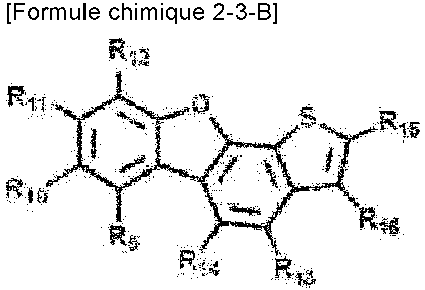 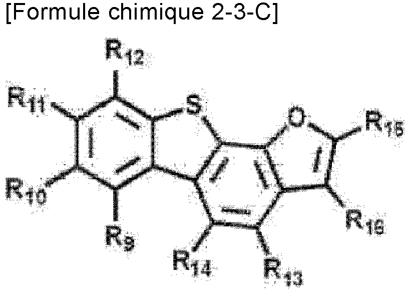 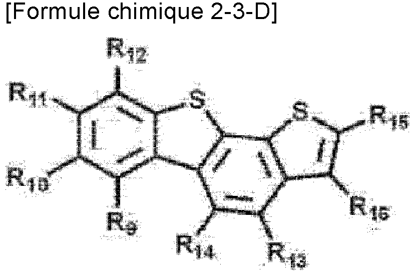 dans lequel, dans les formules chimiques 2-3-A à 2-3-D,
R₉ à R₁₆ sont identiques à ceux définis dans les formules chimiques 2-1 à 2-6.

8. Composé selon la revendication 1, dans lequel la formule chimique 2-4 est l'une quelconque des formules chimiques 2-4-A à 2-4-D suivantes : 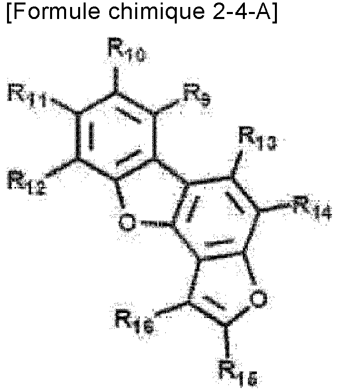 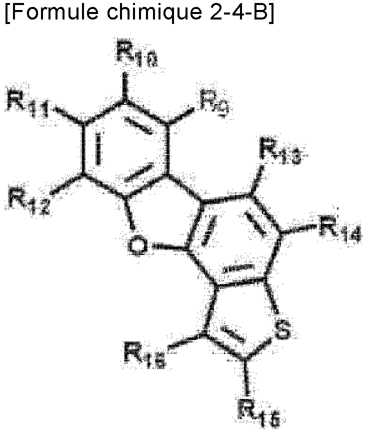 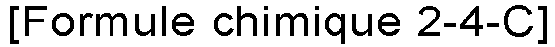 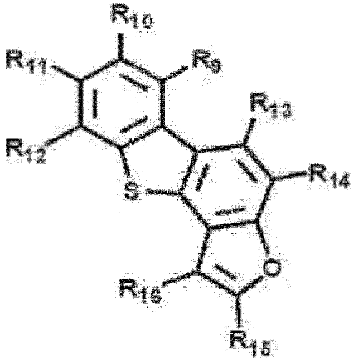 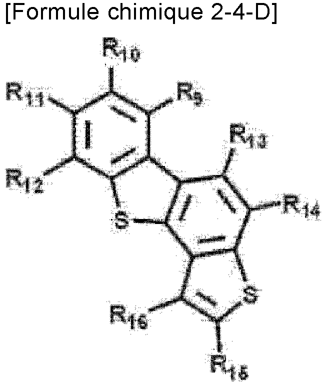 dans lequel, dans les formules chimiques 2-4-A à 2-4-D,
R₉ à R₁₆ sont identiques à ceux définis dans les formules chimiques 2-1 à 2-6.

9. Composé selon la revendication 1, dans lequel la formule chimique 2-5 est l'une quelconque des formules chimiques 2-5-A à 2-5-D suivantes : 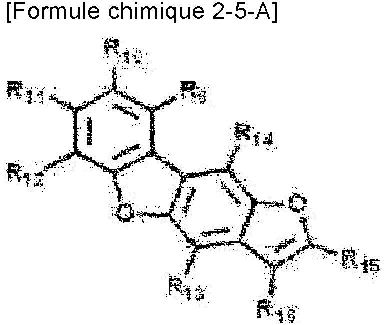 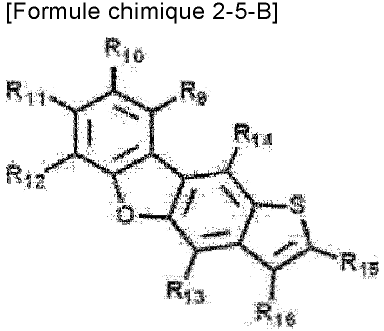 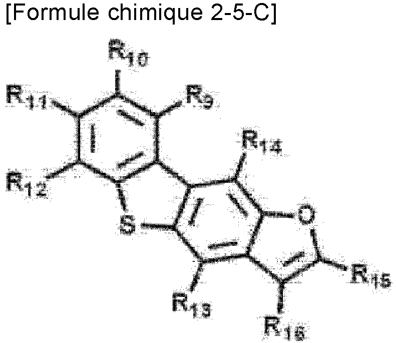 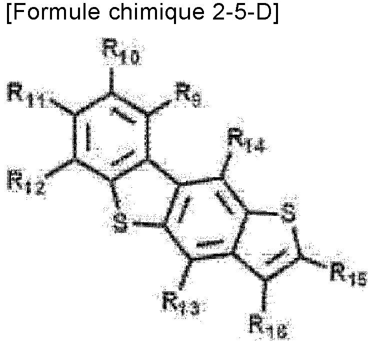 dans lequel, dans les formules chimiques 2-5-A à 2-5-D,
R₉ à R₁₆ sont identiques à ceux définis dans les formules chimiques 2-1 à 2-6.

10. Composé selon la revendication 1, dans lequel la formule chimique 2-6 est l'une quelconque des formules chimiques 2-6-A à 2-6-D suivantes : 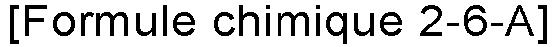 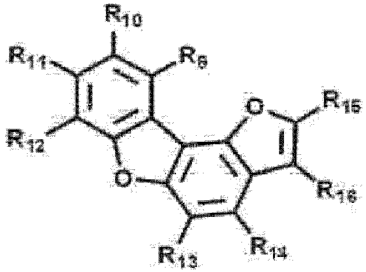 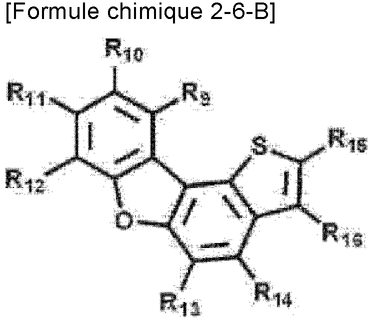 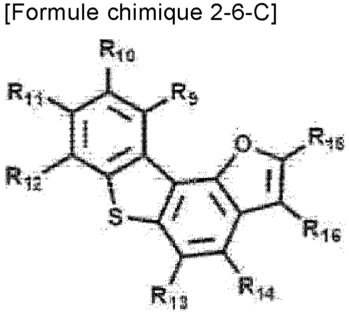 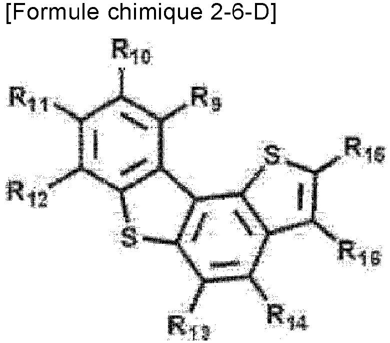 dans lequel, dans les formules chimiques 2-6-A à 2-6-D,
R₉ à R₁₆ sont identiques à ceux définis dans les formules chimiques 2-1 à 2-6.

11. Composé selon la revendication 1, dans lequel, dans la formule chimique 1, lorsque L est une liaison directe, Q est la formule chimique 2-2, X et Y sont tous les deux 0, et l'un quelconque des R₉ à R₁₄ et R16 est lié à L de la formule chimique 1, si l'un quelconque de Ar₁ et R₁₅ est un groupe phényle non substitué, l'autre est un groupe biphényle substitué ou non substitué, un groupe naphtyle substitué ou non substitué, un groupe dibenzofurane substitué ou non substitué ou un groupe dibenzothiophène substitué ou non substitué, et
le terme 'substitué ou non substitué' signifie étant non substitué ou substitué par un ou plusieurs substituants sélectionnés dans le groupe consistant en deutérium, un groupe halogène, un groupe alkyle présentant 1 à 60 atomes de carbone, un groupe aryle présentant 6 à 60 atomes de carbone et un groupe hétéroaryle présentant 2 à 60 atomes de carbone, ou les un ou plusieurs substituants adjacents sont liés les uns aux autres pour former un cycle.

12. Composé selon la revendication 1, dans lequel les autres groupes qui ne sont pas liés à L de la formule chimique 1 parmi R₉ à R₁₆ sont identiques les uns aux autres ou différents les uns des autres, et sont chacun indépendamment de l'hydrogène, du deutérium, un groupe phényle substitué ou non substitué, un groupe biphényle substitué ou non substitué, un groupe naphtyle substitué ou non substitué, un groupe dibenzofurane substitué ou non substitué ou un groupe dibenzothiophène substitué ou non substitué, et
le terme 'substitué ou non substitué' signifie étant substitué par un ou plusieurs substituants sélectionnés dans le groupe consistant en deutérium, un groupe halogène, un groupe alkyle présentant 1 à 60 atomes de carbone ; un groupe aryle présentant 6 à 60 atomes de carbone ; et un groupe hétéroaryle présentant 2 à 60 atomes de carbone, ou les un ou plusieurs substituants adjacents sont liés les uns aux autres pour former un cycle.

13. Composé selon la revendication 1, dans lequel le composé de la formule chimique 1 est l'un quelconque des composés suivants :

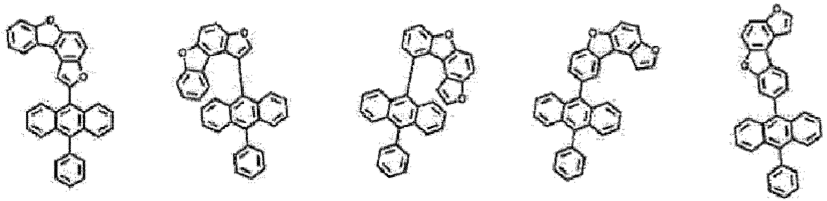

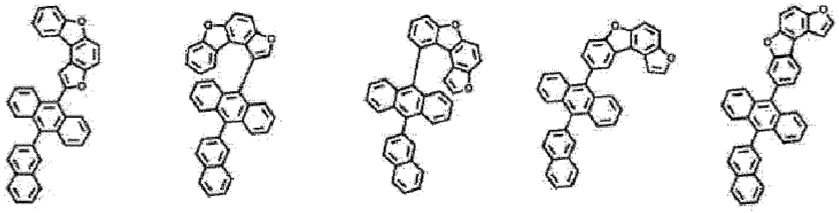

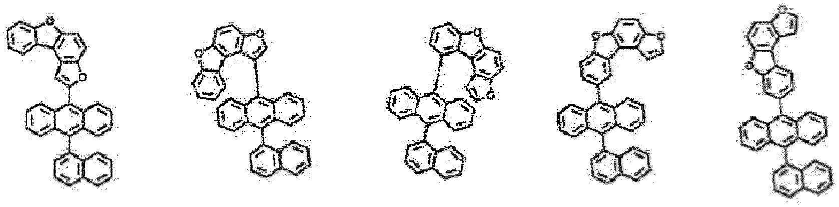

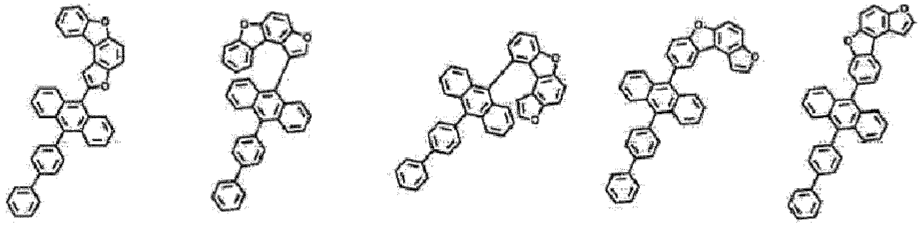

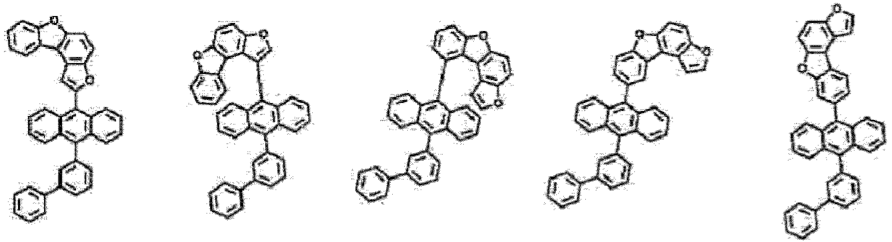

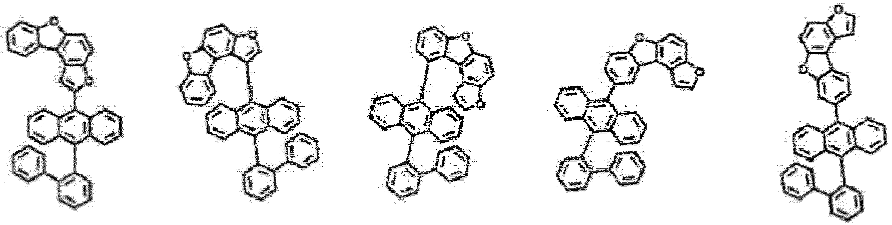

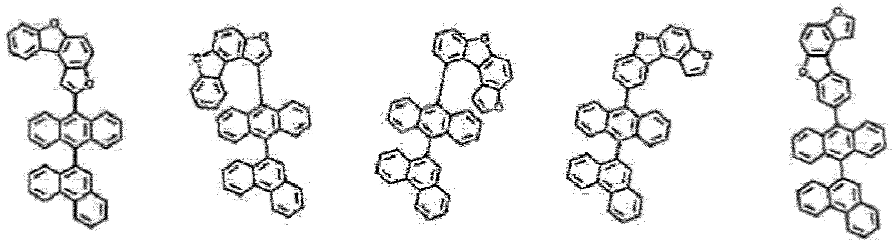

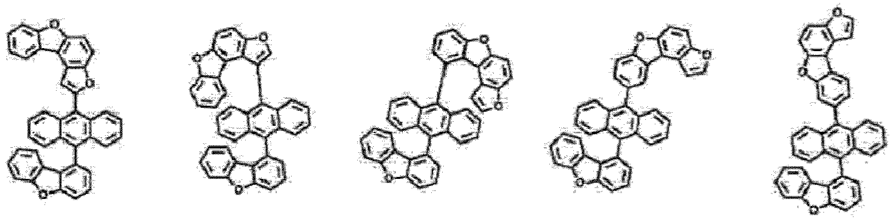

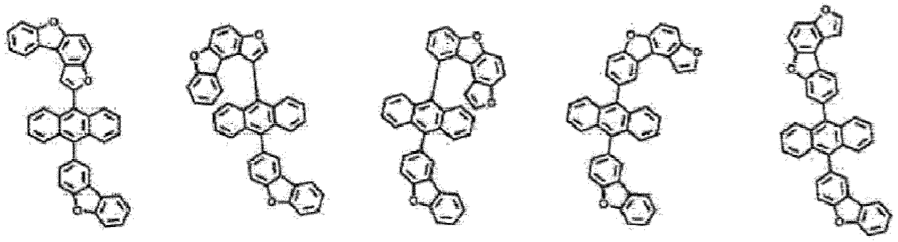

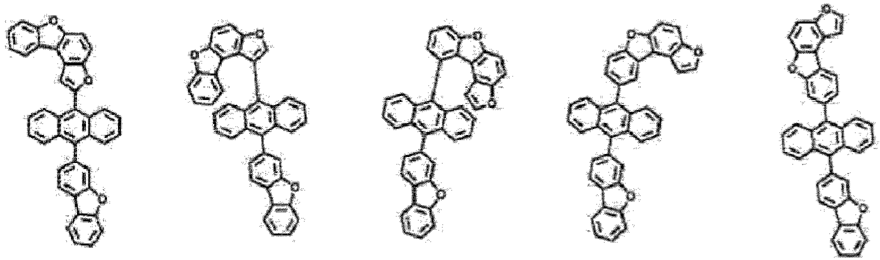

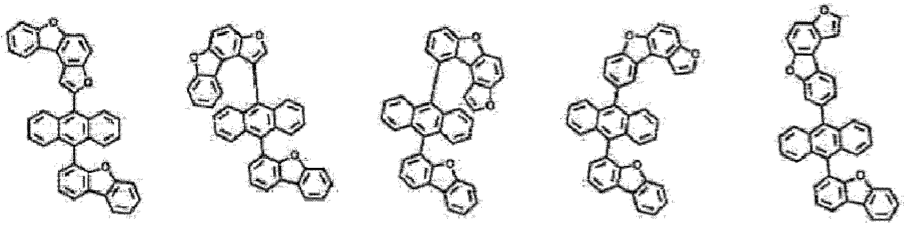

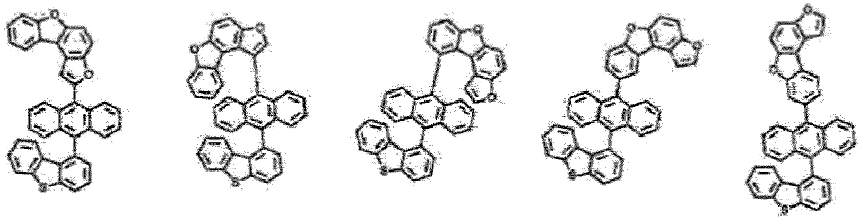

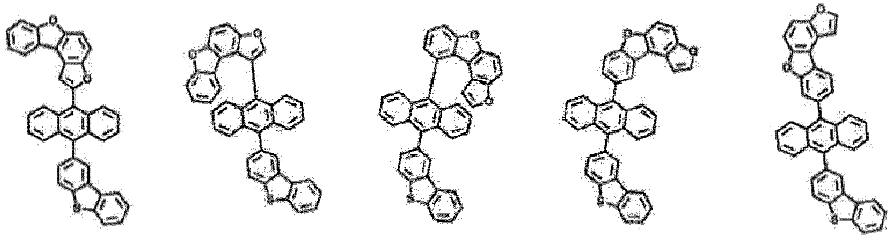

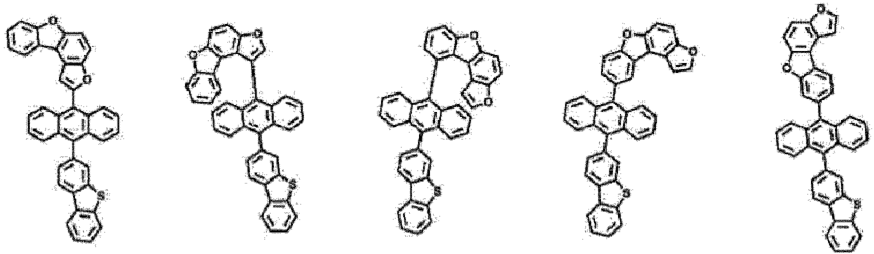

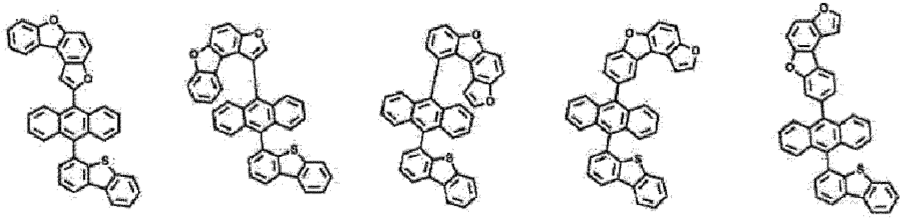

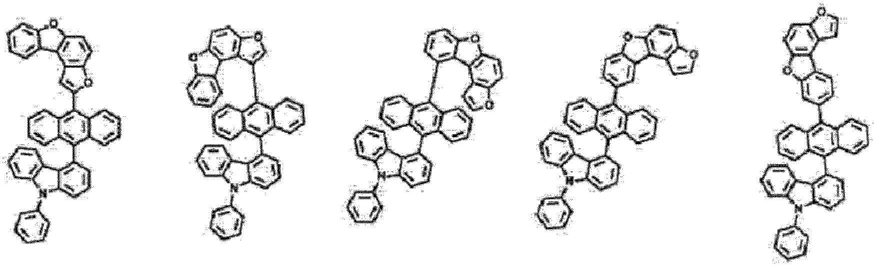

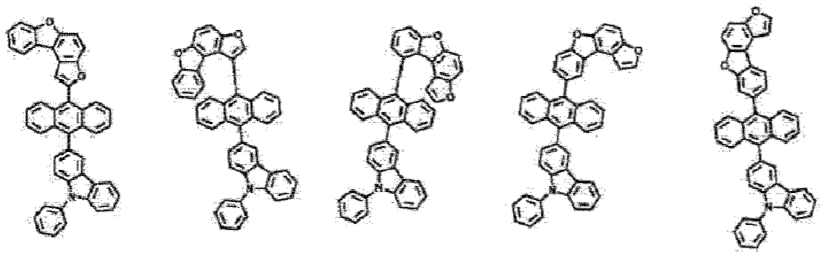

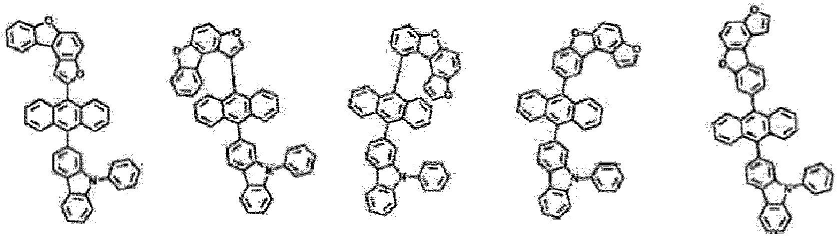

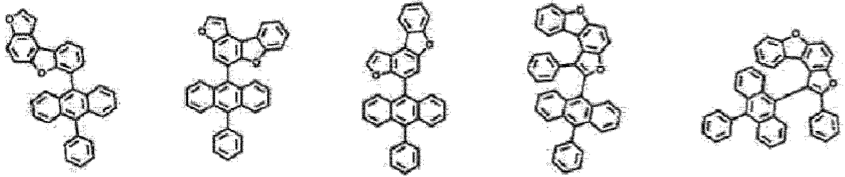

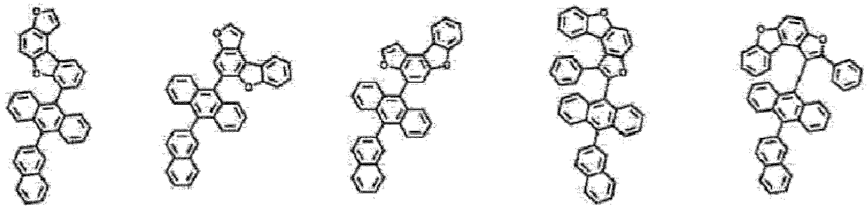

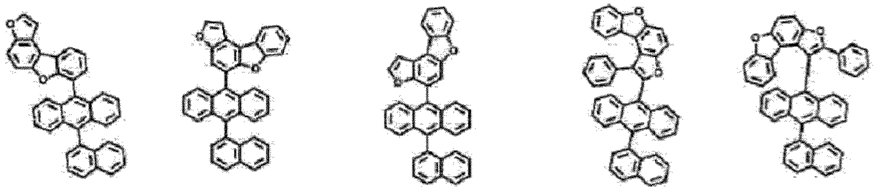

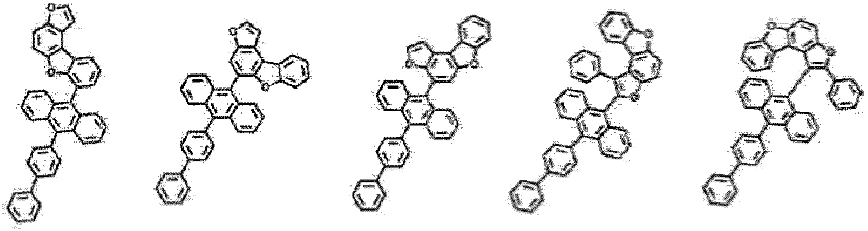

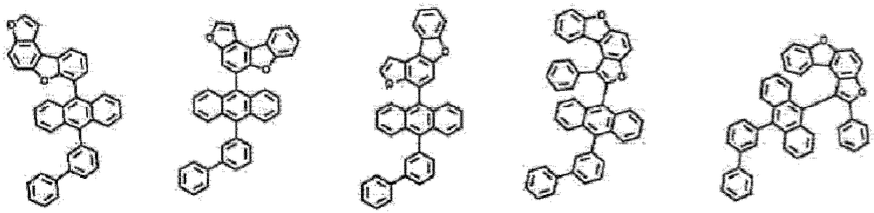

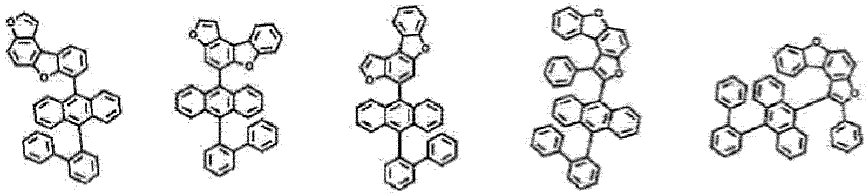

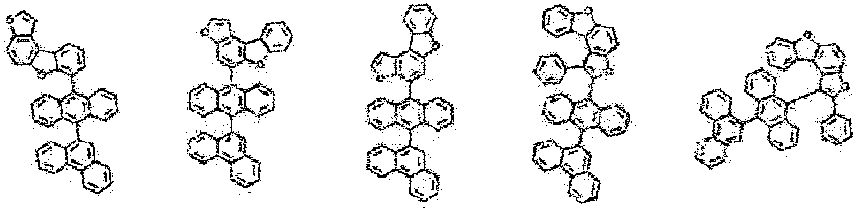

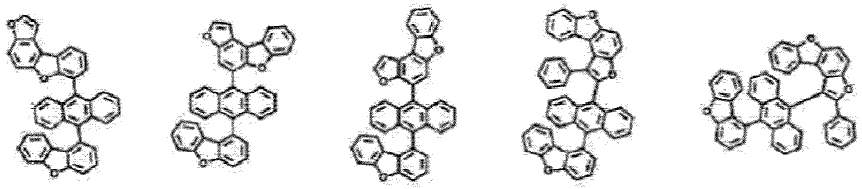

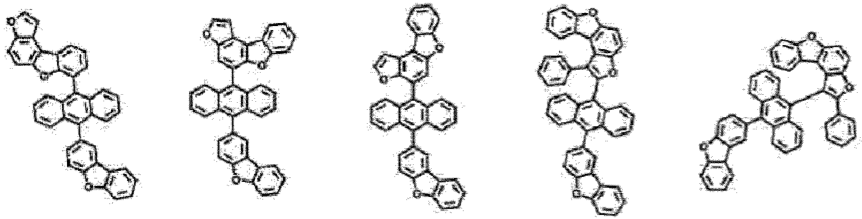

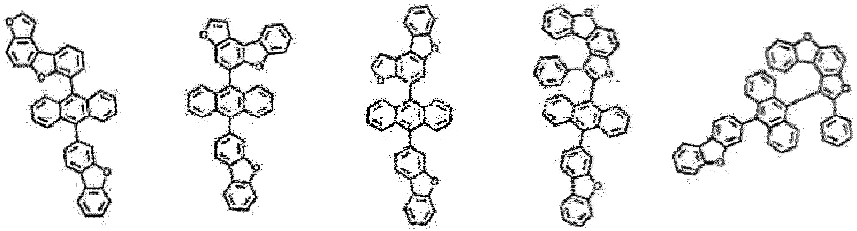

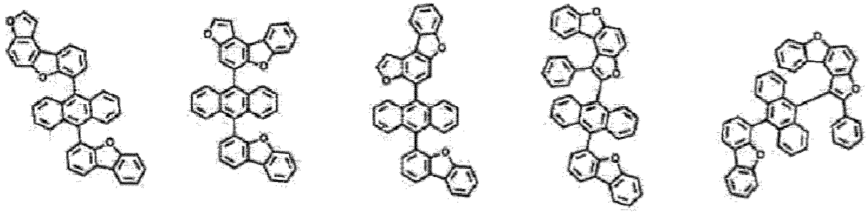

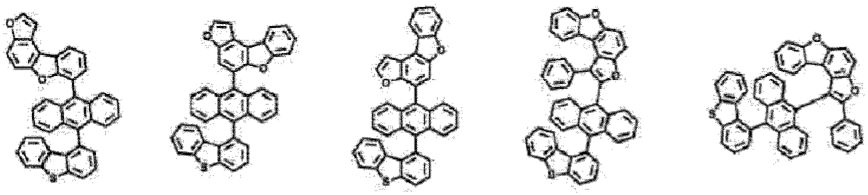

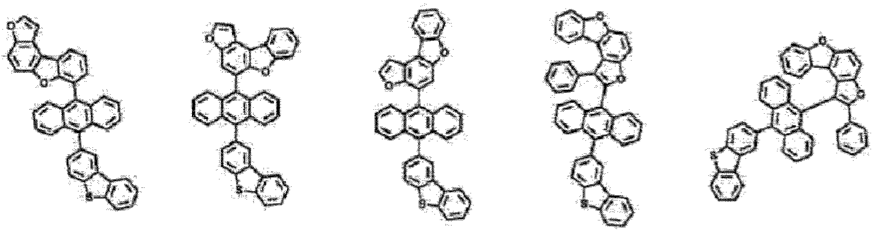

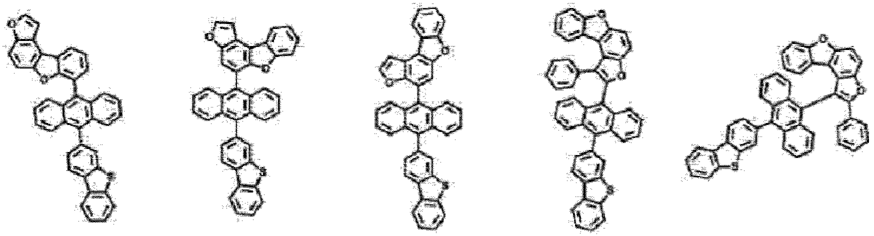

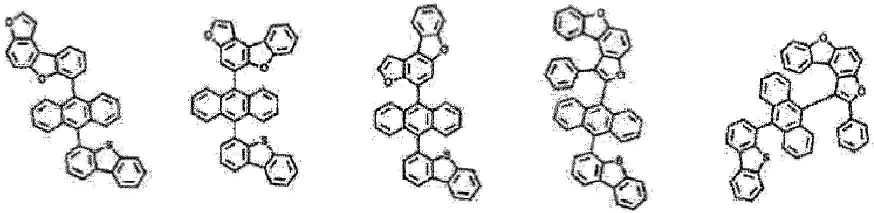

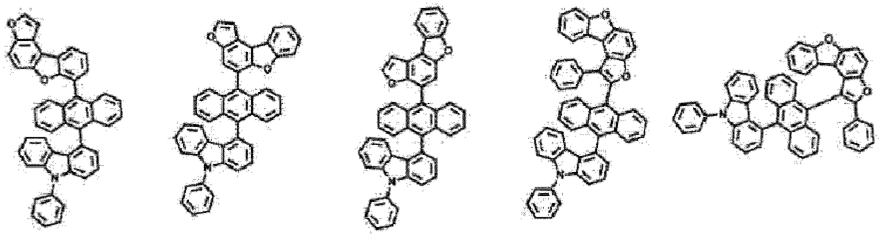

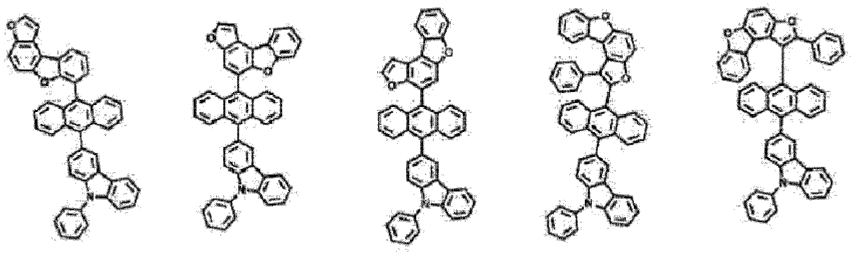

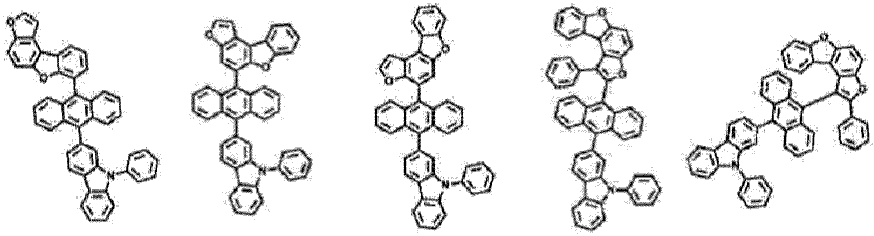

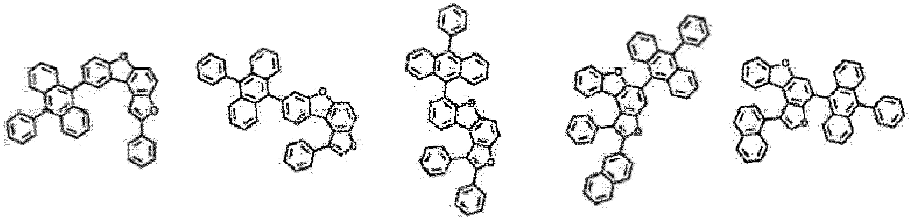

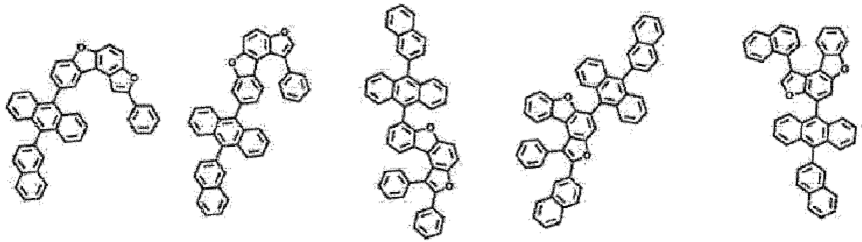

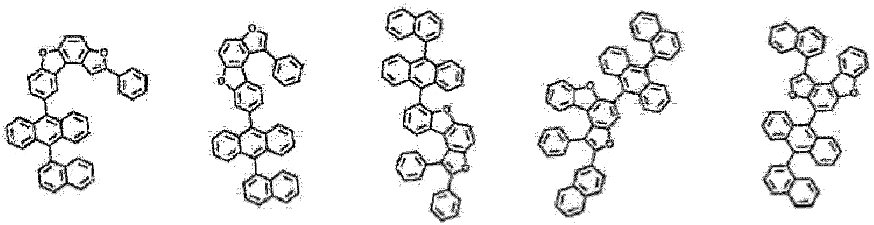

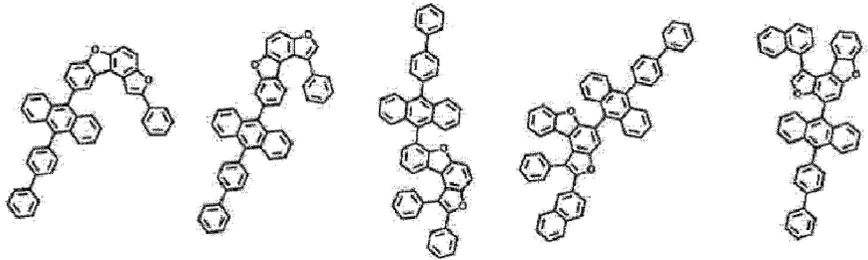

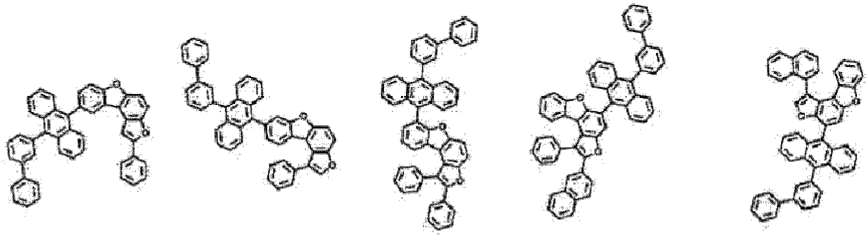

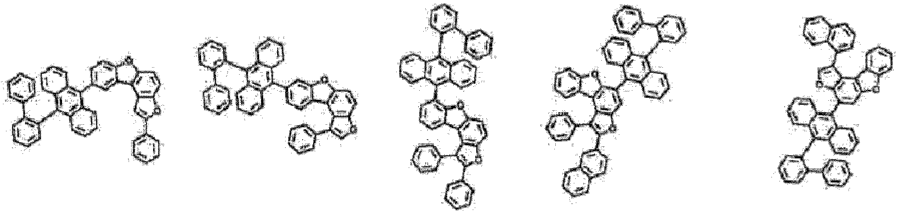

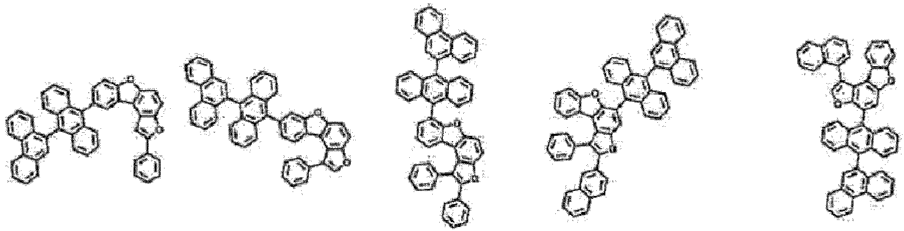

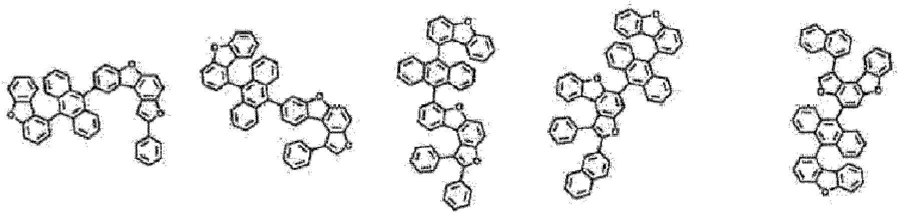

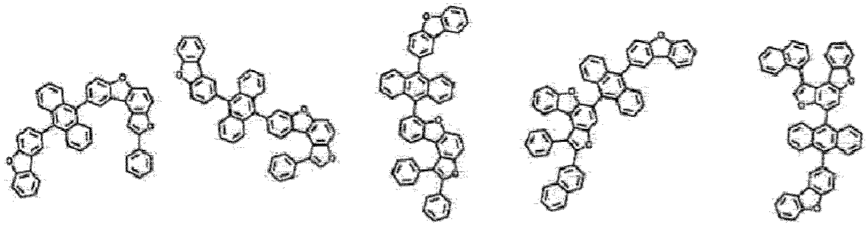

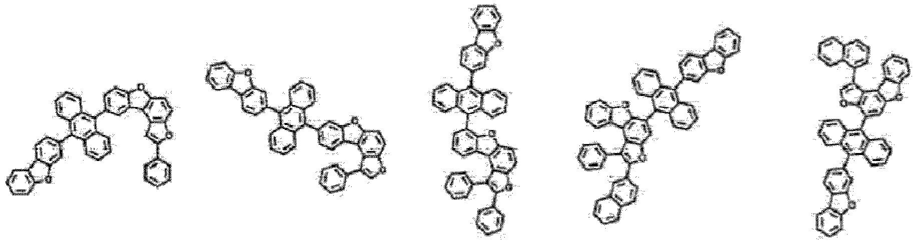

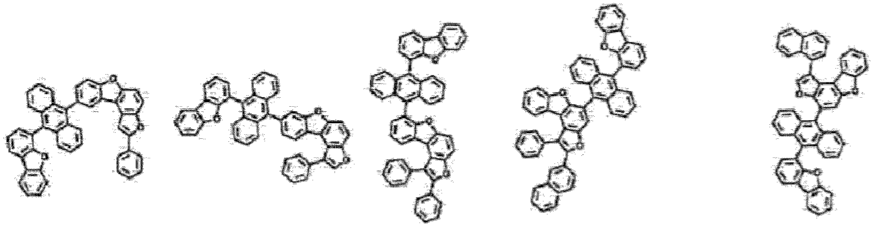

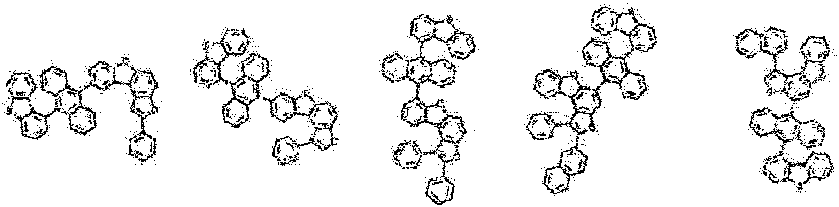

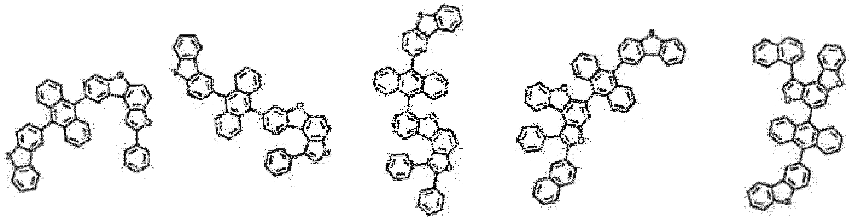

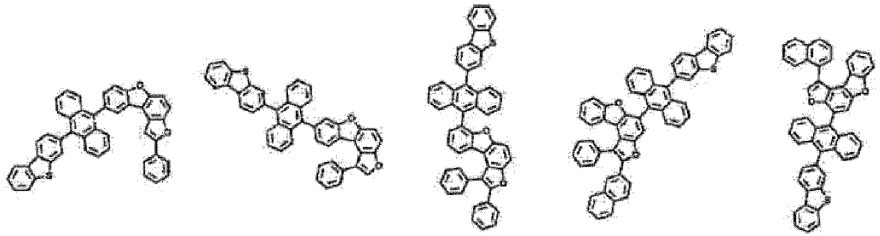

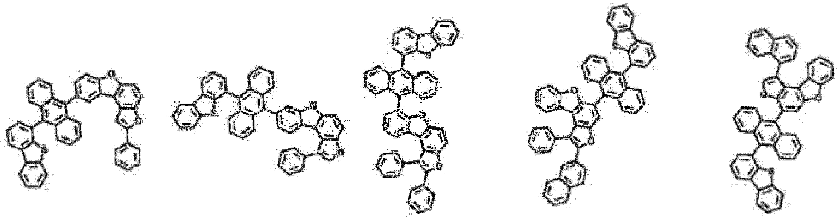

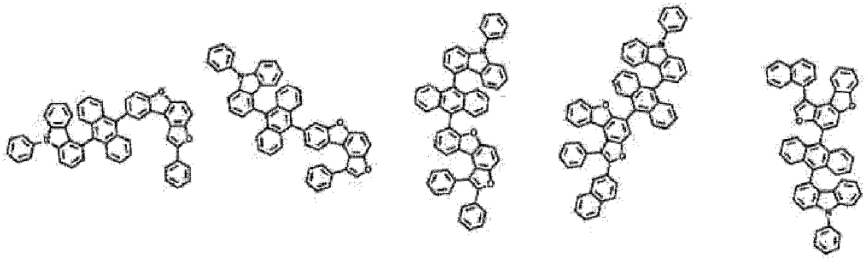

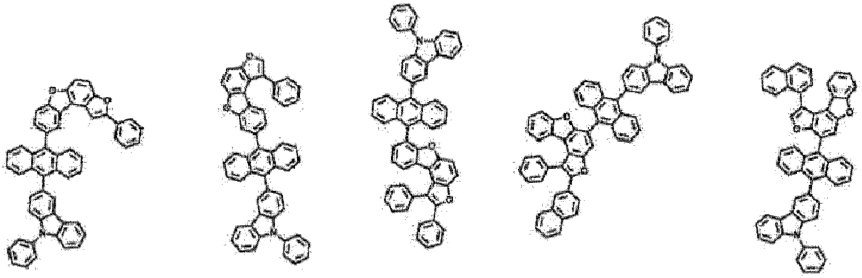

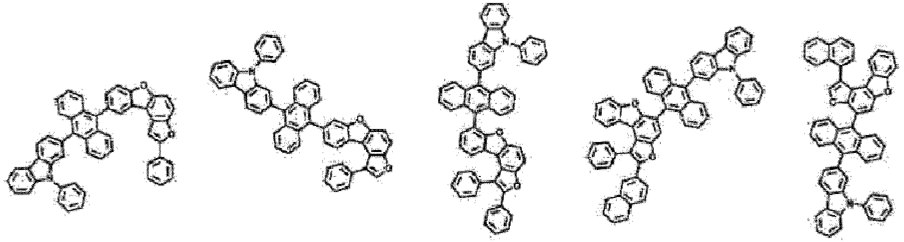

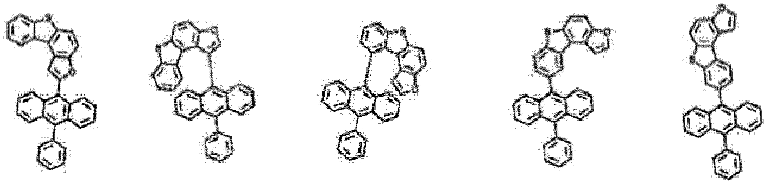

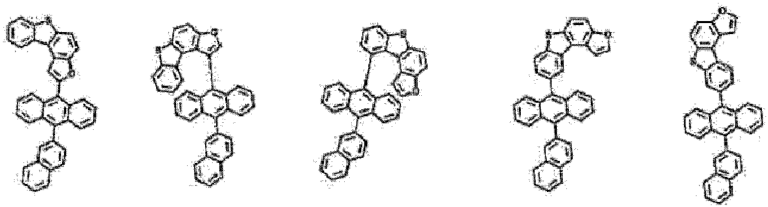

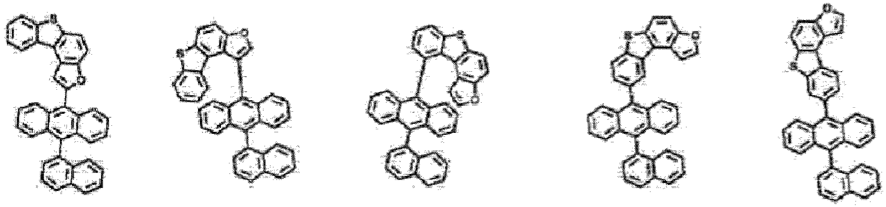

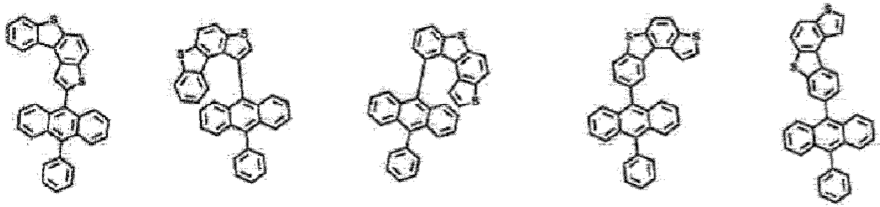

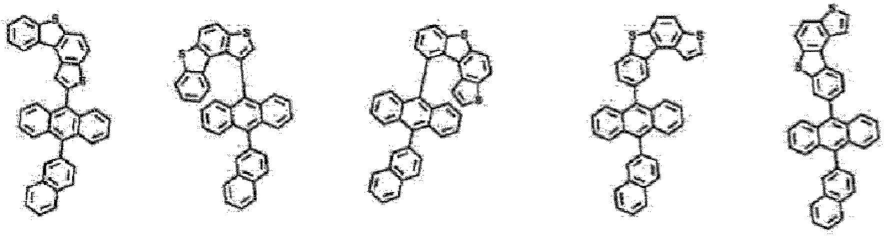

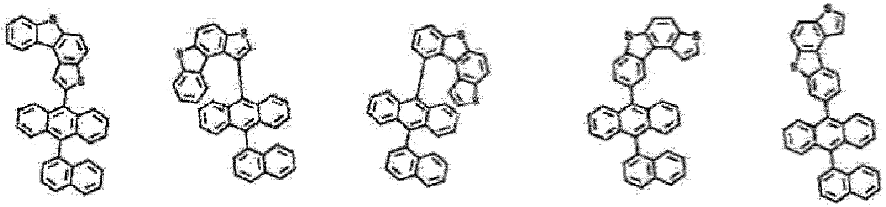

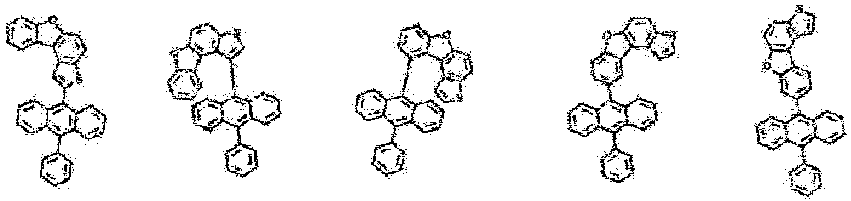

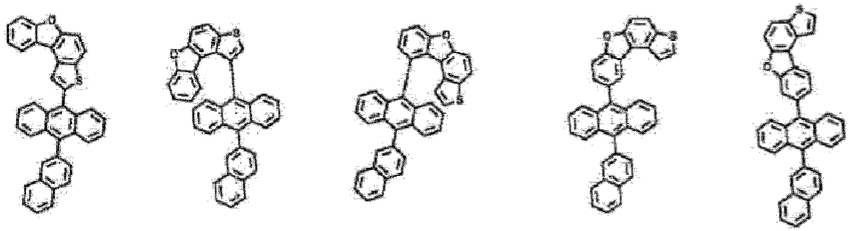

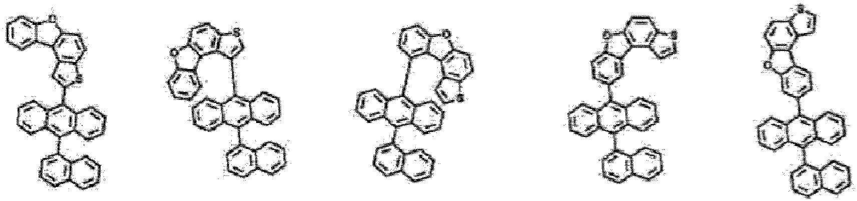

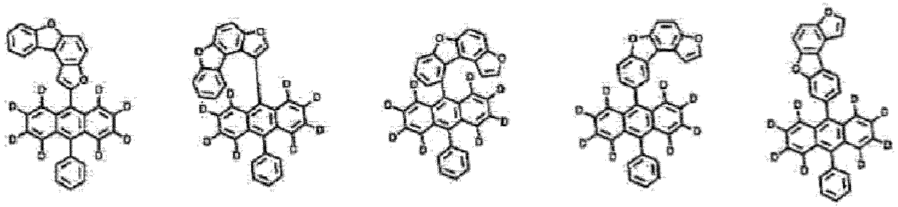

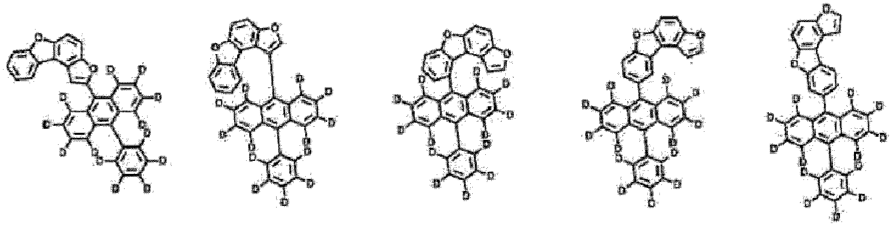

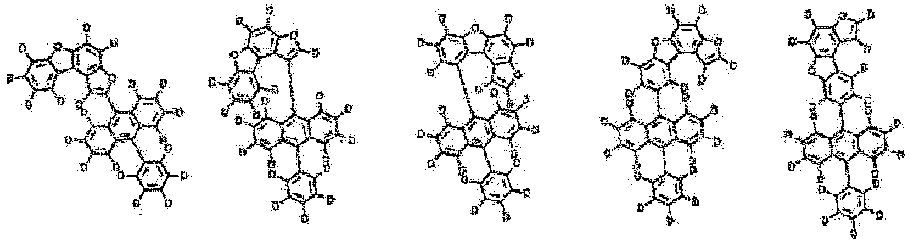

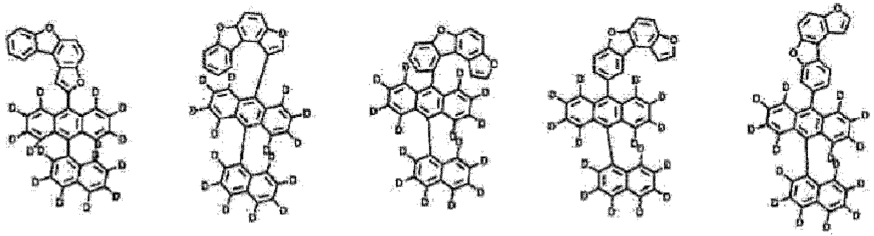

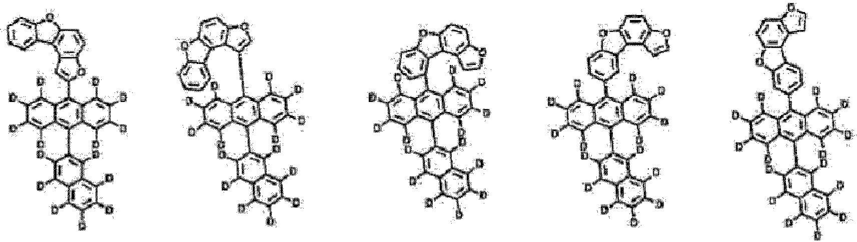

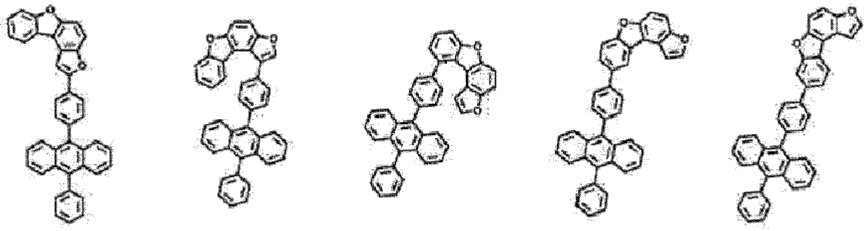

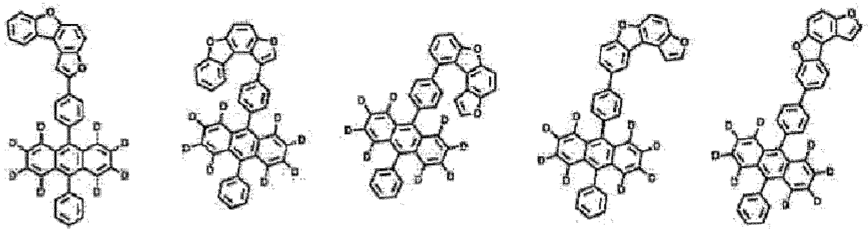

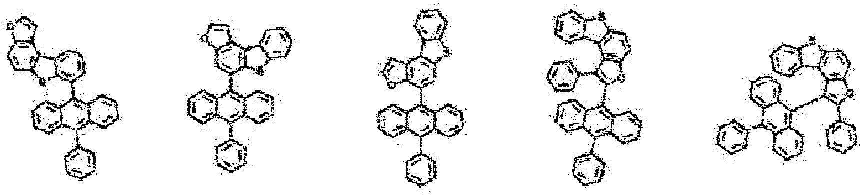

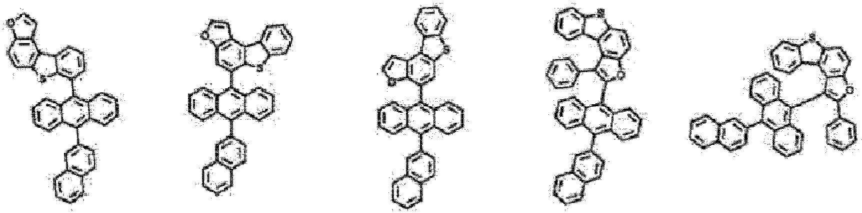

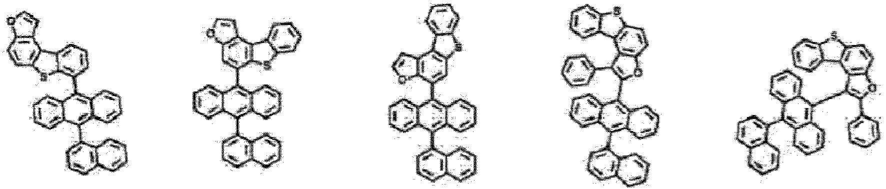

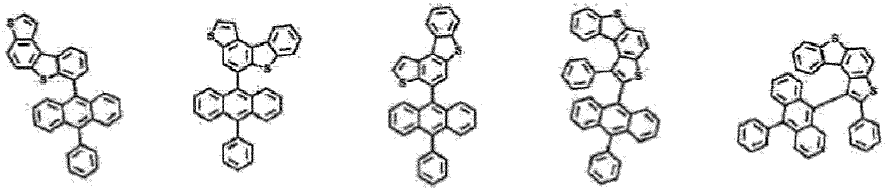

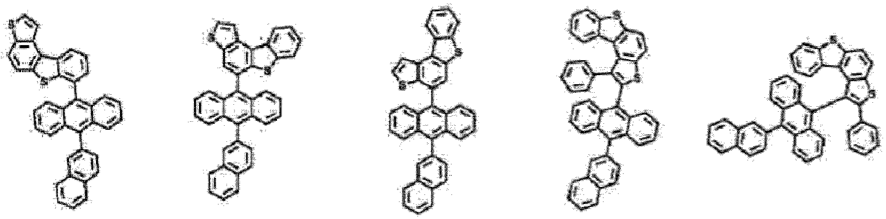

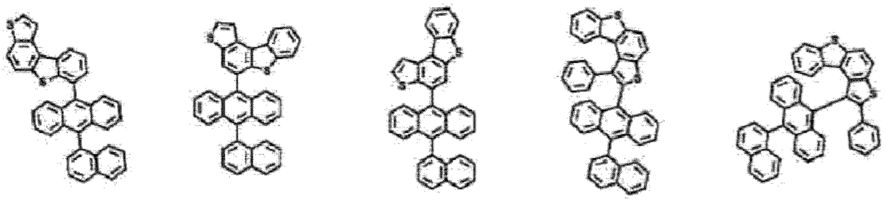

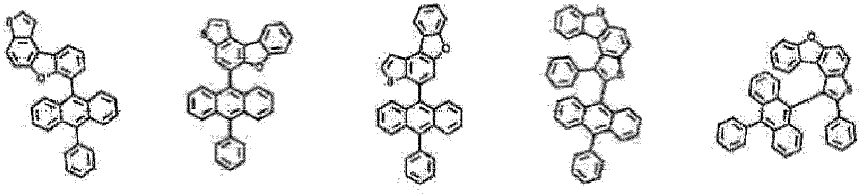

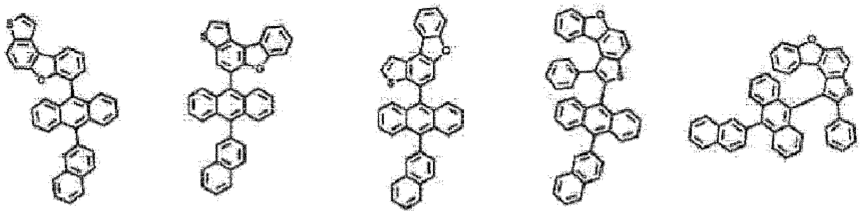

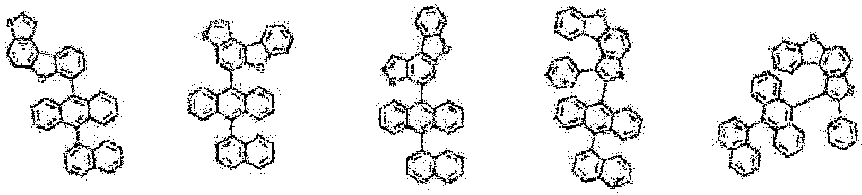

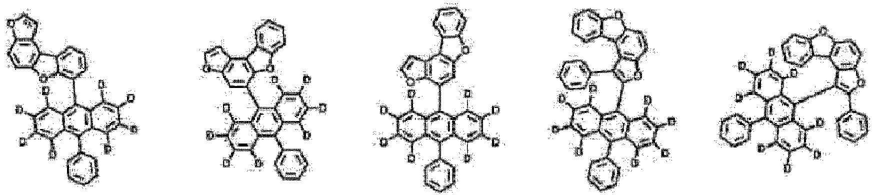

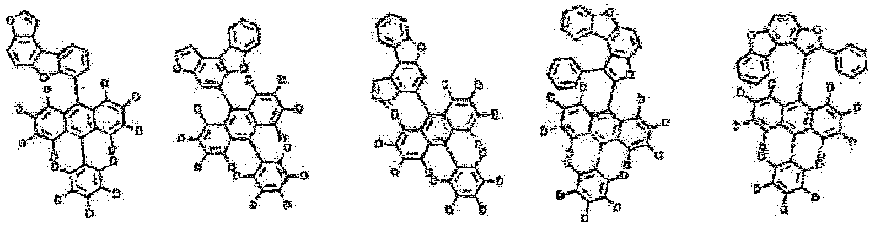

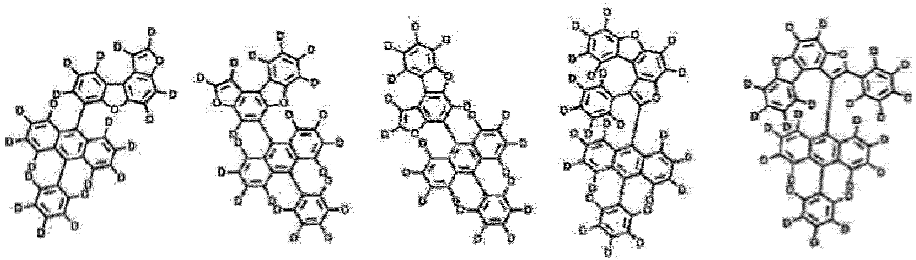

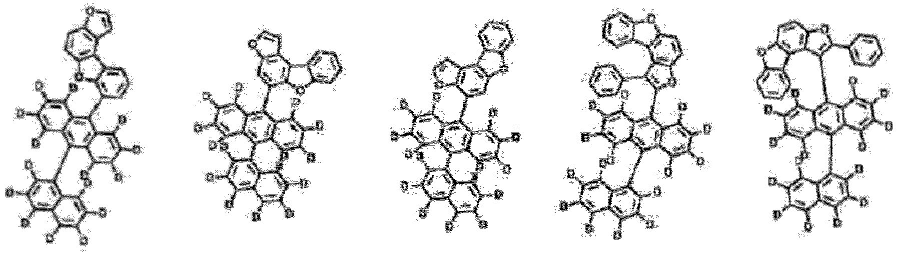

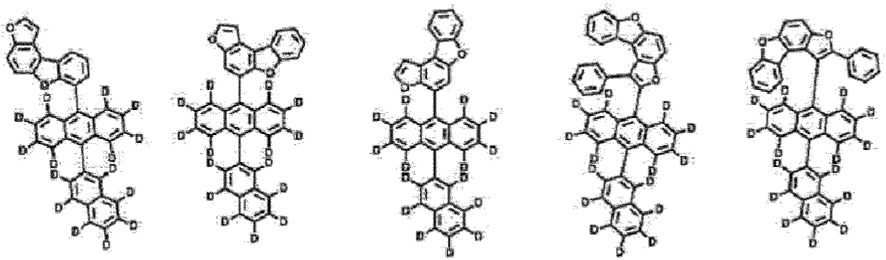

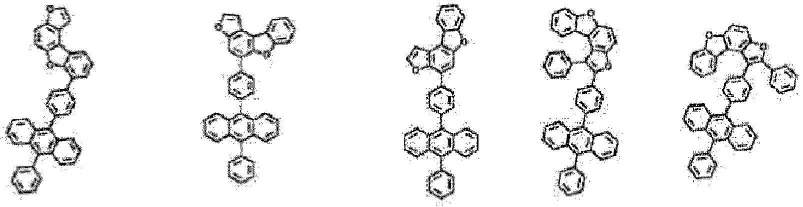

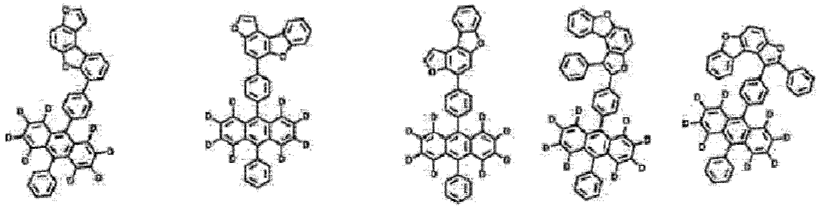

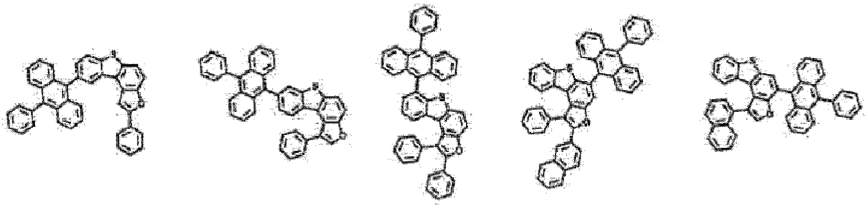

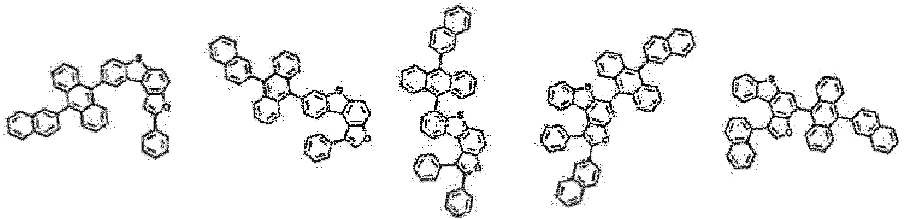

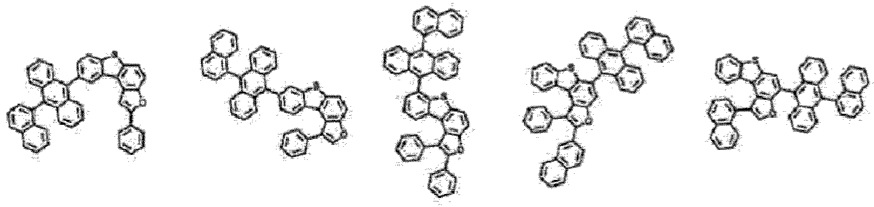

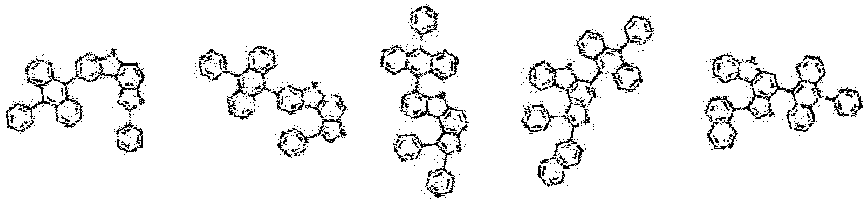

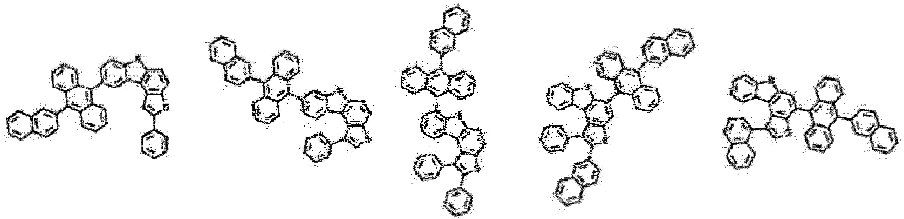

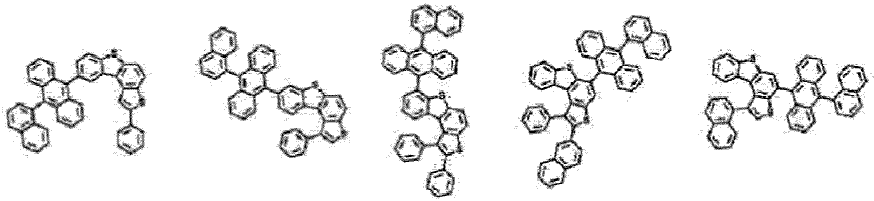

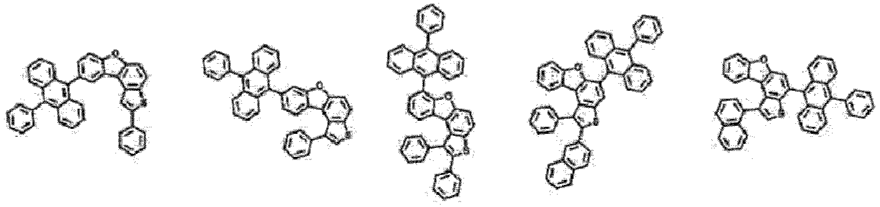

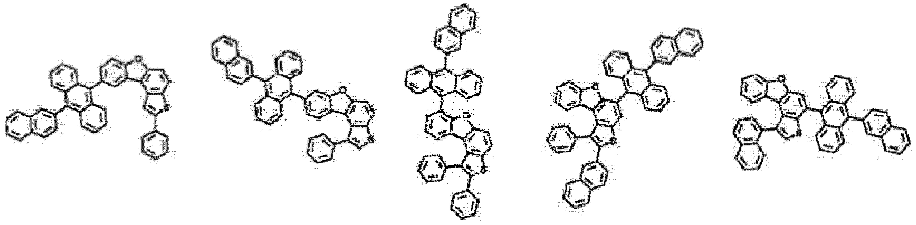

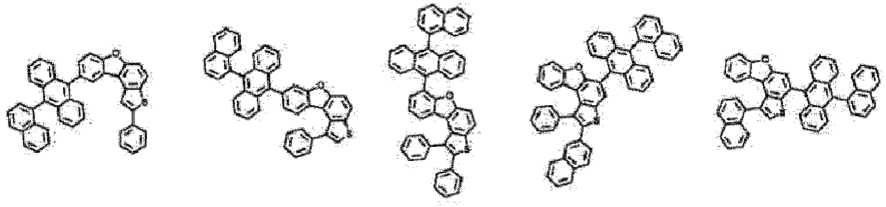

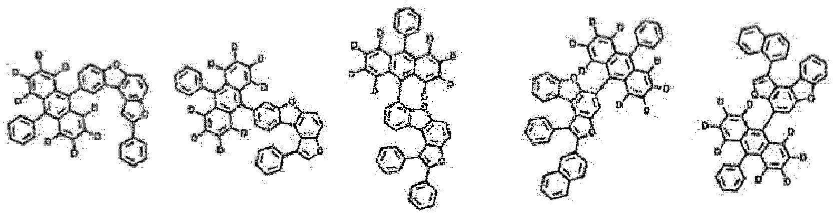

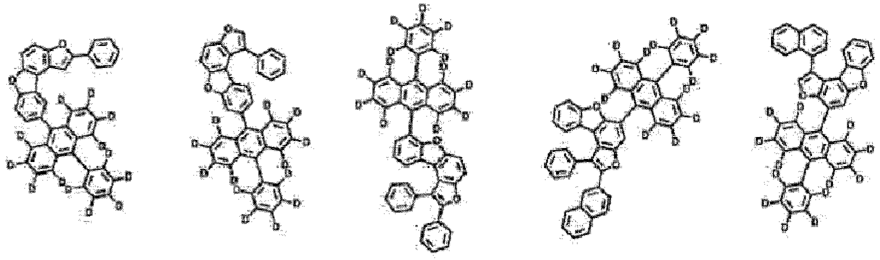

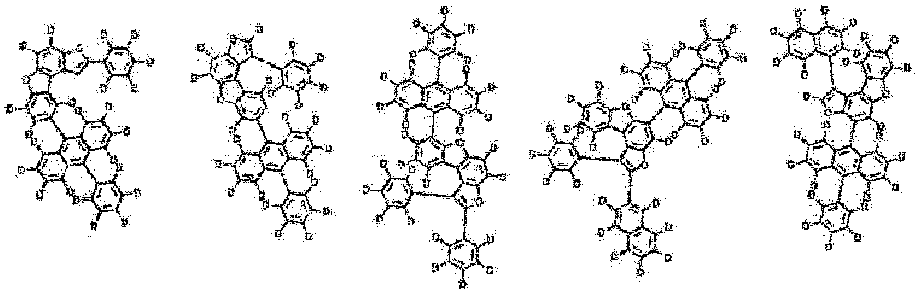

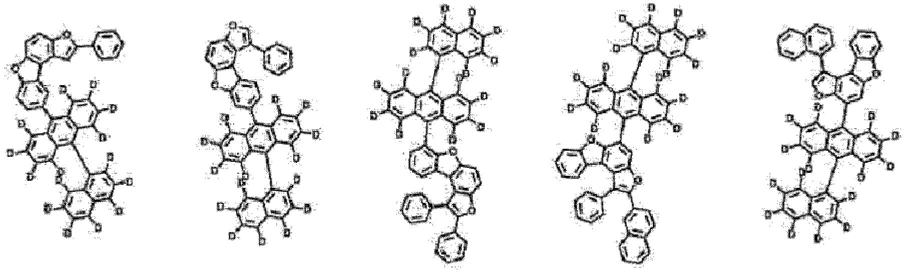

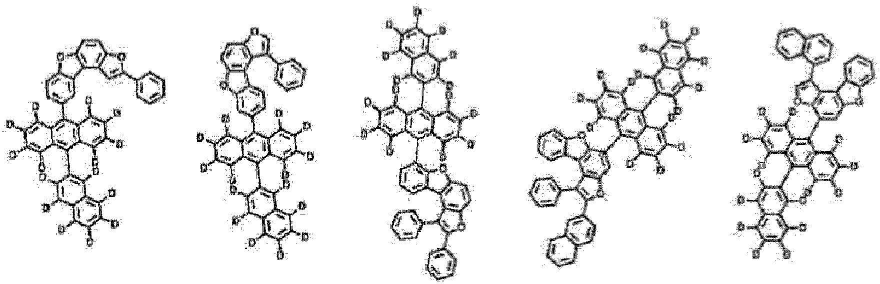

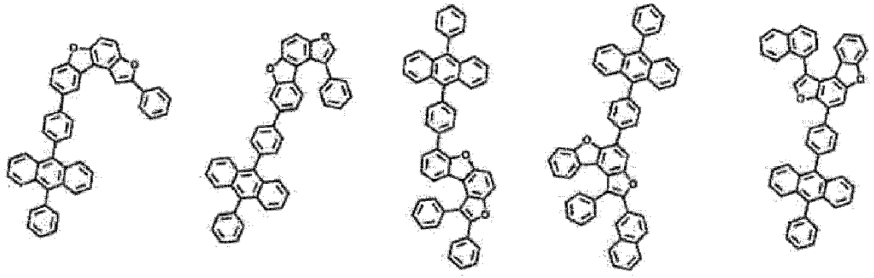

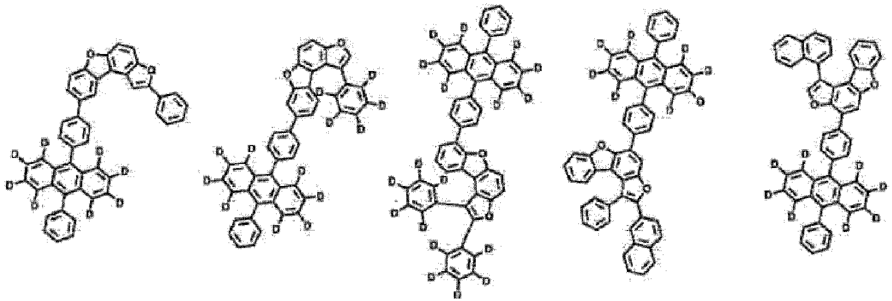

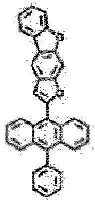

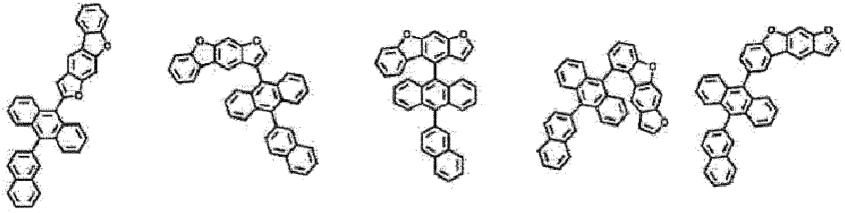

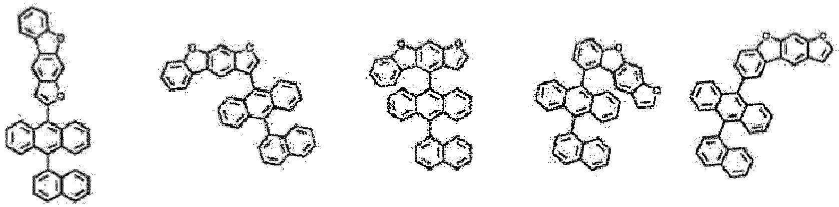

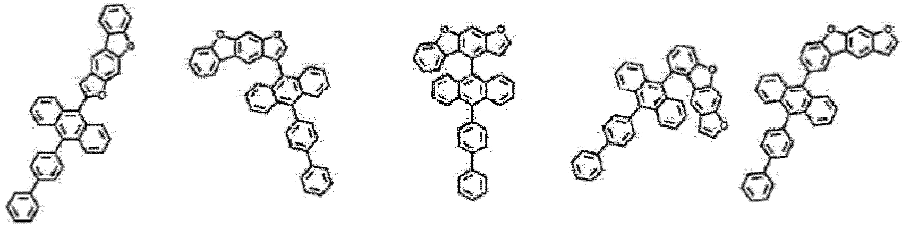

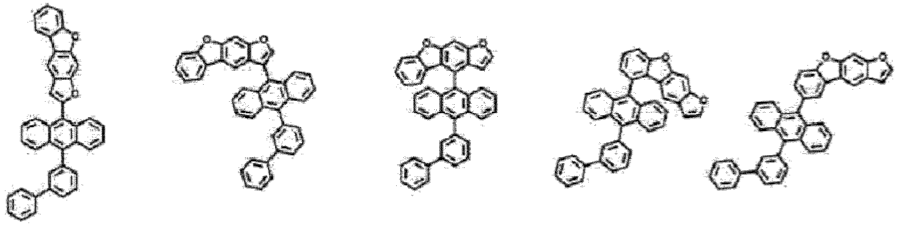

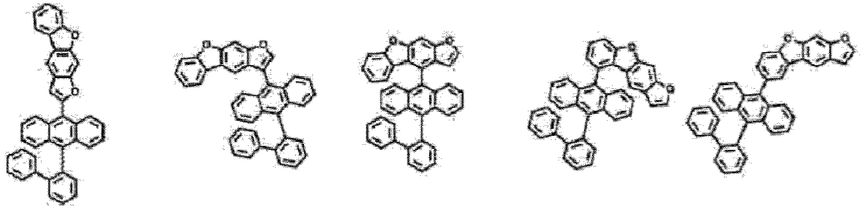

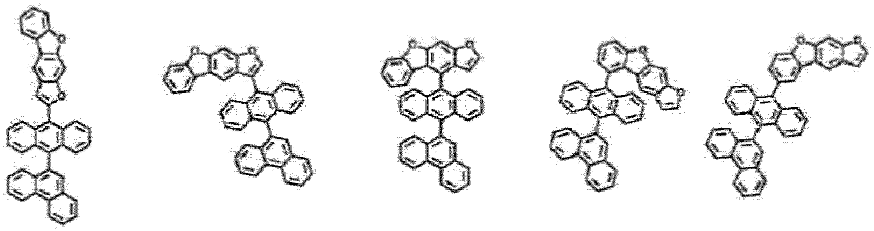

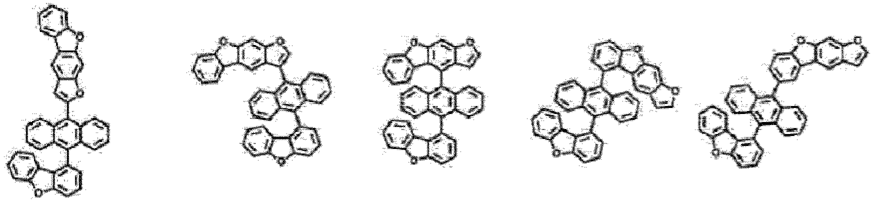

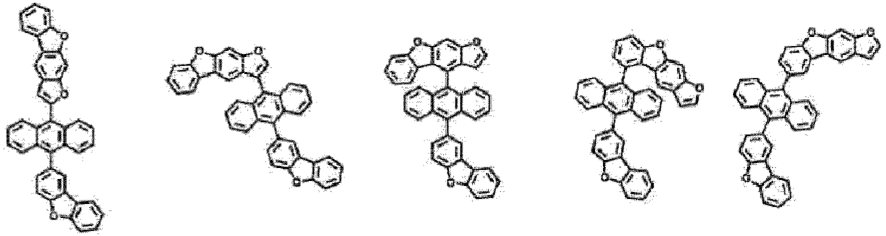

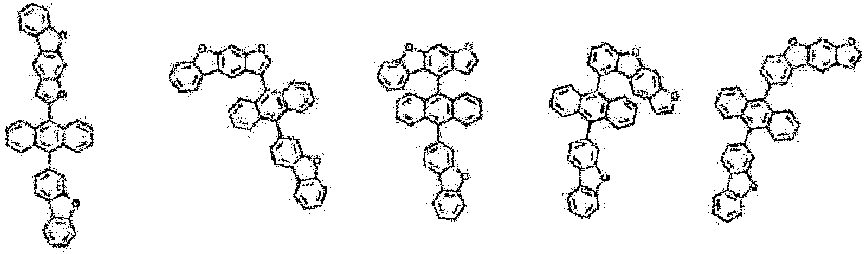

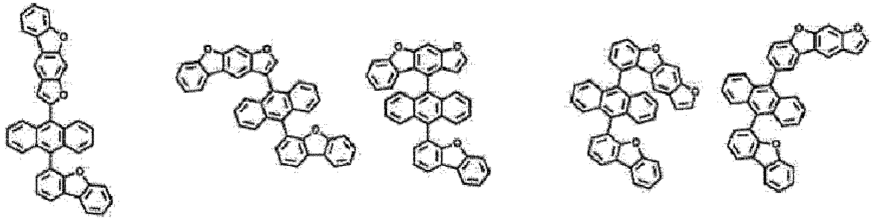

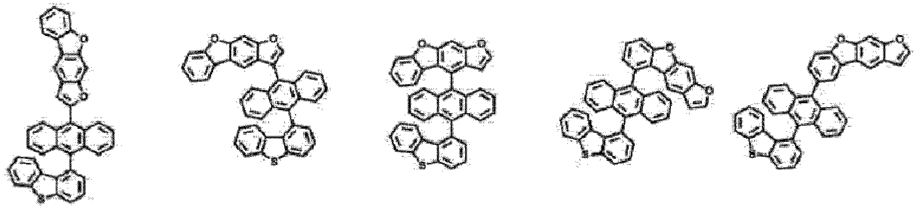

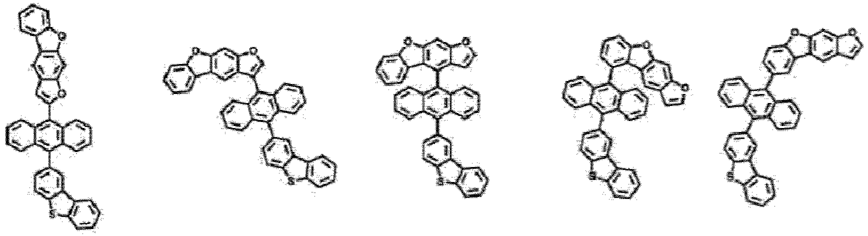

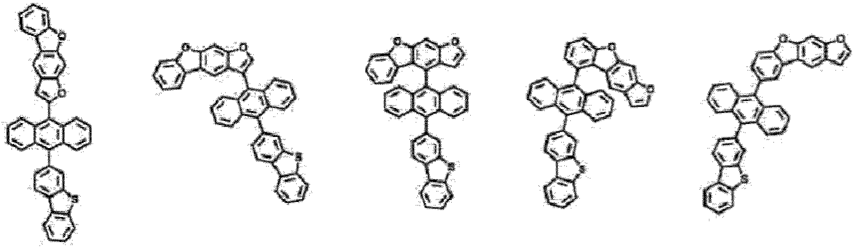

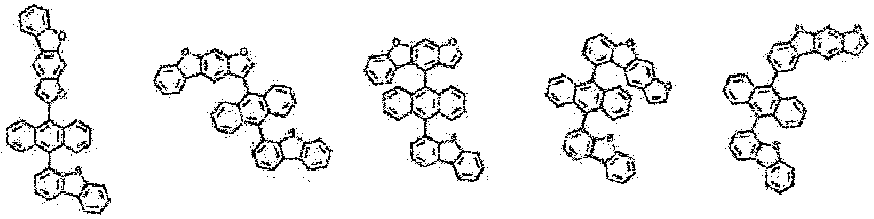

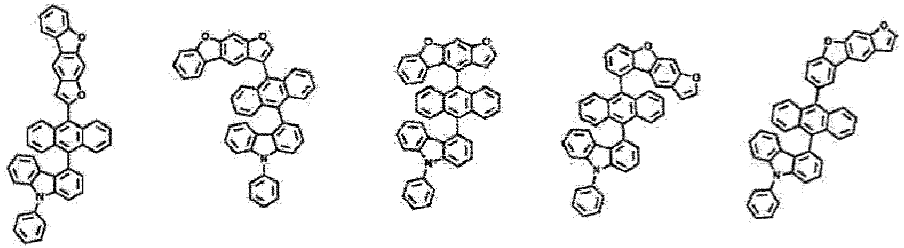

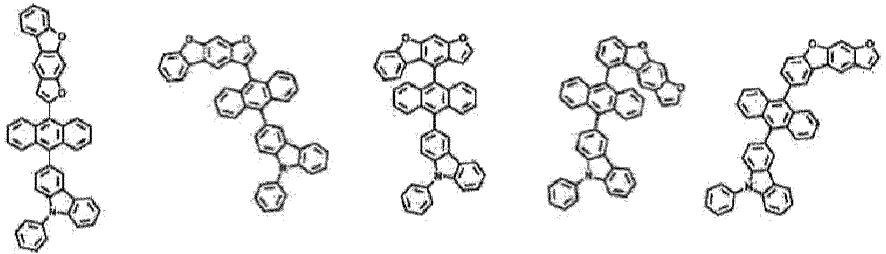

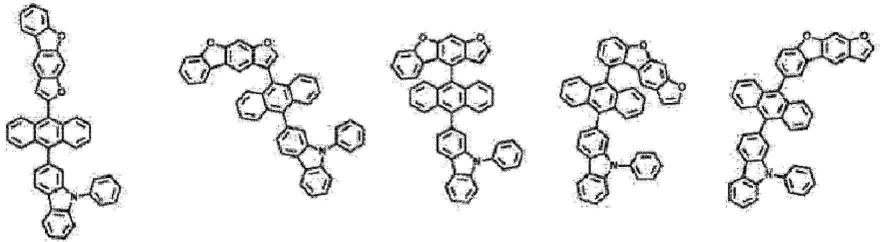

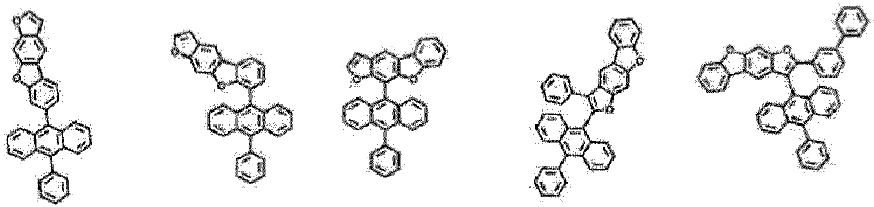

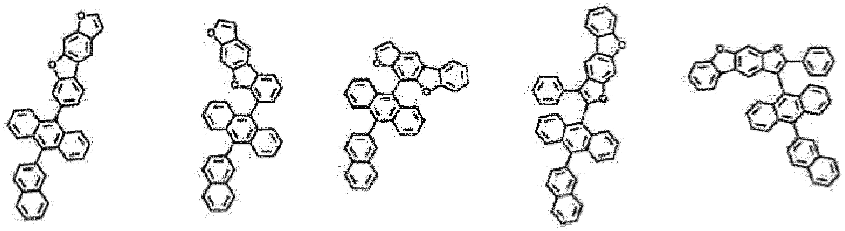

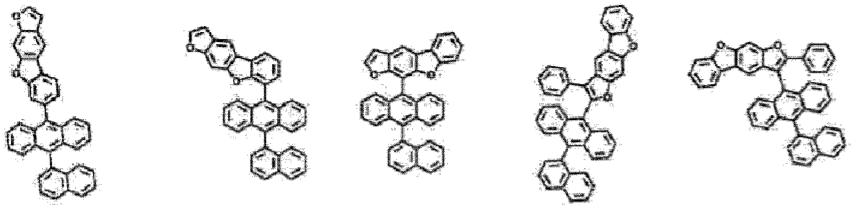

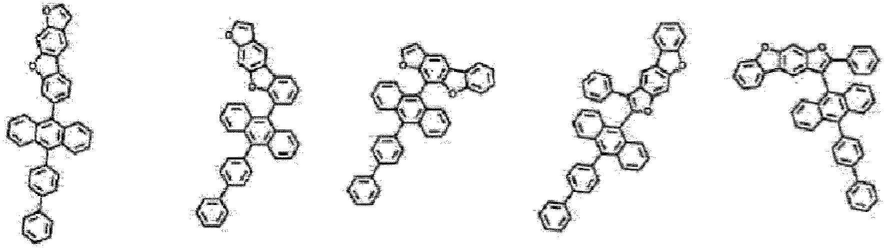

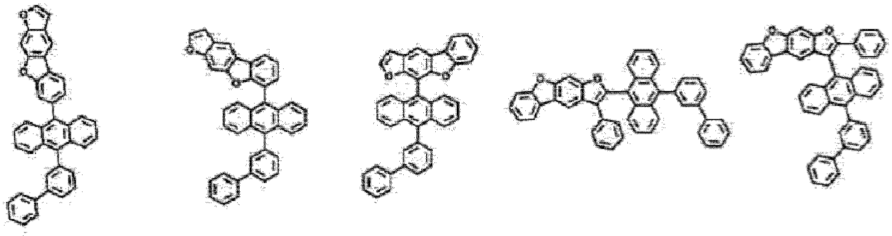

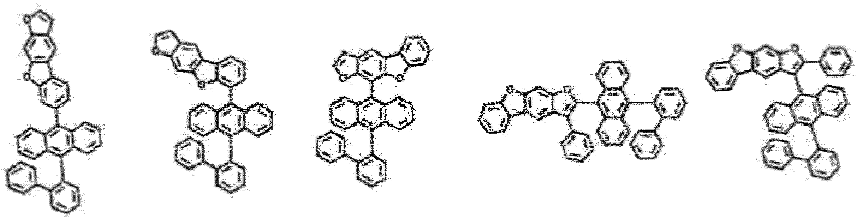

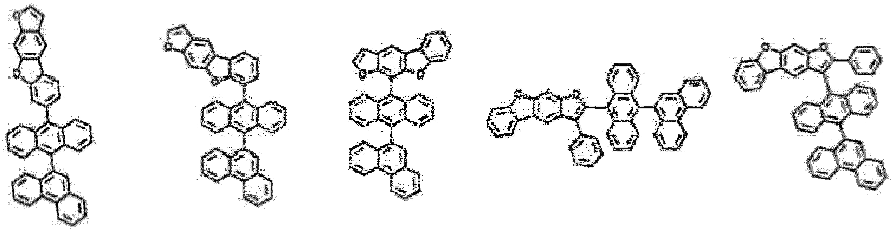

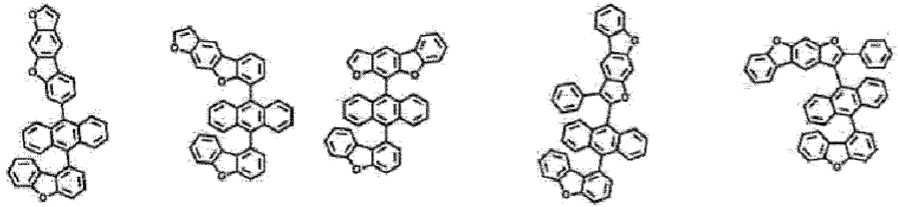

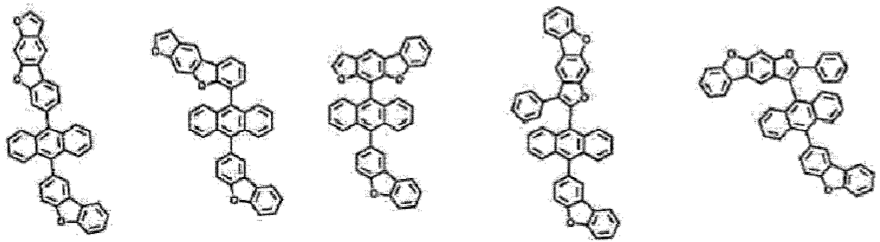

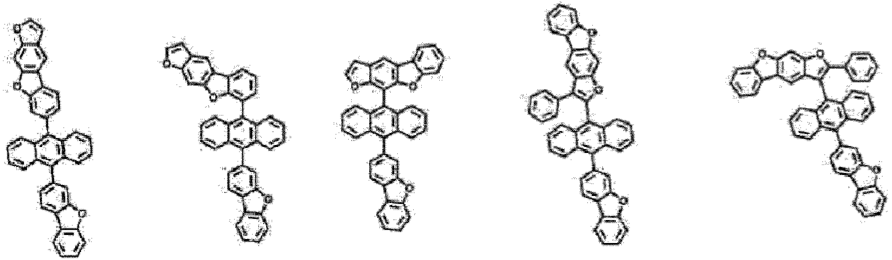

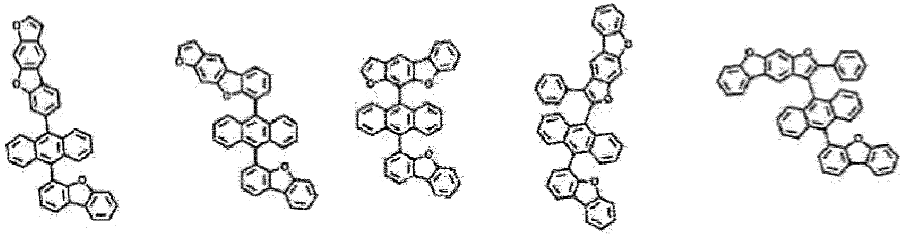

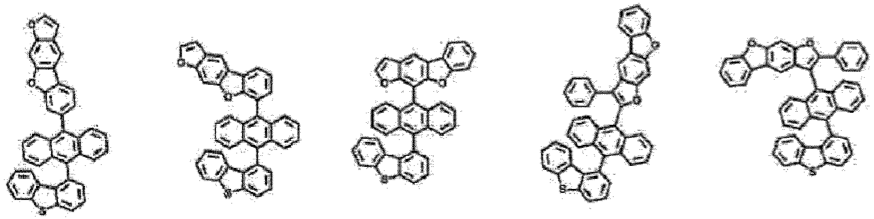

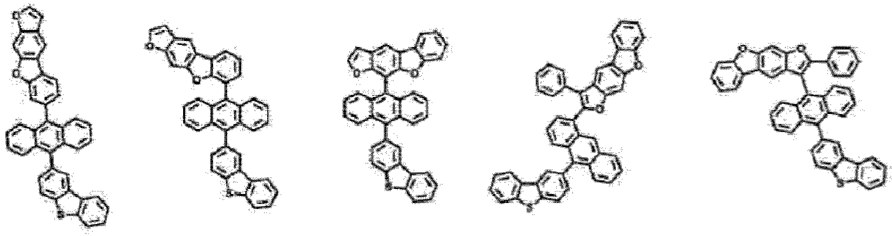

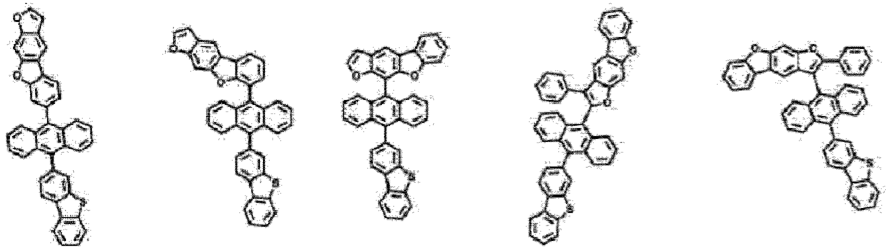

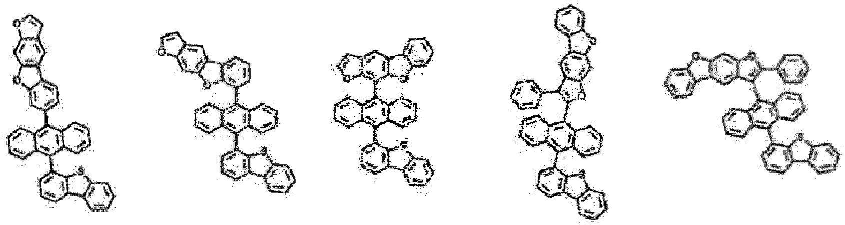

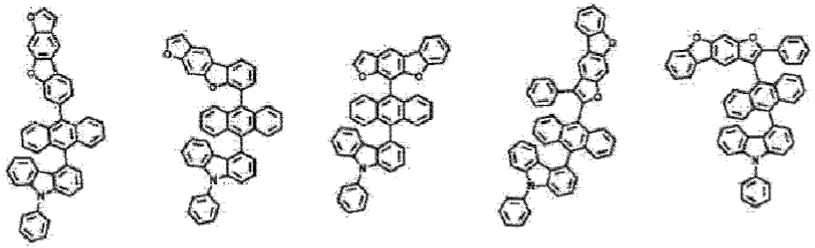

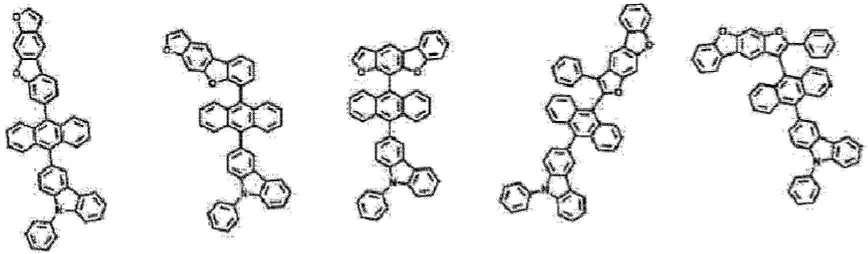

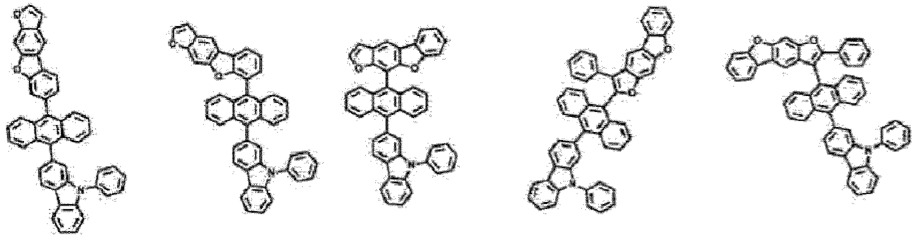

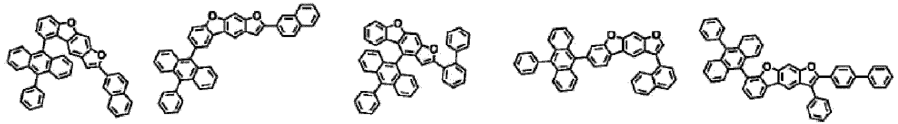

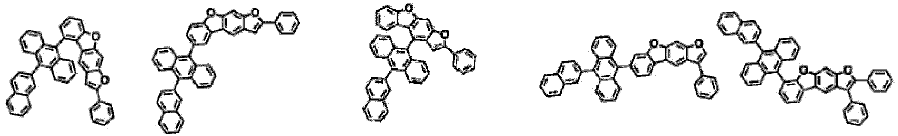

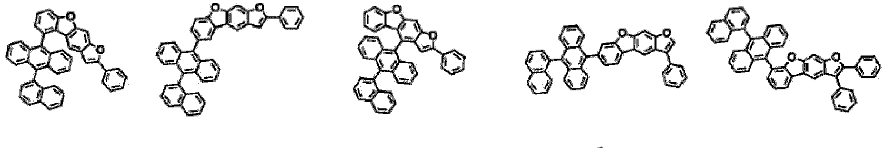

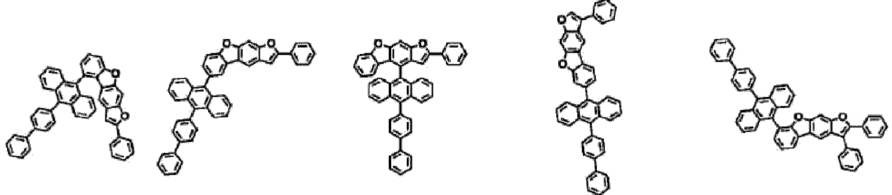

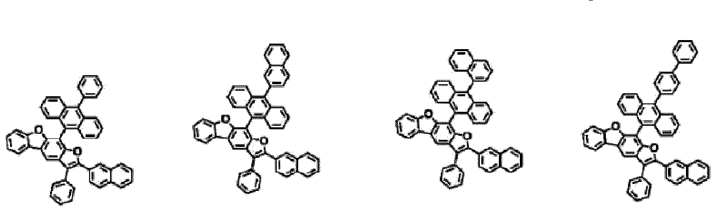

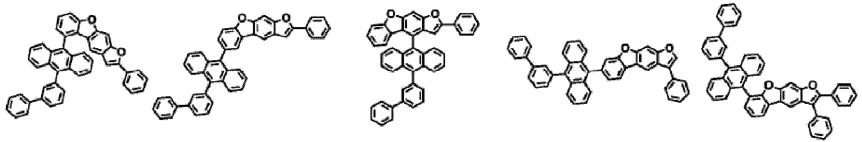

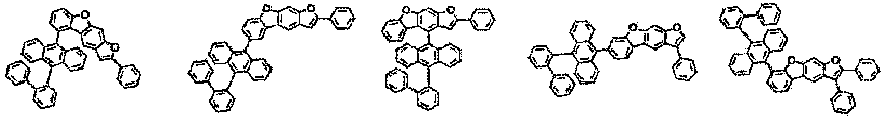

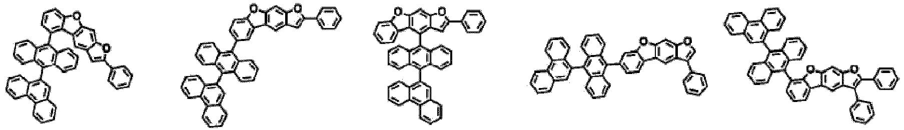

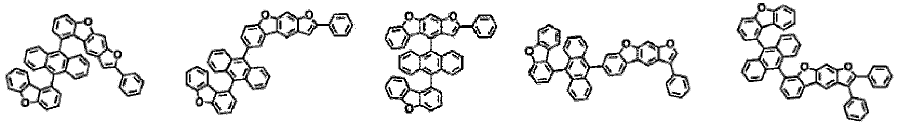

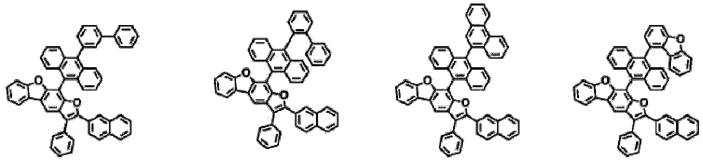

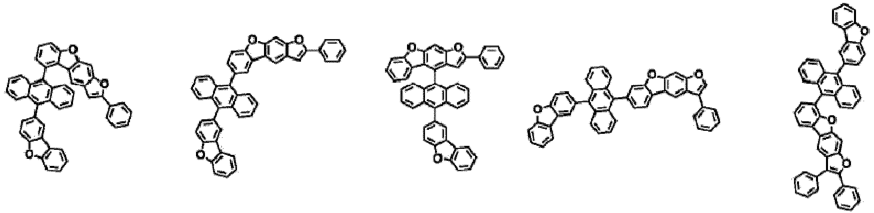

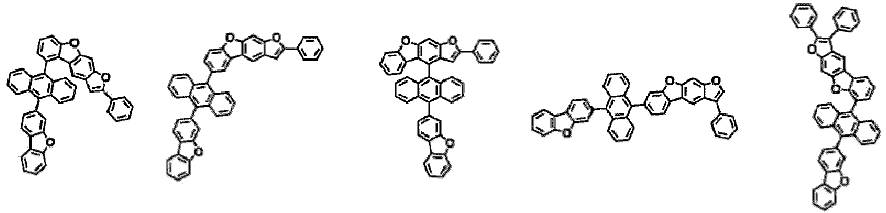

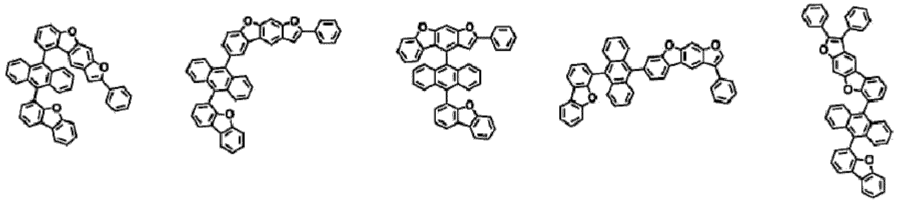

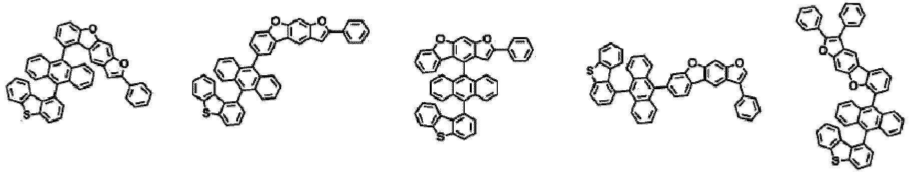

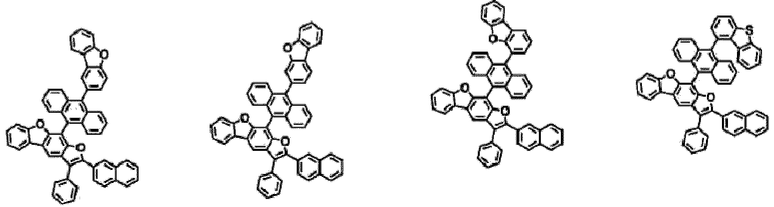

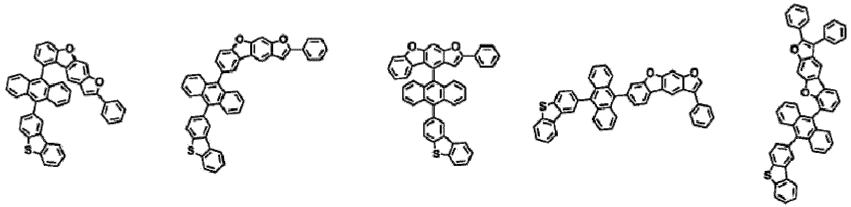

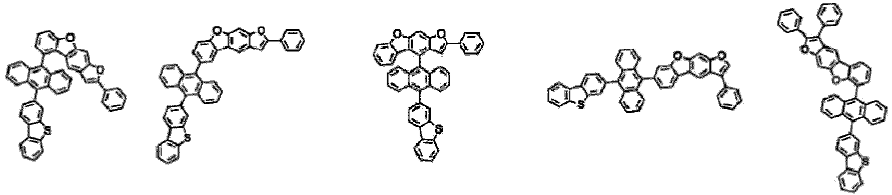

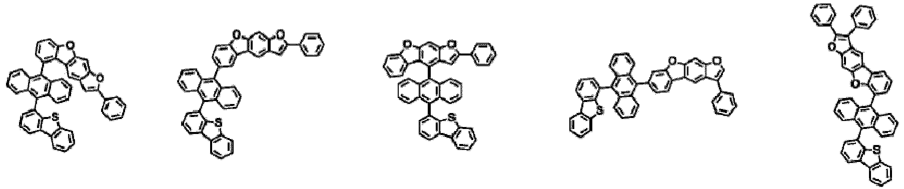

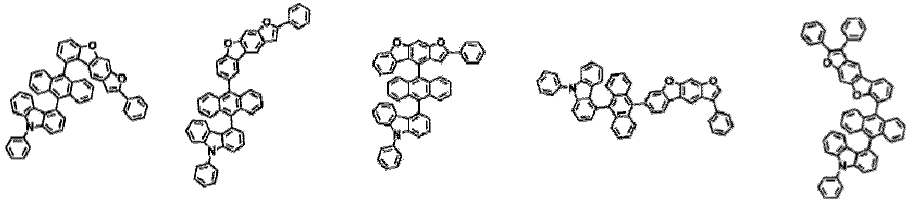

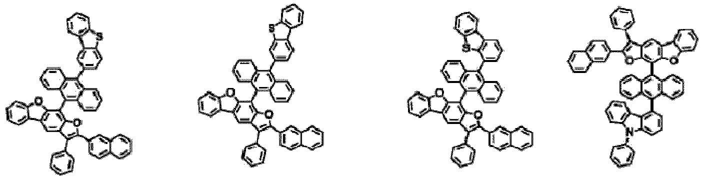

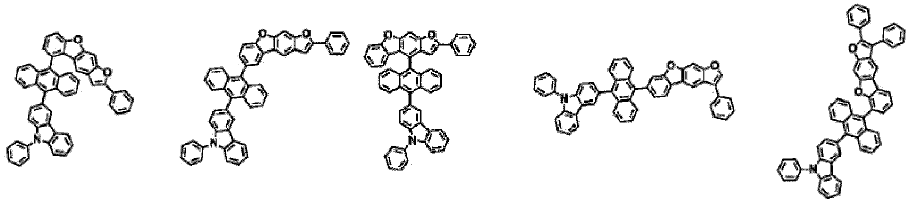

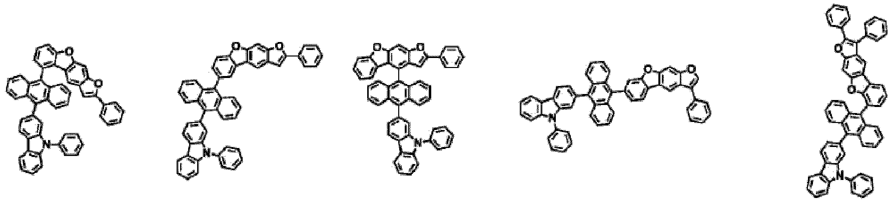

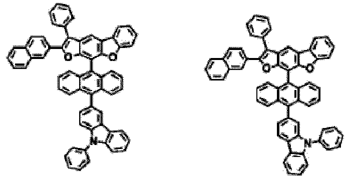

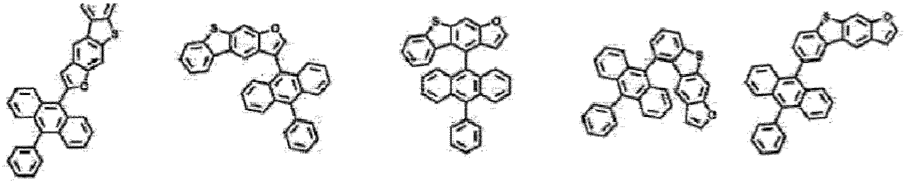

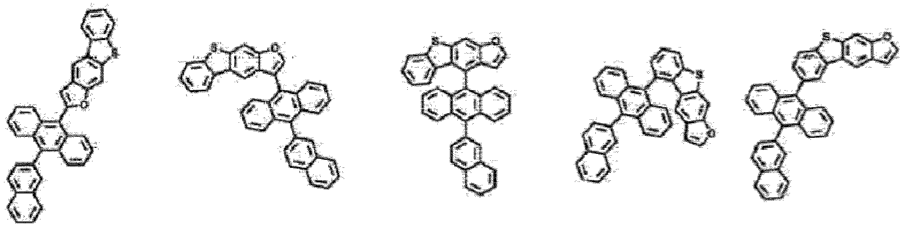

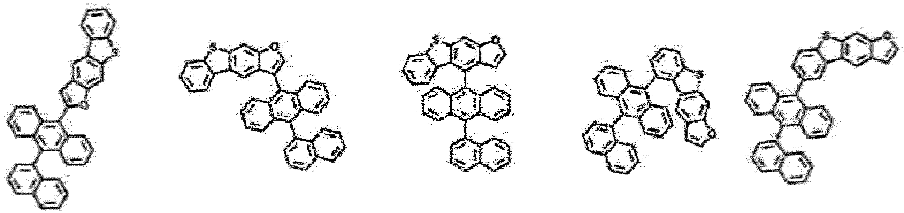

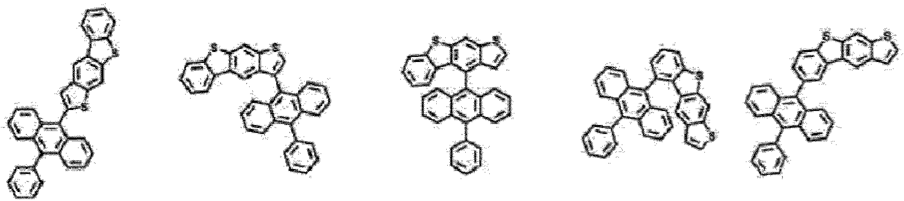

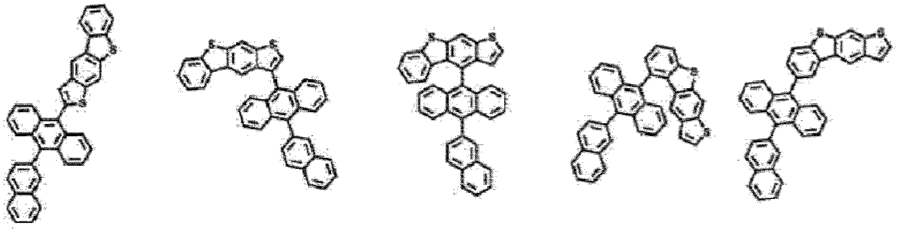

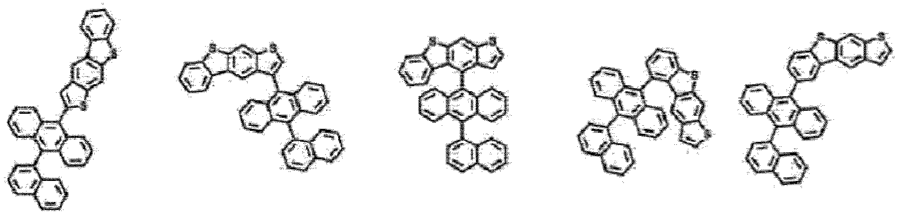

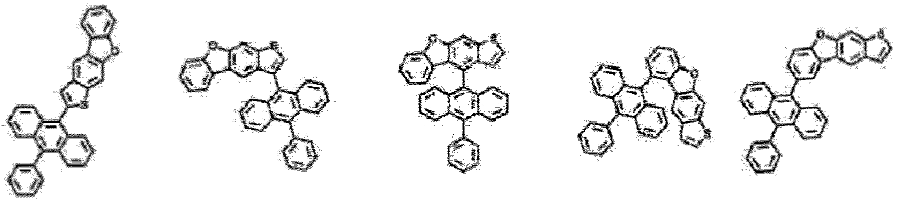

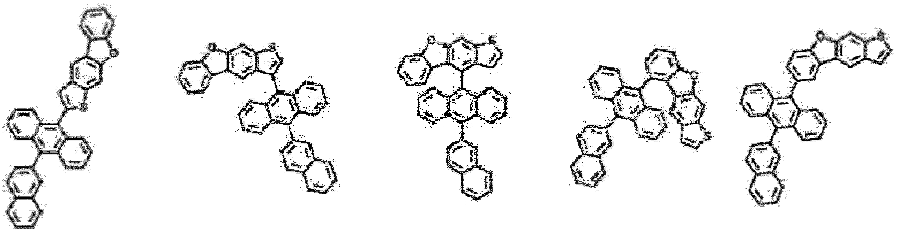

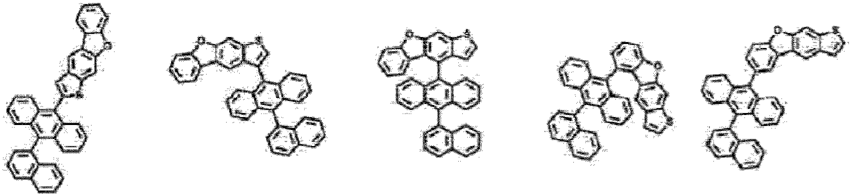

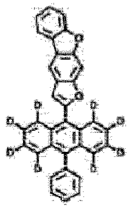

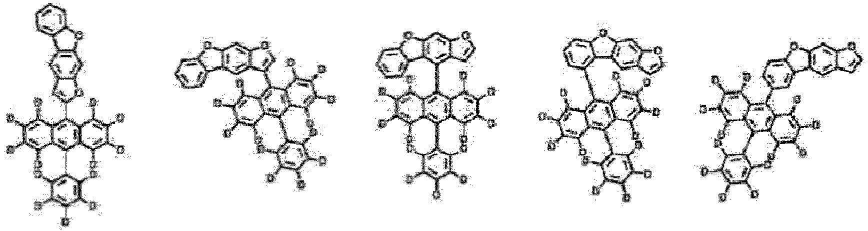

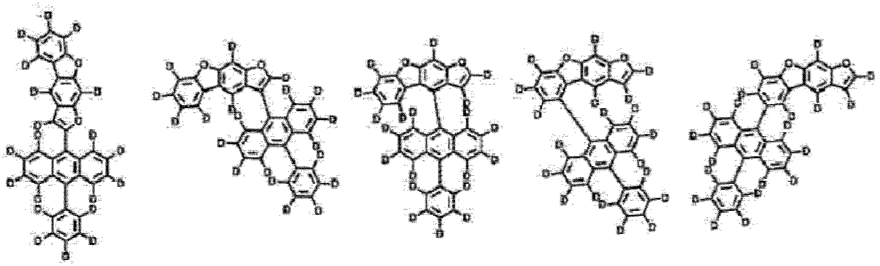

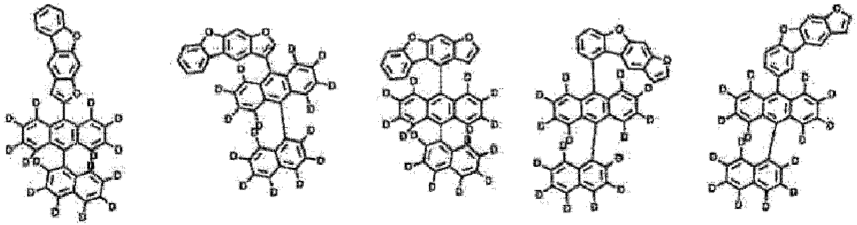

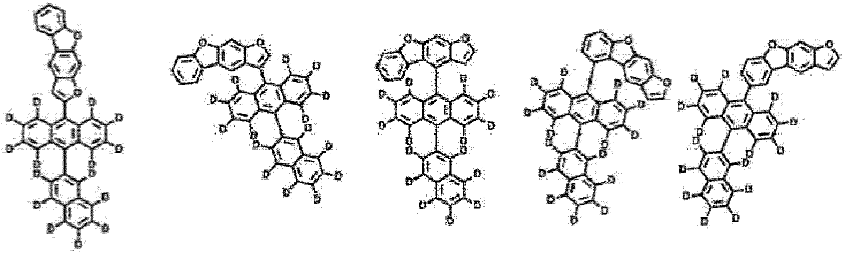

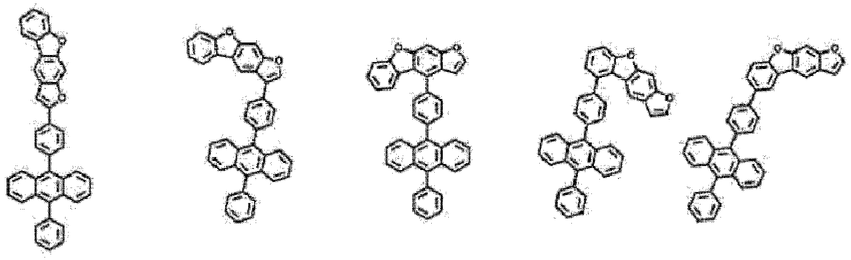

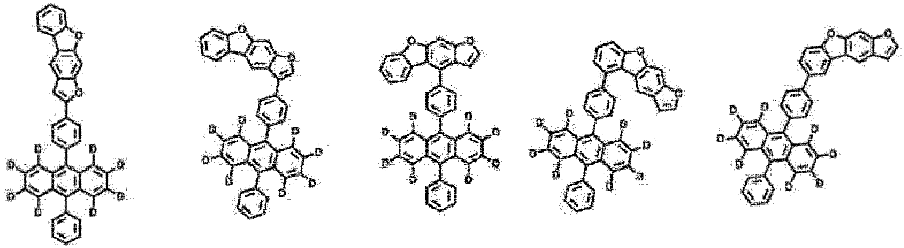

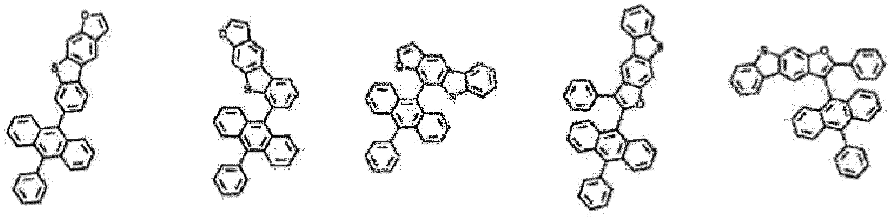

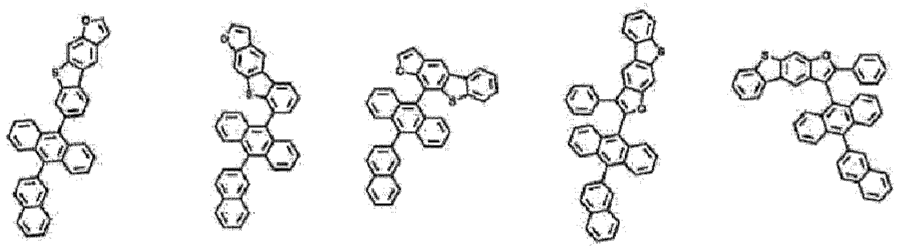

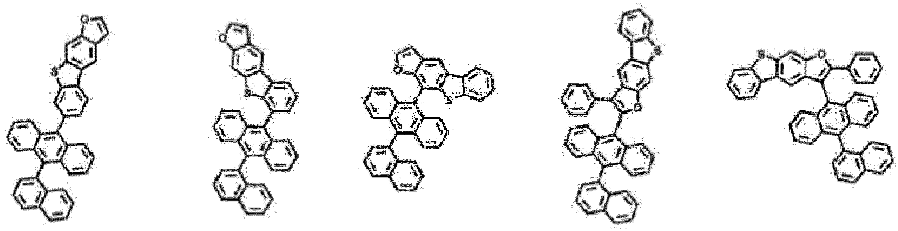

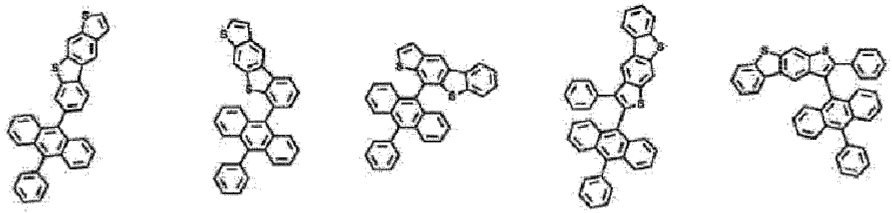

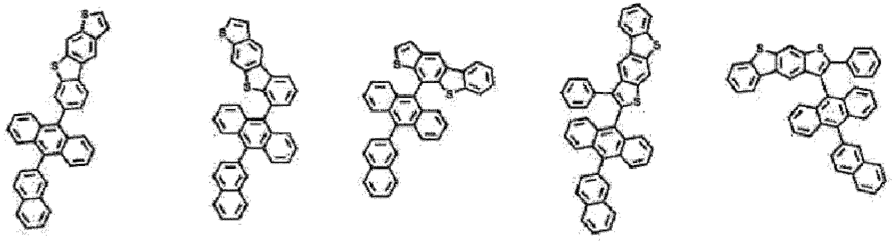

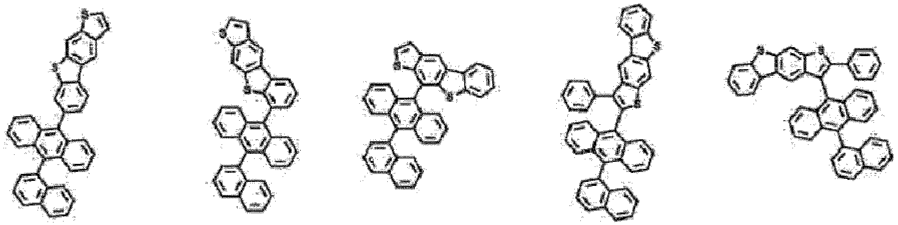

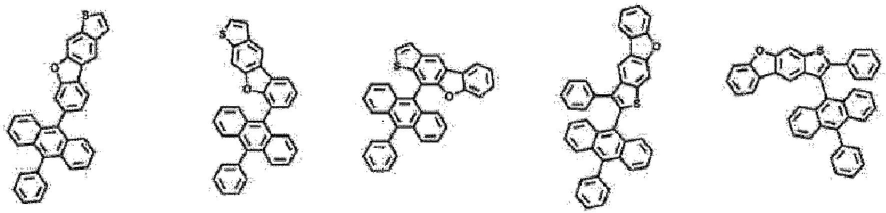

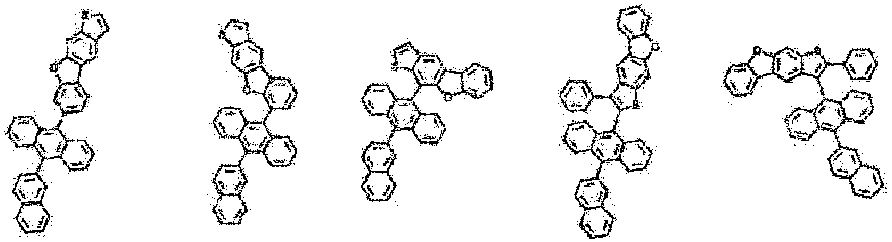

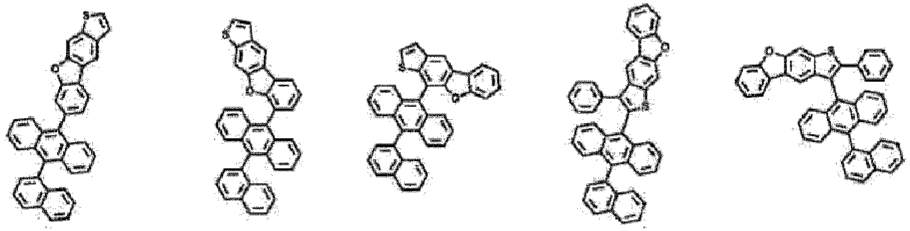

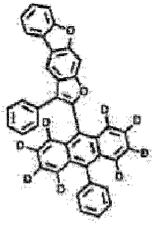

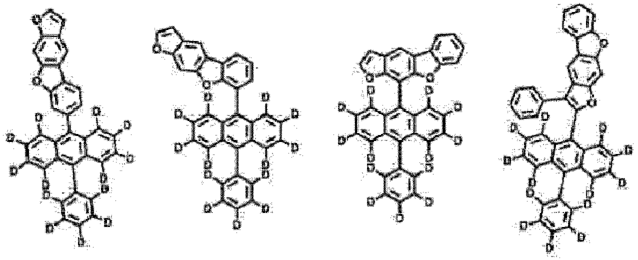

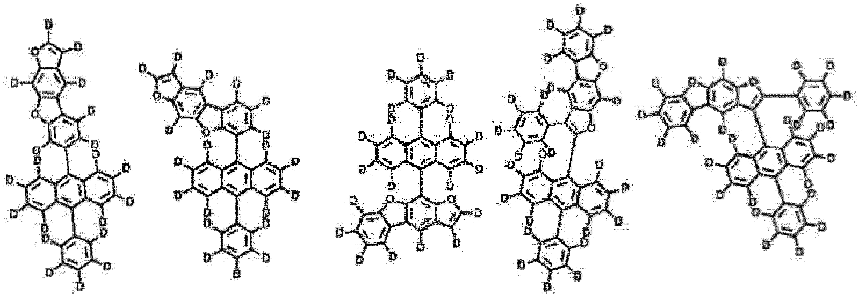

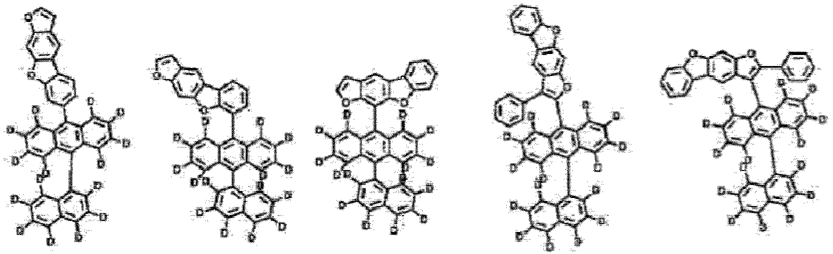

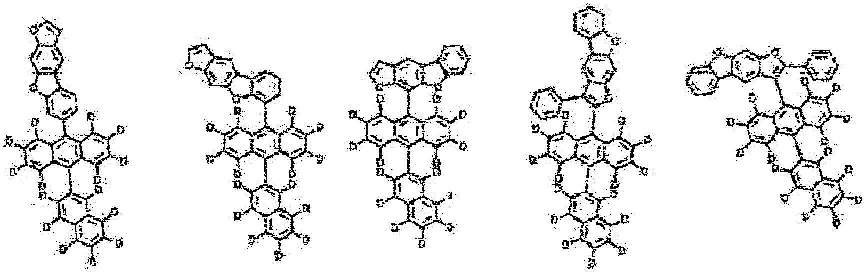

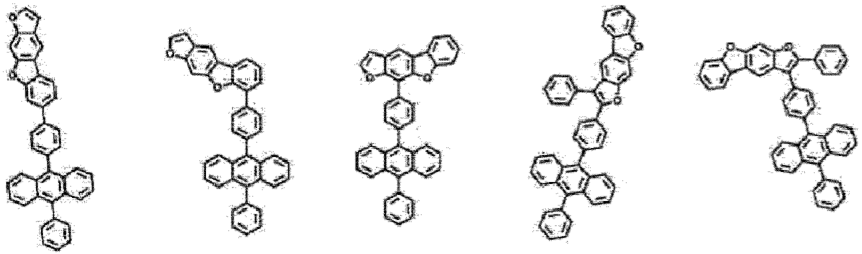

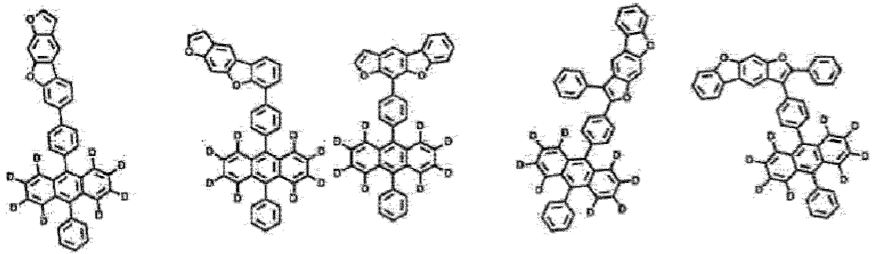

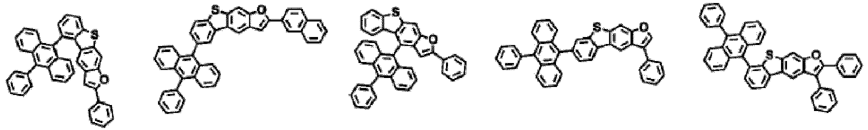

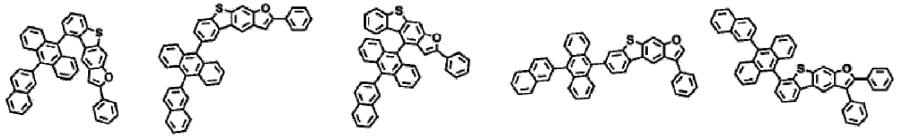

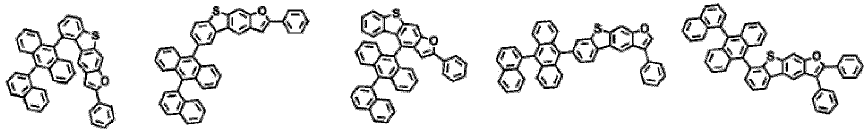

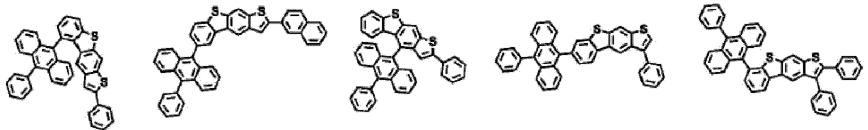

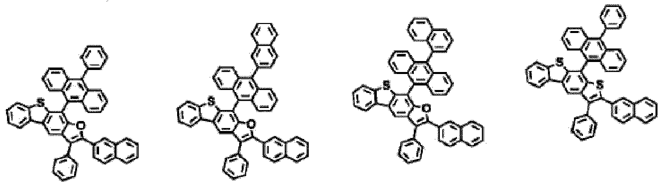

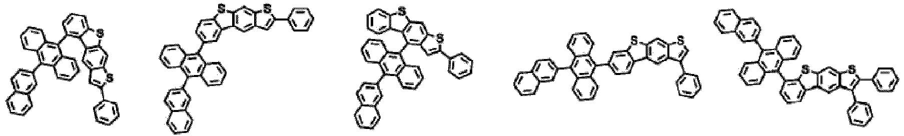

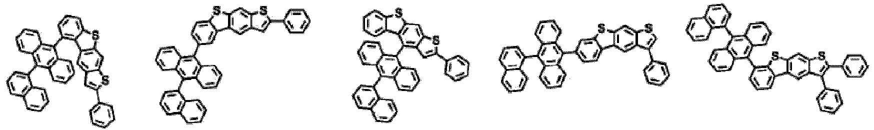

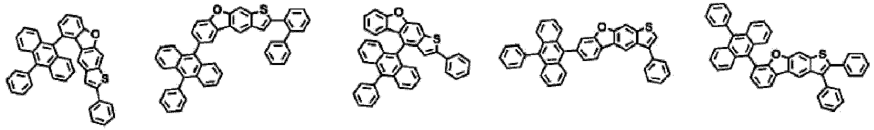

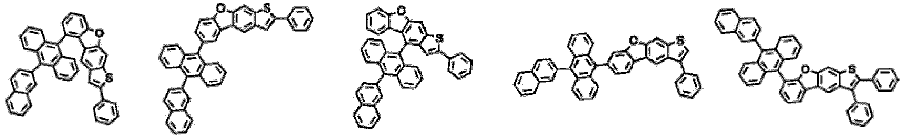

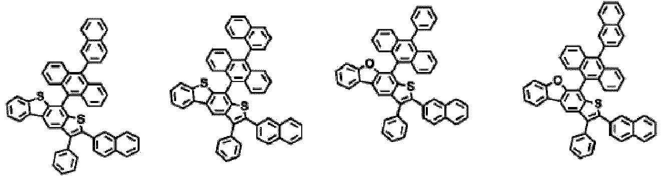

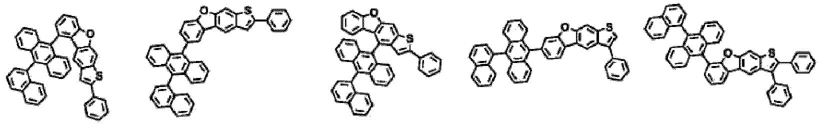

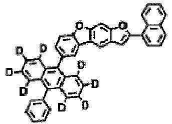

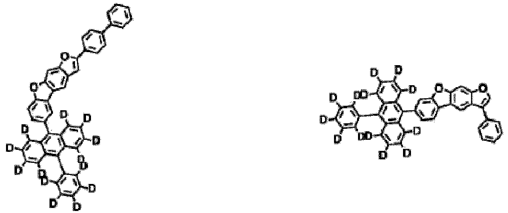

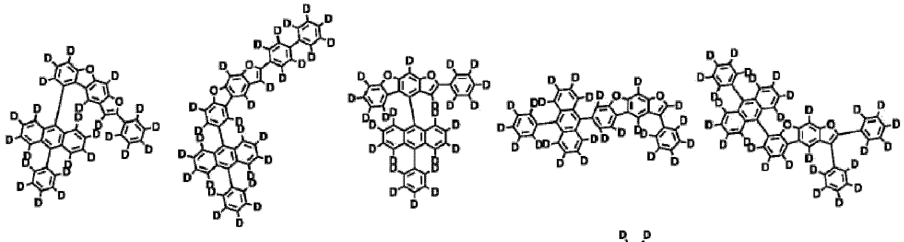

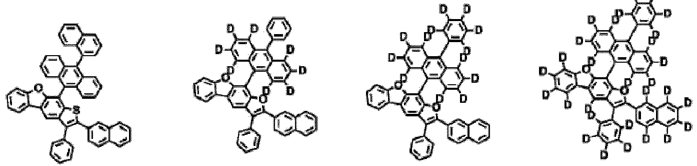

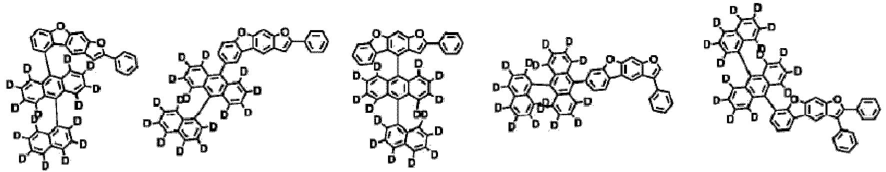

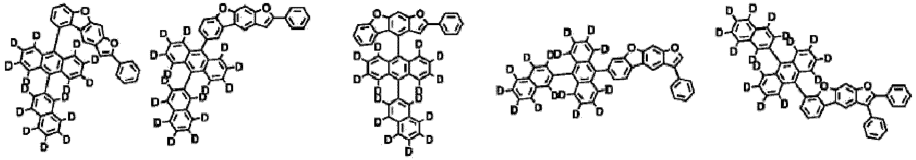

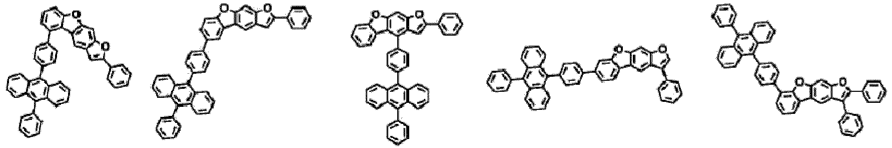

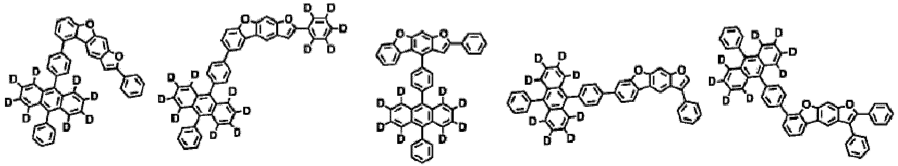

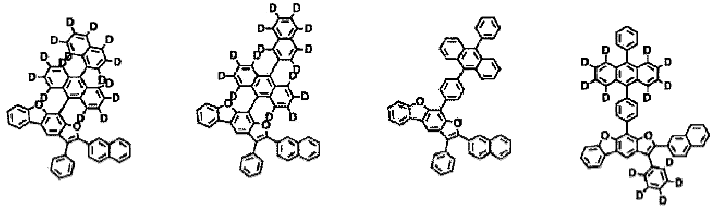

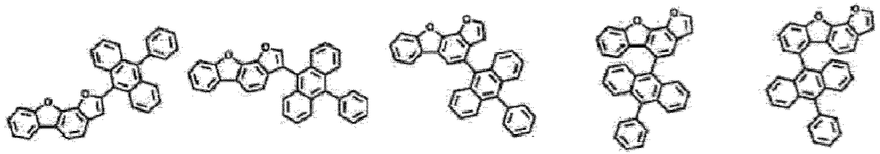

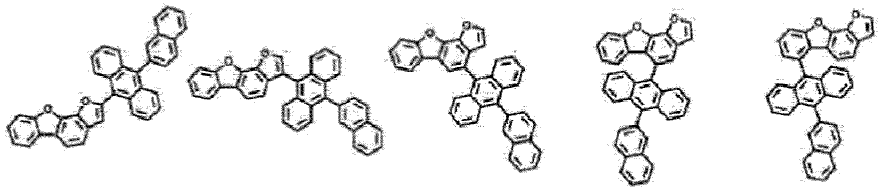

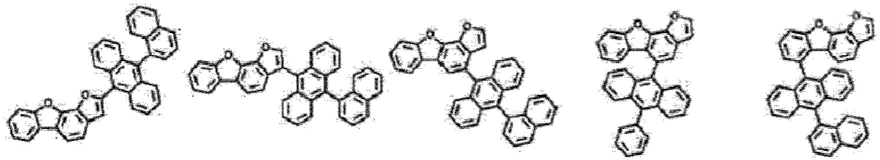

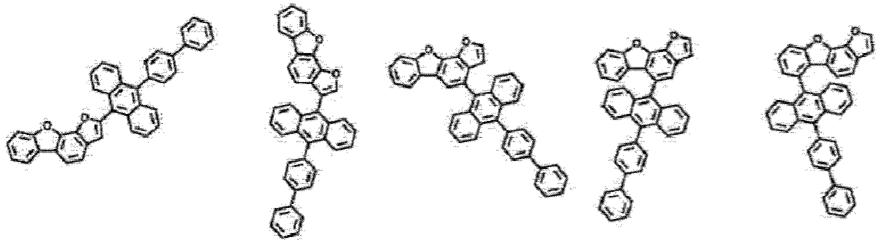

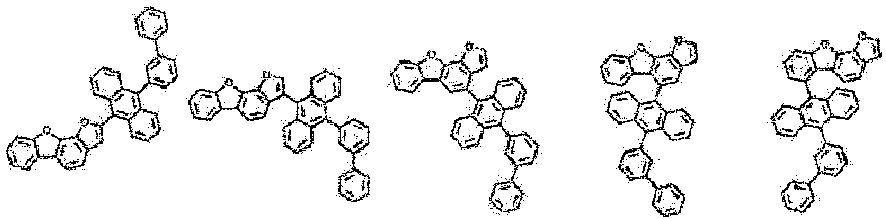

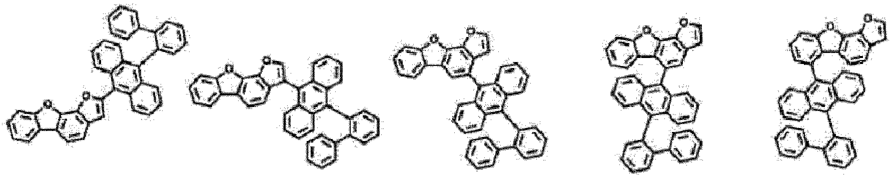

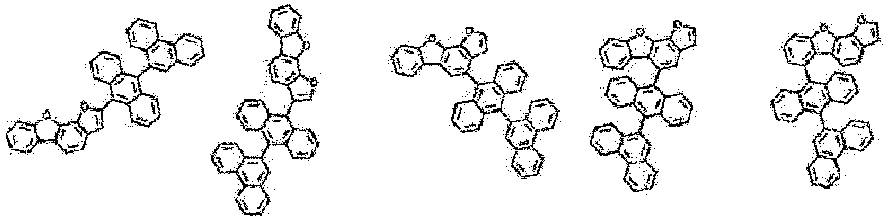

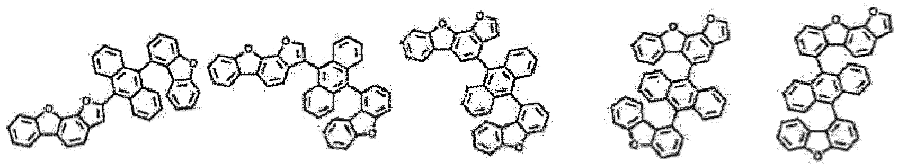

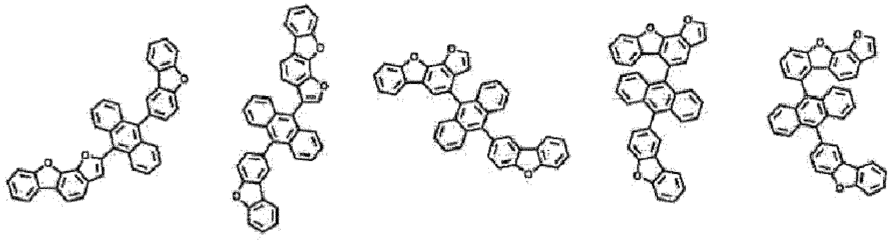

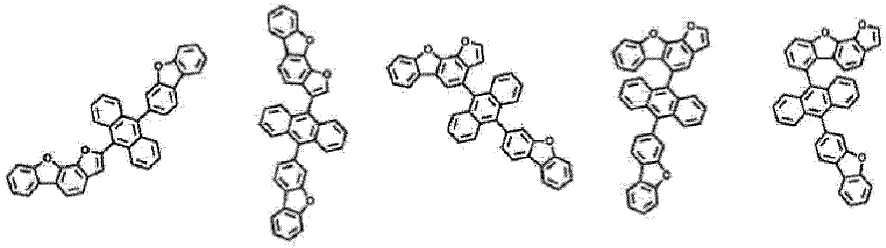

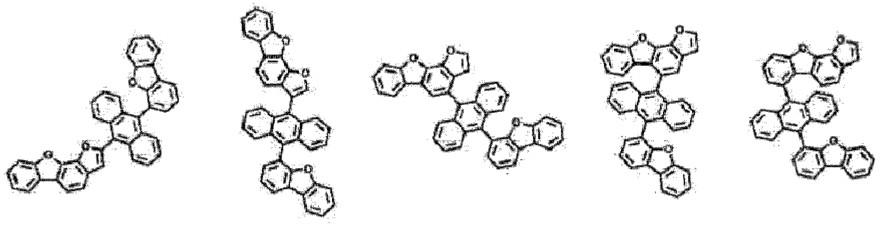

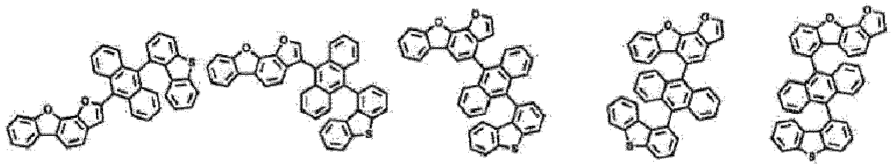

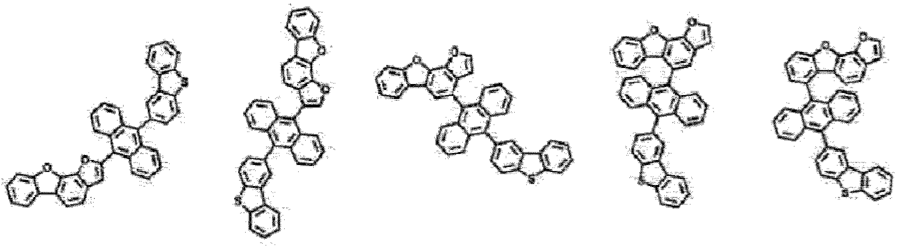

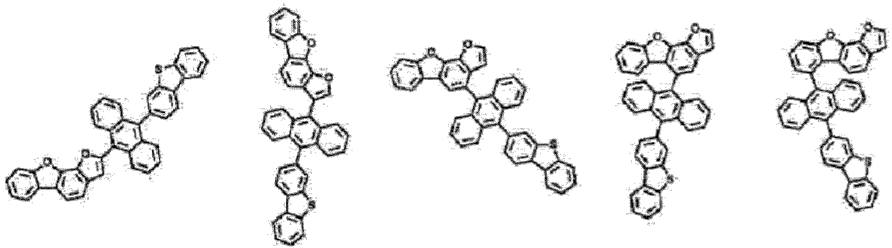

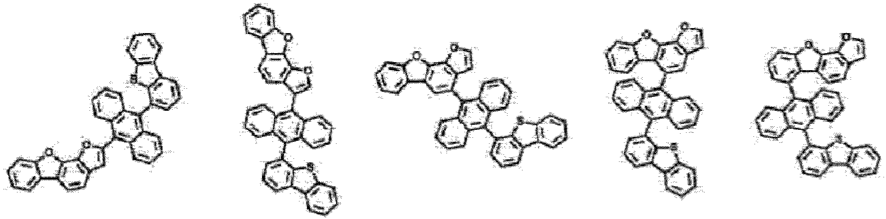

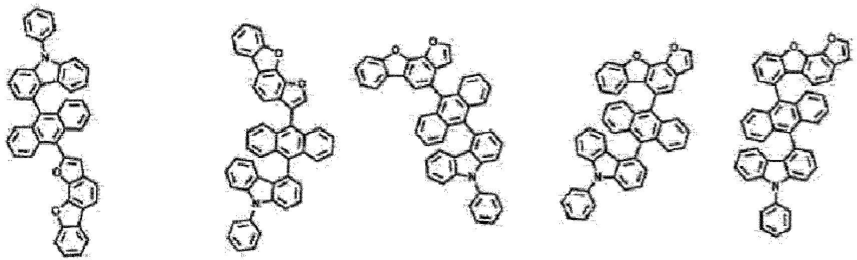

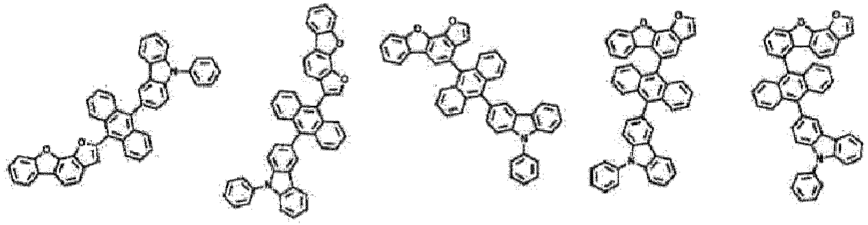

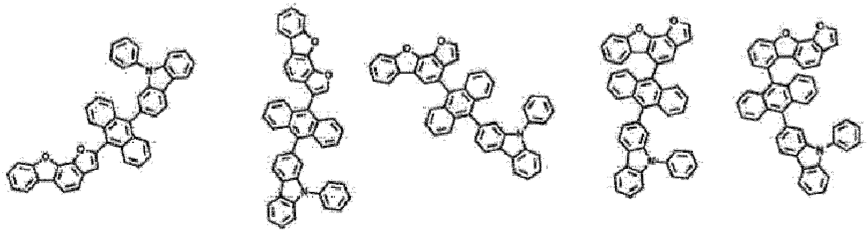

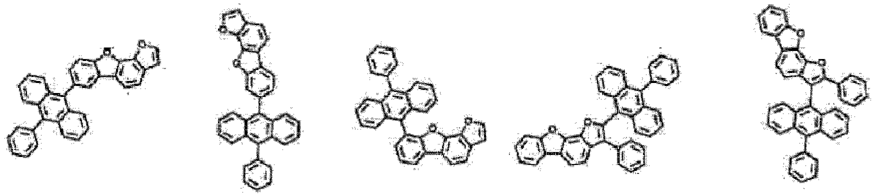

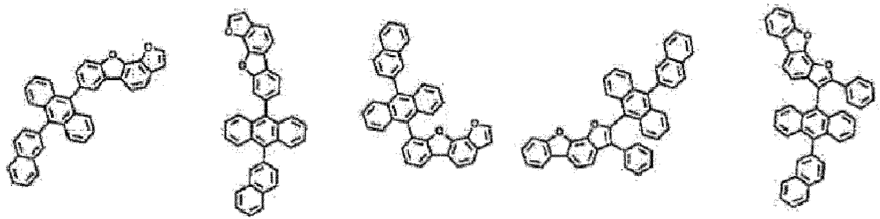

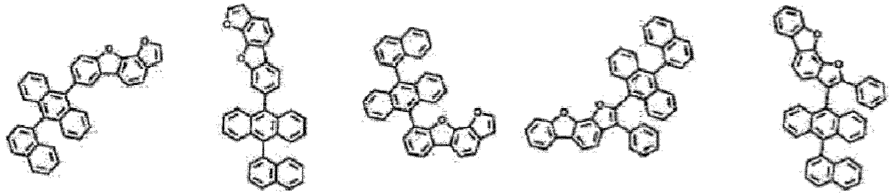

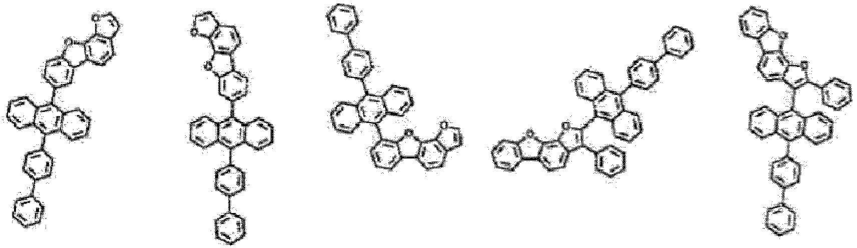

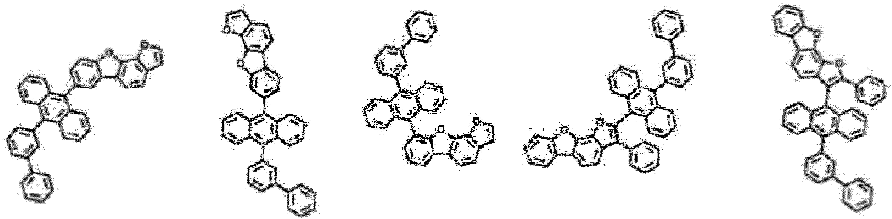

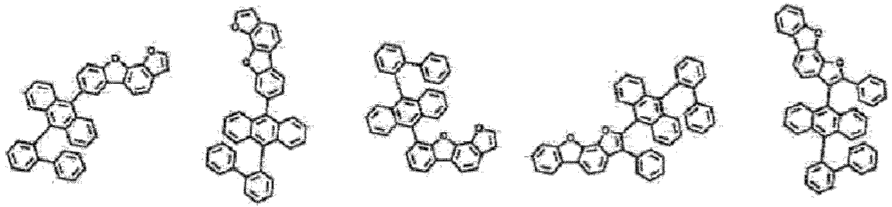

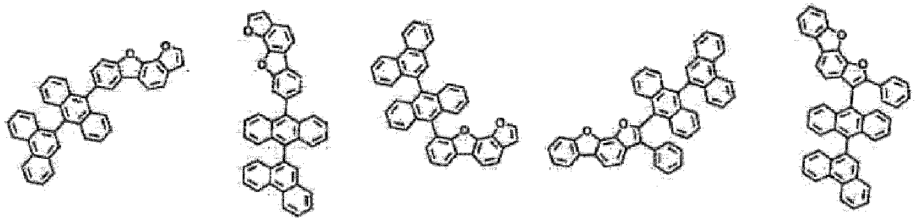

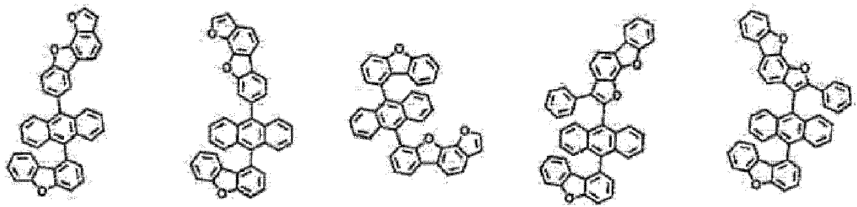

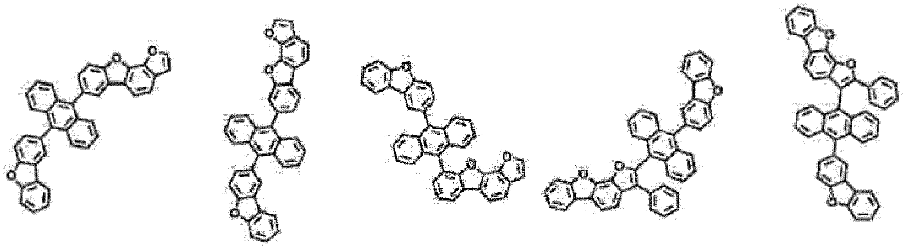

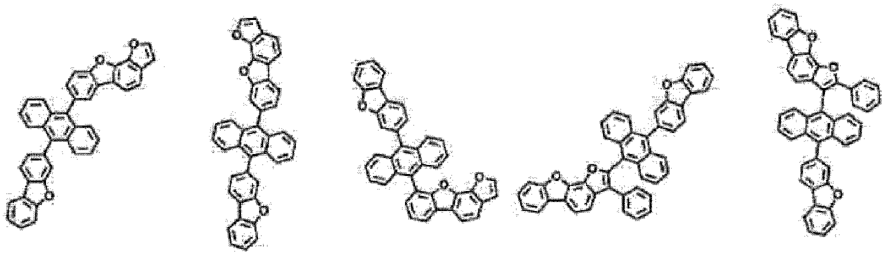

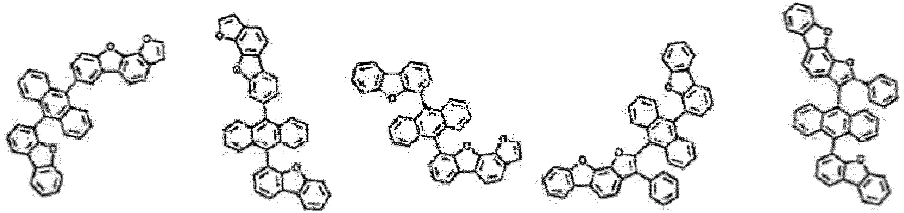

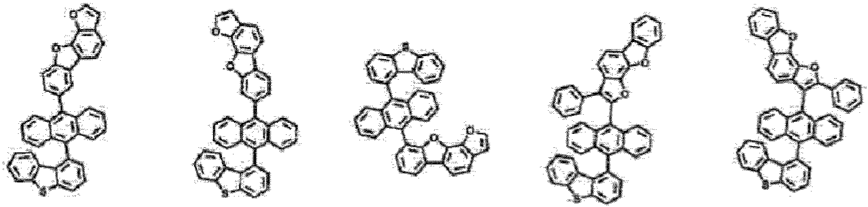

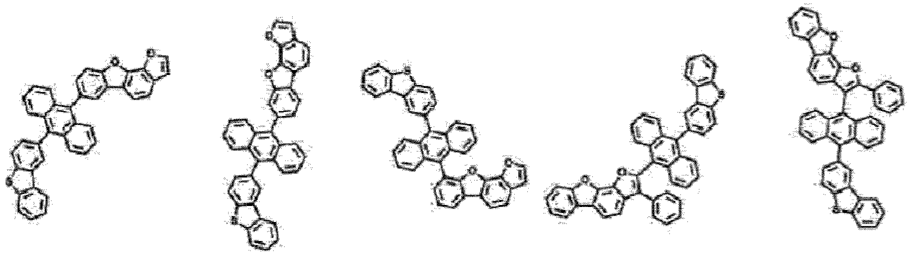

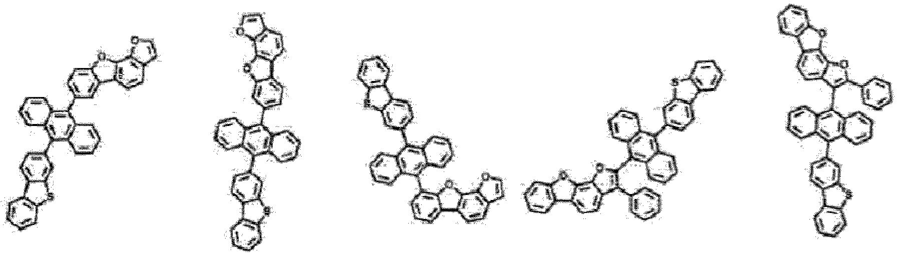

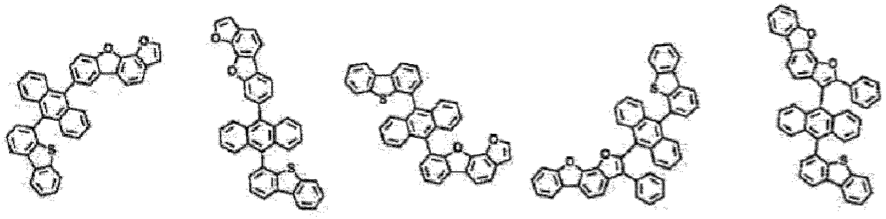

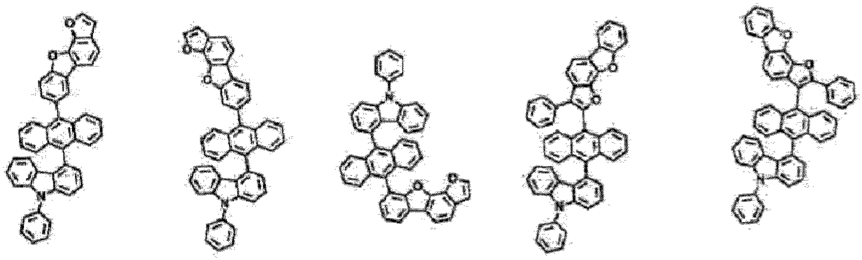

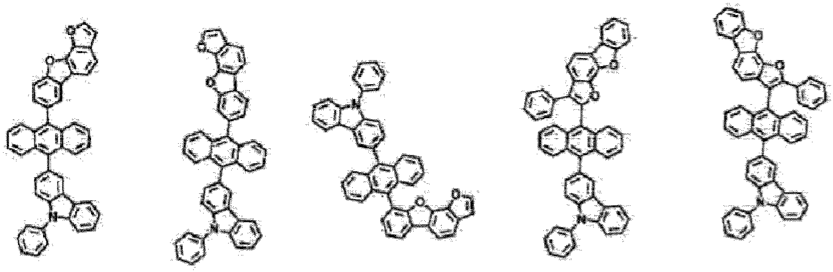

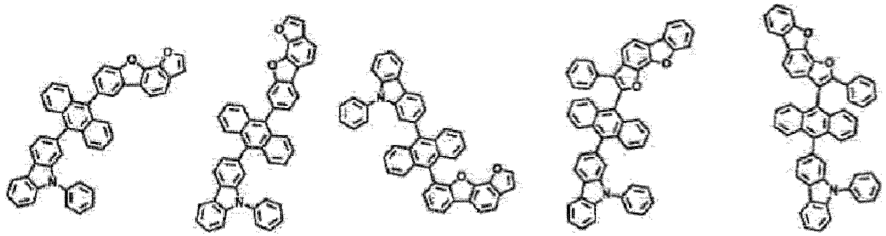

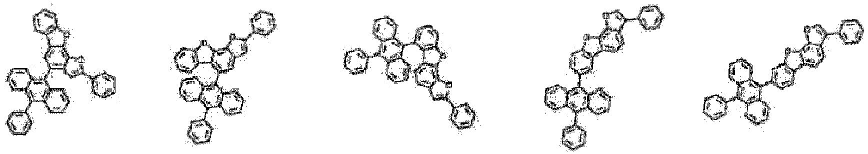

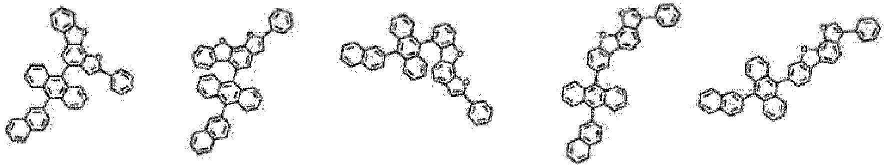

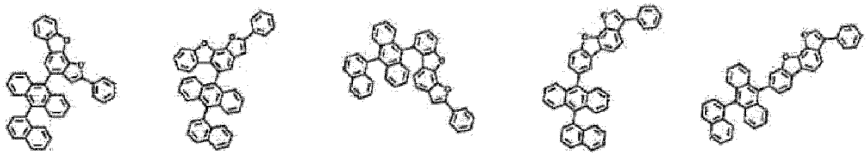

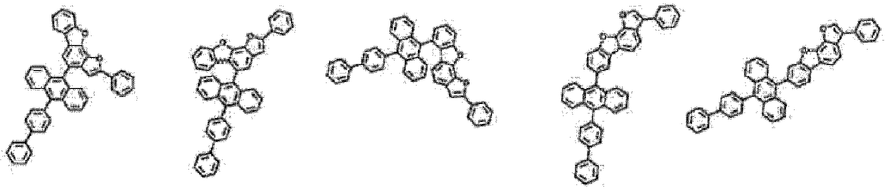

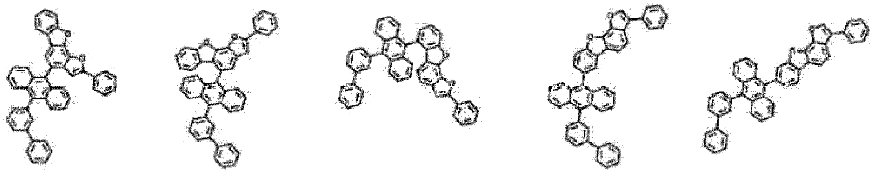

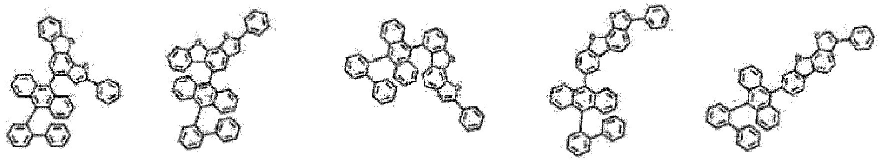

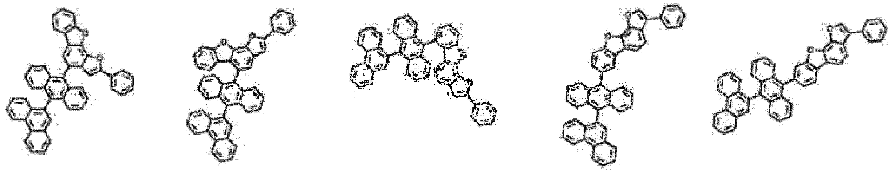

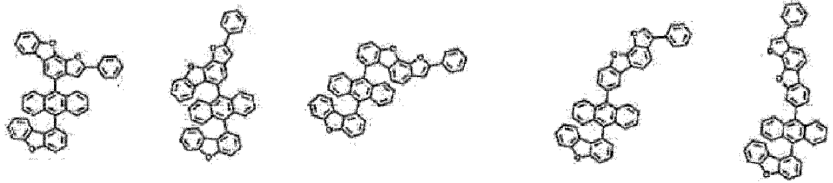

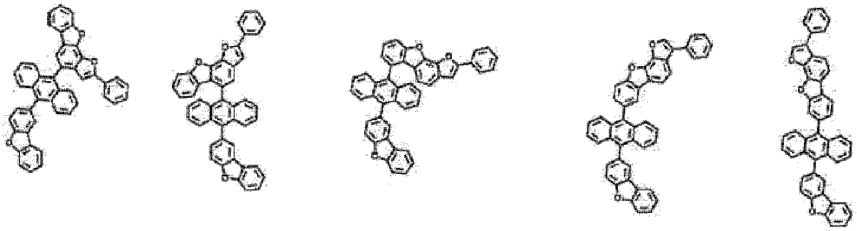

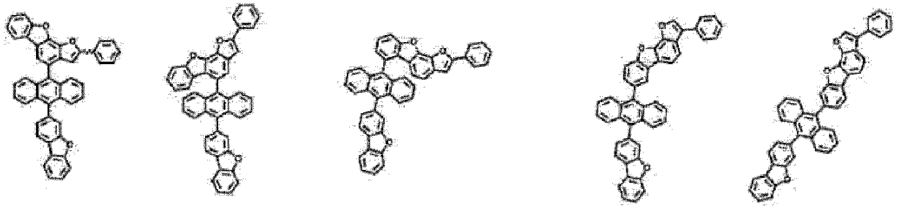

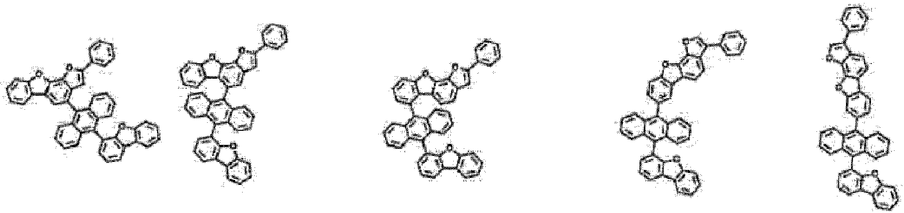

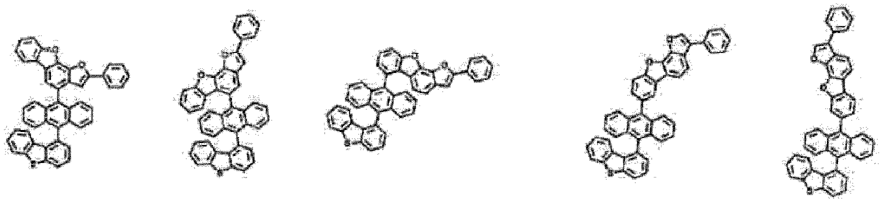

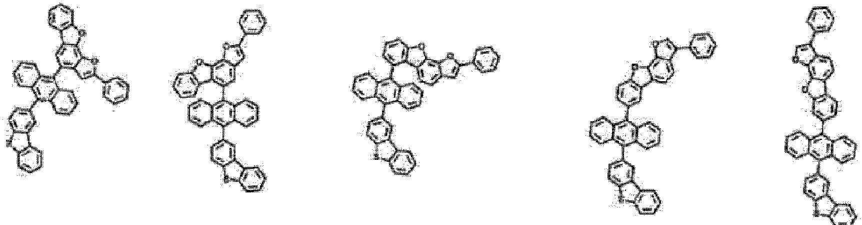

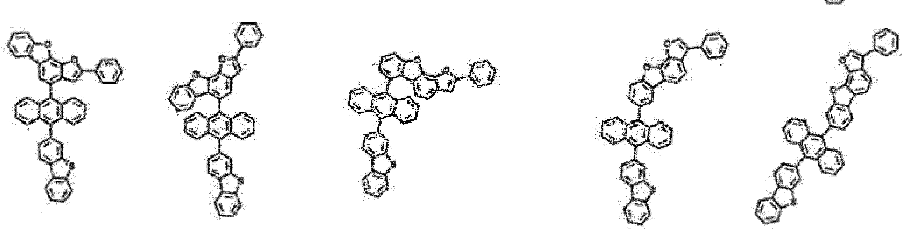

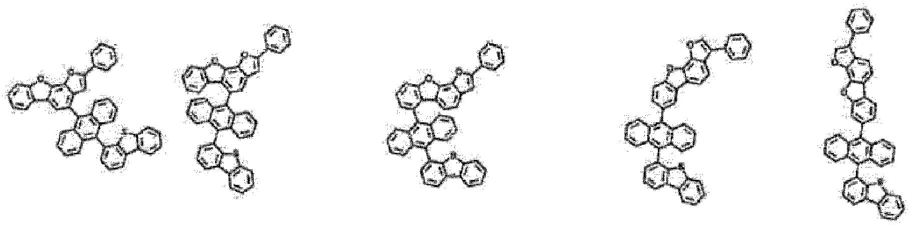

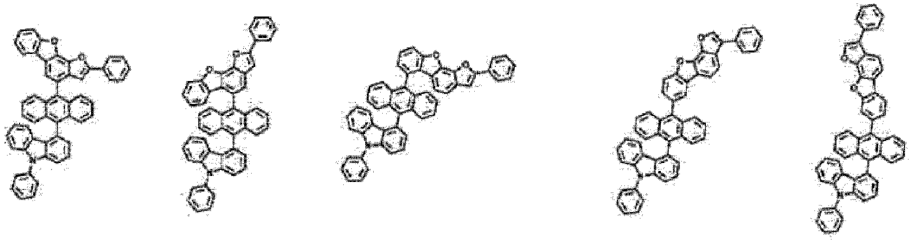

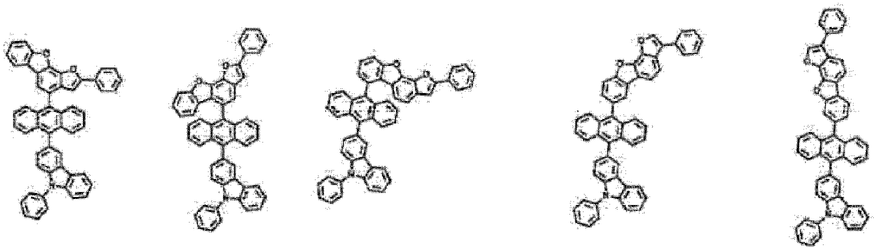

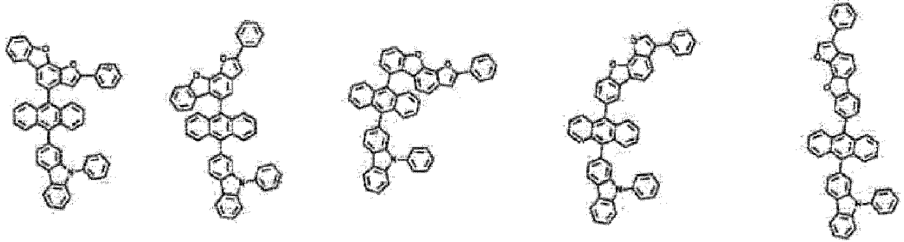

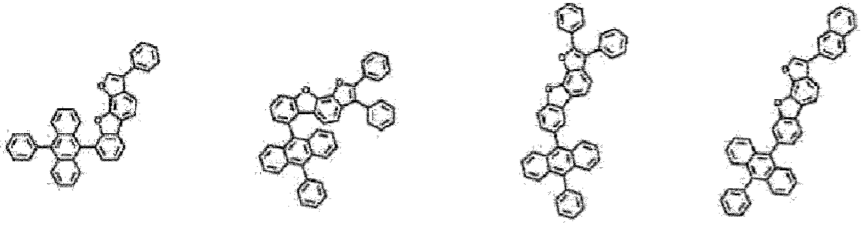

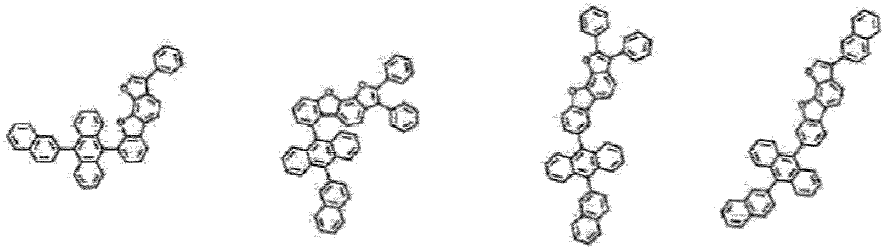

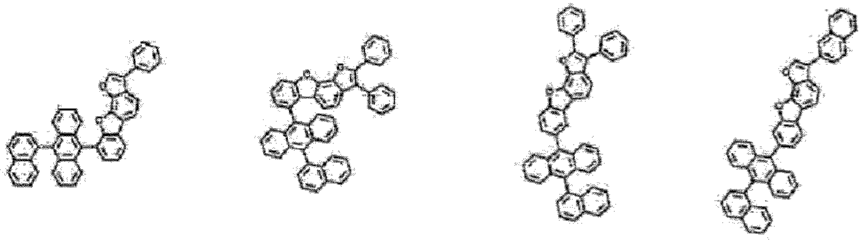

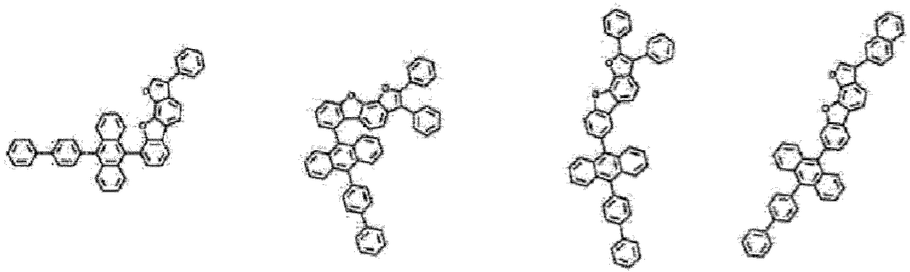

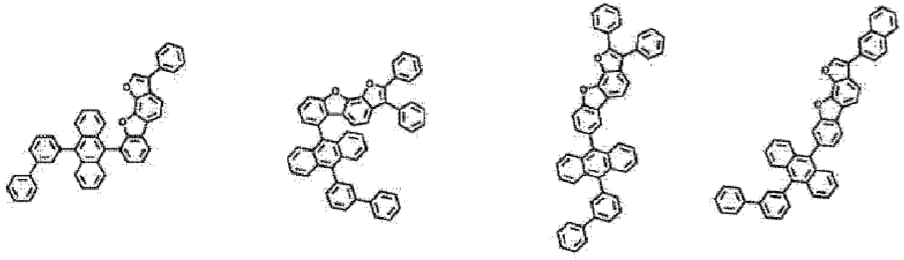

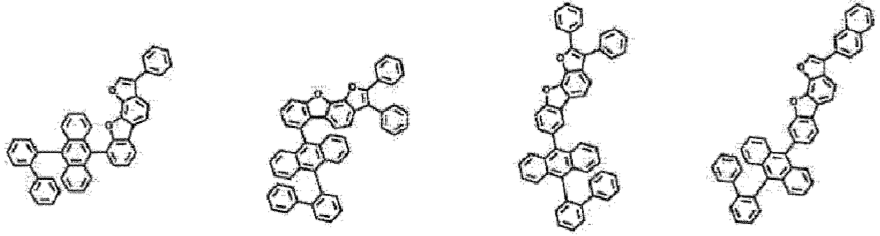

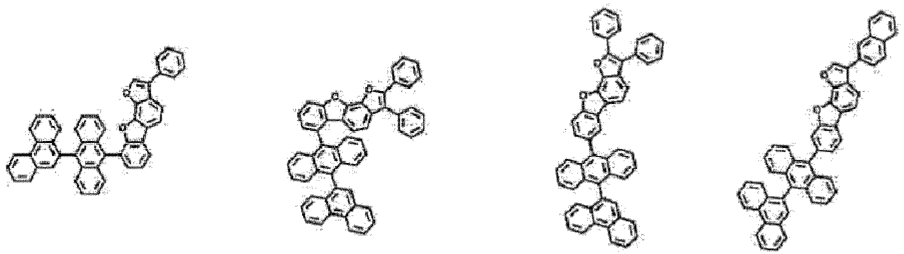

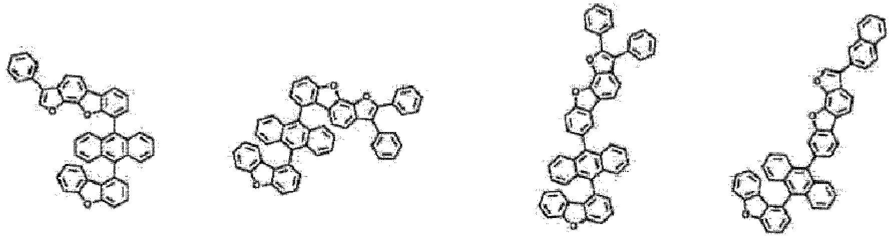

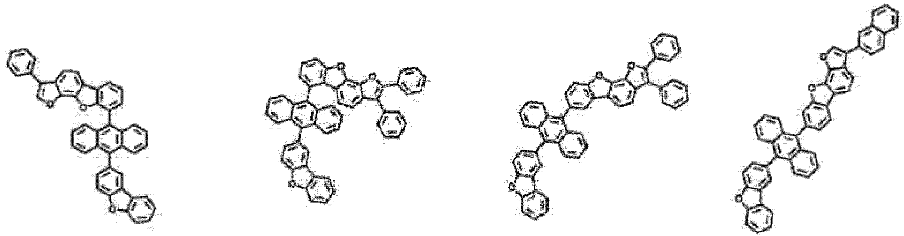

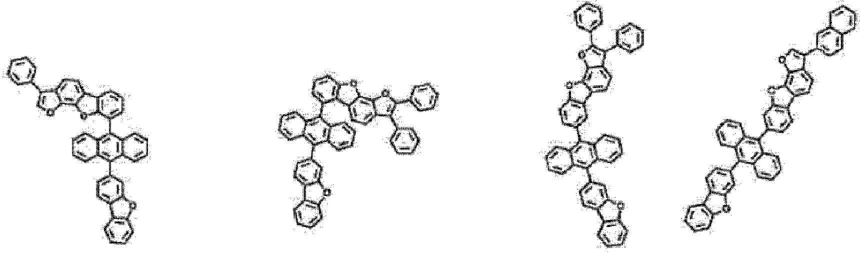

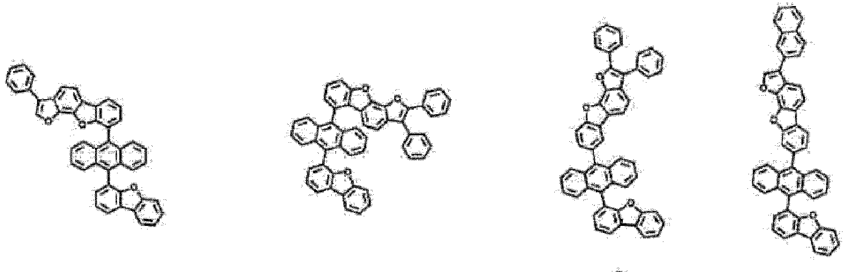

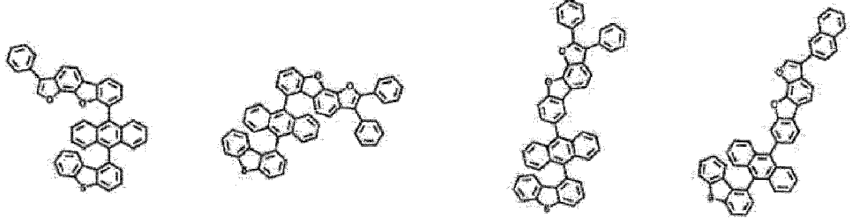

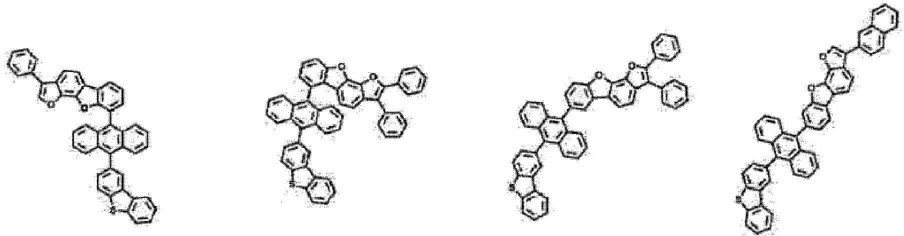

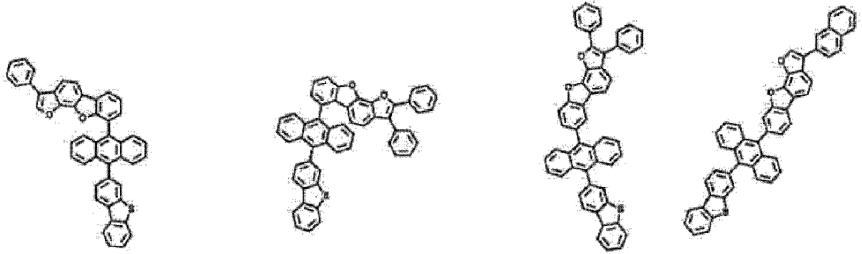

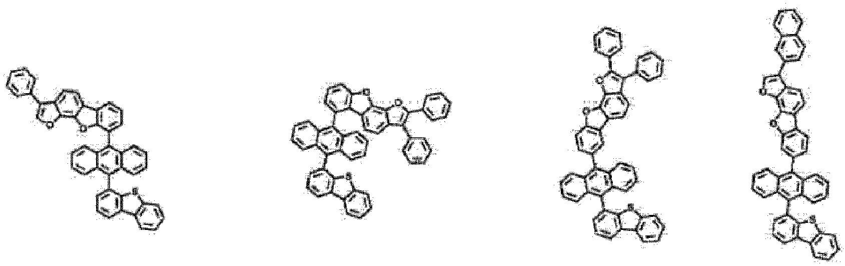

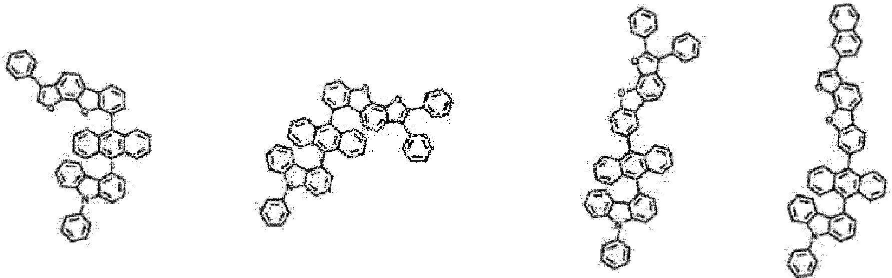

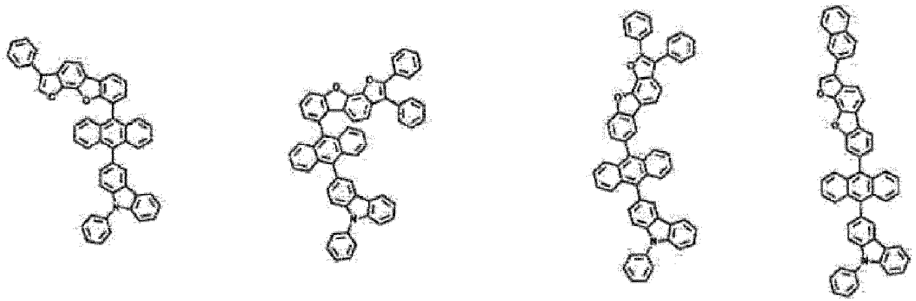

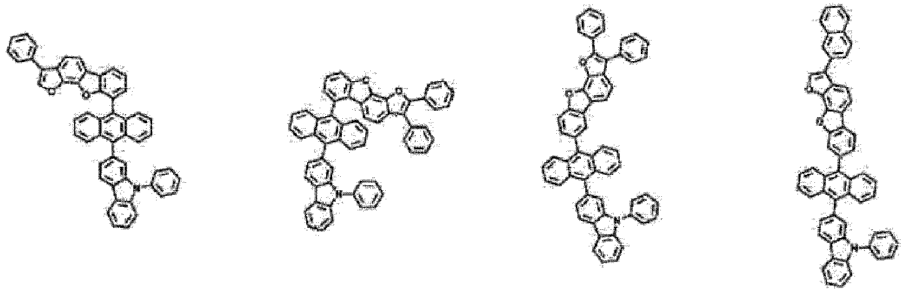

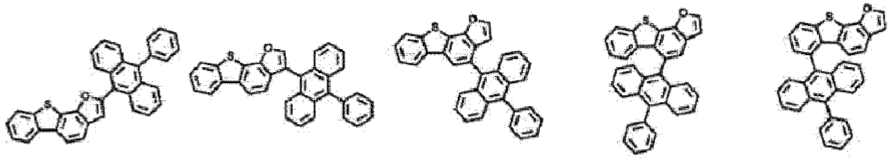

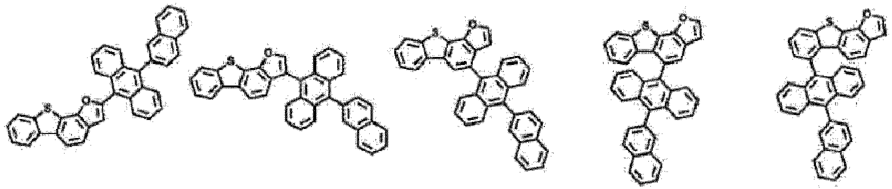

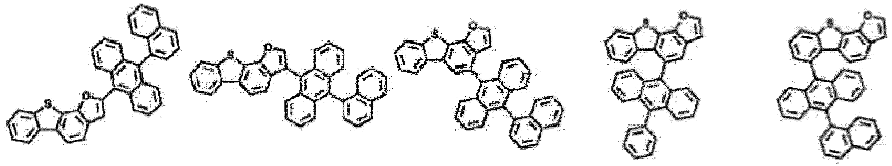

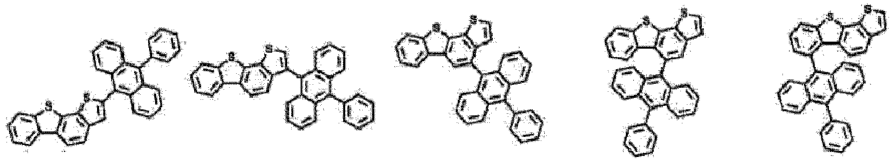

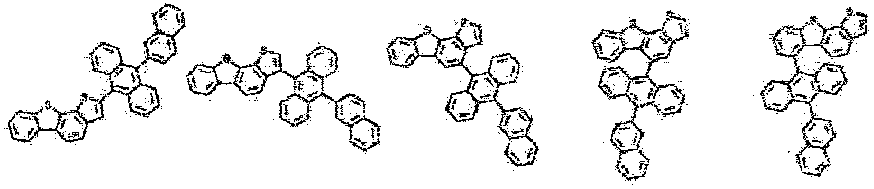

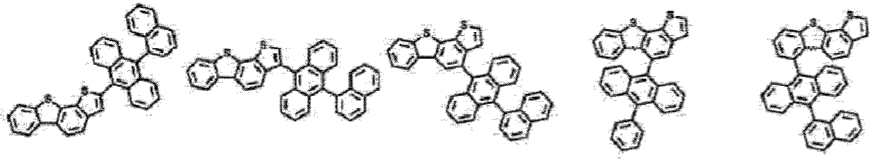

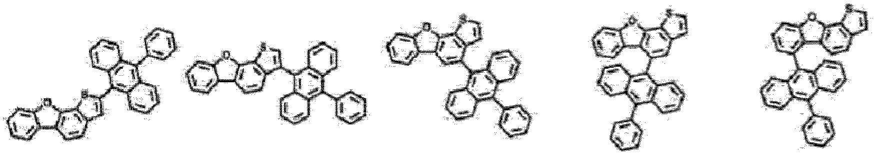

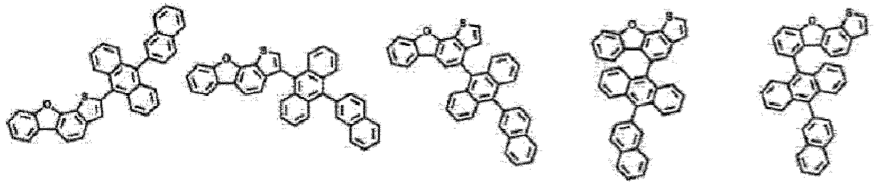

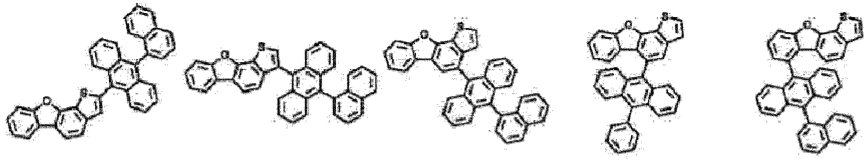

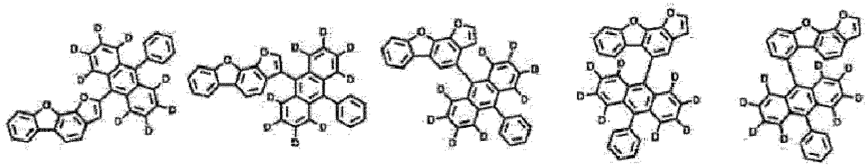

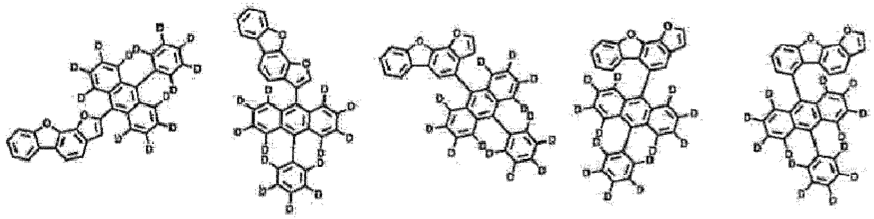

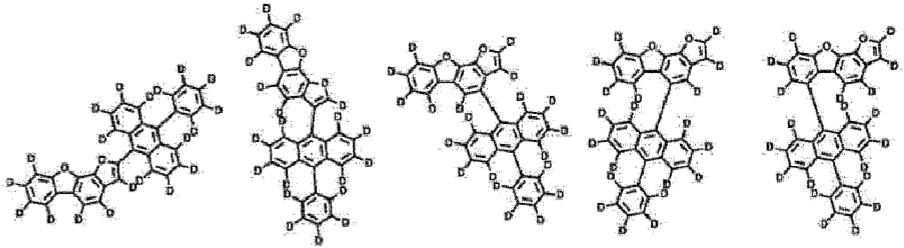

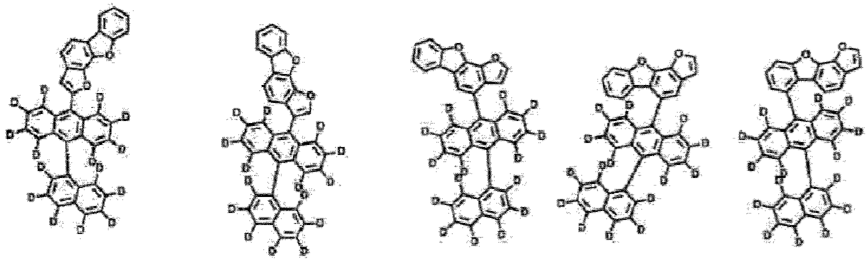

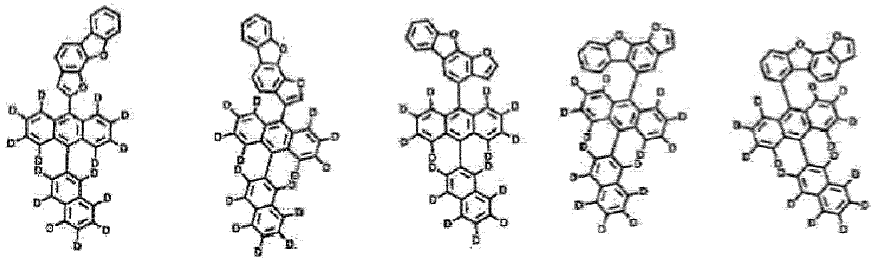

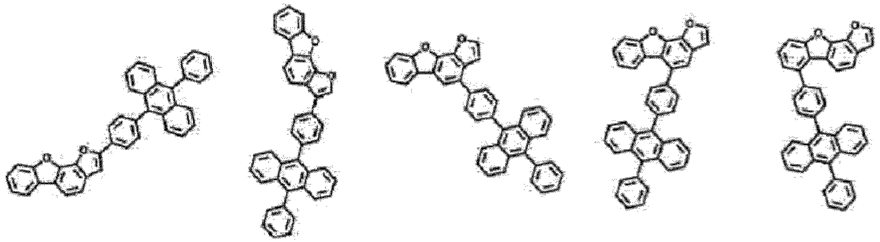

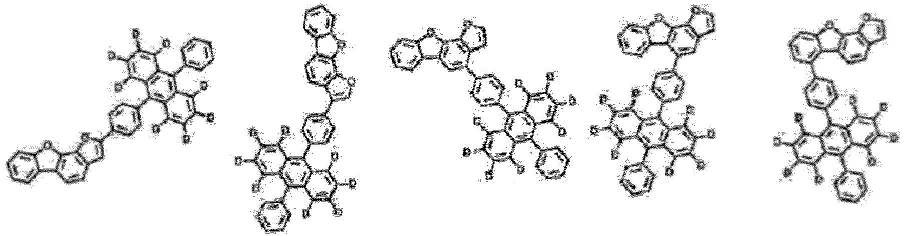

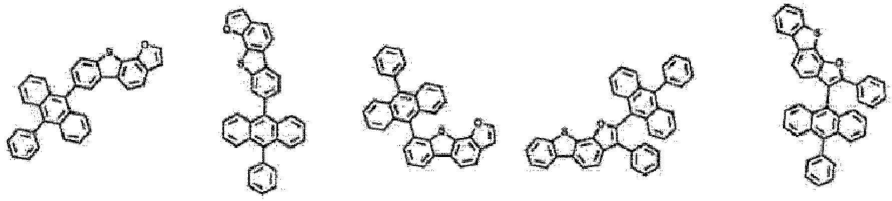

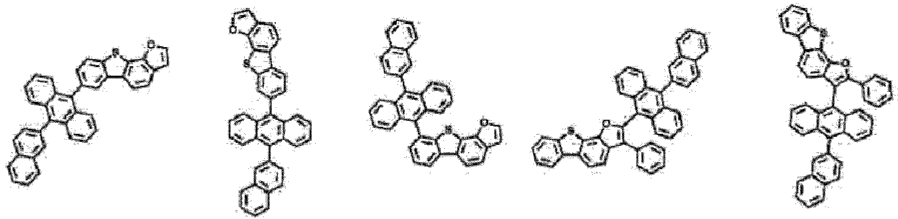

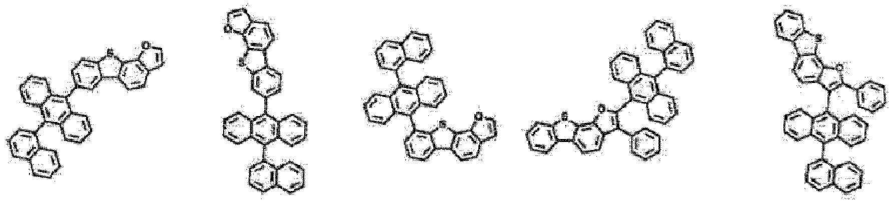

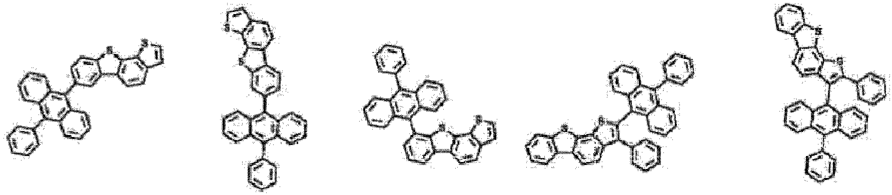

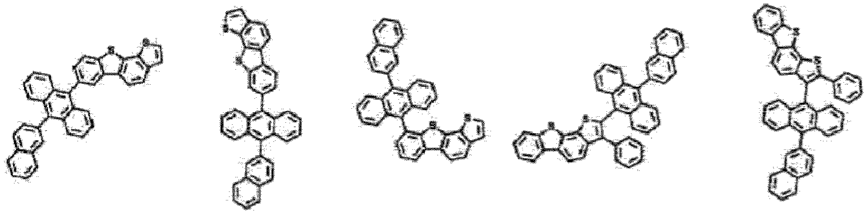

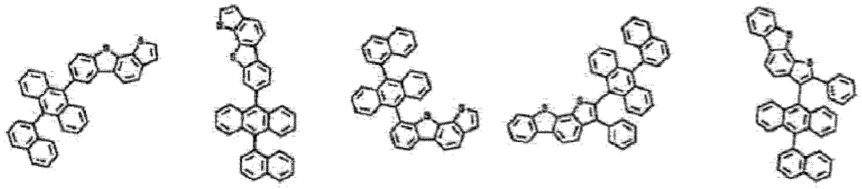

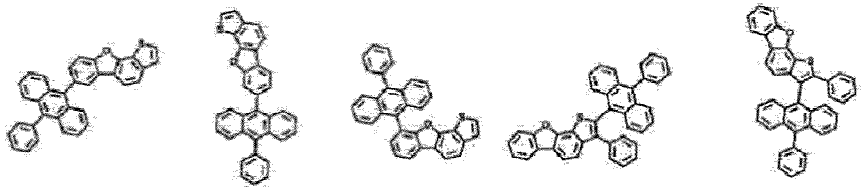

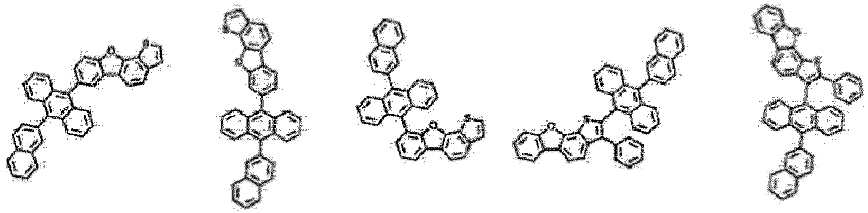

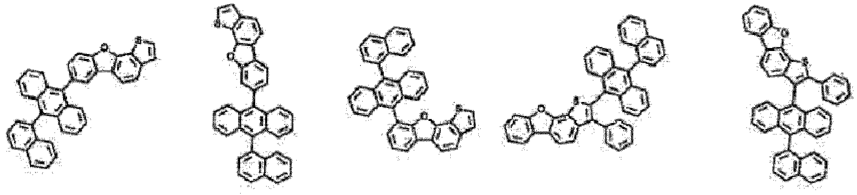

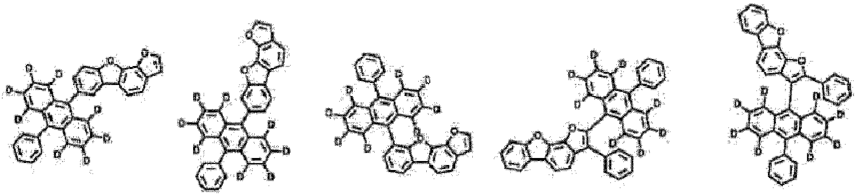

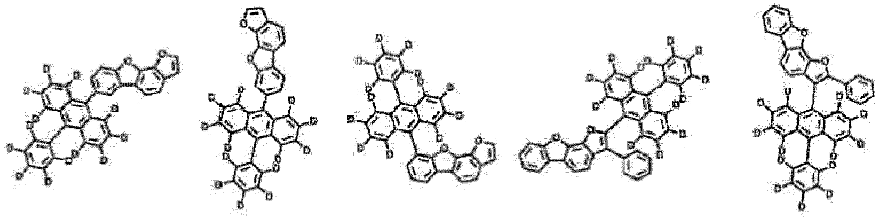

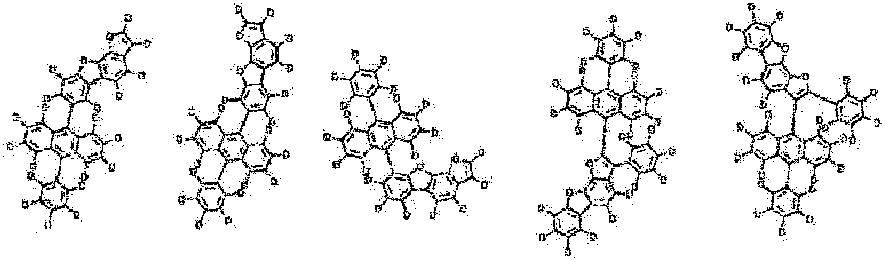

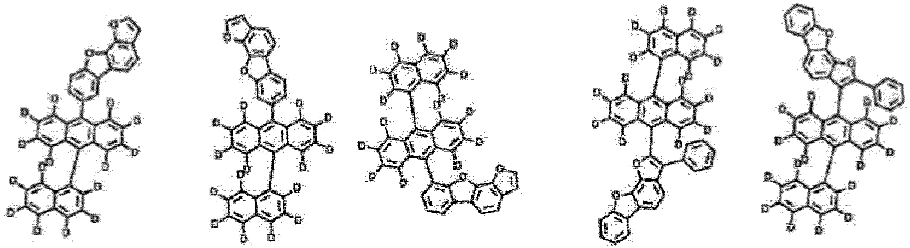

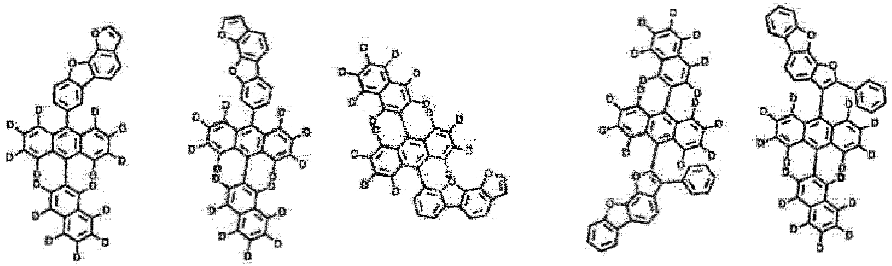

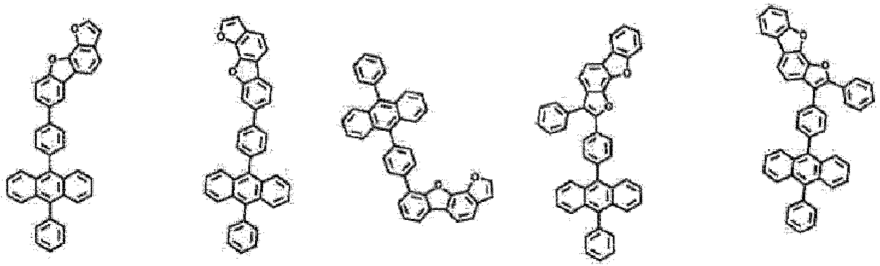

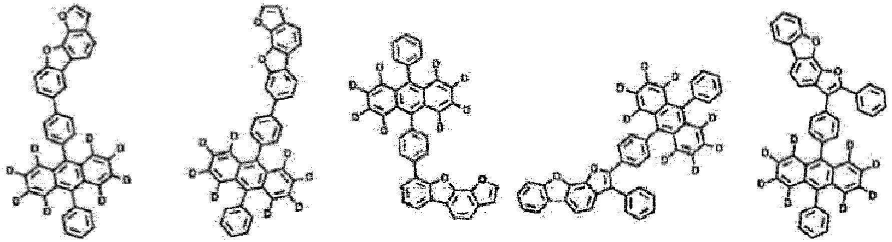

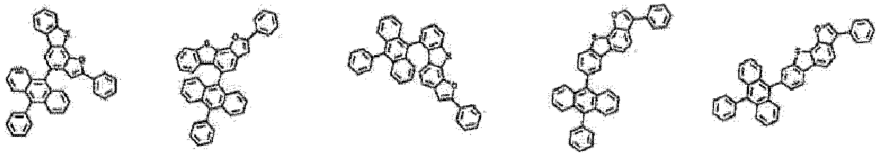

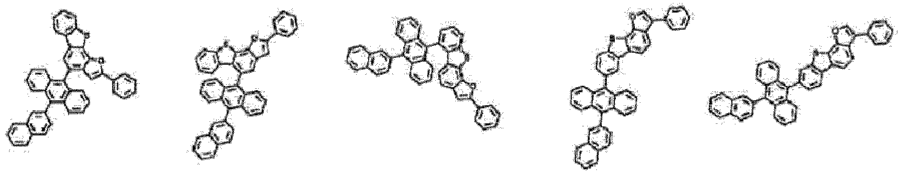

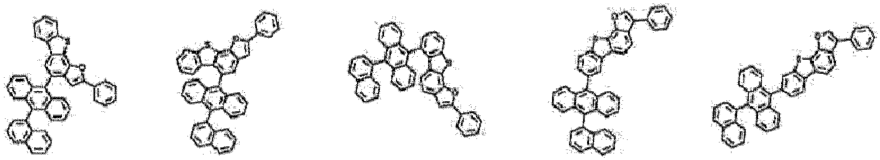

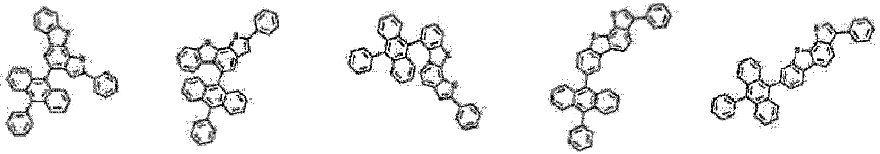

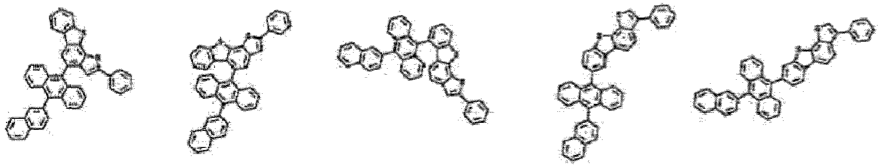

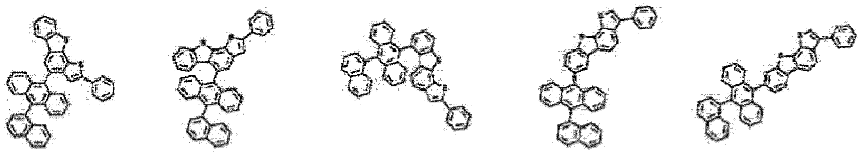

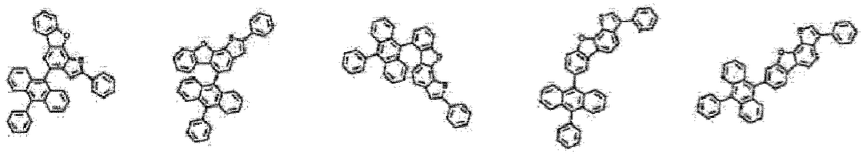

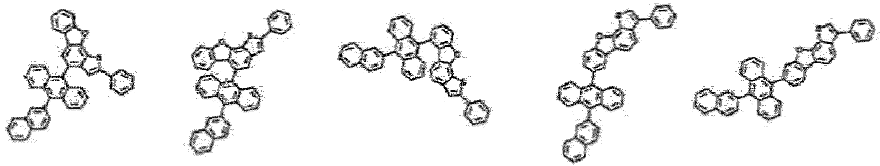

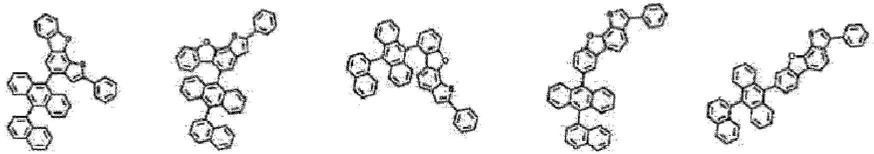

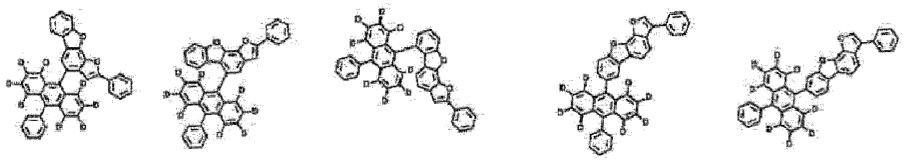

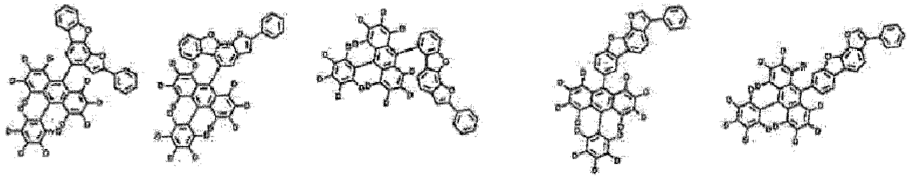

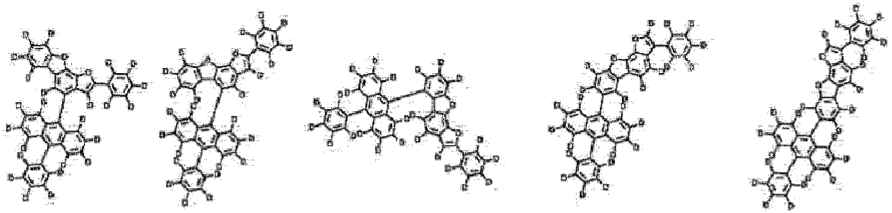

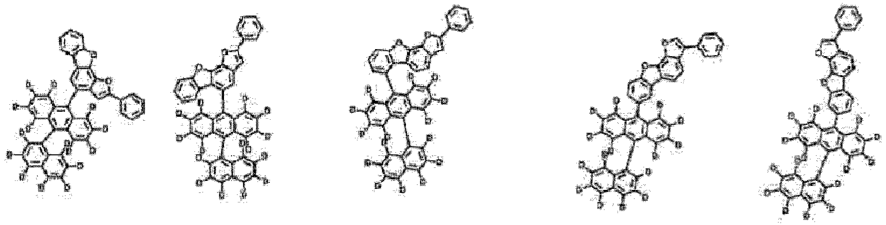

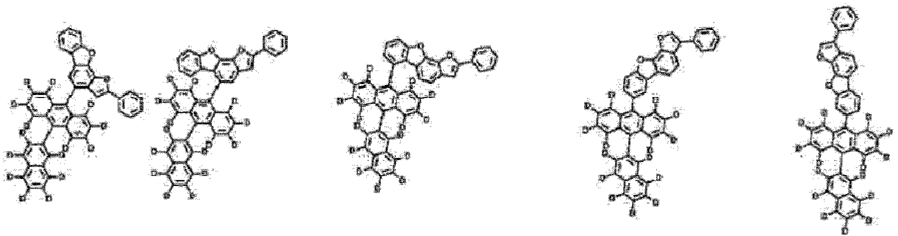

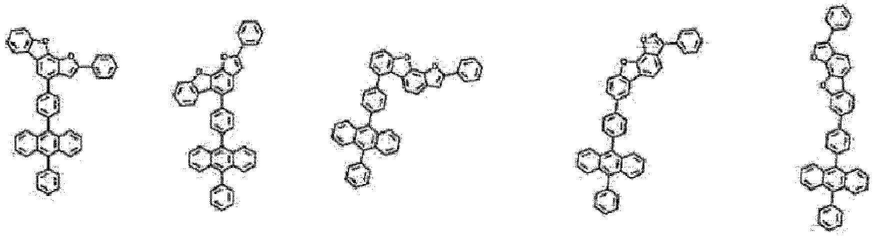

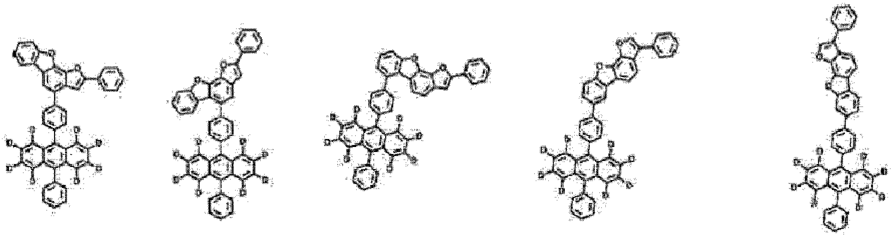

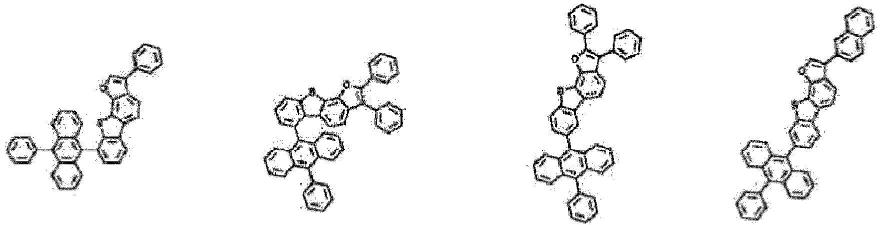

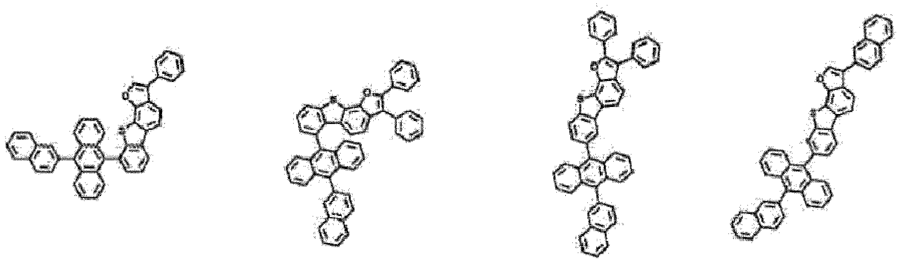

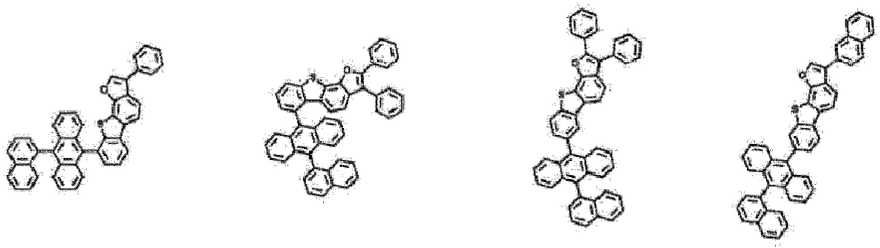

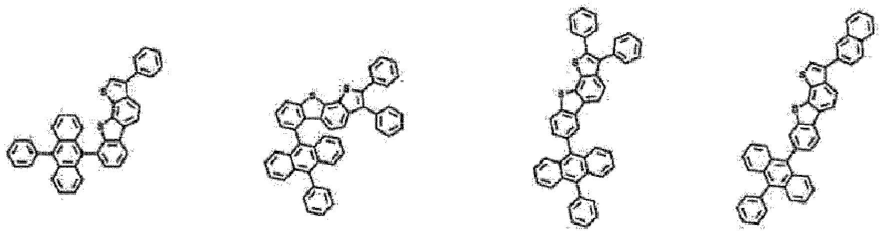

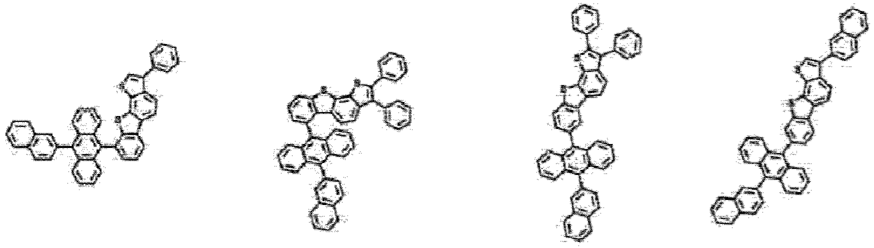

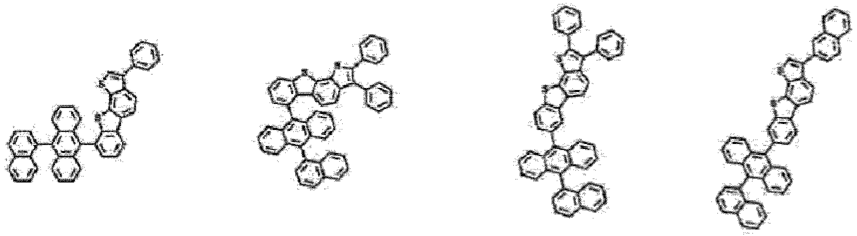

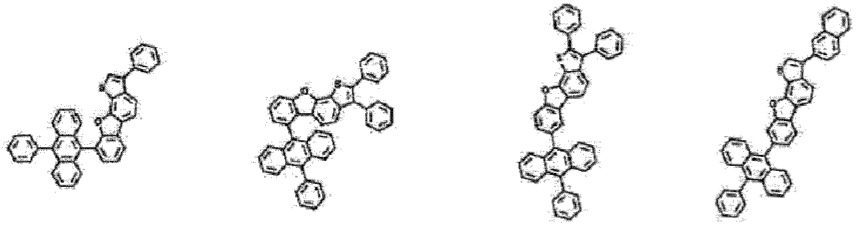

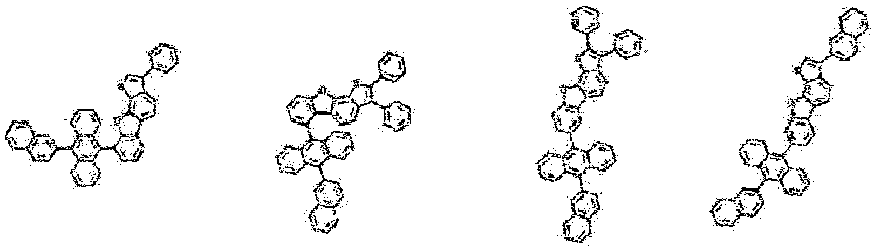

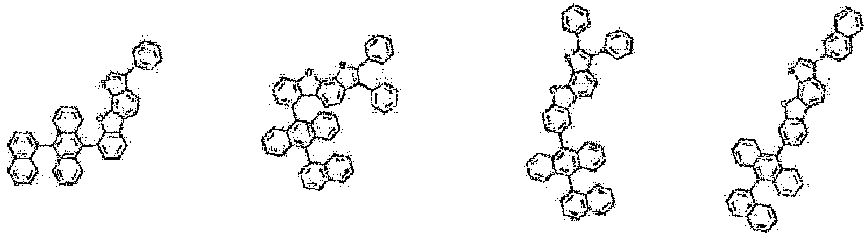

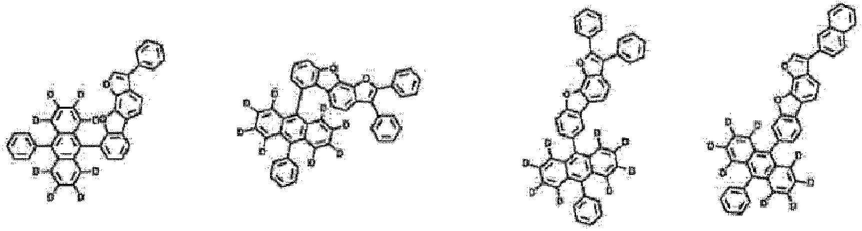

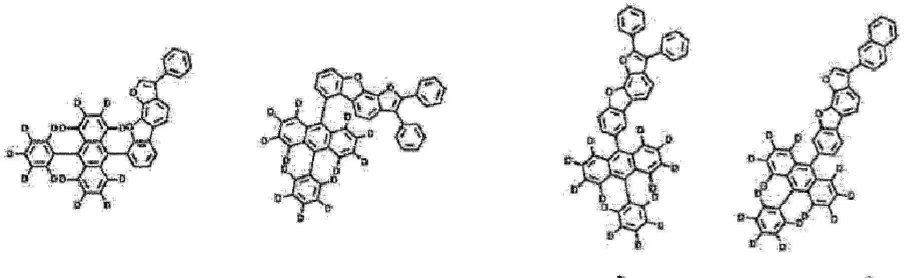

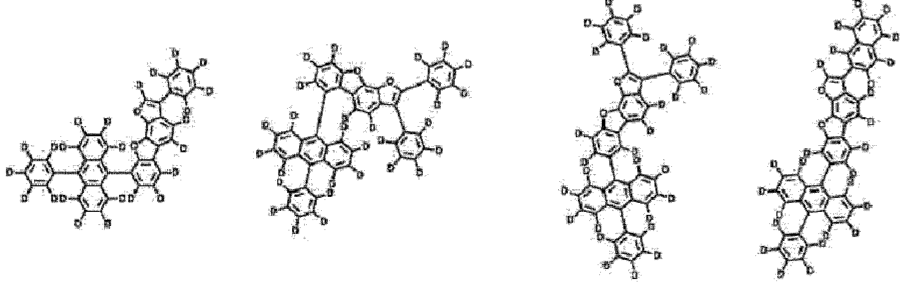

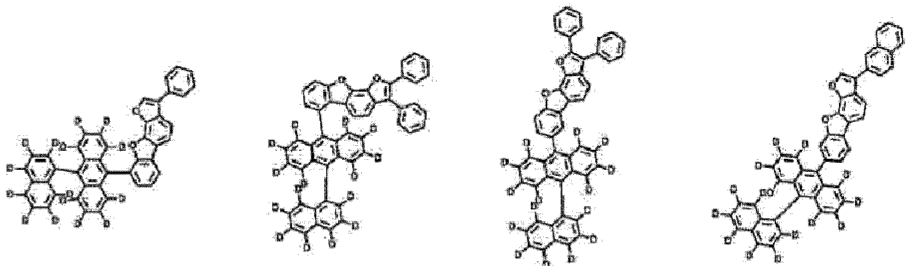

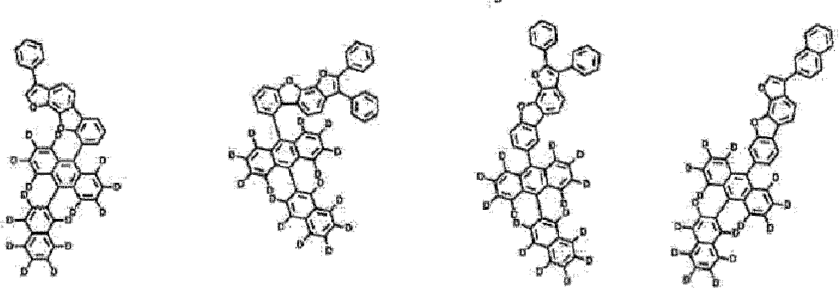

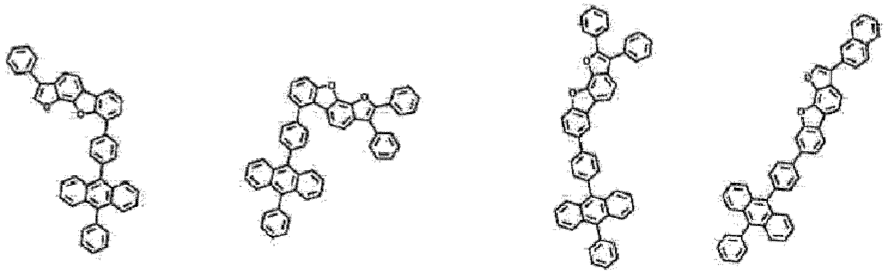

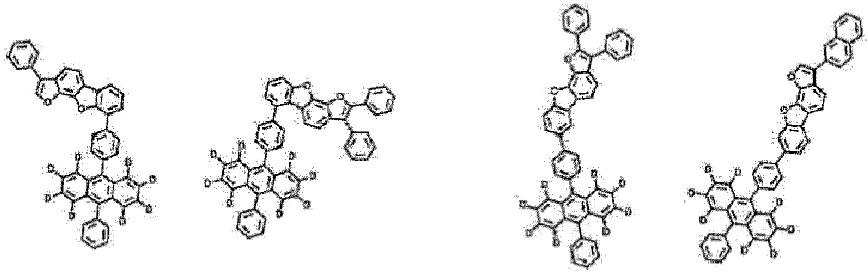

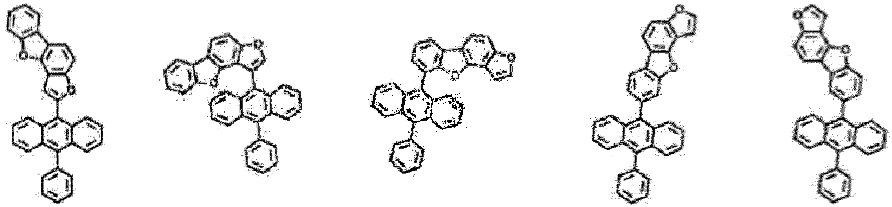

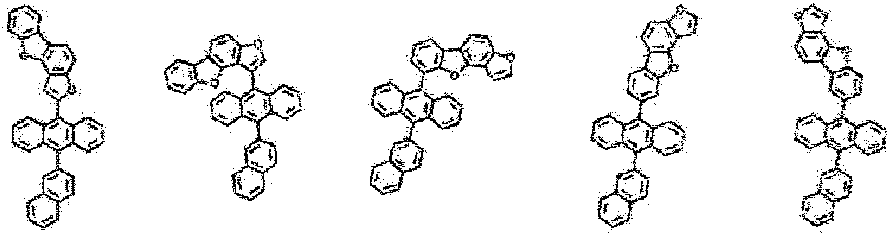

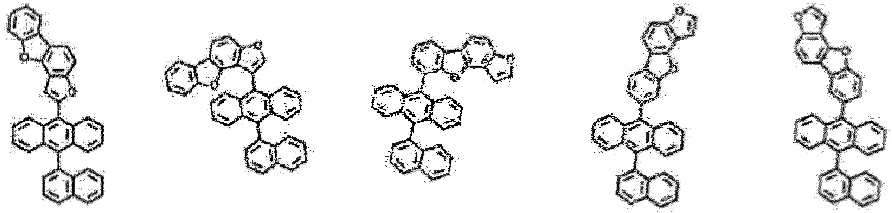

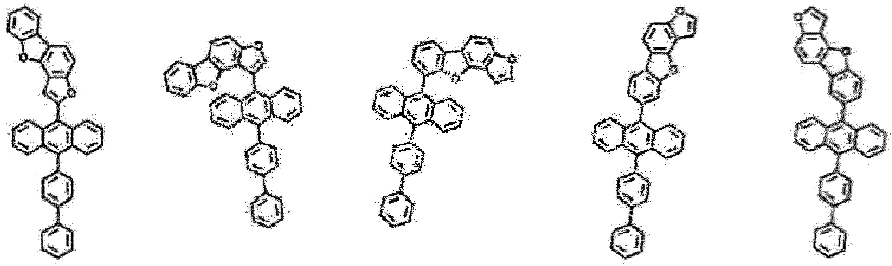

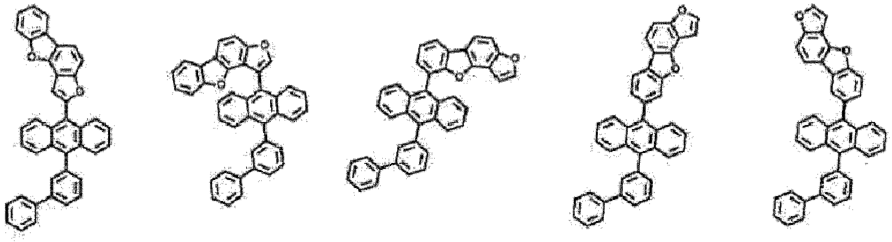

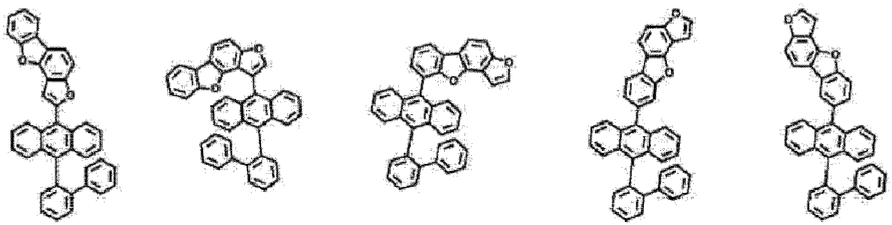

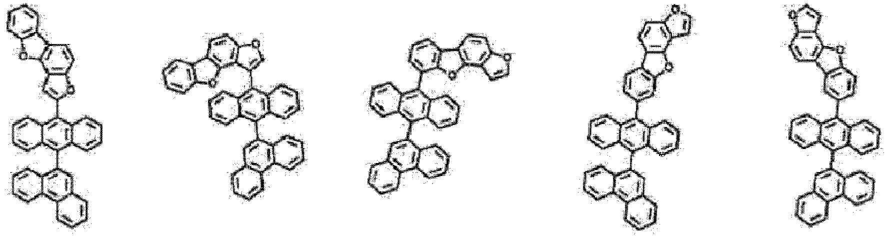

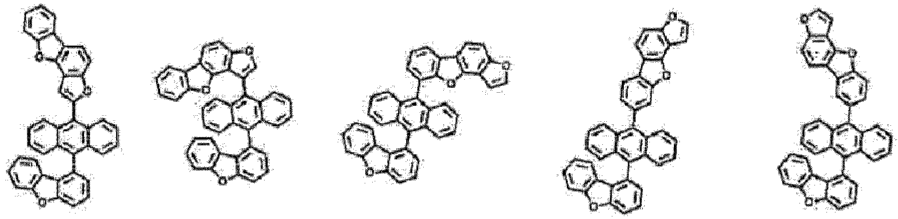

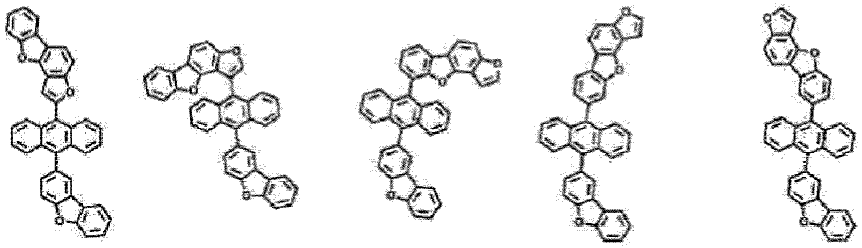

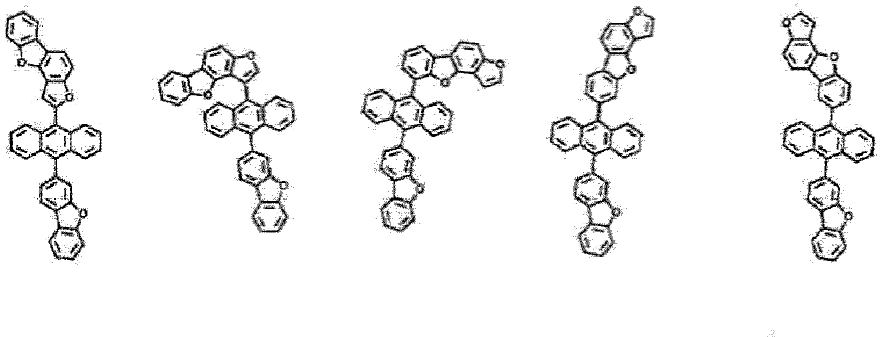

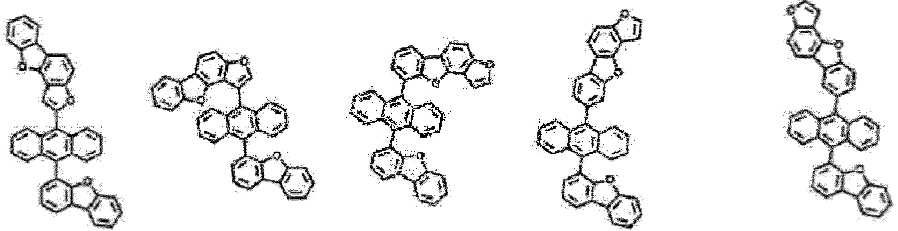

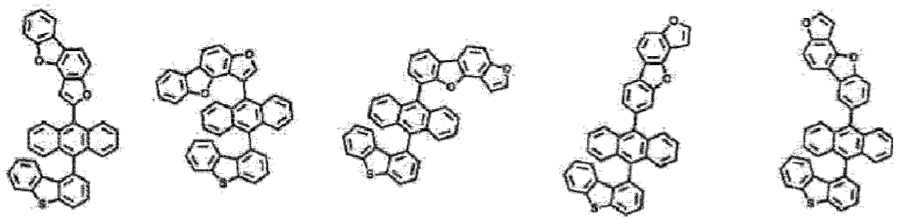

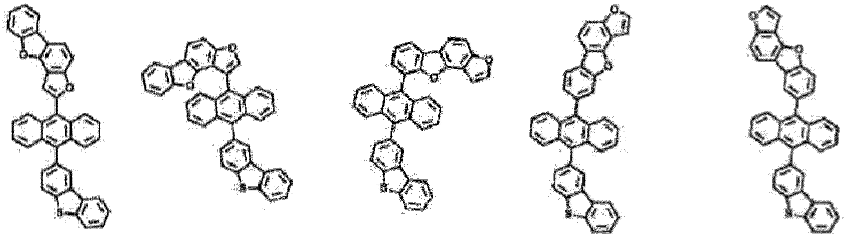

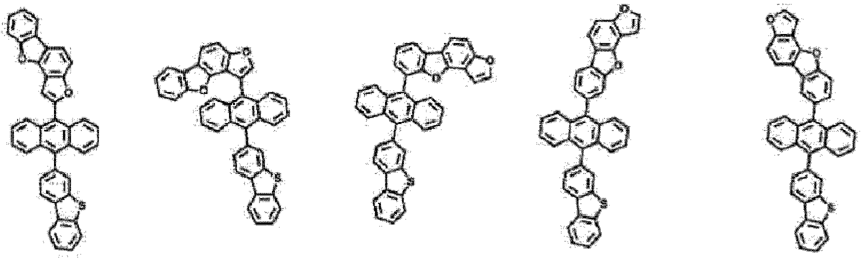

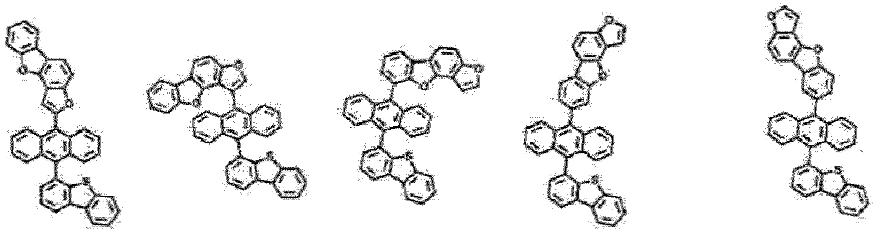

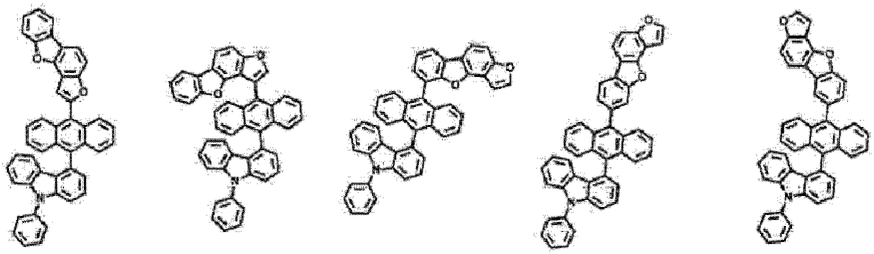

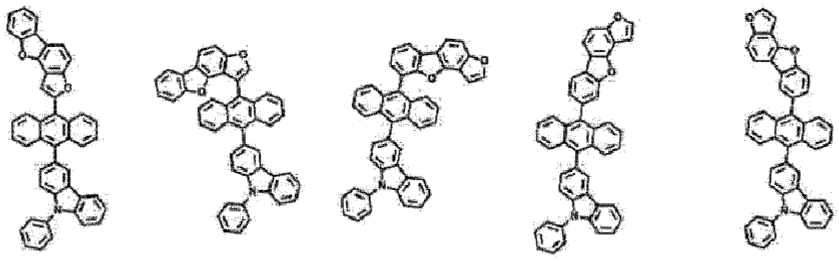

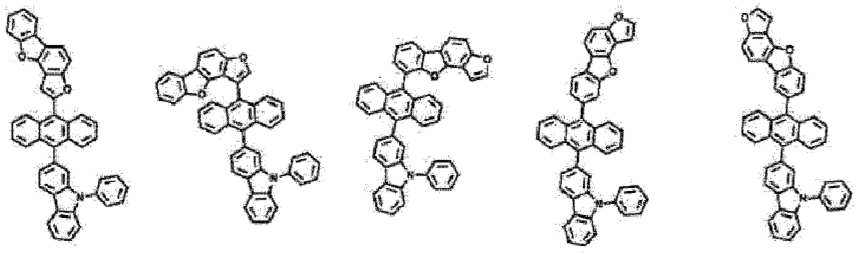

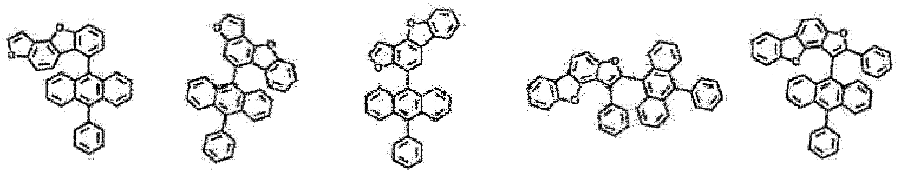

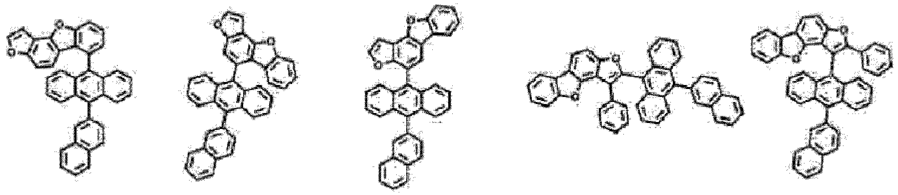

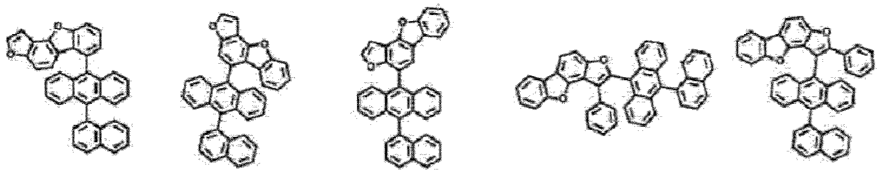

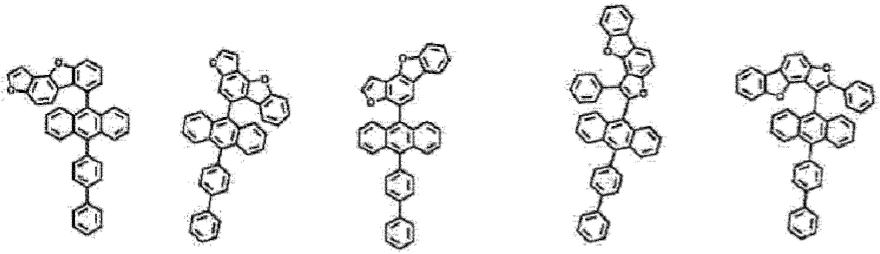

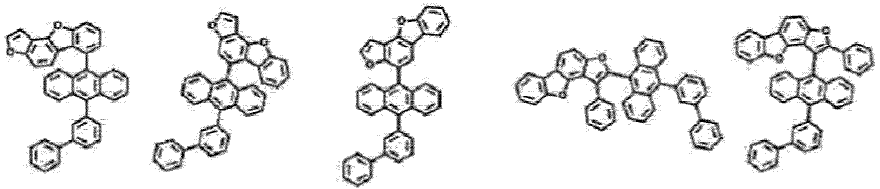

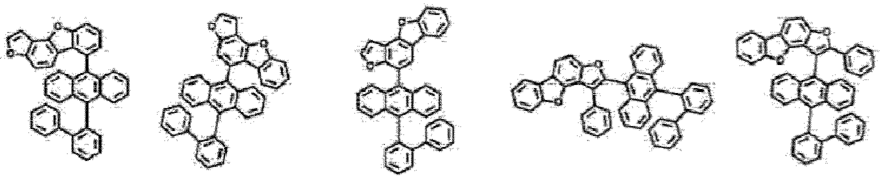

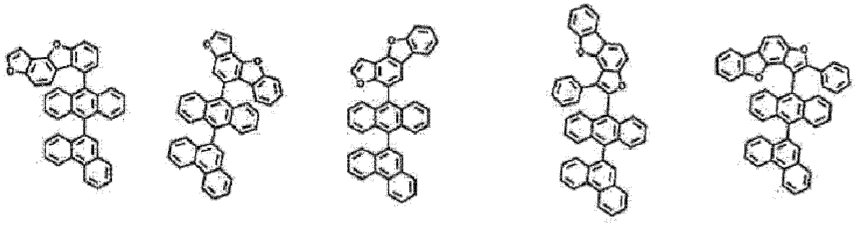

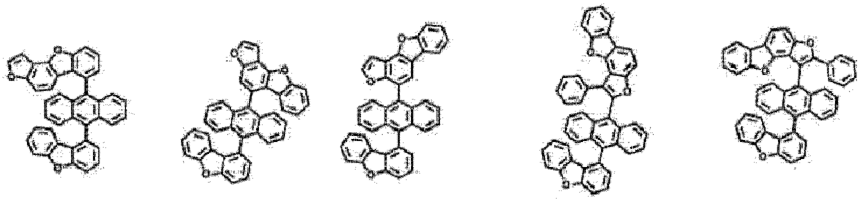

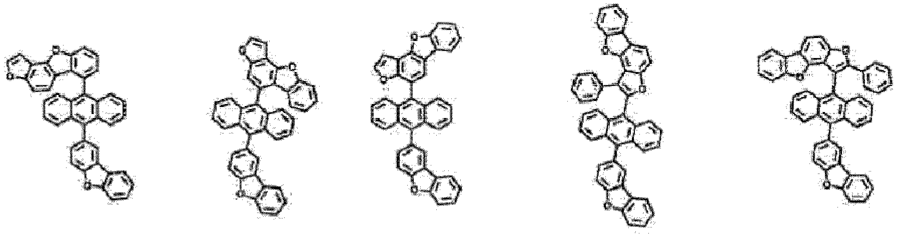

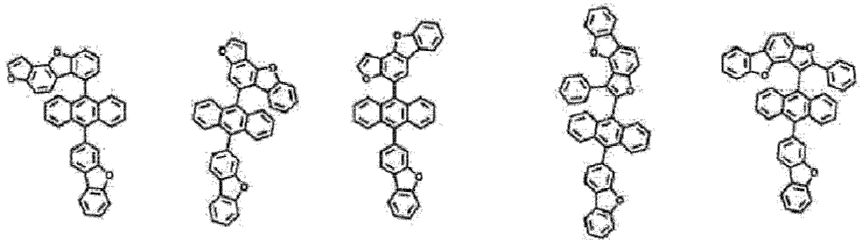

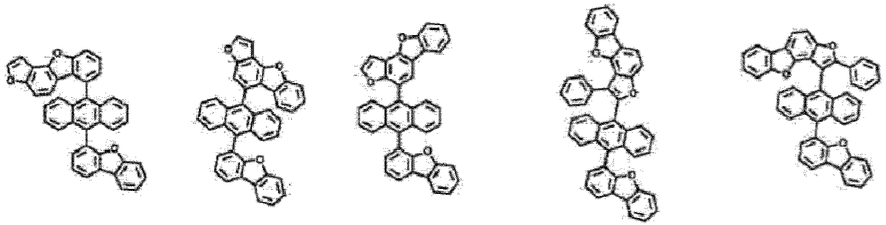

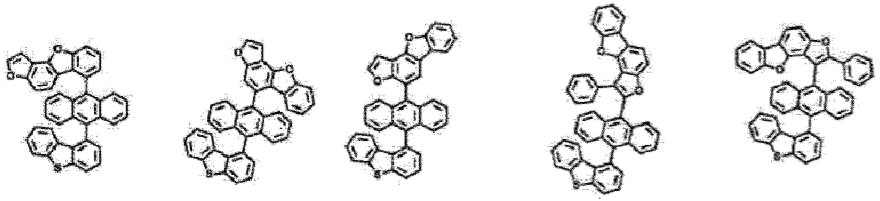

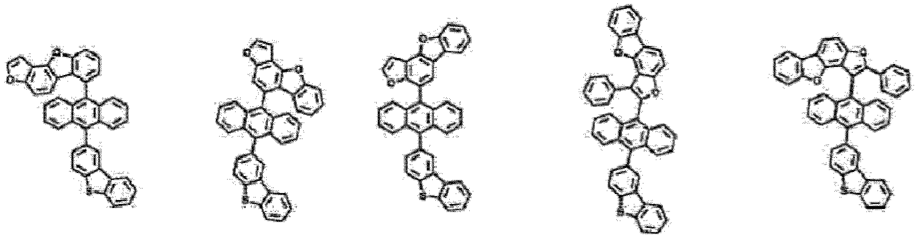

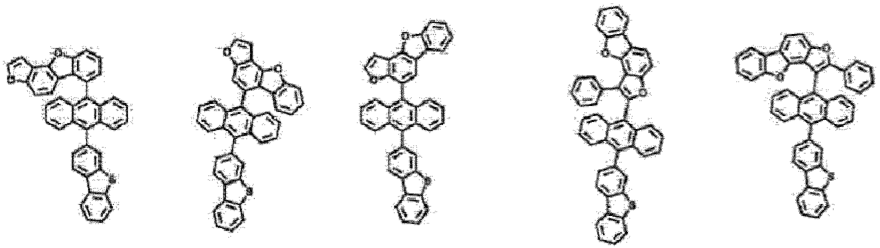

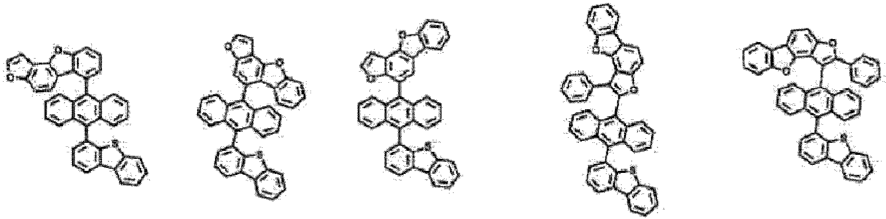

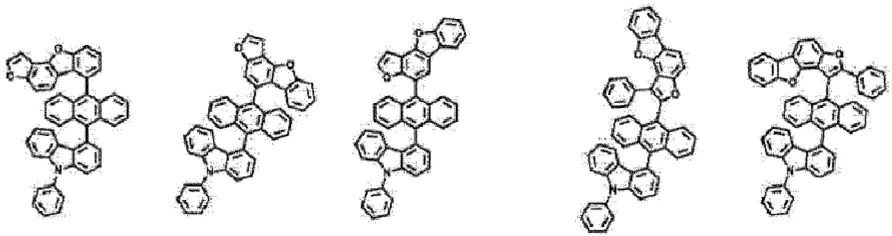

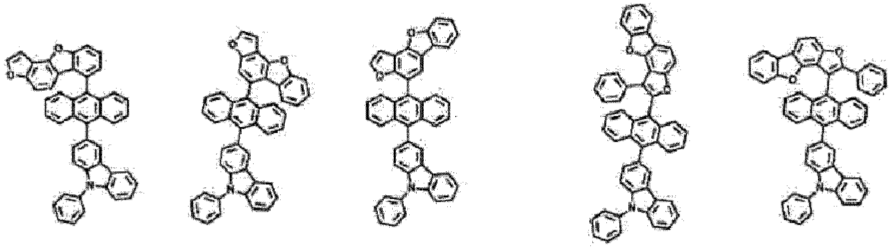

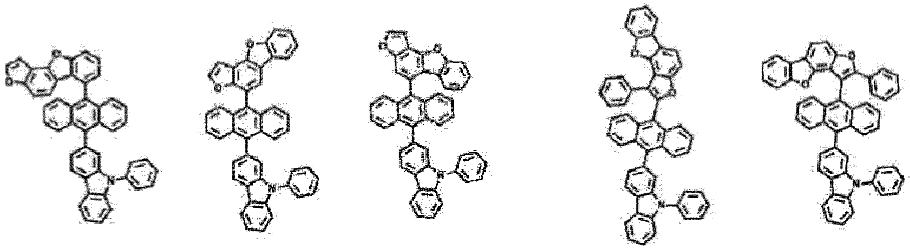

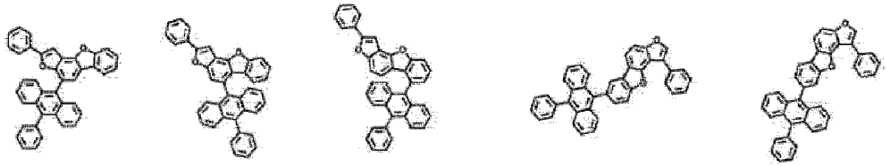

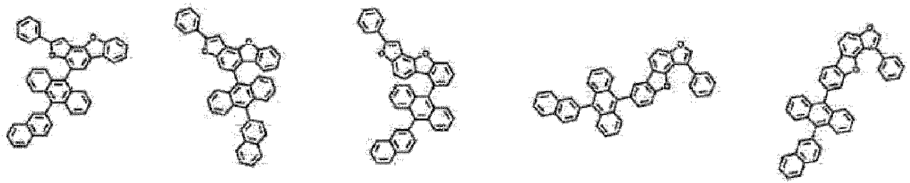

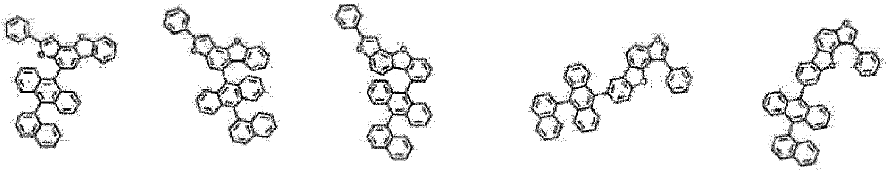

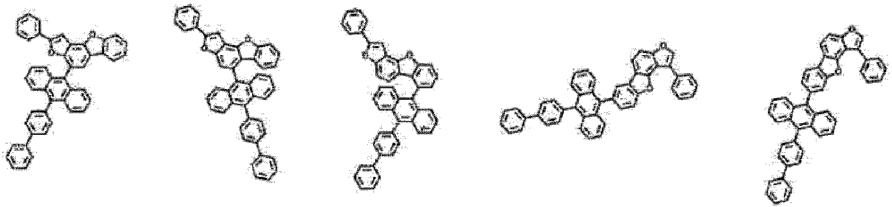

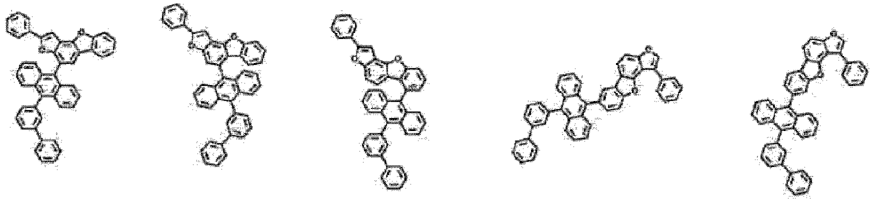

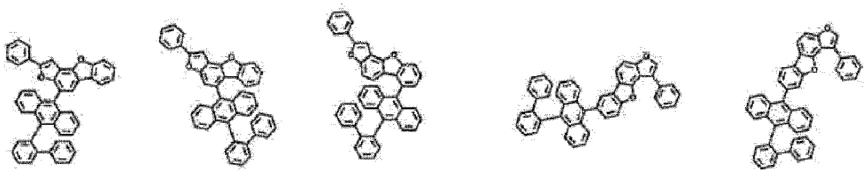

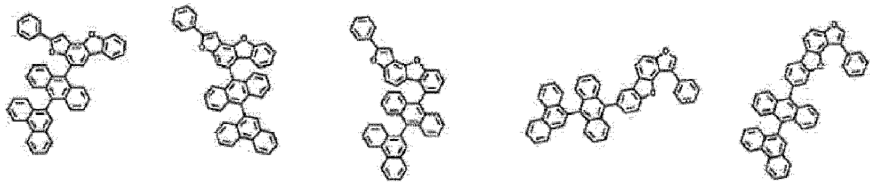

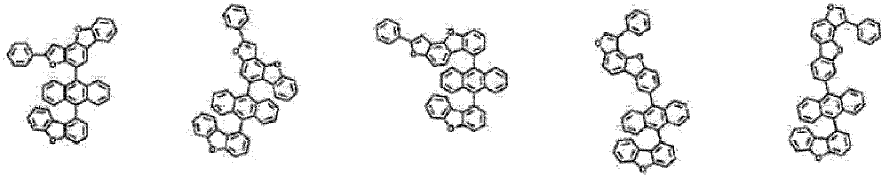

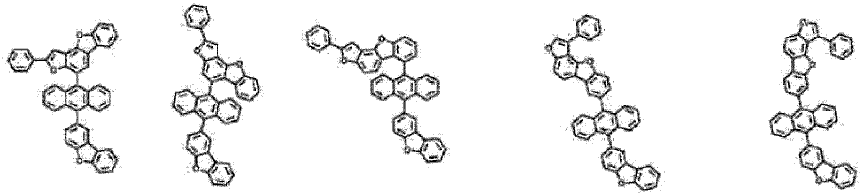

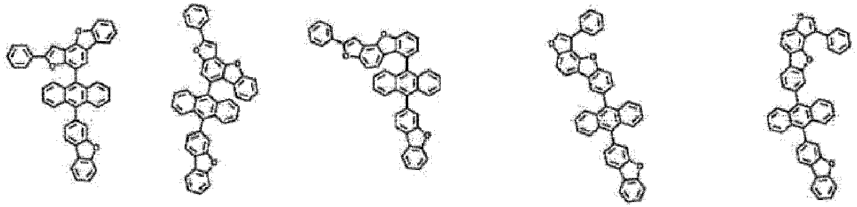

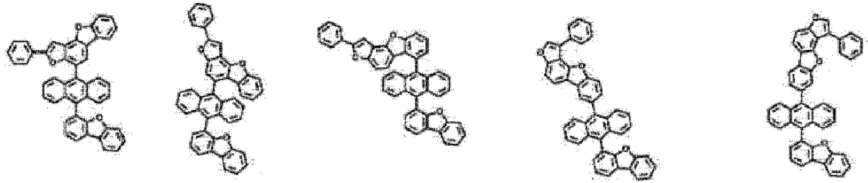

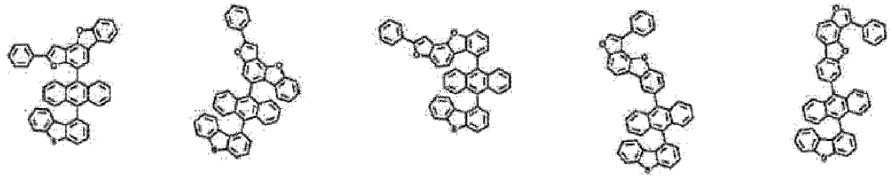

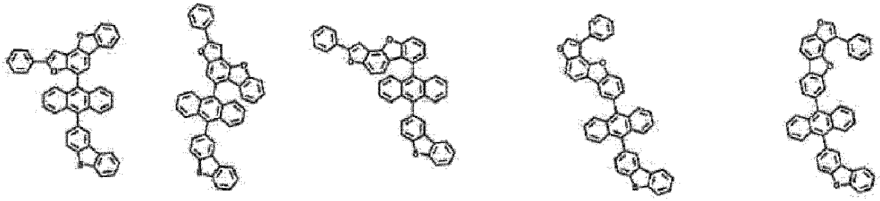

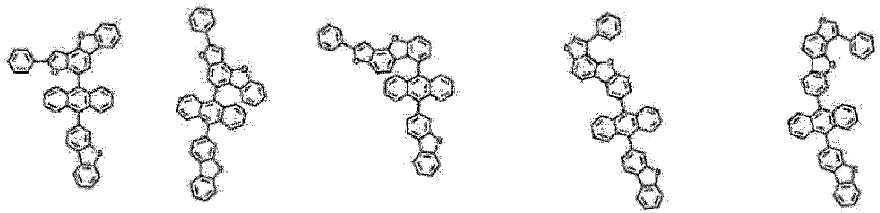

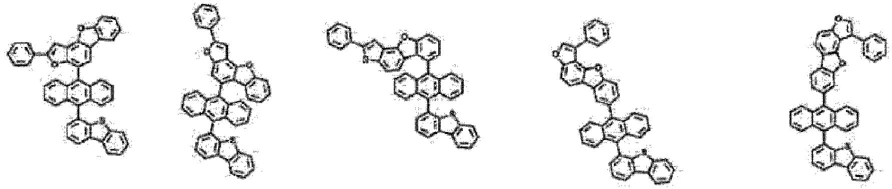

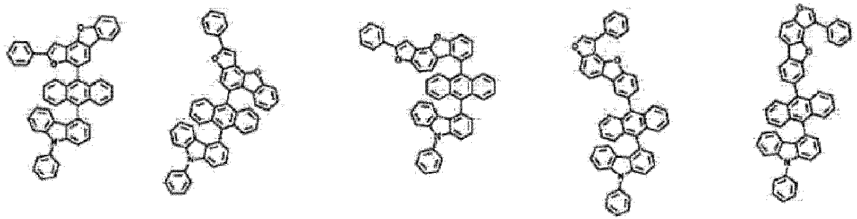

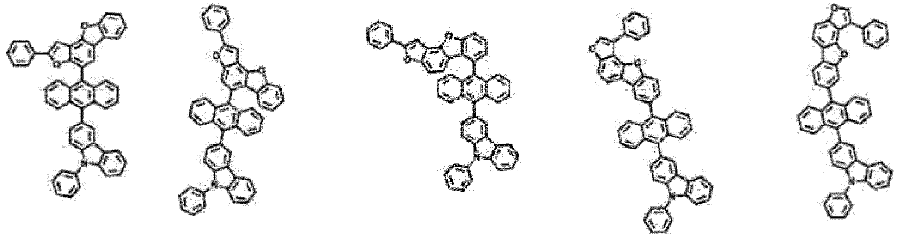

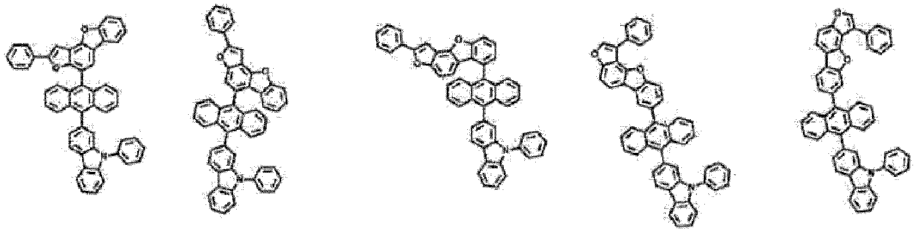

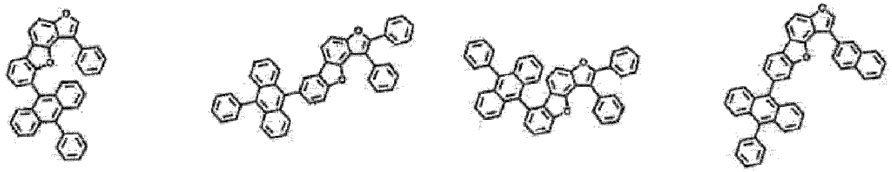

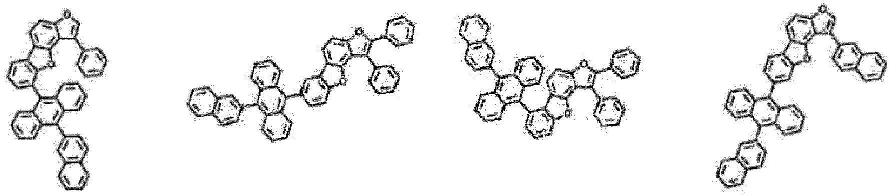

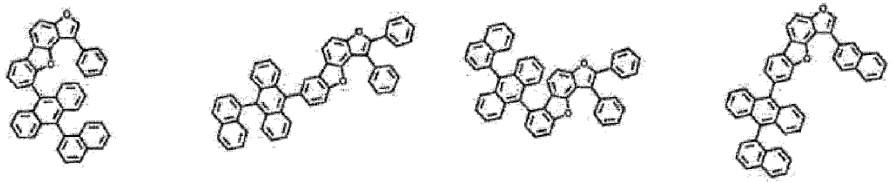

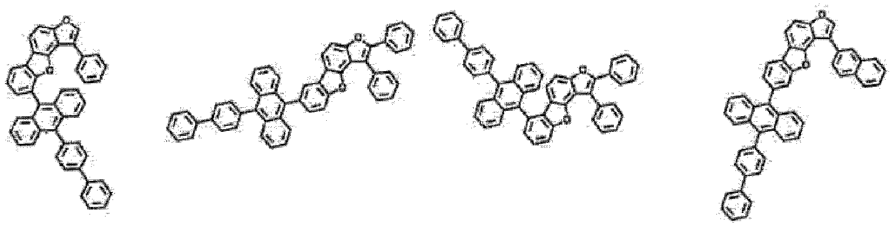

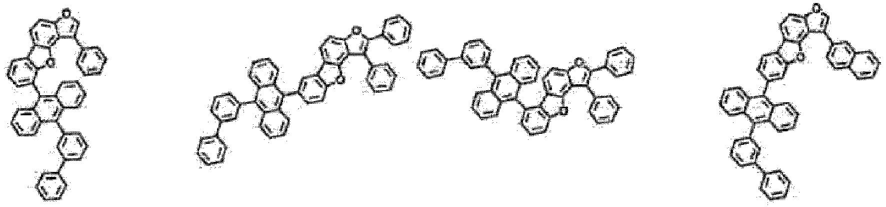

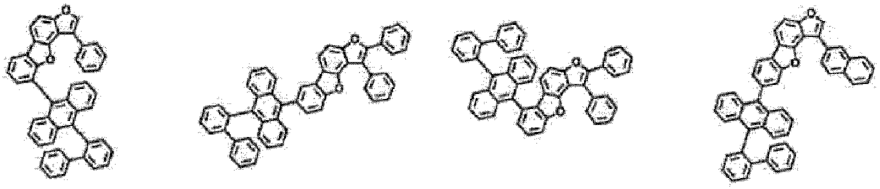

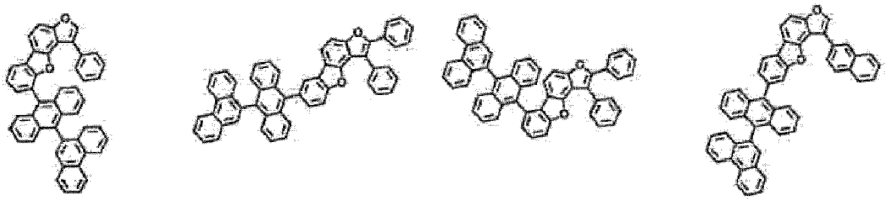

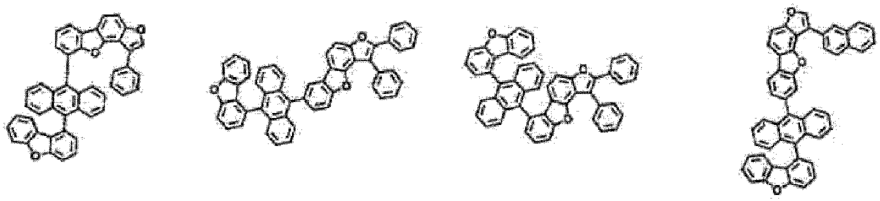

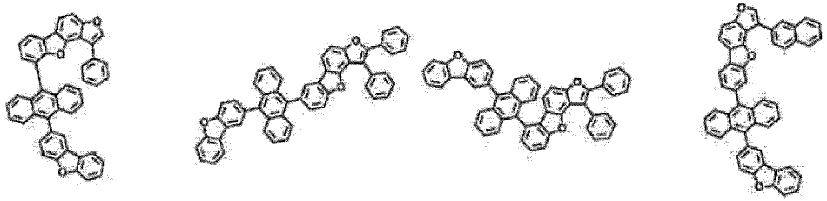

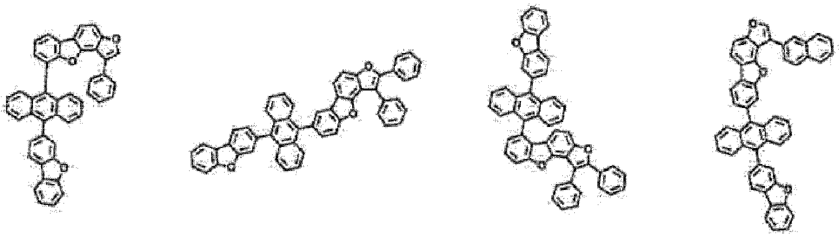

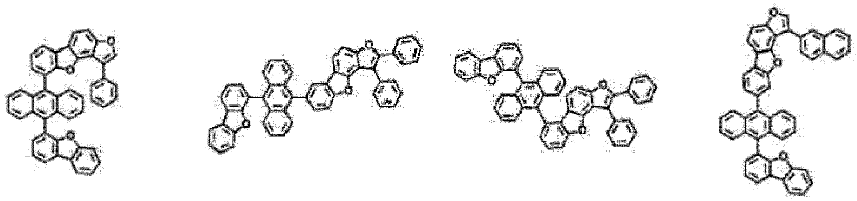

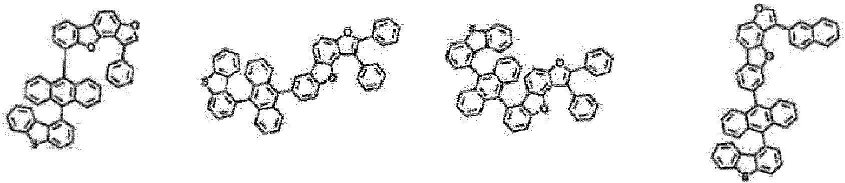

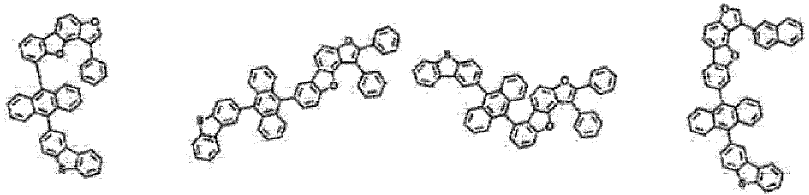

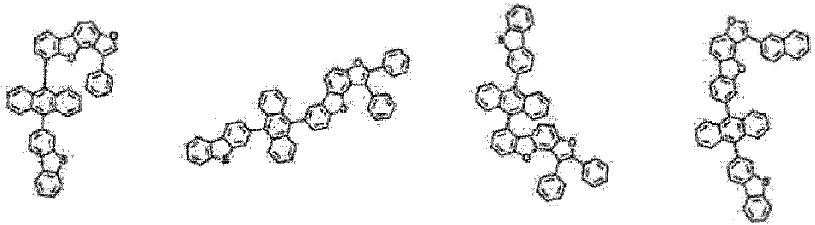

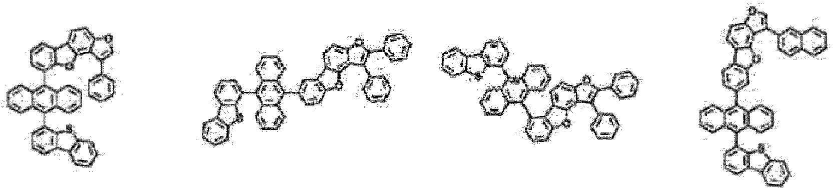

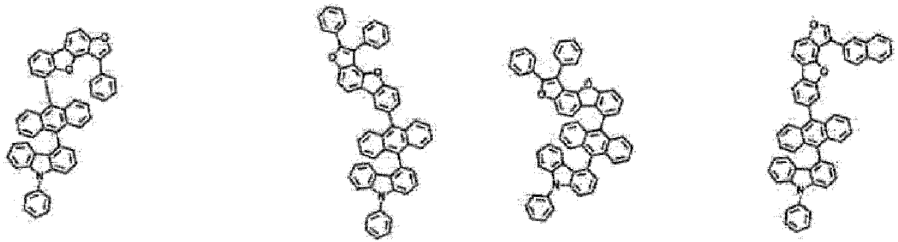

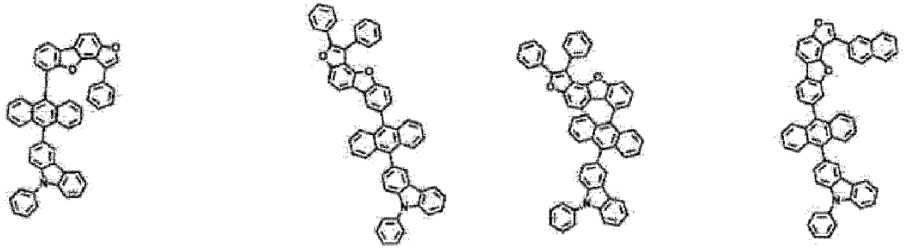

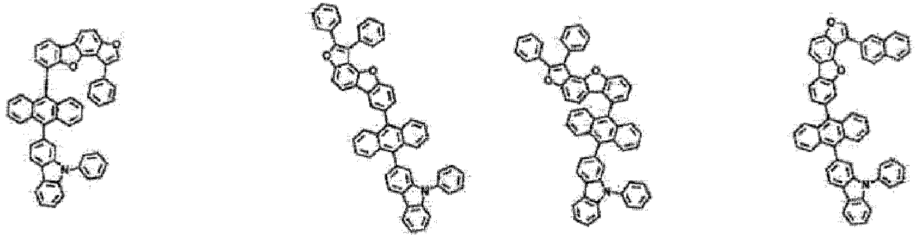

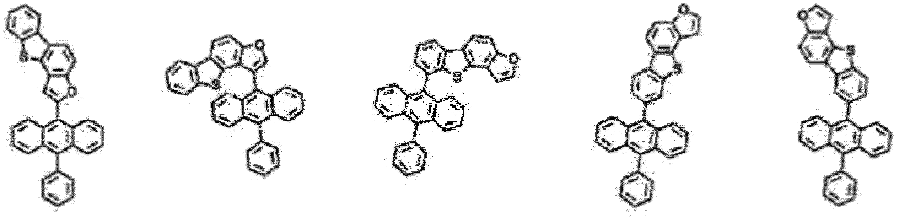

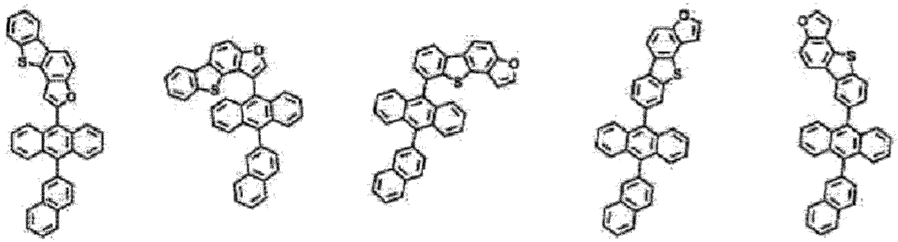

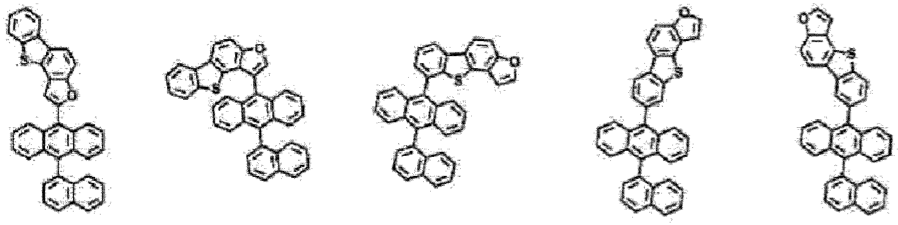

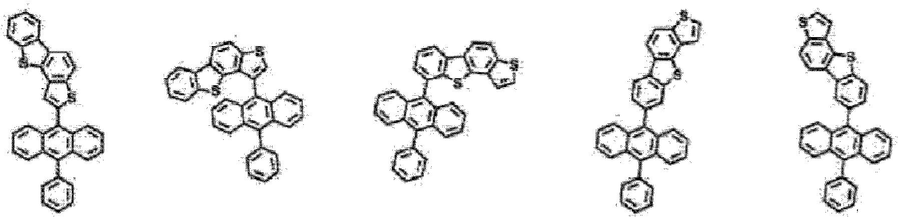

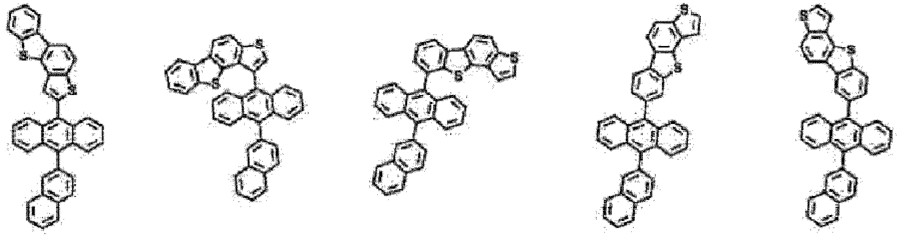

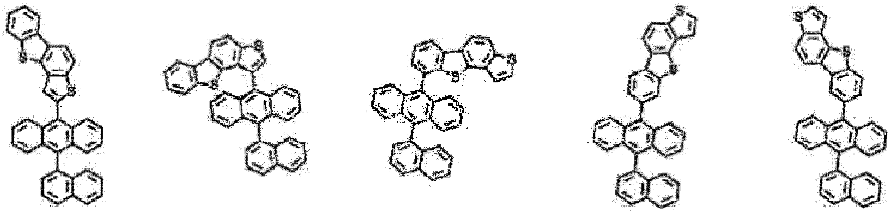

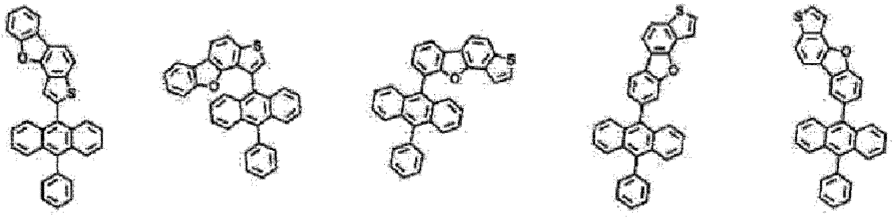

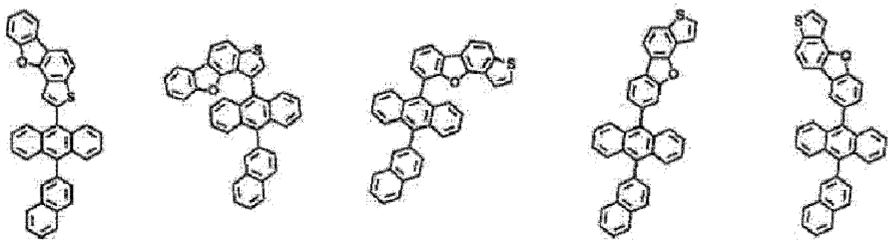

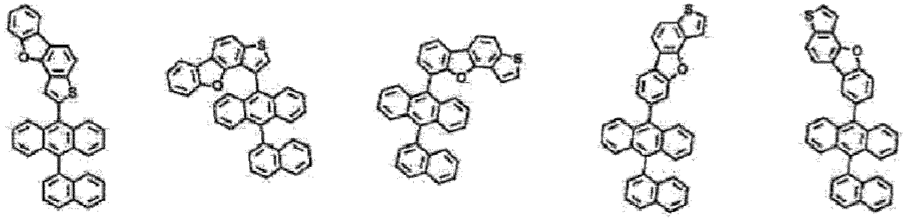

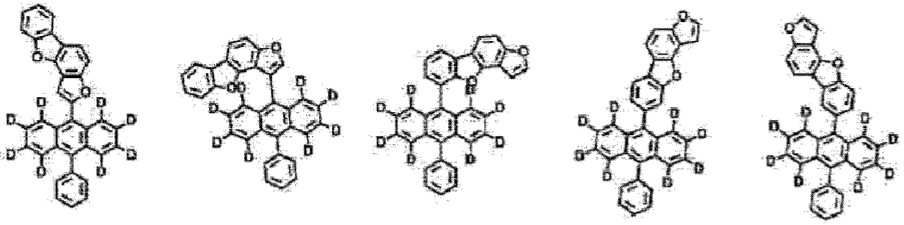

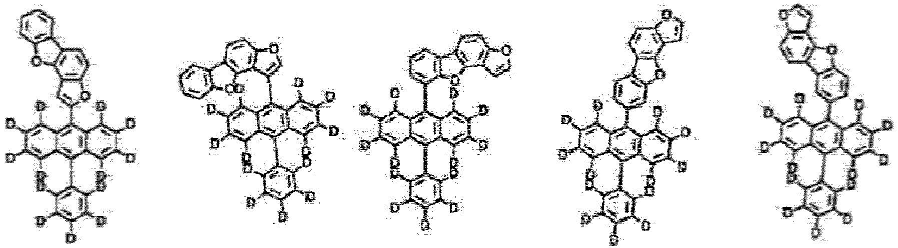

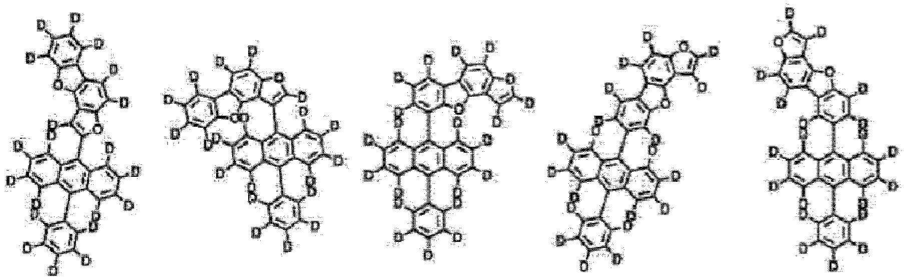

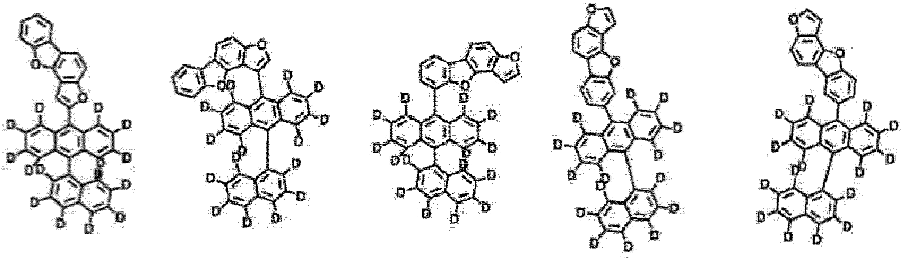

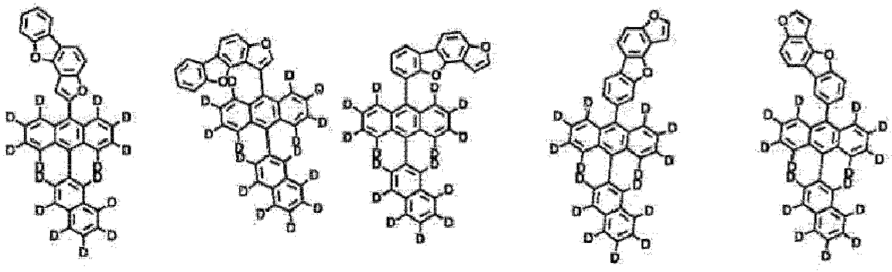

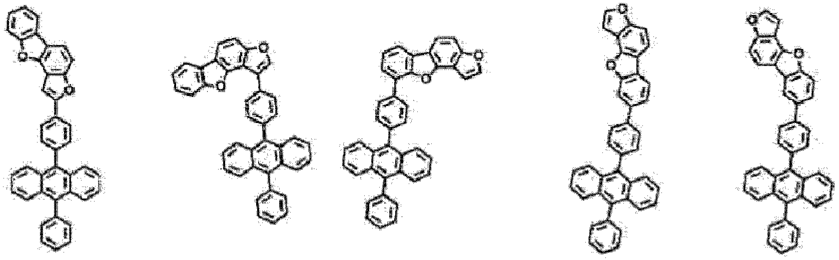

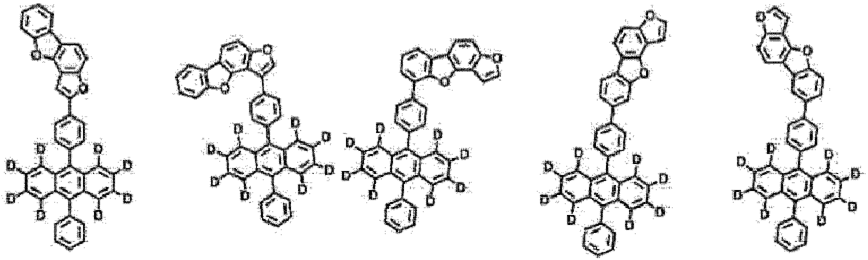

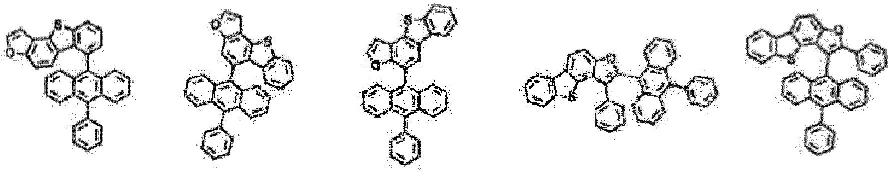

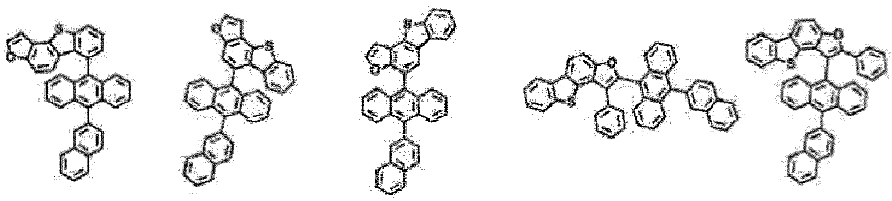

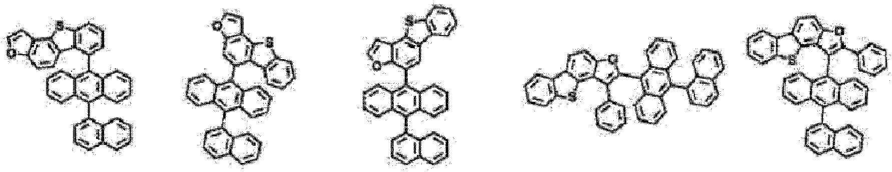

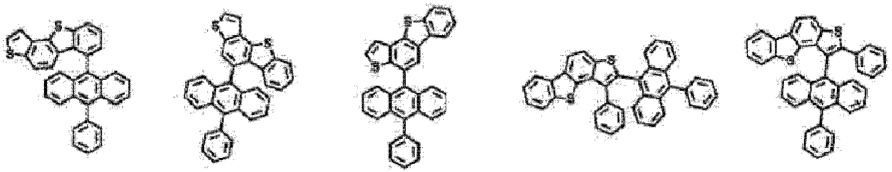

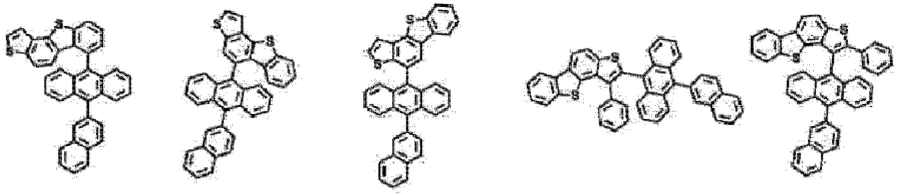

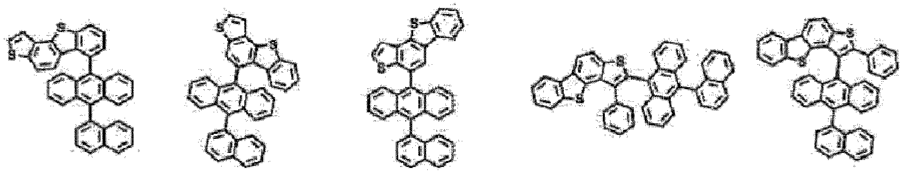

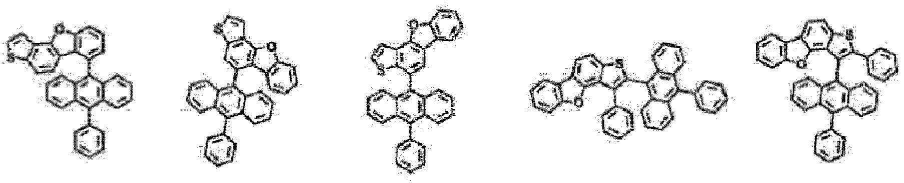

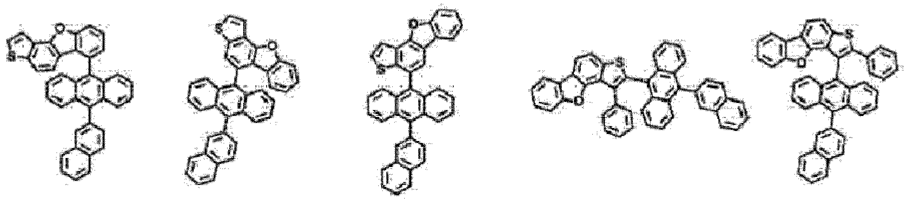

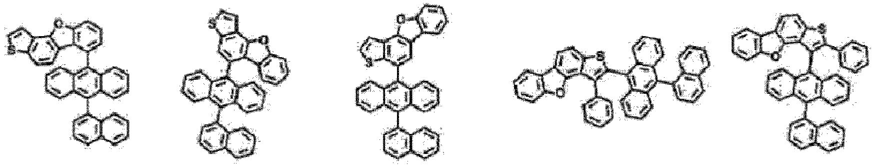

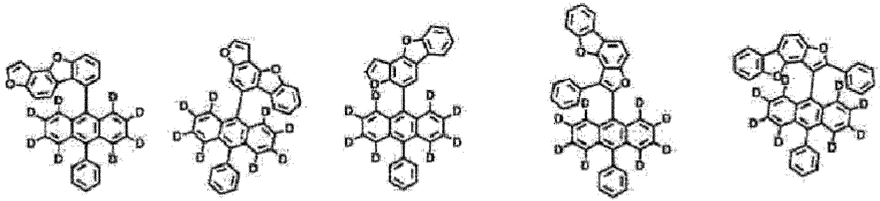

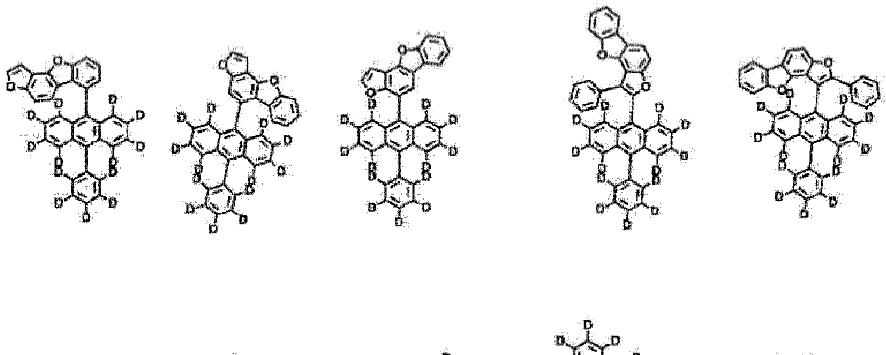

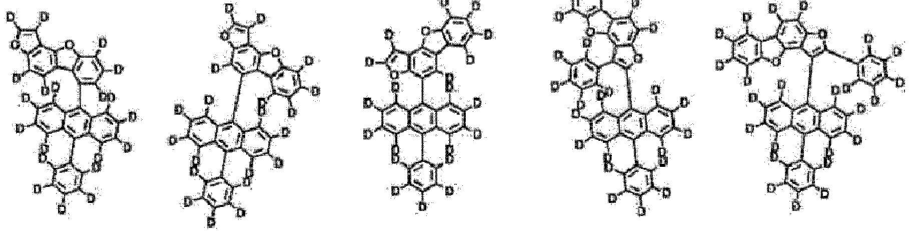

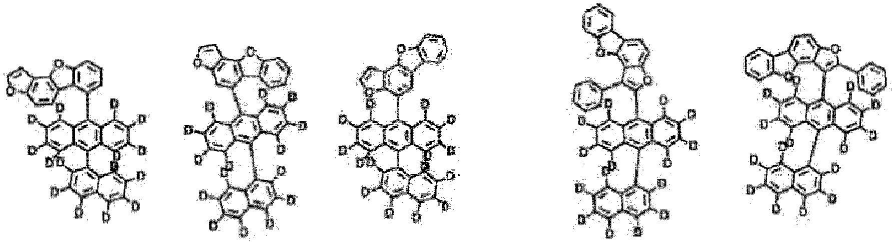

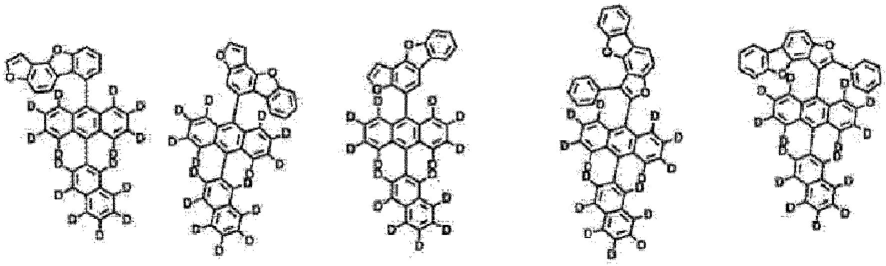

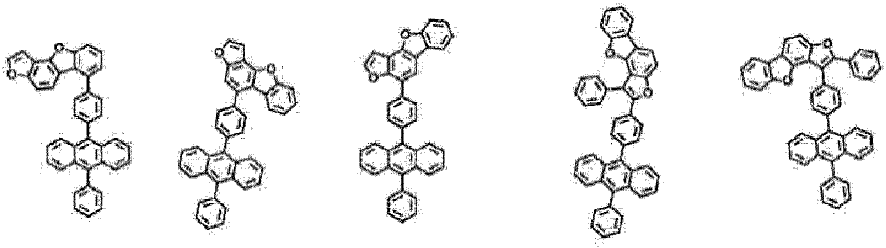

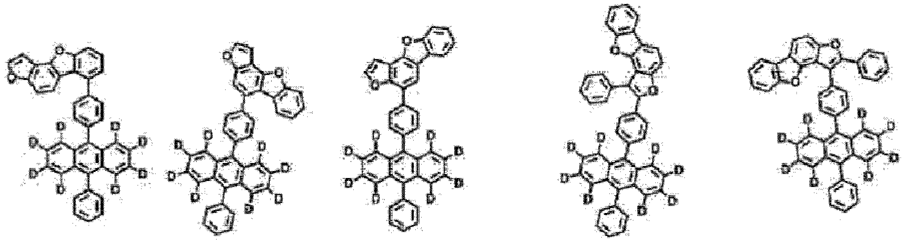

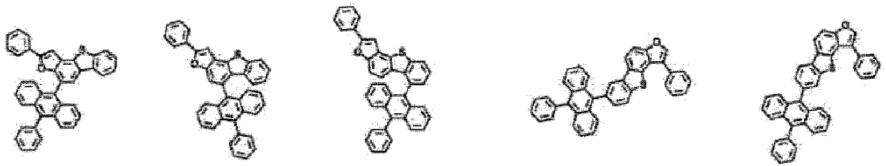

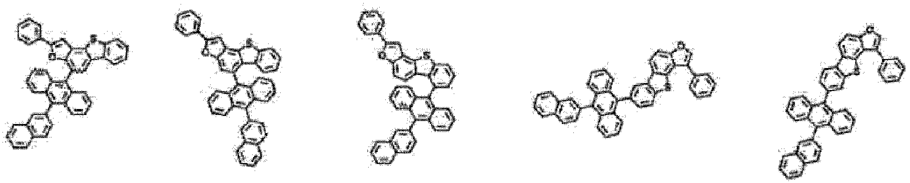

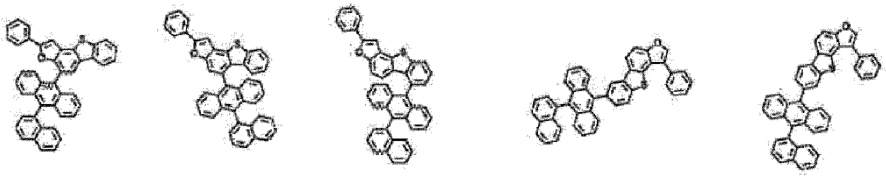

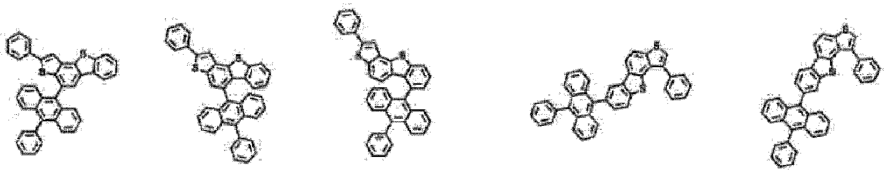

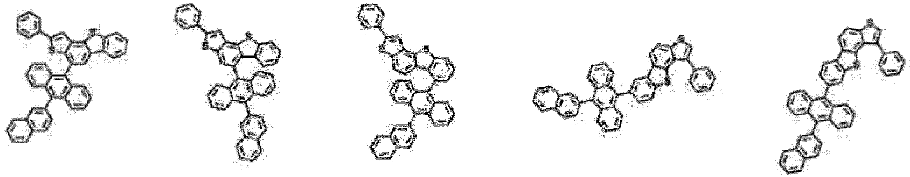

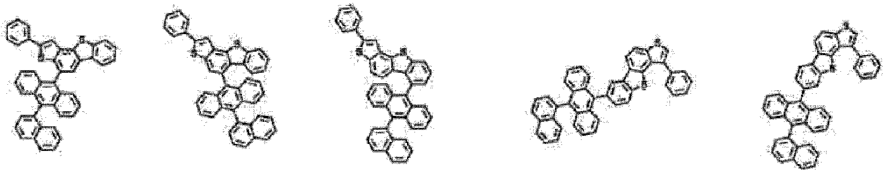

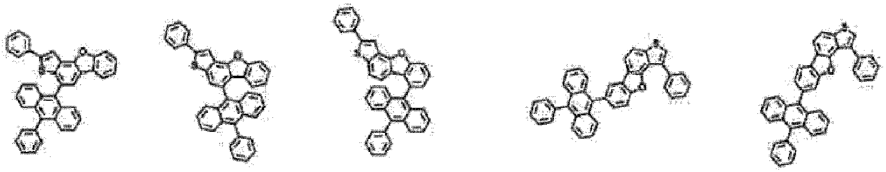

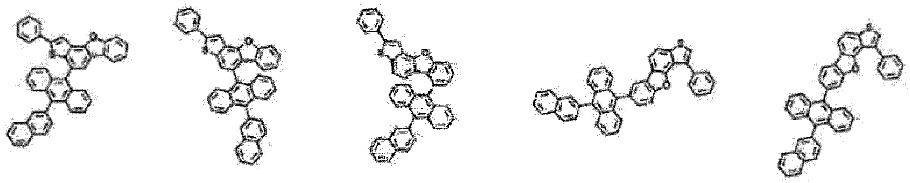

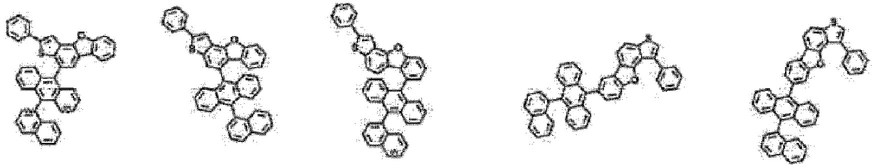

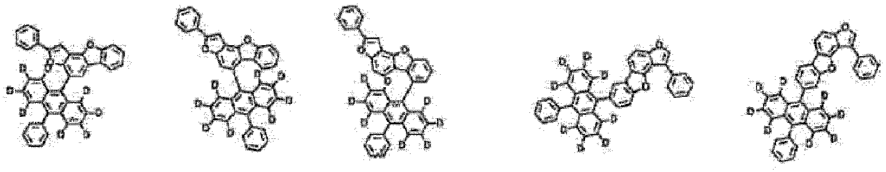

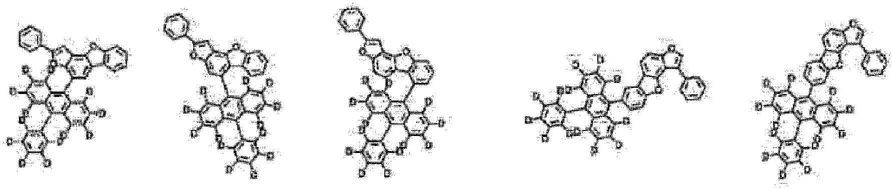

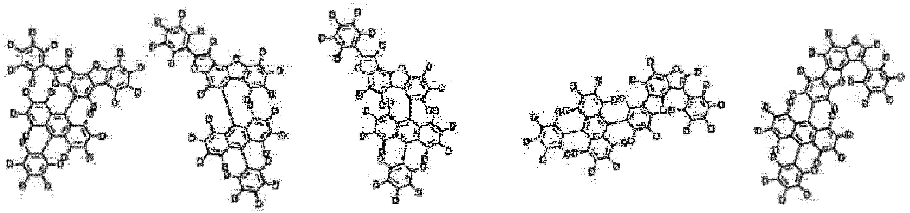

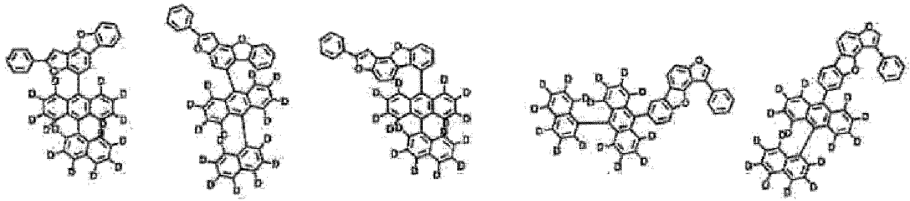

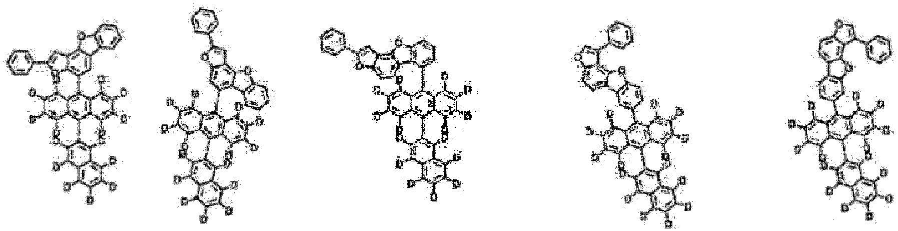

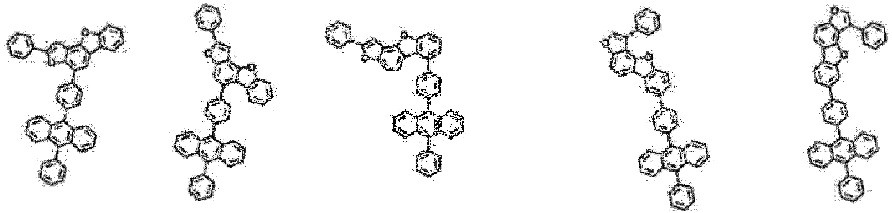

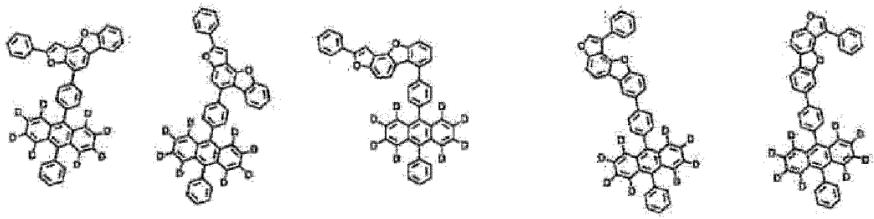

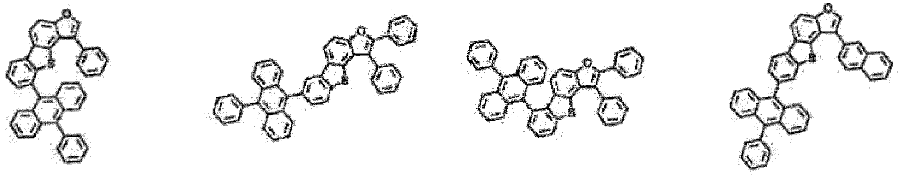

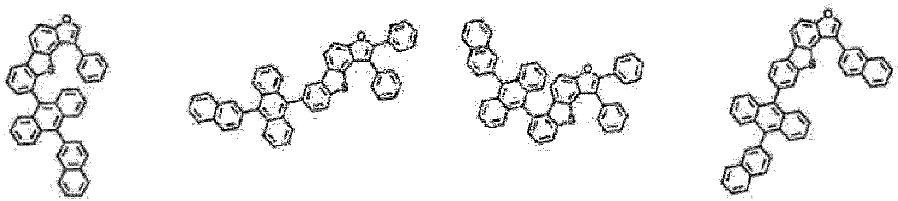

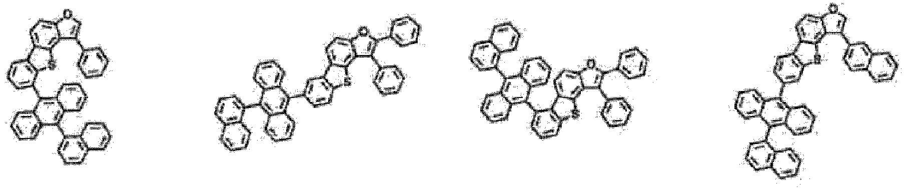

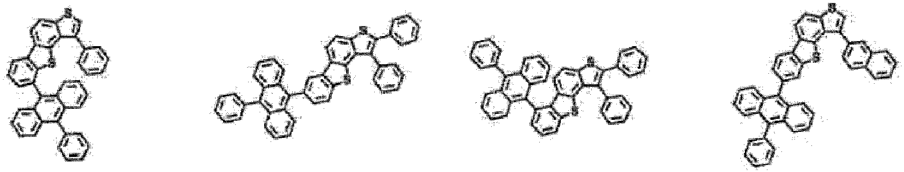

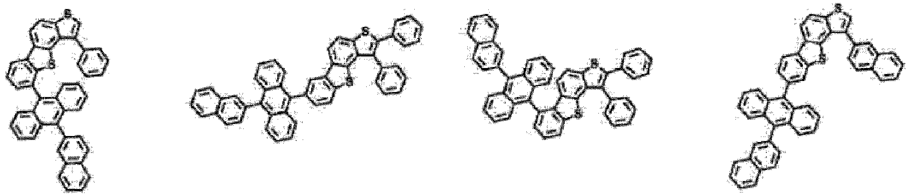

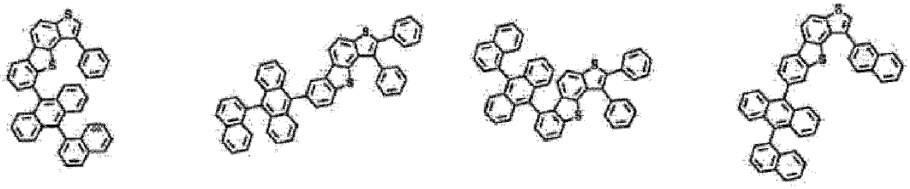

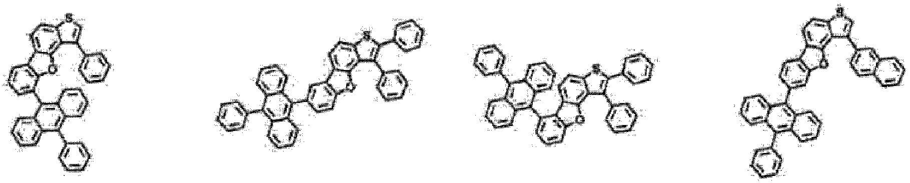

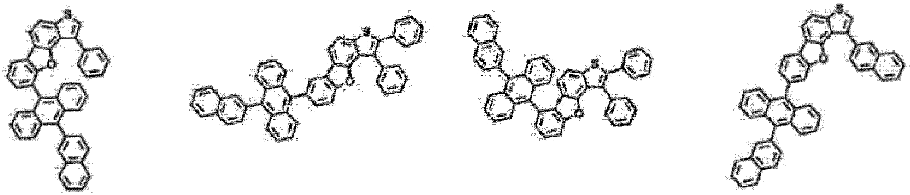

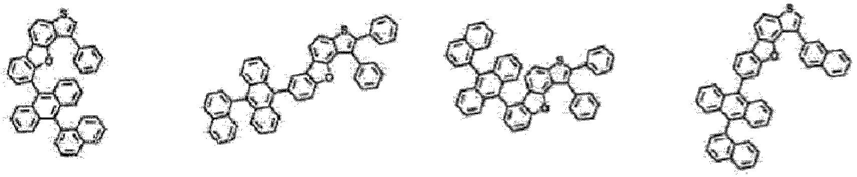

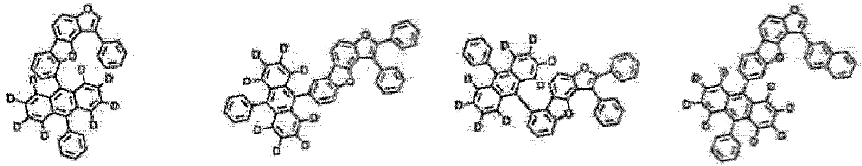

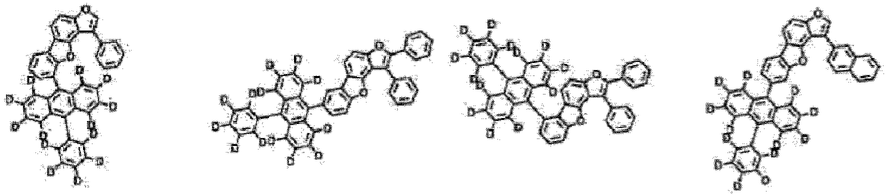

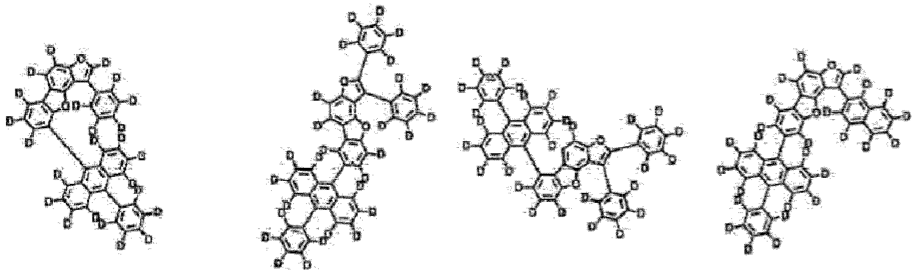

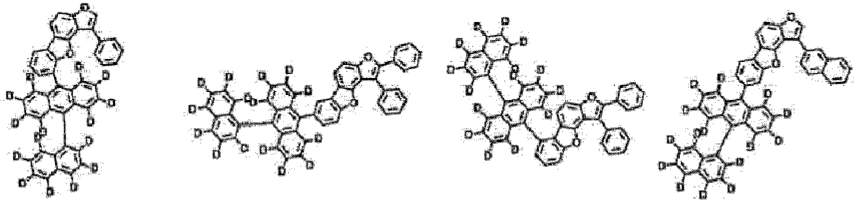

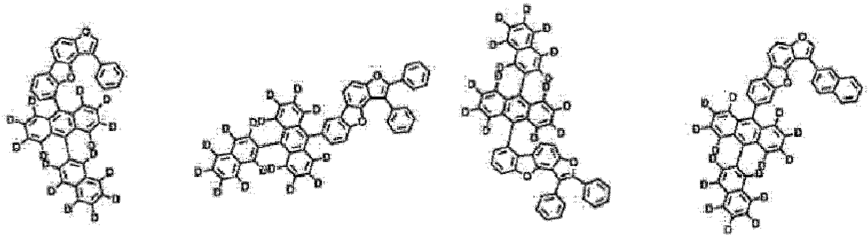

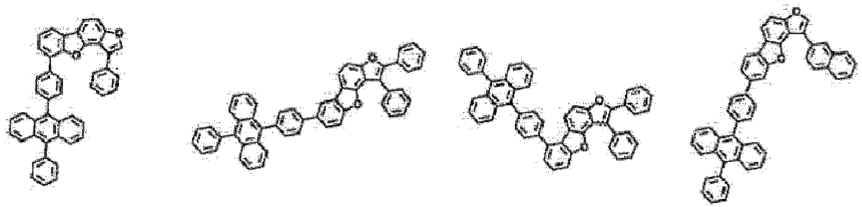

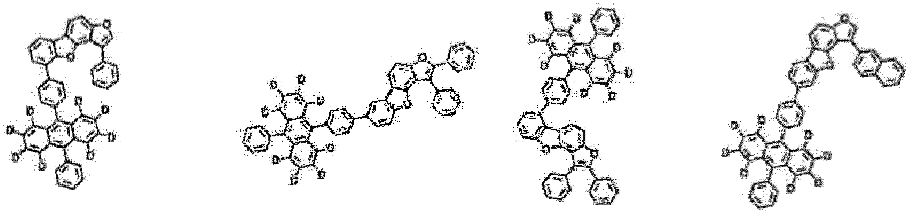

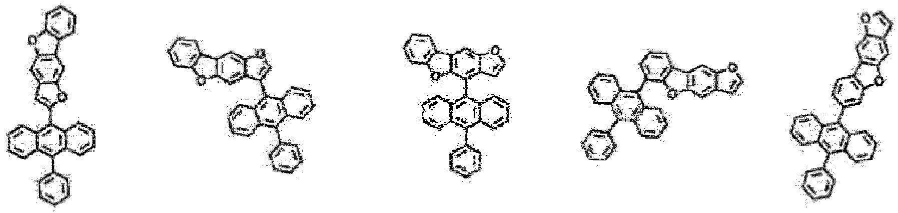

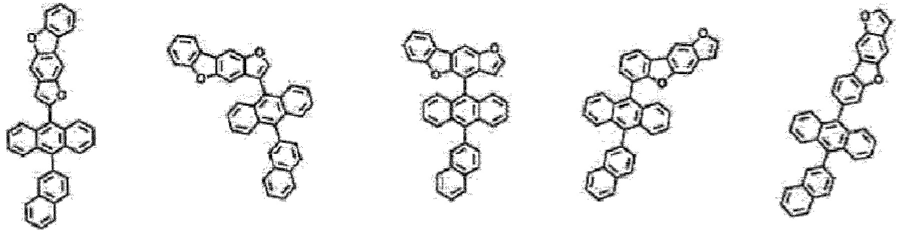

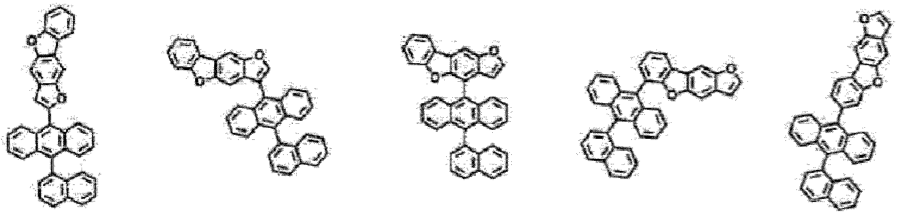

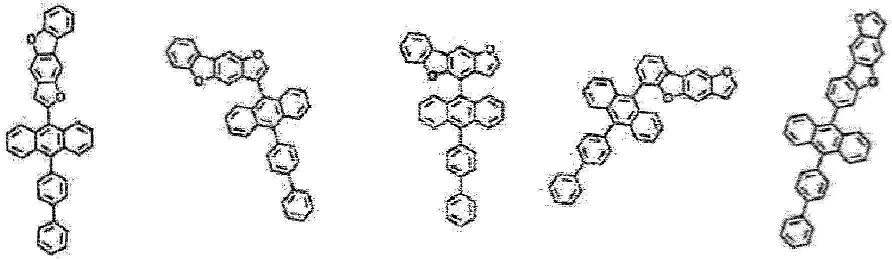

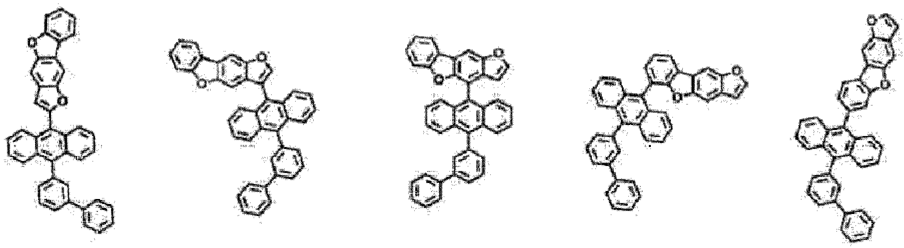

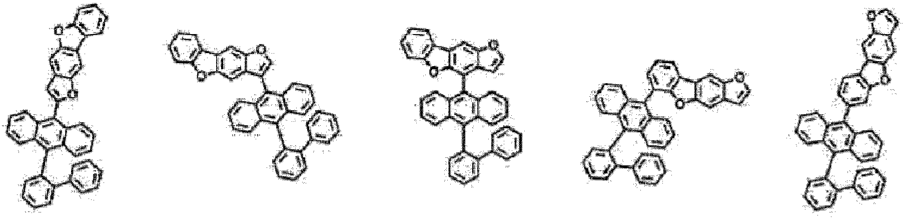

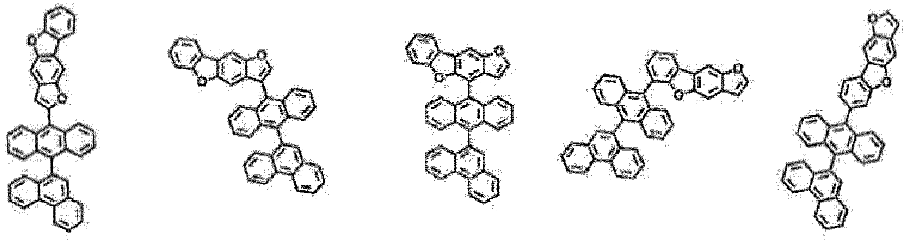

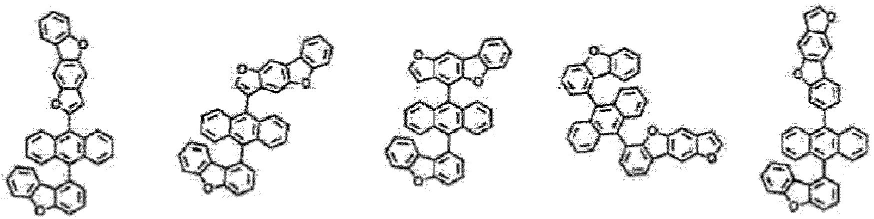

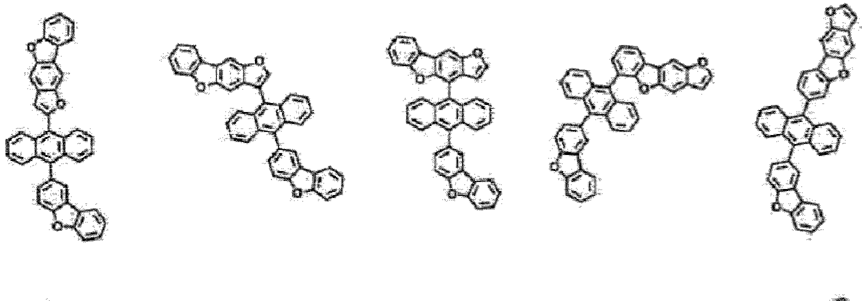

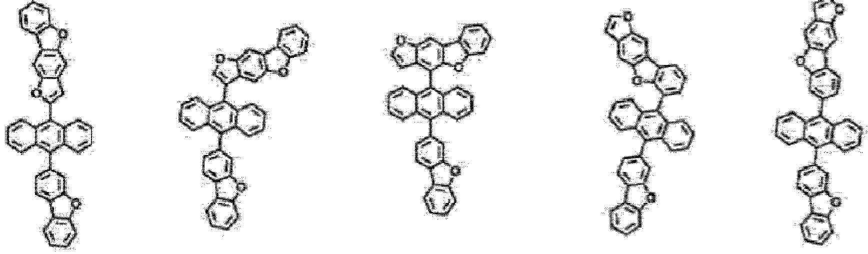

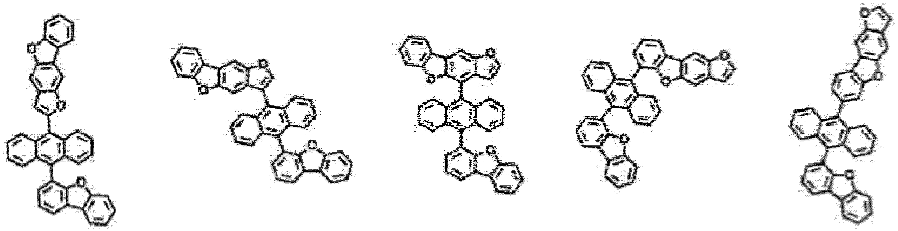

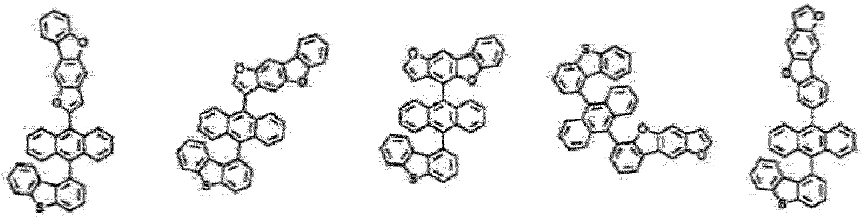

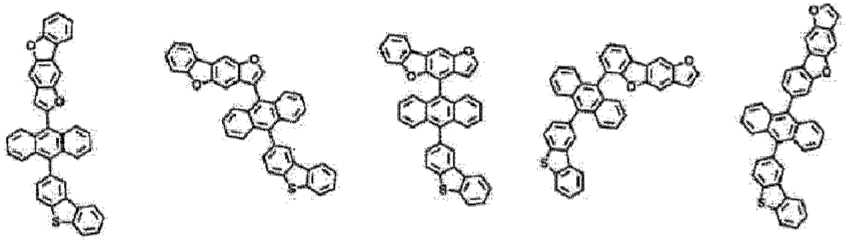

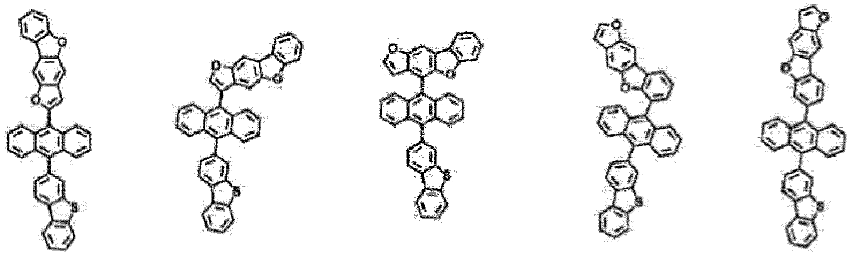

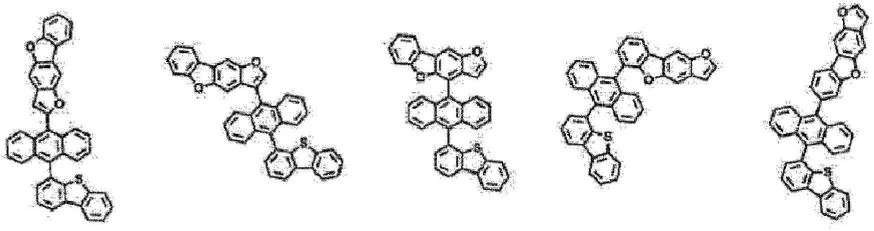

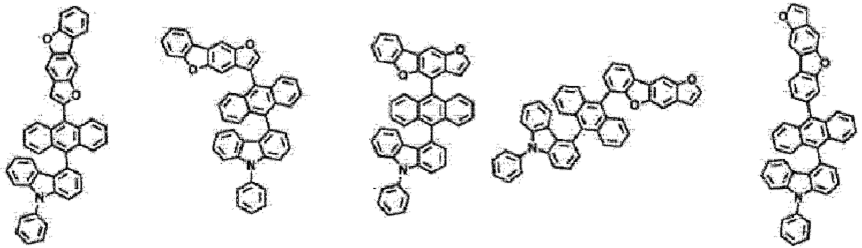

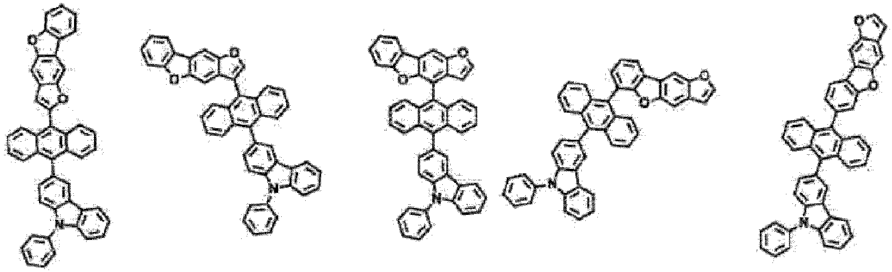

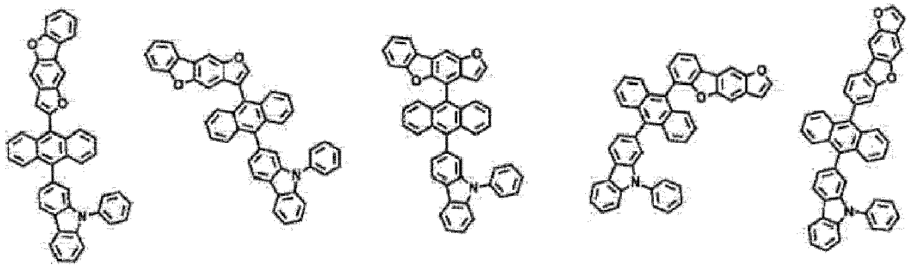

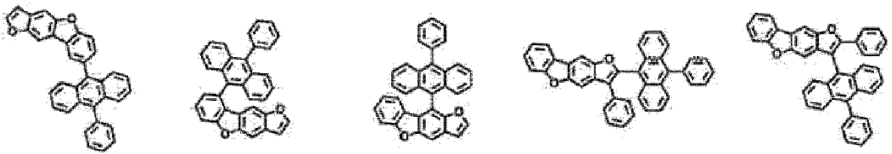

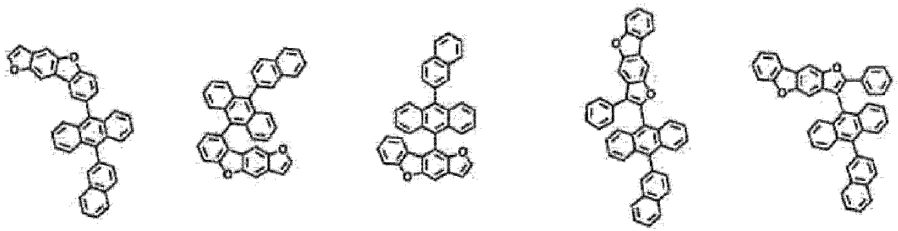

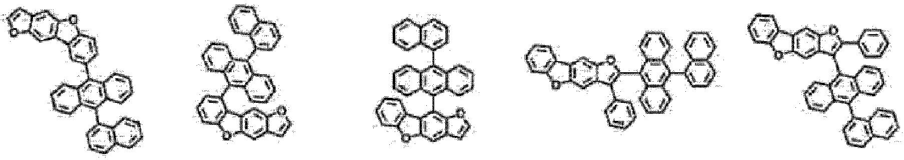

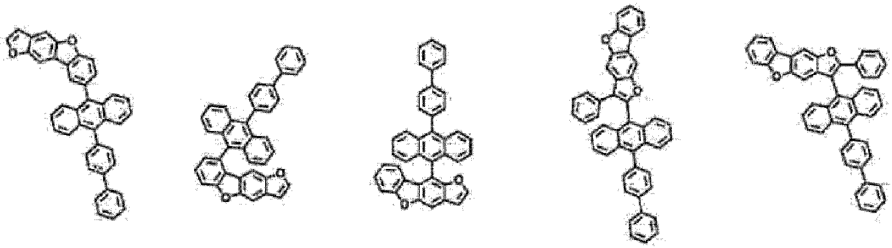

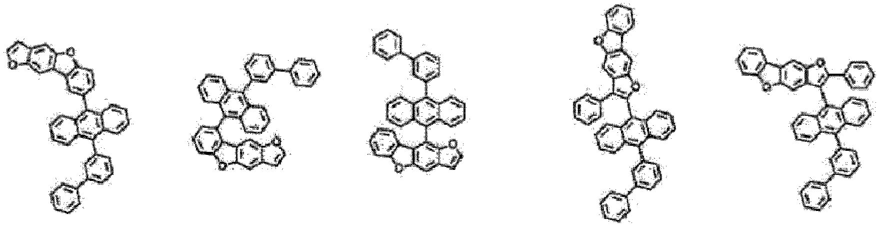

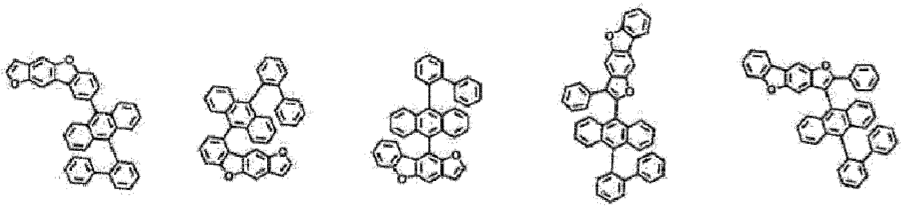

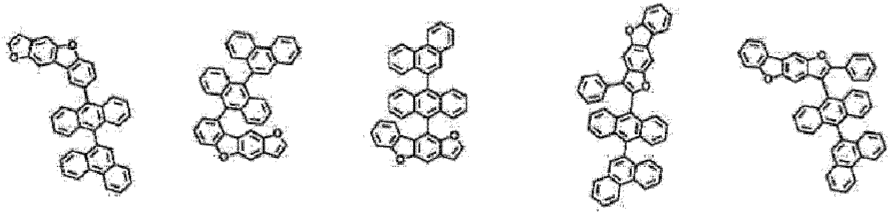

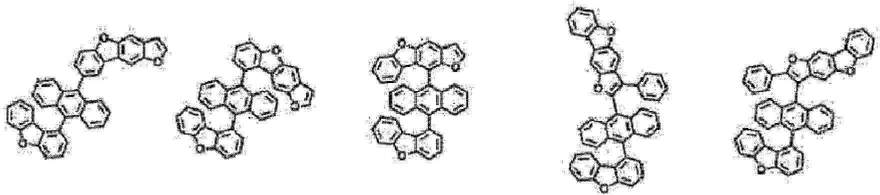

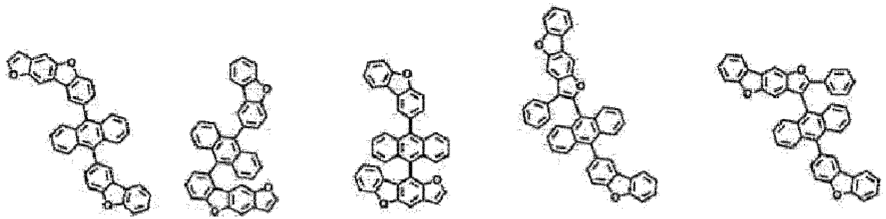

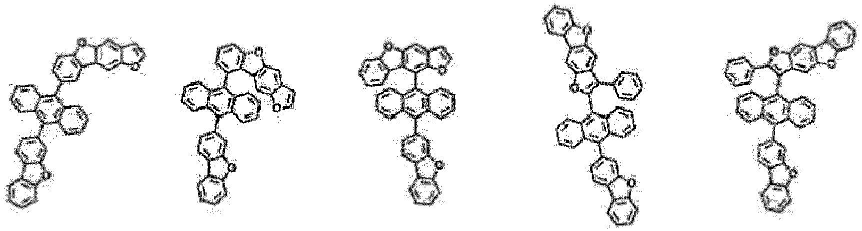

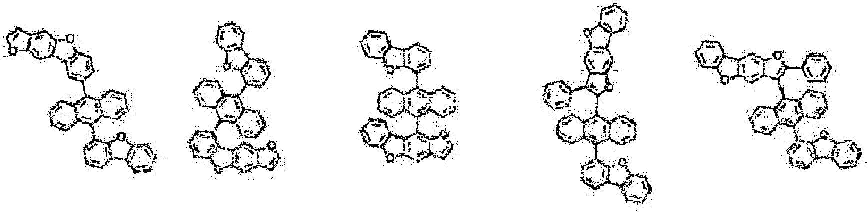

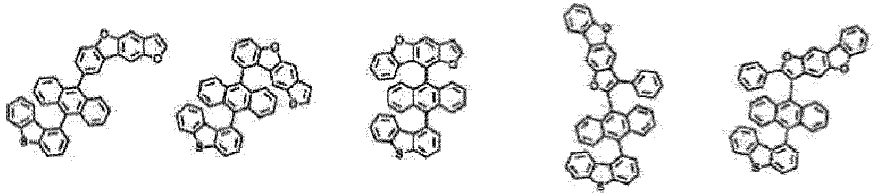

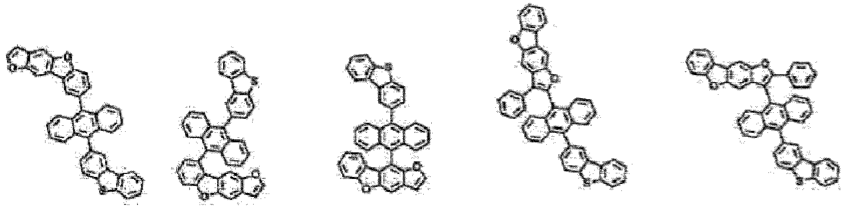

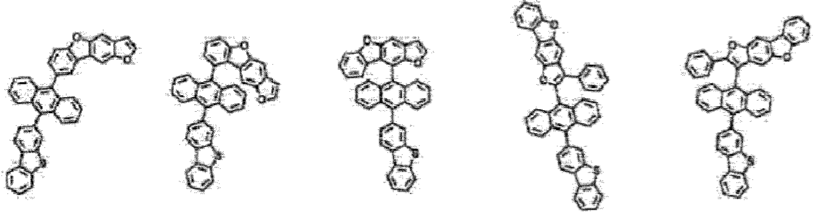

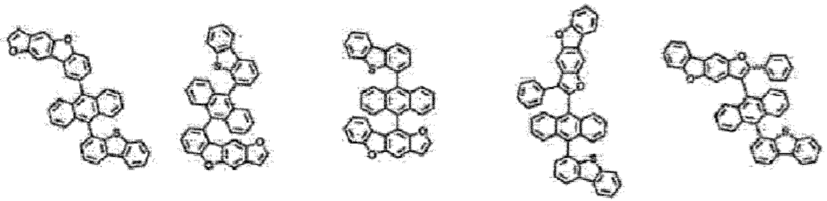

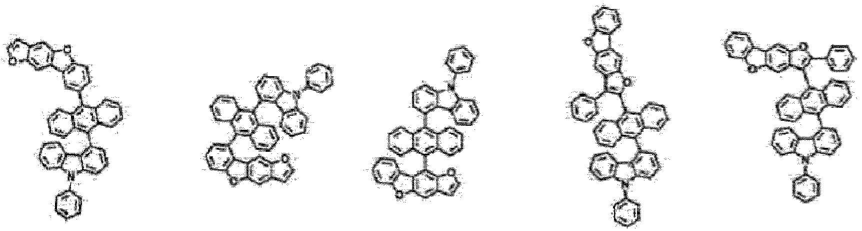

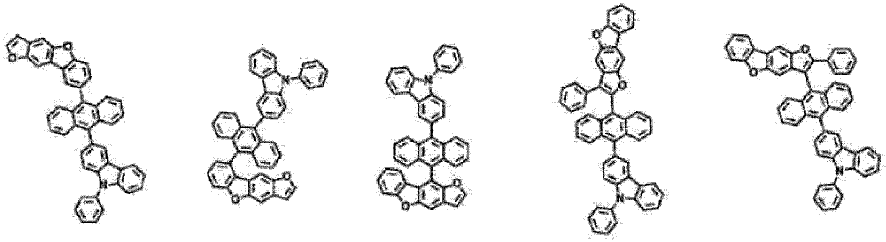

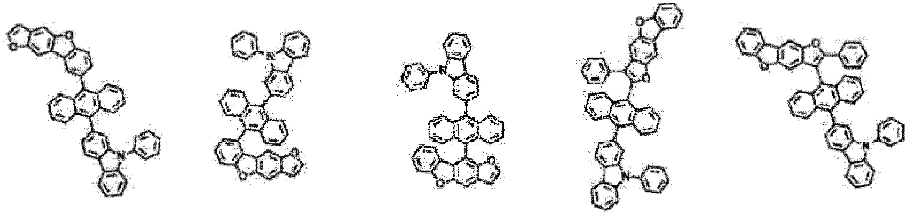

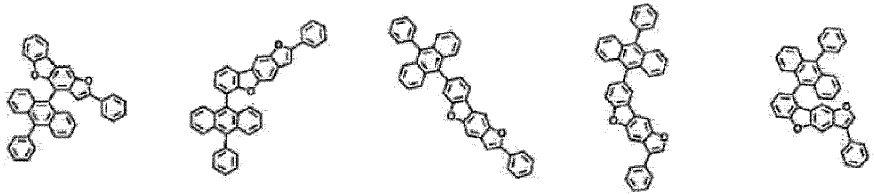

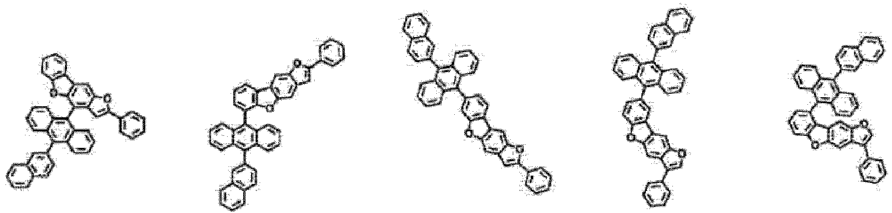

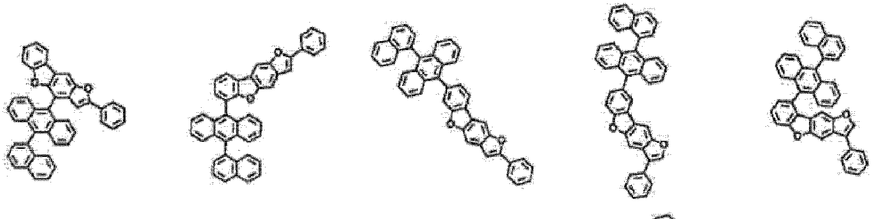

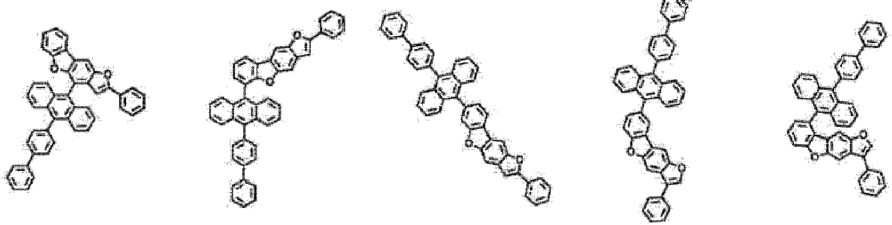

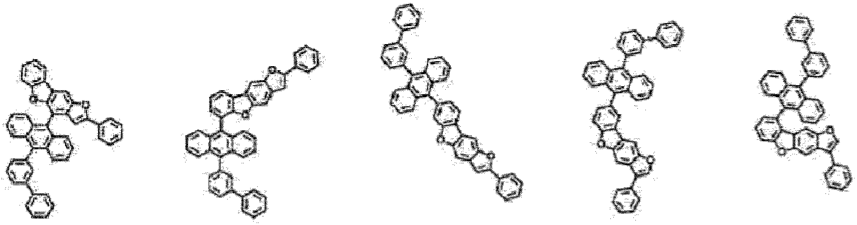

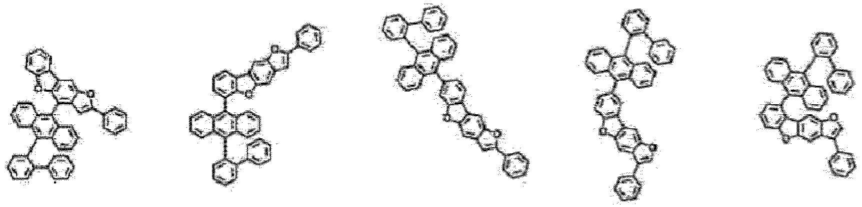

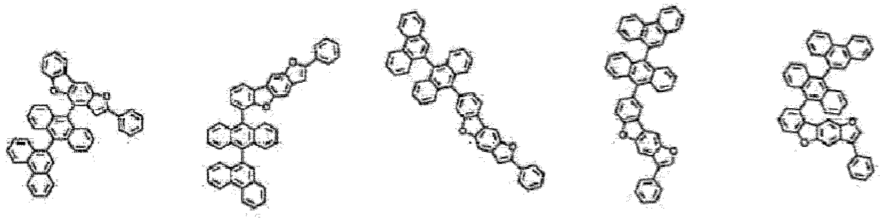

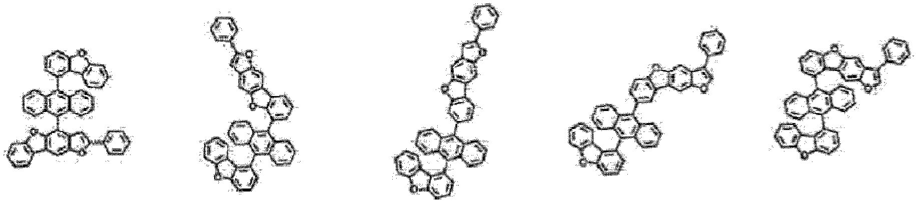

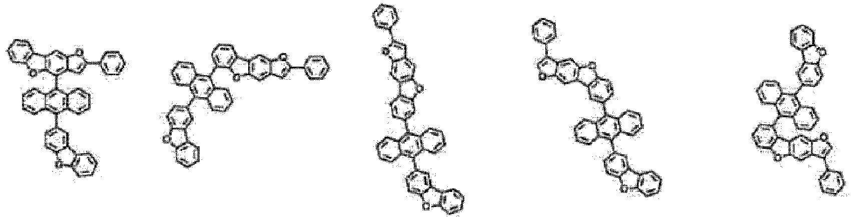

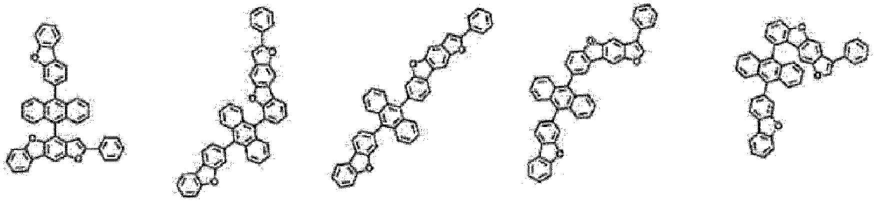

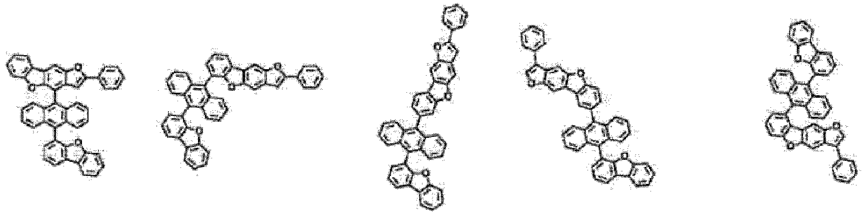

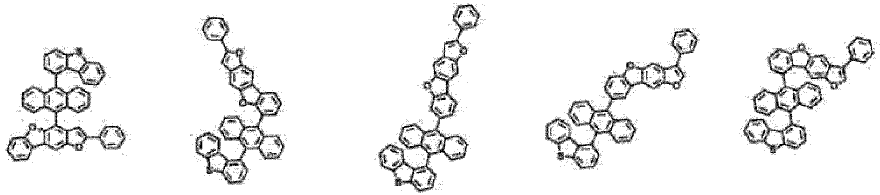

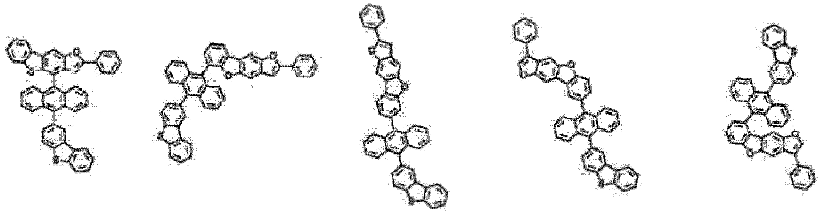

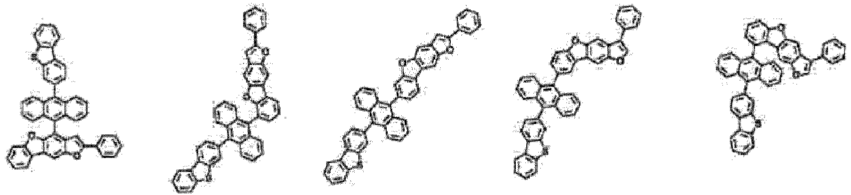

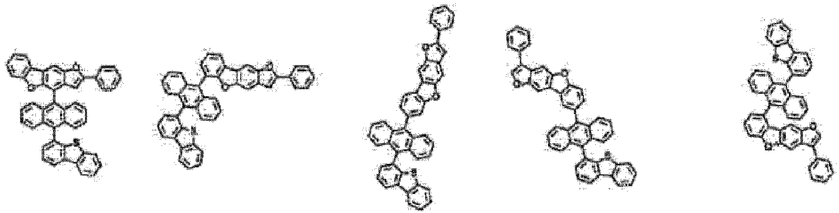

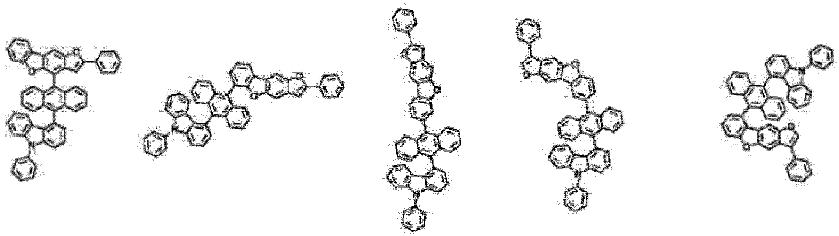

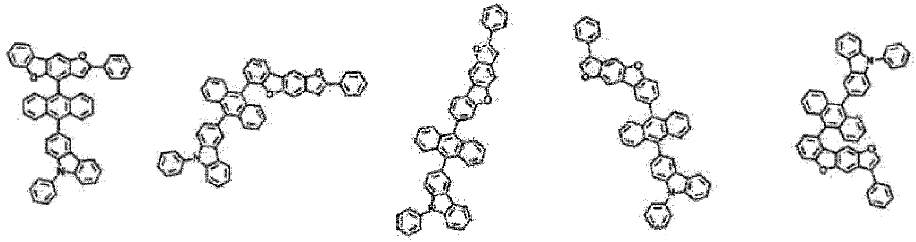

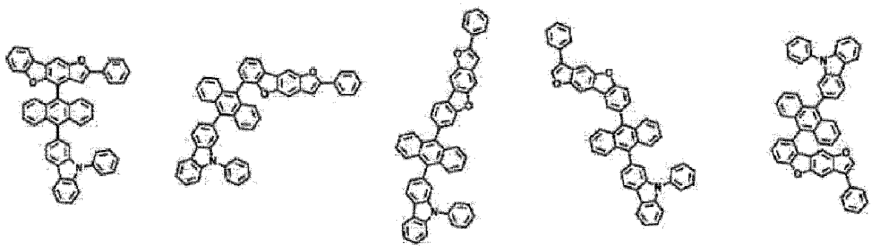

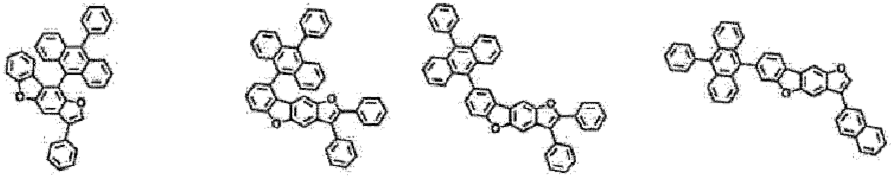

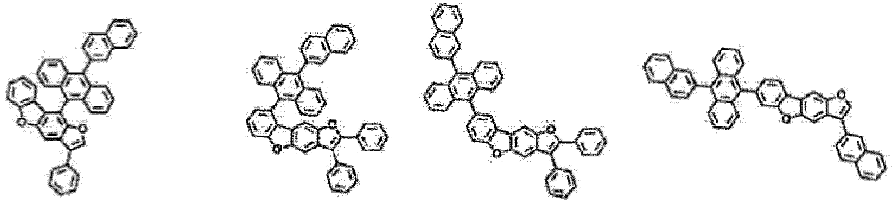

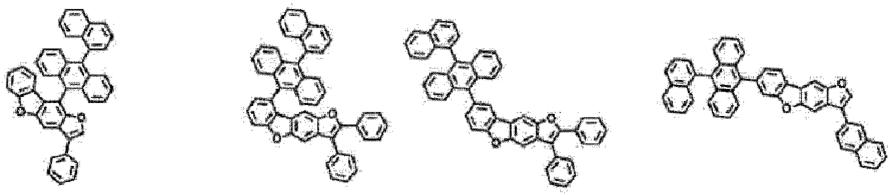

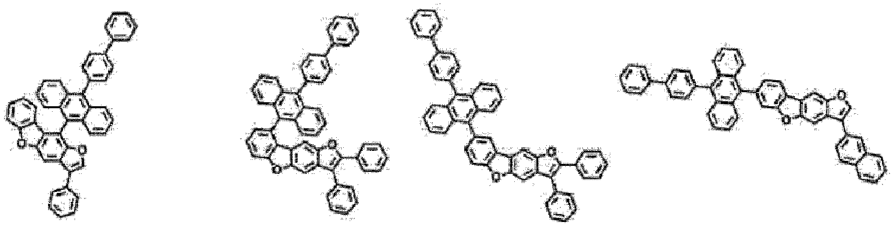

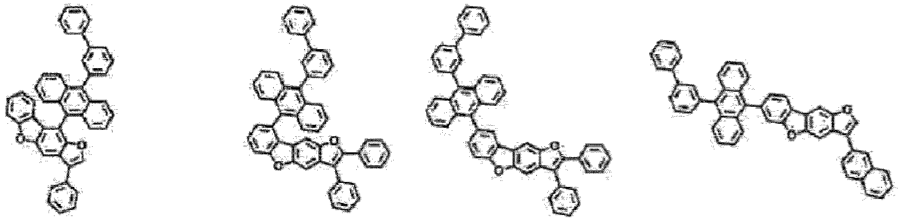

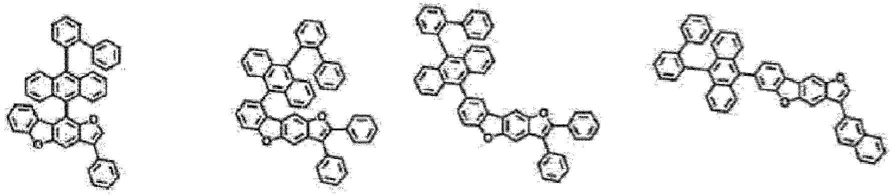

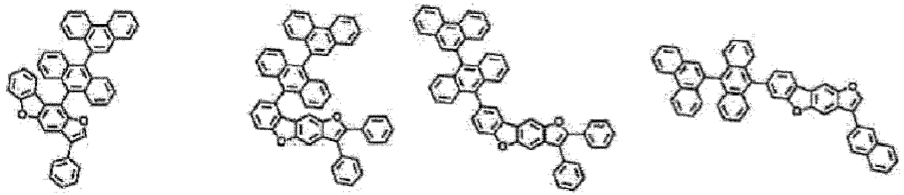

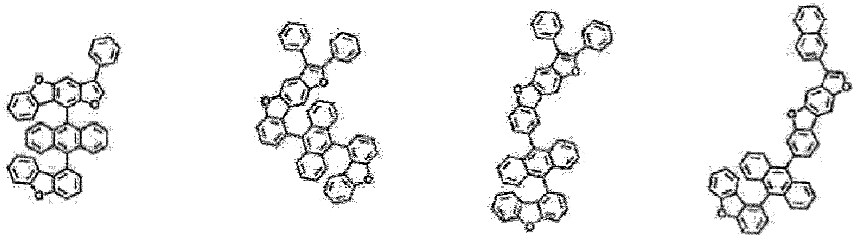

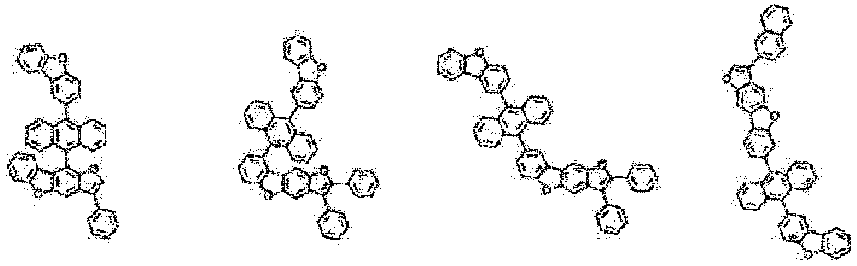

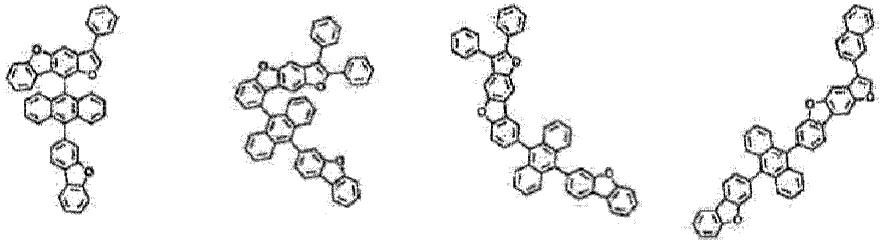

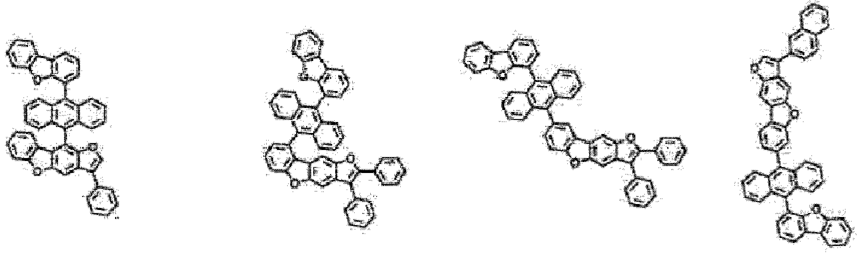

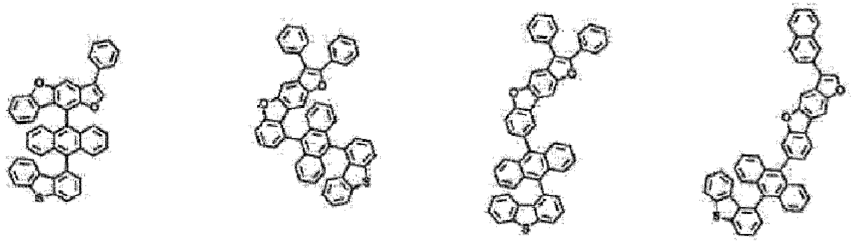

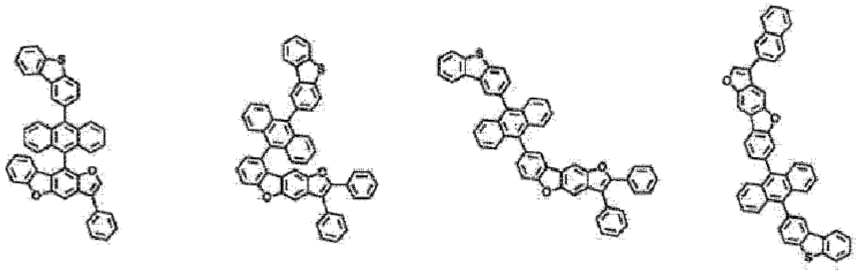

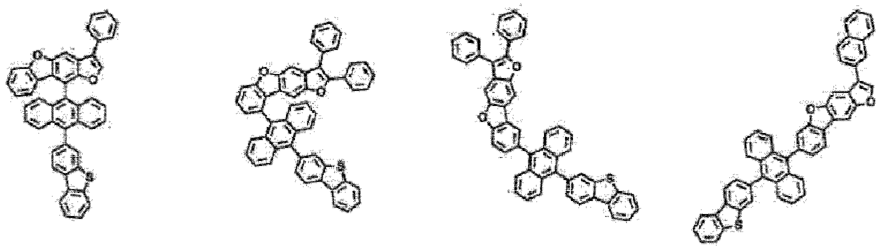

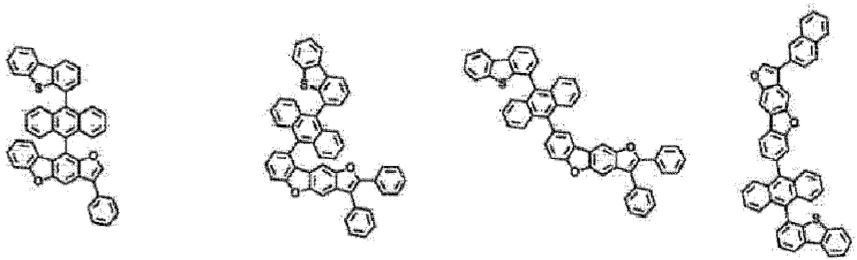

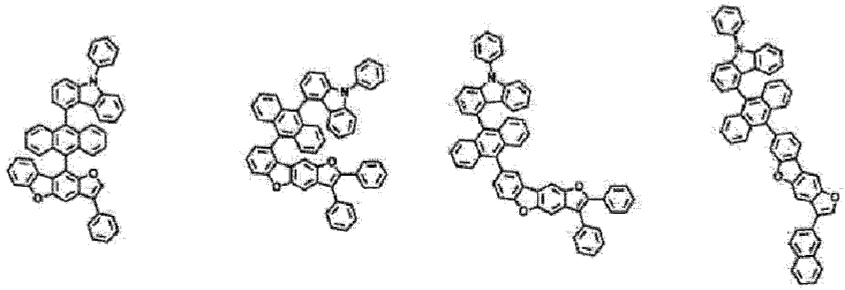

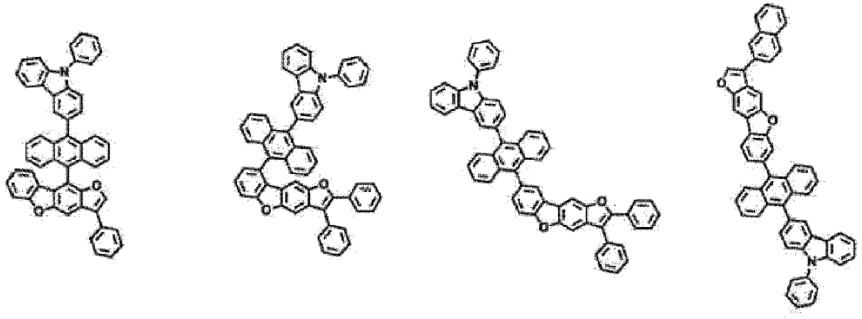

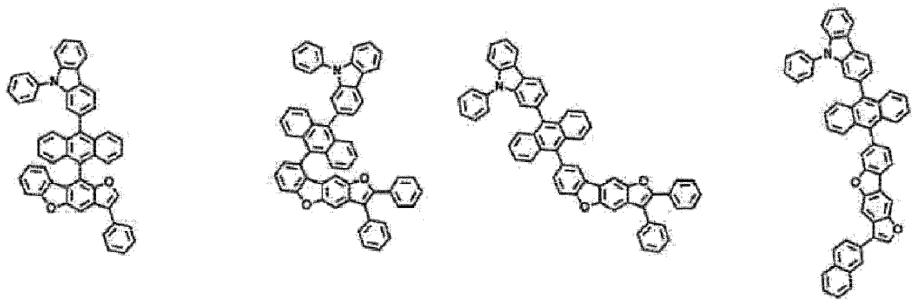

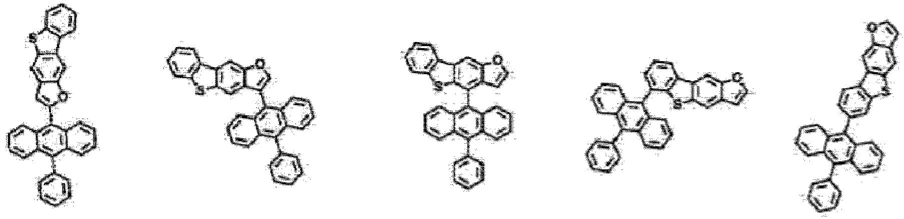

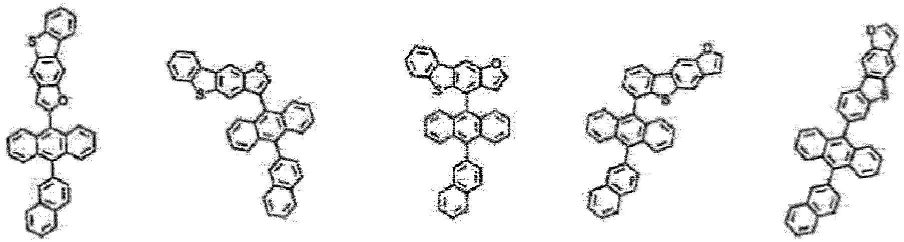

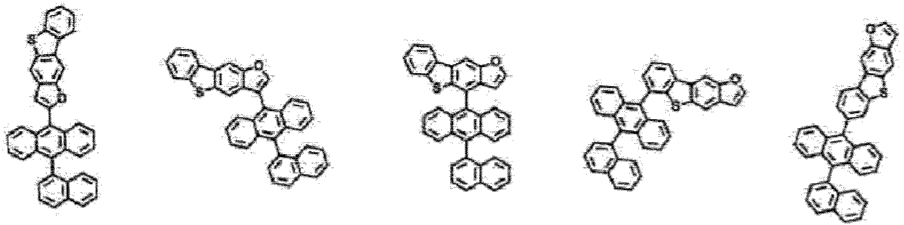

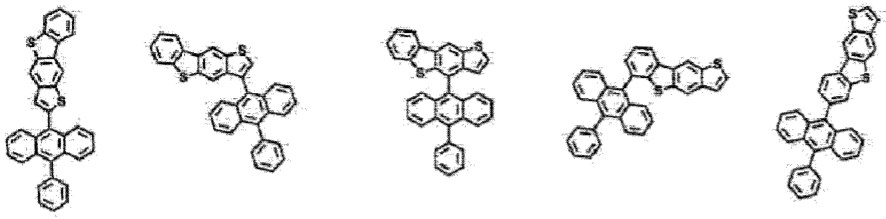

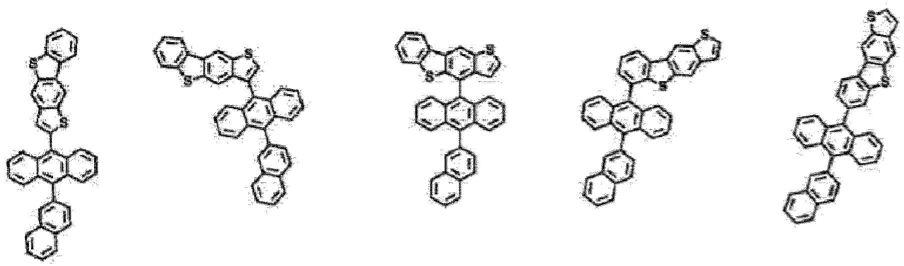

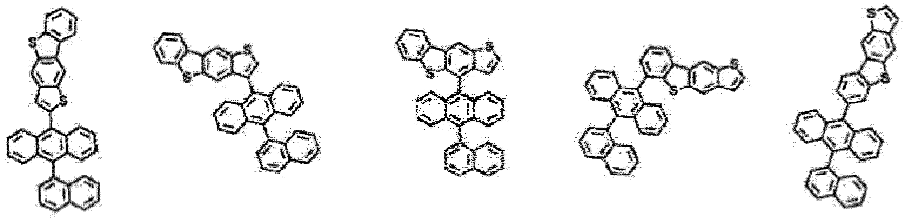

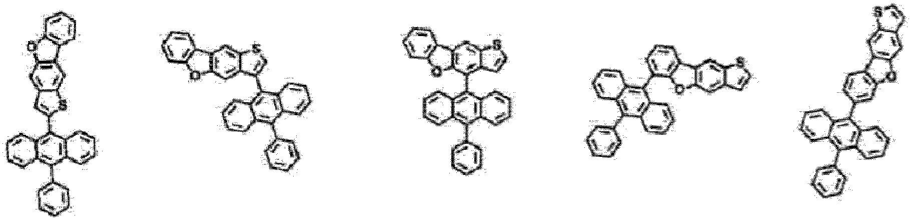

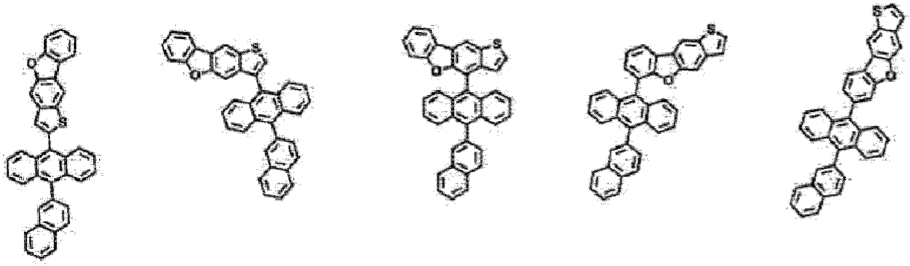

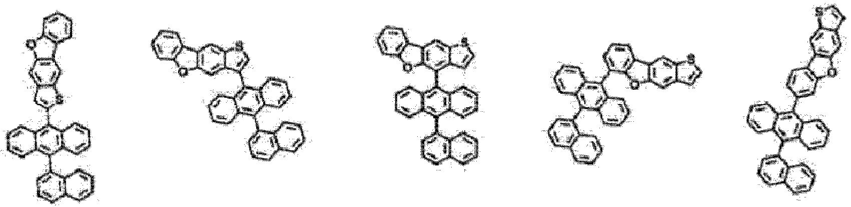

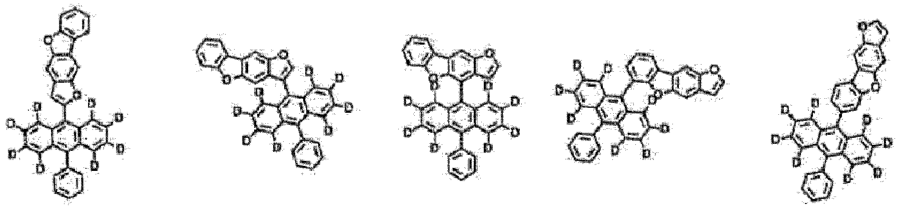

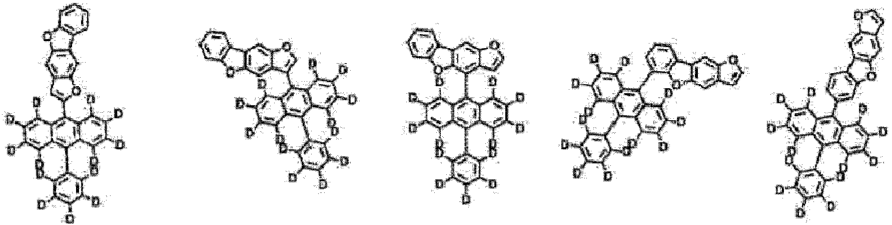

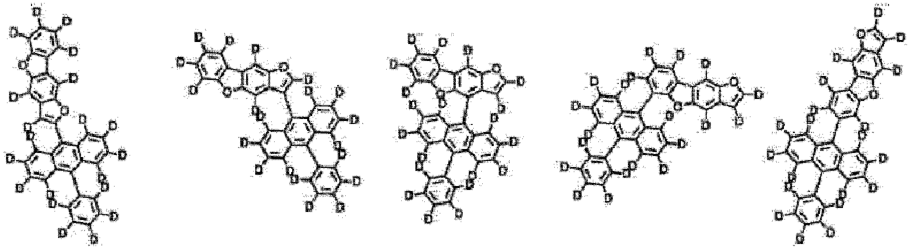

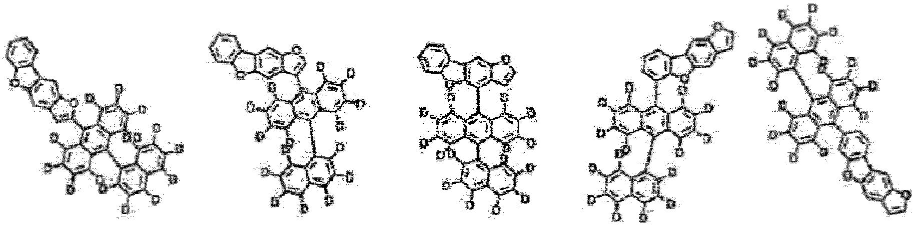

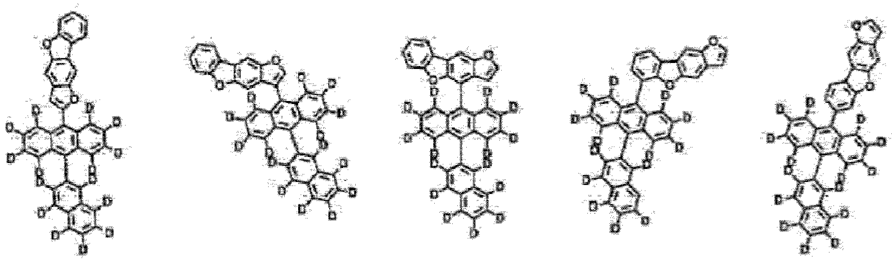

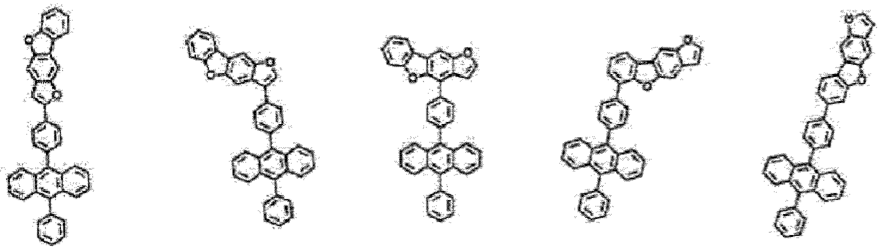

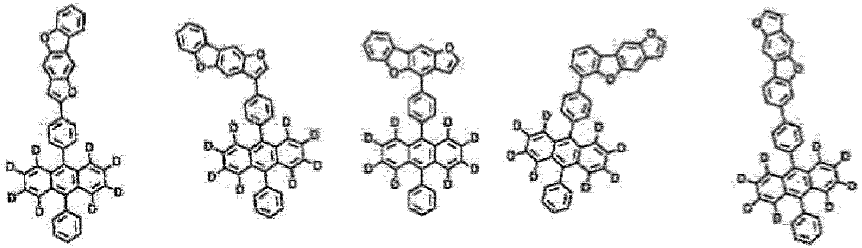

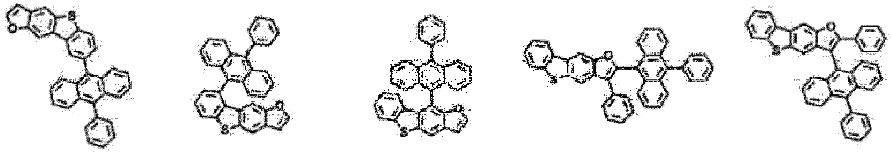

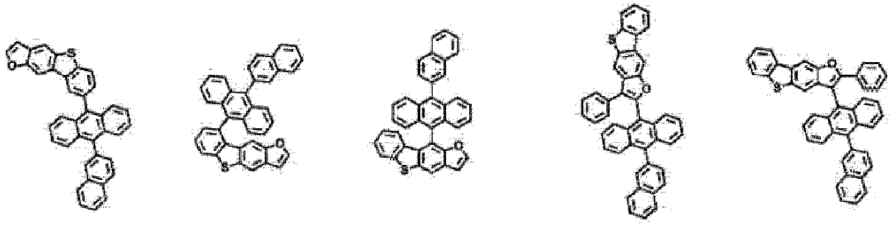

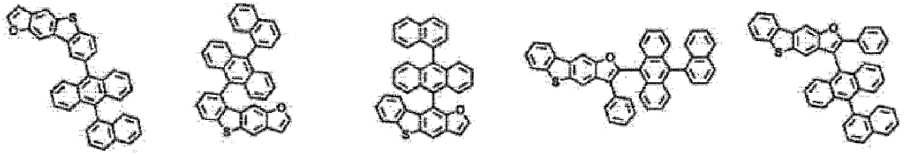

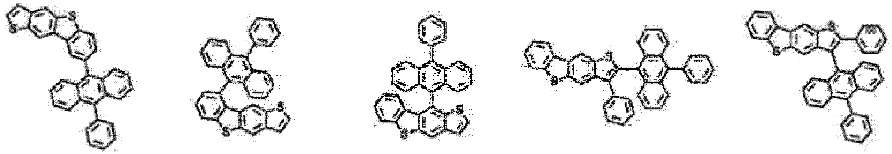

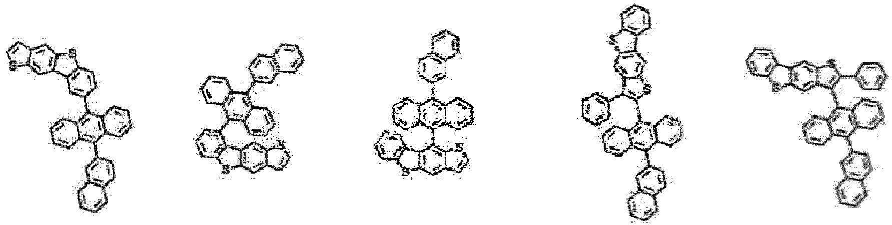

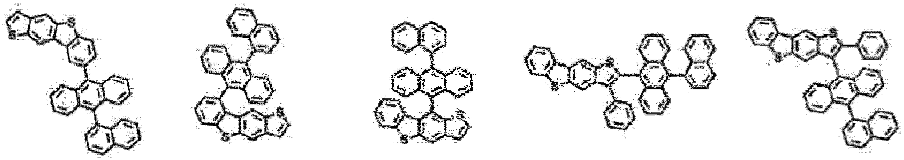

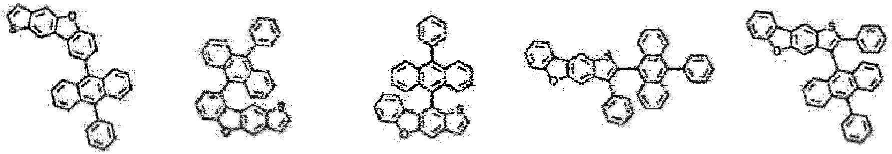

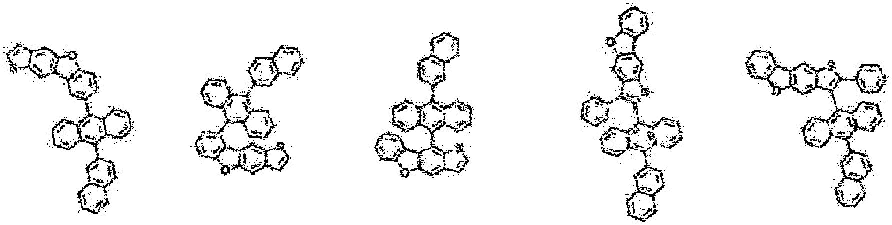

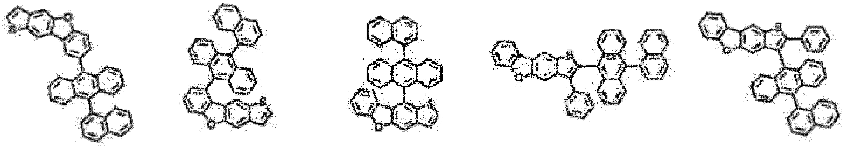

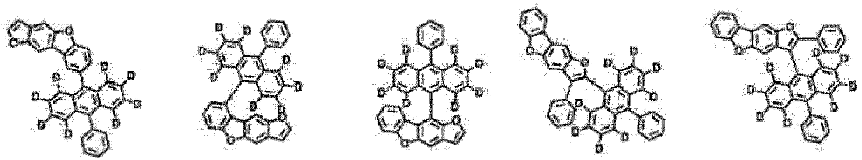

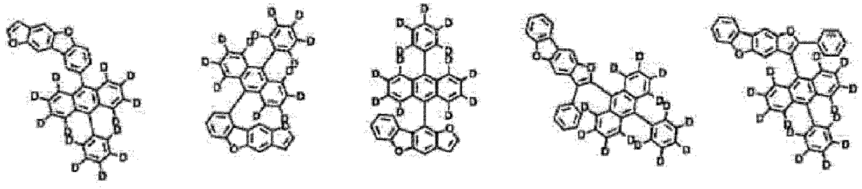

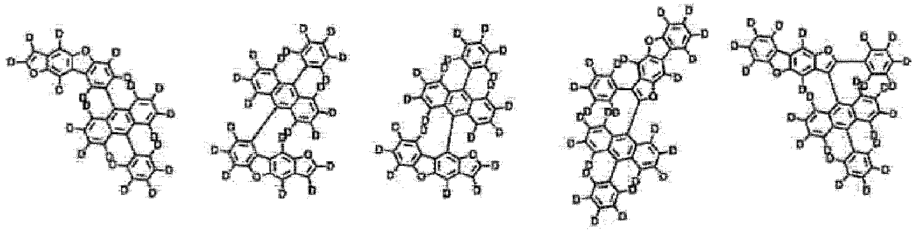

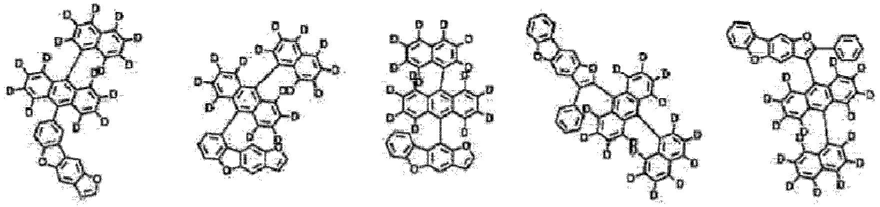

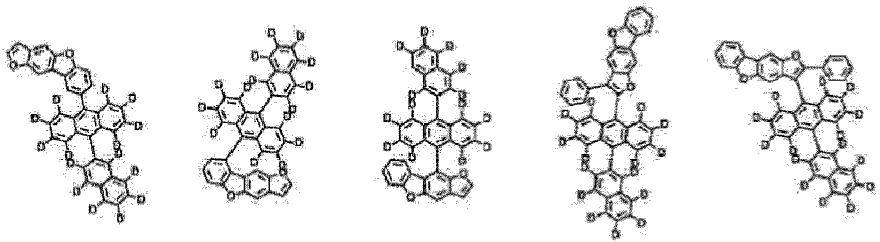

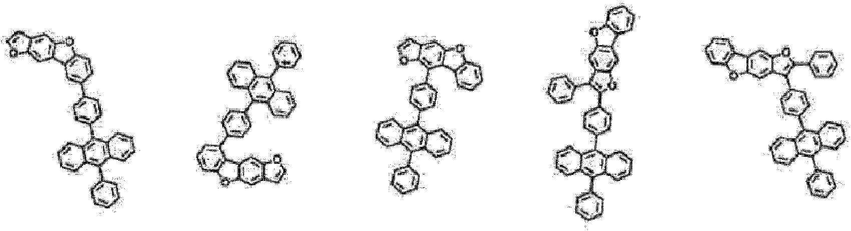

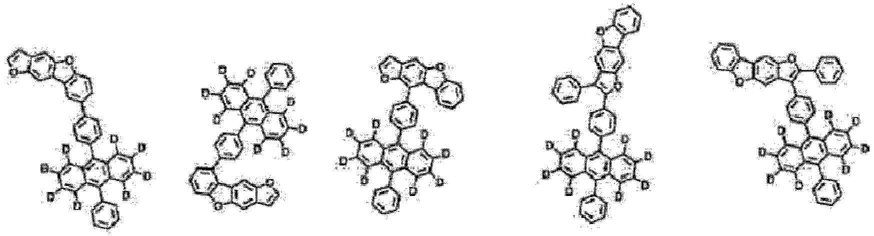

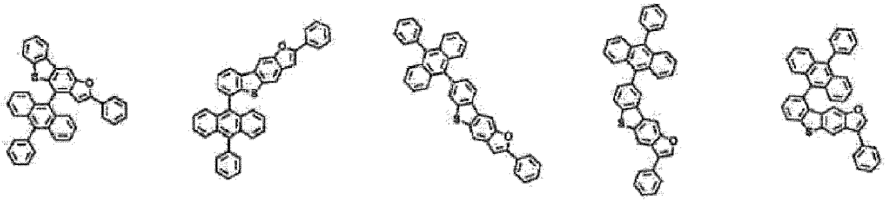

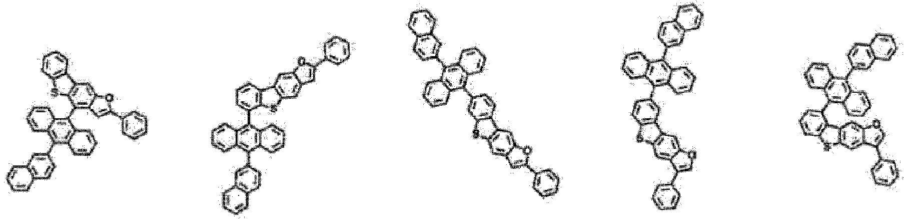

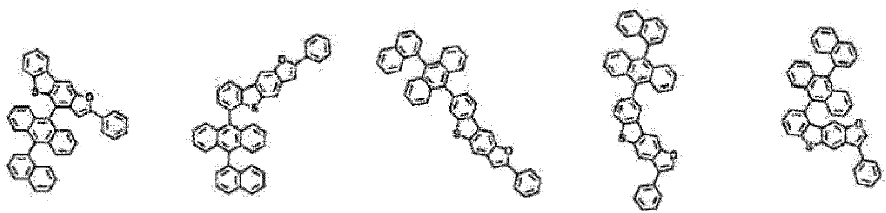

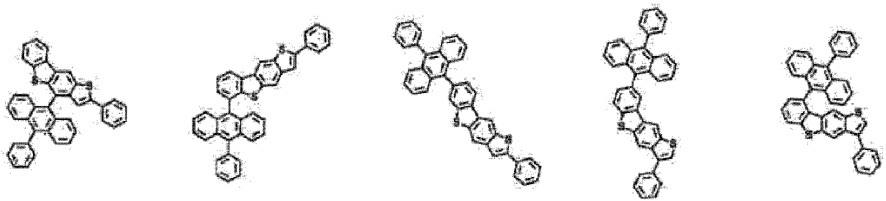

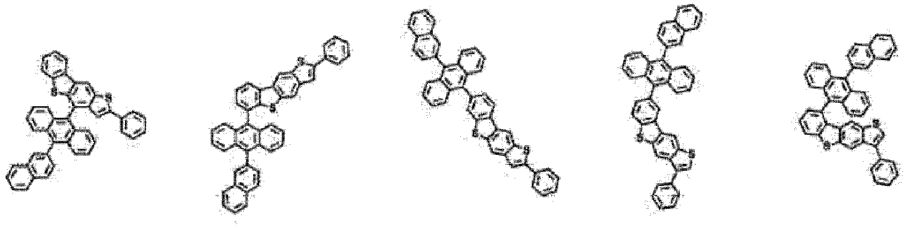

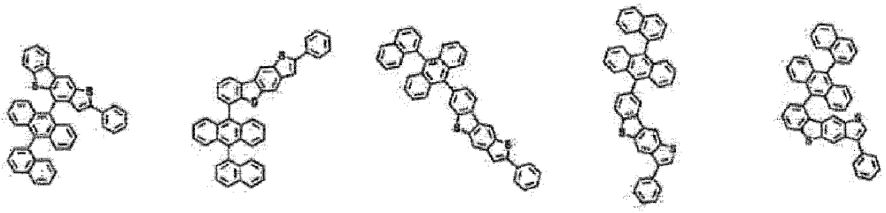

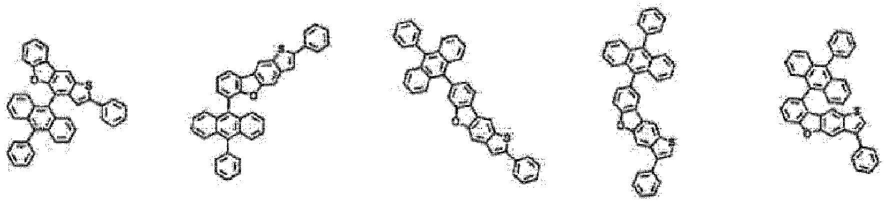

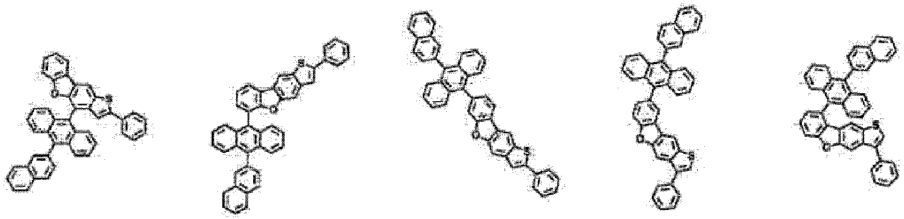

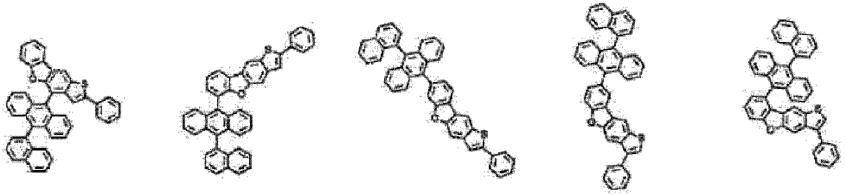

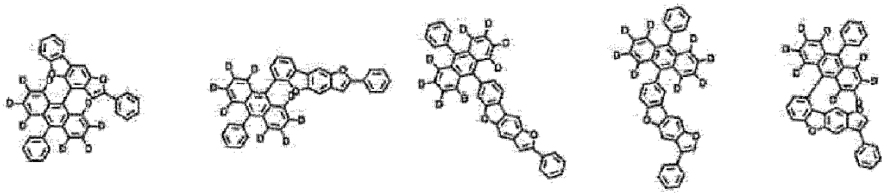

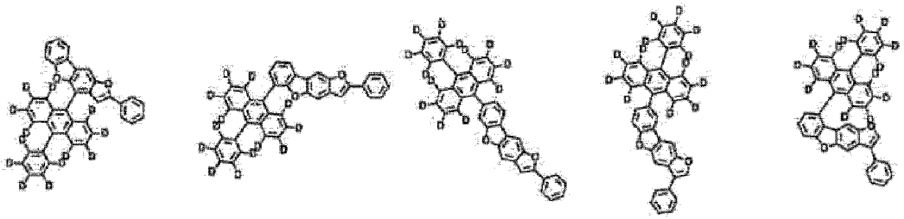

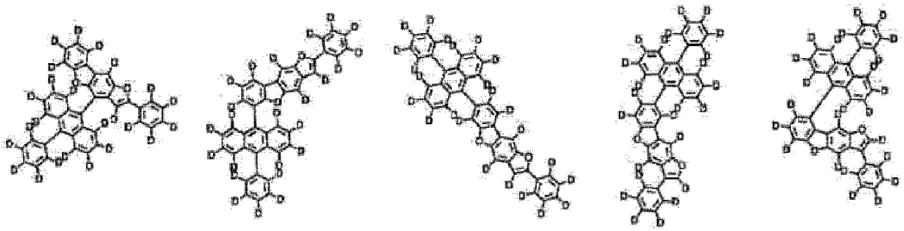

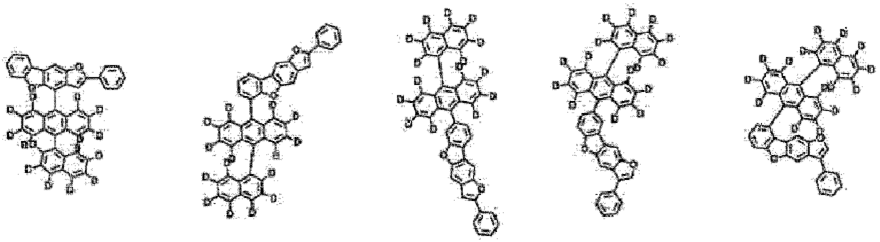

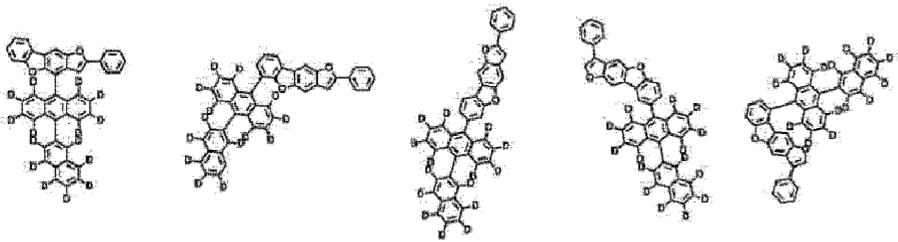

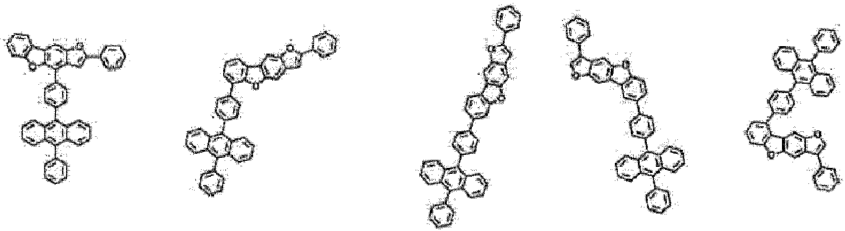

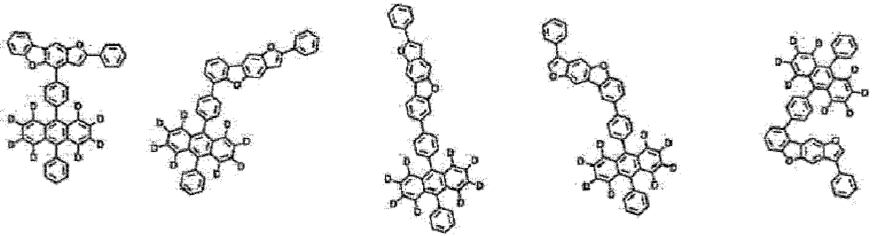

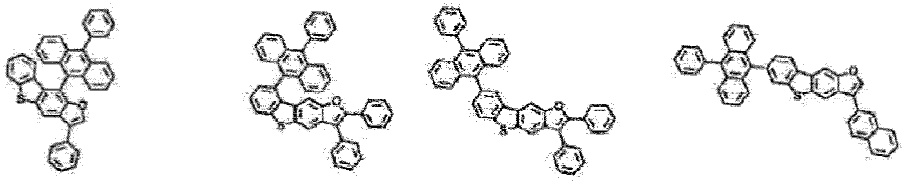

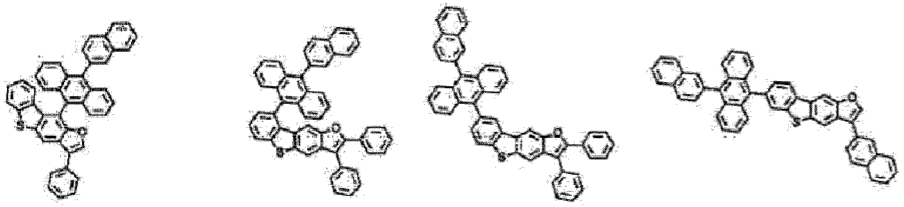

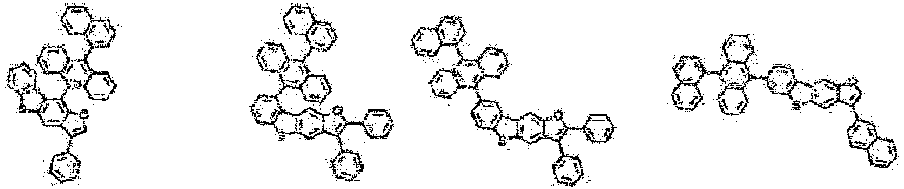

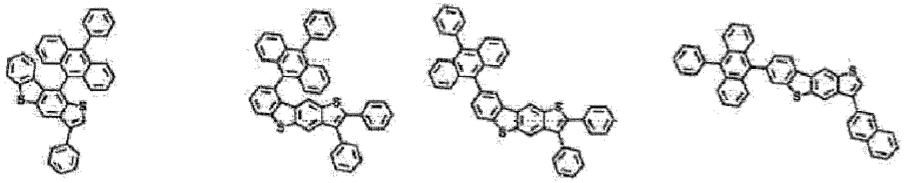

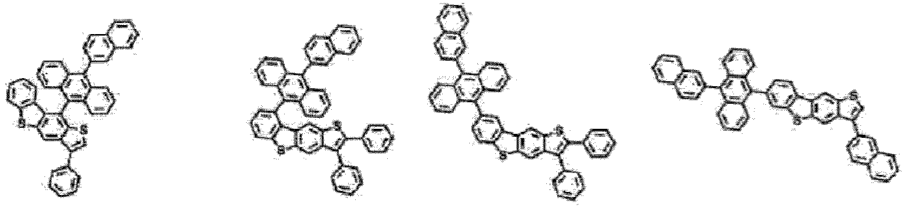

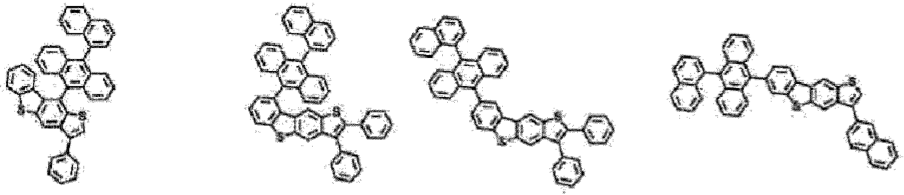

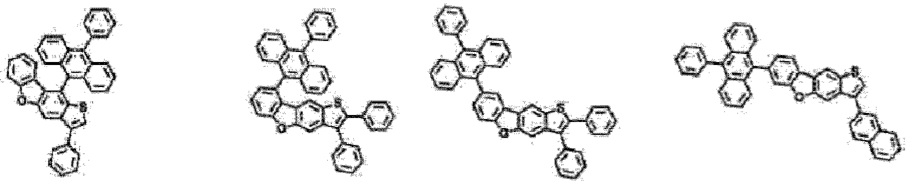

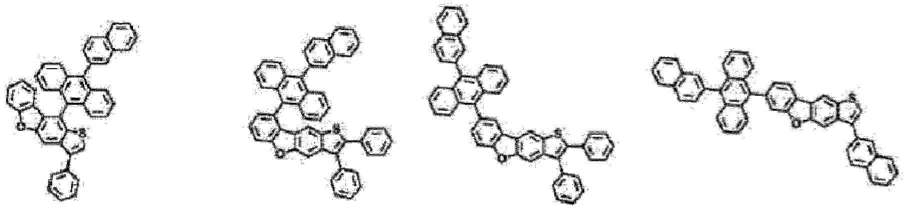

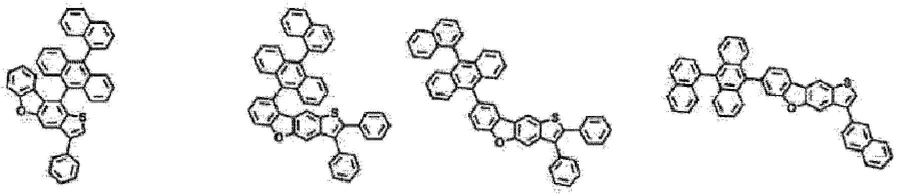

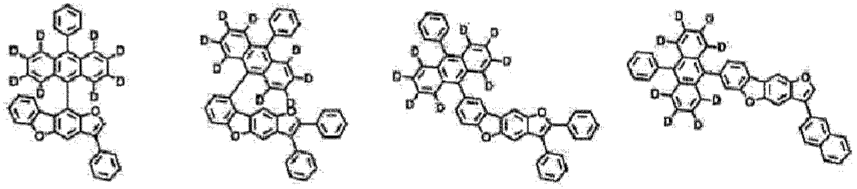

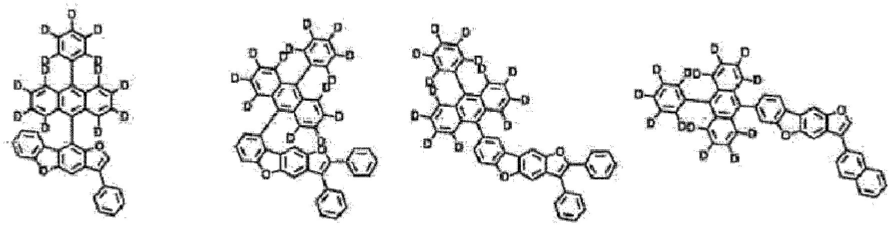

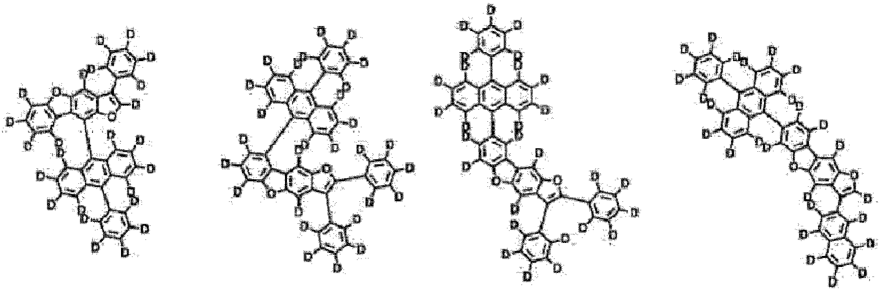

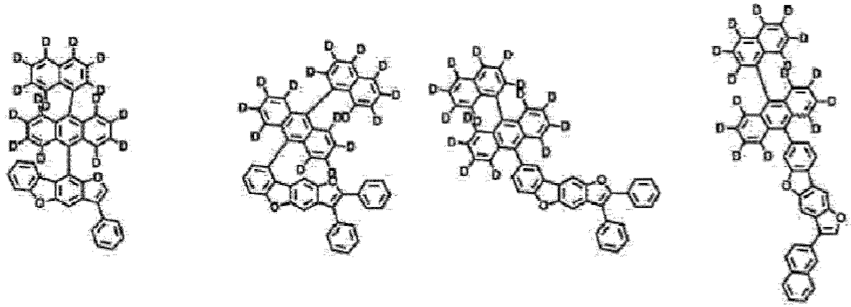

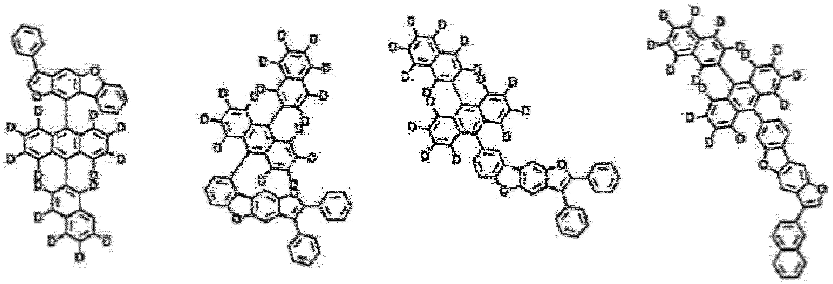

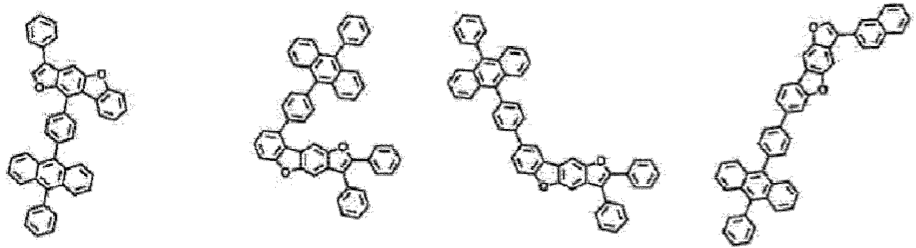

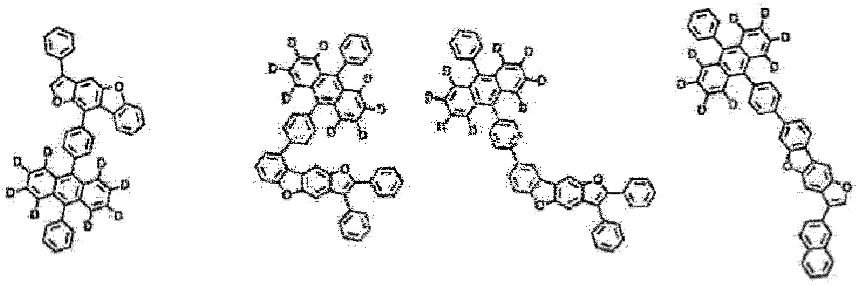

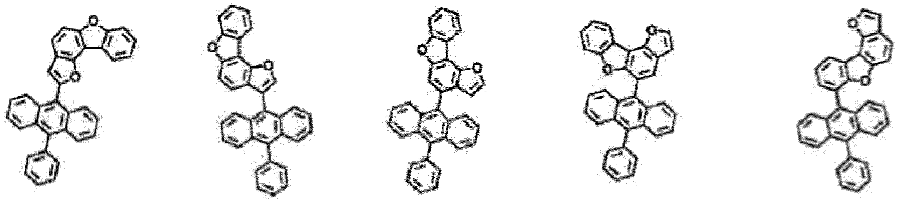

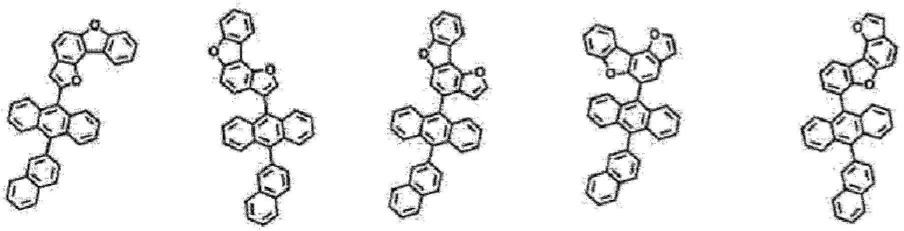

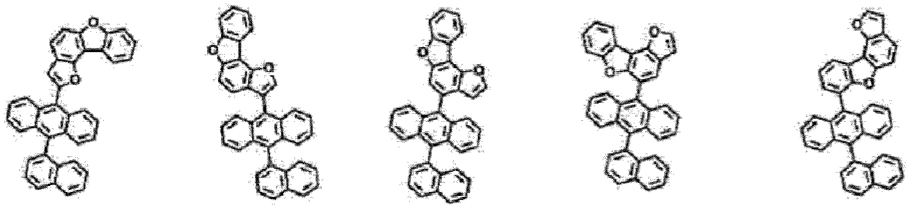

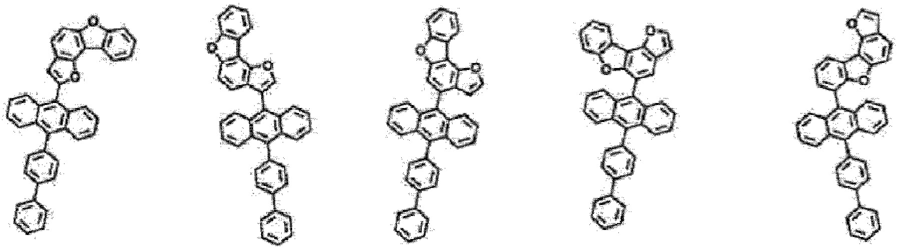

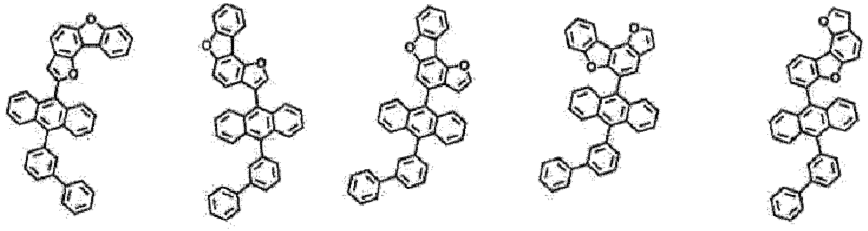

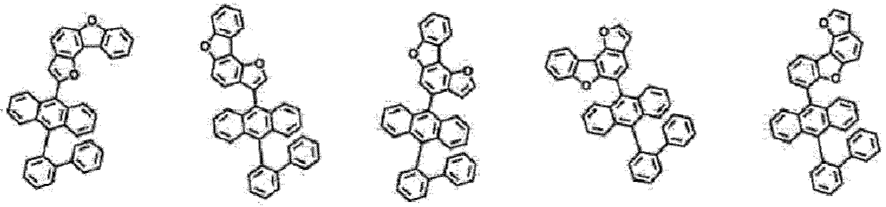

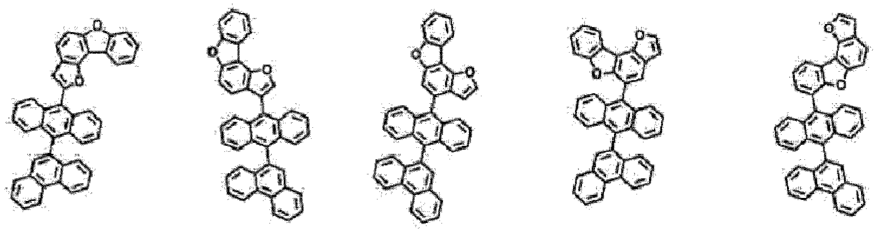

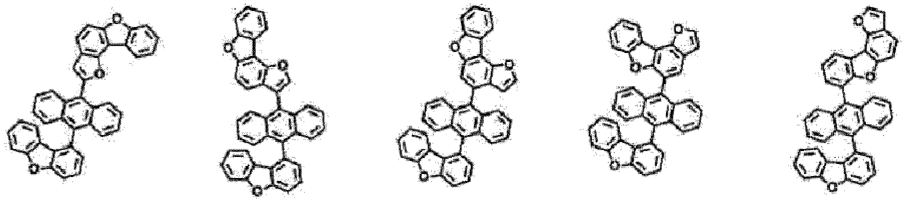

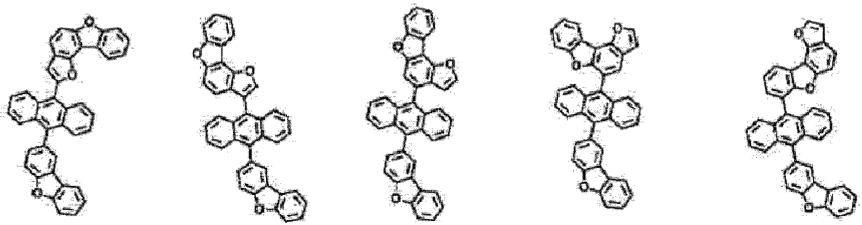

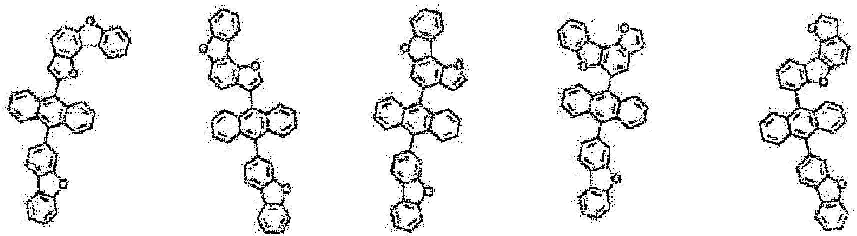

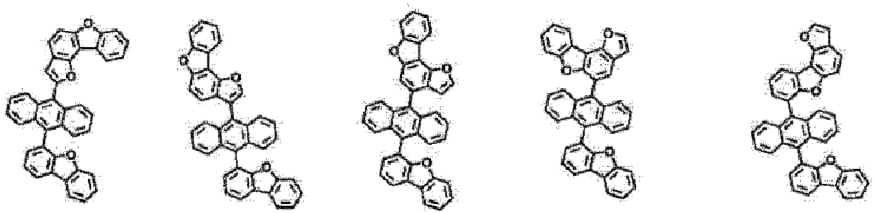

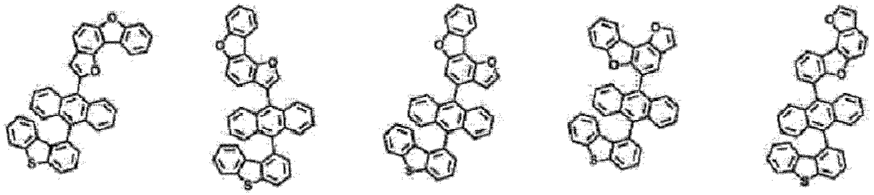

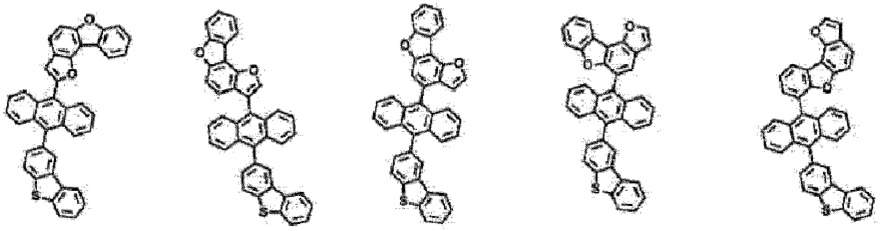

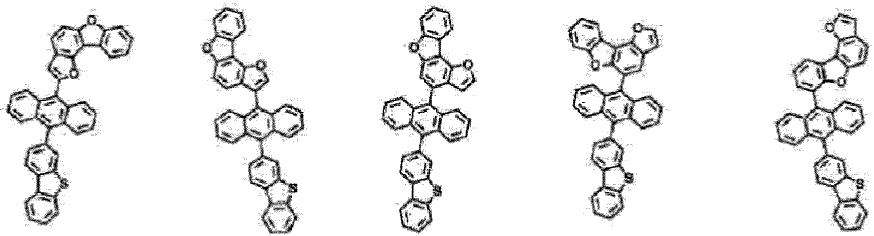

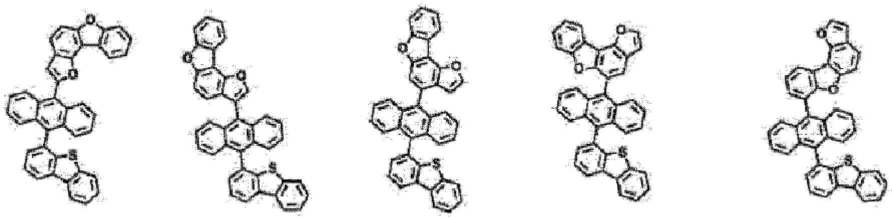

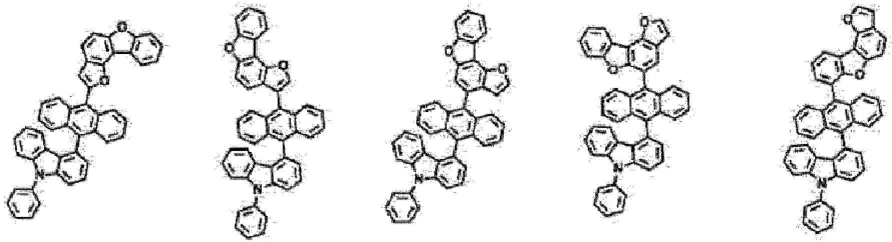

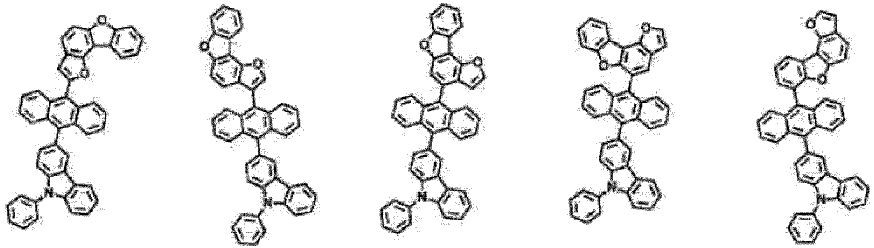

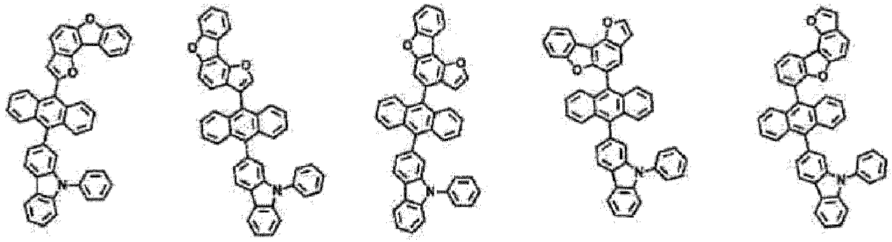

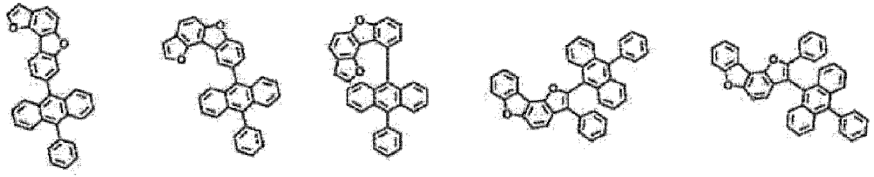

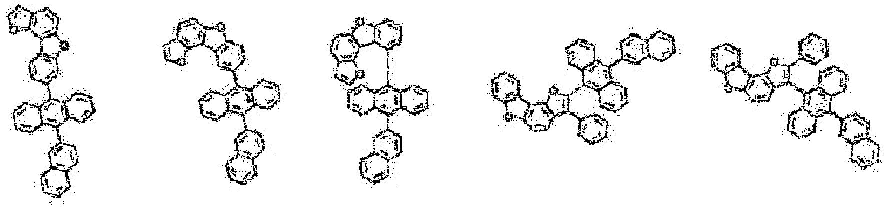

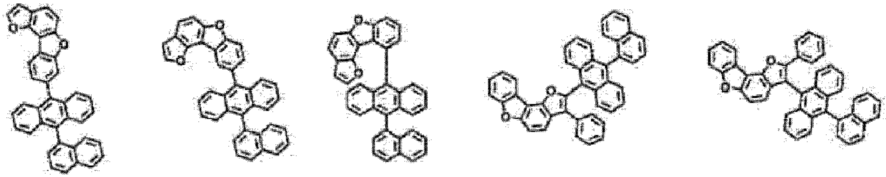

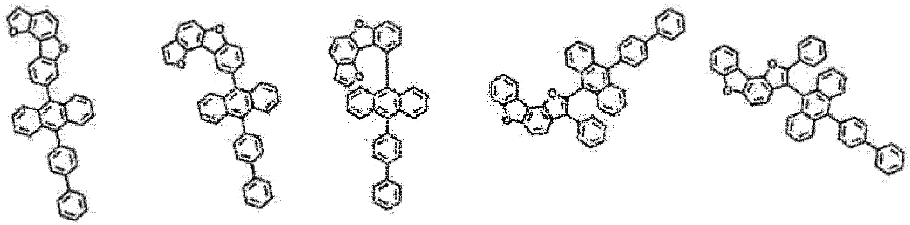

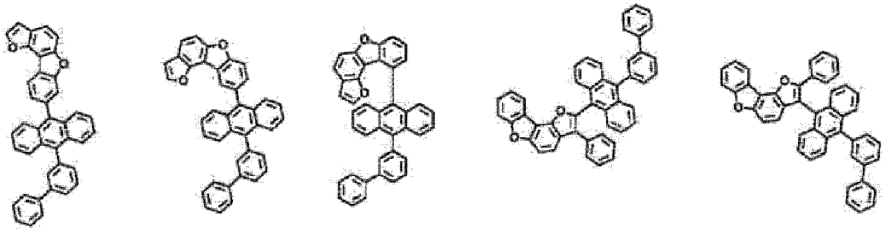

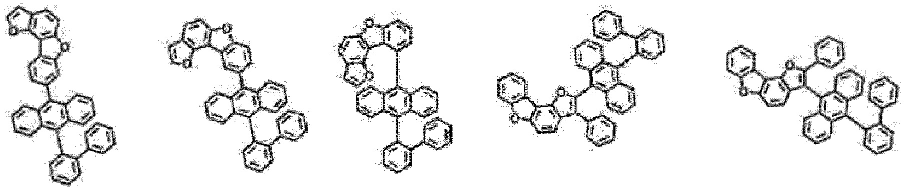

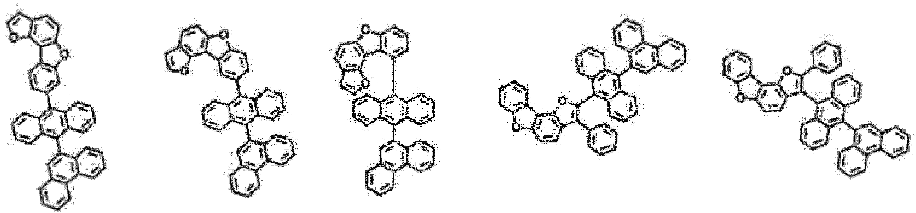

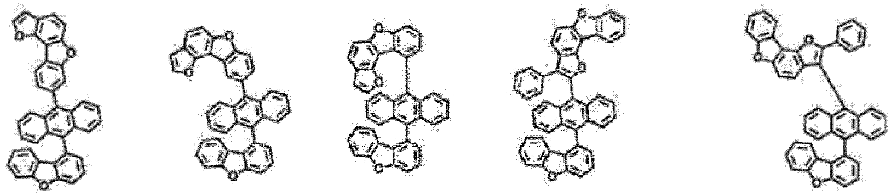

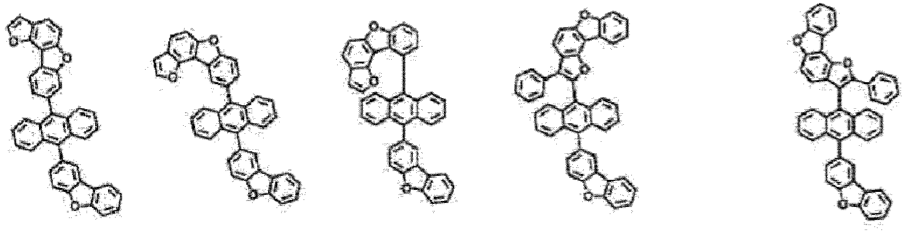

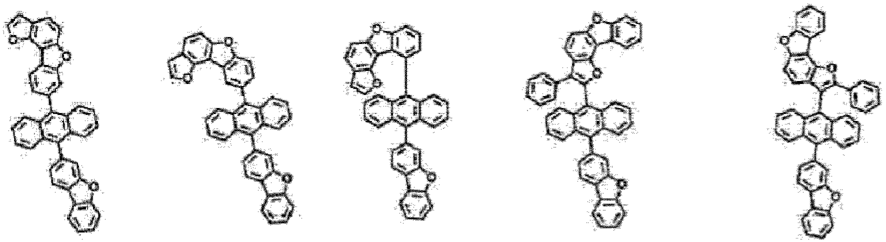

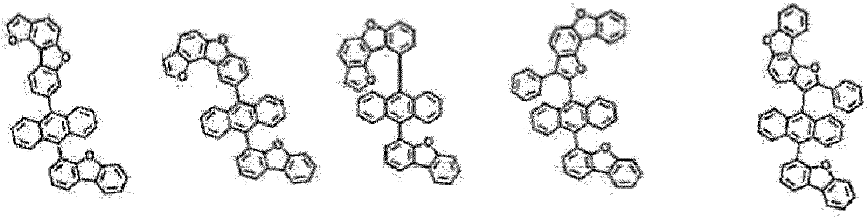

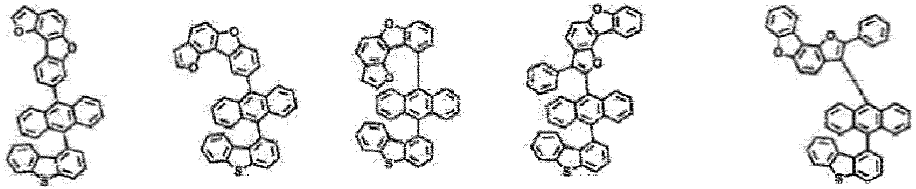

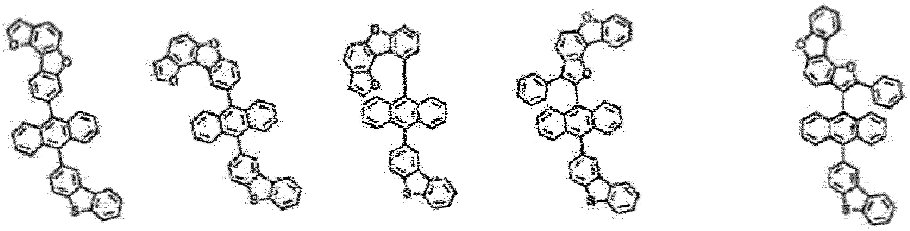

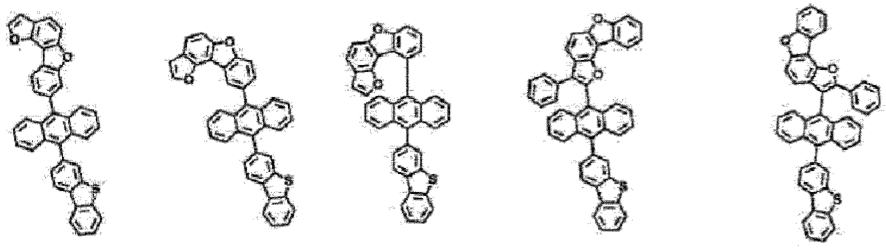

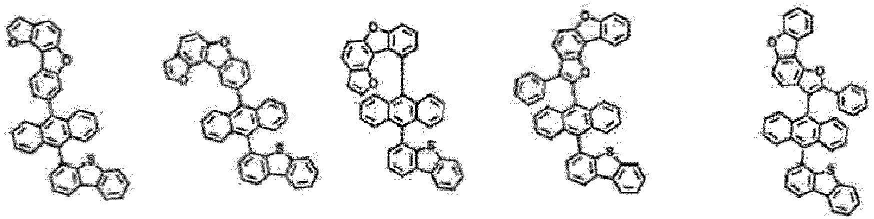

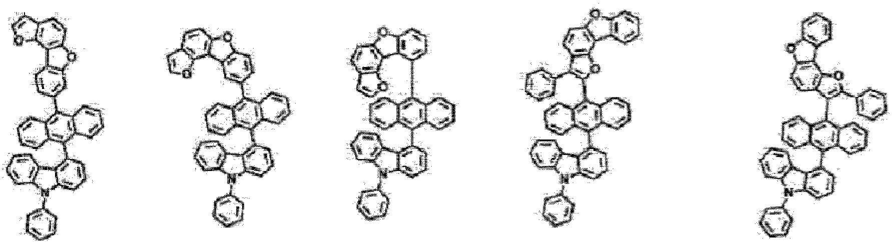

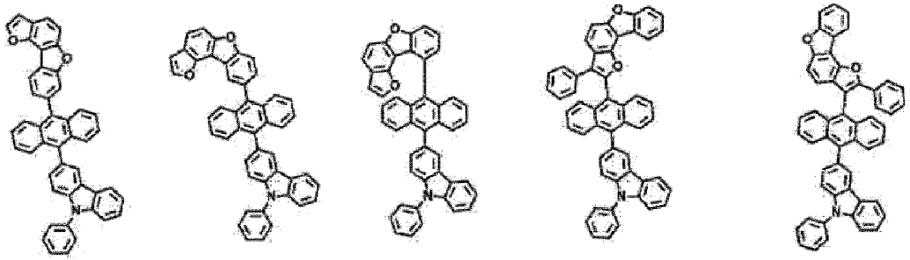

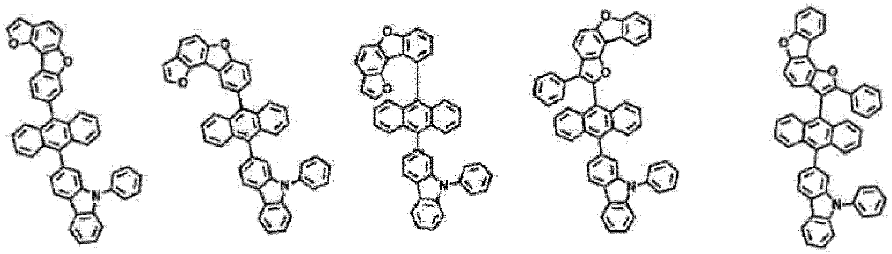

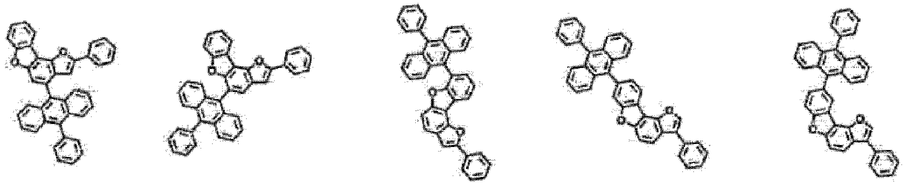

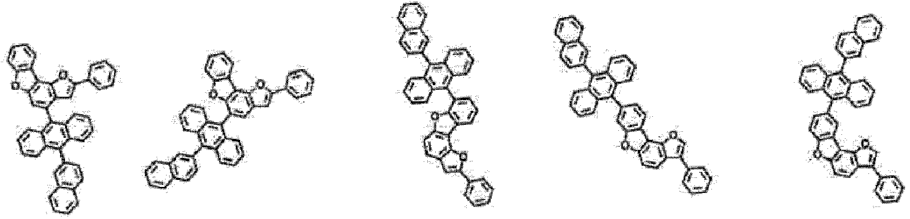

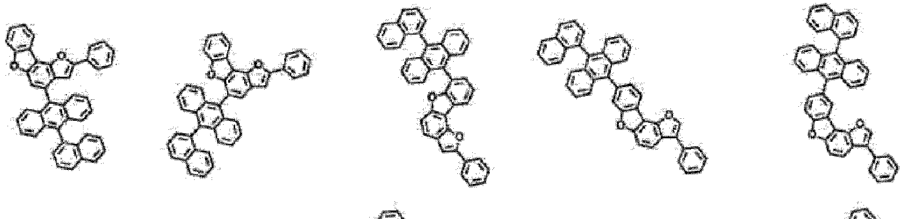

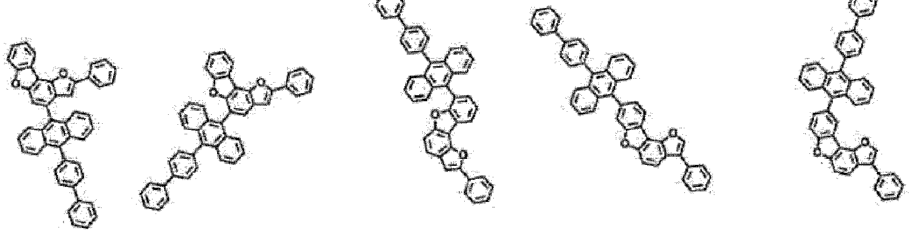

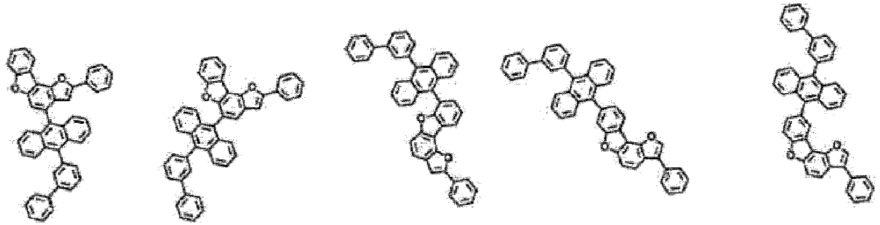

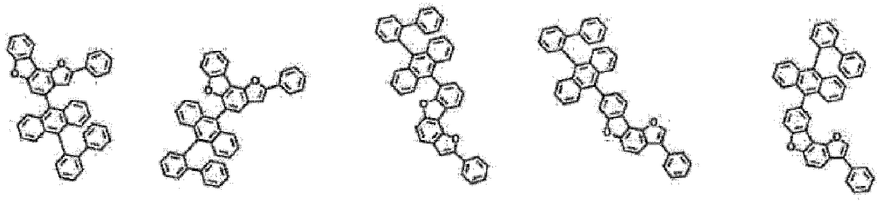

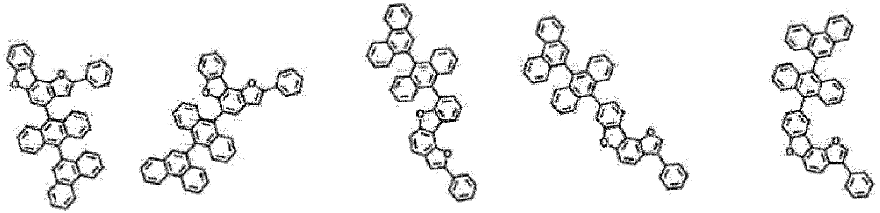

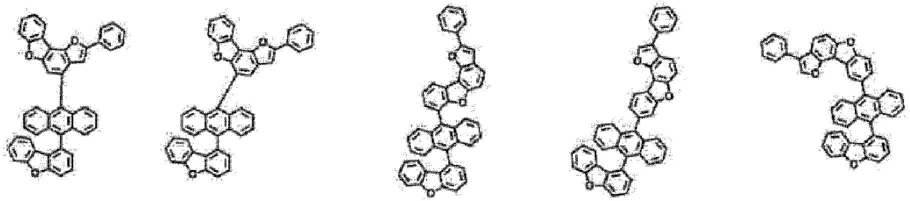

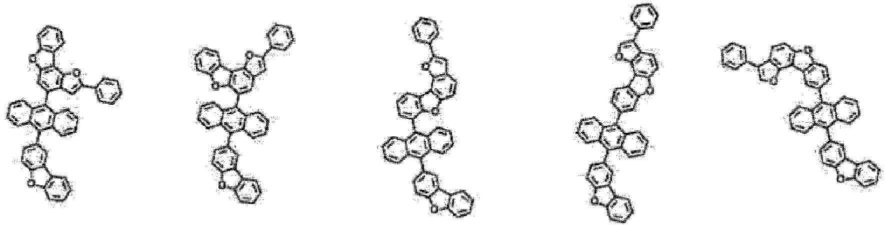

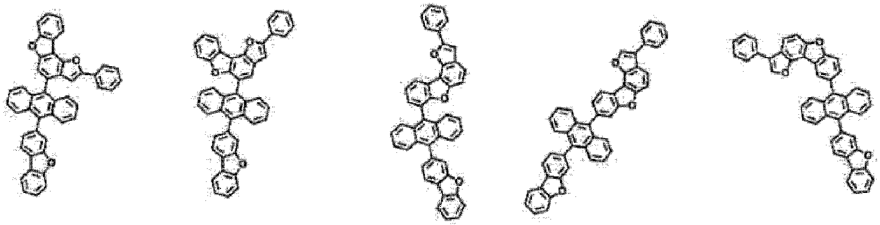

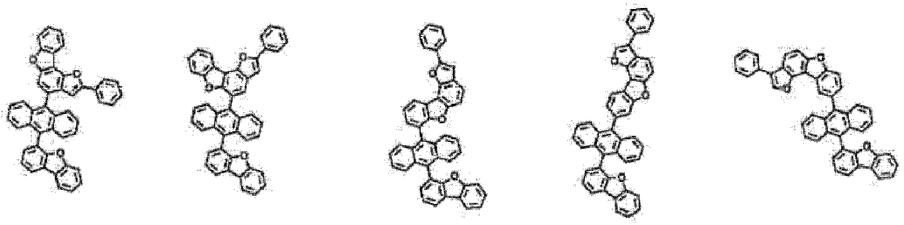

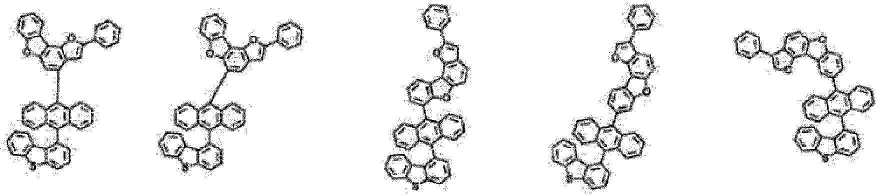

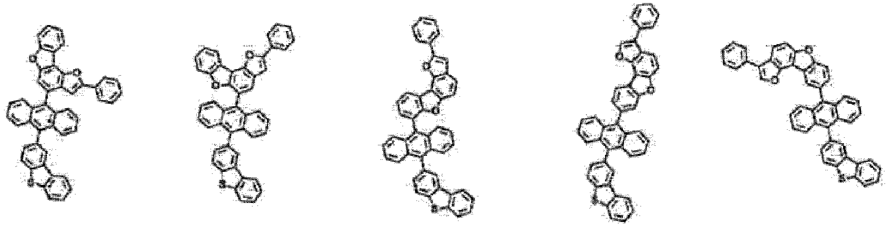

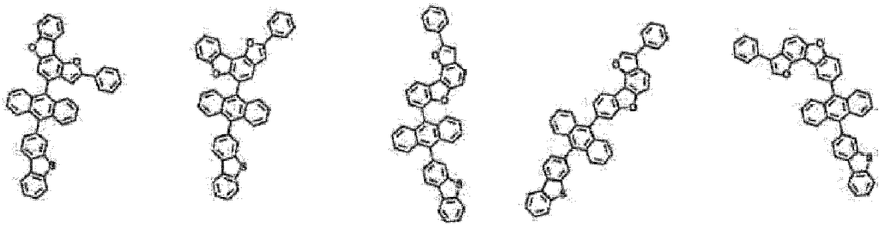

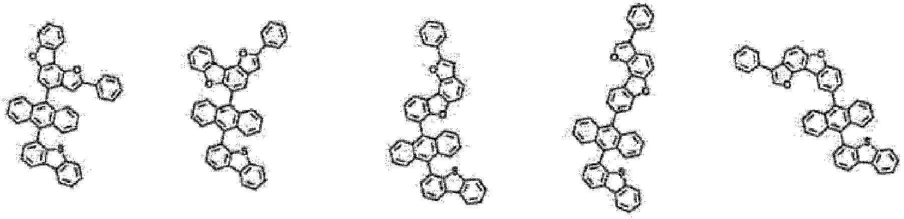

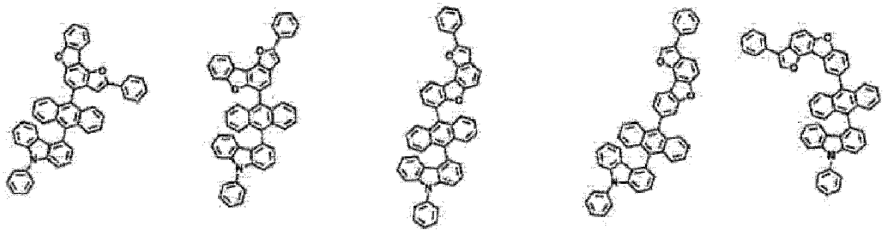

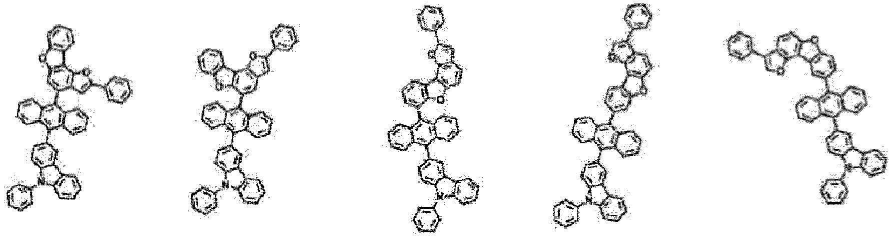

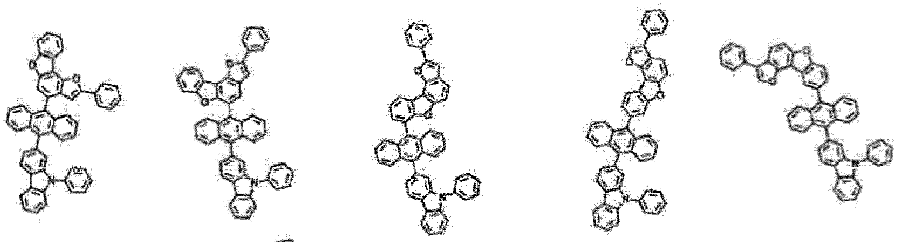

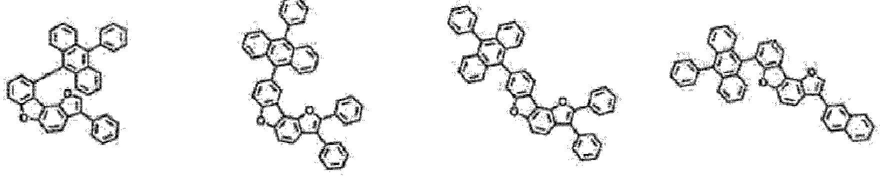

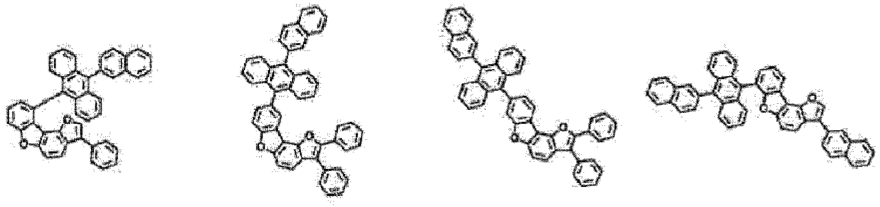

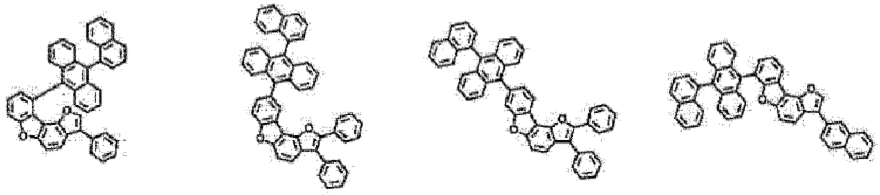

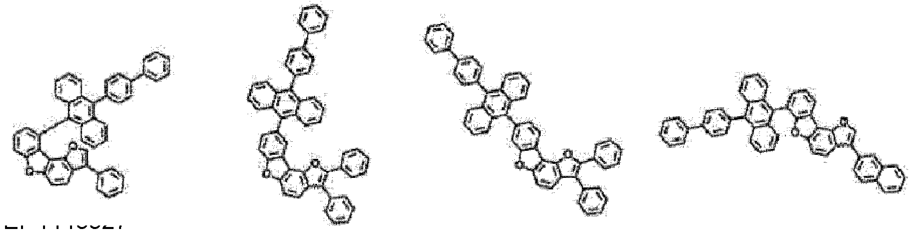

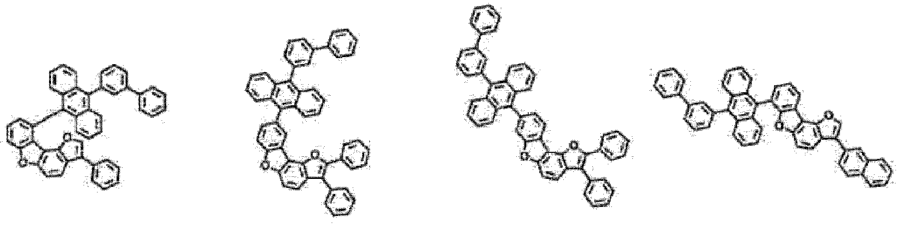

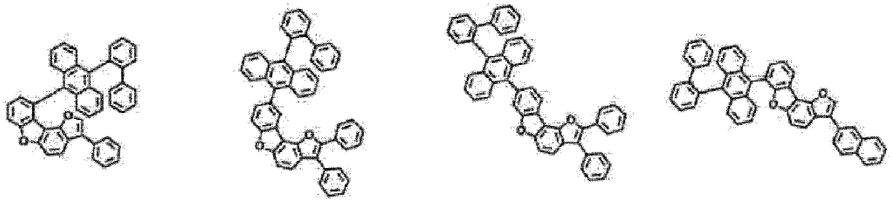

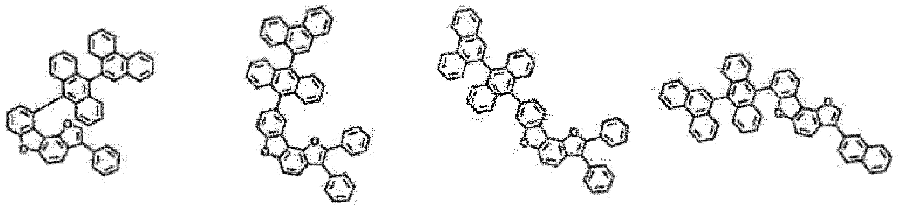

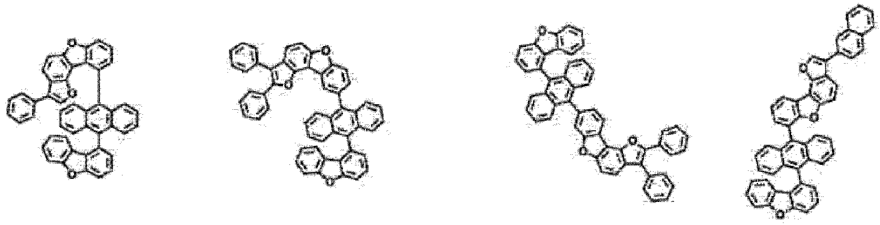

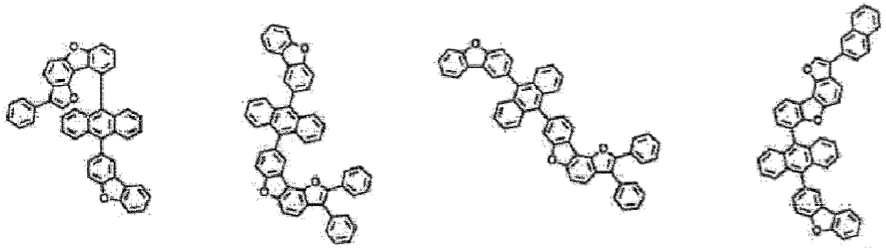

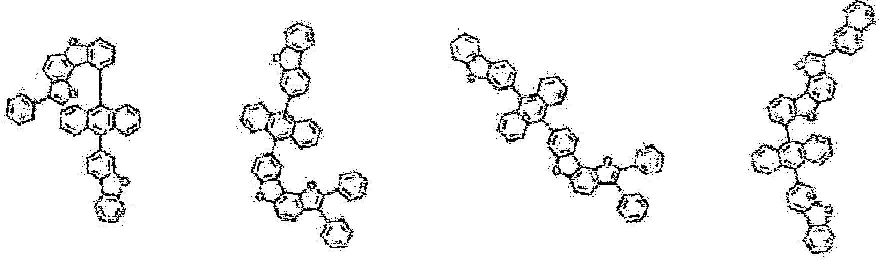

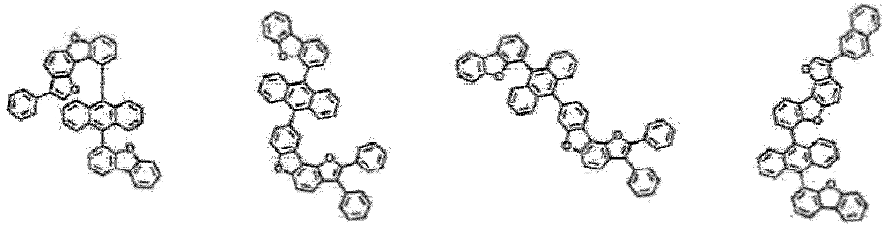

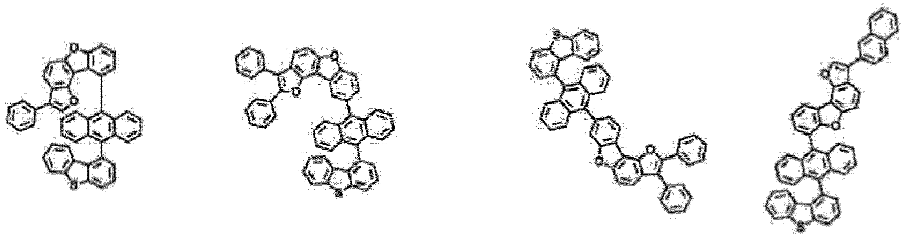

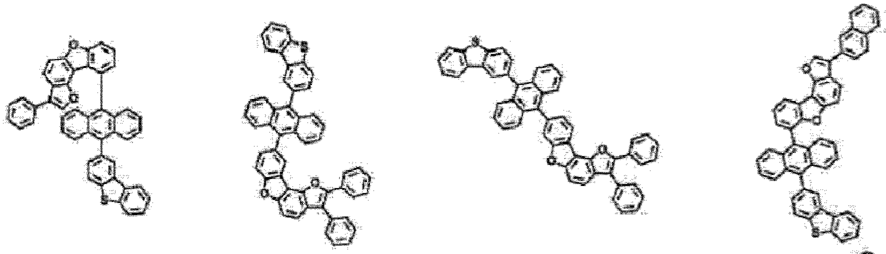

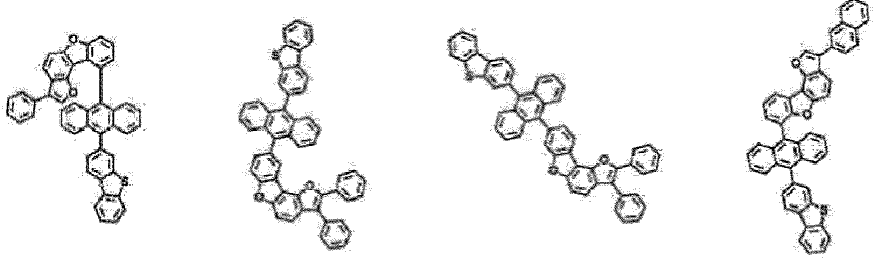

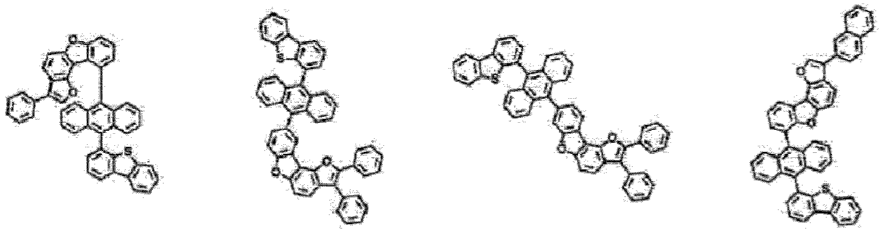

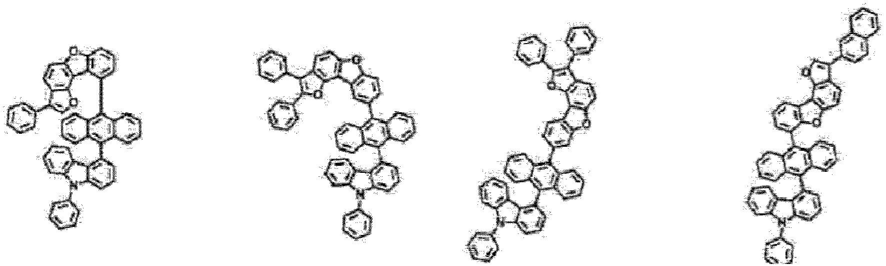

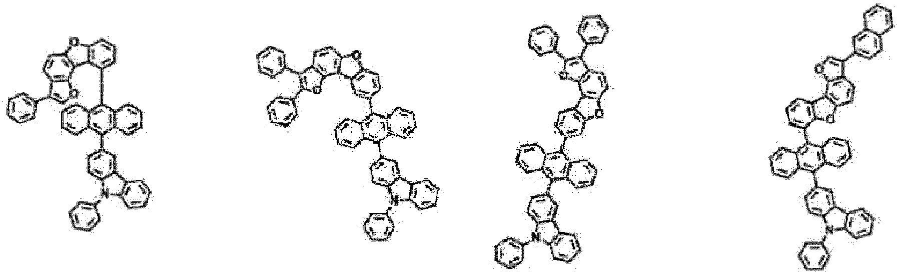

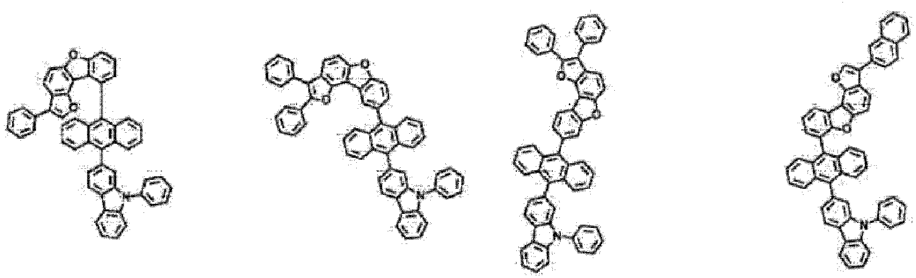

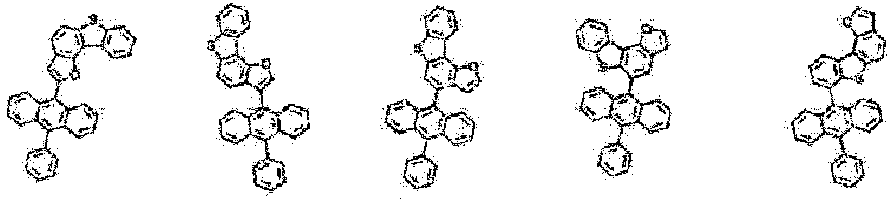

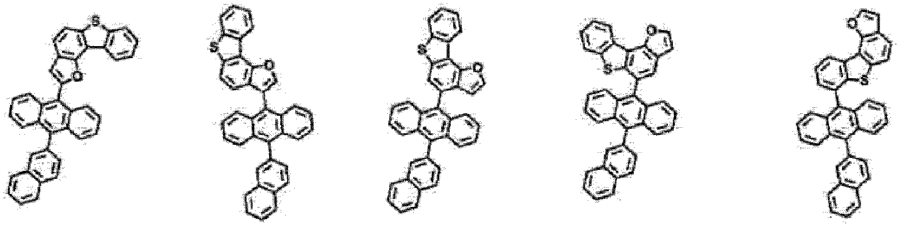

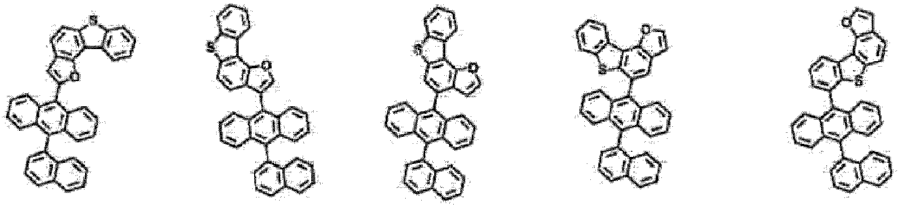

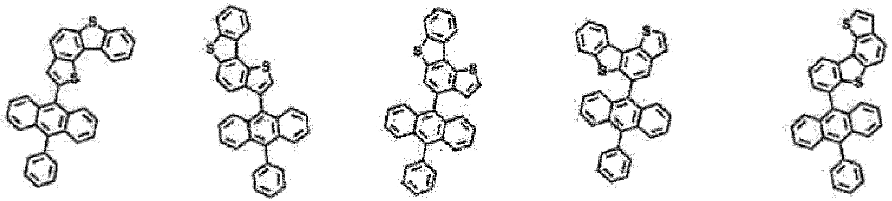

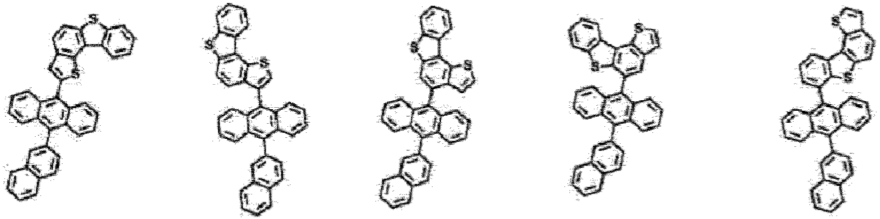

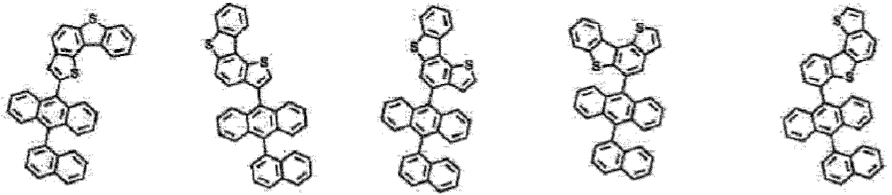

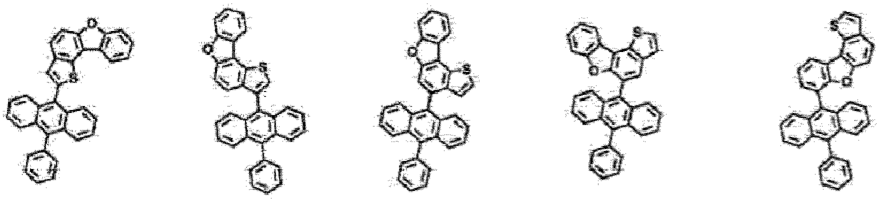

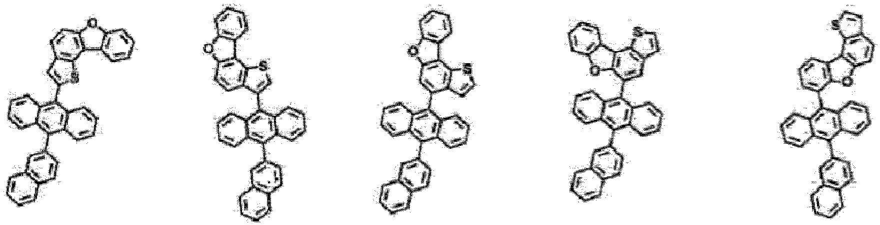

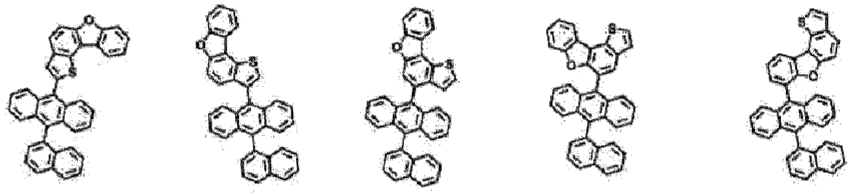

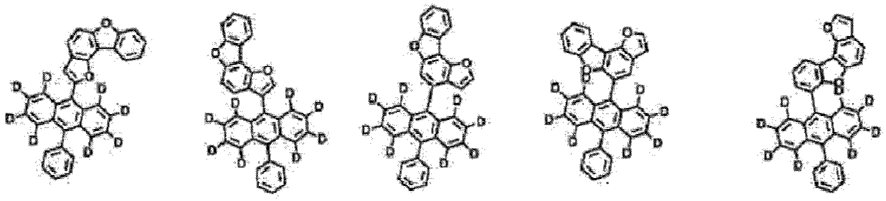

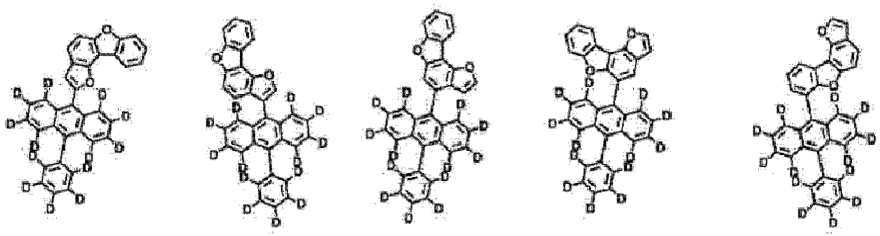

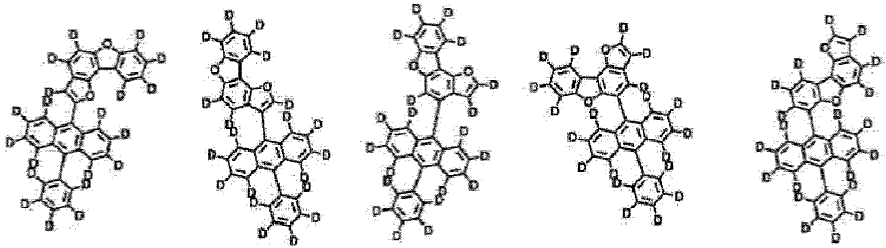

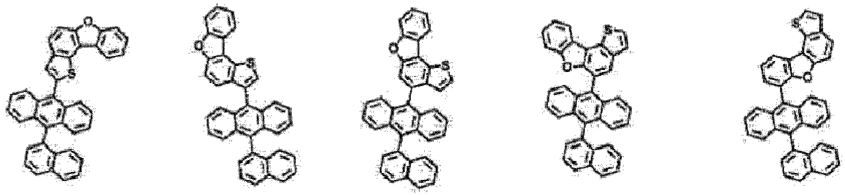

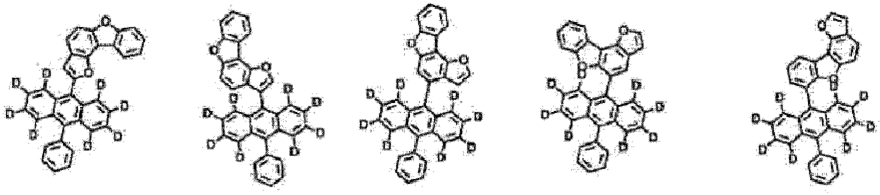

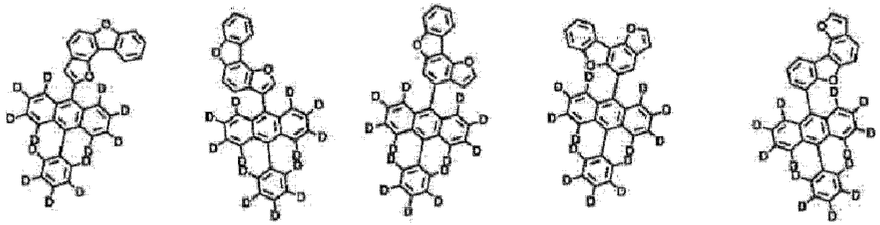

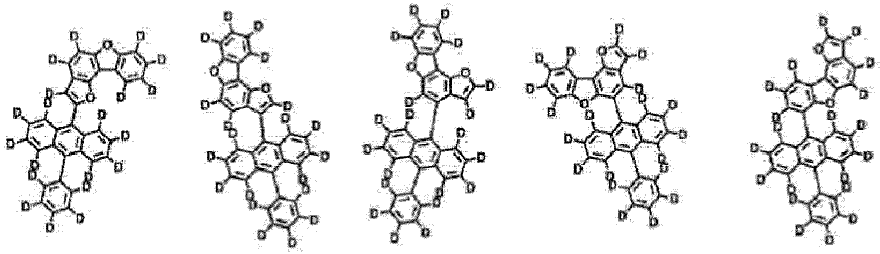

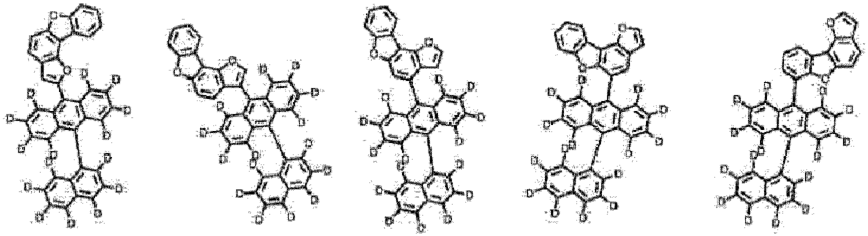

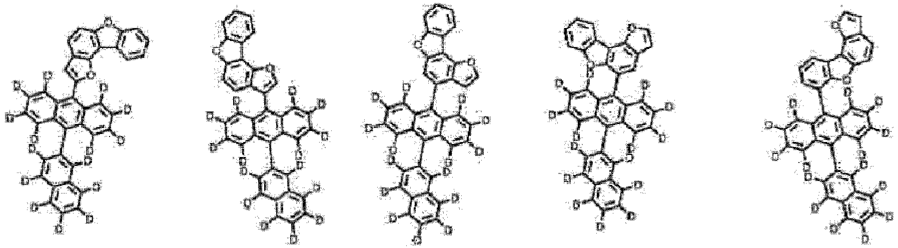

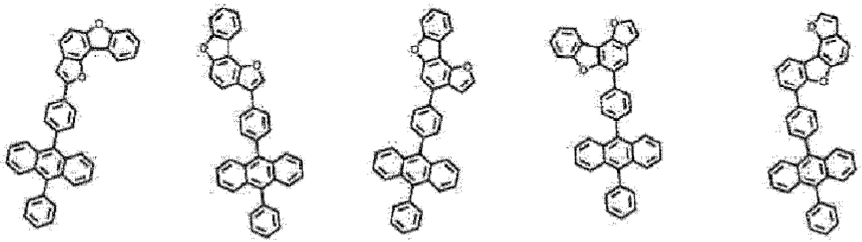

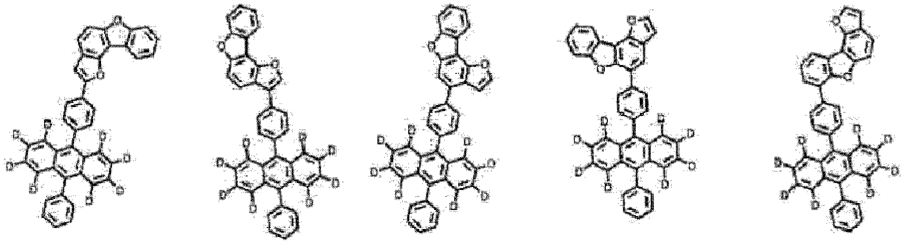

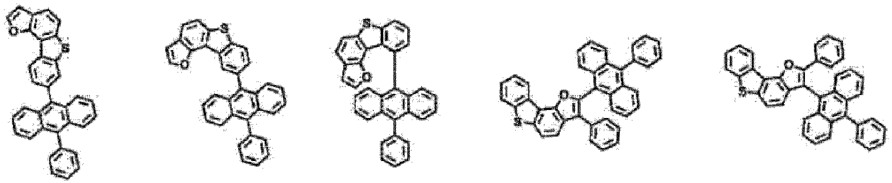

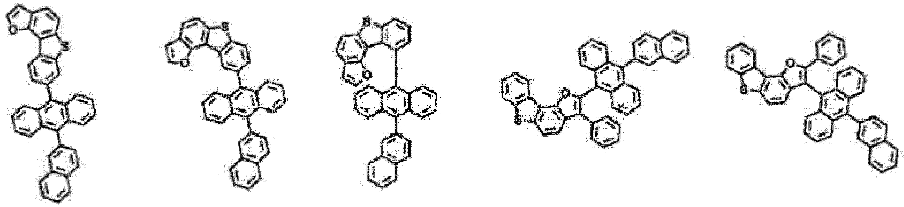

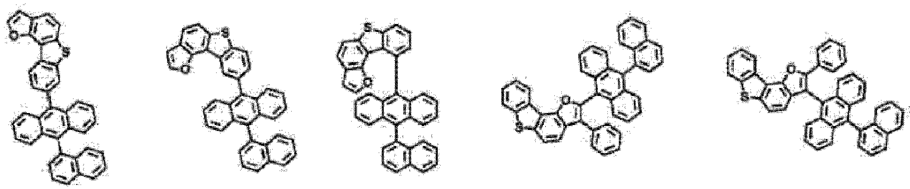

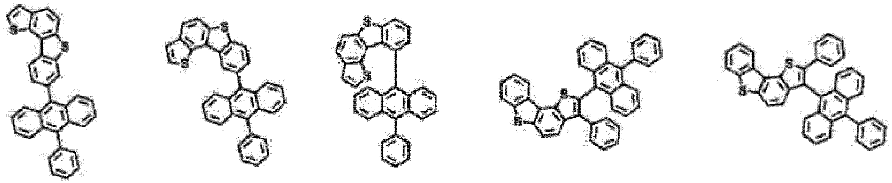

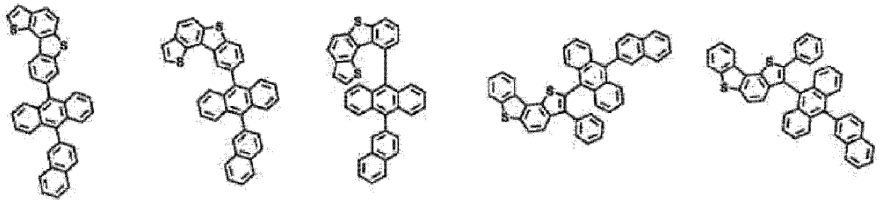

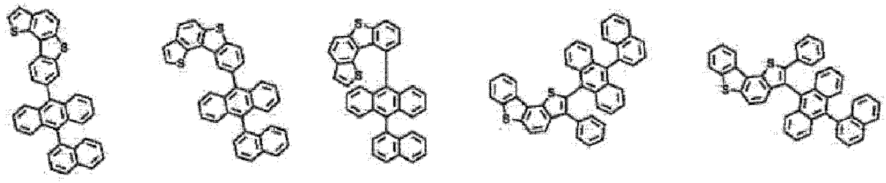

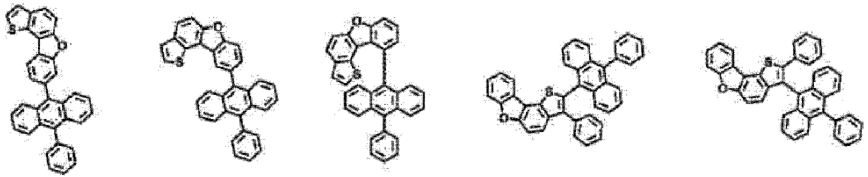

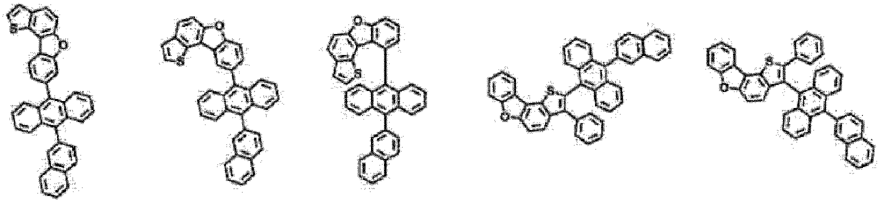

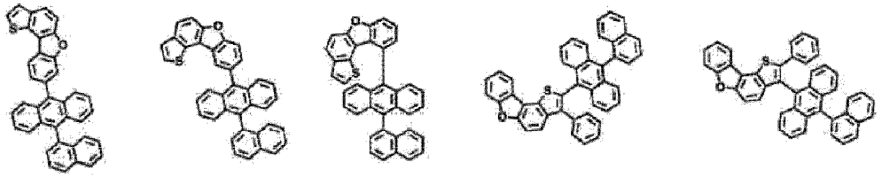

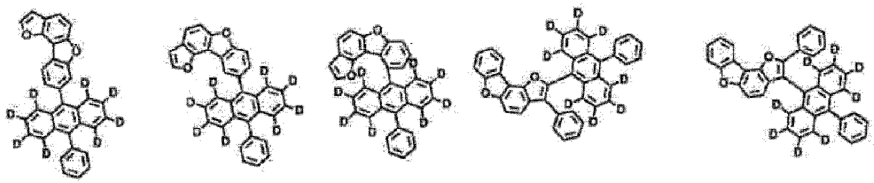

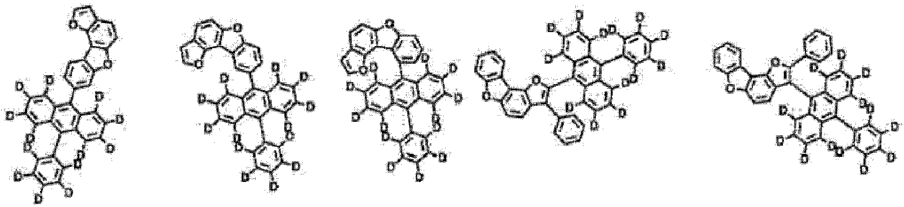

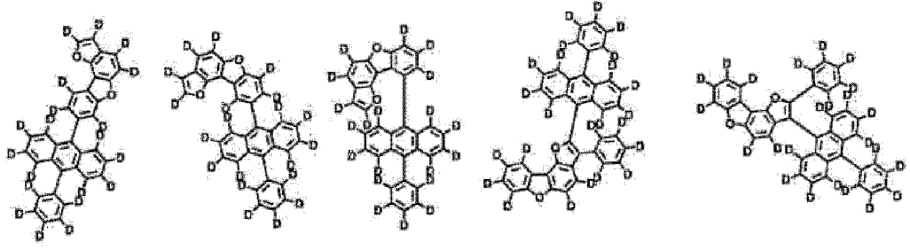

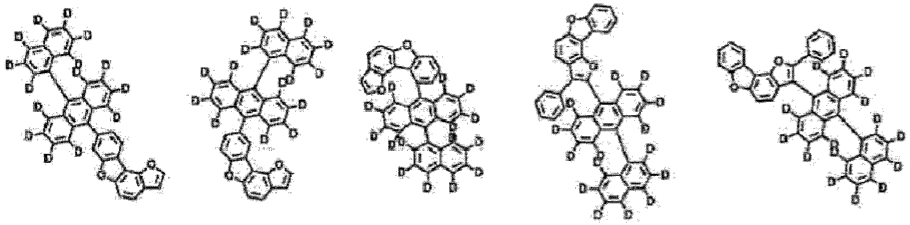

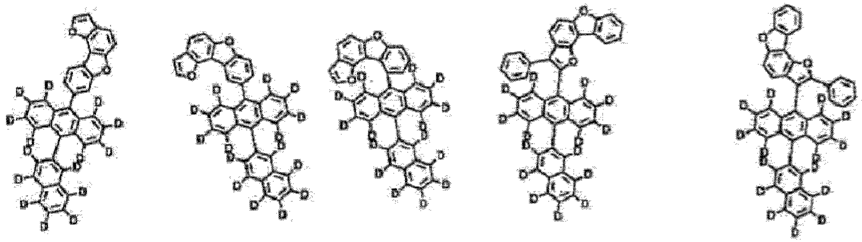

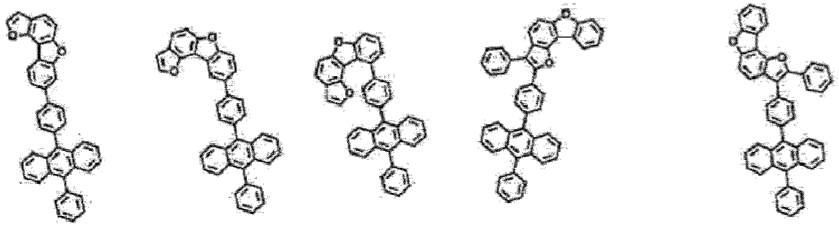

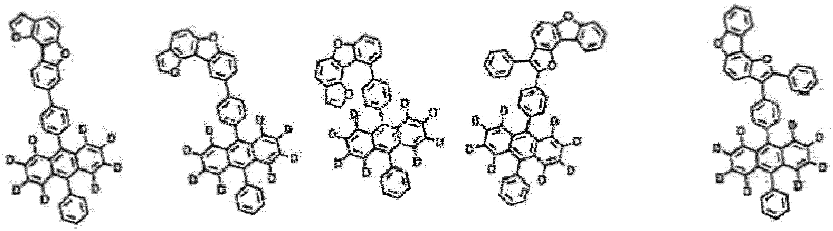

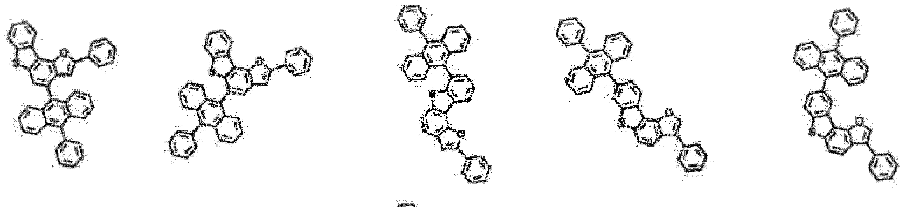

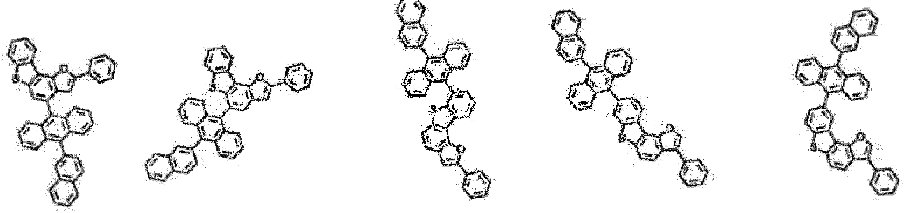

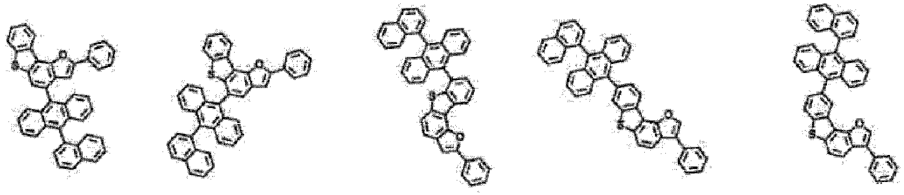

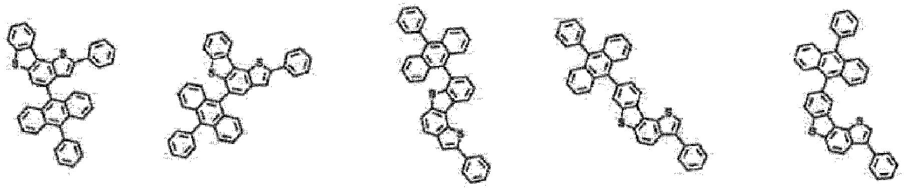

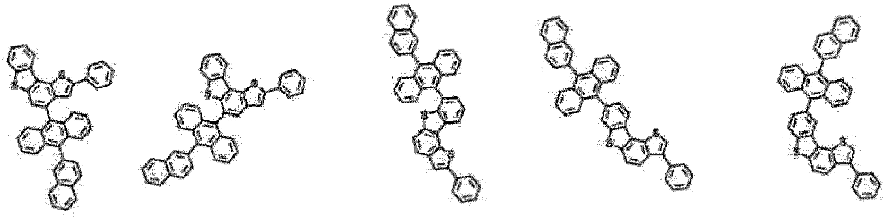

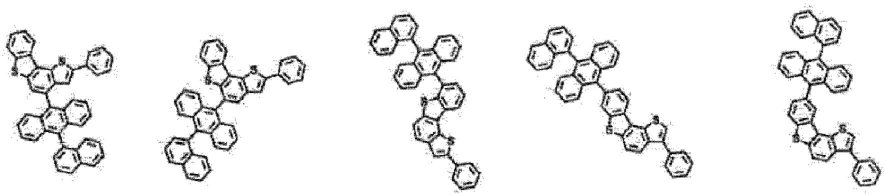

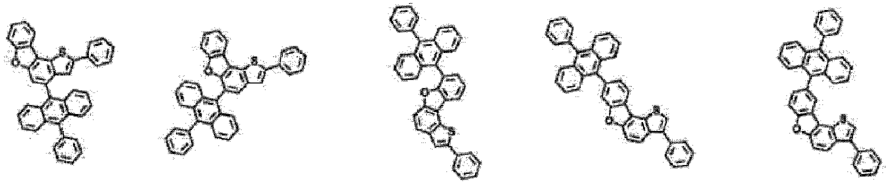

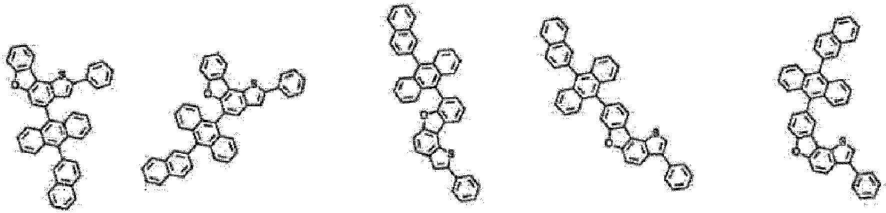

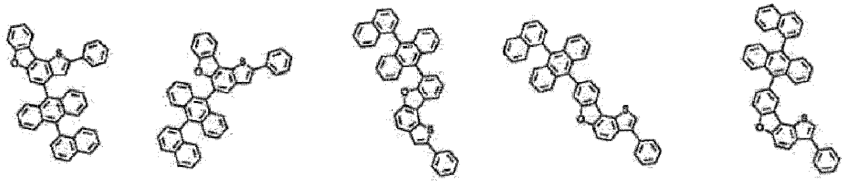

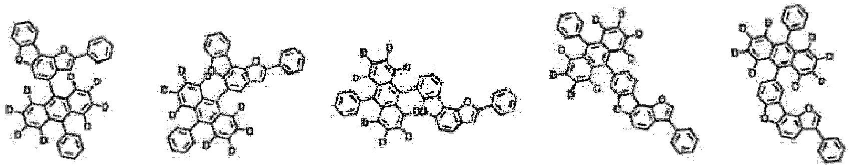

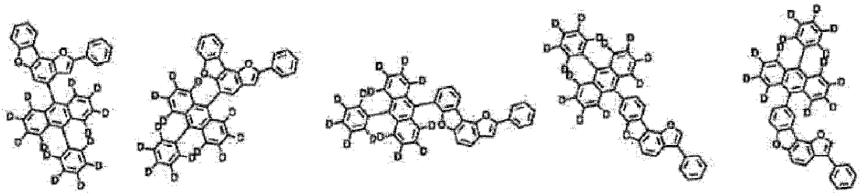

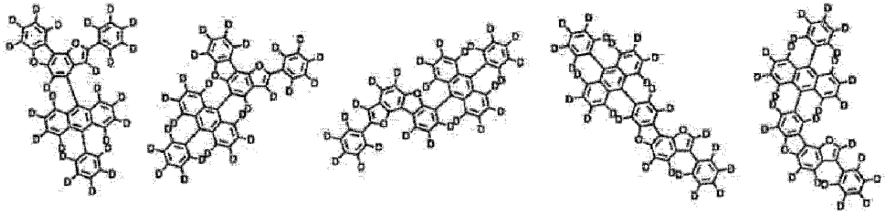

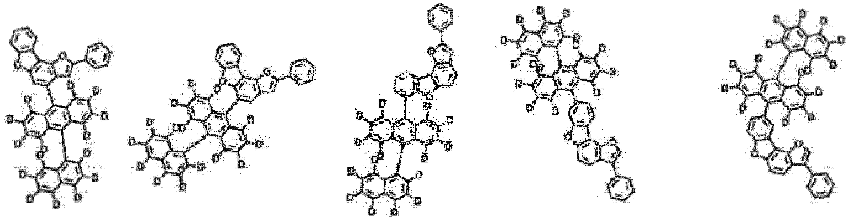

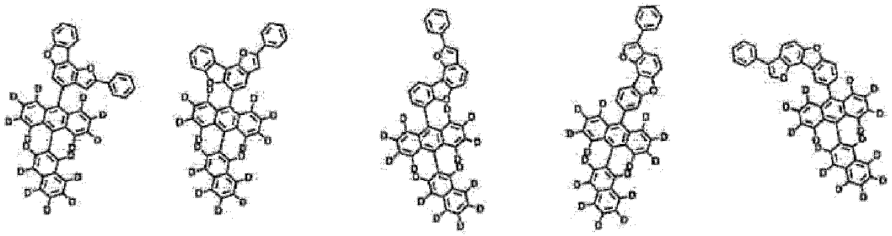

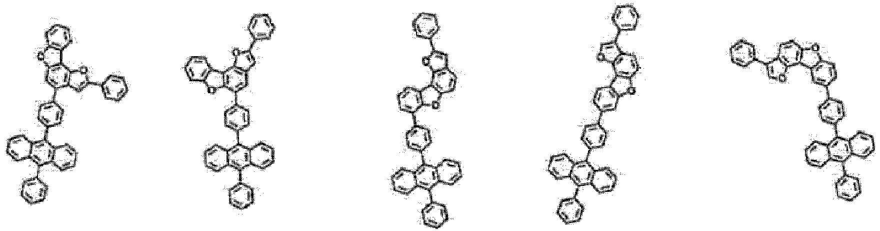

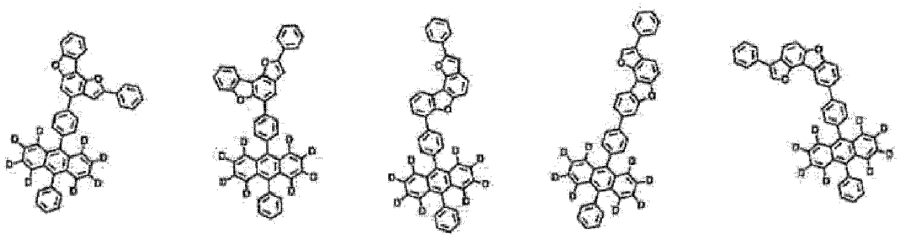

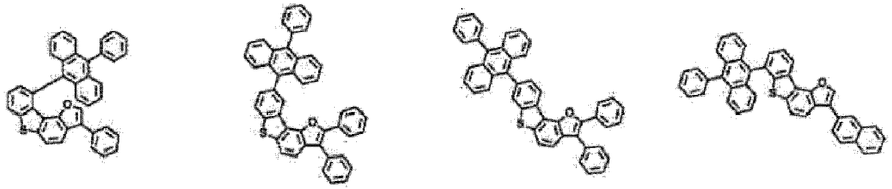

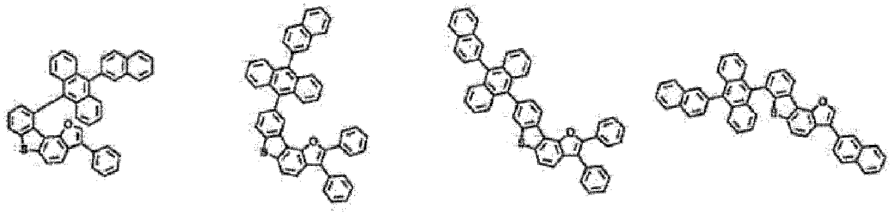

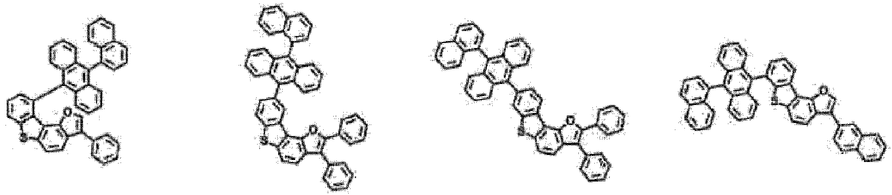

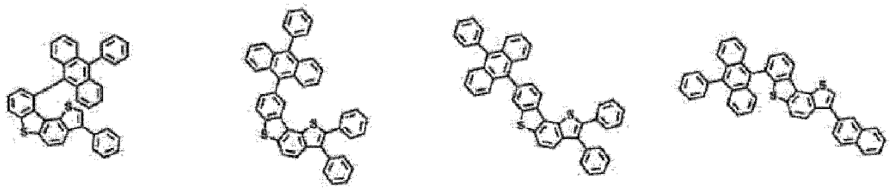

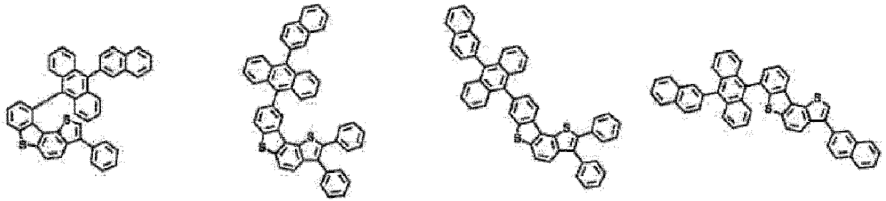

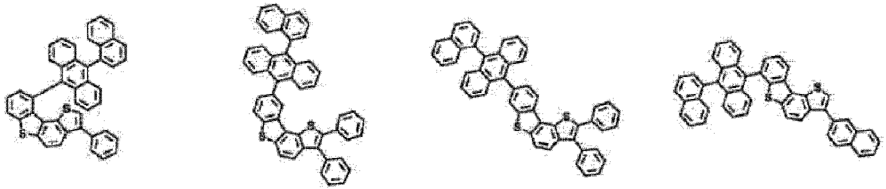

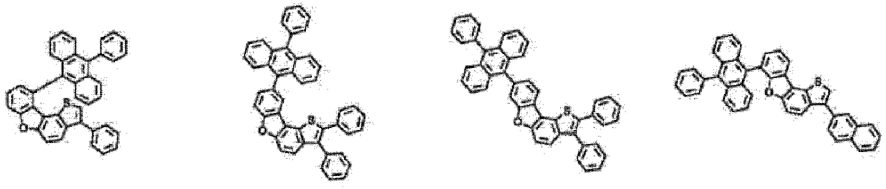

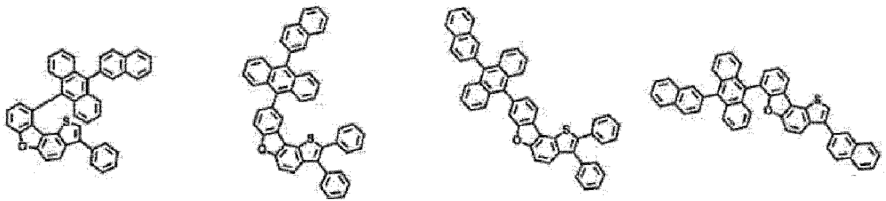

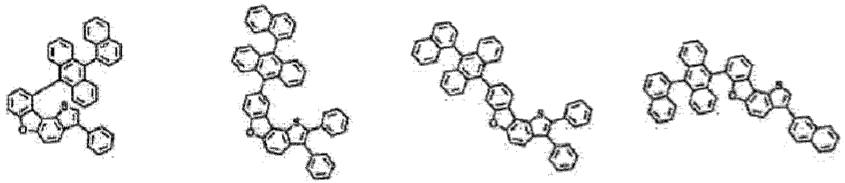

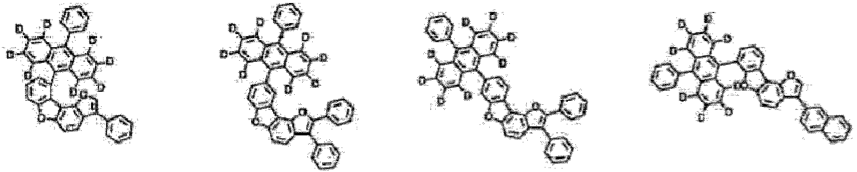

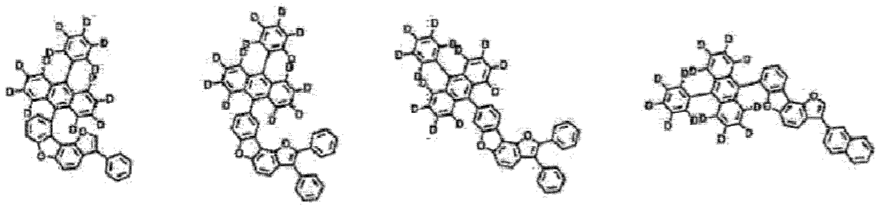

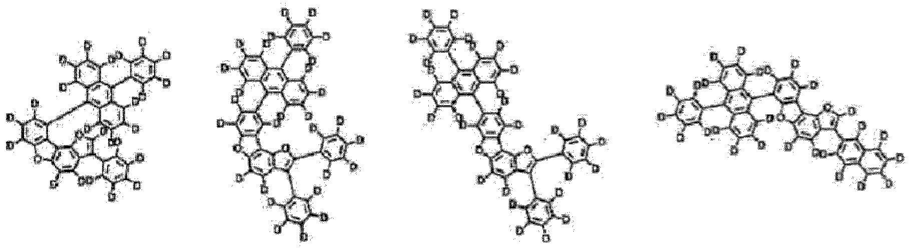

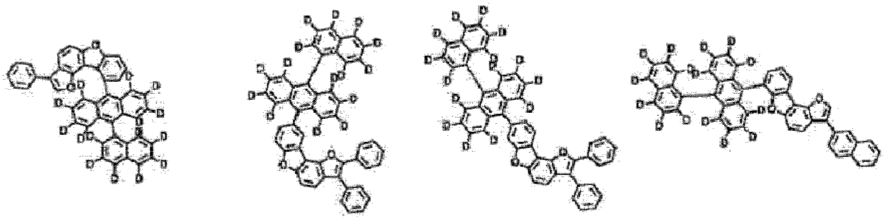

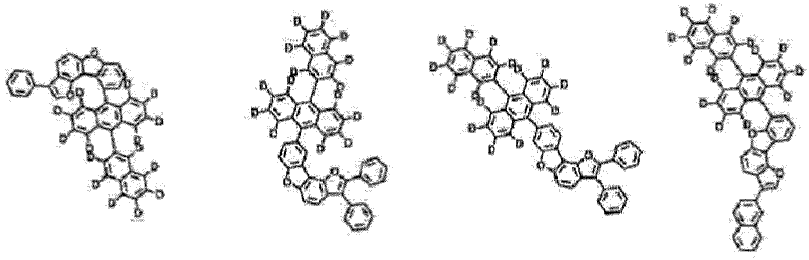

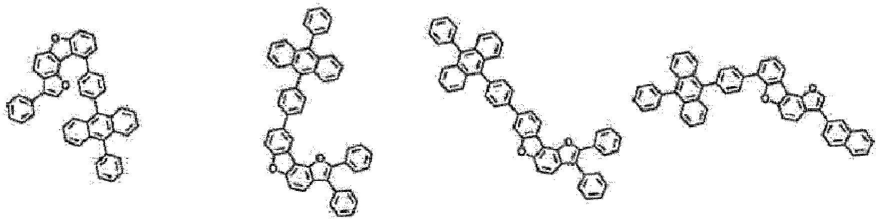

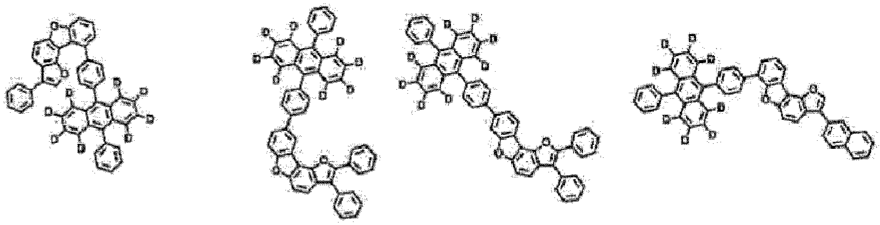

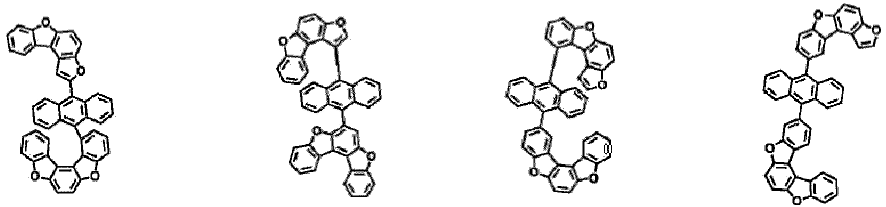

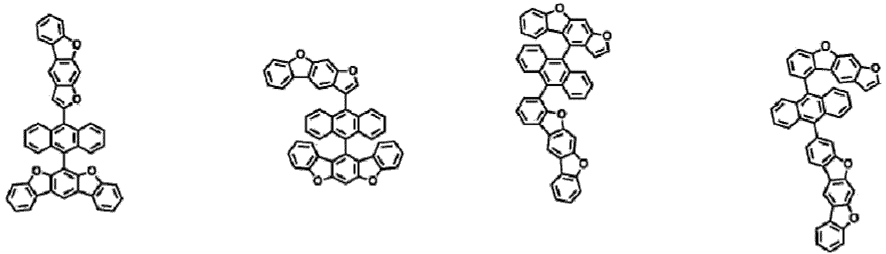

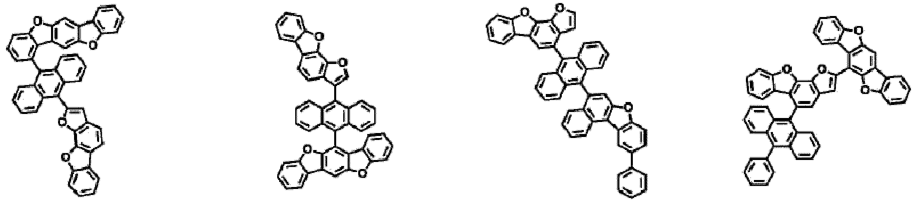

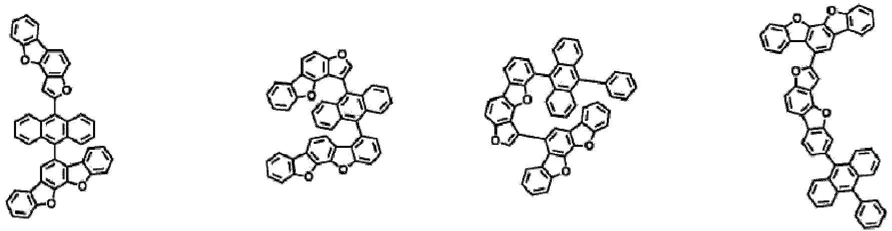

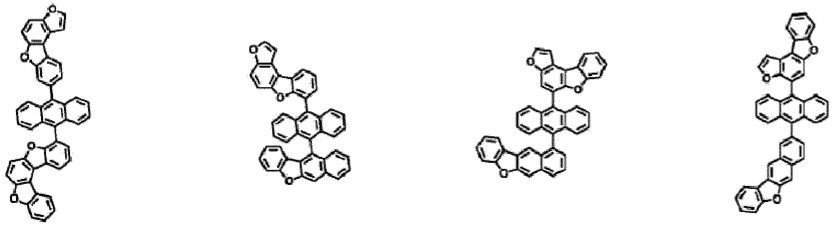

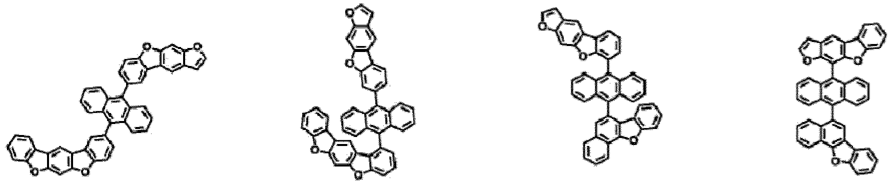

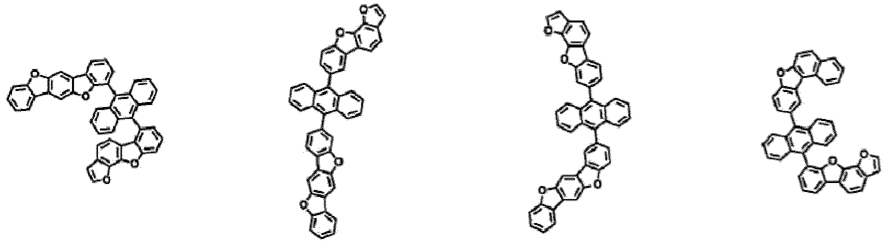

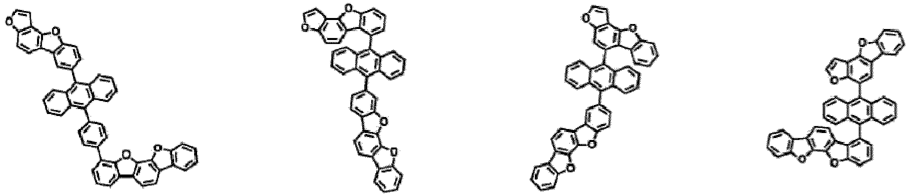

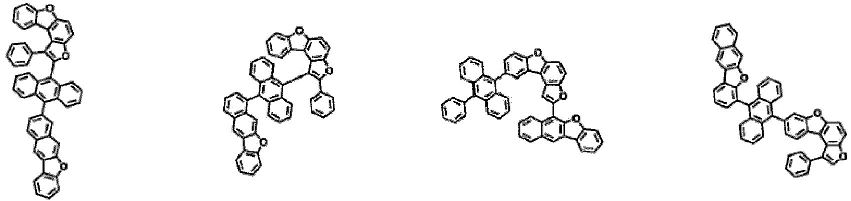

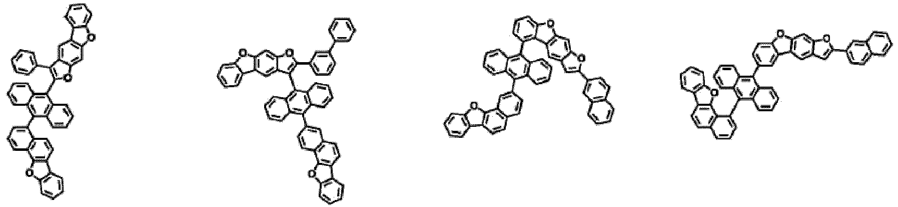

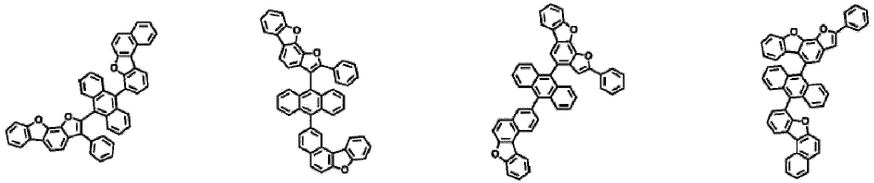

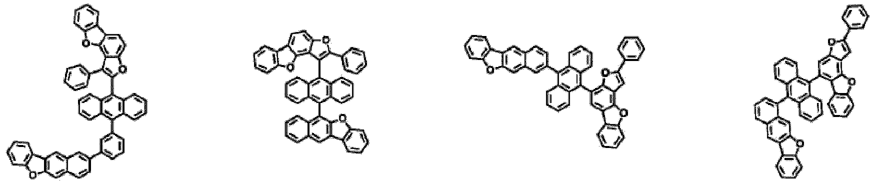

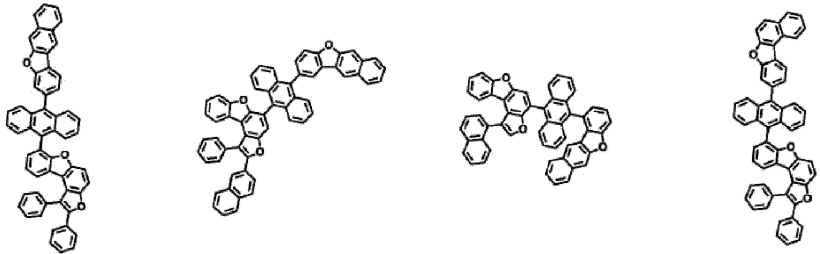

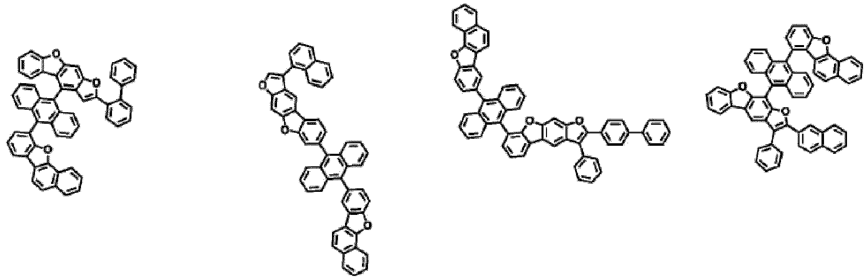

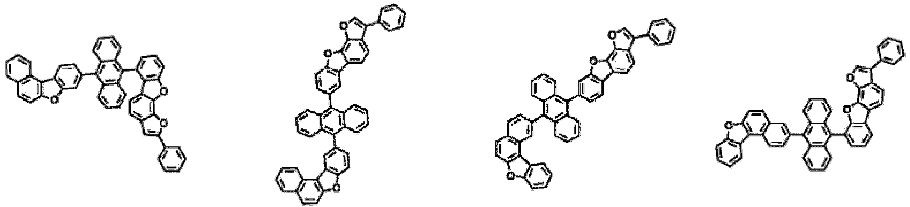

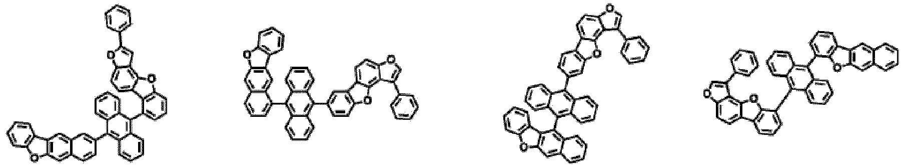

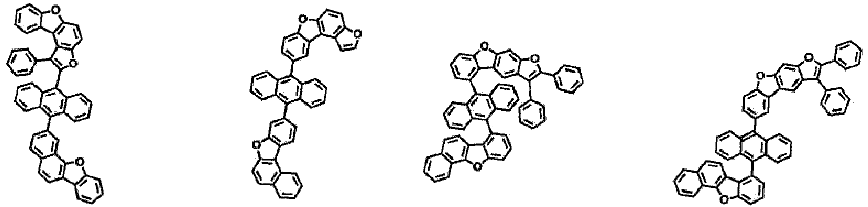

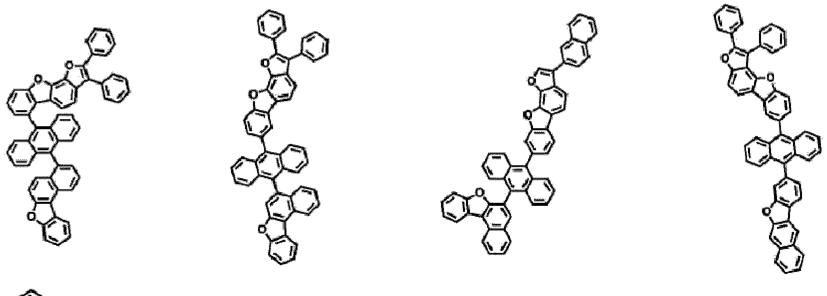

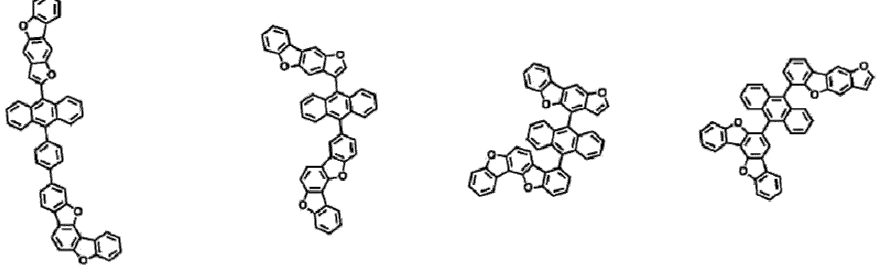

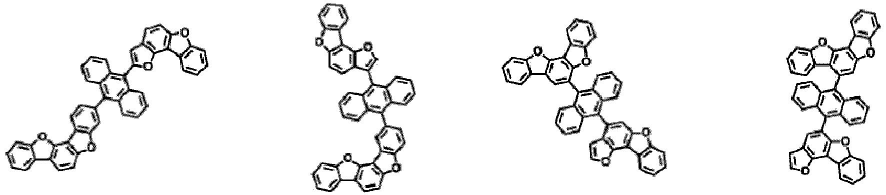

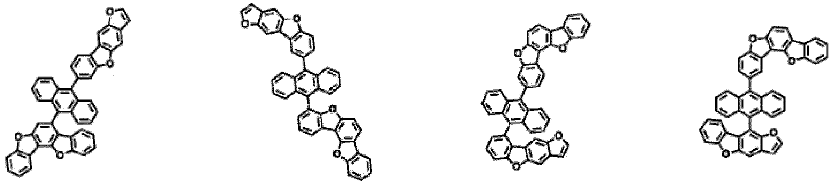

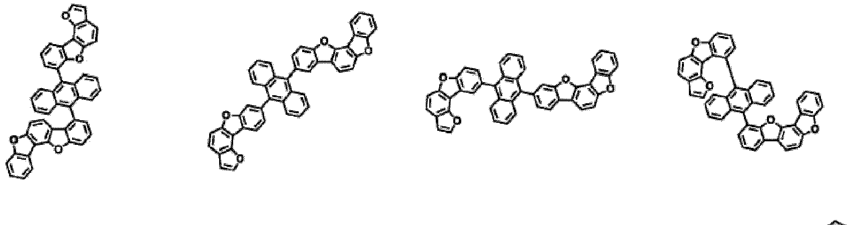

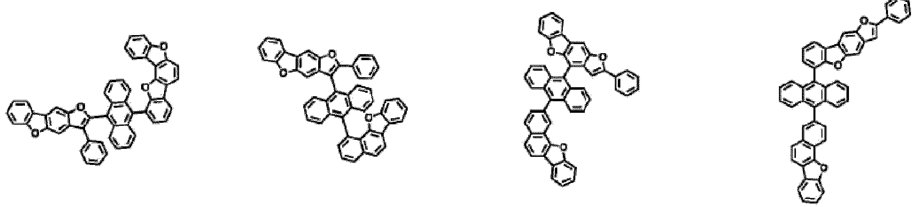

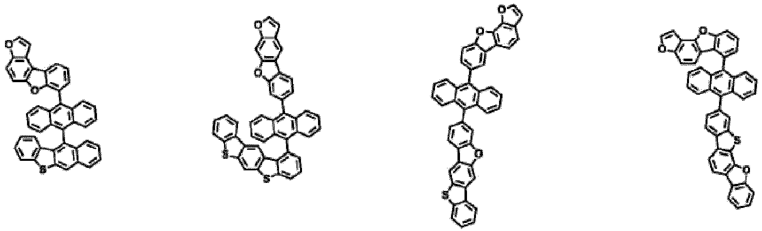

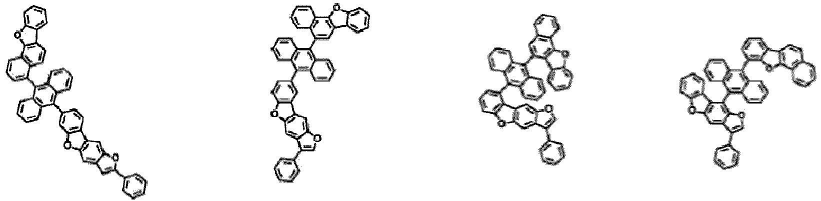

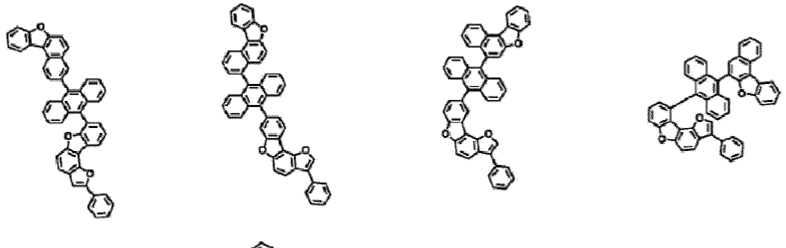

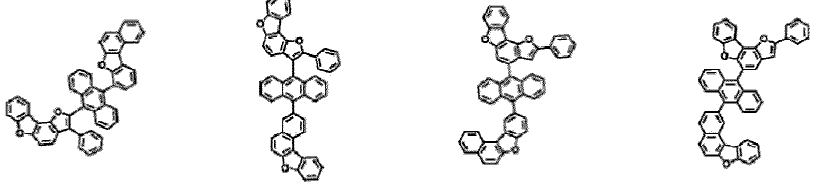

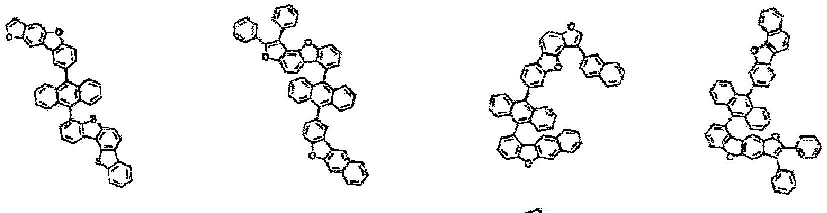

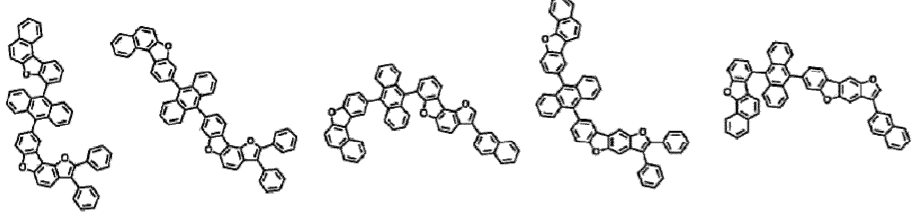

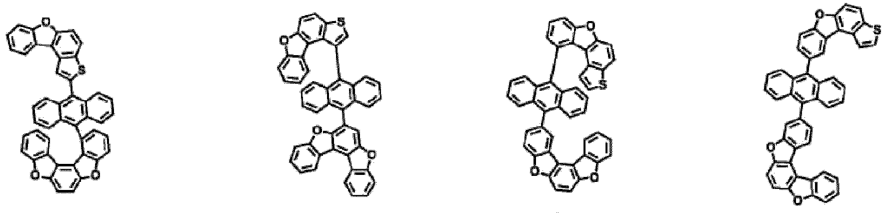

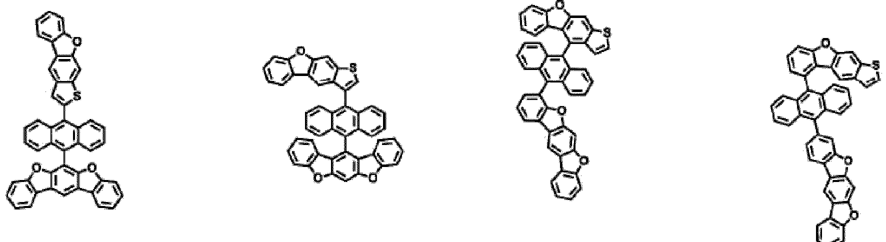

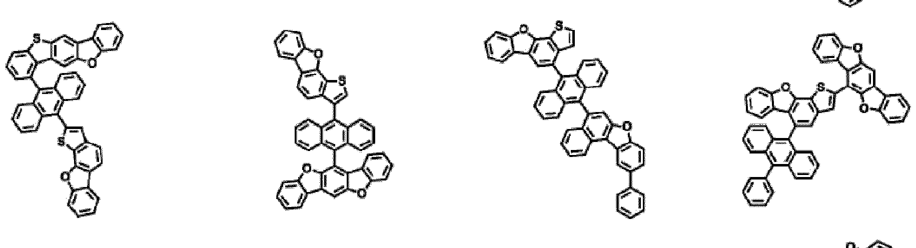

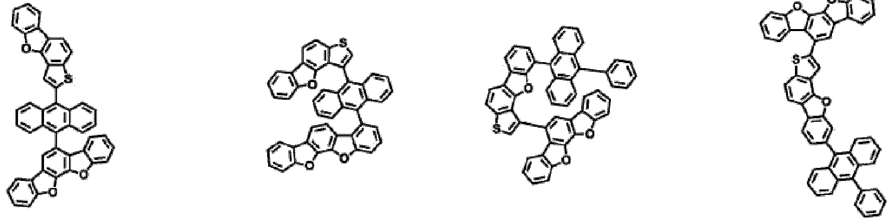

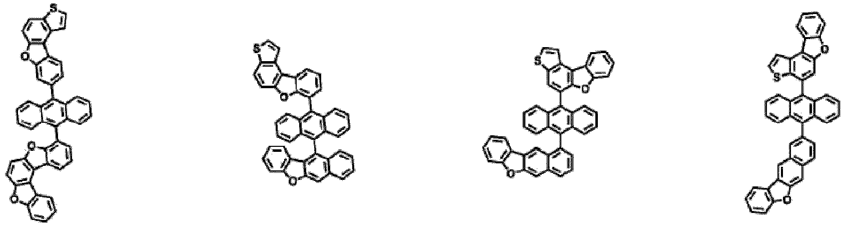

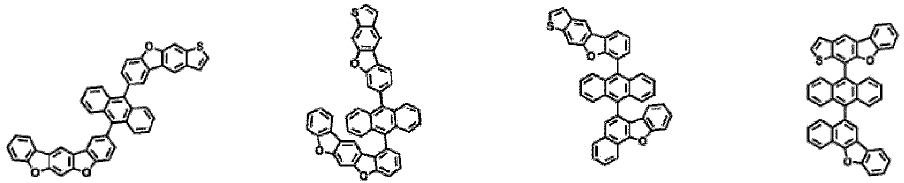

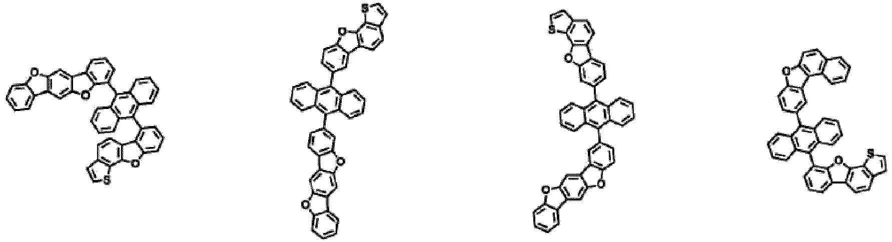

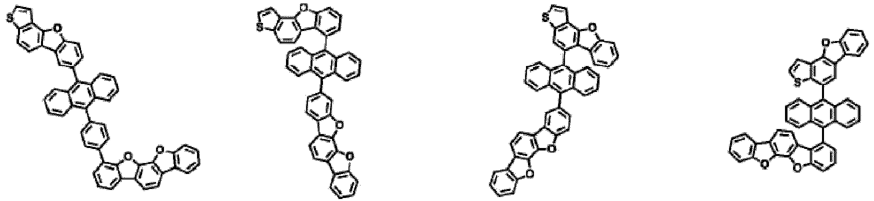

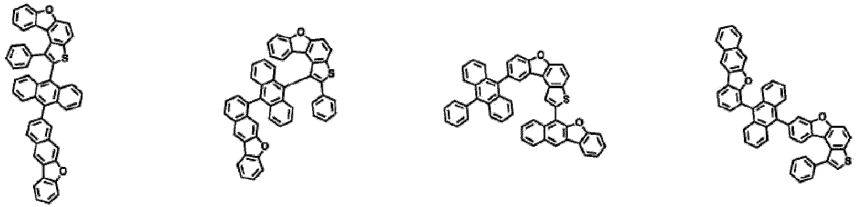

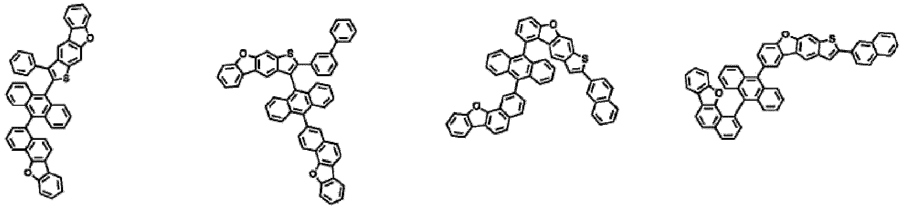

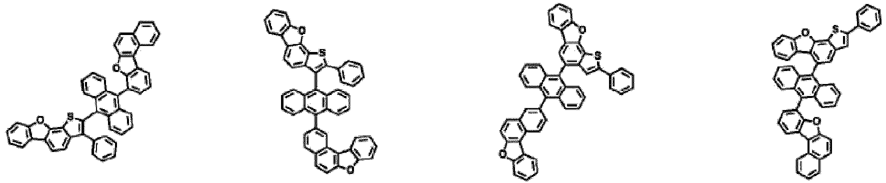

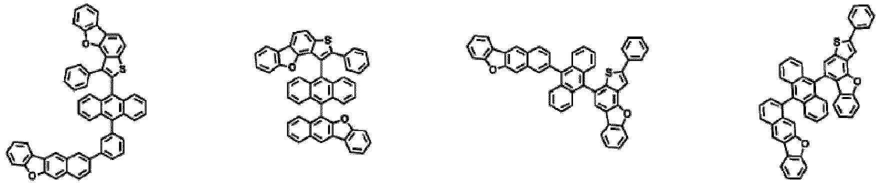

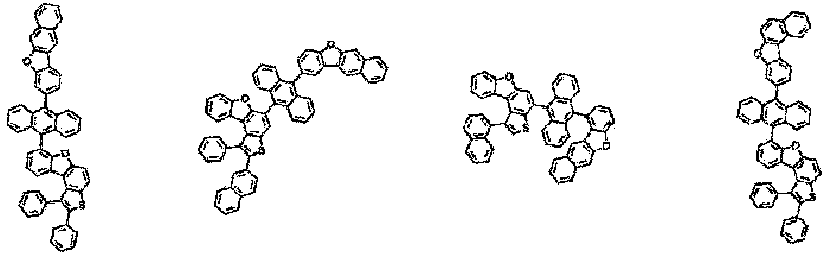

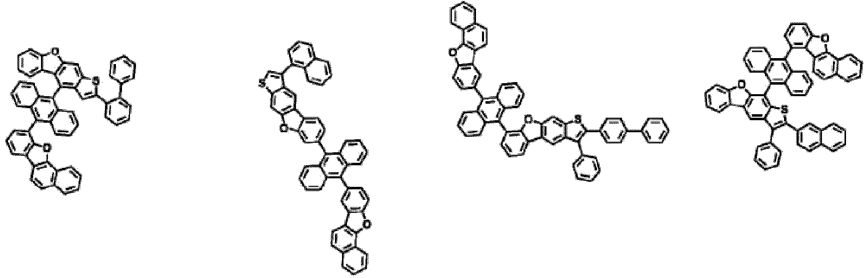

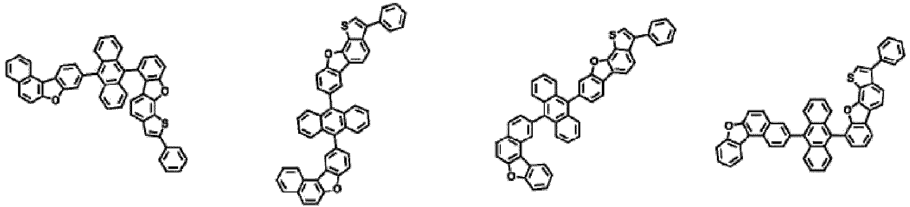

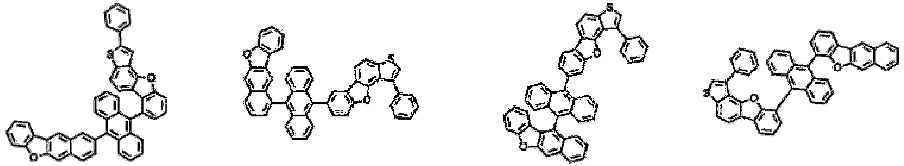

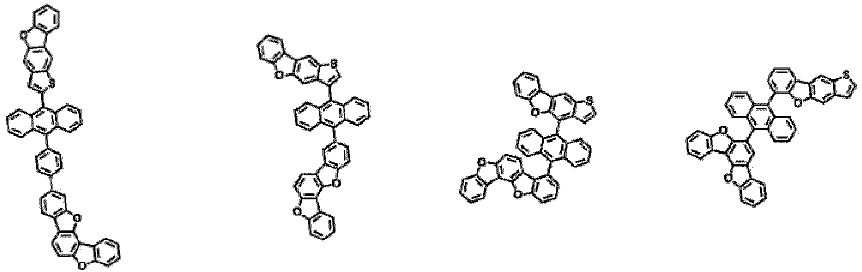

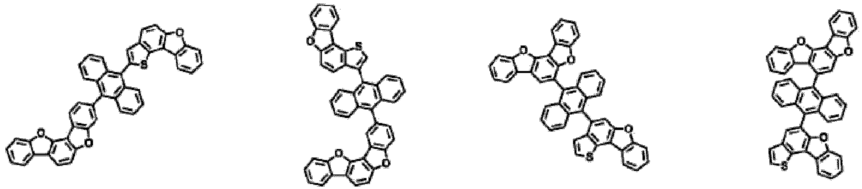

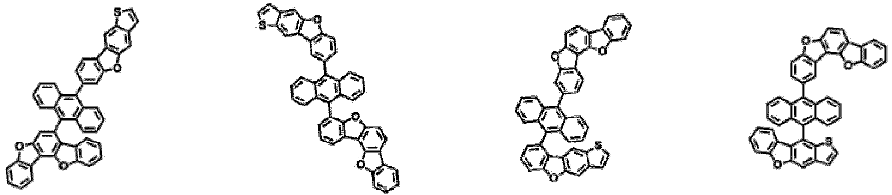

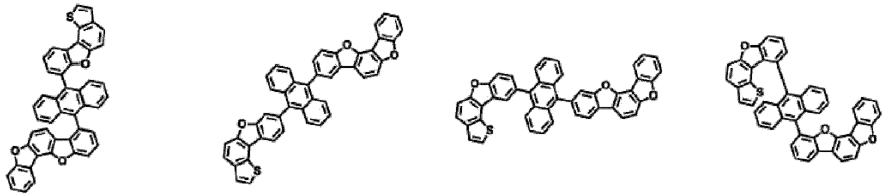

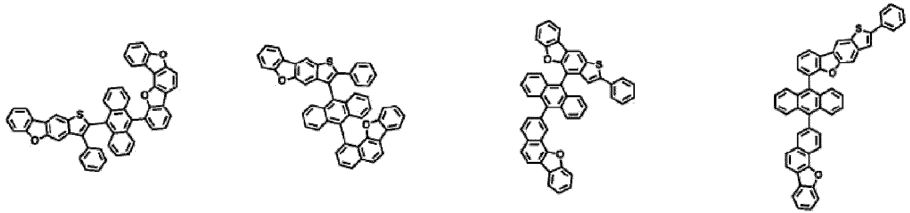

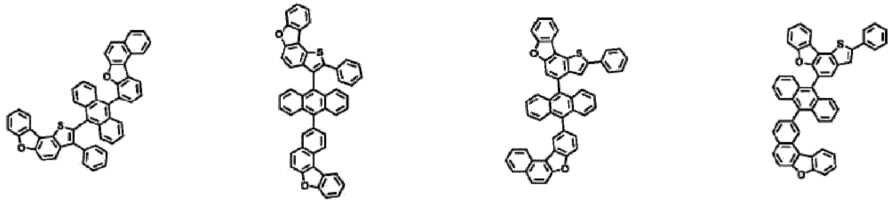

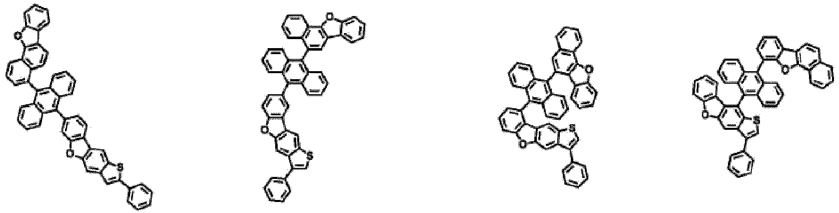

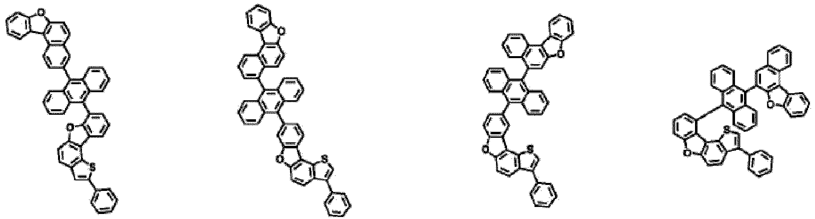

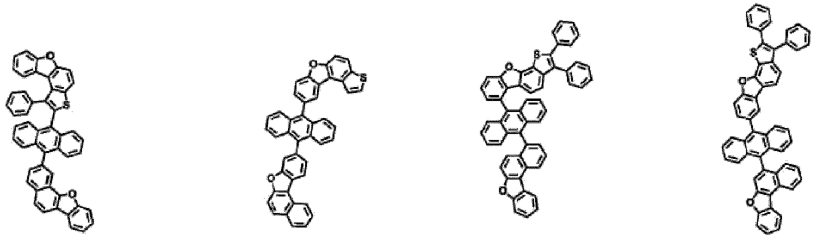

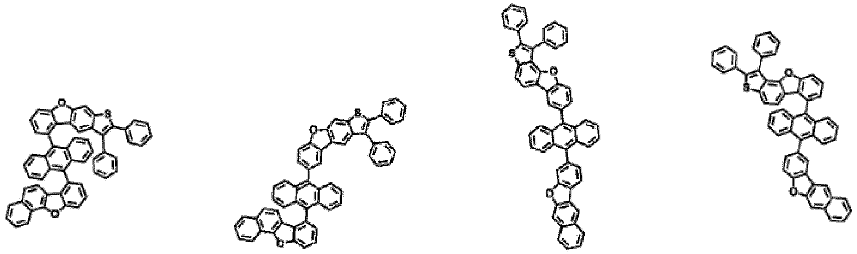

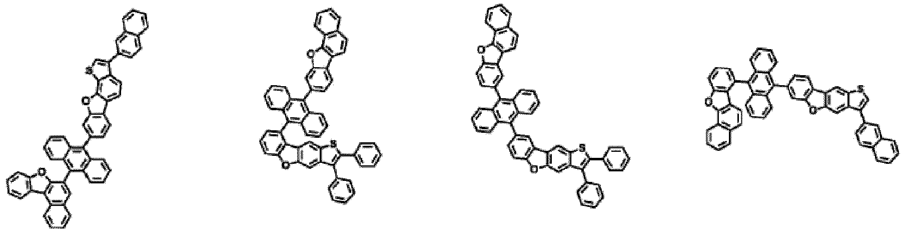

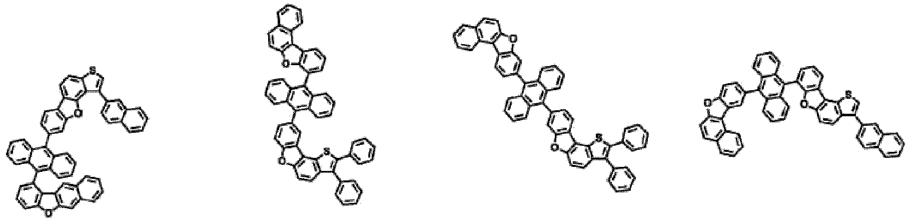

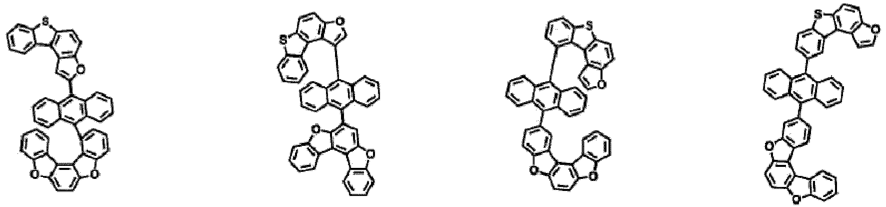

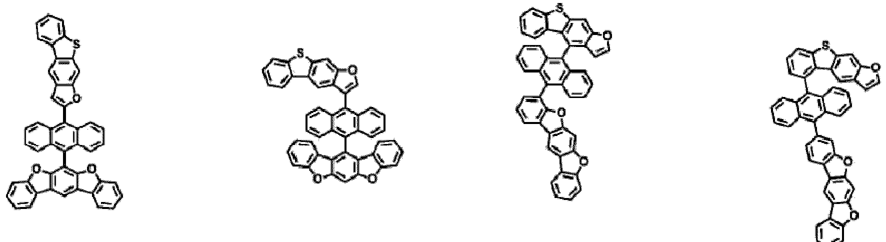

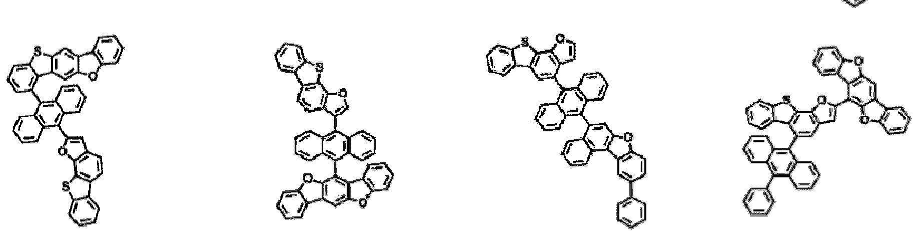

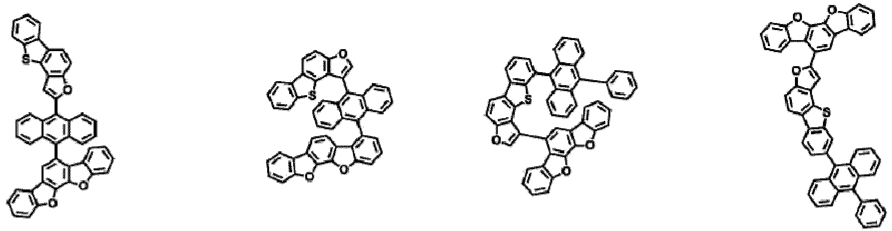

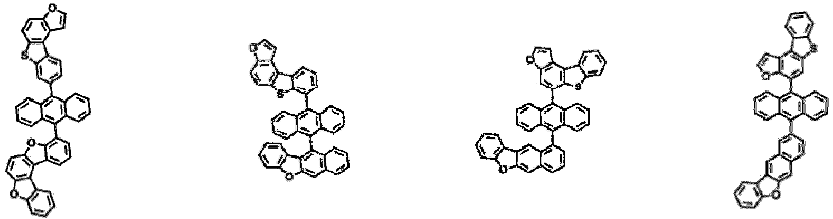

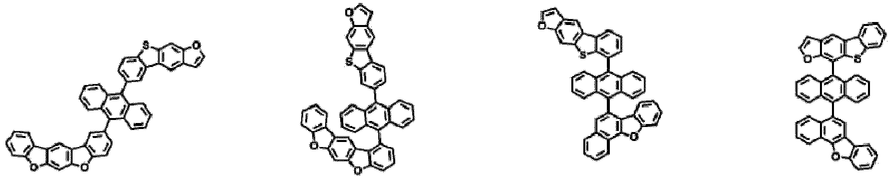

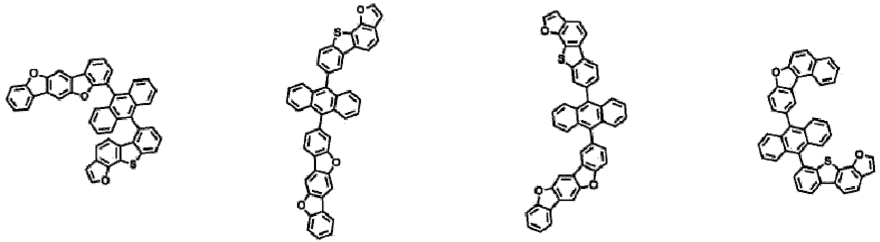

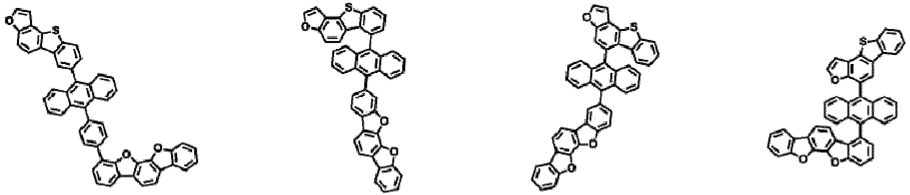

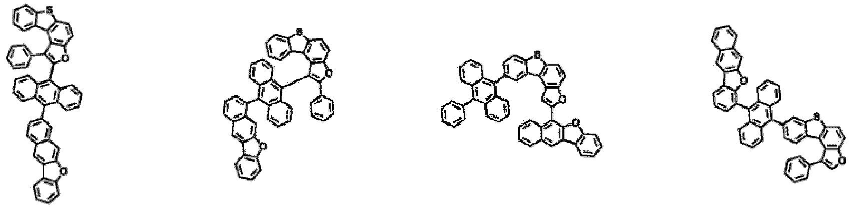

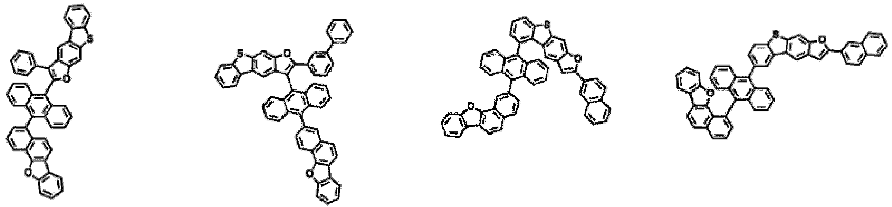

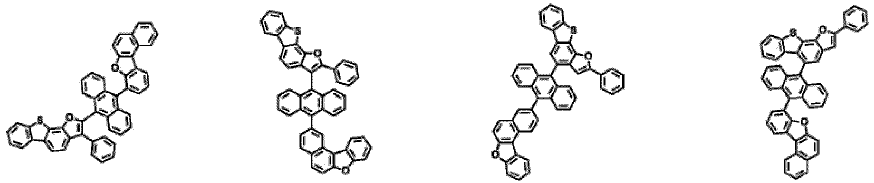

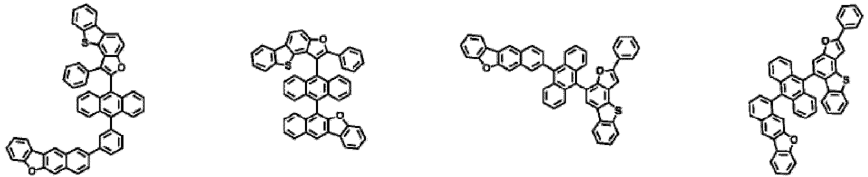

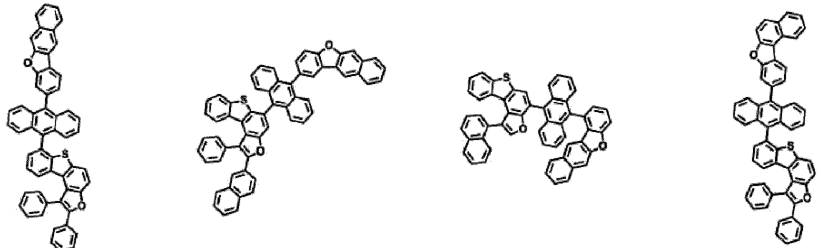

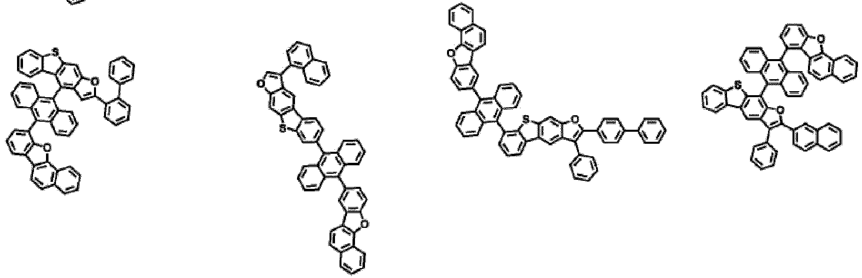

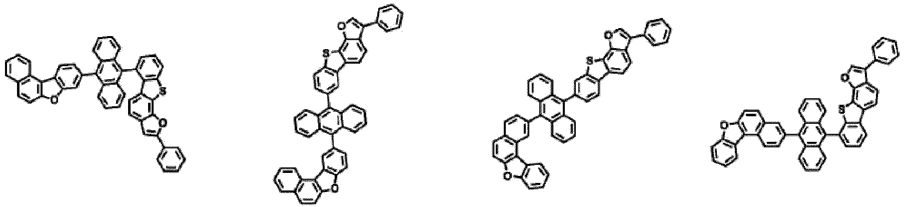

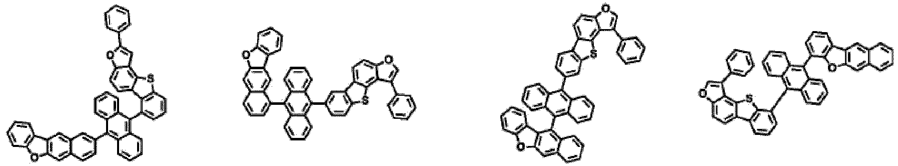

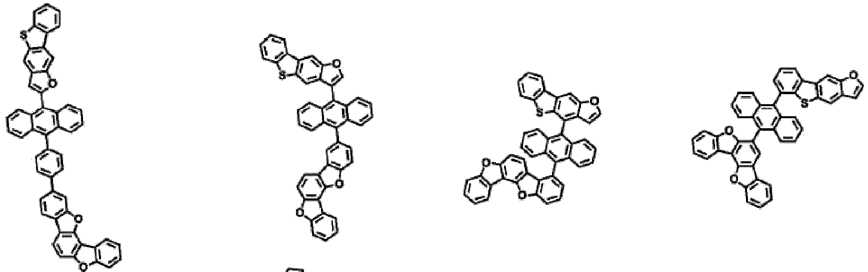

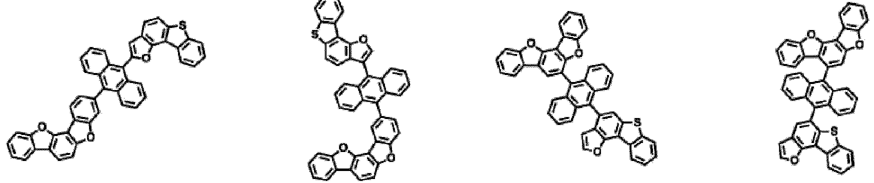

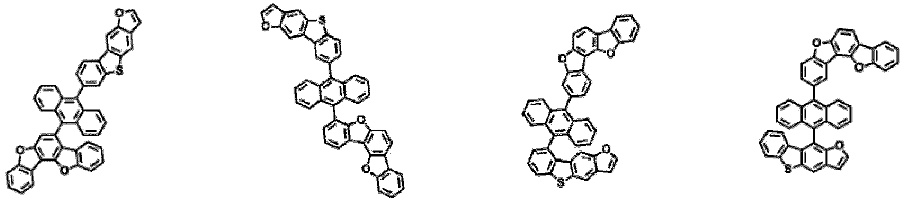

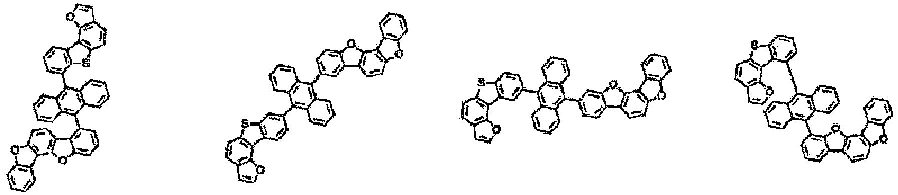

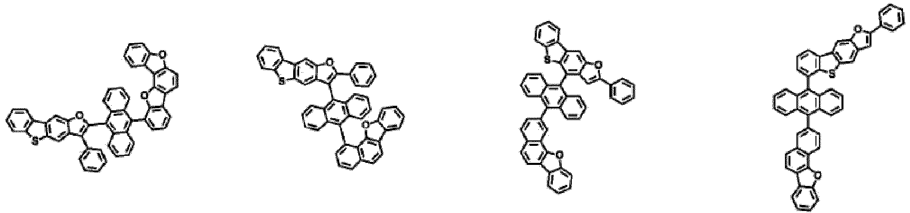

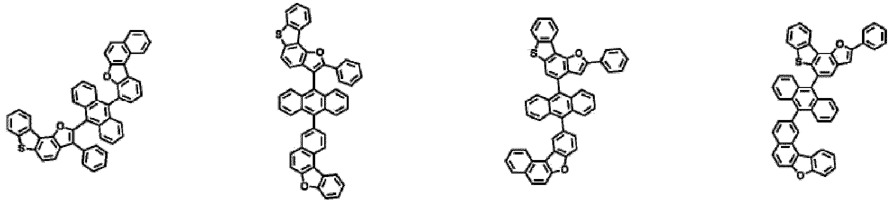

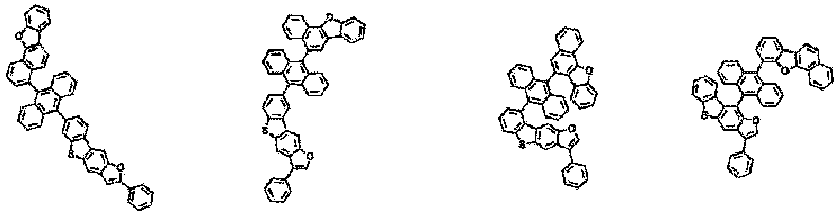

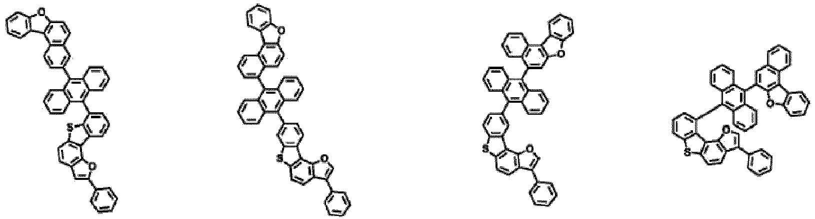

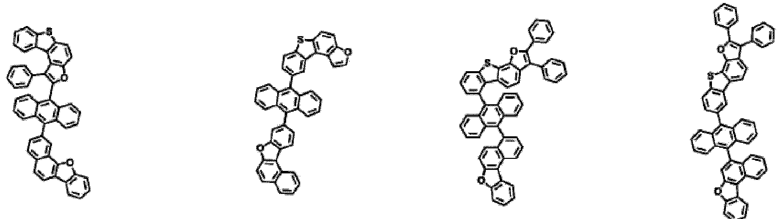

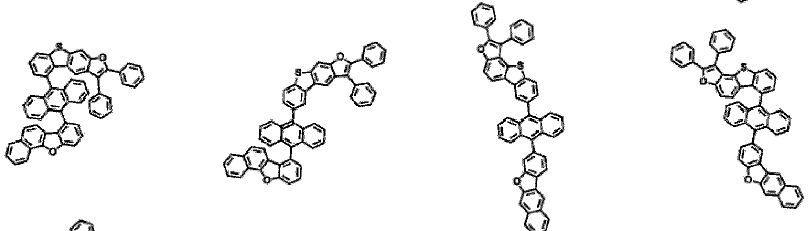

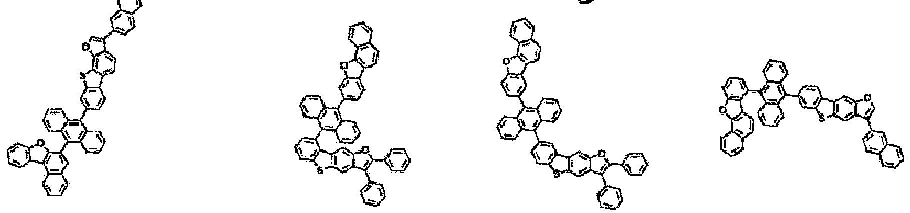

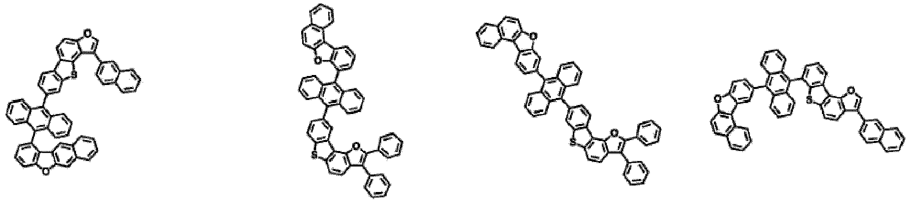

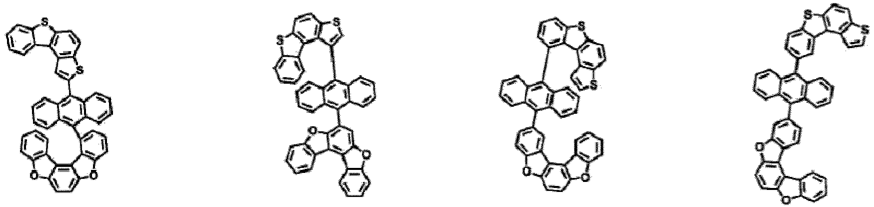

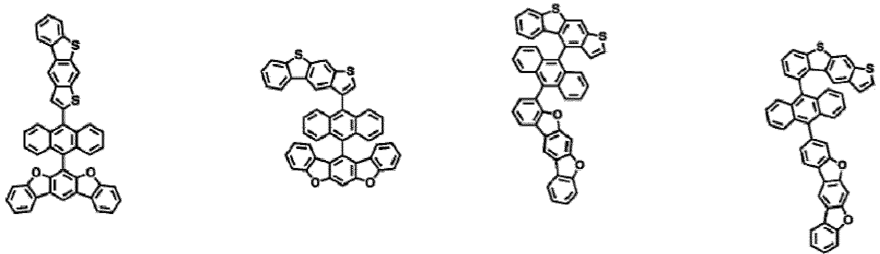

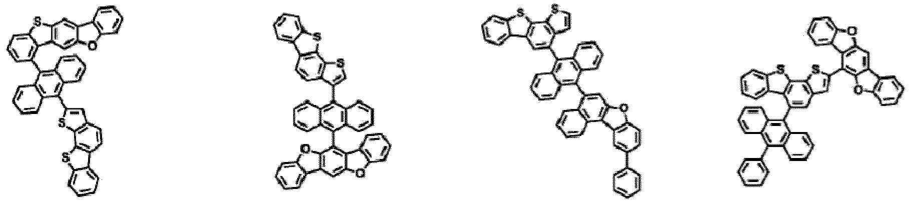

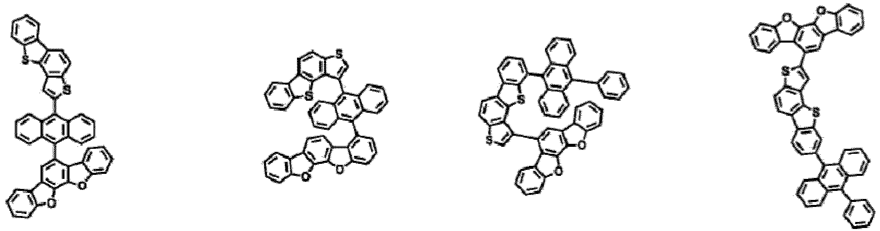

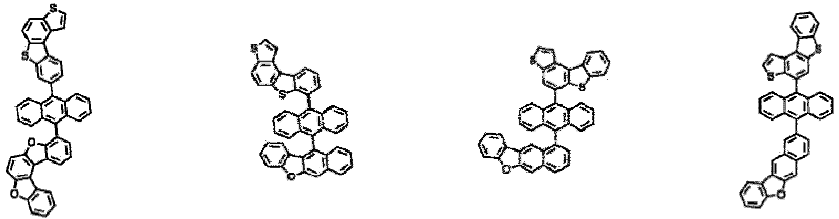

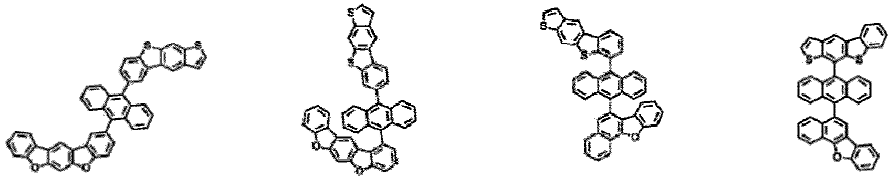

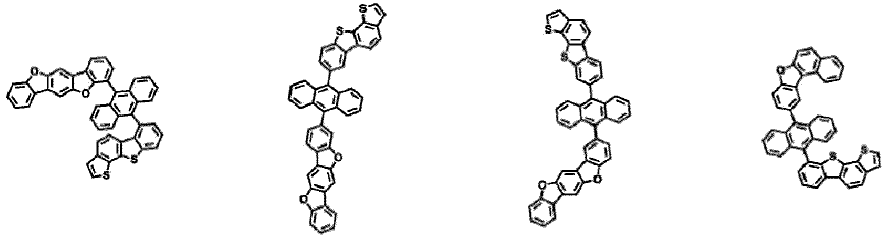

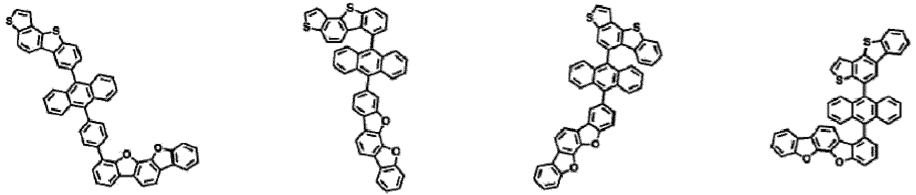

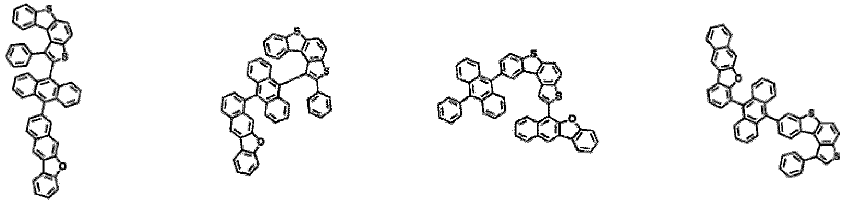

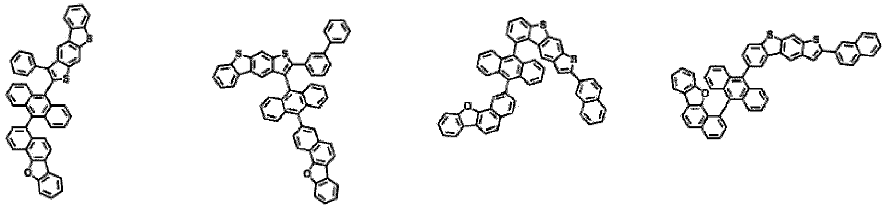

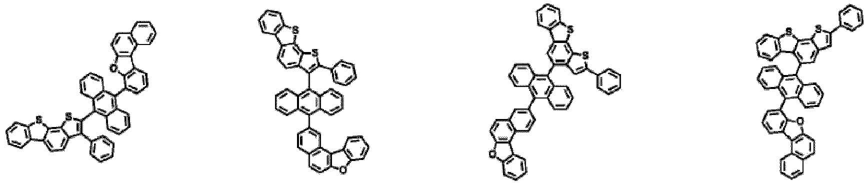

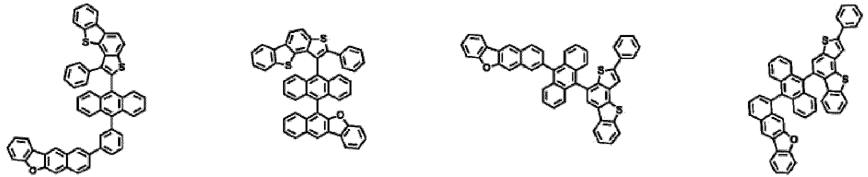

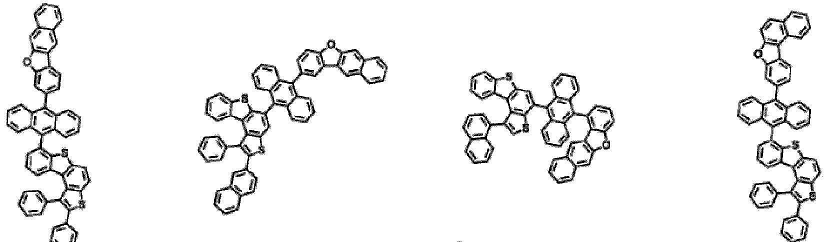

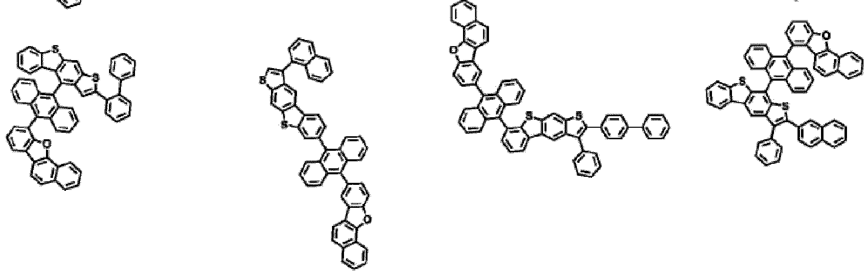

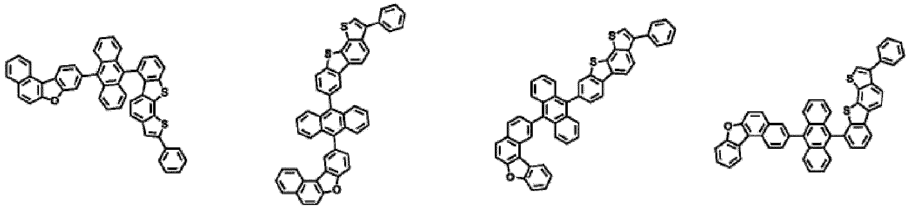

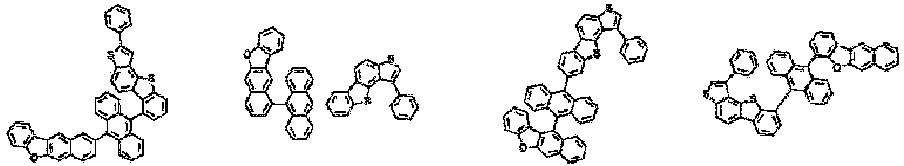

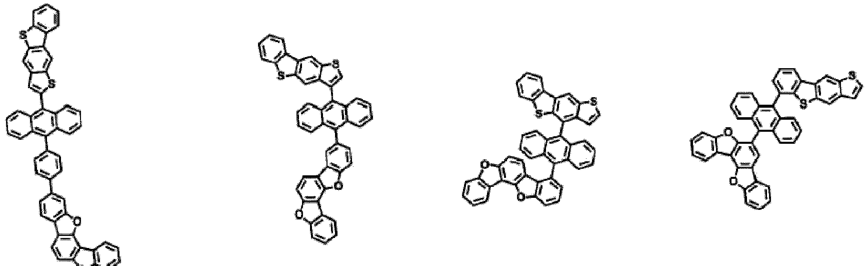

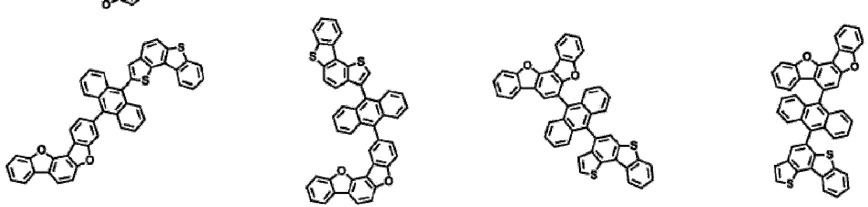

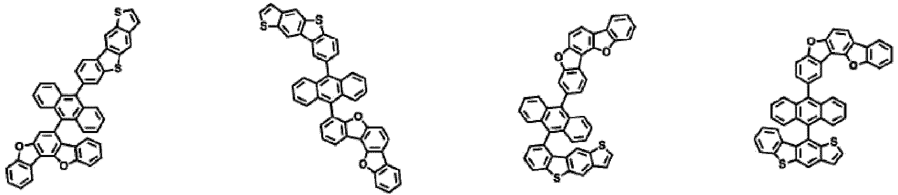

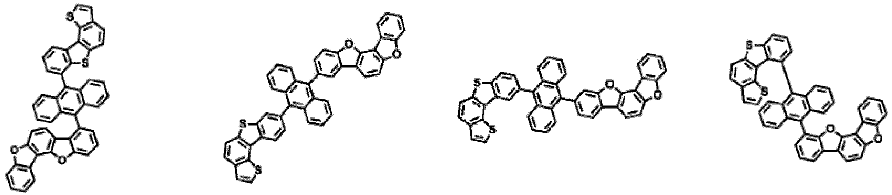

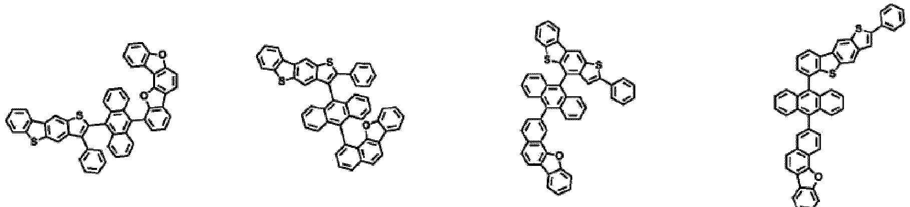

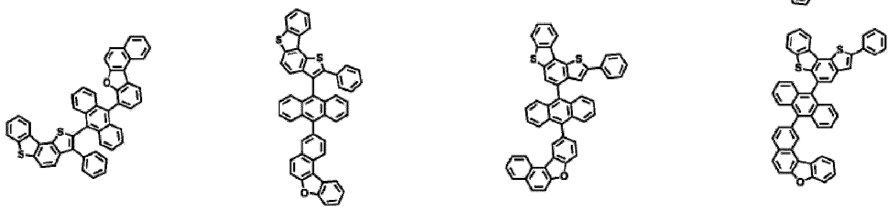

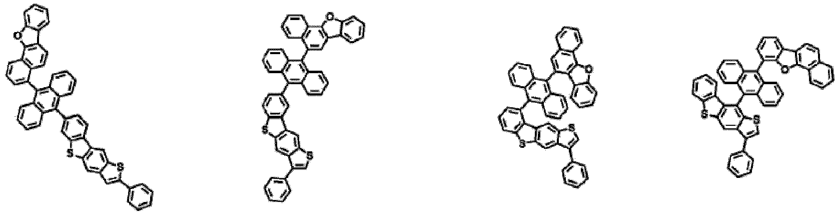

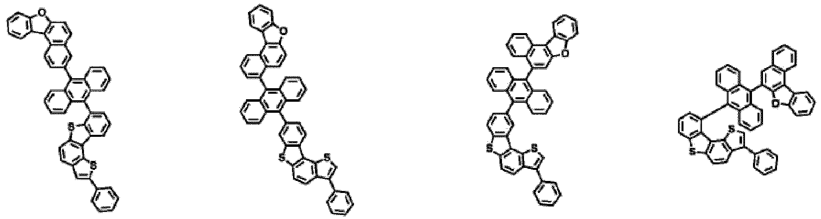

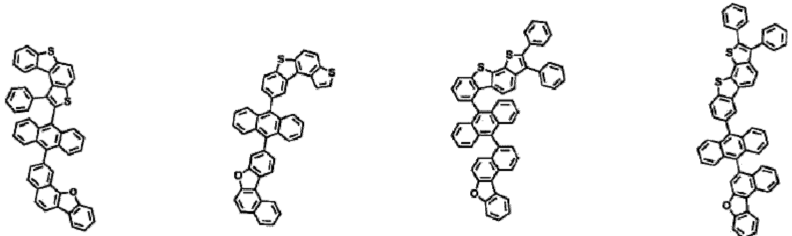

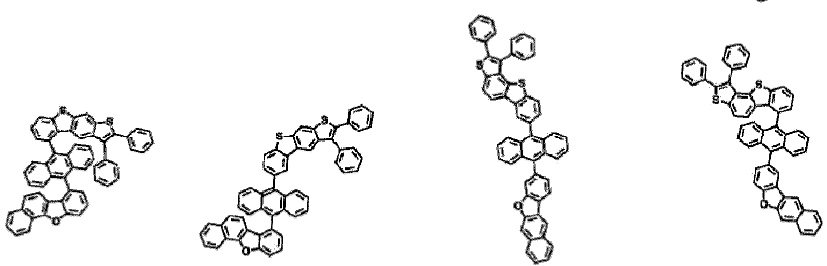

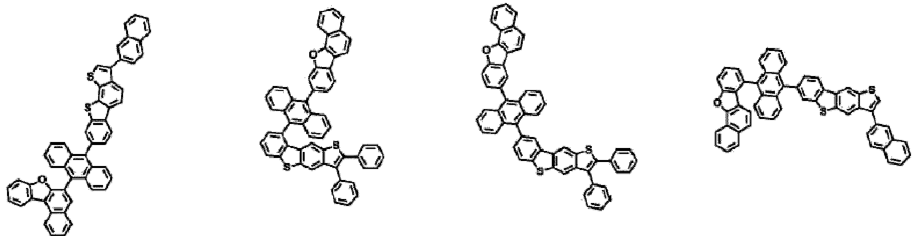

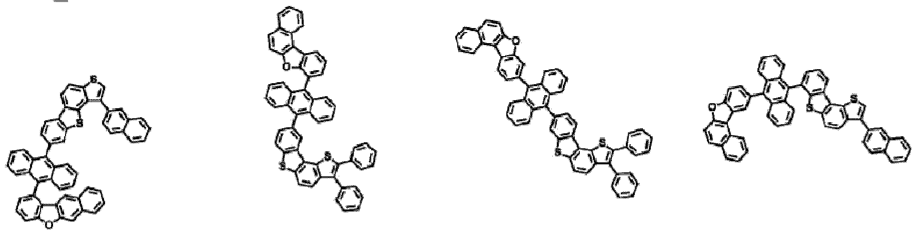

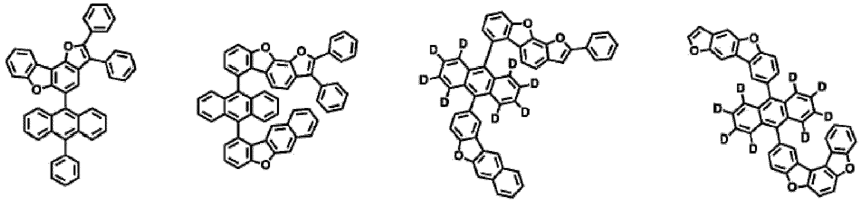

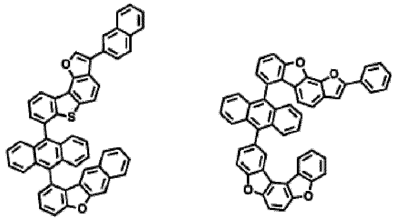

14. Dispositif électroluminescent organique comprenant :
une première électrode ;
une seconde électrode disposée de façon à être en face de la première électrode ; et
une couche de matériau organique présentant une ou plusieurs couches disposées entre la première électrode et la seconde électrode,
dans lequel une ou plusieurs couches de la couche de matériau organique comprennent le composé selon l'une quelconque des revendications 1 à 13.

15. Dispositif électroluminescent organique selon la revendication 14, dans lequel la couche de matériau organique comprend une couche électroluminescente et la couche électroluminescente comprend le composé.
